# EUROPEAN PATENT APPLICATION

(11) **EP 4 610 269 A1**
(43) Date of publication of application: **03.09.2025**
(21) Application number: 24160628.4
(22) Date of filing: 29.02.2024
(51) Int. Cl.: C07K 7/06, C07K 7/08, A61K 38/00

(54) **UROKINASE PLASMINOGEN ACTIVATOR SURFACE RECEPTOR (UPAR) LIGANDS FOR DIAGNOSTIC OR THERAPEUTIC USE**

(71) Applicant: 3B Pharmaceuticals GmbH, 12489 Berlin (DE)
(72) Inventor: The designation of the inventor has not yet been filed
(74) Representative: Bohmann, Armin K.

(57) **Abstract**

The present invention is related to a compound comprising a cyclic peptide of formula (1)

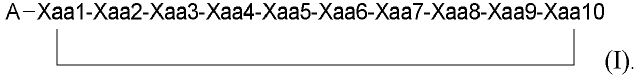

## Description

### FIELD OF THE INVENTION

The present invention is related to a chemical compound; a peptide; an Urokinase plasminogen activator surface receptor (uPAR) binding compound; an Urokinase plasminogen activator surface receptor (uPAR) binding peptide; a composition comprising the compound; a composition comprising the Urokinase plasminogen activator surface receptor (uPAR) binding compound; a composition comprising the peptide; a composition comprising the Urokinase plasminogen activator surface receptor (uPAR) peptide; the compound, the Urokinase plasminogen activator surface receptor (uPAR) binding compound, the peptide, the Urokinase plasminogen activator surface receptor (uPAR) peptide and the compositions, respectively, for use in a method for the diagnosis of a disease; the compound, the Urokinase plasminogen activator surface receptor (uPAR) binding compound, the peptide, the Urokinase plasminogen activator surface receptor (uPAR) peptide and the compositions, respectively, for use in a method for the treatment of a disease; the compound, the Urokinase plasminogen activator surface receptor (uPAR) binding compound, the peptide, the Urokinase plasminogen activator surface receptor (uPAR) peptide and the compositions, respectively, for use in a method of diagnosis and treatment of a disease which is also referred to as "thera(g)nosis" or "thera(g)nostics"; the compound, the Urokinase plasminogen activator surface receptor (uPAR) binding compound, the peptide, the Urokinase plasminogen activator surface receptor (uPAR) binding peptide, and the compositions, respectively, for use in a method for delivering a radionuclide to an Urokinase plasminogen activator surface receptor (uPAR) to a cell, preferably a uPAR overexpressing tumor cell; a method for the diagnosis of a disease using the compound, the Urokinase plasminogen activator surface receptor (uPAR) binding compound, the peptide, the Urokinase plasminogen activator surface receptor (uPAR) binding peptide and the compositions, respectively; a method for the treatment of a disease using the compound, the Urokinase plasminogen activator surface receptor (uPAR) binding compound, the peptide, the Urokinase plasminogen activator surface receptor (uPAR) binding peptide and the compositions, respectively; a method for the diagnosis and treatment of a disease which is also referred to as "thera(g)nosis" or "thera(g)nostics", using the compound, the Urokinase plasminogen activator surface receptor (uPAR) binding compound, the peptide, the Urokinase plasminogen activator surface receptor (uPAR) binding peptide and the compositions, respectively; a method for the delivery of a radionuclide to an Urokinase plasminogen activator surface receptor (uPAR) expressing tissue using the compound, the Urokinase plasminogen activator surface receptor (uPAR) binding compound, the peptide, the Urokinase plasminogen activator surface receptor (uPAR) binding peptide and the compositions, respectively.

### BACKGROUND OF THE INVENTION

Metastasis is a hallmark of cancer which is in many cases responsible for the fatal outcome of the disease. The majority of cancer deaths is caused by metastasis (Dillekas, Rogers et al. 2019). Even though new therapeutic options that support the patient's immune system to fight own cancer cells have been immensely successful for a number of cancer entities, the fraction of patients who benefit from this treatment is estimated to be below 15% (Haslam and Prasad 2019) with many solid tumors showing low susceptibility to this immune therapy. These two aspects highlight the need for new therapeutic options in cancer which specifically target invasive tumor cells which can lead to the formation and expansion of metastasis into other tissues. Since uPAR plays an important role in the remodelling of tissue and epithelial-mesenchymal transition (EMT), it is a promising target for diagnosis and treatment of aggressive tumors.

### uPAR as part of the plasminogen activation system

Urokinase plasminogen activator surface receptor (uPAR) was identified as the cell surface receptor for the Urokinase-type plasminogen activator (uPA) (Bajpai and Baker 1985, Stoppelli, Corti et al. 1985, Vassalli, Baccino et al. 1985) and isolated from a human cell line (Roldan, Cubellis et al. 1990). uPAR is also known as Monocyte activation antigen Mo3, Urokinase receptor, or CD87 (Cluster of Differentiation 87). It is a glycosylphosphotidylinositol-membrane-anchored multidomain protein and part of the plasminogen activation system and facilitates the focalization of the conversion of plasmin from the inactive precursor plasminogen (Stephens, Pollanen et al. 1989). This is achieved by the high affinity-binding of uPA.

Plasminogen is a central serine protease for the cleavage and thus activation or deactivation of enzymatic activities as collagenases, fibrin, fibronectin, thrombospondin, laminin, and von Willebrand factor. One of the factors activating Plasminogen to Plasmin is the Urokinase Plasminogen activator (uPA), another protease. The activity of uPA is focalized by its membrane bound receptor, uPAR. uPAR binds the progenitor form of uPA as well as the mature, active form of uPA which is generated by the proteolytic cleavage by Plasmin, the product of cleavage of plasminogen by uPA in a positive feedback loop (Nielsen, Hansen et al. 1982).

uPAR plays a role in tissue degradation and remodelling as well as in extravascular fibrinolysis and embryogenesis, angiogenesis, cell migration, wound healing, inflammatory response, and apoptotic cell death (McMahon and Kwaan 2015). Even though uPAR has no own connection between extracellular and intracellular space it is involved in the control of cell morphology, migratory behaviour, angiogenesis and cell proliferation by interaction with vitronectin and transmembrane receptors as Integrins or Formyl Peptide Receptors (Metrangolo, Ploug et al. 2021).

Human uPAR is a 36978 Da protein of 335 amino acids coded on chromosome 19. It consists of an N-terminal signal peptide of 22 amino acids, the Urokinase plasminogen activator surface receptor of 283 amino acids and a C-terminal peptide of 30 amino acids which is cleaved off in the mature form. The structure is characterised by 14 disulfide bonds, at least 5 glycosylation sites and a lipidation in form of a GPI-membrane anchor at amino acid 305 (www.uniprot.org/uniprotkb/Q03405; 08-NOV-2023, entry version 221, SwissProt release 2023_05/2023_05). The receptor consists of three consecutive domains of the snake toxin like family which collects short proteins with a disulphide-rich structure. uPAR is the namesake for a family of proteins which contain such domains: Ly6/PLAUR domain containing proteins. However, the sequence similarity between proteins with such domains is low and they possess different functions. There are at least 8 different Ly6/PLAUR domain containing proteins known to date. The closest relative for uPAR is Ly6/PLAUR domain-containing protein 3 with only 25% sequence identity.

Three Ly6/PLAUR domains form the binding site for uPA, while on the outside of Ly6/PLAUR domains 1 and 2 a Vitronectin binding site is formed.

Table 1 below indicates sequence identity for uPAR and uPA between species. More specifically, % identity between the human sequence and the indicated species are shown. The columns of Table 1 indicate the comparison between full length Proteins (PLAUR/uPAR; PLAU/uPA) and residues which are in the vicinity (≤5Å) of a binding ligand in a Complex structure for uPAR and a peptide (Contact Peptide; pdb1ywh) or uPA (Contact uPA, pdb4k24) and uPA with uPAR (Contact uPAR, pdb4k24).

**Table 1: Sequence identity for uPAR and uPA between species**

| | | PLAUR/UPAR | Contact Peptide | Contact UPA | PLAU/UPA | Contact UPAR |
|---|---|---|---|---|---|---|
| **MACFA** | **Macaca fascicularis** | 95.3 | 92.1 | 93.6 | 94.8 | 84.6 |
| **CANLF** | **Canis lupus familiaris** | 69.8 | 76.3 | 72.3 | 63.6 | 76.9 |
| **DELLE** | **Delphinapterus leucas** | 68.6 | 71.1 | 68.1 | 84.3 | 76.9 |
| **FELCA** | **Felis catus** | 68 | 68.4 | 68.1 | 69.6 | 76.9 |
| **PIG** | **Sus scrofa** | 67.2 | 71.1 | 68.1 | 83.6 | 76.9 |
| **MYOLU** | **Myotis lucifugus (bat)** | 66.6 | 63.2 | 61.7 | 81.9 | 84.6 |
| **CAPHI** | **Capra hircus (goat)** | 61 | 52.6 | 44.7 | 81.5 | 76.9 |
| **SHEEP** | **Ovis aries** | 60.7 | 52.6 | 44.7 | 82.6 | 76.9 |
| **BOVIN** | **Bos taurus** | 60.7 | 57.9 | 46.8 | 81.3 | 69.2 |
| **MOUSE** | **Mus musculus** | 60.7 | 55.3 | 55.3 | 76.9 | 69.2 |
| **RAT** | **Rattus norvegicus** | 60.4 | 52.6 | 61.7 | 78.4 | 69.2 |
| **CAVPO** | **Cavia porcellus** | 58.7 | 60.5 | 48.9 | 78.4 | 61.5 |

Sequence similarity between species is moderate and even the binding pocket for uPA is not totally conserved. This is also the case for the natural ligand uPA (Table 1). This leads to species specificity for the interaction with e.g. a ~80-fold lower affinity for the human-uPAR - mouse-uPA interaction (Lin, Gårdsvoll et al. 2010).

uPAR is internalized as a complex with its ligands uPA and PAI-1 mediated by LRP1 (Conese, Nykjaer et al. 1995, Nykjaer, Conese et al. 1997). However, a second ligand independent constitutive way of internalization and recycling has been observed (Cortese, Sahores et al. 2008).

Two mechanisms lead to 5 different uPAR forms in organisms: the proteolytic cleavage of the full-length form behind an Arginine in position 105 or 111 in the linker region between domains 1 and 2 by its ligand uPA and the cleavage of the GPI anchor to a soluble form of uPAR (suPAR). Aditional to the membrane bound full-length uPAR the cleavage products are (1) soluble full length uPAR (suPAR), (2) membrane bound domains 1 and 2, (3) soluble domains 1 and 2 and (4) soluble domain 1.

Upon proteolytic cleavage of domain 1 both products lose the ability to bind to ligands as uPA or Vitronectin (Høyer-Hansen, Rønne et al. 1992). Soluble uPAR (suPAR) can also be a result of alternative splicing (Stewart and Sayers 2009). suPAR has been established as a prognostic marker for acute medical patients, kidney disease, infections and more (Thuno, Macho et al. 2009, Rasmussen, Ladelund et al. 2018). Also in cancer patients, suPAR levels are frequently elevated (Paraskevas, Mulita et al. 2022).

Apart from its physiological role, uPAR is involved in pathological processes. Elevated uPAR levels are observed in cardiac fibrosis (Saxena, Izmirly et al. 2015), chronic liver disease and Hepatidis B (Zimmermann, Koch et al. 2012, Akdogan, Atak Yucel et al. 2019), Cystic fibrosis, COPD (Xiao, Hsu et al. 2005), idiopathic pulmonary fibrosis (Desai, Mattson et al. 2011), glomerulosclerosis (Trimarchi, Canzonieri et al. 2017, Chebotareva, Vinogradov et al. 2022), systemic sclerosis (suPAR; (Legany, Toldi et al. 2015)), rheumatoid arthritis (Fibbi, Pucci et al. 1998), neurologic disorders as autism spectrum disorder or epileptogenic tissue remodelling (Campbell, D'Oronzio et al. 2007, Campbell, Li et al. 2008, Lahtinen, Huusko et al. 2009).

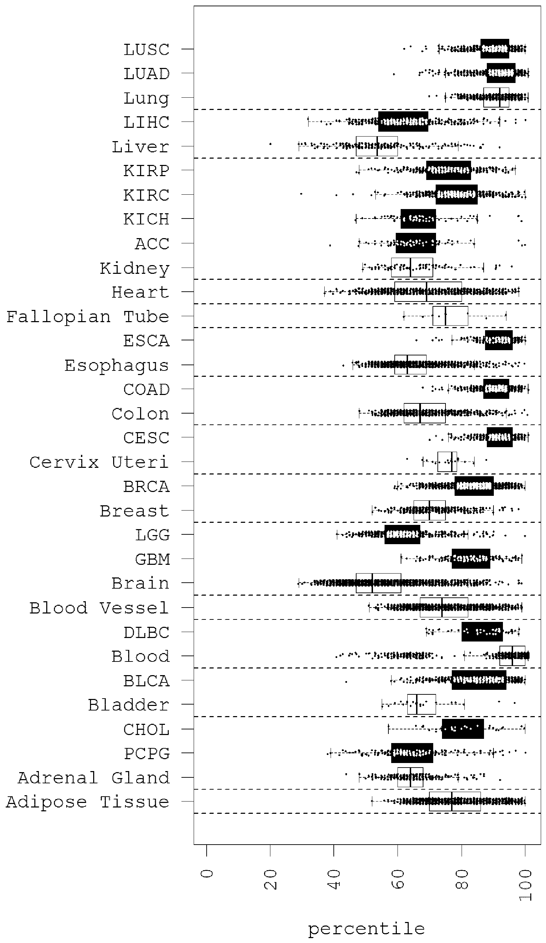

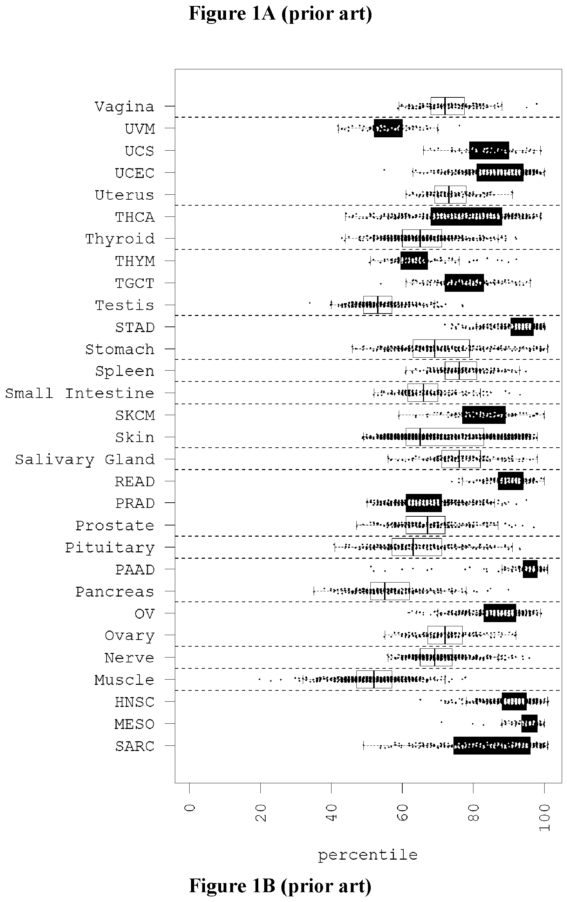

uPAR expression in different cancers is extensively studied and it has a role in tumor growth, angiogenesis, tumor cell invasion, migration, epithelial-mesenchymal transition (EMT), and has been associated with the development of metastatic phenotypes (Andreasen, Egelund et al. 2000, Lund, Illemann et al. 2011, Madunić 2018, Mahmood, Mihalcioiu et al. 2018, Paraskevas, Mulita et al. 2022). In most cancers uPAR-expression is at higher levels as in the comparable healthy tissue (see, Figure 1).

Elevated uPAR expression is indicative for increased aggressiveness, postoperative progression, metastasis, poor response to therapy and poor prognosis in multiple cancer types as breast, prostate, ovarian, colorectal or lung cancer (Mahmood, Mihalcioiu et al. 2018).

### Rationale for Targeting uPAR

Given the expression pattern of uPAR in healthy and tumor tissues and the role of uPAR for tissue reorganization and promotion of invasive tumor growth and metastasis, its targeting presents a promising strategy for cancer diagnosis, stratification and therapy. Since uPAR is mainly expressed in tumor or tumor-related cells as tumour-associated macrophages, neutrophils, fibroblasts, endothelial and disseminated tumour cells (Smith and Marshall 2010), it represents an attractive tumor-specific target, reducing potential off-target effects.

### State of the Art

Multiple approaches are used for targeting uPAR. Different Antibodies have been developed. The monoclonal antibody ATN-658 (huATN-658; MNPR-101) is developed for diagnosis and treatment of cancer and infectious disease (Van Buren, Gray et al. 2009, Mahmood, Arakelian et al. 2020) (WO002005116077, WO002007134274, WO002021257552) including as targeting moiety to carry alpha particle-emitting radionuclides.

Other uPAR-directed antibodies for the diagnosis and/or treatment of cancer, autoimmune disease are described in the literature (Elvin, Foltz et al. 2009) (WO002007120693; Amgen/AstraZeneca), (Duriseti, Goetz et al. 2010 WO002011100620, LeBeau, Duriseti et al. 2013, Lu, Cong et al. 2021, WO002023125842), targeting deletion variants of uPAR (WO002002082077, Luther, Kotzsch et al. 2003) and for targeting uPAR-expressing cells for Immunotherapy of senescence related diseases (Amor, Feucht et al. 2020, WO002022261405).

Also proteins have been used as targeting moieties or for the inhibition of the uPA/uPAR interaction. These were mostly based on the sequence of the uPAR-binding fraction in the amino-terminal fragment (ATF) of uPA and focus on diagnosis or treatment of cancer or infectious disease (WO002001025410, Guo, Higazi et al. 2000, WO002002058714, Alfano, Sidenius et al. 2002). Different combinations of proteins which bind and block the uPA-binding site of uPAR and comprise a second functionality as binding of the Vitronectin binding site of uPAR (WO002012085076) or Cytokines (WO002022204267) or comprising an additional transmembrane domain, and an intracellular signaling domain for targeting immunotherapy (WO002022261398). The second functionality can also be a contrast agent for imaging of tumors (Yang, Mao et al. 2009, Yang, Sajja et al. 2013) or toxins for the targeted destruction of tumor cells (Rustamzadeh, Li et al. 2003, Hall 2006).

Also small molecules for the disruption of the uPA/uPAR (Mani, Wang et al. 2013, Liu, Xu et al. 2017, Zhou, Bum-Erdene et al. 2018, Bum-Erdene, Liu et al. 2021) and the Vitronectin/uPAR interaction (Rea, Lavecchia et al. 2013, WO002021198844) have been developed.

The uPAR binding region of uPA was identified (Appella, Robinson et al. 1987) and respective peptides were optimized (Magdolen, Burgle et al. 2001). Bacteriophage phage display was used for the identification of alternative peptide sequences for binding in the uPA site of uPAR which were optimized and resulted in a peptide AE105 with a K_{d} of 0.36 nM (Goodson, Doyle et al. 1994, Ploug, Ostergaard et al. 2001). AE105 was extensively investigated as targeting moiety for magnetic resonance- and near infrared-guided photothermal therapy and surgery for tumors (Li, Wang et al. 2021, WO002021130237), and for radioligand therapy (WO002014086364) in animals (Li, Niu et al. 2008, Persson, Madsen et al. 2012, Persson, Madsen et al. 2012, Persson, Rasmussen et al. 2012, Persson, Liu et al. 2013, Persson, Juhl et al. 2014) as well as in humans (Persson, El Ali et al. 2014, Persson, Skovgaard et al. 2015, Skovgaard, Persson et al. 2017, Skovgaard, Persson et al. 2017, Skovgaard, Persson et al. 2017, Fosbøl, Kurbegovic et al. 2020, Fosbol, Kurbegovic et al. 2021, Carlsen, Loft et al. 2022, Risør, Clausen et al. 2022).

AE105 was also used in modified forms as dimers (Knor, Sato et al. 2008) and cyclized (Leth, Newcombe et al. 2023).

The problem underlying the present invention is the provision of a compound which is suitable as a diagnostic agent and/or a pharmaceutical agent, particularly if conjugated to a diagnostically and/or therapeutically active effector. A further problem underlying the present invention is the provision of a compound which is suitable as a diagnostic agent and/or a pharmaceutical agent, particularly if conjugated to a diagnostically and/or therapeutically active effector, whereby the compound is a potent binder of urokinase plasminogen activator surface receptor (uPAR); preferably the pIC50 of the compound is equal to or greater than 6.0, preferably equal or greater than 7.0. A further problem underlying the present invention is the provision of a compound which is suitable as a diagnostic agent and/or a pharmaceutical agent, particularly if conjugated to a diagnostically and/or therapeutically active effector, in the diagnosis and/or therapy of a disease where the diseased cells and/or diseased tissues express urokinase plasminogen activator surface receptor (uPAR). A still further problem underlying the instant invention is the provision of a compound which is suitable for delivering a diagnostically and/or therapeutically effective agent to a diseased cell and/or diseased tissue, respectively, and more particularly a urokinase plasminogen activator surface receptor (uPAR)-expressing diseased cell and/or diseased tissue, preferably the diseased tissue comprises or contains cancer associated fibroblasts. Also, a problem underlying the present invention is the provision of a method for the diagnosis of a disease, of a method for the treatment and/or prevention of a disease, and a method for the combined diagnosis and treatment of a disease; preferably such disease is a disease involving urokinase plasminogen activator surface receptor (uPAR)-expressing cells and/or tissues, more particularly a urokinase plasminogen activator surface receptor (uPAR)-expressing diseased cell and/or diseased tissue, preferably the diseased tissue comprises or contains cancer associated fibroblasts. A still further problem underlying the present invention is the provision of a method for the identification of a subject, wherein the subject is likely to respond or likely not to respond to a treatment of a disease, a method for the selection of a subject from a group of subjects, wherein the subject is likely to respond or likely not to respond to a treatment of a disease. Also, a problem underlying the present invention is the provision of a pharmaceutical composition containing a compound having the characteristics as outlined above. Furthermore, a problem underlying the present invention is the provision of a kit which is suitable for use in any of the above methods.

There is a need for compounds that are suitable as a diagnostic agent and/or pharmaceutical agent, particularly if conjugated to a diagnostically and/or therapeutically active effector. Furthermore, there is a need for compounds that are suitable as a diagnostic agent and/or a pharmaceutical agent, particularly if conjugated to a diagnostically and/or therapeutically active effector, whereby the compound is a potent binder of urokinase plasminogen activator surface receptor (uPAR) activity; preferably the pIC50 of the compound is equal to or greater than 6.0, preferably equal to or greater than 7.0. Further, there is a need for compounds suitable as diagnostic agents and/or pharmaceutical agents, particularly if conjugated to a diagnostically and/or therapeutically active effector, in the diagnosis and/or therapy of a disease where the diseased cells and/or diseased tissues express urokinase plasminogen activator surface receptor (uPAR). Furthermore, there is a need for a compound which is suitable for delivering a diagnostically and/or therapeutically effective agent to a diseased cell and/or diseased tissue, respectively, and more particularly a urokinase plasminogen activator surface receptor (uPAR)-expressing diseased cell and/or diseased tissue, preferably the diseased tissue comprises or contains cancer associated fibroblasts. Also, there is a need for a method for the diagnosis of a disease, of a method for the treatment and/or prevention of a disease, and a method for the combined diagnosis and treatment of a disease; preferably such disease is a disease involving urokinase plasminogen activator surface receptor (uPAR)-expressing cells and/or tissues, more particularly a urokinase plasminogen activator surface receptor (uPAR)-expressing diseased cell and/or diseased tissue, preferably the diseased tissue comprises or contains cancer associated fibroblasts. Furthermore, there is a need for a method for the identification of a subject, wherein the subject is likely to respond or likely not to respond to a treatment of a disease, a method for the selection of a subject from a group of subjects, wherein the subject is likely to respond or likely not to respond to a treatment of a disease. Further, there is a need for a pharmaceutical composition containing a compound having the characteristics as outlined above. Furthermore, there is a need for a kit which is suitable for use in any of the above methods. The present invention satisfies these needs.

These and other problems are solved by the subject matter of the attached claims.

These and other problems underlying the present invention are also solved by the following embodiments which is also referred to as list of embodiments herein.

Embodiment 1. A compound comprising a cyclic peptide of formula (I) 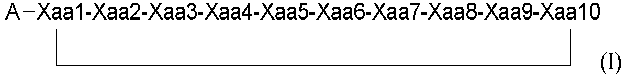 and optionally comprising a C-terminal modification group Cterm covalently attached to Xaa10, wherein the C-terminal modification group Cterm comprises a Z group,
wherein
the peptide sequence is drawn from left to right in N to C-terminal direction,
Xaa1 is a residue of an L-α-amino acid comprising a side chain,
   wherein the side chain comprises a sulfur atom which is covalently attached to the sulfur atom of the thiol group of XaalO,
   wherein the carbonyl group of Xaa1 is covalently attached to the α-nitrogen atom of Xaa2,
Xaa2 is a residue of an α-amino acid comprising a side chain, preferably an L-α-amino acid comprising a side chain,
   wherein the side chain optionally comprises a Z group,
   wherein the carbonyl group of Xaa2 is covalently attached to the α-nitrogen atom of Xaa3,
Xaa3 is a residue of an α-amino acid comprising a side chain, preferably an L-α-amino acid comprising a side chain,
   wherein the side chain optionally comprises a Z group,
   wherein the carbonyl group of Xaa3 is covalently attached to the α-nitrogen atom of Xaa4,
Xaa4 is a residue of an L-α-amino acid comprising a side chain,
   wherein the side chain comprises a group selected from the group comprising an aryl group, a heteroaryl group, a carbocycle group and a heterocycle group, preferably an aryl or heteroaryl group,
   wherein the carbonyl group of Xaa4 is covalently attached to the α-nitrogen atom of Xaa5,
Xaa5 is a residue of an L-α-amino acid comprising a side chain,
   wherein the side chain comprises an aryl group or a heteroaryl group,
   wherein the carbonyl group of Xaa5 is covalently attached to the α-nitrogen atom of Xaa6,
Xaa6 is a residue of an L-α-amino acid comprising a side chain and optionally comprising a substituent covalently attached to the α-nitrogen atom of Xaa6,
   wherein the side chain comprises a hydrophobic group,
   wherein the carbonyl group of Xaa6 is covalently attached to the α-nitrogen atom of Xaa7, preferably the side chain of Xaa6 and the α-nitrogen atom form a heterocycle,
Xaa7 is a residue of an α-amino acid comprising a side chain and optionally comprising a substituent covalently attached to the α-nitrogen atom of Xaa7,
   wherein the side chain optionally comprising a Z group,
   wherein the carbonyl group of Xaa7 is covalently attached to the α-nitrogen atom of Xaa8, preferably Xaa7 is a residue of an L-α-amino acid comprising a side chain,
   wherein the side chain of Xaa7 and the α-nitrogen atom optionally form a heterocycle,
Xaa8 is a residue of an α-amino acid comprising a side chain, preferably a residue of an L-α-amino acid comprising a side chain,
   wherein the side chain comprises a hydrophobic group,
   wherein the carbonyl group of Xaa8 is covalently attached to the α-nitrogen atom of Xaa9,
Xaa9 is a residue of an α-amino acid comprising a side chain, preferably a residue of an L-α-amino acid comprising a side chain,
   wherein the side chain comprises an aliphatic group,
   wherein the carbonyl group of Xaa9 is covalently attached to the nitrogen atom of the amine group of XaalO,
Xaa10 is a residue of an amino thiol comprising an amine group, a thiol group and optionally a carbonyl group,
   wherein if the carbonyl group is present, the C atom of the carbonyl group is covalently attached to the amine group bearing C atom of Xaa10,
   wherein the sulfur atom of the thiol group is covalently attached to the sulfur atom of the thiol group of Xaa1,
preferably Xaa10 is a residue of an L-α-amino acid comprising a side chain,
   wherein the side chain comprises a sulfur atom which is covalently attached to the sulfur atom of the thiol group of Xaa1,
   wherein, if the carbonyl group is present, the C atom of the carbonyl group of Xaa10 is optionally covalently attached to the C-terminal modification group Cterm,
an N-terminal modification group A, wherein the N-terminal modification group A is either a hydrophobic group Abl or Xaa0,
   wherein A is covalently attached to the α-nitrogen atom of Xaa1,
   wherein Xaa0 is a residue of an α-amino acid comprising a side chain and optionally comprising up to three substituents covalently attached to the α-nitrogen atom of Xaa0,
   wherein the side chain comprises a hydrophobic group,
   and wherein, if present, one substituent covalently attached to the α-nitrogen atom of Xaa0 is an N-terminal extension group Nterm, and wherein the N-terminal extension group Nterm optionally comprises a Z group;
or a pharmaceutically acceptable salt or hydrate or pharmaceutically acceptable solvate thereof.

Embodiment 2. The compound or pharmaceutically acceptable salt or hydrate or pharmaceutically acceptable solvate of Embodiment 1,
wherein
the N-terminal modification group A is either a blocking group Abl, preferably a hydrophobic blocking group Abl, or Xaa0,
the N-terminal modification group A is covalently attached to the α-nitrogen atom of Xaa1, wherein if the N-terminal modification group A is the blocking group Abl, the blocking group Abl is preferably of formula (IIa), of formula (IIb) or of formula (IIc) 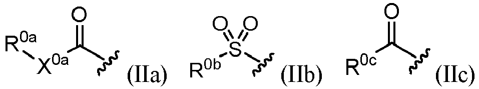
   wherein X^{0a} is selected from the group consisting of -O-, -NH- and -S-preferably X^{0a} is selected from the group consisting of -O- and -NH-,
   wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0a} is selected from the group consisting of a (C₃-C₆)alkyl group, a phenyl group and a (C₅-C₆)carbocycle,
   wherein R^{0b} is selected from the group consisting of a (C₃-C₉)alkyl group, a (C₃-C₈)carbocycle, an aryl group, a heteroaryl group, and a (C₃-C₈)heterocycle, and wherein R^{0b} is optionally linked to the -SOz- moiety in formula (IIb) by a linker comprising one, two or three -CH₂- groups, preferably the linker is absent or consists of one or two -CH₂- groups, and wherein R^{0b} is optionally substituted by one or two substituents, wherein, preferably, each substituent is independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0b} is selected from the group consisting of a (C₄-C₆)alkyl group, a phenyl group and a (C₅-C₆)carbocycle,
   wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- groups, wherein one -CH₂-group of R^{0c} is optionally replaced by -O- or -S-, and wherein R^{0c} is optionally substituted by one or two substituents, wherein, preferably, each substituent is independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0c} is selected from the group consisting of a (C₄-C₆)alkyl group, a phenyl group and a (C₅-C₆)carbocycle,

more preferably Abl is of formula (IIa) or (IIc),
most preferably Abl is of formula (IIa),
   wherein if the N-terminal modification group A is Xaa0,
      Xaa0 is a residue of an α-amino acid comprising a side chain and optionally comprising up to three substituents covalently attached to the α-nitrogen atom of Xaa0, wherein the side chain comprises a hydrophobic group, and wherein, if present, one substituent covalently attached to the α-nitrogen atom of Xaa0 is an N-terminal extension group Nterm, and wherein the N-terminal extension group Nterm optionally comprises a Z group,
      and
   Xaa0 is a residue of formula (IId) or of formula (IIe) 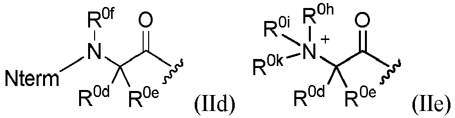
      wherein R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is independently selected from the group consisting of -OH, -F, -Cl, a (C₁-C₆)alkyl group, a (C₃-C₈)carbocycle group, an aryl group, a heteroaryl group, and a (C₃-C₈)heterocycle,
      preferably R^{0d} is selected from the group consisting of a (C₂-C₅)alkyl group, a (C₅-C₆)carbocycle, and a phenyl group
      wherein R^{0e} is selected from the group consisting of -H, an aryl group, a (C₁-C₆)alkyl group, and a (C₃-C₇) carbocycle,
      preferably R^{0e} is selected from the group consisting of -H and a methyl group, or wherein R^{0d} and R^{0e} together form a 4-, 5-, 6-, 7- or 8- membered carbocycle or heterocycle, wherein the carbocycle and the heterocycle are optionally substituted by one or two substituents, wherein, preferably, each substituent is independently selected from the group consisting of a methyl group, -F, -Cl, and -OH,
      preferably R^{0d} and R^{0e} together form a (C₅-C₆) carbocycle, more preferably an unsubstituted (C₅-C₆) carbocycle,
      wherein R^{0f} is selected from the group consisting of -H and a (C₁-C₄)alkyl group, preferably R^{0f} is selected from the group consisting of -H and a methyl group, more preferably R^{0f} is -H,
      wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide, preferably the linker is absent or comprises one α-amino acid or a dipeptide composed of two α-amino acids, most preferably the linker is absent or comprises one amino acid or a dipeptide wherein at least one amino acid comprises a polar or charged side chain,
      wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SOz-, -O-, or -NH-, and wherein R^{0g} is optionally substituted by one, two or three substituents, wherein, preferably, each substituent is independently selected from the group consisting of -OH, - NH₂, a halogen atom, -COOH, -CONH₂, -SO₃H, -NH-CNH-NHz, a (C₁-C₆)alkyl group, a (C₃-C₇)carbocycle, an aryl group, a heteroaryl group, and a (C₃-C₇)heterocycle, preferably R^{0g} is a (C₁-C₄)alkyl group and is optionally substituted by one substituent preferably selected from the group consisting of -OH, -NH₂, -COOH, -CONH₂, -SO₃H, -NH-CNH-NHz and a (C₁-C₄)alkyl group, more preferably R^{0g} is a (C₁-C₄)alkyl group and is optionally substituted by one substituent preferably selected from the group consisting of -OH, - COOH and -CONH₂,
      preferably Nterm is selected from the group consisting of -H, -R^{0g}, R^{0g}-CO-, R^{0g}-NH-CO- and a Z group, more preferably Nterm is selected from the group consisting of -H, -Ac, a succinyl group, a methyl group and a Z group, wherein R^{0h}, R⁰ⁱ and R^{0k} are each and independently selected from the group consisting of a (C₁-C₄)alkyl group, preferably R^{0h}, R⁰ⁱ and R^{0k} each are methyl groups,
Xaa1 is a residue of an L-α-amino acid comprising a side chain,
   wherein the side chain comprises a sulfur atom which is covalently attached to the sulfur atom of the thiol group of Xaa 10,
   wherein the carbonyl group of Xaa1 is covalently attached to the α-nitrogen atom of Xaa2, preferably Xaa1 is a residue of an amino acid of formula (III) 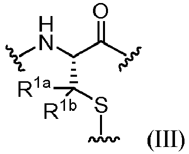 wherein
      R^{1a} and R^{1b} are each and independently selected from a group consisting of -H and a methyl group,
      the carbonyl group of Xaa1 is covalently attached to the α-nitrogen atom of Xaa2,
      and the sulfur atom of Xaa1 is covalently attached to the sulfur atom of the thiol group of Xaa10, preferably as disulfide,
Xaa2 is a residue of an α-amino acid comprising a side chain and optionally comprising a Z group,
   wherein the carbonyl group of Xaa2 is covalently attached to the α-nitrogen atom of Xaa3, preferably Xaa2 is a residue of an amino acid of formula (IVa), (IVb), or (IVc) 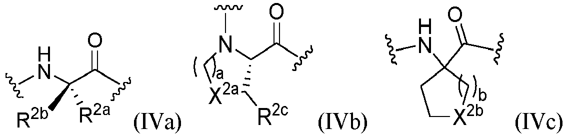 wherein
in formula (IVa)
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
   wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), - SO₂N(R^{2e})(R^{2m}), -CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
   wherein R^{2e}, R^{2k} and R^{2l} are each and independently selected from the group consisting of -H and a (C₁-C₂)alkyl group,
   wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂-group of -(CH₂)_{c}R^{2d} which is different from the terminal -CH₂- groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is each and independently selected from the group consisting of a methyl group, a halogen atom, -COOH, -CONH₂, -NH₂ and - OH, preferably said cycle is of formula (IVc)
in formula (IVb)
   X^{2a} is selected from the group consisting of -CH₂-, -CH(OH)-, -CH(F)-, - CH(NHR^{2p})-, -NH-, -S- and -O-, wherein R^{2p} is selected from the group consisting of -H, -Ac, a (C₁-C₄)alkyl and a Z group, preferably R^{2p} is selected from the group consisting of -H, a methyl group and a Z group, preferably X^{2a} is -CH₂-,
   R^{2c} is selected from the group consisting of -H, -OH, -F, -NH₂ and a (C₁-C₃)alkyl group, preferably R^{2c} is -H,
   a = 1 or 2, preferably a = 1,
in formula (IVc)
   b = 1, 2 or 3,
   X^{2b} is selected from the group consisting of -N(R^{2p})-, -O-, -S-, -SO-, -SOz-, - NH-, and -CH₂-, wherein R^{2p} is selected from the group consisting of -Ac, a (C₁-C₃)alkyl group and a Z group,
the carbonyl group of Xaa2 is covalently attached to the α-nitrogen atom of Xaa3,
preferably Xaa2 is a residue of an L-α amino acid of formula (IVa) or Xaa2 is a residue of an amino acid of formula (IVc), and more preferably Xaa2 is a residue of an L-α amino acid of formula (IVa),
Xaa3 is a residue of an α-amino acid comprising a side chain and optionally comprising a Z group,
   wherein the carbonyl group of Xaa3 is covalently attached to the α-nitrogen atom of Xaa4, preferably Xaa3 is a residue of an amino acid of formula (Va), or (Vb), 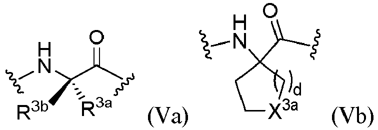 wherein
   in formula (Va)
      R^{3a} is selected from the group consisting of -H, a (C₁-C₆)alkyl group, a (C₃-C₇)carbocycle, (C₃-C₇)heterocycle, an aryl group, a heteroaryl group and - (CH₂)ₑR^{3c},
         wherein R^{3c} is selected from the group consisting of a polar, a positively charged, a negatively charged, a zwitterionic and a hydrophobic group, wherein R^{3c} optionally comprises a Z group,
         wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂-group of -(CH₂)ₑR^{3c} which is different from the terminal groups is optionally replaced by -O-, -S-, -SO- or -SOz-,
      R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent is preferably selected from the group consisting of -OH, -COOH, and -F, preferably an -OH group,
      or R^{3b} is -H under the proviso that R^{3a} is -H,
      orR^{3a} and R^{3b} form together a 3-, 4-, 5-, 6-, or 7- membered carbocycle or heterocycle that is optionally substituted by one or two substituents, wherein, preferably, each substituent is independently selected from the group consisting of a methyl group, a halogen, -COOH, -CONH₂, -NHz and -OH, preferably said cycle is of formula (Vb)
   in formula (Vb)
      d = 1, 2 or 3,
      X^{3a} is selected from the group consisting of -N(R^{3o})-, -O-, -S-, -SO-, - SOz-, -NH-, and -CH₂-, wherein R^{3o} is selected from the group consisting of - Ac, a (C₁-C₃)alkyl group and a Z group,
   the carbonyl group of Xaa3 is covalently attached to the α-nitrogen atom of Xaa4,
      preferably Xaa3 is a residue of an amino acid of formula (Va), more preferably Xaa3 is a residue of an L-α amino acid of formula (Vc) 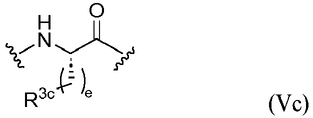 wherein
   in formula (Vc)
      R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
         wherein R^{3d}, R³ⁱ and R^{3k} are each and independently selected from the group consisting of -H and a (C₁-C₂)alkyl group,
         wherein R^{3f} and R^{3g} are each and independently selected from the group consisting of a (C₁-C₃)alkyl group,
      wherein R^{3e} is selected from the group consisting of -H, -Ac, a (C₁-C₃)alkyl group and a Z group,
         wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
      wherein R^{3l} is selected from the group consisting of -H and R^{3m}, preferably R^{3l} is selected from the group consisting of -H and a methyl group, most preferably R^{3l} is -H,
         wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
         wherein e = 1, 2, 3, 4, or 5,
         wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid comprising a side chain,
   wherein the side chain comprises an aryl, heteroaryl, carbocycle or heterocycle group, wherein the carbonyl group of Xaa4 is covalently attached to the α-nitrogen atom of Xaa5, preferably Xaa4 is a residue of an amino acid of formula (VI), 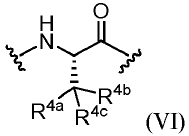 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents wherein, preferably, each substituent is individually and independently selected from the group consisting of a halogen atom, a (C₁-C₆)alkyl group, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group,
   the carbonyl group of Xaa4 is covalently attached to the α-nitrogen atom of Xaa5,
Xaa5 is a residue of an L-α-amino acid comprising a side chain,
   wherein the side chain comprises an aryl or heteroaryl group,
   wherein the carbonyl group of Xaa5 is covalently attached to the α-nitrogen atom of Xaa6, preferably Xaa5 is a residue of an amino acid of formula (VII), 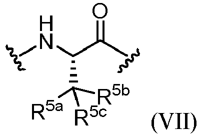 wherein
      R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
      wherein R^{5b} and R^{5c} are each and independently selected from the group consisting of -H and a methyl group,
      the carbonyl group of Xaa5 is covalently attached to the α-nitrogen atom of Xaa6,
Xaa6 is a residue of an L-α-amino acid comprising a side chain and optionally comprising a substituent covalently attached to the α-nitrogen atom of the amino acid,
   wherein the side chain comprises a hydrophobic group,
   wherein the carbonyl group of Xaa6 is covalently attached to the α-nitrogen atom of Xaa7,
   preferably the side chain of Xaa6 and the α-nitrogen atom form a heterocycle,
   and preferably Xaa6 is a residue of an amino acid of formula (Villa), (VIIIb) or (VIIIc) 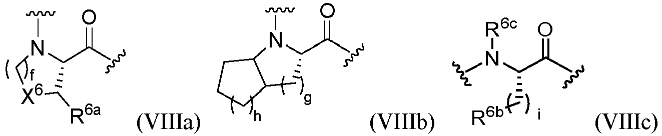 wherein
   in formula (Villa)
      f = 1 or 2, preferably f = 1,
      R^{6a} is selected from the group consisting of -H, -OH, -F, and a (C₁-C₄)alkyl group,
      X⁶ is absent or selected from the group consisting of -CH(R^{6d})-, -S-, -O- and - NH-, preferably X⁶ is -CH(R^{6d})- or -S-,
         wherein R^{6d} is selected from the group consisting of -H, a (C₁-C₄)alkyl group, -OH and -F,
   in formula (VIIIb)
      g = 0, 1 or 2, preferably g = 1,
      h = 1, 2 or 3, preferably h = 1 or 2,
   in formula (VIIIc)
      i = 1 or 2, preferably i= 1,
      R^{6b} is selected from the group consisting of a (C₂-C₆)alkyl group, an aryl group, an aryl group substituted with 1 or 2 substituents, a (C₅-C₆)heteroaryl group, a (C₅-C₆)heteroaryl group substituted with 1 or 2 substituents, a (C₅-C₆)carbocycle, and a (C₅-C₆)carbocycle substituted with 1 or 2 substituents, wherein, preferably, each substituent is individually and independently selected from the group consisting of a halogen atom, a (C₁-C₆)alkyl group, -CN, -OH, and an -O(C₁-C₆)alkyl group,
      R^{6c} is selected from the group consisting of -H and a (C₁-C₃)alkyl group,
the carbonyl group of Xaa6 is covalently attached to the α-nitrogen atom of Xaa7, more preferably Xaa6 is a residue of an amino acid of formula (Villa),
Xaa7 is a residue of an α-amino acid comprising a side chain, optionally comprising a Z group, and optionally comprising a substituent covalently attached to the α-nitrogen atom of the amino acid,
   wherein the carbonyl group of Xaa7 is covalently attached to the α-nitrogen atom of Xaa8, preferably Xaa7 is a residue of an amino acid of formula (IXa), (IXb), (IXc), (IXd), and (IXe) 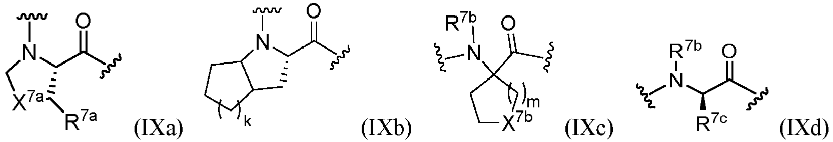 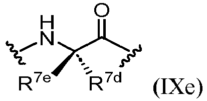 wherein
   in formula (IXa)
      R^{7a} is selected from the group consisting of -H, -OH, -F, -NH₂, and a (C₁-C₃)alkyl group,
      X^{7a} is absent or selected from the group consisting of -CH(R^{7f})-, -S-, -O-, and - NH-,
         wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   in formula (IXb)
      k = 1 or 2,
   in formula (IXc)
      m = 1, 2 or 3,
      R^{7b} is selected from the group consisting of -H, and a (C₁-C₂)alkyl group,
      X^{7b} is absent or selected from the group consisting of -N(R^{7g})-, -O-, -S-, -SO-, -SOz- and -CH₂-,
         wherein R^{7g} is selected from the group consisting of -H, -Ac, a (C₁-C₃)alkyl group and a Z group,
   in formula (IXd)
      R^{7b} is selected from the group consisting of -H, and a (C₁-C₂)alkyl group,
      R^{7c} is selected from the group consisting of -H, and -(CH₂)ₙR^{7h},
         wherein n = 1, 2, or 3, preferably n = 1, and wherein one of the one, two, or three -CH₂-groups of -(CH₂)ₙR^{7h} which is different from the terminal groups in formula (IXd) is optionally replaced by -O-, -S-, -SO-, or -SO-z,
         wherein R^{7h} is selected from the group consisting of -H, -OH, - NR⁷ⁱR^{7k}, -N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein, preferably, each substituent is individually and independently selected from the group consisting of a methyl group, a halogen atom, -NH₂ and -OH, wherein R⁷ⁱ and R^{7k} are each and independently selected from the group consisting of -H and a methyl group,
   in formula (IXe)
      R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l}, preferably R^{7d} is selected from the group consisting of -(CH₂)ₒR^{7l}, R^{7l} is selected from the group consisting of a polar, a positively charged, a negatively charged, a zwitterionic and a hydrophobic group, wherein R^{7l} optionally comprises a Z group,
      o = 1, 2, 3, 4 or 5, and wherein one of the one, two, three, four or five -CH₂-groups linking R^{7l} to the α-C atom of Xaa7 in formula (IXe) are optionally substituted by a methyl group, and/or wherein one of said -CH₂-groups which is different from the terminal groups is optionally replaced by -O-, -S-, - SO-, or -SOz-,
      preferably R^{7l} is selected from the group consisting of -H, -OH, - N(R^{7m})(R⁷ⁿ), -N(R^{7o})(R^{7p})(R^{7q})⁺, -COOH, a halogen atom, -CN, -N₃, -NO₂, -N(R^{7m})-CN(R⁷ⁿ)-N(R^{7r})(R^{7s}), -SO₂N(R^{7m})(R^{7u}), - CON(R^{7m})(R^{7u}), -NH-CO-N(R^{7m})(R^{7u}), -SO₃H, -R^{7t}, -NH-SO₂-R^{7t}, and -NH-CO-R^{7t},
         wherein R^{7m}, R^{7r} and R^{7s} are each and independently selected from the group consisting of -H and a (C₁-C₂)alkyl group,
         wherein R^{7o} and R^{7p} are each and independently selected from the group consisting of a (C₁-C₃)alkyl group,
      wherein R⁷ⁿ is selected from the group consisting of -H, -Ac, a (C₁-C₃)alkyl group and a Z group,
         wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{7q} is selected from the group consisting of a methyl group and a -(CH₂)₃SO₃H group,
         wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{7t} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{7t} is a (C₁-C₃)alkyl group,
      wherein R^{7u} is selected from the group consisting of -H and R^{7t}, preferably R^{7u} is selected from the group consisting of -H and a methyl group, most preferably R^{7u} is -H,
      R^{7e} is selected from the group consisting of -H, and a (C₁-C₃)alkyl group, wherein said (C₁-C₃)alkyl group is optionally substituted by a substituent preferably selected from the group consisting of -F, -Cl and -OH,
   the carbonyl group of Xaa7 is covalently attached to the α-nitrogen atom of Xaa8,
   preferably Xaa7 is a residue of an amino acid of formula (IXa), (IXd), and (IXe), more preferably Xaa7 is a residue of an amino acid of formula (IXa),
Xaa8 is a residue of an α-amino acid comprising a side chain, preferably a residue of an L-α-amino acid comprising a side chain,
   wherein in the side chain comprises a hydrophobic group,
   wherein the carbonyl group of Xaa8 is covalently attached to the α-nitrogen atom of Xaa9,
preferably Xaa8 is a residue of an amino acid of formula (Xa) or (Xb) 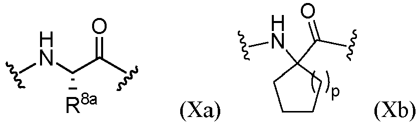 wherein
   in formula (Xa)
      R^{8a} is selected from the group consisting of a (C₄-C₈)carbocycle, an aryl group, a heteroaryl group and a -CH(R^{8b})(R^{8c}) group,
      wherein R^{8b} is selected from the group consisting of -H and a (C₁-C₃)alkyl group,
      wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
      wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
      wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   in formula (Xb)
      p = 1, 2, or 3,
      a -CH₂- group in the ring of formula (Xb) is optionally replaced by one atom selected from the group consisting of -O- and -S-, and one or more H atoms of the ring -CH₂- groups are optionally substituted by a -F or -Cl atom,
   the carbonyl group of Xaa8 is covalently attached to the α-nitrogen atom of Xaa9,
Xaa9 is a residue of an α-amino acid comprising a side chain,
   wherein the side chain is an aliphatic side chain,
   wherein the carbonyl group of Xaa9 is covalently attached to the nitrogen atom of the amine group of XaalO,
preferably Xaa9 is a residue of an amino acid of formula (XI) 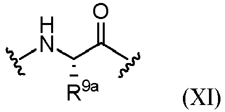 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl,
   wherein the carbonyl group of Xaa9 is covalently attached to the nitrogen atom of the amine group of Xaa10,
Xaa10 is a residue of an amino thiol comprising an amine group, a thiol group and optionally a carbonyl group, wherein if the carbonyl group is present, the C atom of the carbonyl group is covalently attached to the amine group bearing C atom of Xaa10,
wherein the sulfur atom of the thiol group is covalently attached to the sulfur atom of Xaa1, preferably as disulfide, and
wherein under the proviso that the amino thiol comprises a carbonyl group, a C-terminal modification group Cterm is optionally covalently attached to the C-atom of the carbonyl group, wherein the C-terminal modification group Cterm comprises a Z group, wherein the Z group is covalently attached to the C atom of the carbonyl group,
preferably Xaa10 comprises a substituent preferably selected from the group consisting of - CONH₂, -CO-Cterm, -CH₂(OH) and -CON(R^{10d})(R^{10e}), wherein said substituent is covalently attached to the amine group bearing C atom of Xaa10, and wherein R^{10d} and R^{10e} are each and independently selected from the group consisting of -H and a methyl group,
more preferably Xaa10 is a residue of formula (XII), 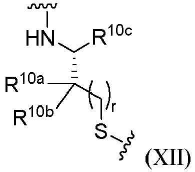 wherein
   R^{10a} and R^{10b} are each and independently selected from the group consisting of -H and a methyl group,
   r = 1 or 2,
      R^{10c} is selected from the group consisting of -H, -CO-Cterm, -CONH₂, - CH₂(OH), and -CON(R^{10d})(R^{10e}), wherein Cterm comprises a Z group,
      wherein R^{10d} and R^{10e} are each and independently selected from the group consisting of -H and a methyl group,
      and the sulfur atom of Xaa10 is covalently attached to the sulfur atom of Xaa1, preferably as disulfide.

Embodiment 3. The compound or pharmaceutically acceptable salt or hydrate or pharmaceutically acceptable solvate of any one of Embodiments 1 and 2,
wherein
the N-terminal modification group A is either a blocking group Abl, preferably a hydrophobic blocking group Abl, or Xaa0,
the N-terminal modification group A is covalently attached to the α-nitrogen atom of Xaa1,
wherein if the N-terminal modification group A is the blocking group Abl, the blocking group Abl is of formula (IIa), of formula (IIb) or of formula (IIc) 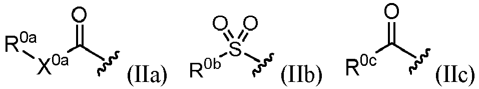
   wherein X^{0a} is selected from the group consisting of -NH-, -S- and -O-, preferably X^{0a} is selected from the group consisting of -NH- and -O-,
   wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0a} is selected from the group consisting of a (C₃-C₆)alkyl group, a phenyl group and a (C₅-C₆)carbocycle,
   wherein R^{0b} is selected from the group consisting of a (C₃-C₉)alkyl group, a (C₃-C₈)carbocycle, an aryl group, a heteroaryl group, and a (C₃-C₈)heterocycle, and wherein R^{0b} is optionally linked to the -SOz- moiety in formula (IIb) by a linker comprising one, two or three -CH₂- groups, preferably the linker is absent or consists of one or two -CH₂- groups, and wherein R^{0b} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0b} is selected from the group consisting of a (C₄-C₆)alkyl group, a phenyl group and a (C₅-C₆)carbocycle,
   wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- groups, wherein one -CH₂-group of R^{0c} is optionally replaced by -O- or -S-, and wherein R^{0c} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0c} is selected from the group consisting of a (C₄-C₆)alkyl group, a phenyl group and a (C₅-C₆)carbocycle,
more preferably Abl is of formula (IIa) or (IIc),
most preferably Abl is of formula (IIa),
   wherein if the N-terminal modification group A is Xaa0,
      Xaa0 is a residue of an α-amino acid comprising a side chain and optionally comprising up to three substituents covalently attached to the α-nitrogen atom of Xaa0, wherein the side chain comprises a hydrophobic group, and wherein, if present, one substituent covalently attached to the α-nitrogen atom of Xaa0 is an N-terminal extension group Nterm, and wherein the N-terminal extension group Nterm optionally comprises a Z group, and
   Xaa0 is a residue of formula (IId) or of formula (IIe) 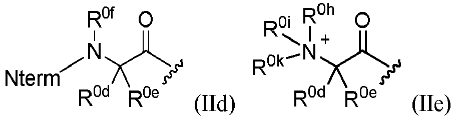
      wherein R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
      preferably R^{0d} is selected from the group consisting of a (C₂-C₅)alkyl group, a (C₅-C₆)carbocycle, and a phenyl group
      wherein R^{0e} is selected from the group consisting of -H, an aryl group, a (C₁-C₆)alkyl group, and a (C₃-C₇) carbocycle,
      preferably R^{0e} is selected from the group consisting of -H and a methyl group, or wherein R^{0d} and R^{0e} together form a 4-, 5-, 6-, 7- or 8- membered carbocycle or heterocycle, wherein the carbocycle and the heterocycle are optionally substituted by one or two substituents, wherein preferably each substituent is independently selected from the group consisting of a methyl group, -F, -Cl, and -OH,
      preferably R^{0d} and R^{0e} together form a (C₅-C₆) carbocycle, more preferably an unsubstituted (C₅-C₆) carbocycle,
      wherein R^{0f} is selected from the group consisting of -H and a (C₁-C₄)alkyl group, preferably R^{0f} is selected from the group consisting of -H and a methyl group, more preferably R^{0f} is -H,
      wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide, preferably the linker is absent or comprises one α-amino acid or a dipeptide composed of two α-amino acids, most preferably the linker is absent or comprises one amino acid or a dipeptide wherein at least one amino acid comprises a polar or charged side chain,
         wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SOz-, -O-, or -NH-, and wherein R^{0g} is optionally substituted by one, two or three substituents, wherein, preferably, each substituent is individually and independently selected from the group consisting of -OH, -NH₂, a halogen atom, -COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle, preferably R^{0g} is a (C₁-C₄)alkyl group and is optionally substituted by one substituent preferably selected from the group consisting of -OH, -NH₂, -COOH, -CONH₂, -SO₃H, -NH-CNH-NHz and a (C₁-C₄)alkyl group, more preferably R^{0g} is a (C₁-C₄)alkyl group and is optionally substituted by one substituent preferably selected from the group consisting of -OH, -COOH and -CONH₂,
         preferably Nterm is selected from the group consisting of -H, -R^{0g}, R^{0g}-CO-, R^{0g}-NH-CO- and a Z group, more preferably Nterm is selected from the group consisting of -H, -Ac, a succinyl group, a methyl group and a Z group,
      wherein R^{0h}, R⁰ⁱ and R^{0k} are each and independently selected from the group consisting of a (C₁-C₄)alkyl group, preferably R^{0h}, R⁰ⁱ and R^{0k} each are methyl groups,
Xaa1 is a residue of an amino acid of formula (III) 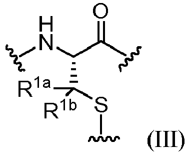 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of Xaa10, preferably as disulfide,
Xaa2 is a residue of an α-amino acid of formula (IVa), (IVb), or (IVc) 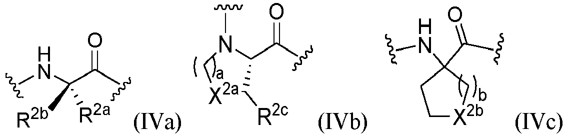 wherein
   in formula (IVa)
      R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
         wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), - SO₂N(R^{2e})(R^{2m}), -CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
         wherein R^{2e}, R^{2k} and R^{2l} are each and independently selected from the group consisting of -H and a (C₁-C₂)alkyl group,
         wherein R^{2g} and R^{2h} are each and independently selected from the group consisting of a (C₁-C₃)alkyl group,
      wherein R^{2f} is selected from the group consisting of -H, -Ac, a (C₁-C₃)alkyl group and a Z group,
         wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
      wherein R^{2m} is selected from the group consisting of -H and R²ⁿ, preferably R^{2m} is selected from the group consisting of -H and a methyl group, most preferably R^{2m} is -H,
         wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
         wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
      R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F, preferably an -OH group,
      or R^{2b} is -H under the proviso that R^{2a} is -H,
      or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NHz and -OH, preferably said cycle is of formula (IVc),
   in formula (IVb)
      X^{2a} is selected from the group consisting of -CH₂-, -CH(OH)-, -CH(F)-, - CH(NHR^{2p})-, -NH-, -S- and -O-, wherein R^{2p} is selected from the group consisting of -H, -Ac, a (C₁-C₄)alkyl and a Z group, preferably R^{2p} is selected from the group consisting of -H, a methyl group and a Z group, preferably X^{2a} is -CH₂-,
      R^{2c} is selected from the group consisting of -H, -OH, -F, -NH₂ and a (C₁-C₃)alkyl group, preferably R^{2c} is -H,
      a = 1 or 2, preferably a = 1,
   in formula (IVc)
      b = 1, 2 or 3,
         X^{2b} is selected from the group consisting of -N(R^{2p})-, -O-, -S-, -SO-, -SOz-, - NH-, and -CH₂-, wherein R^{2p} is selected from the group consisting of -Ac, a (C₁-C₃)alkyl group and a Z group,
      wherein the carbonyl group of Xaa2 is covalently attached to the α-nitrogen atom of Xaa3,
      preferably Xaa2 is a residue of an L-α amino acid of formula (IVa) or Xaa2 is a residue of an amino acid of formula (IVc), and more preferably Xaa2 is a residue of an L-α amino acid of formula (IVa),
Xaa3 is a residue of an α-amino acid formula (Va), or (Vb) optionally comprising a Z group, 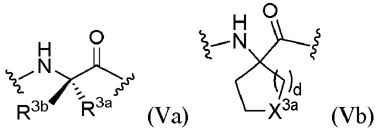 wherein
   in formula (Va)
      R^{3a} is selected from the group consisting of -H, a (C₁-C₆)alkyl group, a (C₃-C₇)carbocycle, (C₃-C₇)heterocycle, an aryl group, a heteroaryl group and - (CH₂)ₑR^{3c},
         wherein R^{3c} is selected from the group consisting of a polar, a positively charged, a negatively charged, a zwitterionic and a hydrophobic group, wherein R^{3c} optionally comprises a Z group,
         wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups is optionally replaced by -O-, -S-, -SO- or -SOz-,
      R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent is preferably selected from the group consisting of -OH, -COOH, and -F, preferably an -OH group,
      or R^{3b} is -H under the proviso that R^{3a} is -H,
      orR^{3a} and R^{3b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NHz and -OH, preferably said cycle is of formula (Vb)
   in formula (Vb)
      d = 1, 2 or 3,
      X^{3a} is selected from the group consisting of -N(R^{3o})-, -O-, -S-, -SO-, -SOz, -NH-, and -CH₂-, wherein R^{3o} is selected from the group consisting of -Ac, a (C₁-C₃)alkyl group and a Z group,
   wherein the carbonyl group of Xaa3 is covalently attached to the α-nitrogen atom of Xaa4,
   preferably Xaa3 is a residue of an amino acid of formula (Va), more preferably Xaa3 is a residue of an L-α amino acid of formula (Vc) 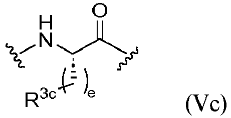 wherein
   in formula (Vc)
      R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
         wherein R^{3d}, R³ⁱ and R^{3k} are each and independently selected from the group consisting of -H and a (C₁-C₂)alkyl group,
         wherein R^{3f} and R^{3g} are each and independently selected from the group consisting of a (C₁-C₃)alkyl group,
      wherein R^{3e} is selected from the group consisting of -H, -Ac, a (C₁-C₃)alkyl group and a Z group,
         wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
      wherein R^{3l} is selected from the group consisting of -H and R^{3m}, preferably R^{3l} is selected from the group consisting of -H and a methyl group, most preferably R^{3l} is -H,
         wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
         wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 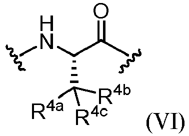 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl group, -CN, -COOH, -CONH₂, -NOz, -NH₂, -NH-CNH-NH₂, -SO₃H, -OH, an -O(C₁-C₆)alkyl group, wherein each substituent is optionally linked via a linker, preferably a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 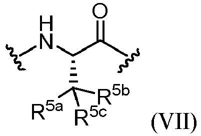 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa), (VIIIb) or (VIIIc) 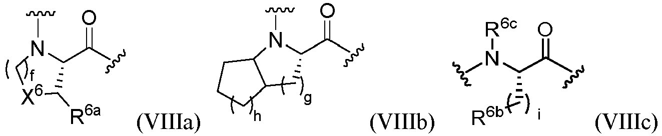 wherein
   in formula (Villa)
      f = 1 or 2, preferably f = 1,
      R^{6a} is selected from the group consisting of -H, -OH, -F, and a (C₁-C₄)alkyl group,
      X⁶ is absent or selected from the group consisting of -CH(R^{6d})-, -S-, -O- and - NH-, preferably X⁶ is -CH(R^{6d})- or -S-,
         wherein R^{6d} is selected from the group consisting of -H, a (C₁-C₄)alkyl group, -OH and -F,
   in formula (VIIIb)
      g = 0, 1 or 2, preferably g = 1,
      h = 1, 2 or 3, preferably h = 1 or 2,
   in formula (VIIIc)
      i = 1 or 2, preferably i= 1,
      R^{6b} is selected from the group consisting of a (C₂-C₆)alkyl group, an aryl group, an aryl group substituted with 1 or 2 substituents, a (C₅-C₆)heteroaryl group, a (C₅-C₆)heteroaryl group substituted with 1 or 2 substituents, a (C₅-C₆)carbocycle, and a (C₅-C₆)carbocycle substituted with 1 or 2 substituents, wherein, preferably, each substituent is individually and independently selected from the group consisting of a halogen atom, a (C₁-C₆)alkyl group, -CN, -OH, and an -O(C₁-C₆)alkyl group,
      R^{6c} is selected from the group consisting of -H and a (C₁-C₃)alkyl group, and
   wherein the carbonyl group of Xaa6 is covalently attached to the α-nitrogen atom of Xaa7, more preferably Xaa6 is a residue of an amino acid of formula (Villa),
Xaa7 is a residue of an α-amino acid of formula (IXa), (IXb), (IXc), (IXd), or (IXe), each optionally comprising a Z group, 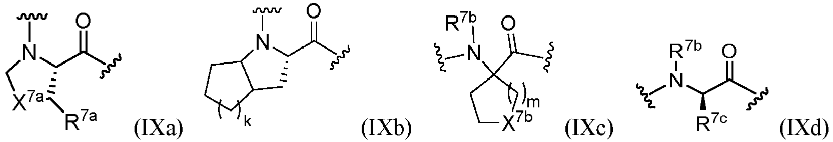 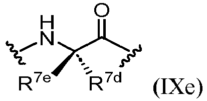 wherein
   in formula (IXa)
      R^{7a} is selected from the group consisting of -H, -OH, -F, -NH₂, and a (C₁-C₃)alkyl group,
      X^{7a} is absent or selected from the group consisting of -CH(R^{7f})-, -S-, -O-, and - NH-,
         wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   in formula (IXb)
      k = 1 or 2,
   in formula (IXc)
      m = 1, 2 or 3,
      R^{7b} is selected from the group consisting of -H, and a (C₁-C₂)alkyl group,
      X^{7b} is absent or selected from the group consisting of -N(R^{7g})-, -O-, -S-, -SO-, - SOz- and -CH₂-,
         wherein R^{7g} is selected from the group consisting of -H, -Ac, a (C₁-C₃)alkyl group and a Z group,
   in formula (IXd)
      R^{7b} is selected from the group consisting of -H, and a (C₁-C₂)alkyl group,
      R^{7c} is selected from the group consisting of -H, and -(CH₂)ₙR^{7h},
         wherein n = 1, 2, or 3, preferably n = 1, and wherein one of the one, two, or three -CH₂-groups of -(CH₂)ₙR^{7h} which is different from the terminal groups is optionally replaced by -O-, -S-, -SO-, or -SOz-,
         wherein R^{7h} is selected from the group consisting of -H, -OH, - NR⁷ⁱR^{7k}, -N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein each substituent is preferably selected from the group consisting of a methyl group, a halogen atom, -NH₂ and -OH, wherein R⁷ⁱ and R^{7k} are each and independently selected from the group consisting of -H and a methyl group,
   in formula (IXe)
      R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l}, preferably R^{7d} is selected from the group consisting of -(CH₂)ₒR^{7l}, R^{7l} is selected from the group consisting of a polar, a positively charged, a negatively charged, a zwitterionic and a hydrophobic group, wherein R^{7l} optionally comprises a Z group,
      o = 1, 2, 3, 4 or 5, and wherein one of the one, two, three, four or five -CH₂-groups linking R^{7l} to the α-C atom of Xaa7 in formula (IXe) are optionally substituted by a methyl group, and/or wherein one of said -CH₂-groups which is different from the terminal groups is optionally replaced by -O-, -S-, - SO-, or -SOz-,
      preferably R^{7l}is selected from the group consisting of -H, -OH, - N(R^{7m})(R⁷ⁿ), -N(R^{7o})(R^{7p})(R^{7q})⁺, -COOH, a halogen atom, -CN, -N₃, -NO₂, -N(R^{7m})-CN(R⁷ⁿ)-N(R^{7r})(R^{7s}), -SO₂N(R^{7m})(R^{7u}), - CON(R^{7m})(R^{7u}), -NH-CO-N(R^{7m})(R^{7u}), -SO₃H, -R^{7t}, -NH-SO₂-R^{7t}, and -NH-CO-R^{7t},
         wherein R^{7m}, R^{7r} and R^{7s} are each and independently selected from the group consisting of -H and a (C₁-C₂)alkyl group,
         wherein R^{7o} and R^{7p} are each and independently selected from the group consisting of a (C₁-C₃)alkyl group,
      wherein R⁷ⁿ is selected from the group consisting of -H, -Ac, a (C₁-C₃)alkyl group and a Z group,
         wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of -OH, -COOH, and -SO₃H, preferably R^{7q} is selected from the group consisting of a methyl group and a -(CH₂)₃SO₃H group,
         wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{7t} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{7t} is a (C₁-C₃)alkyl group,
      wherein R^{7u} is selected from the group consisting of -H and R^{7t}, preferably R^{7u} is selected from the group consisting of -H and a methyl group, most preferably R^{7u} is -H,
      R^{7e} is selected from the group consisting of -H, and a (C₁-C₃)alkyl group, wherein said (C₁-C₃)alkyl group is optionally substituted by a substituent preferably selected from the group consisting of -F, -Cl and -OH, and
   wherein the carbonyl group of Xaa7 is covalently attached to the α-nitrogen atom of Xaa8,
   preferably Xaa7 is a residue of an amino acid of formula (IXa), (IXd), and (IXe), more preferably Xaa7 is a residue of an amino acid of formula (IXa),
Xaa8 is a residue of an α-amino acid of formula (Xa) or (Xb) 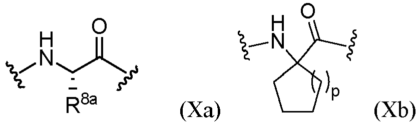 wherein
   in formula (Xa)
      R^{8a} is selected from the group consisting of a (C₄-C₈)carbocycle, an aryl group, a heteroaryl group and a -CH(R^{8b})(R^{8c}) group,
      wherein R^{8b} is selected from the group consisting of -H and a (C₁-C₃)alkyl group,
      wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
      wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
      wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   in formula (Xb)
      p = 1, 2, or 3,
      a -CH₂- group in the ring of formula (Xb) is optionally replaced by one atom selected from the group consisting of -O- and -S-, and one or more H atoms of the ring -CH₂- groups are optionally substituted by -F or -Cl,
      the carbonyl group of Xaa8 is covalently attached to the α-nitrogen atom of Xaa9, and
   wherein the carbonyl group of Xaa8 is covalently attached to the α-nitrogen atom of Xaa9,
Xaa9 is a residue of an α-amino acid of formula (XI) 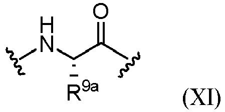 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
Xaa10 is a residue of formula (XII), 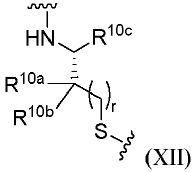 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, - CH₂(OH), and -CON(R^{10d})(R^{10e}), wherein Cterm comprises a Z group,
   wherein R^{10d} and R^{10e} are each and independently selected from the group consisting of -H and a methyl group,
   and the sulfur atom of Xaa10 is covalently attached to the sulfur atom of Xaa1, preferably as disulfide.

Embodiment 4. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1, wherein the N-terminal modification group A is the blocking group Abl of formula (IIa) 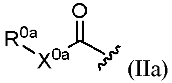 wherein X^{0a} is selected from the group consisting of -NH-, -S- and -O-, preferably X^{0a} is selected from the group consisting of -NH- and -O-, wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle, preferably R^{0a} is selected from the group consisting of a (C₃-C₆)alkyl group, a phenyl group and a (C₅-C₆)carbocycle,
Xaa1 is a residue of an amino acid of formula (III) 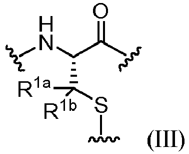 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of Xaa10, preferably as disulfide,
Xaa2 is a residue of an α-amino acid of formula (IVa) 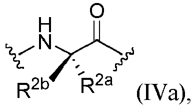 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), - SO₂N(R^{2e})(R^{2m}), -CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group,
   wherein is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid formula (Va), or (Vb) optionally comprising a Z group, 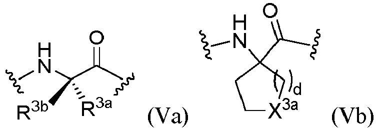 wherein
   in formula (Va)
      R^{3a} is selected from the group consisting of -H, a (C₁-C₆)alkyl group, a (C₃-C₇)carbocycle, (C₃-C₇)heterocycle, an aryl group, a heteroaryl group and - (CH₂)ₑR^{3c},
         wherein R^{3c} is selected from the group consisting of a polar, a positively charged, a negatively charged, a zwitterionic and a hydrophobic group, wherein R^{3c} optionally comprises a Z group,
         wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups is optionally replaced by -O-, -S-, -SO- or -SOz-,
      R^{3b} is selected from the group consisting of H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent preferably is selected from the group consisting of -OH, -COOH, and -F, preferably an -OH group,
      or R^{3b} is -H under the proviso that R^{3a} is -H,
      orR^{3a} and R^{3b} form together a 3-, 4-, 5-, 6-, or 7- membered carbocycle or heterocycle that is optionally substituted by one or two substituents, wherein, preferably, each substituent individually and independently selected from the group consisting of a methyl group, a halogen atom, -COOH, -CONH₂, -NHz and -OH, preferably said cycle is of formula (Vb)
   in formula (Vb)
      d = 1, 2 or 3,
      X^{3a} is selected from the group consisting of -N(R^{3o})-, -O-, -S-, -SO-, -SOz-, - NH-, and -CH₂-, wherein R^{3o} is selected from the group consisting of -Ac, a (C₁-C₃)alkyl group and a Z group,
   wherein the carbonyl group of Xaa3 is covalently attached to the α-nitrogen atom of Xaa4,
   preferably Xaa3 is a residue of an amino acid of formula (Va), more preferably Xaa3 is a residue of an L-α amino acid of formula (Vc) 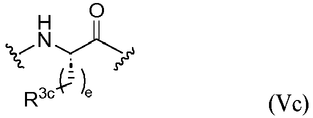 wherein
   in formula (Vc)
      R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SOz-R^{3m}, -CO-R³ⁿ, and -NH-CO-R^{3m},
         wherein R^{3d}, R³ⁱ and R^{3k} are each and independently selected from the group consisting of -H and a (C₁-C₂)alkyl group,
         wherein R^{3f} and R^{3g} are each and independently selected from the group consisting of a (C₁-C₃)alkyl group,
      wherein R^{3e} is selected from the group consisting of -H, -Ac, a (C₁-C₃)alkyl group and a Z group,
         wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
      wherein R^{3l} is selected from the group consisting of -H and R^{3m}, preferably R^{3l} is selected from the group consisting of -H and a methyl group, most preferably R^{3l} is -H,
         wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
         wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 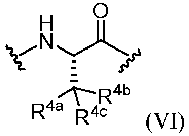 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl group, -CN, -COOH, -CONH₂, -NOz, -NH₂, -NH-CNH-NH₂, -SO₃H, -OH, an -O(C₁-C₆)alkyl group, wherein each substituent is optionally linked via a linker, preferably a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 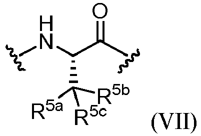 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 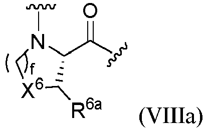 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXa) 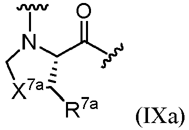 wherein
   R^{7a} is selected from the group consisting of -H, -OH, -F, -NH₂, and a (C₁-C₃)alkyl group,
   X^{7a} is absent or selected from the group consisting of -CH(R^{7f})-, -S-, -O-, and - NH-,
      wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   wherein the carbonyl group of Xaa7 is covalently attached to the α-nitrogen atom of Xaa8,
Xaa8 is a residue of an α-amino acid of formula (Xa) 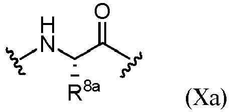 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group, and
   wherein the carbonyl group of Xaa8 is covalently attached to the α-nitrogen atom of Xaa9,
Xaa9 is a residue of an α-amino acid of formula (XI) 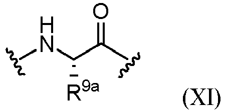 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 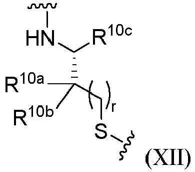 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, - CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of XaalO is covalently attached to the sulfur atom of Xaal, preferably as disulfide.

Embodiment 5. The compound or pharmaceutically acceptable salt or hydrate or pharmaceutically acceptable solvate of any one of Embodiments 1 to 4, wherein
the N-terminal modification group A is covalently attached to the α-nitrogen atom of Xaa1,
wherein the N-terminal modification group A is the blocking group Abl of formula (IIa) 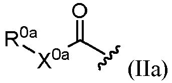
   wherein X^{0a} is selected from the group consisting of -NH-, -S- and -O-, preferably X^{0a} is selected from the group consisting of -NH- and -O-,
   wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle, preferably R^{0a} is selected from the group consisting of a (C₃-C₆)alkyl group, a phenyl group and a (C₅-C₆)carbocycle,
Xaal is a residue of an amino acid of formula (III) 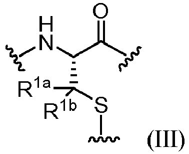 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of XaalO, preferably as disulfide,
Xaa2 is a residue of an α-amino acid of formula (IVa) 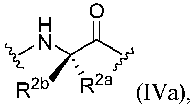 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F, preferably an -OH group,
   or R^{2b} is -H under the proviso that R^{2a} is -H,
   or, R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid formula (Va) optionally comprising a Z group, 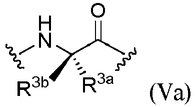 wherein
   R^{3a} is selected from the group consisting of -H, a (C₁-C₆)alkyl group, a (C₃-C₇)carbocycle, (C₃-C₇)heterocycle, an aryl group, a heteroaryl group and - (CH₂)ₑR^{3c},
      wherein R^{3c} is selected from the group consisting of a polar, a positively charged, a negatively charged, a zwitterionic and a hydrophobic group, wherein R^{3c} optionally comprises a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups of Xaa3 is optionally replaced by -O-, -S-, -SO- or -SOz-,
   R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent is preferably selected from the group consisting of -OH, -COOH, and -F, preferably an -OH group,
   or R^{3b} is -H under the proviso that R^{3a} is -H,
   or R^{3a} and R^{3b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (Vb),
   wherein the carbonyl group of Xaa3 is covalently attached to the α-nitrogen atom of Xaa4,
Xaa4 is a residue of an L-α-amino acid of formula (VI), 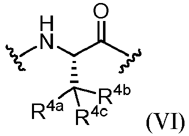 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl group, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 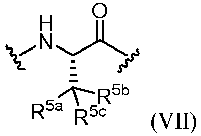 wherein
   R^{5a} is selected from the group consisting of a (C₆-C₁₀)aryl and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 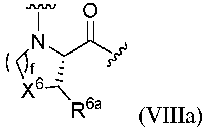 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXa) 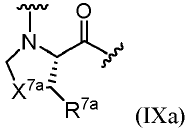 wherein
   R^{7a} is selected from the group consisting of -H, -OH, -F, -NH₂, and a (C₁-C₃)alkyl group,
   X^{7a} is absent or selected from the group consisting of -CH(R^{7f})-, -S-, -O-, and - NH-,
      wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   wherein the carbonyl group of Xaa7 is covalently attached to the α-nitrogen atom of Xaa8,
Xaa8 is a residue of an L-α-amino acid of formula (Xa) 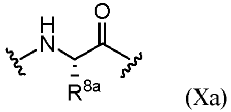 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group, and
   wherein the carbonyl group of Xaa8 is covalently attached to the α-nitrogen atom of Xaa9,
Xaa9 is a residue of an α-amino acid of formula (XI) 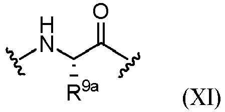 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 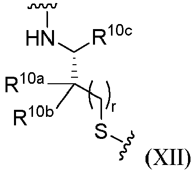 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, - CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of XaalO is covalently attached to the sulfur atom of Xaa1, preferably as disulfide.

Embodiment 6. The compound or pharmaceutically acceptable salt or hydrate or pharmaceutically acceptable solvate of any one of Embodiments 1, 2, 3 and 4,
wherein
the N-terminal modification group A is covalently attached to the α-nitrogen atom of Xaa1, wherein the N-terminal modification group A is the blocking group Abl of formula (IIa) 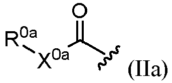
wherein X^{0a} is selected from the group consisting of -NH-, -S- and -O-, preferably X^{0a} is selected from the group consisting of -NH- and -O-,
wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle, preferably R^{0a} is selected from the group consisting of a (C₃-C₆)alkyl group, a phenyl group and a (C₅-C₆)carbocycle,

Xaa1 is a residue of an amino acid of formula (III) 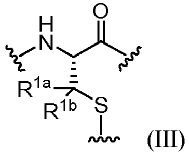 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of XaalO, preferably as disulfide,
Xaa2 is a residue of an α-amino acid of formula (IVa) 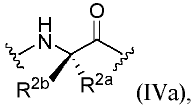 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NO₂, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 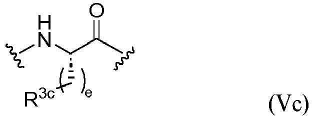 wherein
   in formula (Vc)
      R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
         wherein R^{3d}, R³ⁱ and R^{3k} are each and independently selected from the group consisting of -H and a (C₁-C₂)alkyl group,
         wherein R^{3f} and R^{3g} are each and independently selected from the group consisting of a (C₁-C₃)alkyl group,
      wherein R^{3e} is selected from the group consisting of -H, -Ac, a (C₁-C₃)alkyl group and a Z group,
         wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
      wherein R^{3l} is selected from the group consisting of -H and R^{3m}, preferably R³ⁱ is selected from the group consisting of -H and a methyl group, most preferably R³ⁱ is -H,
         wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group,
      wherein R³ⁿ is a Z group,
         wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 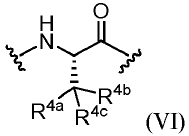 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 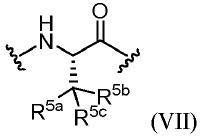 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 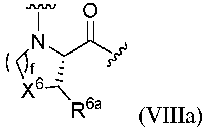 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXa) 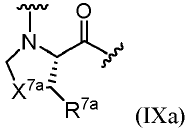 wherein
   R^{7a} is selected from the group consisting of -H, -OH, -F, -NH₂, and a (C₁-C₃)alkyl group,
   X^{7a} is absent or selected from the group consisting of -CH(R^{7f})-, -S-, -O-, and - NH-,
      wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   wherein the carbonyl group of Xaa7 is covalently attached to the α-nitrogen atom of Xaa8,
Xaa8 is a residue of an L-α-amino acid of formula (Xa) 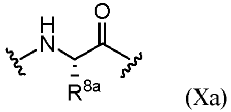 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 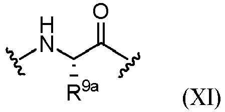 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 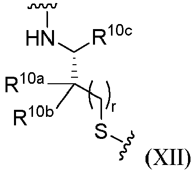 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, - CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of Xaa10 is covalently attached to the sulfur atom of Xaa1.

Embodiment 7. The compound or pharmaceutically acceptable salt or hydrate or pharmaceutically acceptable solvate of any one of Embodiments 1, 2 and 3, wherein
the N-terminal modification group A is covalently attached to the α-nitrogen atom of Xaa1, wherein the N-terminal modification group A is the blocking group Abl of formula (IIa) 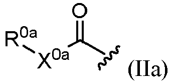
wherein X^{0a} is selected from the group consisting of -NH-, -S- and -O-, preferably X^{0a} is selected from the group consisting of -NH- and -O-,
wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
preferably R^{0a} is selected from the group consisting of a (C₃-C₆)alkyl group, a phenyl group and a (C₅-C₆)carbocycle,

Xaal is a residue of an amino acid of formula (III) 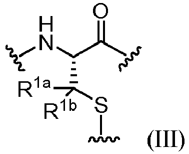 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of XaalO, preferably as disulfide,
Xaa2 is a residue of an α-amino acid of formula (IVa) 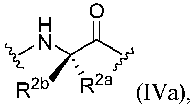 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 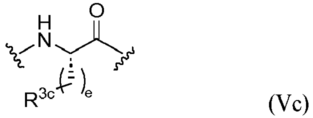 wherein
   in formula (Vc)
   R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 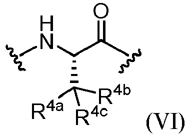 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 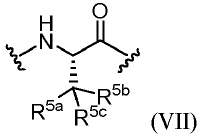 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 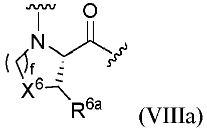 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXd) 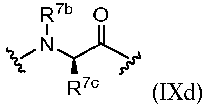 wherein
   R^{7b} is selected from the group consisting of -H, and a (C₁-C₂)alkyl group,
   R^{7c} is selected from the group consisting of -H, and -(CH₂)ₙR^{7h},
      wherein n = 1, 2, or 3, preferably n = 1, and wherein one of the one, two, or three -CH₂- groups of -(CH₂)ₙR^{7h} which is different from the terminal groups is optionally replaced by -O-, -S-, -SO-, or -SOz-,
      wherein R^{7h} is selected from the group consisting of -H, -OH, -NR^{7l}R^{7k}, - N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein each substituent is preferably selected from the group consisting of a methyl group, a halogen atom, -NH₂ and -OH, wherein R⁷ⁱ and R^{7k} are each and independently selected from the group consisting of -H and a methyl group,
   wherein the carbonyl group of Xaa7 is covalently attached to the α-nitrogen atom of Xaa8,
Xaa8 is a residue of an L-α-amino acid of formula (Xa) 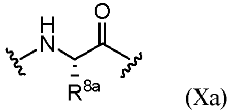 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 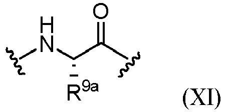 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 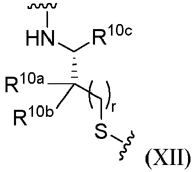 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of XaalO is covalently attached to the sulfur atom of Xaa1.

Embodiment 8. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1, wherein the N-terminal modification group A is the blocking group Abl of formula (IIa) 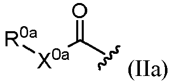
wherein X^{0a} is selected from the group consisting of -NH-, -S- and -O-, preferably X^{0a} is selected from the group consisting of -NH- and -O-,
wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
preferably R^{0a} is selected from the group consisting of a (C₃-C₆)alkyl group, a phenyl group and a (C₅-C₆)carbocycle,

Xaa1 is a residue of an amino acid of formula (III) 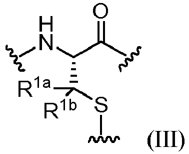 wherein
   R^{1a} and R^{1b} are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of XaalO, preferably as disulfide,
Xaa2 is a residue of an α-amino acid of formula (IVa) 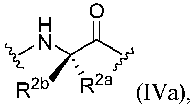 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 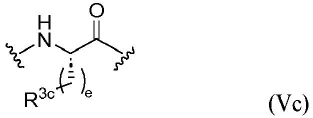 wherein
   in formula (Vc)
   R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 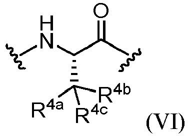 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 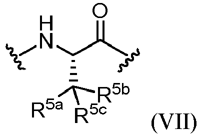 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 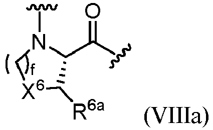 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXe) 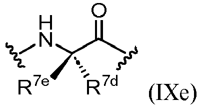 wherein
   R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l}, preferably R^{7d} is selected from the group consisting of -(CH₂)ₒR^{7l},
   R^{7l} is selected from the group consisting of a polar, a positively charged, a negatively charged, a zwitterionic and a hydrophobic group, wherein R^{7l} optionally comprises a Z group,
   o = 1, 2, 3, 4 or 5, and wherein one of the one, two, three, four or five -CH₂-groups linking R^{7l} to the α-C atom of Xaa7 in formula (IXe) are optionally substituted by a methyl group, and/or wherein one of said -CH₂- groups which is different from the terminal groups is optionally replaced by -O-, -S-, -SO-, or -SOz-,
   preferably R^{7l} is selected from the group consisting of -H, -OH, -N(R^{7m})(R⁷ⁿ), -N(R^{7o})(R^{7p})(R^{7q})⁺, -COOH, a halogen atom, -CN, -N₃, -NO₂, -N(R^{7m})-CN(R⁷ⁿ)-N(R^{7r})(R^{7s}), -SO₂N(R^{7m})(R^{7u}), -CON(R^{7m})(R^{7u}), -NH-CON(R^{7m})(R^{7u}), -SO₃H, -R^{7t}, -NH-SO₂-R^{7t}, and -NH-CO-R^{7t},
      wherein R^{7m}, R^{7r} and R^{7s} are each and independently selected from the group consisting of -H and a (C₁-C₂)alkyl group,
      wherein R^{7o} and R^{7p} are each and independently selected from the group consisting of a (C₁-C₃)alkyl group,
   wherein R⁷ⁿ is selected from the group consisting of -H, -Ac, a (C₁-C₃)alkyl group and a Z group,
      wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of -OH, -COOH, and -SO₃H, preferably R^{7q} is selected from the group consisting of a methyl group and a -(CH₂)₃SO₃H group,
      wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{7t} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{7t} is a (C₁-C₃)alkyl group, wherein R^{7u} is selected from the group consisting of -H and R^{7t}, preferably R^{7u} is selected from the group consisting of -H and a methyl group, most preferably R^{7u} is -H,
   R^{7e} is selected from the group consisting of -H, and a (C₁-C₃)alkyl group, wherein said (C₁-C₃)alkyl group is optionally substituted by a substituent preferably selected from the group consisting of -F, -Cl and -OH, and
   wherein the carbonyl group of Xaa7 is covalently attached to the α-nitrogen atom of Xaa8,
Xaa8 is a residue of an L-α-amino acid of formula (Xa) 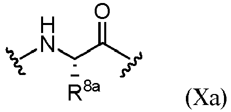 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 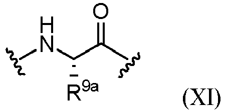 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 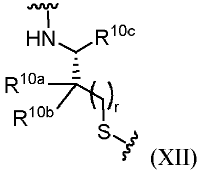 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of Xaa10 is covalently attached to the sulfur atom of Xaa1.

Embodiment 9. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1, wherein the N-terminal modification group A is the blocking group Abl of formula (IIc) 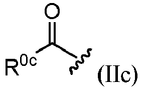
wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- groups, wherein one -CH₂- group of R^{0c} is optionally replaced by -O- or -S-, and wherein R^{0c} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
preferably R^{0c} is selected from the group consisting of a (C₄-C₆)alkyl group, a phenyl group and a (C₅-C₆)carbocycle,

Xaal is a residue of an amino acid of formula (III) 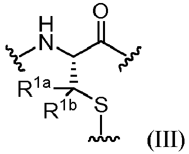 wherein
   R^{1a} and R^{1b} are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of XaalO, preferably as disulfide,
Xaa2 is a residue of an α-amino acid of formula (IVa), 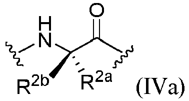 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc), 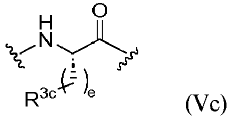 wherein
   in formula (Vc)
   R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, -N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 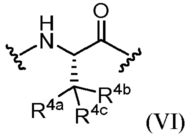 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 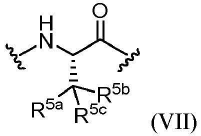 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 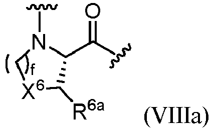 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXa) 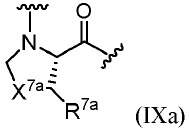 wherein
   R^{7a} is selected from the group consisting of -H, -OH, -F, -NH₂, and a (C₁-C₃)alkyl group,
   X^{7a} is absent or selected from the group consisting of -CH(R^{7f})-, -S-, -O-, and - NH-,
      wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   wherein the carbonyl group of Xaa7 is covalently attached to the α-nitrogen atom of Xaa8,
Xaa8 is a residue of an L-α-amino acid of formula (Xa) 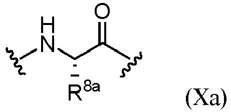 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 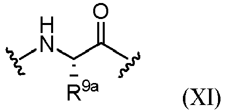 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 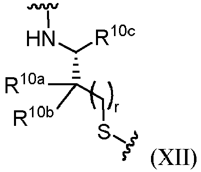 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of XaalO is covalently attached to the sulfur atom of Xaa1.

Embodiment 10. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1, wherein the N-terminal modification group A is the blocking group Abl of formula (IIc) 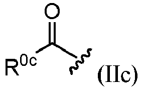
wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- groups, wherein one -CH₂- group of R^{0c} is optionally replaced by -O- or -S-, and wherein R^{0c} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
preferably R^{0c} is selected from the group consisting of a (C₄-C₆)alkyl group, a phenyl group and a (C₅-C₆)carbocycle,

Xaa1 is a residue of an amino acid of formula (III) 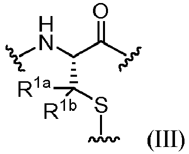 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of XaalO, preferably as disulfide,
Xaa2 is a residue of an α-amino acid of formula (IVa) 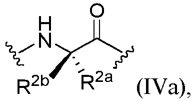 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R²¹), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 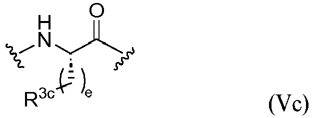 wherein
   in formula (Vc)
   R^{3c} is selected from the group consisting of -H, -OH, -N(R^{3d})(R^{3e}), -N(R^{3f})(R^{3g})(R^{3h})⁺, -COOH, a halogen atom, -CN, -N₃, -NO₂, -N(R^{3d})-CN(R^{3e})-N(R³ⁱ)(R^{3k}), -SO₂N(R^{3d})(R³¹), -CON(R^{3d})(R³¹), -NH-CON(R^{3d})(R³¹), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 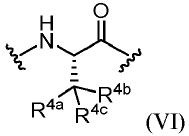 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 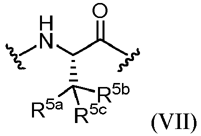 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 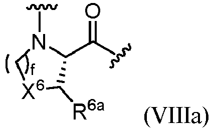 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXd) 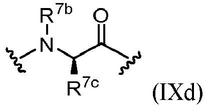 wherein
   R^{7b} is selected from the group consisting of -H, and a (C₁-C₂)alkyl group,
   R^{7c} is selected from the group consisting of -H, and -(CH₂)ₙR^{7h},
      wherein n = 1, 2, or 3, preferably n = 1, and wherein one of the one, two, or three -CH₂- groups of -(CH₂)ₙR^{7h} which is different from the terminal groups is optionally replaced by -O-, -S-, -SO-, or -SOz-,
      wherein R^{7h} is selected from the group consisting of -H, -OH, -NR⁷ⁱR^{7k}, - N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein each substituent is preferably selected from the group consisting of a methyl group, a halogen atom, -NH₂ and -OH, wherein R⁷ⁱ and R^{7k} are each and independently selected from the group consisting of -H and a methyl group,
   wherein the carbonyl group of Xaa7 is covalently attached to the α-nitrogen atom of Xaa8,
Xaa8 is a residue of an L-α-amino acid of formula (Xa) 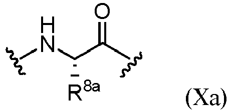 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 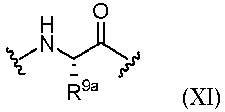 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of XaalO,
XaalO is a residue of formula (XII), 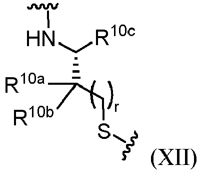 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of XaalO is covalently attached to the sulfur atom of Xaa1.

Embodiment 11. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1, wherein the N-terminal modification group A is the blocking group Abl of formula (IIc) 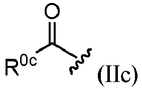
wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- groups, wherein one -CH₂- group of R^{0c} is optionally replaced by -O- or -S-, and wherein R^{0c} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
preferably R^{0c} is selected from the group consisting of a (C₄-C₆)alkyl group, a phenyl group and a (C₅-C₆)carbocycle,

Xaa1 is a residue of an amino acid of formula (III) 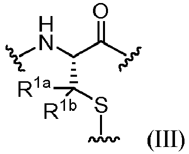 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of Xaa10, preferably as disulfide,
Xaa2 is a residue of an α-amino acid of formula (IVa) 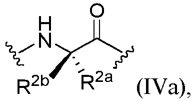 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NO₂, -N(R^{2e})-CN(R^{2f})-N(R^{2k})(R²¹), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 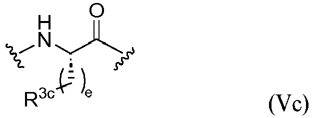 wherein
   in formula (Vc)
   R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂,-N(R^{3d})-CN(R^{3e})-N(R³ⁱ)(R^{3k}), -SO₂N(R^{3d})(R³¹), -CON(R^{3d})(R³¹), -NH-CON(R^{3d})(R³¹), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 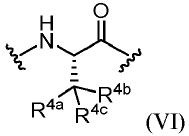 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 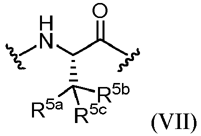 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 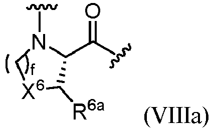 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXe) 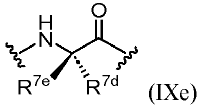 wherein
   R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l}, preferably R^{7d} is selected from the group consisting of -(CH₂)ₒR^{7l}, R^{7l} is selected from the group consisting of a polar, a positively charged, a negatively charged, a zwitterionic and a hydrophobic group, wherein R^{7l} optionally comprises a Z group,
   o = 1, 2, 3, 4 or 5, and wherein one of the one, two, three, four or five -CH₂-groups linking R^{7l} to the α-C atom of Xaa7 in formula (IXe) are optionally substituted by a methyl group, and/or wherein one of said -CH₂- groups which is different from the terminal groups is optionally replaced by -O-, -S-, -SO-, or -SOz-,
   preferably R^{7l} is selected from the group consisting of -H, -OH, -N(R^{7m})(R⁷ⁿ), -N(R^{7o})(R^{7p})(R^{7q})⁺, -COOH, a halogen atom, -CN, -N₃, -NO₂, -N(R^{7m})-CN(R⁷ⁿ)-N(R^{7r})(R^{7s}), -SO₂N(R^{7m})(R^{7u}), -CON(R^{7m})(R^{7u}), -NH-CO-N(R^{7m})(R^{7u}), -SO₃H, -R^{7t}, -NH-SO₂-R^{7t}, and -NH-CO-R^{7t},
      wherein R^{7m}, R^{7r} and R^{7s} are each and independently selected from the group consisting of -H and a (C₁-C₂)alkyl group,
      wherein R^{7o} and R^{7p} are each and independently selected from the group consisting of a (C₁-C₃)alkyl group,
   wherein R⁷ⁿ is selected from the group consisting of -H, -Ac, a (C₁-C₃)alkyl group and a Z group,
      wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of -OH, -COOH, and -SO₃H, preferably R^{7q} is selected from the group consisting of a methyl group and a -(CH₂)₃SO₃H group,
      wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{7t} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{7t} is a (C₁-C₃)alkyl group,
   wherein R^{7u} is selected from the group consisting of -H and R^{7t}, preferably R^{7u} is selected from the group consisting of -H and a methyl group, most preferably R^{7u} is -H,
   R^{7e} is selected from the group consisting of -H, and a (C₁-C₃)alkyl group, wherein said (C₁-C₃)alkyl group is optionally substituted by a substituent preferably selected from the group consisting of -F, -Cl and -OH, and
   wherein the carbonyl group of Xaa7 is covalently attached to the α-nitrogen atom of Xaa8,
Xaa8 is a residue of an L-α-amino acid of formula (Xa) 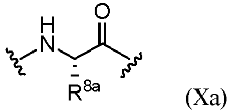 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 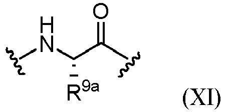 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of Xaa10,
Xaa10 is a residue of formula (XII), 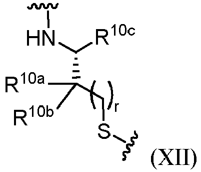 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
and the sulfur atom of Xaa10 is covalently attached to the sulfur atom of Xaa1.

Embodiment 12. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1,
wherein the N-terminal modification group A is Xaa0,
   wherein Xaa0 is a residue of formula (IId), 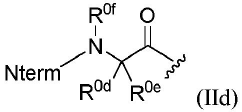 wherein
   R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
   preferably R^{0d} is selected from the group consisting of a (C₂-C₅)alkyl group, a (C₅-C₆)carbocycle, and a phenyl group
   wherein R^{0e} is selected from the group consisting of -H, an aryl group, a (C₁-C₆)alkyl group, and a (C₃-C₇) carbocycle,
   preferably R^{0e} is selected from the group consisting of -H and a methyl group, or wherein R^{0d} and R^{0e} together form a 4-, 5-, 6-, 7- or 8- membered carbocycle or heterocycle, wherein the carbocycle and the heterocycle are optionally substituted by one or two substituents, wherein each substituent is independently selected from the group consisting of a methyl group, -F, -Cl, and -OH,
   preferably R^{0d} and R^{0e} together form a (C₅-C₆) carbocycle, more preferably an unsubstituted (C₅-C₆) carbocycle,
   wherein R^{0f} is selected from the group consisting of -H and a (C₁-C₄)alkyl group, preferably R^{0f} is selected from the group consisting of -H and a methyl group, more preferably R^{0f} is -H,
   wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide, preferably the linker is absent or comprises one α-amino acid or a dipeptide composed of two α-amino acids, most preferably the linker is absent or comprises one amino acid or a dipeptide wherein at least one amino acid comprises a polar or charged side chain,
      wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SOz-, -O-, or -NH-, and wherein R^{0g} is optionally substituted by one, two or three substituents, wherein, preferably, each substituent is individually and independently selected from the group consisting of -OH, -NH₂, a halogen atom, -COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle, preferably R^{0g} is a (C₁-C₄)alkyl group and is optionally substituted by one substituent preferably selected from the group consisting of -OH, -NH₂, -COOH, -CONH₂, -SO₃H, -NH-CNH-NHz and a (C₁-C₄)alkyl group, more preferably R^{0g} is a (C₁-C₄)alkyl group and is optionally substituted by one substituent preferably selected from the group consisting of -OH, -COOH and -CONH₂,
      preferably Nterm is selected from the group consisting of -H, -R^{0g}, R^{0g}-CO-, R^{0g}-NH-CO- and a Z group, more preferably Nterm is selected from the group consisting of -H, -Ac, a succinyl group, a methyl group and a Z group,

Xaa1 is a residue of an amino acid of formula (III) 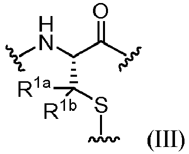 wherein
   R^{1a} and R^{1b}are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of Xaa10, preferably as disulfide,
Xaa2 is a residue of an α-amino acid of formula (IVa), 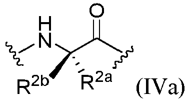 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R²¹), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group,
   wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc), 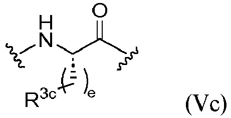 wherein in formula (Vc)
   R^{3c} is selected from the group consisting of -H, -OH, -N(R^{3d})(R^{3e}), -N(R^{3f})(R^{3g})(R^{3h})⁺, -COOH, a halogen atom, -CN, -N₃, -NO₂, -N(R^{3d})-CN(R^{3e})-N(R³ⁱ)(R^{3k}), -SO₂N(R^{3d})(R³¹), -CON(R^{3d})(R³¹), -NH-CON(R^{3d})(R³¹), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group,
   wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 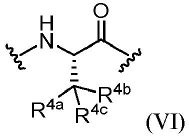 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 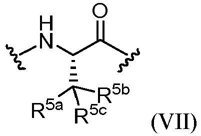 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 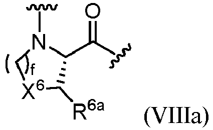 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXa) 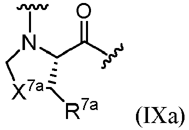 wherein
   R^{7a} is selected from the group consisting of -H, -OH, -F, -NH₂, and a (C₁-C₃)alkyl group,
   X^{7a} is absent or selected from the group consisting of -CH(R^{7f})-, -S-, -O-, and - NH-,
      wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
   wherein the carbonyl group of Xaa7 is covalently attached to the α-nitrogen atom of Xaa8,
Xaa8 is a residue of an L-α-amino acid of formula (Xa) 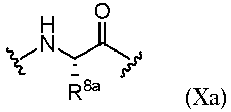 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 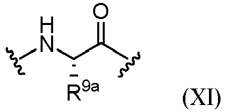 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of Xaa10,
Xaa10 is a residue of formula (XII), 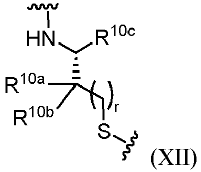 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of Xaa10 is covalently attached to the sulfur atom of Xaa1.

Embodiment 13. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1,
wherein the N-terminal modification group A is Xaa0,
wherein Xaa0 is a residue of formula (IId), 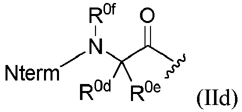 wherein
   R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
   preferably R^{0d} is selected from the group consisting of a (C₂-C₅)alkyl group, a (C₅-C₆)carbocycle, and a phenyl group
   wherein R^{0e} is selected from the group consisting of -H, an aryl group, a (C₁-C₆)alkyl group, and a (C₃-C₇) carbocycle,
   preferably R^{0e} is selected from the group consisting of -H and a methyl group, or wherein R^{0d} and R^{0e} together form a 4-, 5-, 6-, 7- or 8- membered carbocycle or heterocycle, wherein the carbocycle and the heterocycle are optionally substituted by one or two substituents, wherein each substituent is independently selected from the group consisting of a methyl group, -F, -Cl, and -OH,
   preferably R^{0d} and R^{0e} together form a (C₅-C₆) carbocycle, more preferably an unsubstituted (C₅-C₆) carbocycle,
   wherein R^{0f} is selected from the group consisting of -H and a (C₁-C₄)alkyl group, preferably R^{0f} is selected from the group consisting of -H and a methyl group, more preferably R^{0f} is -H,
   wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide, preferably the linker is absent or comprises one α-amino acid or a dipeptide composed of two α-amino acids, most preferably the linker is absent or comprises one amino acid or a dipeptide wherein at least one amino acid comprises a polar or charged side chain,
      wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SOz-, -O-, or -NH-, and wherein R^{0g} is optionally substituted by one, two or three substituents, wherein, preferably, each substituent is individually and independently selected from the group consisting of -OH, -NH₂, a halogen atom, -COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle, preferably R^{0g} is a (C₁-C₄)alkyl group and is optionally substituted by one substituent preferably selected from the group consisting of -OH, -NH₂, -COOH, -CONH₂, -SO₃H, -NH-CNH-NHz and a (C₁-C₄)alkyl group, more preferably R^{0g} is a (C₁-C₄)alkyl group and is optionally substituted by one substituent preferably selected from the group consisting of -OH, -COOH and -CONH₂,
      preferably Nterm is selected from the group consisting of -H, -R^{0g}, R^{0g}-CO-, R^{0g}-NH-CO- and a Z group, more preferably Nterm is selected from the group consisting of -H, -Ac, a succinyl group, a methyl group and a Z group,
Xaa1 is a residue of an amino acid of formula (III) 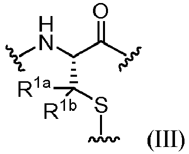 wherein
   R^{1a} and R^{1b} are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of Xaa10, preferably as disulfide,
Xaa2 is a residue of an α-amino acid of formula (IVa) 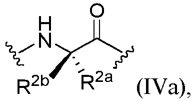 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 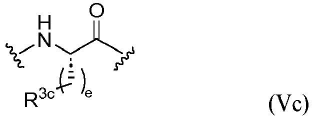 wherein in formula (Vc)
   R^{3c} is selected from the group consisting of -H, -OH, -N(R^{3d})(R^{3e}), -N(R^{3f})(R^{3g})(R^{3h})⁺, -COOH, a halogen atom, -CN, -N₃, -NO₂, -N(R^{3d})-CN(R3^{e})-N(R³ⁱ)(R^{3k}), -SO₂N(R^{3d})(R³¹), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group,
   wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 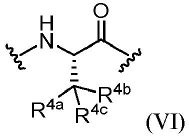 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 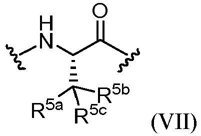 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 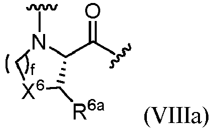 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXd) 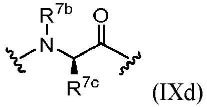 wherein
   R^{7b} is selected from the group consisting of -H, and a (C₁-C₂)alkyl group,
   R^{7c} is selected from the group consisting of -H, and -(CH₂)ₙR^{7h},
      wherein n = 1, 2, or 3, preferably n = 1, and wherein one of the one, two, or three -CH₂-groups of -(CH₂)ₙR^{7h} which is different from the terminal groups is optionally replaced by -O-, -S-, -SO-, or -SOz-,
      wherein R^{7h} is selected from the group consisting of -H, -OH, -NR⁷ⁱR^{7k}, -N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein each substituent is preferably selected from the group consisting of a methyl group, a halogen atom, -NH₂ and -OH, wherein R⁷ⁱ and R^{7k} are each and independently selected from the group consisting of -H and a methyl group,
   wherein the carbonyl group of Xaa7 is covalently attached to the α-nitrogen atom of Xaa8,
Xaa8 is a residue of an L-α-amino acid of formula (Xa) 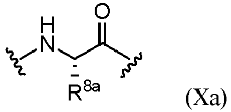 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 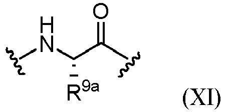 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of Xaa10,
Xaa10 is a residue of formula (XII), 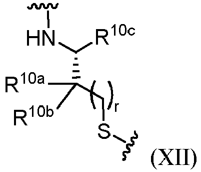 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of Xaa10 is covalently attached to the sulfur atom of Xaa1.

Embodiment 14. The compound or pharmaceutically acceptable salt or hydrate or pharmaceutically acceptable solvate of any one of Embodiments 1, 2 and 3,
wherein
the N-terminal modification group A is covalently attached to the α-nitrogen atom of Xaa1,
wherein the N-terminal modification group A is Xaa0,
   wherein Xaa0 is a residue of formula (IId), 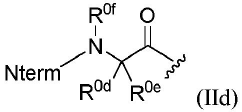 wherein
   R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
   preferably R^{0d} is selected from the group consisting of a (C₂-C₅)alkyl group, a (C₅-C₆)carbocycle, and a phenyl group
   wherein R^{0e} is selected from the group consisting of -H, an aryl group, a (C₁-C₆)alkyl group, and a (C₃-C₇) carbocycle,
   preferably R^{0e} is selected from the group consisting of -H and a methyl group, or wherein R^{0d} and R^{0e} together form a 4-, 5-, 6-, 7- or 8- membered carbocycle or heterocycle, wherein the carbocycle and the heterocycle are optionally substituted by one or two substituents, wherein each substituent is independently selected from the group consisting of a methyl group, -F, -Cl, and -OH,
   preferably R^{0d} and R^{0e} together form a (C₅-C₆) carbocycle, more preferably an unsubstituted (C₅-C₆) carbocycle,
   wherein R^{0f} is selected from the group consisting of -H and a (C₁-C₄)alkyl group, preferably R^{0f} is selected from the group consisting of -H and a methyl group, more preferably R^{0f} is -H,
   wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide, preferably the linker is absent or comprises one α-amino acid or a dipeptide composed of two α-amino acids, most preferably the linker is absent or comprises one amino acid or a dipeptide wherein at least one amino acid comprises a polar or charged side chain,
      wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SOz-, -O-, or -NH-, and wherein R^{0g} is optionally substituted by one, two or three substituents, wherein, preferably, each substituent is individually and independently selected from the group consisting of -OH, -NH₂, a halogen atom, -COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle, preferably R^{0g} is a (C₁-C₄)alkyl group and is optionally substituted by one substituent preferably selected from the group consisting of -OH, -NH₂, -COOH, -CONH₂, -SO₃H, -NH-CNH-NHz and a (C₁-C₄)alkyl group, more preferably R^{0g} is a (C₁-C₄)alkyl group and is optionally substituted by one substituent preferably selected from the group consisting of -OH, -COOH and -CONH₂,
      preferably Nterm is selected from the group consisting of -H, -R^{0g}, R^{0g}-CO-, R^{0g}-NH-CO- and a Z group, more preferably Nterm is selected from the group consisting of -H, -Ac, a succinyl group, a methyl group and a Z group,

Xaa1 is a residue of an amino acid of formula (III) 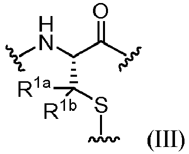 wherein
   R^{1a} and R^{1b} are each and independently selected from a group consisting of -H and a methyl group,
   the carbonyl group of Xaa1 is covalently attached to the α-nitrogen atom of Xaa2, and
   the sulfur atom of Xaa1 is covalently attached to the sulfur atom of the thiol group of Xaa10, preferably as disulfide,
Xaa2 is a residue of an α-amino acid of formula (IVa) 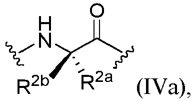 wherein
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group,
   wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent preferably is selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)

   wherein the carbonyl group of Xaa2 is covalently attached to the α-nitrogen atom of Xaa3,
   preferably Xaa2 is a residue of an L-α amino acid of formula (IVa),
Xaa3 is a residue of an α-amino acid of formula (Vc) 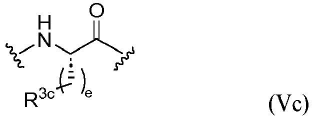 wherein in formula (Vc)
   R^{3c} is selected from the group consisting of -H, -OH, -N(R^{3d})(R^{3e}), -N(R^{3f})(R^{3g})(R^{3h})⁺, -COOH, a halogen atom, -CN, -N₃, -NO₂, -N(R^{3d})-CN(R^{3e})-N(R³ⁱ)(R^{3k}), -SO₂N(R^{3d})(R³¹), -CON(R^{3d})(R³¹), -NH-CON(R^{3d})(R³¹), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,
Xaa4 is a residue of an L-α-amino acid of formula (VI), 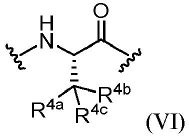 wherein
   R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl, -CN, -COOH, -CONH₂, -NOz, -NH₂, -NH-CNH-NH₂, -SO₃H, -OH, an -O(C₁-C₆)alkyl group, wherein each substituent is optionally linked, preferably via a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
   R^{4b} and R^{4c} are each and independently selected from the group consisting of - H and a methyl group, and
   wherein the carbonyl group of Xaa4 is covalently attached to the α-nitrogen atom of Xaa5,
Xaa5 is a residue of an L-α-amino acid of formula (VII), 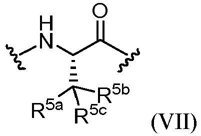 wherein
   R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
   wherein R^{5b} and R^{5c} are each and independently selected from the group consisting of -H and a methyl group, and
   wherein the carbonyl group of Xaa5 is covalently attached to the α-nitrogen atom of Xaa6,
Xaa6 is a residue of an L-α-amino acid of formula (Villa) 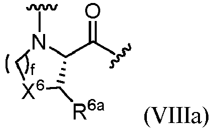 wherein
   f = 1 or 2, preferably f = 1,
   R^{6a} is selected from the group consisting of -H, -OH, -F, and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
   wherein the carbonyl group of Xaa6 is covalently attached to the α-nitrogen atom of Xaa7,
Xaa7 is a residue of an α-amino acid of formula (IXe) 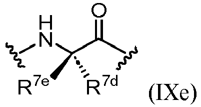 wherein
   R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l}, preferably R^{7d} is selected from the group consisting of -(CH₂)ₒR^{7l}, R^{7l} is selected from the group consisting of a polar, a positively charged, a negatively charged, a zwitterionic and a hydrophobic group, wherein R^{7l} optionally comprises a Z group,
   o = 1, 2, 3, 4 or 5, and wherein one of the one, two, three, four or five -CH₂-groups linking R^{7l} to the α-C atom of Xaa7 in formula (IXe) are optionally substituted by a methyl group, and/or wherein one of said -CH₂- groups which is different from the terminal groups is optionally replaced by -O-, -S-, -SO-, or -SOz-,
   preferably R^{7l} is selected from the group consisting of -H, -OH, -N(R^{7m})(R⁷ⁿ), -N(R^{7o})(R^{7p})(R^{7q})⁺, -COOH, a halogen atom, -CN, -N₃, -NO₂, -N(R^{7m})-CN(R⁷ⁿ)-N(R^{7r})(R^{7s}), -SO₂N(R^{7m})(R^{7u}), -CON(R^{7m})(R^{7u}), -NH-CO-N(R^{7m})(R^{7u}), -SO₃H, -R^{7t}, -NH-SO₂-R^{7t}, and -NH-CO-R^{7t},
      wherein R^{7m}, R^{7r} and R^{7s} are each and independently selected from the group consisting of -H and a (C₁-C₂)alkyl group,
      wherein R^{7o} and R^{7p} are each and independently selected from the group consisting of a (C₁-C₃)alkyl group,
   wherein R⁷ⁿ is selected from the group consisting of -H, -Ac, a (C₁-C₃)alkyl group and a Z group,
      wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of -OH, -COOH, and -SO₃H, preferably R^{7q} is selected from the group consisting of a methyl group and a -(CH₂)₃SO₃H group,
      wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{7t} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{7t} is a (C₁-C₃)alkyl group,
   wherein R^{7u} is selected from the group consisting of -H and R^{7t}, preferably R^{7u} is selected from the group consisting of -H and a methyl group, most preferably R^{7u} is -H,
   R^{7e} is selected from the group consisting of -H, and a (C₁-C₃)alkyl group, wherein said (C₁-C₃)alkyl group is optionally substituted by a substituent preferably selected from the group consisting of -F, -Cl and -OH, and
   wherein the carbonyl group of Xaa7 is covalently attached to the α-nitrogen atom of Xaa8,
Xaa8 is a residue of an L-α-amino acid of formula (Xa) 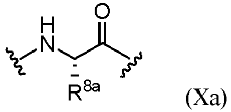 wherein
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
   wherein the carbonyl group of Xaa8 is covalently attached to the α-nitrogen atom of Xaa9,
Xaa9 is a residue of an α-amino acid of formula (XI) 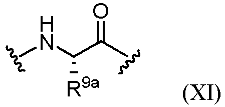 wherein
   R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl, and
   wherein the carbonyl group of Xaa9 is covalently attached to the nitrogen atom of the amine group of Xaa10,
Xaa10 is a residue of formula (XII), 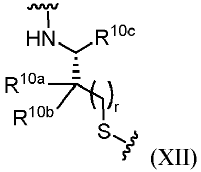 wherein
   R^{10a} and R^{10b} are each and independently selected from the group consisting of -H and a methyl group,
   r = 1 or 2,
   R^{10c} is selected from the group consisting of -H, -CO-Cterm, -CONH₂, -CH₂(OH), -CON(R^{10d})(R^{10e}), wherein Cterm comprises a Z group,
   wherein R^{10d} and R^{10e} are each and independently selected from the group consisting of -H and a methyl group,
   and the sulfur atom of Xaa10 is covalently attached to the sulfur atom of Xaa1.

Embodiment 15. The compound or pharmaceutically acceptable salt or hydrate or pharmaceutically acceptable solvate of any one of Embodiments 1, 2 and 3, wherein
the N-terminal modification group A is covalently attached to the α-nitrogen atom of Xaa1, and
the N-terminal modification group A is of formula (IIa), of formula (IIb) or of formula (IIc) 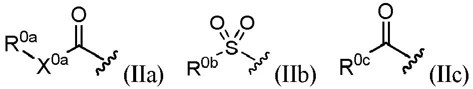
   wherein X^{0a} is selected from the group consisting of -NH-, -S- and -O-, preferably X^{0a} is selected from the group consisting of -NH- and -O-,
   wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0a} is selected from the group consisting of a (C₃-C₆)alkyl group, a phenyl group and a (C₅-C₆)carbocycle,
   wherein R^{0b} is selected from the group consisting of a (C₃-C₉)alkyl group, a (C₃-C₈)carbocycle, an aryl group, a heteroaryl group, and a (C₃-C₈)heterocycle, and wherein R^{0b} is optionally linked to the -SOz- moiety in formula (IIb) by a linker comprising one, two or three -CH₂- groups, preferably the linker is absent or consists of one or two -CH₂- group, and wherein R^{0b} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0b} is selected from the group consisting of a (C₄-C₆)alkyl group, a phenyl group and a (C₅-C₆)carbocycle,
   wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- group, wherein one -CH₂- group of R^{0c} is optionally replaced by -O- or -S-, and wherein R^{0c} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,
   preferably R^{0c} is selected from the group consisting of a (C₄-C₆)alkyl group, a phenyl group and a (C₅-C₆)carbocycle.

Embodiment 16. The compound or pharmaceutically acceptable salt or hydrate or pharmaceutically acceptable solvate of any one of Embodiments 1, 2, 3 and 15, wherein the N-terminal modification group A is of formula (IIa) 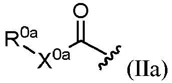
wherein X^{0a} is selected from the group consisting of -NH-, -S- and -O-,
wherein R^{0a} is selected from the group consisting of a (C₃-C₉)alkyl group, a (C₃-C₈)carbocycle, an aryl group, a heteroaryl group, and a (C₃-C₈)heterocycle, and wherein R^{0a} is optionally linked to X^{0a} by a linker comprising one or two - CH₂- groups, preferably the linker is absent or consists of one -CH₂- group, and wherein R^{0a} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle,

Embodiment 17. The compound or pharmaceutically acceptable salt or hydrate or pharmaceutically acceptable solvate of any one of Embodiments 15 and 16, wherein X^{0a} is selected from the group consisting of -NH- and -O-.

Embodiment 18. The compound or pharmaceutically acceptable salt or hydrate or pharmaceutically acceptable solvate of Embodiment 17, wherein X^{0a} is -O-.

Embodiment 19. The compound or pharmaceutically acceptable salt or hydrate or pharmaceutically acceptable solvate of any one of Embodiments 15, 16, 17 and 18, preferably of Embodiment 18, wherein R^{0a} is selected from the group consisting of a (C₃-C₆)alkyl group, a phenyl group and a (C₅-C₆)carbocycle.

Embodiment 20. The compound or pharmaceutically acceptable salt or hydrate or pharmaceutically acceptable solvate of Embodiment 19, wherein R^{0a} is selected from the group consisting of a (C₃-C₆)alkyl group, preferably a n-butyl group.

Embodiment 21. The compound or pharmaceutically acceptable salt or hydrate or pharmaceutically acceptable solvate of any one of Embodiments 1, 2, 3 and 15, wherein the N-terminal modification group A is of formula (IIc) 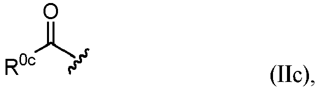 wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂- groups, preferably the linker is absent or consists of one or two -CH₂- group, wherein one -CH₂- group of R^{0c} is optionally replaced by -O- or -S-, and wherein R^{0c} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle.

Embodiment 22. The compound or pharmaceutically acceptable salt or hydrate or pharmaceutically acceptable solvate of any one of Embodiments 15 and 21, wherein R^{0c} is selected from the group consisting of a (C₄-C₆)alkyl group, a phenyl group and a (C₅-C₆)carbocycle.

Embodiment 23. The compound or pharmaceutically acceptable salt or hydrate or pharmaceutically acceptable solvate of Embodiment 22, wherein R^{0c} is selected from the group consisting of a (C₄-C₆)alkyl group.

Embodiment 24. The compound or pharmaceutically acceptable salt or hydrate or pharmaceutically acceptable solvate of any one of Embodiments 1, 2, 3 and 15, wherein the N-terminal modification group A is of formula (IIb) 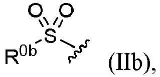 wherein R^{0b} is selected from the group consisting of a (C₃-C₉)alkyl group, a (C₃-C₈)carbocycle, an aryl group, a heteroaryl group, and a (C₃-C₈)heterocycle, and wherein R^{0b} is optionally linked to the -SO₂-moiety in formula (IIb) by a linker comprising one, two or three -CH₂- groups, preferably the linker is absent or consists of one or two -CH₂- groups, and wherein R^{0b} is optionally substituted by one or two substituents, wherein, preferably, each substituent is individually and independently selected from the group consisting of -OH, a halogen atom, a (C₁-C₆)alkyl group, a (C₃-C₈)carbocycle, an aryl group, a heteroaryl group, and a (C₃-C₈)heterocycle.

Embodiment 25. The compound or pharmaceutically acceptable salt or hydrate or pharmaceutically acceptable solvate of any one of Embodiments 15 and 24, wherein R^{0b} is selected from the group consisting of a (C₄-C₆)alkyl group, a phenyl group and a (C₅-C₆)carbocycle.

Embodiment 26. The compound or pharmaceutically acceptable salt or hydrate or pharmaceutically acceptable solvate of Embodiment 25, wherein R^{0b} is selected from the group consisting of a (C₄-C₆)alkyl group.

Embodiment 27. The compound or pharmaceutically acceptable salt or hydrate or pharmaceutically acceptable solvate of any one of Embodiments 1, 2 and 3, wherein the N-terminal modification group A is covalently attached to the α-nitrogen atom of Xaa1, and wherein the N-terminal modification group A is Xaa0, wherein
Xaa0 is a residue of an α-amino acid comprising a side chain and optionally comprising up to three substituents covalently attached to the α-nitrogen atom of Xaa0, wherein the side chain comprises a hydrophobic group, and wherein, if present, one substituent covalently attached to the α-nitrogen atom of Xaa0 is an N-terminal extension group Nterm, and wherein the N-terminal extension group Nterm optionally comprises a Z group, and
Xaa0 is a residue of formula (IId) or of formula (IIe)

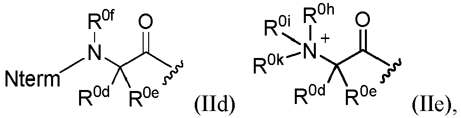

wherein R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
preferably R^{0d} is selected from the group consisting of a (C₂-C₅)alkyl group, a (C₅-C₆)carbocycle, and a phenyl group
wherein R^{0e} is selected from the group consisting of -H, an aryl group, a (C₁-C₆)alkyl group, and a (C₃-C₇) carbocycle,
preferably R^{0e} is selected from the group consisting of -H and a methyl group,
or wherein R^{0d} and R^{0e} together form a 4-, 5-, 6-, 7- or 8- membered carbocycle or heterocycle, wherein the carbocycle and the heterocycle are optionally substituted by one or two substituents, wherein, preferably, each substituent is individually and independently selected from the group consisting of a methyl group, -F, -Cl, and -OH,
preferably R^{0d} and R^{0e} together form a (C₅-C₆) carbocycle, more preferably an unsubstituted (C₅-C₆) carbocycle,
wherein R^{0f} is selected from the group consisting of -H and a (C₁-C₄)alkyl group, preferably R^{0f} is selected from the group consisting of -H and a methyl group, more preferably R^{0f} is -H,
wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide, preferably the linker is absent or comprises one α-amino acid or a dipeptide composed of two α-amino acids, most preferably the linker is absent or comprises one amino acid or a dipeptide wherein at least one amino acid comprises a polar or charged side chain,
   wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SO₂-, -O-, or -NH-, and wherein R^{0g} is optionally substituted by one, two or three substituents, wherein, preferably, each substituent is individually and independently selected from the group consisting of -OH, -NH₂, a halogen atom, - COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle, preferably R^{0g} is a (C₁-C₄)alkyl group and is optionally substituted by one substituent preferably selected from the group consisting of -OH, -NH₂, -COOH, - CONH₂, -SO₃H, -NH-CNH-NH₂ and a (C₁-C₄)alkyl group, more preferably R^{0g} is a (C₁-C₄)alkyl group and is optionally substituted by one substituent preferably selected from the group consisting of -OH, - COOH and -CONH₂,
   preferably Nterm is selected from the group consisting of -H, R^{0g}, R^{0g}-CO-, R^{0g}-NH-CO- and a Z group, more preferably Nterm is selected from the group consisting of -H, -Ac, a succinyl group, a methyl group and a Z group,
wherein R^{0h}, R⁰ⁱ and R^{0k} are each and independently selected from the group consisting of a (C₁-C₄)alkyl group, preferably R^{0h}, R⁰ⁱ and R^{0k} each are methyl groups.

Embodiment 28. The compound or pharmaceutically acceptable salt or hydrate or pharmaceutically acceptable solvate of any one of Embodiment 1, 2, 3 and 27, wherein
Xaa0 is a residue of formula (IId) 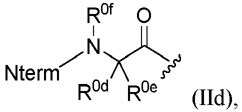 wherein
R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
wherein R^{0e} is selected from the group consisting of -H, an aryl group, a (C₁-C₆)alkyl group, and a (C₃-C₇) carbocycle,
or wherein R^{0d} and R^{0e} together form a 4-, 5-, 6-, 7- or 8- membered carbocycle or heterocycle, wherein the carbocycle and the heterocycle are optionally substituted by one or two substituents, wherein, preferably, each substituent is individually and independently selected from the group consisting of a methyl group, -F, -Cl, and -OH,
wherein R^{0f} is selected from the group consisting of -H and a (C₁-C₄)alkyl group,
wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide,
   wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SO₂-, -O-, or -NH-, and wherein R^{0g} is optionally substituted by one, two or three substituents, wherein, preferably, each substituent is individually and independently selected from the group consisting of -OH, -NH₂, a halogen atom, - COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle,

Embodiment 29. The compound or pharmaceutically acceptable salt or hydrate or pharmaceutically acceptable solvate of any one of Embodiments 27 and 28, wherein R^{0d} is selected from the group consisting of a (C₂-C₅)alkyl group, a (C₅-C₆)carbocycle, and a phenyl group.

Embodiment 30. The compound or pharmaceutically acceptable salt or hydrate or pharmaceutically acceptable solvate of any one of Embodiments 27, 28 and 29, preferably Embodiment 29, wherein R^{0e} is selected from the group consisting of -H and a methyl group.

Embodiment 31. The compound or pharmaceutically acceptable salt or hydrate or pharmaceutically acceptable solvate of Embodiment 30, wherein R^{0e} is -H.

Embodiment 32. The compound or pharmaceutically acceptable salt or hydrate or pharmaceutically acceptable solvate of any one of Embodiments 27, 28, 29, 30 and 31, preferably any one of Embodiments 29, 30 and 31, wherein R^{0f} is selected from the group consisting of -H and a methyl group.

Embodiment 33. The compound or pharmaceutically acceptable salt or hydrate or pharmaceutically acceptable solvate of Embodiment 32, wherein R^{0f} is -H.

Embodiment 34. The compound or pharmaceutically acceptable salt or hydrate or pharmaceutically acceptable solvate of any one of Embodiments 27, 28, 29, 30, 31, 32 and 33, preferably any one of Embodiments 29, 30, 31, 32 and 33, wherein Nterm is selected from the group consisting of -H, -R^{0g}, R^{0g}-CO-, R^{0g}-NH-CO- and a Z group.

Embodiment 35. The compound or pharmaceutically acceptable salt or hydrate or pharmaceutically acceptable solvate of Embodiment 34, wherein Nterm is selected from the group consisting of -H, -Ac, a succinyl group, a methyl group and a Z group.

Embodiment 36. The compound or pharmaceutically acceptable salt or hydrate or pharmaceutically acceptable solvate of any one of Embodiments 27, 28, 29, 30, 31, 32, 33, 34 and 35, preferably any one of Embodiments 29, 30, 31, 32, 33, 34 and 35, wherein R^{0g} is a (C₁-C₄)alkyl group and is optionally substituted by one substituent preferably selected from the group consisting of -OH, -NH₂, -COOH, - CONH₂, -SO₃H, -NH-CNH-NHz and a (C₁-C₄)alkyl group.

Embodiment 37. The compound or pharmaceutically acceptable salt or hydrate or pharmaceutically acceptable solvate of Embodiment 36, wherein R^{0g} is a (C₁-C₄)alkyl group and is optionally substituted by one substituent preferably selected from the group consisting of -OH, -COOH and -CONH₂.

Embodiment 38. The compound or pharmaceutically acceptable salt or hydrate or pharmaceutically acceptable solvate of any one of Embodiments 27, 28, 29, 30, 31, 32, 33, 34, 35, 36 and 37, wherein Nterm is linked to the α-nitrogen atom of Xaa0, preferably without a linker.

Embodiment 39. The compound or pharmaceutically acceptable salt or hydrate or pharmaceutically acceptable solvate of any one of Embodiments 27, 28, 29, 30, 31, 32, 33, 34, 35, 36, 37 and 38, wherein Nterm is linked to the α-nitrogen atom of Xaa0 by a linker comprising one amino acid or a dipeptide.

Embodiment 40. The compound or pharmaceutically acceptable salt or hydrate or pharmaceutically acceptable solvate of Embodiment 39, wherein the linker is a linker comprising one amino acid and the one amino acid is an α-amino acid.

Embodiment 41. The compound or pharmaceutically acceptable salt or hydrate or pharmaceutically acceptable solvate of Embodiment 39, wherein the linker is a linker comprising a dipeptide, wherein the dipeptide consists of two α-amino acids, wherein at least one of the two α-amino acids comprises a polar or charged side chain.

Embodiment 42. The compound or pharmaceutically acceptable salt or hydrate or pharmaceutically acceptable solvate of any one of Embodiments 27, 28, 29, 30, 31, 32, 33, 34, 35, 36, 37, 38, 39, 40 and 41, wherein Xaa0 is an L-α-amino acid of formula (IId).

Embodiment 43. The compound or pharmaceutically acceptable salt or hydrate or pharmaceutically acceptable solvate of any one of Embodiments 27, 28, 29, 30, 31, 32, 33, 34, 35, 36, 37, 38, 39, 40, 41 and 42, wherein R^{0d} and R^{0e} together form a (C₅-C₆) carbocycle.

Embodiment 44. The compound or pharmaceutically acceptable salt or hydrate or pharmaceutically acceptable solvate of Embodiment 43, wherein R^{0d} and R^{0e} together form an unsubstituted (C₅-C₆) carbocycle.

Embodiment 45. The compound or pharmaceutically acceptable salt or hydrate or pharmaceutically acceptable solvate of any one of Embodiments 1, 2, 3 and 27,
wherein Xaa0 is a residue of formula (IIe) 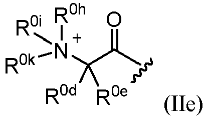
wherein R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, preferably the linker is absent or comprises one -CH₂- group, and wherein R^{0d} is optionally substituted by one or two substituents, wherein, preferably, each substituent is individually and independently selected from the group consisting of -OH, -F, -Cl, a (C₁-C₆)alkyl group, a (C₃-C₈)carbocycle group, an aryl group, a heteroaryl group, and a (C₃-C₈)heterocycle,
wherein R^{0e} is selected from the group consisting of -H, an aryl group, a (C₁-C₆)alkyl group, and a (C₃-C₇) carbocycle,
or wherein R^{0d} and R^{0e} together form a 4-, 5-, 6-, 7- or 8- membered carbocycle or heterocycle, wherein the carbocycle and the heterocycle are optionally substituted by one or two substituents, wherein, preferably each substituent is individually and independently selected from the group consisting of a methyl group, -F, -Cl, and -OH,
wherein R^{0h}, R⁰ⁱ and R^{0k} are each and independently selected from the group consisting of a (C₁-C₄)alkyl group.

Embodiment 46. The compound or pharmaceutically acceptable salt or hydrate or pharmaceutically acceptable solvate of any one of Embodiments 27 and 45, wherein R^{0h}, R⁰ⁱ and R^{0k} each are methyl groups.

Embodiment 47. The compound or pharmaceutically acceptable salt or hydrate or pharmaceutically acceptable solvate of any one of Embodiments 27, 45 and 46, wherein R^{0d} is selected from the group consisting of a (C₂-C₅)alkyl group, a (C₅-C₆)carbocycle, and a phenyl group.

Embodiment 48. The compound or pharmaceutically acceptable salt or hydrate or pharmaceutically acceptable solvate of any one of Embodiments 27, 45, 46 and 47, preferably any of Embodiments 46 and 47, wherein R^{0e} is selected from the group consisting of -H and a methyl group.

Embodiment 49. The compound or pharmaceutically acceptable salt or hydrate or pharmaceutically acceptable solvate of Embodiment 48, wherein R^{0e} is -H.

Embodiment 50. The compound or pharmaceutically acceptable salt or hydrate or pharmaceutically acceptable solvate of any one of Embodiments 27, 45, 46, 47, 48 and 49, wherein Xaa0 is an L-α-amino acid of formula (IIe).

Embodiment 51. The compound or pharmaceutically acceptable salt or hydrate or pharmaceutically acceptable solvate of any one of Embodiments 27, 45 and 46, wherein R^{0d} and R^{0e} together form a (C₅-C₆)carbocycle.

Embodiment 52. The compound or pharmaceutically acceptable salt or hydrate or pharmaceutically acceptable solvate of Embodiment 51, wherein R^{0d} and R^{0e} together form an unsubstituted (C₅-C₆) carbocycle.

Embodiment 53. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51 and 52, preferably any one of Embodiments 1, 2, 3, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51 and 52, wherein Xaa1 is a residue of an amino acid of formula (III) 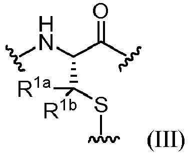 wherein
R^{1a} and R^{1b} are each and independently selected from a group consisting of -H and a methyl group,
the carbonyl group of Xaa1 is covalently attached to the α-nitrogen atom of Xaa2, and the sulfur atom of Xaa1 is covalently attached to the sulfur atom of the thiol group of Xaa10.

Embodiment 54. The compound or pharmaceutically acceptable salt or hydrate or pharmaceutically acceptable solvate of Embodiment 53, wherein the sulfur atom of Xaa1 is covalently attached as disulfide to the sulfur atom of the thiol group of Xaa10.

Embodiment 55. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51, 52, 53 and 54, preferably any one of Embodiments 1, 2, 3, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51, 52, 53 and 54, wherein Xaa2 is a residue of an amino acid of formula (IVa), (IVb), or (IVc) 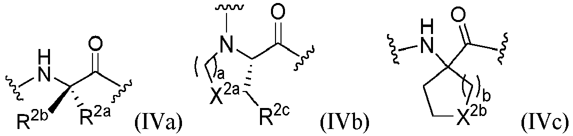 wherein
in formula (IVa)
   R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d}, preferably R^{2a} is selected from the group consisting of -H and - (CH₂)_{c}R^{2d},
      wherein R^{2d} is selected from the group consisting of a polar, a positively charged, a negatively charged, a zwitterionic and a hydrophobic group, wherein R^{2d} optionally comprises a Z group, preferably R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NOz, -N(R^{2e})-CN(R^{2f})-N(R^{2k})(R^{2l}), -SO₂N(R^{2e})(R^{2m}), - CON(R^{2e})(R^{2m}), -NH-CO-N(R^{2e})(R^{2m}), -SO₃H, -R²ⁿ, -NH-SO₂-R²ⁿ, -CO-R^{2o}, and -NH-CO-R²ⁿ,
      wherein R^{2e}, R^{2k} and R^{2l} are each and independently selected from the group consisting of -H and a (C₁-C₂)alkyl group,
      wherein R^{2g} and R^{2h} are each and independently selected from the group consisting of a (C₁-C₃)alkyl group,
   wherein R^{2f} is selected from the group consisting of -H, -Ac, a (C₁-C₃)alkyl group and a Z group,
      wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R²ⁱ is selected from the group consisting of a methyl group and a -(CH₂)₃SO₃H group,
   wherein R^{2m} is selected from the group consisting of -H and R²ⁿ, preferably R^{2m} is selected from the group consisting of -H and a methyl group, most preferably R^{2m} is -H,
      wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R²ⁿ is a (C₁-C₄)alkyl group which is optionally substituted by a substituent preferably selected from the group consisting of -CONH₂, -COOH, a halogen atom, - NH-CNH-NH₂, -NH₂ and -OH, more preferably R²ⁿ is a (C₁-C₃)alkyl group, wherein R^{2o} is a Z group,
      wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
   R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F, preferably an -OH group,
   or R^{2b} is -H under the proviso that R^{2a} is -H,
   or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH, preferably said cycle is of formula (IVc)
in formula (IVb)
   X^{2a} is selected from the group consisting of -CH₂-, -CH(OH), -CH(F)-, -CH(NHR^{2p})-, -NH-, -S- and -O-, wherein R^{2p} is selected from the group consisting of -H, -Ac, a (C₁-C₄)alkyl and a Z group, preferably R^{2p} is selected from the group consisting of -H, a methyl groupand a Z group, preferably X^{2a} is -CH₂-,
   R^{2c} is selected from the group consisting of -H, -OH, -F, -NH₂ and a (C₁-C₃)alkyl group, preferably R^{2c} is -H,
   a = 1 or 2, preferably a = 1,
in formula (IVc)
   b = 1, 2 or 3,
   X^{2b} is selected from the group consisting of -N(R^{2p})-, -O-, -S-, -SO-, -SOz-,
   -NH-, and -CH₂-, wherein R^{2p} is selected from the group consisting of -Ac, a (C₁-C₃)alkyl group and a Z group,
preferably Xaa2 is a residue of an L-α amino acid of formula (IVa) or Xaa2 is a residue of an amino acid of formula (IVc), and more preferably Xaa2 is a residue of an L-α amino acid of formula (IVa).

Embodiment 56. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51, 52, 53, 54 and 55, preferably any one of Embodiments 1, 2, 3, 15, 16, 17, 18, 19, 20, 21, 22, 23, 24, 25, 26, 27, 28, 29, 30,31, 32, 33, 34, 35, 36, 37, 38, 39, 40, 41, 42, 43, 44, 45, 46, 47, 48, 49, 50, 51, 52, 53, 54 and 55, wherein Xaa2 is a residue of an amino acid of formula (IVa) 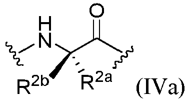 wherein
R^{2a} is selected from the group consisting of -H, a (C₁-C₆)alkyl group, a (C₃-C₇)carbocycle, (C₃-C₇)heterocycle, an aryl group, a heteroaryl group and - (CH₂)_{c}R^{2d},
   wherein R^{2d} is selected from the group consisting of a polar, a positively charged, a negatively charged, a zwitterionic and a hydrophobic group, wherein R^{2d} optionally comprises a Z group,
   wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F,
or R^{2b} is -H under the proviso that R^{2a} is -H,
or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH.

Embodiment 57. The compound or pharmaceutically acceptable salt or hydrate or pharmaceutically acceptable solvate of any one of Embodiments 55 and 56, wherein R^{2a} is selected from the group consisting of -H and -(CH₂)_{c}R^{2d}.

Embodiment 58. The compound or pharmaceutically acceptable salt or hydrate or pharmaceutically acceptable solvate of any one of Embodiments 56 and 57, wherein R^{2d} is selected from the group consisting of -H, -OH, -N(R^{2e})(R^{2f}), -N(R^{2g})(R^{2h})(R²ⁱ)⁺, -COOH, a halogen atom, -CN, -N₃, -NO₂, -N(R^{2e})-CN(R^{2f})-N(R^{2k})(R^{2l}), -SO₂N(R^{2e})(R^{2m}), -CON(R^{2e})(R^{2m}), -NH-CO-N(R^{2e})(R^{2m}), -SO₃H, - R²ⁿ, -NH-SO₂-R²ⁿ, -CO-R^{2o}, and -NH-CO-R²ⁿ,
wherein R^{2e}, R^{2k} and R^{2l} are each and independently selected from the group consisting of -H and a (C₁-C₂)alkyl group,
wherein R^{2g} and R^{2h} are each and independently selected from the group consisting of a (C₁-C₃)alkyl group,
wherein R^{2f} is selected from the group consisting of -H, -Ac, a (C₁-C₃)alkyl group and a Z group,
wherein R²ⁱ is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H,
wherein R^{2m} is selected from the group consisting of -H and R²ⁿ,
wherein R²ⁿ is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R²ⁿ is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH,
wherein R^{2o} is a Z group.

Embodiment 59. The compound or pharmaceutically acceptable salt or hydrate or pharmaceutically acceptable solvate of Embodiment 58, wherein R^{2e}, R^{2k} and R^{2l} are each -H.

Embodiment 60. The compound or pharmaceutically acceptable salt or hydrate or pharmaceutically acceptable solvate of Embodiment 58, wherein R^{2g} and R^{2h} are each a methyl group.

Embodiment 61. The compound or pharmaceutically acceptable salt or hydrate or pharmaceutically acceptable solvate of any of Embodiments 58 and 59, wherein R^{2f} is selected from the group consisting of -H, a methyl group and a Z group.

Embodiment 62. The compound or pharmaceutically acceptable salt or hydrate or pharmaceutically acceptable solvate of any of Embodiments 58 and 60, wherein R²ⁱ is selected from the group consisting of a methyl group and a -(CH₂)₃SO₃H group.

Embodiment 63. The compound or pharmaceutically acceptable salt or hydrate or pharmaceutically acceptable solvate of Embodiment 58, wherein R²ⁿ is a (C₁-C₄)alkyl group which is optionally substituted by a substituent preferably selected from the group consisting of -CONH₂, -COOH, a halogen atom, -NH-CNH-NH₂, -NH₂ and -OH.

Embodiment 64. The compound or pharmaceutically acceptable salt or hydrate or pharmaceutically acceptable solvate of any one of Embodiment 58 and 63, wherein R²ⁿ is a (C₁-C₃)alkyl group.

Embodiment 65. The compound or pharmaceutically acceptable salt or hydrate or pharmaceutically acceptable solvate of any of Embodiments 58 and 59, wherein R^{2m} is selected from the group consisting of -H and a methyl group.

Embodiment 66. The compound or pharmaceutically acceptable salt or hydrate or pharmaceutically acceptable solvate of Embodiment 65, wherein R^{2m} is H.

Embodiment 67. The compound or pharmaceutically acceptable salt or hydrate or pharmaceutically acceptable solvate of any one of Embodiments 56 and 57, wherein R^{2d} is selected from the group consisting of a polar, a positively charged, a negatively charged and a zwitterionic group, wherein R^{2d} optionally comprises a Z group.

Embodiment 68. The compound or pharmaceutically acceptable salt or hydrate or pharmaceutically acceptable solvate of any one of Embodiments 56, 57, 58, 63, 64 and 67, wherein R^{2d} is selected from the group consisting of -H, -OH, -NH₂, -NH(R^{2f}), -N(CH₃)₂, -N(CH₃)₃⁺, -COOH, -NH-CNH-NHz, - CONH₂, -NH-CO-NH₂, -SO₃H, -CO-R^{2o} and -R²ⁿ, wherein R^{2f} is selected from the group consisting of -H, a methyl group and a Z group, wherein R^{2o} is a Z group, and wherein R²ⁿ is selected from the group consisting of a (C₁-C₃)alkyl group.

Embodiment 69. The compound or pharmaceutically acceptable salt or hydrate or pharmaceutically acceptable solvate of Embodiment 68, wherein R^{2d} is selected from the group consisting of -H, -OH, - NH₂, -NH(R^{2f}), -N(CH₃)₂, -N(CH₃)₃⁺, -NH-CNH-NH₂, -CONH₂, -NH-CO-NH₂ and -CO-R^{2o}, wherein R^{2f} is selected from the group consisting of -H, a methyl group and a Z group and wherein R^{2o} is a Z group.

Embodiment 70. The compound or pharmaceutically acceptable salt or hydrate or pharmaceutically acceptable solvate of any one of Embodiment 1, 2, 3 and 56, wherein R^{2b} is -H or a (C₁-C₃)alkyl group which is optionally substituted by -OH under the proviso that R^{2a} is -(CH₂)_{c}R^{2d}, or wherein R^{2b} is -H under the proviso that R^{2a} is -H.

Embodiment 71. The compound or pharmaceutically acceptable salt or hydrate or pharmaceutically acceptable solvate of Embodiment 70, wherein R^{2b} is H.

Embodiment 72. The compound or pharmaceutically acceptable salt or hydrate or pharmaceutically acceptable solvate of any one of Embodiments 56, 57, 58, 59, 60, 61, 62, 63, 64, 65, 66, 67, 68, 69, 70 and 71, preferably Embodiment 71, wherein c = 1, 2, or 3, and wherein optionally one hydrogen atom of said one, two or three -CH₂- groups of -(CH₂)_{c}R^{2d} is substituted by a methyl group, preferably c = 1, one hydrogen atom is substituted by a methyl group and R^{2d} is OH.

Embodiment 73. The compound or pharmaceutically acceptable salt or hydrate or pharmaceutically acceptable solvate of any one of Embodiments 56, 57, 58, 59, 60, 61, 62, 63, 64, 65, 66, 67, 68, 69, 70, 71 and 72, wherein Xaa2 is a residue of an L-α amino acid of formula (IVa).

Embodiment 74. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 and 56, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 and 56, wherein Xaa2 is a residue of an amino acid of formula (IVc) 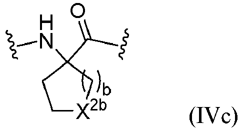 wherein
b = 1, 2 or 3,
X^{2b} is selected from the group consisting of -N(R^{2p})-, -O-, -S-, -SO-, -SOz-, -NH-, and -CH₂-, wherein R^{2p} is selected from the group consisting of -Ac, a (C₁-C₃)alkyl group and a Z group.

Embodiment 75. The compound or pharmaceutically acceptable salt or hydrate or pharmaceutically acceptable solvate of Embodiment 74, wherein b = 2.

Embodiment 76. The compound or pharmaceutically acceptable salt or hydrate or pharmaceutically acceptable solvate of any one of Embodiments 74 and 75, wherein X^{2b} is selected from the group consisting of -N(R^{2p})-, -O-, -NH-, and -CH₂-, wherein R^{2p} is selected from the group consisting of a methyl group and a Z group.

Embodiment 77. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 and 55, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 and 55, wherein Xaa2 is a residue of an amino acid of formula (IVb) 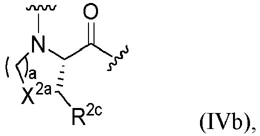 wherein
X^{2a} is selected from the group consisting of -CH₂-, -CH(OH)-, -CH(F)-, -CH(NHR^{2p})-, -NH-, - S- and -O-, wherein R^{2p} is selected from the group consisting of -H, -Ac, a (C₁-C₄)alkyl and a Z group,
R^{2c} is selected from the group consisting of -H, -OH, -F, -NH₂ and a (C₁-C₃)alkyl group,
a = 1 or 2.

Embodiment 78. The compound or pharmaceutically acceptable salt or hydrate or pharmaceutically acceptable solvate of any one of Embodiments 55 and 77, wherein R^{2p} is selected from the group consisting of -H, a methyl group and a Z group.

Embodiment 79. The compound or pharmaceutically acceptable salt or hydrate or pharmaceutically acceptable solvate of any one of Embodiments 77 and 78, wherein R^{2c} is -H.

Embodiment 80. The compound or pharmaceutically acceptable salt or hydrate or pharmaceutically acceptable solvate of any one of Embodiments 77and 79, wherein X^{2a} is -CH₂-.

Embodiment 81. The compound or pharmaceutically acceptable salt or hydrate or pharmaceutically acceptable solvate of any one of Embodiments 77, 78, 79 and 80, wherein a = 1.

Embodiment 82. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 and, 81, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 and 81, wherein Xaa3 is a residue of an amino acid of formula (Va), or (Vb), 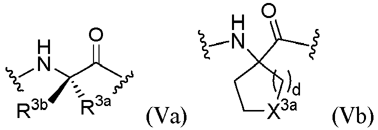 wherein
in formula (Va)
   R^{3a} is selected from the group consisting of -H, a (C₁-C₆)alkyl group, a (C₃-C₇)carbocycle, (C₃-C₇)heterocycle, an aryl group, a heteroaryl group and - (CH₂)ₑR^{3c},
      wherein R^{3c} is selected from the group consisting of a polar, a positively charged, a negatively charged, a zwitterionic and a hydrophobic group, wherein R^{3c} optionally comprises a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups is optionally replaced by -O-, -S-, -SO- or -SOz-,
   R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent is preferably selected from the group consisting of -OH, -COOH, and -F, preferably an -OH group,
   or R^{3b} is -H under the proviso that R^{3a} is -H,
   or R^{3a} and R^{3b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NHz and -OH, preferably said cycle is of formula (Vb)
in formula (Vb)
   d = 1, 2 or 3,
   X^{3a} is selected from the group consisting of -N(R^{3o})-, -O-, -S-, -SO-, -SOz, -NH- , and -CH₂-, wherein R^{3o} is selected from the group consisting of -Ac, a (C₁-C₃)alkyl group and a Z group,
wherein the carbonyl group of Xaa3 is covalently attached to the α-nitrogen atom of Xaa4,
preferably Xaa3 is a residue of an amino acid of formula (Va), more preferably Xaa3 is a residue of an L-α amino acid of formula (Vc) 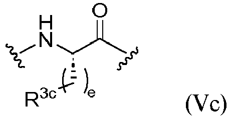 wherein
in formula (Vc)
   R^{3c} is selected from the group consisting of -H, -OH, -N(R^{3d})(R^{3e}), -N(R^{3f})(R^{3g})(R^{3h})⁺, -COOH, a halogen atom, -CN, -N₃, -NO₂, -N(R^{3d})-CN(R^{3e})-N(R³ⁱ)(R^{3k}), -SO₂N(R^{3d})(R^{3l}), -CON(R^{3d})(R^{3l}), -NH-CON(R^{3d})(R^{3l}), -SO₃H, -R^{3m}, -NH-SO₂-R^{3m}, -CO-R³ⁿ, and -NH-CO-R^{3m},
      wherein R^{3d}, R³ⁱ and R^{3k} are each and independently selected from the group consisting of -H and a (C₁-C₂)alkyl group,
      wherein R^{3f} and R^{3g} are each and independently selected from the group consisting of a (C₁-C₃)alkyl group,
   wherein R^{3e} is selected from the group consisting of -H, -Ac, a (C₁-C₃)alkyl group and a Z group,
      wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H, preferably R^{3h} is selected from the group consisting of a methyl group and a -(CH₂)₃SO₃H group,
   wherein R^{3l} is selected from the group consisting of -H and R^{3m}, preferably R^{3l} is selected from the group consisting of -H and a methyl group, most preferably R^{3l} is -H,
      wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{3m} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{3m} is a (C₁-C₃)alkyl group, wherein R³ⁿ is a Z group,
      wherein e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-,

Embodiment 83. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81 and 82, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81 and 82, wherein Xaa3 wherein Xaa3 is a residue of an amino acid of formula (Va), 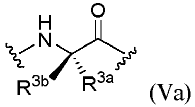 wherein
R^{3a} is selected from the group consisting of -H, a (C₁-C₆)alkyl group, a (C₃-C₇)carbocycle, (C₃-C₇)heterocycle, an aryl group, a heteroaryl group and - (CH₂)ₑR^{3c},
   wherein R^{3c} is selected from the group consisting of a polar, a positively charged, a negatively charged, a zwitterionic and a hydrophobic group, wherein R^{3c} optionally comprises a Z group,
   wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups is optionally replaced by -O-, -S-, -SO- or -SOz-,
R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent is preferably selected from the group consisting of -OH, -COOH, and -F,
or R^{3b} is -H under the proviso that R^{3a} is -H,
   or
R^{3a} and R^{3b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -NH₂ and -OH.

Embodiment 84. The compound or pharmaceutically acceptable salt or hydrate or pharmaceutically acceptable solvate of Embodiment 83, wherein R^{3a} is selected from the group consisting of -H and -(CH₂)ₑR^{3c}.

Embodiment 85. The compound or pharmaceutically acceptable salt or hydrate or pharmaceutically acceptable solvate of any one of Embodiments 83 and 84, wherein R^{3b} is -H or a (C₁-C₃)alkyl group which is optionally substituted by -OH under the proviso that R^{3a} is -(CH₂)ₑR^{3c}, or wherein R^{3b} is -H under the proviso that R^{3a} is -H.

Embodiment 86. The compound or pharmaceutically acceptable salt or hydrate or pharmaceutically acceptable solvate of Embodiment 85, wherein R^{3b} is H.

Embodiment 87. The compound or pharmaceutically acceptable salt or hydrate or pharmaceutically acceptable solvate of Embodiment 83, wherein Xaa3 is a residue of an L-α amino acid of formula (Vc) 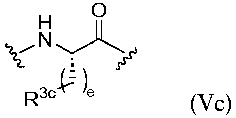 wherein
R^{3c} is selected from the group consisting of -H, -OH, -N(R^{3d})(R^{3e}), - N(R^{3f})(R^{3g})(R^{3h})⁺, -COOH, a halogen atom, -CN, -N₃, -NO₂, - N(R^{3d})-CN(R^{3e})-N(R³ⁱ)(R^{3k}), -SO₂N(R^{3d})(R^{3l}), -CON(R^{3d})(R^{3l}), - NH-CO-N(R^{3d})(R^{3l}), -SO₃H, -R^{3m}, -NH-SO₂-R^{3m}, -CO-R³ⁿ, and -NH-CO-R^{3m},
   wherein R^{3d}, R³ⁱ and R^{3k} are each and independently selected from the group consisting of -H and a (C₁-C₂)alkyl group,
   wherein R^{3f} and R^{3g} are each and independently selected from the group consisting of a (C₁-C₃)alkyl group,
wherein R^{3e} is selected from the group consisting of -H, -Ac, a (C₁-C₃)alkyl group and a Z group,
   wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and-SO₃H,
wherein R^{3l} is selected from the group consisting of -H and R^{3m},
   wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH,
wherein R³ⁿ is a Z group,
e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-.

Embodiment 88. The compound or pharmaceutically acceptable salt or hydrate or pharmaceutically acceptable solvate of any of Embodiments 83, 84, 85, 86 and 87, wherein R^{3d}, R³ⁱ and R^{3k} are each -H.

Embodiment 89. The compound or pharmaceutically acceptable salt or hydrate or pharmaceutically acceptable solvate of any of Embodiments 83, 84, 85, 86 and 87, wherein R^{3f} and R^{3g} are each a methyl group.

Embodiment 90. The compound or pharmaceutically acceptable salt or hydrate or pharmaceutically acceptable solvate of any of Embodiments 83, 84, 85, 86, 87 and 88, wherein R^{3e} is selected from the group consisting of -H, a methyl group and a Z group.

Embodiment 91. The compound or pharmaceutically acceptable salt or hydrate or pharmaceutically acceptable solvate of any of Embodiments 83, 84, 85, 86, 87 and 89, wherein R^{3h} is selected from the group consisting of a methyl group and a -(CH₂)₃SO₃H group.

Embodiment 92. The compound or pharmaceutically acceptable salt or hydrate or pharmaceutically acceptable solvate of any of Embodiments 83, 84, 85, 86 and 87, wherein R^{3m} is a (C₁-C₄)alkyl group which is optionally substituted by a substituent preferably selected from the group consisting of - CONH₂, -COOH, a halogen atom, -NH-CNH-NH₂, -NH₂ and -OH.

Embodiment 93. The compound or pharmaceutically acceptable salt or hydrate or pharmaceutically acceptable solvate of Embodiment 92, wherein R^{3m} is a (C₁-C₃)alkyl group.

Embodiment 94. The compound or pharmaceutically acceptable salt or hydrate or pharmaceutically acceptable solvate of any of Embodiment 83, 84, 85, 86, 87, 88, 92 and 93, wherein R^{3l} is selected from the group consisting of -H and a methyl group.

Embodiment 95. The compound or pharmaceutically acceptable salt or hydrate or pharmaceutically acceptable solvate of Embodiment 94, wherein R^{3l} is -H.

Embodiment 96. The compound or pharmaceutically acceptable salt or hydrate or pharmaceutically acceptable solvate of any one of Embodiments 83, 84, 85, 86 and 87, wherein R^{3c} is selected from the group consisting of a polar, a positively charged, a negatively charged and a zwitterionic group, wherein R^{3c} optionally comprises a Z group.

Embodiment 97. The compound or pharmaceutically acceptable salt or hydrate or pharmaceutically acceptable solvate of any one of Embodiments 83, 84, 85, 86, 87 and 96, wherein R^{3c} comprises a Z group.

Embodiment 98. The compound or pharmaceutically acceptable salt or hydrate or pharmaceutically acceptable solvate of any one of Embodiments 83, 84, 85, 86, 87 and 96, wherein R^{3c} is selected from the group consisting of -H, -OH, -NH₂, -NH(R^{3e}), -N(CH₃)₂, -N(CH₃)₃⁺, -COOH, -NH-CNH-NHz, - CONH₂, -NH-CO-NHz, -SO₃H and -CO-R³ⁿ, wherein R^{3e} is selected from the group consisting of a methyl group and a Z group, and wherein R³ⁿ is a Z group.

Embodiment 99. The compound or pharmaceutically acceptable salt or hydrate or pharmaceutically acceptable solvate of Embodiment 98, wherein wherein R^{3c} is selected from the group consisting of -NH(R^{3e}) and -CO-R³ⁿ, wherein R^{3e} and R³ⁿ are each a Z group, preferably R^{3c} is -NH(R^{3e}) and e is 3 or 4.

Embodiment 100. The compound or pharmaceutically acceptable salt or hydrate or pharmaceutically acceptable solvate of any one of Embodiments 83 to 99, wherein e = 1, 2, 3 and 4 and wherein optionally one hydrogen atom of said one, two, three or four -CH₂- groups of -(CH₂)ₑR^{3c} is substituted by a methyl group.

Embodiment 101. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81 and 82, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81 and 82, wherein Xaa3 wherein Xaa3 is a residue of an amino acid of formula (Vb), 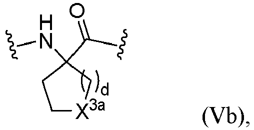 wherein d = 1, 2 or 3,
X^{3a} is selected from the group consisting of -N(R^{3o})-, -O-, -S-, -SO-, - SOz-, -NH-, and -CH₂-, wherein R^{3o} is selected from the group consisting of - Ac, a (C₁-C₃)alkyl group and a Z group,

Embodiment 102. The compound or pharmaceutically acceptable salt or hydrate or pharmaceutically acceptable solvate of Embodiment 101, wherein d = 2.

Embodiment 103. The compound or pharmaceutically acceptable salt or hydrate or pharmaceutically acceptable solvate of any one of Embodiments 101 and 102, wherein X^{3a} is selected from the group consisting of -N(R^{3o})-, -O-, -NH-, and -CH₂-, wherein R^{3o} is selected from the group consisting of a methyl group and a Z group.

Embodiment 104. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102 and 103, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102 and 103, wherein Xaa4 is a residue of an L-α-amino acid of formula (VI), 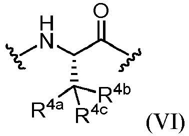 wherein
R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents, wherein, preferably, each substituent is individually and independently selected from the group consisting of a halogen atom, a (C₁-C₆)alkyl group, -CN, -COOH, -CONH₂, -NOz, - NH₂, -NH-CNH-NH₂, -SO₃H, -OH, an -O(C₁-C₆)alkyl group, wherein each substituent is optionally linked via a linker, preferably a methylene bridge, to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
R^{4b} and R^{4c} are each and independently selected from the group consisting of -H and a methyl group.

Embodiment 105. The compound or pharmaceutically acceptable salt or hydrate or pharmaceutically acceptable solvate of Embodiment 104, wherein R^{4b} and R^{4c} are each -H.

Embodiment 106. The compound or pharmaceutically acceptable salt or hydrate or pharmaceutically acceptable solvate of any one of Embodiments 104 and 105, wherein R^{4a} is selected from the group consisting of a (C₆-C₁₀)aryl group and a (C₅-C₁₀)heteroaryl group, wherein each and any of the (C₆-C₁₀)aryl group and the (C₅-C₁₀)heteroaryl group, is optionally substituted by 1 or 2 substituents, wherein, preferably, each substituent is individually and independently selected from the group consisting of a halogen atom, a (C₁-C₃)alkyl group, -CN, -COOH, -CONH₂, -NOz, -NH₂, -NH-CNH-NH₂, -SO₃H, -OH and an -O(C₁-C₃)alkyl group, wherein each substituent is optionally linked via a linker, preferably a methylene bridge, to the ring system of the (C₆-C₁₀)aryl group and the (C₅-C₁₀)heteroaryl group.

Embodiment 107. The compound or pharmaceutically acceptable salt or hydrate or pharmaceutically acceptable solvate of Embodiment 106, wherein R^{4a} is selected from the group consisting of a phenyl group and a (C₅-C₆)heteroaryl group, wherein each and any of the phenyl group and the (C₅-C₆)heteroaryl group, is optionally substituted by 1 or 2 substituents, wherein, preferably, each substituent is individually and independently selected from the group consisting of a halogen atom, a (C₁-C₃)alkyl group, -CN, -CONH₂, -NOz, -NH₂, -NH-CNH-NH₂, -OH and an -O(C₁-C₃)alkyl group, wherein each substituent is optionally linked via a linker, preferably a methylene bridge, to the ring system of the phenyl group and the (C₅-C₆)heteroaryl group.

Embodiment 108. The compound or pharmaceutically acceptable salt or hydrate or pharmaceutically acceptable solvate of Embodiment 107, wherein R^{4a} is selected from the group consisting of a phenyl group, wherein the phenyl group, is optionally substituted by 1 or 2 substituents, wherein, preferably, each substituent is individually and independently selected from the group consisting of -F, -Cl, a methyl group, -CN, -CONH₂, -NH₂, -OH and an -OCH₃ group, wherein each substituent is optionally linked via a linker, preferably a methylene bridge, to the ring system of the phenyl group, more preferably R^{4a} is either a 4-carbamoylphenyl or a 3,4-dihydroxyphenyl group.

Embodiment 109. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107 and 108, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107 and 108, wherein Xaa5 is a residue of an L-α-amino acid of formula (VII), 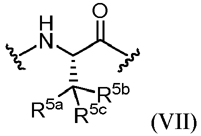 wherein
R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (Ci-C₃)alkyl group, -CN, -NH₂, an -O(C₁-C₃)alkyl group, wherein the (C₁-C₃)alkyl group is optionally substituted by one or more fluorine atoms, and
R^{5b} and R^{5c} are each and independently selected from the group consisting of - H and a methyl group.

Embodiment 110. The compound or pharmaceutically acceptable salt or hydrate or pharmaceutically acceptable solvate of Embodiment 109, wherein R^{5b} and R^{5c} are each -H.

Embodiment 111. The compound or pharmaceutically acceptable salt or hydrate or pharmaceutically acceptable solvate of any one of Embodiments 109 and 110, wherein R^{5a} is selected from the group consisting of a phenyl group, a 2-thienyl group, a 3-pyridyl group, an 1-naphthyl group and a 3-benzothienyl group, wherein each and any of these aryl and heteroaryl groups, is optionally substituted by 1 or 2 substituents, wherein, preferably, each substituent is individually and independently selected from the group consisting of a halogen atom, a methyl group, -CN, -NH₂ and an -OCH₃ group, wherein the methyl group is optionally substituted by one or more fluorine atoms.

Embodiment 112. The compound or pharmaceutically acceptable salt or hydrate or pharmaceutically acceptable solvate of Embodiment 111, wherein R^{5a} is selected from the group consisting of a phenyl group and a 2-thienyl group, wherein each and any of the phenyl group and a 2-thienyl group, is optionally substituted by 1 or 2 substituents, wherein, preferably, each substituent is individually and independently selected from the group consisting of -F, -Cl and a methyl group, more preferably R^{5a} is a phenyl group.

Embodiment 113. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111 and 112, preferably any one of Embodiments 1, 2, 3, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111 and 112, wherein Xaa6 is a residue of an L-α-amino acid of formula (Villa), (VIIIb) or (VIIIc) 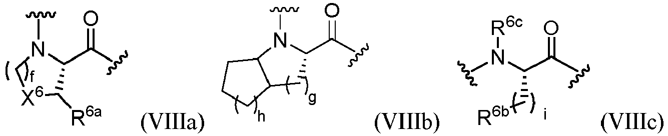 wherein
in formula (Villa)
   f = 1 or 2, preferably f = 1,
   R^{6a} is selected from the group consisting of -H, -OH, -F and a (C₁-C₄)alkyl group,
   X⁶ is absent or selected from the group consisting of -CH(R^{6d})-, -S-, -O- and - NH-, preferably X⁶ is -CH(R^{6d})- or -S-,
      wherein R^{6d} is selected from the group consisting of -H, a (C₁-C₄)alkyl group, -OH and -F,
in formula (VIIIb)
   g = 0, 1 or 2, preferably g = 1,
   h = 1, 2 or 3, preferably h = 1 or 2,
in formula (VIIIc)
   i = 1 or 2, preferably i= 1,
   R^{6b} is selected from the group consisting of a (C₂-C₆)alkyl group, an aryl group, an aryl group substituted with 1 or 2 substituents, a (C₅-C₆)heteroaryl group, a (C₅-C₆)heteroaryl group substituted with 1 or 2 substituents, a (C₅-C₆)carbocycle, and a (C₅-C₆)carbocycle substituted with 1 or 2 substituents, wherein, preferably, each substituent is individually and independently selected from the group consisting of a halogen atom, a (C₁-C₆)alkyl group, -CN, -OH, and an -O(C₁-C₆)alkyl group,
   R^{6c} is selected from the group consisting of -H and a (C₁-C₃)alkyl group.

Embodiment 114. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, wherein Xaa6 is a residue of an L-α-amino acid of formula (Villa) 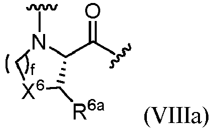 wherein
f = 1 or 2,
R^{6a} is selected from the group consisting of -H, -OH, -F and a (C₁-C₄)alkyl group,
X⁶ is absent or selected from the group consisting of -CH(R^{6d})-, -S-, -O- and - NH-,
   wherein R^{6d} is selected from the group consisting of -H, a (C₁-C₄)alkyl group, -OH and -F.

Embodiment 115. The compound or pharmaceutically acceptable salt or hydrate or pharmaceutically acceptable solvate of Embodiment 114, wherein f = 1.

Embodiment 116. The compound or pharmaceutically acceptable salt or hydrate or pharmaceutically acceptable solvate of any one of Embodiments 114 and 115, wherein X⁶ is -CH(R^{6d})- or -S-, and wherein R^{6d} is selected from the group consisting of -H, a (C₁-C₄)alkyl group, -OH and -F.

Embodiment 117. The compound or pharmaceutically acceptable salt or hydrate or pharmaceutically acceptable solvate of Embodiment 116, wherein X⁶ is -CH(R^{6d})-, and wherein R^{6d} is selected from the group consisting of -H, a methyl group, -OH and -F, preferably R^{6d} is -F.

Embodiment 118. The compound or pharmaceutically acceptable salt or hydrate or pharmaceutically acceptable solvate of any one of Embodiments 114, 115, 116 and 117 preferable Embodiment 117, wherein R^{6a} is -H.

Embodiment 119. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, preferably any one of Embodiments 1,2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, wherein Xaa6 is a residue of an L-α-amino acid of formula (VIIIb) 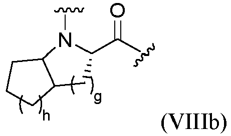
g = 0, 1 or 2, and
h= 1, 2 or 3.

Embodiment 120. The compound or pharmaceutically acceptable salt or hydrate or pharmaceutically acceptable solvate of Embodiment 119, wherein g = 1.

Embodiment 121. The compound or pharmaceutically acceptable salt or hydrate or pharmaceutically acceptable solvate of any one of Embodiments 119 and 120, wherein h = 1 or 2.

Embodiment 122. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, wherein Xaa6 is a residue of an L-α-amino acid of formula (VIIIc) 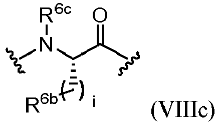 wherein
i = 1 or 2,
R^{6b} is selected from the group consisting of a (C₂-C₆)alkyl group, an aryl group, an aryl group substituted with 1 or 2 substituents, a (C₅-C₆)heteroaryl group, a (C₅-C₆)heteroaryl group substituted with 1 or 2 substituents, a (C₅-C₆)carbocycle, and a (C₅-C₆)carbocycle substituted with 1 or 2 substituents, wherein, preferably, each substituent is individually and independently selected from the group consisting of a halogen atom, a (C₁-C₆)alkyl group, -CN, -OH, and an -O(C₁-C₆)alkyl group, and
R^{6c} is selected from the group consisting of -H and a (C₁-C₃)alkyl group.

Embodiment 123. The compound or pharmaceutically acceptable salt or hydrate or pharmaceutically acceptable solvate of Embodiment 122, wherein i= 1.

Embodiment 124. The compound or pharmaceutically acceptable salt or hydrate or pharmaceutically acceptable solvate of any one of Embodiments 122 and 123, wherein R^{6c} is selected from the group consisting of -H and a methyl group.

Embodiment 125. The compound or pharmaceutically acceptable salt or hydrate or pharmaceutically acceptable solvate of Embodiment 124, wherein R^{6c} is -H.

Embodiment 126. The compound or pharmaceutically acceptable salt or hydrate or pharmaceutically acceptable solvate of any one of Embodiments 122, 123, 124 and 125, wherein R^{6b} is selected from the group consisting of a (C₂-C₅)alkyl group, a phenyl group, a 2-thienyl group and a (C₅-C₆)carbocycle, wherein each and any of these groups is optionally substituted with one or two substituents, wherein, preferably, each substituent is individually and independently selected from the group consisting of a halogen atom, a methyl group, -CN, -OH, and an -OCH₃ group.

Embodiment 127. The compound or pharmaceutically acceptable salt or hydrate or pharmaceutically acceptable solvate of Embodiment 126, wherein R^{6b} is selected from the group consisting of a (C₂-C₄)alkyl group and a (C₅-C₆)carbocycle, wherein each and any of these groups is optionally substituted with one or two substituents, wherein, preferably, each substituent is individually and independently selected from the group consisting of a halogen atom and a methyl group.

Embodiment 128. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126 and 127, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126 and 127, wherein Xaa7 is a residue of an α-amino acid of formula (IXa), (IXb), (IXc), (IXd), or (IXe), each optionally comprising a Z group, 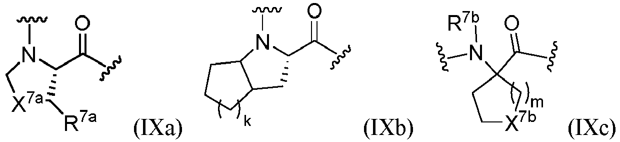 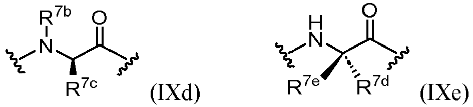 wherein
in formula (IXa)
   R^{7a} is selected from the group consisting of -H, -OH, -F, -NH₂, and a (C₁-C₃)alkyl group,
   X^{7a} is absent or selected from the group consisting of -CH(R^{7f})-, -S-, -O-, and - NH-,
      wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group,
in formula (IXb)
   k = 1 or 2,
in formula (IXc)
   m = 1, 2 or 3,
   R^{7b} is selected from the group consisting of -H, and a (C₁-C₂)alkyl group,
   X^{7b} is absent or selected from the group consisting of -N(R^{7g})-, -O-, -S-, -SO-, - SOz- and -CH₂-,
      wherein R^{7g} is selected from the group consisting of -H, -Ac, a (C₁-C₃)alkyl group and a Z group,
in formula (IXd)
   R^{7b} is selected from the group consisting of -H, and a (C₁-C₂)alkyl group,
   R^{7c} is selected from the group consisting of -H, and -(CH₂)ₙR^{7h},
      wherein n = 1, 2, or 3, preferably n = 1, and wherein one of the one, two, or three -CH₂-groups of -(CH₂)ₙR^{7h} which is different from the terminal groups is optionally replaced by -O-, -S-, -SO-, or -SOz-,
      wherein R^{7h} is selected from the group consisting of -H, -OH, - NR⁷ⁱR^{7k}, -N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein each substituent is preferably selected from the group consisting of a methyl group, a halogen atom, -NH₂ and -OH, wherein R⁷ⁱ and R^{7k} are each and independently selected from the group consisting of -H and a methyl group,
in formula (IXe)
   R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l}, preferably R^{7d} is -(CH₂)ₒR^{7l},
   R^{7l} is selected from the group consisting of a polar, a positively charged, a negatively charged, a zwitterionic and a hydrophobic group, wherein R^{7l} optionally comprises a Z group,
   o = 1, 2, 3, 4 or 5, and wherein one of the one, two, three, four or five -CH₂- groups linking R^{7l} to the α-C atom of Xaa7 in formula (IXe) are optionally substituted by a methyl group, and/or wherein one of said -CH₂- groups which is different from the terminal groups is optionally replaced by -O-, -S-, -SO-, or -SOz-,
   preferably R^{7l} is selected from the group consisting of -H, -OH, -N(R^{7m})(R⁷ⁿ), - N(R^{7o})(R^{7p})(R^{7q})⁺, -COOH, a halogen atom, -CN, -N₃, -NO₂, - N(R^{7m})-CN(R⁷ⁿ)-N(R^{7r})(R^{7s}), -SO₂N(R^{7m})(R^{7u}), -CON(R^{7m})(R^{7u}), -NH-CON(R^{7m})(R^{7u}), -SO₃H, -R^{7t}, -NH-SO₂-R^{7t}, and -NH-CO-R^{7t},
      wherein R^{7m}, R^{7r} and R^{7s} are each and independently selected from the group consisting of -H and a (C₁-C₂)alkyl group,
      wherein R^{7o} and R^{7p} are each and independently selected from the group consisting of a (C₁-C₃)alkyl group,
   wherein R⁷ⁿ is selected from the group consisting of -H, -Ac, a (C₁-C₃)alkyl group and a Z group,
      wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of -OH, -COOH, and -SO₃H, preferably R^{7q} is selected from the group consisting of a methyl group and a -(CH₂)₃SO₃H group,
      wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH, preferably R^{7t} is a (C₁-C₄)alkyl group which is optionally substituted by a substituent preferably selected from the group consisting of -CONH₂, -COOH, a halogen atom, - NH-CNH-NH₂, -NH₂ and -OH, more preferably R^{7t} is a (C₁-C₃)alkyl group,
   wherein R^{7u} is selected from the group consisting of -H and R^{7t}, preferably R^{7u} is selected from the group consisting of -H and a methyl group, most preferably R^{7u} is -H,
   R^{7e} is selected from the group consisting of -H, and a (C₁-C₃)alkyl group, wherein said (C₁-C₃)alkyl group is optionally substituted by a substituent preferably selected from the group consisting of -F, -Cl and -OH.

Embodiment 129. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, wherein Xaa7 is a residue of an α-amino acid of formula (IXa) optionally comprising a Z group 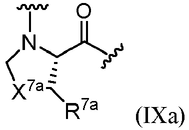 wherein
R^{7a} is selected from the group consisting of -H, -OH, -F, -NH₂ and a (C₁-C₃)alkyl group,
X^{7a} is absent or selected from the group consisting of -CH(R^{7f})-, -S-, -O-, -NH-,
   wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group.

Embodiment 130. The compound or pharmaceutically acceptable salt or hydrate or pharmaceutically acceptable solvate of Embodiment 129, wherein R^{7a} is -H.

Embodiment 131. The compound or pharmaceutically acceptable salt or hydrate or pharmaceutically acceptable solvate of any one of Embodiments 129 and 130, wherein X^{7a} is -CH(R^{7f})- or -S-, and wherein R^{7f} is selected from the group consisting of -H, -OH, -F, -NH(R^{7g}) and a (C₁-C₆)alkyl group, and wherein R^{7g} is selected from the group consisting of -H, -Ac, a (C₁-C₃)alkyl group and a Z group.

Embodiment 132. The compound or pharmaceutically acceptable salt or hydrate or pharmaceutically acceptable solvate of Embodiment 131, wherein X^{7a} is -CH(R^{7f})-, and wherein R^{7f} is selected from the group consisting of -H, -OH, -F, -NH(R^{7g}) and a methyl group, and wherein R^{7g} is selected from the group consisting of -H, a methyl group and a Z group, preferably R^{7f} is -OH.

Embodiment 133. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, wherein Xaa7 is a residue of an α-amino acid of formula (IXd), 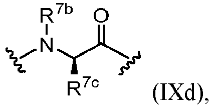 wherein
R^{7b} is selected from the group consisting of -H, and a (C₁-C₂)alkyl group,
R^{7c} is selected from the group consisting of -H, and -(CH₂)ₙR^{7h},
   wherein n = 1, 2, or 3, and wherein one of the one, two, or three -CH₂- groups of -(CH₂)ₙR^{7h} which is different from the terminal groups is optionally replaced by -O-, -S-, -SO-, or -SOz-,
   wherein R^{7h} is selected from the group consisting of -H, -OH, -NR⁷ⁱR^{7k}, - N(CH₃)₃⁺, -NH-CNH-NH₂, -CONH₂, -NH-CO-NHz and a (C₁-C₃)alkyl group, an aryl group, a substituted aryl group substituted by one substituent, a heteroaryl group, a heteroaryl group substituted by one substituent, wherein each substituent is preferably selected from the group consisting of a methyl group, a halogen atom, -NH₂ and -OH, wherein R⁷ⁱ and R^{7k} are each and independently selected from the group consisting of -H and a methyl group.

Embodiment 134. The compound or pharmaceutically acceptable salt or hydrate or pharmaceutically acceptable solvate of Embodiment 133, wherein R^{7b} is -H.

Embodiment 135. The compound or pharmaceutically acceptable salt or hydrate or pharmaceutically acceptable solvate of any one of Embodiments 133 and 134, wherein n = 1.

Embodiment 136. The compound or pharmaceutically acceptable salt or hydrate or pharmaceutically acceptable solvate of any one of Embodiments 133, 134 and 135, wherein R^{7h} is selected from the group consisting of -H, -OH, -NR⁷ⁱR^{7k}, -N(CH₃)₃⁺, -NH-CNH-NH₂, a phenyl group and a 2-thienyl group, wherein the phenyl group and the 2-thienyl group is optionally substituted by one substituent, wherein each substituent is preferably selected from the group consisting of a methyl group, a halogen atom, - NH₂ and -OH, wherein R⁷ⁱ and R^{7k} are each and independently selected from the group consisting of -H and a methyl group.

Embodiment 137. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, wherein Xaa7 is a residue of an α-amino acid of formula (IXe) optionally comprising a Z group 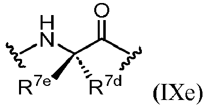 wherein
R^{7d} is selected from the group consisting of a (C₁-C₆)alkyl group, a (C₃-C₇)carbocycle, (C₃-C₇)heterocycle, an aryl group, a heteroaryl group and - (CH₂)ₒR^{7l},
   wherein R^{7l} is selected from the group consisting of a polar, a positively charged, a negatively charged, a zwitterionic and a hydrophobic group, wherein R^{7l} optionally comprises a Z group,
   wherein o = 1, 2, 3, 4 or 5, and wherein one of the one, two, three, four or five -CH₂- groups linking R^{7l} to the α-C atom of Xaa7 in formula (IXe) are optionally substituted by a methyl group, and/or wherein one of said -CH₂- groups which is different from the terminal groups is optionally replaced by - O-, -S-, -SO-, or -SOz-,
R^{7e} is selected from the group consisting of -H, and a (C₁-C₃)alkyl group, wherein said (C₁-C₃)alkyl group is optionally substituted by a substituent preferably selected from the group consisting of -F, -Cl and -OH.

Embodiment 138. The compound or pharmaceutically acceptable salt or hydrate or pharmaceutically acceptable solvate of Embodiment 137, wherein R^{7e} is selected from the group consisting of -H, and a methyl group.

Embodiment 139. The compound or pharmaceutically acceptable salt or hydrate or pharmaceutically acceptable solvate of Embodiment 138, wherein R^{7e} is -H.

Embodiment 140. The compound or pharmaceutically acceptable salt or hydrate or pharmaceutically acceptable solvate of any one of Embodiments 137, 138 and 139, wherein R^{7d} is -(CH₂)ₒR^{7l}.

Embodiment 141. The compound or pharmaceutically acceptable salt or hydrate or pharmaceutically acceptable solvate of any one of Embodiments 137, 138, 139 and 140, wherein o = 1, 2, 3 or 4.

Embodiment 142. The compound or pharmaceutically acceptable salt or hydrate or pharmaceutically acceptable solvate of Embodiment 141, wherein o = 1 or 2.

Embodiment 143. The compound or pharmaceutically acceptable salt or hydrate or pharmaceutically acceptable solvate of any one of Embodiments 137, 138, 139, 140, 141 and 142, wherein
R^{7l} is selected from the group consisting of -H, -OH, -N(R^{7m})(R⁷ⁿ), -N(R^{7o})(R^{7p})(R^{7q})⁺, -COOH, a halogen atom, -CN, -N₃, -NO₂, -N(R^{7m})-CN(R⁷ⁿ)-N(R^{7r})(R^{7s}), -SO₂N(R^{7m})(R^{7u}), -CON(R^{7m})(R^{7u}), -NH-CON(R^{7m})(R^{7u}), -SO₃H, -R^{7t}, -NH-SO₂-R^{7t}, and -NH-CO-R^{7t},
   wherein R^{7m}, R^{7r} and R^{7s} are each and independently selected from the group consisting of -H and a (C₁-C₂)alkyl group,
   wherein R^{7o} and R^{7p} are each and independently selected from the group consisting of a (C₁-C₃)alkyl group,
wherein R⁷ⁿ is selected from the group consisting of -H, -Ac, a (C₁-C₃)alkyl group and a Z group,
   wherein R^{7q} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of -OH, -COOH, and -SO₃H,
   wherein R^{7t} is selected from the group consisting of a (C₁-C₆)alkyl group, a (C₃-C₇)carbocycle, a (C₃-C₇)heterocycle, a phenyl group and a (C₅-C₆)heteroaryl group, and R^{7t} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH,
wherein R^{7u} is selected from the group consisting of -H and R^{7t}.

Embodiment 144. The compound or pharmaceutically acceptable salt or hydrate or pharmaceutically acceptable solvate of any one of Embodiments 137, 138, 139, 140, 141, 142 and 143, wherein R^{7m}, R^{7r} and R^{7s} are each -H.

Embodiment 145. The compound or pharmaceutically acceptable salt or hydrate or pharmaceutically acceptable solvate of any one of Embodiments 137, 138, 139, 140, 141, 142 and 143, wherein R^{7o} and R^{7p} are each a methyl group.

Embodiment 146. The compound or pharmaceutically acceptable salt or hydrate or pharmaceutically acceptable solvate of any one of Embodiments 137, 138, 139, 140, 141, 142, 143 and 144, wherein R⁷ⁿ is selected from the group consisting of -H, a methyl group and a Z group.

Embodiment 147. The compound or pharmaceutically acceptable salt or hydrate or pharmaceutically acceptable solvate of any one of Embodiments 137, 138, 139, 140, 141, 142, 143 and 145, wherein R^{7q} is selected from the group consisting of a methyl group and a -(CH₂)₃SO₃H group.

Embodiment 148. The compound or pharmaceutically acceptable salt or hydrate or pharmaceutically acceptable solvate of any one of Embodiments 137, 138, 139, 140, 141, 142 and 143, wherein R^{7t} is a (C₁-C₄)alkyl group which is optionally substituted by a substituent preferably selected from the group consisting of -CONH₂, -COOH, a halogen atom, -NH-CNH-NH₂, -NH₂ and -OH.

Embodiment 149. The compound or pharmaceutically acceptable salt or hydrate or pharmaceutically acceptable solvate of any one of Embodiments 143 and 148, wherein R^{7t} is a (C₁-C₃)alkyl group.

Embodiment 150. The compound or pharmaceutically acceptable salt or hydrate or pharmaceutically acceptable solvate of any one of Embodiments 137, 138, 139, 140, 141, 142, 143, 148 and 149, wherein R^{7u} is selected from the group consisting of -H and a methyl group.

Embodiment 151. The compound or pharmaceutically acceptable salt or hydrate or pharmaceutically acceptable solvate of Embodiment 150, wherein R^{7u} is -H.

Embodiment 152. The compound or pharmaceutically acceptable salt or hydrate or pharmaceutically acceptable solvate of any one of Embodimentd 137, 138, 139, 140, 141, 142 and 143, wherein R^{7l} is selected from the group consisting of -H, -OH, -NH₂, -NH(R⁷ⁿ), -N(CH₃)₂, -N(CH₃)₃⁺, -NH-CNH-NH₂, -CONH₂, -NH-CO-NHz and a (C₁-C₃)alkyl group, wherein R⁷ⁿ is selected from the group consisting of a methyl group and a Z group.

Embodiment 153. The compound or pharmaceutically acceptable salt or hydrate or pharmaceutically acceptable solvate of any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, wherein Xaa7 is a residue of an α-amino acid of formula (IXb) 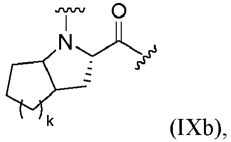 wherein
k = 1 or 2.

Embodiment 154. The compound or pharmaceutically acceptable salt or hydrate or pharmaceutically acceptable solvate of any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, wherein Xaa7 is a residue of an α-amino acid of formula (IXc) optionally comprising a Z group 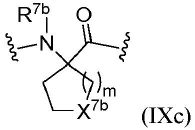 wherein
m = 1, 2 or 3,
R^{7b} is selected from the group consisting of -H, and a (C₁-C₂)alkyl group,
X^{7b} is absent or selected from the group consisting of -N(R^{7g})-, -O-, -S-, -SO-, - SOz- and -CH₂-,
   wherein R^{7g} is selected from the group consisting of -H, -Ac, a (C₁-C₃)alkyl group and a Z group.

Embodiment 155. The compound or pharmaceutically acceptable salt or hydrate or pharmaceutically acceptable solvate of Embodiment 154, wherein m = 2.

Embodiment 156. The compound or pharmaceutically acceptable salt or hydrate or pharmaceutically acceptable solvate of any one of Embodiments 154 and 155, wherein R^{7b} is -H.

Embodiment 157. The compound or pharmaceutically acceptable salt or hydrate or pharmaceutically acceptable solvate of any one of Embodiments 154, 155 and 156, wherein X^{7b} is selected from the group consisting of -N(R^{7g})-, -O- and -CH₂-, wherein R^{7g} is selected from the group consisting of -H, a methyl group and a Z group.

Embodiment 158. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157, wherein Xaa8 is a residue of an α-amino acid of formula (Xa) or (Xb) 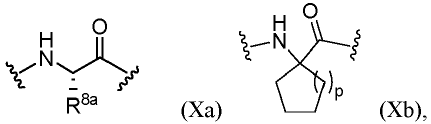 wherein
in formula (Xa)
   R^{8a} is selected from the group consisting of a (C₄-C₈)carbocycle, an aryl group, a heteroaryl group and a -CH(R^{8b})(R^{8c}) group,
   wherein R^{8b} is selected from the group consisting of -H and a (C₁-C₃)alkyl group,
   wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
   wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
   wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
in formula (Xb)
   p = 1, 2, or 3,
   a -CH₂- group in the ring of formula (Xb) is optionally replaced by one atom selected from the group consisting of -O- and -S-, and one or more H atoms of the ring -CH₂- groups are optionally substituted by a -F or -Cl atom.

Embodiment 159. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 and 158, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 and 158, wherein Xaa8 is a residue of an L-α-amino acid of formula (Xa) 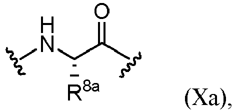 wherein
R^{8a} is selected from the group consisting of a (C₄-C₈)carbocycle, an aryl group, a heteroaryl group and a -CH(R^{8b})(R^{8c}) group,
wherein R^{8b} is selected from the group consisting of -H and a (C₁-C₃)alkyl group,
wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group.

Embodiment 160. The compound or pharmaceutically acceptable salt or hydrate or pharmaceutically acceptable solvate of Embodiment 159, wherein R^{8a} is selected from the group consisting of a (C₄-C₆)carbocycle, a phenyl group, a 2-thienyl group and a -CH(R^{8b})(R^{8c}) group,
wherein R^{8b} is selected from the group consisting of -H and a (C₁-C₃)alkyl group,
wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
wherein the -CH₂- groups of the optional linker or any -CH₂- groups of the carbocycle groups of R^{8a} or of the (C₁-C₆)alkyl group of R^{8c} are optionally replaced by one atom selected from the group consisting of -O- and -S-, and
wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group.

Embodiment 161. The compound or pharmaceutically acceptable salt or hydrate or pharmaceutically acceptable solvate of any one of Embodiments 159 and 160, wherein R^{8b} is selected from the group consisting of -H and a methyl group, preferably R^{8b} is a methyl group.

Embodiment 162. The compound or pharmaceutically acceptable salt or hydrate or pharmaceutically acceptable solvate of any one of Embodiments 159, 160 and 161, wherein R^{8c} is selected from the group consisting of a (C₁-C₅)alkyl group, a (C₃-C₆)carbocycle, a phenyl group and a 2-thienyl group, and
wherein R^{8c} is optionally linked to the β-carbon atom of Xaa8 by a linker comprising one or or two -CH₂- groups,
wherein the -CH₂- groups of the optional linker or any -CH₂- groups of the (C₃-C₆)carbocycle group or the (C₁-C₅)alkyl group of R^{8c} are optionally replaced by one atom selected from the group consisting of -O- and -S-, and
wherein one, two or three hydrogen atoms of the R^{8b} or the R^{8c} group, are optionally replaced by atoms or groups each and independently selected from the group consisting of -F, -Cl or a methyl group.

Embodiment 163. The compound or pharmaceutically acceptable salt or hydrate or pharmaceutically acceptable solvate of any one of Embodiments 159, 160, 161 and 162, wherein R^{8c} is selected from the group consisting of a (C₁-C₅)alkyl group, a (C₃-C₆)carbocycle, a phenyl group and a 2-thienyl group, and wherein R^{8c} is directly bound to the β-carbon atom of Xaa8 without a linker,
wherein one, two or three hydrogen atoms of the R^{8b} or the R^{8c} group, are optionally replaced by atoms or groups each and independently selected from the group consisting of -F, -Cl or a methyl group.

Embodiment 164. The compound or pharmaceutically acceptable salt or hydrate or pharmaceutically acceptable solvate of any one of Embodiments 161 and 163, wherein R^{8c} is selected from the group consisting of a (C₂-C₄)alkyl group, a (C₃-C₆)carbocycle, a phenyl group and a 2-thienyl group, preferably R^{8c} is an ethyl group.

Embodiment 165. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 and 158, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 and 158, wherein Xaa8 is a residue of an α-amino acid of formula (Xb) 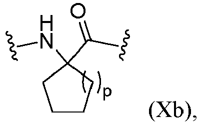 wherein
p = 1, 2, or 3,
a -CH₂- group in the ring of formula (Xb) is optionally replaced by one atom selected from the group consisting of -O- and -S-, and one or more -H atoms of the ring -CH₂- groups are optionally substituted by -F or -Cl.

Embodiment 166. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164 and 165, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164 and 165, wherein Xaa9 is a residue of an α-amino acid of formula (XI) 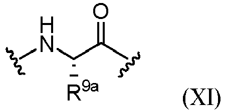 wherein
R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂-group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl.

Embodiment 167. The compound or pharmaceutically acceptable salt or hydrate or pharmaceutically acceptable solvate of Embodiment 166, wherein R^{9a} is selected from the group consisting of a (C₂-C₃)alkyl group, a cyclobutyl group and -CH₂R^{9b}, wherein R^{9b} is selected from the group consisting of a (C₃-C₄)carbocycle and a (C₁-C₃)alkyl group, wherein the -CH₂- group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, preferably R^{9a} is a propyl group.

Embodiment 168. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166 and 167, preferably any one of Embodiments 1, 2, 3, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166 and 167, wherein Xaa10 is a residue of formula (XII) 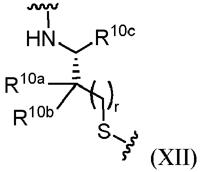 wherein
R^{10a} and R^{10b} are each and independently selected from the group consisting of -H and a methyl group,
r = 1 or 2,
R^{10c} is selected from the group consisting of -H, -CO-Cterm, -CONHz, -CH₂(OH), - CON(R^{10d})(R^{10e}), wherein Cterm comprises a Z group,
wherein
   R^{10d} and R^{10e} are each and independently selected from the group consisting of -H and a methyl group,
   and the sulfur atom of Xaa10 is covalently attached to the sulfur atom of Xaa1.

Embodiment 169. The compound or pharmaceutically acceptable salt or hydrate or pharmaceutically acceptable solvate of Embodiment 168, wherein R^{10a} and R^{10b} are each -H.

Embodiment 170. The compound or pharmaceutically acceptable salt or hydrate or pharmaceutically acceptable solvate of any one of Embodiments 168 and 169, preferably Embodiment 169, wherein r = 1.

Embodiment 171. The compound or pharmaceutically acceptable salt or hydrate or pharmaceutically acceptable solvate of any one of Embodiments 168, 169 and 170, wherein R^{10c} is selected from the group consisting of -CO-Cterm, -CONH₂ and -CHz(OH), and wherein Cterm is a Z group.

Embodiment 172. The compound or pharmaceutically acceptable salt or hydrate or pharmaceutically acceptable solvate of any one of Embodiments 168, 169, 170 and 171, wherein the sulfur atom of Xaa10 is covalently attached as disulfide to the sulfur atom of Xaa1.

Embodiment 173. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171 and 172, preferably of any one of Embodiments 1, 2, 3, 4, 5, 6, 7, 8, 9, 10, 11, 12, 13, 14, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171 and 172, wherein the blocking group Abl is selected from the group consisting of Hex, Pen, But, iHex, Butoc, nBuCAyl, PrCAyl, Oct, EtOPr, Bz, Pha, Chex, Cp and PentylSOz.

Embodiment 174. The compound or pharmaceutically acceptable salt or hydrate or pharmaceutically acceptable solvate of one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172 and 173, preferably of any one of Embodiment 1, 2, 3, 4, 5, 6, 7, 8, 9, 10, 11, 12, 13, 14, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172 and 173, wherein the blocking group Abl is selected from the group consisting of Hex, Pen, iHex, Butoc and nBuCAyl, preferably the blocking group Abl is Butoc.

Embodiment 175. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171 and 172, preferably of any one of Embodiments 1, 2, 3, 4, 5, 6, 7, 8, 9, 10, 11, 12, 13, 14, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171 and 172, wherein Xaa0 is a residue of an amino acid selected from the group consisting of Met, Leu, leu, Aml, Egz, Nva, nva, Nle, nle, Nmb, Phg and Cha.

Embodiment 176. The compound or pharmaceutically acceptable salt or hydrate or pharmaceutically acceptable solvate of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, and 175, preferably of any one of Embodiment 1 to 14, 53 to 172 and 175, wherein Xaa0 is a residue of an amino acid selected from the group consisting of Phg, Leu, Nva, Aml, Egz, Nle and Cha.

Embodiment 177. The compound or pharmaceutically acceptable salt or hydrate or pharmaceutically acceptable solvate of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175 and 176, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175 and 176, wherein Xaa1 is a residue of an amino acid selected from the group consisting of Cys and Pen.

Embodiment 178. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176 and 177, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176 and 177, wherein Xaa1 is a residue of Cys.

Embodiment 179. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177 and 178, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177 and 178, wherein Xaa2 is a residue of an amino acid selected from the group consisting of Arg, Arg(Me), Asp, Glu, Gln, Lys, Lys(Me), Lys(Z), Cit, Ala, Aib, Har, Asn, Thr, Glu(Z), Om(mPEG12), KMe3, Orn, Om(Me), Om(Z), Ser, Thp, Ape, Apc(Z), Gly, Pro, Tyr and Leu, wherein Z is a Z group.

Embodiment 180. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178 and 179, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178 and 179, wherein Xaa2 is a residue of an amino acid selected from the group consisting of Arg, Arg(Me), Asp, Glu, Gln, Om(DOTA), Lys, Lys(Me), Lys(DOTA), Cit, Ala, Aib, Har, Asn, Thr, Glu(AGLU), Om(mPEG12), KMe3, Orn, Om(Me), Om(Tris-Nta(Tris)), Ser, Thp, Ape, Apc(DOTA), Gly, Pro, Tyr and Leu.

Embodiment 181. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179 and 180, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179 and 180, wherein Xaa2 is a residue of an amino acid selected from the group consisting of Thr and Cit, preferably Xaa2 is a residue of Thr.

Embodiment 182. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180 and 181, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180 and 181, wherein Xaa3 is a residue of an amino acid selected from the group consisting of Met, Arg, Arg(Me), Dap, Dab, Leu, Tyr, Gln, Glu, Glu(Z), Lys(Ac), Lys(Z), Cit, Ala, Nle, Asp, Har, Asn, Thr, Apc(Z), Om(Z), Dab(Z), Dap(Z), Lys(AF488-Ttds), Lys, Lys(Me), KMe3, Orn, Om(Me), Ser and Gly, wherein Z is a Z group.

Embodiment 183. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181 and 182, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181 and 182, wherein Xaa3 is a residue of an amino acid selected from the group consisting of Met, Arg, Arg(Me), Dap, Dab, Leu, Tyr, Gln, Glu, Lys(Ac), Lys(DOTA), Cit, Ala, Nle, Asp, Har, Asn, Thr, Apc(DOTA), Om(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Om(Me), Ser and Gly.

Embodiment 184. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182 and 183, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182 and 183, wherein Xaa3 is a residue of an amino acid selected from the group consisting of Gln, Lys(DOTA), Om(DOTA), Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw), preferably Xaa3 is a residue of an amino acid selected from the group consisting of Om(DOTA) and Lys(DOTA).

Embodiment 185. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183 and 184, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183 and 184, wherein Xaa4 is a residue of an amino acid selected from the group consisting of Tyr, Dopa, Phe, Aph, His, Mpa, My, Mcy, Guf, Ppa, Nif, Pcnf, Cha, Trp, Ay3, 2Ni and Ocf.

Embodiment 186. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184 and 185, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184 and 185, wherein Xaa4 is a residue of an amino acid selected from the group consisting of Tyr, Dopa, Aph, Mpa, Nif, Ocf, My and Pcnf, preferably Xaa4 is a residue of an amino acid selected from the group consisting of Pcnf and Dopa.

Embodiment 187. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185 and 186, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185 and 186, wherein Xaa5 is a residue of an amino acid selected from the group consisting of Phe, Thi, Pcf, Ocf, Egm, Mcf, 1Ni, Pnf, Mpa, Bta and Trp.

Embodiment 188. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, and 187, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, and 187, wherein Xaa5 is a residue of an amino acid selected from the group consisting of Phe, Thi, Pcf, Ocf, Egm, Mcf, 1Ni, Pnf and Mpa.

Embodiment 189. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 and 188, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 and 188, wherein Xaa5 is a residue of an amino acid selected from the group consisting of Phe, Thi, Pcf, Ocf and Egm, preferably Xaa5 is a residue of Phe.

Embodiment 190. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188 and 189, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188 and 189, wherein Xaa6 is a residue of an amino acid selected from the group consisting of Pro, 4Cfp, Hyp, Eaz, Oic, Nmb, Leu, Cha, Chy, 4Tfp, Pip, Oxa, Aze and Phe.

Embodiment 191. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 and 190, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 and 190, wherein Xaa6 is a residue of an amino acid selected from the group consisting of Pro, 4Cfp and Hyp, preferably Xaa6 is a residue of 4Cfp.

Embodiment 192. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190 and 191, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190 and 191, wherein Xaa7 is a residue of an amino acid selected from the group consisting of Pro, 4Cfp, Hyp, Tap, Ala, Aib, Asp, Ser, Tyr, ser, dap, tyr, Leu, 4Tfp, Chy, 4Ap, ala, Arg, Apc(Z), Tap(Z), Gly, nma, Aze, Oic, Oxa, Lys(Z) and Nmg, wherein Z is a Z group, preferably Z is DOTA

Embodiment 193. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191 and 192, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191 and 192, wherein Xaa7 is a residue of an amino acid selected from the group consisting of Pro, 4Cfp, Hyp, Tap, Ala, Aib, 4Tfp, Chy, 4Ap, ala, Arg, Tap(Z), Lys(Z), Ser, ser, Leu, tyr and nma, wherein Z is a Z group, preferably Z is DOTA

Embodiment 194. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192 and 193, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192 and 193, wherein Xaa7 is a residue of an amino acid selected from the group consisting of Pro, Aib, Hyp, ala, nma, 4Tfp, Lys(DOTA) and Tap, preferably Xaa7 is a residue of Hyp.

Embodiment 195. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193 and 194, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 166, 167, 168, 169, 170, 171, 172, 173, 174, 175, 176, 177, 178, 179, 180, 181, 182, 183, 184, 185, 186, 187, 188, 189, 190, 191, 192, 193 and 194, wherein Xaa8 is a residue of an amino acid selected from the group consisting of Ile, Chg, Cha, Cpg, Cbg, Npg, Phe, Pcf, Egz, Hfe, Phg, Leu, Nle and Nva.

Embodiment 196. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194 and 195, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 166, 167, 168, 169, 170, 171, 172, 173, 174, 175, 176, 177, 178, 179, 180, 181, 182, 183, 184, 185, 186, 187, 188, 189, 190, 191, 192, 193, 194 and 195, wherein Xaa8 is a residue of an amino acid selected from the group consisting of Ile, Chg, Cha, Cpg, Phg, Pcf, Cbg and Npg.

Embodiment 197. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195 and 196, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195 and 196, wherein Xaa8 is a residue of an amino acid selected from the group consisting of Ile, Chg, Phg and Cha, preferably Xaa8 is a residue of Ile.

Embodiment 198. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196 and 197, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 168, 169, 170, 171, 172, 173, 174, 175, 176, 177, 178, 179, 180, 181, 182, 183, 184, 185, 186, 187, 188, 189, 190, 191, 192, 193, 194, 195, 196 and 197, wherein Xaa9 is a residue of an amino acid selected from the group consisting of Leu, Nva, Cpra, Abu, Ser(Me), Cbg, Npg, Ala, Nle, Ile and Val.

Embodiment 199. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 and 198, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 and 198, wherein Xaa9 is a residue of an amino acid selected from the group consisting of Leu, Nva, Cpra, Abu, Ser(Me) and Cbg.

Embodiment 200. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198 and 199, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198 and 199, wherein Xaa9 is a residue of an amino acid selected from the group consisting of Leu, Nva and Cpra, preferably Xaa9 is a residue of Nva.

Embodiment 201. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199 and 200, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 173, 174, 175, 176, 177, 178, 179, 180, 181, 182, 183, 184, 185, 186, 187, 188, 189, 190, 191, 192, 193, 194, 195, 196, 197, 198, 199 and 200, wherein Xaa10 is a residue of an amino acid selected from the group consisting of Cys-NH₂, Cysol-OH, AET, Hcy-NH₂, Pen-NH₂ and Cys--O2Oc-Lys(Z)-NH2, wherein Z is a Z group, preferably Z is DOTA.

Embodiment 202. The compound or pharmaceutically acceptable salt or hydrate or pharmaceutically acceptable solvate of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200 and 201, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 173, 174, 175, 176, 177, 178, 179, 180, 181, 182, 183, 184, 185, 186, 187, 188, 189, 190, 191, 192, 193, 194, 195, 196, 197, 198, 199, 200 and 201, wherein Xaa10 is a residue of an amino acid selected from the group consisting of Cys-NH₂ and Cysol-OH.

Embodiment 203. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201 and 202, preferably of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 173, 174, 175, 176, 177, 178, 179, 180, 181, 182, 183, 184, 185, 186, 187, 188, 189, 190, 191, 192, 193, 194, 195, 196, 197, 198, 199, 200, 201 and 202, wherein Xaa10 is a residue of Cys-NH₂.

Embodiment 204. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202 and 203, preferably any one of Embodiments 17,174, 177, 178, 179, 180, 181, 182, 183, 184, 185, 186, 187, 188, 189, 190, 191, 192, 193, 194, 195, 196, 197, 198, 199, 200, 201, 202 and 203, wherein
the blocking group Abl is selected from the group consisting of Butoc, Hex, Pen, But, iHex, nBuCAyl, PrCAyl, Oct, EtOPr, Bz, Pha, Chex, Cp and PentylSO₂,
Xaa1 is a residue of an amino acid selected from the group consisting of Cys and Pen,
Xaa2 is a residue of an amino acid selected from the group consisting of is a residue of an amino acid selected from the group consisting of Thr, Arg, Arg(Me), Asp, Glu, Gln, Lys, Lys(Me), Lys(Z), Cit, Ala, Aib, Har, Asn, Glu(Z), Om(mPEG12), KMe3, Orn, Om(Me), Om(Z), Ser, Thp, Ape, Apc(Z), Gly, Pro, Tyr and Leu,
Xaa3 is a residue of an amino acid selected from the group consisting of Om(Z), Lys(Z), Met, Arg, Arg(Me), Dap, Dab, Leu, Tyr, Gln, Glu, Glu(Z), Lys(Ac), Cit, Ala, Nle, Asp, Har, Asn, Thr, Apc(Z), Dab(Z), Dap(Z), Lys(AF488-Ttds), Lys, Lys(Me), KMe3, Orn, Om(Me), Ser and Gly,
Xaa4 is a residue of an amino acid selected from the group consisting of Pcnf, Dopa, Tyr, Phe, Aph, His, Mpa, My, Mcy, Guf, Ppa, Nif, Cha, Trp, Ay3, 2Ni and Ocf,
Xaa5 is a residue of an amino acid selected from the group consisting of Phe, Thi, Pcf, Ocf, Egm, Mcf, 1Ni, Pnf, Mpa, Bta and Trp,
Xaa6 is a residue of an amino acid selected from the group consisting of 4Cfp, Pro, Hyp, Eaz, Nmb, Oic, Leu, Cha, Chy, 4Tfp, Pip, Oxa, Aze and Phe,
Xaa7 is a residue of an amino acid selected from the group consisting of Hyp, Pro, 4Cfp, Tap, Ala, Aib, Asp, Ser, Tyr, ser, dap, tyr, Leu, 4Tfp, Chy, 4Ap, ala, Arg, Apc(Z), Tap(Z), Gly, nma, Aze, Oic, Oxa, Lys(Z) and Nmg,
Xaa8 is a residue of an amino acid selected from the group consisting of Ile, Chg, Cha, Cpg, Cbg, Npg, Phe, Pcf, Egz, Hfe, Phg, Leu, Nle and Nva,
Xaa9 is a residue of an amino acid selected from the group consisting of Nva, Leu, Cpra, Abu, Ser(Me), Cbg, Npg, Ala, Nle, Ile and Val, and
Xaa10 is a residue of an amino acid selected from the group consisting of Cys-NH₂, Cysol-OH, AET, Hcy-NH₂, Pen-NH₂ and Cys-O2Oc-Lys(Z)-NH₂,
wherein Z is a Z group.

Embodiment 205. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203 and 204, preferably any one of Embodiments 173, 174, 177, 178, 179, 180, 181, 182, 183, 184, 185, 186, 187, 188, 189, 190, 191, 192, 193, 194, 195, 196, 197, 198, 199, 200, 201, 202, 203 and 204, wherein
the blocking group Abl is selected from the group consisting of Butoc, Hex, Pen, iHex, Butoc and nBuCAyl,
Xaa1 is a residue of Cys,
Xaa2 is a residue of an amino acid selected from the group consisting of Thr, Arg, Arg(Me), Asp, Glu, Gln, Om(DOTA), Lys, Lys(Me), Lys(DOTA), Cit, Ala, Aib, Har, Asn, Glu(AGLU), Om(mPEG12), KMe3, Orn, Om(Me), Om(Tris-Nta(Tris)), Ser, Thp, Ape, Apc(DOTA), Gly, Pro, Tyr and Leu,
Xaa3 is a residue of an amino acid selected from the group consisting of Om(DOTA), Lys(DOTA), Met, Arg, Arg(Me), Dap, Dab, Leu, Tyr, Gln, Glu, Lys(Ac), Cit, Ala, Nle, Asp, Har, Asn, Thr, Apc(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Om(Me), Ser and Gly,
Xaa4 is a residue of an amino acid selected from the group consisting of Pcnf, Dopa, Tyr, Aph, Mpa, Nif, Ocf and My and
Xaa5 is a residue of an amino acid selected from the group consisting of Phe, Thi, Pcf, Ocf, Egm, Mcf, 1Ni, Pnf and Mpa,
Xaa6 is a residue of an amino acid selected from the group consisting of 4Cfp, Proand Hyp,
Xaa7 is a residue of an amino acid selected from the group consisting of of Hyp, Pro, 4Cfp, Tap, Ala, Aib, 4Tfp, Chy, 4Ap, ala, Arg, Tap(DOTA), Lys(DOTA), Ser, ser, Leu, tyr and nma,
Xaa8 is a residue of an amino acid selected from the group consisting of Ile, Chg, Cha, Cpg, Phg, Pcf, Cbg and Npg,
Xaa9 is a residue of an amino acid selected from the group consisting of Nva, Leu, Cpra, Abu, Ser(Me) and Cbg, and
Xaa10 is a residue of an amino acid selected from the group consisting of Cys-NH₂ and Cysol-OH.

Embodiment 206. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204 and 205, preferably any one of Embodiments 173, 174, 177, 178, 179, 180, 181, 182, 183, 184, 185, 186, 187, 188, 189, 190, 191, 192, 193, 194, 195, 196, 197, 198, 199, 200, 201, 202, 203, 204 and 205, wherein
the blocking group Abl is selected from the group consisting of Butoc, Hex, Pen, iHex and nBuCAyl,
Xaa1 is a residue of Cys,
Xaa2 is a residue of an amino acid selected from the group consisting of Thr and Cit,
Xaa3 is a residue of an amino acid selected from the group consisting of Om(DOTA), Lys(DOTA), Gln, Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw),
Xaa4 is a residue of an amino acid selected from the group consisting of Pcnf, Dopa, Tyr, Aph, Mpa, Nif, Ocf, My and,
Xaa5 is a residue of an amino acid selected from the group consisting of Phe, Thi, Pcf, Ocf and Egm,
Xaa6 is a residue of an amino acid selected from the group consisting of 4Cfp, Pro and Hyp.
Xaa7 is a residue of an amino acid selected from the group consisting of Hyp, Pro, Aib, ala, nma, 4Tfp, Lys(DOTA) and Tap,
Xaa8 is a residue of an amino acid selected from the group consisting of Ile, Phg, Chg and Cha,
Xaa9 is a residue of an amino acid selected from the group consisting of Nva, Leu and Cpra, and
Xaa10 is a residue of Cys-NH₂.

Embodiment 207. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202 and 203, preferably any one of Embodiments 175, 176, 177, 178, 179, 180, 181, 182, 183, 184, 185, 186, 187, 188, 189, 190, 191, 192, 193, 194, 195, 196, 197, 198, 199, 200, 201, 202 and 203, wherein
XaaO is a residue of an amino acid selected from the group consisting of Met, Leu, leu, Aml, Egz, Nva, nva, Nle, nle, Nmb, Phg and Cha,
   Xaa1 is a residue of an amino acid selected from the group consisting of Cys and Pen,
Xaa2 is a residue of an amino acid selected from the group consisting of is a residue of an amino acid selected from the group consisting of Arg, Arg(Me), Asp, Glu, Gln, Lys, Lys(Me), Lys(Z), Cit, Ala, Aib, Har, Asn, Thr, Glu(Z), Om(mPEG12), KMe3, Orn, Om(Me), Om(Z), Ser, Thp, Ape, Apc(Z), Gly, Pro, Tyr and Leu,
Xaa3 is a residue of an amino acid selected from the group consisting of Met, Arg, Arg(Me), Dap, Dab, Leu, Tyr, Gln, Glu, Glu(Z), Lys(Ac), Lys(Z), Cit, Ala, Nle, Asp, Har, Asn, Thr, Apc(Z), Om(Z), Dab(Z), Dap(Z), Lys(AF488-Ttds), Lys, Lys(Me), KMe3, Orn, Om(Me), Ser and Gly,
Xaa4 is a residue of an amino acid selected from the group consisting of Tyr, Dopa, Phe, Aph, His, Mpa, My, Mcy, Guf, Ppa, Nif, Pcnf, Cha, Trp, Ay3, 2Ni and Ocf,
Xaa5 is a residue of an amino acid selected from the group consisting of Phe, Thi, Pcf, Ocf, Egm, Mcf, 1Ni, Pnf, Mpa, Bta and Trp,
Xaa6 is a residue of an amino acid selected from the group consisting of Pro, 4Cfp, Hyp, Eaz, Nmb, Oic, Leu, Cha, Chy, 4Tfp, Pip, Oxa, Aze and Phe,
Xaa7 is a residue of an amino acid selected from the group consisting of Pro, 4Cfp, Hyp, Tap, Ala, Aib, Asp, Ser, Tyr, ser, dap, tyr, Leu, 4Tfp, Chy, 4Ap, ala, Arg, Apc(Z), Tap(Z), Gly, nma, Aze, Oic, Oxa, Lys(Z) and Nmg,
Xaa8 is a residue of an amino acid selected from the group consisting of Ile, Chg, Cha, Cpg, Cbg, Npg, Phe, Pcf, Egz, Hfe, Phg, Leu, Nle and Nva,
Xaa9 is a residue of an amino acid selected from the group consisting of Leu, Nva, Cpra, Abu, Ser(Me), Cbg, Npg, Ala, Nle, Ile and Val, and
Xaa10 is a residue of an amino acid selected from the group consisting of Cys-NH₂, Cysol-OH, AET, Hcy-NHz, Pen-NH₂ and Cys-O2Oc-Lys(Z)-NH₂,
wherein Z is a Z group, and wherein an N-terminal modification group Nterm is covalently attached to the α-nitrogen atom of Xaa0, wherein Nterm is preferably selected from the group consisting of a -Ac, a methyl group and a Z group.

Embodiment 208. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203 and 207, preferably any one of Embodiments 175, 176, 177, 178, 179, 180, 181, 182, 183, 184, 185, 186, 187, 188, 189, 190, 191, 192, 193, 194, 195, 196, 197, 198, 199, 200, 201, 202, 203 and 207, wherein
Xaa0 is a residue of an amino acid selected from the group consisting of Phg, Leu, Nva, Aml, Egz, Nle and Cha,
Xaa1 is a residue of Cys,
Xaa2 is a residue of an amino acid selected from the group consisting of Thr, Arg, Arg(Me), Asp, Glu, Gln, Om(DOTA), Lys, Lys(Me), Lys(DOTA), Cit, Ala, Aib, Har, Asn, Glu(AGLU), Om(mPEG12), KMe3, Orn, Om(Me), Om(Tris-Nta(Tris)), Ser, Thp, Ape, Apc(DOTA), Gly, Pro, Tyr and Leu,
Xaa3 is a residue of an amino acid selected from the group consisting of Om(DOTA), Lys(DOTA), Met, Arg, Arg(Me), Dap, Dab, Leu, Tyr, Gln, Glu, Lys(Ac), Cit, Ala, Nle, Asp, Har, Asn, Thr, Apc(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Om(Me), Ser and Gly,
Xaa4 is a residue of an amino acid selected from the group consisting of Pcnf, Dopa, Tyr, Aph, Mpa, Nif, Ocf and My ,
Xaa5 is a residue of an amino acid selected from the group consisting of Phe, Thi, Pcf, Ocf, Egm, Mcf, 1Ni, Pnf and Mpa,
Xaa6 is a residue of an amino acid selected from the group consisting of 4Cfp, Pro, and Hyp,
Xaa7 is a residue of an amino acid selected from the group consisting of of Hyp, Pro, 4Cfp, Tap, Ala, Aib, 4Tfp, Chy, 4Ap, ala, Arg, Tap(DOTA), Lys(DOTA), Ser, ser, Leu, tyr and nma,
Xaa8 is a residue of an amino acid selected from the group consisting of Ile, Chg, Cha, Cpg, Phg, Pcf, Cbg and Npg,
Xaa9 is a residue of an amino acid selected from the group consisting of Nva, Leu, Cpra, Abu, Ser(Me) and Cbg, and
Xaa10 is a residue of an amino acid selected from the group consisting of Cys-NH₂ and Cysol-OH,
and wherein an N-terminal modification group Nterm is covalently attached to the α-nitrogen atom of Xaa0, wherein Nterm is preferably selected from the group consisting of a -Ac, a methyl group and a Z group.

Embodiment 209. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 207 and 208, preferably any one of Embodiments 175, 176, 177, 178, 179, 180, 181, 182, 183, 184, 185, 186, 187, 188, 189, 190, 191, 192, 193, 194, 195, 196, 197, 198, 199, 200, 201, 202, 203, 207 and 208, wherein
Xaa0 is a residue of an amino acid selected from the group consisting of Phg, Leu, Nva, Aml, Egz, Nle and Cha,
   Xaa1 is a residue of Cys,
Xaa2 is a residue of an amino acid selected from the group consisting of Thr and Cit,
Xaa3 is a residue of an amino acid selected from the group consisting of Om(DOTA), Lys(DOTA), Gln, Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw),
Xaa4 is a residue of an amino acid selected from the group consisting of Pcnf, Dopa, Tyr, Aph, Mpa, Nif, Ocf and My,
Xaa5 is a residue of an amino acid selected from the group consisting of Phe, Thi, Pcf, Ocf and Egm,
Xaa6 is a residue of an amino acid selected from the group consisting of 4Cfp, Pro and Hyp.
Xaa7 is a residue of an amino acid selected from the group consisting of Hyp, Pro, Aib, ala, nma, 4Tfp, Lys(DOTA) and Tap,
Xaa8 is a residue of an amino acid selected from the group consisting of Ile, Phg, Chg and Cha,
Xaa9 is a residue of an amino acid selected from the group consisting of Nva, Leu and Cpra, and
Xaa10 is a residue of Cys-NH₂,
and wherein an N-terminal modification group Nterm is covalently attached to the α-nitrogen atom of Xaa0, wherein Nterm is preferably selected from the group consisting of a -Ac, a methyl group and a Z group.

Embodiment 210. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208 and 209, of any one of Embodiments 1, 2, 3, 4, 5, 6, 7, 8, 9, 10, 11, 12, 13 and 14, or of any one of Embodiments 1, 2 and 3, wherein the compound is selected from the group consisting of
compound iHex-[Cys-Cit-Gln-Tyr-Phe-Pro-Hyp-Ile-Leu-Cys]-NH₂ (UPAR-174) of the following formula 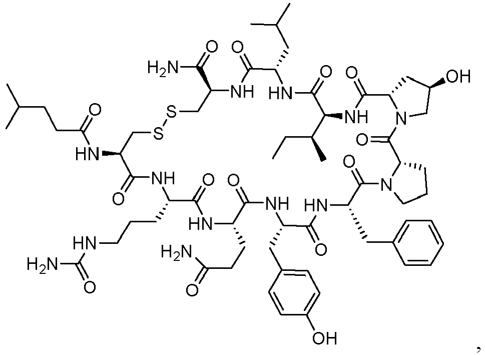
compound iHex-[Cys-Cit-Gln-Tyr-Phe-Pro-4Tfp-Ile-Leu-Cys]-NH₂ (UPAR-220) of the following formula 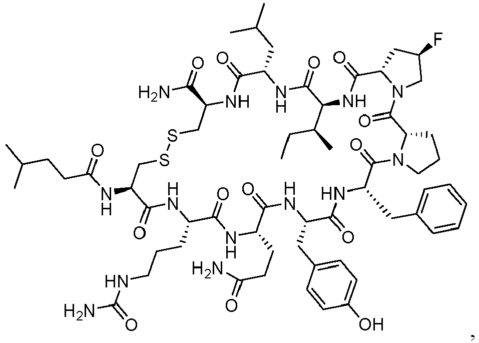
compound Pent-[Cys-Arg-Gln-2Ni-Phe-Pro-Hyp-Ile-Nva-Cys]-NH₂ (UPAR-259) of the following formula 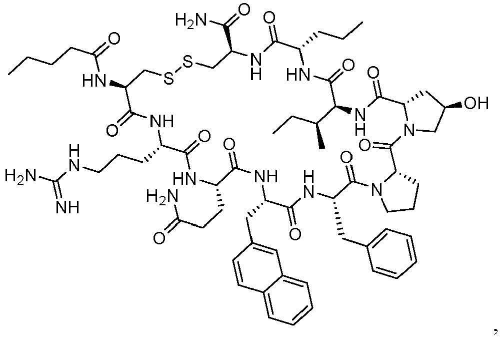
compound AGLU-Glutar-Nva-[Cys-Cit-Gln-Tyr-Phe-Pro-Hyp-Ile-Nva-Cys]-NH₂ (UPAR-268) of the following formula 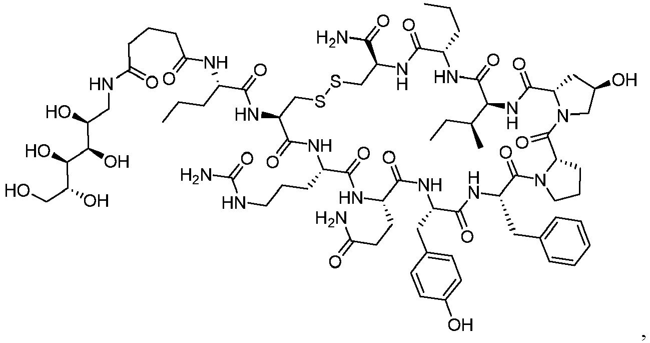
compound Pent-[Cys-Cit-Gln-Tyr-Phe-Pro-Hyp-Ile-Nva-Cys]-NHz (UPAR-309) of the following formula 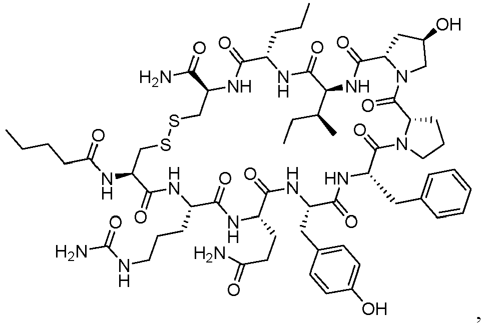
compound Pent-[Cys-Arg-Arg-Tyr-Phe-Pro-Tap-Ile-Nva-Cys]-NH₂ (UPAR-313) of the following formula 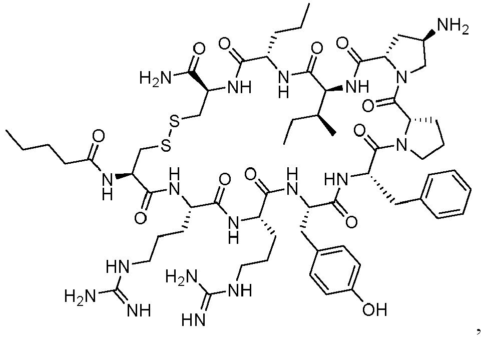
compound Butoc-[Cys-Thr-Gln-Tyr-Phe-4Cfp-Hyp-Ile-Nva-Cys]-NH₂ (UPAR-358) of the following formula 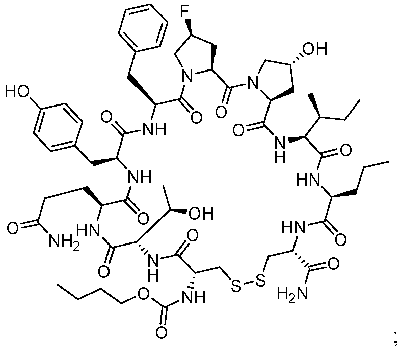
compound Pent-[Cys-Arg-Gln-Tyr-Phe-Pro-Hyp-Ile-Cpra-Cys]-NHz (UPAR-379) of the following formula 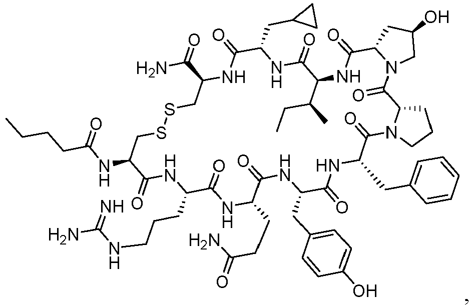
compound Pent-[Cys-Glu(AGLU)-Gln-Tyr-Phe-Pro-Hyp-Ile-Nva-Cys]-NH₂ (UPAR-386) of the following formula 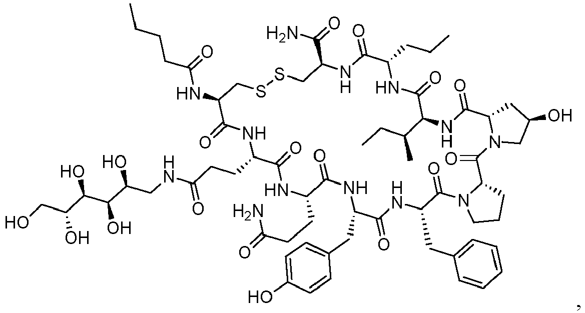
compound Pent-[Cys-Arg-Gln-Ocf-Phe-Pro-Hyp-Ile-Nva-Cys]-NH₂ (UPAR-395) of the following formula 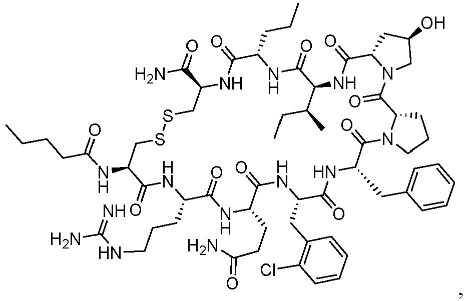
compound iHex-[Cys-Cit-Asp-Tyr-Phe-Pro-Hyp-Ile-Leu-Cys]-NHz (UPAR-410) of the following formula 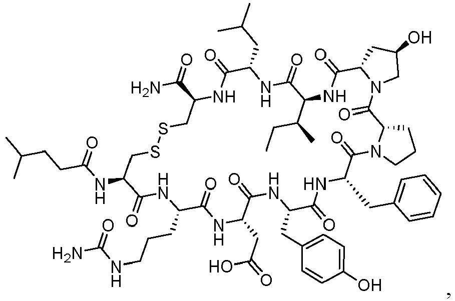
compound Pent-[Cys-Arg-Gln-Tyr-Phe-Pro-Hyp-Phg-Nva-Cys]-NHz (UPAR-437) of the following formula 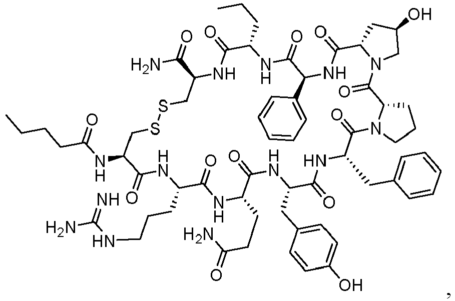
compound Ac-Asp-Leu-[Cys-Arg-Nle-Tyr-Phe-Pro-Aib-Ile-Leu-Cys]-NH₂ (UPAR-440) of the following formula 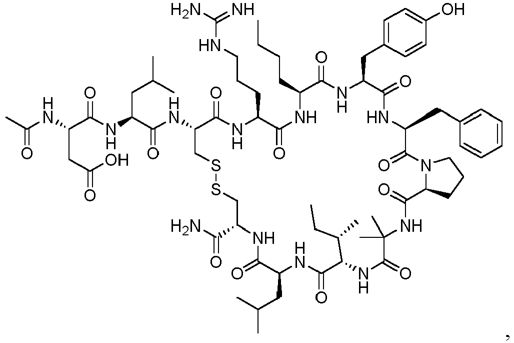
compound Pent-[Cys-Arg-Lys-Tyr-Phe-Pro-Hyp-Ile-Nva-Cys]-NHz (UPAR-448) of the following formula 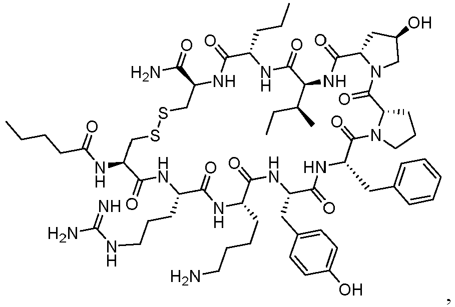
compound iHex-[Cys-Arg-Asp-Tyr-Phe-Pro-Hyp-Ile-Leu-Cys]-NHz (UPAR-458) of the following formula 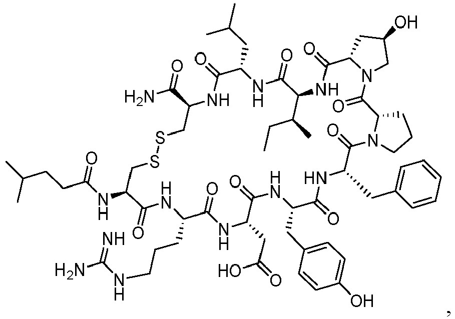
compound Butoc-[Cys-Thr-Lys(AF488-Ttds)-Dopa-Phe-4Cfp-Hyp-Ile-Nva-Cys]-NH₂ (UPAR-479) of the following formula 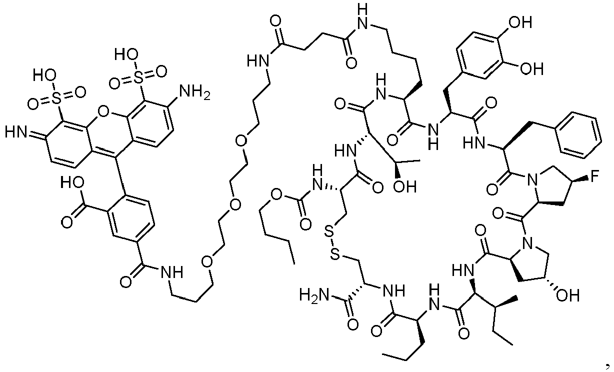
compound Ac-Egz-[Cys-Arg-Gln-Tyr-Phe-Pro-Hyp-Ile-Nva-Cys]-NH₂ (UPAR-482) of the following formula 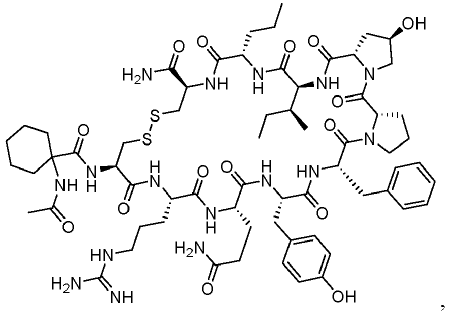
compound Ac-Aml-[Cys-Arg-Gln-Tyr-Phe-Pro-Hyp-Ile-Nva-Cys]-NH₂ (UPAR-485) of the following formula 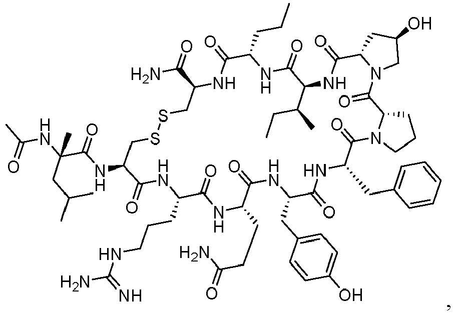
compound Ac-Phg-[Cys-Arg-Gln-Tyr-Phe-Pro-Hyp-Ile-Nva-Cys]-NHz (UPAR-486) of the following formula 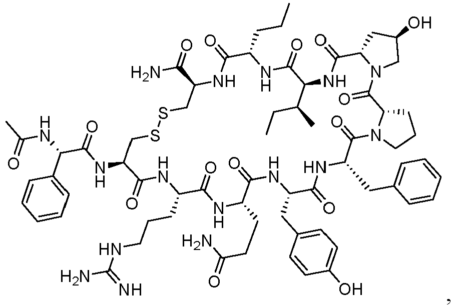
or a pharmaceutically acceptable salt or hydrate or pharmaceutically acceptable solvate thereof.

Embodiment 211. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208 and 209, wherein each Z group is each and independently selected from the group consisting of a chelator optionally comprising a linker moiety L and a bio-distribution modifier optionally comprising a linker.

Embodiment 212. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209 and 211, wherein each Z group is each and independently a chelator optionally comprising a linker moiety L.

Embodiment 213. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211 and 212, wherein the chelator is selected from the group consisting of DOTA, DOTAGA, DOTAM, Crown, DOTP, NOTA, NODAGA, NODA-MPAA, HBED, TETA, CB-TE2A, DTPA, CHX-A"-DTPA, DFO, Macropa, HOPO ligand platform, TRAP, THP, AAZTA, DATA, NOPO, PCTA, PSC, sarcophagine, FSC, DEPA, DE4TA, NETA, NE3TA, H₄octapa, H₄CHXoctapa, H₄pypa, H₄py₄pa, HYNIC, NxS4-x (N4, N2S2, N3S) and ^{99m}Tc(CO)₃-chelators.

Embodiment 214. The compound or pharmaceutically acceptable salt or hydrate or pharmaceutically acceptable solvate of Embodiment 213, wherein the chelator is selected from the group consisting of DOTA, DOTAGA, DOTAM, Crown, NOTA, NODAGA, NODA-MPAA, CB-TE2A, CHX-A"-DTPA, DFO, Macropa, THP, AAZTA, NOPO, PCTA, PSC, sarcophagine, NETA, H₄CHXoctapa, H₄pypa and N4, preferably the chelator is selected from the group consisting DOTA, DOTAGA, DOTAM, NOTA, NODAGA, Macropa, Crown, NOPO, PCTA, PSC and N4, more preferably the chelator is selected from the group consisting DOTA, DOTAM, Macropa, NOTA, PSC and NODAGA, and most preferably the chelator is DOTA.

Embodiment 215. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213 and 214, wherein each Z group is independently a moiety represented by the following formula (XIII):

E-* (XIII)

wherein,
* indicates covalent attachment to Xaa0, Xaa2, Xaa3 or Xaa7, and
E is a residue of a chelator represented by any one of the following formulae (XIVa) to (XIVn): 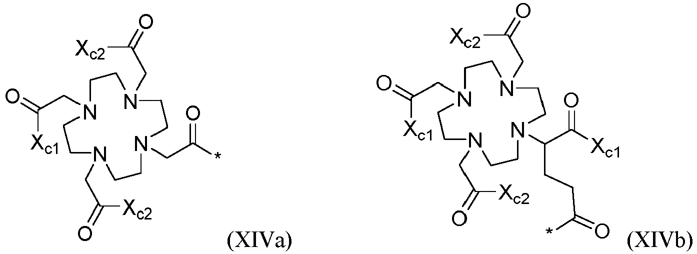 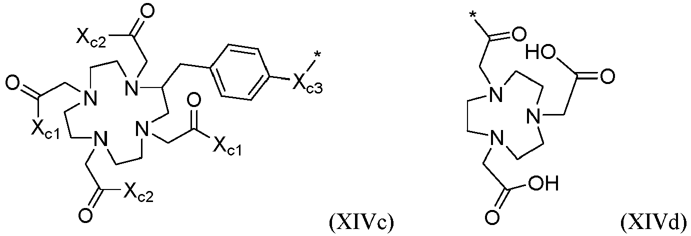 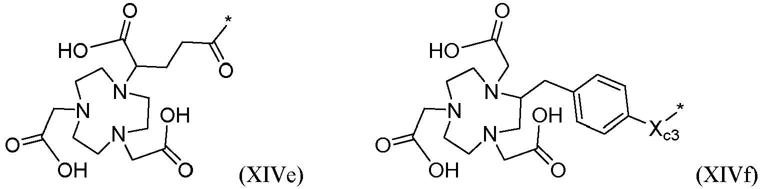 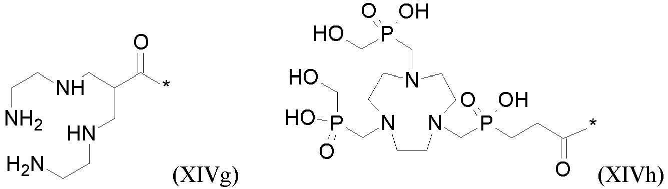 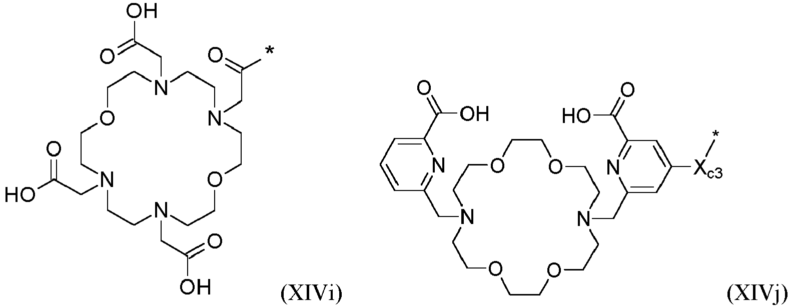 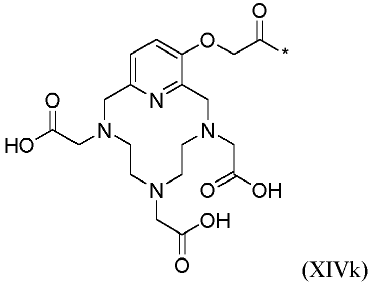 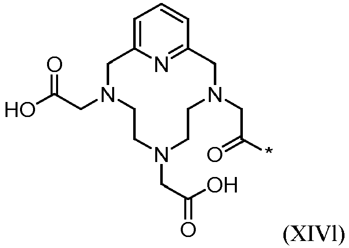 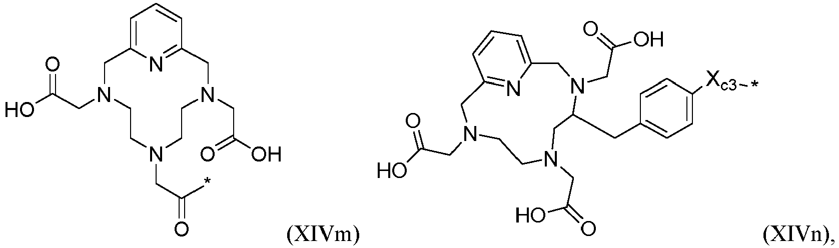 wherein,
X_{c1} and X_{c2} are each and independently selected from the group consisting of -OH and -NH₂, and
X_{c3} is independently selected from the group consisting of -NH-* and -N-C(S)-*,
* indicates covalent attachment to Xaa0, Xaa2, Xaa3 or Xaa7, and
the residue E optionally comprises a nuclide, preferably a radionuclide.

Embodiment 216. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213 and 214, wherein each Z group is independently a moiety represented by the following formula (XV):

E-L-* (XV)

wherein,
* indicates covalent attachment to Xaa0, Xaa2, Xaa3 or the C-terminal carbonyl group of Xaa10, and
   (a)
      E is a residue represented by any one of the following formulae (XVIa) to (XVIn): 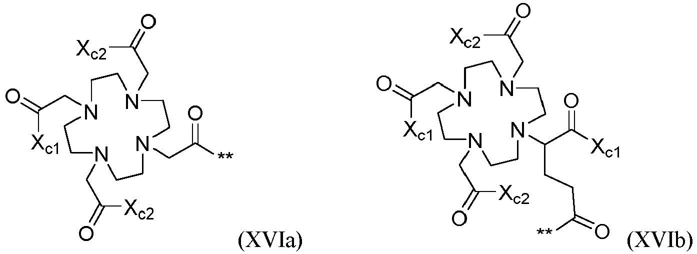 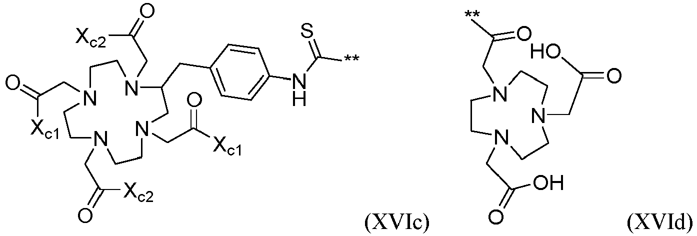 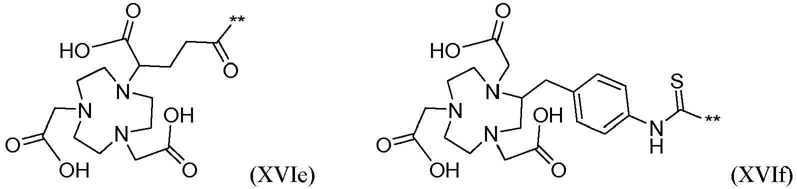 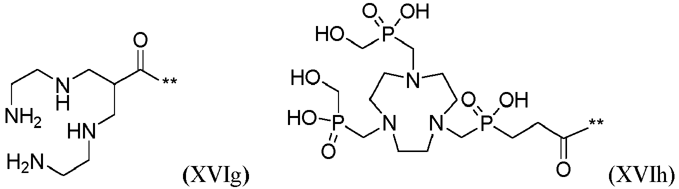 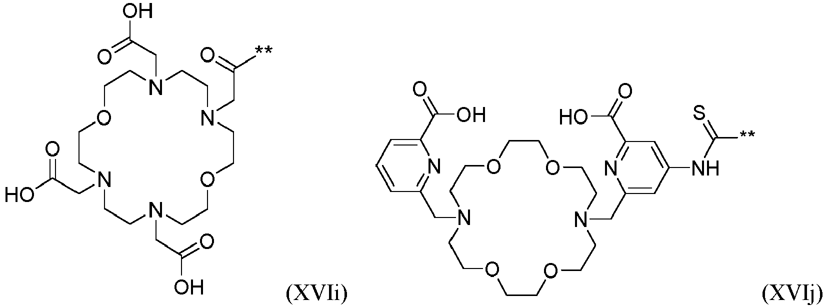 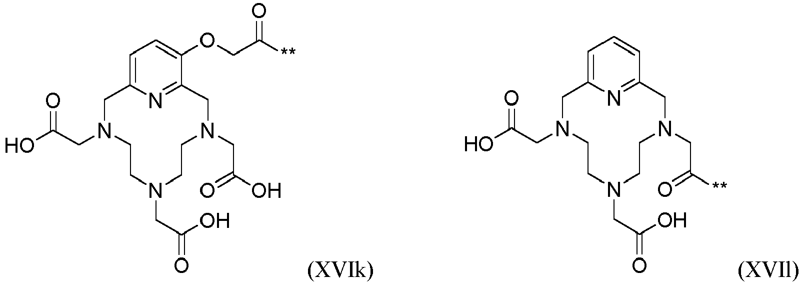 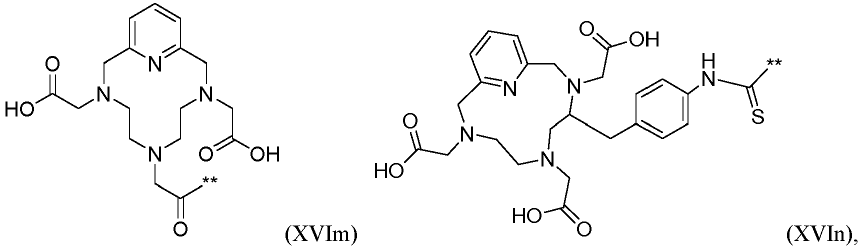 wherein,
         X_{c1} and X_{c2} are each independently selected from the group consisting of -OH and -NH₂, and
         * * indicates covalent attachment to L,
         the residue E optionally comprises a nuclide, preferably a radionuclide, and
      L is a linker moiety represented by any one of the formulae (XVIIa) to (XVIIt): 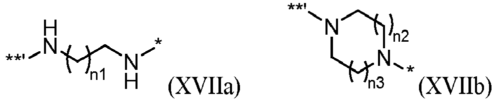 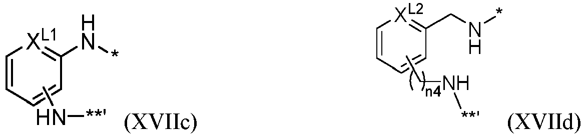 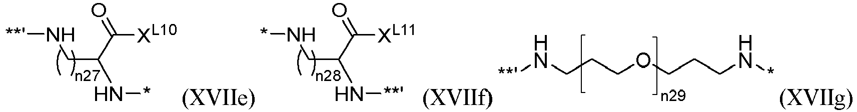 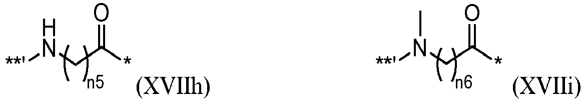 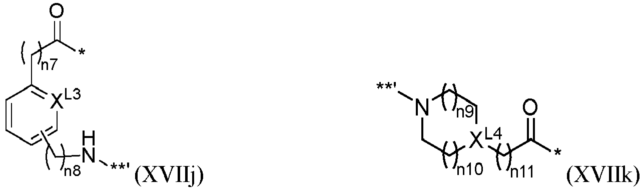 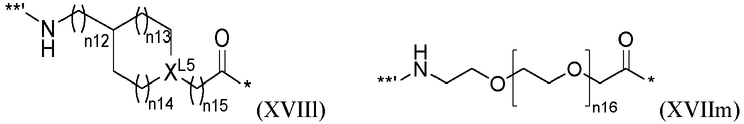 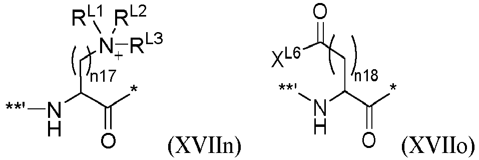 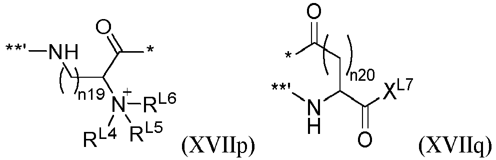 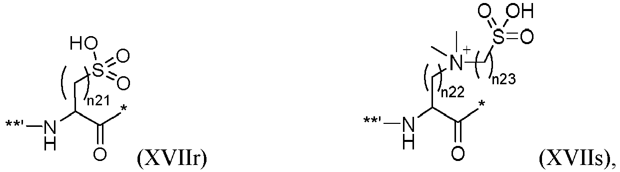 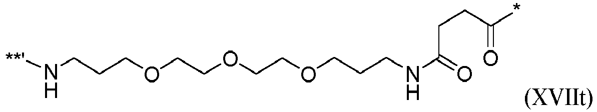 wherein,
         n1 is 1, 2, 3, 4, or 5,
         n2, n3, n4 and n12 are each and independently 0, 1 or 2,
         n5, n6 and n29 are each and independently 1, 2, 3, 4, 5, 6, 7, 8, 9 or 10,
         n7 and n8 are each and independently 0, 1, 2, 3 or,
         n9, n13, n14, n15, n18, n20 and n21 are each and independently 1 or 2,
         n 10 and n11 are each and independently 0 or 1,
         n16 is 0, 1, 2, 3, 4, 5, 6, 7, 8, 9, 10, 11 or 12,
         n17, n19, n22, n27 and n28 are each and independently 1, 2, 3, or 4,
         n23 is 2, 3 or 4,
         X^{L1}, X^{L2}, X^{L3}, X^{L4} and X^{L5} are each and independently =CH- or =N-,
         X^{L6}, X^{L7}, X^{L10} and X^{L11} are each and independently -OH or -NH₂,
         R^{L1}, R^{L2}, R^{L3}, R^{L4}, R^{L5} and R^{L6} are each and independently -H or a methyl group,
         * indicates covalent attachment to Xaa0, Xaa2, Xaa3 or the C-terminal carbonyl group of Xaa10,
         * *' indicates covalent attachment to E;
         or,
   (b)
      E is a residue represented by the following formula (XVIo) or (XVIr): 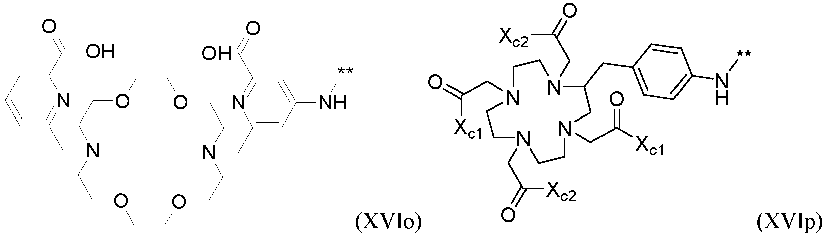 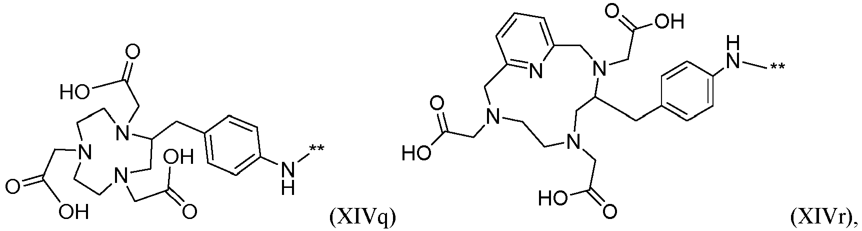 wherein,
         X_{c1} and X_{c2} are each and independently selected from the group consisting of -OH and -NH₂, and
         * * indicates covalent attachment to L, and
         the residue E optionally comprises a nuclide, preferably a radionuclide,
      L is a linker moiety represented by any one of the formulae (XVIIu) to (XVIIy): 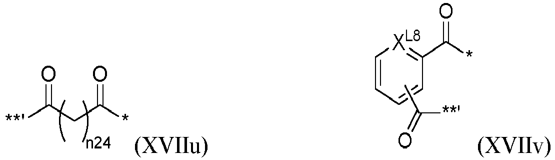 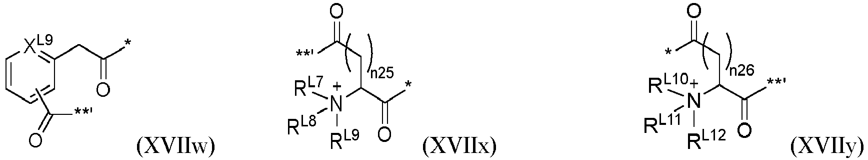 wherein,
         n24 is 1, 2, 3, 4, or 5,
         n25 and n26 are each and independently 1 or 2,
         X^{L8} and X^{L9} are each and independently =CH- or =N-,
         R^{L7}, R^{L8}, R^{L9}, R^{L10}, R^{L12} and R^{L12} are each and independently -H or a methyl group, and
         * indicates covalent attachment to Xaa0, Xaa2 or Xaa3, and
         **' indicates covalent attachment to E.

Embodiment 217. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213 and 214, wherein each Z group is independently a moiety represented by the following formula (XV'):

E-L₁-L₂-* (XV')

wherein,
* indicates covalent attachment to a carbonyl group of Xaa2, Xaa3 or the C-terminal carbonyl group of Xaa10, and
E is a residue represented by any one of the following formulae (XVIa) to (XVIn): 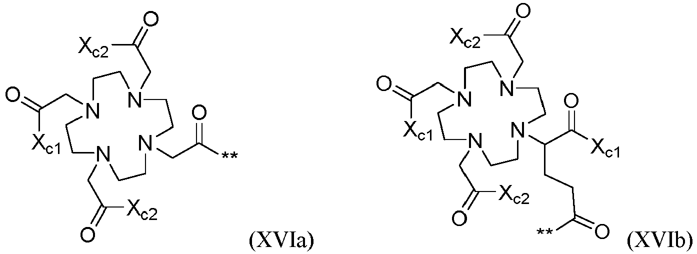 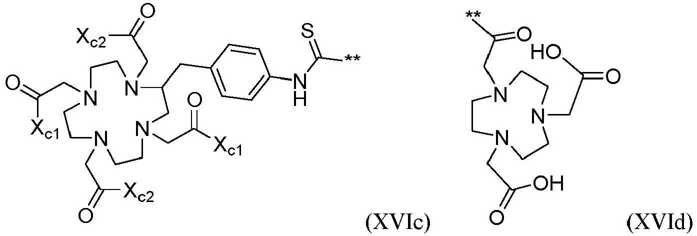 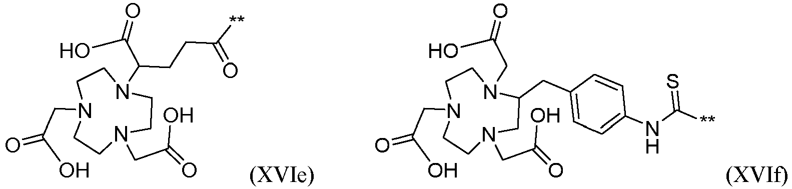 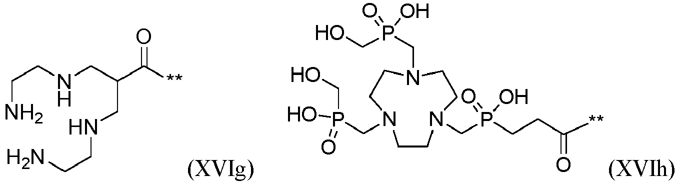 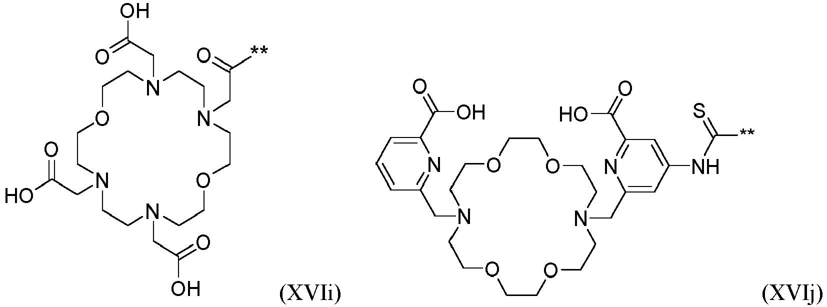 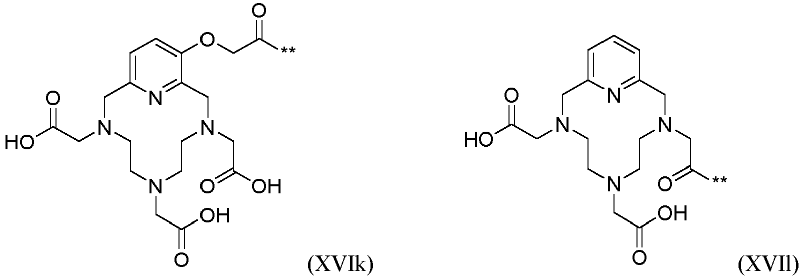 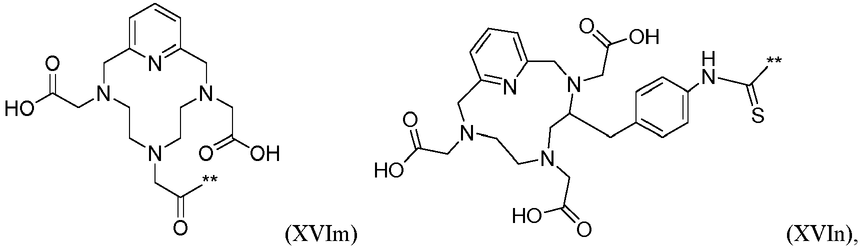 wherein,
   X_{c1} and X_{c2} are each and independently selected from the group consisting of -OH and -NH₂, and
   * * indicates covalent attachment to L₁,
   the residue E optionally comprises a nuclide, preferably a radionuclide, and
L₁ is a linker moiety represented by any one of the formulae (XVII'a) to (XVII'g): 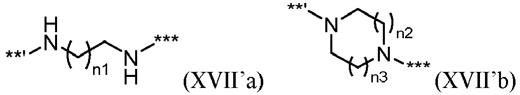 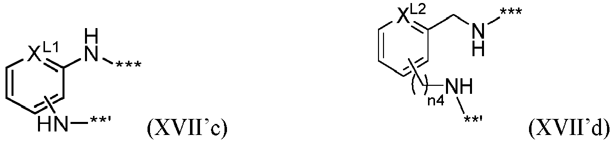 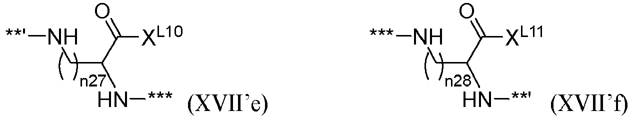 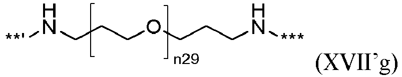 wherein,
   n1 is 1, 2, 3, 4 or 5,
   n2, n3 and n4 are each and independently 0, 1 or 2,
   n29 is 1, 2, 3, 4, 5, 6, 7, 8, 9 or 10,
   n27 and n28 are each and independently 1, 2, 3 or 4,
   X^{L1} and X^{L2} are each and independently =CH- or =N-,
   X^{L10} and X^{L11} are each and independently -OH or -NH₂,
   *** indicates covalent attachment to L₂, and
   * *' indicates covalent attachment to E; and
L₂ is a linker moiety represented by any one of the formulae (XVII'h) to (XVII't): 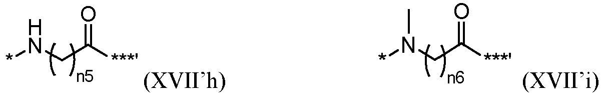 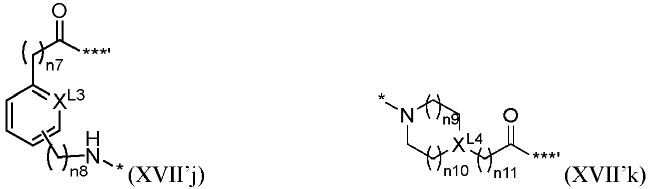 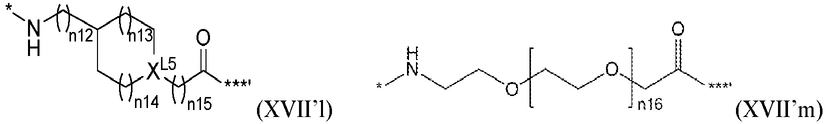 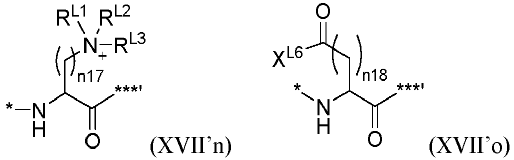 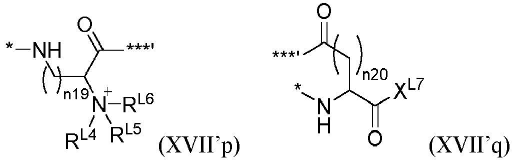 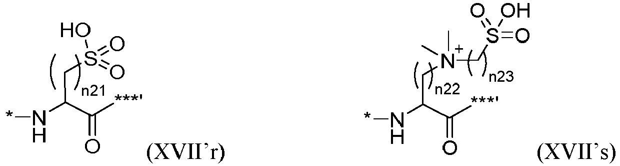 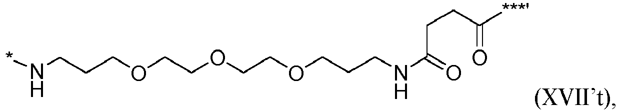 wherein,
   n12 is 0, 1 or 2,
   n5 and n6 are each and independently 1, 2, 3, 4, 5, 6, 7, 8, 9 or 10,
   n7 and n8 are each and independently 0, 1, 2, 3 or 4,
   n9, n13, n14, n15, n18, n20 and n21 are each and independently 1 or 2,
   n 10 and n11 are each and independently 0 or 1,
   n16 is 0, 1, 2, 3, 4, 5, 6, 7, 8, 9, 10, 11 or 12,
   n17, n19 and n22 are each and independently 1, 2, 3 or 4,
   n23 is 2, 3 or 4,
   X^{L3}, X^{L4} and X^{L5} are each and independently =CH- or =N-,
   X^{L6} and X^{L7} are each and independently -OH or -NH₂,
   R^{L1}, R^{L2}, R^{L3}, R^{L4}, R^{L5} and R^{L6} are each and independently -H or a methyl group,
   * indicates covalent attachment to a carbonyl group of Xaa2, Xaa3 or Xaa10, and
   ***' indicates covalent attachment to L₁.

Embodiment 218. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213 and 214, wherein each Z group is independently a moiety represented by the following formula (XV"):

E-L-* (XV")

wherein,
* indicates covalent attachment to a carbonyl group of Xaa2, Xaa3 or Xaa10, and
E is a residue represented by the following formula (XVIo) or (XVIr): 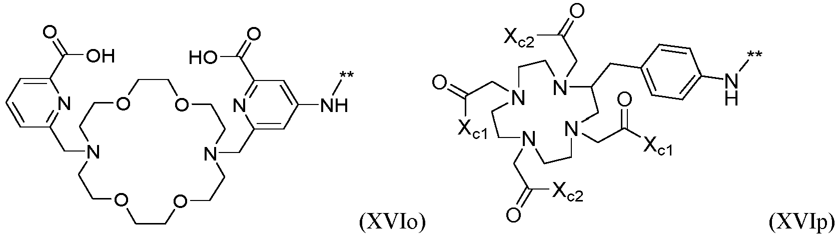 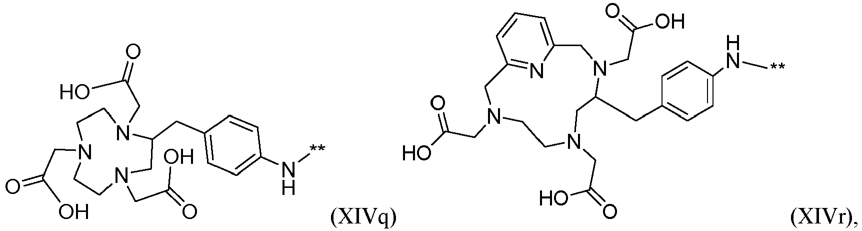 wherein,
   X_{c1} and X_{c2} are each and independently selected from the group consisting of -OH and -NH₂, and
   ** indicates covalent attachment to L, and
   the residue E optionally comprises a nuclide, preferably a radionuclide,
L is a linker moiety represented by any one of the formulae (XVII"h) to (XVII"t): 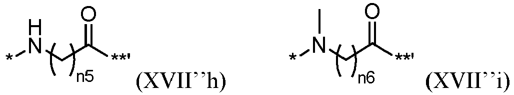 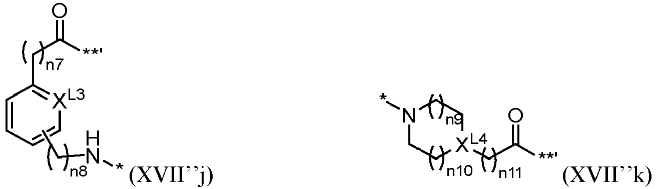 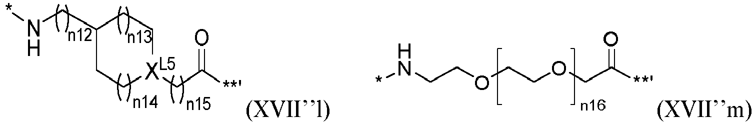 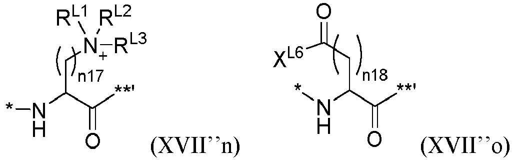 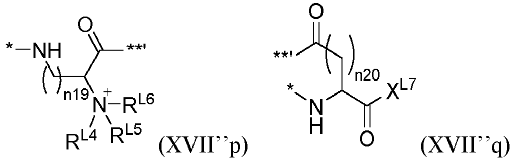 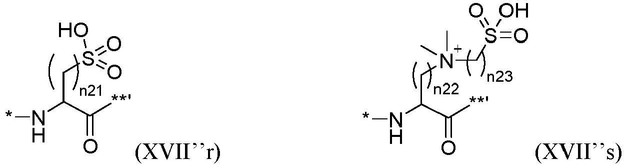 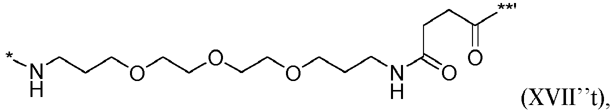 wherein,
   n12 is 0, 1 or 2,
   n5 and n6 are each and independently 1, 2, 3, 4, 5, 6, 7, 8, 9 or 10,
   n7 and n8 are each and independently 0, 1, 2, 3, or 4,
   n9, n13, n14, n15, n18, n20 and n21 are each and independently 1 or 2,
   n 10 and n11 are each and independently 0 or 1,
   n16 is 0 to 12,
   n17, n19 and n22 are each and independently 1, 2, 3, or 4,
   n23 is 2, 3 or 4,
   X^{L3}, X^{L4} and X^{L5} are each and independently =CH- or =N-,
   X^{L6} and X^{L7} are each and independently -OH or -NH₂,
   R^{L1}, R^{L2}, R^{L3}, R^{L4}, R^{L5} and R^{L6} are each and independently -H or a methyl group,
   * indicates covalent attachment to a carbonyl group of Xaa2, Xaa3 or Xaa10, and
   **' indicates covalent attachment to L.

Embodiment 219. The compound or pharmaceutically acceptable salt or hydrate or pharmaceutically acceptable solvate any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214 and 216, wherein each Z group is independently a moiety represented by any one of the following formulae (XVIIIa) to (XVIIIn): 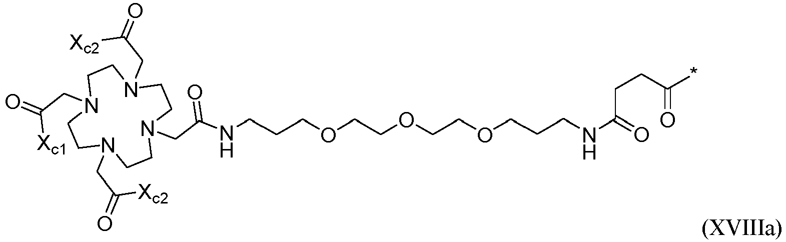 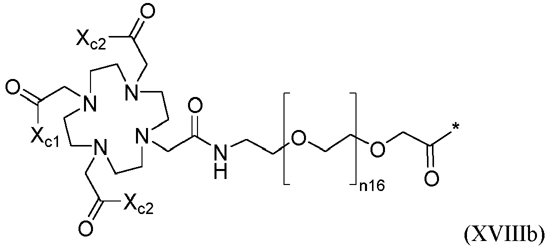 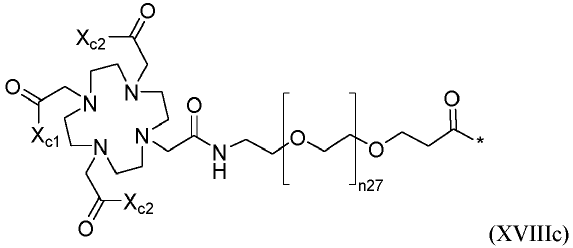 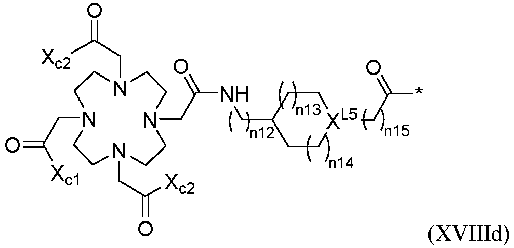 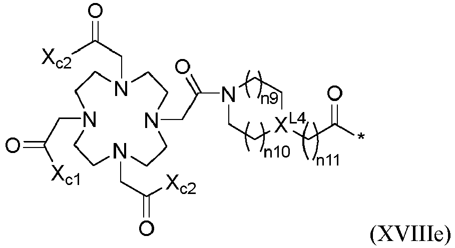 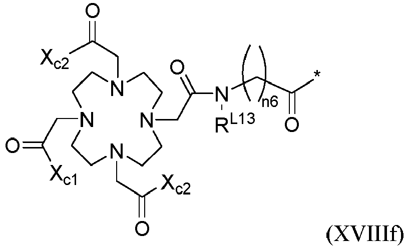 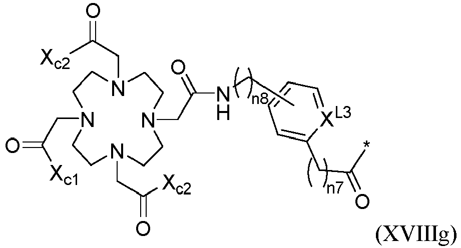 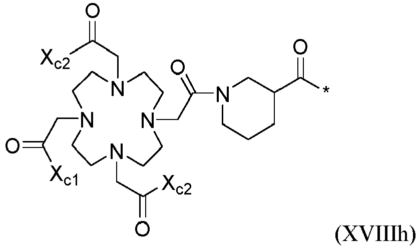 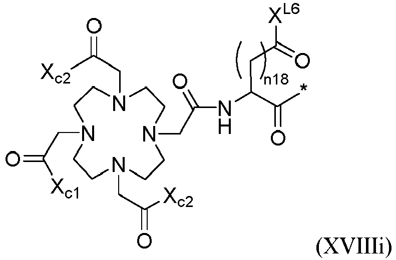 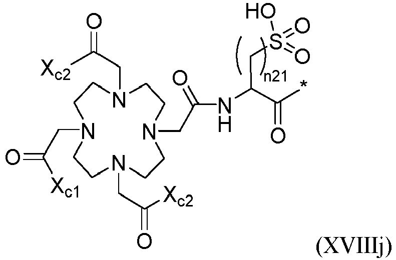 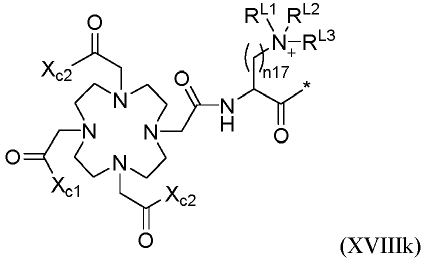 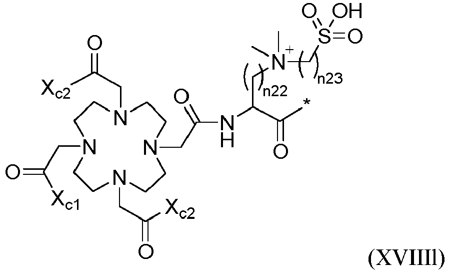 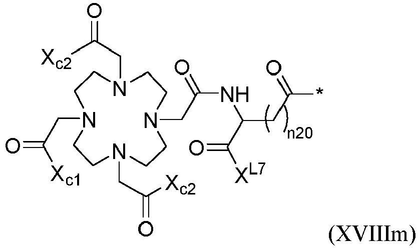 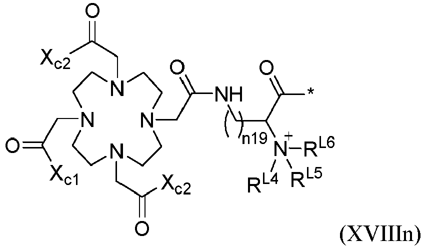 wherein,
X_{c1}, X_{c2}, X^{L6} and X^{L7} are each and independently selected from the group consisting of -OH and -NH₂, and
n6 is 1, 2, 3, 4, 5, 6, 7, 8, 9 or 10, and
n7 and n8 are each and independently 0, 1, 2, 3 or 4,
n9, n13, n14, n15, n18, n20 and n21 are each and independently 1 or 2, and n10 and n11 are each and independently 0 or 1, and
n12 is 0 to 2,
n16 and n27 are each and independently 0, 1, 2, 3, 4, 5, 6, 7, 8, 9, 10, 11 or 12, and n17, n19 and n22 are each and independently 1, 2, 3 or 4, and
n23 is 2, 3 or 4, and
X^{L3}, X^{L4} and X^{L5} are each and independently =N- or =CH-, and
R^{L1}, R^{L2}, R^{L3}, R^{L4}, R^{L5}, R^{L6} and R^{L13} are each and independently -H or a methyl group, and
* indicates covalent attachment to XaaO, Xaa2 or Xaa3, and
the moiety represented by any one of the above formulae (XVIIIa) to (XVIIIn) optionally comprises a nuclide, preferably a radionuclide.

Embodiment 220. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214, 215, 216, 217, 218 and 219, any one of Embodiments 1, 2, 3, 4, 5, 6, 7, 8, 9, 10, 11, 12, 13 and 14 or any one of Embodiments 1, 2 and 3, wherein the compound is selected from the group consisting of
compound Butoc-[Cys-Thr-Lys(DOTA)-Tyr-Phe-Hyp-Hyp-Ile-Nva-Cys]-NH₂ (UPAR-128) of the following formula 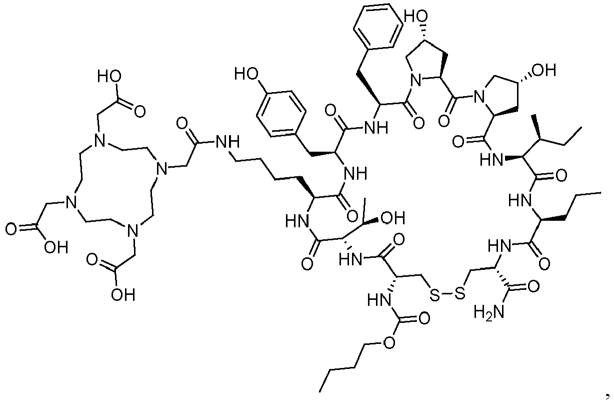
compound Butoc-[Cys-Thr-Lys(DOTA)-Tyr-Thi-Pro-Hyp-Ile-Nva-Cys]-NH₂ (UPAR-134) of the following formula 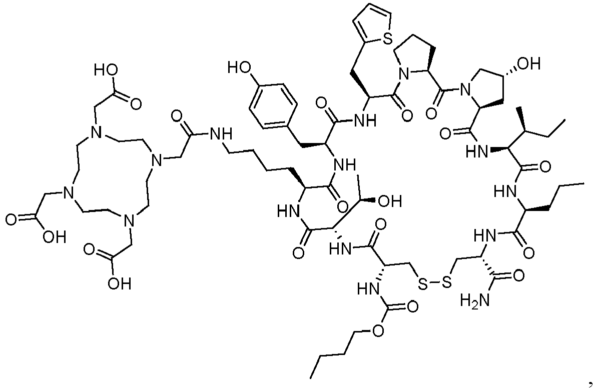
compound Pent-[Cys-Thr-Lys(DOTA)-Tyr-Phe-Hyp-Hyp-Ile-Nva-Cys]-NH₂ (UPAR-135) of the following formula 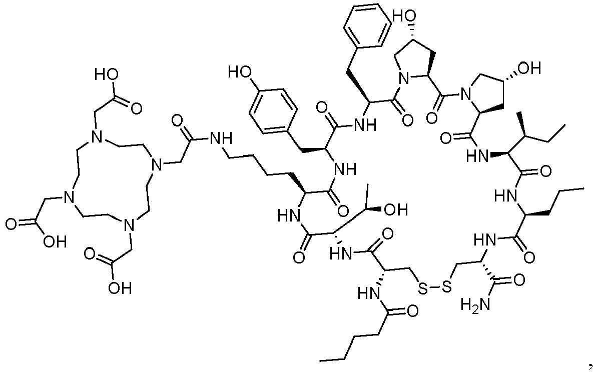
compound Pent-[Cys-Cit-Lys(DOTA)-Mpa-Phe-Pro-Hyp-Ile-Nva-Cys]-NH₂ (UPAR-138) of the following formula 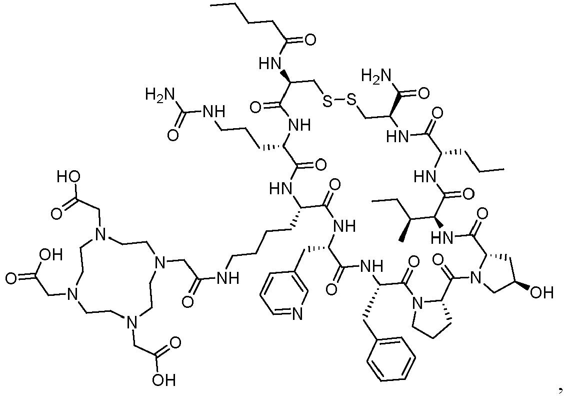
compound Hex-Lys(DOTA)-Leu-[Cys-Arg-Met-Tyr-Phe-Pro-Ala-Ile-Leu-Cys]-NH₂ (UPAR-140) of the following formula 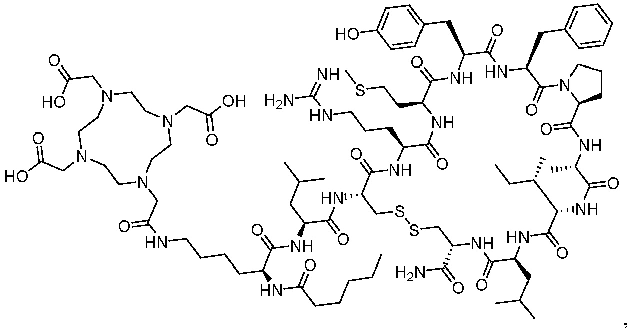
compound iHex-[Cys-Cit-Gln-Tyr-Phe-Pro-Lys(DOTA)-Ile-Leu-Cys]-NH₂ (UPAR-145) of the following formula 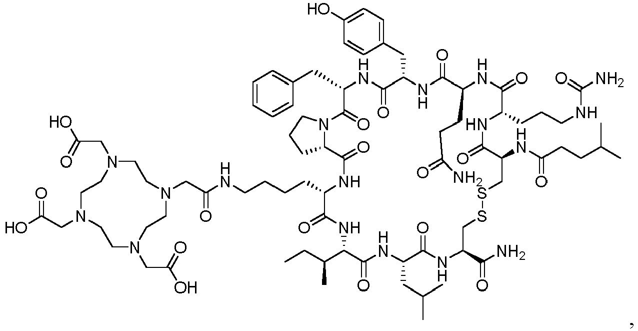
compound Pent-[Cys-Cit-Lys(DOTA)-Guf-Phe-Pro-Hyp-Ile-Nva-Cys]-NH₂ (UPAR-149) of the following formula 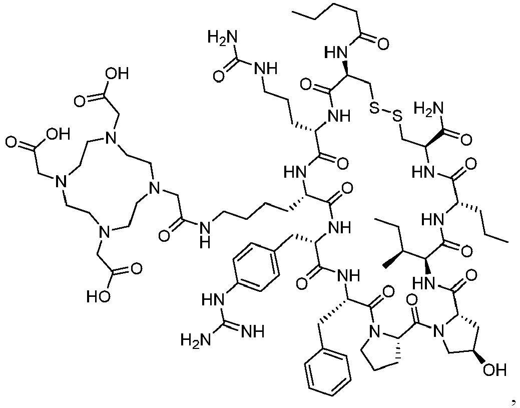
compound Pent-[Cys-KMe3-Lys(DOTA)-Tyr-Phe-Pro-Hyp-Ile-Nva-Cys]-NH₂ (UPAR-153) of the following formula 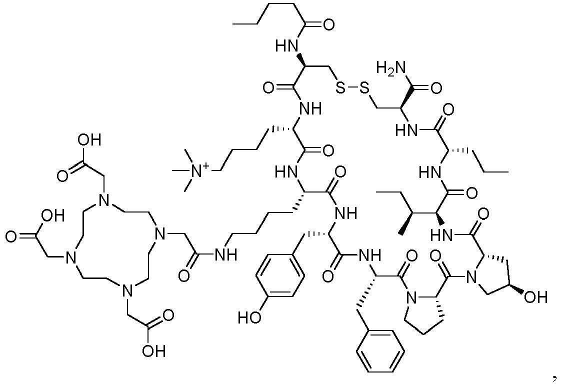
compound iHex-[Cys-Lys(DOTA)-Gln-Tyr-Phe-Pro-ala-Ile-Leu-Cys]-NH₂ (UPAR-157) of the following formula 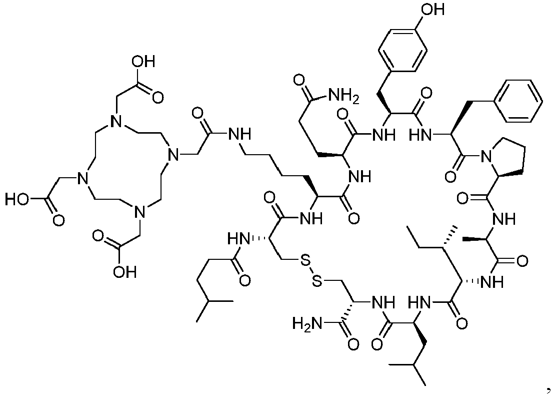
,compound Ac-Leu-[Cys-Arg-Lys(DOTA)-Tyr-Phe-Pro-ala-Ile-Leu-Cys]-NH₂ (UPAR-162) of the following formula 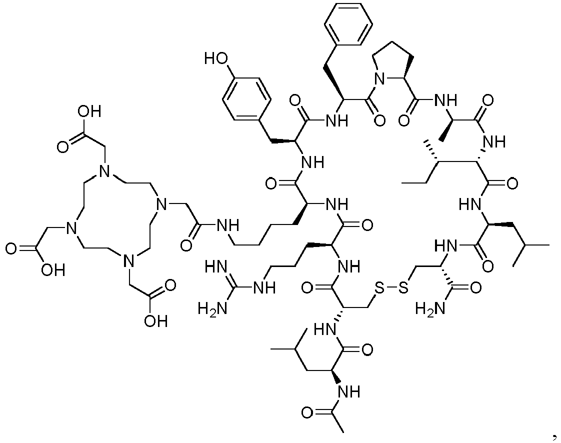
compound Butoc-[Cys-Thr-Lys(DOTA)-Mpa-Phe-4Cfp-Hyp-Ile-Nva-Cys]-NH₂ (UPAR-165) of the following formula 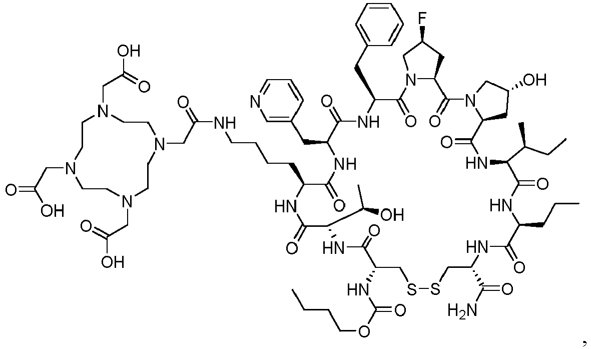
compound iHex-[Cys-Arg-Lys(DOTA)-Tyr-Phe-Pro-ala-Ile-Leu-Cys]-NH₂ (UPAR-176) of the following formula 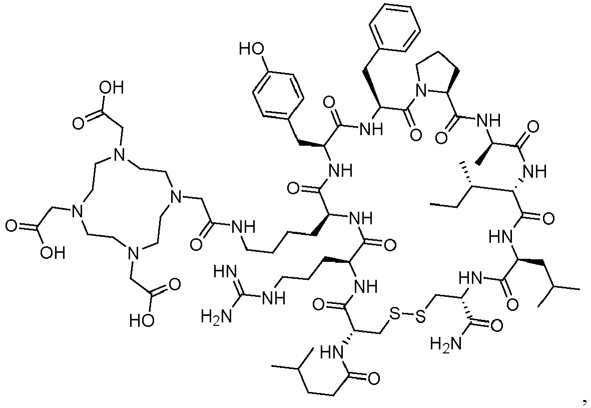
compound Butoc-[Cys-Thr-Lys(DOTA-PPAc)-Tyr-Phe-4Cfp-Hyp-Ile-Nva-Cys]-NH₂ (UPAR-181) of the following formula 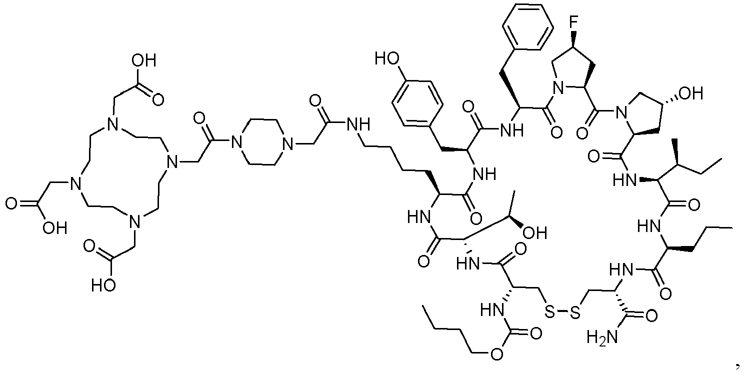
compound Ac-Asp-Leu-[Cys-Cit-Lys(DOTA)-Tyr-Phe-Pro-ala-Ile-Leu-Cys]-NH₂ (UPAR-189) of the following formula 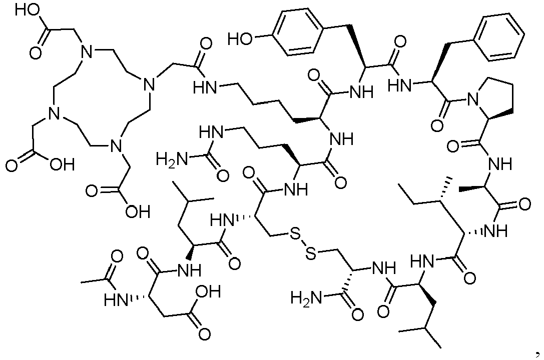
compound Butoc-[Cys-Thr-Orn(DOTA)-Tyr-Phe-4Cfp-Hyp-Ile-Nva-Cys]-NH₂ (UPAR-192) of the following formula 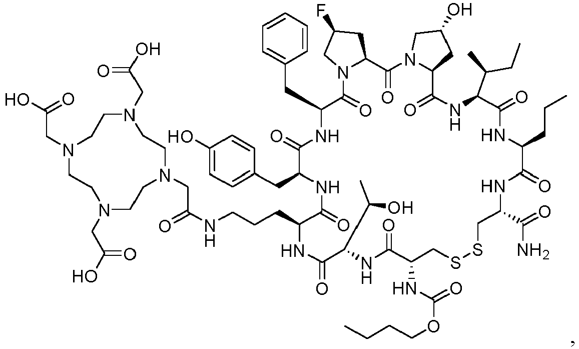
compound Butoc-[Cys-Thr-Lys(DOTA-Kzw)-Dopa-Phe-4Cfp-Hyp-Ile-Nva-Cys]-NH₂ (UPAR-194) of the following formula 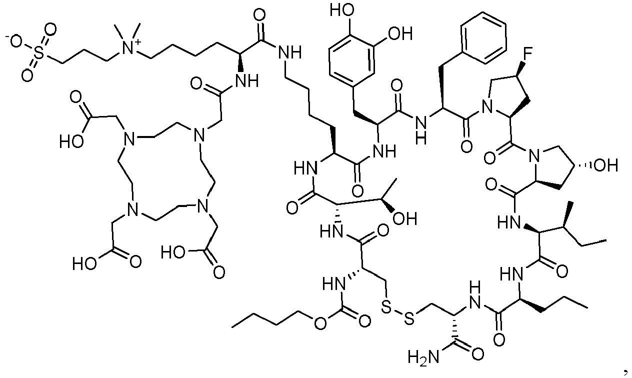
compound Butoc-[Cys-Thr-Lys(DOTA-PPAc)-Dopa-Phe-4Cfp-Hyp-Ile-Nva-Cys]-NH₂ (UPAR-199) of the following formula 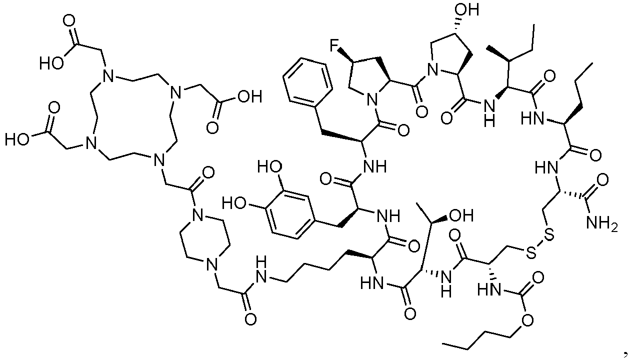
compound Pent-[Cys-Cit-Lys(DOTA-PEG12)-Tyr-Phe-Pro-Hyp-Ile-Nva-Cys]-NH₂ (UPAR-200) of the following formula 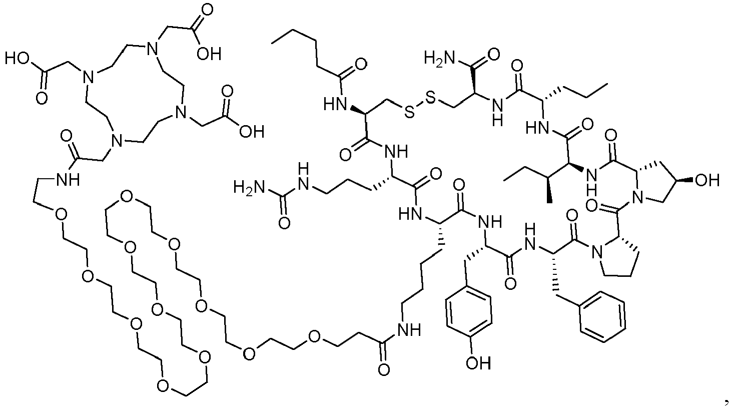
compound PrCAyl-[Cys-Cit-Lys(DOTA)-Tyr-Phe-Pro-Hyp-Ile-Nva-Cys]-NH₂ (UPAR-210) of the following formula 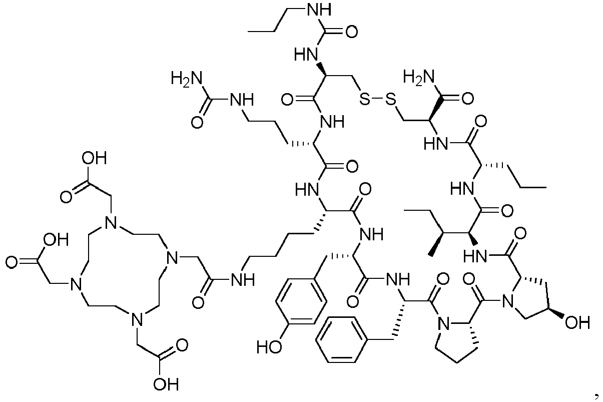
compound Pent-[Cys-Cit-Lys(DOTA)-Tyr-Phe-Pro-Chy-Ile-Nva-Cys]-NH₂ (UPAR-214) of the following formula 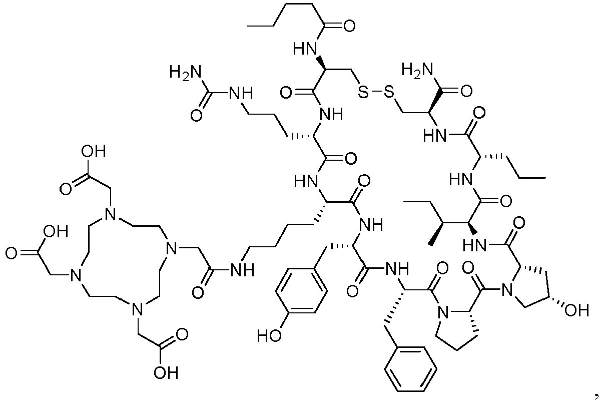
compound Pent-[Cys-Orn(Tris-Nta(Tris'))-Lys(DOTA)-Tyr-Phe-Pro-Hyp-Ile-Nva-Cys]-NH₂ (UPAR-224) of the following formula 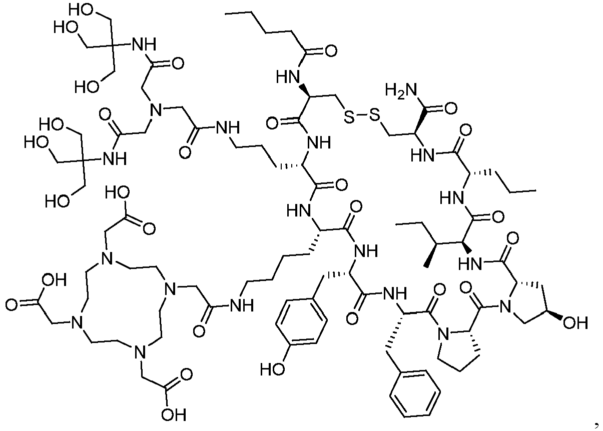
compound Butoc-[Cys-Thr-Dab(DOTA)-Tyr-Phe-4Cfp-Hyp-Ile-Nva-Cys]-NH₂ (UPAR-228) of the following formula 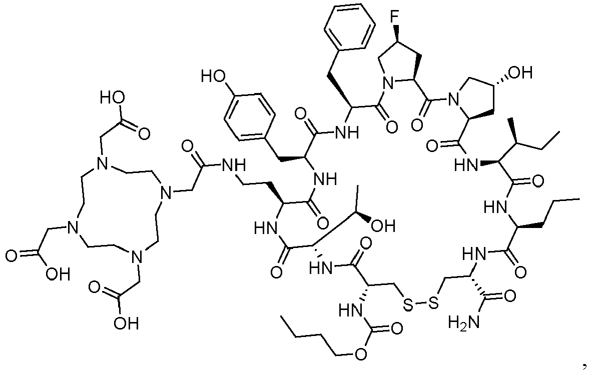
compound DOTA-O2Oc-Leu-[Cys-Cit-Gln-Tyr-Phe-Pro-Pro-Ile-Leu-Cys]-NH₂ (UPAR-229) of the following formula 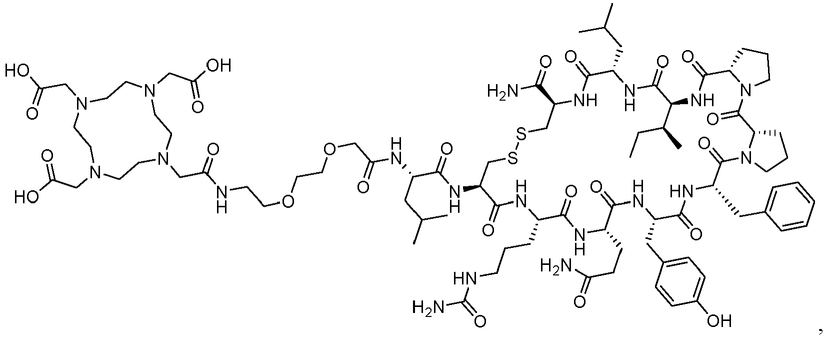
compound Pent-[Cys-Cit-Lys(DOTA)-Tyr-Pcf-Pro-Hyp-Ile-Nva-Cys]-NH₂ (UPAR-233) of the following formula 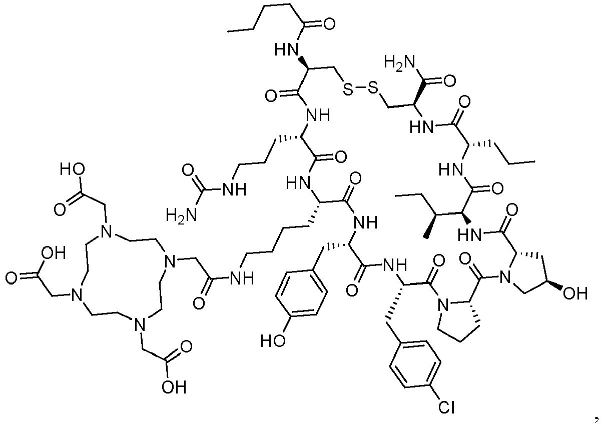
compound Butoc-[Cys-Thr-Orn(DOTA)-Ay3-Phe-4Cfp-Hyp-Ile-Nva-Cys]-NH₂ (UPAR-234) of the following formula 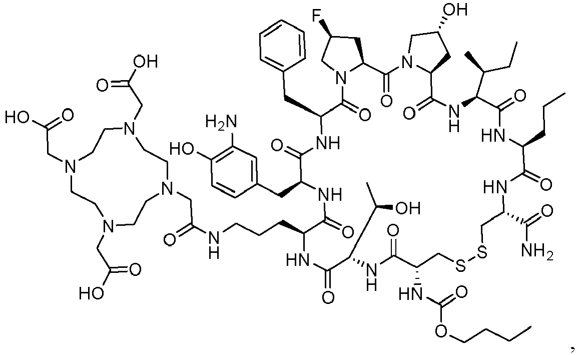
compound Hex-Asp-Leu-[Cys-Lys(DOTA)-Met-Tyr-Phe-Pro-Ala-Ile-Leu-Cys]-NH₂ (UPAR-235) of the following formula 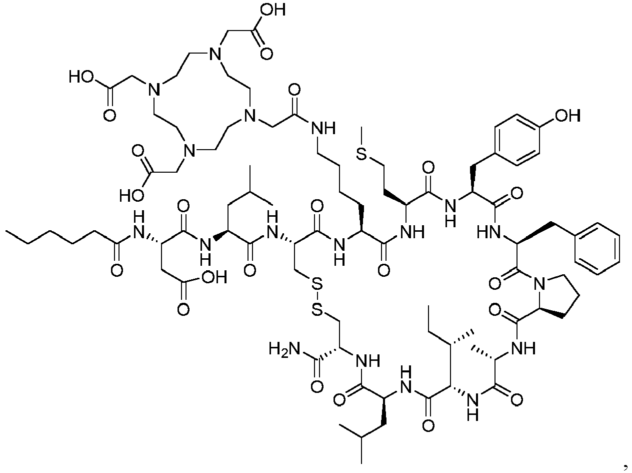
compound Butoc-[Cys-Thr-Lys(DOTA)-Tyr-Phe-4Cfp-Hyp-Ile-Nva-Cys]-NH₂ (UPAR-237) of the following formula 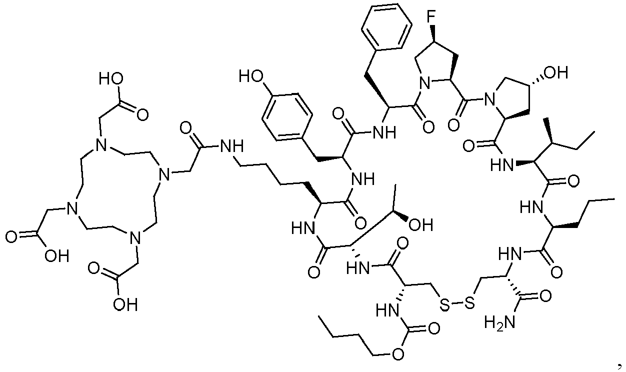
compound iHex-[Cys-Cit-Lys(DOTA)-Tyr-Phe-Pro-Hyp-Ile-Leu-Cys]-NH₂ (UPAR-240) of the following formula 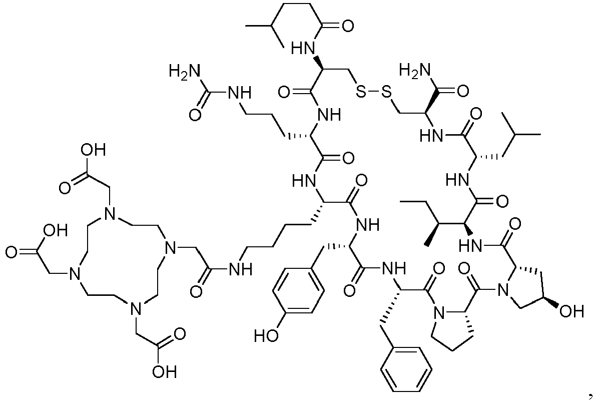
compound nBuCAyl-[Cys-Thr-Lys(DOTA)-Tyr-Phe-Pro-Hyp-Ile-Nva-Cys]-NH₂ (UPAR-242) of the following formula 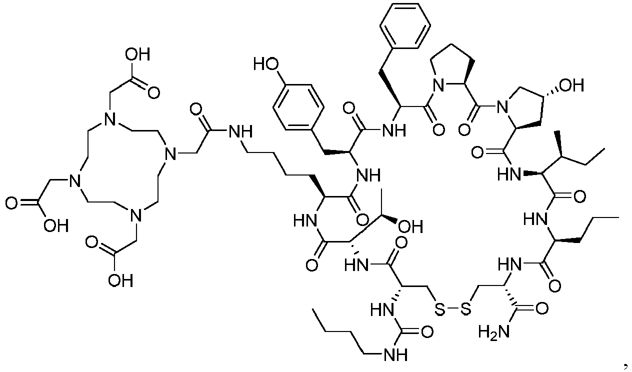
compound Pent-[Cys-Cit-Lys(DOTA)-Tyr-Phe-Pro-4Tfp-Ile-Nva-Cys]-NH₂ (UPAR-246) of the following formula 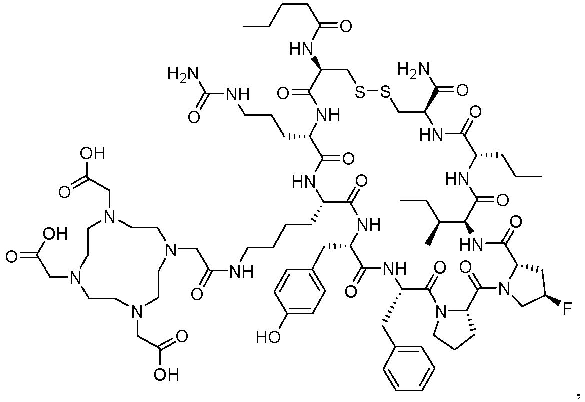
compound Butoc-[Cys-Thr-Lys(DOTA)-Tyr-Phe-Pro-Hyp-Ile-Nva-Cys]-NH₂ (UPAR-248) of the following formula 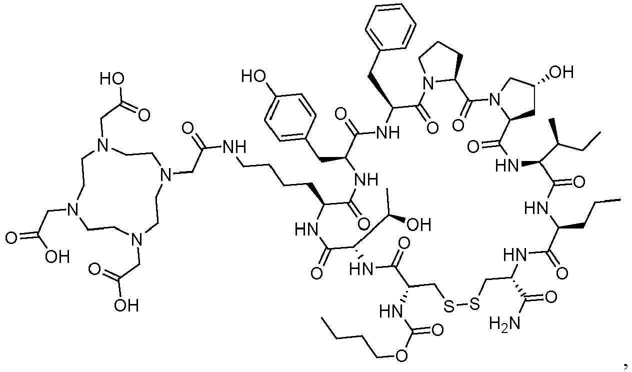
compound Butoc-[Cys-Thr-Lys(DOTA)-Dopa-Phe-4Cfp-Hyp-Ile-Nva-Cys]-NH₂ (UPAR-249) of the following formula 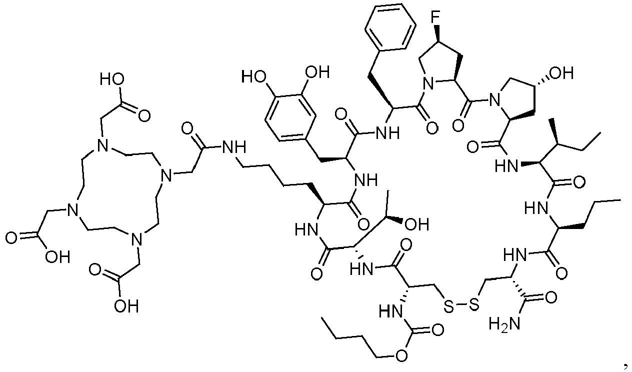
compound Butoc-[Cys-Thr-Lys(NOTA)-Dopa-Phe-4Cfp-Hyp-Ile-Nva-Cys]-NH₂ (UPAR-250) of the following formula 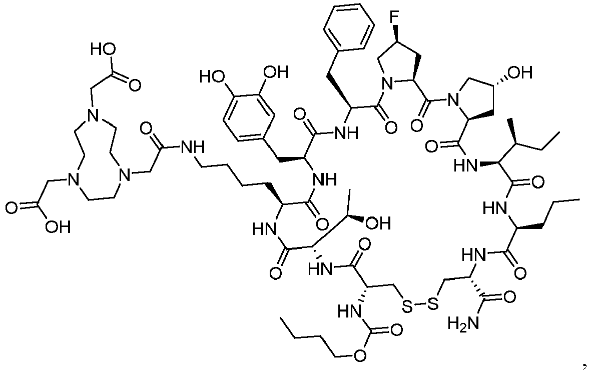
compound iHex-[Cys-Cit-Lys(DOTA)-Tyr-Phe-Pro-ala-Ile-Leu-Cys]-NH₂ (UPAR-256) of the following formula 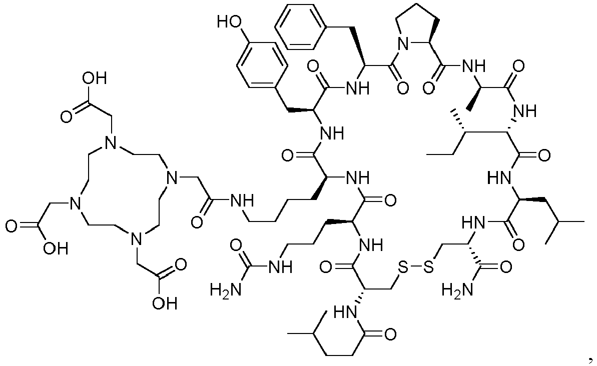
compound Pent-[Cys-Cit-Lys(DOTA)-Nif-Phe-Pro-Hyp-Ile-Nva-Cys]-NH₂ (UPAR-257) of the following formula 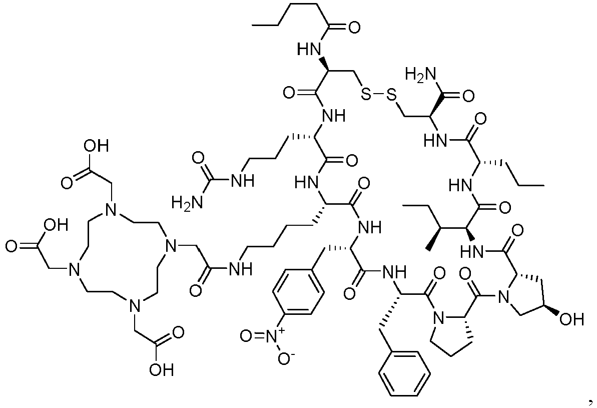
compound Pent-[Cys-Cit-Lys(DOTA)-Tyr-Phe-Pro-Hyp-Ile-Nva-Cys]-NH₂ (UPAR-261) of the following formula 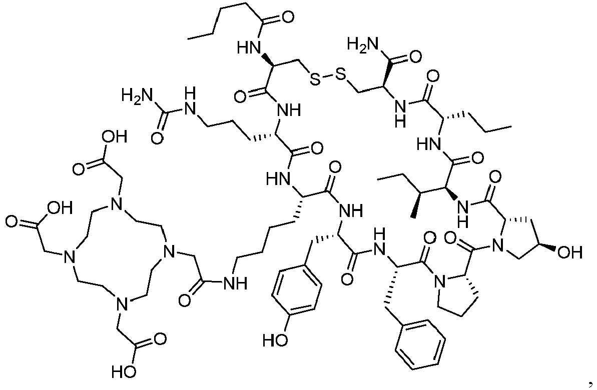
compound Butoc-[Cys-Cit-Lys(DOTA)-Tyr-Phe-Pro-Hyp-Ile-Nva-Cys]-NH₂ (UPAR-275) of the following formula 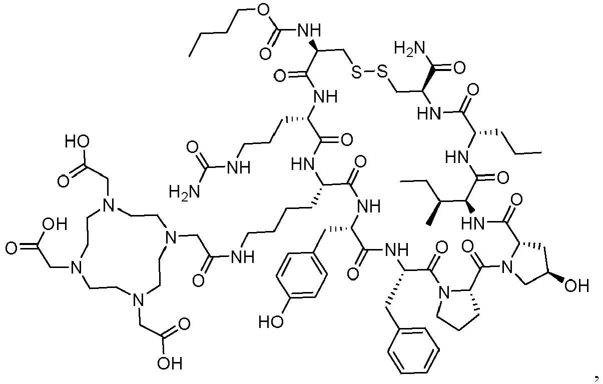
compound Butoc-[Cys-Thr-Orn(DOTA)-Pcnf-Phe-4Cfp-Hyp-Ile-Nva-Cys]-NH₂ (UPAR-276) of the following formula 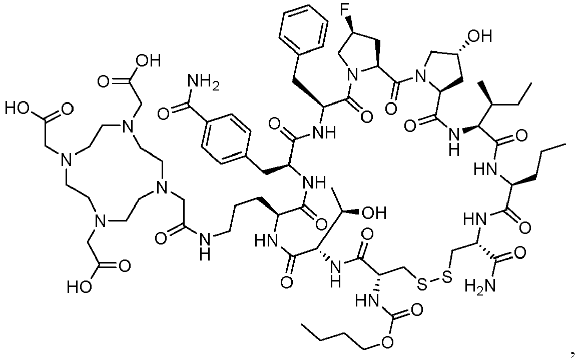
compound Pent-[Cys-Har-Lys(DOTA)-Tyr-Phe-Pro-Hyp-Ile-Nva-Cys]-NH₂ (UPAR-278) of the following formula 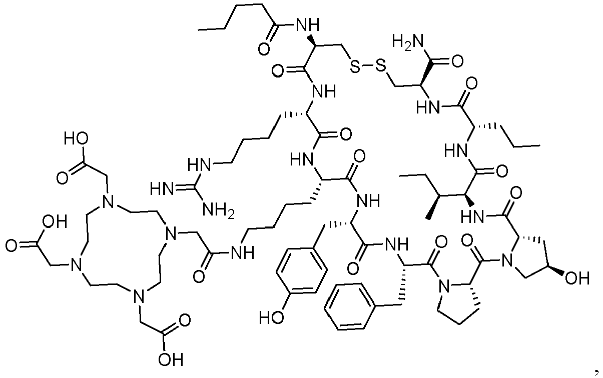
compound Butoc-[Cys-Thr-Orn(DOTA)-Dopa-Phe-4Cfp-Hyp-Ile-Nva-Cys]-NH₂ (UPAR-280) of the following formula 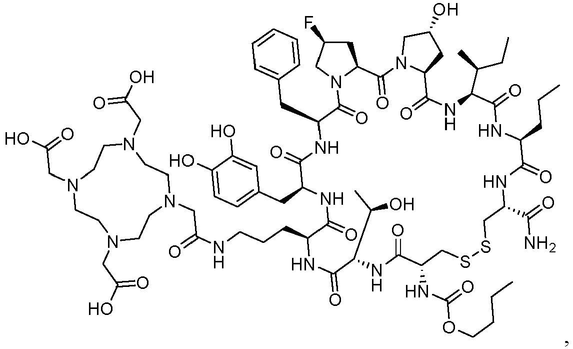
compound Pent-[Cys-Asn-Lys(DOTA)-Tyr-Phe-Pro-Hyp-Ile-Nva-Cys]-NH₂ (UPAR-283) of the following formula 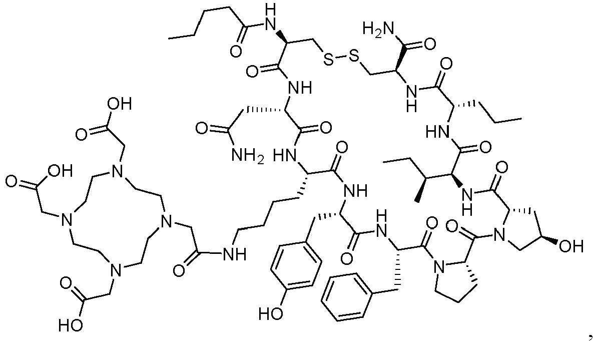
compound Butoc-[Cys-Thr-Lys(DOTA)-Dopa-Phe-Hyp-Hyp-Ile-Nva-Cys]-NH₂ (UPAR-284) of the following formula 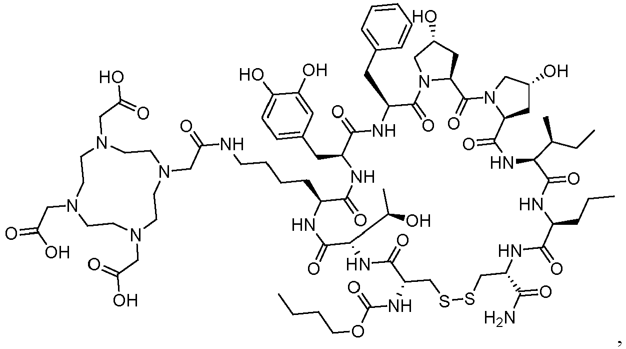
compound Butoc-[Cys-Thr-Lys(DOTA)-Tyr-Phe-Pro-4Tfp-Ile-Nva-Cys]-NH₂ (UPAR-289) of the following formula 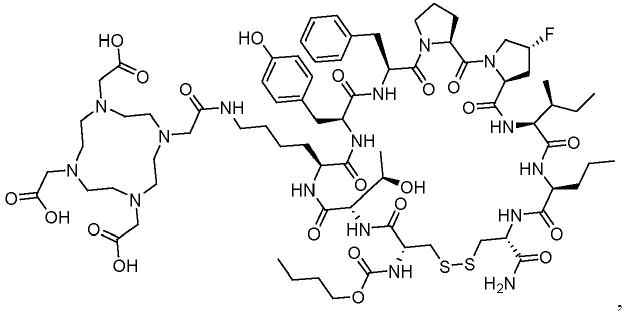
compound DOTA-Ahx-Leu-[Cys-Arg-Gln-Tyr-Phe-Pro-ala-Ile-Leu-Cys]-NH₂ (UPAR-291) of the following formula 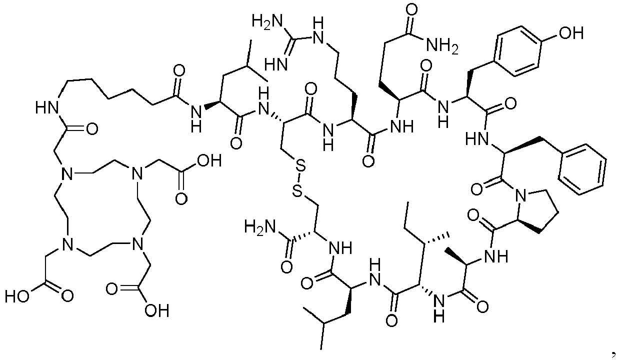
compound HO-Succinyl-Nva-[Cys-Cit-Lys(DOTA)-Tyr-Phe-Pro-Hyp-Ile-Nva-Cys]-NH₂ (UPAR-293) of the following formula 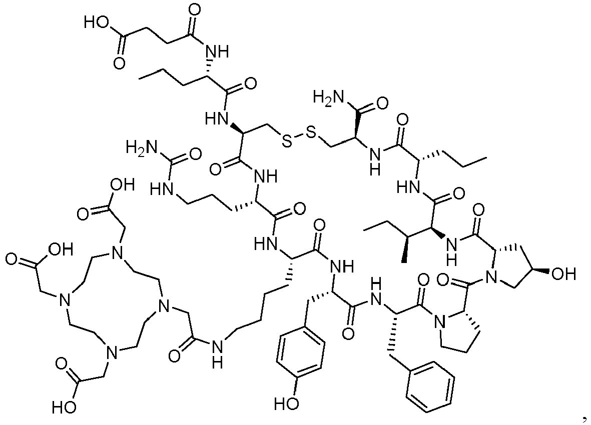
compound Succinyl-Nva-[Cys-Thr-Lys(DOTA)-Tyr-Phe-Pro-Hyp-Ile-Nva-Cys]-NH₂ (UPAR-297) of the following formula 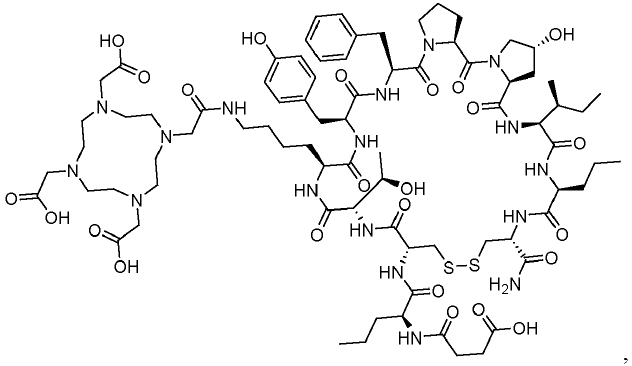
compound Pent-[Cys-Cit-Lys(DOTA)-Tyr-Phe-Pro-Aib-Ile-Nva-Cys]-NH₂ (UPAR-299) of the following formula 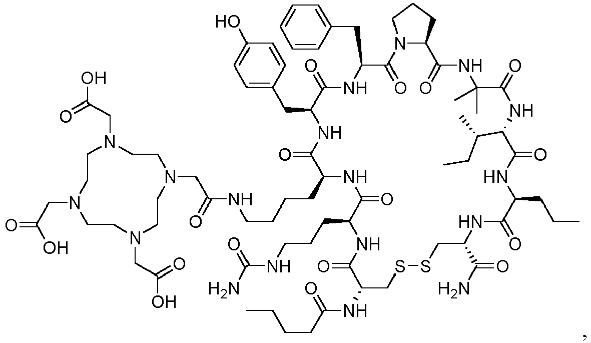
compound Pent-[Cys-Thr-Lys(DOTA)-Tyr-Phe-Pro-Hyp-Ile-Nva-Cys]-NH₂ (UPAR-301) of the following formula 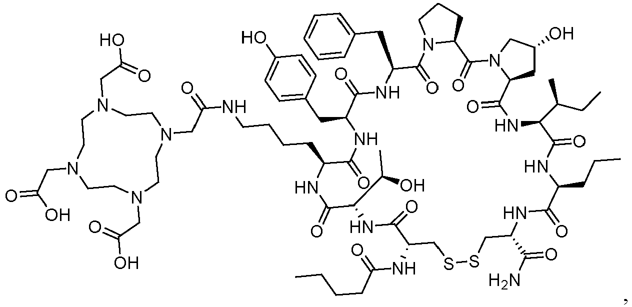
compound Butoc-[Cys-Cit-Lys(DOTA)-Tyr-Pcf-4Cfp-Hyp-Ile-Nva-Cys]-NH₂ (UPAR-304) of the following formula 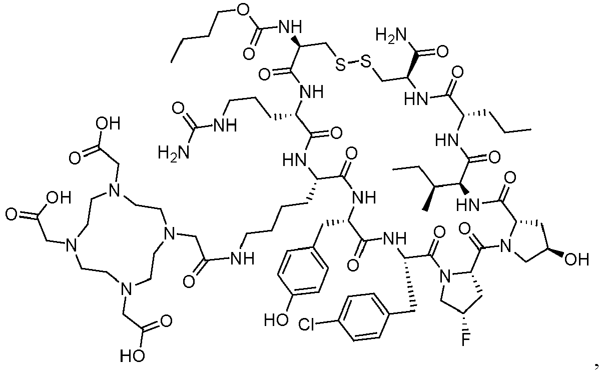
compound Pent-[Cys-Cit-Lys(DOTA)-Tyr-Egm-Pro-Hyp-Ile-Nva-Cys]-NHz (UPAR-315) of the following formula 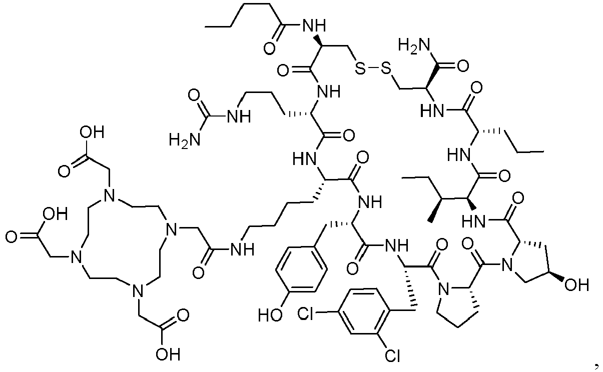
compound Pent-[Cys-Cit-Lys(DOTA)-Aph-Phe-Pro-Hyp-Ile-Nva-Cys]-NH₂ (UPAR-320) of the following formula 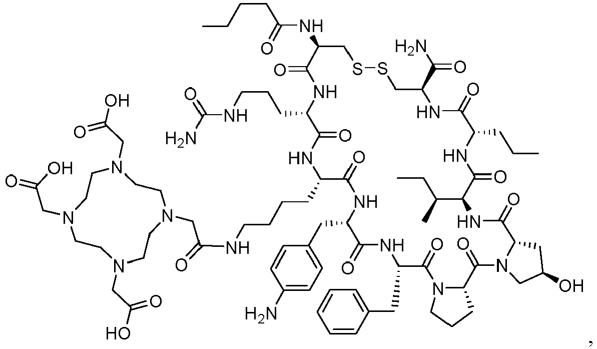
compound Pent-[Cys-Cit-Lys(DOTA)-Tyr-Phe-Pro-4Ap-Ile-Nva-Cys]-NH₂ (UPAR-325) of the following formula 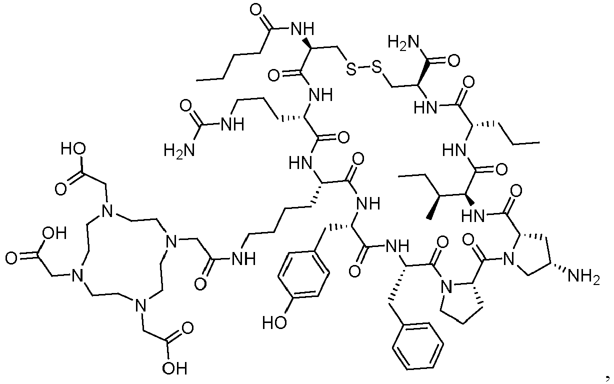
compound Pent-[Cys-Cit-Lys(DOTA)-Ppa-Phe-Pro-Hyp-Ile-Nva-Cys]-NH₂ (UPAR-336) of the following formula 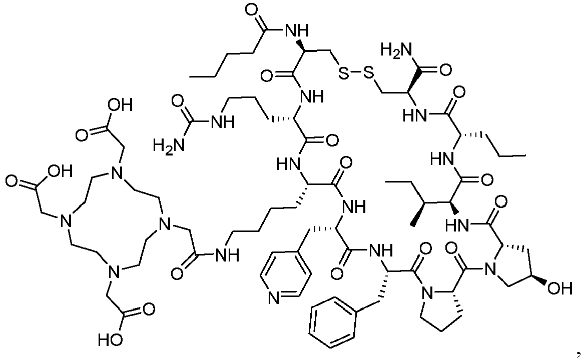
compound Pent-[Cys-Ser-Lys(DOTA)-Tyr-Phe-Pro-Hyp-Ile-Nva-Cys]-NH₂ (UPAR-342) of the following formula 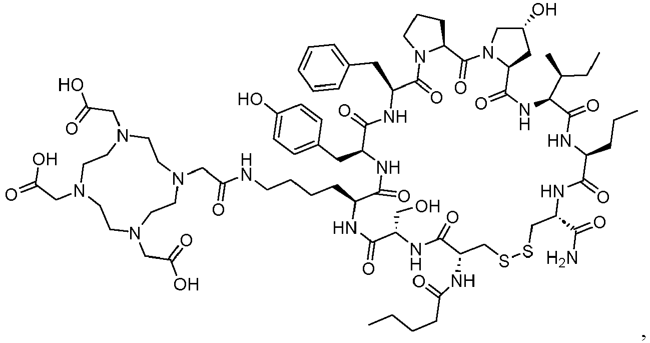
compound nBu-CAyl-[Cys-Cit-Lys(DOTA)-Tyr-Phe-Pro-Hyp-Ile-Nva-Cys]-NH₂ (UPAR-346) of the following formula 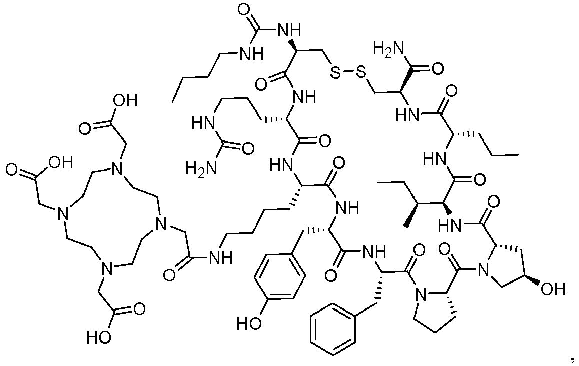
compound DOTA-Ttds-Nle-Asp-Leu-[Cys-Arg-Met-Tyr-Phe-Pro-Ala-Ile-Leu-Cys]-NH₂ (UPAR-347) of the following formula 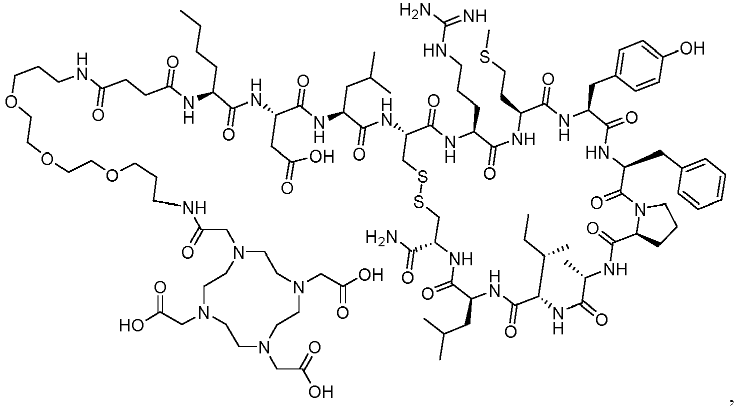
compound Hex-Asp-Leu-[Cys-Arg-Met-Tyr-Phe-Pro-Lys(DOTA)-Ile-Leu-Cys]-NH₂ (UPAR-351) of the following formula 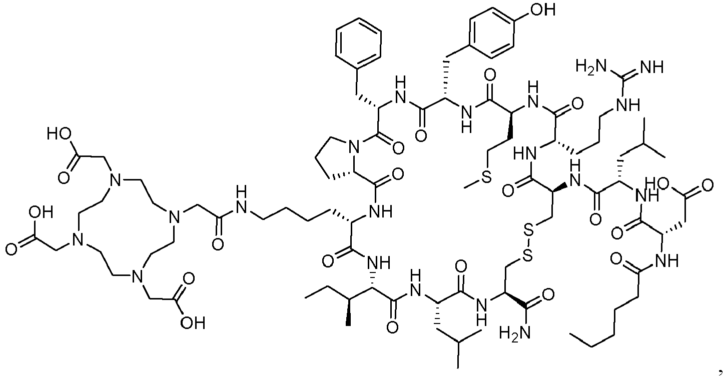
compound Pent-[Cys-Cit-Lys(DOTA)-Tyr-Thi-Pro-Hyp-Ile-Nva-Cys]-NH₂ (UPAR-360) of the following formula 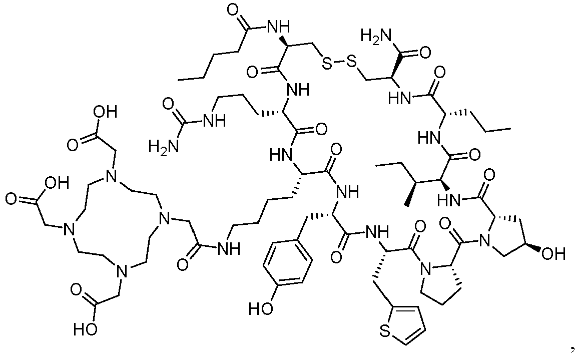
compound Pent-[Cys-Cit-Lys(DOTA)-Tyr-Phe-4Cfp-Hyp-Ile-Nva-Cys]-NH₂ (UPAR-365) of the following formula 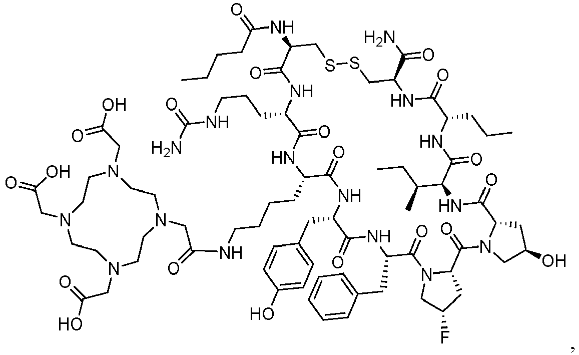
compound Pent-[Cys-Cit-Lys(DOTA)-Tyr-Phe-Pro-Tap-Ile-Nva-Cys]-NH₂ (UPAR-377) of the following formula 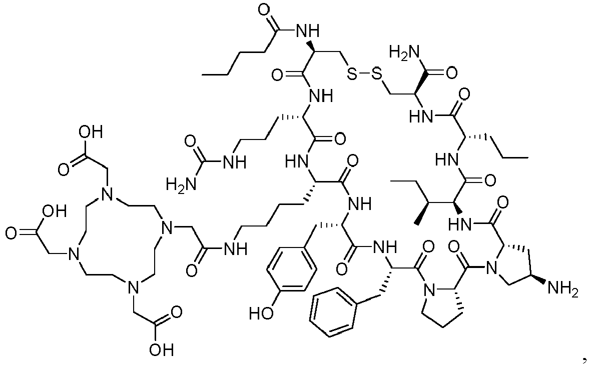
compound Butoc-[Cys-Thr-Lys(DOTA)-Tyr-Pnf-4Cfp-Hyp-Ile-Nva-Cys]-NH₂ (UPAR-378) of the following formula 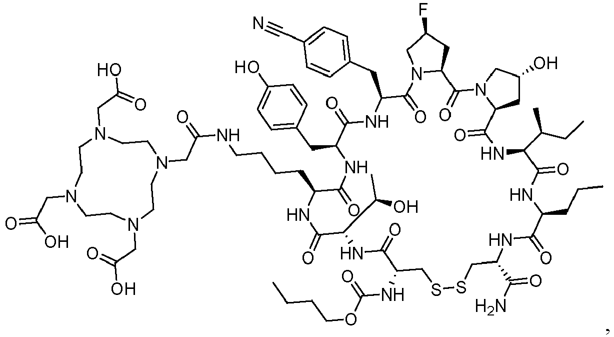
compound iHex-[Cys-Cit-Lys(DOTA)-Tyr-Phe-Pro-Pro-Ile-Leu-Cys]-NHz (UPAR-382) of the following formula 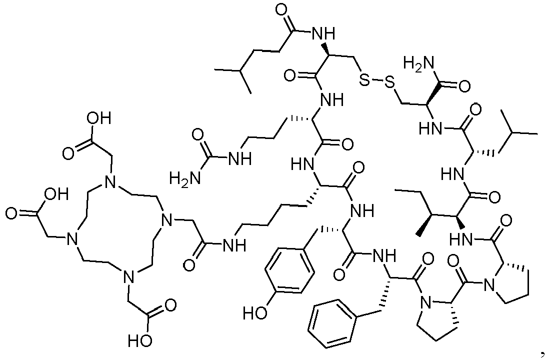
compound Pent-[Cys-Orn(mPEG12)-Lys(DOTA)-Tyr-Phe-Pro-Hyp-Ile-Nva-Cys]-NH₂ (UPAR-383) of the following formula 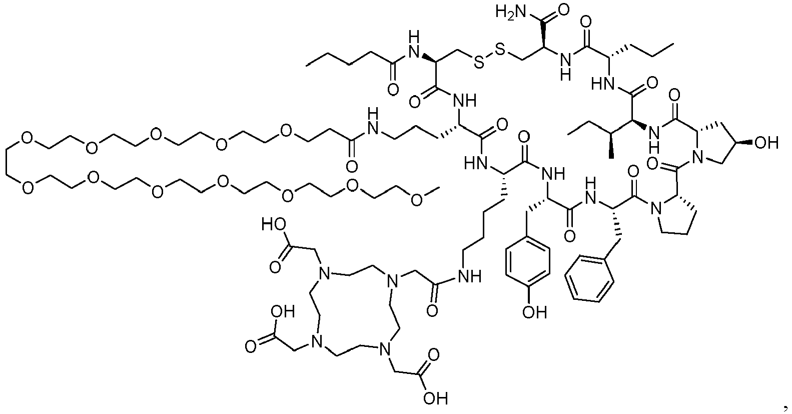
compound Pent-[Cys-Cit-Lys(DOTA)-Tyr-Phe-Pro-nma-Ile-Nva-Cys]-NH₂ (UPAR-384) of the following formula 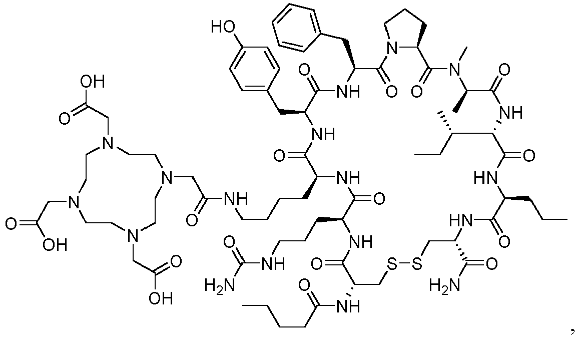
compound iHex-[Cys-Cit-Gln-Tyr-Phe-Pro-Tap(DOTA)-Ile-Leu-Cys]-NH₂ (UPAR-389) of the following formula 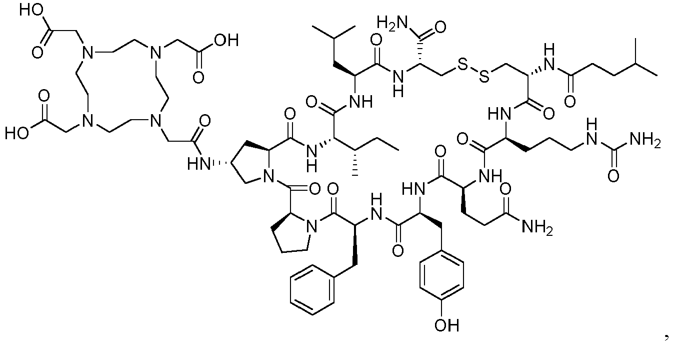
compound Pent-[Cys-Cit-Lys(DOTA)-Tyr-Phe-Pro-Pro-Ile-Leu-Cys]-NH₂ (UPAR-396) of the following formula 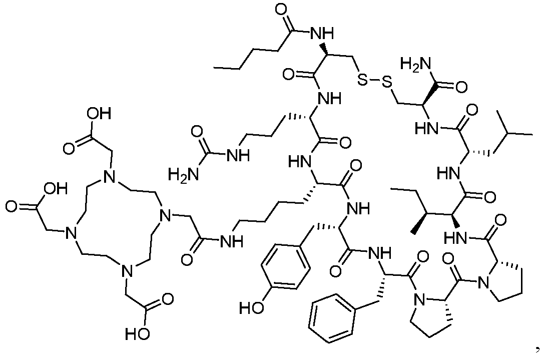
compound Pent-[Cys-Cit-Lys(DOTA)-Dopa-Phe-Pro-Hyp-Ile-Nva-Cys]-NH₂ (UPAR-399) of the following formula 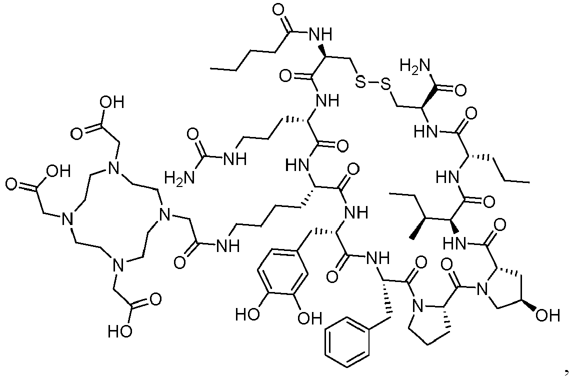
compound Pent-[Cys-Cit-Lys(DOTA)-Tyr-Phe-Hyp-Hyp-Ile-Nva-Cys]-NH₂ (UPAR-403) of the following formula 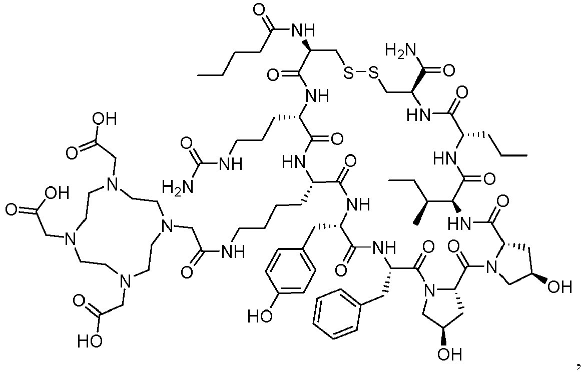
compound Butoc-[Cys-Thr-Orn(DOTA)-My-Phe-4Cfp-Hyp-Ile-Nva-Cys]-NH₂ (UPAR-415) of the following formula 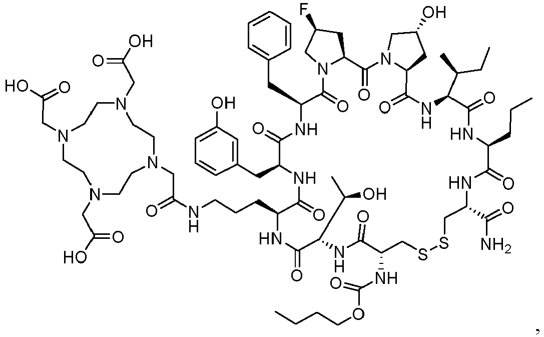
compound Butoc-[Cys-Thr-Lys(DOTA-Kzw)-Tyr-Phe-4Cfp-Hyp-Ile-Nva-Cys]-NH₂ (UPAR-422) of the following formula 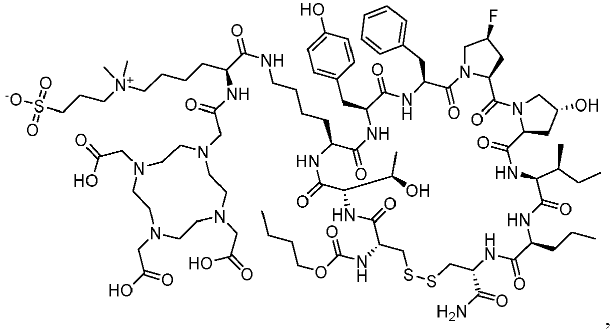
compound Ac-Asp-Leu-[Cys-Cit-Lys(DOTA)-Tyr-Phe-Pro-Arg-Ile-Leu-Cys]-NH₂ (UPAR-423) of the following formula 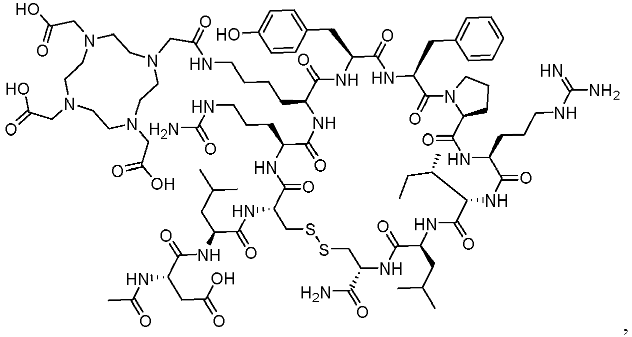
compound Butoc-[Cys-Thr-Lys(DOTA-asp)-Tyr-Phe-4Cfp-Hyp-Ile-Nva-Cys]-NH₂ (UPAR-424) of the following formula 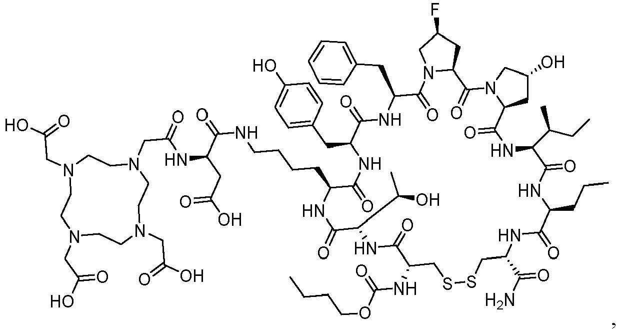
compound Hex-Asp-Leu-[Cys-Arg-Lys(DOTA)-Tyr-Phe-Pro-Ala-Ile-Leu-Cys]-NH₂ (UPAR-427) of the following formula 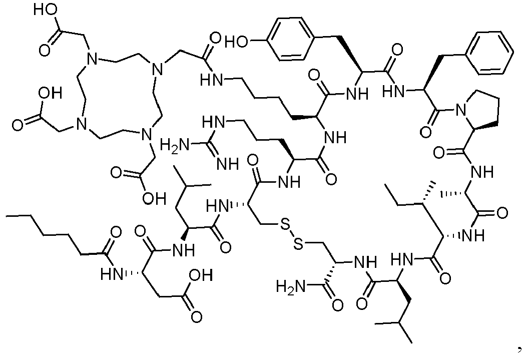
compound H-Nmb-[Cys-Thr-Lys(DOTA)-Tyr-Phe-4Cfp-Hyp-Ile-Nva-Cys]-NH₂ (UPAR-428) of the following formula 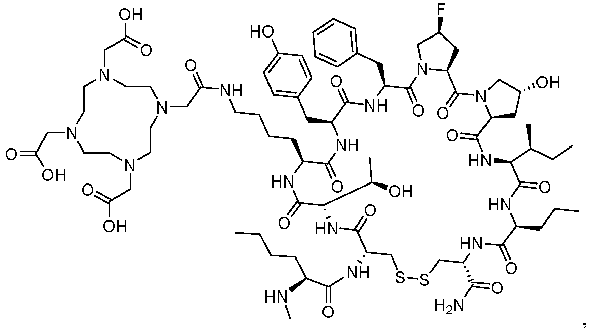
compound Butoc-[Cys-Thr-Lys(NODAGA)-Dopa-Phe-4Cfp-Hyp-Ile-Nva-Cys]-NH₂ (UPAR-444) of the following formula 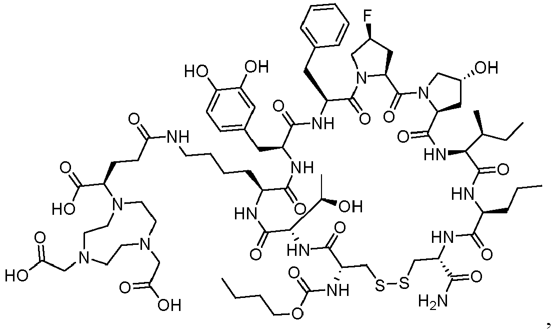
compound Pent-[Cys-Cit-Lys(DOTA)-Tyr-Ocf-Pro-Hyp-Ile-Nva-Cys]-NH₂ (UPAR-450) of the following formula 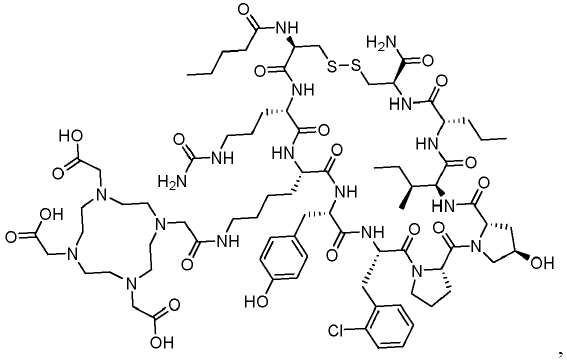
compound iHex-[Cys-Cit-Lys(DOTA)-Tyr-Phe-Pro-Aib-Ile-Leu-Cys]-NH₂ (UPAR-452) of the following formula 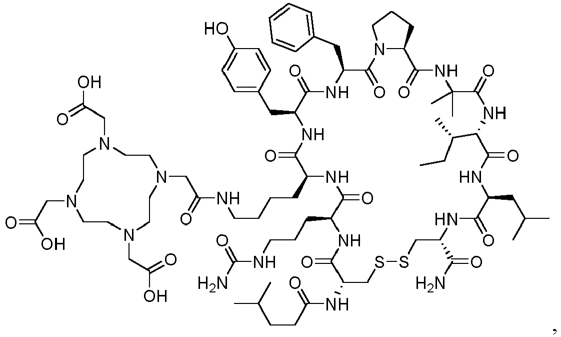
compound Butoc-[Cys-Thr-Dap(DOTA)-Tyr-Phe-4Cfp-Hyp-Ile-Nva-Cys]-NH₂ (UPAR-455) of the following formula 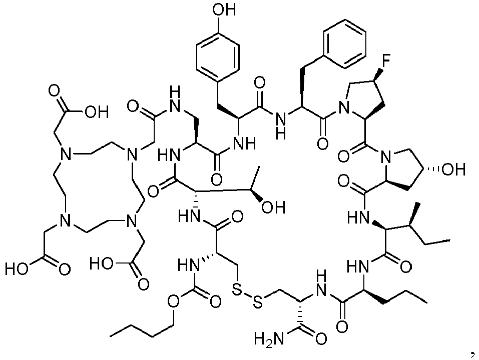
compound Butoc-[Cys-Thr-Dab(DOTA)-Dopa-Phe-4Cfp-Hyp-Ile-Nva-Cys]-NH₂ (UPAR-456) of the following formula 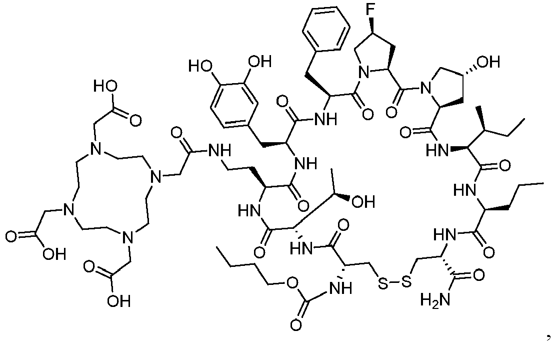
compound Butoc-[Cys-Thr-Lys(DOTA-asp)-Dopa-Phe-4Cfp-Hyp-Ile-Nva-Cys]-NH₂ (UPAR-459) of the following formula 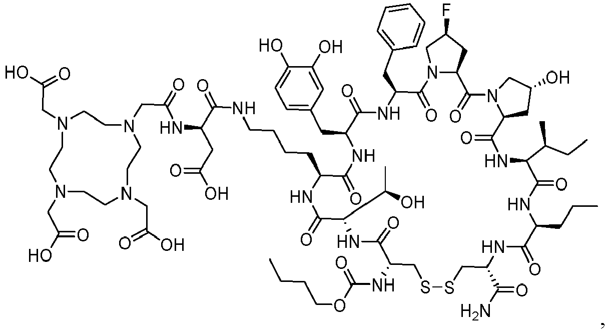
compound iHex-[Cys-Lys(DOTA)-Gln-Tyr-Phe-Pro-Pro-Ile-Leu-Cys]-NH₂ (UPAR-466) of the following formula 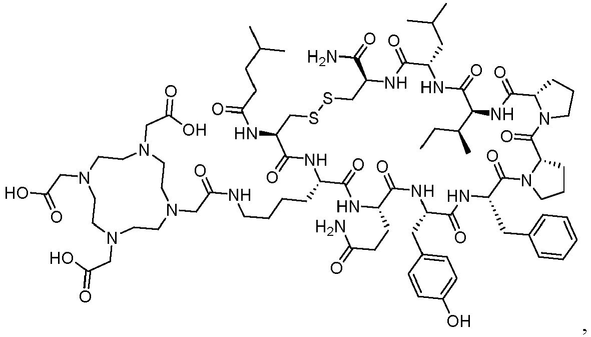
compound Pent-[Cys-Glu-Lys(DOTA)-Tyr-Phe-Pro-Hyp-Ile-Nva-Cys]-NH₂ (UPAR-471) of the following formula 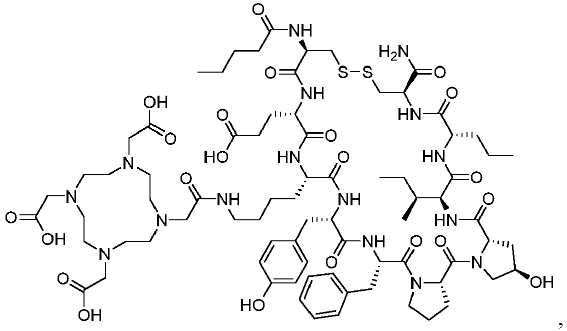
compound Pent-[Cys-Cit-Lys(DOTA)-Tyr-Phe-Pro-Pro-Ile-Nva-Cys]-NH₂ (UPAR-473) of the following formula 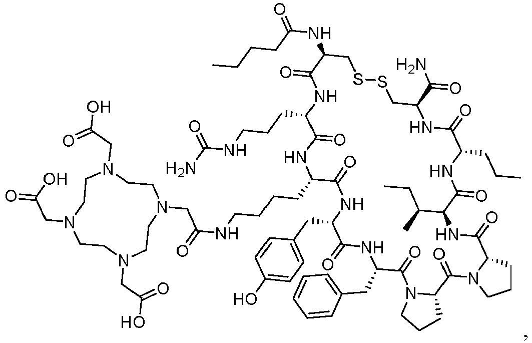
compound Butoc-[Cys-Cit-Lys(DOTA)-Tyr-Pcf-4Cfp-Hyp-Chg-Nva-Cys]-NH₂ (UPAR-476) of the following formula 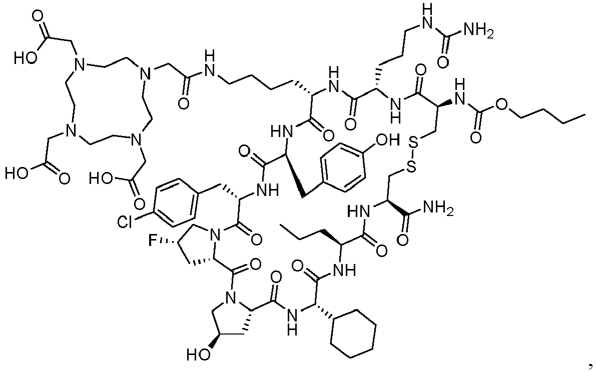
or a pharmaceutically acceptable salt or hydrate or pharmaceutically acceptable solvate thereof.

Embodiment 221. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214, 215, 216, 217, 218, 219 and 220, any one of Embodiments 1, 2, 3, 4, 5, 6, 7, 8, 9, 10, 11, 12, 13 and 14, or any one of Embodiments 1, 2 and 3, wherein the compound is selected from the group consisting of
compound Butoc-[Cys-Thr-Lys(DOTA)-Tyr-Phe-Hyp-Hyp-Ile-Nva-Cys]-NH₂ (UPAR-128) of the following formula 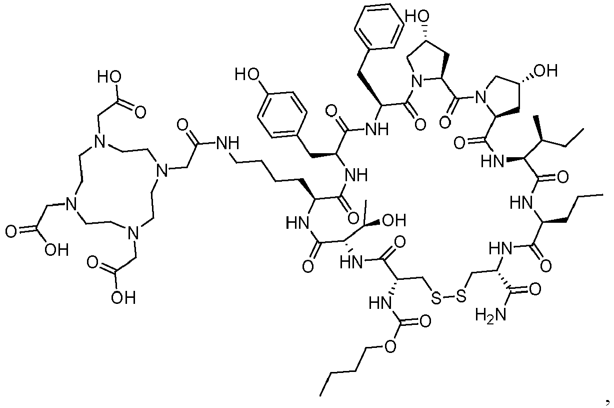
compound Butoc-[Cys-Thr-Lys(DOTA)-Tyr-Thi-Pro-Hyp-Ile-Nva-Cys]-NH₂ (UPAR-134) of the following formula 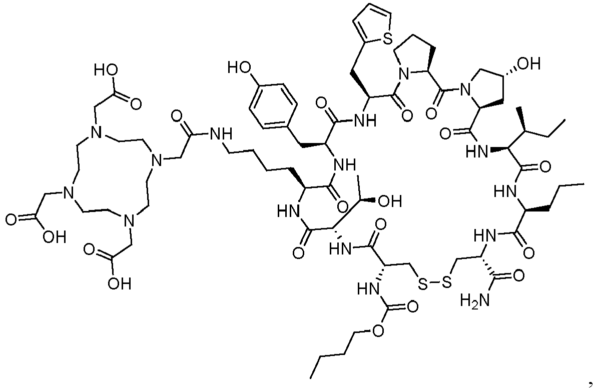
compound Butoc-[Cys-Thr-Lys(DOTA)-Mpa-Phe-4Cfp-Hyp-Ile-Nva-Cys]-NH₂ (UPAR-165) of the following formula 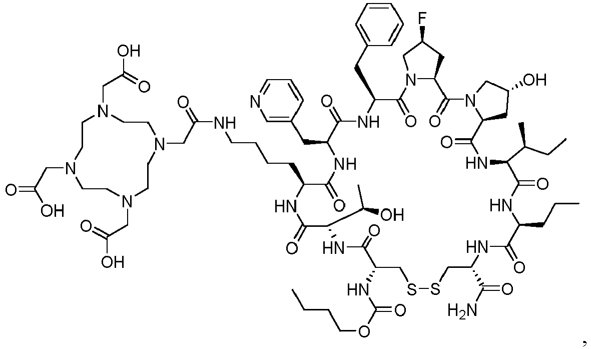
compound Butoc-[Cys-Thr-Lys(DOTA-PPAc)-Tyr-Phe-4Cfp-Hyp-Ile-Nva-Cys]-NH₂ (UPAR-181) of the following formula 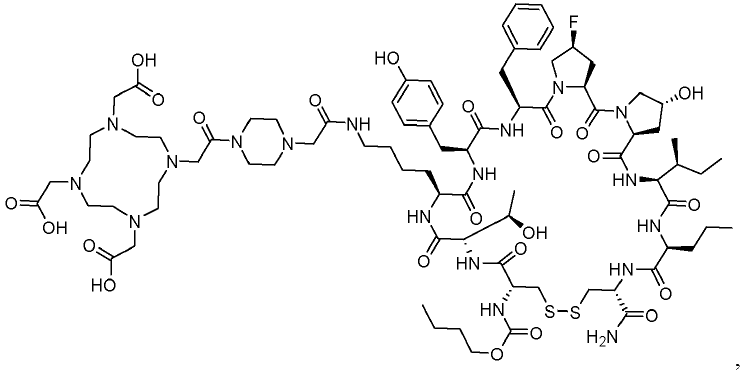
compound Butoc-[Cys-Thr-Orn(DOTA)-Tyr-Phe-4Cfp-Hyp-Ile-Nva-Cys]-NH₂ (UPAR-192) of the following formula 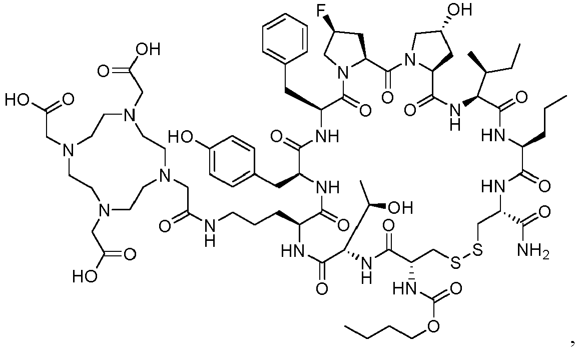
compound Butoc-[Cys-Thr-Lys(DOTA-Kzw)-Dopa-Phe-4Cfp-Hyp-Ile-Nva-Cys]-NH₂ (UPAR-194) of the following formula 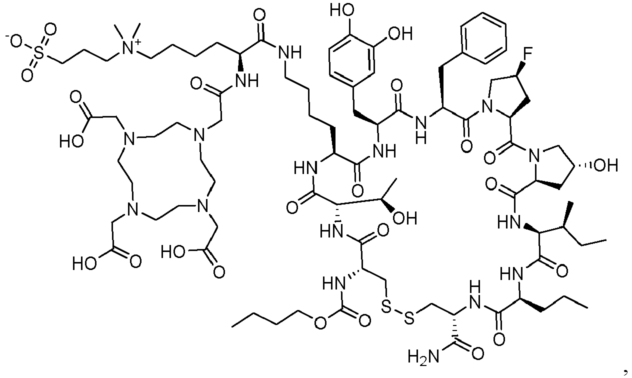
compound Butoc-[Cys-Thr-Lys(DOTA-PPAc)-Dopa-Phe-4Cfp-Hyp-Ile-Nva-Cys]-NH₂ (UPAR-199) of the following formula 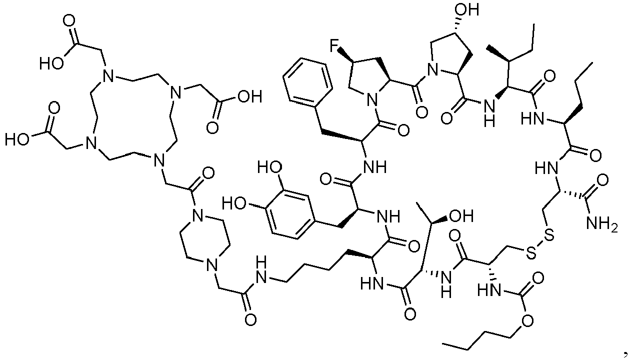
compound Butoc-[Cys-Thr-Dab(DOTA)-Tyr-Phe-4Cfp-Hyp-Ile-Nva-Cys]-NH₂ (UPAR-228) of the following formula 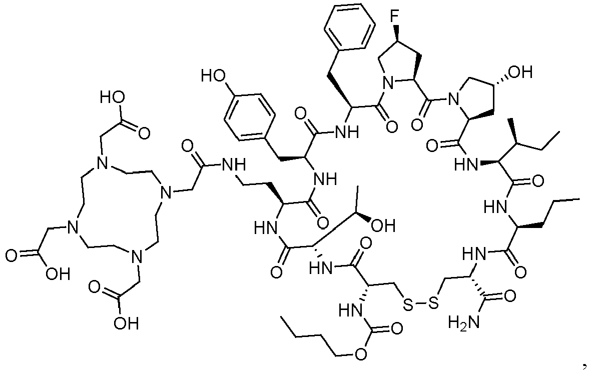
compound DOTA-O2Oc-Leu-[Cys-Cit-Gln-Tyr-Phe-Pro-Pro-Ile-Leu-Cys]-NH₂ (UPAR-229) of the following formula 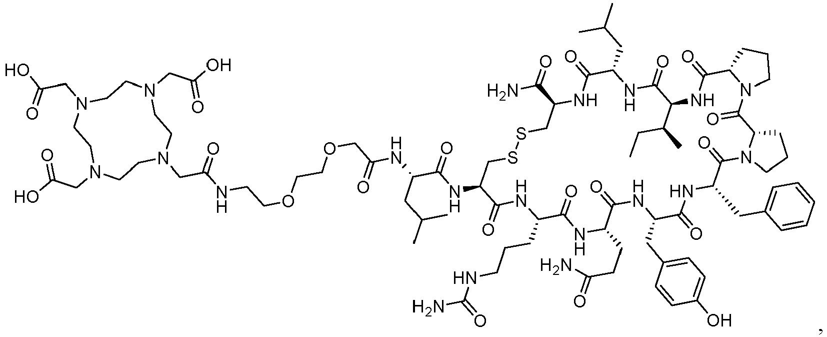
compound Butoc-[Cys-Thr-Lys(DOTA)-Tyr-Phe-4Cfp-Hyp-Ile-Nva-Cys]-NH₂ (UPAR-237) of the following formula 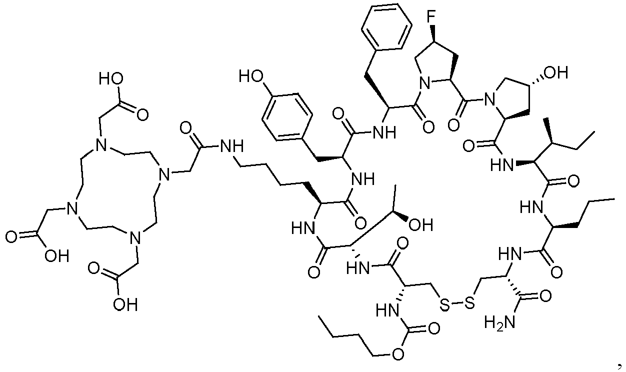
compound iHex-[Cys-Cit-Lys(DOTA)-Tyr-Phe-Pro-Hyp-Ile-Leu-Cys]-NH₂ (UPAR-240) of the following formula 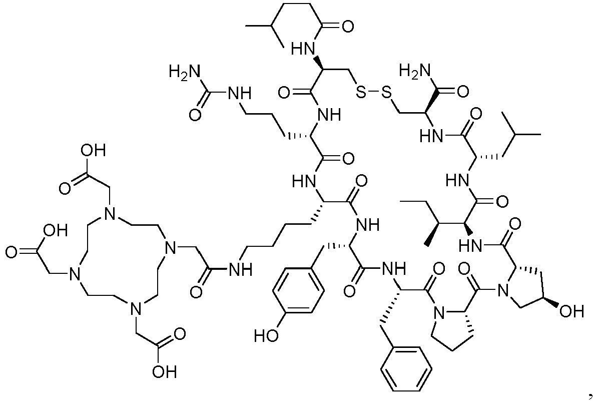
compound Pent-[Cys-Cit-Lys(DOTA)-Tyr-Phe-Pro-4Tfp-Ile-Nva-Cys]-NH₂ (UPAR-246) of the following formula 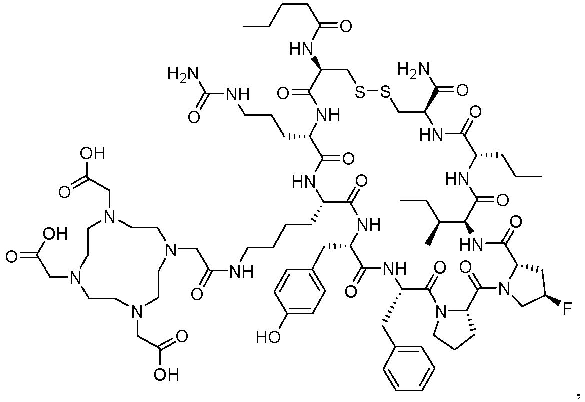
compound Butoc-[Cys-Thr-Lys(DOTA)-Dopa-Phe-4Cfp-Hyp-Ile-Nva-Cys]-NH₂ (UPAR-249) of the following formula 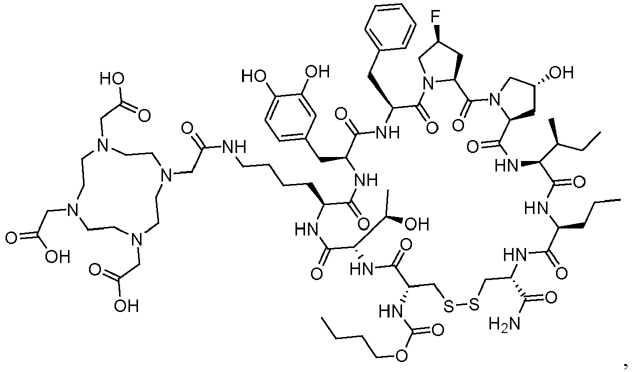
compound Butoc-[Cys-Thr-Lys(NOTA)-Dopa-Phe-4Cfp-Hyp-Ile-Nva-Cys]-NH₂ (UPAR-250) of the following formula 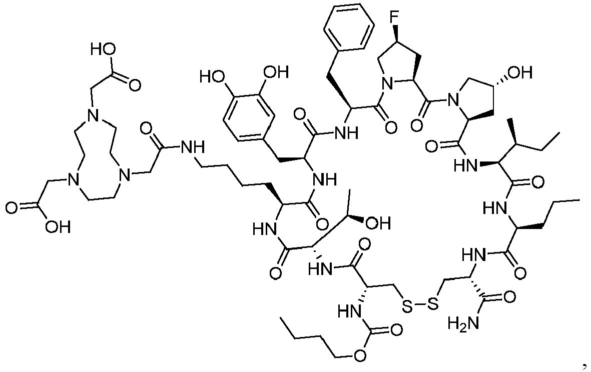
compound Pent-[Cys-Cit-Lys(DOTA)-Tyr-Phe-Pro-Hyp-Ile-Nva-Cys]-NH₂ (UPAR-261) of the following formula 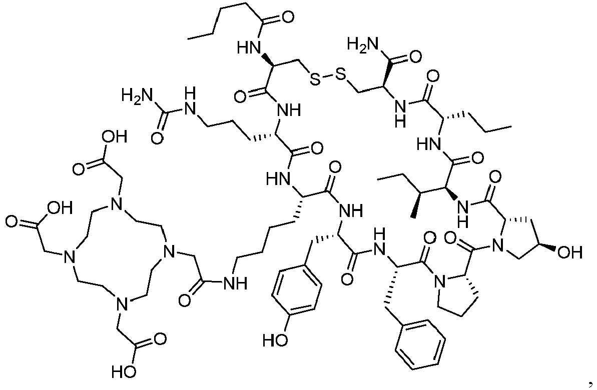
compound Butoc-[Cys-Cit-Lys(DOTA)-Tyr-Phe-Pro-Hyp-Ile-Nva-Cys]-NH₂ (UPAR-275) of the following formula 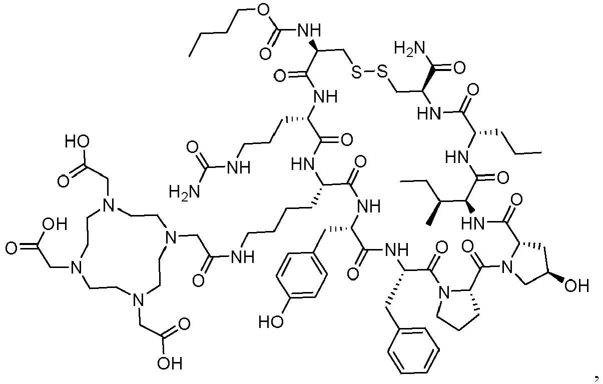
compound Butoc-[Cys-Thr-Orn(DOTA)-Pcnf-Phe-4Cfp-Hyp-Ile-Nva-Cys]-NH₂ (UPAR-276) of the following formula 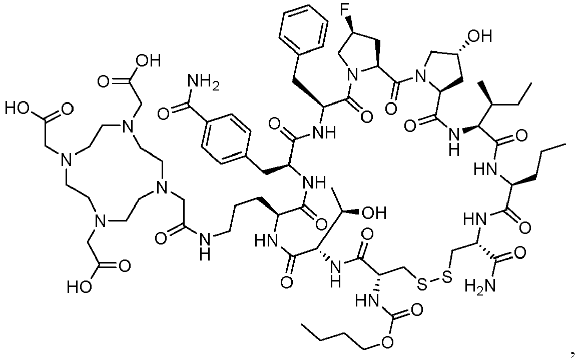
compound Pent-[Cys-Har-Lys(DOTA)-Tyr-Phe-Pro-Hyp-Ile-Nva-Cys]-NH₂ (UPAR-278) of the following formula 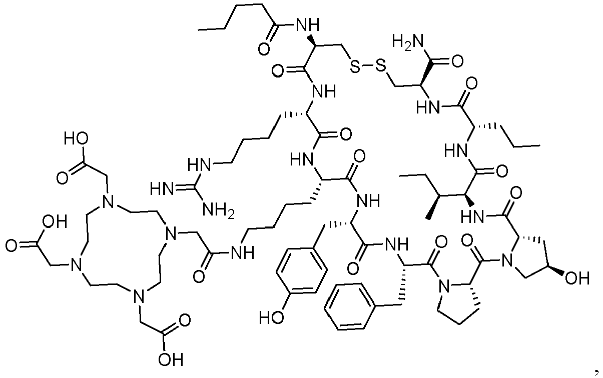
compound Butoc-[Cys-Thr-Orn(DOTA)-Dopa-Phe-4Cfp-Hyp-Ile-Nva-Cys]-NH₂ (UPAR-280) of the following formula 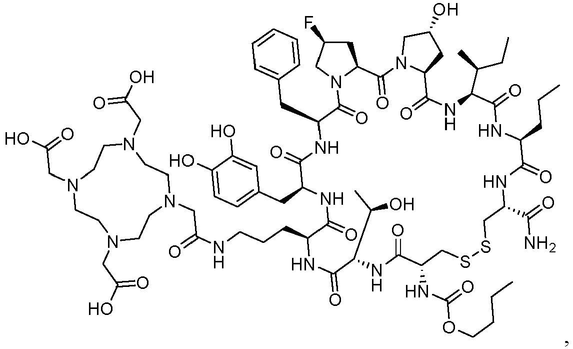
compound Butoc-[Cys-Thr-Lys(DOTA)-Tyr-Phe-Pro-4Tfp-Ile-Nva-Cys]-NH₂ (UPAR-289) of the following formula 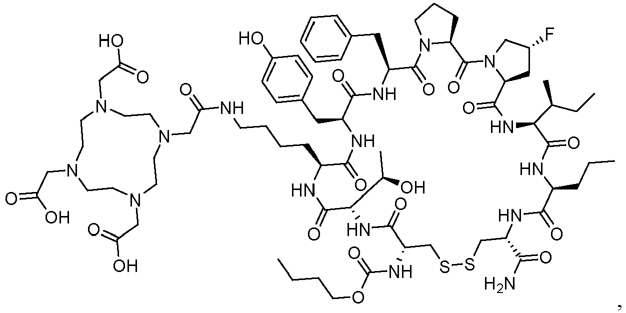
compound Pent-[Cys-Thr-Lys(DOTA)-Tyr-Phe-Pro-Hyp-Ile-Nva-Cys]-NH₂ (UPAR-301) of the following formula 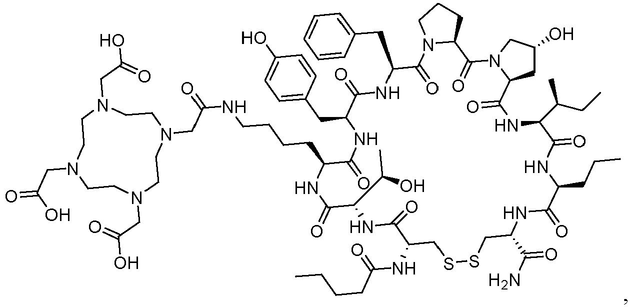
compound Butoc-[Cys-Cit-Lys(DOTA)-Tyr-Pcf-4Cfp-Hyp-Ile-Nva-Cys]-NH₂ (UPAR-304) of the following formula 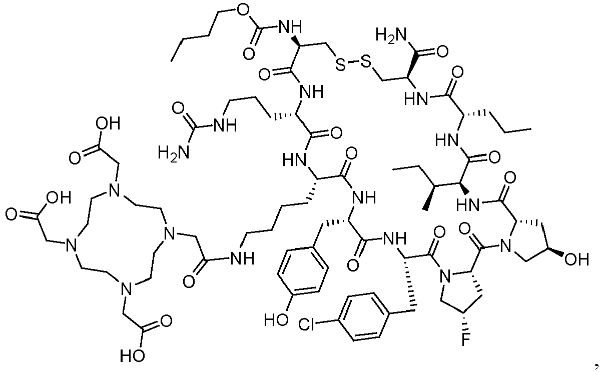
compound nBu-CAyl-[Cys-Cit-Lys(DOTA)-Tyr-Phe-Pro-Hyp-Ile-Nva-Cys]-NHz (UPAR-346) of the following formula 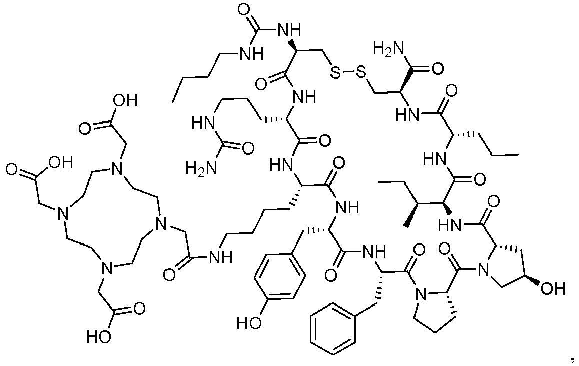
compound Pent-[Cys-Cit-Lys(DOTA)-Tyr-Thi-Pro-Hyp-Ile-Nva-Cys]-NH₂ (UPAR-360) of the following formula 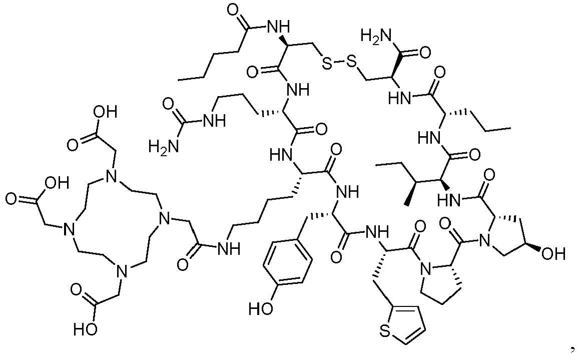
compound Pent-[Cys-Cit-Lys(DOTA)-Tyr-Phe-4Cfp-Hyp-Ile-Nva-Cys]-NH₂ (UPAR-365) of the following formula 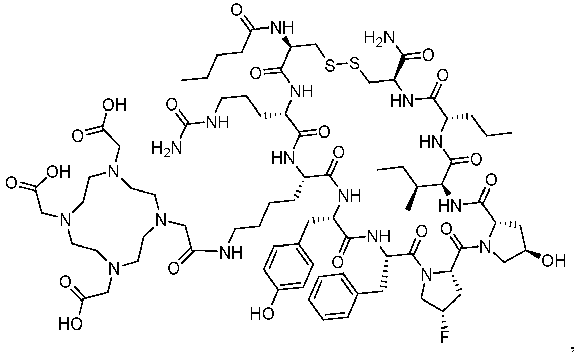
compound Pent-[Cys-Cit-Lys(DOTA)-Dopa-Phe-Pro-Hyp-Ile-Nva-Cys]-NH₂ (UPAR-399) of the following formula 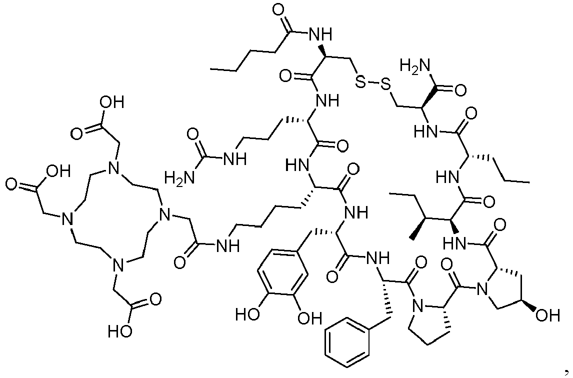
compound Pent-[Cys-Cit-Lys(DOTA)-Tyr-Phe-Hyp-Hyp-Ile-Nva-Cys]-NH₂ (UPAR-403) of the following formula 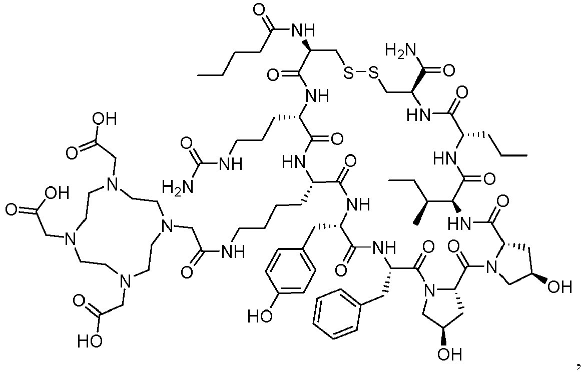
compound Butoc-[Cys-Thr-Orn(DOTA)-My-Phe-4Cfp-Hyp-Ile-Nva-Cys]-NH₂ (UPAR-415) of the following formula 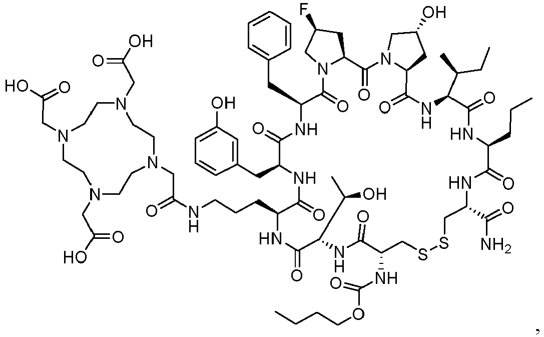
compound Butoc-[Cys-Thr-Lys(DOTA-Kzw)-Tyr-Phe-4Cfp-Hyp-Ile-Nva-Cys]-NH₂ (UPAR-422) of the following formula 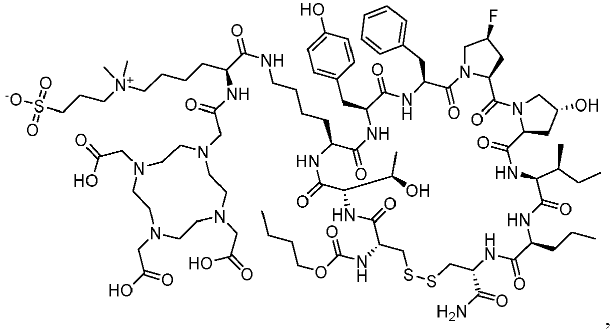
compound Butoc-[Cys-Thr-Lys(DOTA-asp)-Tyr-Phe-4Cfp-Hyp-Ile-Nva-Cys]-NH₂ (UPAR-424) of the following formula 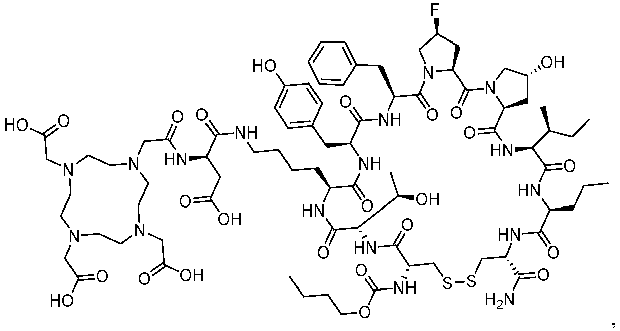
compound Butoc-[Cys-Thr-Lys(NODAGA)-Dopa-Phe-4Cfp-Hyp-Ile-Nva-Cys]-NH₂ (UPAR-444) of the following formula 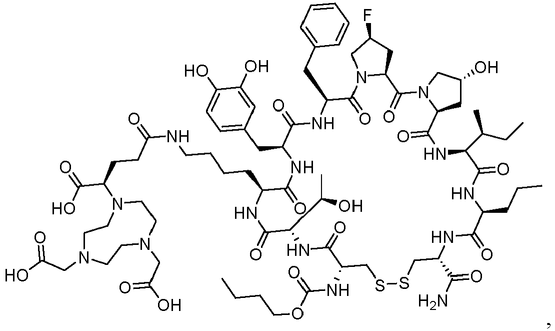
compound Pent-[Cys-Cit-Lys(DOTA)-Tyr-Ocf-Pro-Hyp-Ile-Nva-Cys]-NH₂ (UPAR-450) of the following formula 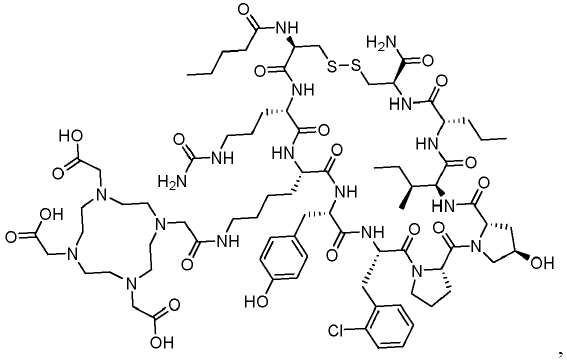
compound Butoc-[Cys-Thr-Dap(DOTA)-Tyr-Phe-4Cfp-Hyp-Ile-Nva-Cys]-NH₂ (UPAR-455) of the following formula 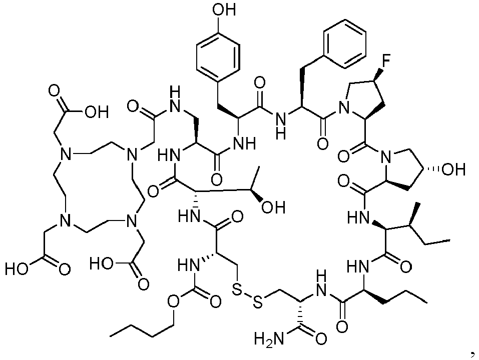
compound Butoc-[Cys-Thr-Dab(DOTA)-Dopa-Phe-4Cfp-Hyp-Ile-Nva-Cys]-NH₂ (UPAR-456) of the following formula 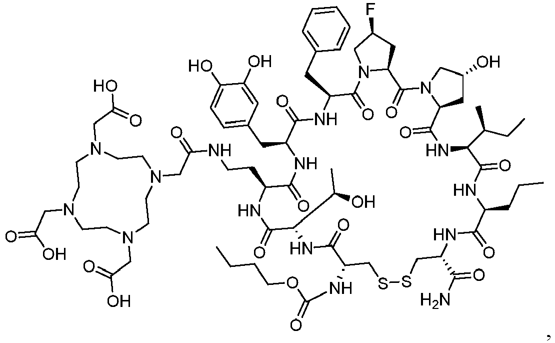
compound Butoc-[Cys-Thr-Lys(DOTA-asp)-Dopa-Phe-4Cfp-Hyp-Ile-Nva-Cys]-NH₂ (UPAR-459) of the following formula 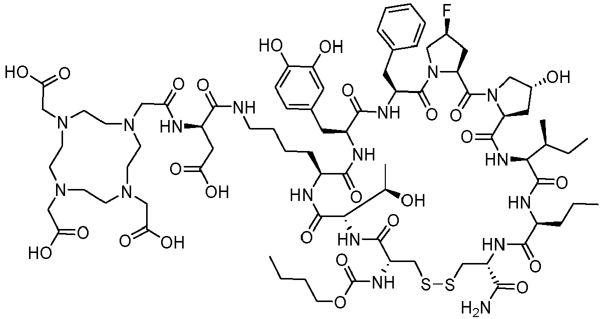
or a pharmaceutically acceptable salt or hydrate or pharmaceutically acceptable solvate thereof.

Embodiment 222. The compound or pharmaceutically acceptable salt or hydrate or pharmaceutically acceptable solvate of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214, 215, 216, 217, 218, 219, 220 and 221, or any one of Embodiments 1, 2, 3, 4, 5, 6, 7, 8, 9, 10, 11, 12, 13 and 14 or any one of Embodiments 1, 2 and 3, wherein the compound is selected from the group consisting of
compound Butoc-[Cys-Thr-Lys(DOTA)-Mpa-Phe-4Cfp-Hyp-Ile-Nva-Cys]-NH₂ (UPAR-165) of the following formula 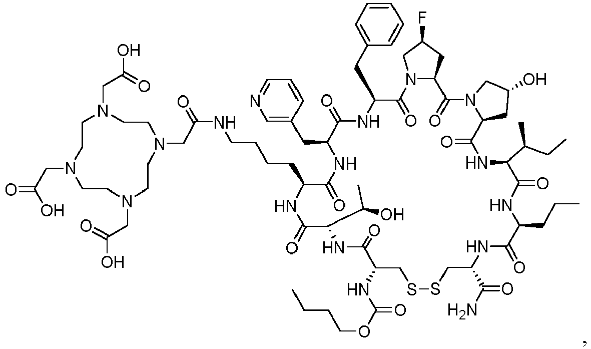
compound Butoc-[Cys-Thr-Lys(DOTA-PPAc)-Tyr-Phe-4Cfp-Hyp-Ile-Nva-Cys]-NH₂ (UPAR-181) of the following formula 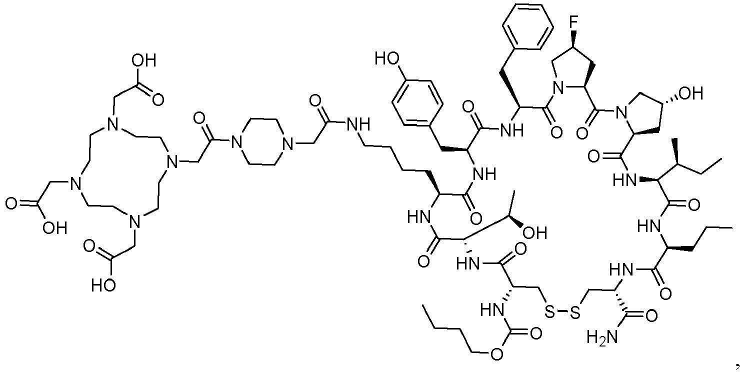
compound Butoc-[Cys-Thr-Orn(DOTA)-Tyr-Phe-4Cfp-Hyp-Ile-Nva-Cys]-NH₂ (UPAR-192) of the following formula 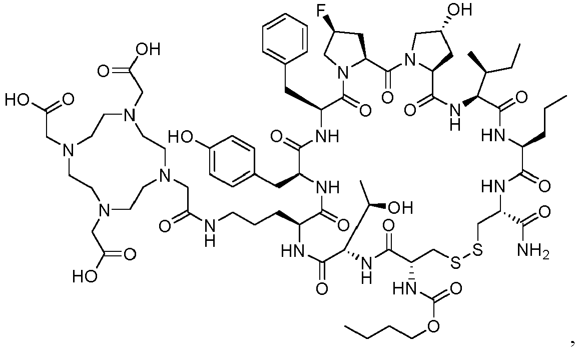
compound Butoc-[Cys-Thr-Lys(DOTA-PPAc)-Dopa-Phe-4Cfp-Hyp-Ile-Nva-Cys]-NH₂ (UPAR-199) of the following formula 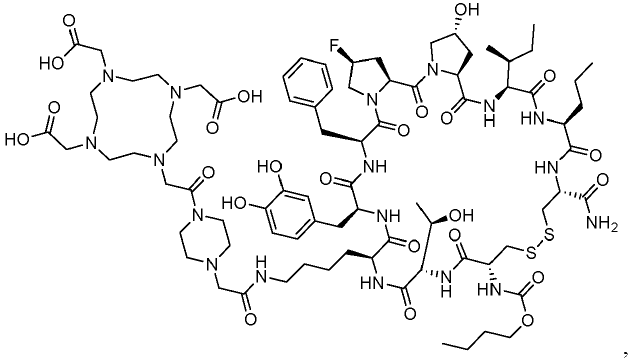
compound Butoc-[Cys-Thr-Lys(DOTA)-Tyr-Phe-4Cfp-Hyp-Ile-Nva-Cys]-NH₂ (UPAR-237) of the following formula 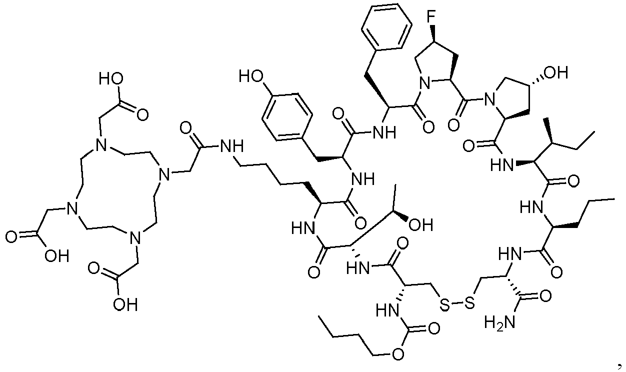
compound Butoc-[Cys-Thr-Lys(DOTA)-Dopa-Phe-4Cfp-Hyp-Ile-Nva-Cys]-NH₂ (UPAR-249) of the following formula 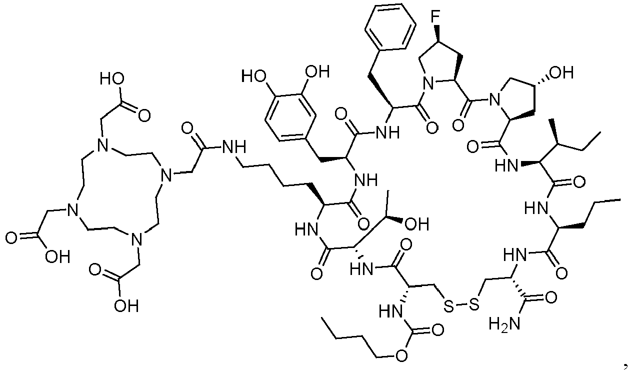
compound Butoc-[Cys-Thr-Lys(NOTA)-Dopa-Phe-4Cfp-Hyp-Ile-Nva-Cys]-NH₂ (UPAR-250) of the following formula 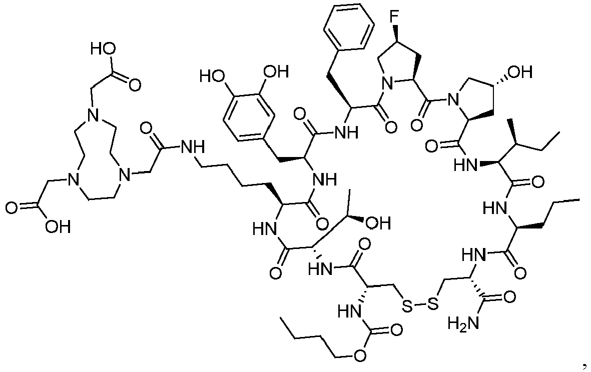
compound Butoc-[Cys-Thr-Orn(DOTA)-Pcnf-Phe-4Cfp-Hyp-Ile-Nva-Cys]-NH₂ (UPAR-276) of the following formula 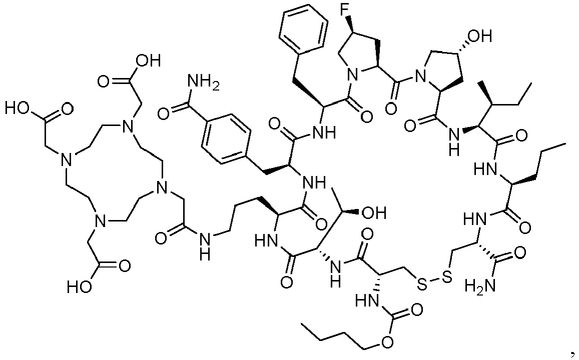
compound Butoc-[Cys-Thr-Orn(DOTA)-Dopa-Phe-4Cfp-Hyp-Ile-Nva-Cys]-NH₂ (UPAR-280) of the following formula 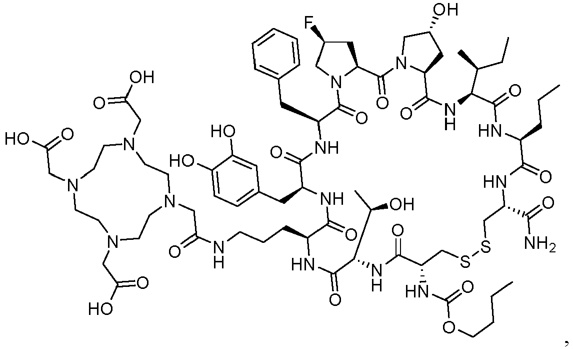
compound Butoc-[Cys-Thr-Orn(DOTA)-My-Phe-4Cfp-Hyp-Ile-Nva-Cys]-NH₂ (UPAR-415) of the following formula 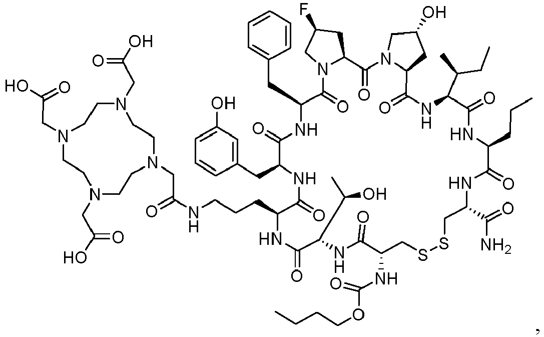
compound Butoc-[Cys-Thr-Lys(DOTA-Kzw)-Tyr-Phe-4Cfp-Hyp-Ile-Nva-Cys]-NH₂ (UPAR-422) of the following formula 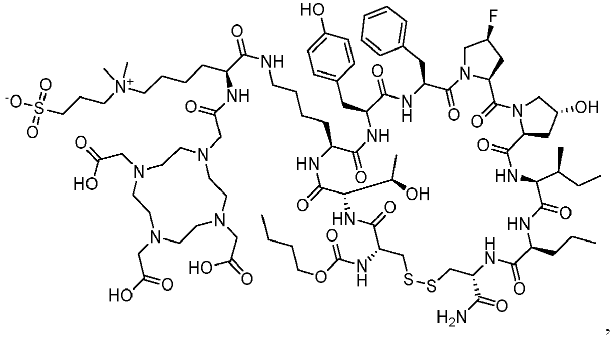
compound Butoc-[Cys-Thr-Lys(NODAGA)-Dopa-Phe-4Cfp-Hyp-Ile-Nva-Cys]-NH₂ (UPAR-444) of the following formula 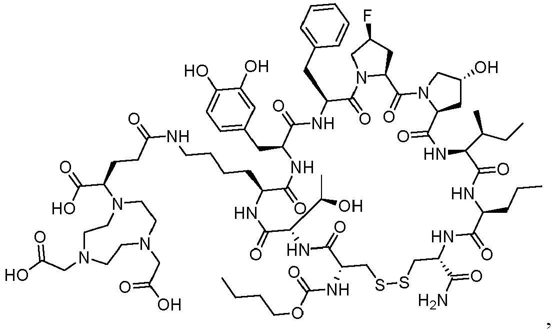
compound Butoc-[Cys-Thr-Dab(DOTA)-Dopa-Phe-4Cfp-Hyp-Ile-Nva-Cys]-NH₂ (UPAR-456) of the following formula 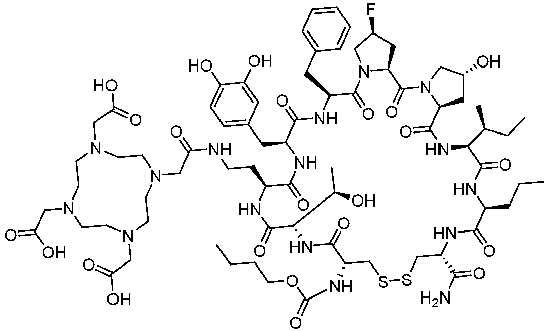
or a pharmaceutically acceptable salt or hydrate or pharmaceutically acceptable solvate thereof.

Embodiment 223. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214, 215, 216, 217, 218, 219, 220, 221 and 222, any one of Embodiments 1, 2, 3, 4, 5, 6, 7, 8, 9, 10, 11, 12, 13 and 14 or any one of Embodiments 1, 2 and 3, wherein the compound is selected from the group consisting of
compound Butoc-[Cys-Thr-Lys(DOTA)-Dopa-Phe-4Cfp-Hyp-Ile-Nva-Cys]-NH₂ (UPAR-249) of the following formula 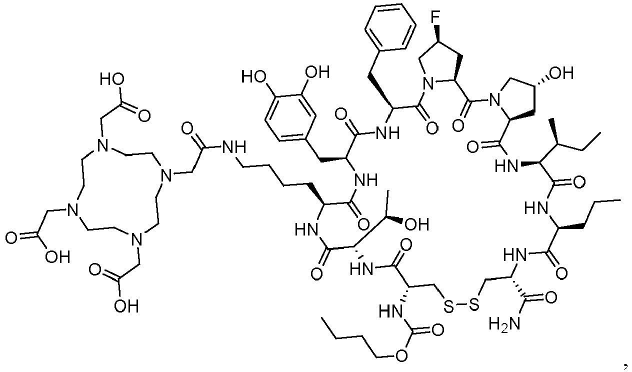
compound Butoc-[Cys-Thr-Orn(DOTA)-Pcnf-Phe-4Cfp-Hyp-Ile-Nva-Cys]-NH₂ (UPAR-276) of the following formula 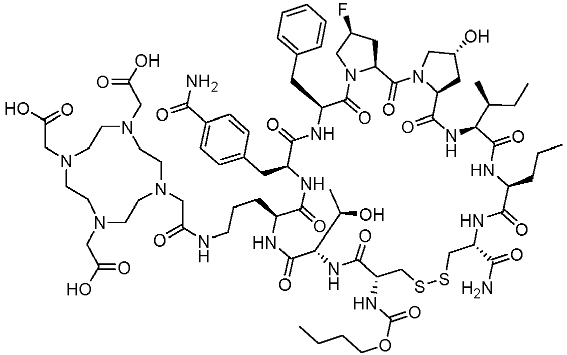
or a pharmaceutically acceptable salt or hydrate or pharmaceutically acceptable solvate thereof.

Embodiment 224. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214, 215, 216, 217, 218, 219, 220, 221, 222, 223, , wherein the compound is selected from the group consisting of
compound iHex-[Cys-Arg-Lys(InDOTA)-Tyr-Phe-Pro-ala-Ile-Leu-Cys]-NH₂ (UPAR-148) of the following formula 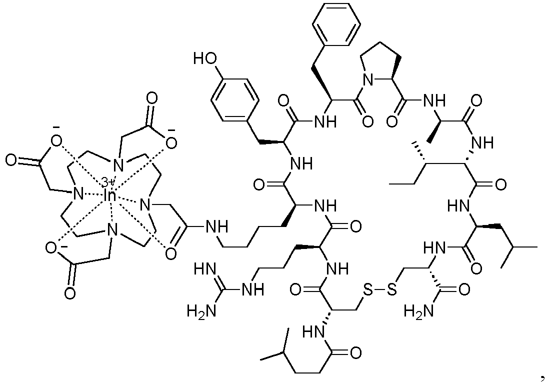
compound Butoc-[Cys-Thr-Lys(InDOTA)-Dopa-Phe-4Cfp-Hyp-Ile-Nva-Cys]-NH₂ (UPAR-191) of the following formula 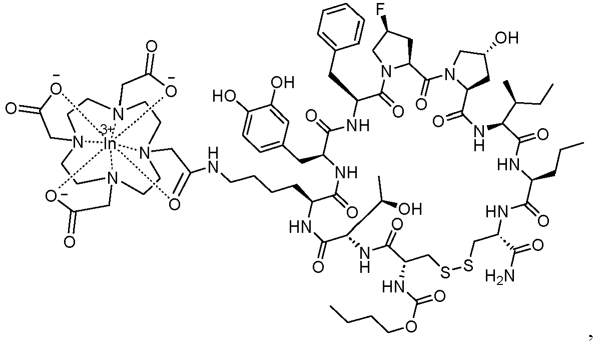
compound Butoc-[Cys-Thr-Lys(LuDOTA)-Dopa-Phe-4Cfp-Hyp-Ile-Nva-Cys]-NH₂ (UPAR-238) of the following formula 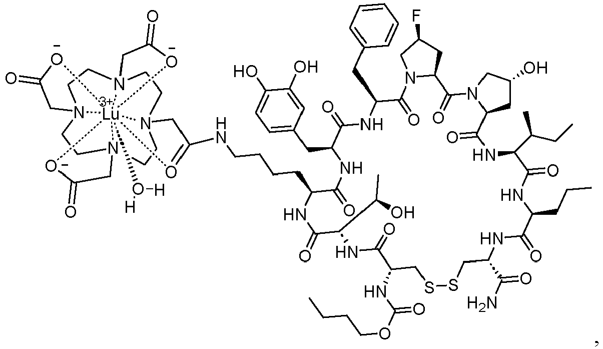
compound Butoc-[Cys-Thr-Lys(GaDOTA)-Dopa-Phe-4Cfp-Hyp-Ile-Nva-Cys]-NH₂ (UPAR-241) of the following formula 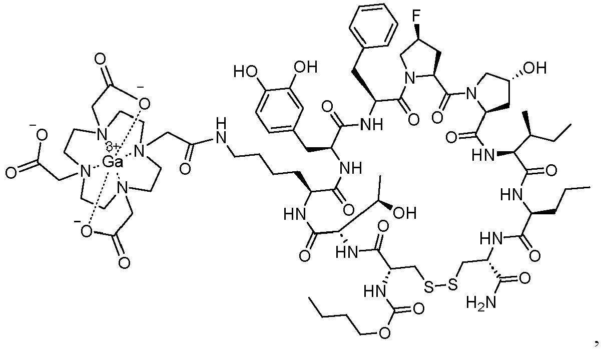
compound Butoc-[Cys-Thr-Lys(InDOTA)-Tyr-Phe-4Cfp-Hyp-Ile-Nva-Cys]-NH₂ (UPAR-294) of the following formula 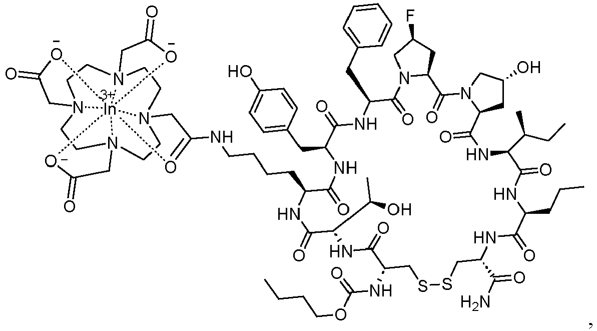
compound Butoc-[Cys-Thr-Orn(GaDOTA)-Pcnf-Phe-4Cfp-Hyp-Ile-Nva-Cys]-NH₂ (UPAR-489) of the following formula 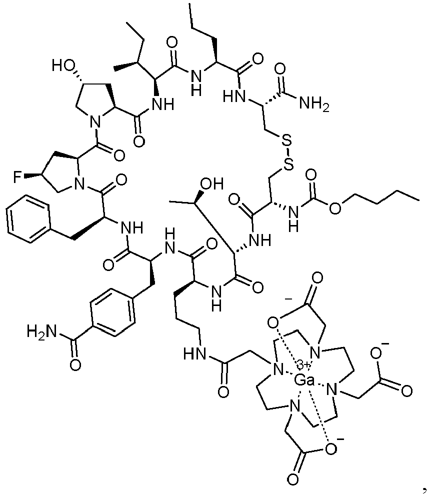
compound Butoc-[Cys-Thr-Orn(InDOTA)-Pcnf-Phe-4Cfp-Hyp-Ile-Nva-Cys]-NH₂ (UPAR-490) of the following formula 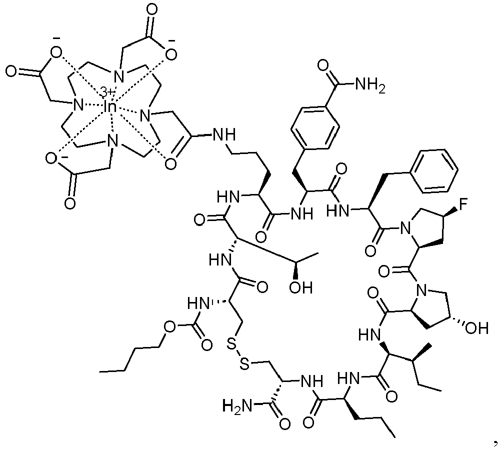
compound Butoc-[Cys-Thr-Om(LuDOTA)-Pcnf-Phe-4Cfp-Hyp-Ile-Nva-Cys]-NH₂ (UPAR-491) of the following formula

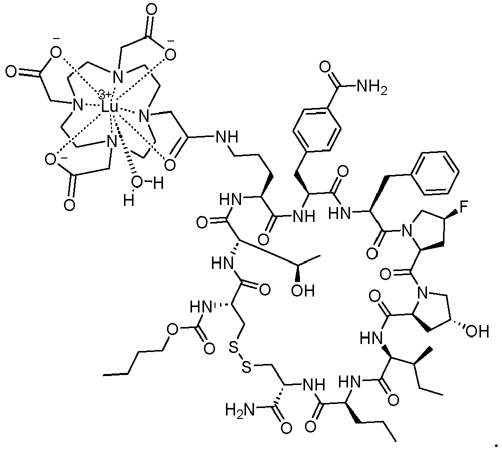

Embodiment 225. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223 and 224, wherein any S atom which can be oxidized, preferably S atoms of the disulfide group, is present as -S-, -S(O)- or -S(Oz)- or a mixture thereof.

Embodiment 226. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 211, 212, 213, 214, 215, 216, 217, 218, 219, 220, 221, 222, 223, 224 and 225, wherein the compound comprises a diagnostically active nuclide or a therapeutically active nuclide.

Embodiment 227. The compound or pharmaceutically acceptable salt or hydrate or pharmaceutically acceptable solvate of Embodiment 226, wherein the diagnostically active nuclide is a diagnostically active radionuclide.

Embodiment 228. The compound or pharmaceutically acceptable salt or hydrate or pharmaceutically acceptable solvate of Embodiment 227, wherein the diagnostically active radionuclide is selected from the group consisting of Al-¹⁸F, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I, preferably selected from the group consisting of Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb, and more preferably selected from the group consisting of ⁶⁴Cu, ⁶⁸Ga, ¹¹¹In and ²⁰³Pb.

Embodiment 229. The compound or pharmaceutically acceptable salt or hydrate or pharmaceutically acceptable solvate of Embodiment 226, wherein the therapeutically active nuclide is a therapeutically active radionuclide.

Embodiment 230. The compound or pharmaceutically acceptable salt or hydrate or pharmaceutically acceptable solvate of Embodiment 229, wherein the therapeutically active radionuclide is selected from the group consisting of ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹²⁵I, ¹³¹I, and ²¹¹At, preferably selected from the group consisting of ⁶⁷Cu, ⁹⁰Y, ¹⁴⁹Tb, ¹⁶¹Tb, ¹⁷⁷Lu, ²¹²Pb, ²¹³Bi, ²²⁵Ac, ²²⁷Th, and more preferably selected from the group consisting of ⁹⁰Y, ¹⁷⁷Lu, ²¹²Pb and ²²⁵Ac.

Embodiment 231. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229 and 230, wherein the compound interacts with a urokinase plasminogen activator surface receptor (uPAR), preferably with human uPAR having an amino acid sequence of SEQ ID NO: 1 or a homolog thereof, wherein the amino acid sequence of the homolog has an identity of at least 85% to the amino acid sequence of SEQ ID NO: 1.

Embodiment 232. The compound or pharmaceutically acceptable salt or hydrate or pharmaceutically acceptable solvate of Embodiment 231, wherein the compound binds or is capable of binding to urokinase plasminogen activator surface receptor (uPAR)

Embodiment 233. The compound or pharmaceutically acceptable salt or hydrate or pharmaceutically acceptable solvate of any one of Embodiments 231 and 232, wherein the compound has a pIC₅₀ value for human uPAR of SEQ ID NO: 1 of ≥ 6.0, preferably of ≥ 7.0, even more preferably ≥ 7.5, and most preferably of ≥ 8.0.

Embodiment 234. The compound or pharmaceutically acceptable salt or hydrate or pharmaceutically acceptable solvate of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably any one of Embodiments 224, 226 , 227, 228, 231, 232 and 233, for use in a method for the diagnosis of a disease.

Embodiment 235. The compound or pharmaceutically acceptable salt or hydrate or pharmaceutically acceptable solvate for use of Embodiment 234, wherein the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

Embodiment 236. The compound or pharmaceutically acceptable salt or hydrate or pharmaceutically acceptable solvate for use of any one of Embodiments 234 and 235, wherein the disease involves cells showing upregulated expression of urokinase plasminogen activator surface receptor (uPAR), preferably diseased tissue contains cells showing upregulated expression of urokinase plasminogen activator surface receptor (uPAR), more preferably the disease involves tumor-associated fibroblasts, tumor-associated macrophages, tumor-associated neutrophils, tumor-associated endothelial cells, disseminated tumor cells and/or senescent cells, whereby the senescent cells are preferably associated with lung fibrosis, atherosclerosis, Alzheimer's disease, diabetes, liver fibrosis, chronic kidney disease, osteoarthritis, cardiac fibrosis, and Parkinson's disease.

Embodiment 237. The compound or pharmaceutically acceptable salt or hydrate or pharmaceutically acceptable solvate for use of any one of Embodiment 234, 235 and 236, wherein the disease is a neoplasm, preferably a cancer or tumor.

Embodiment 238. The compound or pharmaceutically acceptable salt or hydrate or pharmaceutically acceptable solvate for use of Embodiment 237, wherein a cell or cells of the neoplasm, the cancer and/or the tumor expresses urokinase plasminogen activator surface receptor (uPAR).

Embodiment 239. The compound or pharmaceutically acceptable salt or hydrate or pharmaceutically acceptable solvate for use of any one of Embodiments 234, 235, 236, 237 and 238, wherein the neoplasm, the cancer and/or the tumor are each and individually selected from the group comprising solid tumors and non-solid tumors.

Embodiment 240. The compound or pharmaceutically acceptable salt or hydrate or pharmaceutically acceptable solvate for use of any one of Embodiments 234, 235, 236, 237, 238 and 239, wherein the neoplasm, the cancer and/or the tumor are each and individually selected from the group comprising adrenocortical carcinoma, bladder cancer, bladder urothelial carcinoma, breast cancer, breast invasive carcinoma, cervical squamous cell carcinoma, cholangiocarcinoma, colon adenocarcinoma, colorectal cancer, diffuse large B-cell lymphoma, gastric cancer, endocervical adenocarcinoma, esophageal carcinoma, glioblastoma, glioblastoma multiforme, brain lower grade glioma, haematological malignancies such as acute myeloid leukaemia (AML), multiple myeloma (MM), head and neck squamous cell carcinoma, hepatocellular carcinoma (in particular at the invasion front), kidney chromophobe, kidney renal clear cell carcinoma, kidney renal papillary cell carcinoma, liver hepatocellular carcinoma, lung adenocarcinoma, lung cancer such as non-small lung carcinoma (NSCLC) and squamous cell carcinoma (SCC), lymphoid neoplasm, mesothelioma, ovarian cancer, ovarian serous cystadenocarcinoma, pancreatic cancer (in particular tumor cells associated with invasion), pancreatic adenocarcinoma, paraganglioma, prostate cancer, prostate adenocarcinoma, pheochromocytoma, lymph node metastases of prostate cancer rectum adenocarcinoma, sarcoma, skin cutaneous melanoma, stomach adenocarcinoma, testicular germ cell tumors, thyroid carcinoma, thymoma, uterine corpus endometrial carcinoma, uterine carcinosarcoma, and uveal melanoma.

Embodiment 241. The compound or pharmaceutically acceptable salt or hydrate or pharmaceutically acceptable solvate for use of any one of Embodiments 237, 238, 239 and 240, wherein the neoplasm, the cancer and/or the tumor shows at least one characteristic selected from the group comprising aggressive growth, postoperative progression, metastasis and poor response to therapy.

Embodiment 242. The compound or pharmaceutically acceptable salt or hydrate or pharmaceutically acceptable solvate for use of any one of Embodiments 234, 235 and 236, wherein the disease is selected from the group comprising a cardiac disease, a liver disease, a lung disease, an autoimmune disease,a neurological disorder and a senescence related disease.

Embodiment 243. The compound or pharmaceutically acceptable salt or hydrate or pharmaceutically acceptable solvate for use of Embodiment 242, wherein the disease is a cardiac disease, preferably the cardiac disease is cardiac fibrosis.

Embodiment 244. The compound or pharmaceutically acceptable salt or hydrate or pharmaceutically acceptable solvate for use of Embodiment 242, wherein the disease is a liver disease, preferably the liver disease is selected from the group comprising chronic liver disease and hepatitis B.

Embodiment 245. The compound or pharmaceutically acceptable salt or hydrate or pharmaceutically acceptable solvate for use of Embodiment 242, wherein the disease is a lung disease, preferably the lung disease is selected from the group comprising cystic fibrosis, COPD and idiopathic pulmonary fibrosis.

Embodiment 246. The compound or pharmaceutically acceptable salt or hydrate or pharmaceutically acceptable solvate for use of Embodiment 242, wherein the disease is an autoimmune disease, preferably the autoimmune disease is selected from the group comprising glomerulosclerosis, systemic sclerosis, arthritis, rheumatoid arthritis, atherosclerosis and allergic conjunctivitis.

Embodiment 247. The compound or pharmaceutically acceptable salt or hydrate or pharmaceutically acceptable solvate for use of Embodiment 242, wherein the disease is a neurological disorder, preferably the neurological disorder is selected from the group comprising autism spectrum disorder and epileptogenic tissue remodelling.

Embodiment 248. The compound or pharmaceutically acceptable salt or hydrate or pharmaceutically acceptable solvate for use of Embodiment 242, wherein the disease is senescence related diseases, whereby the senescence related diseases is selected from the group comprising lung fibrosis, atherosclerosis, Alzheimer's disease, diabetes, liver fibrosis, chronic kidney disease, osteoarthritis, cardiac fibrosis, and Parkinson's disease.

Embodiment 249. The compound or pharmaceutically acceptable salt or hydrate or pharmaceutically acceptable solvate for use of any one of Embodiments 234, 235, 236, 237, 238, 239, 240, 241, 242, 243, 244, 245, 246, 247 and 248, , wherein the compound comprises a diagnostically active nuclide, preferably a diagnostically active radionuclide.

Embodiment 250. The compound or pharmaceutically acceptable salt or hydrate or pharmaceutically acceptable solvate for use of Embodiment 249, wherein the diagnostically active radionuclide is selected from the group consisting of Al-¹⁸F, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I, preferably selected from the group consisting of Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb, and more preferably selected from the group consisting of ⁶⁴Cu, ⁶⁸Ga, ¹¹¹In and ²⁰³Pb.

Embodiment 251. The compound or pharmaceutically acceptable salt or hydrate or pharmaceutically acceptable solvate for use of any one of Embodiments 234, 235, 236, 237, 238, 239, 240, 241, 242, 243, 244, 245, 246, 247, 248, 249 and 250, wherein the method for the diagnosis is an imaging method.

Embodiment 252. The compound or pharmaceutically acceptable salt or hydrate or pharmaceutically acceptable solvate for use of Embodiment 251, wherein the imaging method is selected from the group consisting of scintigraphy, Single Photon Emission Computed Tomography (SPECT) and Positron Emission Tomography (PET).

Embodiment 253. The compound or pharmaceutically acceptable salt or hydrate or pharmaceutically acceptable solvate for use of any one of Embodiments 234, 235, 236, 237, 238, 239, 240, 241, 242, 243, 244, 245, 246, 247, 248, 249, 250, 251 and 252, wherein the method comprises the administration of a diagnostically effective amount of the compound to a subject, preferably to a mammal, wherein the mammal is selected from the group comprising man, companion animals, pets, and livestock, more preferably the subject is selected from the group comprising man, dog, cat, horse, and cow, and most preferably the subject is a human being.

Embodiment 254. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably of any one of Embodiments 224, 226, 229, 230, 231, 232 and 233, for use in a method for the treatment of a disease.

Embodiment 255. The compound or pharmaceutically acceptable salt or hydrate or pharmaceutically acceptable solvate for use of Embodiment 254, wherein the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

Embodiment 256. The compound or pharmaceutically acceptable salt or hydrate or pharmaceutically acceptable solvate for use of any one of Embodiments 254 and 255, wherein the disease involves cells showing upregulated expression of urokinase plasminogen activator surface receptor (uPAR), preferably diseased tissue contains cells showing upregulated expression of urokinase plasminogen activator surface receptor (uPAR), more preferably the disease involves tumor-associated fibroblasts, tumor-associated macrophages, tumor associated neutrophils, tumor-associated endothelial cells, disseminated tumor cells and/or senescent cells, whereby the senescent cells are preferably associated with lung fibrosis, atherosclerosis, Alzheimer's disease, diabetes, liver fibrosis, chronic kidney disease, osteoarthritis, cardiac fibrosis, and Parkinson's disease.

Embodiment 257. The compound or pharmaceutically acceptable salt or hydrate or pharmaceutically acceptable solvate for use of any one of Embodiments 254, 255 and 256, wherein the disease is a neoplasm, preferably a cancer or tumor.

Embodiment 258. The compound or pharmaceutically acceptable salt or hydrate or pharmaceutically acceptable solvate for use of Embodiment 257, wherein a cell or cells of the neoplasm, the cancer and/or the tumor expresses urokinase plasminogen activator surface receptor (uPAR).

Embodiment 259. The compound or pharmaceutically acceptable salt or hydrate or pharmaceutically acceptable solvate for use of any one of Embodiments 254, 255, 256, 257 and 258, wherein the neoplasm, the cancer and/or the tumor are each and individually selected from the group comprising solid tumors and non-solid tumors.

Embodiment 260. The compound or pharmaceutically acceptable salt or hydrate or pharmaceutically acceptable solvate for use of any one of Embodiments 254, 255, 256, 257, 258 and 259, wherein the neoplasm, the cancer and/or the tumor are each and individually selected from the group comprising adrenocortical carcinoma, bladder cancer, bladder urothelial carcinoma, breast cancer, breast invasive carcinoma, cervical squamous cell carcinoma, cholangiocarcinoma, colon adenocarcinoma, colorectal cancer, diffuse large B-cell lymphoma, gastric cancer, endocervical adenocarcinoma, esophageal carcinoma, glioblastoma, glioblastoma multiforme, brain lower grade glioma, haematological malignancies such as acute myeloid leukaemia (AML), multiple myeloma (MM), head and neck squamous cell carcinoma, hepatocellular carcinoma (in particular at the invasion front), kidney chromophobe, kidney renal clear cell carcinoma, kidney renal papillary cell carcinoma, liver hepatocellular carcinoma, lung adenocarcinoma, lung cancer such as non-small lung carcinoma (NSCLC) and squamous cell carcinoma (SCC), lymphoid neoplasm, mesothelioma, ovarian cancer, ovarian serous cystadenocarcinoma, pancreatic cancer (in particular tumor cells associated with invasion), pancreatic adenocarcinoma, paraganglioma, prostate cancer, prostate adenocarcinoma, pheochromocytoma, lymph node metastases of prostate cancer rectum adenocarcinoma, sarcoma, skin cutaneous melanoma, stomach adenocarcinoma, testicular germ cell tumors, thyroid carcinoma, thymoma, uterine corpus endometrial carcinoma, uterine carcinosarcoma, and uveal melanoma.

Embodiment 261. The compound or pharmaceutically acceptable salt or hydrate or pharmaceutically acceptable solvate for use of any one of Embodiments 256, 257, 258 and 259, wherein the neoplasm, the cancer and/or the tumor shows at least one characteristic selected from the group comprising aggressive growth, postoperative progression, metastasis and poor response to therapy.

Embodiment 262. The compound or pharmaceutically acceptable salt or hydrate or pharmaceutically acceptable solvate for use of any one of Embodiments 257, 258, 259 and 260, wherein the treatment comprises inhibiting tumor growth, angiogenesis, tumor cell invasion, migration of tumor cells and/or epithelial-mesenchymal transition (EMT), preferably the compound inhibits tumor growth, angiogenesis, tumor cell invasion, migration of tumor cells and/or epithelial-mesenchymal transition (EMT), more preferably the binding of the compound to urokinase plasminogen activator surface receptor (uPAR) inhibits tumor growth, angiogenesis, tumor cell invasion, migration of tumor cells and/or epithelial-mesenchymal transition (EMT), most preferably the compound inhibits tumor growth, angiogenesis, tumor cell invasion, migration of tumor cells and/or epithelial-mesenchymal transition (EMT) by inhibiting urokinase plasminogen activator surface receptor (uPAR).

Embodiment 263. The compound or pharmaceutically acceptable salt or hydrate or pharmaceutically acceptable solvate for use of any one of Embodiments 254, 255, 256, 257, 258, 259, 260, 261 and 262, wherein the disease is selected from the group comprising a cardiac disease, a liver disease, a lung disease, an autoimmune disease, a neurological disorder and a senescence related disease.

Embodiment 264. The compound or pharmaceutically acceptable salt or hydrate or pharmaceutically acceptable solvate for use of Embodiment 263, wherein the disease is a cardiac disease, preferably the cardiac disease is cardiac fibrosis.

Embodiment 265. The compound or pharmaceutically acceptable salt or hydrate or pharmaceutically acceptable solvate for use of Embodiment 263, wherein the disease is a liver disease, preferably the liver disease is selected from the group comprising chronic liver disease and hepatitis B.

Embodiment 266. The compound or pharmaceutically acceptable salt or hydrate or pharmaceutically acceptable solvate for use of Embodiment 263, wherein the disease is a lung disease, preferably the lung disease is selected from the group comprising cystic fibrosis, COPD and idiopathic pulmonary fibrosis.

Embodiment 267. The compound or pharmaceutically acceptable salt or hydrate or pharmaceutically acceptable solvate for use of Embodiment 263, wherein the disease is an autoimmune disease, preferably the autoimmune disease is selected from the group comprising glomerulosclerosis, systemic sclerosis, arthritis, rheumatoid arthritis, atherosclerosis and allergic conjunctivitis.

Embodiment 268. The compound or pharmaceutically acceptable salt or hydrate or pharmaceutically acceptable solvate for use of Embodiment 263, wherein the disease is a neurological disorder, preferably the neurological disorder is selected from the group comprising autism spectrum disorder and epileptogenic tissue remodelling.

Embodiment 269. The compound or pharmaceutically acceptable salt or hydrate or pharmaceutically acceptable solvate for use of Embodiment 263, wherein the disease is senescence related diseases, whereby the senescence related diseases is selected from the group comprising lung fibrosis, atherosclerosis, Alzheimer's disease, diabetes, liver fibrosis, chronic kidney disease, osteoarthritis, cardiac fibrosis, and Parkinson's disease.

Embodiment 270. The compound or pharmaceutically acceptable salt or hydrate or pharmaceutically acceptable solvate for use of any one of Embodiments 254, 255, 256, 257, 258, 259, 260, 261, 262, 263, 264, 265, 266, 267, 268 and 269, wherein the compound comprises a therapeutically active nuclide, preferably a therapeutically active radionuclide.

Embodiment 271. The compound or pharmaceutically acceptable salt or hydrate or pharmaceutically acceptable solvate for use of Embodiment 270, wherein the therapeutically active radionuclide selected from the group consisting of ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹²⁵I, ¹³¹I, and ²¹¹At, preferably selected from the group consisting of ⁶⁷Cu, ⁹⁰Y, ¹⁴⁹Tb, ¹⁶¹Tb, ¹⁷⁷Lu, ²¹²Pb, ²¹³Bi, ²²⁵Ac, ²²⁷Th, and more preferably selected from the group consisting of ⁹⁰Y, ¹⁷⁷Lu, ²¹²Pb and ²²⁵Ac.

Embodiment 272. The compound or pharmaceutically acceptable salt or hydrate or pharmaceutically acceptable solvate for use of any one of Embodiments 254, 255, 256, 257, 258, 259, 260, 261, 262, 263, 264, 265, 266, 267, 268, 269, 270 and 271, wherein the method comprises the administration of a therapeutically effective amount of the compound to a subject, preferably to a mammal, wherein the mammal is selected from the group comprising man, companion animals, pets, and livestock, more preferably the subject is selected from the group comprising man, dog, cat, horse, and cow, and most preferably the subject is a human being.

Embodiment 273. The compound or pharmaceutically acceptable salt or hydrate or pharmaceutically acceptable solvate for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably of any one of Embodiments 224, 226, 227, 228, 231, 232 and 233, for use in a method for the identification of a subject, wherein the subject is likely to respond or likely not to respond to a treatment of a disease, wherein the method for the identification of a subject comprises carrying out a method of diagnosis using the compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably of any one of Embodiments 224, 226, 227, 228, 231, 232 and 233, preferably a method for the diagnosis of a disease as described in any one of Embodiments 234, 235, 236, 237, 238, 239, 240, 241, 242, 243, 244, 245, 246, 247, 248, 249, 250, 251, 252 and 253.

Embodiment 274. The compound or pharmaceutically acceptable salt or hydrate or pharmaceutically acceptable solvate for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably of any one of Embodiments 224, 226, 227, 228, 231, 232 and 233, for use in a method for the selection of a subject from a group of subjects, wherein the subject is likely to respond or likely not to respond to a treatment of a disease, wherein the method for the selection of a subject from a group of subjects comprises carrying out a method of diagnosis using the compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, and 233, preferably of any one of Embodiments 224, 226, 227, 228, 231, 232 and 233, preferably a method for the diagnosis of a disease as described in any one of Embodiments 234, 235, 236, 237, 238, 239, 240, 241, 242, 243, 244, 245, 246, 247, 248, 249, 250, 251, 252 and 253.

Embodiment 275. The compound or pharmaceutically acceptable salt or hydrate or pharmaceutically acceptable solvate for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably of any one of Embodiments 224, 226, 227, 228, 231, 232 and 233, for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosis using the compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, preferably of any one of Embodiments 224, 226, 227, 228, 231, 232 and 233, preferably a method for the diagnosis of a disease as described in any one of Embodiments 234, 235, 236, 237, 238, 239, 240, 241, 242, 243, 244, 245, 246, 247, 248, 249, 250, 251, 252 and 253.

Embodiment 276. The compound or pharmaceutically acceptable salt or hydrate or pharmaceutically acceptable solvate for use of any one of Embodiments 273, 274 and 275, wherein the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

Embodiment 277. The compound or pharmaceutically acceptable salt or hydrate or pharmaceutically acceptable solvate for use of any one of Embodiments 273, 274, 275 and 276, wherein the disease involves cells showing upregulated expression of urokinase plasminogen activator surface receptor (uPAR), preferably diseased tissue contains cells showing upregulated expression of urokinase plasminogen activator surface receptor (uPAR), more preferably the disease involves tumor-associated fibroblasts, tumor-associated macrophages, tumor-associated neutrophils, tumor-associated endothelial cells, disseminated tumor cells and/or senescent cells, whereby the senescent cells are preferably associated with lung fibrosis, atherosclerosis, Alzheimer's disease, diabetes, liver fibrosis, chronic kidney disease, osteoarthritis, cardiac fibrosis, and Parkinson's disease.

Embodiment 278. The compound or pharmaceutically acceptable salt or hydrate or pharmaceutically acceptable solvate for use of any one of Embodiments 273, 274, 275, 276 and 277, wherein the disease is a neoplasm, preferably a cancer or tumor.

Embodiment 279. The compound or pharmaceutically acceptable salt or hydrate or pharmaceutically acceptable solvate for use of Embodiment 278, wherein a cell or cells of the neoplasm, the cancer and/or the tumor expresses urokinase plasminogen activator surface receptor (uPAR).

Embodiment 280. The compound or pharmaceutically acceptable salt or hydrate or pharmaceutically acceptable solvate for use of any one of Embodiments 278 and 279, wherein the neoplasm, the cancer and/or the tumor are each and individually selected from the group comprising solid tumors and non-solid tumors.

Embodiment 281. The compound or pharmaceutically acceptable salt or hydrate or pharmaceutically acceptable solvate for use of any one of Embodiments 278, 279 and 280, wherein the neoplasm, the cancer and/or the tumor are each and individually selected from the group comprising adrenocortical carcinoma, bladder cancer, bladder urothelial carcinoma, breast cancer, breast invasive carcinoma, cervical squamous cell carcinoma, cholangiocarcinoma, colon adenocarcinoma, colorectal cancer, diffuse large B-cell lymphoma, gastric cancer, endocervical adenocarcinoma, esophageal carcinoma, glioblastoma, glioblastoma multiforme, brain lower grade glioma, haematological malignancies such as acute myeloid leukaemia (AML), multiple myeloma (MM), head and neck squamous cell carcinoma, hepatocellular carcinoma (in particular at the invasion front), kidney chromophobe, kidney renal clear cell carcinoma, kidney renal papillary cell carcinoma, liver hepatocellular carcinoma, lung adenocarcinoma, lung cancer such as non-small lung carcinoma (NSCLC) and squamous cell carcinoma (SCC), lymphoid neoplasm, mesothelioma, ovarian cancer, ovarian serous cystadenocarcinoma, pancreatic cancer (in particular tumor cells associated with invasion), pancreatic adenocarcinoma, paraganglioma, prostate cancer, prostate adenocarcinoma, pheochromocytoma, lymph node metastases of prostate cancer rectum adenocarcinoma, sarcoma, skin cutaneous melanoma, stomach adenocarcinoma, testicular germ cell tumors, thyroid carcinoma, thymoma, uterine corpus endometrial carcinoma, uterine carcinosarcoma, and uveal melanoma.

Embodiment 282. The compound or pharmaceutically acceptable salt or hydrate or pharmaceutically acceptable solvate for use of any one of Embodiment 278, 279, 280 and 281, wherein the neoplasm, the cancer and/or the tumor shows at least one characteristic selected from the group comprising aggressive growth, postoperative progression, metastasis and poor response to therapy.

Embodiment 283. The compound or pharmaceutically acceptable salt or hydrate or pharmaceutically acceptable solvate for use of any one of Embodiments 273, 274, 275, 276, 277 and 278, wherein the disease is selected from the group comprising a cardiac disease, a liver disease, a lung disease, an autoimmune disease, a neurological disorder and a senescence related disease.

Embodiment 284. The compound or pharmaceutically acceptable salt or hydrate or pharmaceutically acceptable solvate for use of Embodiment 283, wherein the disease is a cardiac disease, preferably the cardiac disease is cardiac fibrosis.

Embodiment 285. The compound or pharmaceutically acceptable salt or hydrate or pharmaceutically acceptable solvate for use of Embodiment 283, wherein the disease is a liver disease, preferably the liver disease is selected from the group comprising chronic liver disease and hepatitis B.

Embodiment 286. The compound or pharmaceutically acceptable salt or hydrate or pharmaceutically acceptable solvate for use of Embodiment 283, wherein the disease is a lung disease, preferably the lung disease is selected from the group comprising cystic fibrosis, COPD and idiopathic pulmonary fibrosis.

Embodiment 287. The compound or pharmaceutically acceptable salt or hydrate or pharmaceutically acceptable solvate for use of Embodiment 283, wherein the disease is an autoimmune disease, preferably the autoimmune disease is selected from the group comprising glomerulosclerosis, systemic sclerosis, arthritis, rheumatoid arthritis, atherosclerosis and allergic conjunctivitis.

Embodiment 288. The compound or pharmaceutically acceptable salt or hydrate or pharmaceutically acceptable solvate for use of Embodiment 283, wherein the disease is a neurological disorder, preferably the neurological disorder is selected from the group comprising autism spectrum disorder and epileptogenic tissue remodelling.

Embodiment 289. The compound or pharmaceutically acceptable salt or hydrate or pharmaceutically acceptable solvate for use of Embodiment 283, wherein the disease is senescence related diseases, whereby the senescence related diseases is selected from the group comprising lung fibrosis, atherosclerosis, Alzheimer's disease, diabetes, liver fibrosis, chronic kidney disease, osteoarthritis, cardiac fibrosis, and Parkinson's disease.

Embodiment 290. The compound or pharmaceutically acceptable salt or hydrate or pharmaceutically acceptable solvate for use of any one of Embodiments 273, 274, 275, 276, 277, 278, 279, 280, 281, 282, 283, 284, 285, 286, 287, 288 and 289, wherein the compound comprises a diagnostically active nuclide, preferably a diagnostically active radionuclide.

Embodiment 291. The compound or pharmaceutically acceptable salt or hydrate or pharmaceutically acceptable solvate for use of Embodiment 290, wherein the diagnostically active radionuclide is selected from the group consisting of Al-¹⁸F, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I, preferably selected from the group consisting of Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb, and more preferably selected from the group consisting of ⁶⁴Cu, ⁶⁸Ga, ¹¹¹In and ²⁰³Pb.

Embodiment 292. The compound or pharmaceutically acceptable salt or hydrate or pharmaceutically acceptable solvate for use of any one of Embodiments 273, 274, 275, 276, 277, 278, 279, 280, 281, 282, 283, 284, 285, 286, 287, 288, 289, 290 and 291, wherein the method for the diagnosis is an imaging method.

Embodiment 293. The compound or pharmaceutically acceptable salt or hydrate or pharmaceutically acceptable solvate for use of Embodiment 292, wherein the imaging method is selected from the group consisting of scintigraphy, Single Photon Emission Computed Tomography (SPECT) and Positron Emission Tomography (PET).

Embodiment 294. The compound or pharmaceutically acceptable salt or hydrate or pharmaceutically acceptable solvate for use of any one of Embodiments 273, 274, 275, 276, 277, 278, 279, 280, 281, 282, 283, 284, 285, 286, 287, 288, 289, 290, 291, 292 and 293, wherein the method comprises the administration of a diagnostically effective amount of the compound to a subject, preferably to a mammal, wherein the mammal is selected from the group comprising man, companion animals, pets, and livestock, more preferably the subject is selected from the group comprising man, dog, cat, horse, and cow, and most preferably the subject is a human being.

Embodiment 295. The compound or pharmaceutically acceptable salt or hydrate or pharmaceutically acceptable solvate for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, for use in a method for delivering an effector to urokinase plasminogen activator surface receptor (uPAR), preferably human urokinase plasminogen activator surface receptor (uPAR), wherein the effector is selected from the group comprising a diagnostically active agent and a therapeutically active agent.

Embodiment 296. The compound or pharmaceutically acceptable salt or hydrate or pharmaceutically acceptable solvate for use of Embodiment 295, the effector is selected from the group comprising a diagnostically active nuclide and a therapeutically active nuclide.

Embodiment 297. The compound or pharmaceutically acceptable salt or hydrate or pharmaceutically acceptable solvate for use of Embodiment 296, wherein the diagnostically active nuclide is a diagnostically active radionuclide.

Embodiment 298. The compound or pharmaceutically acceptable salt or hydrate or pharmaceutically acceptable solvate for use of Embodiment 297, wherein the diagnostically active radionuclide is selected from the group consisting of Al-¹⁸F, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I, preferably selected from the group consisting of Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb, and more preferably selected from the group consisting of ⁶⁴Cu, ⁶⁸Ga, ¹¹¹In and ²⁰³Pb.

Embodiment 299. The compound or pharmaceutically acceptable salt or hydrate or pharmaceutically acceptable solvate for use of any one of Embodiments 295, 296, 297 and 298, wherein the urokinase plasminogen activator surface receptor (uPAR) is expressed by a cell, preferably a tumor cell, a cancer cell, a fibroblast, a macrophage, a neutrophil, an endothelial cell or a senescent cell, more preferably a human tumor cell, a human cancer cell, a human fibroblast, a human macrophage, a human neutrophil, a human endothelial cell or a human senescent cell, most preferably a human tumor cell expressing urokinase plasminogen activator surface receptor (uPAR), a human cancer cell expressing urokinase plasminogen activator surface receptor (uPAR), a human fibroblast expressing urokinase plasminogen activator surface receptor (uPAR), a human macrophage, a human neutrophil expressing urokinase plasminogen activator surface receptor (uPAR), a human endothelial cell expressing urokinase plasminogen activator surface receptor (uPAR) or a human senecscent cell expressing urokinase plasminogen activator surface receptor (uPAR).

Embodiment 300. The compound or pharmaceutically acceptable salt or hydrate or pharmaceutically acceptable solvate for use of Embodiment 299, wherein the cell is contained in or part of a tissue, preferably a diseased tissue of a subject suffering from a disease.

Embodiment 301. The compound or pharmaceutically acceptable salt or hydrate or pharmaceutically acceptable solvate for use of Embodiment 300, wherein the disease involves cells showing upregulated expression of urokinase plasminogen activator surface receptor (uPAR), preferably diseased tissue containing cells showing upregulated expression of urokinase plasminogen activator surface receptor (uPAR), more preferably disease involving tumor associated fibroblasts.

Embodiment 302. The compound or pharmaceutically acceptable salt or hydrate or pharmaceutically acceptable solvate for use of Embodiment 301, wherein the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

Embodiment 303. The compound or pharmaceutically acceptable salt or hydrate or pharmaceutically acceptable solvate for use of any one of Embodiments 301 and 302, wherein the disease involves cells showing upregulated expression of urokinase plasminogen activator surface receptor (uPAR), preferably diseased tissue contains cells showing upregulated expression of urokinase plasminogen activator surface receptor (uPAR), more preferably the disease involves tumor-associated fibroblasts, tumor-associated macrophages, tumor-associated neutrophils, tumor-associated endothelial cells, disseminated tumor cells and/or senescent cells, whereby the senescent cells are preferably associated with lung fibrosis, atherosclerosis, Alzheimer's disease, diabetes, liver fibrosis, chronic kidney disease, osteoarthritis, cardiac fibrosis, and Parkinson's disease.

Embodiment 304. The compound or pharmaceutically acceptable salt or hydrate or pharmaceutically acceptable solvate for use of any one of Embodiments 301, 302 and 303, wherein the disease is a neoplasm, preferably a cancer or tumor.

Embodiment 305. The compound or pharmaceutically acceptable salt or hydrate or pharmaceutically acceptable solvate for use of Embodiment 304, wherein a cell or cells of the neoplasm, the cancer and/or the tumor expresses urokinase plasminogen activator surface receptor (uPAR).

Embodiment 306. The compound or pharmaceutically acceptable salt or hydrate or pharmaceutically acceptable solvate for use of any one of Embodiments 301, 302, 303, 304 and 305, wherein the neoplasm, the cancer and/or the tumor are each and individually selected from the group comprising solid tumors and non-solid tumors.

Embodiment 307. The compound or pharmaceutically acceptable salt or hydrate or pharmaceutically acceptable solvate for use of any one of Embodiments 301, 302, 303, 304, 305 and 306, wherein the neoplasm, the cancer and/or the tumor are each and individually selected from the group comprising adrenocortical carcinoma, bladder cancer, bladder urothelial carcinoma, breast cancer, breast invasive carcinoma, cervical squamous cell carcinoma, cholangiocarcinoma, colon adenocarcinoma, colorectal cancer, diffuse large B-cell lymphoma, gastric cancer, endocervical adenocarcinoma, esophageal carcinoma, glioblastoma, glioblastoma multiforme, brain lower grade glioma, haematological malignancies such as acute myeloid leukaemia (AML), multiple myeloma (MM), head and neck squamous cell carcinoma, hepatocellular carcinoma (in particular at the invasion front), kidney chromophobe, kidney renal clear cell carcinoma, kidney renal papillary cell carcinoma, liver hepatocellular carcinoma, lung adenocarcinoma, lung cancer such as non-small lung carcinoma (NSCLC) and squamous cell carcinoma (SCC), lymphoid neoplasm, mesothelioma, ovarian cancer, ovarian serous cystadenocarcinoma, pancreatic cancer (in particular tumor cells associated with invasion), pancreatic adenocarcinoma, paraganglioma, prostate cancer, prostate adenocarcinoma, pheochromocytoma, lymph node metastases of prostate cancer rectum adenocarcinoma, sarcoma, skin cutaneous melanoma, stomach adenocarcinoma, testicular germ cell tumors, thyroid carcinoma, thymoma, uterine corpus endometrial carcinoma, uterine carcinosarcoma, and uveal melanoma.

Embodiment 308. The compound or pharmaceutically acceptable salt or hydrate or pharmaceutically acceptable solvate for use of any one of Embodiments 301, 302, 303, 304, 305, 306 and 307, wherein the neoplasm, the cancer and/or the tumor shows at least one characteristic selected from the group comprising aggressive growth, postoperative progression, metastasis and poor response to therapy.

Embodiment 309. The compound or pharmaceutically acceptable salt or hydrate or pharmaceutically acceptable solvate for use of any one of Embodiments 301, 302 and 303, wherein the disease is selected from the group comprising a cardiac disease, a liver disease, a lung disease, an autoimmune disease, a neurological disorder and a senescence related disease.

Embodiment 310. The compound or pharmaceutically acceptable salt or hydrate or pharmaceutically acceptable solvate for use of Embodiment 309, wherein the disease is a cardiac disease, preferably the cardiac disease is cardiac fibrosis.

Embodiment 311. The compound or pharmaceutically acceptable salt or hydrate or pharmaceutically acceptable solvate for use of Embodiment 309, wherein the disease is a liver disease, preferably the liver disease is selected from the group comprising chronic liver disease and hepatitis B.

Embodiment 312. The compound or pharmaceutically acceptable salt or hydrate or pharmaceutically acceptable solvate for use of Embodiment 309, wherein the disease is a lung disease, preferably the lung disease is selected from the group comprising cystic fibrosis, COPD and idiopathic pulmonary fibrosis.

Embodiment 313. The compound or pharmaceutically acceptable salt or hydrate or pharmaceutically acceptable solvate for use of Embodiment 309, wherein the disease is an autoimmune disease, preferably the autoimmune disease is selected from the group comprising glomerulosclerosis, systemic sclerosis, arthritis, rheumatoid arthritis and allergic conjunctivitis.

Embodiment 314. The compound or pharmaceutically acceptable salt or hydrate or pharmaceutically acceptable solvate for use of Embodiment 309, wherein the disease is a neurological disorder, preferably the neurological disorder is selected from the group comprising autism spectrum disorder and epileptogenic tissue remodelling.

Embodiment 315. The compound or pharmaceutically acceptable salt or hydrate or pharmaceutically acceptable solvate for use of Embodiment 309, wherein the disease is senescence related diseases, whereby the senescence related diseases is selected from the group comprising lung fibrosis, atherosclerosis, Alzheimer's disease, diabetes, liver fibrosis, chronic kidney disease, osteoarthritis, cardiac fibrosis, and Parkinson's disease.

Embodiment 316. The compound or pharmaceutically acceptable salt or hydrate or pharmaceutically acceptable solvate for use of Embodiment 296, wherein the therapeutically diagnostically active nuclide is a therapeutically active radionuclide.

Embodiment 317. The compound or pharmaceutically acceptable salt or hydrate or pharmaceutically acceptable solvate for use of Embodiment 316, wherein the therapeutically active radionuclide is selected from the group consisting therapeutically active radionuclide selected from the group consisting of ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹²⁵I, ¹³¹I, and ²¹¹At, preferably from the group consisting of ⁶⁷Cu, ⁹⁰Y, ¹⁴⁹Tb, ¹⁶¹Tb, ¹⁷⁷Lu, ²¹²Pb, ²¹³Bi, ²²⁵Ac, ²²⁷Th, and more preferably from the group consisting of ⁹⁰Y, ¹⁷⁷Lu, ²¹²Pb and ²²⁵Ac.

Embodiment 318. The compound or pharmaceutically acceptable salt or hydrate or pharmaceutically acceptable solvate for use of any one of Embodiments 316 and 317, wherein the urokinase plasminogen activator surface receptor (uPAR) is expressed by a cell, preferably a tumor cell, a cancer cell, a fibroblast, a macrophage, a neutrophil, an endothelial cell or a senescent cell, more preferably a human tumor cell, a human cancer cell, a human fibroblast, a human macrophage, a human neutrophil, a human endothelial cell or a human senescent cell, most preferably a human tumor cell expressing urokinase plasminogen activator surface receptor (uPAR), a human cancer cell expressing urokinase plasminogen activator surface receptor (uPAR), a human fibroblast expressing urokinase plasminogen activator surface receptor (uPAR), a human macrophage, a human neutrophil expressing urokinase plasminogen activator surface receptor (uPAR), a human endothelial cell expressing urokinase plasminogen activator surface receptor (uPAR) or a human senecscent cell expressing urokinase plasminogen activator surface receptor (uPAR).

Embodiment 319. The compound or pharmaceutically acceptable salt or hydrate or pharmaceutically acceptable solvate for use of Embodiment 318, wherein the cell is contained in or part of a tissue, preferably a diseased tissue of a subject suffering from a disease.

Embodiment 320. The compound or pharmaceutically acceptable salt or hydrate or pharmaceutically acceptable solvate for use of Embodiment 319, wherein the disease involves cells showing upregulated expression of urokinase plasminogen activator surface receptor (uPAR), preferably diseased tissue containing cells showing upregulated expression of urokinase plasminogen activator surface receptor (uPAR), more preferably disease involving tumor associated fibroblasts.

Embodiment 321. The compound or pharmaceutically acceptable salt or hydrate or pharmaceutically acceptable solvate for use of Embodiment 320, wherein the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

Embodiment 322. The compound or pharmaceutically acceptable salt or hydrate or pharmaceutically acceptable solvate for use of any one of Embodiments 320 and 321, wherein the disease involves cells showing upregulated expression of urokinase plasminogen activator surface receptor (uPAR), preferably diseased tissue contains cells showing upregulated expression of urokinase plasminogen activator surface receptor (uPAR), more preferably the disease involves tumor-associated fibroblasts, tumor-associated macrophages tumor-associated neutrophils, tumor-associated endothelial cells, disseminated tumor cells and/or senescent cells, whereby the senescent cells are preferably associated with lung fibrosis, atherosclerosis, Alzheimer's disease, diabetes, liver fibrosis, chronic kidney disease, osteoarthritis, cardiac fibrosis, and Parkinson's disease.

Embodiment 323. The compound or pharmaceutically acceptable salt or hydrate or pharmaceutically acceptable solvate for use of any one of Embodiments 320, 321 and 322, wherein the disease is a neoplasm, preferably a cancer or tumor.

Embodiment 324. The compound or pharmaceutically acceptable salt or hydrate or pharmaceutically acceptable solvate for use of Embodiment 323, wherein a cell or cells of the neoplasm, the cancer and/or the tumor expresses urokinase plasminogen activator surface receptor (uPAR).

Embodiment 325. The compound or pharmaceutically acceptable salt or hydrate or pharmaceutically acceptable solvate for use of any one of Embodiments 320, 321, 322, 323 and 324, wherein the neoplasm, the cancer and/or the tumor are each and individually selected from the group comprising solid tumors and non-solid tumors.

Embodiment 326. The compound or pharmaceutically acceptable salt or hydrate or pharmaceutically acceptable solvate for use of any one of Embodiments 320, 321, 322, 323, 324 and 325, wherein the neoplasm, the cancer and/or the tumor are each and individually selected from the group comprising adrenocortical carcinoma, bladder cancer, bladder urothelial carcinoma, breast cancer, breast invasive carcinoma, cervical squamous cell carcinoma, cholangiocarcinoma, colon adenocarcinoma, colorectal cancer, diffuse large B-cell lymphoma, gastric cancer, endocervical adenocarcinoma, esophageal carcinoma, glioblastoma, glioblastoma multiforme, brain lower grade glioma, haematological malignancies such as acute myeloid leukaemia (AML), multiple myeloma (MM), head and neck squamous cell carcinoma, hepatocellular carcinoma (in particular at the invasion front), kidney chromophobe, kidney renal clear cell carcinoma, kidney renal papillary cell carcinoma, liver hepatocellular carcinoma, lung adenocarcinoma, lung cancer such as non-small lung carcinoma (NSCLC) and squamous cell carcinoma (SCC), lymphoid neoplasm, mesothelioma, ovarian cancer, ovarian serous cystadenocarcinoma, pancreatic cancer (in particular tumor cells associated with invasion), pancreatic adenocarcinoma, paraganglioma, prostate cancer, prostate adenocarcinoma, pheochromocytoma, lymph node metastases of prostate cancer rectum adenocarcinoma, sarcoma, skin cutaneous melanoma, stomach adenocarcinoma, testicular germ cell tumors, thyroid carcinoma, thymoma, uterine corpus endometrial carcinoma, uterine carcinosarcoma, and uveal melanoma.

Embodiment 327. The compound or pharmaceutically acceptable salt or hydrate or pharmaceutically acceptable solvate for use of any one of Embodiments 320, 321, 322, 323, 324, 325 and 326, wherein the neoplasm, the cancer and/or the tumor shows at least one characteristic selected from the group comprising aggressive growth, postoperative progression, metastasis and poor response to therapy.

Embodiment 328. The compound or pharmaceutically acceptable salt or hydrate or pharmaceutically acceptable solvate for use of any one of Embodiments 320, 321 and 322, wherein the disease is selected from the group comprising a cardiac disease, a liver disease, a lung disease, an autoimmune disease, a neurological disorder and a senescence related disease.

Embodiment 329. The compound or pharmaceutically acceptable salt or hydrate or pharmaceutically acceptable solvate for use of Embodiment 328, wherein the disease is a cardiac disease, preferably the cardiac disease is cardiac fibrosis.

Embodiment 330. The compound or pharmaceutically acceptable salt or hydrate or pharmaceutically acceptable solvate for use of Embodiment 328, wherein the disease is a liver disease, preferably the liver disease is selected from the group comprising chronic liver disease and hepatitis B.

Embodiment 331. The compound or pharmaceutically acceptable salt or hydrate or pharmaceutically acceptable solvate for use of Embodiment 328, wherein the disease is a lung disease, preferably the lung disease is selected from the group comprising cystic fibrosis, COPD and idiopathic pulmonary fibrosis.

Embodiment 332. The compound or pharmaceutically acceptable salt or hydrate or pharmaceutically acceptable solvate for use of Embodiment 328, wherein the disease is an autoimmune disease, preferably the autoimmune disease is selected from the group comprising glomerulosclerosis, systemic sclerosis, arthritis, rheumatoid arthritis, atherosclerosis and allergic conjunctivitis.

Embodiment 333. The compound or pharmaceutically acceptable salt or hydrate or pharmaceutically acceptable solvate for use of Embodiment 328, wherein the disease is a neurological disorder, preferably the neurological disorder is selected from the group comprising autism spectrum disorder and epileptogenic tissue remodelling.

Embodiment 334. The compound or pharmaceutically acceptable salt or hydrate or pharmaceutically acceptable solvate for use of Embodiment 328, wherein the disease is senescence related diseases, whereby the senescence related diseases is selected from the group comprising lung fibrosis, atherosclerosis, Alzheimer's disease, diabetes, liver fibrosis, chronic kidney disease, osteoarthritis, cardiac fibrosis, and Parkinson's disease.

Embodiment 335. A composition, preferably a pharmaceutical composition, wherein the composition comprises a compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, and a pharmaceutically acceptable excipient.

Embodiment 336. The composition of Embodiment 335, for use in any method as defined in the preceding Embodiments.

Embodiment 337. The compound or pharmaceutically acceptable salt or hydrate or pharmaceutically acceptable solvate of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, for use in a method of diagnosing and/or treating a disease, preferably cancer, and more preferably a uPAR expressing cancer.

Embodiment 338. A method for the diagnosis of a disease in a subject, wherein the method comprises administering to the subject a diagnostically effective amount of a compound or pharmaceutically acceptable salt or hydrate or pharmaceutically acceptable solvate according to any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233.

Embodiment 339. The method of Embodiment 338, wherein the compound or pharmaceutically acceptable salt or hydrate or pharmaceutically acceptable solvate comprises a diagnostically active agent, whereby the agent is preferably a diagnostically active radionuclide.

Embodiment 340. A method for the treatment a subject suffering from a disease, wherein the method comprises administering to the subject a therapeutically effective amount of a compound or pharmaceutically acceptable salt or hydrate or pharmaceutically acceptable solvate according to any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233.

Embodiment 341. The method of Embodiment 340, wherein the compound or pharmaceutically acceptable salt or hydrate or pharmaceutically acceptable solvate comprises a therapeutically active agent, whereby the agent is preferably a therapeutically active radionuclide.

Embodiment 342. The method of any one of Embodiments 338, 339, 340 and 341, wherein the disease involves cells showing upregulated expression of urokinase plasminogen activator surface receptor (uPAR), preferably diseased tissue containing cells showing upregulated expression of urokinase plasminogen activator surface receptor (uPAR), more preferably disease involving tumor associated fibroblasts.

Embodiment 343. The compound or pharmaceutically acceptable salt or hydrate or pharmaceutically acceptable solvate for use of Embodiment 342, wherein the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

Embodiment 344. The compound or pharmaceutically acceptable salt or hydrate or pharmaceutically acceptable solvate for use of any one of Embodiments 342 and 343, wherein the disease involves cells showing upregulated expression of urokinase plasminogen activator surface receptor (uPAR), preferably diseased tissue contains cells showing upregulated expression of urokinase plasminogen activator surface receptor (uPAR), more preferably the disease involves tumor-associated fibroblasts, tumor-associated macrophages, tumor-associated neutrophils, tumor-associated endothelial cells, disseminated tumor cells and/or senescent cells, whereby the senescent cells are preferably associated with lung fibrosis, atherosclerosis, Alzheimer's disease, diabetes, liver fibrosis, chronic kidney disease, osteoarthritis, cardiac fibrosis, and Parkinson's disease.

Embodiment 345. The compound or pharmaceutically acceptable salt or hydrate or pharmaceutically acceptable solvate for use of any one of Embodiments 342, 343 and 344, wherein the disease is a neoplasm, preferably a cancer or tumor.

Embodiment 346. The compound or pharmaceutically acceptable salt or hydrate or pharmaceutically acceptable solvate for use of Embodiment 345, wherein a cell or cells of the neoplasm, the cancer and/or the tumor expresses urokinase plasminogen activator surface receptor (uPAR).

Embodiment 347. The compound or pharmaceutically acceptable salt or hydrate or pharmaceutically acceptable solvate for use of any one of Embodiments 342, 343, 344, 345 and 346, wherein the neoplasm, the cancer and/or the tumor are each and individually selected from the group comprising solid tumors and non-solid tumors.

Embodiment 348. The compound or pharmaceutically acceptable salt or hydrate or pharmaceutically acceptable solvate for use of any one of Embodiments 342, 343, 344, 345, 346 and 347, wherein the neoplasm, the cancer and/or the tumor are each and individually selected from the group comprising adrenocortical carcinoma, bladder cancer, bladder urothelial carcinoma, breast cancer, breast invasive carcinoma, cervical squamous cell carcinoma, cholangiocarcinoma, colon adenocarcinoma, colorectal cancer, diffuse large B-cell lymphoma, gastric cancer, endocervical adenocarcinoma, esophageal carcinoma, glioblastoma, glioblastoma multiforme, brain lower grade glioma, haematological malignancies such as acute myeloid leukaemia (AML), multiple myeloma (MM), head and neck squamous cell carcinoma, hepatocellular carcinoma (in particular at the invasion front), kidney chromophobe, kidney renal clear cell carcinoma, kidney renal papillary cell carcinoma, liver hepatocellular carcinoma, lung adenocarcinoma, lung cancer such as non-small lung carcinoma (NSCLC) and squamous cell carcinoma (SCC), lymphoid neoplasm, mesothelioma, ovarian cancer, ovarian serous cystadenocarcinoma, pancreatic cancer (in particular tumor cells associated with invasion), pancreatic adenocarcinoma, paraganglioma, prostate cancer, prostate adenocarcinoma, pheochromocytoma, lymph node metastases of prostate cancer rectum adenocarcinoma, sarcoma, skin cutaneous melanoma, stomach adenocarcinoma, testicular germ cell tumors, thyroid carcinoma, thymoma, uterine corpus endometrial carcinoma, uterine carcinosarcoma, and uveal melanoma.

Embodiment 349. The compound or pharmaceutically acceptable salt or hydrate or pharmaceutically acceptable solvate for use of any one of Embodiments 342, 343, 344, 345, 346, 347 and 348, wherein the neoplasm, the cancer and/or the tumor shows at least one characteristic selected from the group comprising aggressive growth, postoperative progression, metastasis and poor response to therapy.

Embodiment 350. The compound or pharmaceutically acceptable salt or hydrate or pharmaceutically acceptable solvate for use of any one of Embodiments 342, 343 and 344, wherein the disease is selected from the group comprising a cardiac disease, a liver disease, a lung disease, an autoimmune disease, a neurological disorder and a senescence related disease.

Embodiment 351. The compound or pharmaceutically acceptable salt or hydrate or pharmaceutically acceptable solvate for use of Embodiment 350, wherein the disease is a cardiac disease, preferably the cardiac disease is cardiac fibrosis.

Embodiment 352. The compound or pharmaceutically acceptable salt or hydrate or pharmaceutically acceptable solvate for use of Embodiment 350, wherein the disease is a liver disease, preferably the liver disease is selected from the group comprising chronic liver disease and hepatitis B.

Embodiment 353. The compound or pharmaceutically acceptable salt or hydrate or pharmaceutically acceptable solvate for use of Embodiment 350, wherein the disease is a lung disease, preferably the lung disease is selected from the group comprising cystic fibrosis, COPD and idiopathic pulmonary fibrosis.

Embodiment 354. The compound or pharmaceutically acceptable salt or hydrate or pharmaceutically acceptable solvate for use of Embodiment 350, wherein the disease is an autoimmune disease, preferably the autoimmune disease is selected from the group comprising glomerulosclerosis, systemic sclerosis, arthritis, rheumatoid arthritis atherosclerosis and allergic conjunctivitis.

Embodiment 355. The compound or pharmaceutically acceptable salt or hydrate or pharmaceutically acceptable solvate for use of Embodiment 350, wherein the disease is a neurological disorder, preferably the neurological disorder is selected from the group comprising autism spectrum disorder and epileptogenic tissue remodelling.

Embodiment 356. The compound or pharmaceutically acceptable salt or hydrate or pharmaceutically acceptable solvate for use of Embodiment 350, wherein the disease is senescence related diseases, whereby the senescence related diseases is selected from the group comprising lung fibrosis, atherosclerosis, Alzheimer's disease, diabetes, liver fibrosis, chronic kidney disease, osteoarthritis, cardiac fibrosis, and Parkinson's disease.

Embodiment 357. A kit comprising a compound according to any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233, one or more optional excipient(s) and optionally one or more device(s), whereby the device(s) is/are selected from the group comprising a labeling device, a purification device, a handling device, a radioprotection device, an analytical device or an administration device.

Embodiment 358. The kit of Embodiment 357, for use in any method as defined in any of the preceding Embodiments.

In an embodiment and as preferably used herein, the term "linkage" refers an attachment of two atoms of two independent moieties. A preferred linkage is a chemical bond or a plurality of chemical bonds. More preferably, a chemical bond is a covalent bond or a plurality of chemical bonds. Most preferably, the linkage is a covalent bond or a coordinate bond. As preferably used herein, an embodiment of a coordinate bond is a bond or group of bonds as realized when a metal is bound (e.g., chelated) by a chelator. Depending on the type of atoms linked and their atomic environment different types of linkages are created. These types of linkage are defined by the type of atom arrangements created by the linkage.

For instance, the linking of a moiety comprising an amine with a moiety comprising a carboxylic acid leads to a linkage named "amide" (which may also be referred to as amide linkage, -CO-N-, -N-CO-). It will be acknowledged by a person skilled in the art that this and the following examples of creating linkages are only prototypical examples and are by no means limiting the scope of the present invention. It will be acknowledged by a person in the art that the linking of a moiety comprising an isothiocyanate with a moiety comprising an amine leads to a thiourea (which may also be referred to as a thiourea linkage, -N-C(=S)-N-), and linking of a moiety comprising a C atom with a moiety comprising a thiol-group (-C-SH) leads to a thioether (which may also be referred to as a thioether linkage, -C-S-C-). A non-limiting list of linkages as used, for example, in connection with the effector and linker of the invention and their characteristic type of atom arrangement is given Table 2.

**Table 2: List of linkages**

| **Linkage** | **Characteristic atom arrangement** | **Linkage** | **Characteristic atom arrangement** |
|---|---|---|---|
| Amide | 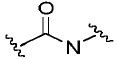 | Ether | 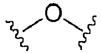 |
| Sulfonamide | 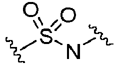 | Ester | 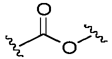 |
| Urea | 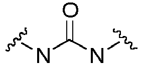 | Carbamate | 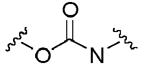 |
| Thioether | 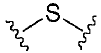 | Thiourea | 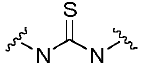 |
| Disulfide | 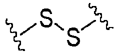 | Triazole | 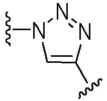 |

Examples of reactive groups which, in some embodiments of the invention, are used in the formation of linkages, for example between the effector and the linker or directly between the effector and the rest of the compound of the invention (peptide), are summarized in Table 3. It will, however, be understood by a person skilled in the art that neither the linkages which may be realized in embodiments of the present invention are limited to the ones of Table 3 nor the reactive groups forming such linkages.

**Table 3: Selected reactive groups as used in the formation of linkages**

| **first reactive group** | **second reactive group** | **(type of) linkage** |
|---|---|---|
| amino | carboxylic acid (carboxyl) | amide |
| amino | activated carboxylic acid | amide |
| amino | sulfonyl halide | sulfonamide |
| amino | isocyanate | urea |
| amino | p-nitrophenylcarbamate | urea |
| sulfhydryl | Michael acceptor (e.g. maleimide) | thioether |
| bromo | sulfhydryl | thioether |
| isothiocyanate | amino | thiourea |
| hydroxyl | carboxylic acid (carboxyl) | ester |
| azide | alkyne | triazole |
| sulfhydryl | sulfhydryl | disulfide |
| sulfhydryl | 2-pyridine-disulfide | disulfide |
| carbonate ester | amino | carbamate |
| chloroformate | amino | carbamate |
| bromo | hydroxyl | ether |

The following are reactive groups and functionalities which are utilized or amenable of forming linkages between moieties or structures as used in embodiments of the present invention: Primary or secondary amino, carboxylic acid (carboxyl), activated carboxylic acid, chloro, bromo, iodo, sulfhydryl, hydroxyl, sulfonic acid, activated sulfonic acid, sulfonic acid esters like mesylate or tosylate, Michael acceptors, strained alkenes like *trans* cyclooctene, isocyanate, isothiocyanate, azide, alkyne and tetrazine.

In an embodiment and as preferably used herein, the term **"activated carboxylic acid"** refers to a carboxylic acid group with the general formula -CO-X, wherein X is a leaving group. For example, activated forms of a carboxylic acid group may include, but are not limited to, acyl chlorides, symmetrical or unsymmetrical anhydrides, and esters. Preferably, the activated carboxylic acid group is an ester with pentafluorophenol, nitrophenol, benzotriazole, azabenzotriazole, thiophenol or N-hydroxysuccinimide (NHS) as leaving group.

In an embodiment and as preferably used herein, the expression **"mediating a linkage"** means that a linkage or a type of linkage is established, preferably a linkage between two moieties. In a preferred embodiment, the linkage and the type of linkage is as defined herein.

Unless indicated otherwise, when it is referred in the present disclosure to a range indicated by a lower integer and a higher integer such as, for example, 1-4, such range is a representation of the lower integer, the higher integer and any integer between the lower integer and the higher integer. Insofar, the range is actually an individualized disclosure of said integer. In said example, the range of 1-4 thus means 1, 2, 3 and 4.

In an embodiment and as preferably used herein, the term **"alkyl"** characterizes a saturated, straight-chain or branched hydrocarbon group and may be accompanied by a qualifier which specifies the number of carbon atoms it contains. For example, the expression (C₁-C₃)alkyl means each and individually any of methyl, ethyl, n-propyl and isopropyl. An alkyl group can be unsubstituted or substituted with one or more groups, including, but not limited to, -O-[(C₁-C₈)alkyl], -aryl, -CO-R', -O-CO-R', -CO-OR', -CO-NH₂, -CO-NHR', -CO-NR'₂, -NH-CO-R', -SOz-R', -SO-R', -OH, -halogen, -N₃, -NH₂, -NHR', -NR'₂ and -CN; where each R' is independently selected from -(C₁-C₈)alkyl and aryl. If the alkyl group comprises CH₂ groups, a CH₂ group can optionally be replaced by an S or O atom.

In an embodiment and as preferably used herein, the term **"(C₁-C₂)alkyl"** refers any of methyl and ethyl.

In an embodiment and as preferably used herein, the term **"(C₁-C₃)alkyl"** refers to any of methyl, ethyl, n-propyl and isopropyl.

In an embodiment and as preferably used herein, the term **"(C₁-C₄)alkyl"** refers to a saturated or unsaturated, straight-chain or branched hydrocarbon group having from 1 to 4 carbon atoms. Representative examples of (C₁-C₄)alkyl include, but are not limited to, any of methyl, ethyl, n-propyl, isopropyl, n-butyl, isobutyl, sec-butyl and tert-butyl.

In an embodiment and as preferably used herein, the term **"(C₁-C₅)alkyl"** refers to a saturated or unsaturated, straight-chain or branched hydrocarbon group having from 1 to 5 carbon atoms. Representative examples of (C₁-C₅)alkyl include, but are not limited to, any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl and 2,2-dimethylpropyl.

In an embodiment and as preferably used herein, the term **"(C₁-C₆)alkyl"** refers to a saturated or unsaturated, straight-chain or branched hydrocarbon group having from 1 to 6 carbon atoms. Representative examples of (C₁-C₆)alkyl include, but are not limited to, any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl. A (C₁-C₆)alkyl group can be unsubstituted or substituted with one or more groups, including, but not limited to, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₁-C₈)alkyl"** refers to a saturated or unsaturated, straight-chain or branched hydrocarbon group having from 1 to 8 carbon atoms. Representative (C₁-C₈)alkyl groups include, but are not limited to, any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethyl-butyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methyl-hept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl and 2-ethyl-3-methyl-pentyl. A (C₁-C₈)alkyl group can be unsubstituted or substituted with one or more groups, including, but not limited to, -O-[(C₁-C₈)alkyl], -aryl, -CO-R', -O-CO-R', -CO-OR', -CO-NH₂, -CO-NHR', -CO-NR'₂, -NH-COR', -SO₂-R', -SO-R', -OH, -N₃, -NH₂, -NHR', -NR'₂ and -CN; where each R' is independently selected from -(C₁-C₈)alkyl and aryl.

In an embodiment and as preferably used herein, the term **"(C₂-C₃)alkyl"** refers to a saturated or unsaturated, straight-chain or branched hydrocarbon group having from 2 to 3 carbon atoms. Representative examples of (C₂-C₃)alkyl include, but are not limited to, any of ethyl, n-propyl, and isopropyl.

In an embodiment and as preferably used herein, the term **"(C₂-C₄)alkyl"** refers to a saturated or unsaturated, straight-chain or branched hydrocarbon group having from 2 to 4 carbon atoms. Representative examples of (C₂-C₄)alkyl include, but are not limited to, any of ethyl, n-propyl, isopropyl, n-butyl, isobutyl, sec-butyl and tert-butyl.

In an embodiment and as preferably used herein, the term **"(C₂-C₅)alkyl"** refers to a saturated or unsaturated, straight-chain or branched hydrocarbon group having from 2 to 5 carbon atoms. Representative examples of (C₂-C₅)alkyl include, but are not limited to, any of ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl and 2,2-dimethylpropyl.

In an embodiment and as preferably used herein, the term **"(C₂-C₆)alkyl"** refers to a saturated or unsaturated, straight-chain or branched hydrocarbon group having from 2 to 6 carbon atoms. Representative examples of (C₂-C₆)alkyl include, but are not limited to, any of ethyl, n-propyl, isopropyl, n-butyl, isobutyl, *sec*-butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl. A (C₂-C₆)alkyl group can be unsubstituted or substituted with one or more groups, including, but not limited to, -(C₁-C₈)alkyl, -O-[(Ci-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₂-C₈)alkyl"** refers to a saturated or unsaturated, straight-chain or branched hydrocarbon group having from 2 to 8 carbon atoms. Representative (C₂-C₈)alkyl groups include, but are not limited to, any of ethyl, n-propyl, isopropyl, n-butyl, isobutyl, *sec-*butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethyl-butyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methyl-hept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl and 2-ethyl-3-methyl-pentyl. A (C₂-C₈)alkyl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₃-C₆)alkyl"** refers to a saturated or unsaturated, straight-chain or branched hydrocarbon group having from 3 to 6 carbon atoms. Representative examples of (C₃-C₆)alkyl include, but are not limited to, any of n-propyl, isopropyl, n-butyl, isobutyl, *sec*-butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl. A (C₃-C₆)alkyl group can be unsubstituted or substituted with one or more groups, including, but not limited to, -(C₁-C₈)alkyl, -O-[(Ci-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₃-C₉)alkyl"** refers to a saturated or unsaturated, straight-chain or branched hydrocarbon group having from 3 to 9 carbon atoms. Representative (C₃-C₉)alkyl groups include, but are not limited to, any of n-propyl, isopropyl, n-butyl, isobutyl, *sec*-butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethyl-butyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methyl-hept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl, 2-ethyl-3-methyl-pentyl, n-nonanyl, 2-nonanyl, 3-nonanyl, 4-nonanyl, 5-nonanyl, 2-methyl-octanyl, 2-methyl-octan-2-yl, 2-methyl-octan-3-yl, 2-methyl-octan-4-yl, 3-methyl-octanyl, 3-methyl-octan-2-yl, 3-methyl-octan-3-yl, 3-methyl-octan-4-yl, 4-methyl-octanyl, 4-methyl-octan-2-yl, 4-methyl-octan-3-yl, 4-methyl-octan-4-yl, 2,2-dimethyl-heptanyl, 2,2-dimethyl-heptan-3-yl, 2,2-dimethyl-heptan-4-yl, 2,3-dimethyl-heptanyl, 2,3-dimethyl-heptan-2-yl, 2,3-dimethyl-heptan-3-yl, 2,3-dimethyl-heptan-4-yl, 2,4-dimethyl-heptanyl, 2,4-dimethyl-heptan-2-yl, 2,4-dimethyl-heptan-3-yl, 2,4-dimethyl-heptan-4-yl, 2,5-dimethyl-heptanyl, 2,5-dimethyl-heptan-2-yl, 2,5-dimethyl-heptan-3-yl, 2,5-dimethyl-heptan-4-yl, 2,6-dimethyl-heptanyl, 2,6-dimethyl-heptan-2-yl, 2,6-dimethyl-heptan-3-yl, 2,6-dimethyl-heptan-4-yl, 3,3-dimethyl-heptanyl, 3,3-dimethyl-heptan-2-yl, 3,3-dimethyl-heptan-4-yl, 3,4-dimethyl-heptanyl, 3,4-dimethyl-heptan-2-yl, 3,4-dimethyl-heptan-3-yl, 3,4-dimethyl-heptan-4-yl, 3,5-dimethyl-heptanyl, 3,5-dimethyl-heptan-2-yl, 3,5-dimethyl-heptan-3-yl, 3,5-dimethyl-heptan-4-yl, 4,4-dimethyl-heptanyl, 4,4-dimethyl-heptan-2-yl, 4,4-dimethyl-heptan-3-yl, 3-ethyl-heptanyl, 3-ethyl-heptan-2-yl, 3-ethyl-heptan-3-yl, 3-ethyl-heptan-4-yl, 4-ethyl-heptanyl, 4-ethyl-heptan-2-yl, 4-ethyl-heptan-3-yl, 4-ethyl-heptan-4-yl, 2,2,3-trimethyl-hexanyl, 2,2,3-trimethyl-hexan-3-yl, 2,2,4-trimethyl-hexanyl, 2,2,4-trimethyl-hexan-3-yl, 2,2,5-trimethyl-hexanyl, 2,2,5-trimethyl-hexan-3-yl, 2,3,3-trimethyl-hexanyl, 2,3,3-trimethyl-hexan-2-yl, 2,3,4-trimethyl-hexanyl, 2,3,4-trimethyl-hexan-2-yl, 2,3,4-trimethyl-hexan-3-yl, 2,3,5-trimethyl-hexanyl, 2,3,5-trimethyl-hexan-2-yl, 2,3,5-trimethyl-hexan-3-yl, 2,4,4-trimethyl-hexanyl, 2,4,4-trimethyl-hexan-2-yl, 2,4,4-trimethyl-hexan-3-yl, 3,3,4-trimethyl-hexanyl, 3,3,4-trimethyl-hexan-2-yl, 3-ethyl-2-methyl-hexanyl, 3-ethyl-2-methyl-hexan-2-yl, 3-ethyl-2-methyl-hexan-3-yl, 4-ethyl-2-methyl-hexanyl, 4-ethyl-2-methyl-hexan-2-yl, 4-ethyl-2-methyl-hexan-3-yl, 3-ethyl-3-methyl-hexanyl, 3-ethyl-3-methyl-hexan-2-yl, 3-ethyl-4-methyl-hexanyl, 3-ethyl-4-methyl-hexan-2-yl, 3-ethyl-4-methyl-hexan-3-yl, 2,2,3,3-tetramethyl-pentanyl, 2,2,4,4-tetramethyl-pentanyl, 2,2,4,4-tetramethyl-pentan-3-yl, 2,3,3,4-tetramethyl-pentanyl, 2,3,3,4-tetramethyl-pentan-2-yl, 3-ethyl-2,2-dimethyl-pentanyl, 3-ethyl-2,2-dimethyl-pentan-3-yl, 3-ethyl-2,3 -dimethyl-pentanyl, 3 -ethyl-2,3 -dimethyl-pentan-2-yl, 3 -ethyl-2,4-dimethyl-pentanyl, 3 -ethyl-2,4-dimethyl-pentan-2-yl, 3-ethyl-2,4-dimethyl-pentan-3-yl, 3,3-diethyl-pentanyl and 3,3-diethyl-pentan-2-yl. A (C₃-C₉)alkyl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₄-C₆)alkyl"** refers to a saturated or unsaturated, straight-chain or branched hydrocarbon group having from 4 to 6 carbon atoms. Representative examples of (C₄-C₆)alkyl include, but are not limited to, any of n-butyl, isobutyl, *sec-*butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl. A (C₄-C₆)alkyl group can be unsubstituted or substituted with one or more groups, including, but not limited to, -(C₁-C₈)alkyl, -O-[(Ci-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"carbocycle"** refers to a saturated or unsaturated non-aromatic hydrocarbon ring and is usually accompanied by a qualifier which specifies the number of carbon atoms it may contain. For example, representative (C₃-C₄)carbocycles include but are not limited to any of a cyclopropyl and cyclobutyl group. A carbocycle may contain one ring or two or more rings that are fused together. A carbocycle can be unsubstituted or substituted with one or more groups, including, but not limited to, -(C₁-C₈)alkyl, -O-[(C₁-C₈)alkyl], -aryl, -CO-R', -O-CO-R', - CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl. A ring CH₂ group of the carbocycle can optionally be replaced by an S or O atom.

In an embodiment and as preferably used herein, the term **"heterocycle"** refers to a saturated or unsaturated non-aromatic heterocyclic ring and is usually accompanied by a qualifier which specifies the number of carbon atoms it may contain. For example, representative (C₅-C₆)heterocycles include but are not limited to any of a tetrahydrofuranyl, tetrahydrothiophenyl, pyrrolidinyl, oxathiolanyl, sulfolanyl, thianyl, tetrahydropyranyl, piperidinyl, morpholinyl, thiomorpholinyl, dioxanyl, dithianyl and trithianyl group. A heterocycle may contain one ring or two or more rings that are fused together, preferably it contains one ring.

In an embodiment and as preferably used herein, the term **"(C₃-C₄)carbocycle"** refers to a 3- or 4-membered saturated or unsaturated non-aromatic carbocyclic ring. Representative (C₃-C₄)carbocycles include, but are not limited to, any of a cyclopropyl, cyclobutyl, and cyclobutenyl group. A (C₃-C₄)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'z, -NH-CO-R', -SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₃-C₄)carbocycle ring can optionally be replaced by an S or O atom.

In an embodiment and as preferably used herein, the term **"(C₃-C₅)carbocycle"** refers to a 3-, 4- or 5-membered saturated or unsaturated non-aromatic carbocyclic ring. Representative (C₃-C₅)carbocycles include, but are not limited to, any of a cyclopropyl, cyclobutyl, cyclopentyl, cyclopropenyl, cyclobutenyl, cyclopentenyl and cyclopentadienyl group. A (C₃-C₅)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', - SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₃-C₅)carbocycle ring can optionally be replaced by an S or O atom.

In an embodiment and as preferably used herein, the term **"(C₃-C₆)carbocycle"** refers to a 3-, 4-, 5- or 6-membered saturated or unsaturated non-aromatic carbocyclic ring. Representative (C₃-C₆)carbocycles include, but are not limited to, any of a cyclopropyl, cyclobutyl, cyclopentyl, cyclopentadienyl, cyclohexyl, cyclohexenyl, 1,3-cyclohexadienyl, and 1,4-cyclohexadienyl group. A (C₃-C₆)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₃-C₇)carbocycle ring can optionally be replaced by an S or O atom.

In an embodiment and as preferably used herein, the term **"(C₃-C₇)carbocycle"** refers to a 3-, 4-, 5-, 6-or 7-membered saturated or unsaturated non-aromatic carbocyclic ring. Representative (C₃-C₇)carbocycles include, but are not limited to, any of a cyclopropyl, cyclobutyl, cyclobutenyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl and 1,3,5-cycloheptatrienyl group. A (C₃-C₇)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', - SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₃-C₇)carbocycle ring can optionally be replaced by an S or O atom.

In an embodiment and as preferably used herein, the term **"(C₃-C₈)carbocycle"** refers to a 3-, 4-, 5-, 6-, 7- or 8-membered saturated or unsaturated non-aromatic carbocyclic ring. Representative (C₃-C₈)carbocycles include, but are not limited to, any of a cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and cyclooctadienyl group. A (C₃-C₈)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'z, -NH-CO-R', -SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₃-C₈)carbocycle ring can optionally be replaced by an S or O atom.

In an embodiment and as preferably used herein, the term **"(C₄-C₆)carbocycle"** refers to a 4-, 5- or 6-membered saturated or unsaturated non-aromatic carbocyclic ring. Representative (C₄-C₆)carbocycles include, but are not limited to, any of a cyclobutyl, cyclopentyl, cyclopentadienyl, cyclohexyl, cyclohexenyl, 1,3-cyclohexadienyl, and 1,4-cyclohexadienyl group. A (C₄-C₆)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', - SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₃-C₇)carbocycle ring can optionally be replaced by an S or O atom.

In an embodiment and as preferably used herein, the term **"(C₄-C₈)carbocycle"** refers to a 4-, 5-, 6-, 7- or 8-membered saturated or unsaturated non-aromatic carbocyclic ring. Representative (C₄-C₈)carbocycles include, but are not limited to, any of a cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and cyclooctadienyl group. A (C₄-C₈)carbocycle group can be unsubstituted or substituted with one or more groups, including, but not limited to, (Ci-C₈)alkyl, -O-[(C₁-C₈)alkyl], -aryl, -CO-R', -O-CO-R', -CO-OR', -CO-NH₂, -CO-NHR', -CO-NR'₂, - NH-CO-R', -SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₄-C₈)carbocycle ring can optionally be replaced by an S or O atom.

In an embodiment and as preferably used herein, the term **"(C₅-C₆)carbocycle"** refers to a 5- or 6-membered saturated or unsaturated non-aromatic carbocyclic ring. Representative (C₅-C₆)carbocycles include, but are not limited to, any of a cyclopentyl, cyclopentenyl, cyclopentadienyl, cyclohexyl, cyclohexenyl, 1,3-cyclohexadienyl and 1,4-cyclohexadienyl group. A (C₅-C₆)carbocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', - SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₅-C₆)carbocycle ring can optionally be replaced by an S or O atom.

In an embodiment and as preferably used herein, the term **"(C₅-C₁₀)carbocycle"** refers to a 5-, 6-, 7-, 8-, 9 or 10-membered saturated or unsaturated non-aromatic carbocyclic ring or two fused carbocyclic rings with a total of 5, 6, 7, 8, 9 or 10 ring carbon atoms whereof at least one ring is non-aromatic. Representative (C₅-C₁₀)carbocycles include, but are not limited to, any of a cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, cyclooctadienyl, cyclononyl, cyclononenyl, cyclononadienyl cyclononatrienyl, cyclodecyl, cyclodecadienyl, octahydropentalenyl, octahydroindenyl and decalinyl group. A (C₅-C₁₀)carbocycle group can be unsubstituted or substituted with one or more groups, including, but not limited to, (C₁-C₈)alkyl, -O-[(C₁-C₈)alkyl], -aryl, -CO-R', - O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl. A CH₂ group of the (C₅-C₁₀)carbocycle ring can optionally be replaced by an S or O atom.

In an embodiment and as preferably used herein, the term **"aliphatic"** refers to an alkyl or carbocycle group as defined herein.

In an embodiment and as preferably used herein, the term **"(C₃-C₇)heterocycle"** refers to a 3-, 4-, 5-, 6- or 7-membered saturated or unsaturated non-aromatic heterocyclic ring. Representative (C₃-C₇)heterocycles include, but are not limited to, any of a aziridinyl, oxiranyl, thiiranyl, azetidinyl, oxetanyl, thietanyl, tetrahydrofuranyl, tetrahydrothiophenyl, pyrrolidinyl, oxathiolanyl, sulfolanyl, thianyl, tetrahydropyranyl, piperidinyl, morpholinyl, thiomorpholinyl, dioxanyl, dithianyl, trithianyl, oxepanyl, thiepanyl and azepanyl, group. A (C₃-C₇)heterocycle group can be unsubstituted or substituted with one or more groups, including, but not limited to, (C₁-C₈)alkyl, -O-[(C₁-C₈)alkyl], -aryl, -CO-R', - O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₃-C₈)heterocycle"** refers to a 3-, 4-, 5-, 6-, 7- or 8-membered saturated or unsaturated non-aromatic heterocyclic ring. Representative (C₃-C₈)heterocycles include, but are not limited to, any of a aziridinyl, oxiranyl, thiiranyl, azetidinyl, oxetanyl, thietanyl, tetrahydrofuranyl, tetrahydrothiphenyl, pyrrolidinyl, oxathiolanyl, sulfolanyl, thianyl, tetrahydropyranyl, piperidinyl, morpholinyl, thiomorpholinyl, dioxanyl, dithianyl, trithianyl, oxepanyl, thiepanyl, azepanyl, thiocanyl, oxocanyl and azocanyl group. A (C₃-C₈)heterocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', - SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"(C₅-C₁₀)heterocycle"** refers to a 5-, 6-, 7-, 8-, 9 or 10-membered saturated or unsaturated non-aromatic heterocyclic ring or two fused heterocyclic rings with a total of 5, 6, 7, 8, 9 or 10 ring atoms, whereof at least one ring is non-aromatic. Representative (C₅-C₁₀)heterocycles include, but are not limited to, any of a tetrahydrofuranyl, tetrahydrothiphenyl, pyrrolidinyl, oxathiolanyl, sulfolanyl, thianyl, tetrahydropyranyl, piperidinyl, morpholinyl, thiomorpholinyl, dioxanyl, dithianyl, trithianyl, oxepanyl, thiepanyl, azepanyl, thiocanyl, azocanyl, oxocanyl, thiocanyl, azonanyl, oxonanyl, thionanyl, azecanyl, thiecanyl, oxecanyl, decahydroquinolinyl, decahydroisoquinolinyl and pyrrolizidinyl group. A (C₅-C₁₀)heterocycle group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"aryl"** or **"aryl ring"** refers to a carbocyclic aromatic group, wherein the carbocyclic aromatic group is selected from the group consisting of aromatic groups with a single ring and aromatic groups with two or more fused rings, preferably two rings. Examples of carbocyclic aromatic groups with a single benzene ring include, but are not limited to, phenyl, and examples of carbocyclic aromatic groups with two or more fused benzene rings include, but are not limited to naphthyl and anthracenyl.

In an embodiment and as preferably used herein, the term **"(C₆-C₁₀)aryl"** refers to a 6-, 7-, 8-, 9- or 10-membered carbocyclic aromatic ring or two fused carbocyclic rings with a total of 6, 7, 8, 9 or 10 ring atoms. For example, representative (C₆-C₁₀)aryl groups include but are not limited to any of a phenyl and naphthyl group. A carbocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, - NHR', -NR'₂ and -CN; where each R' is independently selected from -(C₁-C₈)alkyl and aryl. In a more preferred embodiment the carbocyclic aromatic group comprises two of the aforementioned one or more substituent groups, wherein the first of said two groups is attached to a first ring atom of the carbocyclic aromatic group, and the second of said two groups is attached either to a second ring atom of the carbocyclic aromatic group directly adjacent to said first ring atom or to a second ring atom of the carbocyclic aromatic group, the second ring atom being separated from the first ring atom by a further ring atom.

In an embodiment and as preferably used herein, the term **"heteroaryl"** or **"heteroaryl ring"** refers to a heterocyclic aromatic group, wherein the heterocyclic aromatic group is selected from the group consisting of heterocyclic aromatic groups with a single ring and heterocyclic aromatic group with two or more fused rings, preferably two rings. Examples of heterocyclic aromatic groups with a single ring include, but are not limited to, furanyl, thienyl, pyrrolyl, pyrazolyl, oxazolyl, thiazolyl, imidazolyl, pyridinyl pyrimidinyl and examples of heterocyclic aromatic groups with two or more fused rings include, but are not limited to benzothiophenyl, benzofuranyl, indolyl and quinolinyl. A heterocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN, wherein each R' is independently selected from -(C₁-C₈)alkyl and aryl. In a more preferred embodiment the heterocyclic aromatic group comprises two of the aforementioned one or more substituent groups, wherein the first of said two groups is attached to a first ring atom of the heterocyclic aromatic group, and the second of said two groups is attached either to a second ring atom of the heterocyclic aromatic group directly adjacent to said first ring atom or to a second ring atom of the heterocyclic aromatic group, the second ring atom being separated from the first ring atom by a further ring atom.

In an embodiment and as preferably used herein, the term **"(C₅-C₆)heteroaryl"** refers to a 5- or 6-membered heterocyclic aromatic ring. Representative (C₅-C₆)heteroaryl groups include, but are not limited to, any of a furanyl, 2-thienyl, 2-pyridinyl, 3-pyridinyl, 4-pyridinyl, imidazolyl, thiazolyl, oxazolyl, pyrimidinyl and pyrazinyl group. A (C₅-C₆)heteroaryl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(Ci-C₈)alkyl and aryl.

In an embodiment and as preferably used herein, the term **"(C₅-C₁₀)heteroaryl"** refers to a 5-, 6-, 7-, 8-, 9- or 10-membered heterocyclic aromatic ring or two fused heterocyclic rings with a total of 5, 6, 7, 8, 9 or 10 ring atoms. Representative (C₅-C₁₀)heterocycles include, but are not limited to, any of a furanyl, 2-thienyl, 2-pyridinyl, 3-pyridinyl, 4-pyridinyl, imidazolyl, thiazolyl, oxazolyl, pyrimidinyl, pyrazinyl, quinolinyl, isoquinolinyl, indolyl, benzothienyl, benzofuranyl, benzthiazolyl and benzoxazolyl group. A (C₅-C₁₀)heteroaryl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'z, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the expression **"hydrophobic group"** refers to a group that comprises an aliphatic group of two or more carbon atoms, a heterocyclic group, an aryl group or a heteroaryl group as defined herein wherein the ratio of heteroatoms to carbon atoms is up to 1:2 and/or wherein said aliphatic, heterocyclic, aryl or heteroaryl group each and individually can be substituted by no more than one polar or charged group, preferably no polar or charged group is present. Representative hydrophobic groups include, but are not limited to, any of an ethyl, n-propyl, iso-propyl, n-butyl, isobutyl, t-butyl, phenyl, naphthyl, thienyl, cyclopropyl, cyclobutyl, cyclopentyl, cyclohexyl, thietanyl, tetrahydrothiophenyl, thianyl, butanoyl group, hexanoyl group, n-butyloxycarbonyl group and n-butylcarbamoyl.

In an embodiment and as preferably used herein, the expression **"hydrophobic side chain"** refers to a side chain wherein the side chain comprises a "hydrophobic group" as defined herein.

In an embodiment and as preferably used herein, the expressions **"aromatic residue"** or/and **"aromatic ring"** refer to an aryl as defined herein.

In an embodiment and as preferably used herein, the expression **"aromatic side chain"** refers to a side chain wherein the side chain comprises an "aromatic ring" as defined herein.

In an embodiment and as preferably used herein, the term **"arylene"** refers to an aryl group which has two covalent bonds and can be in the *ortho, meta,* or *para* configurations as shown in the following structures: 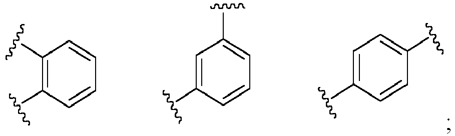 in which the phenyl group can be unsubstituted or substituted with four groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, the term **"polar group"** refers to a group of atoms that feature an uneven distribution of electrons between them due to differences in the electronegativity between the elements of a bond. Elements with higher electronegativity can withdraw bonding electrons from their neighbour, resulting in that the former presents a partial negative charge and the latter a partial positive charge. Polar groups typically contain one or more atoms selected from the group consisting of O, N, S and P. Examples of polar groups include, but are not limited to, -OH, -OR', -CONH₂, -CO-NHR', -CO-NR'R", -NHC(O)NH₂, -NHC(NH)NH₂, -COOH, -O-CO-R', -CO-OR', -SO₃H, SO₃R', - CN, -NH₂, -NHR', -NR'R", -SO-, -SOz-, -SOzNHz, -NOz, -P(O)(OH)₂, and tetrazole, wherein each R' and R" independently represent a group comprising one or more atoms selected from C, N, O, and S, preferably a group selected from -(C₁-C₈)alkyl and aryl. Preferred examples of polar groups as used herein include -OH, -OR', -CONH₂, -CO-NHR', -CO-NR'R", -NHC(O)NH₂, -NHC(NH)NH₂, -COOH, -O-CO-R', -CO-OR', -SO₃H, -SO₃R', -CN, -NH₂, -NHR', -NR'R", -SO-, -SOz-, -SOzNHz, -NOz, - P(O)(OH)₂, and tetrazole, wherein each of R and R' is independently selected from -(C₁-C₈)alkyl and aryl. More preferred examples of polar groups as used herein include -OH, -CONH₂, -COOH, -SO₃H, -CN, -NH₂, -SO-, -SOz-, and NOz.

In an embodiment and as preferably used herein, the expression **"polar side chain"** refers to a side chain wherein the side chain comprises an "polar group" as defined herein.

In an embodiment and as preferably used herein, the expression **"charged group"** refers to a functional group which can be charged at a certain pH, esp. in the range of 4 to 8. Examples of groups that can be positively charged include, but are not limited to, guanidine, ammonium and amino nitrogen. Examples of groups that can be negatively charged include, but are not limited to, COOH, phosphate, phosphonate, sulfate and sulfonate.

In an embodiment and as preferably used herein, the expression **"positvely charged side chain"** refers to a side chain wherein the side chain comprises an "positively charged group" as defined herein, and the expression **"negatively charged side chain"** refers to a side chain wherein the side chain comprises an "negatively charged group" as defined herein.

Unless specified otherwise, atoms with unspecified atomic mass numbers in any structural formula or in any passage of the instant specification including the claims are either of unspecified isotopic composition, naturally occurring mixtures of isotopes or individual isotopes. This applies in particular to carbon, oxygen, nitrogen, sulfur, phosphorus, halogens and metal atoms, including but not limited to C, O, N, S, F, P, Cl, Br, At, Sc, Cr, Mn, Co, Fe, Cu, Ga, Sr, Zr, Y, Mo, Tc, Ru, Rh, Pd, Pt, Ag, In, Sb, Sn, Te, I, Pr, Pm, Dy, Sm, Gd, Tb, Ho, Dy, Er, Yb, Tm, Lu, Sn, Re, Rd, Os, Ir, Au, Pb, Bi, Po, Fr, Ra, Ac, Th and Fm.

In an embodiment and as preferably used herein, the term **"chelator"** (or **"chelating agent")** refers to a molecule containing two or more electron donor atoms that can form coordinate bonds to a single central metal ion, e.g. to a radionuclide. Typically, chelating agents coordinate metal ions through oxygen, nitrogen, or sulfur donor atoms, or combinations thereof. After the first coordinate bond is formed, each successive donor atom that binds creates a ring containing the metal ion. A chelating agent may be bidentate, tridentate, tetradentate, etc., depending on whether it contains 2, 3, 4, or more donor atoms capable of binding to the metal ion. However, the chelating mechanism is not fully understood and depends on the chelating agent and/or radionuclide. For example, it is believed that DOTA can coordinate a radionuclide via carboxylate and amino groups (donor groups) thus forming complexes having high stability (Dai et al. Nature Com. 2018, 9, 857). The term "chelating agent" is to be understood as including the chelating agent as well as salts thereof. Chelating agents having carboxylic acid groups, e.g., DOTA, TRITA, HETA, HEXA, EDTA, DTPA etc., may, for example, be derivatized to convert one or more carboxylic acid groups to amide groups for attachment to the compound, i.e. to the reactive moiety or the linker, alternatively, for example, said compounds may be derivatized to enable attachment to the compound via one of the CH2 groups in the chelate ring.

In an embodiment and as preferably used herein, the term **"residue of a chelator"** refers to all atoms of a chelator which remain as a structure in the compound of the invention after the combination of said chelator with or the attachment of the chelator to the rest of the compound of the invention, i.e. the moiety of the compound of the invention without the chelator.

In an embodiment and as preferably used herein, the term **"radionuclide"** refers to an atom with an unstable nucleus, which is a nucleus characterized by excess energy that is released by different types of radioactive decay. Radionuclides occur naturally or can be artificially produced. In one embodiment, references to **"nuclide(s)"** made in the present specification and claims are preferably to be understood as references to **"radionuclide(s)".**

In an embodiment and as preferably used herein, the term **"bio-distribution modifier"** refers to a compound or moiety whose function is the attenuation of unintended or undesired bio-distribution of the compound of the invention by altering physio-chemical properties of the compound of the invention.

In an embodiment and as preferably used herein, the expression **"Z group"** (or "group Z") refers to (a) a chelator optionally comprising a linker or (b) a bio-distribution modifier optionally comprising a linker, wherein said linker covalently links the chelator or the bio-distribution modifier to the rest of the compound (peptide). The group and position, respectively, of the compound of the invention where a Z group may be covalently attached, is also referred to herein as Z group attachment point.

In accordance with the present invention, Z group attachment points are in particular one or more of the following ones: Xaa0 (alpha amino group) comprising one Z group; Xaa2 (side chain) comprising one Z group; Xaa3 (side chain) comprising one Z group; Xaa7 (side chain) comprising one Z group; and Xaa10 (C-terminal modification group Cterm attached to the C-terminal carbonyl group) comprising one Z group.

More preferred Z group attachment points are one or more, i.e., one, two or three, of the following ones: Xaa0 (alpha amino group) comprising one Z group; Xaa2 (side chain) comprising one Z group; and Xaa3 (side chain) comprising one Z group.

As will be appreciated by a person skilled in the art, the chemical nature of the effector and of the linker, if present, define the kind of amino acid residue to be used for attaching any one of them to the compound of the invention. Typically, the effector and the linker, if present, comprise a functional group suitable for reacting with another functional group in the amino acid residue, whereby such functional group is preferably selected from the group comprising a carboxylic acid group and an amino group.

In an embodiment and as preferably used herein, the term **"amino acid residue"** refers to all atoms of an amino acid, which remain in a peptide chain after the combination of said amino acid with one or more other amino acids, an N-terminal modification group A, a C-terminal modification group Cterm, and/or a Z group to the α-nitrogen atom and/or the carbonyl group of said amino acid.

In an embodiment and as preferably used herein, the term **"derivative"** is used to characterize moieties bonded to adjacent moieties, which moieties differ from the molecules from which they are derived only by the structural elements responsible for bonding to adjacent moieties. This may include covalent bonds formed by existing functional groups or covalent bonds and adjacent functional groups newly introduced for this purpose.

In an embodiment and as preferably used herein, the term **"N-terminal end group"** refers to an atom or moiety that is covalently attached to the N atom of the N-terminus of the peptide chain or to the N atom of the N-terminus of an amino acid residue and is usually an N-terminal blocking group or simply an H atom.

In an embodiment and as preferably used herein, the term **"N-terminal blocking group"** refers to a moiety which, when attached to the peptide N-terminus, caps the peptide N-terminus and prevents elongation of the peptide chain in N-terminal direction (during synthesis). Representative N-terminal blocking groups include, but are not limited to, any of an acetyl group, butanoyl group, hexanoyl group, n-butyloxycarbonyl group (Butoc), t-butyloxycarbonyl group (Boc), allyloxycarbonyl (Alloc), 9-fluorenylmethoxycarbonyl (Fmoc), n-butylcarbamoyl group and n-butylsulfonyl group. If a peptide or an amino acid has a free N-terminus, the N-terminal end group is an H atom.

In contrast, in an embodiment and as preferably used herein, the term **"N-terminal modification group"** (also referred to as "A") refers specifically to a moiety that is covalently attached to the α-nitrogen atom of Xaa1. Similarly, in an embodiment and as preferably used herein, the term **"N-terminal extension group"** (also referred to as "Nterm") refers specifically to a moiety that is covalently attached to the α-nitrogen atom of Xaa0.

In an embodiment and as preferably used herein, the term **"C-terminal end group"** refers to a moiety that is covalently attached to the C atom of the carbonyl group of the C-terminal (last) amino acid residue of the peptide chain and is usually NH₂ or OH.

In contrast, in an embodiment and as preferably used herein, the term **"C-terminal modification group"** (also referred to as "Cterm") refers to a moiety that can be covalently attached to the carboxy terminus of the peptide chain of formula (I), i.e., specifically to the C atom of the carbonyl group of Xaa10. If Cterm is a Z group, Cterm can comprise a linker to which a chelator is covalently attached, e.g. Cterm can be the Z group -Ttds-Lys(DOTA)-NH₂ attached to the carbonyl group of Xaa10, wherein the C-terminal end group is -NH₂ which is attached to the carbonyl group of the very C-terminal Lys(DOTA) residue.

In an embodiment and as preferably used herein, the terms **"L-configuration"** and **"D-configuration"** refer to the stereo configuration of the α-carbon atom of an α-amino acid or a residue of an α-amino acid.

In an embodiment and as preferably used herein, the term **"side chain"** refers to all atoms of an amino acid residue that are not comprised in the "main chain" (or "backbone") portion of said amino acid residue. The "main chain" refers to the structure that is formed by the consecutive connection of at least a first amino acid and a second amino acid, whereby the α-nitrogen atom of an α-amino acid being the second amino acid, the β-nitrogen atom of a β-amino acid being the second amino acid, the γ-nitrogen of a γ-amino acid being the second amino acid, the δ-nitrogen atom of a δ-amino acid being the second amino acid, the ε-nitrogen of an ε-amino acid being the second amino acid or the ω-nitrogen of an ω-amino acid being the second amino acid is connected or covalently linked to the C-1 carbonyl group of a preceding amino acid being the first amino acid.

In an embodiment and as preferably used herein, the term **"linking", "linked"** or **"link"** refers to or describes a scenario, where two or more moieties are connected either directly or indirectly with an interspersed group. Preferably, such moiety is a chelator, an amino acid residue or a bio-distribution modifier. In a more preferred embodiment, such interspersed group is a linker.

In an embodiment and as preferably used herein, the term **"linker"** refers to an element, moiety, or structure which connects two parts of a molecule.

In an embodiment and as preferably used herein, the term **"interspersed"** refers to or describes a scenario in which a group, preferably a linker, is inserted between a first moiety and a second moiety. Such moieties, for example, can be an amino acid residue, a chelator or a bio-distribution modifier. The inserted group is covalently attached to both of the moieties.

Compounds of the invention typically contain amino acid sequences as provided herein. Conventional amino acids, also referred to as natural amino acids are identified according to their standard three-letter codes, as set forth in Table 4.

**Table 4: Conventional amino acids and their abbreviations**

| **3-letter codes** | **Amino acid** | **3-letter codes** | **Amino acid** |
|---|---|---|---|
| Ala | Alanine | Met | Methionine |
| Cys | Cysteine | Asn | Asparagine |
| Asp | Aspartic acid | Pro | Proline |
| Glu | Glutamic acid | Gln | Glutamine |
| Phe | Phenylalanine | Arg | Arginine |
| Gly | Glycine | Ser | Serine |
| His | Histidine | Thr | Threonine |
| Ile | Isoleucine | Val | Valine |
| Lys | Lysine | Trp | Tryptophan |
| Leu | Leucine | Tyr | Tyrosine |

A **"non-conventional amino acid",** also referred to as **"non-natural amino acid",** is any kind of non-oligomeric compound, which comprises an amino group and a carboxylic group and is not a conventional amino acid.

Examples of conventional amino acids, non-conventional amino acids and other building blocks as used for the description of compounds of the invention are identified according to their abbreviation or name found in Table 5. The structures of some building blocks are depicted as resiudes of the corresponding amino acids (amino acid residues) or these building blocks are shown as residue, which is completely attached to another structure like a peptide or amino acid residue. Structures of amino acids are shown as building blocks and as residues of the corresponding amino acids how they are present after implementation in the peptide sequence. Some larger chemical moieties consisting of more than one moiety are also shown for the reason of clarity (e.g. Om(DOTA)).

**Table 5: Abbreviation, name and structure of non-natural amino-acid and other building blocks and chemical moieties**

| **Abbr.** | **Name** | **Residue** | **Building Block** |
|---|---|---|---|
| 1Ni | 3-(1-naphthyl)-L-alanine | 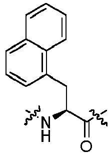 | 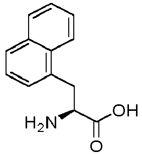 |
| 2MeBn | 2-methylbenzylidene (derived from 1,2-xylene) | 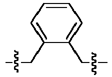 | |
| 2Ni | 3-(2-naphthyl)-L-alanine | 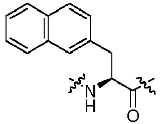 | 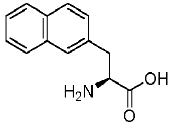 |
| 4Ap | 4-(*S*)-Amino-L-proline | 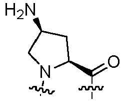 | 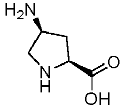 |
| 4Cfp | (2*S*,4*S*)-4-fluoro-pyrrolidine-2-carboxylic acid | 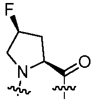 | 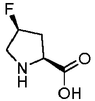 |
| 4Tfp | 4-(*R*)-Fluoro-L-proline | 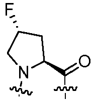 | 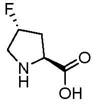 |
| AET | cysteamine (at C-terminus) | 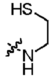 | 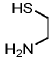 |
| AGLU | 1-amino-1-deoxy-D-glucitol | 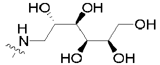 | 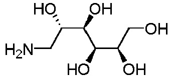 |
| AGLU^{∗} | 1-amino-1-deoxy-3,4:5,6-di-*O-*isopropylidene-D-glucitol | 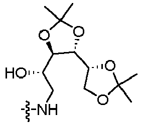 | 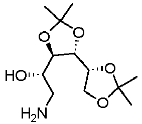 |
| Abu | 2*S*-aminobutyric acid | 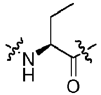 | 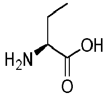 |
| Ac | Acetyl | 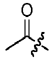 | 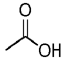 |
| AF488 | Alexa Fluor 488 Dye | 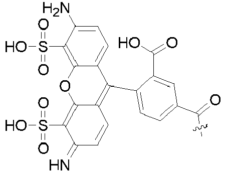 | 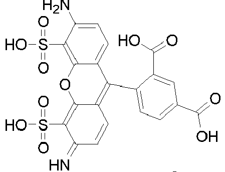 |
| Ahx | 6-aminohexanoic acid | 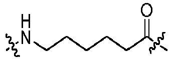 | 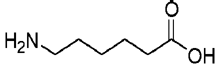 |
| Aib | 1-amino-isobutyric acid | 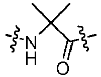 | 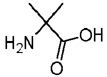 |
| Aic | 2-Aminoindane-2-carboxylic acid | 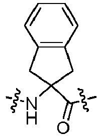 | 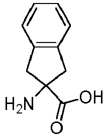 |
| ala | D-alanine | 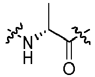 | 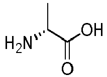 |
| Ala | L-alanine | 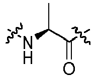 | 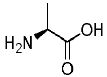 |
| Amf | α-methyl-L-phenyl alanine | 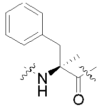 | 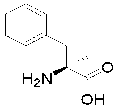 |
| Aml | α-methyl-L-leucine | 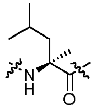 | 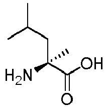 |
| APAc | 2-(4-aminopiperidin-1-yl)acetic acid | 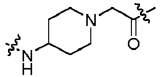 | 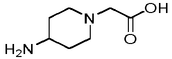 |
| Apc | 4-aminopiperidine-4-carboxylic acid | 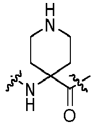 | 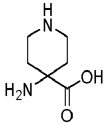 |
| Apc(DOTA) | 4-aminopiperidine-4-carboxylic acid DOTA conjugate | 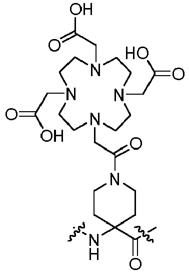 | 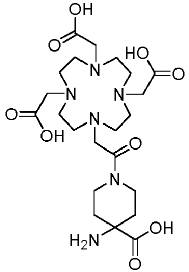 |
| Aph | 4-amino-L-phenylalanine | 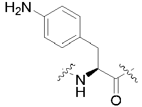 | 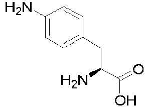 |
| arg | D-arginine | 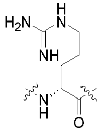 | 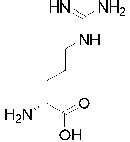 |
| Arg | L-arginine | 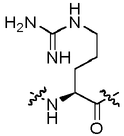 | 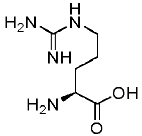 |
| Asn | L-asparagine | 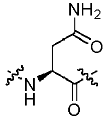 | 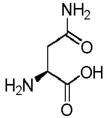 |
| asp | D-aspartic acid | 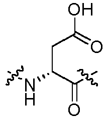 | 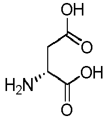 |
| Asp | L-aspartic acid | 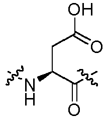 | 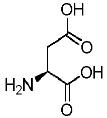 |
| Ava | 5-aminopentanoic acid | 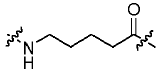 | 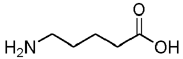 |
| Ay3 | 3-amino-L-tyrosine | 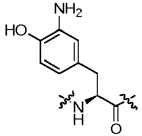 | 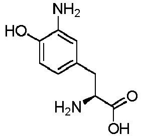 |
| Aze | (*S*)-azetidine-2-carboxylic acid | 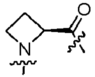 | 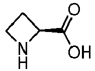 |
| Bal | β-alanine | 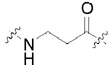 | 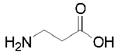 |
| Bio | D-(+)-biotin | 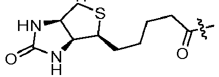 | 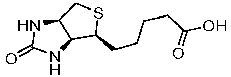 |
| Bta | 3 -(3-benzothienyl)-L-alanine | 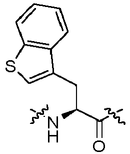 | 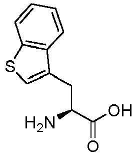 |
| But | butyric acid | 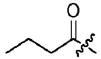 | 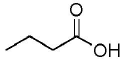 |
| Butoc- | n-butyloxycarbonyl- | 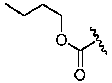 | 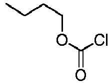 |
| Bz | benzoic acid | 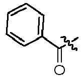 | 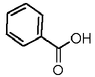 |
| Cbg | L-cyclobutyl-glycine | 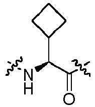 | 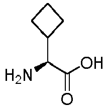 |
| Cha | L-cyclohexylalanine | 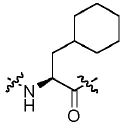 | 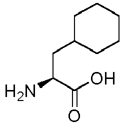 |
| Chex | cyclohexanecarboxylic acid | 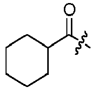 | 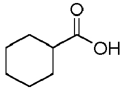 |
| Chg | L-cyclohexyl-glycine | 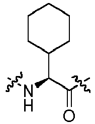 | 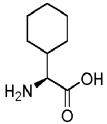 |
| Chy | 4-(*S*)-hydroxy-L-proline | 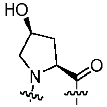 | 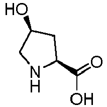 |
| cit | D-citrulline | 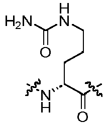 | 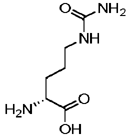 |
| Cit | L-Citrulline | 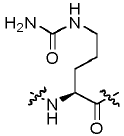 | 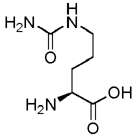 |
| Cmp | 4-Carboxymethyl-piperidine | 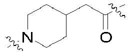 | 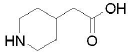 |
| Cp | Cyclopentane carboxylic acid | 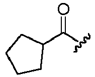 | 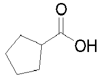 |
| Cpa | L-cyclopropyl glycine | 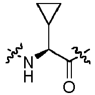 | 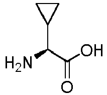 |
| Cpg | L-cyclopentyl-glycine | 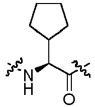 | 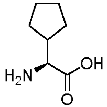 |
| Cpra | L-cyclopropyl alanine | 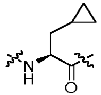 | 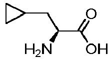 |
| cys | D-cysteine | 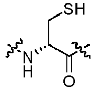 | 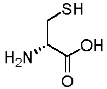 |
| Cys | L-cysteine | 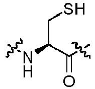 | 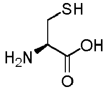 |
| Cysol | L-cysteinol | 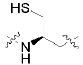 | 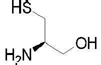 |
| Cys(2MeBn) | L-cysteine bound to 2-methylbenzylidene | 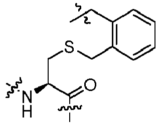 | 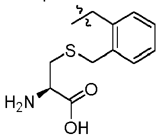 |
| Cys(meth) | L-cysteine bound to methylene (in thioacetal linkage) | 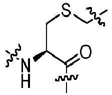 | 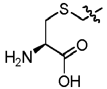 |
| Dab | (*S*)-2,4-diaminobutyric acid | 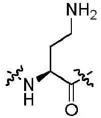 | 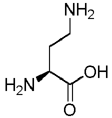 |
| Dab(DOTA) | Dab DOTA conjugate | 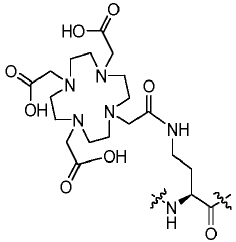 | 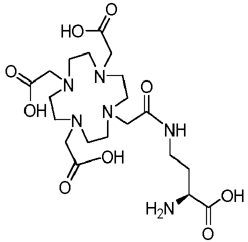 |
| Dap | (*S*)-2,3-diaminopropionic acid | 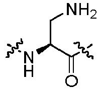 | 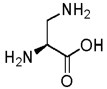 |
| Dap(DOTA) | Dap DOTA conjugate | 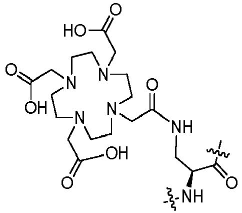 | 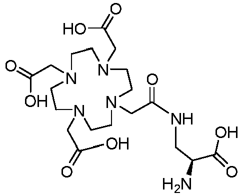 |
| dap | (*R*)-2,3-diaminopropionic acid | 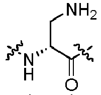 | 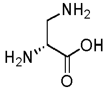 |
| Dde | 1-(4,4-dimethyl-2,6-dioxocyclohex-1-ylidene)ethyl | 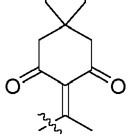 | |
| Deg | diethylgylcine | 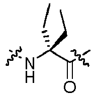 | 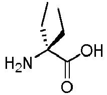 |
| Dfp | 4,4-difluoro-L-proline | 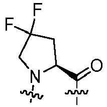 | 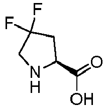 |
| Dmba | *N*,*N*-dimethyl β-alanine | 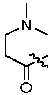 | 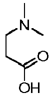 |
| Dmdp | *N,N*-dimethyl diamino propionic acid | 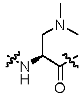 | 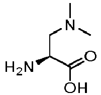 |
| Dopa | Levodopa / L-3,4-dihydroxyphenylala nine | 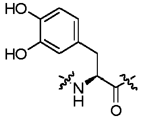 | 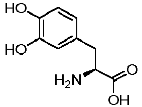 |
| DOTA | 2,2',2",2‴-(1,4,7,10-Tetraazacyclododec ane-1,4,7,10-tetrayl)tetraacetic acid | 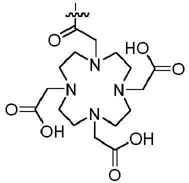 | 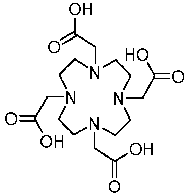 |
| DOTAGA | (*R*)-2-(4,7,10-tris(carboxymethyl) -1,4,7,10-tetraazacyclododec an-1-yl)pentanedioic acid | 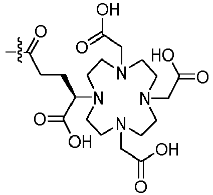 | 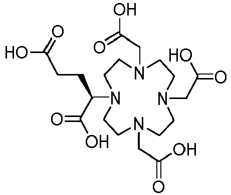 |
| DOTAM | 2-(4,7,10-tris(2-amino-2-oxoethyl)-1,4,7,10-tetraazacyclododec an-1-yl)acetic acid | 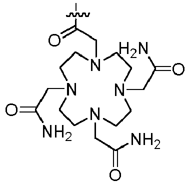 | 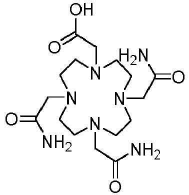 |
| DOTAMGAM | 5-amino-5-oxo-4-(4,7,10-tris(2-amino-2-oxoethyl)-1,4,7,10-tetraazacyclododec an-1-yl)pentanoic acid | 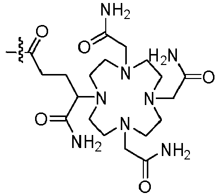 | 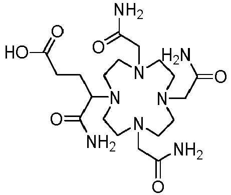 |
| Eaz | L-thiazolidine-4-carboxylic acid | 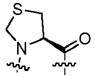 | 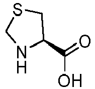 |
| Egm | 2,4-dichloro-L-phenylalanine | 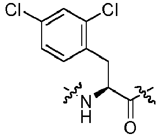 | 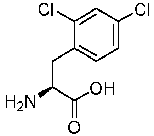 |
| Egz | 1-amino-1-cyclohexan-carboxylic acid | 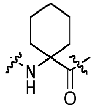 | 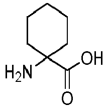 |
| en | ethylenediamine (used as C-terminus) | 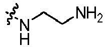 | 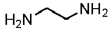 |
| EtOPr | 3-ethoxypropanoic acid | 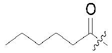 | 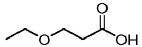 |
| Fso | 4-sulfo phenylalanine | 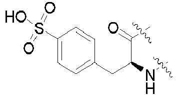 | 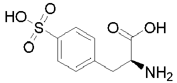 |
| Gab | γ-aminobutyric acid | 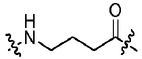 | 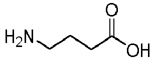 |
| GaDOTA | Ga(III) DOTA complex | 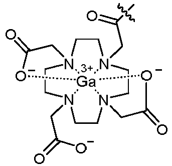 | 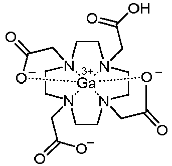 |
| GdDOTA | Gd(III) DOTA complex | 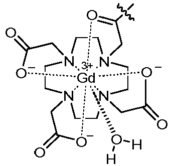 | 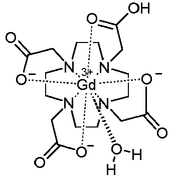 |
| Gln | L-glutamine | 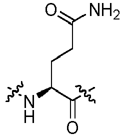 | 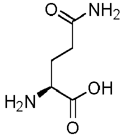 |
| glu | D-glutamic acid | 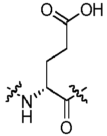 | 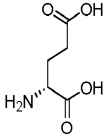 |
| Glu | L-glutamic acid | 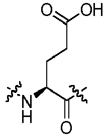 | 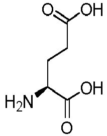 |
| Glutar | glutaric acid as linker | 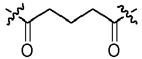 | 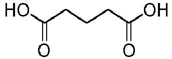 |
| Glutar | glutaric acid when used at the N-terminus | 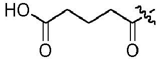 | 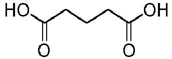 |
| Glu(AGLU) | γ-glutamyl AGLU conjugate | 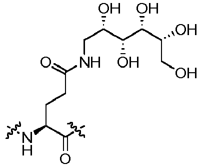 | 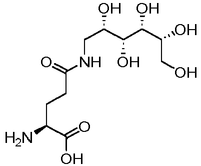 |
| Gly | glycine | 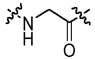 | 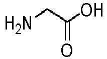 |
| Guf | L-4-guanidino-phenylalanine | 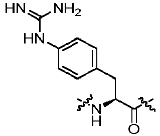 | 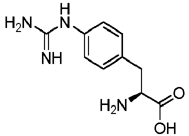 |
| H | hydrogen (at the N-terminus) | 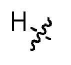 | |
| Har | L-homoarginine | 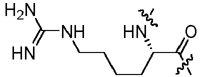 | 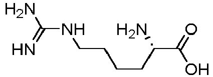 |
| Hey | L-homocysteine | 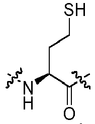 | 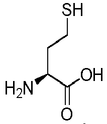 |
| Hex | hexanoic acid | 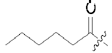 | 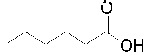 |
| Hfe | L-homophenylalanine | 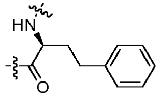 | 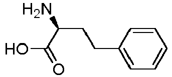 |
| His | L-histidine | 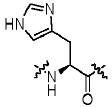 | 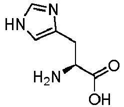 |
| HPA | 3-hydroxy propionic acid at the N-terminus | 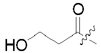 | 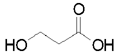 |
| Hse | L-homoserine | 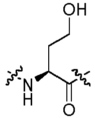 | 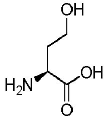 |
| Hyp | 4-(*R*)-hydroxy-L-proline | 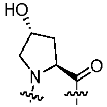 | 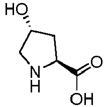 |
| iHex | isocaproic acid | 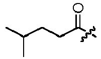 | 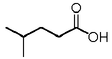 |
| Ile | L-isoleucine | 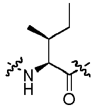 | 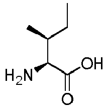 |
| Inp | isonipecotic acid | 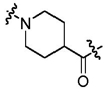 | 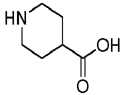 |
| InDOTA | In(III) DOTA complex | 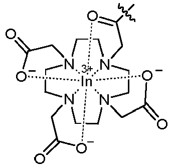 | 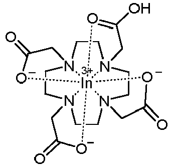 |
| KMe3 | *N_{ε}*,*N_{ε}*,*N_{ε},*-trimethyl-L-lysine | 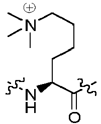 | 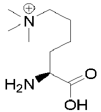 |
| Kzw | (*S*)-3-((5-amino-5-carboxypentyl) dimethylammonio) propane-1-sulfonate | 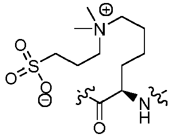 | 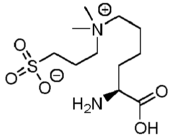 |
| LaDOTA | La(III) DOTA complex | 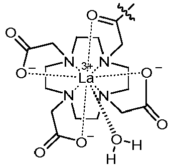 | 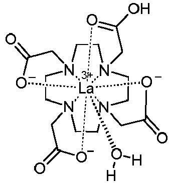 |
| leu | D-leucine | 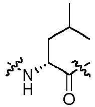 | 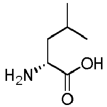 |
| Leu | L-leucine | 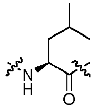 | 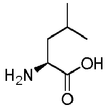 |
| LuDOTA | Lu(III) DOTA complex | 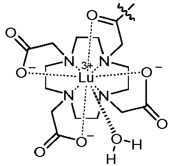 | 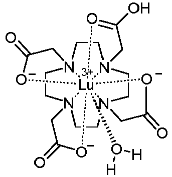 |
| lys | D-lysine | 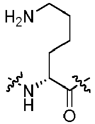 | 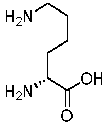 |
| Lys | L-lysine | 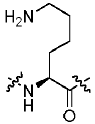 | 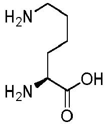 |
| Lys(Ac) | *N_{ε}-*acetyl-lysine | 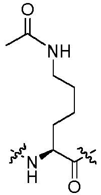 | 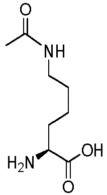 |
| Lys(AF488-Ttds) | L-lysine Ttds AF488 conjugate | 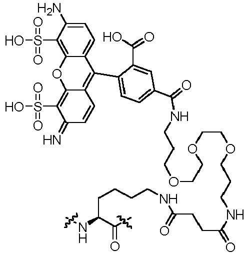 | 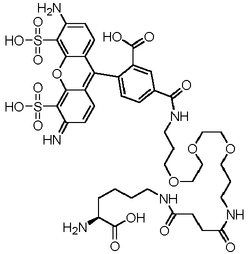 |
| Lys(DOTA) | L-lysine DOTA conjugate | 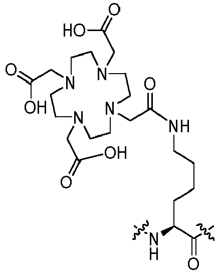 | 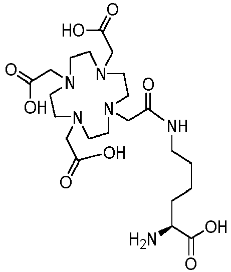 |
| Lys(DOTA-asp) | L-lysine D-aspartate DOTA conjugate | 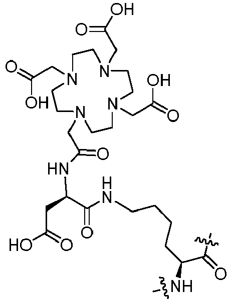 | 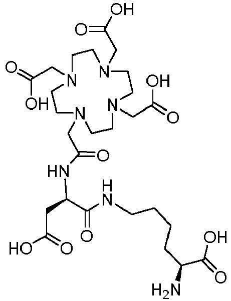 |
| Lys(DOTA-Kzw) | L-lysine Kzw DOTA conjugate | 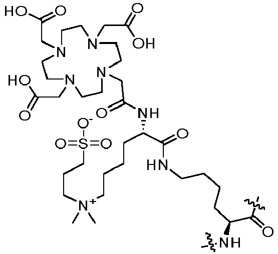 | 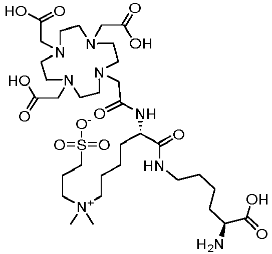 |
| Lys(DOTA-PEG12) | L-lysine PEG12 DOTA conjugate | 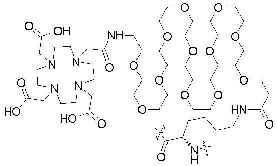 | 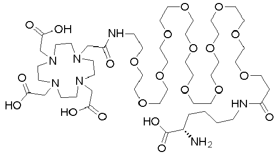 |
| Lys(DOTA-PPAc) | L-lysine PPAc DOTA conjugate | 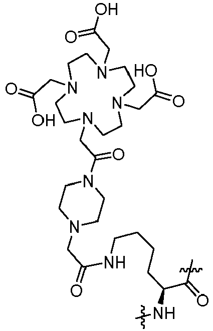 | 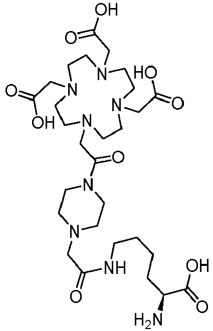 |
| Lys (NODAGA) | L-lysine NODAGA conjugate | 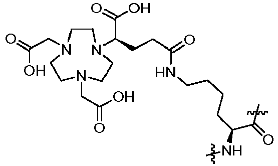 | 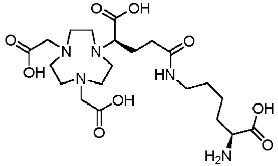 |
| Lys(NOTA) | L-lysine NOTA conjugate | 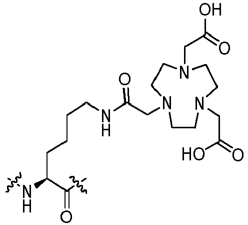 | 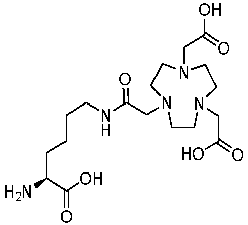 |
| Mcf | 3-chloro-L-phenylalanine | 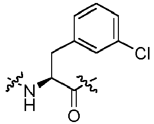 | 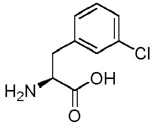 |
| Mcy | L-3-chloro tyrosine | 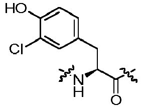 | 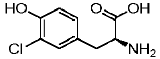 |
| Me | methyl | 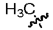 | |
| Met | L-methionine | 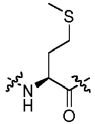 | 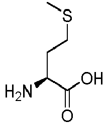 |
| meth | methylene bridge (as a linkage between thiols in a thioacetal) | 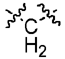 | |
| Mpa | 3-pyridyl-alanine | 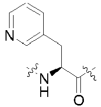 | 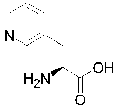 |
| mPEG12 | 2,5,8,11,14,17,20, 23,26,29,32,35-dodecaoxaoctatriac ontan-38-oic acid | 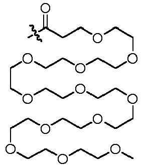 | 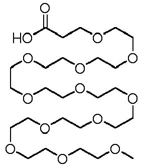 |
| My | L-3-hydroxy-phenylalanine | 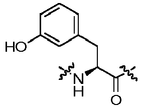 | 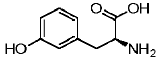 |
| nBuCAyl | n-butyl-carbamoyl | 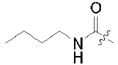 | 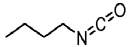 |
| NEtg | *N*-ethyl glycine | 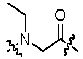 | 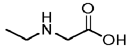 |
| NH₂ | usually represents a C-terminus of a peptide sequence | 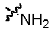 | |
| NHEt | n-ethylamine at C-terminus | 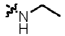 | 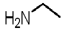 |
| Nif | 4-nitro-L-phenylalanine | 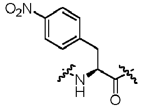 | 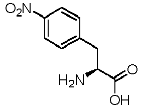 |
| Nle | L-norleucine | 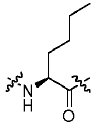 | 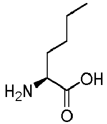 |
| nle | D-norleucine | 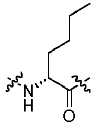 | 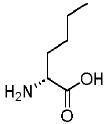 |
| nma | *N*-methyl-D-alanine | 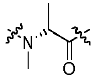 | 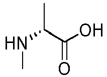 |
| Nma | *N*-methyl-L-alanine | 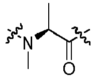 | 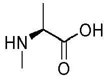 |
| Nmb | *N*-methyl-L-norleucine | 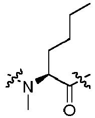 | 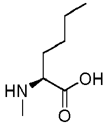 |
| Nmc | *N*-methyl-L-cysteine | 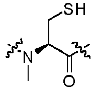 | 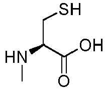 |
| Nme | *N*-methyl-L-glutamic acid | 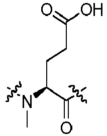 | 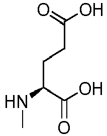 |
| Nmf | *N*-methyl-L-phenylalanine | 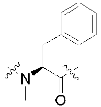 | 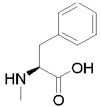 |
| Nmg | *N*-methyl-glycine | 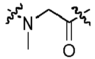 | 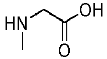 |
| Nmi | *N*-methyl-L-isoleucine | 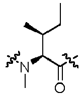 | 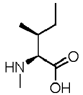 |
| Nmk | *Nₐ*-methyl-L-lysine | 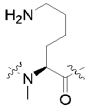 | 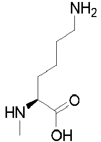 |
| Nml | *N*-methyl-L-leucine | 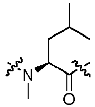 | 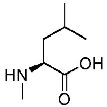 |
| Nmo | *Nₐ*-methyl-L-ornithine | 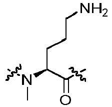 | 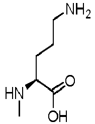 |
| Nmq | *Nₐ*-methyl-L-glutamine | 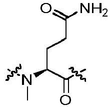 | 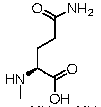 |
| Nmr | *Nₐ*-methyl-L-arginine | 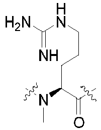 | 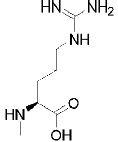 |
| Nms | *N*-methyl-L-serine | 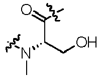 | 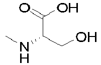 |
| Nmv | *N*-methyl-L-valine | 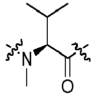 | 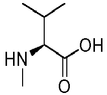 |
| Nmy | *N*-methyl-L-tyrosine | 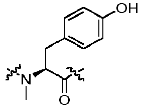 | 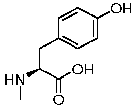 |
| NODAGA | (*R*)-1,4,7-triazacyclononane,1 -glutaric acid-4,7-acetic acid | 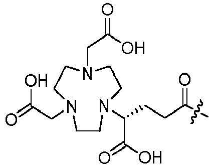 | 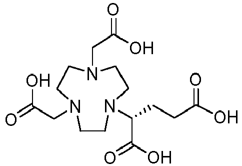 |
| NOTA | 1,4,7-Triazacyclononane-1,4,7-triacetic acid | 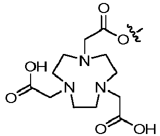 | 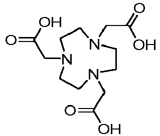 |
| Npg | L-neopentyl-glycine | 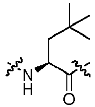 | 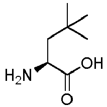 |
| Nta | nitrilotriacetic acid as combined linking and branching unit | 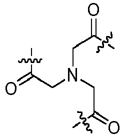 | 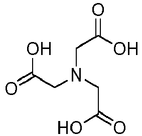 |
| Nva | L-norvaline | 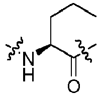 | 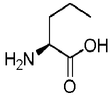 |
| nva | D-norvaline | 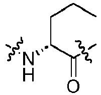 | 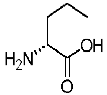 |
| Ny3 | 3-nitro-L-tyrosine | 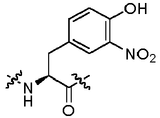 | 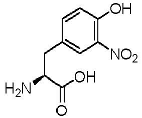 |
| O1Pen | 5-amino-3-oxapentanoic acid | 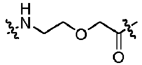 | 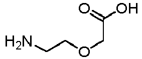 |
| O2Oc | 8-amino-3,6-dioxaoctanoic acid | 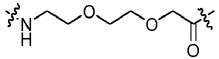 | 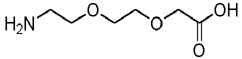 |
| Ocf | ortho-chloro-L-phenylalanine | 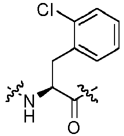 | 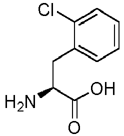 |
| Oct | octanoic acid | 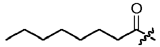 | 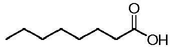 |
| OH | usually represents a C-terminus of a peptide sequence | 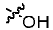 | |
| Oic | (2*S*,3a*S*,7a*S*)-octahydroindolecar boxylic acid | 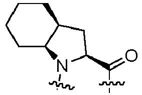 | 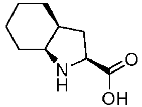 |
| orn | (*R*)-ornithine | 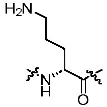 | 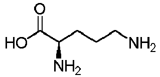 |
| Orn | (*S*)-ornithine | 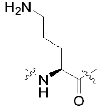 | 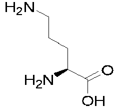 |
| Orn(DOTA) | L-omithine DOTA conjugate | 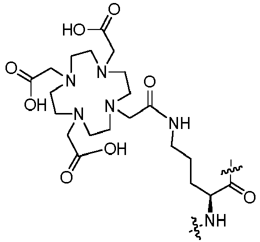 | 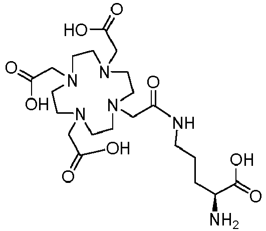 |
| Orn(mPEG 12) | L-omithine mPEG12 conjugate | 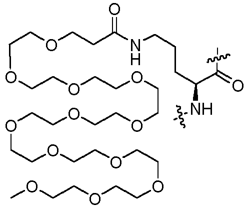 | 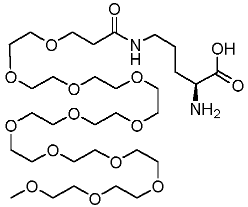 |
| Orn(Tris-Nta(Tris)) | L-ornithine Tris-Nta(Tris) conjugate | 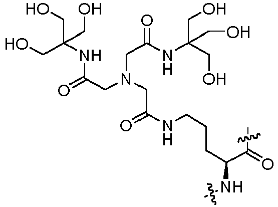 | 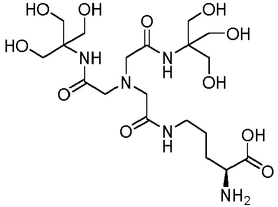 |
| Oxa | (*S*)-oxazolidine-4-carboxylic acid | 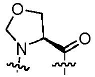 | 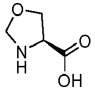 |
| Pcf | 4-chloro-L-phenylalanine | 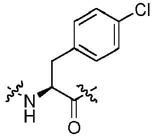 | 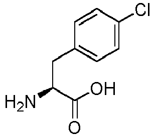 |
| Pcnf | 4-carbamoyl-L-phenylalanine | 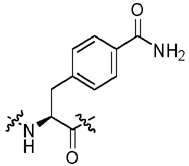 | 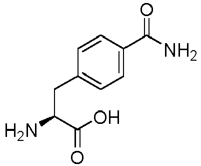 |
| PEG6 | 1-amino-3,6,9,12,15,18-hexaoxahenicosan-21-oic acid | 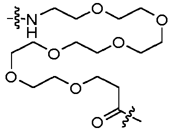 | 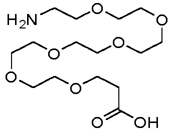 |
| PEG12 | 1-amino-3,6,9,12,15,18,21,2 4,27,30,33,36-dodecaoxanonatriac ontan-39-oic acid | 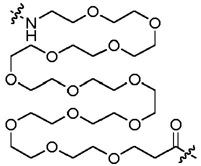 | 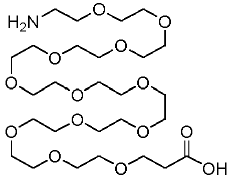 |
| Pen | L-penicillamine | 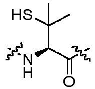 | 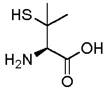 |
| Pent | pentanoic acid | 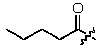 | 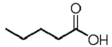 |
| PentylSO2 | pentyl sulfonyl | 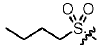 | 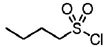 |
| Pha | phenyl acetic acid | 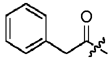 | 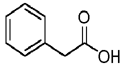 |
| phe | D-phenylalanine | 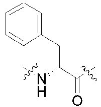 | 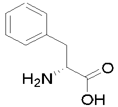 |
| Phe | L-phenylalanine | 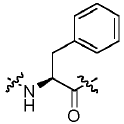 | 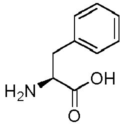 |
| Phg | L-phenylglycine | 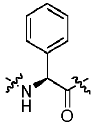 | 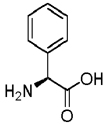 |
| Pip | (*S*)-pipecolic Acid | 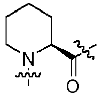 | 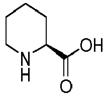 |
| Pnf | 4-cyano-L-phenylalanine | 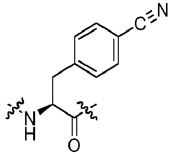 | 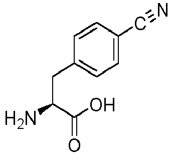 |
| Ppa | (*S*)-4-pyridyl-alanine | 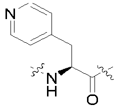 | 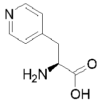 |
| PPAc | 4-carboxymethyl piperazine | 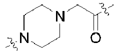 | 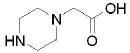 |
| PrCAyl | n-propyl-carbamoyl | 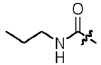 | 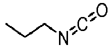 |
| pro | D-proline | 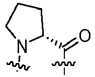 | 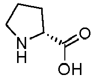 |
| Pro | L-proline | 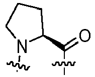 | 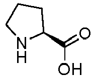 |
| PrOAc | 2-propoxyacetic acid | 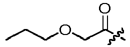 | 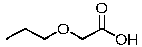 |
| Prpoc- | n-propyloxycarbonyl- | 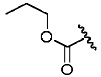 | 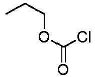 |
| PSC | 2,2',2"-(10-(2-amino-2-oxoethyl)-1,4,7,10-tetraazacyclododec ane-1,4,7-triyl)triacetic acid | 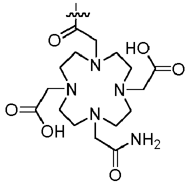 | 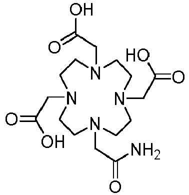 |
| PSCGA | 2-(4,10-bis(2-amino-2-oxoethyl)-7-(carboxymethyl)-1,4,7,10-tetraazacyclododec an-1-yl)pentanedioic acid | 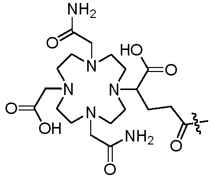 | 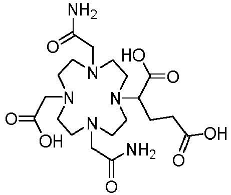 |
| Rni | (*R*)-nipecotic acid | 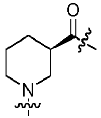 | 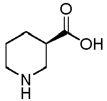 |
| ser | D-serine | 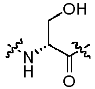 | 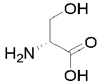 |
| Ser | L-serine | 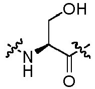 | 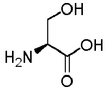 |
| Ser(Me) | L-*O*-methylserine | 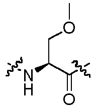 | 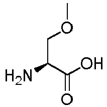 |
| Smc | *S*-methyl-L-cysteine | 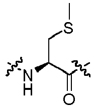 | 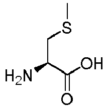 |
| Succinyl | succinic acid at N-terminus | 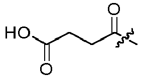 | 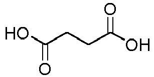 |
| Tap | (2*S*,4*R*)-4-amino-1-pyrrolidin-2-carboxylic acid | 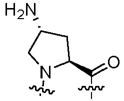 | 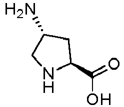 |
| Tap(DOTA) | Tap DOTA conjugate | 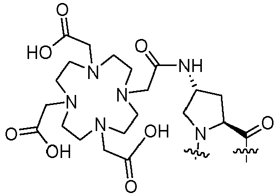 | 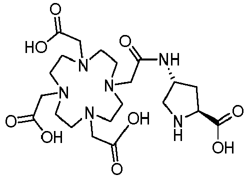 |
| Thi | L-thienylalanine | 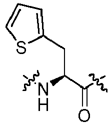 | 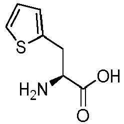 |
| Thp | 4-amino-4-tetrahydropyran carboxylic acid | 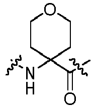 | 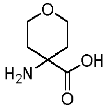 |
| Thr | L-threonine | 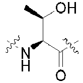 | 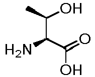 |
| Tic | (*S*)-1,2,3,4-tetrahydroisoquinol ine-3-carboxylic acid | 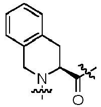 | 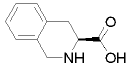 |
| Tie | L-tert-leucine | 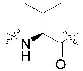 | 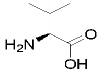 |
| Tris | tris(hydroxymethyl ) aminomethane | 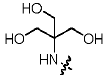 | 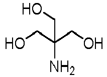 |
| Tris-Nta(Tris) | nitrilotriacetic acid amidylated with two Tris molecules | 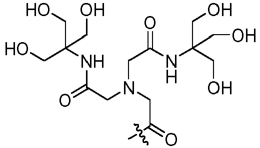 | 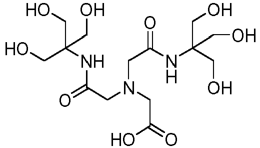 |
| TrisPEG | a branched PEG carboxylic acid | 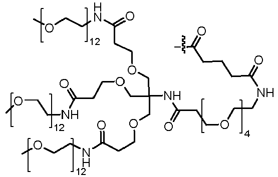 | 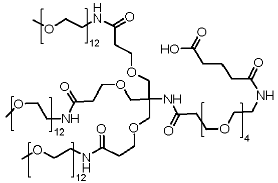 |
| Trp | L-tryptophan | 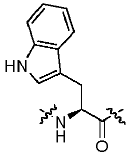 | 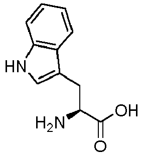 |
| Ttds | 1,13-diamino-4,7,10-trioxatridecan-succinamic acid | 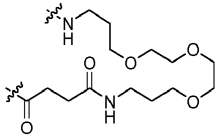 | 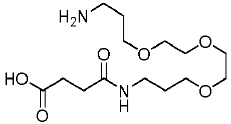 |
| tyr | D-tyrosine | 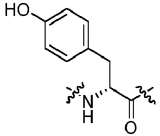 | 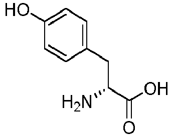 |
| Tyr | L-tyrosine | 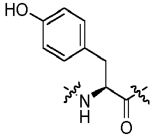 | 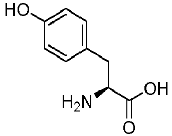 |
| Val | L-valine | 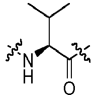 | 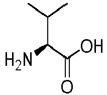 |

The amino acid sequences of the peptides disclosed herein are depicted in typical peptide sequence format, as will be understood by a person skilled in the art. For example, the three-letter code for a residue of a conventional amino acid, the code for a residue of a non-conventional amino acid or the abbreviation for a residue of a building block other than a conventional or non-conventional amino acid indicates the presence of the residue, of the amino acid residue or the presence of the residue of the building block at a specified position within the peptide sequence. The code for a residue of an amino acid and the abbreviation for a residue of a building block, respectively, is connected to the code for a subsequent residue of an amino acid and/or to the code for a preceding residue of an amino acid and, respectively, is connected to the abbreviation for a subsequent residue of a building block and/or to the abbreviation for a preceding residue of a building block, respectively, in the peptide sequence by a hyphen which typically represents an amide linkage. For example, if the hyphen is located before the code for a residue of an amino acid, this usually symbolizes that the amino group (as indicated in the corresponding entry in Table 5) of the residue of the amino acid is covalently attached to a carbonyl group of a preceding residue of an amino acid, and if the hyphen is located after the code for a residue of an amino acid, this usually symbolizes that the carbonyl group (as indicated in the corresponding entry in Table 5) of the residue of the amino acid is covalently attached to an amino group of a subsequent residue of an amino acid.

Depending on the spacing between the amino- and the carboxy group in amino acids they are classified into α-, β-, γ-, δ-, ε-, (and so forth) -amino acids, which means that these groups are typically are spaded apart by 1, 2, 3, 4, and 5 heavy-atoms (typically carbon), respectively.

For amino acids, in their abbreviations the first letter indicates the stereochemistry of the C-α-atom if applicable. For example, a capital first letter indicates that the L-form of the amino acid is present in the peptide sequence, while a lower case first letter indicating that the D-form of the correspondent amino acid is present in the peptide sequence. If the abbreviation starts with a number, the first letter in the abbreviation will be characteristic for the stereochemistry, if applicable. However, for enhanced clarity it is at any abbreviation an option to further clearly specify the stereochemistry of an amino acid abreviation by adding for instance the prefix "D-". As example "lys", "D-Lys" or "D-lys" describe all a D-configured Lys.

For someone skilled in the art it is evident that many amino acids can be N-methylated at their amino group. These N-methyl amino acid feature can occur in combination with some other attributes like L-α- or D-α-N-methyl amino acids which are N-methylated L-α- or D-α-amino acids.

In an embodiment and as preferably used herein, the term **α,α-dialkylamino acid**" refers to amino acid which comprise independently two alkyl groups at the α-carbon atom which maybe in some cases form a ring-structure with each other. Representative α,α-dialkylamino acids include, but are not limited to, any of Aib, Deg, Egz, Thp and Ape.

In an embodiment and as preferably used herein, the term **"cyclic amino acid"** refers to an amino acid that comprises a cyclic structure wherein in some cases the amino nitrogen of the amino acid is part of a heterocyclic structure usually consisting of 4 to 7 ring members. Cyclic maybe combined with other amino acid attribute as for instance in cyclic D-α-amino acid or cyclic α,α-dialkylamino acid.

In an embodiment and as preferably used herein, the term **"aromatic amino acid"** refers to an amino acid, which comprises an aromatic structure and this includes a heteroaromatic structure whereas the term "non-aromatic amino acid" refers to an amino acid which is devoid of any aromatic structure. Examples of aromatic amino acids include, but are not limited to, phenylalanine, tyrosine, chloro phenylalanine, guanidino phenylalanine, naphthylalanine, carboxymethyl phenylalanine, fluoro phenylalanine, phenyl glycine, homophenylalanine and Levodopa.

In an embodiment and as preferably used herein, the term **"heteroaromatic amino acid"** refers to an amino acid, which comprises any kind of heteroaromatic structure. Examples of heteroaromatic amino acids include, but are not limited to, histidine, tryptophan, 2-thienyl alanine, thiazolyl alanine, 3-pyridyl alanine, 7-chloro tryptophan, benzothienyl alanine, furanyl alanine, 4-pyridyl alanine and 2-quinolyl alanine.

In an embodiment and as preferably used herein, an **"aliphatic amino acid"** is a non-aromatic amino acid, which comprises an aliphatic group. Examples of aliphatic amino acids include, but are not limited to, noreleucine, leucine, isoleucine, valine, cyclohexyl alanine, α-aminobutyric acid, tert-leucine, neopentyl glycine and 2-cyclopropyl glycine.

In an embodiment and as preferably used herein, a **"polar amino acid"** is any kind of amino acid, which comprises apart from the amino and carboxy group one or more polar groups as defined herein. Examples of polar amino acids include, but are not limited to, serine, threonine, asparagine, glutamine, citrulline, aspartic acid, glutamic acid, lysine, arginine and homoglutamine.

In an embodiment and as preferably used herein, a **"charged amino acid"** is any kind of amino acid which comprises, apart from the amino and carboxy group, one or more charged groups as defined herein. Such charged groups can be positively or negatively charged and a charged amino acid can as well be zwitterionic, i.e. comprising both positively and negatively charged groups at the same time. Examples of positively charged amino acids include, but are not limited to, lysine, arginine, ornithine, homoarginine, KMe3, Dab, Dap and *N_{ε}-*methyl-L-lysine. Examples of negatively charged amino acids include, but are not limited to, aspartic acid, glutamic acid, cysteic acid, 4-carboxy-L-phenylalanine, Fso and γ-carboxyglutamic acid. Examples of zwitterionic amino acids include, but are not limited to, Kzw and ε-(γ-glutamyl)-lysine.

In an embodiment and as preferably used herein, a **"neutral amino acid"** is any kind of amino acid, which comprises apart from the amino and carboxy group, no further functional groups which can be charged at a certain pH, esp. in the range of 4-8. Examples of neutral amino acids include, but are not limited to serine, threonine, asparagine, glutamine, phenylalanine, valine, leucine and citrulline.

If an amino acid contains more than one amino and/or carboxy group all orientations of this amino acid are in principle possible for formation of a covalent bond, but in α-amino acid the utilization of the α-amino and the α-carboxy group is preferred for the attachment to the neighbouring moieties and if other orientations are preferred, they are explicitly specified.

Those skilled in the art will recognize if a stereocenter exists in the compounds disclosed herein irrespective thereof whether such stereocenter is part of an amino acid moiety or any other part or moiety of the compound of the invention. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-lnterscience, 1994).

In the present disclosure, the structural formula of the compound represents a certain isomer for convenience in some cases, but the present disclosure includes all isomers, such as geometrical isomers, optical isomers based on an asymmetrical carbon, stereoisomers, tautomers, and the like.

In an embodiment and as preferably used herein, the term **"amino thiol"** refers to any kind of non-oligomeric compound, which comprises a thiol group and an amino group. Examples of amino thiols include, but are not limited to, cysteamine, 3-aminopropanethiol, cysteine, homocysteine and cysteinol.

In an embodiment and as preferably used herein, the term **"residue of an amino thiol"** refers to a all atoms of an amino thiol that remain as a structure when an amino thiol is combined with or attached to one or more amino acids to form an amino acid derivative or a peptide derivative or analog. For example, the N-atom of the amino group of an amino thiol Xaa10 such as cysteamine can be covalently attached to the carbonyl group of an amino acid Xaa9 to form an amino acid derivative Xaa9-Xaa10. Similarly, the S-atom of the thiol group of an amino thiol Xaa10 can be covalently attached to the S atom of a thiol group of a cysteine to form a disulfide.

Unless indicated to the contrary, the amino acid sequences are presented herein in N- to C-terminus direction (from left to right).

### Linear Peptides

A general linear peptide is typically written from N-to C-terminal direction (from left to right) as shown below:

NT1-Xaa1-Xaa2-Xaa3-Xaa4-.......Xaan-CT;

Therein
1. "Xaax" is the abbreviation, descriptor or symbol for amino acids or building blocks at specific sequence position x as shown in Table 5,
2. "NT1" is an N-terminal end group, which is typically a blocking group, an H atom or a Z group, linked to the N-terminal amino acid code (Xaa1) via a hyphen and
3. "CT" is an C-terminal end group the C-terminal group, which is typically -NH₂ or a Z group and linked to the C-terminal amino acid code (Xaan) via a hyphen.

### Branched peptides with side chains modified by specific building blocks or peptides

Linear, branched peptides are written from the N-to C-terminal direction as shown below:

NT1-Xaa1-Xaa2-Xaa3(*NT2-Xab1-Xab2-... ... .Xabn*)-.......Xaan-CT

Therein, the statements 1. - 3. of the description of linear peptides for the specification of "Xaax", "NT1" and "CT" in the main chain of the branched peptide apply. An individual branch is specified by an expression in parentheses, wherein the positioning of the branch within the peptide sequence/structure is indicated by the positioning of the parenthesized expression right next to an "Xaax" abbreviation (e.g. "Xaa3"). Branches typically either occur at lysine (Lys) residues, ornithine (Orn) residues or 4-aminopiperidine-4-carboxylic acid (Apc) (or similar), which means that the branch is attached to side chain ε-amino function of the lysine, the δ-amino function of the ornithine or to the nitrogen atom within the piperidine ring of 4-aminopiperidine-4-carboxylic acid via an amide bond.

The content of the parentheses describes the sequence/structure of the peptide branch *'NT2-Xab1-Xab2-... ... .Xabn'.* Herein
*1. "Xabx"* is the abbreviation, descriptor or symbol for amino acids or building blocks at specific sequence position x of the branch as shown in Table 5, whereby the linear connection of individual Xabx is indicated by a hyphen,
2. "*NT2*" is an N-terminal end group, e.g. an abbreviation for a specific terminating carboxylic acid like "Ac" for acetyl or other chemical group or structural formula of chemical groups, more specifically "*NT2*" is a Z group, linked to the N-terminal amino acid code (*Xab1*) via a hyphen and
3. the last building block of the branch "*Xabn*", which connects the branch with the main chain by forming an amide bond with its own carboxyl function with the side chain amino function of this lysine or 4-aminopiperidine-4-carboxylic acid (or similar residue).

### Cyclic peptides

An exemplary general cyclic peptide written from the N- to C-terminal direction is shown below:

NT1-Xaal-[Xaa2-Xaa3-Xaa4-.......Xaan]-CT;

Therein the statements 1. - 3. of the description of linear peptides for the specification of "Xaax", "NT1" and "CT" in the main chain of the cyclic peptide apply. The characteristics of the peptide cycle are specified by square brackets.
1. The opening square bracket indicates the building block at whose side chain the cycle is initiated and
2. the closing square bracket indicates the building block at whose side chain the cycle is terminated.

The chemical nature of the connection between these two residues within the peptide macrocycle is
1. a disulfide bond in case that those indicated residues/amino acids contain sulfhydryl moieties (e.g., Cys), or
2. an amide bond in case that among those indicated residues one residue contains an amino function in its side chain (e.g. Dab) while the other contains a carboxyl function in its side chain (e.g. Glu).

### Cyclic peptides containing an additional cyclization element (Yc)

A general extended cyclic peptide written from the N-to C-terminal direction is shown below:

NT1-Xaa1-[Xaa2(Yc)-Xaa3-Xaa4-.......Xaan]-CT;

Therein the statements 1. - 3. of the description of linear peptides for the specification of "Xaax", "NT1" and "CT" in the main chain of the cyclic peptide apply. In addition, "Yc" is the cyclization element. As in case of cyclic peptides, the characteristics of the cycle are specified by square brackets which indicate the building block at whose side chain the cycle is initiated and the building block at whose side chain the cycle is terminated (statements 1. and 2. of cyclic peptides).

The abbreviation of the structure representing the Yc element of the extended cycle is the content of the parentheses left adjacent to the building block at whose side chain the cycle is initiated. The Yc element is covalently attached to the side chain of said building block. Furthermore, the Yc element is covalently attached to the side chain of the building block at whose side chain the cycle is terminated. The chemical nature of the linkages between either of these residues the Yc element depend on side chain functionality of both corresponding amino acids Xaan. The linkage is a thioether if the side chain of Xaan contains a sulfhydryl group (e.g., Cys). If the cyclization element Yc is 2MeBn as in ... -[Cys(2MeBn)-... -Cys]-..., the side chains of the two cysteine residues are linked via thioether linkages to 2-methylbenzylidene. If the cyclization element Yc is a methylene bridge as in ... -[Cys(meth)-... -Cys]-..., the linkage between the side chains of the two cysteine residues is a thioacetal.

As non-limiting example for a branched, cyclic peptide containing a Z group, the peptide structure of

Butoc-[Cys-Thr-Lys(DOTA-PPAc)-Tyr-Phe-4Cfp-Hyp-Ile-Nva-Cys]-NH₂

is depicted below:

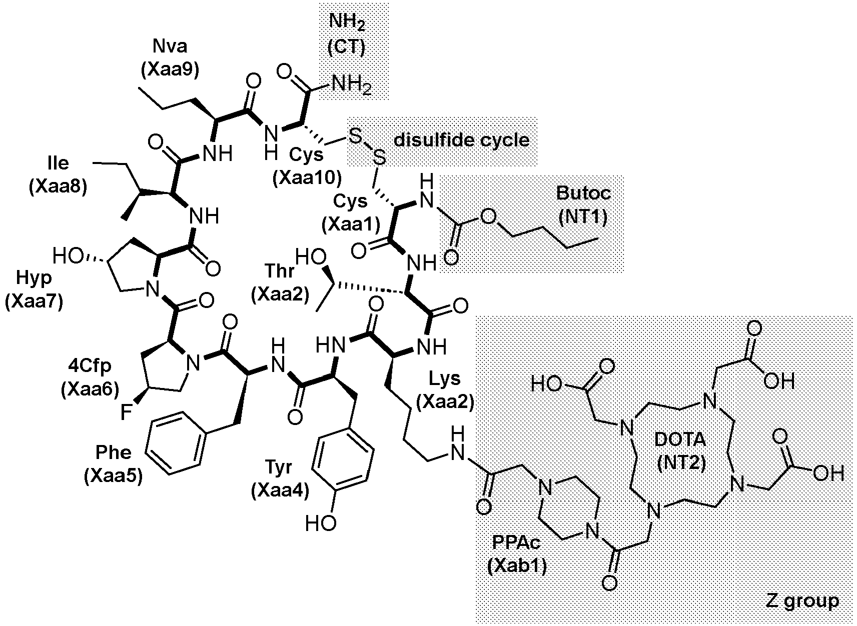

Therein
1. Butoc is the N-terminal end group NT1 in the general formula and represents an N-terminal blocking group which is linked to the alpha amino group of the Cys residue (Xaa1)
2. Cys, Thr, Lys, Tyr, Phe, 4Cfp, Hyp, Ile, Nva and Cys correspond to "Xaa1" to "Xaa10" in the general formula. For clarity the main chain is represented with bold line thickness.
3. NH₂ is the C-terminal end group CT in the general formula and is linked to the carbonyl group of the Cys residue (Xaa10)
4. The opening square bracket (,[') adjacent to the N-terminal cysteine (Xaa1) in the sequence indicates that at this residue the cycle is initiated.
5. The closing square bracket (,]') adjacent to the N-terminal cysteine (Xaa10) in the sequence indicates that at this residue the cycle is terminated.
6. The absence of parentheses adjacent to the Cys (Xaa1) indicates the initiation of the peptide macrocycle with no additional cyclization element, i.e. a direct disulfide linkage to the Cys (Xaa10), which indicates the termination of the cycle.
7. To the amino group of the Lys (Xaa3) residue a Z group is attached. The Z group contains two elements, the chelator DOTA which is N-terminal end group NT2 in the general formula for branched peptides, and the linker PPAc which corresponds to Xab1.

In an embodiment and as preferably used herein, a compound of the present invention also encompasses any pharmaceutically acceptable salt of the compound.

In an embodiment and as preferably used herein, a compound of the present invention also encompasses any pharmaceutically acceptable solvate of the compound.

In an embodiment and as preferably used herein, a compound of the present invention also encompasses any hydrate of the compound.

In an embodiment and as preferably used herein, a compound of the present invention also encompasses a radionuclide chelate complex, wherein such complex comprises a chelator bearing compound, a pharmaceuticaly acceptable salt or sovate thereof, each of the invention, and a radionuclide, preferably the radionuclide being bound by the chelator, wherein preferably the radionuclide is a diagnostically active radionuclide or a therapeutically active radionuclide.

According to the present invention, the use of the compound of the present invention as disclosed herein, also encompasses such use of a pharmaceutically acceptable salt of the compound, of a pharmaceutically acceptable solvate of the compound and/or of a hydrate of the compound.

According to the present invention, a method making use of the compound of the present invention as disclosed herein, also encompasses such method making use of a pharmaceutically acceptable salt of the compound, of a pharmaceutically acceptable solvate of the compound and/or of a hydrate of the compound.

A **"pharmaceutically acceptable salt"** of the compound of the present invention is preferably an acid salt or a base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Compounds of the invention are capable of forming internal salts which are also pharmaceutically acceptable salts.

Suitable pharmaceutically acceptable salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, *i.e.,* 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

A **"pharmaceutically acceptable solvate"** of the compound of the invention is preferably a solvate of the compound of the invention formed by association of one or more solvent molecules to one or more molecules of a compound of the invention. Preferably, the solvent is one which is generally considered in the art to be suitable for use in contact with the tissues of human beings or animals without excessive toxicity or carcinogenicity, and preferably without irritation, allergic response, or other problem or complication. Such solvent includes an organic solvent such as alcohols, ethers, esters and amines.

A **"hydrate"** of the compound of the invention is formed by association of one or more water molecules to one or more molecules of a compound of the invention. Such hydrate includes but is not limited to a hemihydrate, monohydrate, dihydrate, trihydrate and tetrahydrate. Independent of the hydrate composition all hydrates are generally considered as pharmaceutically acceptable.

Hereinafter, in the present description of the invention and the claims, the use of the terms "containing" and **"comprising"** is to be understood such that additional unmentioned elements may be present in addition to the mentioned elements. However, these terms should also be understood as disclosing, as a more restricted embodiment, the term **"consisting of**" as well, such that no additional unmentioned elements may be present, as long as this is technically meaningful.

Unless specified otherwise or the context dictates otherwise, references to groups being "substituted" or "optionally substituted" are to be understood as references to the presence (or optional presence, as the case may be) of at least one substituent selected from F, Cl, Br, I, CN, NOz, NH₂, NH-(C₁-C₆)alkyl, N[(C₁-C₆)alkyl]₂, -X-(C₁-C₆)alkyl, -X-(C₂-C₆)alkenyl, -X-(C₂-C₆)alkynyl, -X-(C₆-C₁₄)aryl, -X-(5-14-membered heteroalkyl with 1-3 heteroatoms selected from N, O, S), wherein X represents a single bond, -(CH₂)-, -O-, -S-, -S(O)-, -S(O)z-, -NH-, -CO-, or any combination thereof including, for instance, - C(O)-NH-, -NH-C(O)-. The number of substituents is not particularly limited and may range from 1 to the maximum number of valences that can be saturated with substituents. It is typically 1, 2 or 3 and usually 1 or 2, most typically 1.

Unless specified otherwise, all valencies of the individual atoms of the compounds or moieties described herein are saturated. In particular, they are saturated by the indicated binding partners. If no binding partner or a too small number of binding partners is indicated, the remaining valencies of the respective atom are saturated by a corresponding number of hydrogen atoms.

Unless specified otherwise, chiral compounds and moieties may be present in the form of a pure stereoisomer or in the form of a mixture of stereoisomers, including the 50:50 racemate. In the context of the present invention, references to specific stereoisomers are to be understood as references to compounds or moieties, wherein the designated stereoisomer is present in at least 90% enantiomeric excess (ee), more preferably at least 95 %ee and most preferably 100 %ee, wherein %ee is defined as (|R-S|)/(R+S)* 100% with R and S representing the amount of moles of the respective enantiomers.

In an embodiment and as preferably used herein, the term **"specifically binds"** refers to the ability of the compound of the invention to bind to its target (i.e., uPAR) with greater affinity than it binds to a non-target. In certain embodiments, specific binding refers to binding for uPAR with an affinity that is at least 10, 50, 100, 250, 500, or 1000 times greater than the affinity for a non-target.

In an embodiment and as preferably used herein, the term **"binding affinity"** refers to the strength of interaction between the compound of the invention and its target (i.e., uPAR) as a function of its association and dissociation constants. Higher affinities typically mean that the compound has a fast on rate (association) and a slow off rate (dissociation). Binding affinities can change under various physiological conditions due to changes that occur to the compound or target under those conditions. Binding affinities of the compound may also change when effectors and/or linkers are attached.

In an embodiment and as preferably used herein, the term **"inhibit"** or **"inhibition of"** means to reduce by a measurable amount, or to prevent entirely.

According to the present invention, a compound comprising a chelator and a nuclide, preferably a metal nuclide, more preferably a radionuclide, whereby such nuclide is chelated, i.e. bound by the chelator by means of coordinative bonds, is also referred to herein as nuclide chelate complex and radionuclide chelate complex, respectively.

It will be appreciated and acknowledged by a person skilled in the art that any reference to a range defined by an upper and a lower value will encompass the upper value, the lower value and any value in between, particularly any integer in between. For example, the term 1 to 4 means 1, 2, 3 and 4.

In an embodiment and as preferably used herein, the term **"therapeutically effective amount"** refers to an amount of compound effective to treat a disease or disorder in a mammal. In the case of cancer, the therapeutically effective amount of the compound may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. When the compound comprises a drug, and to the extent the drug may inhibit growth and/or kill existing cancer cells, it may be cytostatic and/or cytotoxic. For cancer therapy, efficacy can, for example, be measured by assessing the time to disease progression (TTP) and/or determining the response rate (RR).

In an embodiment and as preferably used herein, tthe term **"cytotoxic drug"** (or "cytotoxic agent") refers to a substance that has cytotoxic activity and causes destruction of cells. The term is intended to include radioactive isotopes, chemotherapeutic agents, and toxins such as small molecule toxins or enzymatically active toxins of bacterial, fungal, plant or animal origin, including synthetic analogs and derivatives thereof.

In an embodiment and as preferably used herein, t term **"diagnostically active compound"** refers to a compound which is suitable for or useful in the diagnosis of a disease.

In an embodiment and as preferably used herein, ttThe term **"diagnostic agent"** or **"diagnostically active agent"** refers to a compound which is suitable for or useful in the diagnosis of a disease.

In an embodiment and as preferably used herein, the term **"therapeutically active compound"** refers to a compound which is suitable for or useful in the treatment of a disease.

In an embodiment and as preferably used herein, the term **"therapeutic agent"** or **"therapeutically active agent"** refers to a compound which is suitable for or useful in the treatment of a disease.

In an embodiment and as preferably used herein, t term **"theragnostically active compound"** refers to a compound which is suitable for or useful in both the diagnosis and therapy of a disease.

In an embodiment and as preferably used herein, the terms **"theragnostic agent"** or **"theragnostically active agent"** refers to a compound which is suitable for or useful in both the diagnosis and therapy of a disease.

In an embodiment and as preferably used herein, the term **"thera(g)nostics"** refers to a method for the combined diagnosis and therapy of a disease; preferably, the combined diagnostically and therapeutically active compounds used in theragnostics are radiolabeled.

In an embodiment and as preferably used herein, the expression **"treatment of a disease"** refers to treatment and/or prevention of a disease, e.g., cancer.

In an embodiment and as preferably used herein, the expression **"disease involving uPAR"** refers to a disease where cells including, but not limited to, tumor and tumor-related cells as tumour-associated tumour-associated macrophages, tumour-associated neutrophils, tumour-associated fibroblasts, tumour-associated endothelial and disseminated tumour cells as well as senescent cells, preferably senescent cells associated with lung fibrosis, atherosclerosis, Alzheimer's disease, diabetes, liver fibrosis, chronic kidney disease, osteoarthritis, cardiac fibrosis, and Parkinson's disease, whereby each of said cells express, preferably in an upregulated manner, uPAR and tissue either expressing uPAR, preferably in an upregulated manner respectively, are either a or the cause for the disease and/or the symptoms of the disease, or are part of the pathology underlying the disease. A preferred uPAR-expressing cell is a tumor cell. In an embodiment of the disease, preferably when used in connection with the treatment, treating and/or therapy of the disease, affecting the cells, the tissue and pathology, respectively, results in cure, treatment or amelioration of the disease and/or the symptoms of the disease. In an embodiment of the disease, preferably when used in connection with the diagnosis and/or diagnosing of the disease, labeling of the uPAR-expressing cells and/or of the uPAR-expressing tissue allows discriminating or distinguishing said cells and/or said tissue from healthy or uPAR-non-expressing cells and/or healthy or uPAR-non-expressing tissue. More preferably such discrimination or distinction forms the basis for said diagnosis and diagnosing, respectively. In an embodiment thereof, "labeling" means the interaction of a detectable label either directly or indirectly with the uPAR-expressing cells and/or with the uPAR-expressing tissue or tissue containing such uPAR-expressing cells; more preferably such interaction involves or is based on the interaction of the label or a compound bearing such label with uPAR.

In an embodiment and as preferably used herein, a disease targetable with a urokinase plasminogen activator surface receptor binding compound is a disease which can be treated and/or diagnosed by a compound of the present invention. Preferably, the symptoms of the disease in a subject suffering from the disease will at least be ameliorated upon administering a compound of the present invention to the subject, preferably upon administration of a therapeutically efftive amount of said compound. Alternatively, the disease can be diagnosed in a subject suffering from the disease upon administering a compound of the present invention to the subject, preferably upon administration of a diagnostically effective amount of said compound.

In an embodiment and as preferably used herein associated with urokinase plasminogen activator surface receptor (uPAR) is a disease which can be treated and/or diagnosed by the binding of a compound to uPAR. In case of treatment, the binding of the compound to uPAR will preferably result in the symptoms of the disease in a subject suffering from the disease being at least ameliorated upon administering the compound to the subject, more preferably upon administration of a therapeutically efftive amount of said compound. Alternatively, the disease can be diagnosed in a subject suffering from the disease upon administering the compound of the present invention to the subject, preferably upon administration of a diagnostically effective amount of said compound. In a preferred embodiment, the compound is a compound of the present invention.

In an embodiment and as preferably used herein, the term **"target cell"** refers to a cell which is expressing uPAR and is a or the cause for a disease and/or the symptoms of a disease, or is part of the pathology underlying a disease. uPAR expression can be determined by methods known in the art such as Western blotting.

In an embodiment and as preferably used herein, the term **"non-target cell"** refers to a cell which is either not expressing uPAR and/or is not a or the cause for a disease and/or the symptoms of a disease, or is part of the pathology underlying a disease.

In an embodiment and as preferably used herein, the term **"neoplasm"** refers an abnormal new growth of cells. Typically, the cells in a neoplasm grow more rapidly than normal cells and will continue to grow if not treated. A neoplasm may be benign or malignant.

In an embodiment and as preferably used herein, the term **"tumor"** refers to a mass lesion that may be benign or malignant.

In an embodiment and as preferably used herein, the terms **"cancer"** and **"cancerous"** refer to or describe the physiological condition or disorder in mammals that is typically characterized by unregulated cell growth. A "tumor" comprises one or more cancerous cells. A "cancer" may be a malignant neoplasm.

In an embodiment and as preferably used herein, the term **"patient"** refers to a subject to which the compound of the invention is administered. Examples of a "patient" include, but are not limited to, a human, rat, mouse, guinea pig, non-human primate, pig, goat, cow, horse, dog, cat, bird and fowl. Typically, a patient is a rat, mouse, dog, non-human primate or human. In some aspects, the patient is a human in need of an effective amount of the compound.

In an embodiment and as preferably used herein, the terms **"treat"** or **"treatment"** unless otherwise indicated by context, refer to therapeutic treatment and prophylactic measures to prevent relapse, wherein the object is to inhibit or slow down (lessen) an undesired physiological change or disorder, such as, for example, the development or spread of cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder.

In the context of cancer, the term **"treating"** includes any or all of inhibiting growth of tumor cells, cancer cells, or of a tumor; inhibiting replication of tumor cells or cancer cells, lessening of overall tumor burden or decreasing the number of cancerous cells, and ameliorating one or more symptoms associated with the disease.

Unless the context dictates otherwise, and/or alternative meanings are explicitly provided herein, all terms are intended to have meanings generally accepted in the art, as reflected by IUPAC Gold Book (status of 1st Nov. 2023), or the Dictionary of Chemistry, Oxford, 8th Ed.

In accordance with the present invention, the compound of the present invention may comprise one or more of a Z group. The Z group comprises a bio-distribution modifier with an optional linker or a chelator with an optional linker. If the compound comprises more than one Z group, the groups Z may be identical or different and each Z group may be independently selected from the group consisting of bio-distribution modifier with an optional linker and a chelator with an optional linker.

In one embodiment, the compound of the invention comprises one or more of Z groups, wherein each Z group comprises a bio-distribution modifier with an optional linker or a chelator with an optional linker, wherein each Z group may be independently selected from the group consisting of bio-distribution modifier with an optional linker and a chelator with an optional linker. If the compound comprises more than one Z group, the groups Z may be identical or different and each Z group may be independently selected from the group consisting of bio-distribution modifier with an optional linker and a chelator with an optional linker.

In a preferred embodiment, the compound of the invention comprises one or more groups Z, wherein each group Z is independently a moiety represented by the following formula (XVa):

E-L-* (XVa)

wherein,
* indicates covalent attachment to Xaa0, Xaa2, Xaa3 or the C-terminal carbonyl group of Xaa10, L is a linker, and
E is selected from the group consisting of a chelator optinally comprising a linker and further optionally comprising a nuclide, preferably a radionuclide, and a bio-distribution modifier optionally comprising a linker.

In a more preferred embodiment, each Z group comprises a chelator and an optional linker, wherein said chelator optionally comprises a nuclide, preferably a radionuclide.

As preferably used herein, a linker is an element, moiety, or structure, which connects two parts of a molecule. In the present invention, the linker forms a covalent bond with the chelator or the bio-distribution modifier or both and the respective part of the compounds of invention where the Z group is attached to. A first functional group of the linker forming the bond with the chelator or the bio-distribution modifier may in principle, be any chemical group, which is capable of forming a bond, preferably a covalent bond with the chelator or the bio-distribution modifier group. In addition, the linker comprises a second functional group forming the bond, preferably a covalent bond with the part of the compound of the invention at the specified positions, where the Z group is attached. Such second functional group may in principle, be any chemical group, which is capable of forming such bond with said part of the compound of the invention at the specific positions where the Z group is attached.

An important property or feature of a linker is that it spaces apart the chelator or the bio-distribution modifier from the peptide part of the compound of invention. This is especially important in cases where the target binding ability of the peptide is compromised by the close proximity of the chelator or the bio-distribution modifier. However, the overall linker length in its most extended conformer should not exceed 200 Å, preferably not more than 150 Å and most preferably not more than 100 Å.

A further important feature or property of a linker is that it enables the attachment of the chelator or the bio-distribution modifier to suitable positions of the peptide part of the compound of invention.

In an embodiment, a residue of a dicarboxylic acid represents a building block for a linker. Dicarboxylic acid-based linkers are used to enable the attachment of a chelator or a bio-distribution modifier to the N-terminal amine of the peptide of invention or an amino group of the side chain of an amino acid residue of the peptide of invention. Such N-terminal amine or amino side chain provides an amino group as functional group to which one of the at least two carboxyl groups provided by the dicarboxylic acid building block is covalently attached by an amide linkage. The bio-distribution modifier or chelator provides an amino group as functional group to which another of the at least two carboxyl groups provided by the dicarboxylic acid building block is covalently attached by an amide linkage, too. The very kind of dicarboxylic acid used as building block for a linker is dependent on the nature of the bio-distribution modifier or chelator and the attachment site on the peptide of invention and more specifically any functional group available at or provided by the bio-distribution modifier and chelator, respectively, which is reacted with one of the at least two carboxyl groups of the dicarboxylic acid building block.

Preferred building blocks are dicarboxylic acids, which are selected from formulae (Lin1), (Lin2) or (Lin3)

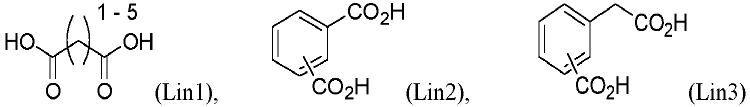

More preferred dicarboxylic acid building blocks such as formula (Lin1) are malonic acid, succinic acid, glutaric acid, adipic acid, wherein the two carboxylic acid groups are attached to ends of the aliphatic chain; such as formula (Lin2) phthalic acid, terephthalic acid, isophthalic acid and such as formula (Lin3) 2-, 3- or 4-carboxy-phenyl acetic acid. The most preferred dicarboxylic acid building block is glutaric acid (Glutar).

In an embodiment, a residue of a diamine represents a building block for a linker. Diamine based linkers are used to enable the attachment of a chelator or a bio-distribution modifier to the C-terminal carboxylic acid moiety of the peptide of invention or a carboxyl group of the side chain of an amino acid residue of the peptide of invention. Such carboxyl-containing C-terminal group or carboxyl-containing side chain provides a carboxyl group as functional group to which one of the at least two amino groups provided by the diamine is covalently attached forming an amide linkage. The bio-distribution modifier or chelator provides a carboxyl group as functional group to which another of the at least two amino groups provided by the diamine building block is covalently attached forming an amide linkage, too. The very kind of diamine used as building block for a linker is dependent on the nature of the bio-distribution modifier or chelator and the attachment site on the peptide of invention and more specifically any functional group available at or provided by the bio-distribution modifier and chelator, respectively, which is reacted with one of the at least two amino groups of the diamine building block.

Preferred building blocks are diamines, which are selected from building blocks of the following formulae (Lin4), (Lin5), (Lin6), (Lin7) and (Lin8):

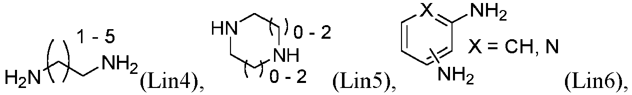

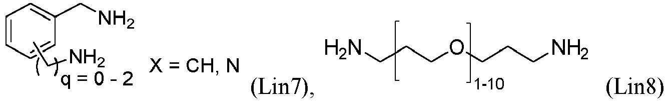

More preferred diamine building blocks are ethylenediamine (en), 1,4-diaminobutane, 1,5-diaminopentane (Ape), 1,6-diaminohexane, 4,7,10-trioxa-1,13-tridecanediamine, 1,3-dazetine, piperazine (PP), 1,3-diazinane, o-, m- and p-phenylenediamine, 4-aminobenzylamine, and 1,4-bis(aminomethyl)benzene. Most preferred diamino building blocks are 1,5-diaminepentane (Ape), ethylenediamine (en), and piperazine (PP).

In an embodiment, a residue of an amino acid represents the building block for a linker. Amino acid-based linkers are used to enable the attachment of a chelator or a bio-distribution modifier to the N-terminal amine, an amino group comprised in the side chain of an amino acid residue or to the C-terminal carboxyl group of the compound of invention. Such N-terminal amine or amino side chain of the compound of invention provides an amino group to which the carboxyl group of the amino acid linker is attached covalently by formation of an amide bond. The bio-distribution modifier or chelator provides a carboxyl group as functional group, to which the amino group of the amino acid linker is attached covalently by formation of an amide bond. Furthermore, to the amino group of the linker, the carboxyl group of a further linker can be attached covalently by an amide bond, which preferably is an α-amino acid linker. As an alternative such C-terminal carboxyl group of the compound of the invention provides a carboxyl group to which the amino group of the amino acid linker is attached covalently by formation of an amide bond. In this case, the bio-distribution modifier or chelator provides an amino group as functional group to which the carboxyl group of the linker is attached covalently by formation of an amide bond. Furthermore, to the carboxyl group of the linker, the amino group of another linker can be attached covalently by formation of an amide bond, which preferably is an α-amino acid linker. It is within the present invention that two or more amino acid linkers can be covalently attached to each other, forming a peptidic linker with an amino group and a carboxyl group which equally well functions as an amino acid-based linker.

Preferred building blocks are amino acids, which are selected from amino acids of any of the following formulae (Lin9), (Lin10), (Lin11), (Lin12) and (Lin13):

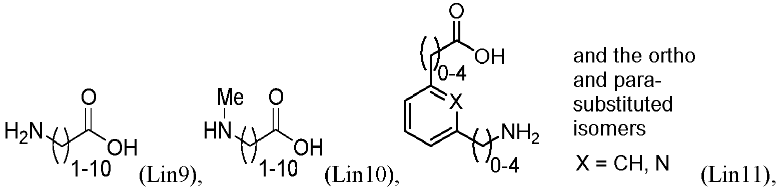

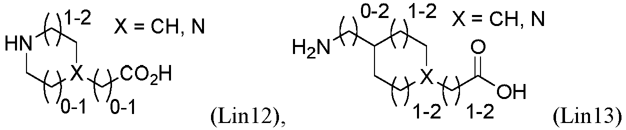

It is within the present invention that such amino acid linker is not further substituted. It is, however, also within the present invention that such amino acid linker is further substituted; preferably such substitution is CO-NH₂ and/or Ac-NH-.

Representative amino acids of the formula Lin8 inlcude glycine (Gly), β-alanine (Bal), γ-aminobutyric acid (Gab), 5-amino-pentanoic acid, 6-amino-hexanoic acid and homologs thereof with up to 10 CH₂ groups.

Representative amino acids of the formula Lin10 include N-methylglycine (Nmg), *N*-methyl-β-alanine, A-methyl-gamma-aminobutyric acid, N-methyl-5-amino-pentanoic acid, N-methyl-6-amino-hexanoic acid, and homologs thereof with up to 10 CH₂ groups.

Representative amino acids of the formula Lin11 inlcude 3-aminomethyl-benzoic acid, 4-aminomethyl-benzoic acid, anthranilic acid, 3-amino-benzoic acid, and 4-amino-benzoic acid.

Representative amino acids of the formula Lin12 include 4-carboxymethyl-piperidine (Cmp), 3-piperidinecarboxylic acid (nipecotic acid, Rni), and 4-piperidinecarboxylic acid (isonipecotic acid, Inp).

Representative amino acids of the formula Lin13 include (4-aminopiperidin-1-yl)acetic acid (APAc), 2-(piperazin-1-yl)-acetic acid (PPAc), and 4-(aminomethyl)cyclohexanecarboxylic acid (tranexamic acid, 4Amc).

It will be appreciated by a person skilled in the art that in case of amino acids with stereogenic centers all stereoisomeric forms may be used as such a linker.

In a further embodiment, the amino acid is an amino acid which contains, preferably as a backbone, a polyether. Preferably, such polyether is polyethylene glycol and consists of up to 30 monomer (ethylene oxide) units. Preferably, an amino acid comprising such polyether shows an increase in hydrophilicity compared to an amino acid not comprising such polyether. If incorporated as a linker the result is typically an increase in hydrophilicity which may, in turn, improve the solubility of the compound of the invention. A preferred embodiment of this kind of amino acid is depicted by the following formula Lin14, wherein it will be acknowledged that such amino acid may comprise 0, 1, 2, 3, 4, 5, 6, 7, 8, 9, 10, 11 or 12 ethylene oxide units:

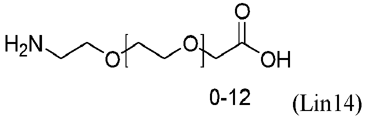

Preferred ethylene glycol containing amino acids are Ttds, O2Oc, PEG6, and PEG12.

In a further embodiment, a residue of an alpha amino acid represents a building block for a linker. Alpha amino acid-based linkers are used to enable the attachment of a chelator and/or a bio-distribution modifier to the N-terminal amine, an amino group comprised in the side chain of an amino acid residue of the compound (peptide) of invention or the C-terminal carboxyl group of the compound (peptide) of the invention. Thereby, up to two chelators, a chelator and a bio-distribution modifier or two bio-distribution modifiers can be attached to one site of the compound of the invention. Such N-terminal amino group or amino-containing side chain provides an amino group to which one of the one or more carboxyl groups provided by the alpha amino acid building block is covalently attached by formation of an amide linkage. Such C-terminal carboxyl group provides a carboxyl group as functional group to which one of the one or more amino groups provided by the alpha amino acid is covalently attached by formation of an amide linkage. In such cases, wherein the alpha amino acid has more than one amino function, the bio-distribution modifier or chelator provides a carboxyl group as functional group, which is attached covalently to said amino group of the alpha amino acid by an amide linkage, while the other one of the amino groups is used as a linkage to the compound (peptide) of the invention. In such cases wherein the alpha amino acid has more than one carboxyl groups, the bio-distribution modifier or chelator provides an amino group as functional group, which is attached covalently to said carboxyl groups of the alpha amino acid by an amide linkage, while the other one of the carboxyl groups is used as a linkage to the compound of the invention. It is within the present invention that when an alpha amino acid building block contains multiple amino groups, that the carboxyl group and any of the amino groups can be used to mediate linkages to both the chelator or bio-distribution modifier and the compound (peptide) of the invention while the other amino groups which are not engaged in amide linkages can fine-tune the physicochemical properties of the compound. In one embodiment, such amino groups (which are not engaged in amide linkages) can be modified by up to three methyl groups. It is also within the present invention that when an alpha amino acid building block contains multiple carboxyl groups, that the amino group and any of the carboxyl groups can be used to mediate linkages to both the chelator or bio-distribution modifier and the compound (peptide) of the invention while the other carboxyl groups which are not engaged in amide linkages can fine tune the physicochemical properties of the compound. In one embodiment, such carboxyl groups (which are not engaged in amide linkages) can optionally be replaced by carboxamide groups or sulfonate groups.

Preferred building blocks are alpha amino acids, which are selected from building blocks of the following formula (Lin15), (Lin16), (Lin17) or (Lin18):

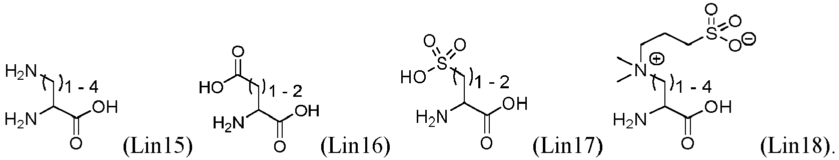

More preferred alpha amino acids building blocks are di-amino-propionic acid (Dap), di-amino-butyric acid (Dab), ornithine (Om), lysine (Lys), aspartic acid (Asp), cysteic acid (Cya) and glutamic acid (Glu). The most preferred alpha amino acid building blocks are lysine and glutamic acid. It will be appreciated by a person skilled in the art that in case of alpha amino acids with stereogenic centers all stereoisomeric forms may be used as such a linker.

In a further embodiment, the branching property allowing attachment of multiple chelators and/or bio-distribution modifiers in the compound of the invention can also be realized when using residues of tricarboxylic acids or triamines as a linker. Preferred building blocks are represented by formula (Lin19) or (Lin20):

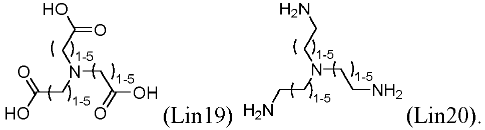

More preferred building blocks are nitrilotriacetic acid (Nta) and tris(2-aminoethyl)amine.

The use of linkers usually follows a purpose. In some circumstances, a linker can be used to space apart moieties in a compound, such as a larger moiety from a bioactive molecule, in order to retain high bioactivity. In other circumstances, the introduction of a linker allows for tuning physicochemical properties of the compound by introducing polarity and/or multiple charges. In certain circumstances, the site of attachment within the sequence of the compound of the invention and the chemical nature of the bio-distribution modifier or chelator necessitate the use of a linker. Further, in certain circumstances, it might be desirable to combine the chelator with a compound without the need of a linker.

Compounds of the present invention where the chelator or bio-distribution modifier is directly attached to the N-terminal amino group, the side chain of an amino acid residue or the C-terminal carboxyl group by forming an amide bond typically perform excellently without the use of any dedicated linker.

In a preferred embodiment, the compound of the invention comprises one or more Z group, wherein each Z group is independently a moiety represented by the following formula (XVb):

E-L-* (XVb)

wherein,
* indicates covalent attachment to Xaa0, Xaa2, Xaa3 or the C-terminal carbonyl group of Xaa10, and
E is selected from the group consisting of a chelator optionally comprising a nuclide, preferably a radionuclide, and a bio-distribution modifier, and
L is a linker moiety represented by any one of the formulae (XVIIa) to (XVIIy): 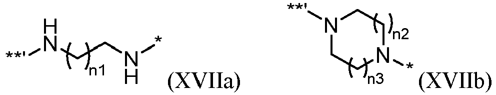 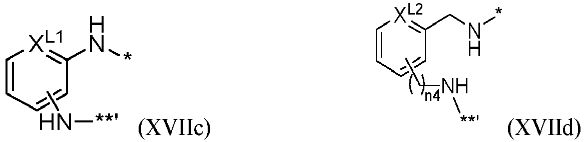 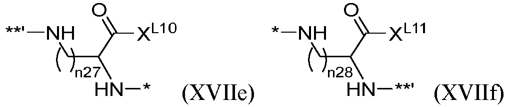 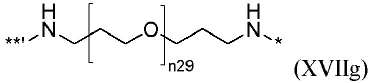 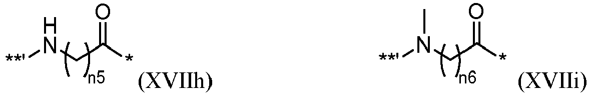 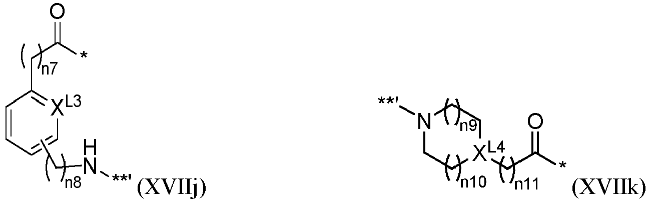 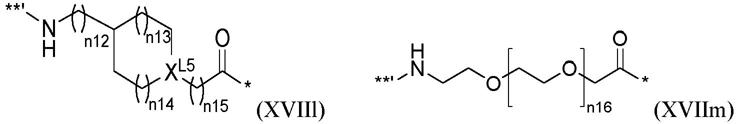 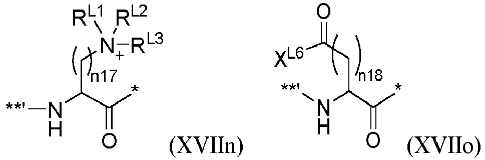 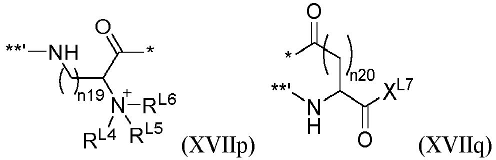 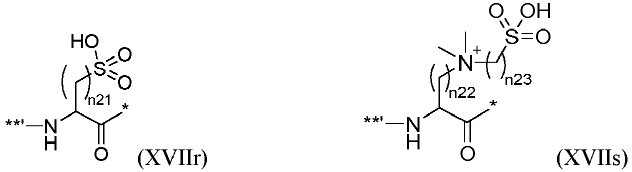 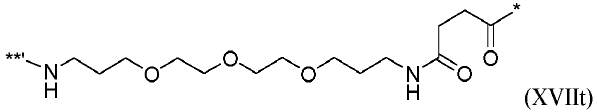 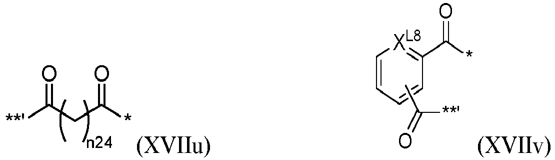 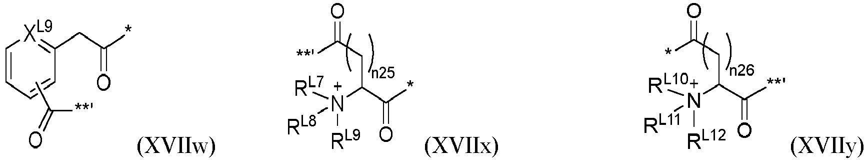 wherein,
   n1 and n24 are each and independently 1, 2, 3, 4, or 5,
   n2, n3, n4 and n12 are each and independently 0, 1 or 2,
   n5, n6 and n29 are each and independently 1, 2, 3, 4, 5, 6, 7, 8, 9 or 10,
   n7 and n8 are each and independently 0, 1, 2, 3 or 4,
   n9, n13, n14, n15, n18 n20, n21, n25 and n26 are each and independently 1 or 2,
   n 10 and n11 are each and independently 0 or 1,
   n16 is 0 to 12,
   n17, n19, n22, n27 and n28 are each and independently 1, 2, 3 or 4,
   n23 is 2, 3 or 4,
   X^{L1}, X^{L2}, X^{L3}, X^{L4}, X^{L5}, X^{L8} and X^{L9} are each and independently =CH- or =N-,
   X^{L6}, X^{L7}, X^{L10} and X^{L11} are each and independently -OH or -NH₂,
   R^{L1}, R^{L2}, R^{L3}, R^{L4}, R^{L5}, R^{L6}, R^{L7}, R^{L8}, R^{L9}, R^{L10}, R^{L12}, and R^{L12} are each and independently -H or a methyl group,
   * indicates covalent attachment to Xaa0, Xaa2, Xaa3 or the C-terminal carbonyl group of Xaa10, and
   **' indicates covalent attachment to E.

It will be recognized by a person skilled in the art that when a radioactive nuclide is present in the compound of the invention, the radioactive nuclide is selected taking into consideration the disease to be treated and/or the disease to be diagnosed, respectively, and/or the particularities of the patient and/or patient group, respectively, to be treated and/or to be diagnosed, respectively.

In the present invention, the "radioactive nuclide" may also be referred to as "radionuclide" and vice versa. Radioactive decay is the process by which an atomic nucleus of an unstable atom loses energy by emitting ionizing particles (ionizing radiation). There are different types of radioactive decay. A decay, or loss of energy, results when an atom with one type of nucleus, called the parent radionuclide, transforms to an atom with a nucleus in a different state, or to a different nucleus containing different numbers of protons and neutrons. Either of these products is named the daughter nuclide. In some decays the parent and daughter are different chemical elements, and thus the decay process results in nuclear transmutation (creation of an atom of a new element). For example, the radioactive decay can be alpha decay, beta decay, and gamma decay. Alpha decay occurs when the nucleus ejects an alpha particle (helium nucleus). This is the most common process of emitting nucleons, but in rarer types of decays, nuclei can eject protons, or specific nuclei of other elements (in the process called cluster decay). Beta decay occurs when the nucleus emits an electron (β'-decay) or positron (β⁺-decay) and a type of neutrino, in a process that changes a proton to a neutron or the other way around. By contrast, there exist radioactive decay processes that do not result in transmutation. The energy of an excited nucleus may be emitted as a gamma ray in gamma decay, or used to eject an orbital electron by interaction with the excited nucleus in a process called internal conversion, or used to absorb an inner atomic electron from the electron shell whereby the change of a nuclear proton to neutron causes the emission of an electron neutrino in a process called electron capture (EC), or may be emitted without changing its number of proton and neutrons in a process called isomeric transition (IT). Another form of radioactive decay, the spontaneous fission (SF), is found only in very heavy chemical elements resulting in a spontaneous breakdown into smaller nuclei and a few isolated nuclear particles.

In a preferred embodiment of the present invention, the radionuclide can be used for labeling of the compound of the invention.

In an embodiment of the present invention, the radionuclide is suitable for complexing with a chelator, leading to a radionuclide chelate complex.

In a further embodiment, one or more atoms of the compound of the invention are of non-natural isotopic composition, preferably these atoms are radionuclides; more preferably radionuclides of carbon, oxygen, nitrogen, sulfur, phosphorus and halogens: These radioactive atoms are typically part of amino acids, in some case halogen containing amino acids, and/or building blocks and in some cases halogenated building blocks each of the compound of the invention.

In a preferred embodiment of the present invention, the radionuclide has a half-life that allows for diagnostic and/or therapeutic medical use. Specifically, the half-life is between 1 min and 100 days.

In a preferred embodiment of the present invention, the radionuclide has a decay energy that allows for diagnostic and/or therapeutic medical use. Specifically, for γ-emitting isotopes, the decay energy is between 0.004 and 10 MeV, preferably between 0.05 and 4 MeV, for diagnostic use. For positron-emitting isotopes, the mean decay energy is between 0.6 and 13 MeV, preferably between 1 and 6 MeV, for diagnostic use. For β⁻-emitting isotopes, the mean decay energy is between 0.02 and 3 MeV, preferably between 0.1 and 1 MeV, for therapeutic use. For α-emitting isotopes, the decay energy is between 3 and 10 MeV, preferably between 3.9 and 7 MeV, for therapeutic use.

In a preferred embodiment of the present invention, the radionuclide is industrially produced for medical use. Specifically, the radionuclide is available in GMP quality.

In a preferred embodiment of the present invention, the daughter nuclide(s) after radioactive decay of the radionuclide are compatible with the diagnostic and/or therapeutic medical use. Furthermore, the daughter nuclides are either stable or further decay in a way that does not interfere with or that even support the diagnostic and/or therapeutic medical use. Representative radionuclides which may be used in connection with the present invention are summarized in Table 6.

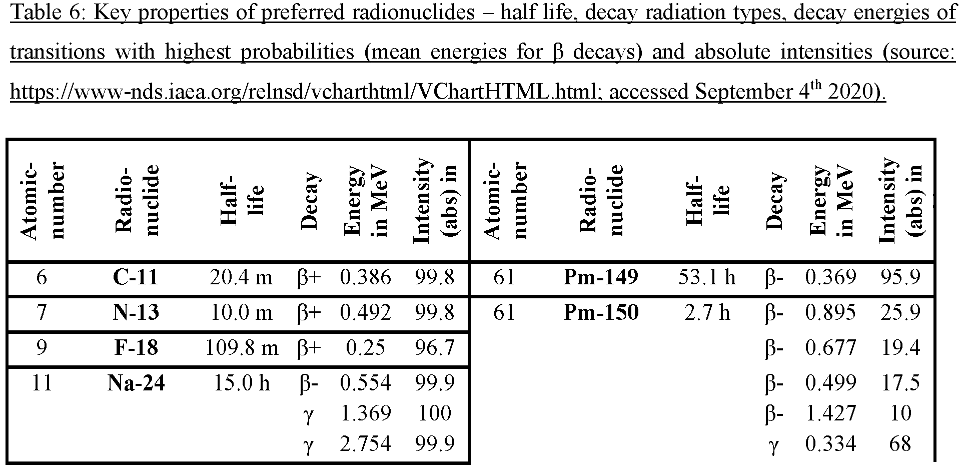

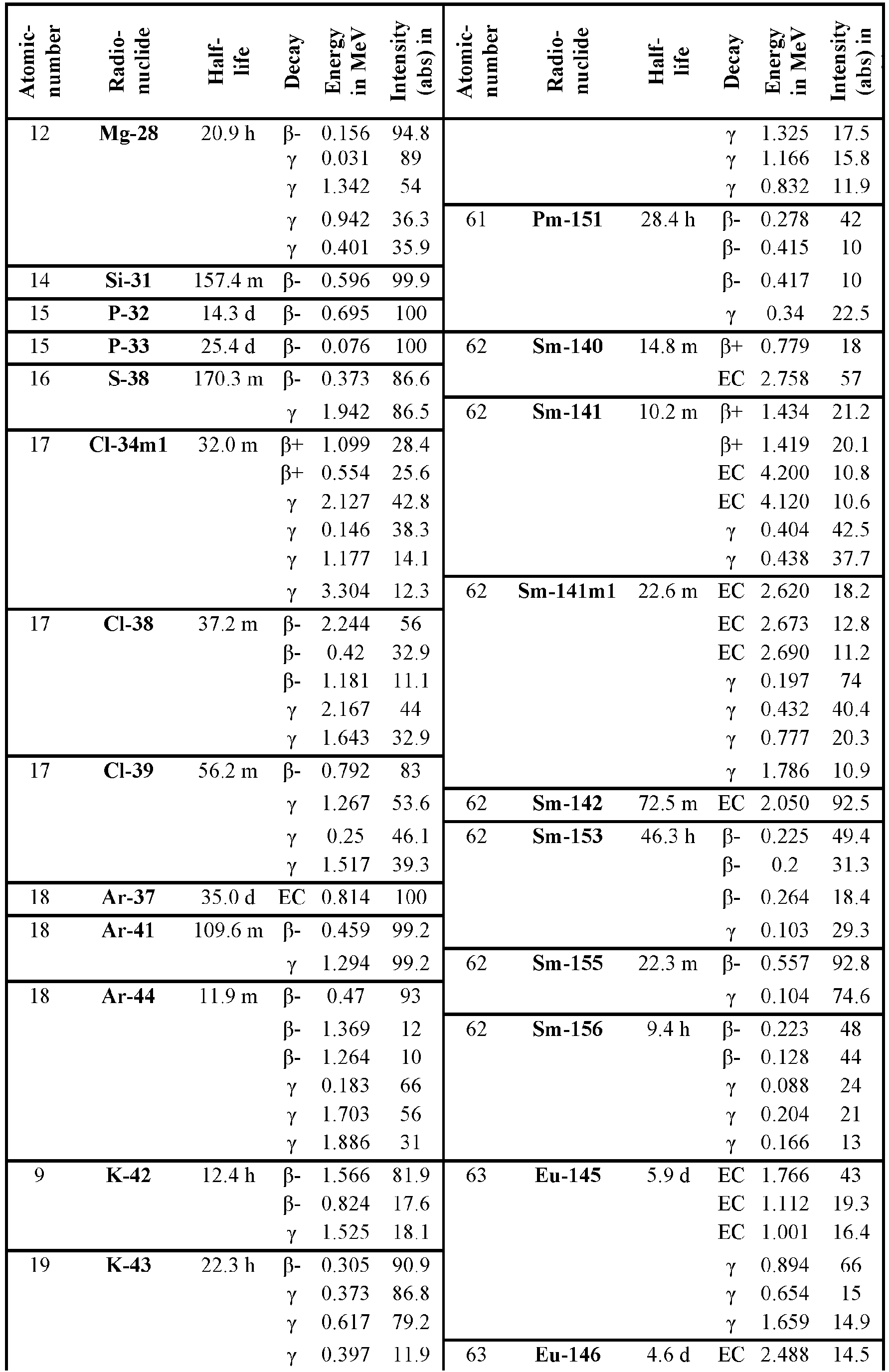

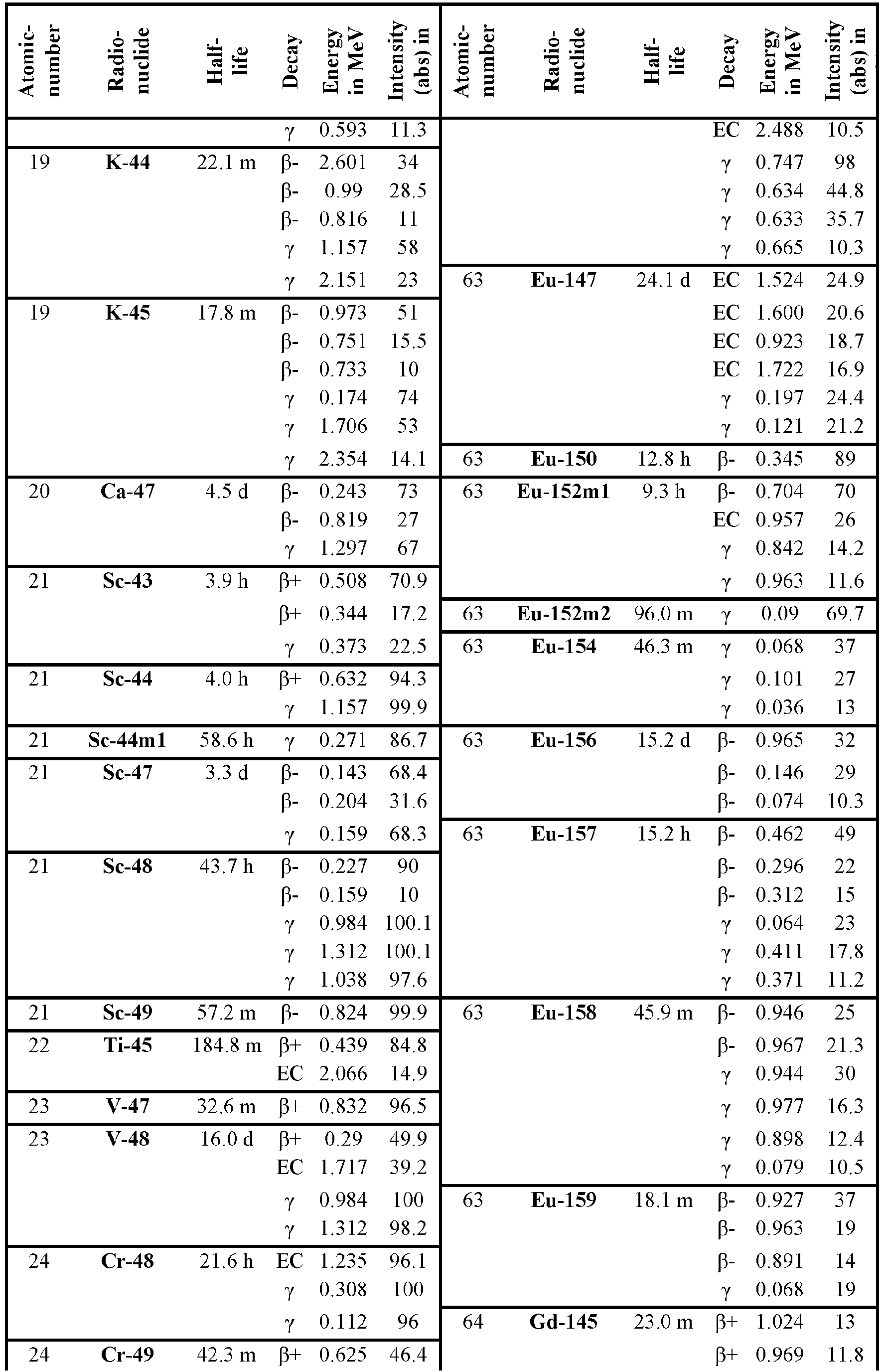

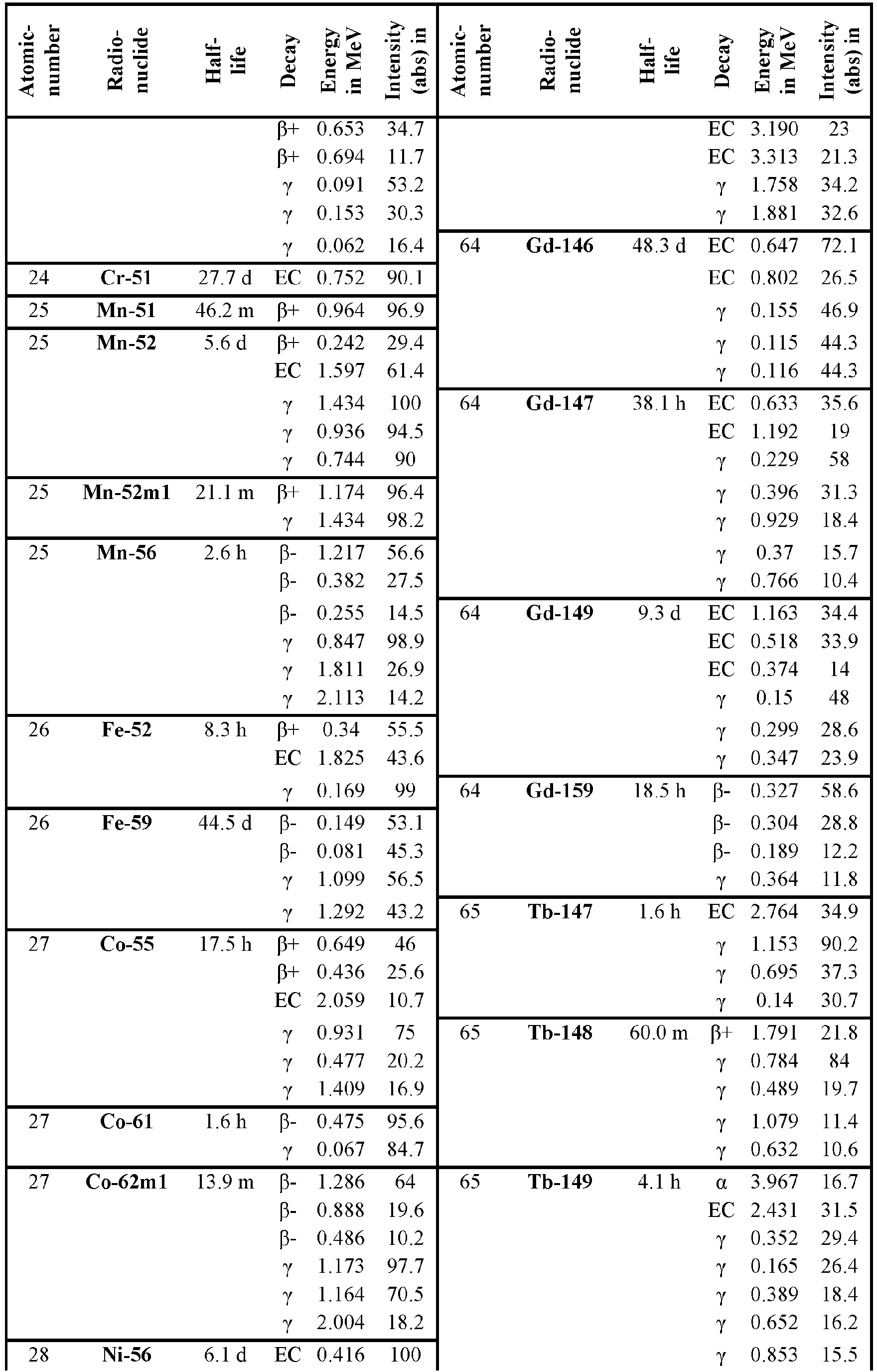

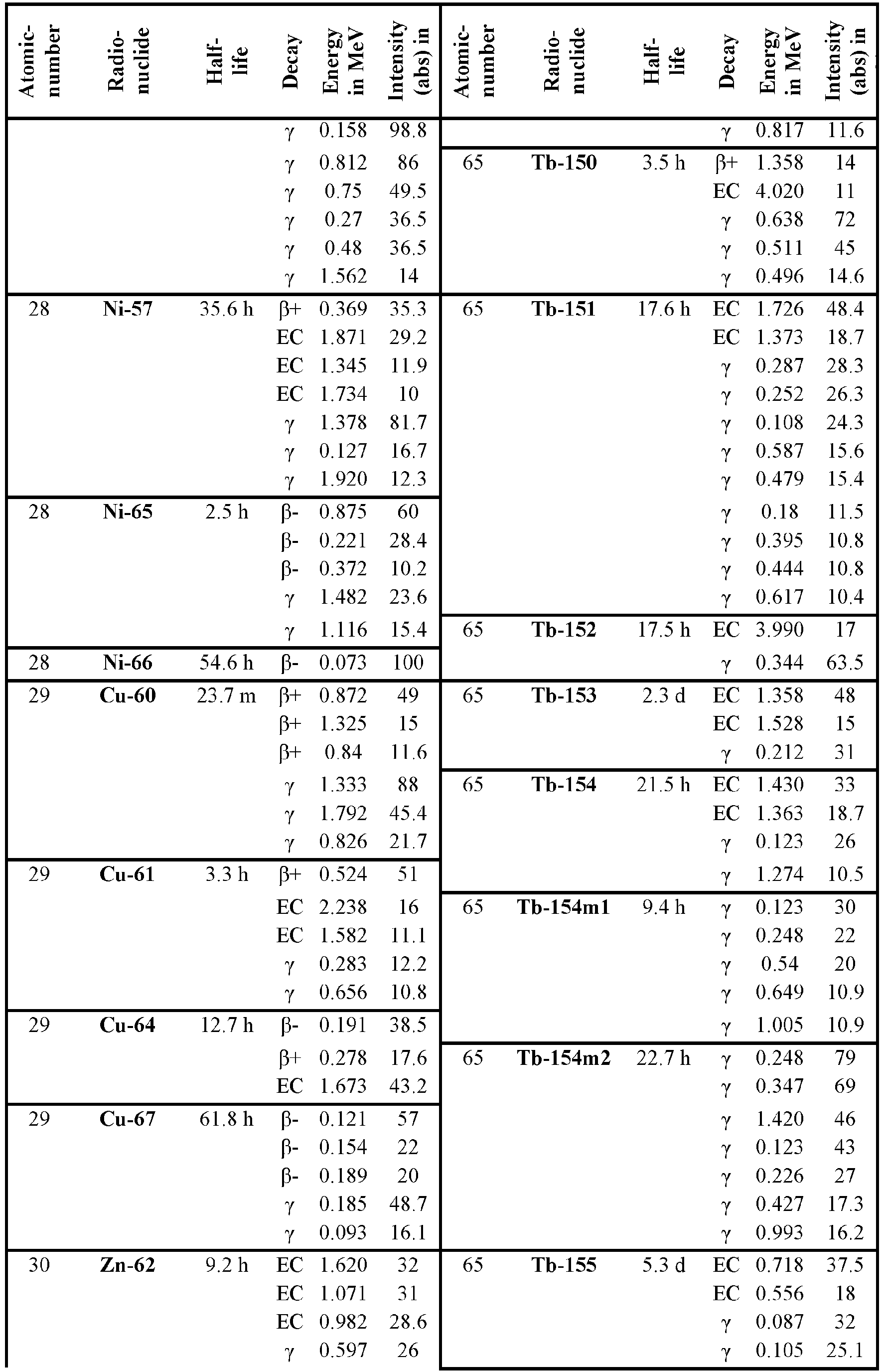

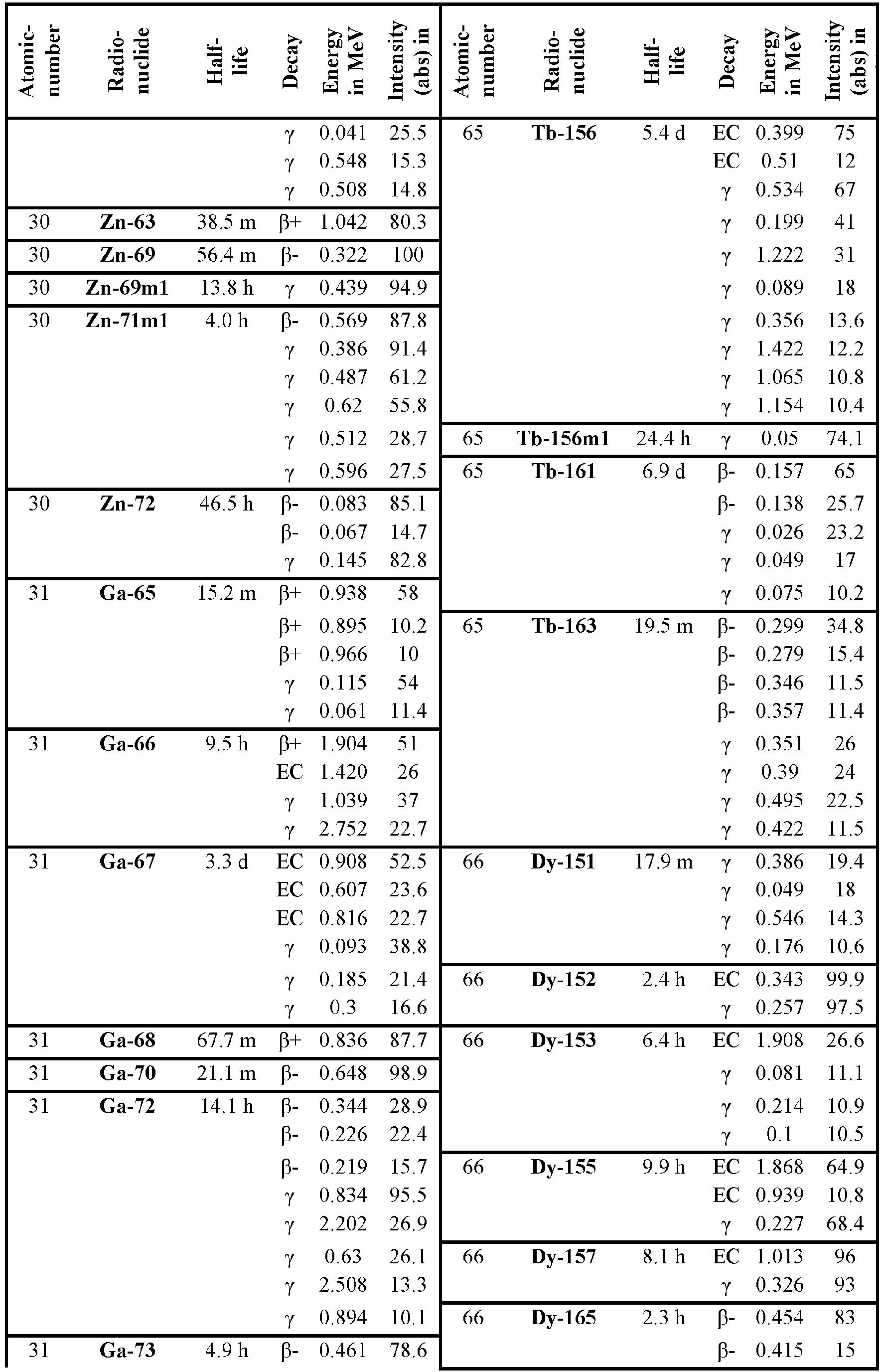

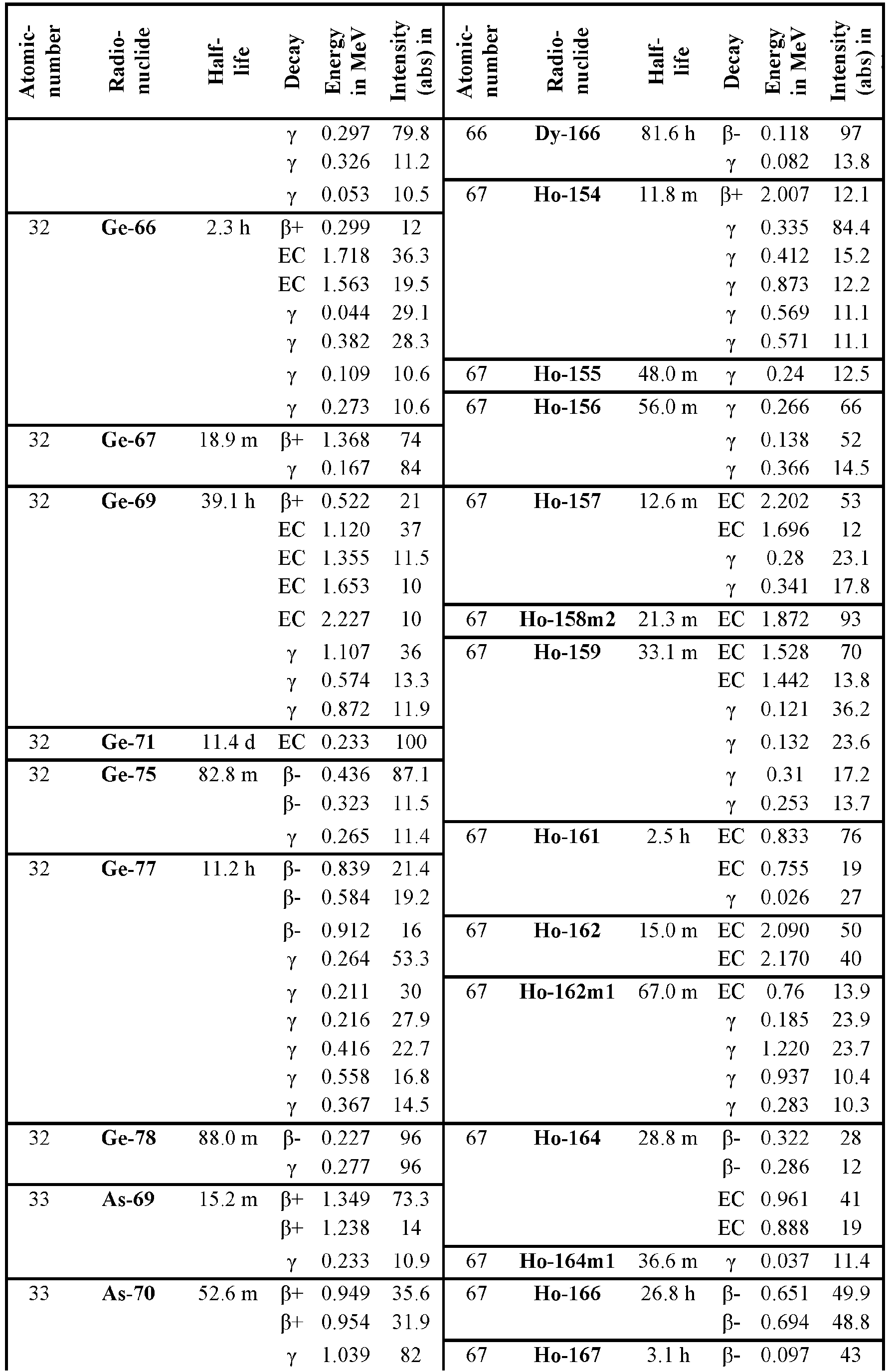

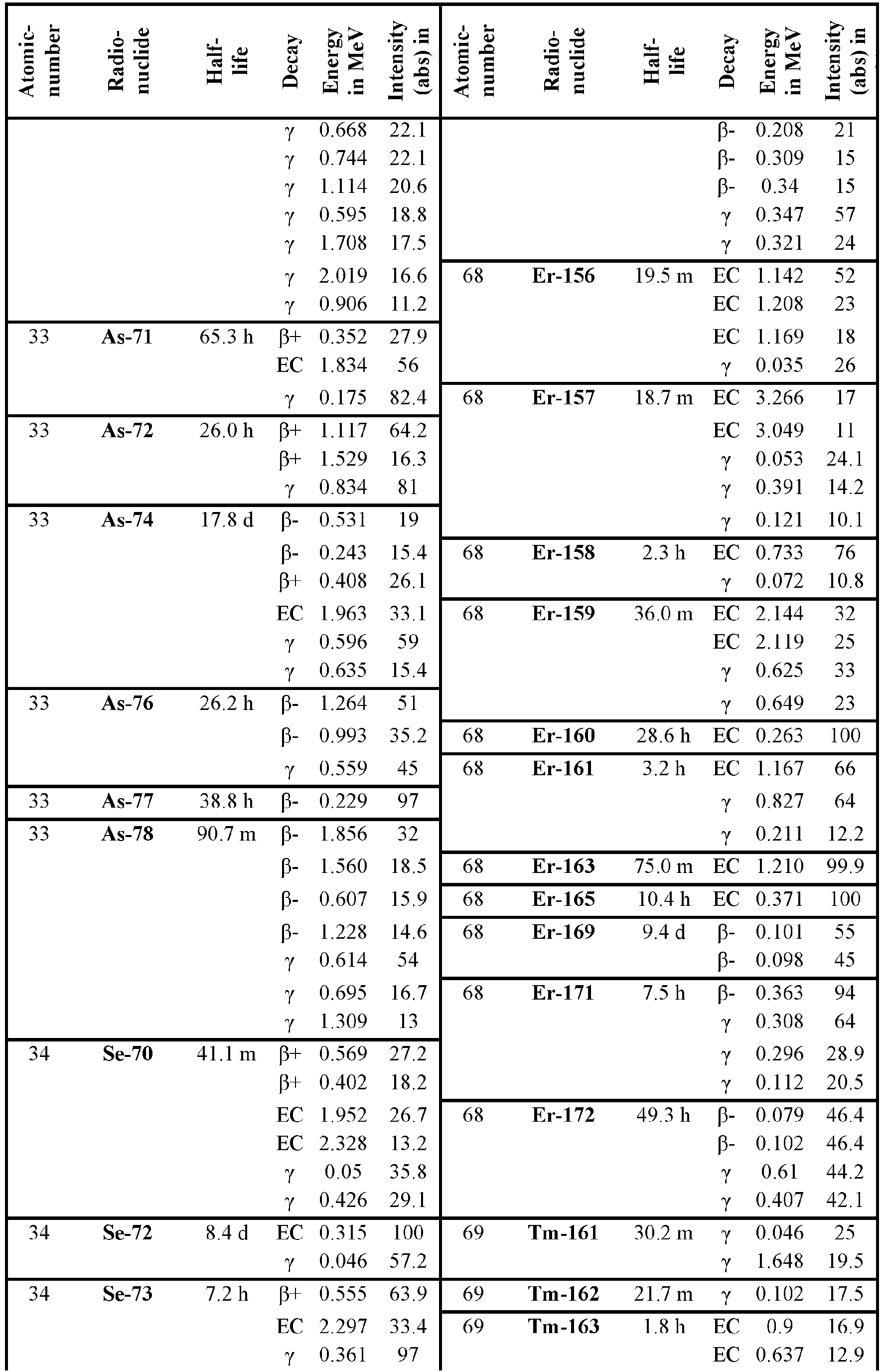

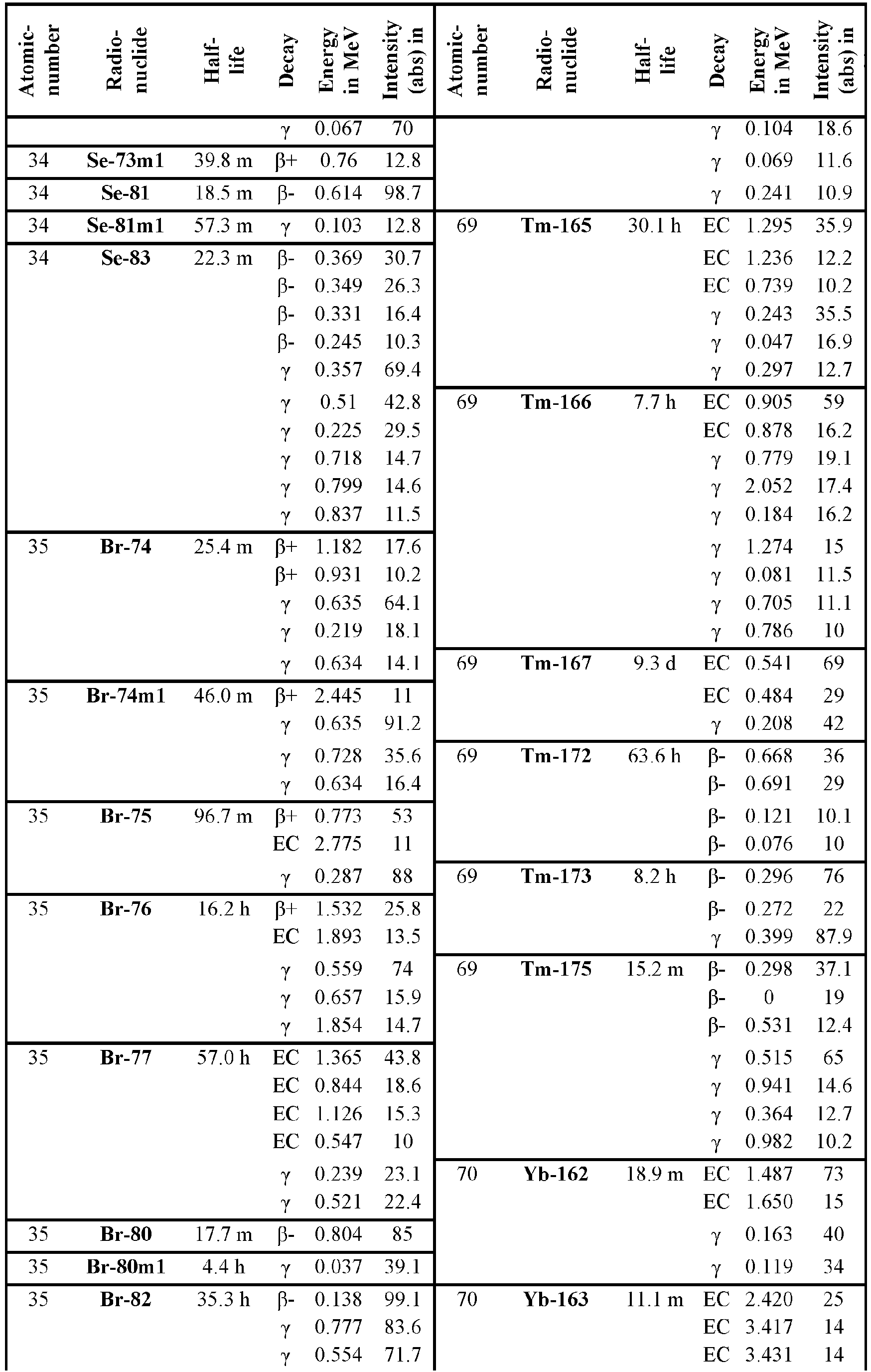

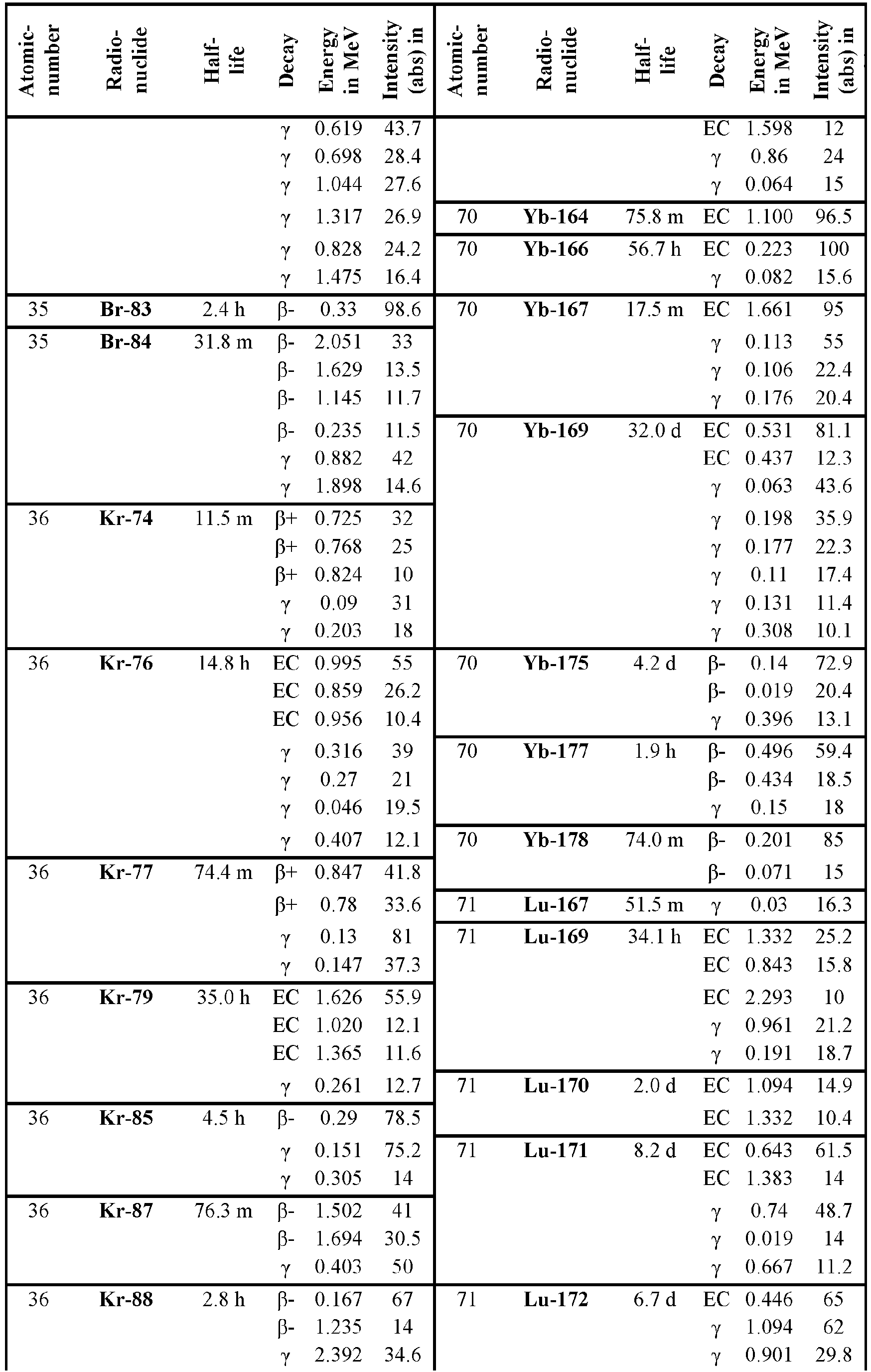

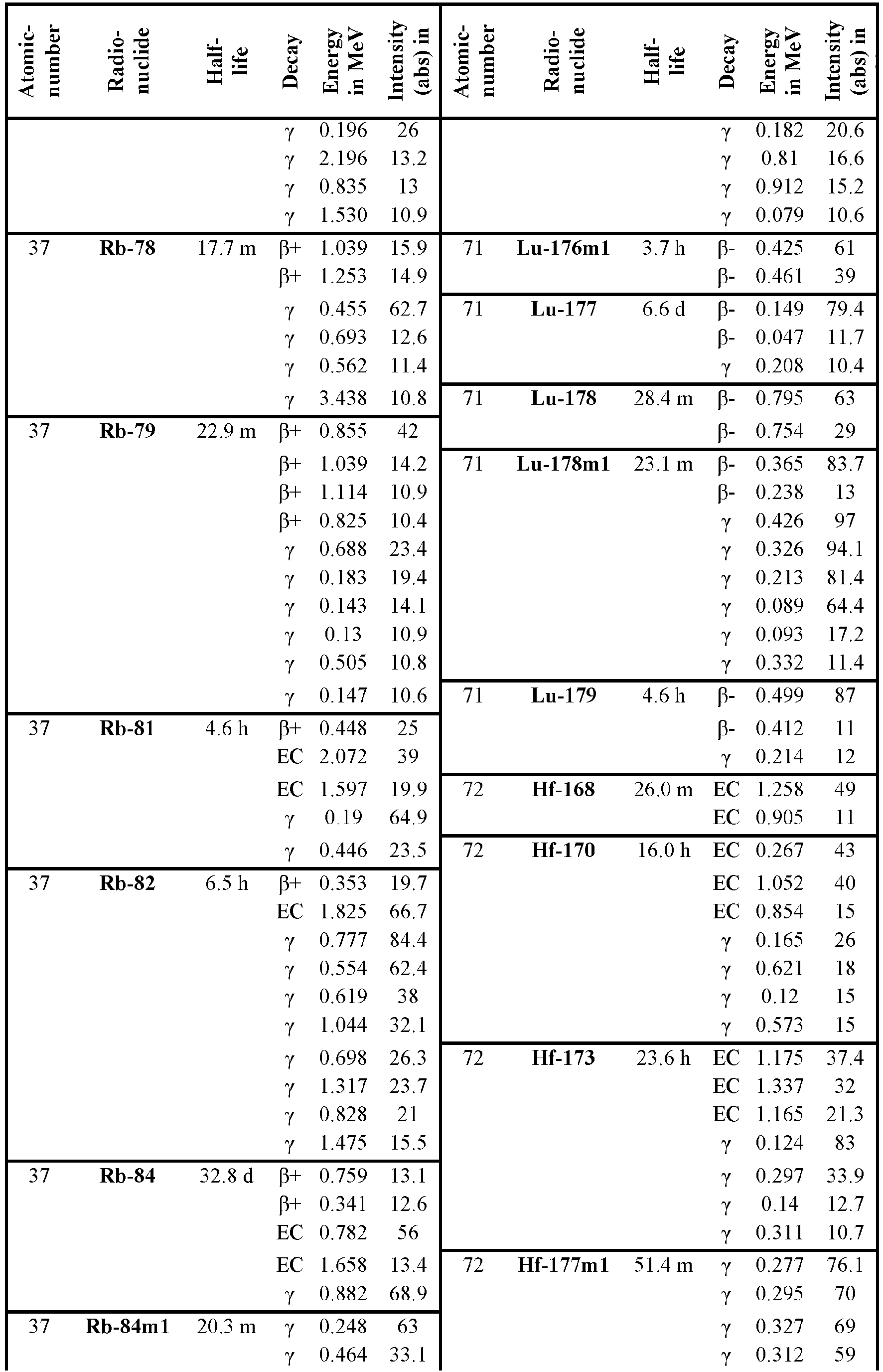

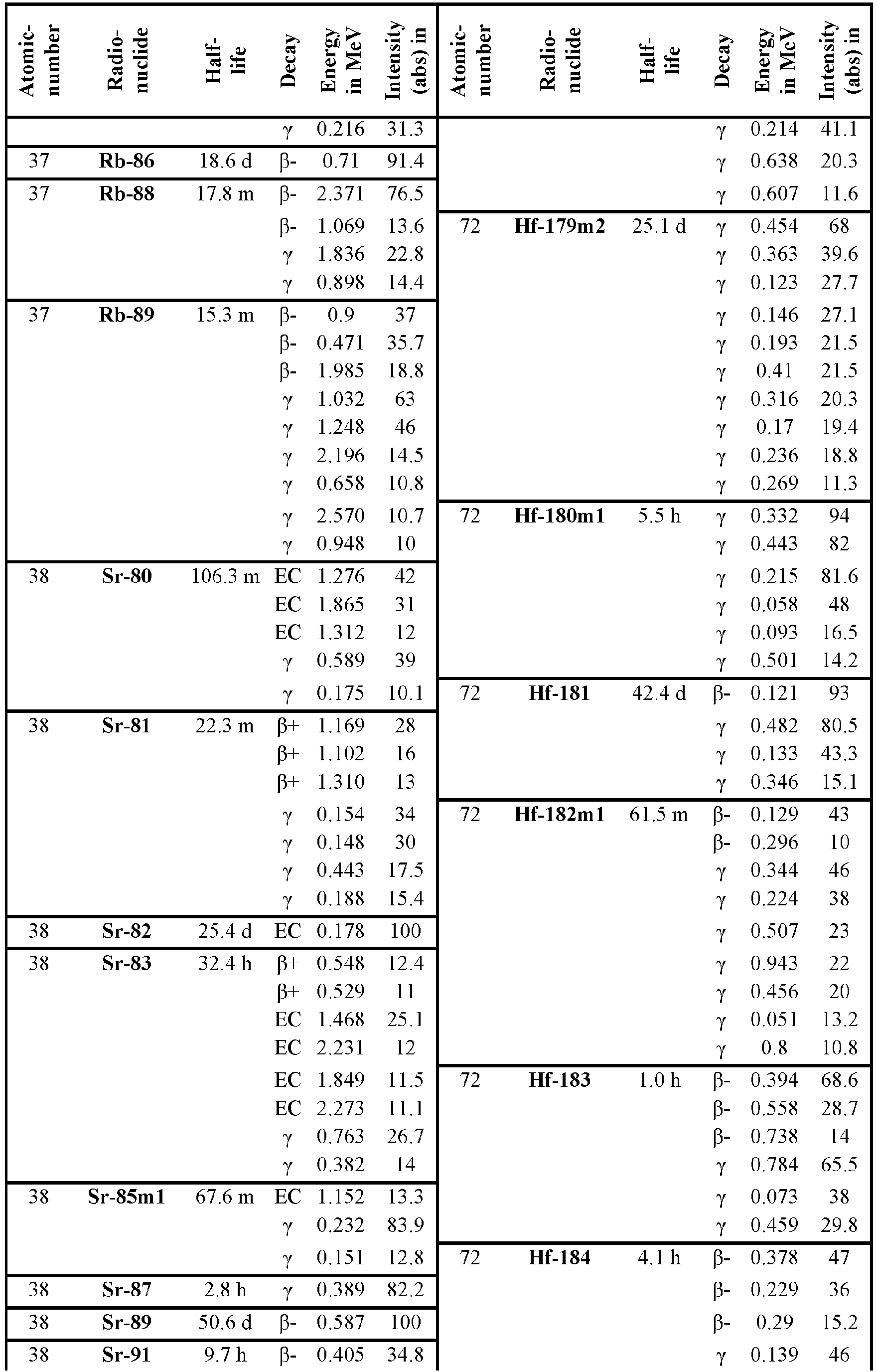

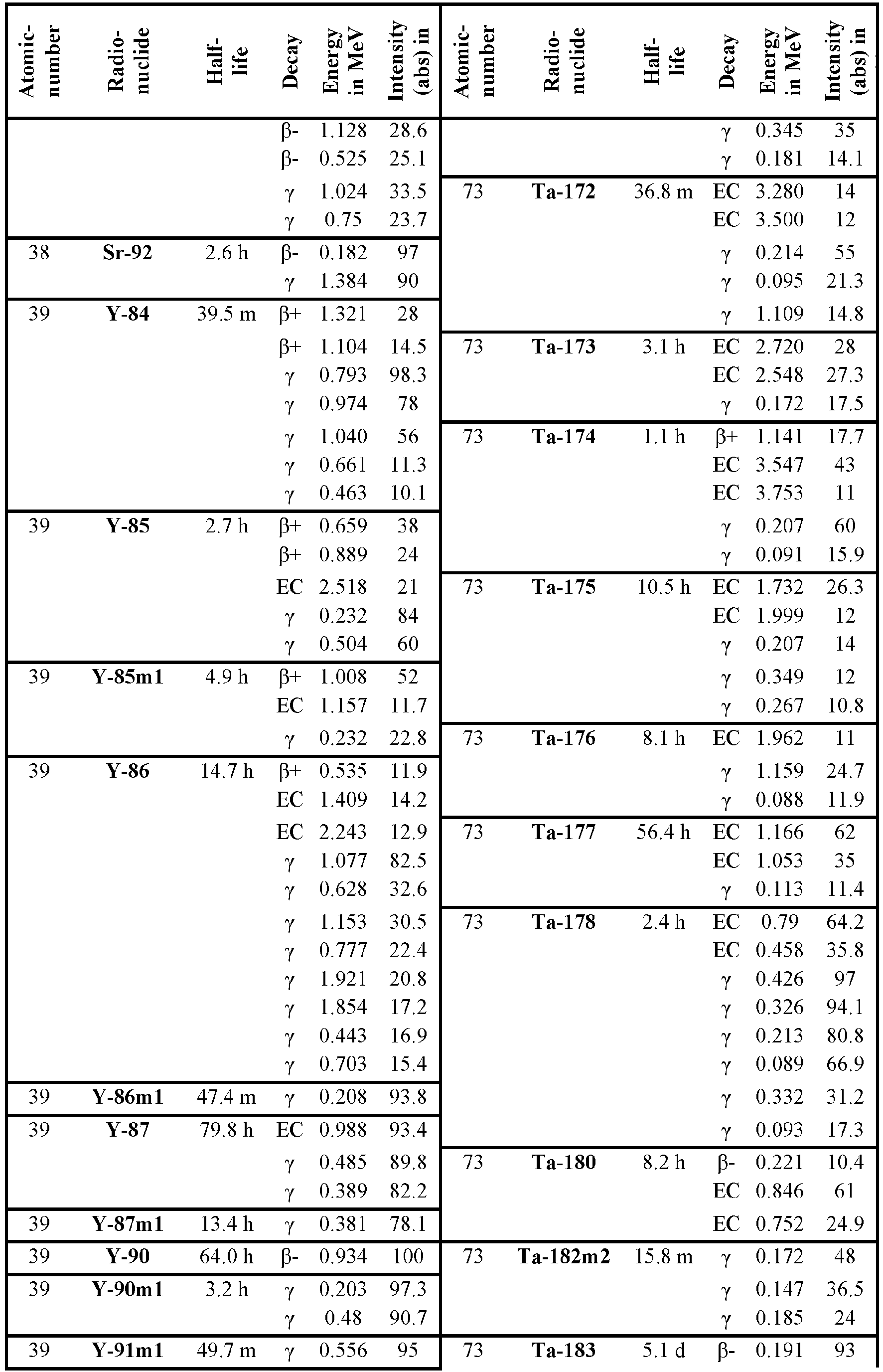

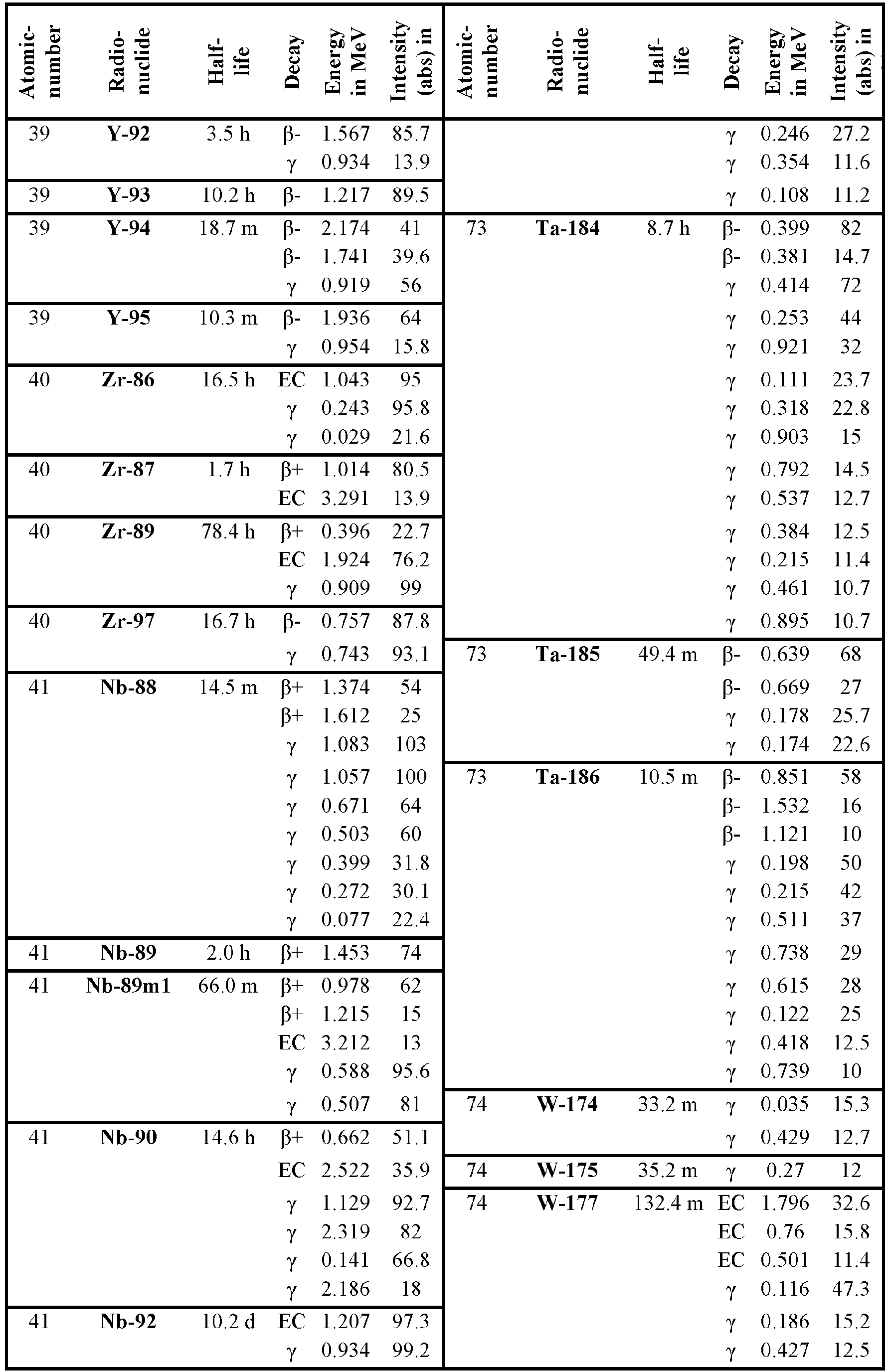

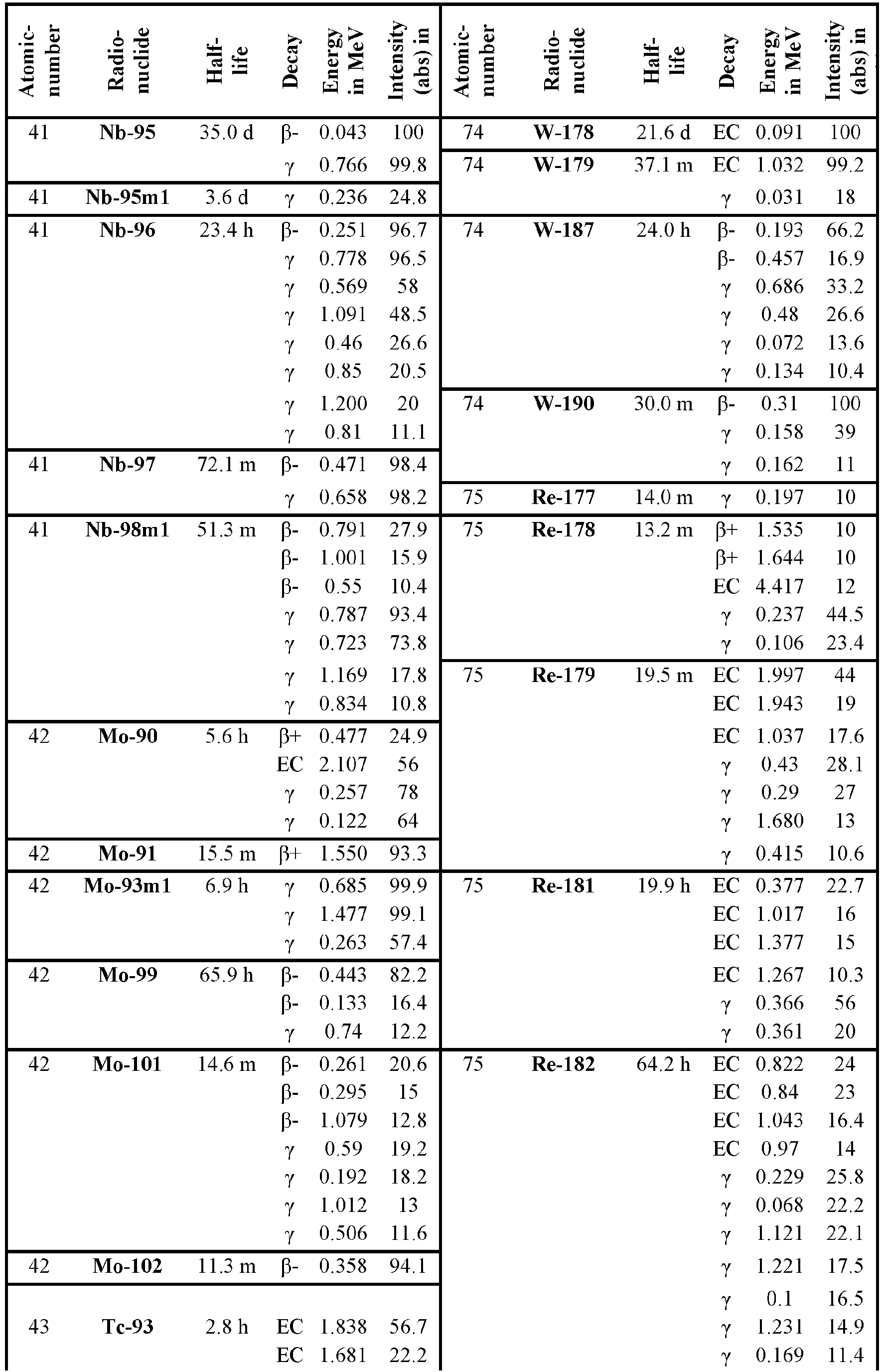

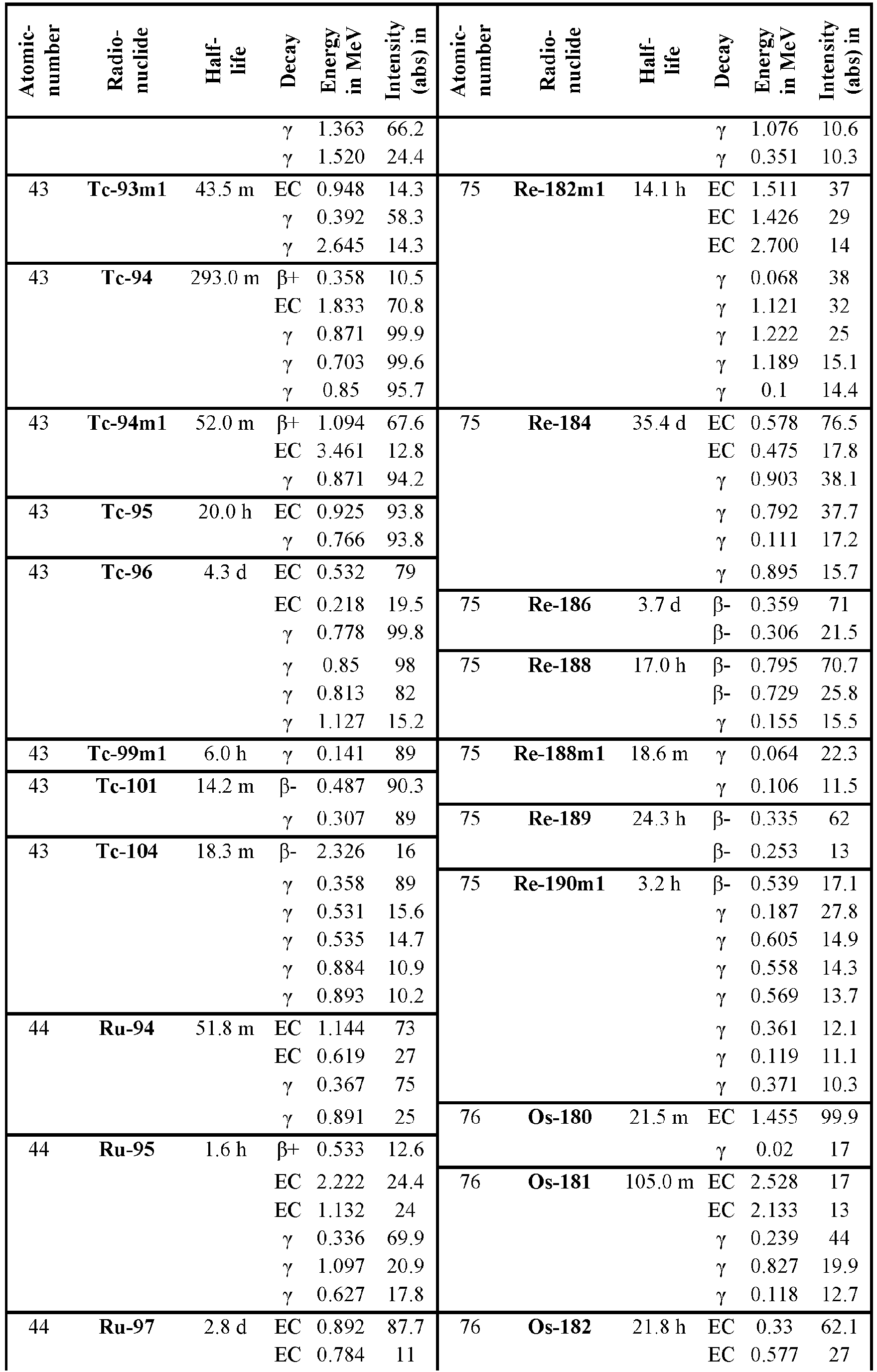

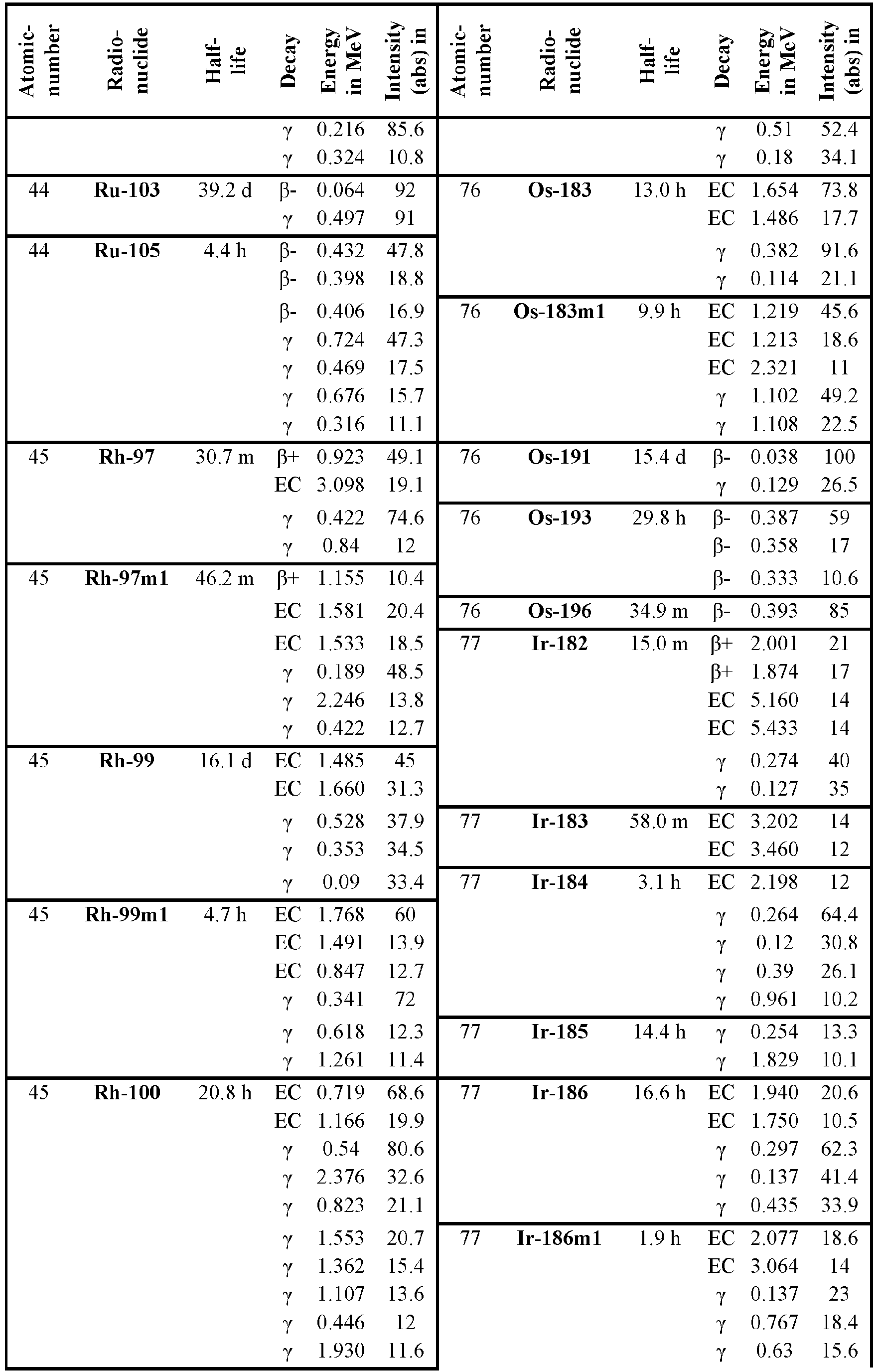

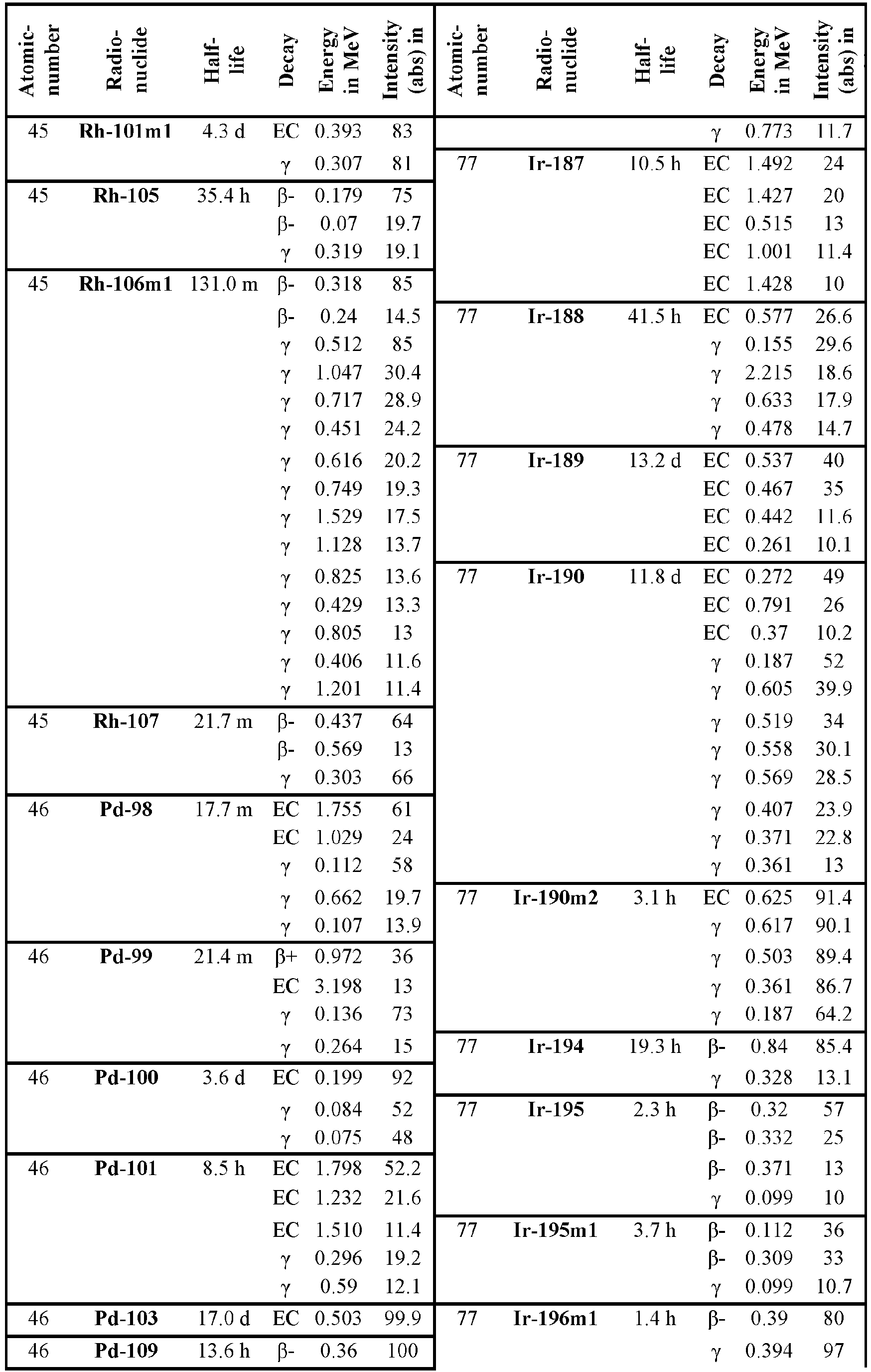

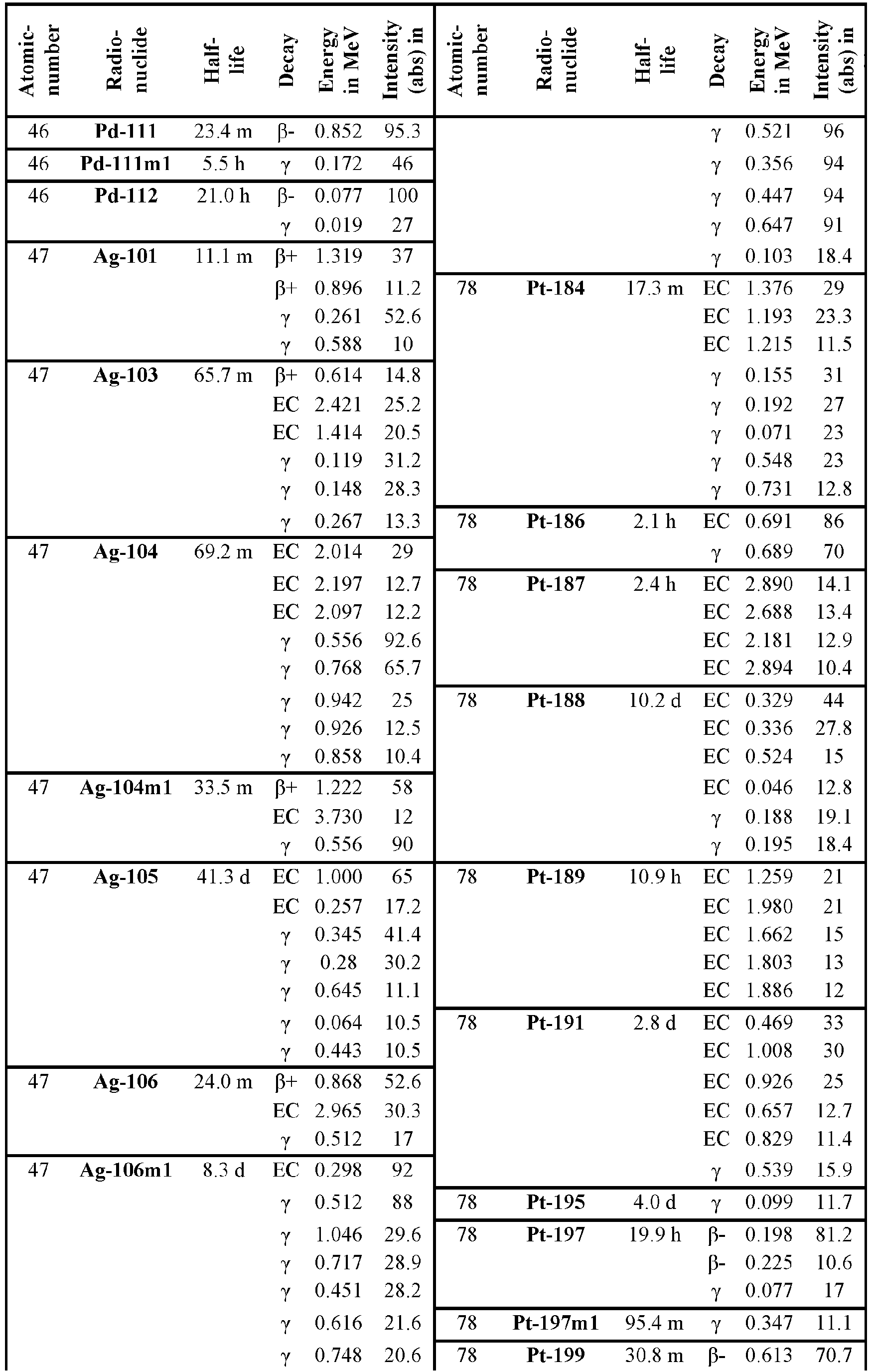

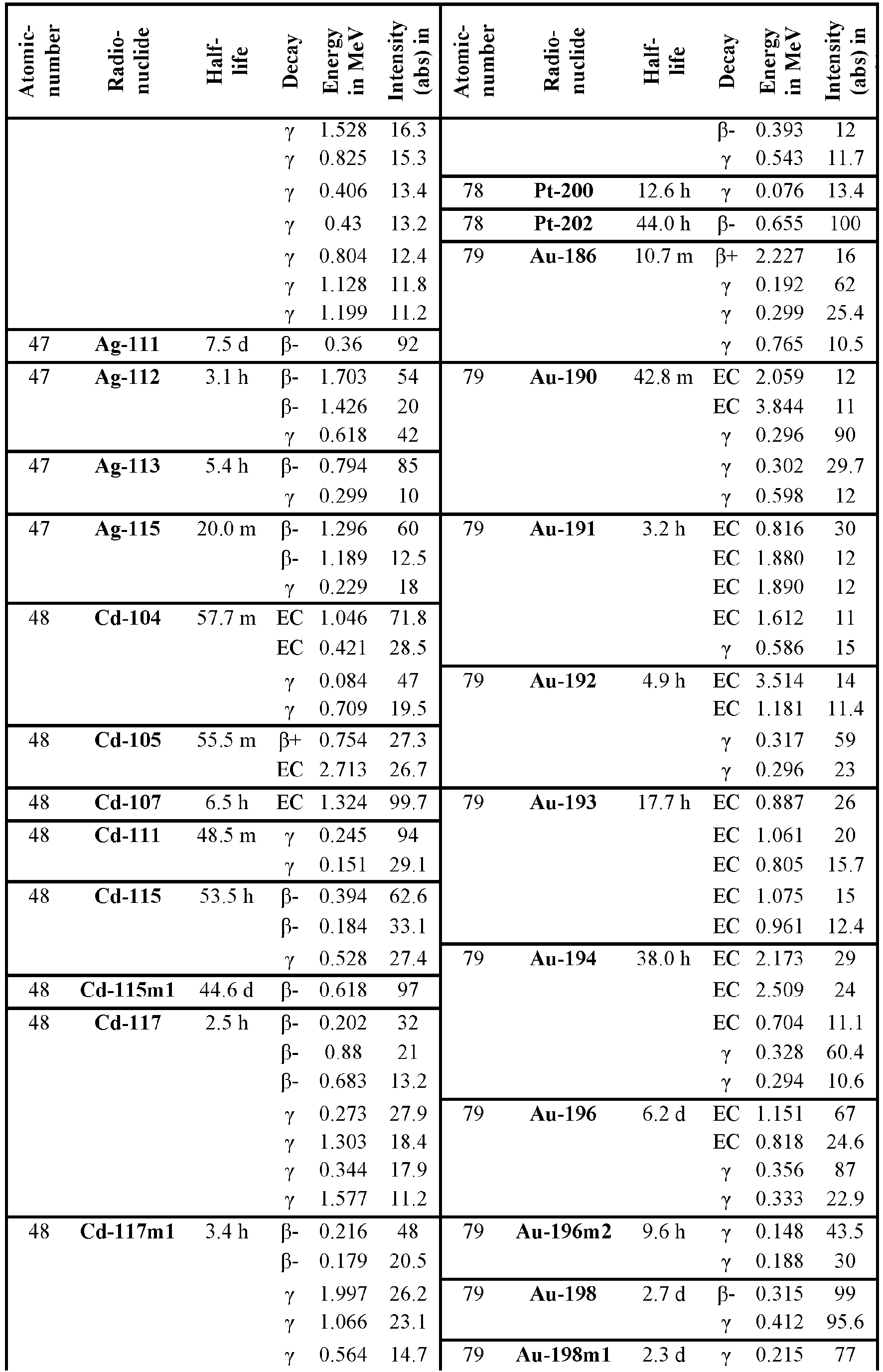

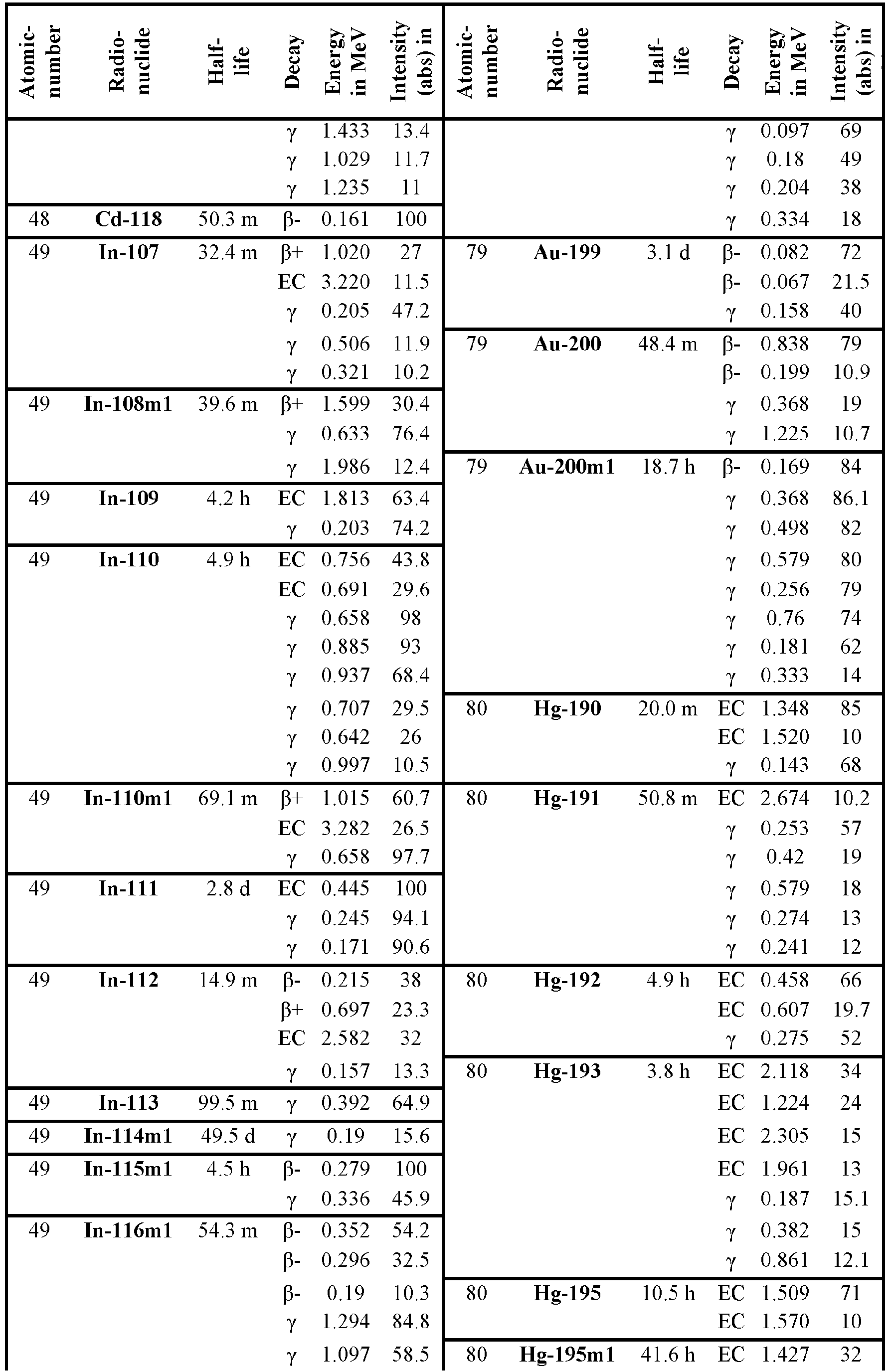

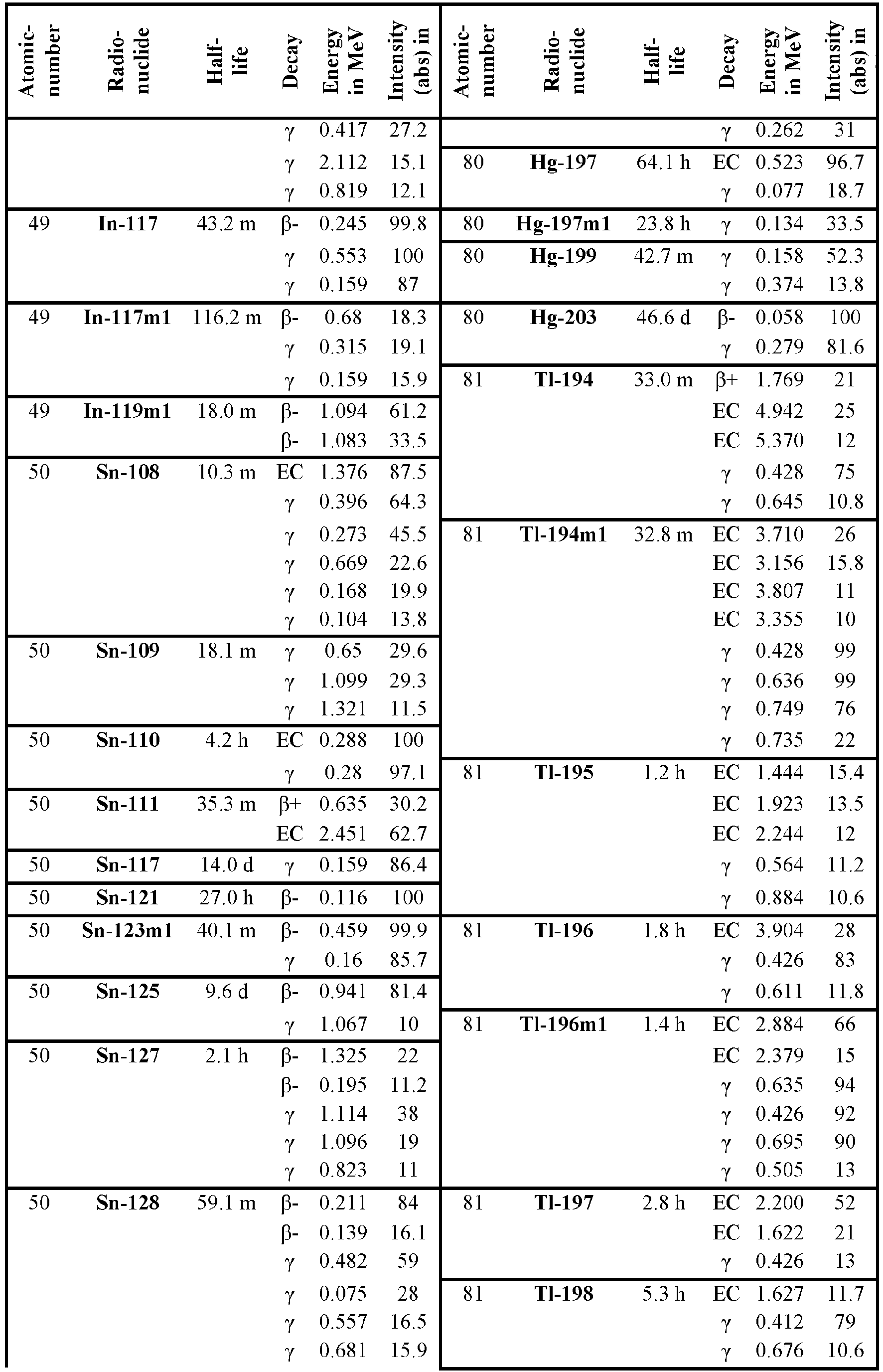

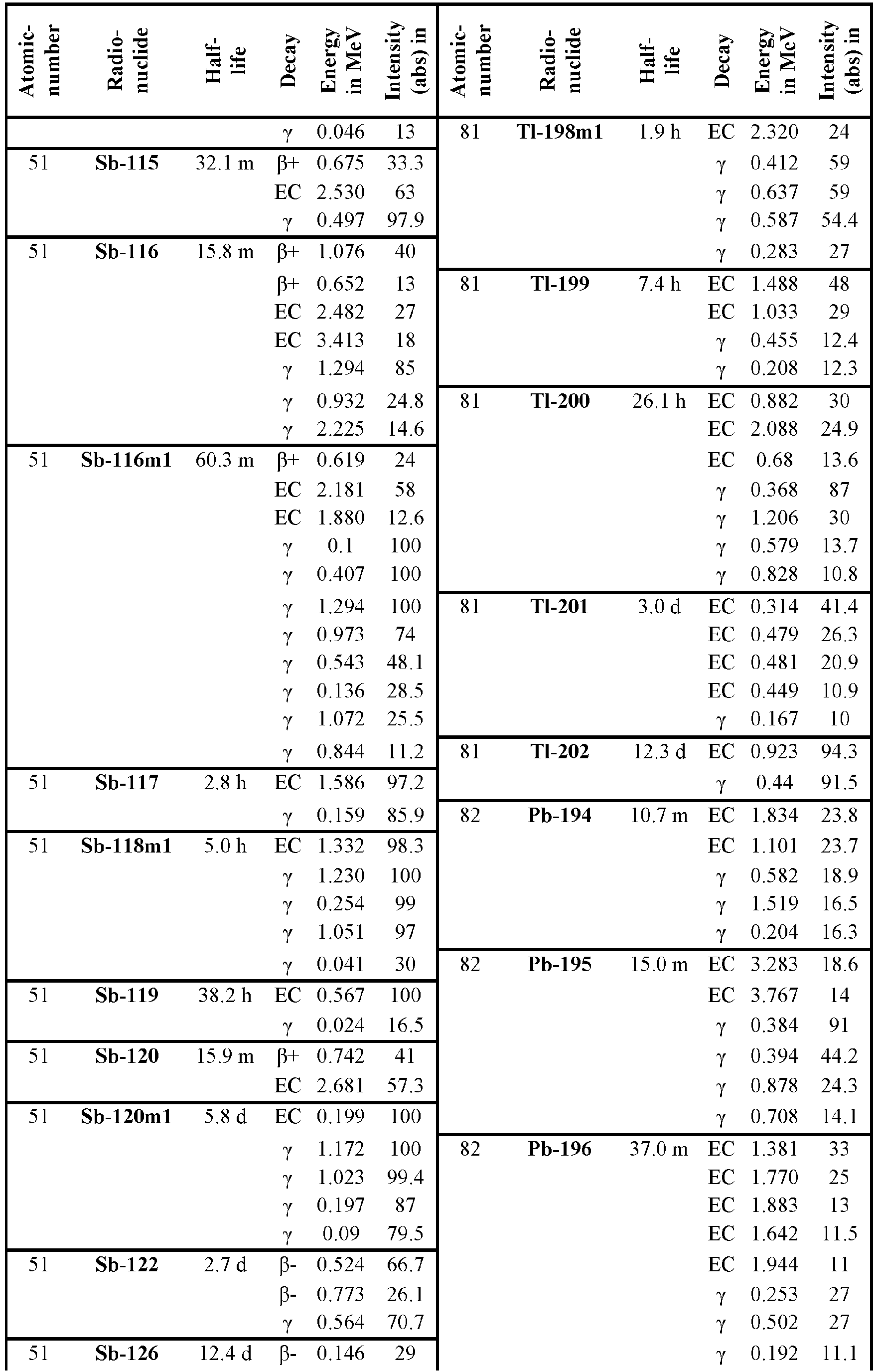

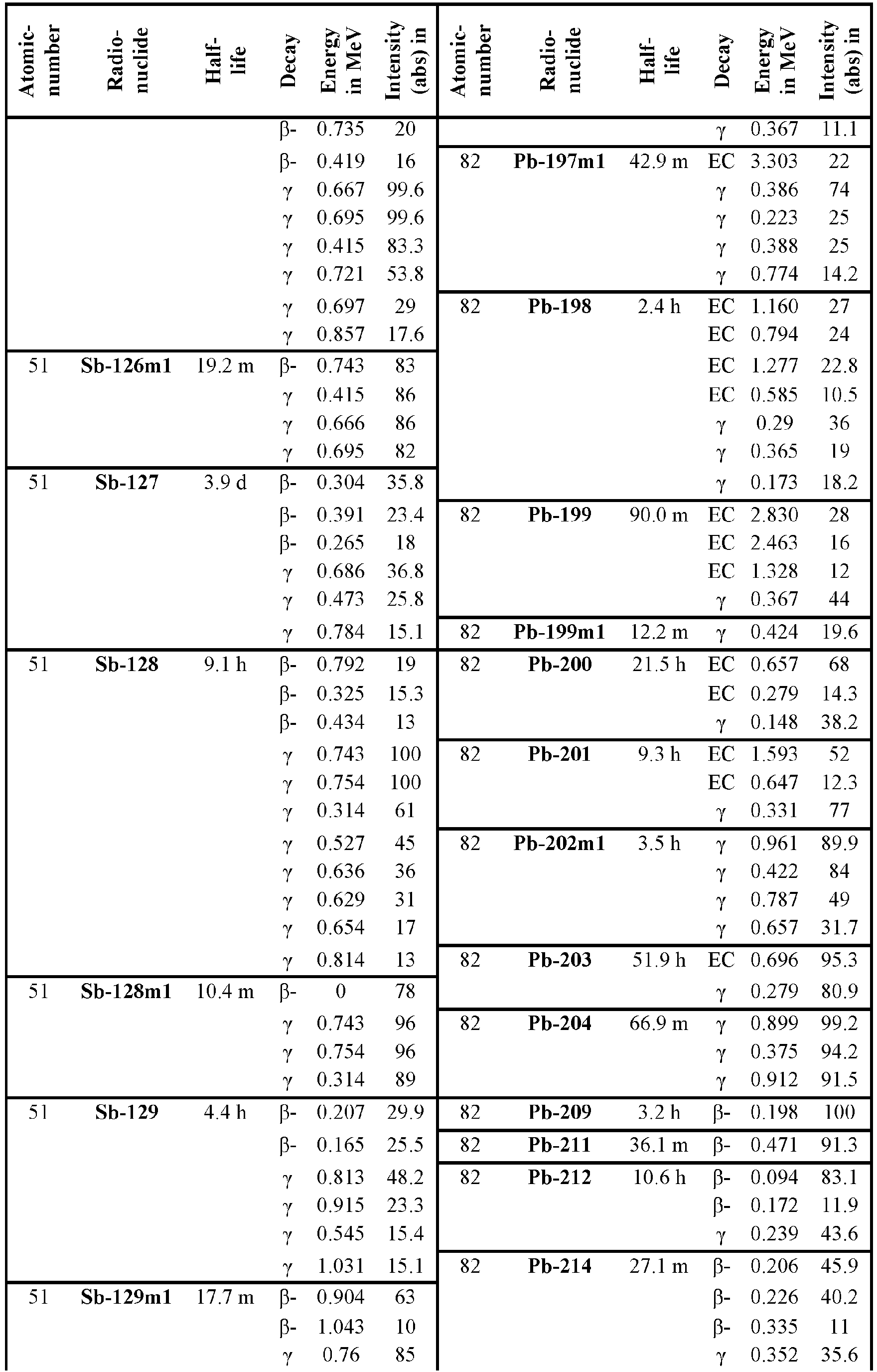

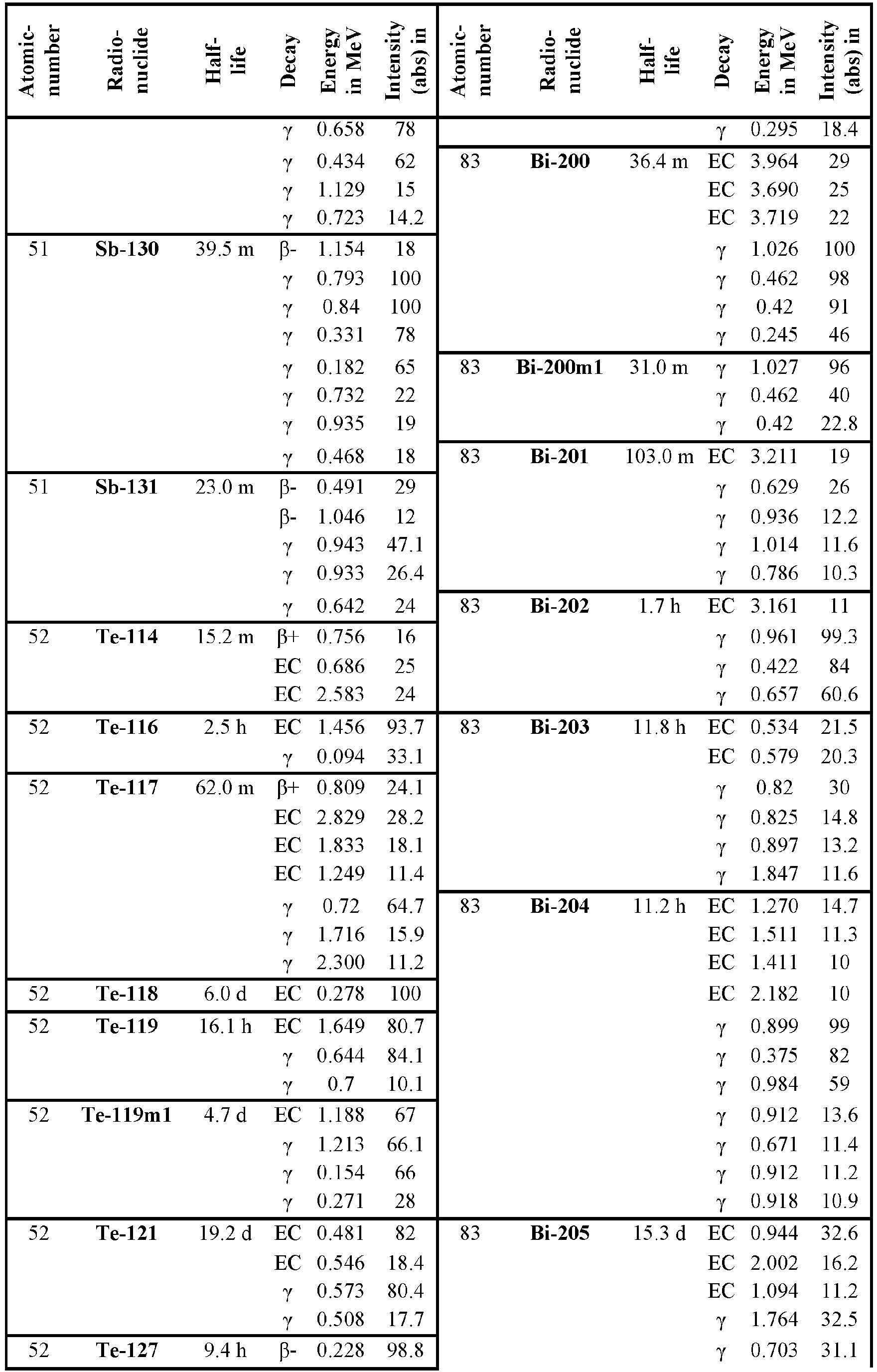

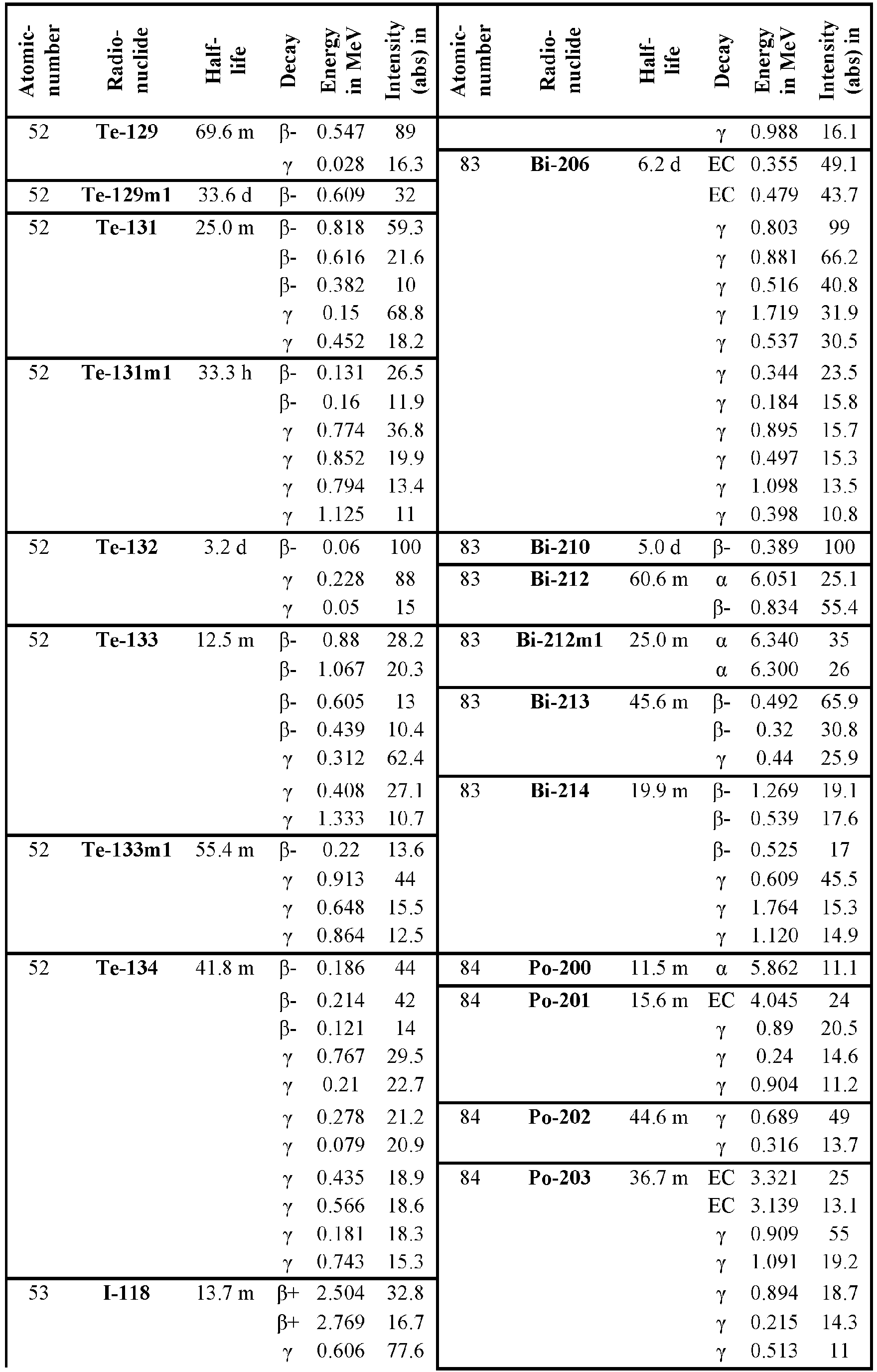

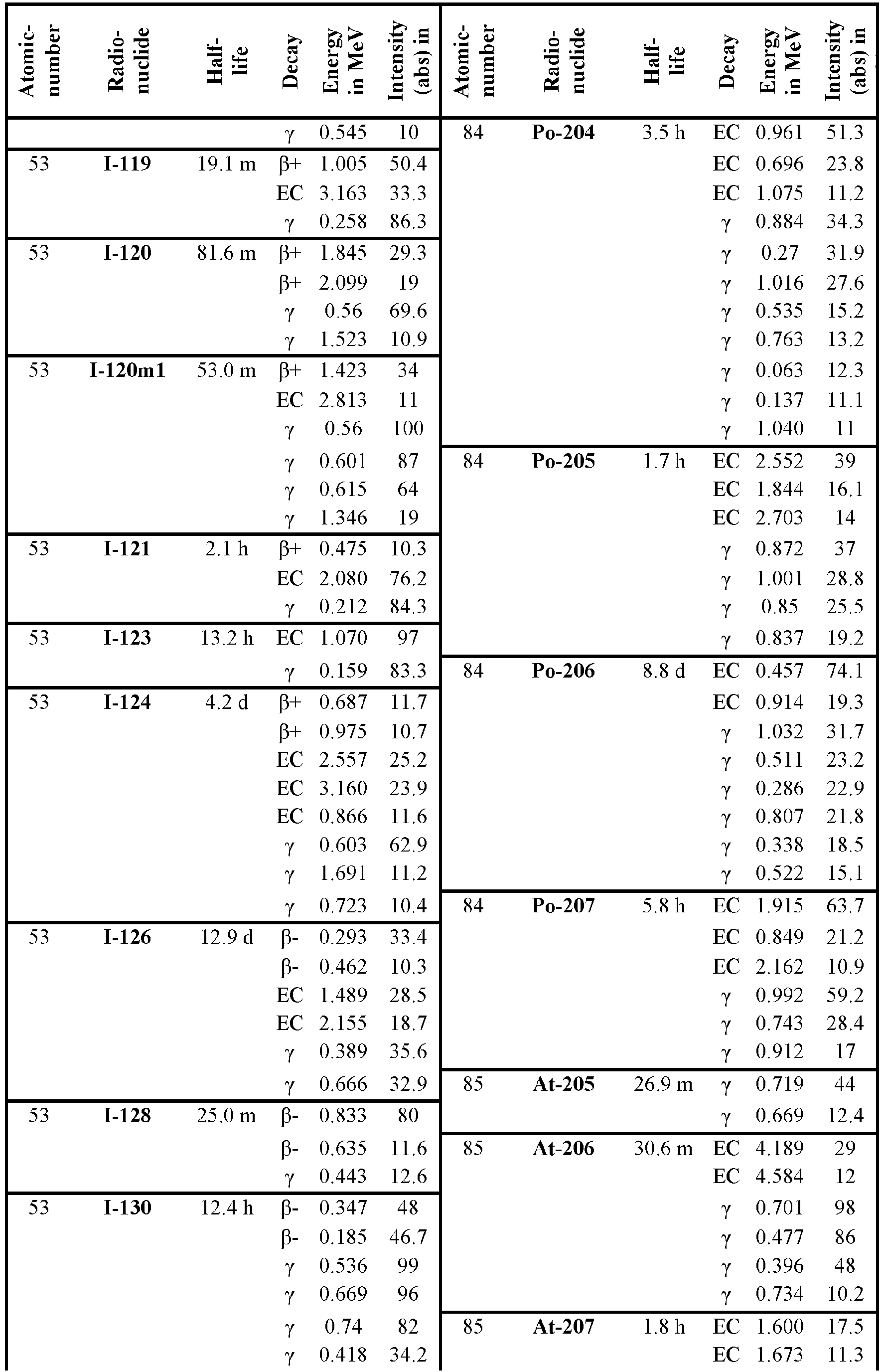

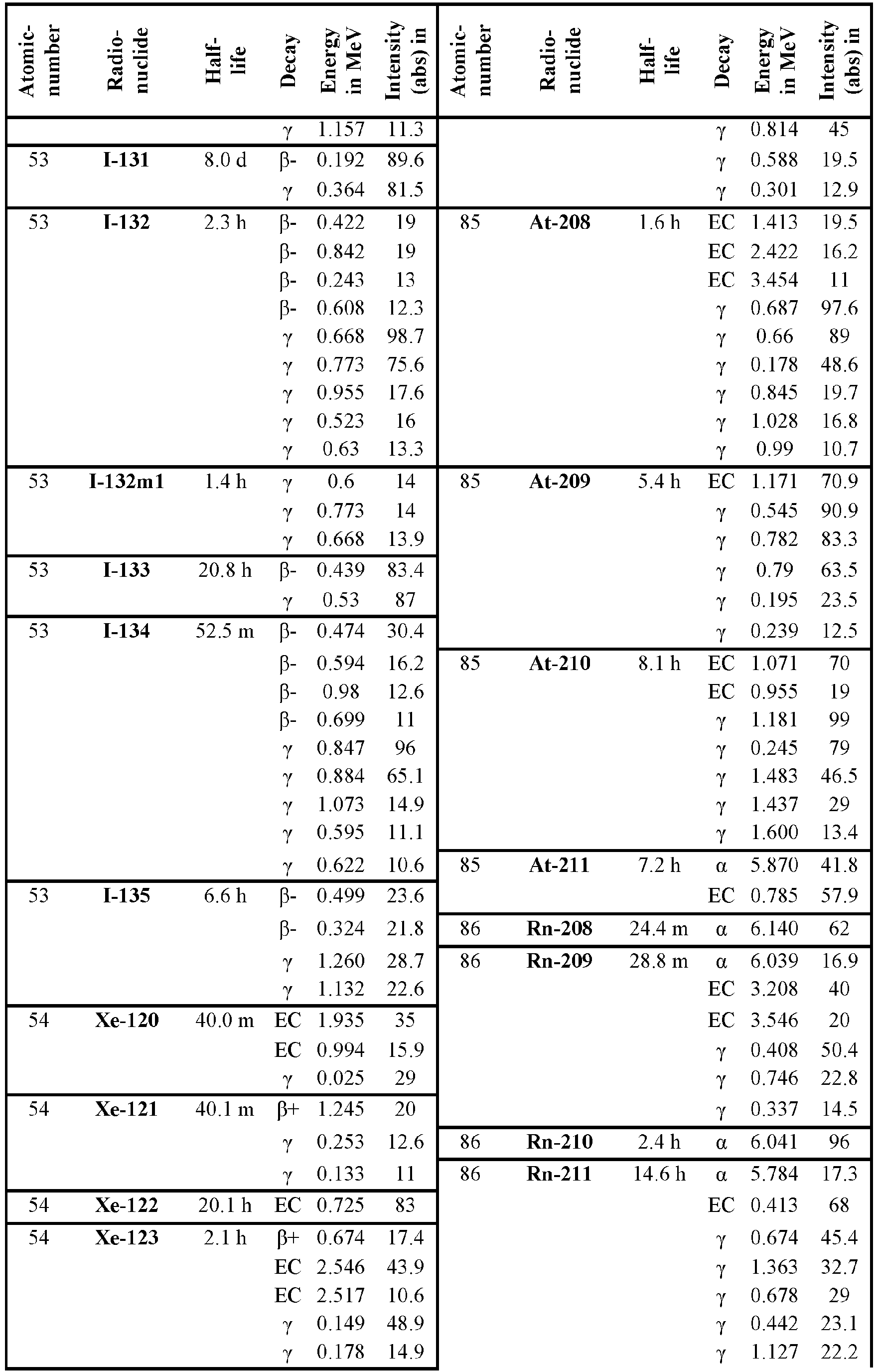

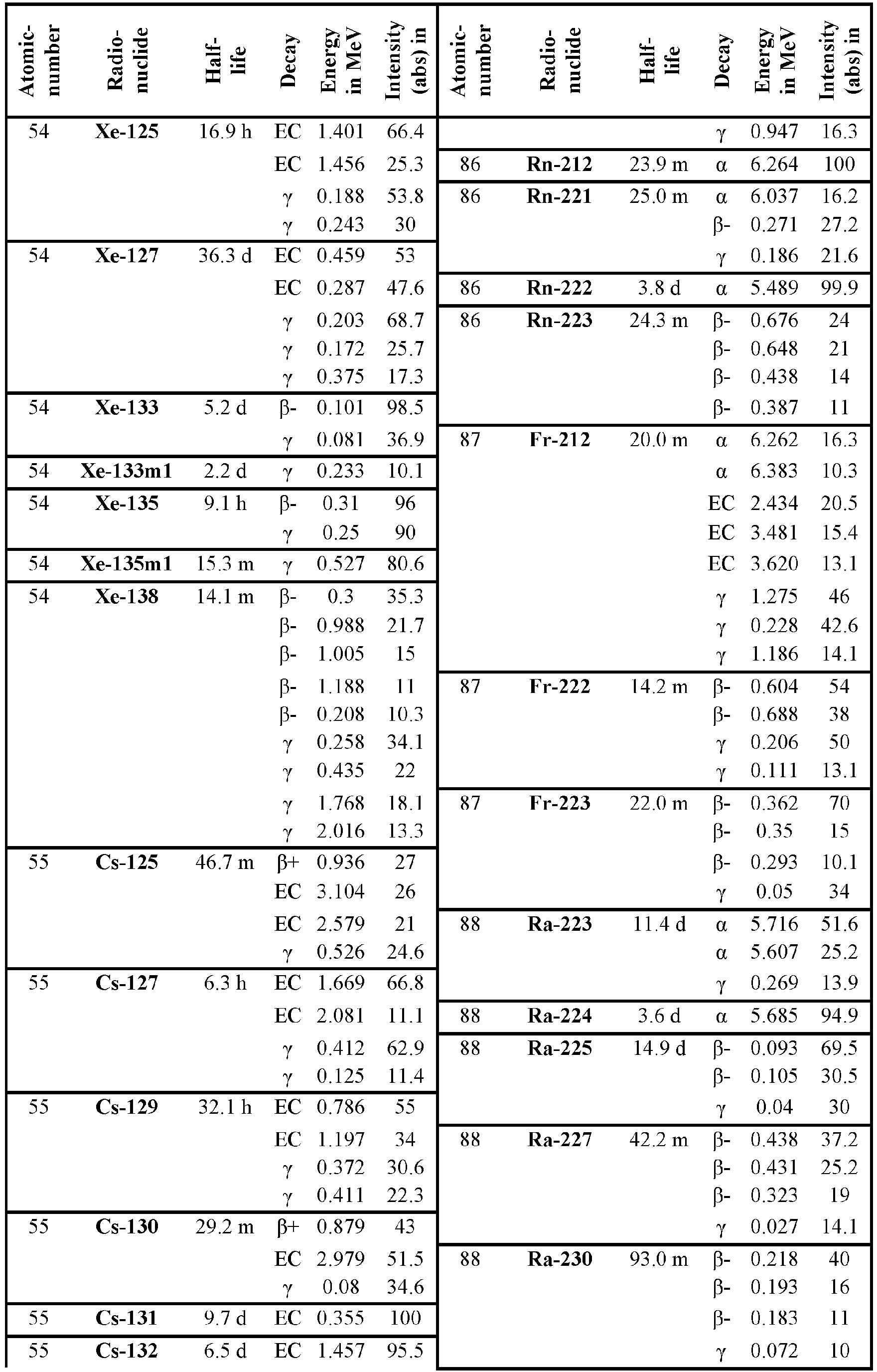

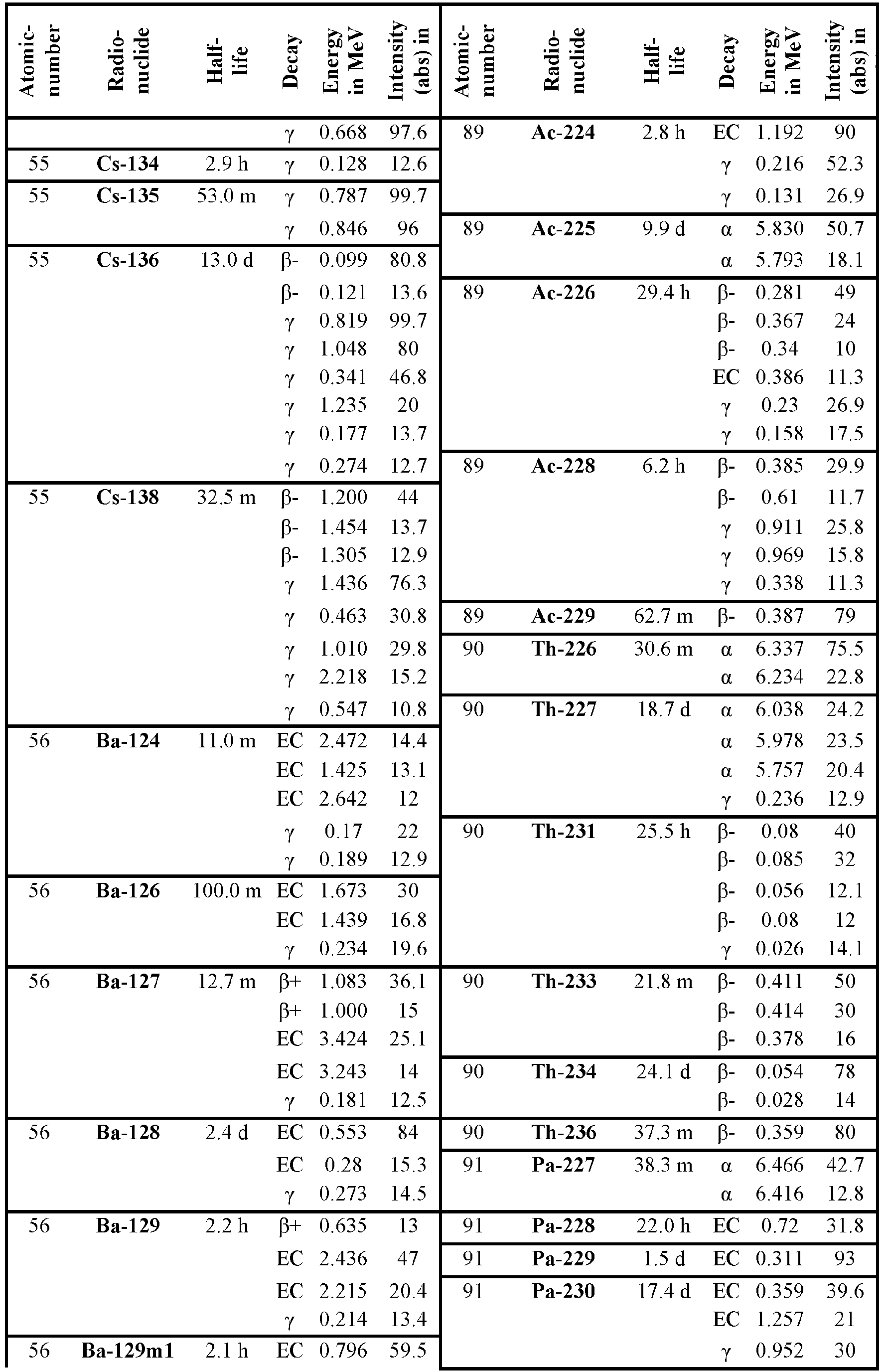

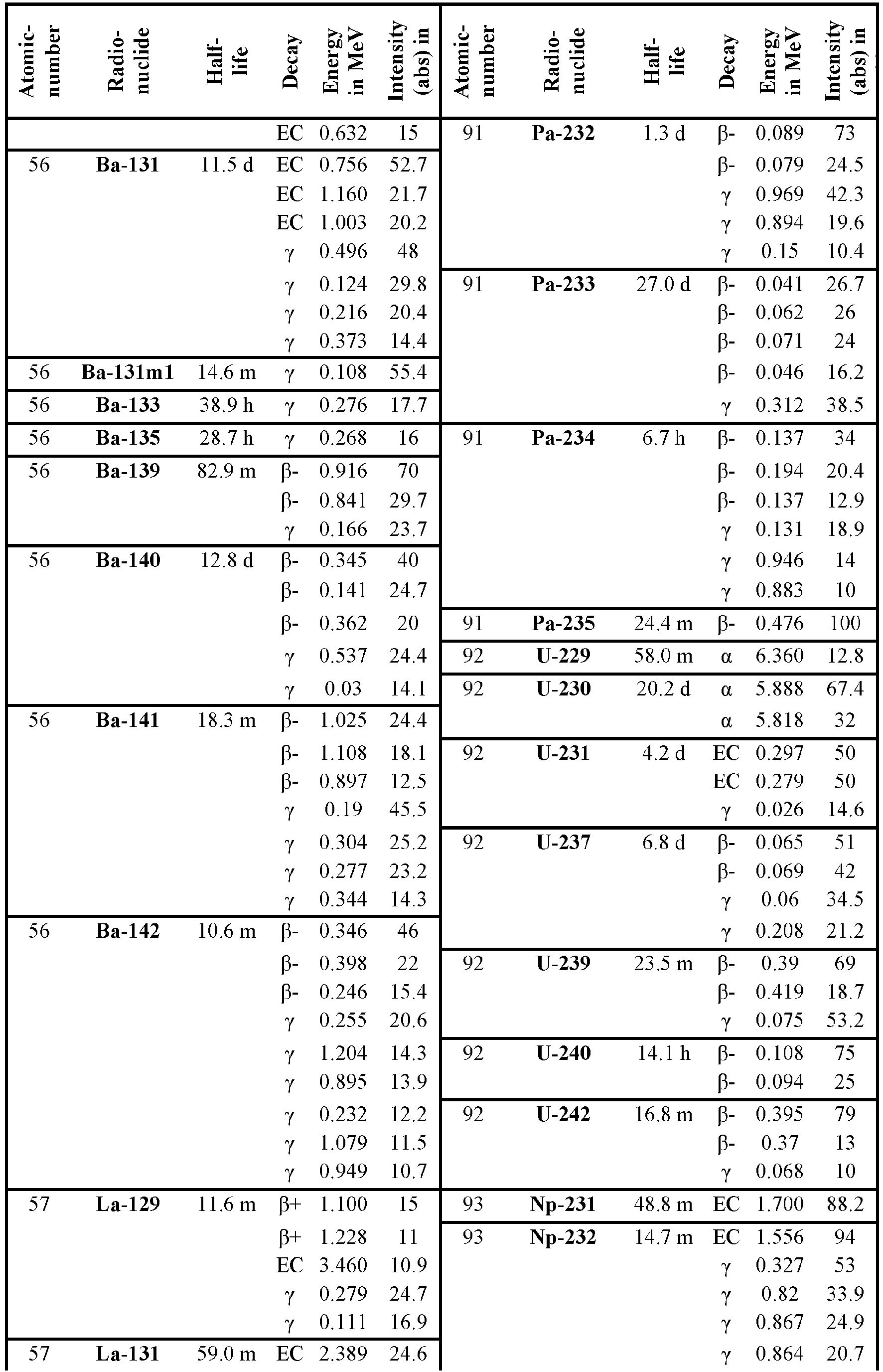

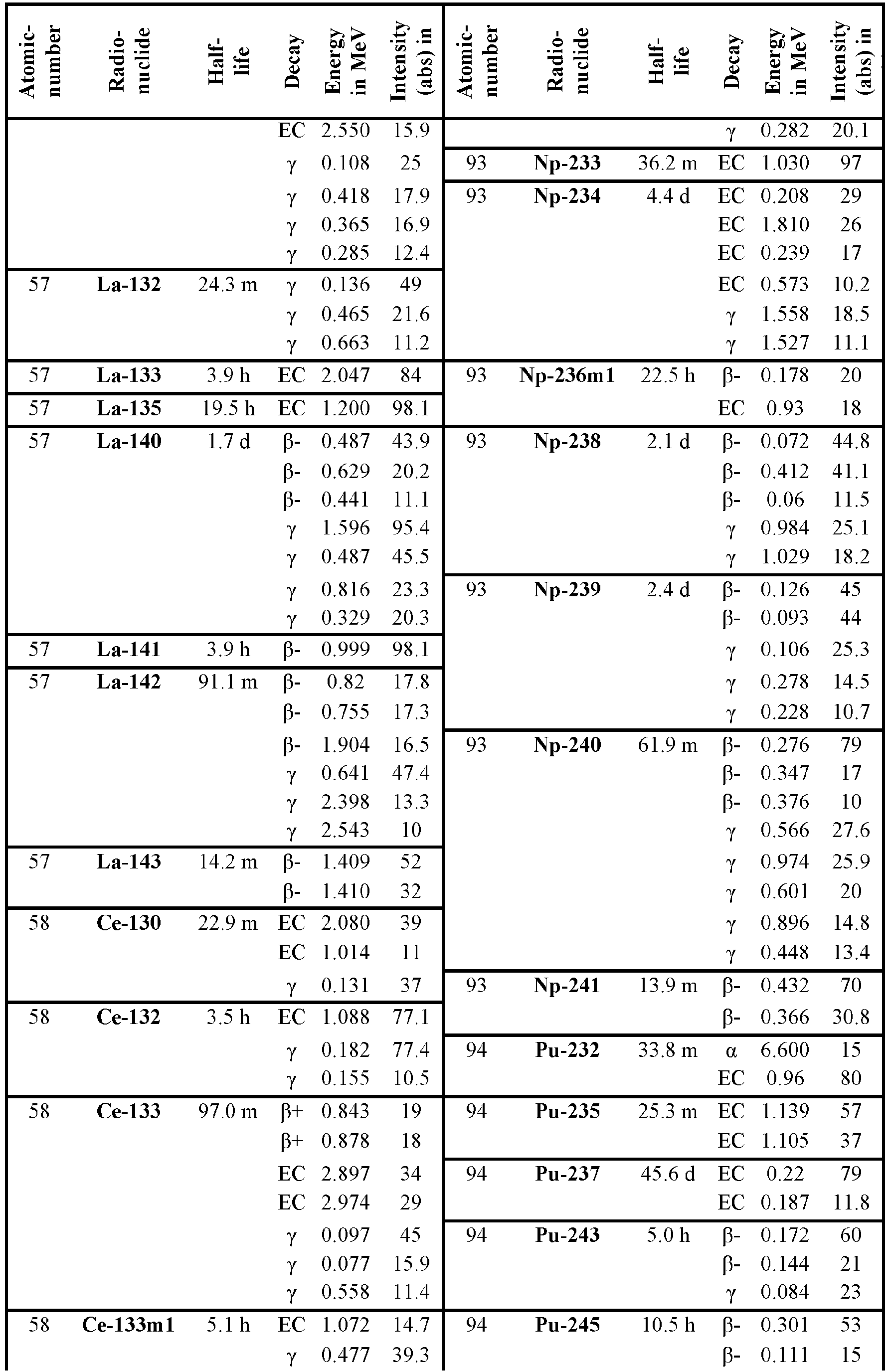

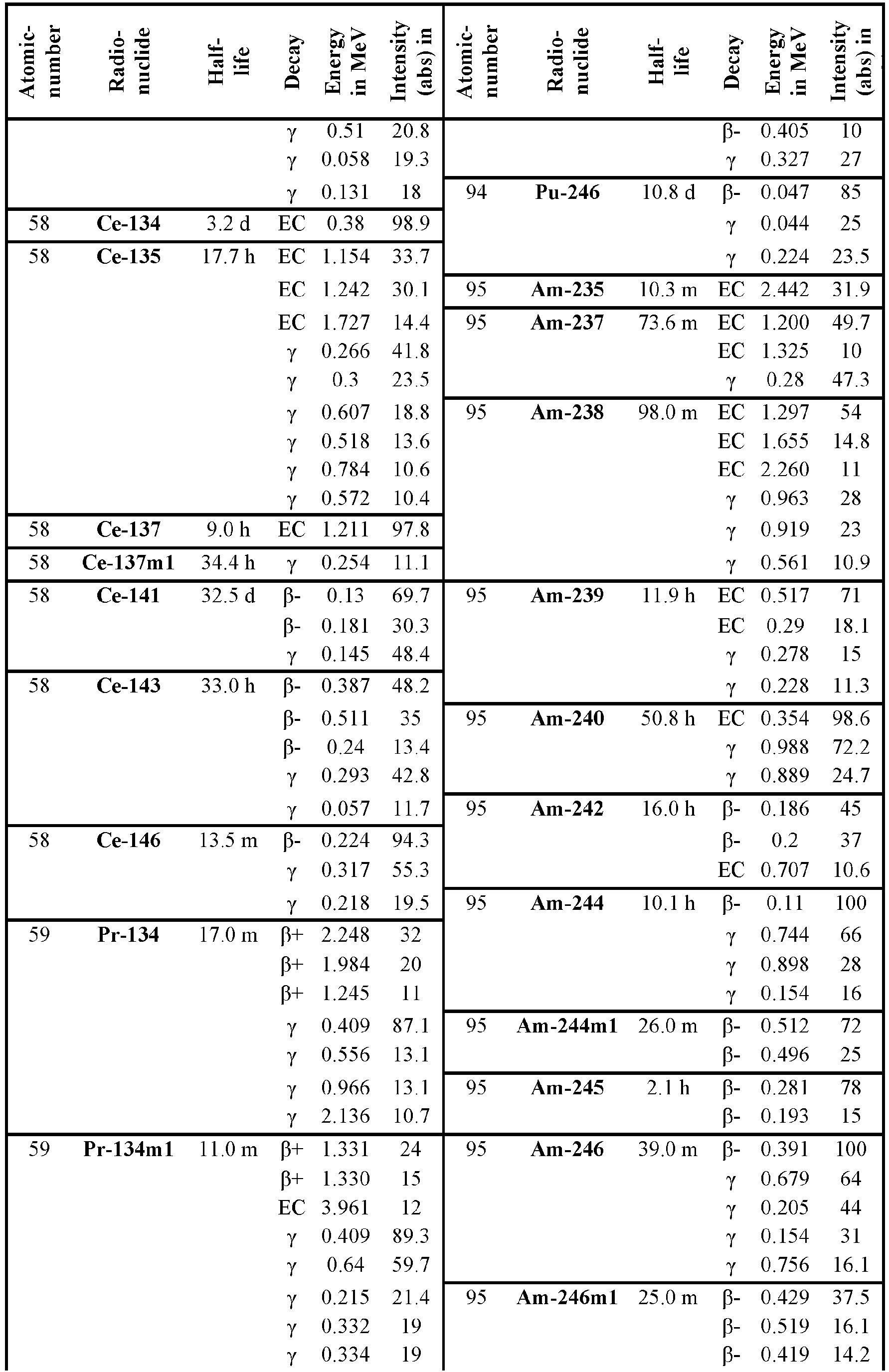

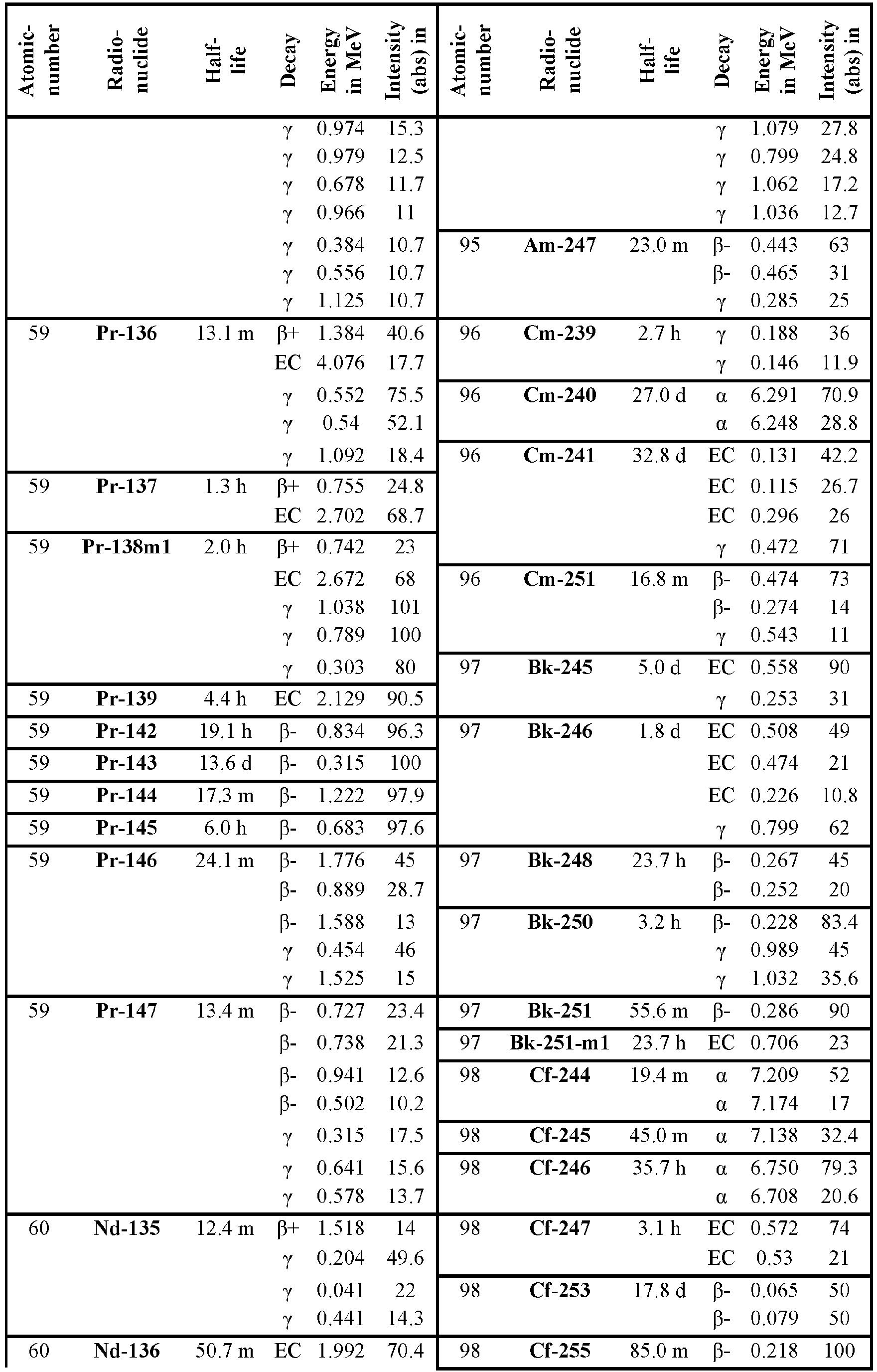

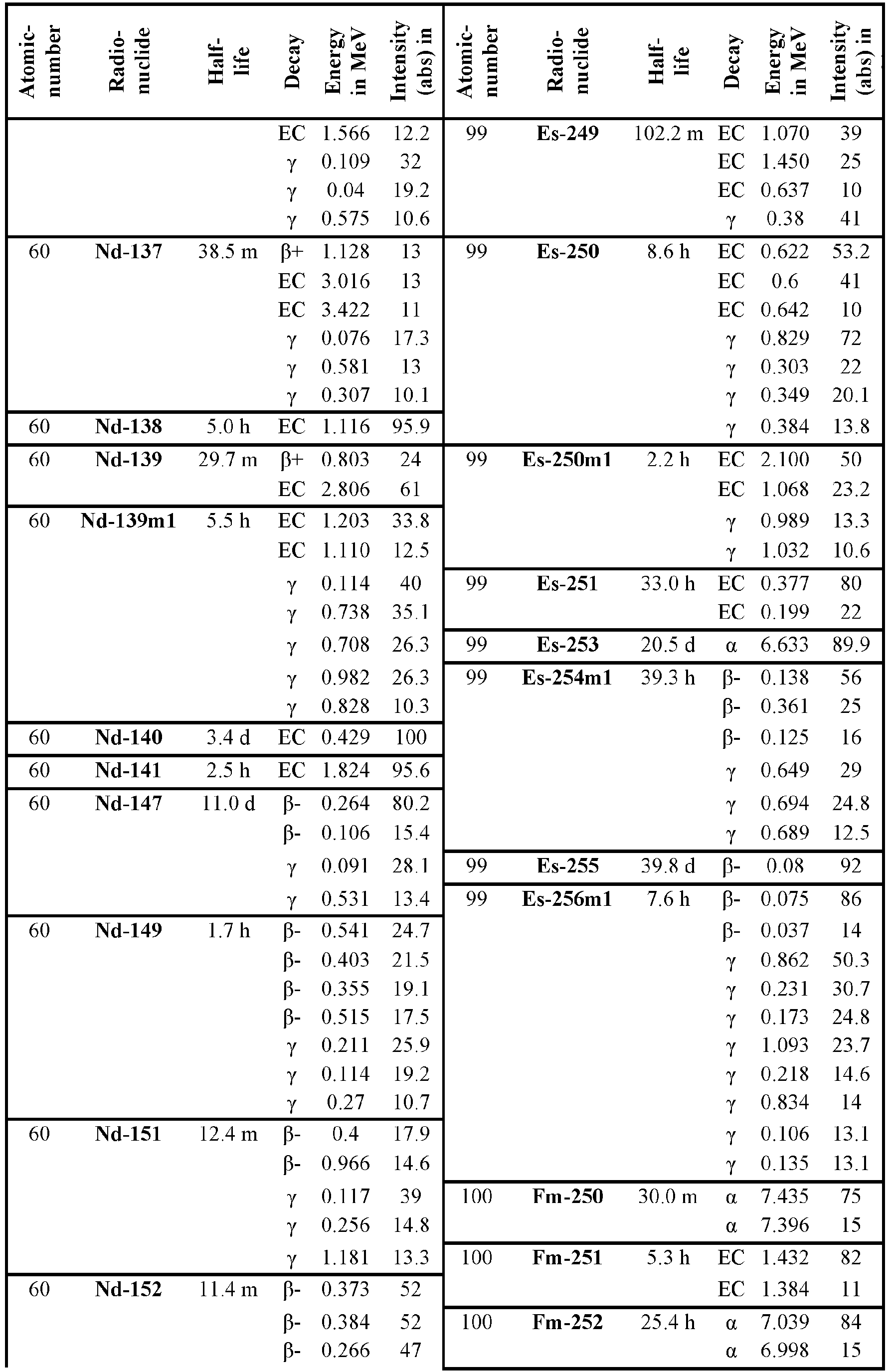

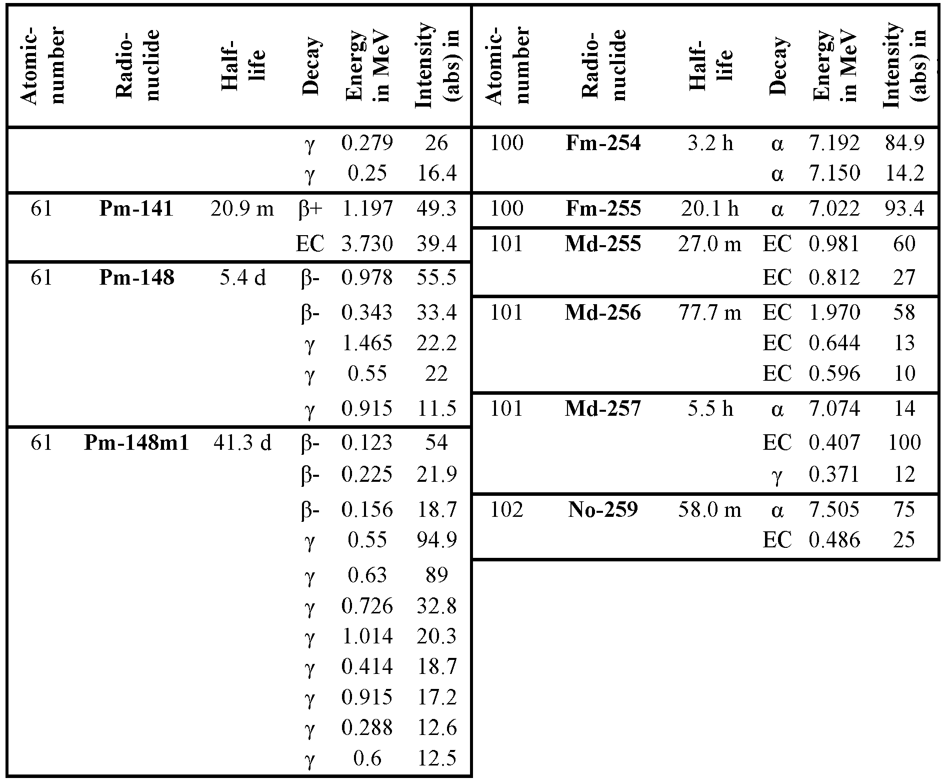

In an embodiment of the present invention, the radionuclide is used for diagnosis (in this context, the radionuclide may also be referred to as "diagnostically active radionuclide"). Preferably, the radionuclide is selected from the group comprising ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F for example as Al-¹⁸F, ⁷⁶ Br, ⁷⁷Br, ¹²³I, ¹²⁴I, and ¹²⁵I. More preferably, the radionuclide is selected from the group comprising Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb. Even more preferably, the radionuclide is ⁶⁴Cu, ⁶⁸Ga, ¹¹¹In and ²⁰³Pb. It will, however, also be acknowledged by a person skilled in the art that the use of said radionuclide is not limited to diagnostic purposes, but also encompasses their use in therapy and theragnostics when conjugated to the compound of the invention.

In an embodiment of the present invention, the radionuclide is used for therapy (in this context, the radionuclide may also be referred to as "therapeutically active radionuclide"). Preferably, the radionuclide is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹²⁵I ¹³¹I, and ²¹¹At. More preferably, the radioactive isotope is selected from the group comprising ⁶⁷Cu, ⁹⁰Y, ¹⁴⁹Tb, ¹⁶¹Tb, ¹⁷⁷Lu, ²¹²Pb, ²¹³Bi, ²²⁵Ac, ²²⁷Th. Even more preferably, the radionuclide is selected from the group comprising ⁹⁰Y, ¹⁷⁷Lu, ²¹²Pb and ²²⁵Ac. It will, however, also be acknowledged by a person skilled in the art that the use of said radionuclide is not limited to therapeutic purposes, but also encompasses their use in diagnostic and theragnostics when conjugated to the compound of the invention.

In a preferred embodiment, the radionuclide is selected from:
(i) a diagnostically active radionuclide selected from the group consisting of Al-¹⁸F, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I, preferably from the group consisting of Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb, and more preferably from the group consisting of ⁶⁴Cu, ⁶⁸Ga, ¹¹¹In and ²⁰³Pb; and/or
(ii) a therapeutically active radionuclide selected from the group consisting of ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹²⁵I, ¹³¹I, and ²¹¹At, preferably from the group consisting of ⁶⁷Cu, ⁹⁰Y, ¹⁴⁹Tb, ¹⁶¹Tb, ¹⁷⁷Lu, ²¹²Pb, ²¹³Bi, ²²⁵Ac, ²²⁷Th, and more preferably from the group consisting of ⁹⁰Y, ¹⁷⁷Lu, ²¹²Pb and ²²⁵Ac.

In an embodiment, the compound of the invention comprises a chelator. The chelator is part of the compound of the invention, whereby the chelator is either directly or indirectly, such as by a linker, attached to the rest of the compound of the invention. A preferred chelator is a chelator which forms metal chelates and preferably comprises at least one radionuclide. The at least one radionuclide is useful in or suitable for diagnostic and/or therapeutic and/or theranostic purposes, and is preferably useful in or suitable for imaging and/or radiotherapy.

Chelators in principle useful in and/or suitable for the present invention including diagnosis and/or therapy of a disease are known to the person skilled in the art. A variety of respective chelators is available and has been reviewed, e.g., by Banerjee et al. (Banerjee, Schaffer et al. 2005), and references therein (Wadas, Wong et al. 2010, Price and Orvig 2014). Such chelators include, but are not limited to linear, cyclic, macrocyclic, tetrapyridine, N3S, N2S2 and N4 chelators as disclosed in US 5,367,080 A, US 5,364,613 A, US, 5,021,556 A, US 5,075,099 A and US 5,886,142 A.

Representative chelators and their derivatives including any bifunctional versions that can be attached to the compound of the invention include, but are not limited to, the examples listed in Table 7.

**Table 7: Examples of chelators with their corresponding chemical names and exemplary reference of their disclosure.**

| **Chelator** | **Chemical name** | **Ref.** |
|---|---|---|
| AAZTA | 1,4-bis(carboxymethyl)-6-[bis(carboxymethyl)]amino-6-methylperhydro-1,4-diazepine | a |
| ATMP | (nitrilotris(methylene))tris(phosphonic acid) | aa |
| ATSM | Diacetyl bis(N⁴-methylthiosemicarbazone) | b |
| Aza crown ether | 1,10-dioxa-4,7,13,16-tetraazacyclooctadecane | dd |
| AZEP-DTPA | cis-(2,7-bis-(bis-carboxymethyl-amino)-methyl)-azepan-1-yl)-acetic | c |
| BATA | 2,3,4,5,6,7,8,9,10,11,12,13,14,15-tetradecahydro-1,16,4,7,10,13-benzodioxatetraaza-cyclooctadecine-4,7,10,13-yl-tetraacetic acid | c |
| BATPA | 1,2-bis(2-aminophenoxy)ethane-N,N,N',N'-tetraacetic acid | s |
| BPCA | 2,2',2",2‴-(([2,2'-bipyridine]-6,6'-diylbis(methylene))bis(azanetriyl))tetraacetic acid | a |
| Bp44mT | 2-benzoylpyridine 4,4-dimethyl-3-thiosemicarbazone | rr |
| C3B-DO2A | 2,2'-(1,4,7,10-tetraazabicyclo[5.5.3]pentadecane-4,10-diyl)diacetic acid | j |
| CB-DO2A | 4,10-bis(carboxymethyl)-1,4,7,10-tetraazabicyclo [5.5.2]tetradecane | a |
| CB-TE1A1P | 2-(11-(phosphonomethyl)-1,4,8,11-tetraazabicyclo[6.6.2]hexadecan-4-yl)acetic acid | a |
| CB-TE2A | 4,11-bis-(carboxymethyl)-1,4,8,11-tetraazabicyclo[6.62]-hexadecane | a |
| CB-TE2P | ((1,4,8,11-tetraazabicyclo[6.6.2]hexadecane-4,11-diyl)bis(methylene))bis(phosphonic acid) | a |
| CB-TEAMA | 2-(11-(2-amino-2-oxoethyl)-1,4,8,11-tetraazabicyclo[6.6.2]hexadecan-4-yl)acetic acid | gg |
| CB-TR2A | 2,2'-(1,4,8,11-tetraazabicyclo[6.5.2]pentadecane-4,11-diyl)diacetic acid | j |
| CDTA | trans-1,2-diaminocyclohexane-N,N,N',N'-tetraacetic acid | b |
| C-DTPA | bis(2-(2,6-dioxomorpholino)ethyl)glycine | c |
| CHX-A"-DTPA | N-(2-aminoethyl)-trans-1,2-diaminocyclohexane-N,N',N"-pentaacetic acid | c |
| CHX-macropa | rac-6,6'-(((16aS,20aS)/(16aR,20aR)-Hexadecahydro-4H,13Hbenzo[b][1,4,10,13]tetraoxa[7,16]diazacyclooctadecine-4,13-diyl)bis(methylene))dipicolinic acid | c |
| CHX-octapa | 6,6'-((cyclohexane-1,2-diylbis((carboxymethyl)azanediyl))bis(methylene))dipicolinic acid | h |
| Crown | 2,2',2",2‴-1,10-dioxa-4,7,13,16-tetraazacyclooctadecane-4,7,13,16-tetrayl-tetraacetic acid | h |
| Crown-N6₃A | 2,16-dioxo-3,6,9,12,15,21-hexaazabicyclo[15.3.1]henicosa-1(21), 17,19-triene-N(6),N(9),N(12)-triacetic acid | c |
| Cyclam | 1,4,8,11-tetraazacyclotetradecane | b |
| Cyclen | 1,4,7,10-tetraazacyclododecane | b |
| DATA(M) | 2,2'-(6-((carboxymethyl)(methyl)amino)-6-methyl-1,4-diazepane-1,4-diyl)diacetic acid | m |
| DATA(P) | (2S,2'S)-2,2'-(6-(((S)-1-carboxyethyl)amino)-6-methyl-1,4-diazepane-1,4-diyl)dipropionic acid | m |
| DATA(Ph) | d2,2'-(5-((carboxymethyl)amino)-5-phenyldihydropyrimidine-1,3(2H,4H)-diyl)diacetic acid | m |
| DATA(PPh) | (2S,2'S)-2,2'-(5-(((S)-1-carboxyethyl)amino)-5-phenyldihydropyrimidine-1,3(2H,4H)-diyl)dipropionic acid | m |
| DE4TA | 2,2',2"-(10-(2-((carboxymethyl)amino)ethyl)-1,4,7,10-tetraazacyclododecane-1,4,7-triyl)triacetic acid | s |
| Deferiprone | 3-hydroxy-1,2-dimethyl-4(1H)-pyridone | e |
| DEPA | 7-[2-(bis-carboxymethylamino)-ethyl]-4,10-bis-carboxymethyl-1,4,7,10-tetraaza-cyclododec-1-yl-acetic acid | c |
| DFC | N-[3-[(2S,5S,8S)-5,8-bis[3-[acetyl(hydroxy)amino]propyl]-3,6,9,12,15,18-hexaoxo-1,4,7,10,13,16-hexazacyclooctadec-2-yl]propyl] -N -hydroxyacetamide | oo |
| DFO | N1-(5-aminopentyl)-N1-hydroxy-N4-(5-(N-hydroxy-4-((5-(N-hydroxyacetamido)pentyl)amino)-4-oxobutanamido)pentyl)succinamide | a |
| DFO-HOPO | N1-hydroxy-N1-(5-(4-(hydroxy(5-(1-hydroxy-6-oxo-1,6-dihydropyridine-2-carboxamido)pentyl)amino)-4-oxobutanamido)pentyl)-N4-(5-(N-hydroxyacetamido)pentyl)succinamide | dd |
| DiAmSar | 1,8-diamino-3,6,10,13,16,19-hexaazabicyclo[6.6.6]icosane | a |
| Diphosphine | 2,3-bis(diphenylphosphaneyl)maleic acid | b |
| DM-TE2A | 2,2'-(4,11-dimethyl-1,4,8,11-tetraazacyclotetradecane-1,8-diyl)diacetic acid | a |
| DO2A | 1,4,7,10-tetraazacyclododecane-1,7-diacetic acid | n |
| DO2a2p | 2,2'-(4,10-bis(phosphonomethyl)-1,4,7,10-tetraazacyclododecane-1,7-diyl)diacetic acid | n |
| DO2AP | 2,2'-(4-(phosphonomethyl)-1,4,7,10-tetraazacyclododecane-1,7-diyl)diacetic acid | o |
| DO2P | ((1,4,7,10-tetraazacyclododecane-1,7-diyl)bis(methylene))bis(phosphonic acid) | b |
| DO2PA | 6,6' -((1,4,7,10-tetraazacyclododecane-1,7-diyl)bis(methylene))dipicolinic acid | g |
| DO2Py2Am | 1,7-bis(2-pyridylmethyl)-4,10-bis((2-(2-hydroxyethoxy)ethyl)-2-aminoacetamido)-1,4,7,10-tetraazacyclododecane | c |
| DO3A | 1,4,7,10-tetraazacyclododecane-1,4,7-triacetic acid | c |
| DO3A-Bu | 10-(2,3-dihydroxy-(1-hydroxymethyl)propyl)-1,4,7,10-tetraazacyclododecane-1,4,7-triacetic acid | c |
| DO3P | ((4-(phosphonomethyl)-1,4,7,10-tetraazacyclododecane-1,7-diyl)bis(methylene))bis(phosphonic acid) | b |
| DO3PA | 2-(4,7,10-tris(phosphonomethyl)-1,4,7,10-tetraazacyclododecan-1-yl)acetic acid | n |
| DOTA | 1,4,7,10-tetrazacyclododecane-1,4,7,10-tetraacetic acid | a |
| DOTA-2Py | 2,2'-(7,10-bis(pyridin-2-ylmethyl)-1,4,7,10-tetraazacyclododecane-1,4-diyl)diacetic acid | h |
| DOTA-3Py | 2-(4,7,10-tris(pyridin-2-ylmethyl)-1,4,7,10-tetraazacyclododecan-1-yl)acetic acid | h |
| DOTAGA | 2-(4,7,10-tris(carboxymethyl)-1,4,7,10-tetraazacyclododecan-1- yl)pentanedioic acid | a |
| DOTG | 2,2',2",2‴-(1,4,7,10-tetraazacyclododecane-1,4,7,10-tetrayl)tetraglutaric acid | s |
| DOTHA₂ | 2-(4,7,10-tris(2-(hydroxy(methyl)amino)-2-oxoethyl)-1,4,7,10-tetraazacyclododecan-1-yl)acetic acid | l |
| DOTMA | (2R,2'R,2"R,2‴R)-2,2',2",2‴-(1,4,7,10-tetraazacyclododecane-1,4,7,10-tetrayl)tetrapropionic acid | p |
| DOTP | ((1,4,7,10-tetraazacyclododecane-1,4,7,10-tetrayl)tetrakis(methylene))tetrakis(phosphonic acid) | c |
| DOTPA | 3,3',3",3‴-(1,4,7,10-tetraazacyclododecane-1,4,7,10-tetrayl)tetrapropionic acid | s |
| DOTPH | 1,4,7,10-tetraazacyclododecane-1,4,7,10-tetrakis(methylenephosphinic acid) | c |
| DOTPI | 1,4,7,10-tetraazacyclododecane-1,4,7,10-tetrakis(methylene(2-carboxyethylphosphinic acid)) | c |
| DOTP-OEt | 1,4,7,10-tetraazacyclododecane-1,4,7,10-tetrakis(methylene(phosphonic acid monoethyl ester)) | c |
| DOTPy/TPC | 1,4,7,10-tetrakis(2-pyridylmethyl)-1,4,7,10-tetraazacyclododecane | c |
| DOTPyd | 1,4,7,10-tetrakis(3-pyridazylmethyl)-1,4,7,10-tetraazacyclododecane | c |
| DOTPyr | 1,4,7,10-tetrakis(4-pyrimidylmethyl)-1,4,7,10-tetraazacyclododecane | c |
| DOTPz | 1,4,7,10-tetrakis(2-pyrazinylmethyl)-1,4,7,10-tetraazacyclododecane | c |
| Dp44mT | 2-(di(pyridin-2-yl)methylene)-N,N-dimethylhydrazine-1-carbothioamide | ff |
| DpC | di-2-pyridylketone-4-cyclohexyl-4-methyl-3-thiosemicarbazone | mm |
| DTCBP | (3 -hydroxy-3,3 -diphosphonopropyl)(methyl)carbamodithioic acid | ll |
| DTPA | diethylenetriaminepentaacetic acid | a |
| DTPAm | 2,2',2",2‴-((((2-amino-2-oxoethyl)azanediyl)bis(ethane-2,1-diyl))bis(azanetriyl))tetraacetamide | i |
| ECC | ethylenecysteamine cysteine | s |
| EDTA | ethylenediaminetetraacetic acid | c |
| EDTMP | ethylenediamine-N,N,N',N"-tetramethylenephosphonic acid | g |
| EGTA | ethylene glycol bis(2-aminoethyl ether)-N,N,N',N'-tetraacetic acid | s |
| FSC | 3,15,27-triamino-7,19,31-trihydroxy-10,22,34-trimethyl-1,13,25-trioxa-7,19,31-triaza-cyclohexatriaconta-9,21,33-triene-2,8,14,20,26,32-hexaone | s |
| H₂ampa | 6,6'-(2,11-bis(2-amino-2-oxoethyl)-5,8-dioxa-2,11-diazadodecane-1,12-diyl)dipicolinic acid | ss |
| Hzazapa | 6,6'-((ethane-1,2-diylbis(((1-benzyl-1H-1,2,3-triazol-4-yl)methyl)azanediyl))bis(methylene))dipicolinic acid | a |
| H₂BA2A1Py | 2,2'-(7-(pyridin-2-ylmethyl)-2,3,6,7, 8,9,11,12-octahydro-4H-benzo[b][1,4]dioxa[7,10,13]triazacyclopentadecine-4,10(5H)-diyl)diacetic acid | bb |
| H₂Bispa² | 6,6'-((9-hydroxy-1-methoxy-5-(methoxycarbonyl)-2,4-di(pyridin-2-yl)-3,7-diazabicyclo(3.3.1)nonane-3,7-diyl)bis(methylene)) dipicolinic acid | i |
| H₂CHX-DEDPA | 6,6'-((((1R,2R)-cyclohexane-1,2-diyl)bis(azanediyl))bis(methylene))dipicolinic acid | q |
| H₂CHX-macropa | 6,6'-(((16aR,20aR)-hexadecahydro-4H,13H-benzo[b][1,4,10,13]tetraoxa[7,16]diazacyclooctadecine-4,13-diyl)bis(methylene))dipicolinic acid | i |
| H₂dedpa | 1,2-[[6-(carboxy)-pyridin-2-yl]-methylamino]ethane | a |
| H₂macrodipa | 6,6'-((1,4,7,13-tetraoxa-10,16-diazacyclooctadecane-10,16-diyl)bis(methylene))dipicolinic acid | i |
| H₂macropa | N,N'-bis[(6-carboxy-2-pyridyl)methyl]-4,13-diaza-18-crown-6 | i |
| H₂ODO2A | 1-oxa-4,7,10-triazacyclododecane-4,10-diaceticacid | qq |
| H₃BA3A | 2,2',2"-(5,6,8,9,11,12-hexahydro-2H-benzo[b][1,4]dioxa[7,10,13]triazacyclopentadecine-4,7,10(3H)-triyl)triacetic acid | bb |
| H₃macrotripa | 6,6',6"-((1,7,13-trioxa-4,10,16-triazacyclooctadecane-4,10,16-triyl)tris(methylene))tripicolinic acid | i |
| H₃THP | 4-amino-N1,N7-bis((3-hydroxy-1,6-dimethyl-4-oxo-1,4-dihydropyridin-2-yl)methyl)-4-(3-(((3-hydroxy-1,6-dimethyl-4-oxo-1,4-dihydropyridin-2-yl)methyl)amino)-3-oxopropyl)heptanediamide | e |
| H₄(3,4,3-(LI-1,2-HOPO)) | N,N'-(butane-1,4-diyl)bis(1-hydroxy-N-(3-(1-hydroxy-6-oxo-1,6-dihydropyridine-2-carboxamido)propyl)-6-oxo-1,6-dihydropyridine-2-carboxamide) | i |
| H₄(3,4,3-LI(Me-3,2-HOPO)) | N,N'-(butane-1,4-diyl)bis(3-hydroxy-N-(3-(3-hydroxy-1-methyl-2-oxo-1,2-dihydropyridine-4-carboxamido)propyl)-1-methyl-2-oxo-1,2-dihydropyridine-4-carboxamide) | i |
| H₄(Me-3,2-HOPO-OH) | 4-((4-(3-(bis(2-(3-hydroxy-1-methyl-2-oxo-1,2-dihydropyridine-4-carboxamido)ethyl)amino)-2-((bis(2-(3-hydroxy-1-methyl-2-oxo-1,2-dihydropyridine-4-carboxamido)ethyl)amino)methyl)propyl)phenyl)amino)-4-oxobutanoic acid | i |
| H₄neunpa | 6,6'-(((azanediylbis(ethane-2,1-diyl))bis((carboxymethyl)azanediyl))bis(methylene))dipicolinic acid | i |
| H₄noneunpa | 6,6'-(((oxybis-(ethane-2,1-diyl))bis((carboxymethyl)azanediyl))bis(methylene))-dipicolinic acid | i |
| H₄octapa | 6,6'-((ethane-1,2-diylbis((carboxymethyl)azanediyl))bis(methylene))dipicolinic acid | a |
| Hsdecapa | 6,6'-(((((carboxymethyl)azanediyl)bis(ethane-2,1-diyl))bis((carboxymethyl)azanediyl))bis(methylene))dipicolinic acid | a |
| H₅DO3AP | 1,4,7,10-tetraazacyclododecane-1,4,7-triacetic-10-methylphosphonic acid | i |
| H₆phospha | N,N'-(methylenephosphonate)-N,N'-[6-(methoxycarbonyl)pyridin-2-yl] -methyl-1 ,2-diaminoethane | a |
| H₆Sbbpen | N,N'-bis(2-hydroxy-5-sulfonylbenzyl)-N,N'-bis-(2-methylpyridyl) ethylenediamine | s |
| H₈(3,4,3-LI(CAM)) | N,N'-(butane-1,4-diyl)bis(N-(3-(2,3- dihydroxybenzamido)propyl)-2,3-dihydroxybenzamide) | i |
| Hs(terephthalamide) | N¹,N¹'-((1²,1³,11²,11³-tetrahydroxy-2,10,12,20-tetraoxo-3,6,9,13,16,19-hexaaza-1,11(1,4)-dibenzenacycloicosaphane-6,16-diyl)bis(ethane-2,1-diyl))bis(2,3-dihydroxy-N⁴-(2-methoxyethyl)terephthalamide) | i |
| HBED | 2,2'-(ethane-1,2-diylbis((2-hydroxybenzyl)azanediyl))diacetic acid | a |
| HBED-CC | 3,3'-(((ethane-1,2-diylbis((carboxymethyl)azanediyl))bis(methylene))bis(4-hydroxy-3,1-phenylene))dipropionic acid | a |
| Hcb-te1pa | 6-((1,4,8,11-tetraazabicyclo[6.6.2]hexadecan-4-yl)methyl)picolinic acid | kk |
| HEHA | 2,2',2",2‴,2ʺʺ,2‴ʺ-(1,4,7,10,13,16-hexaazacyclooctadecane-1,4,7,10,13,16-hexayl)hexaacetic acid | a |
| HP-DO3A | 10-(2-hydroxypropyl)-1,4,7-tetraazacyclododecane-1,4,7-triacetic acid | r |
| HYNIC | 6-hydrazineylnicotinic acid | w |
| macropaquin | 6-((16-((8-hydroxyquinolin-2-yl)methyl)-1,4,10,13-tetraoxa-7,16-diazacyclooctadecan-7-yl)methyl)picolinic acid | c |
| macrophosphi | (((1,4,10,13-tetraoxa-7,16-diazacyclooctadecane-7,16-diyl)bis(methylene))bis(pyridine-6,2-diyl))bis(methylphosphinic acid) | c |
| macrophospho | (((1,4,10,13-tetraoxa-7,16-diazacyclooctadecane-7,16-diyl)bis(methylene))bis(pyridine-6,2-diyl))bis(phosphonic acid) | c |
| macroquin-SO3 | 8-hydroxy-2-((16-((1-hydroxy-4-sulfoisoquinolin-3-yl)methyl)-1,4,10,13-tetraoxa-7,16-diazacyclooctadecan-7-yl)methyl)quinoline-5-sulfonic acid | c |
| MAG3 | (2-mercaptoacetyl)glycylglycylglycine | w |
| MAMA | N-(2-mercaptoethyl)-2-((2-mercaptoethyl)amino)acetamide | w |
| MA-NOTMP | ((1,4,7-triazonane-1,4,7-triyl)tris(methylene))tris(methylphosphinic acid) | y |
| Me-DO2Pa | 6,6'-((4,10-dimethyl-1,4,7,10-tetraazacyclododecane-1,7-diyl)bis(methylene))dipicolinic acid | c |
| Me-DO2Sc | (2E,2'E)-2,2'-((4,10-dimethyl-1,4,7,10-tetraazacyclododecane-1,7-diyl)bis(propan-1-yl-2-ylidene))bis(hydrazine-1-carboxamide) | c |
| MM-TE2A | 2,2'-(4-methyl-1,4,8,11-tetraazacyclotetradecane-1,8-diyl)diacetic acid | a |
| MX-DTPA | 2,2'-((1-((2-(bis(carboxymethyl)amino)ethyl)(carboxymethyl)amino)propan-2-yl)azanediyl)diacetic acid | g |
| N2S2 | 2,2'-(ethane-1,2-diylbis(azanediyl))bis(ethane-1-thiol) | w |
| N4 | N,N'-bis-(2-amino-ethyl)-propane-1,3-diamine | w |
| N₅3Pa | N,N"-bis(2-aminoethyl)-diethylenetriamino-N,N',N"-tripicolinic acid | c |
| NE3TA | 2,2'-(7-(2-((carboxymethyl)amino)ethyl)-1,4,7-triazonane-1,4-diyl)diacetic acid | l |
| NETA | 2,2'-((2-(4,7-bis(carboxymethyl)-1,4,7-triazonan-1-yl)ethyl)azanediyl)diacetic acid | a |
| NO2A | 2,2'-(1,4,7-triazonane-1,4-diyl)diacetic acid | s |
| NO2AP | 2,2'-(7-(((2-carboxyethyl)(hydroxy)phosphoryl)methyl)-1,4,7-triazonane-1,4-diyl)diacetic acid | s |
| NODAGA | 2-(4,7-bis(carboxymethyl)-1,4,7-triazonan-1-yl)pentanedioic acid | a |
| NODA-MPAA | 2,2'-(7-(4-(carboxymethyl)benzyl)-1,4,7-triazonane-1,4-diyl)diacetic acid | tt |
| NODASA | 2-(4,7-bis(carboxymethyl)-1,4,7-triazonan-1-yl)succinic acid | s |
| NOPO | 3-(((4,7-bis((hydroxy(hydroxymethyl)phosphoryl)methyl)-1,4,7-triazonan-1-yl)methyl)(hydroxy)phosphoryl)propanoic acid | a |
| NOTA | 1,4,7-triazacyclononane-N,N',N"-triacetic acid | a |
| NOTAM | 2,2',2"-(1,4,7-triazonane-1,4,7-triyl)triacetamide | z |
| NOTHA₂ | 2-(4,7-bis(2-(hydroxy(methyl)amino)-2-oxoethyl)-1,4,7-triazonan-1-yl)acetic acid | l |
| NOTP | ((1,4,7-triazonane-1,4,7-triyl)tris(methylene))tris(phosphonic acid) | s |
| NPTA | ((4-carboxymethyl-7-(2-(carboxymethylamino)-ethyl)-perhydro-1,4,7-triazonin-1-yl)-acetic acid | c |
| NxS4-x | stands for a group of tetradentate chelators with N-atoms (basic amine or non-basic amide) and thiols as donors stabilizing Tc-complexes, especially Tc(V)-oxo complexes | w |
| Oxabiphore | (((oxybis(ethane-2,1-diyl))bis(azanetriyl))tetrakis(methylene)) tetrakis(phosphonic acid) | g |
| oxo-DO3A | 2,2',2"-(1-oxa-4,7,10-triazacyclododecane-4,7,10-triyl)triacetic acid | a |
| PCBA | 2-((1,4,7,10,13-pentaazacyclopentadecan-1-yl)methyl)benzoic acid | ee |
| PCB-TE2A | 2,2'-(1,4,8,11-tetraazabicyclo[6.6.3]heptadecane-4,11-diyl)diacetic acid | k |
| PCTA | 2,2',2"-(3,6,9-triaza-1(2,6)-pyridinacyclodecaphane-3,6,9-triyl)triacetic acid | a |
| p-EDDHA | 2,2'-(ethane-1,2-diylbis(azanediyl))bis(2-(4-hydroxyphenyl)acetic acid) | w |
| PEPA | 2,2',2",2‴,2ʺʺ-(1,4,7,10,13-pentaazacyclopentadecane-1,4,7,10,13-pentayl)pentaacetic acid | a |
| PIH | (E)-N'-((3-hydroxy-5-(hydroxymethyl)-2-methylpyridin-4-yl)methylene)isonicotinohydrazide | ff |
| PIP-DOTA | cis-((1R,11S)-6,9,15-tris-car-boxymethyl-3,6,9,15-tetraazabicyclo[9.3.1]pentadec-3-yl)-acetic acid | c |
| PIP-DTPA | cis-2,6-bis(N,N-bis(carboxymethyl)aminomethyl)-1-piperidine acetic acid | c |
| PSC | 2,2',2"-(10-(2-amino-2-oxoethyl)-1,4,7,10-tetraazacyclododecane-1,4,7-triyl)triacetic acid | t |
| PTSM | Pyruvaldehyde bis(N⁴-methylthiosemicarbazone) | b |
| Py4pa | 6,6'-(((pyridine-2,6-diylbis(methylene))-bis((carboxymethyl)azanediyl))bis(methylene))dipicolinic acid | h |
| Pycup2A | 1,8-(2,6-Pyridinedimethylene)-1,4,8,11-tetraazacyclotetradecane-2,4-diacetic acid | l |
| QT | quinoline thiosemicarbazone | rr |
| Sarcophagine | 3,6,10,13,16,19-hexazabicyclo[6.6.6]icosane | b |
| SHBED | 2,2'-(ethane-1,2-diylbis((2-hydroxy-5 - sulfobenzyl)azanediyl))diacetic acid | a |
| Tachpyr | N1,N3,N5-tris(pyridin-2-ylmethyl)cyclohexane-1,3,5-triamine | b |
| TACN | 1,4,7-triazonane | v |
| TACN-TM | tris(2-mercaptoethyl)-1,4,7-triazacyclononane | a |
| TAM | 2,3-dihydroxyterephthalamide | u |
| TAME | 2-(aminomethyl)-2-methylpropane-1,3-diamine | v |
| TAME-Hex | (1,8-N,N'-bis(carboxymethyl)-1,4,8,11-tetraazacyclotetradecane | v |
| TCMC | 2,2',2",2‴-(1,4,7,10-tetraazacyclododecane-1,4,7,10-tetrayl)tetraacetamide | a |
| TE2A | 2,2'-(1,4,8,11-tetraazacyclotetradecane-1,8-diyl)diacetic acid | a |
| TE2Py2Am | 1,8-bis(2-pyridylmethyl)-4,11-bis((2-(2-hydroxyethoxy)ethyl)-2-aminoacetamido)-1,4,8,11-tetraaza-cyclotetradecane | a |
| TE3A | 2,2',2"-(1,4,8,11-tetraazacyclotetradecane-1,4,8-triyl)triacetic acid | a |
| TETA | 2,2',2",2‴-(1,4,8,11-tetraazacyclotetradecane-1,4,8,11-tetrayl)tetraacetic acid | a |
| tet-a | (7S,14R)-5,5,7,12,12,14-hexamethyl-1,4,8,11-tetraazacyclotetradecane | jj |
| TETAM | 2,2',2",2‴-(1,4,8,11-tetraazacyclotetradecane-1,4,8,11-tetrayl)tetraacetamide | pp |
| TETPA | 3,3'-(4,11-bis(carboxymethyl)-1,4,8,11-tetraazacyclotetradecane-1,8-diyl)dipropionic acid | s |
| THPN | 1,3-propanediamine-N,N,N',N'-tetrakis[(2-(aminomethyl)-3-hydroxy-1,6-dimethyl-4(1H)-pyridinone)acetamide] | oo |
| TMT-amine | 2,2',2",2‴-(((4'-(3-amino-4-methoxyphenyl)-[2,2':6',2"-terpyridine]-6,6"-diyl)bis(methylene))bis(azanetriyl))tetraacetic acid | hh |
| TRAP | 3,3',3"-(((1,4,7-triazonane-1,4,7-triyl)tris(methylene))tris(hydroxyphosphoryl))tripropanoic acid | a |
| TREN(Me-3,2-HOPO) | N,N'-(((2-(3-hydroxy-2-oxo-1,2-dihydropyridine-4-carboxamido)ethyl)azanediyl)bis(ethane-2,1-diyl))bis(3-hydroxy-1-methyl-2-oxo-1,2-dihydropyridine-4-carboxamide) | ii |
| Triapine | (E)-2-((3 -aminopyridin-2-yl)methylene)hydrazine-1-carbothioamide | ff |
| TRITA | 2,2',2",2‴-(1,4,7,10-tetraazacyclotridecane-1,4,7,10-tetrayl)tetraacetic acid | s |
| TRITAM | 2,2',2",2‴-(1,4,7,10-tetraazacyclotridecane-1,4,7,10-tetrayl)tetraacetamide | pp |
| TTHA | triethylenetetramine-N,N,N',N",N‴,N‴-hexaacetic acid | s |
| [16]aneS₄ | 1,5,9,13-tetrathiacyclohexadecane | cc |
| [2,2,2]-cryptand | 4,7,13,16,21,24-hexaoxa-1,10-diazabicyclo(8.8.8) hexacosane | x |
| 6SS | N,N'-bis(2,2-dimethyl-2-mercaptoethyl)ethylenediamine-N,N'-diacetic acid | s |
| ^{99m}Tc(CO)₃-Chelators | Ligand platform that accommodates the ^{99m}Tc(CO)₃-core | w |

| | | |
|---|---|---|
| a. (Price and Orvig 2014) b. (Wadas, Wong et al. 2007) c. (Franchi, Di Marco et al. 2022) d. (Allott, Da Pieve et al. 2017) e. (Cusnir, Imberti et al. 2017) f. (Demoin, Dame et al. 2016) g. (Egorova, Fedorova et al. 2019) h. (Yang, Wilson et al. 2022) i. (Grieve and Paterson 2021) j. (Odendaal, Fiamengo et al. 2011) k. (Pandya, Dale et al. 2012) l. (Gai, Sun et al. 2016) m. (Seemann, Waldron et al. 2015) n. (Kalman, Baranyai et al. 2008) o. (Giovenzana, Lattuada et al. 2020) p. (Woods, Payne et al. 2019) q. (Ramogida, Cawthray et al. 2015) r. (Barge, Cappelletti et al. 2009) s. (Kostelnik and Orvig 2019) t. (Li, Baumhover et al. 2023) u. (McAuley, Beveridge et al. 1989) v. (Maecke and Andre 2007) w. (Mazzi, Schibli et al. 2007) x. (Poty, Desogere et al. 2015, McDonagh, McNeil et al. 2021) y. (Poty, Desogere et al. 2015) z. (AlHokbany, AlJammaz et al. 2022) aa. (Urbanovsky, Kotek et al. 2020) bb. (Egorova, Zamurueva et al. 2022) cc. (Straathof, Magnus et al. 2021) dd. (Yu, Li et al. 2016) ee. (Lozza, Navarro-Teulon et al. 2013) ff. (Heath, Weiss et al. 2013) gg. (Wadas, Wong et al. 2010) hh. (Stimmel, Stockstill et al. 1995) ii. (Yokel, Fredenburg et al. 2000) jj. (Woodin, Heroux et al. 2005) kk. (Lima, Halime et al. 2014) ll. (Cai and Anderson 2014) mm. (Guo, Richardson et al. 2016) nn. (Kanchi, Singh et al. 2014) oo. (Feiner, Brandt et al. 2021) pp. (Lyczko, Lyczko et al. 2020) qq. (Chang, Lee et al. 2013) rr. (Serda, Kalinowski et al. 2014) ss. (Ingham, Wharton et al. 2022) tt. (McBride, Sharkey et al. 2013) | | |

The diagnostic and/or therapeutic use of some of the above chelators is described in the prior art. For example, 2-hydrazino nicotinamide (HYNIC) has been widely used in the presence of a coligand for incorporation of ^{99m}Tc and ^{186,188}Re (Schwartz, Abrams et al. 1991, Babich, Solomon et al. 1993, Babich and Fischman 1995); DTPA is used in Octreoscan^{®} for complexing ¹¹¹In and several modifications are described in the literature (Brechbiel and Gansow 1991, Li, Ma et al. 2001); DOTA-type chelators for radiotherapy applications are described by Tweedle *et al.* (US Pat 4,885,363); other polyaza macrocycles for chelating trivalent isotopes metals are described by Eisenwiener *et al.* (Eisenwiener, Prata et al. 2002); and N4-chelators such as a ^{99m}Tc-N4-chelator have been used for peptide labeling in the case of mini gastrin for targeting CCK-2 receptors (Nock, Maina et al. 2005).

In preferred embodiments, the chelator used in the compound of the invention is selected from the group, but not limited to, comprising DOTA, DOTAGA, DOTAM, Crown, DOTP, NOTA, NODAGA, NODA-MPAA, HBED, TETA, CB-TE2A, DTPA, CHX-A"-DTPA, DFO, Macropa, HOPO ligand platform, TRAP, THP, AAZTA, DATA, NOPO, PCTA, PSC, sarcophagine, FSC, DEPA, DE4TA, NETA, NE3TA, H₄octapa, H₄CHXoctapa, H₄pypa, H₄py₄pa, HYNIC, NxS4-x (N4, N2S2, N3S), ^{99m}Tc(CO)₃-chelators and their analogs. Preferably, the chelator additionally comprises one or more functional groups or functionalities allowing for attachment to the compound of the invention.

The chemical structures of these chelators and the residues resulting from preferred functionalization and/or conjugation (exemplifying possible attachment points) are shown in Table 8 below.

**Table 8: Chemical structures of chelators and preferred residues.**

| **Abbreviation** | **Chelator structure** | **Residue after preferred attachment to linker or peptide** |
|---|---|---|
| AAZTA | 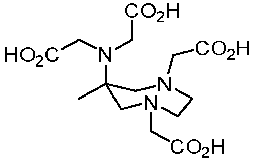 | 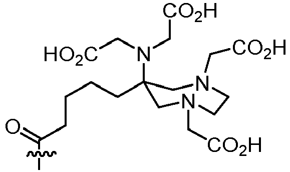 |
| DOTA (m=n=1) | 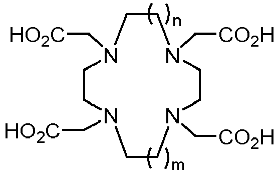 | 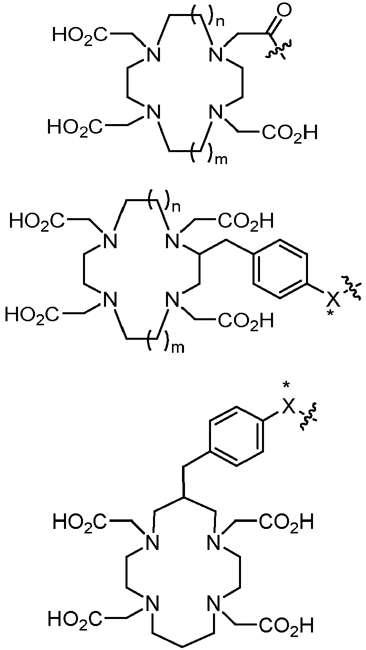 |
| TETA (m=n=2) | | |
| DOTAGA | 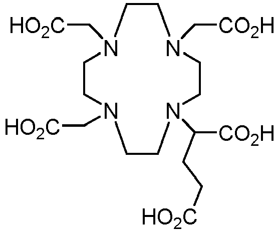 | 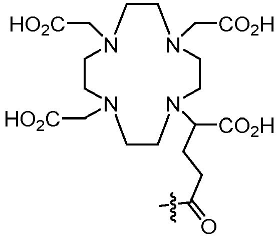 |
| DOTAM | 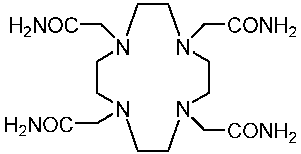 | 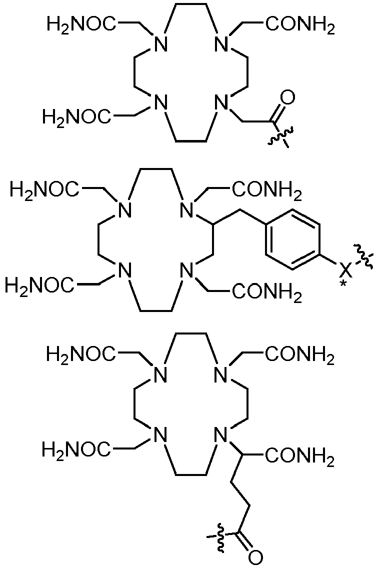 |
| NOTA | 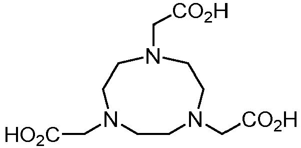 | 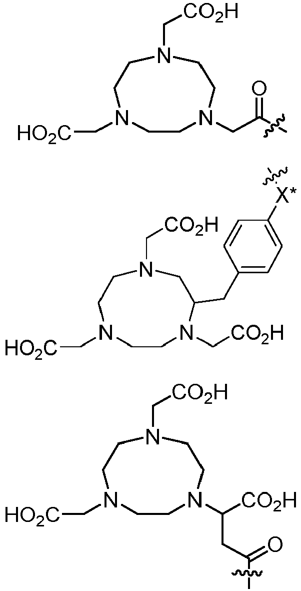 |
| NODAGA | 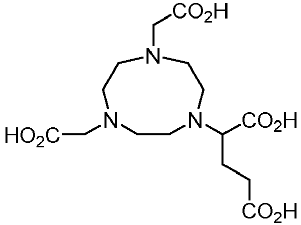 | 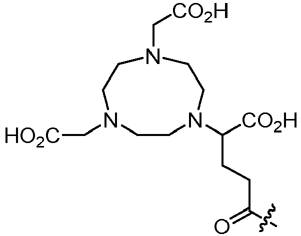 |
| NODA-MPAA | 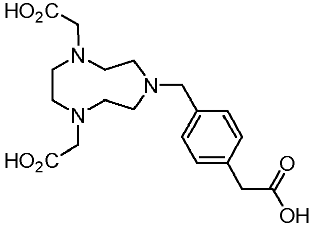 | 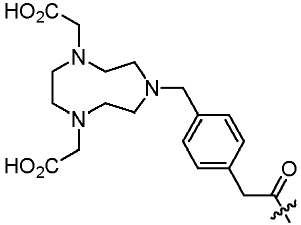 |
| DTPA | 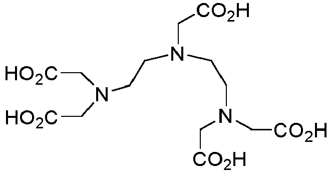 | 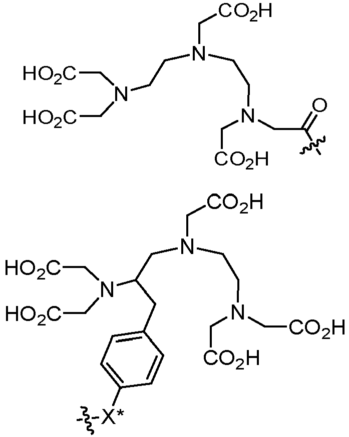 |
| CHX-A"-DTPA | 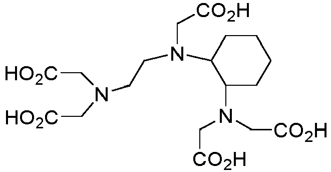 | 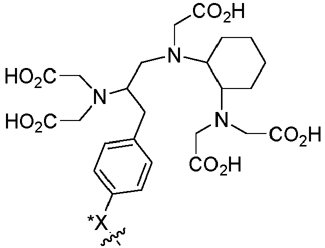 |
| N4 | 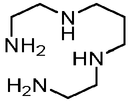 | 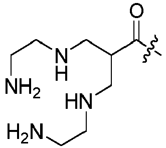 |
| N2S2^{†} | 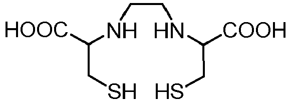 | 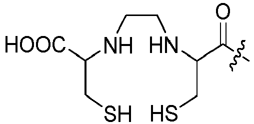 |
| Exemplary structure: EC | | |
| N3S^{†} | 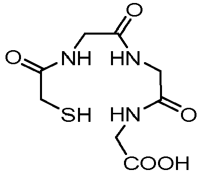 | 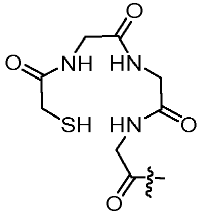 |
| Exemplary structure: MAG3 | | |
| ^{99m}Tc(CO)₃-chelators^{†} | 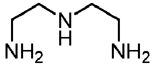 | 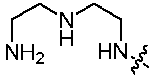 |
| Exemplary structure: triamine chelator | | |
| NETA | 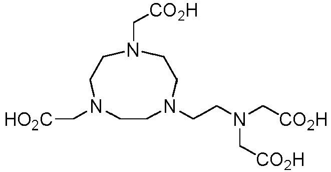 | 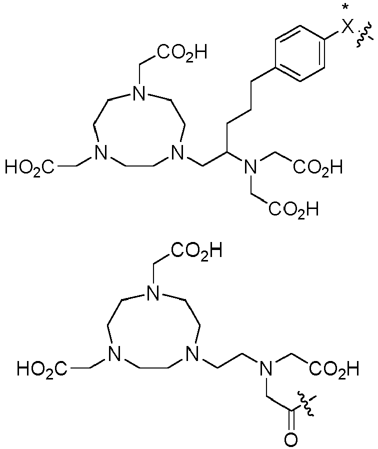 |
| NE3TA | 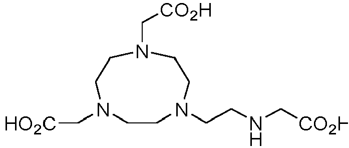 | 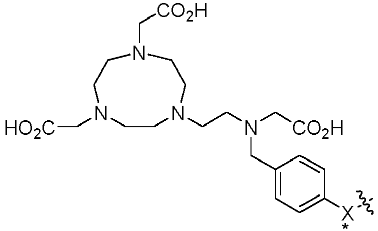 |
| HYNIC | 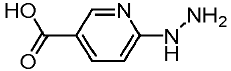 | 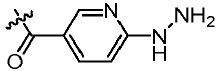 |
| TRAP | 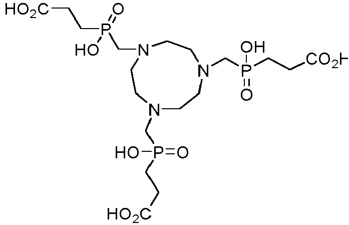 | 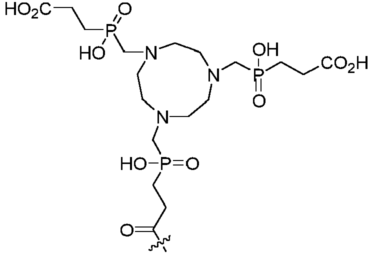 |
| NOPO | 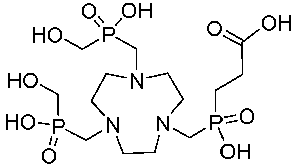 | 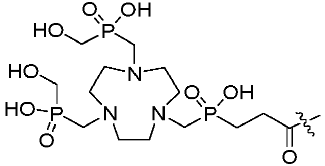 |
| DOTP | 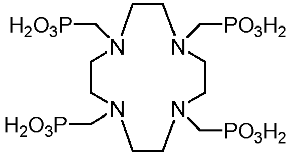 | 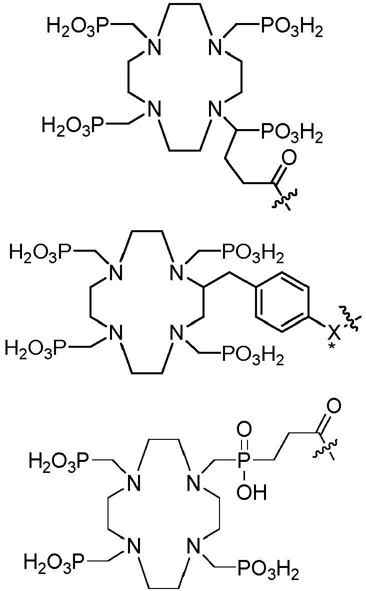 |
| CB-TE2A | 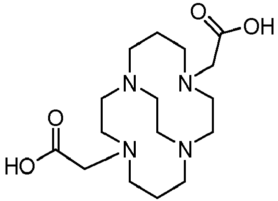 | 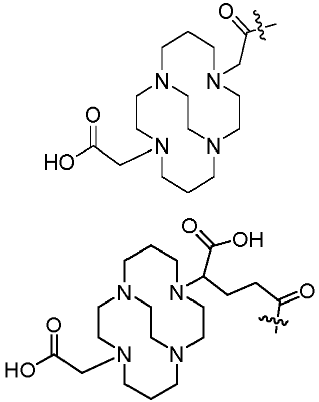 |
| Sarcophagine | 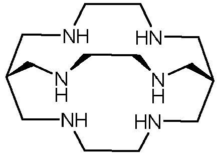 | 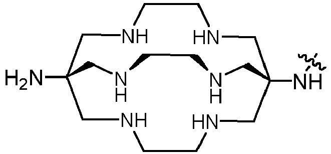 |
| DATA | 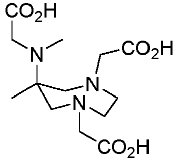 | 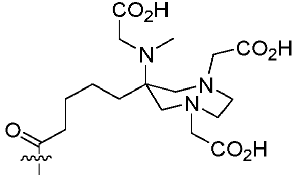 |
| HBED | 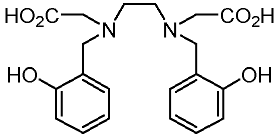 | 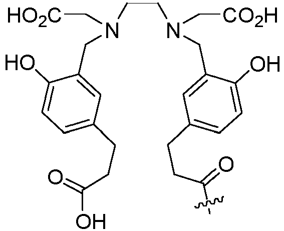 |
| H4octapa | 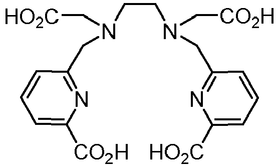 | 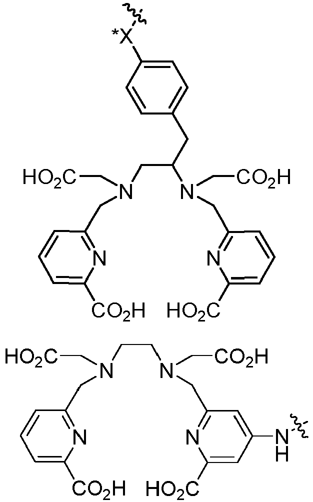 |
| H₄CHXoctapa | 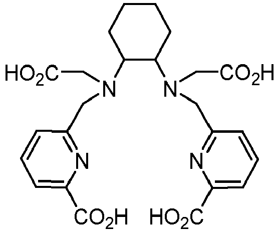 | 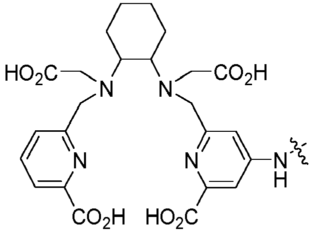 |
| H₄pypa | 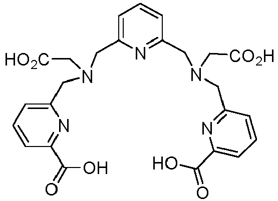 | 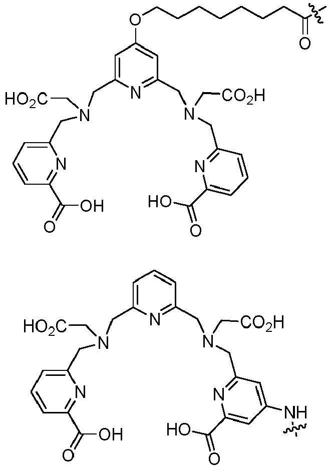 |
| H₄py4pa | 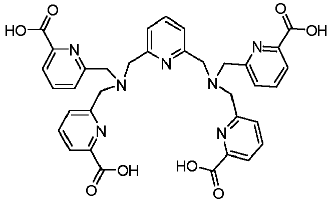 | 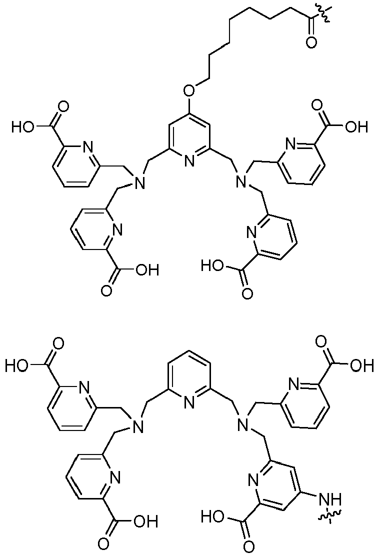 |
| Macropa | 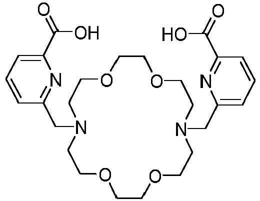 | 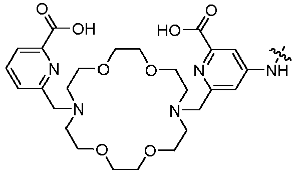 |
| FSC | 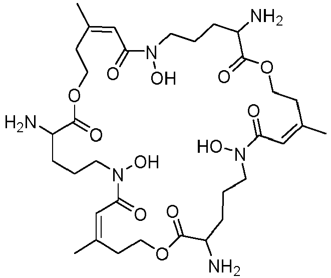 | 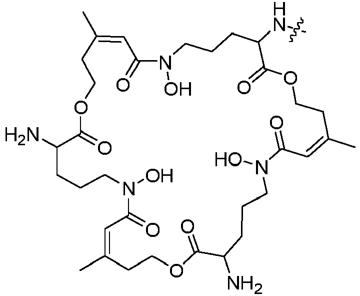 |
| DEPA | 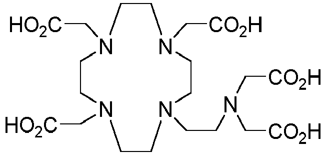 | 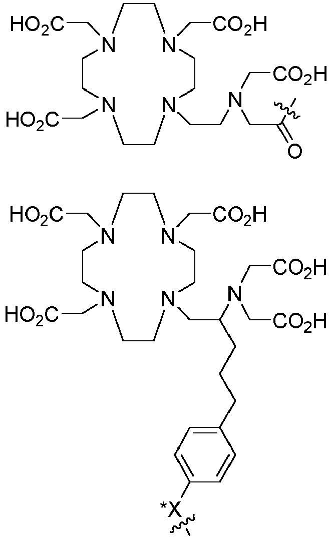 |
| DE4TA | 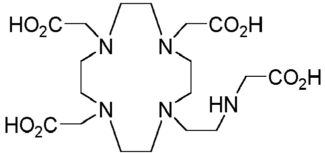 | 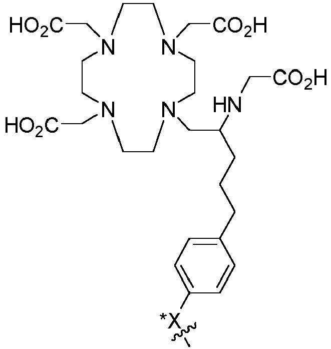 |
| Crown | 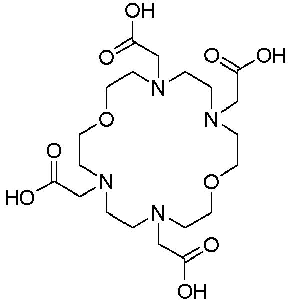 | 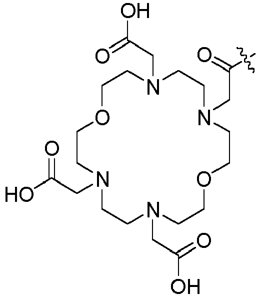 |
| DFO | 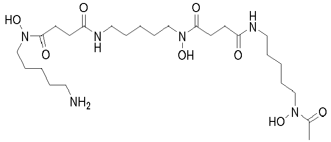 | 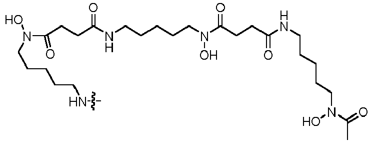 |
| PCTA | 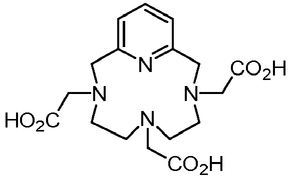 | 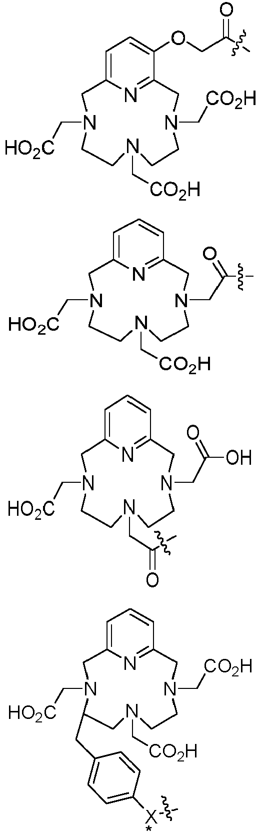 |
| HOPO ligand platform^{‡} | 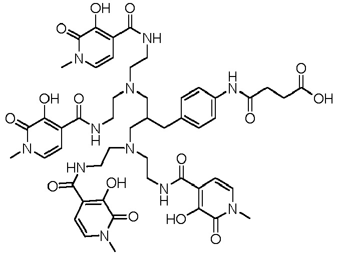 | 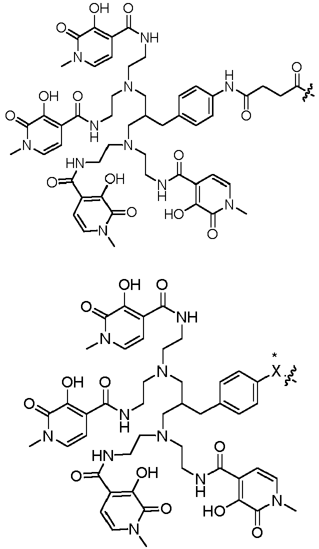 |
| Exemplary structure: | | |
| H₄(Me-3,2-HOPO-OH) | | |
| PSC | 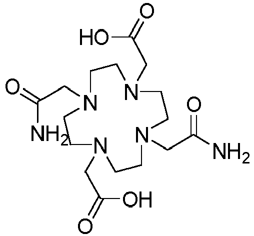 | 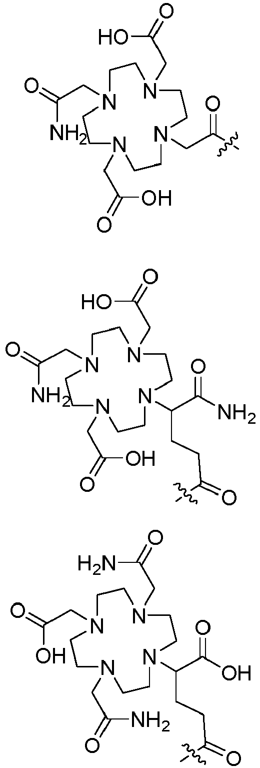 |
| THP | 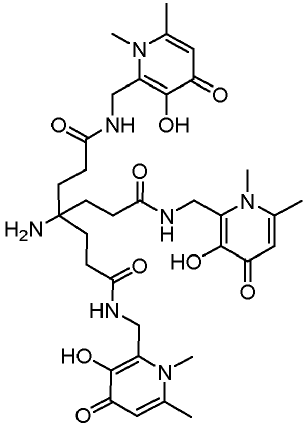 | 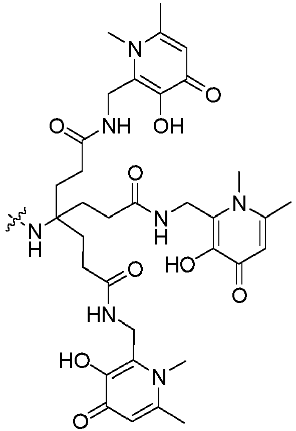 |

| | | |
|---|---|---|
| ^{†} as disclosed in: (Mazzi, Schibli et al. 2007) ^{‡} (Zhou, Dong et al. 2021) | | |

wherein X* is selected from the group consisting of (a) -NH- and (b) -NH-C(S)-, wherein in (a) the N atom is the attachment point of both the chelator and the rest of the compound of the invention and in (b) the N atom is the attachment point of the chelator and the C atom of the thiocarbonyl group is the attachment point of the rest of the compound of the invention.

In a more preferred embodiment, the chelator is selected from the group consisting of DOTA, DOTAGA, DOTAM, Crown, NOTA, NODAGA, NODA-MPAA, CB-TE2A, CHX-A"-DTPA, DFO, Macropa, THP, AAZTA, NOPO, PCTA, PSC, sarcophagine, NETA, H₄CHXoctapa, H₄pypa and N4, preferably from the group consisting of DOTA, DOTAGA, DOTAM, NOTA, NODAGA, Macropa, Crown, NOPO, PCTA, PSC and N4, more preferably from the group consisting of DOTA, DOTAM, Macropa, NOTA, PSC and NODAGA.

Most preferably, the chelator is DOTA.

Preferably, the chelator additionally comprises one or more functional groups or functionalities allowing attachment to the compounds of the invention.

It will be acknowledged by the persons skilled in the art that the chelator, in principle, may be used regardless of whether the compound of the invention is used in or suitable for diagnosis or therapy (as described, for example, in international patent application WO 2009/109332 A1).

It will be further acknowledged by the persons skilled in the art that the presence of a chelator in the compound of the invention includes, if not stated otherwise, the possibility that the chelator is complexed to any metal complex partner, i.e., any metal which, in principle, can be complexed by the chelator. An explicitly mentioned chelator of a compound of the invention or the general term chelator in connection with the compound of the invention refers either to the uncomplexed chelator as such or to the chelator to which any metal complex partner is bound, wherein the metal complex partner is any radioactive or non-radioactive metal complex partner. Preferably the chelator-metal complex, i.e., the chelator to which the metal complex partner is bound, is a stable chelator-metal complex.

Non-radioactive chelator-metal complexes have several applications, e.g., for assessing properties like stability or activity, which are otherwise difficult to determine. One aspect is that cold variants of the radioactive versions of the metal complex partner (e.g., non-radioactive indium complexes as described in the examples) can act as surrogates of the radioactive compounds. Furthermore, they are valuable tools for identifying metabolites *in vitro* or *in vivo,* as well as for assessing toxicity properties of the compounds of the invention. Additionally, chelator-metal complexes can be used in binding assays utilizing the fluorescence properties of some metal complexes with distinct ligands (e.g., Europium salts).

Chelators can be synthesized or are commercially available with a wide variety of (possibly already activated) groups for the conjugation to peptides or amino acids.

Due to the ready availability of carboxyl groups, DTPA, DOTA, DOTAGA, NOTA, NODAGA, NODA-MPAA, HBED, TETA, CB-TE2A, DATA, AAZTA, NETA, DEPA, Crown, NOPO, TRAP, HYNIC, PCTA, PSC and N2S2- as well as N3S-based chelators are particularly suitable for direct attachment to an amino nitrogen of the compound or to an amino group of a linker attached to the compound. The resulting linkage in this respect is an amide linkage.

Isothiocynate-functionalized chelator derivatives of for example DOTA, NOTA, DOTAM, DTPA, CHX-A"-DTPA, H₄octapa, PCTA, DOTP, DEPA, DE4TA, NETA, NE3TA and HOPO ligand platform chelators are particularly suitable for direct attachment to an amino nitrogen of the compound or to an amino group of a linker attached to the compound. The resulting linkage in this respect is a thiourea linkage.

Functional groups in a chelator which are preferred groups for the direct attachment of a chelator to an amino-nitrogen are known to the person skilled in the art and include, but are not limited to, carboxylic acid, activated carboxylic acid, e.g., active ester like for instance NHS-ester, pentafluorophenol-ester, HOBt-ester, HOAt-ester, and isothiocyanate.

Functional groups in a chelator which are preferred reactive groups for the direct attachment of a chelator to a carboxyl group are known to the person skilled in the art and include, but are not limited to, alkylamino and arylamino groups. Respective chelator reagents are commercially available, e.g., alkylamino or arylamino-functionalized DOTA.

Functional groups in a chelator which are preferred groups for the direct attachment of a chelator to a thiol group are known to the person skilled in the art and include, but are not limited to maleimide nitrogens. Respective chelator reagents are commercially available, e.g., maleimide-functionalized DOTA.

Functional groups in a chelator which are preferred groups for the direct attachment of a chelator to an azido group are known to the person skilled in the art and include, but are not limited to, acyclic and cyclic alkynes. Respective chelator reagents are commercially available, e.g., propargyl or butynyl-functionalized DOTA.

Functional groups in a chelator which are preferred groups for the direct attachment of a chelator to an alkyne group are known to the person skilled in the art and include, but are not limited to, alkyl and aryl azines. Respective chelator reagents are commercially available, e.g., azidopropyl-functionalized DOTA.

In an embodiment, each Z group of the compound of the invention independently comprises a chelator and a linker. It will be acknowledged by the persons skilled in the art that, in principle, a chelator as defined herein can be combined with an appropriate linker as defined herein so as to form a linkage to both the chelator and to a functional group in the compound of the invention (i.e., a functional group that provides a suitable attachment point for the linker). A non-limiting list of preferred Z groups comprising a chelator and a linker (exemplified for DOTA as the chelator) is as follows:

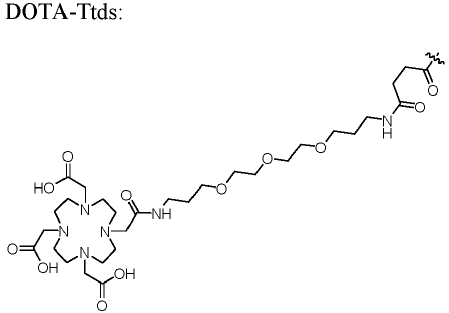

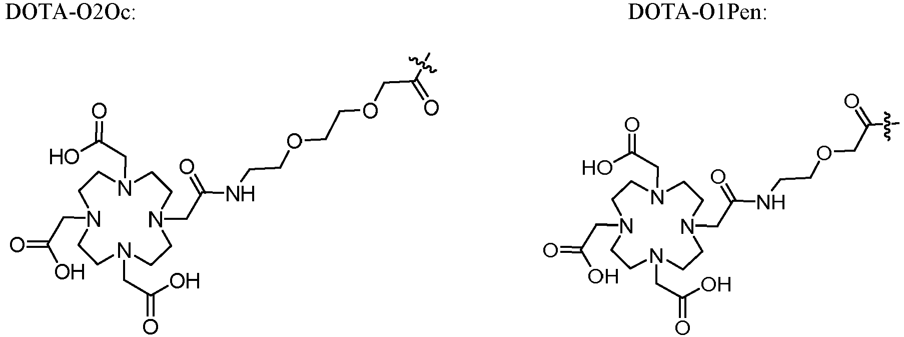

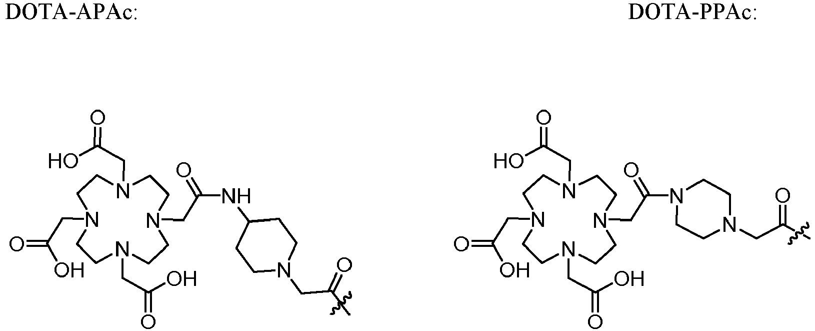

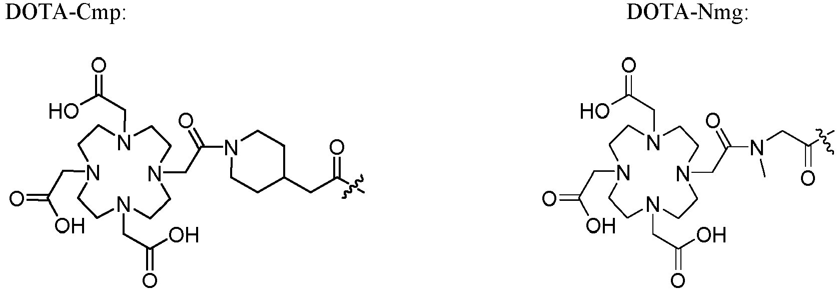

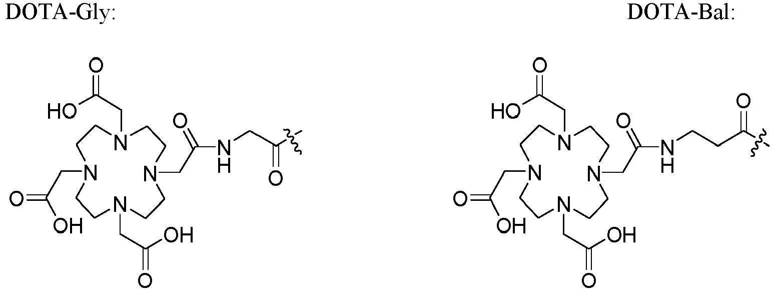

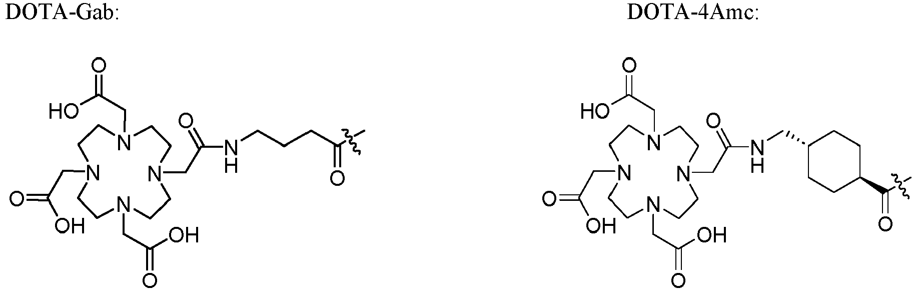

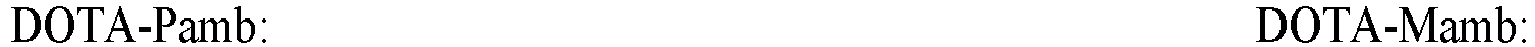

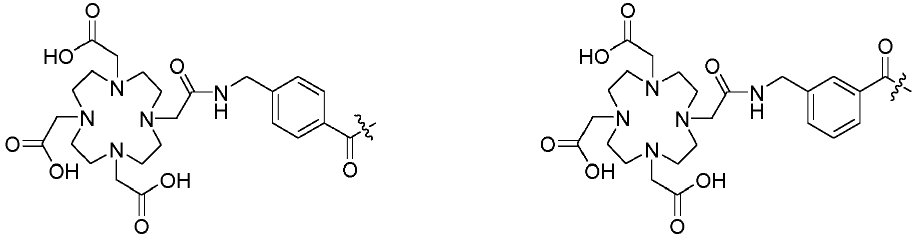

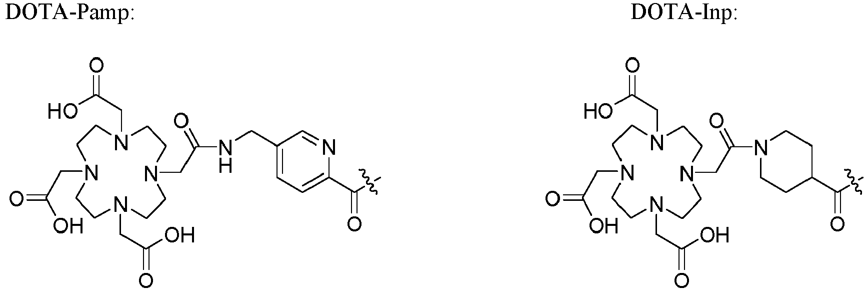

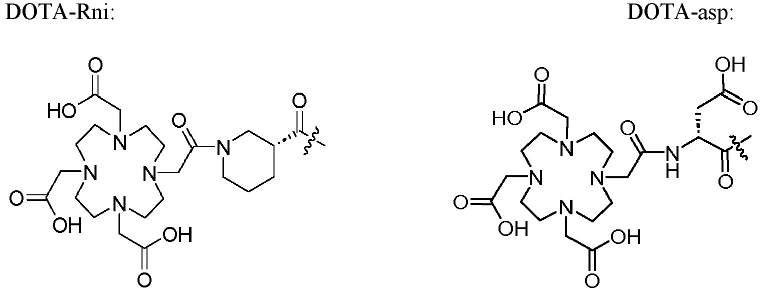

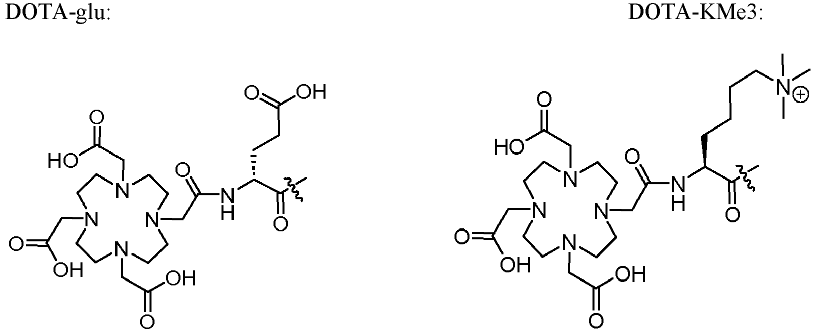

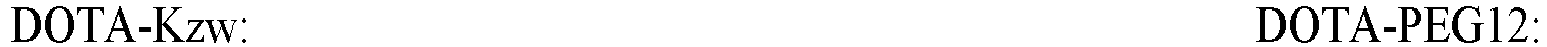

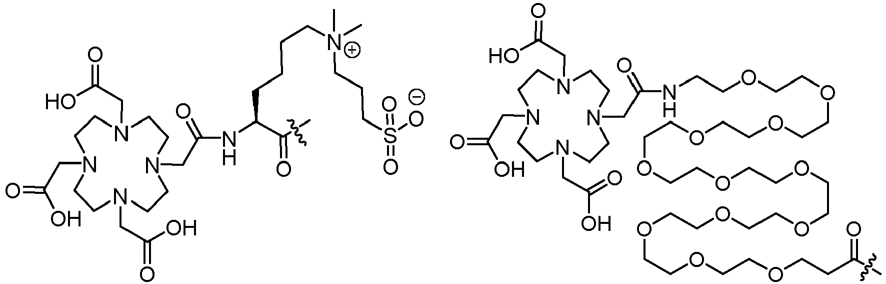

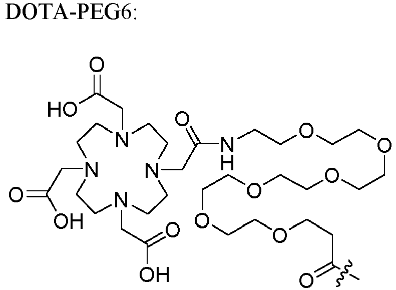

In preferred embodiments, the chelator (which may be comprised in each group Z) is selected from the group comprising DOTA, DOTAGA, DOTAM, Crown, DOTP, NOTA, NODAGA, NODA-MPAA, HBED, TETA, CB-TE2A, DTPA, CHX-A"-DTPA, DFO, Macropa, HOPO ligand platform, TRAP, THP, AAZTA, DATA, NOPO, PCTA, PSC, sarcophagine, FSC, DEPA, DE4TA, NETA, NE3TA, H₄octapa, H₄CHXoctapa, H₄pypa, H₄py₄pa, HYNIC, NxS4-x (N4, N2S2, N3S) and ^{99m}Tc(CO)₃-chelators.

In more preferred embodiments, the chelator (which may be comprised in each group Z) is selected from the group comprising DOTA, DOTAGA, DOTAM, Crown, NOTA, NODAGA, NODA-MPAA, CB-TE2A, CHX-A"-DTPA, DFO, Macropa, THP, AAZTA, NOPO, PCTA, PSC, sarcophagine, NETA, H₄CHXoctapa, H₄pypa and N4, preferably the chelator is selected from the group comprising DOTA, DOTAGA, DOTAM, NOTA, NODAGA, Macropa, Crown, NOPO, PCTA, PSC and N4, more preferably the chelator is selected from the group comprising DOTA, DOTAM, Macropa, NOTA, PSC and NODAGA, and most preferably the chelator is DOTA.

In a more preferred embodiment, in formula (I), each Z group is independently a moiety represented by the following formula (XIII):

E-* (XIII)

wherein,
* indicates covalent attachment to Xaa0, Xaa2, Xaa3 or Xaa7, and
E is a residue of a chelator represented by any one of the following formulae (XIVa) to (XIVn): 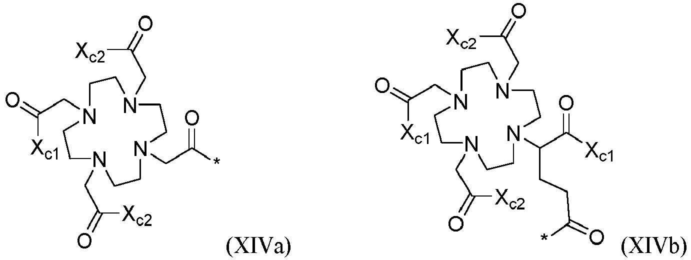 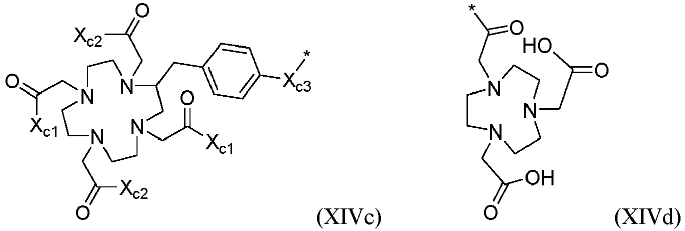 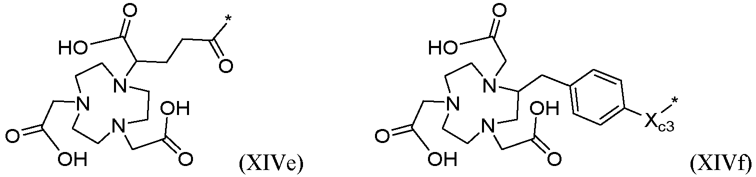 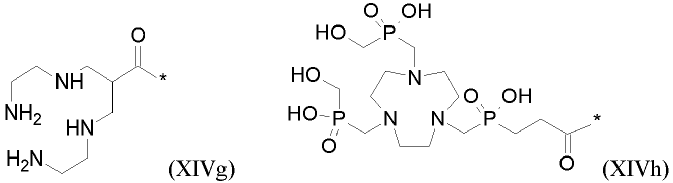 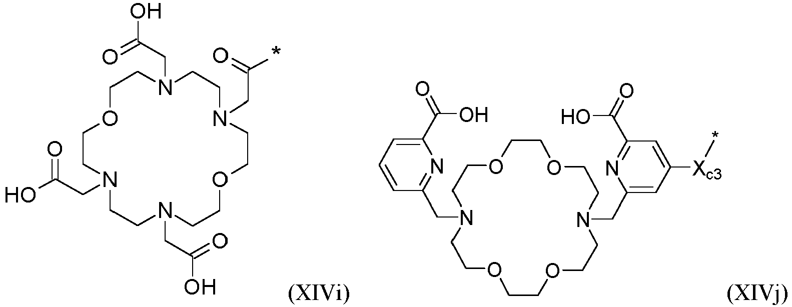 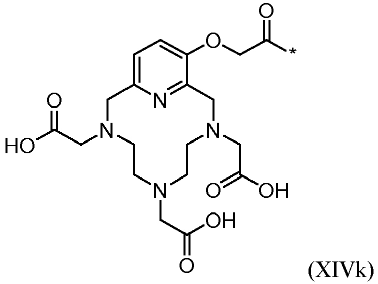 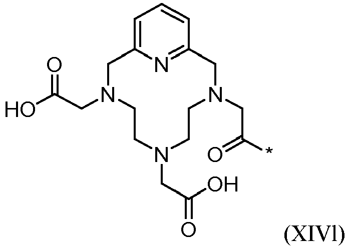 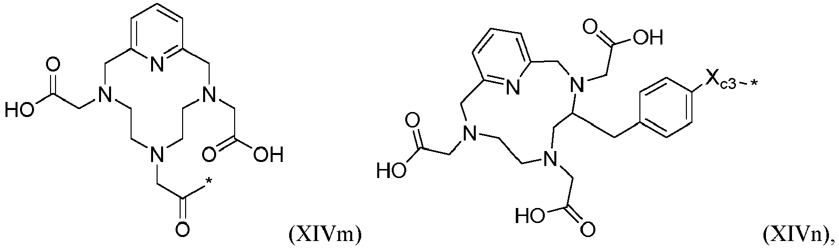 wherein,
   X_{c1} and X_{c2} are each and independently selected from the group consisting of -OH and -NH₂, and
   X_{c3} is independently selected from the group consisting of -NH-* and -N-C(S)-*,
   * indicates covalent attachment to Xaa0, Xaa2, Xaa3 or Xaa7, and
   the residue E optionally comprises a nuclide, preferably a radionuclide.

Preferably, if Z (or E) is covalently attached to an N atom of an amino group of (or comprised in) the side chain of any one of Xaa2, Xaa3 or Xaa7 or of (or comprised in) the main chain of Xaa0, E is a residue represented by any one of the formulae (XIVa) to (XIVn), wherein X_{c3} is -N-C(S)-*; and if Z (or E) is covalently attached to a C atom of a carbonyl group of (or comprised in) the side chain of any of Xaa2, Xaa3 or Xaa7, E is a residue represented by any one of the formulae (XIVc), (XIVf), (XIVj) and (XIVn), wherein X_{c3} is -NH*-.

In a more preferred embodiment, in formula (I), each Z group is independently a moiety represented by the following formula (XV):

E-L-* (XV)

wherein,
* indicates covalent attachment to Xaa0, Xaa2, Xaa3 or the C-terminal carbonyl group of Xaa10, and
   (a)
      E is a residue represented by any one of the following formulae (XVIa) to (XVIn): 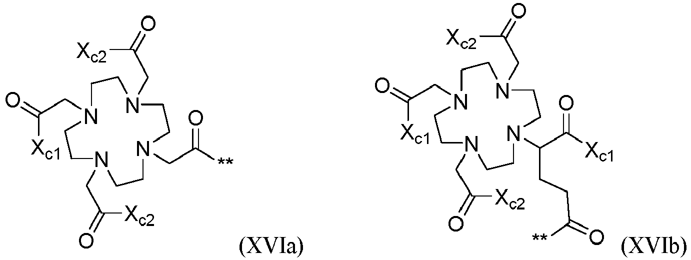 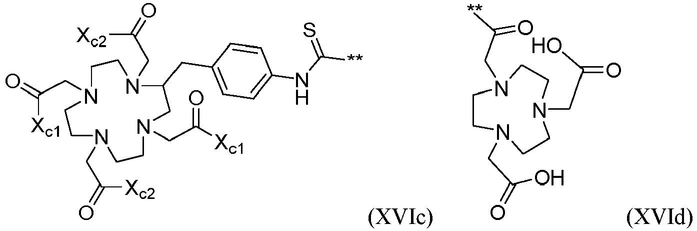 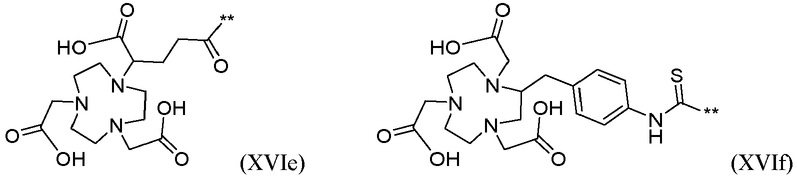 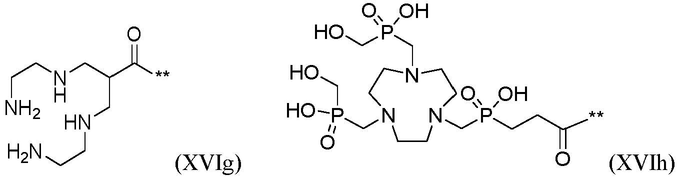 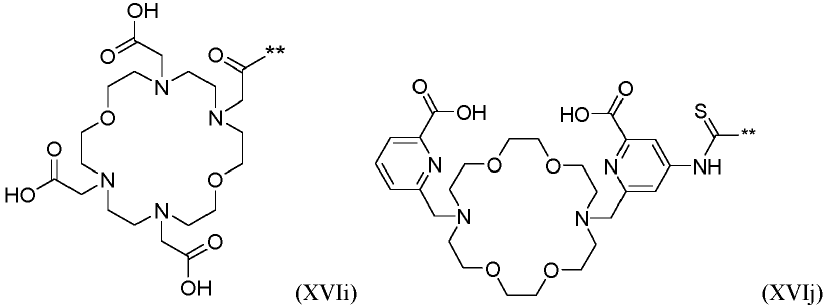 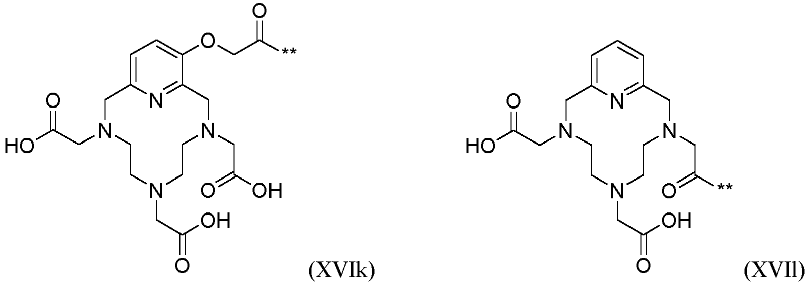 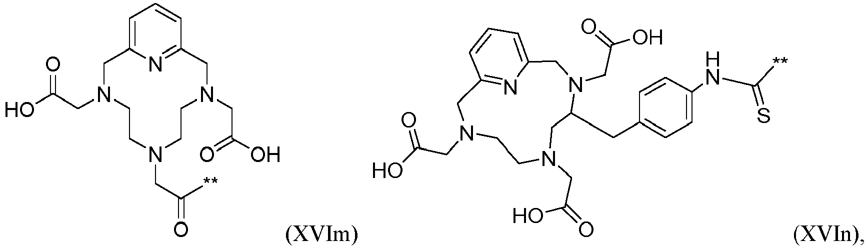 wherein,
         X_{c1} and X_{c2} are each independently selected from the group consisting of -OH and -NH₂, and
         * * indicates covalent attachment to L,
         the residue E optionally comprises a nuclide, preferably a radionuclide, and
      L is a linker moiety represented by any one of the formulae (XVIIa) to (XVIIt): 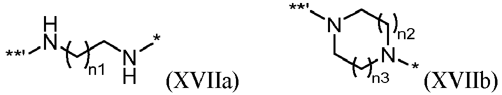 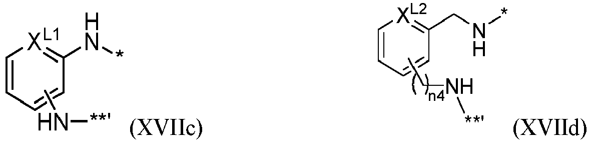 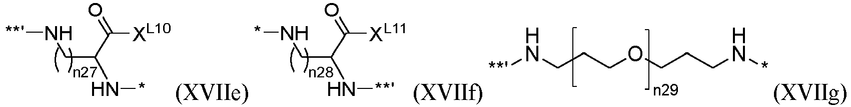 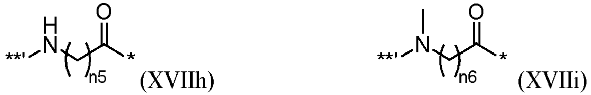 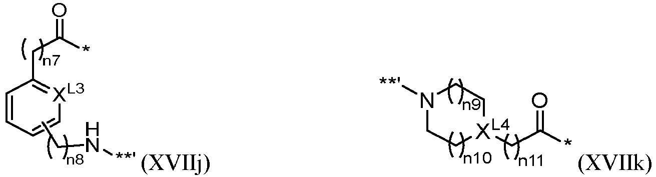 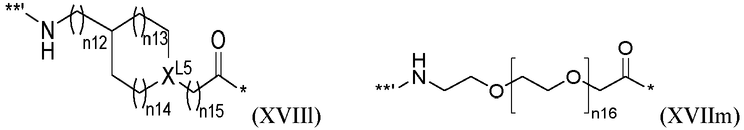 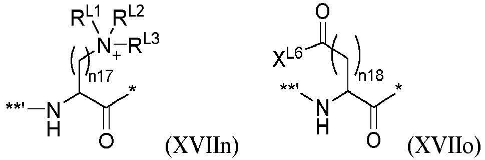 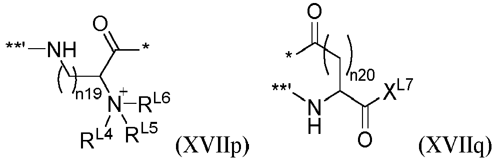 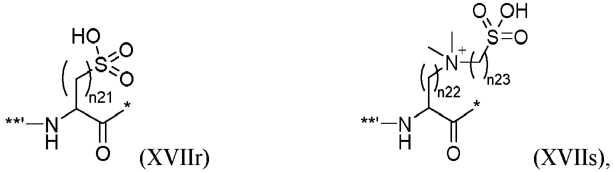 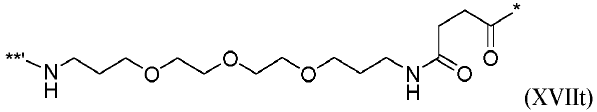 wherein,
         n1 is 1, 2, 3, 4 or to 5,
         n2, n3, n4 and n12 are each and independently 0, 1 or to 2,
         n5, n6 and n29 are each and independently 1, 2, 3, 4, 5, 6, 7, 8, 9 or 10,
         n7 and n8 are each and independently 0, 1, 2, 3 or 4,
         n9, n13, n14, n15, n18, n20 and n21 are each and independently 1 or 2,
         n 10 and n11 are each and independently 0 or 1,
         n16 is 0, 1, 2, 3, 4, 5, 6, 7, 8, 9, 10, 11 or 12,
         n17, n19, n22, n27 and n28 are each and independently 1, 2, 3 or 4,
         n23 is 2, 3 or 4,
         X^{L1}, X^{L2}, X^{L3}, X^{L4} and X^{L5} are each and independently =CH- or =N-,
         X^{L6}, X^{L7}, X^{L10} and X^{L11} are each and independently -OH or -NH₂,
         R^{L1}, R^{L2}, R^{L3}, R^{L4}, R^{L5} and R^{L6} are each and independently -H or a methyl group,
         * indicates covalent attachment to Xaa0, Xaa2, Xaa3 or the C-terminal carbonyl group of Xaa10,
         * *' indicates covalent attachment to E;
      or,
   (b)
      E is a residue represented by the following formula (XVIo) or (XVIr): 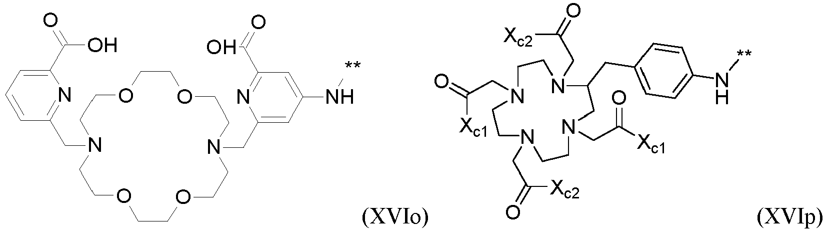 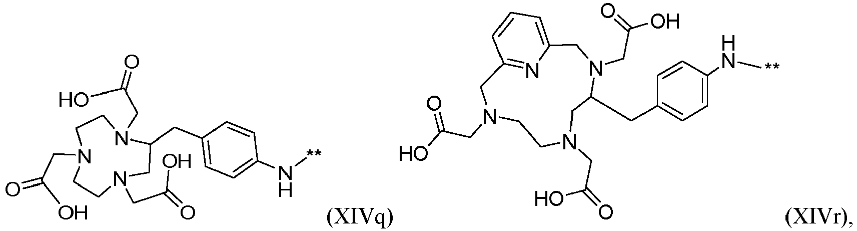 wherein,
         X_{c1} and X_{c2} are each and independently selected from the group consisting of -OH and -NH₂, and
         ** indicates covalent attachment to L, and
         the residue E optionally comprises a nuclide, preferably a radionuclide,
      L is a linker moiety represented by any one of the formulae (XVIIu) to (XVIIy): 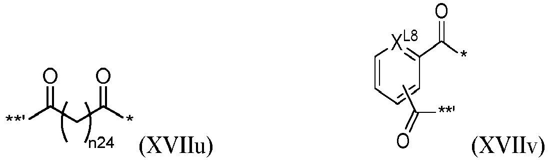 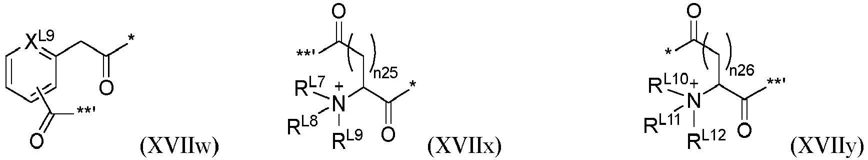 wherein,
         n24 is 1, 2, 3, 4 or 5,
         n25 and n26 are each and independently 1 or 2,
         X^{L8} and X^{L9} are each and independently =CH- or =N-,
         R^{L7}, R^{L8}, R^{L9}, R^{L10}, R^{L12} and R^{L12} are each and independently -H or a methyl group, and
         * indicates covalent attachment to Xaa0, Xaa2 or Xaa3, and
         **' indicates covalent attachment to E.

Preferably, in the above formula (XV), if Z (or L) is covalently attached to a C atom of a carbonyl group of (or comprised in) the side chain of any of Xaa2 or Xaa3 or of (or comprised in) the main chain of Xaa10, L is selected from any one of the formulae (XVIIa) to (XVIIg); and if Z (or L) is covalently attached to an N atom of an amino group of (or comprised in) the main chain (backbone) of Xaa0 or the side chain of any one of Xaa2 or Xaa3, L is selected from any one of the formulae (XVIIh) to (XVIIy).

In a more preferred embodiment, in formula (I), each Z group is independently a moiety represented by the following formula (XV'):

E-L₁-L₂-* (XV')

wherein,
* indicates covalent attachment to a carbonyl group of Xaa2, Xaa3 or the C-terminal carbonyl group of Xaa10, and
E is a residue represented by any one of the following formulae (XVIa) to (XVIn): 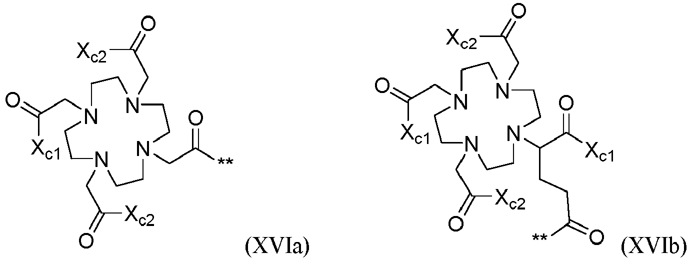 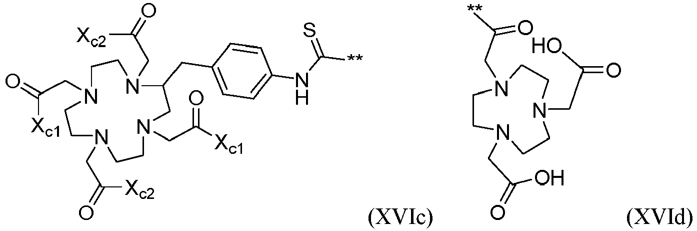 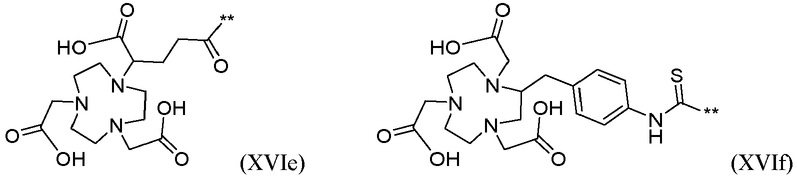 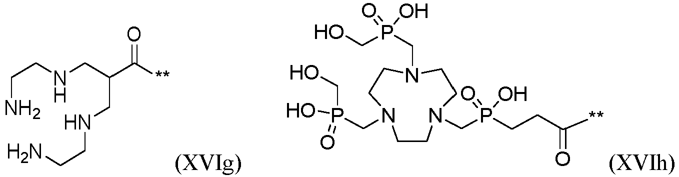 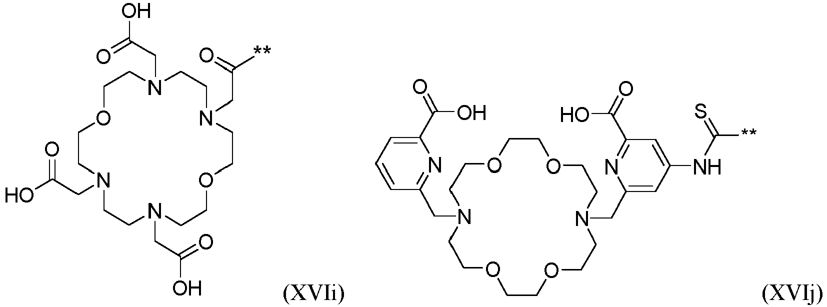 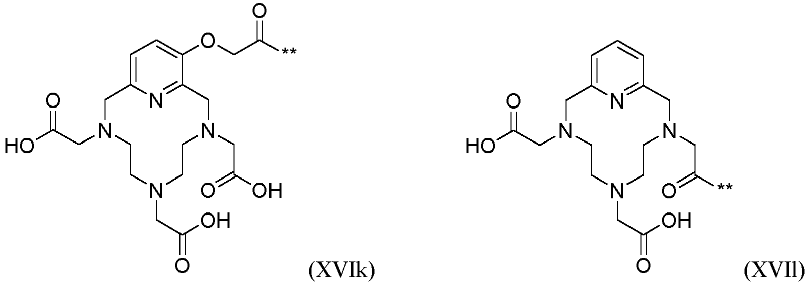 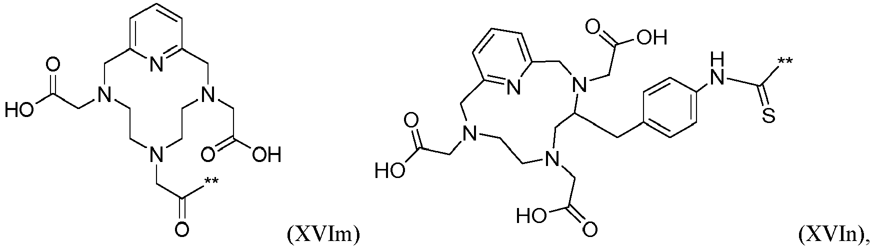 wherein,
   X_{c1} and X_{c2} are each and independently selected from the group consisting of -OH and -NH₂, and
   * * indicates covalent attachment to L₁,
   the residue E optionally comprises a nuclide, preferably a radionuclide, and
L₁ is a linker moiety represented by any one of the formulae (XVII'a) to (XVII'g): 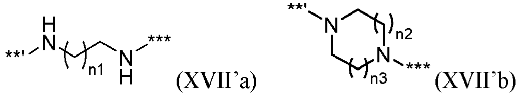 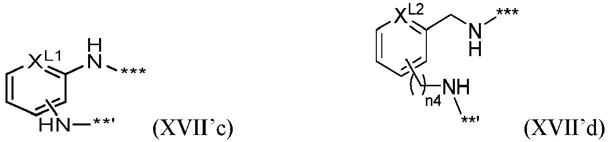 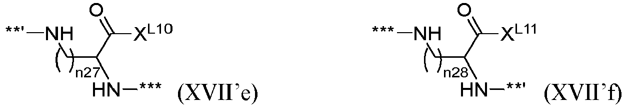 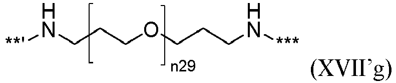 wherein,
   n1 is 1,2, 3, 4 or 5,
   n2, n3 and n4 are each and independently 0, 1 or 2,
   n29 is 1, 2, 3, 4, 5, 6, 7, 8, 9 or 10,
   n27 and n28 are each and independently 1, 2, 3 or 4,
   n23 is 2, 3 or 4,
   X^{L1} and X^{L2} are each and independently =CH- or =N-,
   X^{L10} and X^{L11} are each and independently -OH or -NH₂,
   *** indicates covalent attachment to L₂, and
   * *' indicates covalent attachment to E; and
L₂ is a linker moiety represented by any one of the formulae (XVII'h) to (XVII't): 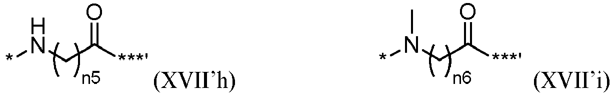 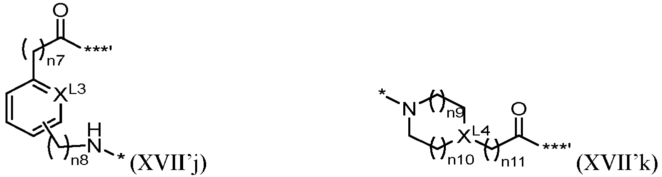 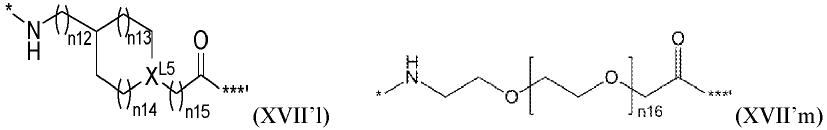 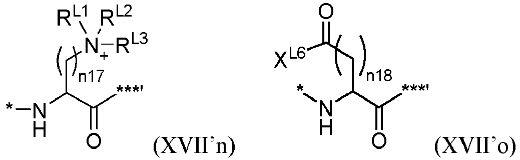 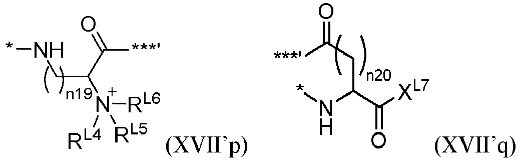 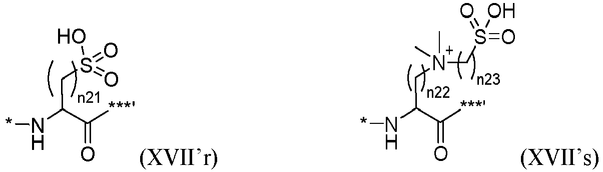 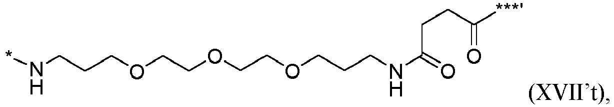 wherein,
   n12 is 0 to 2,
   n5 and n6 are each and independently 1, 2, 3, 4, 5, 6, 7, 8, 9 or 10,
   n7 and n8 are each and independently 0, 1, 2, 3 or 4,
   n9, n13, n14, n15, n18, n20 and n21 are each and independently 1 or 2,
   n 10 and n11 are each and independently 0 or 1,
   n16 is 1, 2, 3, 4, 5, 6, 7, 8, 9, 10, 11 or 12,
   n17, n19 and n22 are each and independently 1, 2, 3 or 4,
   n23 is 2, 3 or to 4,
   X^{L3}, X^{L4} and X^{L5} are each and independently =CH- or =N-,
   X^{L6} and X^{L7} are each and independently -OH or -NH₂,
   R^{L1}, R^{L2}, R^{L3}, R^{L4}, R^{L5} and R^{L6} are each and independently -H or a methyl group,
   * indicates covalent attachment to a carbonyl group of Xaa2, Xaa3 or Xaa10, and
   ***' indicates covalent attachment to L₁.

Preferably, in the above formula (XV'), L₂ is covalently attached to a C atom of a carbonyl group of (or comprised in) the side chain of any of Xaa2 or Xaa3, or of (or comprised in) the main chain of Xaa10.

In a more preferred embodiment, in formula (I), each Z group is independently a moiety represented by the following formula (XV"):

E-L-* (XV")

wherein,
* indicates covalent attachment to a carbonyl group of Xaa2, Xaa3 or Xaa10, and
E is a residue represented by the following formula (XVIo) or (XVIr): 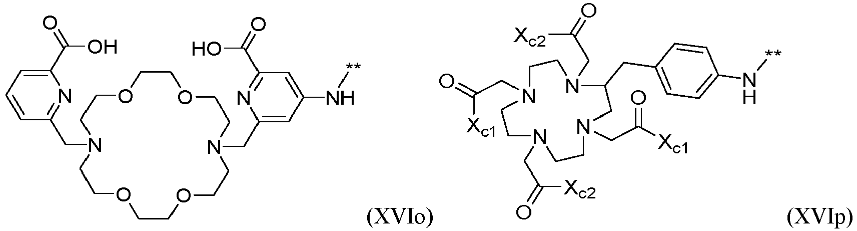 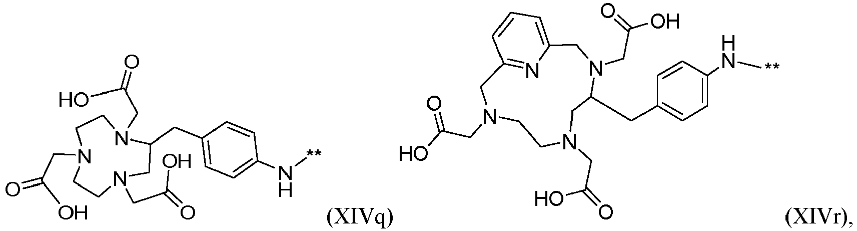 wherein,
   X_{c1} and X_{c2} are each and independently selected from the group consisting of -OH and -NH₂, and
   ** indicates covalent attachment to L, and
   the residue E optionally comprises a nuclide, preferably a radionuclide,
L is a linker moiety represented by any one of the formulae (XVII"h) to (XVII"t): 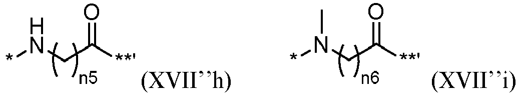 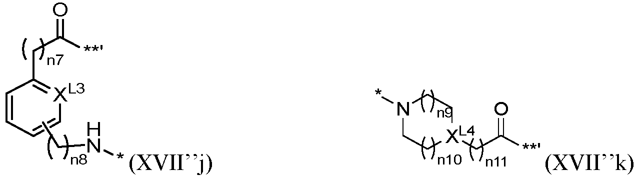 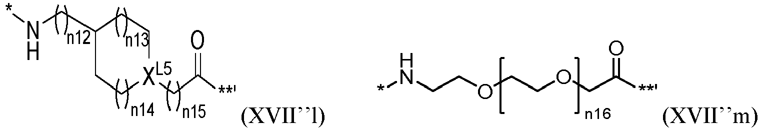 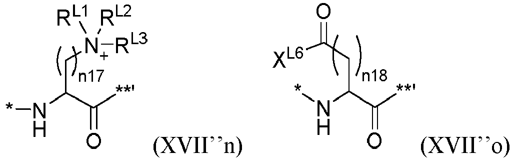 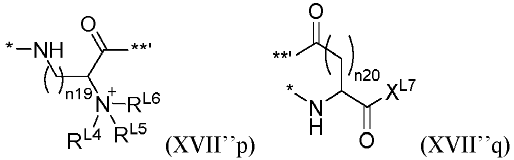 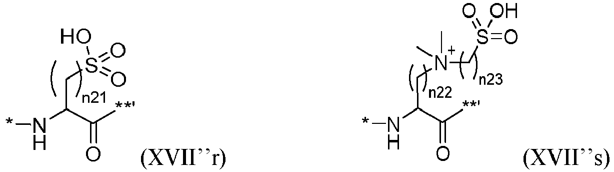 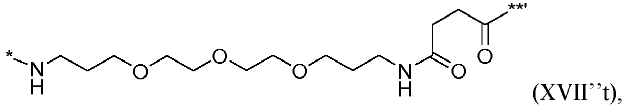 wherein,
   n12 is 0, 1 or 2,
   n5 and n6 are each and independently 1, 2, 3, 4, 5, 6, 7, 8, 9 or 10,
   n7 and n8 are each and independently 0, 1, 2, 3 or 4,
   n9, n13, n14, n15, n18, n20 and n21 are each and independently 1 or 2,
   n 10 and n11 are each and independently 0 or 1,
   n16 is 0, 1, 2, 3, 4, 5, 6, 7, 8, 9, 10,11 or 12,
   n17, n19 and n22 are each and independently 1, 2, 3 or 4,
   n23 is 2, 3 or 4,
   X^{L3}, X^{L4} and X^{L5} are each and independently =CH- or =N-,
   X^{L6} and X^{L7} are each and independently -OH or -NH₂,
   R^{L1}, R^{L2}, R^{L3}, R^{L4}, R^{L5} and R^{L6} are each and independently -H or a methyl group,
   * indicates covalent attachment to a carbonyl group of Xaa2, Xaa3 or Xaa10, and
   **' indicates covalent attachment to L.

Preferably, in the above formula (XV"), L is covalently attached to a C atom of a carbonyl group of (or comprised in) the side chain of any of Xaa2 or Xaa3, or of (or comprised in) the main chain of Xaa10.

Moreover, if the compound of formula (I) contains more than one Z group, each group Z may be independently selected from the moieties of formulae (XIII), (XV), (XV') and (XV") described above.

In the above formulae (XIII), (XV), (XV') and (XV"), the radionuclide, if present, is preferably as described above, and more preferably a diagnostically active radionuclide or a therapeutically active radionuclide (as described above).

In an embodiment of the present invention, it is preferred that the chelator E and the radionuclide comprised therein are as defined in table 9. The left column indicates the chelator and the right columns indicate, for each chelator, the corresponding radionuclides by order of preference.

**Table 9: Overview of preferred chelator-nuclide combinations in the context of the present invention.**

| **Chelator** | **Preferred nuclides** | **More preferred nuclides** | **Most preferred nuclides** |
|---|---|---|---|
| DOTA | ⁴⁴Sc, ⁶⁷Ga, ⁶⁸Ga, ⁸⁶Y, ⁹⁰Y, ¹¹¹In, ¹⁴⁹Tb, ¹⁶¹Tb, ¹⁷⁷Lu, ²⁰³Pb, ²¹²Pb, ²¹³Bi, ²²⁵Ac | ⁴⁴Sc, ⁶⁸Ga, ⁸⁶Y, ⁹⁰Y, ¹¹¹In, ¹⁴⁹Tb, ¹⁶¹Tb, ¹⁷⁷Lu, ²⁰³Pb, ²¹²Pb, ²²⁵Ac | ⁶⁸Ga, ¹¹¹In, ¹⁷⁷Lu, ²²⁵Ac |
| DOTAGA | ⁸⁹Zr, ²²⁵Ac | - | - |
| DOTAM | ²⁰³Pb, ²¹²Pb | - | - |
| Crown | ²⁰³Pb, ²¹²Pb, ²¹³Bi, ²²⁵Ac | ²¹³Bi, ²²⁵Ac | ²²⁵Ac |
| NOTA | Al-¹⁸F, ⁶⁴Cu, ⁶⁷Cu, ⁶⁷Ga, ⁶⁸Ga | Al-¹⁸F, ^{6a}Cu ⁶⁷Cu, ⁶⁸Ga | Al-¹⁸F |
| NODAGA | ⁶⁴Cu, ⁶⁷Cu, ⁶⁷Ga, ⁶⁸Ga | ⁶⁴Cu, ⁶⁷Cu, ⁶⁸Ga | ⁶⁸Ga |
| NODA-MPAA | Al-¹⁸F | - | - |
| CB-TE2A | ⁶⁴Cu, ⁶⁷Cu | - | - |
| CHX-A"-DTPA | ⁸⁶Y, ⁹⁰Y, ¹¹¹In, ¹⁴⁹Tb, ¹⁶¹Tb, ¹⁷⁷Lu | - | - |
| DFO | ⁸⁹Zr | | |
| Macropa | ²⁰³Pb, ²¹²Pb, ²¹³Bi, ²²⁵Ac | ²¹³Bi, ²²⁵Ac | ²²⁵Ac |
| HOPO ligand platform^{†} | ⁶⁸Ga, ⁸⁹Zr, ²²⁷Th | ⁸⁹Zr, ²²⁷Th | ²²⁷Th |
| THP | ⁶⁷Ga, ⁶⁸Ga | ⁶⁸Ga | - |
| AAZTA | ⁴⁴Sc, ⁸⁶Y, ⁹⁰Y, ¹⁴⁹Tb, ¹⁶¹Tb, ¹⁷⁷Lu | ¹⁷⁷Lu | - |
| NOPO | Al-¹⁸F, ⁶⁷Ga, ⁶⁸Ga | ⁶⁸Ga | - |
| PCTA | ⁶⁷Ga, ⁶⁸Ga, ¹⁷⁷Lu | ⁶⁸Ga, ¹⁷⁷Lu | - |
| PSC | ¹¹¹In, ²⁰³Pb, ²¹²Pb, ²¹³Bi | ²⁰³Pb, ²¹²Pb | - |
| Sarcophagine | ⁶⁴Cu, ⁶⁷Cu | - | - |
| NETA | ²¹³Bi | - | - |
| H₄CHXoctapa | ¹¹¹In, ¹⁷⁷Lu, ²²⁵Ac | ¹¹¹In, ¹⁷⁷Lu | - |
| H₄pypa | ⁴⁴Sc, ⁸⁹Zr, ¹¹¹In, ¹⁷⁷Lu | ⁴⁴Sc, ¹¹¹In, ¹⁷⁷Lu | - |
| N4 | ^{94m}Tc, ^{99m}Tc, ¹⁸⁶Re, ¹⁸⁸Re | ^{99m}Tc | - |

| | | | |
|---|---|---|---|
| ^{†} as disclosed in (Zhou, Dong et al. 2021) | | | |

In an embodiment, the compound of the invention and disclosure, respectively, comprise a nuclide which is bound, preferably coordinatively bound, by a chelator forming part of the compound. It will be acknowledged and appreciated by a person skilled in the art that the coordination geometry of such complex of the nuclide and the chelator may vary. Embodiments of the structure, coordination geometry and overall complex charge for various exemplary chelate complexes are shown in Table 10.

**Table 10: Selected chelate complexes with structure, coordination geometry and overall complex charge (coordinate bonds between metal center and ligand are shown as dotted lines).**

| **Nuclide** | **Chelator** | **Structure** | **Donor sphere (total coordination number)** | **Coordination geometry (nuclide - chelator)** | **Complex charge** |
|---|---|---|---|---|---|
| Al(III)-F | NOTA | 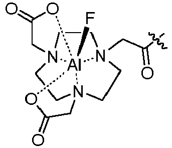 | N₃O₂ (6) | distorted octahedral^{h} | neutral |
| Cu(II) | DOTA | 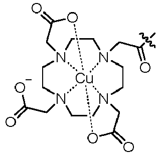 | N₄O₂ (6) | distorted octahedral^{a} | monoanionic (-1) |
| | NODAGA | 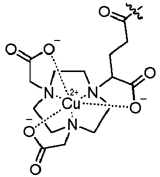 | N₃O₃ (6) | distorted trigonal prismatic^{a} | monoanionic (-1) |
| Ga(III) | DOTA | 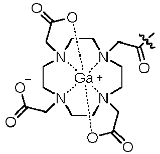 | N₄O₂ (6) | distorted octahedral^{a} | neutral |
| | NODAGA | 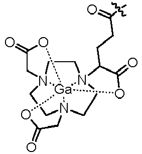 | N₃O₃ (6) | distorted octahedral^{a} | neutral |
| Y(III) | DOTA | 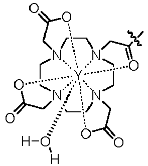 | N₄O₄ (9)^{c} | monocapped square antiprismatic^{a} | neutral |
| In(III) | DOTA | 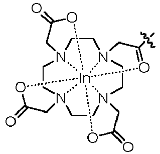 | N₄O₄ (8) | twisted square antiprismatic^{f} | neutral |
| | PSC | 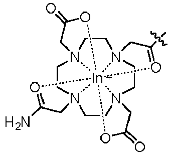 | N₄O₄ (-) | - | mono-cationic (+1) |
| Tb(III) | DOTA | 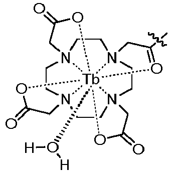 | N₄O₄ (9)^{c} | monocapped square antiprismatic^{d} | neutral |
| Lu(III) | DOTA | 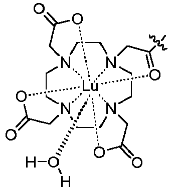 | N₄O₄ (9)^{c} | monocapped square antiprismatic^{b} | neutral |
| Pb(II) | DOTA | 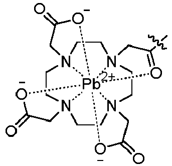 | N₄O₄ (8) | twisted square prismatic^{e} | monoanionic (-1) |
| | DOTAM | 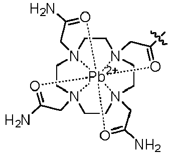 | N₄O₄ (8/9)^{g} | twisted square antiprismatic^{e} | di-cationic (+2) |
| | PSC | 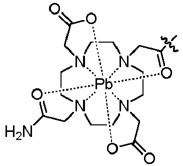 | N₄O₄ (-) | - | neutral |
| Ac(III) | DOTA | 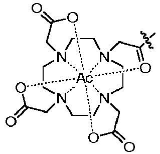 | N₄O₄ (-) | capped inverted square antiprismatic^{e} | neutral |
| | Macropa | 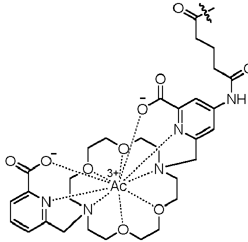 | N₄O₆ (-) | irregular tridecahedron^{e} | mono-cationic (+1) |

| | | | | | |
|---|---|---|---|---|---|
| a) (Wadas, Wong et al. 2010) b) (Aime, Barge et al. 1996) c) The ligand DOTA acts as an octadentate ligand. To saturate the coordination sphere of the metal center, an additional (monodentate) ligand occupies the ninth coordination side. In aqueous solution, the additional ligand is usually a water molecule but can vary depending on the chemical composition of the environment. Exemplarily, the adjoining structure shows a water molecule as additional ligand. d) (Shiells, Natrajan et al. 2011) e) (Grieve and Paterson 2021) f) (Liu, He et al. 2003) g) Two crystal structures of the Pb²⁺ complex of DOTAM have been published. The structures both showed the eight donor atoms encapsulating the ion but differed by the presence or absence of a water molecule weakly interacting with the Pb²⁺ ion (Grieve and Paterson 2021). h) (Archibald and Allott 2021) | | | | | |

In an even more preferred embodiment, in formula (I), each Z group is independently a moiety represented by any one of the following formulae (XVIIIa) to (XVIIIn): 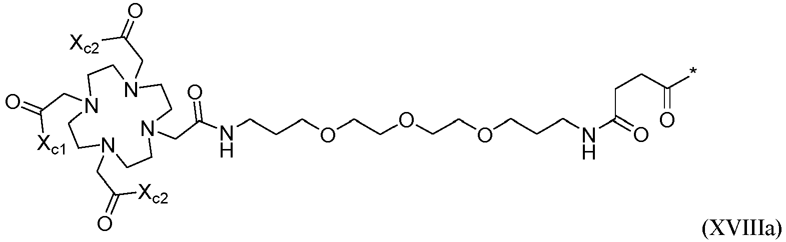 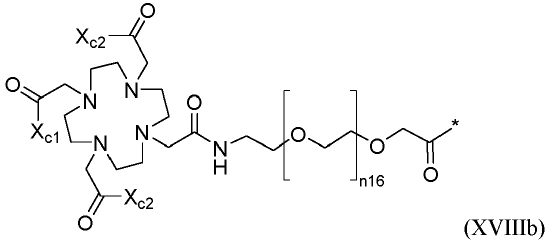 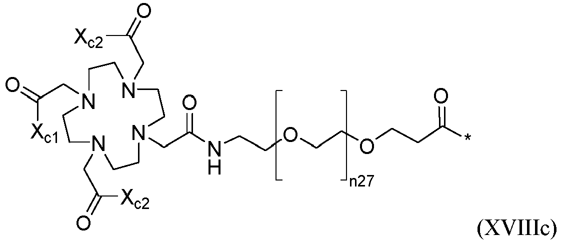 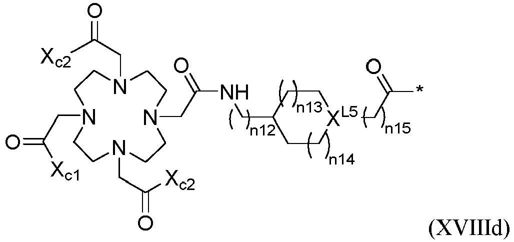 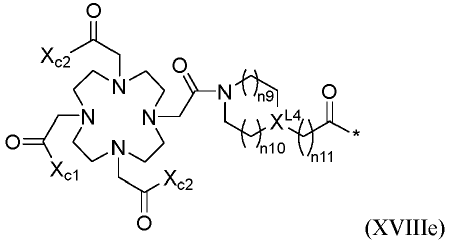 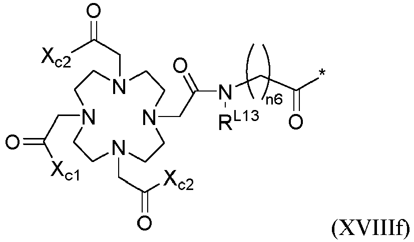 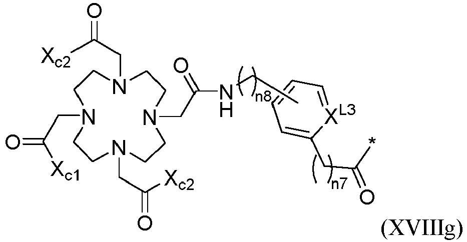 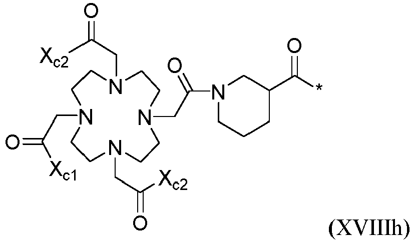 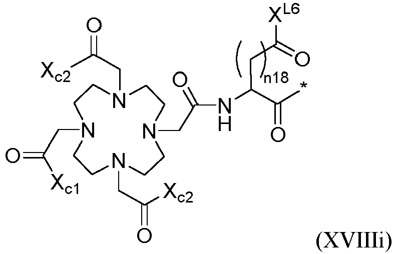 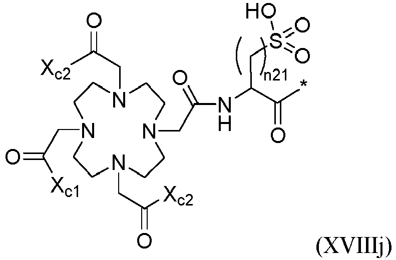 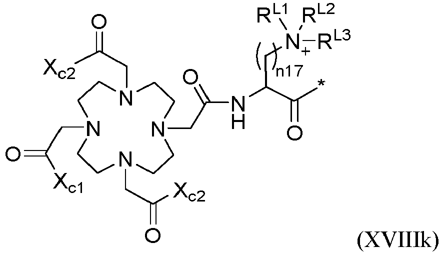 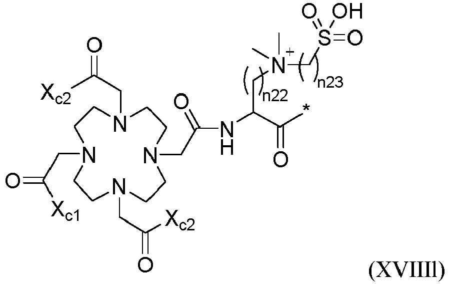 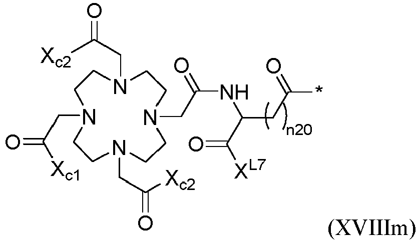 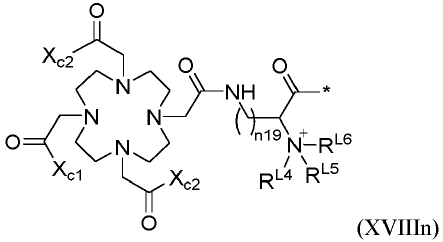 wherein,
X_{c1}, X_{c2}, X^{L6} and X^{L7} are each and independently selected from the group consisting of -OH and -NH₂, and
n6 is 1, 2, 3, 4, 5, 6, 7, 8, 9 or 10, and
n7 and n8 are each and independently 0, 1, 2,3 or 4,
n9, n13, n14, n15, n18, n20 and n21 are each and independently 1 or 2, and
n10 and n11 are each and independently 0 or 1, and
n12 is 0, 1 or 2,
n16 and n27 are each and independently 0, 1, 2, 3, 4, 5, 6, 7, 8, 9, 10, 11 or 12, and
n17, n19 and n22 are each and independently 1, 2, 3 or 4, and
n23 is 2, 3 or 4, and
X^{L3}, X^{L4} and X^{L5} are each and independently =N- or =CH-, and
R^{L1}, R^{L2}, R^{L3}, R^{L4}, R^{L5}, R^{L6} and R^{L13} are each and independently -H or a methyl group, and
* indicates covalent attachment to Xaa0, Xaa2 or Xaa3, and
the moiety represented by any one of the above formulae (XVIIIa) to (XVIIIn) optionally comprises a radionuclide, the radionuclide being preferably selected from the group consisting of Al¹⁸F, ⁴³Sc, ⁴⁴Sc, ⁴⁷Sc, ⁵¹Mn, ⁵²Mn, ⁶⁴Cu, ⁶⁴Cu, ⁶⁷Cu, ⁶⁷Ga, ⁶⁸Ga, ⁸⁶Y, ⁸⁹Zr, ⁹⁰Y, ¹¹¹In, ¹⁴⁹Tb, ¹⁵²Tb, ¹⁵³Sm, ¹⁵⁵Tb, ¹⁶¹Tb, ¹⁷⁷Lu, ²⁰Tl , ²⁰³Pb, ²¹²Pb, ²¹³Bi, ²²⁵Ac, ²²⁶Th and ²²⁷Th.

In a still more preferred embodiment, in formula (I), each Z group is independently a moiety represented by any one of the following formulae (XVIIIa) to (XVIIIv): 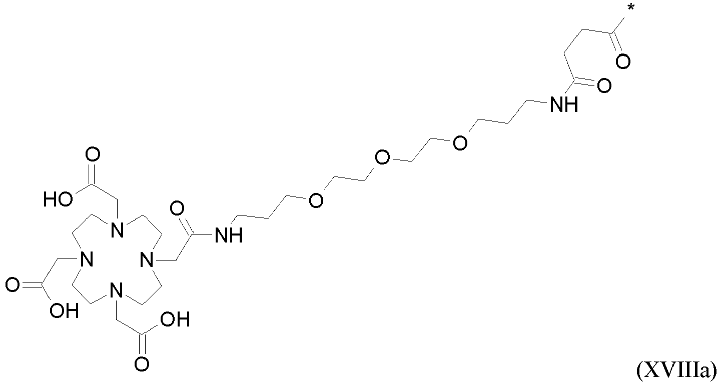 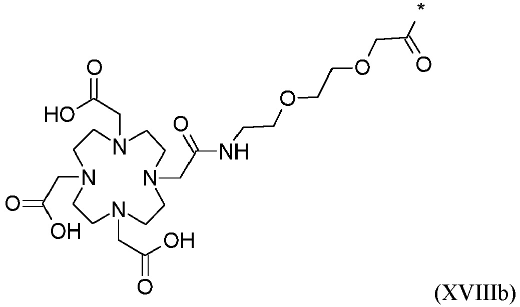 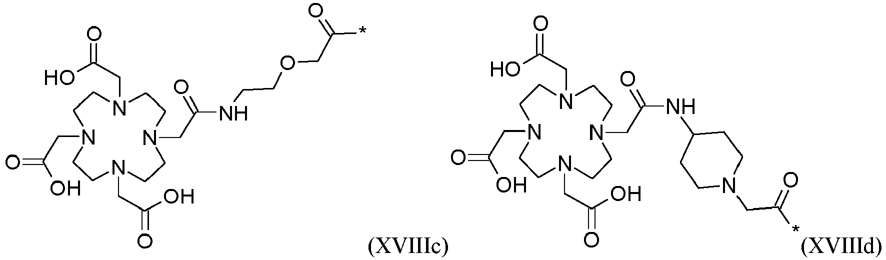 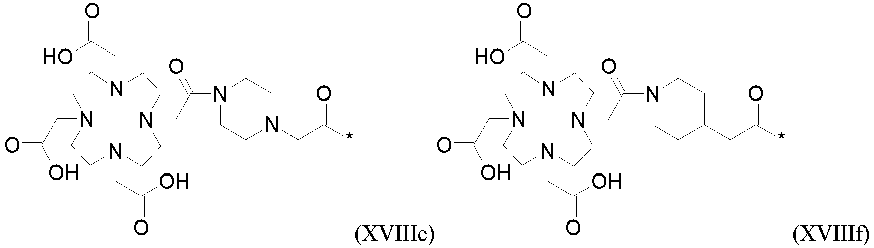 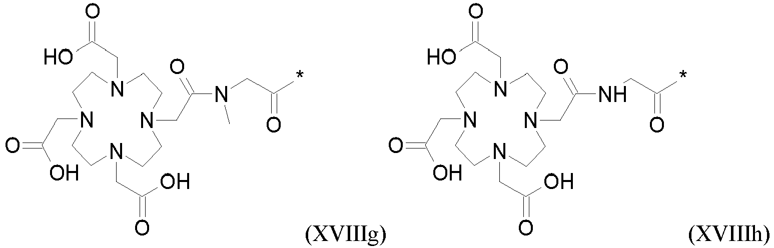 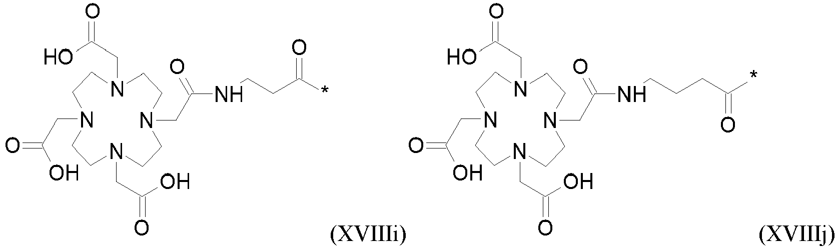 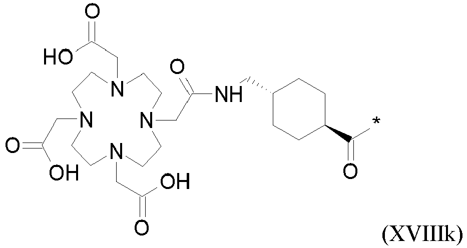 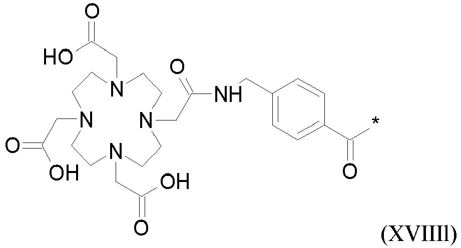 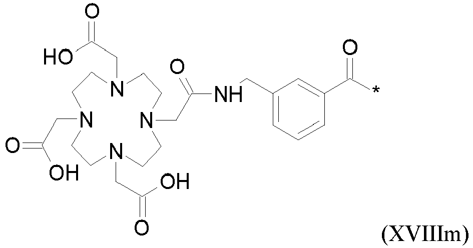 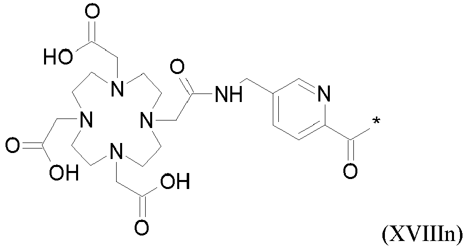 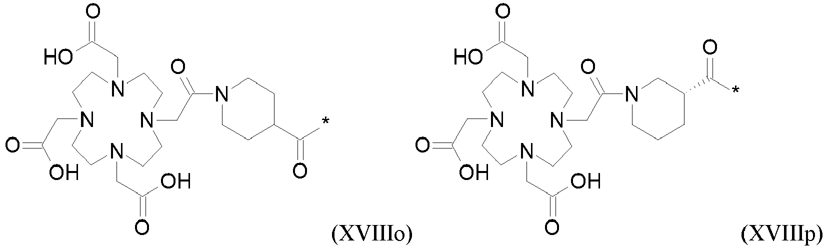 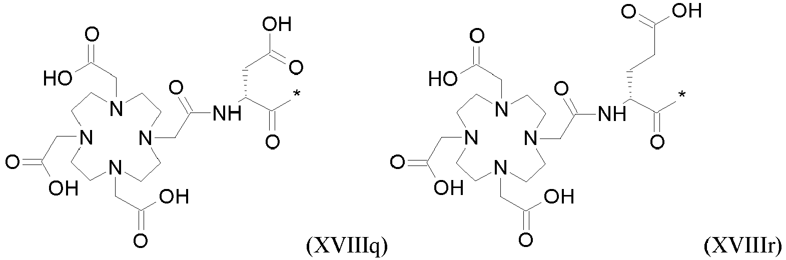 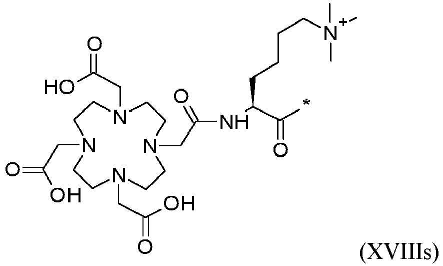 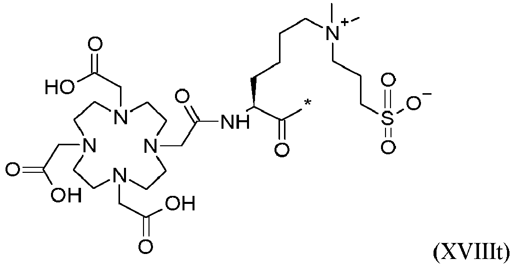 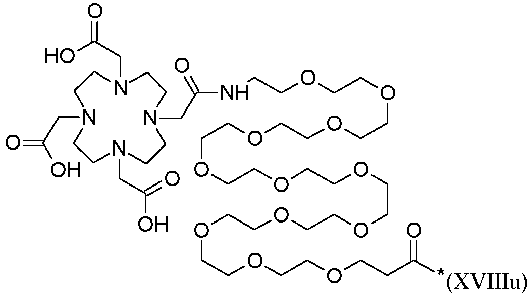 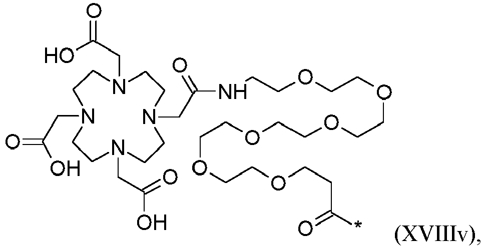 wherein,
* indicates covalent attachment to Xaa1, Xaa3, Xaa5, Xaa6, Xaa7, Xaa8, Xaa9, Xaa10 or Xaa11, and
the moiety represented by any one of the above formulae (XVIIIa) to (XVIIIv) optionally comprises a radionuclide, the radionuclide being preferably selected from the group consisting of Al¹⁸F, ⁴³Sc, ⁴⁴Sc, ⁴⁷Sc, ⁵¹Mn, ⁵²Mn, ⁶⁴Cu, ⁶⁴Cu, ⁶⁷Cu, ⁶⁷Ga, ⁶⁸Ga, ⁸⁶Y, ⁸⁹Zr, ⁹⁰Y, ¹¹¹In, ¹⁴⁹Tb, ¹⁵²Tb, ¹⁵³Sm, ¹⁵⁵Tb, ¹⁶¹Tb, ¹⁷⁷Lu, ²⁰¹Tl, ²⁰³Pb, ²¹²Pb, ²¹³Bi, ²²⁵Ac, ²²⁶Th and ²²⁷Th.

In an embodiment, the compound of the invention comprises one or more bio-distribution modifier(s), wherein the bio-distribution modifier(s) is (are) either directly or indirectly (i.e. through a linker) attached to the compound of the invention. Preferably, no dedicated bio-distribution modifier as defined herein is part of the compound of the invention.

The function of a bio-distribution can be the attenuation of the unintended or undesired bio-distribution of the compound of the invention by altering physio-chemical properties of the compound of the invention. To that end, elements with a multitude of hydroxyl groups are implemented within or are attached to the peptide of the invention. The function of a bio-distribution can be the alteration of serum half-life of the compound by modulation of the renal clearance (Du, Jiang et al. 2020). The function of a bio-distribution can be the modulation of serum half-life of the compound of the invention by binding to serum components (Lorenz, Evers et al. 2013).

In an embodiment, an amino-oligo-alcohol represents the building block for a bio-distribution modifier. The term oligo means that building block may contain 1 to 7 hydroxyl groups. Within the amino-oligo-alcohol the amino group is the functional group for the attachment to the compound of the invention. Usually the amino-oligo-alcohol is linked to the compound of the invention via a dicarboxylic acid linker (such as glutaric acid) or a tricarboxylic acid (such as nitrilotriacetic acid) or an alpha amino acid linker (such as glutamic acid). Preferred amino-oligo-alcohol building blocks comply with formulae (bio00) or (bio01).

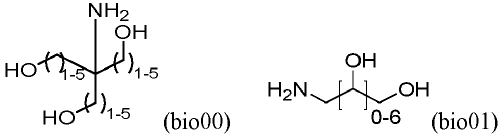

More preferred amino-oligo-alcohol building blocks such as of formula (bio01) are 3-Amino-propane-1,2-diol, 4-Amino-butane-1,2,3-triol, 5-Amino-pentane-1,2,3,4-tetraol, 6-Amino-hexane-1,2,3,4,5-pentaol and 1-amino-1-deoxy-D-glucitol or tris(hydroxymethyl)aminomethane (*Tris)* of formula (bio00). The most preferred amino-oligo-alcohol building block of formula (bio00) is Tris and the most preferred amino-oligo-alcohol building block of formula (bio01) is 1-amino-1-deoxy-D-glucitol (AGLU).

In an embodiment, conjugates consisting of two amino-oligo-alcohol building blocks such as of formulae (bio01) or (bio02) attached to the carboxylic acid moieties of an alpha amino acid linker such as glutamic acid, represents the building block for a bio-distribution modifier. The resulting construct is connected to the compound of the invention by dicarboxylic acid linker. Preferably, said dicarboxylic acid linker is glutaric acid. A preferred conjugate containing two amino-oligo-alcohol building blocks is a structure of formula (bio02)

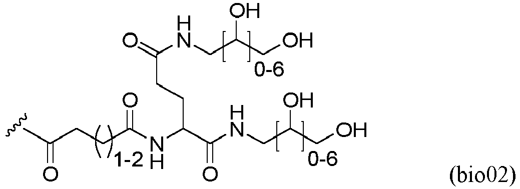

In an embodiment, an inosamine (1-amino-1-deoxy-inositol) represents the building block for a bio-distribution modifier. Within the inosamine, the amino function is the conjugation group for the attachment to the compound of the invention. Usually, the inosamine is linked to the compound of the invention via a dicarboxylic acid linker (such as glutaric acid) or an alpha amino acid linker (such as glutamic acid). Preferred inosamine building blocks comply with formulae (bio03), a more preferred inosamine building block is *scyllo*-inosamine (bio04).

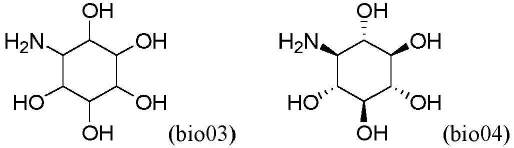

In an embodiment, a glyosylated building block represents the building block for a bio-distribution modifier. Preferred glycosyl building blocks comply with formula (bio05).

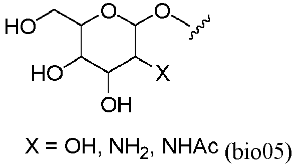

More preferred glycosyl building blocks are glucose, galactose, mannose, glucosamine, galactosamine, mannosamine, N-acetyl glucosamine, N-acetyl galactosamine and N-acetyl mannosamine.

The sugar moiety is attached to the side chain of an amino acid to from an O- or N-glycosylation as shown in formulae (bio06) and (bio07) to form conjugates which can be employed to attach the glycosyl moiety to the compound of the invention.

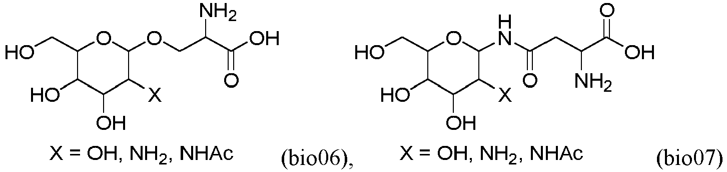

It will be appreciated by a person skilled in the art that the amino acids in formulae (bio06) and (bio07) can be replaced by omega-hydroxy carbonic acids and di-carbonic acids, respectively. The resulting conjugates can be employed to attach the glycosyl moiety to compound of the invention likewise.

Furthermore, any suitable form of neo-glycosylation (Walther and Zelikin 2021) for example such as the attachment of the glycosyl residue via a thioether linkage and or via a triazole linkage may employed to attach a glycosyl moiety to the compound of the invention.

In an embodiment, a linear monodisperse polyethylene glycol (PEG), represents the building block for a bio-distribution modifier. PEG is derived from ethylene glycol. PEGs with a precisely defined number of ethylene glycol units, which is also termed monodisperse, are preferred as building blocks for PEGylation as bio-distribution modifiers for the present invention. As a non-limiting example formula (bio08) is shown as a building block for a bio-distribution modifier.

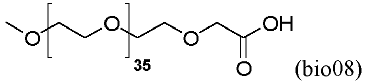

The carboxyl group in (bio08) is attached covalently to the amino group at the N-terminus of the compound of the invention or to an amino group comprised in the side chain of an amino acid residue of the compound of the invention. A linker may be interspersed between the monodisperse PEG building block and the compound of the invention.

In an embodiment, a linear polydisperse polyethylene glycol (PEG), represents the building block for a bio-distribution modifier. The synthesis of monodisperse PEGs is rather laborious and hence expensive. Therefore, usually also polydisperse PEGs are employed for the purpose of PEGylation, which means that the polydisperse PEG of a certain molecular weight contains a mixture of different PEGs with a discrete distribution numbers of polyethylene glycol units. For example, a polydiserse PEG with an average molecular weight of 2000 Da consists of a discrete mixture of PEG molecules ranging from about 40 ethylene glycol units to about 55 ethylene glycol units. In the present invention, also polydisperse PEGs can be employed as building blocks for a bio-distribution modifier.

Polydisperse PEG building blocks used as bio-distribution modifier have an average molecular weight of about 2000 Da (40 - 55 ethylen glycol units), about 5000 Da (95 - 135 ethylen glycol units), about 10000 Da (195 - 265 ethylen glycol units) and about 20000 Da (380 - 540 ethylene glycol units). As non-limiting examples formulae (bio09) - (biol2) are shown as building blocks for a bio-distribution modifier.

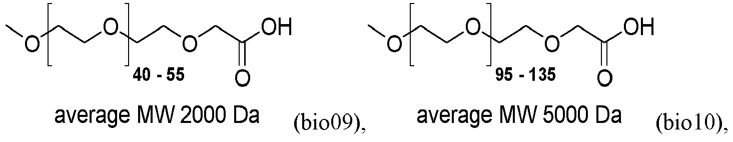

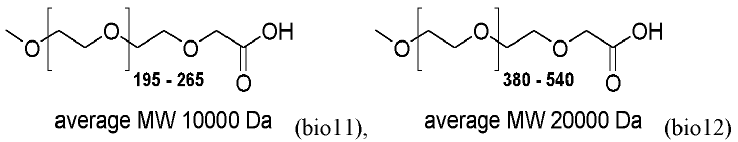

The carboxyl group in structure (bio08) is attached covalently to the amino group at the N-terminus of the compound of the invention or to an amino group comprised in the side chain of an amino acid residue of the compound of the invention. A linker may be interspersed between the monodisperse PEG building block and the compound of the invention.

In an embodiment, a branched polyethylene glycol (PEG), represents the building block for a bio-distribution modifier. The branched PEG may consist of 2 or 3 linear PEG branches/arms/chains. The linear PEG branches/arms/chains implemented in the branched PEG can be monodisperse or polydispers. The individual linear PEG branches/arms/chains implemented in the branched PEG are usually equal. Exemplary branching elements structures are shown in bold in formulae (bio13) to (bio16).

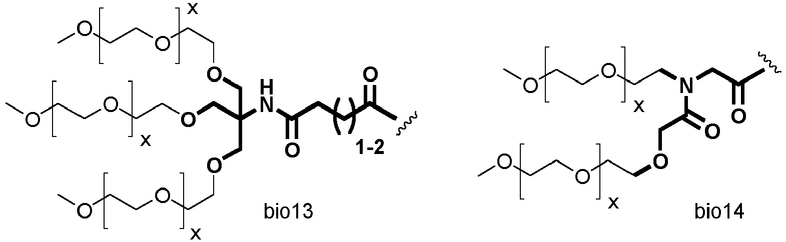

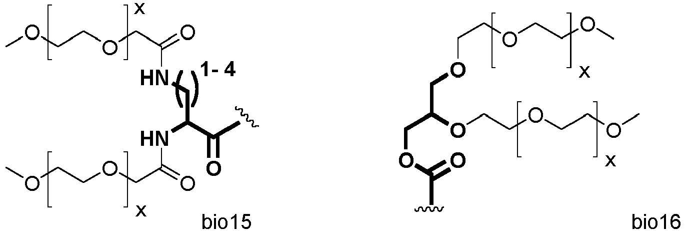

In an embodiment, a carboxylic acid with a long aliphatic chain or an α,ω-dicarboxylic acid with a long aliphatic chain separating both of the carboxylic acid moieties within said building block, represents a building block for a bio-distribution modifier, which is covalently attached to the compound of the invention. In case a carboxylic acid is the building block for a bio-distribution modifier, the carboxyl group is attached covalently to the amino group at the N-terminus of the compound of the invention or to an amino group comprised in the side chain of an amino acid residue of the compound of the invention. In case a dicarboxylic acid is the building block for a bio-distribution modifier, one of the two carboxyl groups is attached to the amino group at the N-terminus of the compound of the invention or to an amino group comprised in the side chain of an amino acid residue of the compound of the invention. A linker may be interspersed between either of the carboxylic acid or the dicarboxylic acid and the compound of the invention.

Preferred carboxylic acid building blocks comply with formula (bio17) and preferred carboxylic dicarboxylic acids comply with formula (bio18).

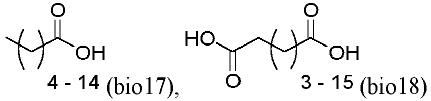

More preferred carboxylic acid building blocks such as formula (bio17) are hexanoic acid, octanoic acid, decanoic acid, lauric acid, myristic acid and palmitic acid. More preferred dicarboxylic acid building blocks such as formula (bio18) are suberic acid, sebacic acid, dodecanedioic acid, tetradecanedioic acid, hexadecanedioic acid and octadecanedioic acid.

In a further embodiment, a glutamic acid is interspersed between either the carboxylic acid or the dicarboxylic acid as shown in formulae (bio19) and (bio20).

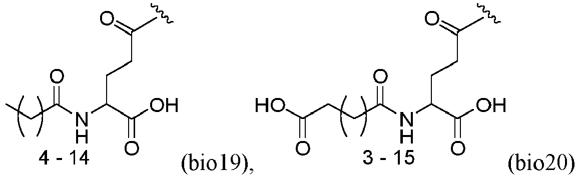

In case a glutamic acid is included in the bio-distribution modifier, a linker may be interspersed between the peptide of invention and the glutamic acid.

In an embodiment, the bio-distribution modifier is a molecular structure comprising at least one alkyl group comprising two or more primary hydroxyl groups, a linking chemical moiety and an attachment moiety, whereby the attachment moiety is, depending on the position of the bio-distribution modifier within the compound of the invention, either a carboxylic acid or an amino acid comprising a side chain. The attachment moiety covalently links the bio-distribution modifier to the intended position within a compound of the invention; the attachment moiety is covalently linked to the linking chemical moiety; and the linking chemical moiety is covalently linked to the at least one alkyl group comprising two or more primary hydroxyl groups. Without being bound by any theory, the function of the primary hydroxyl groups is the increased interaction with water molecules in the surrounding of the compound of the invention, thereby attenuating hydrophobic properties of the compound. It is within the present invention, that there are at least two types of alkyl groups comprising two or more primary hydroxyl groups, which are categorized depending on their position and/or topology of attachment within the bio-distribution modifier: A first type is a first level alkyl group, and a second type is a second level alky group. A first level alkyl group is covalently attached to the linking chemical moiety of the bio-distribution modifier. A second level alkyl group is covalently attached to a first level alkyl group via an ether linkage.

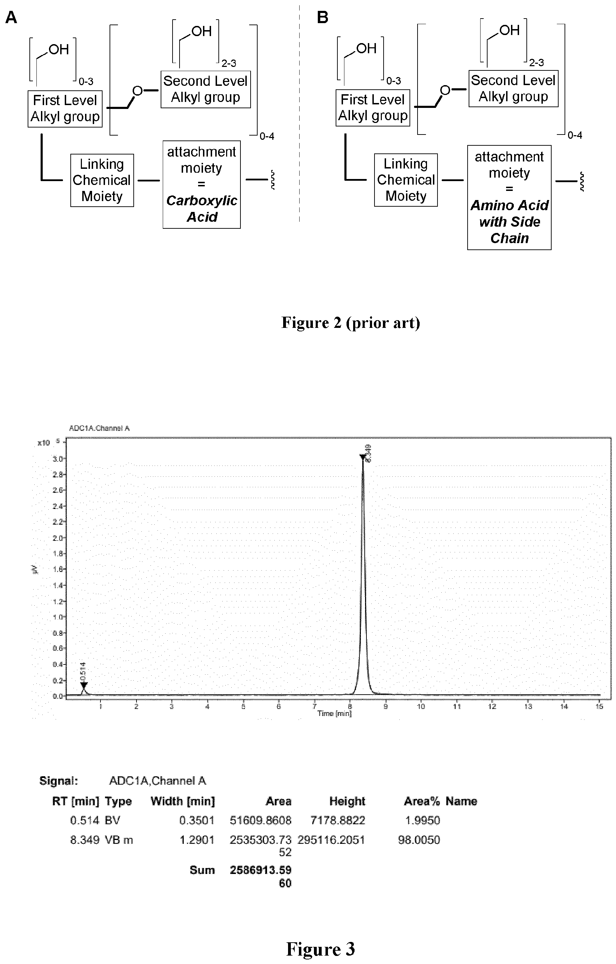

In an embodiment the linking chemical moiety, which is covalently attached to the attachment moiety, which is either a carboxylic acid or an amino acid with a side chain is preferably selected from the group comprising a nitrogen atom, a triazolyl moiety, a hydroxymethyl-triazolyl moiety and a urea moiety. The linking chemical moiety, depicted in Fig. 2, also serves to form a linkage of the carboxylic acid to one or two first level alkyl groups. The number of first level alkyl groups depends on the chemical nature of the linking chemical moiety. To a nitrogen atom one or two first level alkyl groups can be attached. To a triazolyl moiety, a hydroxymethyl-triazolyl moiety or a urea moiety one first level alkyl group can be attached. A first level alkyl group is a (C₃-C₅)alkyl group, preferably a first level alkyl group is a structure selected from the group comprising Alkyl-1, Alkyl-2, Alkyl-3 and Alkyl-4, with Alkyl-1 being as follows 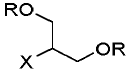 (Alkyl-1), Alkyl-2 being as follows 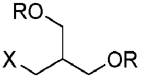 (Alkyl-2), Alkyl-3 being as follows 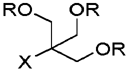 (Alkyl-3) and Alkyl-4 being as follows 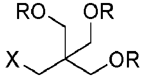 (Alkyl-4). In each and any of first level alkyl groups Alkyl-1, Alkyl-2, Alkyl-3 and Alkyl-4, 'X' indicates the linking chemical moiety. In each and any of Alkyl-1 and Alkyl-2, each and any R is either a hydrogen atom, thereby forming a primary hydroxyl group, or a second level alkyl group. The second level alkyl group is a (C₃-C₅)alkyl group, preferably the second level alkyl group is selected from the group comprising Alkyl-5, Alkyl-6, Alkyl-7 and Alkyl-8, more preferably the second level alkyl group is selected from Alkyl-5 and Alkyl-6, with Akyl-5 being as follows 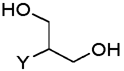 (Alkyl-5), Akyl-6 being as follows 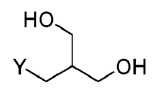 (Alkyl-6), Akyl-7 being as follows 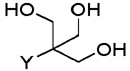 (Alkyl-7) and Akyl-8 being as follows 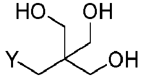 (Alkyl-8). If in Alkyl-1 and Alkyl-2 R is a second level alkyl group, both R in Alkyl-1 and Alkyl-2, respectively, are preferably identical. In each and any of Alkyl-3 and Alkyl-4, each and any R is a hydrogen atom, thereby forming a primary hydroxyl group, and/or a second level alkyl group. The second level alkyl group is a (C₃-C₅)alkyl group, preferably the second level alkyl group is selected from the group comprising Alkyl-5, Alkyl-6, Alkyl-7 and Alkyl-8, more preferably the second level alkyl group is selected from Alkyl-5 and Alkyl-6. If more than one second level alkyl group is attached to a first level alkyl group Alkyl-3 or Alkyl-4, the second level alkyl groups are preferably identical.

In an embodiment, the compound of the invention is present as a pharmaceutically acceptable salt.

A "pharmaceutically acceptable salt" of the compound of the present invention is preferably an acid salt or a base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Compounds of the invention are capable of forming internal salts which are also pharmaceutically acceptable salts.

Suitable pharmaceutically acceptable salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, *i.e.,* 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

In an embodiment, the compound of the invention is present as a solvate or a pharmaceutically acceptable solvate.

"Pharmaceutically acceptable solvate" of the compound of the invention is preferably a solvate of the compound of the invention formed by association of one or more solvent molecules to one or more molecules of a compound of the invention. Preferably, the solvent is one which is generally considered in the art to be suitable for use in contact with the tissues of human beings or animals without excessive toxicity or carcinogenicity, and preferably without irritation, allergic response, or other problem or complication. Such solvent includes an organic solvent such as alcohols, ethers, esters and amines.

In an embodiment, the compound of the invention is present as a hydrate.

A "hydrate" of the compound of the invention is formed by association of one or more water molecules to one or more molecules of a compound of the invention. Such hydrate includes but is not limited to a hemi-hydrate, mono-hydrate, dihydrate, trihydrate and tetrahydrate. Independent of the hydrate composition all hydrates are generally considered as pharmaceutically acceptable.

The compound of the invention has a high binding affinity to uPAR. The affinity is determined as described in example 38 by the FACS binding assay. High binding affinity is pkD₅₀ category preferably A-D, more preferably A-C, even more preferably A-B and most preferably A. Because of this high binding affinity, the compound of the invention is effective as, useful as and/or suitable as a targeting agent and, if conjugated to another moiety, as a targeting moiety. As preferably used herein a targeting agent is an agent which interacts with the target molecule which is in the instant case said uPAR. In terms of cells and tissues thus targeted by the compound of the invention any cell and tissue, respectively, expressing said uPAR is or may be targeted.

In an embodiment, the compound interacts with Urokinase plasminogen activator surface receptor (uPAR, Gene: PLAUR), preferably with human uPAR having an amino acid sequence of SEQ ID NO: 1 or a homolog thereof, wherein the amino acid sequence of the homolog has an identity of uPAR that is at least 85% to the amino acid sequence of SEQ ID NO: 1. In preferred embodiments, the identity is 90%, preferably 95 %, 96 %, 97 %, 98 % or 99%.

The identity between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or protein or polypeptide which is said to be identical or to be tested whether it is identical, and if so, to what extent, to a different protein or polypeptide, whereby such different protein or polypepetide is also referred to as the reference sequence and is preferably the protein or polypeptide of wild type, more preferably the human uPAR of SEQ ID NO: 1.

Optimal alignment of sequences for comparison can be conducted, e.g., by the local homology algorithm of Smith & Waterman (Smith and Waterman 1981), by the homology alignment algorithm of Needleman & Wunsch (Needleman and Wunsch 1970), by the search for similarity method of Pearson & Lipman (Pearson and Lipman 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al., 1990 (Altschul, Gish et al. 1990) and Altschul et al., 1997 (Altschul, Madden et al. 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al. (McGinnis and Madden 2004).

The compound of the invention has a high binding affinity to uPAR. Because of this high binding affinity, the compound of the invention is effective as, useful as and/or suitable as a targeting agent and, if conjugated to another moiety, as a targeting moiety. As preferably used herein a targeting agent is an agent which interacts with the target molecule which is in the instant case said uPAR. In terms of cells and tissues thus targeted by the compound of the invention any cell and tissue, respectively, expressing said uPAR in particular is targeted. As is known from the prior art, uPAR is highly expressed in a mammalian body and a human body in particular on several neoplastic cells in several tumor indications whereas the expression of uPAR in other tissues of the mammalian and the human body is low (Madunić 2018). These uPAR-expressing tumor indications include but are not limited to breast (Fisher, Field et al. 2000), Colon (Pyke, Ralfkiaer et al. 1994), brain (Yamamoto 1994), stomach (Hong, Park et al. 1996), sancreas (Cantero, Friess et al. 1997), liver (Zhou, Hayashi et al. 2000), lung (He, He et al. 2001), ovary (Mabrouk and Ali-Labib 2003), bladder (Dohn, Pappot et al. 2015), prostate Cancer (Kimura, D'Andrea et al. 2020) and haematologic malignancies (Graf, Reif et al. 2005, Shou, Cao et al. 2016).

Further neoplasms, cancers and tumors which may be treated and/or diagnosed by the compounds of the present invention include those indicated in Fig. 1 which is generated from mRNA expression data in normal tissues from the Genotype-Tissue Expression (GTEx) project (https://gtexportal.org/home/; https://storage.googleapis.com/adult-gtex/bulk-gex/v8/ma-seq/GTEx_Analysis_2017-06-05 v8_RNASeQCv1.1.9_gene_tpm.gct.gz, accessed on Apr. 25 2023) and tumor tissues from the Genomic Data Commons Data Portal (GDC, https://portal.gdc.cancer.gov/). Tumor related datasets were accessed using the Application Programming Interface TCGAbiolinks (Colaprico, Silva et al. 2016) in R Statistical Software (v4.2.2; R Core Team (2022). R: A language and environment for statistical computing. R Foundation for Statistical Computing, Vienna, Austria. https://www.R-project.org/). Datasets for the following projects were accessed through the aforementioned API: TCGA-BRCA, TCGA-STAD, TCGA-LUAD, TCGA-CHOL, TCGA-SARC, TCGA-PCPG, TCGA-COAD, TCGA-ACC, TCGA-UCEC, TCGA-MESO, TCGA-KIRP, TCGA-KIRC, TCGA-GBM, TCGA-DLBC, TCGA-LAML, TCGA-KICH, TCGA-THYM, TCGA-UCS, TCGA-SKCM, TCGA-HNSC, TCGA-PAAD, TCGA-TGCT, TCGA-CESC, TCGA-ESCA, TCGA-THCA, TCGA-LGG, TCGA-LIHC, TCGA-PRAD, TCGA-READ, TCGA-OV, TCGA-UVM, TCGA-BLCA, TCGA-LUSC at March 22^{nd} 2023.

Still further neoplasms, cancers and tumors which may be treated and/or diagnosed by the compounds of the present invention include those, e.g. described in review of (Smith and Marshall 2010) and review of (Madunić 2018) including bladder cancer, breast cancer, tumor associated macrophages and fibroblasts associated with breast cancer (in particular at the invasive front), colorectal cancer, tumor associated macrophages associated with colorectal cancer, gastric cancer, glioblastoma, haematological malignancies such as acute myeloid leukaemia (AML), multiple myeloma (MM), hepatocellular carcinoma (in particular at the invasion front), tumor associated macrophages and fibroblasts associated with hepatocellular carcinoma, lung cancer such as non-small lung carcinoma (NSCLC) and squamous cell carcinoma (SCC), ovarian cancer pancreatic cancer (in particular tumor cells associated with invasion), prostate cancer, tumor associated macrophages and neutrophils associated with prostate cancer, lymph node metastases of prostate cancer. The review of (Smith and Marshall 2010) thus also discloses that the tumor encompasses certain tumor associated cells and tumor associated macrophages, tumor associated fibroblasts and tumor associated neutrophils in particular, whereby such cells express uPAR preferably allowing the targeting of these cells by the compounds of the present invention.

uPAR expression in different cancers is extensively studied and it has a role in tumor growth, angiogenesis, tumor cell invasion, migration, epithelial-mesenchymal transition (EMT), and has been associated with the development of metastatic phenotypes (Andreasen, Egelund et al. 2000, Lund, Illemann et al. 2011, Madunić 2018, Mahmood, Mihalcioiu et al. 2018, Paraskevas, Mulita et al. 2022).

In most cancers uPAR-expression is at higher levels as in the comparable healthy tissue (Figure 1). Because of this, the compounds of the present invention can also be used in the treatment and/or diagnosis of neoplasm, cancer and/or tumor which show this kind of characteristics and angiogenesis, tumor cell invasion, migration and epithelial-mesenchymal transition (EMT) in particular.

Apart from its physiological role, uPAR is involved in pathological processes. Elevated uPAR levels are observed in cardiac fibrosis (Saxena, Izmirly et al. 2015), chronic liver disease and Hepatidis B (Zimmermann, Koch et al. 2012, Akdogan, Atak Yucel et al. 2019), cystic fibrosis, COPD (Xiao, Hsu et al. 2005), idiopathic pulmonary fibrosis (Desai, Mattson et al. 2011), glomerulosclerosis (Trimarchi, Canzonieri et al. 2017, Chebotareva, Vinogradov et al. 2022), systemic sclerosis (suPAR; Legany, Toldi et al. 2015), rheumatoid arthritis (Fibbi, Pucci et al. 1998), neurologic disorders as autism spectrum disorder, epileptogenic tissue remodelling (Campbell, D'Oronzio et al. 2007, Campbell, Li et al. 2008, Lahtinen, Huusko et al. 2009), autoimmune disease (Elvin, Foltz et al. 2009, WO002007120693, Duriseti, Goetz et al. 2010, WO002011100620, LeBeau, Duriseti et al. 2013, Lu, Cong et al. 2021, WO002023125842, WO002002082077, Luther, Kotzsch et al. 2003) and senescence related diseases (Amor, Feucht et al. 2020, WO002022261405) as well as autoimmune rheumatic and non-rheumatic diseases (Manfredi, Van Hoovels et al. 2023). Autoimmune diseases include, but are not limited to autoimmune rheumatic diseases and non-rheumatic autoimmune diseases. Autoimmune rheumatic diseases include rheumatoid arthritis (Fibbi, Pucci et al. 1998, Pliyev and Menshikov 2010, Enocsson, Lukic et al. 2021), systemic lupus erythematosus (SLE)(Toldi, Szalay et al. 2012, Enocsson, Wettero et al. 2013, Enocsson, Sjowall et al. 2015, Qin, Song et al. 2015), systemic sclerosis (SSc) (Legany, Toldi et al. 2015), Behcet's syndrome (Saylam Kurtipek, Kesli et al. 2018), psoriasis (Kurtipek, Kesli et al. 2015, Hamie, Eid et al. 2020), ankylosing spondylitis (AS)(Toldi, Szalay et al. 2013), ANCA-associated vasculitis (AAV) (Huang, Li et al. 2020, Zabinska, Koscielska-Kasprzak et al. 2021). Non-rheumatic autoimmune diseases include Type 1 Diabetes Mellitus (T1DM)(Theilade, Lyngbaek et al. 2015, Theilade, Rossing et al. 2016), Inflammatory Bowel Diseases (IBD) (Kolho, Valtonen et al. 2012) and Myasthenia Gravis (MG) (Uzawa, Kojima et al. 2020).

In embodiments, the subject exhibits an increased accumulation of senescent cells compared to that observed in a healthy control subject. In preferred embodiments, the senescence-associated pathology is selected from the group consisting of lung fibrosis, atherosclerosis, Alzheimer's disease, diabetes, liver fibrosis, chronic kidney disease, osteoarthritis, cardiac fibrosis, and Parkinson's disease.

It will be acknowleged and appreciated by a person skilled in the art that uPAR expression is increased at diseases sites. Because of this, the compounds of the invention, in particular when comprising a nucleide and preferably a radionuclide suitable for therapy or diagnosis, are targeting such sites and accumulate there, whereupon treatment and/or diagnosis is possible. Such treatment and/or diagnosis is possible both if the target structure such as a diseased cell, including but not limited to a tumor cell, is expressing uPAR and if there is a cell, preferably a multitude of cells, which is expressing uPAR and which is close to or are part of the target structure such as a tumor or a diseased tissue. Of course, such treatment and/or diagnosis is also possible if both the target structure expresses uPAR and the cell close to or part of the target structure express uPAR.

It will be further acknowledged and appreciated by a person skilled in the art that there is good evidence for a correlation of suPAR-levels and increased uPAR expression at disease sites (Mustjoki, Sidenius et al. 2000, Taubert, Wurl et al. 2010, Zhou, Ren et al. 2019). Serological suPAR concentration is routinely measured at several hospitals and used as a biomarker for prediction of severity of COVID-19 (Altintas, Eugen-Olsen et al. 2021, Enocsson, Idoff et al. 2021, Stauning, Altintas et al. 2021), chronical systemic infection (Rasmussen, Petersen et al. 2021), sepsis (Huang, Xiong et al. 2020), acute kidney injury (Sudhini, Wei et al. 2022, Huang, Huang et al. 2023, Jankowski, Pruc et al. 2023), cardiogenic shock (Hongisto, Lassus et al. 2022) or for severity stratification in emergency medicine (Østervig, Køber et al. 2015, Santeri, Peter et al. 2021, Velissaris, Zareifopoulos et al. 2022).

In light thereof, the compound of the invention is thus particularly suitable for and useful in the diagnosis and treatment, respectively, of these diseases, including any metastatic forms and metastases thereof. Insofar, the above indications are indications which can be treated by the compound of the invention. It will be understood by the person skilled in the art that also metastases and metastases of the above indications in particular can be treated and diagnosed by the compound of the invention and the methods of diagnosis and methods of treatment making use of the compound of the invention.

It is within the present invention that the compound of the invention is used or is for use in a method for the treatment of a disease as disclosed herein. Such method, preferably, comprises the step of administering to a subject in need thereof a therapeutically effective amount of the compound of the invention. Such method includes, but is not limited to, curative or adjuvant cancer treatment. It is used as palliative treatment where cure is not possible and the aim is for local disease control or symptomatic relief or as therapeutic treatment where the therapy has survival benefit and it can be curative.

The method for the treatment of a disease as disclosed herein includes the treatment of the disease disclosed herein, including tumors and cancer, and may be used either as the primary therapy or as second, third, fourth or last line therapy. It is also within the present invention to combine the compound of the invention with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

In an embodiment of the present invention, the disease is selected from the group comprising neoplasm nos, neoplasm, benign, neoplasm, uncertain whether benign or malignant, neoplasm, malignant, neoplasm, metastatic, neoplasm, malignant, uncertain whether primary or metastatic, tumor cells, benign, tumor cells, uncertain whether benign or malignant, tumor cells, malignant, malignant tumor, small cell type, malignant tumor, giant cell type, malignant tumor, fusiform cell type, epithelial neoplasms nos, epithelial tumor, benign, carcinoma in situ nos, carcinoma nos, carcinoma, metastatic nos, carcinomatosis, epithelioma, benign, epithelioma, malignant, large cell carcinoma nos, carcinoma, undifferentiated type nos, carcinoma, anaplastic type nos, pleomorphic carcinoma, giant cell and spindle cell carcinoma, giant cell carcinoma, spindle cell carcinoma, pseudosarcomatous carcinoma, polygonal cell carcinoma, spheroidal cell carcinoma, tumorlet, small cell carcinoma nos, oat cell carcinoma, small cell carcinoma, fusiform cell type, papillary and squamous cell neoplasms, papilloma nos, papillary carcinoma in situ, papillary carcinoma nos, verrucous papilloma, verrucous carcinoma nos, squamous cell papilloma, papillary squamous cell carcinoma, inverted papilloma, papillomatosis nos, squamous cell carcinoma in situ nos, squamous cell carcinoma nos, squamous cell carcinoma, metastatic nos, squamous cell carcinoma, keratinizing type nos, squamous cell carcinoma, large cell, nonkeratinizing type, squamous cell carcinoma, small cell, nonkeratinizing type, squamous cell carcinoma, spindle cell type, adenoid squamous cell carcinoma, squamous cell carcinoma in situ with questionable stromal invasion, squamous cell carcinoma, microinvasive, queyrat`s erythroplasia, bowen's disease, lymphoepithelial carcinoma, basal cell neoplasms, basal cell tumor, basal cell carcinoma nos, multicentric basal cell carcinoma, basal cell carcinoma, morphea type, basal cell carcinoma, fibroepithelial type, basosquamous carcinoma, metatypical carcinoma, intraepidermal epithelioma of jadassohn, trichoepithelioma, trichofolliculoma, tricholemmoma, pilomatrixoma, transitional cell papillomas and carcinomas, transitional cell papilloma nos, urothelial papilloma, transitional cell carcinoma in situ, transitional cell carcinoma nos, schneiderian papilloma, transitional cell papilloma, inverted type, schneiderian carcinoma, transitional cell carcinoma, spindle cell type, basaloid carcinoma, cloacogenic carcinoma, papillary transitional cell carcinoma, adenomas and adenocarcinomas, adenoma nos, bronchial adenoma nos, adenocarcinoma in situ, adenocarcinoma nos, adenocarcinoma, metastatic nos, scirrhous adenocarcinoma, linitis plastica, superficial spreading adenocarcinoma, adenocarcinoma, intestinal type, carcinoma, diffuse type, monomorphic adenoma, basal cell adenoma, islet cell adenoma, islet cell carcinoma, insulinoma nos, insulinoma, malignant, glucagonoma nos, glucagonoma, malignant, gastrinoma nos, gastrinoma, malignant, mixed islet cell and exocrine adenocarcinoma, bile duct adenoma, cholangiocarcinoma, bile duct cystadenoma, bile duct cystadenocarcinoma, liver cell adenoma, hepatocellular carcinoma nos, hepatocholangioma, benign, combined hepatocellular carcinoma and cholangiocarcinoma, trabecular adenoma, trabecular adenocarcinoma, embryonal adenoma, eccrine dermal cylindroma, adenoid cystic carcinoma, cribriform carcinoma, adenomatous polyp nos, adenocarcinoma in adenomatous polyp, tubular adenoma nos, tubular adenocarcinoma, adenomatous polyposis coli, adenocarcinoma in adenomatous polyposis coli, multiple adenomatous polyps, solid carcinoma nos, carcinoma simplex, carcinoid tumor nos, carcinoid tumor, malignant, carcinoid tumor, argentaffin nos, carcinoid tumor, argentaffin, malignant, carcinoid tumor, nonargentaffin nos, carcinoid tumor, nonargentaffin, malignant, mucocarcinoid tumor, malignant, composite carcinoid, pulmonary adenomatosis, bronchiolo-alveolar adenocarcinoma, alveolar adenoma, alveolar adenocarcinoma, papillary adenoma nos, papillary adenocarcinoma nos, villous adenoma nos, adenocarcinoma in villous adenoma, villous adenocarcinoma, tubulovillous adenoma, chromophobe adenoma, chromophobe carcinoma, acidophil adenoma, acidophil carcinoma, mixed acidophil-basophil adenoma, mixed acidophil-basophil carcinoma, oxyphilic adenoma, oxyphilic adenocarcinoma, basophil adenoma, basophil carcinoma, clear cell adenoma, clear cell adenocarcinoma nos, hypernephroid tumor, renal cell carcinoma, clear cell adenofibroma, granular cell carcinoma, chief cell adenoma, water-clear cell adenoma, water-clear cell adenocarcinoma, mixed cell adenoma, mixed cell adenocarcinoma, lipoadenoma, follicular adenoma, follicular adenocarcinoma nos, follicular adenocarcinoma, well differentiated type, follicular adenocarcinoma, trabecular type, microfollicular adenoma, macrofollicular adenoma, papillary and follicular adenocarcinoma, nonencapsulated sclerosing carcinoma, multiple endocrine adenomas, juxtaglomerular tumor, adrenal cortical adenoma nos, adrenal cortical carcinoma, adrenal cortical adenoma, compact cell type, adrenal cortical adenoma, heavily pigmented variant, adrenal cortical adenoma, clear cell type, adrenal cortical adenoma, glomerulosa cell type, adrenal cortical adenoma, mixed cell type, endometrioid adenoma nos, endometrioid adenoma, borderline malignancy, endometrioid carcinoma, endometrioid adenofibroma nos, endometrioid adenofibroma, borderline malignancy, endometrioid adenofibroma, malignant, adnexal and skin appendage neoplasms, skin appendage adenoma, skin appendage carcinoma, sweat gland adenoma, sweat gland tumor nos, sweat gland adenocarcinoma, apocrine adenoma, apocrine adenocarcinoma, eccrine acrospiroma, eccrine spiradenoma, hidrocystoma, papillary hydradenoma, papillary syringadenoma, syringoma nos, sebaceous adenoma, sebaceous adenocarcinoma, ceruminous adenoma, ceruminous adenocarcinoma, mucoepidermoid neoplasms, mucoepidermoid tumor, mucoepidermoid carcinoma, cystic, mucinous, and serous neoplasms, cystadenoma nos, cystadenocarcinoma nos, serous cystadenoma nos, serous cystadenoma, borderline malignancy, serous cystadenocarcinoma nos, papillary cystadenoma nos, papillary cystadenoma, borderline malignancy, papillary cystadenocarcinoma nos, papillary serous cystadenoma nos, papillary serous cystadenoma, borderline malignancy, papillary serous cystadenocarcinoma, serous surface papilloma nos, serous surface papilloma, borderline malignancy, serous surface papillary carcinoma, mucinous cystadenoma nos, mucinous cystadenoma, borderline malignancy, mucinous cystadenocarcinoma nos, papillary mucinous cystadenoma nos, papillary mucinous cystadenoma, borderline malignancy, papillary mucinous cystadenocarcinoma, mucinous adenoma, mucinous adenocarcinoma, pseudomyxoma peritonei, mucin-producing adenocarcinoma, signet ring cell carcinoma, metastatic signet ring cell carcinoma, ductal, lobular, and medullary neoplasms, intraductal carcinoma, noninfiltrating nos, infiltrating duct carcinoma, comedocarcinoma, noninfiltrating, comedocarcinoma nos, juvenile carcinoma of the breast, intraductal papilloma, noninfiltrating intraductal papillary adenocarcinoma, intracystic papillary adenoma, noninfiltrating intracystic carcinoma, intraductal papillomatosis nos, subareolar duct papillomatosis, medullary carcinoma nos, medullary carcinoma with amyloid stroma, medullary carcinoma with lymphoid stroma, lobular carcinoma in situ, lobular carcinoma nos, infiltrating ductular carcinoma, inflammatory carcinoma, paget's disease, mammary, paget's disease and infiltrating duct carcinoma of breast, paget's disease, extramammary, acinar cell neoplasms, acinar cell adenoma, acinar cell tumor, acinar cell carcinoma, complex epithelial neoplasms, adenosquamous carcinoma, adenolymphoma, adenocarcinoma with squamous metaplasia, adenocarcinoma with cartilaginous and osseous metaplasia, adenocarcinoma with spindle cell metaplasia, adenocarcinoma with apocrine metaplasia, thymoma, benign, thymoma, malignant, specialized gonadal neoplasms, sex cord-stromal tumor, thecoma nos, theca cell carcinoma, luteoma nos, granulosa cell tumor nos, granulosa cell tumor, malignant, granulosa cell-theca cell tumor, androblastoma, benign, androblastoma nos, androblastoma, malignant, sertoli-leydig cell tumor, gynandroblastoma, tubular androblastoma nos, sertoli cell carcinoma, tubular androblastoma with lipid storage, leydig cell tumor, benign, leydig cell tumor nos, leydig cell tumor, malignant, hilar cell tumor, lipid cell tumor of ovary, adrenal rest tumor, paragangliomas and glomus tumors, paraganglioma nos, paraganglioma, malignant, sympathetic paraganglioma, parasympathetic paraganglioma, glomus jugulare tumor, aortic body tumor, carotid body tumor, extra-adrenal paraganglioma nos, extra-adrenal paraganglioma, malignant, pheochromocytoma nos, pheochromocytoma, malignant, glomangiosarcoma, glomus tumor, glomangioma, nevi and melanomas, pigmented nevus nos, malignant melanoma nos, nodular melanoma, balloon cell nevus, balloon cell melanoma, halo nevus, fibrous papule of the nose, neuronevus, magnocellular nevus, nonpigmented nevus, amelanotic melanoma, junctional nevus, malignant melanoma in junctional nevus, precancerous melanosis nos, malignant melanoma in precancerous melanosis, hutchinson's melanotic freckle, malignant melanoma in hutchinson's melanotic freckle, superficial spreading melanoma, intradermal nevus, compound nevus, giant pigmented nevus, malignant melanoma in giant pigmented nevus, epithelioid and spindle cell nevus, epithelioid cell melanoma, spindle cell melanoma nos, spindle cell melanoma, type a, spindle cell melanoma, type b, mixed epithelioid and spindle cell melanoma, blue nevus nos, blue nevus, malignant, cellular blue nevus, soft tissue tumors and sarcomas nos, soft tissue tumor, benign, sarcoma nos, sarcomatosis nos, spindle cell sarcoma, giant cell sarcoma, small cell sarcoma, epithelioid cell sarcoma, fibromatous neoplasms, fibroma nos, fibrosarcoma nos, fibromyxoma, fibromyxosarcoma, periosteal fibroma, periosteal fibrosarcoma, fascial fibroma, fascial fibrosarcoma, infantile fibrosarcoma, elastofibroma, aggressive fibromatosis, abdominal fibromatosis, desmoplastic fibroma, fibrous histiocytoma nos, atypical fibrous histiocytoma, fibrous histiocytoma, malignant, fibroxanthoma nos, atypical fibroxanthoma, fibroxanthoma, malignant, dermatofibroma nos, dermatofibroma protuberans, dermatofibrosarcoma nos, myxomatous neoplasms, myxoma nos, myxosarcoma, lipomatous neoplasms, lipoma nos, liposarcoma nos, fibrolipoma, liposarcoma, well differentiated type, fibromyxolipoma, myxoid liposarcoma, round cell liposarcoma, pleomorphic liposarcoma, mixed type liposarcoma, intramuscular lipoma, spindle cell lipoma, angiomyolipoma, angiomyoliposarcoma, angiolipoma nos, angiolipoma, infiltrating, myelolipoma, hibernoma, lipoblastomatosis, myomatous neoplasms, leiomyoma nos, intravascular leiomyomatosis, leiomyosarcoma nos, epithelioid leiomyoma, epithelioid leiomyosarcoma, cellular leiomyoma, bizarre leiomyoma, angiomyoma, angiomyosarcoma, myoma, myosarcoma, rhabdomyoma nos, rhabdomyosarcoma nos, pleomorphic rhabdomyosarcoma, mixed type rhabdomyosarcoma, fetal rhabdomyoma, adult rhabdomyoma, embryonal rhabdomyosarcoma, alveolar rhabdomyosarcoma, complex mixed and stromal neoplasms, endometrial stromal sarcoma, endolymphatic stromal myosis, adenomyoma, pleomorphic adenoma, mixed tumor, malignant nos, mullerian mixed tumor, mesodermal mixed tumor, mesoblastic nephroma, nephroblastoma nos, epithelial nephroblastoma, mesenchymal nephroblastoma, hepatoblastoma, carcinosarcoma nos, carcinosarcoma, embryonal type, myoepithelioma, mesenchymoma, benign, mesenchymoma nos, mesenchymoma, malignant, embryonal sarcoma, fibroepithelial neoplasms, brenner tumor nos, brenner tumor, borderline malignancy, brenner tumor, malignant, fibroadenoma nos, intracanalicular fibroadenoma nos, pericanalicular fibroadenoma, adenofibroma nos, serous adenofibroma, mucinous adenofibroma, cellular intracanalicular fibroadenoma, cystosarcoma phyllodes nos, cystosarcoma phyllodes, malignant, juvenile fibroadenoma, synovial neoplasms, synovioma, benign, synovial sarcoma nos, synovial sarcoma, spindle cell type, synovial sarcoma, epithelioid cell type, synovial sarcoma, biphasic type, clear cell sarcoma of tendons and aponeuroses, mesothelial neoplasms, mesothelioma, benign, mesothelioma, malignant, fibrous mesothelioma, benign, fibrous mesothelioma, malignant, epithelioid mesothelioma, benign, epithelioid mesothelioma, malignant, mesothelioma, biphasic type, benign, mesothelioma, biphasic type, malignant, adenomatoid tumor nos, germ cell neoplasms, dysgerminoma, seminoma nos, seminoma, anaplastic type, spermatocytic seminoma, germinoma, embryonal carcinoma nos, endodermal sinus tumor, polyembryoma, gonadoblastoma, teratoma, benign, teratoma nos, teratoma, malignant nos, teratocarcinoma, malignant teratoma, undifferentiated type, malignant teratoma, intermediate type, dermoid cyst, dermoid cyst with malignant transformation, struma ovarii nos, struma ovarii, malignant, strumal carcinoid, trophoblastic neoplasms, hydatidiform mole nos, invasive hydatidiform mole, choriocarcinoma, choriocarcinoma combined with teratoma, malignant teratoma, trophoblastic, mesonephromas, mesonephroma, benign, mesonephric tumor, mesonephroma, malignant, endosalpingioma, blood vessel tumors, hemangioma nos, hemangiosarcoma, cavernous hemangioma, venous hemangioma, racemose hemangioma, kupffer cell sarcoma, hemangioendothelioma, benign, hemangioendothelioma nos, hemangioendothelioma, malignant, capillary hemangioma, intramuscular hemangioma, kaposi`s sarcoma, angiokeratoma, verrucous keratotic hemangioma, hemangiopericytoma, benign, hemangiopericytoma nos, hemangiopericytoma, malignant, angiofibroma nos, hemangioblastoma, lymphatic vessel tumors, lymphangioma nos, lymphangiosarcoma, capillary lymphangioma, cavernous lymphangioma, cystic lymphangioma, lymphangiomyoma, lymphangiomyomatosis, hemolymphangioma, osteomas and osteosarcomas, osteoma nos, osteosarcoma nos, chondroblastic osteosarcoma, fibroblastic osteosarcoma, telangiectatic osteosarcoma, osteosarcoma in paget's disease of bone, juxtacortical osteosarcoma, osteoid osteoma nos, osteoblastoma, chondromatous neoplasms, osteochondroma, osteochondromatosis nos, chondroma nos, chondromatosis nos, chondrosarcoma nos, juxtacortical chondroma, juxtacortical chondrosarcoma, chondroblastoma nos, chondroblastoma, malignant, mesenchymal chondrosarcoma, chondromyxoid fibroma, giant cell tumors, giant cell tumor of bone nos, giant cell tumor of bone, malignant, giant cell tumor of soft parts nos, malignant giant cell tumor of soft parts, miscellaneous bone tumors, ewing`s sarcoma, adamantinoma of long bones, ossifying fibroma, odontogenic tumors, odontogenic tumor, benign, odontogenic tumor nos, odontogenic tumor, malignant, dentinoma, cementoma nos, cementoblastoma, benign, cementifying fibroma, gigantiform cementoma, odontoma nos, compound odontoma, complex odontoma, ameloblastic fibro-odontoma, ameloblastic odontosarcoma, adenomatoid odontogenic tumor, calcifying odontogenic cyst, ameloblastoma nos, ameloblastoma, malignant, odontoameloblastoma, squamous odontogenic tumor, odontogenic myxoma, odontogenic fibroma nos, ameloblastic fibroma, ameloblastic fibrosarcoma, calcifying epithelial odontogenic tumor, miscellaneous tumors, craniopharyngioma, pinealoma, pineocytoma, pineoblastoma, melanotic neuroectodermal tumor, chordoma, gliomas, glioma, malignant, gliomatosis cerebri, mixed glioma, subependymal glioma, subependymal giant cell astrocytoma, choroid plexus papilloma nos, choroid plexus papilloma, malignant, ependymoma nos, ependymoma, anaplastic type, papillary ependymoma, myxopapillary ependymoma, astrocytoma nos, astrocytoma, anaplastic type, protoplasmic astrocytoma, gemistocytic astrocytoma, fibrillary astrocytoma, pilocytic astrocytoma, spongioblastoma nos, spongioblastoma polare, astroblastoma, glioblastoma nos, giant cell glioblastoma, glioblastoma with sarcomatous component, primitive polar spongioblastoma, oligodendroglioma nos, oligodendroglioma, anaplastic type, oligodendroblastoma, medulloblastoma nos, desmoplastic medulloblastoma, medullomyoblastoma, cerebellar sarcoma nos, monstrocellular sarcoma, neuroepitheliomatous neoplasms, ganglioneuroma, ganglioneuroblastoma, ganglioneuromatosis, neuroblastoma nos, medulloepithelioma nos, teratoid medulloepithelioma, neuroepithelioma nos, spongioneuroblastoma, ganglioglioma, neurocytoma, pacinian tumor, retinoblastoma nos, retinoblastoma, differentiated type, retinoblastoma, undifferentiated type, olfactory neurogenic tumor, esthesioneurocytoma, esthesioneuroblastoma, esthesioneuroepithelioma, meningiomas, meningioma nos, meningiomatosis nos, meningioma, malignant, meningotheliomatous meningioma, fibrous meningioma, psammomatous meningioma, angiomatous meningioma, hemangioblastic meningioma, hemangiopericytic meningioma, transitional meningioma, papillary meningioma, meningeal sarcomatosis, nerve sheath tumor, neurofibroma nos, neurofibromatosis nos, neurofibrosarcoma, melanotic neurofibroma, plexiform neurofibroma, neurilemmoma nos, neurinomatosis, neurilemmoma, malignant, neuroma nos, granular cell tumors and alveolar soft part sarcoma, granular cell tumor nos, granular cell tumor, malignant, alveolar soft part sarcoma, lymphomas, nos or diffuse, lymphomatous tumor, benign, malignant lymphoma nos, malignant lymphoma, non hodgkin's type, malignant lymphoma, undifferentiated cell type nos, malignant lymphoma, stem cell type, malignant lymphoma, convoluted cell type nos, lymphosarcoma nos, malignant lymphoma, lymphoplasmacytoid type, malignant lymphoma, immunoblastic type, malignant lymphoma, mixed lymphocytic-histiocytic nos, malignant lymphoma, centroblastic-centrocytic, diffuse, malignant lymphoma, follicular center cell nos, malignant lymphoma, lymphocytic, well differentiated nos, malignant lymphoma, lymphocytic, intermediate differentiation nos, malignant lymphoma, centrocytic, malignant lymphoma, follicular center cell, cleaved nos, malignant lymphoma, lymphocytic, poorly differentiated nos, prolymphocytic lymphosarcoma, malignant lymphoma, centroblastic type nos, malignant lymphoma, follicular center cell, noncleaved nos, reticulosarcomas, reticulosarcoma nos, reticulosarcoma, pleomorphic cell type, reticulosarcoma, nodular, hodgkin's disease, hodgkin's disease nos, hodgkin's disease, lymphocytic predominance, hodgkin's disease, mixed cellularity, hodgkin's disease, lymphocytic depletion nos, hodgkin's disease, lymphocytic depletion, diffuse fibrosis, hodgkin's disease, lymphocytic depletion, reticular type, hodgkin's disease, nodular sclerosis nos, hodgkin's disease, nodular sclerosis, cellular phase, hodgkin's paragranuloma, hodgkin's granuloma, hodgkin's sarcoma, lymphomas, nodular or follicular, malignant lymphoma, nodular nos, malignant lymphoma, mixed lymphocytic-histiocytic, nodular, malignant lymphoma, centroblastic-centrocytic, follicular, malignant lymphoma, lymphocytic, well differentiated, nodular, malignant lymphoma, lymphocytic, intermediate differentiation, nodular, malignant lymphoma, follicular center cell, cleaved, follicular, malignant lymphoma, lymphocytic, poorly differentiated, nodular, malignant lymphoma, centroblastic type, follicular, malignant lymphoma, follicular center cell, noncleaved, follicular, mycosis fungoides, mycosis fungoides, sezary's disease, miscellaneous reticuloendothelial neoplasms, microglioma, malignant histiocytosis, histiocytic medullary reticulosis, letterer-siwe's disease, plasma cell tumors, plasma cell myeloma, plasma cell tumor, benign, plasmacytoma nos, plasma cell tumor, malignant, mast cell tumors, mastocytoma nos, mast cell sarcoma, malignant mastocytosis, burkitt's tumor, burkitt's tumor, leukemias, leukemias nos, leukemia nos, acute leukemia nos, subacute leukemia nos, chronic leukemia nos, aleukemic leukemia nos, compound leukemias, compound leukemia, lymphoid leukemias, lymphoid leukemia nos, acute lymphoid leukemia, subacute lymphoid leukemia, chronic lymphoid leukemia, aleukemic lymphoid leukemia, prolymphocytic leukemia, plasma cell leukemias, plasma cell leukemia, erythroleukemias, erythroleukemia, acute erythremia, chronic erythremia, lymphosarcoma cell leukemias, lymphosarcoma cell leukemia, myeloid leukemias, myeloid leukemia nos, acute myeloid leukemia, subacute myeloid leukemia, chronic myeloid leukemia, aleukemic myeloid leukemia, neutrophilic leukemia, acute promyelocytic leukemia, basophilic leukemias, basophilic leukemia, eosinophilic leukemias, eosinophilic leukemia, monocytic leukemias, monocytic leukemia nos, acute monocytic leukemia, subacute monocytic leukemia, chronic monocytic leukemia, aleukemic monocytic leukemia, miscellaneous leukemias, mast cell leukemia, megakaryocytic leukemia, megakaryocytic myelosis, myeloid sarcoma, hairy cell leukemia, miscellaneous myeloproliferative and lymphoproliferative disorders, polycythemia vera, acute panmyelosis, chronic myeloproliferative disease, myelosclerosis with myeloid metaplasia, idiopathic thrombocythemia, chronic lymphoproliferative disease, whereby preferably the tumor cells of the above disease express uPAR and/or the tumor tissue contains non-tumor cells such as macrophages, fibroblasts and neutrophils, whereby the non-tumor cells express uPAR

In an embodiment of the present invention, the disease is selected from the group comprising tumors of pancreas, pancreatic adenocarcinoma, tumors of head of pancreas, of body of pancreas, of tail of pancreas, of pancreatic duct, of islets of langerhans, neck of pancreas, tumor of prostate, prostate adenocarcinoma, prostate gland, neuroendocrine tumors, breast cancer, tumor of central portion of breast, upper inner quadrant of breast, lower inner quadrant of breast, upper outer quadrant of breast, lower outer quadrant of breast, axillary tail of breast, overlapping lesion of breast, juvenile carcinoma of the breast, tumors of parathyroid gland, myeloma, lung cancer, small cell lung cancer, non-small cell lung cancer, tumor of main bronchus, of upper lobe lung, of middle lobe lung, of lower lobe lung, colorectal carcinoma, tumor of ascending colon, of hepatic flexure of colon, of transverse colon, of splenic flexure of colon, of descending colon, of sigmoid colon, of overlapping lesion of colon, of small intestine, tumors of liver, liver cell adenoma, hepatocellular carcinoma, hepatocholangioma, ombined hepatocellular carcinoma and cholangiocarcinoma, hepatoblastoma, ovarian carcinoma, sarcoma, osteosarcoma, fibrosarcoma, gastrointestinal stroma tumors, gastrointestinal tract, gastric carcinoma, thyroid carcinoma, medullary thyroid carcinoma, thyroid gland, renal cell carcinoma, renal pelvis, tumors of bladder, bladder carcinoma, tumors of trigone bladder, of dome bladder, of lateral wall bladder, of posterior wall bladder, of ureteric orifice, of urachus, overlapping lesion of bladder, basal cell carcinoma, basal cell neoplasms, basal cell tumor, basal cell carcinoma, multicentric basal cell carcinoma, basaloid carcinoma, basal cell adenoma, squamous cell carcinoma, oral squamous cell carcinoma, *squamous cell carcinoma* of the *larynx,* cervical carcinoma, tumors of exocervix, of overlapping lesion of cervix uteri, of cervix uteri, of isthmus uteri, tumors of uterus, tumors of ovary, tumors of cervical esophagus, of thoracic esophagus, of abdominal esophagus, of upper third of esophagus, of esophagus middle third, of esophagus lower third, of overlapping lesion of esophagus, endometrial carcinoma, head and neck cancer, lymphoma, malignant mesothelioma, mesothelial neoplasms, mesothelioma, fibrous mesothelioma, fibrous mesothelioma, epithelioid mesothelioma, epithelioid mesothelioma, duodenal carcinoma, neuroendocrine tumors, neuroendocrine tumors of the lung, neuroendocrine tumors of the pancreas, neuroendocrine tumors of the foregut, neuroendocrine tumors of the midgut, neuroendocrine tumors of the hindgut, gastroenteropancreatic neuroendocrine tumors, neuroendocrine carcinomas, neuroendocrine tumors of the breast, neuroendocrine tumors o the ovaries, testicular cancer, thymic carcinoma, tumors of stomach, fundus stomach, body stomach, gastric antrum, pylorus, lesser curvature of stomach, greater curvature of stomach, overlapping lesion of stomach, paragangliomas, ganglioma, melanomas, malignant melanoma, nodular melanoma, amelanotic melanoma, superficial spreading melanoma, epithelioid cell melanoma, spindle cell melanoma, mixed epithelioid and spindle cell melanoma.

In a still further embodiment, the aforementioned indications may occur in organs and tissues selected from the group comprising external upper lip, external lower lip, external lip nos, upper lip mucosa, lower lip mucosa, mucosa lip nos, commissure lip, overlapping lesion of lip, base of tongue nos, dorsal surface tongue nos, border of tongue, ventral surface of tongue nos, anterior 2/3 of tongue nos, lingual tonsil, overlapping lesion of tongue, tongue nos, upper gum, lower gum, gum nos, anterior floor of mouth, lateral floor of mouth, overlapping lesion of floor of mouth, floor of mouth nos, hard palate, soft palate nos, uvula, overlapping lesion of palate, palate nos, cheek mucosa, vestibule of mouth, retromolar area, overlapping lesion of other and unspecified parts of mouth, mouth nos, parotid gland, submaxillary gland, sublingual gland, overlapping lesion of major salivary glands, major salivary gland nos, tonsillar fossa, tonsillar pillar, overlapping lesion of tonsil, tonsil nos, vallecula, anterior surface of epiglottis, lateral wall oropharynx, posterior wall oropharynx, branchial cleft, overlapping lesion of oropharynx, oropharynx nos, superior wall of nasopharynx, posterior wall nasopharynx, lateral wall nasopharynx, anterior wall nasopharynx, overlapping lesion of nasopharynx, nasopharynx nos, pyriform sinus, postcricoid region, hypopharyngeal aspect of aryepiglottic fold, posterior wall hypopharynx, overlapping lesion of hypopharynx, hypopharynx nos, pharynx nos, laryngopharynx, waldeyer's ring, overlapping lesion of lip oral cavity and pharynx, cervical esophagus, thoracic esophagus, abdominal esophagus, upper third of esophagus, middle third of esophagus, esophagus lower third, overlapping lesion of esophagus, esophagus nos, cardia nos, fundus stomach, body stomach, gastric antrum, pylorus, lesser curvature of stomach nos, greater curvature of stomach nos, overlapping lesion of stomach, stomach nos, duodenum, jejunum, ileum, meckel's diverticulum, overlapping lesion of small intestine, small intestine nos, cecum, appendix, ascending colon, hepatic flexure of colon, transverse colon, splenic flexure of colon, descending colon, sigmoid colon, overlapping lesion of colon, colon nos, rectosigmoid junction, rectum nos, anus nos, anal canal, cloacogenic zone, overlapping lesion of rectum anus and anal canal, liver, intrahepatic bile duct, gallbladder, extrahepatic bile duct, ampulla of vater, overlapping lesion of biliary tract, biliary tract nos, head of pancreas, body pancreas, tail pancreas, pancreatic duct, islets of langerhans, neck of pancreas, overlapping lesion of pancreas, pancreas nos, intestinal tract nos, overlapping lesion of digestive system, gastrointestinal tract nos, nasal cavity, middle ear, maxillary sinus, ethmoid sinus, frontal sinus, sphenoid sinus, overlapping lesion of accessory sinuses, accessory sinus nos, glottis, supraglottis, subglottis, laryngeal cartilage, overlapping lesion of larynx, larynx nos, trachea, main bronchus, upper lobe lung, middle lobe lung, lower lobe lung, overlapping lesion of lung, lung nos, thymus, heart, anterior mediastinum, posterior mediastinum, mediastinum nos, pleura nos, overlapping lesion of heart mediastinum and pleura, upper respiratory tract nos, overlapping lesion of respiratory system and intrathoracic organs, respiratory tract nos, upper limb long bones joints, upper limb short bones joints, lower limb long bones joints, lower limb short bones joints, overlapping lesion of bones joints and articular cartilage of limbs, bone limb nos, skull and facial bone, mandible, vertebral column, rib sternum clavicle, pelvic bone, overlapping lesion of bones joints and articular cartilage, bone nos, blood, bone marrow, spleen, reticuloendothelial system nos, hematopoietic system nos, skin lip nos, eyelid nos, external ear, skin face, skin scalp neck, skin trunk, skin limb upper, skin limb lower, peripheral nerve head neck, peripheral nerve shoulder arm, peripheral nerve leg, peripheral nerve thorax, peripheral nerve abdomen, peripheral nerve pelvis, peripheral nerve trunk, overlapping lesion of peripheral nerves and autonomic nervous system, autonomic nervous system nos, retroperitoneum, peritoneum, peritoneum nos, overlapping lesion of retroperitoneum and peritoneum, connective tissue head, connective tissue arm, connective tissue leg, connective tissue thorax, connective tissue abdomen, connective tissue pelvis, connective tissue trunk nos, overlapping lesion of connective subcutaneous and other soft tissues, connective tissue nos, nipple, central portion of breast, upper inner quadrant of breast, lower inner quadrant of breast, upper outer quadrant of breast, lower outer quadrant of breast, axillary tail of breast, overlapping lesion of breast, breast nos, labium majus, labium minus, clitoris, overlapping lesion of vulva, vulva nos, vagina nos, endocervix, exocervix, overlapping lesion of cervix uteri, cervix uteri, isthmus uteri, endometrium, myometrium, fundus uteri, overlapping lesion of corpus uteri, corpus uteri, uterus nos, ovary, fallopian tube, broad ligament, round ligament, parametrium, uterine adnexa, wolffian body, overlapping lesion of female genital organs, female genital tract nos, prepuce, glans penis, body penis, overlapping lesion of penis, penis nos, prostate gland, undescended testis, descended testis, testis nos, epididymis, spermatic cord, scrotum nos, tunica vaginalis, overlapping lesion of male genital organs, male genital organs nos, kidney nos, renal pelvis, ureter, trigone bladder, dome bladder, lateral wall bladder, posterior wall bladder, ureteric orifice, urachus, overlapping lesion of bladder, bladder nos, urethra, paraurethral gland, overlapping lesion of urinary organs, urinary system nos, conjunctiva, cornea nos, retina, choroid, ciliary body, lacrimal gland, orbit nos, overlapping lesion of eye and adnexa, eye nos, cerebral meninges, spinal meninges, meninges nos, cerebrum, frontal lobe, temporal lobe, parietal lobe, occipital lobe, ventricle nos, cerebellum nos, brain stem, overlapping lesion of brain, brain nos, spinal cord, cauda equina, olfactory nerve, optic nerve, acoustic nerve, cranial nerve nos, overlapping lesion of brain and central nervous system, nervous system nos, thyroid gland, adrenal gland cortex, adrenal gland medulla, adrenal gland nos, parathyroid gland, pituitary gland, craniopharyngeal duct, pineal gland, carotid body, aortic body, overlapping lesion of endocrine glands and related structures, endocrine gland nos, head face or neck nos, thorax nos, abdomen nos, pelvis nos, upper limb nos, lower limb nos, other illdefined sites, overlapping lesion of ill-defined sites, lymph node face head neck, intrathoracic lymph node, intra-abdominal lymph nodes, lymph node axilla arm, lymph node inguinal region leg, lymph node pelvic, lymph nodes of multiple regions, lymph node nos, unknown primary site,

The subjects treated with the presently disclosed and claimed compounds may be treated in combination with other non-surgical anti-proliferative (e.g., anti-cancer) drug therapy. In one embodiment, the compounds may be administered in combination with an anti-cancer compound such as a cytostatic compound. A cytostatic compound is a compound (e.g., a small molecule, a nucleic acid, or a protein) that suppresses cell growth and/or proliferation. In some embodiments, the cytostatic compound is directed towards the malignant cells of a tumor. In yet other embodiments, the cytostatic compound is one which inhibits the growth and/or proliferation of vascular smooth muscle cells or fibroblasts.

Suitable anti-proliferative drugs or cytostatic compounds to be used in combination with the presently disclosed and claimed compounds include anti-cancer drugs. Numerous anti-cancer drugs which may be used are well known and include, but are not limited to: Acivicin; Aclarubicin; Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Fluorocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta-I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Niraparib; Nocodazole; Nogalamycin; Olaparib; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Rucaparib; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talazoparib; Talisomycin; Taxol; Taxotere; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Tubulozole Hydrochloride; Uracil Mustard; Uredepa; Vapreotide; Velaparib; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; and Zorubicin Hydrochloride.

Other anti-cancer drugs include, but are not limited to: 20-epi-1,25 dihydroxyvitamin D3; 5-ethynyluracil; abiraterone; acylfulvene; adecypenol; adozelesin; ALL-TK antagonists; ambamustine; amidox; amifostine; aminolevulinic acid; amrubicin; anagrelide; andrographolide; angiogenesis inhibitors; antagonist D; antagonist G; antarelix; anti-dorsalizing morphogenetic protein-1; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bisaziridinylspermine; bisnafide; bistratene A; breflate; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; casein kinase inhibitors (ICOS); castanospermine; cecropin B; cetrorelix; chlorins; chloroquinoxaline sulfonamide; cicaprost; cis-porphyrin; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; dehydrodidemnin B; deslorelin; dexifosfamide; dexrazoxane; dexverapamil; didemnin B; didox; diethylnorspennine; dihydro-5-azacytidine; dihydrotaxol, 9-; dioxamycin; diphenyl spiromustine; docosanol; dolasetron; doxifluridine; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; filgrastim; finasteride; flavopiridol; flezelastine; fluasterone; fludarabine; fluorodaunorunicin hydrochloride; forfenimex; formestane; fotemustine; gadolinium texaphyrin; gallium nitrate; galocitabine; ganirelix; gelatinase inhibitors; glutathione inhibitors; hepsulfam; heregulin; hexamethylene bisacetamide; hypericin; ibandronic acid; idoxifene; idramantone; ilmofosine; ilomastat; imidazoacridones; imiquimod; immunostimulant peptides; insulin-like growth factor-I receptor inhibitor; interferon agonists; interferons; interleukins; iobenguane; iododoxorubicin; ipomeanol, 4-; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anti cancer compound; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; saintopin; SarCNU; sarcophytol A; sargramostim; Sdi 1 mimetics; senescence derived inhibitor 1; sense oligonucleotides; signal transduction inhibitors; signal transduction modulators; single chain antigen binding protein; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temozolomide; tetrachlorodecaoxide; tetrazomine; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; titanocene dichloride; topsentin; toremifene; totipotent stem cell factor; translation inhibitors; tretinoin; triacetyluridine; triciribine; tropisetron; turosteride; tyrosine kinase inhibitors; tyrphostins; UBC inhibitors; ubenimex; urogenital sinus-derived growth inhibitory factor; urokinase receptor antagonists; variolin B; vector system, erythrocyte gene therapy; velaresol; veramine; verdins; vinorelbine; vinxaltine; vitaxin; zanoterone; zilascorb; and zinostatin stimalamer.

The presently disclosed and claimed compounds can also be used in combination with any of the following treatments:
Therapy in combination with inhibitors of Poly(ADP-ribose) polymerases (PARP), a class of chemotherapeutic agents directed at targeting cancers with defective DNA-damage repair (Yuan, et al., Expert Opin Ther Pat, 2017, 27: 363). Such PARP inhibitors include but are not limited to olaparib, rupacarib, velaparib, niraparib, talazoparib, pamiparib, iniparib, E7449, and A-966492.

Therapy in combination with inhibitors of signaling pathways and mechanisms leading to repair of DNA single and double strand breaks as e.g. nuclear factor-kappaB signaling (Pilie, et al., Nat Rev Clin Oncol, 2019, 16: 81; Zhang, et al., Chin J Cancer, 2012, 31: 359). Such inhibitors include but are not limited to inhibitors of ATM and ATR kinases, checkpoint kinase 1 and 2, DNA-dependen protein kinase, and WEE1 kinase (Pilie, et al., Nat Rev Clin Oncol, 2019, 16: 81).

Therapy in combination with an immunomodulator (Khalil, et al., Nat Rev Clin Oncol, 2016, 13: 394), a cancer vaccine (Hollingsworth, et al., NPJ Vaccines, 2019, 4: 7), an immune checkpoint inhibitor (e.g. PD-1, PD-L1, CTLA-4-inhibitor) (Wei, et al., Cancer Discov, 2018, 8: 1069), a Cyclin-D-Kinase 4/6 inhibitor (Goel, et al., Trends Cell Biol, 2018, 28: 911), an antibody being capable of binding to a tumor cell and/or metastases and being capable of inducing antibody-dependent cellular cytotoxicity (ADCC) (Kellner, et al., Transfus Med Hemother, 2017, 44: 327), a T cell- or NK cell engager (e.g. bispecific antibodies) (Yu, et al., J Cancer Res Clin Oncol, 2019, 145: 941), a cellular therapy using expanded autologous or allogeneic immune cells (e.g. chimeric antigen receptor T (CAR-T) cells) (Khalil, et al., Nat Rev Clin Oncol, 2016, 13: 394). Immune checkpoint inhibitors induce but are not limited to nivolumab, ipilimumab, pembrolizumab, atezolizumab, avelumab, durvalumab, and cemiplimab.

According to the present invention, the compounds may be administered prior to, concurrent with, or following other anti-cancer compounds. The administration schedule may involve administering the different agents in an alternating fashion. In other embodiments, the compounds may be delivered before and during, or during and after, or before and after treatment with other therapies. In some cases, the compound is administered more than 24 hours before the administration of the other anti-proliferative treatment. In other embodiments, more than one anti-proliferative therapy may be administered to a subject. For example, the subject may receive the present compounds, in combination with both surgery and at least one other anti-proliferative compound. Alternatively, the compound may be administered in combination with more than one anti-cancer drug.

In an embodiment, the compounds of the present invention are used to detect cells and tissues overexpressing the uPAR, whereby such detection is achieved by conjugating a detectable label to the compounds of the invention, preferably a detectable radionuclide. In a preferred embodiment, the cells and tissues detected are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) or a non-oncology indication (e.g. inflammatory disease, cardiovascular disease, autoimmune disease, and fibrotic disease).

In another embodiment, the compounds of the present invention are used to treat cells and tissues overexpressing the uPAR. In a preferred embodiment, the cells and tissues treated are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) and the therapeutic activity is achieved by conjugating therapeutically active effector to the compounds of the present invention, preferably a therapeutically active radionuclide. In a further preferred embodiment, the diseased cells and tissues are causing and/or are part of a non-oncology indication (e.g. rheumatoid arthritis, infectious disease) and the therapeutic activity is either achieved by targeting of the uPAR or it is monitored by uPAR imaging.

In a further embodiment, particularly if the disease is a non-oncology disease or a non-oncology indication (e.g. metabolic diseases, insulin resistance, type 1 and type 2 diabetes), the compounds of the present invention are administered in therapeutically effective amounts; preferably the compound of the present invention does not comprise a therapeutically active nuclide such as a therapeutically active nuclide An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In other embodiments, a therapeutically effective amount will be an amount necessary to extend the dormancy of micrometastases or to stabilize any residual primary tumor cells following surgical or drug therapy.

Generally, when using an unconjugated compound without a therapeutically active radionuclide, a therapeutically effective amount will vary with the subject's age, condition, and sex, as well as the nature and extent of the disease in the subject, all of which can be determined by one of ordinary skill in the art. The dosage may be adjusted by the individual physician or veterinarian, particularly in the event of any complication. A therapeutically effective amount is typically, but not limited to, an amount in a range from 0.1 µg/kg to about 2000 mg/kg, or from 1.0 µg/kg to about 1000 mg/kg, or from about 0.1 mg/kg to about 500 mg/kg, or from about 1.0 mg/kg to about 100 mg/kg, in one or more dose administrations daily, for one or more days. If desired, the effective daily dose of the active compound may be administered as two, three, four, five, six, or more sub-doses for example administered separately at appropriate intervals throughout the day, optionally, in unit dosage forms. In some embodiments, the compounds are administered for more than 7 days, more than 10 days, more than 14 days and more than 20 days. In still other embodiments, the compound is administered over a period of weeks, or months. In still other embodiments, the compound is delivered on alternate days. For example, the agent is delivered every two days, or every three days, or every four days, or every five days, or every six days, or every week, or every month.

In a preferred embodiment, the compound of the present invention is for use in the treatment and/or prevention of a disease, whereby such treatment is radionuclide therapy.

Preferably, radionuclide therapy makes use of or is based on different forms of radiation emitted by a radionuclide. Such radiation can, for example, be any one of radiation of photons, radiation of electrons including but not limited to β⁻-particles and Auger-electrons, radiation of protons, radiation of neutrons, radiation of positrons, radiation of α-particles or an ion beam. Depending on the kind of particle or radiation emitted by said radionuclide, radionuclide therapy can, for example, be distinguished as photon radionuclide therapy, electron radionuclide therapy, proton radionuclide therapy, neutron radionuclide therapy, positron radionuclide therapy, α-particle radionuclide therapy or ion beam radionuclide therapy. All of these forms of radionuclide therapy are encompassed by the present invention, and all of these forms of radionuclide therapy can be realized by the compound of the invention, preferably under the proviso that the radionuclide attached to the compound of the invention, more preferably as an effector, is providing for this kind of radiation.

Radionuclide therapy preferably works by damaging the DNA of cells. The damage is caused by a photon, electron, proton, neutron, positron, α-particle or ion beam directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high pressure oxygen tanks, blood substitutes that carry increased oxygen, hypoxic cell radiosensitizers such as misonidazole and metronidazole, and hypoxic cytotoxins, such as tirapazamine may be applied.

Other factors that are considered when selecting a radioactive dose include whether the patient is receiving chemotherapy, whether radiation therapy is being administered before or after surgery, and the degree of success of surgery.

The total radioactive dose may be fractionated, i.e. spread out over time in one or more treatments for several important reasons. Fractionation allows normal cells time to recover, while tumor cells are generally less efficient in repair between fractions. Fractionation also allows tumor cells that were in a relatively radio-resistant phase of the cell cycle during one treatment to cycle into a sensitive phase of the cycle before the next fraction is given. Similarly, tumor cells that were chronically or acutely hypoxic and, therefore, more radioresistant, may reoxygenate between fractions, improving the tumor cell kill.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

It is important to distinguish radiosensitivity of a particular tumor, which to some extent is a laboratory measure, from "curability" of a cancer by an internally delivered radioactive dose in actual clinical practice. For example, leukemias are not generally curable with radiotherapy, because they are disseminated through the body. Lymphoma may be radically curable if it is localized to one area of the body. Similarly, many of the common, moderately radioresponsive tumors can be treated with curative doses of radioactivity if they are at an early stage. This applies, for example, to non-melanoma skin cancer, head and neck cancer, non-small cell lung cancer, cervical cancer, anal cancer, prostate cancer.

The response of a tumor to radiotherapy is also related to its size. For complex reasons, very large tumors respond less well to radiation than smaller tumors or microscopic disease. Various strategies are used to overcome this effect. The most common technique is surgical resection prior to radiotherapy. This is most commonly seen in the treatment of breast cancer with wide local excision or mastectomy followed by adjuvant radiotherapy. Another method is to shrink the tumor with neoadjuvant chemotherapy prior to radical radionuclide therapy. A third technique is to enhance the radiosensitivity of the cancer by giving certain drugs during a course of radiotherapy. Examples of radiosensiting drugs include, but are not limited to Cisplatin, Nimorazole, and Cetuximab.

Introperative radiotherapy is a special type of radiotherapy that is delivered immediately after surgical removal of the cancer. This method has been employed in breast cancer (TARGeted Introperative radioTherapy), brain tumors and rectal cancers.

Radionuclide therapy is in itself painless. Many low-dose palliative treatments cause minimal or no side effects. Treatment to higher doses may cause varying side effects during treatment (acute side effects), in the months or years following treatment (long-term side effects), or after re-treatment (cumulative side effects). The nature, severity, and longevity of side effects depends on the organs that receive the radiation, the treatment itself (type of radionuclide, dose, fractionation, concurrent chemotherapy), and the patient.

It is within the present inventions that the method for the treatment of a disease of the invention may realize each and any of the above strategies which are as such known in the art, and which insofar constitute further embodiments of the invention.

It is also within the present invention that the compound of the invention is used in a method for the diagnosis of a disease as disclosed herein. Such method, preferably, comprises the step of administering to a subject in need thereof a diagnostically effective amount of the compound of the invention.

In accordance with the present invention, an imaging method is selected from the group consisting of scintigraphy, Single Photon Emission Computed Tomography (SPECT) and Positron Emission Tomography (PET).

Scintigraphy is a form of diagnostic test or method used in nuclear medicine, wherein radiopharmaceuticals are internalized by cells, tissues and/or organs, preferably internalized *in vivo,* and radiation emitted by said internalized radiopharmaceuticals is captured by external detectors (gamma cameras) to form and display two-dimensional images. In contrast thereto, SPECT and PET form and display three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled chemical emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled chemical. To acquire SPECT images, the gamma camera is rotated around the patient. Projections are acquired at defined points during the rotation, typically every 3-6 degrees. In most cases, a full 360 degrees rotation is used to obtain an optimal reconstruction. The time taken to obtain each projection is also variable, but 15 - 20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical status or metabolic activity of cells within the human body. PET is unique since it produces images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical processes are also altered by a disease, and may occur before any gross changes in anatomy. PET is an imaging technique that can visualize some of these early biochemical changes. PET scanners rely on radiation emitted from the patient to create the images. Each patient is given a minute amount of a radioactive pharmaceutical that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present inventions that the method for the diagnosis of a disease of the invention may realize each and any of the above strategies which are as such known in the art, and which insofar constitute further embodiments of the invention.

Compounds of the present invention are useful to stratify patients, i.e., to create subsets within a patient population that provide more detailed information about how the patient will respond to a given drug. Stratification can be a critical component to transforming a clinical trial from a negative or neutral outcome to one with a positive outcome by identifying the subset of the population most likely to respond to a novel therapy.

Stratification includes the identification of a group of patients with shared "biological" characteristics to select the optimal management for the patients and achieve the best possible outcome in terms of risk assessment, risk prevention and achievement of the optimal treatment outcome.

A compound of the present invention may be used to assess or detect, a specific disease as early as possible (which is a diagnostic use), the risk of developing a disease (which is a susceptibility/risk use), the evolution of a disease including indolent vs. aggressive (which is a prognostic use) and it may be used to predict the response and the toxicity to a given treatment (which is a predictive use).

It is also within the present invention that the compound of the invention is used in a theragnostic method. The concept of theragnostics is to combine a therapeutic agent with a corresponding diagnostic test that can increase the clinical use of the therapeutic drug. The concept of theragnostics is becoming increasingly attractive and is widely considered the key to improving the efficiency of drug treatment by helping doctors identify patients who might profit from a given therapy and hence avoid unnecessary treatments.

The concept of theragnostics is to combine a therapeutic agent with a diagnostic test that allows doctors to identify those patients who will benefit most from a given therapy. In an embodiment and as preferably used herein, a compound of the present invention is used for the diagnosis of a patient, i.e. identification and localization of the primary tumor mass as well as potential local and distant metastases. Furthermore, the tumor volume can be determined, especially utilizing three-dimensional diagnostic modalities such as SPECT or PET. Only those patients having uPAR-positive tumor masses and who, therefore, might profit from a given therapy are selected for a particular therapy and hence unnecessary treatments are avoided. Preferably, such therapy is a uPAR-targeted therapy using a compound of the present invention. In one particular embodiment, chemically identical tumor-targeted diagnostics, preferably imaging diagnostics for scintigraphy, PET or SPECT and radiotherapeutics are applied. Such compounds only differ in the radionuclide and therefore usually have a very similar if not identical pharmacokinetic profile. This can be realized using a chelator and a diagnostic or therapeutic radiometal. Alternatively, this can be realized using a precursor for radiolabeling and radiolabeling with either a diagnostic or a therapeutic radionuclide. In one embodiment diagnostic imaging is used preferably by means of quantification of the radiation of the diagnostic radionuclide and subsequent dosimetry which is known to those skilled in the art and the prediction of drug concentrations in the tumor compared to vulnerable side effect organs. Thus, a truly individualized drug dosing therapy for the patient is achieved.

In an embodiment and as preferably used herein, the theragnostic method is realized with only one theragnostically active compound such as a compound of the present invention labeled with a radionuclide emitting diagnostically detectable radiation (e.g. positrons or gamma rays) as well as therapeutically effective radiation (e.g. electrons or alpha particles).

The invention also contemplates a method of intraoperatively identifying/disclosing diseased tissues expressing uPAR in a subject. Such method uses a compound of the invention, whereby such compound of the invention preferably comprises as Effector a diagnostically active agent.

According to a further embodiment of the invention, the compound of the invention, particularly if complexed with a radionuclide, may be employed as adjunct or adjuvant to any other tumor treatment including, surgery as the primary method of treatment of most isolated solid cancers, radiation therapy involving the use of ionizing radiation in an attempt to either cure or improve the symptoms of cancer using either sealed internal sources in the form of brachytherapy or external sources, chemotherapy such as alkylating agents, antimetabolites, anthracyclines, plant alkaloids, topoisomerase inhibitors, and other antitumor agents, hormone treatments that modulate tumor cell behavior without directly attacking those cells, targeted agents which directly target a molecular abnormality in certain types of cancer including monoclonal antibodies and tyrosine kinase inhibitors, angiogenesis inhibitors, immunotherapy, cancer vaccination, palliative care including actions to reduce the physical, emotional, spiritual, and psychosocial distress to improve the patient's quality of life and alternative treatments including a diverse group of health care systems, practices, and products that are not part of conventional medicine.

In an embodiment of the methods of the invention, the subject is a patient. In an embodiment, a patient is a subject which has been diagnosed as suffering from or which is suspected of suffering from or which is at risk of suffering from or developing a disease, whereby the disease is a disease as described herein and preferably a disease involving the uPAR.

It will be acknowledged and appreciated by a person skilled in the art that determining dosages and desgining dosing regimens are within the ordinary skills of a person of the art.

Dosages employed in practicing the methods for treatment and diagnosis, respectively, where a radionuclide is used and more specifically attached to or part of the compound of the invention will vary depending, e.g., on the particular condition to be treated, for example the known radiosensitivity of the tumor type, the volume of the tumor and the therapy desired. In general, the dose is calculated on the basis of radioactivity distribution to each organ and on observed target uptake.

A γ-emitting complex may be administered once or at several times for diagnostic imaging. In animals, an indicated dose range may be from 0.1 ng/kg to 5 mg/kg of the compound of the invention complexed, e.g., with 1 kBq to 200 MBq of a γ-emitting radionuclide, including, but not limited to, ¹¹¹In or ⁸⁹Zr. For instance, an indicated dose range of the compound of the invention when complexed with a γ-emitting radionuclide may be from 0.2 mg/kg to 2 mg/kg, e.g., from 0.4 mg/kg to 1 mg/kg, such as about 0.6 mg/kg or 0.8 mg/kg. In an embodiment, an indicated dose range of the compound of the invention when complexed with a γ-emitting radionuclide is from 0.1 µg/kg to 10.0 µg/kg, e.g., 0.1 µg/kg to 5.0 µg/kg, e.g., 0.1 µg/kg to 2.0 µg/kg such as about 0.5 µg/kg, or 0.8 µg/kg, or 1.0 µg/kg.

An γ- or β-emitting complex of the compound of the invention may be administered at several time points, e.g., 1 dose about every 28 days, e.g., over a period of 1 to 3 weeks or longer e.g., over a period of 16 to 32 weeks. In animals, an indicated dosage range may be of from 0.1 ng/kg to 5 mg/kg of the compound of the invention complexed, e.g., with 1 kBq to 200 MBq of an γ- or β-emitting radionuclide, including, but not limited to, ²²⁵Ac or ¹⁷⁷Lu. For instance, an indicated dose range of the compound of the invention when complexed with an γ- or β-emitting radionuclide may be from 0.2 mg/kg to 2 mg/kg, e.g., from 0.4 mg/kg to 1 mg/kg, such as about 0.6 mg/kg or 0.8 mg/kg. In larger mammals, for example humans, an indicated dosage range is from 0.1 to 100 µg/kg, e.g., 0.1 µg/kg to 10.0 µg/kg, e.g., 0.1 µg/kg to 5.0 µg/kg, e.g., such as about 1.0 µg/kg, or 2.0 µg/kg, or 4.0 µg/kg, of the compound of the invention complexed with, e.g., 10 to 400 MBq ¹¹¹In or ⁸⁹Zr.

In larger mammals, for example humans, an indicated dosage range is of from 0.1 ng/kg to 100 µg/kg of the compound of the invention complexed with, e.g., 1 to 100000 MBq of an α- or β-emitting radionuclide, including, but not limited to, ²²⁵Ac or ¹⁷⁷Lu. Preferably, in larger mammals, for example humans, an indicated dosage range of the compound of the invention when complexed with a β-emitting radionuclide such as ¹⁷⁷Lu, e.g., with 200 to 50000 MBq, preferably 500 to 20000 MBq, more preferably 1000 to 15000 MBq, such as 1500 to 10000 MBq of β-emitting radionuclide, is from 0.01 µg/kg to 80 µg/kg, more preferably from 0.1 µg/kg to 50 µg/kg, such as about 1.0 µg/kg to 35 µg/kg, or 2.0 µg/kg to 20 µg/kg.

In one embodiment, in larger mammals, for example humans, the effective dose resulting from, e.g., the intravenous administration of the compound of the invention complexed with, e.g., 1 to 100000 MBq of an γ- or β-emitting radionuclide, including, but not limited to, ²²⁵Ac or ¹⁷⁷Lu, is from 0.01 mSv/MBq to 10.0 mSv/MBq, e.g., 0.1 mSv/MBq to 1 mSv/MBq, such as about 0.1 mSv/MBq to 0.5 mSv/MBq, or 0.2 mSv/MBq to 0.3 mSv/MBq. In one further embodiment, in larger mammals, for example humans, the effective dose resulting from, e.g., the intravenous administration of the compound of the invention complexed with a β-emitting radionuclide such as ¹⁷⁷Lu is typically less than 5.0 mSv/MBq, more typically less than 2.0 mSv/MBq, even more typically less than 1.0 mSv/MBq, and most typically less than 0.5 mSv/MBq. Furthermore, in this embodiment, the effective dose may be 0.05 mSv/MBq or more, e.g., 0.08 mSv/MBq or more, e.g., 0.1 mSv/MBq or more. In another embodiment, the effective dose resulting from, e.g., the intravenous administration of the compound of the invention complexed with a β-emitting radionuclide such as ¹⁷⁷Lu is less than 1.0 mSv/MBq, e.g., less than 0.5 mSv/MBq, e.g., 0.35 mSv/MBq, such as about 0.25 mSv/MBq. Furthermore, in this embodiment, the effective dose may be 0.1 mSv/MBq or more, e.g., 0.1 mSv/MBq or more, e.g., 0.15 mSv/MBq or more. In another embodiment, in larger mammals, for example humans, the radiation dose delivered to a tumor after, e.g., the intravenous administration of the compound of the invention complexed with a β-emitting radionuclide such as ¹⁷⁷Lu results ranges from about 4.4 to about 660 Gy.

In a further aspect, the instant invention is related to a composition and a pharmaceutical composition in particular, comprising the compound of the invention.

The pharmaceutical composition of the present invention comprises at least one compound of the invention and, optionally, one or more carrier substances, excipients and/or adjuvants. The pharmaceutical composition may additionally comprise, for example, one or more of water, buffers such as, *e.g.,* neutral buffered saline or phosphate buffered saline, ethanol, mineral oil, vegetable oil, dimethylsulfoxide, carbohydrates such as *e.g.,* glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical composition of the invention.

The pharmaceutical composition of the invention may be formulated for any appropriate route of administration, including, for example, topical such as, e.g., transdermal or ocular, oral, buccal, nasal, vaginal, rectal or parenteral administration. The term parenteral as preferably used herein includes subcutaneous, intradermal, intravascular such as, e.g., intravenous, intramuscular, intrathecal and intraperitoneal injection, as well as any similar injection or infusion technique. A preferred route of administration is intravenous administration.

In an embodiment of the invention the compound of the invention comprising a radionuclide is administered by any conventional route, in particular intravenously, e.g., in the form of injectable solutions or suspensions. The compound of the invention may also be administered advantageously by infusion, e.g., by an infusion of 30 to 60 min.

Depending on the site of the tumor, the compound of the invention may be administered as close as possible to the tumor site, e.g., by means of a catheter. Such administration may be carried out directly into the tumor tissue or into the surrounding tissue or into the afferent blood vessels. The compound of the invention may also be administered repeatedly in doses, preferably in divided doses.

According to a preferred embodiment of the invention, a pharmaceutical composition of the invention comprises a stabilizer, e.g., a free radical scavenger, which inhibits autoradiolysis of the compound of the invention. Suitable stabilizers include, e.g., serum albumin, ascorbic acid, retinol, gentisic acid or a derivative thereof, or an amino acid infusion solution such, e.g., used for parenteral protein feeding, preferably free from electrolyte and glucose, for example a commercially available amino acid infusion such as Proteinsteril^{®} KE Nephro. Ascorbic acid and gentisic acid are preferred.

A pharmaceutical composition of the invention may comprise further additives, e.g., an agent to adjust the pH between 7.2 and 7.4, e.g., sodium or ammonium acetate or Na₂HPO₄. Preferably, the stabilizer is added to the non-radioactive compound of the invention and introduction of the radionuclide, for instance the complexation with the radionuclide, is performed in the presence of the stabilizer, either at room temperature or, preferably, at a temperature of from 40 to 120° C. The complexation may conveniently be performed under air free conditions, e.g., under N₂ or Ar. Further stabilizer may be added to the composition after complexation.

Excretion of the compound of the invention, particularly if the Effector is a radionuclide, essentially takes place through the kidneys. Further protection of the kidneys from radioactivity accumulation may be achieved by administration of lysine or arginine or an amino acid solution having a high content of lysine and/or arginine, e.g., a commercially available amino acid solution such as Synthamin^{®}-14 or-10, prior to the injection of or together with the compound of the invention, particularly if the Effector is a radionuclide. Protection of the kidneys may also be achieved by administration of plasma expanders such as e.g., gelofusine, either instead of or in addition to amino acid infusion. Protection of the kidneys may also be achieved by administration of diuretics providing a means of forced diuresis which elevates the rate of urination. Such diuretics include high ceiling loop diuretics, thiazides, carbonic anhydrase inhibitors, potassium-sparing diuretics, calcium-sparing diuretics, osmotic diuretics and low ceiling diuretics. A pharmaceutical composition of the invention may contain, apart from a compound of the invention, at least one of these further compounds intended for or suitable for kidney protection, preferably kidney protection of the subject to which the compound of the invention is administered.

It will be understood by a person skilled in the art that the compound of the invention is disclosed herein for use in various methods. It will be further understood by a person skilled in the art that the composition of the invention and the pharmaceutical composition of the invention can be equally used in said various methods. It will also be understood by a person skilled in the art that the composition of the invention and the pharmaceutical composition are disclosed herein for use in various methods. It will be equally understood by a person skilled in the art that the compound of the invention can be equally used in said various methods.

It will be acknowledged by a person skilled in the art that the composition of the invention and the pharmaceutical composition of the invention contain one or more further compounds in addition to the compound of the invention. To the extent that such one or more further compounds are disclosed herein as being part of the composition of the invention and/or of the pharmaceutical composition of the invention, it will be understood that such one or more further compounds can be administered separately from the compound of the invention to the subject which is exposed to or the subject of a method of the invention. Such administration of the one or more further compounds can be performed prior, concurrently with or after the administration of the compound of the invention. It will also be acknowledged by a person skilled in the art that in a method of the invention, apart from a compound of the invention, one or more further compound may be administered to a subject. Such administration of the one or more further compounds can be performed prior, concurrently with or after the administration of the compound of the invention. To the extent that such one or more further compounds are disclosed herein as being administered as part of a method of the invention, it will be understood that such one or more further compounds are part of a composition of the invention and/or of a pharmaceutical composition of the invention. It is within the present invention that the compound of the invention and the one or more further compounds may be contained in the same or a different formulation. It is also within the present invention that the compound of the invention and the one or more further compounds are not contained in the same formulation, but are contained in the same package containing a first formulation comprising a compound of the invention, and a second formulation comprising the one or more further compounds, whereby the type of formulation may be the same or may be different.

It is within the present invention that more than one type of a compound of the invention is contained in the composition of the invention and/or the pharmaceutical composition of the invention. It is also within the present invention that more than one type of a compound of the invention is used, preferably administered, in a method of the invention.

It will be acknowledged that a composition of the invention and a pharmaceutical composition of the invention may be manufactured in conventional manner.

Radiopharmaceuticals have decreasing content of radioactivity with time, as a consequence of the radioactive decay. The physical half-life of the radionuclide is often short for radiopharmaceutical diagnostics. In these cases, the final preparation has to be done shortly before administration to the patient. This is in particular the case for positron emitting radiopharmaceuticals for tomography (PET radiopharmaceuticals). It often leads to the use of semi-manufactured products such as radionuclide generators, radioactive precursors and kits.

Preferably, a kit of the invention comprises apart from one or more than one compounds of the invention typically at least one of the followings: instructions for use, final preparation and/or quality control, one or more optional excipient(s), one or more optional reagents for the labeling procedure, optionally one or more radionuclide(s) with or without shielded containers, and optionally one or more device(s), whereby the device(s) is/are selected from the group comprising a labeling device, a purification device, an analytical device, a handling device, a radioprotection device or an administration device.

Shielded containers known as "pigs" for general handling and transport of radiopharmaceutical containers come in various configurations for holding radiopharmaceutical containers such as bottles, vials, syringes, etc. One form often includes a removable cover that allows access to the held radiopharmaceutical container. When the pig cover is in place, the radiation exposure is acceptable.

A labeling device is selected from the group of open reactors, closed reactors, microfluidic systems, nanoreactors, cartridges, pressure vessels, vials, temperature controllable reactors, mixing or shaking reactors and combinations thereof.

A purification device is preferably selected from the group of ion exchange chromatography columns or devices, size-exclusion chromatography columns or devices, affinity chromatography columns or devices, gas or liquid chromatography columns or devices, solid phase extraction columns or devices, filtering devices, centrifugations vials columns or devices.

An analytical device is preferably selected from the group of tests or test devices to determine the identity, radiochemical purity, radionuclidic purity, content of radioactivity and specific radioactivity of the radiolabelled compound.

A handling device is preferably selected from the group consisting of devices for mixing, diluting, dispensing, labeling, injecting and administering radiopharmaceuticals to a subject.

A radioprotection device is used in order to protect doctors and other personnel from radiation when using therapeutic or diagnostic radionuclides. The radioprotection device is preferably selected from the group consisting of devices with protective barriers of radiation-absorbing material selected from the group consisting of aluminum, plastics, wood, lead, iron, lead glass, water, rubber, plastic, cloth, devices ensuring adequate distances from the radiation sources, devices reducing exposure time to the radionuclide, devices restricting inhalation, ingestion, or other modes of entry of radioactive material into the body and devices providing combinations of these measures.

An administration device is preferably selected from the group of syringes, shielded syringes, needles, pumps, and infusion devices. Syringe shields are commonly hollow cylindrical structures that accommodate the cylindrical body of the syringe and are constructed of lead or tungsten with a lead glass window that allows the handler to view the syringe plunger and liquid volume within the syringe.

It will be appreciated and acknowledged by a person skilled in the art that, in an embodiment, a compound including a compound of the present invention is referred to by a chemical name and a structural formula. In connection with such embodiment the compound referrd to or characterized by its chemical name is a first preferred embodiment of such embodiment and the compound referrd to or characterized by its structural formula is a second preferred embodiment of such embodiment. For the avoidance of doubt, if there is a discrepancy between the compound characterized by its chemical name and the compound characterized by its structural formula, the compound characterized by its chemical name is a first embodiment and the compound characterized by its structural formula is a second embodiment.

It will be appreciated and acknowledged by a person skilled in the art that in connection with the present invention any embodiment constitutes an embodiment of each and any aspect of the present invention, including any embodiment of said each and any aspect of the present invention.

It will be appreciated and acknowledged by a person skilled in the art that in connection with the present invention any embodiment of any aspect of the present invention, including any embodiment thereof, constitutes an embodiment of each and any of the other aspects of the present invention, including any embodiment of said each and any aspect of the present invention. It will be further appreciated that any independent embodiments, in particular any independent embodiments of the above list of embodiments constitutes an aspect of the present invention.

The prior art and the present invention is now further illustrated by reference to the following figures and examples from which further features, embodiments and advantages, may be taken, wherein
**Figure 1** **A and B** (prior art) show a comparison of mRNA-expression of PLAUR/uPAR in healthy subjects (white boxes, 17382 samples from 688 donors and 30 tissues, GTEX, black dots, each dot represents one sample) and cancer tissue (black boxes, 9927 samples primary tumor, TCGA, black/white dots, each dot represents one sample); for each sample the percentile for PLAUR expression was calculated and is shown in the Y-axis, wherein tumor abbreviations are: ACC: Adrenocortical Carcinoma; BLCA: Bladder Urothelial Carcinoma; BRCA: Breast Invasive Carcinoma; CESC: Cervical Squamous Cell Carcinoma and Endocervical Adenocarcinoma; CHOL: Cholangiocarcinoma; COAD: Colon Adenocarcinoma; DLBC: Lymphoid Neoplasm Diffuse Large B-cell Lymphoma; ESCA: Esophageal Carcinoma; GBM: Glioblastoma Multiforme; HNSC: Head and Neck Squamous Cell Carcinoma; KICH: Kidney Chromophobe; KIRC: Kidney Renal Clear Cell Carcinoma; KIRP: Kidney Renal Papillary Cell Carcinoma; LAML: Acute Myeloid Leukemia; LGG: Brain Lower Grade Glioma; LIHC: Liver Hepatocellular Carcinoma; LUAD: Lung Adenocarcinoma; LUSC: Lung Squamous Cell Carcinoma; MESO: Mesothelioma; OV: Ovarian Serous Cystadenocarcinoma; PAAD: Pancreatic Adenocarcinoma; PCPG: Pheochromocytoma and Paraganglioma; PRAD: Prostate Adenocarcinoma; READ: Rectum Adenocarcinoma; SARC: Sarcoma; SKCM: Skin Cutaneous Melanoma; STAD: Stomach Adenocarcinoma; TGCT: Testicular Germ Cell Tumors; THCA: Thyroid Carcinoma; THYM: Thymoma; UCEC: Uterine Corpus Endometrial Carcinoma; UCS: Uterine Carcinosarcoma; UVM: Uveal Melanoma;
**Figure 2** (prior art) shows a scheme illustrating the topology of a bio-distribution modifier comprised by the N-terminal modification group A (Fig. 2A) and a bio-distribution modifier comprised by the C-terminal group C-term (Fig. 2B); in case of the bio-distribution modifier comprised by the N-terminal modification group, the bio-distribution modifier comprises a carboxylic acid to which a linking chemical moiety is connected which links one or two first level alkyl groups which bear primary hydroxyl groups or to which second level alkyl groups bearing primary hydroxyl groups are connected; and in case of the bio-distribution modifier comprised by the C-terminal group C-term, the bio-distribution modifier comprises an amino acid with a side chain to which a linking chemical moiety is connected which links one or two first level alkyl groups which bear primary hydroxyl groups or to which second level alkyl groups bearing primary hydroxyl groups are connected;
**Figure 3** shows a representative chromatogram for ¹¹¹In-UPAR-276 at end of synthesis;
**Figure 4** shows a representative chromatogram for ⁶⁸Ga-UPAR-276 at end of synthesis;
**Figure 5** shows a representative chromatogram for ¹⁷⁷Lu-UPAR-249 at end of synthesis indicating full incorporation;

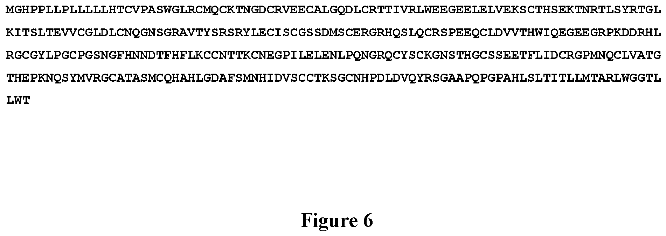

**Figure 6** shows the amino acid sequence of human uPAR (SEQ ID NO: 1);
**Figure** 7 shows the percentage of injected dose per gram of tissue (%ID/g) uptake in the blood pool, kidneys, liver and HEK-uPAR tumor as determined by SPECT-imaging of ¹¹¹In-UPAR-165 (A) and ¹¹¹In-UPAR-181 (B) 1 h, 4 h, 24 h and 48 h post injection into the mouse model;
**Figure 8** shows the percentage of injected dose per gram of tissue (%ID/g) uptake in the blood pool, kidneys, liver and HEK-uPAR tumor as determined by SPECT-imaging of ¹¹¹In-UPAR-192 (A) and ¹¹¹In-UPAR-199 (B) 1 h, 4 h, 24 h and 48 h post injection into the mouse model;
**Figure 9** shows the percentage of injected dose per gram of tissue (%ID/g) uptake in the blood pool, kidneys, liver and HEK-uPAR tumor as determined by SPECT-imaging of ¹¹¹In-UPAR-249 (A) and ¹¹¹In-UPAR-276 (B) 1 h, 4 h, 24 h and 48 h post injection into the mouse model;
**Figure 10** shows the percentage of injected dose per gram of tissue (%ID/g) uptake in the blood pool, kidneys, liver and HEK-uPAR tumor as determined by SPECT-imaging of ¹¹¹In-UPAR-301 (A) and ¹¹¹In-UPAR-294 (B) 1 h, 4 h, 24 h and 48 h post injection into the mouse model;
**Figure 11** shows the percentage of injected dose per gram of tissue (%ID/g) uptake in the blood pool, kidneys, liver and HEK-uPAR tumor as determined by SPECT-imaging of ¹¹¹In-UPAR-280 (A) and ¹¹¹In-UPAR-415 (B) 1 h, 4 h, 24 h and 48 h post injection into the mouse model;
**Figure 12** shows the percentage of injected dose per gram of tissue (%ID/g) uptake in the blood pool, kidneys, liver and HEK-uPAR tumor as determined by SPECT-imaging of ¹¹¹In-UPAR-456 (A) and ¹¹¹In-UPAR-424 (B) 1 h, 4 h, 24 h and 48 h post injection into the mouse model;
**Figure 13** shows the percentage of injected dose per gram of tissue (%ID/g) uptake in the blood pool, kidneys, liver and HEK-uPAR tumor as determined by SPECT-imaging of ¹¹¹In-UPAR-422 (A) and ¹⁷⁷Lu-UPAR-249 (B) 1 h, 4 h, 24 h, 48 h and 72h post injection into the mouse model; and
**Figure 14** shows the percentage of injected dose per gram of tissue (%ID/g) uptake in the blood pool, kidneys, liver and HEK-uPAR tumor as determined by PRT-imaging of ⁶⁸Ga-UPAR-249 (A) and ⁶⁸Ga-UPAR-276 (B) 1 h and 3 h post injection into the mouse model.

### EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter. The synthetic descriptions and specific examples that follow are only intended for the purposes of illustration, and are not to be construed as limiting in any manner to make compounds of the disclosure by other methods.

Abbreviations used in the instant application and the following examples in particular are as follows:

| | |
|---|---|
| 4PL | means four parameter logistic |
| ACE | means Angiotensin Converting Enzyme |
| ACN | means acetonitrile |
| AF488 | means Alexa Fluor 488 Dye |
| Alloc | means allyloxycarbonyl |
| APC | means streptavidin-allophycocyanin |
| Boc | means *tert*-butyloxycarbonyl |
| BPS | means blood pool surrogate, refers to a ROI drawn around the heart ventricle |
| BSA | means bovine serum albumin |
| CT | means computed tomography |
| DAD | means diode array detector/detection |
| DCM | means dichloromethane |
| Dde | means *N*-(1-(4,4-dimethyl-2,6-dioxocyclohexylidene)ethyl) |
| DICOM | means digital imaging and communications in medicine |
| DEG | means PS crosslinked with di-ethylene glycol- dimethacrylate |
| DIC | means diisopropyl carbodiimide |
| DIPEA | means *N*,*N*-diisopropylethylamine |
| DMF | means *N*,*N*-dimethylformamide |
| DMSO | means dimethyl sulfoxide |
| DOTA | means 1,4,7,10-tetraazacyclododecane-1,4,7,10-tetraacetic acid |
| DOTAGA | means 1,4,7,10-tetraazacyclododececane,1-(glutaric acid)-4,7,10-triacetic acid |
| DTPA | means diethylenetriaminepentaacetic acid |
| EDT | means 1,2-ethanedithiol |
| ECE | means endothelin-converting enzyme |
| EDTA | means ethylenediaminetetraacetic acid |
| eq. | means equivalents |
| ESI | means electrospray ionization |
| FACS | means fluorescence-activated cell sorting |
| FBS | means fetal bovine serum |
| Fc | means fragment crystallizable region of an antibody |
| Fmoc | means 9-fluorenylmethoxycarbonyl |
| FMPB | means 4-(4-Formyl-3-methoxyphenoxy)butyryl |
| FOV | means field of view |
| ⁶⁸Ga | means Gallium-68 |
| GuHCl | means guanidinium hydrochloride |
| H₂O | means water |
| HATE | means *O*-(7-azabenzotriazol-1-yl)-*N*,*N*,*N'*,*N'*-tetramethyluronium hexafluorophosphate |
| HCl | means hydrogen chloride |
| HEC-265 | means human endometrial adenocarcinoma cells |
| HEK | means human embryonic kidney |
| HEK-uPAR | means cells of a HEK293 clone stably expressing human uPAR |
| HEPES | means 4-(2-hydroxyethyl)-1-piperazineethanesulfonic acid |
| HOAc | means acetic acid |
| HPLC | means high-performance liquid chromatography |
| HPLC/MS | means high-performance liquid chromatography mass spectrometry |
| IC50 | means half-maximal inhibitory concentration |
| ID | means injected dose |
| ¹¹¹In | means Indium-111 |
| *in vivo* | is latin and means within the living |
| ivDde | means 1-(4,4-dimethyl-2,6-dioxocyclohex-1-ylidene)-3-methylbutyl |
| Kd | means dissociation constant |
| keV | means kilo electron volt |
| LC | means liquid chromatography |
| LC/Q-TOF-MS | means liquid chromatography quadrupole time-of- flight mass spectrometry |
| LC/TOF-MS | means liquid chromatography time-of- flight mass spectrometry |
| LC-MS | means liquid chromatography mass spectrometry |
| ¹⁷⁷Lu | means Lutetium-177 |
| M | means molar, i.e. mol per Liter |
| m/z | means mass divided by charge |
| MBq | means megabecquerel |
| MeOH | means methanol |
| MEM | means minimum essential medium |
| MFI | means median fluorescence intensitiy |
| µg/mL | means microgram per milliliter |
| min | means minute(s) |
| MLEM | means maximum-likelihood expectation-maximization |
| MS | means mass spectrometry |
| MTBE | means methyl-tert-butylether |
| MW | means molecular weight |
| ND | means not determined |
| NEP | means neutral endopeptidase, also known as neprilysin |
| NHS | means *N*-hydroxysuccinimide |
| nm | means nanometer |
| nM | means nanomolar |
| NMP | means *N*-methyl-2-pyrrolidone |
| NMRI nude mice | means Naval Medical Research Institute nude mice |
| NSCLC | means non-small cell lung cancer |
| OSEM | means ordered subset expectation maximization |
| PBS | means phosphate buffered saline |
| PEG | means polyethylene glycol, synonymous for a polyether compound |
| PET | means Positron Emission Tomography |
| pIC50 | means the negative decadic logarithm of the IC50 value when converted to molar |
| pKD | means the negative decadic logarithm of the KD value when converted to molar |
| ppm | means parts per million |
| PS | means polystyrene |
| Pt | means Platinum |
| Q-TOF | means quadrupole time of flight |
| ROI | means region of interest |
| RP | means reversed phase |
| RT | means room temperature |
| RU | means resonance units |
| SCC | means squamous cell carcinoma |
| SCK | means single cycle kinetic |
| SPE | means solid phase extraction |
| SPECT | means single photon emission computed tomography |
| SPPS | means solid phase peptide synthesis |
| SPR | means surface plasmon resonance |
| t_{1/2} | means half-life |
| TBAF | means tetrabutylammonium fluoride |
| tBu | means *tert*-butyl |
| TCEP | means tris(2-carboxyethyl)phosphine |
| TFA | means trifluoroacetic acid or trifluoroacetate |
| THF | means tetrahydrofuran |
| TIPS | means triisopropylsilane |
| TLC | means thin-layer chromatography |
| TOF | means time-of- flight detection |
| t_{R} | means retention time |
| TWEEN-20 | means Polyoxyethylene (20) sorbitan monolaurate |
| UHPLC | means Ultra High Performance Liquid Chromatography |
| uPAR | means urokinase-type plasminogen activator receptor |
| uPAR-Fc | means a fusion protein comprising the extracellular domains of human uPAR C-terminally fused to an Fc fragment of an antibody |
| UV | means ultraviolet |

### Materials, Instruments and Methods

The materials and methods as well as general methods are further illustrated by the following examples.

### Materials:

### Solvents:

Solvents were used in the specified quality without further purification. Acetonitrile (Super Gradient, HPLC, VWR); dichloromethane (synthesis grade, Roth); dimethylsulfoxide (for preparative HPLC: BioScience Grade, Roth - for synthesis: pure, Thermo Scientific), ethyl acetate (synthesis grade, Roth); N,N dimethylformamide (peptide synthesis grade, Biosolve); 1-methyl-2-pyrolidone (peptide grade, IRIS BioTech); methanol (p. a., Merck); cyclohexane (synthesis grade, Roth); methyl-tert-butylether (synthesis grade, Roth); THF (anhydrous, Thermo scientific); water: Milli-Q Plus, Millipore, demineralized.

### Chemicals:

Chemicals were either synthesized according to or in analogy to literature procedures or purchased from Sigma-Aldrich-Merck (Deisenhofen, Germany), Bachem (Bubendorf, Switzerland), BLD Pharmatech GmbH (Kaiserslautern, Germany), VWR (Darmstadt, Germany), Novabiochem (Merck Group, Darmstadt, Germany), Acros Organics (distribution company Fisher Scientific GmbH, Schwerte, Germany), Iris Biotech (Marktredwitz, Germany), Amatek Chemical (Jiangsu, China), Roth (Karlsruhe, Deutschland), Molecular Devices (Chicago, USA), Broadpharm (San Diego, USA), Biochrom (Berlin, Germany), Peptech (Cambridge, MA, USA), Synthetech (Albany, OR, USA), Pharmacore (High Point, NC, USA), PCAS Biomatrix Inc (Saint-Jean-sur-Richelieu, Quebec, Canada), Alfa Aesar (Karlsruhe, Germany), Tianjin Nankai Hecheng S&T Co., Ltd (Tianjin, China), CheMatech (Dijon, France), JenKem Technology (Plano, TX, USA), Polypure (Oslo, Norway), Trimen Chemicals (Lodz, Poland) and Anaspec (San Jose, CA, USA) or other companies and used in the assigned quality without further purification.

AGLU*-OH was synthesized according to a literature procedure (Vlahov et al. J. Org. Chem., 2010, 75, 3685).

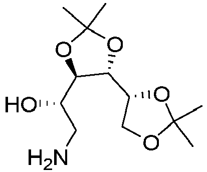

### Solid phase synthesis resins and resin linkers:

Solid-phase synthesis was performed on polystyrene resin (PS - polystyrene cross-linked with 1,4-divinylbenzene or DEG - polystyrene cross-linked with di-ethylene-glycol- dimethacrylate) modified with a Rink amide, 2-chloro-trityl, or FMPB [4-(4-formyl-3-methoxyphenoxy)butyryl] linker.

### Instruments and Instrument-Methods:

### HPLC/MS analyses:

HPLC/MS analyses were performed by injection of 5 µl of a solution of the sample, using a 2-step gradient for all chromatograms (5-65% B in 12 min, followed by 65-90% B in 0.5 min, A: 0.1% TFA in water and B: 0.1% TFA in ACN). RP columns were purchased from Dr. Maisch (ReproSil-Pur 120 C18-AQ, 3 µm, 50 × 3.00 mm, flow 0.8 mL, HPLC at room temperature); Mass spectrometer: Agilent 6230 LC/TOF-MS or Agilent 6530 LC/Q-TOF-MS, ESI ionization. MassHunter Qualitative Analysis B.07.00 SP2 was used as software. UV detection was done at λ = 230 nm. Retention times (Rₜ) are indicated in the decimal system (e.g. 1.9 min = 1 min 54 s) and are referring to detection in the UV spectrometer. For the evaluation of observed compound masses the `Find Compounds by Formula'-feature was used. More precisely, the individual `neutral mass of a compound (in units of Daltons)'-values and the corresponding isotope distribution pattern were used to confirm compound identity. The accuracy of the mass spectrometer was approx. ± 5 ppm.

### Product purification methods - Preparative HPLC:

Preparative HPLC separations were performed on reversed phase columns (General: Kinetex 5µ XB-C18 100 Å, 150 × 30 mm from Phenomenex) as stationary phase. 0.1% TFA in water (A) and 0.1% TFA in ACN (B) were used as mobile phase which were mixed in linear binary gradients. The gradients are described as: "10 to 40% B in 30 min", which means a linear gradient from 10% B (and correspondingly 90% A) to 40% B (and correspondingly 60% A) was run over 30 min. The flow rate was typically 40 mL/min. A typical gradient for the purification of the compounds of the invention started at 15-35% B and ended after 20 min at 35-60% B. The difference between the percentage of B at end and start was at least 10%. A commonly used gradient was "25 to 45% B in 20 min". Samples were preferably dissolved in mixtures of HOAc and water or DMSO.

### Product purification methods - Solid phase extraction (SPE):

In case of solid phase extraction, 250 mg Varian Bondesil-ENV was placed in a 15 mL polystyrene syringe. The column was pre-washed with methanol (1 × 5 mL) and water (3 × 5 mL) before the reaction solution or the solution containing the product to be purified was applied to the column. To remove excess salt, the column was washed again with water (3 × 5 mL Afterwards, the product was eluted with 5 mL of 50% ACN in water (first fraction) followed by at least 5 mL of 50% ACN in water containing 0.1% TFA or until elution of the compound was complete.

### Automated/Semi-automated Solid-Phase Synthesis equipment:

Automated solid-phase synthesis of peptides and polyamides was performed on a Tetras Peptide Synthesizer (Advanced ChemTech) in 25 µmol, 50 µmol or 100 µmol scales. Some manual steps were performed in plastic syringes equipped with frits (material PE, Roland Vetter Laborbedarf OHG, Ammerbuch, Germany). Coupling at elevated temperatures was performed on a Chorus Peptide Synthesizer (Gyros Protein Technologies) or alternatively in a ThermoMixer C machine (Eppendorf SE, Hamburg, Germany). If reactions at elevated temperatures were part of the sequence assembly, usually the whole sequence was synthesized on the Chorus Peptide Synthesizer.

### General procedures for Automated/Semi-automated Solid-Phase Synthesis

The amounts of reagents in the protocols described correspond to the 100 µmol scale, unless stated otherwise.

### Resin loading - Rink amide linker (C-terminal primary amides):

For the synthesis of C-terminal peptide amides (primary amides) the Rink amide linker (on DEG resin - initial resin loading ranging from 0.5 - 0.7 mmol/g) was used. The resin was initially swollen in DMF (5 mL) for at least 30 minutes and subsequently washed with DMF (3 mL, 1 minute). The first building block was loaded onto the linker by performing the procedure for the coupling of carboxylic acid building blocks as described below.

### Resin loading - 2-chloro trityl linker (C-terminal acids, amines or alcohols):

For synthesis of C-terminal peptide acids, amines or alcohols, the 2-chloro trityl linker (on PS resin-initial resin loading 1.8 mmol/g) was used. The resin was initially swollen in DCM (5 mL) for at least 30 minutes and subsequently washed with DCM (3 mL, 1 minute). Then the Fmoc amino acid building block (for a C-terminal acid) or an amine building block such as diethylene amine (for a C-terminal amine) was loaded onto the linker by treating the resin for 1 hour with a mixture of this building block (0.5 mmol, 5 eq.) and DIPEA (623 µL, 3.6 mmol, 36 eq.) in DCM (4 mL). For a C-terminal alcohol, the used building block was an Fmoc amino alcohol (instead of thr Fmoc amino acid) and the reaction was carried out for 12 hours. Afterwards, the resin was washed with methanol (5 mL, 5 minutes) and DMF (3 mL, 3 × 1 minute).

### Resin loading - FMPB linker (C-terminal secondary amides):

For synthesis of C-terminal substituted peptide amides (secondary amides), the FMPB linker [4-(4-formyl-3-methoxyphenoxy)butyryl on PS resin - initial resin loading ~ 1.0 mmol/g) was used. The resin was initially swollen in DMF (5 mL) for at least 30 minutes and subsequently washed with DMF (3 mL, 1 minute). The amine (1.0 mmol, 10 eq. - e.g. n-butylamine) was dissolved in 1% acetic acid in DMF (3 mL). After agitation of the resin for 30 minutes at room temperature, Na(AcO)₃BH (1.0 mmol, 10 eq.) was added and the resin was agitated at 50°C overnight. The resin was washed with DMF, MeOH and DMF (3 mL each, 3x 1 minute). For volatile amines (e.g. ethyl amine), a mixture of 10 eq of the amine hydrochloride and 10 eq of the free amine was employed and the reaction was carried out at room temperature overnight. The first sequence building block was then coupled following the procedure for carboxylic acid building blocks at 50 °C and with an extended coupling time of 2 hours. This coupling was repeated twice.

### Coupling of carboxylic acid building blocks: (e.g. Fmoc amino acids)

Unless stated otherwise, coupling of amino acid building blocks or carboxylic acids in general was performed as follows: to the swollen resin were added the corresponding carboxylic acid building block solution (0.3 M in DMF or NMP, 1.7 mL, 5eq.), the DIPEA solution (0.9 M in DMF, 1.15 mL, 10 eq.), and the HATU solution (0.4 M in DMF, 1.25 mL, 5 eq.). The resin was shaken for 45 min at room temperature. Afterwards the resin was washed with DMF (3 mL, 5 × 1 minute). If necessary, the coupling step was repeated and/or performed at 50°C. For racemization prone building blocks, DIPEA was replaced by 2,4,6-collidine (0.9 M in DMF, 1.15 mL, 10 eq.).

### Coupling of chelator building blocks: (e.g. DOTA, NOTA, or NODAGA)

The corresponding chelator building block (DOTA(tBu)₃-OH or (NOTA(tBu)₂-OH or NODAGA(tBu)₃-OH) was coupled as described for the coupling of ordinary carboxylic acid building blocks but coupling time was increased to 90 min. Afterwards, a DIC solution (3.2 M in DMF, 0.4 mL, 12.5 eq.) was added to the reaction mixture and the resin was agitated for further 90 minutes at room temperature. Afterwards the resin was washed with DMF (3 mL, 5 × 1 minute).

### Acetylation of primary amines (e.g. N-terminal acetylation)

A DIPEA solution (0.9 M in DMF, 1.75 mL, 16 eq.) and an acetic anhydride solution (0.75 M in DMF, 1.75 mL, 13 eq.) were added to the resin, and the latter was shaken for 10 minutes. Afterwards the resin was washed with DMF (3 mL, 5 × 1 minute).

### N-terminal attachment of urea moieties (e.g. n-butyl urea)

A mixture of the corresponding isocyanate (e.g. n-butyl isocyanate) (0.5 mmol, 5 eq.) and DIPEA (1.0 mmol, 10 eq.) in DMF (3 mL) was added to the resin, which was agitated for 2 hours. Afterwards the resin was washed with DMF (3 mL, 5 × 1 minute).

### N-terminal attachment of carbamate moieties (e.g. n-butyl carbamate)

The resin was washed with DCM (3 mL, 3 × 1 minute). A mixture of the corresponding chloroformate (e.g. n-butyl chloroformate) (0.5 mmol, 5 eq.) and DIPEA (1 mmol, 10 eq.) in DCM (3 mL) was added to the resin, which was agitated for 3 hours. Afterwards, the resin was washed with DMF (3 mL, 5 × 1 minute).

### N-terminal attachment of a diacid (e.g. glutaryl moiety)

A DIPEA solution (0.9 M in DMF, 2.3 mL, 20 eq.) and a solution of the diacid anhydride (e.g. glutaric anhydride, 0.3 M in DMF, 1.7 mL, 10 eq.) were added to the resin, and the latter was shaken for 45 minutes. Afterwards the resin was washed with DMF (3 mL, 5 × 1 minute).

### Synthesis of N-terminal sulfonamides (e.g. pentyl sulfonamide)

The resin was washed with DCM (3 mL, 3x1 minute). A mixture of the corresponding sulfonyl chloride (e.g. n-pentyl sulfonyl chloride) (0.6 mmol, 6 eq.) and pyridine (1.2 mmol, 12 eq.) in DCM (4 mL) was added to the resin, which was agitated for 3 hours. The reaction was repeated once. Afterwards, the resin was washed with DMF (3 mL, 5 × 1 minute).

### Synthesis of N-alkylated glycines at the N-terminus (e.g. N-ethyl glycine)

To the resin swollen in DMF was added a mixture of bromoacetic acid (1.0 mmol, 10 eq.) and DIC (0.5 mmol, 5 eq.) in DMF (3 mL). After agitation for 45 minutes, the resin was washed with DMF and DMSO (3 mL, 3 × 1 minute). A mixture of an amine (e.g. ethylamine, 2.5 mmol, 25 eq.) and DIPEA (1.0 mmol, 10 eq.) in DMSO (3 mL) was added and the resin was shaken for 1 hour. Afterwards the resin was washed with DMF (3 mL, 5 × 1 minute).

### Coupling ofNHS esters in solution:

For the coupling of a NHS ester to a peptide precursor in solution, the peptide was dissolved in a minimal volume of DMSO and the pH value of the resulting solution was adjusted to ~8 by careful addition of small volumes of DIPEA. A solution of the NHS ester (e.g. AF488 NHS ester, 1.3 eq. in relation to the amount of peptide) in a minimal volume of DMSO was added to the solution and the the pH value readjusted to 8 by addition of DIPEA. Reaction progress was monitored by LC-MS after approximately 1 hour and if required, more NHS ester was added to drive the reaction to completion. After completion of the reaction, the solution was directly subjected to purification via *Preparative HPLC.*

### Fmoc deprotection:

After swelling in DMF for 30 min, the resin was washed with DMF once (3 mL, 1 × 1 minute), treated with piperidine/DMF (1:4 v/v, 3 mL, 2 and 20 minutes) and subsequently washed with DMF (3 mL, 5x 1 minute).

### Alloc/Allyl deprotection:

DCM was de-oxygenated by passing a stream of nitrogen through the stirred solvent. The oxygen-free solvent was used to wash the resin (3 mL, 3x 1 minute). Then, a mixture of 1,3-dimethylbarbituric acid (0.4 mmol, 4 eq.) and tetrakis(triphenylphosphine)palladium(0) (0.025 mmol, 0.25 eq.) in oxygen-free DCM (4 mL) was added to the resin. The resin was agitated for 30 min and then washed with DCM, MeOH, DMF, 0.5% DIPEA in DMF, 0.5% sodium diethyldithiocarbamate in DMF, DMF and DCM (each washing step was repeated 3 times, 1 minute each).

### DdelivDde deprotection:

After swelling in DMF, the resin was washed with DMF (3 mL, 1x 1 minute), treated with hydrazine-hydrate/DMF (2:98 v/v, 3 mL 2x 10 minutes) and subsequently washed with DMF (3 mL, 5x 1 minute).

### TBDMS deprotection:

After swelling in THF, the resin was washed with THF (3 mL, 1x 1 minute), treated with TBAF in THF (1 M, 3 mL 2x 15 minutes) and subsequently washed thoroughly with THF, DCM, MeOH, water, DMF, DCM (3 mL, 5x 1 minute).

### Preparation of N,N-dimethylamines:

The resin containing a free primary amine (or a methylamine) was swollen in dry THF for 30 min. A solution of formaldehyde (37% in water, 78.7 µL, 1.0 mmol, 10 eq.) and sodium triacetoxyborohydride (212 mg, 1.0 mmol, 10 eq.) in 1% acetic acid in THF (3 mL) was added to the resin and allowed to shake at room termperature overnight. Finally, the resin was washed with DMF, MeOH and DMF (3 mL each, 3x 1 minute).

### Reduction of nitro benzenes to anilines:

The resin was swollen in DMF for 30 min. A solution of SnCl₂ (1 M in DMF, 4 mL) was added to the resin which was shaken at room termperature overnight. Finally, the resin was washed with DMF (3 mL each, 5x 1 minute).

### Alkylation of tertiary amines with 1,3-propane suit one:

To the resin swollen in DMF was added a solution of 1,3-propanesultone (1.0 mmol, 10 eq.) and DIPEA (0.5 mmol, 5 eq.) in DMF (4 mL). The reaction shaken was allowed to shake at 50°C overnight and afterwards washed thoroughly with DMF (3 mL each, 5x 1 minute).

### Solid phase peptide synthesis (SPPS):

After loading of the C-terminal/initial building block onto the resin linker of a resin, the linear sequence of a peptide was assembled by iterative repetition of *Fmoc deprotection* and *Coupling of carboxylic acid building blocks* (Fmoc amino acid building blocks).

Dependent on the desired structure of the target peptide, the following modifications of the N-terminus could be realized:
- a free amine (no further action after the final *'Fmoc deprotection'*)*,*
- a chelator (DOTA or NOTA or NODAGA) was attached by employing the *'Coupling of chelator building blocks'* method,
- an acetyl group was attached to the N-terminus by employing the *'Acetylation of primary amines'* method,
- a carboxylic acid such as hexanoic acid was attached by employing the *'Coupling of carboxylic acid building blocks'* method,
- a urea moiety such as n-butyl urea was generated by employing the *'N-terminal attachment of urea moieties'* method,
- a carbamate moiety such as n-butyl carbamate was generated by employing the *'N-terminal attachment of carbamate moieties'* method,
- a diacid such as glutaric acid was attached by employing the *'N-terminal attachment of a diacid'* method,
- an N-alkylated AA glycine was installed at the N-terminus by following the *'Synthesis of N-alkylated glycines at the N-terminus*' method,
- a sulfonamide such as pentyl sulfonamide was synthesized by employing the *'Synthesis of N-terminal sulfonamides'* method.

### On-resin modification of carboxylic acids with AGLU*-OH:

To the resin swollen in DMF was added a solution of *AGLU*-OH* (0.5 mmol, 5 eq.), Oxyma (0.5 mmol, 5 eq.) and DIC (0.5 mmol, 5 eq.) in DMF (4 mL). The reaction shaken was allowed to shake for 2 hours at room termperature and afterwards washed thoroughly with DMF (3 mL each, 5x 1 minute).

### Complete resin cleavage and deprotection of peptides:

After completion of the assembly of the sequence, the resin was finally washed with DCM (3 mL, 4x 1 minute), dried *in vacuo* overnight and treated with TFA, EDT, water and TIPS (94/2.5/2.5/1 - 4 mL) for 2 hours (or 4 hours in case of sequences containing tBu ester protected chealtors). Afterwards, the cleavage solution was poured into a chilled mixture of MTBE and cyclohexane (1/1, 10-fold excess compared to the volume of cleavage solution), centrifuged at 4 °C for 5 minutes. The residue was lyophilized from water/acetonitrile prior to purification or further modification.

### Cyclization method A: disulfide cyclization:

[Pt(en)₂Cl₂]Cl₂ (Dichlorobis-(ethylendiamine)-platinum(IV) chloride) was added to a solution of the crude peptide precursor in a 1:1 mixture of ammonium acetate buffer (0.1 M, pH 6) and acetonitrile. The reaction mixture was stirred at RT until full conversion was observed by LC-MS. Following addition of TFA, the solvent was removed by lyophilisation and the crude product purified by HPLC. For crude peptide obtained from 100 µmol of initially used resin, 120 mL of the solvent mixture, 45.6 mg (100 µmol) of Pt-reagent and 100 µL of TFA were used.

### Cyclization method B: cyclization with diodomethane:

K₂CO₃ (0.6 mmol, 6 eq.) and TCEP (0.15 mmol, 1.5 eq) were added to a solution of crude peptide precursor (100 µmol based on initially used resin) in water (20 mL) and THF (10 mL). After 5 minutes, triethylamine (0.5 mmol, 5 eq.) and diodomethane (0.8 mmol, 8 eq.) were added and the solution was stirred for 10 minutes at 50 °C. After stirring the reaction mixture for another hour at RT, β-mercaptoethanol (120 µL) was added and the mixture was left to stir for additional 45 minutes. After addition of TFA (100 µL) and acetonitrile (20 mL), the mixture was subjected to lyophilisation.

### Cyclization method C: lactam cyclization on resin:

The resin was swollen in DMF for 30 minutes. A solution of DIC (0.2 mmol, 2 eq.) and Oxyma (0.2 mmol, 2 eq.) in DMF (3 mL) was added to the resin and allowed to shake at room temperature overnight. Afterwards, the resin was washed with DMF (3 mL, 3x 1 minute).

### Cyclization method D: dibromoxylene cyclization

The crude peptide material was dissolved in a 1:1 mixture of ammonium bicarbonate solution (50 mM, pH = 8.5) and acetonitrile. To the resulting mixture was added α,α'-dibromo-*o*-xylene and stirred at RT. Upon completion of the cyclization reaction which was judged by analytical LC-MS, β-mercaptoethanol was added and the solution stirred for a further hour. Then TFA was added, and the solution was subjected to lyophilization. For crude peptide obtained from 100 µmol of initially used resin, 120 mL of the solvent mixture, 28.9 mg (110 µmol, 1.1 eq.) of α,α'-dibromo-o-xylene, 14 µL (200 µmol, 2 eq.) of β-mercaptoethanol and 100 µL of TFA were used.

More relevant Fmoc-solid-phase-peptide synthesis methods are described in detail in "Fmoc Solid Phase Peptide Synthesis" Editors W. Chan, P. White, Oxford University Press, USA, 2000. Compounds were named using MestreNova version 12 Mnova IUPAC Name plugin (Mestrelab Research, S.L.), or AutoNom version 2.2 (Beilstein Informationssysteme Copyright^{©} 1988-1998, Beilstein Institut für Literatur der Organischen Chemie licensed to Beilstein Chemiedaten and Software GmbH), where appropriate.

### Preparation of compounds:

Specific embodiments for the preparation of compounds of the invention are prepared by using the preferred general methods disclosed above, other published methods or methods known by persons skilled in the art. Unless otherwise specified, all starting materials and reagents are either of standard commercial grade and are used without further purification or are readily prepared from such materials by routine methods. Those skilled in the art of organic synthesis will recognize in light of the instant disclosure that starting materials and reaction conditions may be varied including additional steps employed to produce compounds encompassed by the present invention.

### Example 1: Synthesis Ia: Synthesis of compound Pent-[Cys-Cit-Gln-Tyr-Phe-Pro-ala-Ile-Leu-Cys]-NH₂ (UPAR-362)

The linear sequence of the peptide (Pent-Cys-Cit-Gln-Tyr-Phe-Pro-ala-Ile-Leu-Cys-NHz) was assembled according to the *'General procedures for Automated*/*Semi-automated Solid-Phase Synthesis'* in a 25 µmol scale on a DEG Rink amide resin applying the *'Solid phase peptide synthesis (SPPS)'* method with an N-terminal coupling of pentanoic acid as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min - Kinetex) to yield 5.30 mg of the pure title compound (16.4%). HPLC t_{R} = 7.3 min. LC/TOF-MS: exact mass 1294.729 (calculated 1294.620). C₆₀H₉₀N₁₄O₁₄S₂ (MW = 1295.577).

### Example 2: Synthesis Ib: Synthesis of compound DOTA-O2Oc-Leu-[Cys-Cit-Gln-Tyr-Phe-Pro-Pro-Ile-Leu-Cys]-NHz (UPAR-229)

The linear sequence of the peptide (DOTA-O2Oc-Leu-Cys-Cit-Gln-Tyr-Phe-Pro-Pro-Ile-Leu-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with a final N-terminal coupling of DOTA(tBu)₃-OH as described in *'Coupling of chelator building blocks'.* Afterwards, the linear was cleaved from the synthesis resin according to *'Complete resin cleavage and deprotection of peptides"* (cleavage time 4 hours), and the obtained crude material was subjected to cyclization according to *'Cyclization method A: disulfide cyclization".* After lyophilisation of the reaction solution, the crude product was purified by *'Preparative HPLC'* (25 to 45% B in 20 min - Kinetex) to yield 17.68 mg of the pure title compound (18.8%). HPLC t_{R} = 7.4 min. LC/TOF-MS: exact mass 1880.910 (calculated 1880.916). C₈₅H₁₃₂N₂₀O₂₄S₂ (MW = 1882.213).

### Example 3: Synthesis Ic: Synthesis of compound Ac-Phg-[Cys-Arg-Gln-Tyr-Phe-Pro-Hyp-Ile-Nva-Cys]-NH₂ (UPAR-486)

The linear sequence of the peptide (Ac-Phg-Cys-Arg-Gln-Tyr-Phe-Pro-Hyp-Ile-Nva-Cys-NHz) was assembled according to the *'General procedures for Automated*/*Semi-automated Solid-Phase Synthesis'* in a 25 µmol scale on a DEG Rink amide resin applying the *'Solid phase peptide synthesis (SPPS)'* method and introducing the N-terminal acetyl group by the *'Acetylation of primary amines'* method. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min - Kinetex) to yield 4.86 mg of the pure title compound (13.8%). HPLC t_{R} = 7.0 min. LC/TOF-MS: exact mass 1412.638 (calculated 1412.637). C₆₆H₉₂N₁₆O₁₅S₂ (MW = 1413.670).

### Example 4: Synthesis Id: Synthesis of compound Glutar-[Cys-Cit-Gln-Tyr-Phe-Pro-ala-Ile-Leu-Cys]-NH₂ (UPAR-359)

The linear sequence of the peptide (Glutar-Cys-Cit-Gln-Tyr-Phe-Pro-ala-Ile-Leu-Cys-NH₂) was assembled according to the *'General procedures for Automated*/*Semi-automated Solid-Phase Synthesis'* in a 25 µmol scale on a DEG Rink amide resin applying the *'Solid phase peptide synthesis (SPPS)'* method and introducing the N-terminal glutarate residue by reacting gluaric anhydride according to the *'N-terminal attachment of a diacid'* method. After cleavage of the linear peptide from the synthesis resin employing *Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (20 to 40% B in 15 min-Kinetex) to yield 6.76 mg of the pure title compound (20.4%). HPLC t_{R} = 7.1 min. LC/TOF-MS: exact mass 1324.595 (calculated 1324.594). C₆₀H₈₈N₁₄O₁₆S₂ (MW = 1325.559).

### Example 5: Synthesis IIa: Synthesis of compound iHex-[Cys-Lys(DOTA)-Gln-Tyr-Phe-Pro-Pro-Ile-Leu-Cys]-NH₂ (UPAR-466)

The linear sequence of the peptide (iHex-Cys-Lys(Alloc)-Gln-Tyr-Phe-Pro-Pro-Ile-Leu-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with an N-terminal coupling of isohexanoic acid as final step. After removal of the Alloc protecting group from L-lysine employing an *'Alloc*/*Allyl deprotection',* DOTA(tBu)3-OH was coupled employing the *'Coupling of chelator building blocks'* method. Afterwards the branched peptide (iHex-Cys-Lys(DOTA)-Gln-Tyr-Phe-Pro-Pro-Ile-Leu-Cys-NHz) was cleaved from the synthesis resin according to *Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (30 to 50% B in 20 min - Kinetex) to yield 13.14 mg of the pure title compound (15.5%). HPLC t_{R} = 7.9 min. LC/TOF-MS: exact mass 1691.841 (calculated 1691.842). C₇₉H₁₂₁N₁₇O₂₀S₂ (MW = 1693.043).

### Example 6: Synthesis IIb: Synthesis of compound Pent-[Dab-Arg-Gln-Tyr-Phe-Pro-Hyp-Ile-Nva-Glu]-NH₂ (UPAR-390)

The linear sequence of the peptide (Pent-Dab(Alloc)-Arg-Gln-Tyr-Phe-Pro-Hyp-Ile-Nva-Glu(OAll)-NH₂) was assembled according to the *'General procedures for Automated*/*Semi-automated Solid-Phase Synthesis'* in a 50 µmol scale on a DEG Rink amide resin (loading 0.18 mmol/g) applying the *'Solid phase peptide synthesis (SPPS)'* method with an N-terminal coupling of pentanoic acid as final step. After simultaneous removal of the alloc protecting group from the side chain of L-2,4-diaminobutyric acid and the allyl protecting group from the side chain of the C-terminal L-glutamate residue employing the *'Alloc*/*Allyl deprotection'* method, the liberated side chains were reacted using *'Cyclization method C: lactam cyclization on resin*'. Afterwards, the cyclized peptide (Pent-[Dab-Arg-Gln-Tyr-Phe-Pro-Hyp-Ile-Nva-Glu]-NH₂) was cleaved from the synthesis resin according to *Complete resin cleavage and deprotection ofpeptides'* (cleavage time 2 hours). After lyophilisation of the reaction solution, the crude product was purified by *'Preparative HPLC'* (20 to 40% B in 20 min - Kinetex) to yield 2.00 mg of the pure title compound (3.0%). HPLC t_{R} = 6.5 min. LC/TOF-MS: exact mass 1328.725 (calculated 1328.724). C₆₄H₉₆N₁₆O₁₅ (MW = 1329.548).

### Example 7: Synthesis lie: Synthesis of compound Pent-[Cys-Glu(AGLU)-Gln-Tyr-Phe-Pro-Hyp-Ile-Nva-Cys]-NH₂ (UPAR-386)

The linear sequence of the peptide (Pent-Cys-Glu(OAll)-Gln-Tyr-Phe-Pro-Hyp-Ile-Nva-Cys-NH₂) was assembled according to the *'General procedures for Automated*/*Semi-automated Solid-Phase Synthesis'* in a 25 µmol scale on a DEG Rink amide resin applying the *'Solid phase peptide synthesis (SPPS)'* method with coupling of pentanoic acid as final step. After removal of the Allyl protecting group from the L-glutamate employing an *'Alloc*/*Allyl deprotection',* AGLU*-OH was coupled employing the *'On-resin modification of carboxylic acids with AGLU*-OH'*method. Afterwards the branched peptide (Pent-Cys-Glu(AGLU)-Gln-Tyr-Phe-Pro-Hyp-Ile-Nva-Cys-NHz) was cleaved from the synthesis resin according to method *Complete resin cleavage and deprotection of peptides'* (cleavage time 2 hours), and the obtained crude material was subjected to cyclization according to *'Cyclization method A: disulfide cyclization'.* After lyophilisation of the reaction solution, the crude product was purified by *'Preparative HPLC'* (25 to 45% B in 15 min - Kinetex) to yield 3.15 mg of the pure title compound (8.6%). HPLC t_{R} = 7.3 min. LC/TOF-MS: exact mass 1457.656 (calculated 1457.657). C₆₆H₉₉N₁₃O₂₀S₂ (MW = 1458.702).

### Example 8: Synthesis IId: Synthesis of compound Butoc-[Cys-Thr-Orn(DOTA)-Pcnf-Phe-4Cfp-Hyp-Ile-Nva-Cys]-NHz (UPAR-276)

The linear sequence of the peptide (Butoc-Cys-Thr-Orn(Alloc)-Pcnf-Phe-4Cfp-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method and performing the *'N-terminal attachment of carbamate moieties'* with n-butyl chloroformate as final step. After removal of the Alloc protecting group from L-lysine employing an *'Alloc*/*Allyl deprotection',* DOTA(tBu)3-OH was coupled employing the *'Coupling of chelator building blocks'*method. Afterwards the branched peptide (Butoc-Cys-Thr-Orn(DOTA)-Pcnf Phe-4Cfp-Hyp-Ile-Nva-Cys-NH₂) was cleaved from the synthesis resin according to *Complete resin cleavage and deprotection of peptides'* (cleavage time 4 hours), and the obtained crude material was subjected to cyclization using the method *'Cyclization method A: disulfide cyclization'.* After lyophilisation of the reaction solution, the crude product was purified by *'Preparative HPLC'* (25 to 45% B in 20 min-Kinetex) to yield 10.55 mg of the pure title compound (12.4%). HPLC t_{R} = 5.8 min. LC/TOF-MS: exact mass 1699.779 (calculated 1699.775). C₇₆H₁₁₄FN₁₇O₂₂S₂ (MW = 1700.953).

### Example 9: Synthesis IIe: Synthesis of compound nBuCAyl-[Cys-Thr-Lys(DOTA)-Tyr-Phe-Pro-Hyp-Ile-Nva-Cys]-NHz (UPAR-242)

The linear sequence of the peptide (nBuCAyl-Cys-Thr-Lys(Alloc)-Tyr-Phe-Pro-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method and performing the *'N-terminal attachment of urea moieties'* with n-butyl isocyanate as final step. After removal of the Alloc protecting group from L-lysine employing an *'Alloc*/*Allyl deprotection',* DOTA(tBu)3-OH was coupled employing the *'Coupling of chelator building blocks'*method. Afterwards the branched peptide (nBuCAyl-Cys-Thr-Lys(DOTA)-Tyr-Phe-Pro-Hyp-Ile-Nva-Cys-NH₂) was cleaved from the synthesis resin according to *Complete resin cleavage and deprotection of peptides'* (cleavage time 4 hours), and the obtained crude material was subjected to cyclization using the method *'Cyclization method A: disulfide cyclization'.* After lyophilisation of the reaction solution, the crude product was purified by *'Preparative HPLC'* (25 to 45% B in 20 min-Kinetex) to yield 15.38 mg of the pure title compound (18.4%). HPLC t_{R} = 6.8 min. LC/TOF-MS: exact mass 1667.802 (calculated 1667.805). C₇₆H₁₁₇N₁₇O₂₁S₂ (MW = 1668.979).

### Example 10: Synthesis Ilf: Synthesis of compound Butoc-[Cys-Thr-Lys(DOTA)-Tyr-Phe-4Cfp-Hyp-Ile-Nva-Cys]-Bal-OH (UPAR-127)

Starting from a PS resin with a 2-chlorotrityl linker to which Fmoc-Bal-OH was loaded, the linear sequence of the peptide (Butoc-Cys-Thr-Lys(Alloc)-Tyr-Phe-4Cfp-Hyp-Ile-Nva-Cys-Bal-OH) was assembled according to the *'General procedures for Automated*/*Semi-automated Solid-Phase Synthesis'* in a 50 µmol scale applying the *'Solid phase peptide synthesis (SPPS)'* method and performing the *'N-terminal attachment of carbamate moieties'* with n-butyl chloroformate as final step. After removal of the Alloc protecting group from L-lysine employing an *'Alloc*/*Allyl deprotection',* DOTA(tBu)3-OH was coupled employing the *'Coupling of chelator building blocks'* method. Afterwards the branched peptide (nBuCAyl-Cys-Thr-Lys(DOTA)-Tyr-Phe-Pro-Hyp-Ile-Nva-Cys-NH₂) was cleaved from the synthesis resin according to *Complete resin cleavage and deprotection of peptides'* (cleavage time 4 hours), and the obtained crude material was subjected to cyclization using the method *'Cyclization method A: disulfide cyclization'.* After lyophilisation of the reaction solution, the crude product was purified by *'Preparative HPLC'* (25 to 45% B in 20 min - Kinetex) to yield 15.38 mg of the pure title compound (18.4%). HPLC t_{R} = 6.8 min. LC/TOF-MS: exact mass 1667.802 (calculated 1667.805). C₇₆H₁₁₇N₁₇O₂₁S₂ (MW = 1668.979).

### Example 11: Synthesis IIg: Synthesis of compound Butoc-[Cys-Thr-Lys(DOTA-asp)-Tyr-Phe-4Cfp-Hyp-Ile-Nva-Cys]-NHz (UPAR-424)

The linear sequence of the peptide (Butoc-Cys-Thr-Lys(Alloc)-Tyr-Phe-4Cfp-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method and performing the *'N-terminal attachment of carbamate moieties'* with n-butyl chloroformate as final step. After removal of the Alloc protecting group from the L-lysine side chain employing an *'Alloc*/*Allyl deprotection",* SPPS was continued with coupling of D-aspartate employing the *'Coupling of carboxylic acid building blocks'* method followed by '*Fmoc deprotection*', and a final *'Coupling of chelator building blocks'* to attach DOTA(tBu)3-OH. Afterwards, the branched peptide (Butoc-Cys-Thr-Lys(DOTA-asp)-Tyr-Phe-4Cfp-Hyp-Ile-Nva-Cys-NH₂) was cleaved from the synthesis resin according to '*Complete resin cleavage and deprotection of peptides'* (cleavage time 4 hours), and the obtained crude material was subjected to cyclization using the method *'Cyclization method A: disulfide cyclization'.* After lyophilisation of the reaction solution, the crude product was purified by *'Preparative HPLC'* (25 to 45% B in 20 min - Kinetex) to yield 6.99 mg of the pure title compound (7.8%). HPLC t_{R} = 6.4 min. LC/TOF-MS: exact mass 1801.804 (calculated 1801.807). C₈₀H₁₂₀FN₁₇O₂₅S₂ (MW = 1803.041).

### Example 12: Synthesis IIh: Synthesis of compound Pent-[Cys-Cit-Lys(DOTA-PEG12)-Tyr-Phe-Pro-Hyp-Ile-Nva-Cys]-NHz (UPAR-200)

The linear sequence of the peptide (Pent-Cys-Cit-Lys(Alloc)-Tyr-Phe-Pro-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)"* method with an N-terminal coupling of pentanoic acid as final step. After removal of the Alloc protecting group from the L-lysine side chain employing an *'Alloc*/*Allyl deprotection",* SPPS was continued with coupling Fmoc-PEG12-OH employing the *'Coupling of carboxylic acid building blocks'* method followed by '*Fmoc deprotection*', and a final *'Coupling of chelator building blocks'* to attach DOTA(tBu)3-OH. Afterwards, the branched peptide (Pent-Cys-Cit-Lys(DOTA-PEG12)-Tyr-Phe-Pro-Hyp-Ile-Nva-Cys-NH₂) was cleaved from the synthesis resin according to '*Complete resin cleavage and deprotection of peptides'* (cleavage time 4 hours), and the obtained crude material was subjected to cyclization using the method *'Cyclization method A: disulfide cyclization'.* After lyophilisation of the reaction solution, the crude product was purified by *'Preparative HPLC'* (30 to 50% B in 20 min - Kinetex) to yield 9.98 mg of the pure title compound (8.6%). HPLC t_{R} = 7.7 min. LC/TOF-MS: exact mass 2308.175 (calculated 2308.183). C₁₀₅H1₇₃N₁₉O₃₄S₂ (MW = 2309.740).

### Example 13: Synthesis IIi: Synthesis of compound Pent-[Cys-Cit-Lys(Bio-Ttds)-Tyr-Phe-Pro-Nmk-Ile-Nva-Cys]-NH₂ (UPAR-317)

The linear sequence of the peptide (Pent-Cys-Cit-Lys(Alloc)-Tyr-Phe-Pro-Nmk-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with an N-terminal coupling of pentanoic acid as final step. After removal of the Alloc protecting group from the L-lysine side chain employing an *'Alloc*/*Allyl deprotection',* SPPS was continued with coupling Fmoc-Ttds-OH employing the *'Coupling of carboxylic acid building blocks'* method followed by '*Fmoc deprotection*', and a final coupling of biotin employing the *'Coupling of carboxylic acid building blocks'.* Afterwards, the branched peptide (Pent-Cys-Cit-Lys(Bio-Ttds)-Tyr-Phe-Pro-Nmk-Ile-Nva-Cys-NH₂) was cleaved from the synthesis resin according to '*Complete resin cleavage and deprotection of peptides'* (cleavage time 2 hours), and the obtained crude material was subjected to cyclization using the method *'Cyclization method A: disulfide cyclization'.* After lyophilisation of the reaction solution, the crude product was purified by *'Preparative HPLC'* (25 to 45% B in 20 min - Kinetex) to yield 3.91 mg of the pure title compound (4.2%). HPLC t_{R} = 5.9 min. LC/TOF-MS: exact mass 1879.994 (calculated 1879.976). C₈₈H₁₄₁N₁₉O₂₀S₃ (MW = 1881.378).

### Example 14: Synthesis IIIa: Synthesis of compound iHex-[Cys-Cit-Gln-Tyr-Phe-Pro-ala-Ile-Leu-Cys]-OH (UPAR-131)

Starting from a PS resin with a 2-chloro trityl linker to which L-cysteine was loaded, the linear sequence of the peptide (iHex-Cys-Cit-Gln-Tyr-Phe-Pro-ala-Ile-Leu-Cys-OH) was assembled according to the *'General procedures for Automated*/*Semi-automated Solid-Phase Synthesis'* in a 25 µmol scale applying the '*Solid phase peptide synthesis (SPPS)"* method with an N-terminal coupling of isohexanoic acid as final step. After cleavage of the linear peptide from the synthesis resin employing '*Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min - Kinetex) to yield 5.10 mg of the pure title compound (15.6%). HPLC t_{R} = 7.3 min. LC/TOF-MS: exact mass 1309.725 (calculated 1309.620). C₆₁H₉₁N₁₃O₁₅S₂ (MW = 1310.588).

### Example 15: Synthesis IIIb: Synthesis of compound Pent-[Cys-Cit-Gln-Tyr-Phe-Pro-Hyp-Ile-Nva-Cys]-en (UPAR-430)

Starting from a PS resin with a 2-chlorotrityl linker to which ethylene diamine was loaded, the linear sequence of the peptide (Pent-Cys-Cit-Gln-Tyr-Phe-Pro-Hyp-Ile-Nva-Cys-en) was assembled according to the *'General procedures for Automated*/*Semi-automated Solid-Phase Synthesis'* in a 25 µmol scale applying the *'Solidphase peptide synthesis (SPPS)'* method with an N-terminal coupling of pentanoic acid as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min-Kinetex) to yield 2.15 mg of the pure title compound (6.3%). HPLC t_{R} = 5.6 min. LC/TOF-MS: exact mass 1365.656 (calculated 1365.657). C₆₃H₉₅N₁₅O₁₅S₂ (MW = 1366.655).

### Example 16: Synthesis IIIc: Synthesis of compound Pent-[Cys-Cit-Gln-Tyr-Phe-Pro-Hyp-Ile-Nva-Cysol]-OH (UPAR-292)

Starting from a PS resin with a 2-chlorotrityl linker to which L-cysteinol was loaded, the linear sequence of the peptide (Pent-Cys-Cit-Gln-Tyr-Phe-Pro-Hyp-Ile-Nva-Cysol-OH) was assembled according to the *'General procedures for Automated*/*Semi-automated Solid-Phase Synthesis'* in a 25 µmol scale applying the *'Solid phase peptide synthesis (SPPS)'* method with an N-terminal coupling of pentanoic acid as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (30 to 50% B in 15 min - Kinetex) to yield 1.38 mg of the pure title compound (4.2%). HPLC t_{R} = 5.9 min. LC/TOF-MS: exact mass 1309.619 (calculated 1309.620). C₆₁H₉₁N₁₃O₁₅S₂ (MW = 1310.588).

### Example 17: Synthesis IIId: Synthesis of compound Pent-[Cys-Cit-Gln-Tyr-Phe-Pro-Hyp-Ile-Nva-Cys]-NHEt (UPAR-146)

Starting from a PS resin with an FMPB linker which was reacted with ethyl amine, the linear sequence of the peptide (Pent-Cys-Cit-Gln-Tyr-Phe-Pro-Hyp-Ile-Nva-Cys-NHEt) was assembled according to the *'General procedures for Automated*/*Semi-automated Solid-Phase Synthesis'* in a 25 µmol scale applying the *'Solid phase peptide synthesis (SPPS)'* method with an N-terminal coupling of pentanoic acid as final step. After cleavage of the linear peptide from the synthesis resin employing '*Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (30 to 50% B in 15 min - Kinetex) to yield 0.96 mg of the pure title compound (2.8%). HPLC t_{R} = 6.4 min. LC/TOF-MS: exact mass 1350.641 (calculated 1350.646). C₆₃H₉₄N₁₄O₁₅S₂ (MW = 1351.640).

### Example 18: Synthesis IIIe: Synthesis of compound Pent-[Cys-Arg-Gln-Tyr-Phe-Pro-Hyp-Ile-Nva-AET] (UPAR-481)

Starting from a PS resin with a cysteamine 2-chlorotrityl linker, the linear sequence of the peptide (Pent-Cys-Arg-Gln-Tyr-Phe-Pro-Hyp-Ile-Nva-AET) was assembled according to the *'General procedures for Automated*/*Semi-automated Solid-Phase Synthesis'* in a 25 µmol scale applying the *'Solid phase peptide synthesis (SPPS)'* method with an N-terminal coupling of pentanoic acid as final step. After cleavage of the linear peptide from the synthesis resin employing '*Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 30 min - Kinetex) to yield 6.22 mg of the pure title compound (19.4%). HPLC t_{R} = 7.2 min. LC/TOF-MS: exact mass 1278.623 (calculated 1278.625). C₆₀H₉₀N₁₄O₁₃S₂ (MW = 1279.577).

### Example 19: Synthesis IVa: Synthesis of compound Me-Nml-[Cys-Arg-Gln-Tyr-Phe-Pro-Hyp-Ile-Nva-Cys]-NH₂ (UPAR-487)

The linear sequence of the peptide (Me-Nml-Cys-Arg-Gln-Tyr-Phe-Pro-Hyp-Ile-Nva-Cys-NHz) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with a *'Preparation of N,N-dimethylamines'* as final step. After cleavage of the linear peptide from the synthesis resin employing '*Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (20 to 40% B in 20 min - Kinetex) to yield 6.30 mg of the pure title compound (9.1%). HPLC t_{R} = 6.3 min. LC/TOF-MS: exact mass 1378.689 (calculated 1378.689). C₆₄H₉₈N₁₆O₁₄S₂ (MW = 1379.696).

### Example 20: Synthesis IVb: Synthesis of compound H-NEtg-[Cys-Cit-Gln-Tyr-Phe-Pro-ala-Ile-Leu-Cys]-NH₂ (UPAR-462)

The linear sequence of the peptide (H-NEtg-Cys-Cit-Gln-Tyr-Phe-Pro-ala-Ile-Leu-Cys-NHz) was assembled according to the *'General procedures for Automated*/*Semi-automated Solid-Phase Synthesis'* in a 25 µmol scale on a DEG Rink amide resin applying the *'Solid phase peptide synthesis (SPPS)'* method and a *'Synthesis ofN-alkylated glycines at the N-terminus'* (with ethylamine) as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (20 to 40% B in 15 min - Kinetex) to yield 1.08 mg of the pure title compound (3.3%). HPLC t_{R} = 6.5 min. LC/TOF-MS: exact mass 1295.614 (calculated 1295.615). C₅₉H₈₉N₁₅O₁₄S₂ (MW = 1296.565).

### Example 21: Synthesis IVc: Synthesis of compound Butoc-[Cys-Cit-Gln-Tyr-Phe-Pro-ala-Ile-Leu-Cys]-NH₂ (UPAR-155)

The linear sequence of the peptide (Butoc-Cys-Cit-Gln-Tyr-Phe-Pro-ala-Ile-Leu-Cys-NH₂) was assembled according to the *'General procedures for Automated*/*Semi-automated Solid-Phase Synthesis'* in a 25 µmol scale on a DEG Rink amide resin applying the *'Solid phase peptide synthesis (SPPS)'* method and performing the *'N-terminal attachment of carbamate moieties'* with n-butyl chloroformate as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min - Kinetex) to yield 2.15 mg of the pure title compound (6.6%). HPLC t_{R} = 7.9 min. LC/TOF-MS: exact mass 1310.614 (calculated 1310.615). C₆₀H₉₀N₁₄O₁₅S₂ (MW = 1311.576).

### Example 22: Synthesis IVd: Synthesis of compound nBuCAyl-[Cys-Cit-Gln-Tyr-Phe-Pro-ala-Ile-Leu-Cys]-NH₂ (UPAR-286)

The linear sequence of the peptide (nBuCAyl-Cys-Cit-Gln-Tyr-Phe-Pro-ala-Ile-Leu-Cys-NH₂) was assembled according to the *'General procedures for Automated*/*Semi-automated Solid-Phase Synthesis'* in a 25 µmol scale on a DEG Rink amide resin applying the *'Solid phase peptide synthesis (SPPS)'* method and performing the *'N-terminal attachment of urea moieties'* with n-butyl isocyanate as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min - Kinetex) to yield 4.59 mg of the pure title compound (14.0%). HPLC t_{R} = 7.8 min. LC/TOF-MS: exact mass 1309.629 (calculated 1309.631). C₆₀H₉₁N₁₅O₁₄S₂ (MW = 1310.591).

### Example 23: Synthesis IVe: Synthesis of compound iHex-[Cys-Cit-Gln-Mcy-Phe-Pro-ala-Ile-Leu-Cys]-NH₂ (UPAR-188)

The linear sequence of the peptide (iHex-Cys-Cit-Gln-Mcy-Phe-Pro-ala-Ile-Leu-Cys-NHz) was assembled according to the *'General procedures for Automated*/*Semi-automated Solid-Phase Synthesis'* in a 25 µmol scale on a DEG Rink amide resin applying the *'Solid phase peptide synthesis (SPPS)'* method with an N-terminal coupling of isohexanoic acid as final step. The resin-bound peptide was then treated with piperidine as described in '*Fmoc deprotection'* to cleave the undesired isohexanoate ester formed on the unprotected Mcy moiety. After cleavage of the linear peptide from the synthesis resin employing '*Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (25 to 45% B in 15 min-Kinetex) to yield 2.06 mg of the pure title compound (6.1%). HPLC t_{R} = 8.0 min. LC/TOF-MS: exact mass 1342.596 (calculated 1342.597). C₆₁H₉₁ClN₁₄O₁₄S₂ (MW = 1344.048).

### Example 24: Synthesis IVf: Synthesis of compound AGLU-Glutar-Nva-[Cys-Cit-Gln-Tyr-Phe-Pro-Hyp-Ile-Nva-Cys]-NHz (UPAR-268)

The linear sequence of the peptide (AGLU-Glutar-Nva-Cys-Cit-Gln-Tyr-Phe-Pro-Hyp-Ile-Nva-Cys-NH₂) was assembled according to the *'General procedures for Automated*/*Semi-automated Solid-Phase Synthesis'* in a 25 µmol scale on a DEG Rink amide resin applying the *'Solid phase peptide synthesis (SPPS)'* method with an N-terminal coupling of a AGLU*-Glutar-OH building block as final step. After cleavage of the linear peptide from the synthesis resin employing '*Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (20 to 40% B in 15 min - Kinetex) to yield 7.85 mg of the pure title compound (19.4%). HPLC t_{R} = 6.8 min. LC/TOF-MS: exact mass 1614.742 (calculated 1614.742). C₇₂H₁₁₀N₁₆O₂₂S₂ (MW = 1615.873).

### Example 25: Synthesis IVg: Synthesis of compound PentylSO2-[Cys-Arg-Gln-Tyr-Phe-Pro-Hyp-Ile-Nva-Cys]-NH₂ (UPAR-328)

The linear sequence of the peptide (PentylSO2-Cys-Arg-Gln-Tyr-Phe-Pro-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method and performing a *'Synthesis of N-terminal sulfonamides'* with n-pentyl sulfonyl chloride as final step. After cleavage of the linear peptide from the synthesis resin employing *'Complete resin cleavage and deprotection of peptides'* (cleavage time 2 hours), the obtained crude material was subjected to *'Cyclization method A: disulfide cyclization'.* After lyophilisation of the reaction solution, the crude product was purified by *'Preparative HPLC'* (30 to 50% B in 20 min - Kinetex) to yield 10.13 mg of the pure title compound (14.8%). HPLC t_{R} = 7.8 min. LC/TOF-MS: exact mass 1371.619 (calculated 1371.614). C₆₁H₉₃N₁₅O₁₅S₃ (MW = 1372.683).

### Example 26: Synthesis Va: Synthesis of compound Pent-Ala-Cit-Gln-Tyr-Phe-Pro-Hyp-Ile-Nva-Smc-NH₂ (UPAR-205)

The linear sequence of the peptide (Pent-Ala-Cit-Gln-Tyr-Phe-Pro-Hyp-Ile-Nva-Smc-NHz) was assembled according to the *'General procedures for Automated*/*Semi-automated Solid-Phase Synthesis'* in a 25 µmol scale on a DEG Rink amide resin applying the *'Solid phase peptide synthesis (SPPS)'* method with an N-terminal coupling of pentanoic acid as final step. After cleavage of the linear peptide from the synthesis resin employing *'Complete resin cleavage and deprotection of peptides'* (cleavage time 2 hours), the crude product was purified by *'Preparative HPLC'* (25 to 45% B in 15 min - Kinetex) to yield 8.48 mg of the pure title compound (25.9%). HPLC t_{R} = 5.1 min. LC/TOF-MS: exact mass 1306.675 (calculated 1306.674). C₆₂H₉₄N₁₄O₁₅S (MW = 1307.563).

### Example 27: Synthesis Vb: Synthesis of compound Pent-[Smc-Cit-Gln-Tyr-Phe-Pro-Hyp-Ile-Nva-Cys]-NH₂ (UPAR-215)

The linear sequence of the peptide (Pent-Cys-Cit-Gln-Tyr-Phe-Pro-Hyp-Ile-Nva-Cys-NHz) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with an N-terminal coupling of pentanoic acid as final step. After cleavage of the linear peptide from the synthesis resin employing *'Complete resin cleavage and deprotection of peptides'* (cleavage time 2 hours), the obtained crude material was subjected to *'Cyclization method B: cyclization with diodomethane'.* After lyophilisation of the reaction solution, the crude product was purified by *'Preparative HPLC'* (30 to 50% B in 20 min - Kinetex) to yield 3.73 mg of the pure title compound (5.6%). HPLC t_{R} = 5.8 min. LC/TOF-MS: exact mass 1336.626 (calculated 1336.631). C₆₂H₉₂N₁₄O₁₅S₂ (MW = 1337.613).

### Example 28: Synthesis Vc: Synthesis of compound Pent-[Cys(2MeBn)-Arg-Gln-Tyr-Phe-Pro-Hyp-Ile-Nva-Cys]-NH₂ (UPAR-258)

The linear sequence of the peptide (Pent-Cys-Arg-Gln-Tyr-Phe-Pro-Hyp-Ile-Nva-Cys-NHz) was assembled according to the *'General procedures for Automated*/*Semi-automated Solid-Phase Synthesis'* in a 25 µmol scale on a DEG Rink amide resin applying the *'Solid phase peptide synthesis (SPPS)'* method with an N-terminal coupling of pentanoic acid as final step. After cleavage of the linear peptide from the synthesis resin employing *Complete resin cleavage and deprotection of peptides'* (cleavage time 2 hours), the obtained crude material was subjected to *'Cyclization method D: dibromoxylene cyclization'.* After lyophilisation of the reaction solution, the crude product was purified by *'Preparative HPLC'* (30 to 50% B in 15 min - Kinetex) to yield 5.49 mg of the pure title compound (15.4%). HPLC t_{R} = 7.9 min. LC/TOF-MS: exact mass 1425.688 (calculated 1425.694). C₆₉H₉₉N₁₅O₁₄S₂ (MW = 1426.751).

### Example 29: Synthesis VIa: Synthesis of compound Pent-[Cys-Orn(mPEG12)-Lys(DOTA)-Tyr-Phe-Pro-Hyp-Ile-Nva-Cys]-NHz (UPAR-383)

The linear sequence of the peptide (Pent-Cys-Om(Dde)-Lys(Alloc)-Tyr-Phe-Pro-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with an N-terminal coupling of pentanoic acid as final step. The Dde protecting group from L-ornithine was removed employing a *'Dde*/*ivDde deprotection'* and the liberated ornithine amine group was reacted with mPEG12-OH employing the *'Coupling of carboxylic acid building blocks'* method to yield the branched peptide Pent-Cys-Orn(mPEG 12)-Lys(Alloc)-Tyr-Phe-Pro-Hyp-Ile-Nva-Cys-NHz. After removal of the Alloc protecting group from L-lysine employing an *'Alloc*/*Allyl deprotection',* DOTA(tBu)3-OH was coupled employing the *'Coupling of chelator building blocks'* method. Afterwards, the double-branched peptide (Pent-Cys-Om(mPEG12)-Lys(DOTA)-Tyr-Phe-Pro-Hyp-Ile-Nva-Cys-NH₂) was cleaved from the synthesis resin according to *'Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (30 to 50% B in 20 min-Kinetex) to yield 8.49 mg of the pure title compound (7.6%). HPLC t_{R} = 7.7 min. LC/TOF-MS: exact mass 2236.139 (calculated 2236.151). C₁₀₃H₁₆₉N₁₇O₃₃S₂ (MW = 2237.674).

### Example 30: Synthesis VIb: Synthesis of compound Pent-[Cys-Orn(Tris-Nta(Tris))-Lys(DOTA)-Tyr-Phe-Pro-Hyp-Ile-Nva-Cys]-NH₂ (UPAR-224)

The linear sequence of the peptide (Pent-Cys-Om(Dde)-Lys(Alloc)-Tyr-Phe-Pro-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with an N-terminal coupling of pentanoic acid as final step. The Dde protecting group from L-ornithine was removed employing a *'Dde*/*ivDde deprotection'* and the liberated ornithine amine group was reacted with nitrilotriacetic acid (Nta). To this end, nitrilotriacetic acid (76 mg, 400 µmol, 8 eq.) was dissolved in a mixture of DMSO/DMF (4 mL, 2:3) and pre-activated with DIC (7.7 µL, 50 µmol, 1 eq.) for 2 min before addition to the resin. After reaction for one hour at RT, the mix was drained and the reaction repeated once. Afterwards, the two resin-bound carboxylates of Nta were pre-activated with HATU (0.325 mL of 0.4 M in DMF, 2.5 eq.) and DIPEA (0.15 mL of 0.9 M in DMF, 2.5 eq.) for 2 min and reacted with a mixture of Tris(TBDMS)3 (116 mg, 0.25 mmol, 5 eq.) and DIPEA (0.15 mL of 0.9 M in DMF, 2.5 eq.) in DMF (0.15 mL) for 90 min at RT to yield the branched peptide Pent-Cys-Orn(Tris-Nta(Tris))-Lys(Alloc)-Tyr-Phe-Pro-Hyp-Ile-Nva-Cys-NHz. The Alloc protecting group from L-lysine was removed employing an *'Alloc*/*Allyl deprotection'* followed by coupling of DOTA(tBu)3-OH according to the *'Coupling of chelator building blocks'* method. After a *'TBDMS deprotection',* the branched peptide (Pent -Cys-Orn(Tris- Nta(Tris))- L ys(DOT A)- Tyr- Phe- Pro-Hyp- Ile- Nva -Cys- NHz) was cleaved from the synthesis resin according to *'Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (25 to 45% B in 20 min - Kinetex) to yield 5.44 mg of the pure title compound (5.3%). HPLC t_{R} = 7.2 min. LC/TOF-MS: exact mass 2044.978 (calculated 2044.985). C₉₁H₁₄₄N₂₀O₂₉S₂ (MW = 2046.369).

### Example 31: Synthesis VIc: Synthesis of compound Butoc-[Cys-Thr-Lys(DOTA)-Tyr-Phe-4Cfp-Hyp-Ile-Dmdp-Cys]-NH₂ (UPAR-129)

The linear sequence of the peptide (Butoc-Cys-Thr-Lys(Alloc)-Tyr-Phe-4Cfp-Hyp-Ile-Dap(ivDde)-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* and performing the *'N-terminal attachment of carbamate moieties'* with n-butyl chloroformate as final step. The ivDde protecting group from the side chain of the L-2,3-diaminopropionate residue was removed employing a *'Dde*/*ivDde deprotection'* and the liberated amine group was subjected to *'Preparation of N,N-dimethylamines'* to yield Butoc-Cys-Thr-Lys(Alloc)-Tyr-Phe-4Cfp-Hyp-Ile-Dmdp-Cys-NH₂. After removal of the Alloc protecting group from L-lysine employing an *'Alloc*/*Allyl deprotection',* DOTA(tBu)3-OH was coupled employing the *'Coupling of chelator building blocks'* method. Afterwards, the branched peptide (Butoc-Cys-Thr-Lys(DOTA)-Tyr-Phe-4Cfp-Hyp-Ile-Dmdp-Cys-NH₂) was cleaved from the synthesis resin according to *Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (20 to 40% B in 20 min - Kinetex) to yield 5.31 mg of the pure title compound (6.2%). HPLC t_{R} = 4.9 min. LC/TOF-MS: exact mass 1701.792 (calculated 1701.791). C₇₆H₁₁₆FN₁₇O₂₂S₂ (MW = 1702.968).

### Example 32: Synthesis VId: Synthesis of compound Butoc-[Cys-Thr-Lys(DOTA-Kzw)-Tyr-Phe-4Cfp-Hyp-Ile-Nva-Cys]-NHz (UPAR-422)

The linear sequence of the peptide (Butoc-Cys-Thr-Lys(Dde)-Tyr-Phe-4Cfp-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* and performing the *'N-terminal attachment of carbamate moieties'* with n-butyl chloroformate as final step. The Dde protecting group from the side chain of the L-lysine residue was removed employing a *'Dde*/*ivDde deprotection'* and the liberated amine group of the L-lysine was reacted with Alloc-Lys(Fmoc)-OH employing the *'Coupling of carboxylic acid building blocks'* method. Following an *'Fmoc deprotection'* of the lysine side chain, the newly generated amine was subjected to *'Preparation of N,N-dimethylamines'* to yield Butoc-Cys-Thr-Lys(Alloc-KMe2)-Tyr-Phe-4Cfp-Hyp-Ile-Nva-Cys-NH₂. Afterwards, the dimethyllysine was subjected to *'Alkylation of tertiary amines with 1,3-propanesultone* ', yielding Butoc-Cys-Thr-Lys(Alloc-Kzw)-Tyr-Phe-4Cfp-Hyp-Ile-Nva-Cys-NH₂. After removal of the Alloc protecting group employing an *'Alloc*/*Allyl deprotection',* DOTA(tBu)3-OH was coupled employing the *'Coupling of chelator building blocks'* method. Afterwards, the resulting peptide (Butoc-Cys-Thr-Lys(DOTA-Kzw)-Tyr-Phe-4Cfp-Hyp-Ile-Nva-Cys-NH₂) was cleaved from the synthesis resin according to *'Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (25 to 45% B in 20 min - Kinetex) to yield 13.61 mg of the pure title compound (13.8%). HPLC t_{R} = 6.2 min. LC/TOF-MS: exact mass 1964.913 (calculated 1964.910). C₈₇H₁₃₇FN₁₈O₂₆S₃ (MW = 1966.324).

### Example 33: Synthesis VIIa: Synthesis of compound Butoc-[Cys-Thr-Orn(DOTA)-My-Phe-4Cfp-Hyp-Ile-Nva-Cys]-NH₂ (UPAR-415)

The linear sequence of the peptide (Butoc-Cys-Thr-Orn(Alloc)-My-Phe-4Cfp-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method and performing the *'N-terminal attachment of carbamate moieties'* with n-butyl chloroformate as final step. The resin-bound peptide was then treated with piperidine as described in *'Fmoc deprotection'* to remove the undesired and concomitant modification on the unprotected My moiety. The Alloc protecting group from L-ornithine was removed employing an *'Alloc*/*Allyl deprotection'* followed by coupling of DOTA(tBu)3-OH according to the *'Coupling of chelator building blocks'* method, and again the resin was treated with piperidine as described in *'Fmoc deprotection'.* Afterwards, the branched peptide (Butoc-Cys-Thr-Orn(DOTA)-My-Phe-4Cfp-Hyp-Ile-Nva-Cys-NH₂) was cleaved from the synthesis resin according to *'Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (25 to 45% B in 20 min - Kinetex) to yield 10.65 mg of the pure title compound (12.7%). HPLC t_{R} = 6.1 min. LC/TOF-MS: exact mass 1672.768 (calculated 1672.764). C₇₅H₁₁₃FN₁₆O₂₂S₂ (MW = 1673.927).

### Example 34: Synthesis VIIb: Synthesis of compound Butoc-[Cys-Thr-Orn(DOTA)-Ay3-Phe-4Cfp-Hyp-Ile-Nva-Cys]-NHz (UPAR-234)

The linear sequence of the peptide (Butoc-Cys-Thr-Orn(Alloc)-Ny3-Phe-4Cfp-Hyp-Ile-Nva-Cys-NH₂) was assembled according to the *'General procedures for Automated*/*Semi-automated Solid-Phase Synthesis'* in a 50 µmol scale on a DEG Rink amide resin applying the *'Solid phase peptide synthesis (SPPS)'* method and performing the *'N-terminal attachment of carbamate moieties'* with n-butyl chloroformate as final step. The resin-bound peptide was then treated with piperidine as described in *'Fmoc deprotection'* to remove the undesired and concomitant modification on the unprotected Ny3 moiety. The Alloc protecting group from L-ornithine was removed employing an *'Alloc*/*Allyl deprotection'* followed by coupling of DOTA(tBu)3-OH according to the *'Coupling of chelator building blocks'* method, and again the resin was treated with piperidine as described in *'Fmoc deprotection'.* Afterwards, the branched peptide (Butoc-Cys-Thr-Orn(DOTA)-Ny3-Phe-4Cfp-Hyp-Ile-Nva-Cys-NH₂) was subjected to *'Reduction of nitrobenzenes to anilines*'. Te resulting peptide (Butoc-Cys-Thr-Orn(DOTA)-Ay3-Phe-4Cfp-Hyp-Ile-Nva-Cys-NHz) was cleaved from the synthesis resin according to *'Complete resin cleavage and deprotection of peptides'* (cleavage time 4 hours), and the obtained crude material was subjected to cyclization according to *'Cyclization method A: disulfide cyclization'.* After lyophilisation of the reaction solution, the crude product was purified by *'Preparative HPLC'* (25 to 45% B in 20 min - Kinetex) to yield 1.21 mg of the pure title compound (1.4%). HPLC t_{R} = 6.5 min. LC/TOF-MS: exact mass 1687.761 (calculated 1687.775). C₇₅H₁₁₄FN₁₇O₂₂S₂ (MW = 1688.942).

### Example 35: Synthesis Villa: Butoc-[Cys-Thr-Lys(AF488-Ttds)-Dopa-Phe-4Cfp-Hyp-Ile-Nva-Cys]-NH₂ (UPAR-479)

The linear sequence of the peptide (Butoc-Cys-Thr-Lys(Alloc)-Dopa-Phe-4Cfp-Hyp-Ile-Nva-Cys-NH₂) was assembled according to the *'General procedures for Automated*/*Semi-automated Solid-Phase Synthesis'* in a 100 µmol scale on a DEG Rink amide resin applying the *'Solid phase peptide synthesis (SPPS)'* method and performing the *'N-terminal attachment of carbamate moieties'* with n-butyl chloroformate as final step. After removal of the Alloc protecting group from the L-lysine side chain employing an *'Alloc*/*Allyl deprotection',* SPPS was continued with coupling of Fmoc-Ttds-OH employing the *'Coupling of carboxylic acid building blocks'* method followed by *'Fmoc deprotection*'. Afterwards, the branched peptide (Butoc-Cys-Thr-Lys(H-Ttds)-Tyr-Phe-4Cfp-Hyp-Ile-Nva-Cys-NH₂) was cleaved from the synthesis resin according to *Complete resin cleavage and deprotection of peptides'* (cleavage time 2 hours), and the obtained crude material was subjected to cyclization using the method *'Cyclization method A: disulfide cyclization'.* After lyophilisation of the reaction solution, the crude product was subjected to purification by *'Preparative HPLC'* (25 to 45% B in 30 min-Kinetex) to yield 24.9 mg of the cyclized intermidate Butoc-[Cys-Thr-Lys(H-Ttds)-Tyr-Phe-4Cfp-Hyp-Ile-Nva-Cys]-NH₂ (15.4% of theory). AF488-NHS ester was reacted with 11 mg of the cyclized intermediate following the *'Coupling of NHS esters in solution'* method. The obtained crude product was subjected to purification by *'Preparative HPLC'* (25 to 45% B in 20 min - Kinetex) to yield 2.50 mg of the pure title compound (17.2% of theory). HPLC t_{R} = 7.3 min. LC/TOF-MS: exact mass 2134.768 (calculated 2134.772). C₉₅H₁₂₇FN₁₆O₃₁S₄ (MW = 2136.380).

### Example 36: Synthesis VIIIb: Pent-[Cys-Orn(TrisPEG)-Lys(DOTA)-Tyr-Phe-Pro-Hyp-Ile-Nva-Cys]-NH₂ (UPAR-247)

The linear sequence of the peptide (Pent-Cys-Orn-Lys(Alloc)-Tyr-Phe-Pro-Hyp-Ile-Nva-Cys-NHz) was assembled according to the *'General procedures for Automated*/*Semi-automated Solid-Phase Synthesis'* in a 50 µmol scale on a DEG Rink amide resin applying the *'Solid phase peptide synthesis (SPPS)'* method with coupling of pentanoic acid as final step. After removal of the Alloc protecting group from L-lysine employing an *'Alloc*/*Allyl deprotection',* DOTA(tBu)3-OH was coupled employing the *'Coupling of chelator building blocks'* method. Afterwards the branched peptide (Pent-Cys-Orn-Lys(DOTA)-Tyr-Phe-Pro-Hyp-Ile-Nva-Cys-NHz) was cleaved from the synthesis resin according to *'Complete resin cleavage and deprotection of peptides'* (cleavage time 4 hours), and the obtained crude material was subjected to cyclization using the method *'Cyclization method A: disulfide cyclization'.* After lyophilisation of the reaction solution, the crude product was subjected to purification by *'Preparative HPLC'* (20 to 40% B in 20 min - Kinetex) to yield 18.89 mg of the cyclized intermidate Pent-[Cys-Orn-Lys(DOTA)-Tyr-Phe-Pro-Hyp-Ile-Nva-Cys]-NHz (22.7% of theory). TrisPEG 12-PEG4-Glutar-NHS, an activated PEG-NHS ester, was reacted with 6.4 mg of the cyclized intermediate following the *'Coupling ofNHS esters in solution'* method. The obtained crude product was subjected to purification by *'Preparative HPLC'* (35 to 55% B in 20 min - Kinetex) to yield 6.28 mg of the pure title compound (41.3% of theory). HPLC t_{R} = 8.6 min. LC/TOF-MS: exact mass 3970.169 (calculated 3970.165). C₁₈₁H₃₂₀N₂₂O₆₉S₂ (MW = 3972.723).

### Example 37: Synthesis IX: Preparation of 'cold' metal complexes of compounds of the invention

General procedure for the preparation of a peptide comprising a chelator-metal-complex from the corresponding peptide comprising an uncomplexed chelator:
0.1 mM solution of the peptide comprising the uncomplexed chelator in
a) 0.4 M sodium acetate buffer (pH = 5) for In/GalGd/La complexation, or
b) 0.2 M sodium acetate buffer (pH = 5) for Lu complexation
was diluted with a 0.1 mM solution of the corresponding metal salt in water such that the molar ratio of peptide to metal was adjusted to 1 : 3. The solution was stirred at 50 °C for 20 min. The solution was then subjected to solid phase extraction. Fractions containing the pure product were pooled and freeze dried.

### A. Indium complex of Butoc-[Cys-Thr-Orn(DOTA)-Pcnf-Phe-4Cfp-Hyp-Ile-Nva-Cys]-NH₂ (UPAR-490)

Starting from 13.7 mg of peptide (8.1 µmol) and InCl₃ × 4 H₂O as Indium donor, a yield of 14.15 mg of the pure title compound (97.0%) was obtained. HPLC t_{R} = 6.0 min. LC/TOF-MS: exact mass 1809.652 (calculated 1809.656). C₇₆H₁₁₁FInN₁₇O₂₂S₂ (MW = 1812.747).

### B. Lutetium complex of Butoc-[Cys-Thr-Lys(DOTA)-Dopa-Phe-4Cfp-Hyp-Ile-Nva-Cys]-NH₂ (UPAR-238)

Starting from 5 mg of peptide (2.9 µmol) and LuCl₃ as Lutetium donor, a yield of 4.80 mg of the pure title compound (91.3%) was obtained. HPLC t_{R} = 6.1 min. LC/TOF-MS: exact mass 1874.677 (calculated 1874.692). C₇₆H₁₁₂FLuN₁₆O₂₃S₂ (MW = 1875.896).

### C. Gallium complex of Butoc-[Cys-Thr-Orn(DOTA)-Pcnf-Phe-4Cfp-Hyp-Ile-Nva-Cys]-NH₂ (UPAR-489)

Starting from 25.4 mg of peptide (14.9 µmol) and Ga(NO₃)₃ × H₂O as Gallium donor, a yield of 24.56 mg of the pure title compound (93.2%) was obtained. HPLC t_{R} = 6.1 min. LC/TOF-MS: exact mass 1765.681 (calculated 1765.677). C₇₆H₁₁FGaN₁₇O₂₂S₂ (MW = 1767.652).

### D. Lanthanum complex of Pent-[Cys-Cit-Lys(DOTA)-Tyr-Phe-4Cfp-Hyp-Ile-Nva-Cys]-NH₂ (UPAR-303)

Starting from 7.0 mg of peptide (4.1 µmol) and LaCl₃ as Lanthanum donor, a yield of 7.97 mg of the pure title compound (quantitative yield) was obtained. HPLC t_{R} = 7.7 min. LC/TOF-MS: exact mass 1861.705 (calculated 1861.706). C₇₈H₁₁₆FLaN₁₈O₂₁S₂ (MW = 1863.903).

### E. Gadolinium complex of Pent-[Cys-Cit-Lys(DOTA)-Tyr-Ocf-Pro-Hyp-Ile-Nva-Cys]-NH₂ (UPAR-132)

Starting from 7.0 mg of peptide (4.0 µmol) and GdCl₃ × 6 H₂O as Gadolinium donor, a yield of 6.27 mg of the pure title compound (82.6%) was obtained. HPLC t_{R} = 8.2 min. LC/TOF-MS: exact mass 1891.688 (calculated 1891.689). C₇₈H₁₁₆ClGdN₁₈O₂₁S₂ (MW = 1898.704).

### Example 38: Synthesized compounds summary

Characterization data (HPLC/MS) for the compounds shown below is included in Table 12, following Example 39. Reference to the synthetic strategy used to prepare each compound is also included in Table 12.

The following compounds were synthesized:
compound Butoc-[Cys-Thr-Lys(DOTA)-Tyr-Phe-4Cfp-Hyp-Ile-Nva-Cys]-Bal-OH (UPAR-127) of the following formula 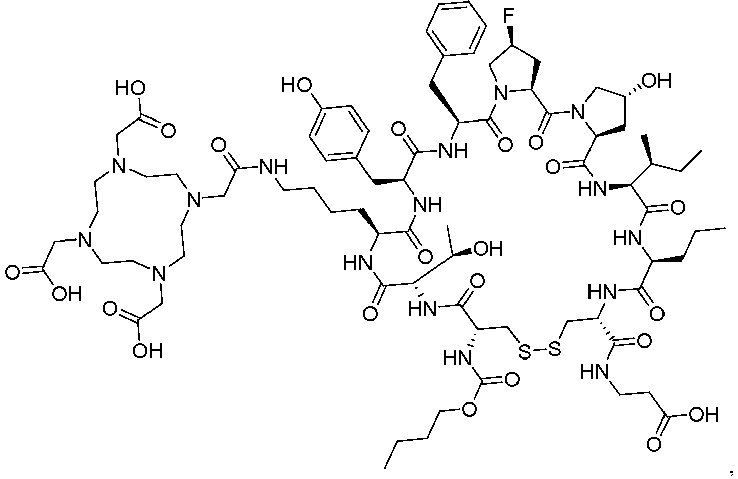
compound Butoc-[Cys-Thr-Lys(DOTA)-Tyr-Phe-Hyp-Hyp-Ile-Nva-Cys]-NH₂ (UPAR-128) of the following formula 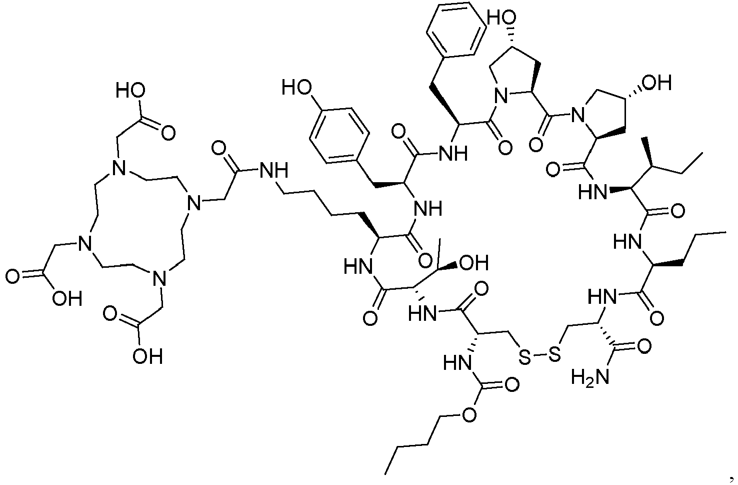
compound Butoc-[Cys-Thr-Lys(DOTA)-Tyr-Phe-4Cfp-Hyp-Ile-Dmdp-Cys]-NH₂ (UPAR-129) of the following formula 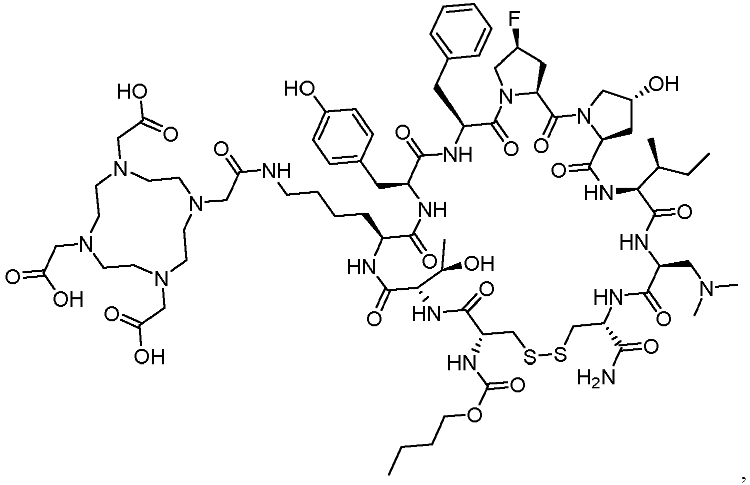
compound iHex-[Cys-lys(DOTA)-Gln-Tyr-Phe-Pro-ala-Ile-Leu-Cys]-NH₂ (UPAR-130) of the following formula 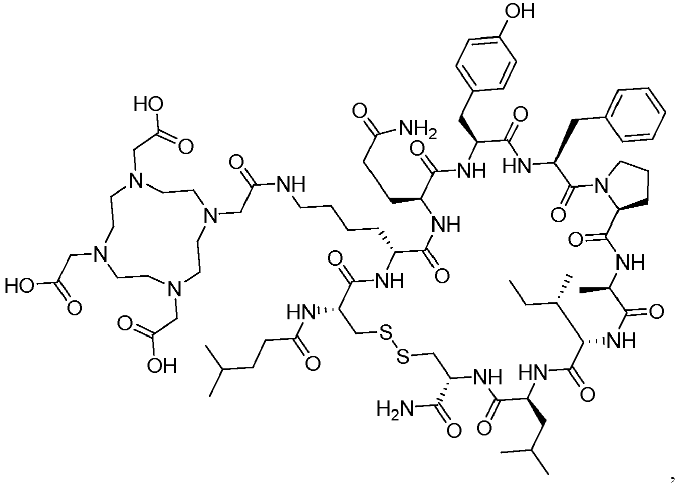
compound iHex-[Cys-Cit-Gln-Tyr-Phe-Pro-ala-Ile-Leu-Cys]-OH (UPAR-131) of the following formula 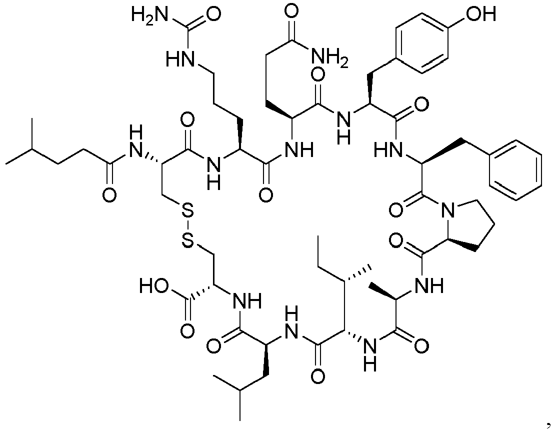
compound Pent-[Cys-Cit-Lys(GdDOTA)-Tyr-Ocf-Pro-Hyp-Ile-Nva-Cys]-NH₂ (UPAR-132) of the following formula 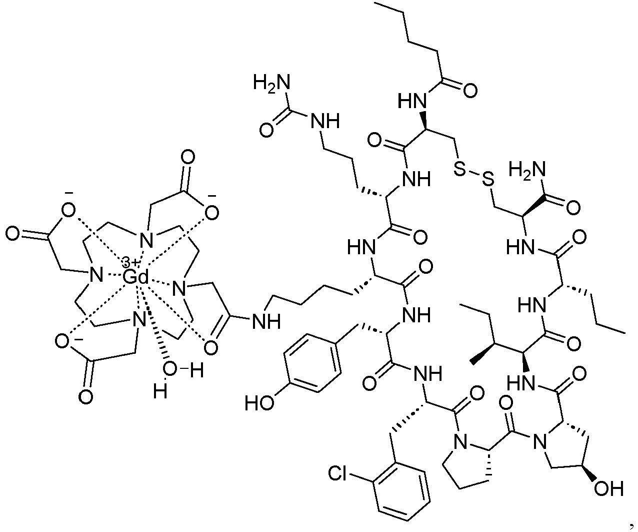
compound Ac-asp-Leu-[Cys-Arg-Nle-Tyr-Phe-Pro-Ala-Ile-Leu-Cys]-NH₂ (UPAR-133) of the following formula 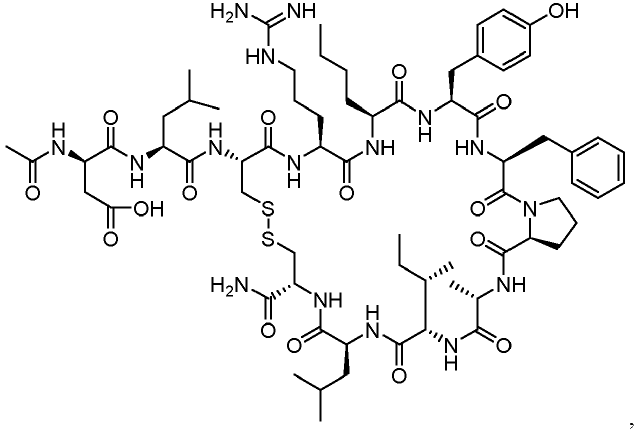
compound Butoc-[Cys-Thr-Lys(DOTA)-Tyr-Thi-Pro-Hyp-Ile-Nva-Cys]-NH₂ (UPAR-134) of the following formula 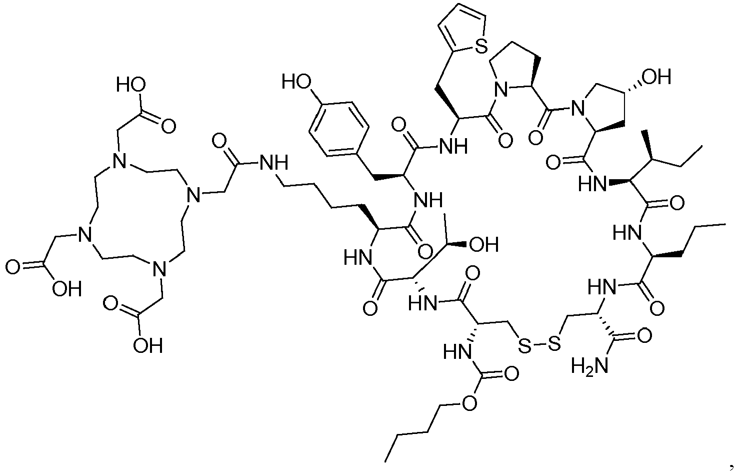
compound Pent-[Cys-Thr-Lys(DOTA)-Tyr-Phe-Hyp-Hyp-Ile-Nva-Cys]-NH₂ (UPAR-135) of the following formula 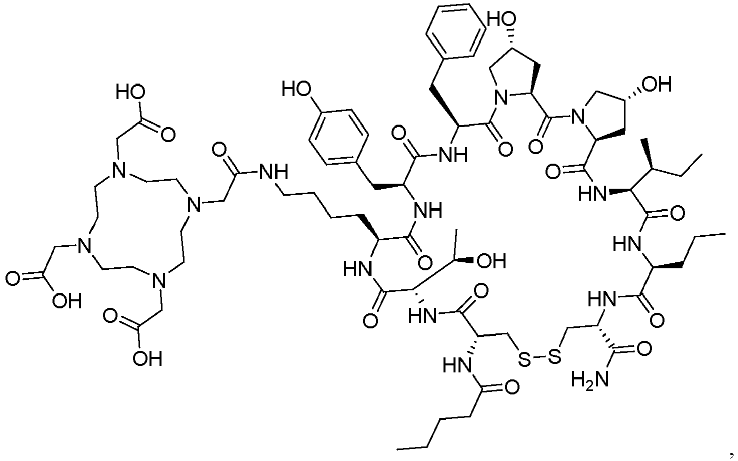
compound iHex-[Cys-Cit-Gln-Tyr-Phe-Pro-ala-Nva-Leu-Cys]-NHz (UPAR-136) of the following formula 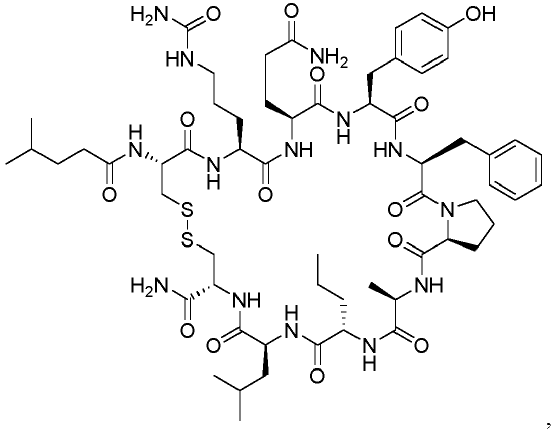
compound Ac-Asp-Leu-[Cys-Arg-Nle-Tyr-Phe-Pro-Ala-Ile-leu-Cys]-NH₂ (UPAR-137) of the following formula 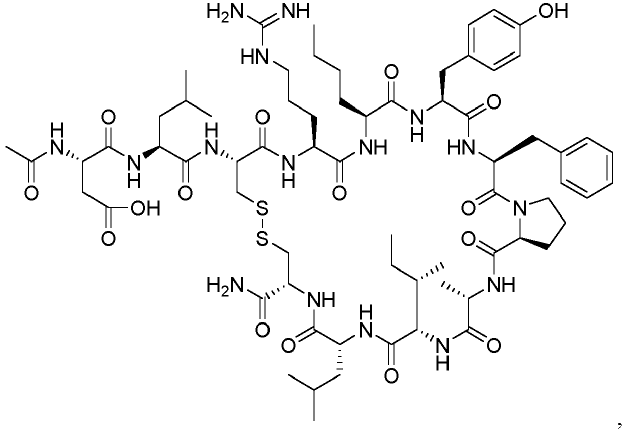
compound Pent-[Cys-Cit-Lys(DOTA)-Mpa-Phe-Pro-Hyp-Ile-Nva-Cys]-NH₂ (UPAR-138) of the following formula 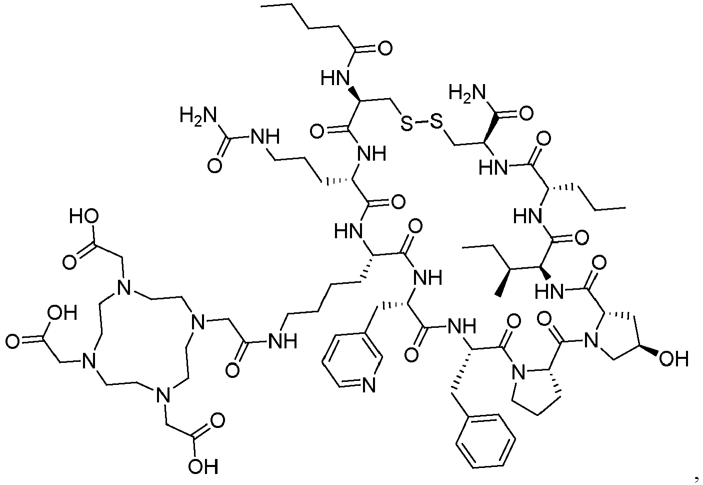
compound But-[Cys-Cit-Gln-Tyr-Phe-Pro-ala-Ile-Leu-Cys]-NH₂(UPAR-139) of the following formula 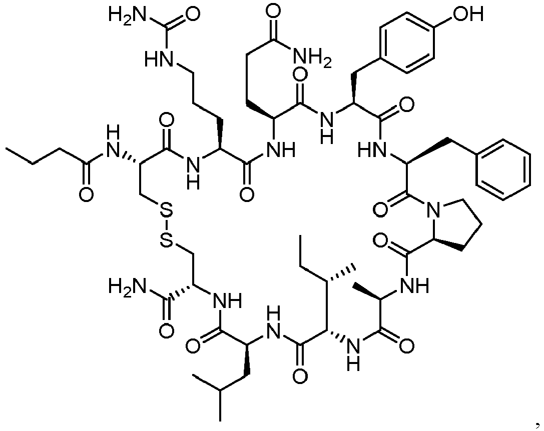
compound Hex-Lys(DOTA)-Leu-[Cys-Arg-Met-Tyr-Phe-Pro-Ala-Ile-Leu-Cys]-NH₂ (UPAR-140) of the following formula 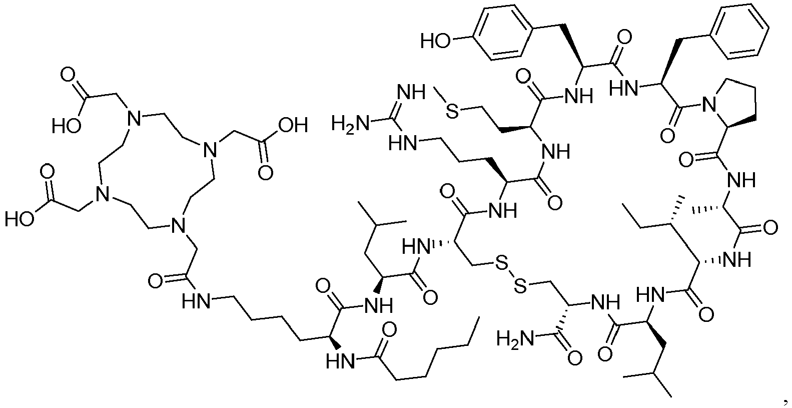
compound Butoc-[Cys-Thr-Lys(DOTA)-Tyr-Phe-4Cfp-Hyp-Ile-Nva-Cys]-Asp-NH₂ (UPAR-141) of the following formula 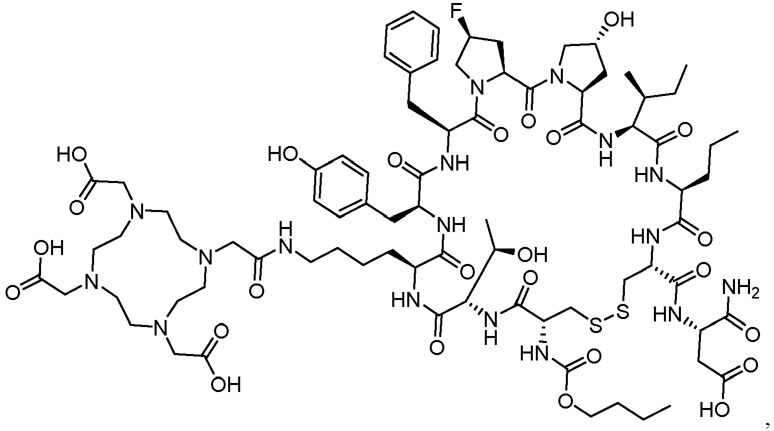
compound DOTA-Leu-[Nmc-Cit-Gln-Tyr-Phe-Pro-Hyp-Ile-Nva-Cys]-NH₂ (UPAR-142) of the following formula 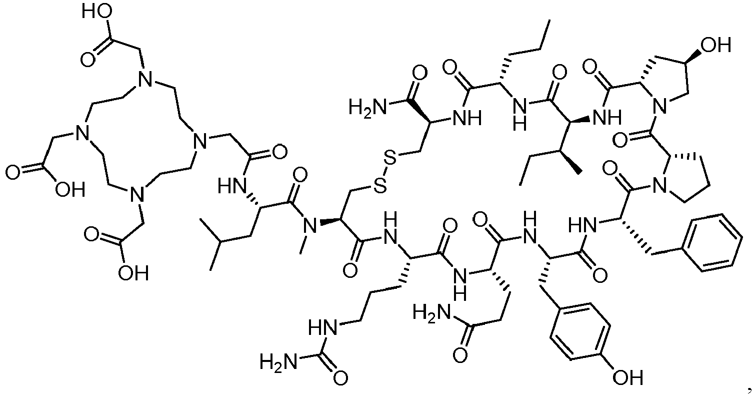
compound Hex-Thr-Leu-[Cys-Glu-Tyr-Tyr-Phe-Pro-Ala-Ile-Leu-Cys]-NH₂ (UPAR-143) of the following formula 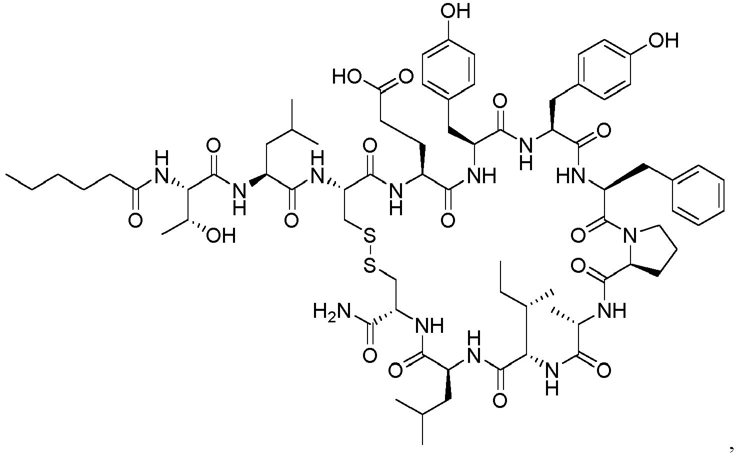
compound iHex-[Cys-Cit-Gln-Dopa-Phe-Pro-ala-Ile-Leu-Cys]-NH₂ (UPAR-144) of the following formula 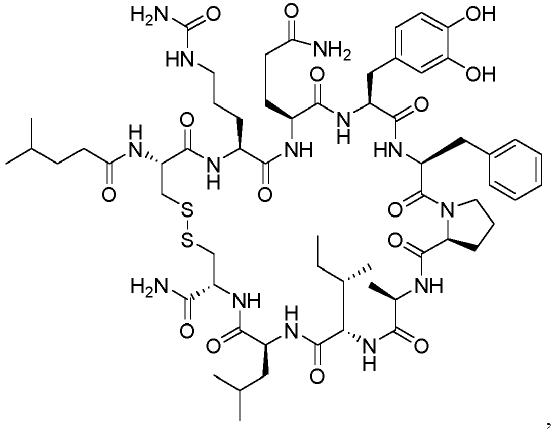
compound iHex-[Cys-Cit-Gln-Tyr-Phe-Pro-Lys(DOTA)-Ile-Leu-Cys]-NH₂ (UPAR-145) of the following formula 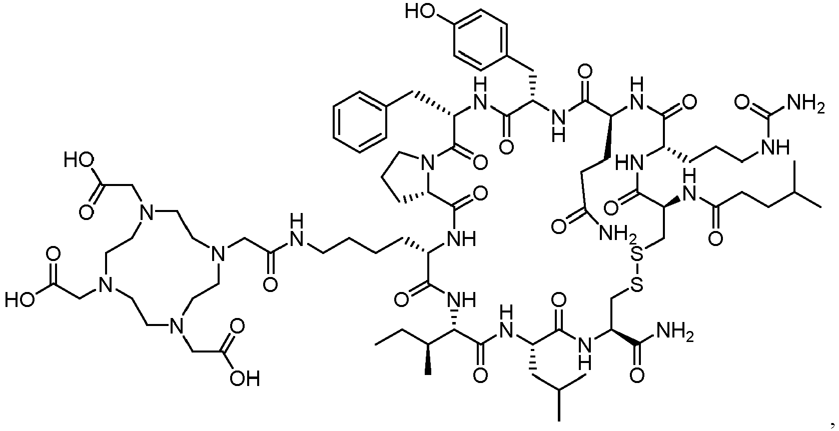
compound Pent-[Cys-Cit-Gln-Tyr-Phe-Pro-Hyp-Ile-Nva-Cys]-NHEt (UPAR-146) of the following formula 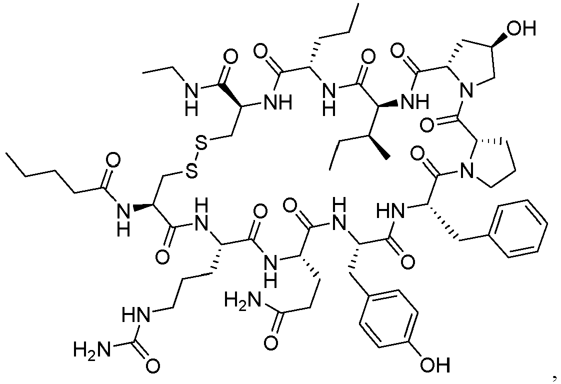
compound iHex-[Cys-Cit-Gln-Tyr-2Ni-Pro-ala-Ile-Leu-Cys]-NH₂ (UPAR-147) of the following formula 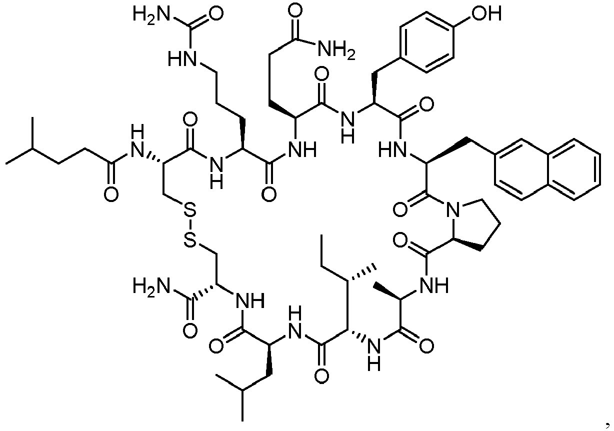
compound iHex-[Cys-Arg-Lys(InDOTA)-Tyr-Phe-Pro-ala-Ile-Leu-Cys]-NH₂ (UPAR-148) of the following formula 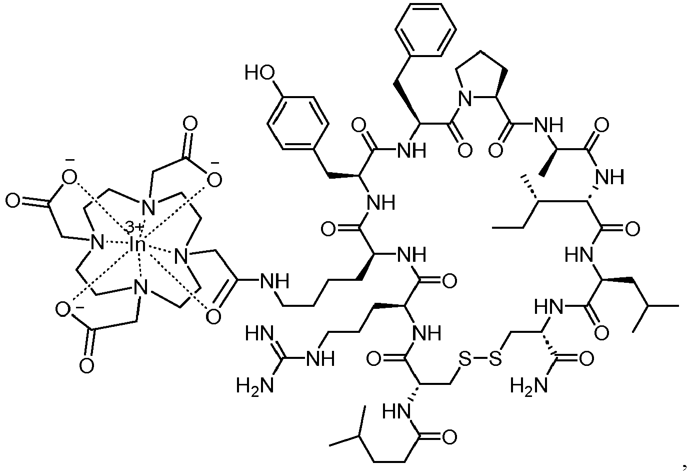
compound Pent-[Cys-Cit-Lys(DOTA)-Guf-Phe-Pro-Hyp-Ile-Nva-Cys]-NH₂ (UPAR-149) of the following formula 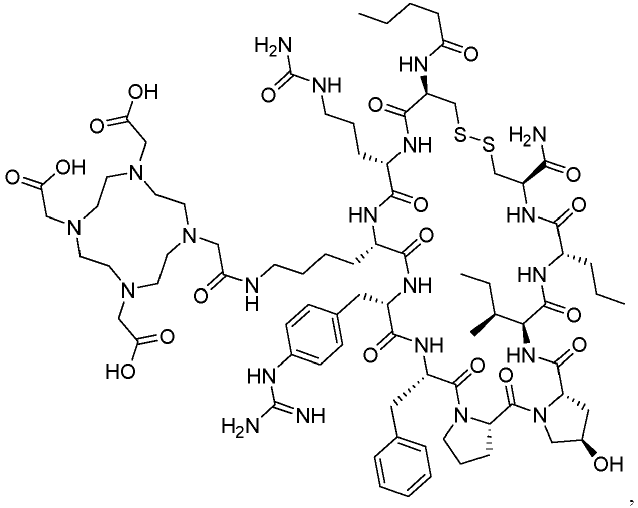
compound Hex-Asp-Leu-[Cys-Arg-Met-Tyr-Phe-Pro-Ala-Ile-Leu-Cys]-Ttds-Lys(DOTA)-NH₂ (UPAR-150) of the following formula 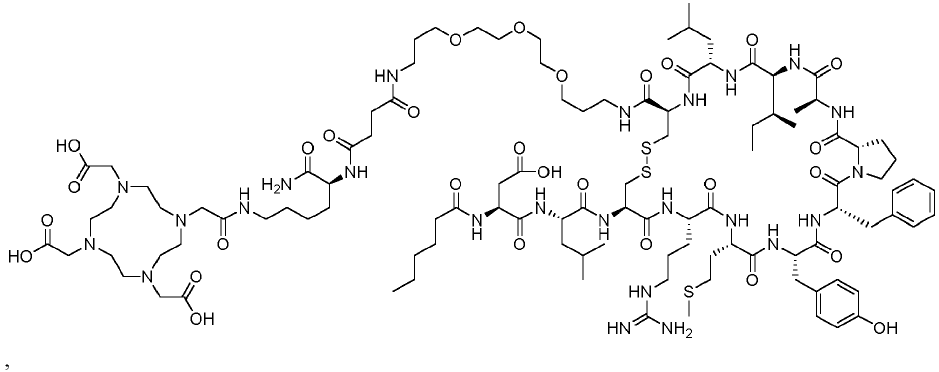
compound Pent-[Cys-Cit-Lys(LuDOTA)-Tyr-Phe-Pro-nma-Ile-Nva-Cys]-NH₂ (UPAR-151) of the following formula 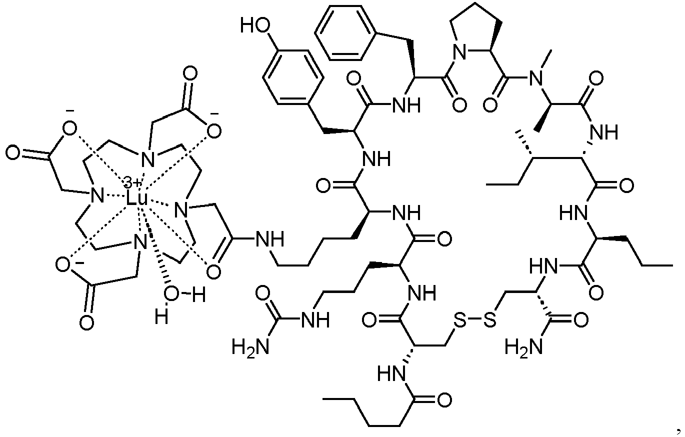
compound iHex-[Cys-Arg-Gln-Tyr-Phe-Pro-Apc(DOTA)-Ile-Leu-Cys]-NH₂ (UPAR-152) of the following formula 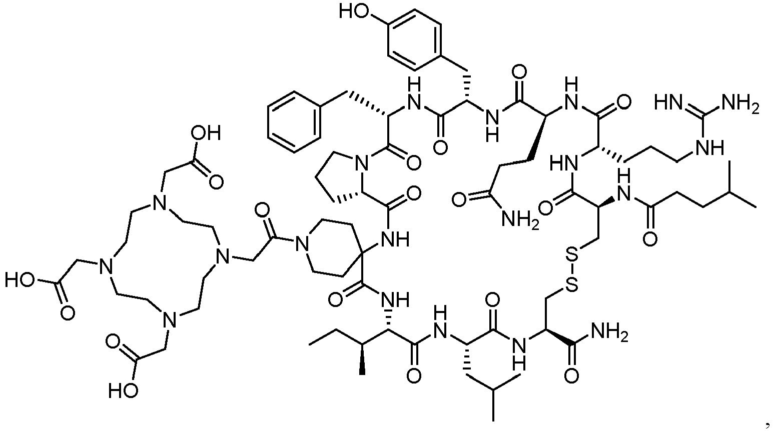
compound Pent-[Cys-KMe3-Lys(DOTA)-Tyr-Phe-Pro-Hyp-Ile-Nva-Cys]-NH₂ (UPAR-153) of the following formula 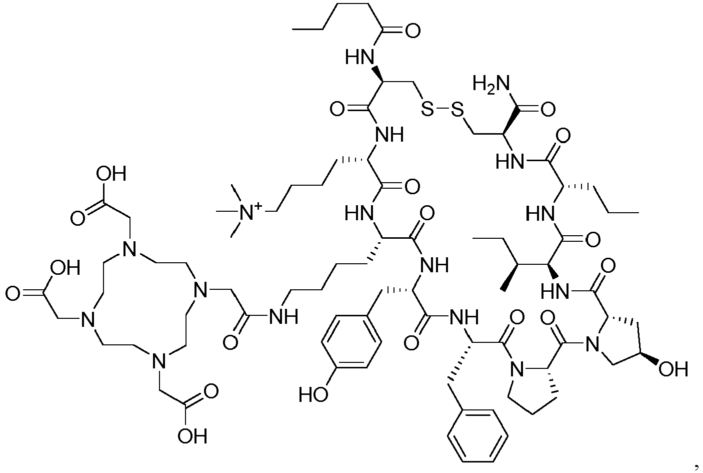
compound Pent-[Cys-Arg-Gln-Tyr-Phe-Pro-Ser-Ile-Nva-Cys]-NHz (UPAR-154) of the following formula 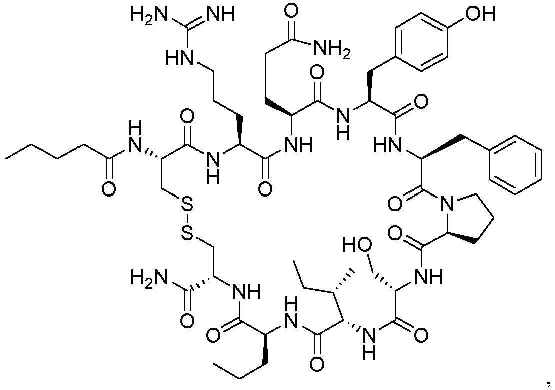
compound Butoc-[Cys-Cit-Gln-Tyr-Phe-Pro-ala-Ile-Leu-Cys]-NH₂ (UPAR-155) of the following formula 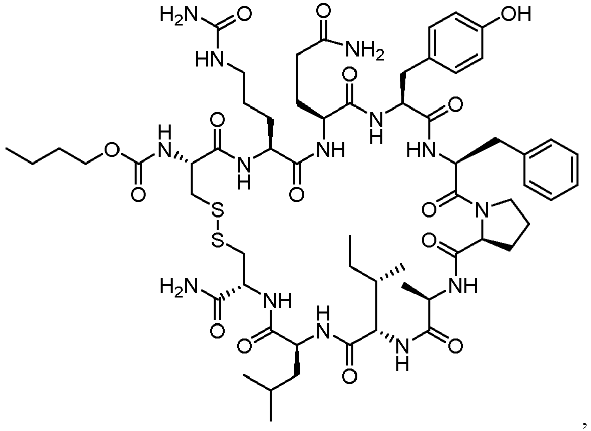
compound Pent-[Cys-Arg-Gln-Tyr-Phe-Pro-Hyp-Ile-Aml-Cys]-NHz (UPAR-156) of the following formula 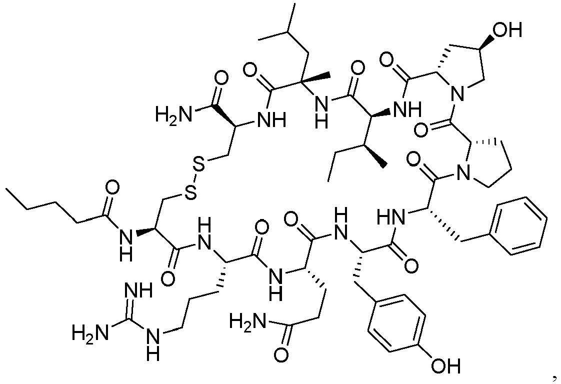
compound iHex-[Cys-Lys(DOTA)-Gln-Tyr-Phe-Pro-ala-Ile-Leu-Cys]-NH₂ (UPAR-157) of the following formula 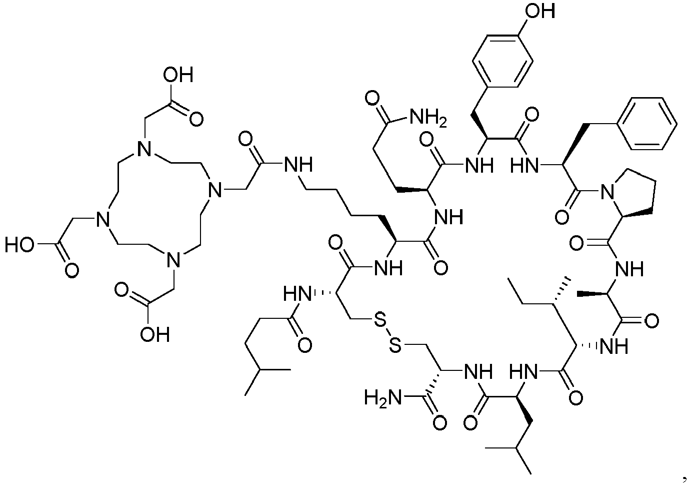
compound iHex-[Cys-Cit-Gln-Ala-Phe-Pro-ala-Ile-Leu-Cys]-NHz (UPAR-159) of the following formula 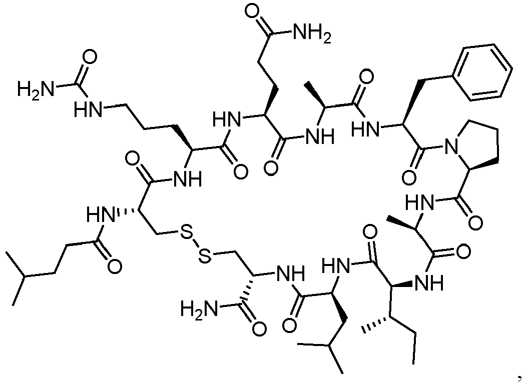
compound iHex-[Cys-Cit-Arg-Tyr-Phe-Pro-ala-Ile-Leu-Cys]-NH₂ (UPAR-160) of the following formula 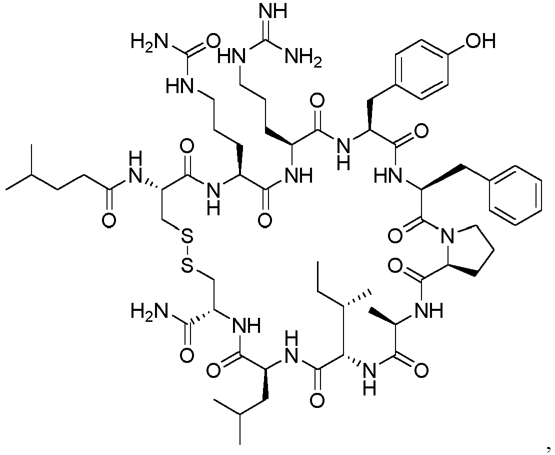
compound Pent-[Cys-Arg-Gln-Tyr-Phe-Pro-asp-Ile-Nva-Cys]-NH₂ (UPAR-161) of the following formula 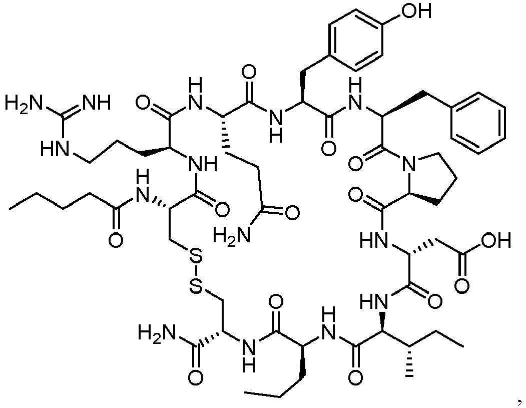
compound Ac-Leu-[Cys-Arg-Lys(DOTA)-Tyr-Phe-Pro-ala-Ile-Leu-Cys]-NH₂ (UPAR-162) of the following formula 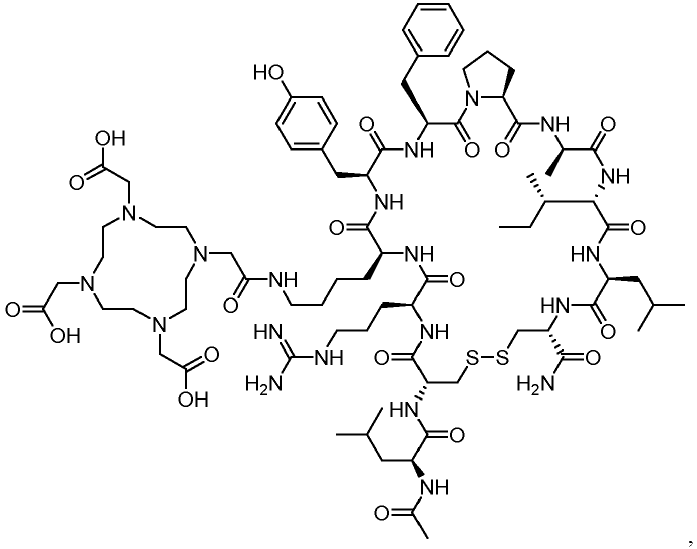
compound iHex-[Cys-Cit-Gln-Tyr-Phe-Tap-ala-Ile-Leu-Cys]-NH₂ (UPAR-163) of the following formula 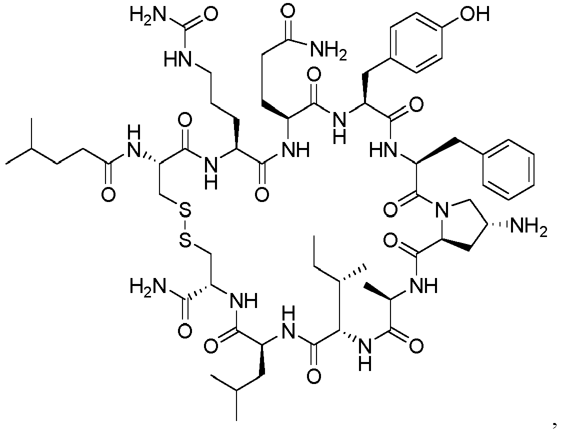
compound iHex-[Cys-Cit-Gln-Tyr-Phe-Pro-ala-Ala-Leu-Cys]-NH₂ (UPAR-164) of the following formula 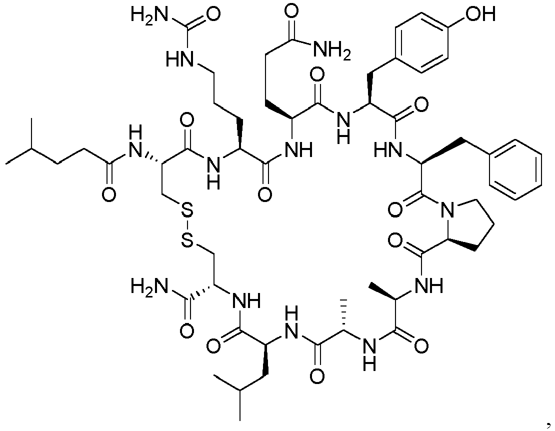
compound Butoc-[Cys-Thr-Lys(DOTA)-Mpa-Phe-4Cfp-Hyp-Ile-Nva-Cys]-NH₂ (UPAR-165) of the following formula 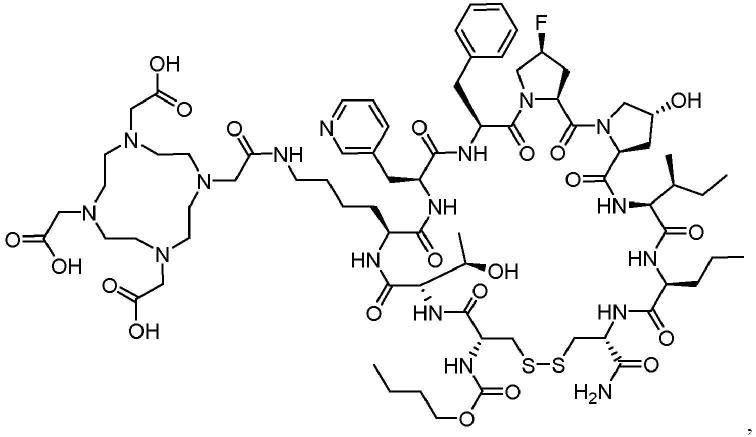
compound Pent-[Cys-Cit-Lys(LaDOTA)-Tyr-Thi-Pro-Hyp-Ile-Nva-Cys]-NH₂ (UPAR-166) of the following formula 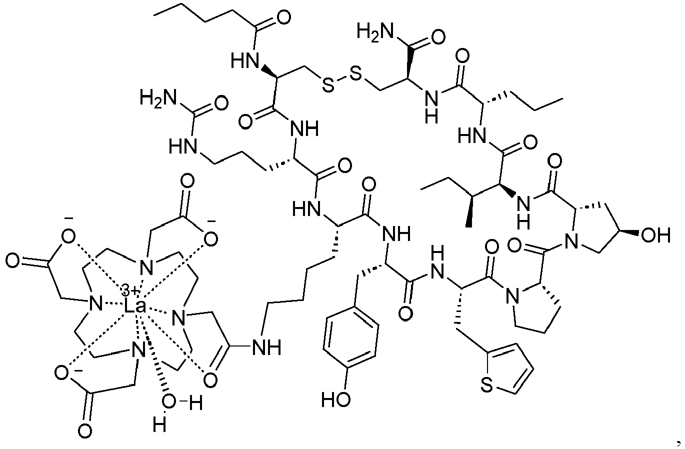
compound iHex-[Cys-Har-Gln-Tyr-Phe-Pro-Hyp-Ile-Leu-Cys]-NH₂ (UPAR-167) of the following formula 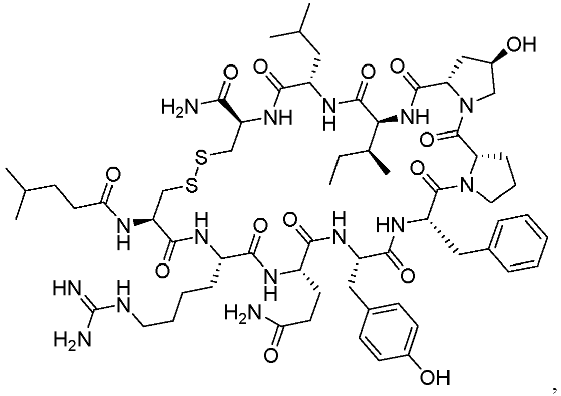
compound iHex-[Cys-Ala-Gln-Tyr-Phe-Pro-ala-Ile-Leu-Cys]-NH₂ (UPAR-168) of the following formula 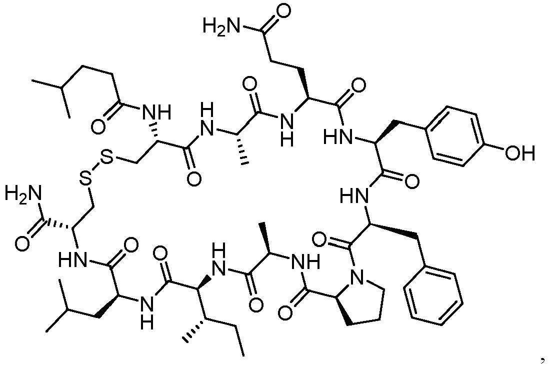
compound Butoc-[Cys-Cit-Lys(LuDOTA)-Tyr-Pcf-4Cfp-Hyp-Chg-Nva-Cys]-NH₂ (UPAR-169) of the following formula 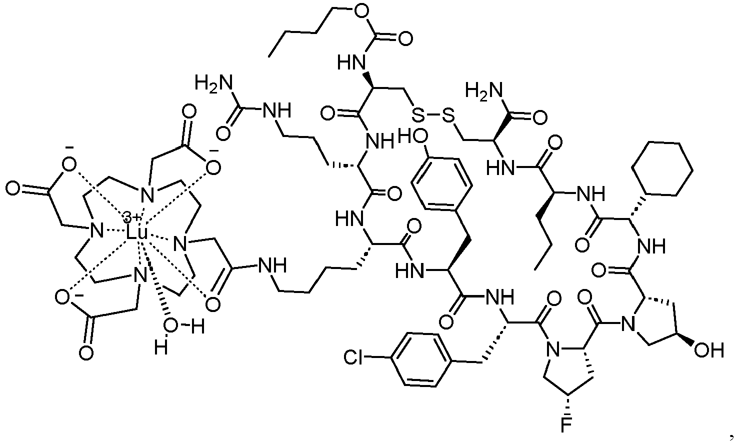
compound LuDOTA-Ahx-Leu-[Cys-Arg-Gln-Tyr-Phe-Pro-ala-Ile-Leu-Cys]-NH₂ (UPAR-170) of the following formula 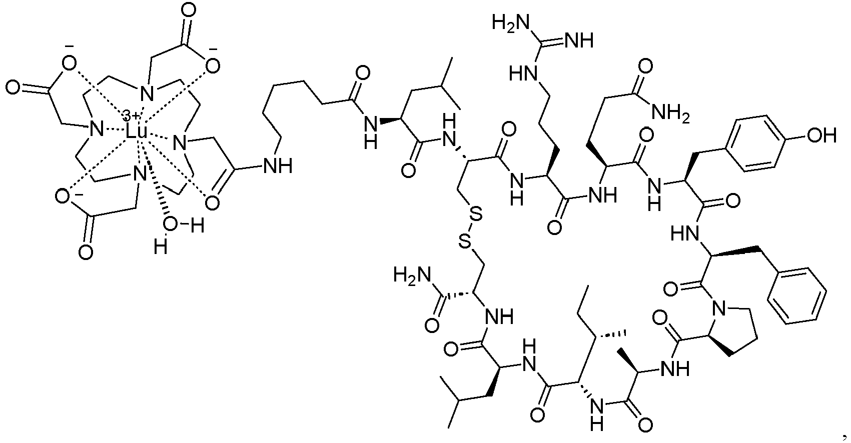
compound Pent-[Cys-Arg-Gln-Tyr-Phe-Pro-Hyp-Ile-Phe-Cys]-NH₂ (UPAR-171) of the following formula 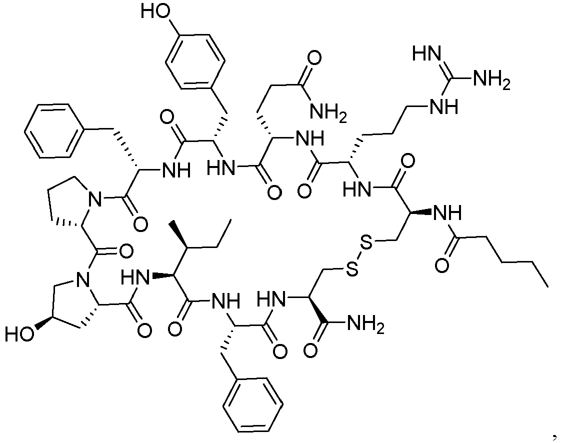
compound EtOPr-[Cys-Cit-Gln-Tyr-Phe-Pro-ala-Ile-Leu-Cys]-NH₂ (UPAR-172) of the following formula 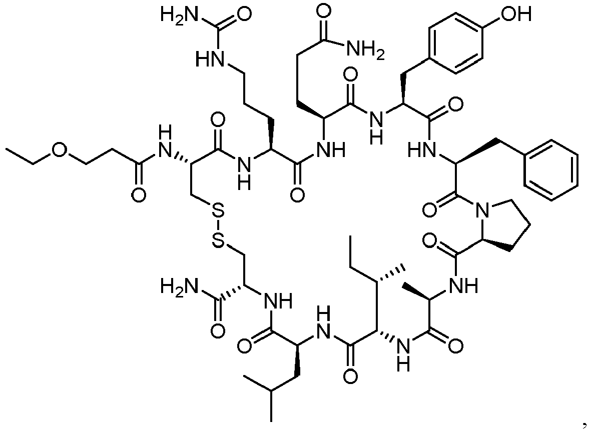
compound Pent-[Cys-Cit-Lys(DOTA)-Tyr-Phe-Pro-Nmk-Ile-Nva-Cys]-NH₂ (UPAR-173) of the following formula 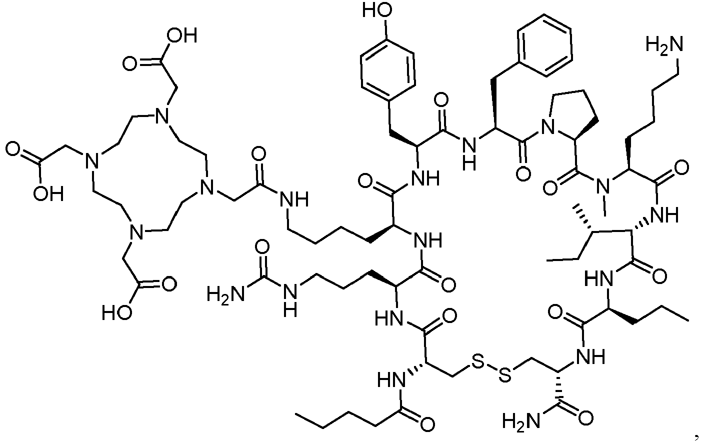
compound iHex-[Cys-Cit-Gln-Tyr-Phe-Pro-Hyp-Ile-Leu-Cys]-NH₂ (UPAR-174) of the following formula 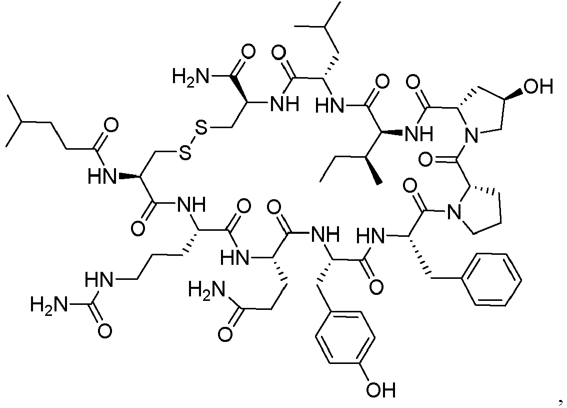
compound iHex-[Cys-Arg-Lys(DOTA)-Tyr-Phe-Pro-ala-Ile-Leu-Cys]-NH₂ (UPAR-176) of the following formula 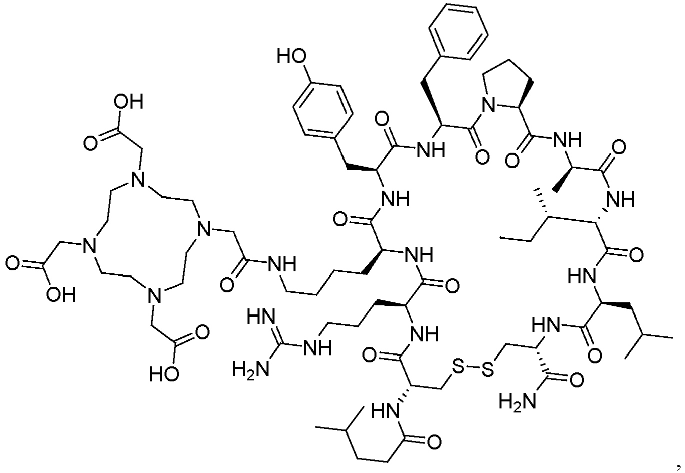
compound Pent-[Cys-Cit-Lys(DOTA)-Tyr-Phe-Pro-Nmg-Ile-Nva-Cys]-NH₂ (UPAR-177) of the following formula 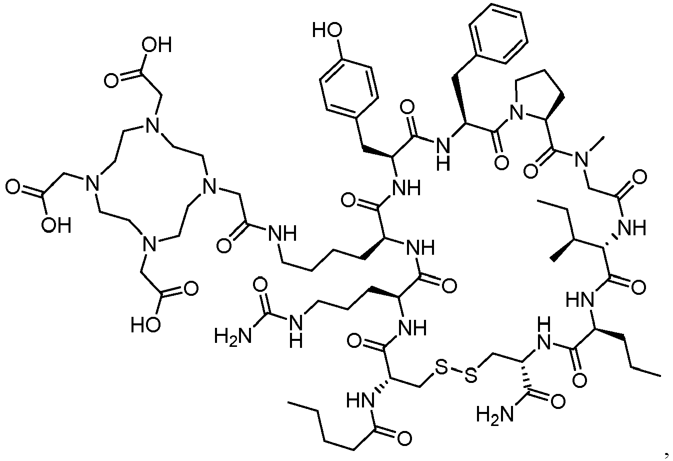
compound iHex-[Cys-Cit-Gln-Tyr-Phe-4Tfp-ala-Ile-Leu-Cys]-NH₂ (UPAR-178) of the following formula 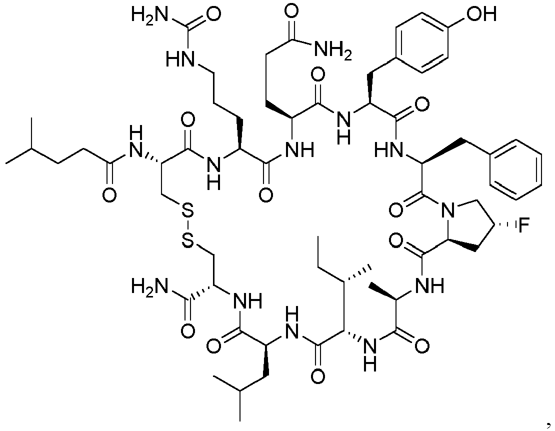
compound Pent-[Cys-Arg-Gln-Tyr-Phe-Pro-Hyp-Deg-Nva-Cys]-NH₂ (UPAR-179) of the following formula 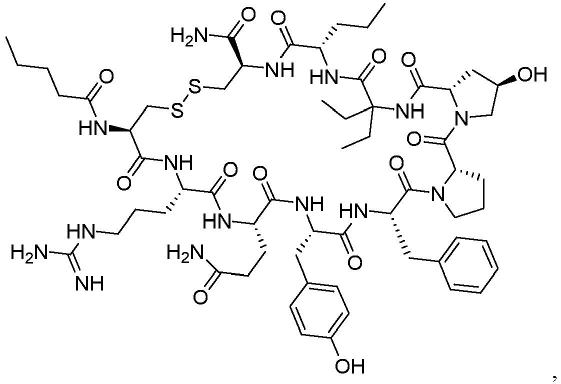
compound iHex-[Cys-Cit-Gln-Tyr-Hfe-Pro-ala-Ile-Leu-Cys]-NH₂ (UPAR-180) of the following formula 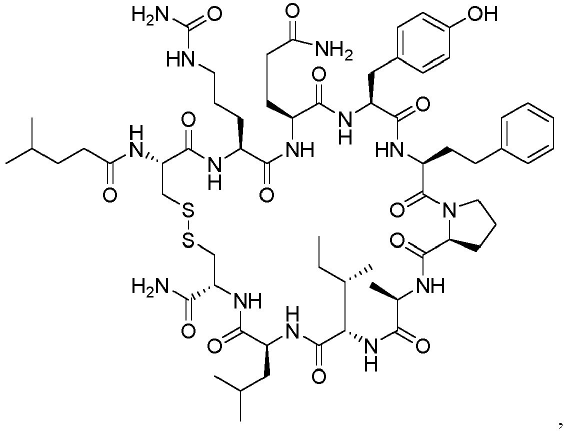
compound Butoc-[Cys-Thr-Lys(DOTA-PPAc)-Tyr-Phe-4Cfp-Hyp-Ile-Nva-Cys]-NH₂ (UPAR-181) of the following formula 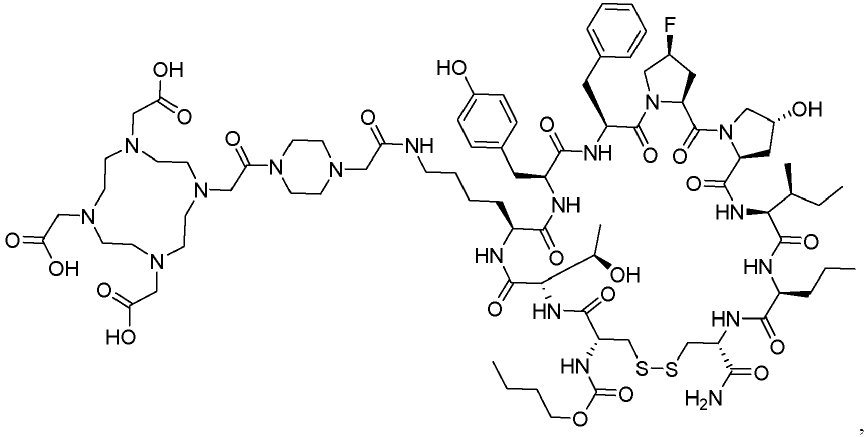
compound Pent-[Cys-Cit-Lys(InDOTA)-Tyr-Phe-Pro-Hyp-Ile-Nva-Cys]-NH₂ (UPAR-182) of the following formula 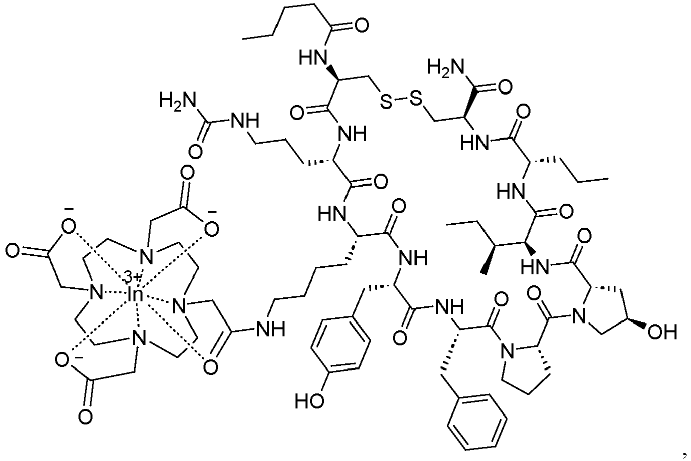
compound Pent-[Cys-KMe3-Lys(LaDOTA)-Tyr-Phe-Pro-Hyp-Ile-Nva-Cys]-NH₂ (UPAR-183) of the following formula 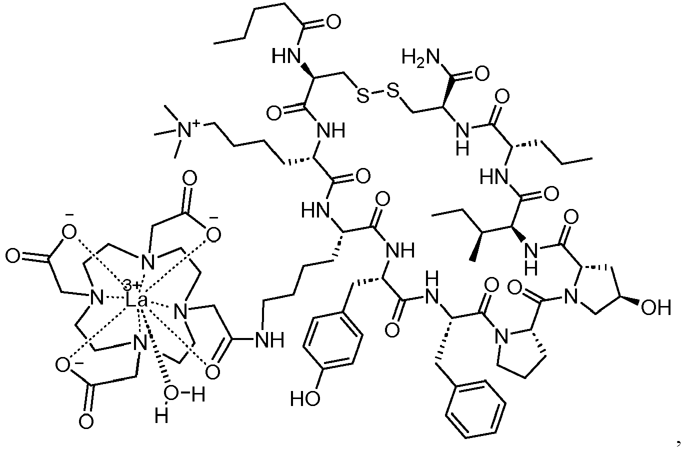
compound iHex-[Cys-Cit-Gln-Tyr-Phe-Pro-ala-Ile-Val-Cys]-NHz (UPAR-184) of the following formula 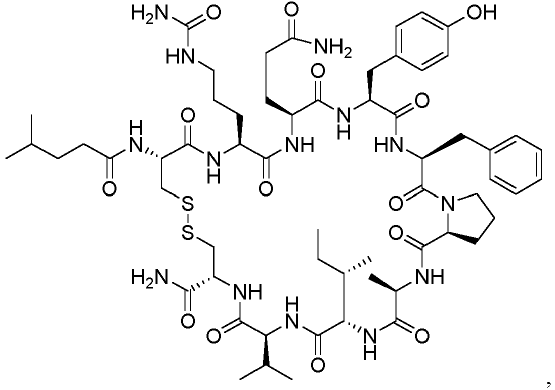
compound Pent-[Cys-Apc-Gln-Tyr-Phe-Pro-Hyp-Ile-Nva-Cys]-NH₂ (UPAR-186) of the following formula 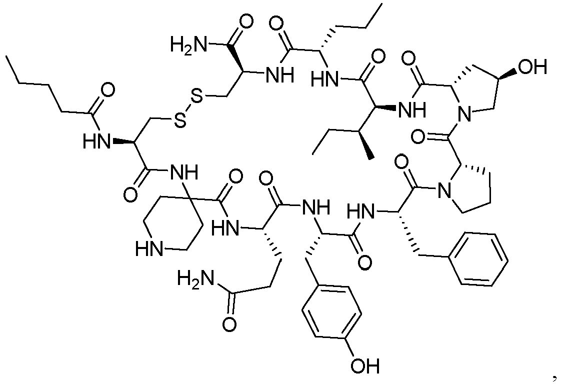
compound iHex-[Cys-Cit-Gln-Tyr-Phe-Pro-ala-Cpa-Leu-Cys]-NHz (UPAR-187) of the following formula 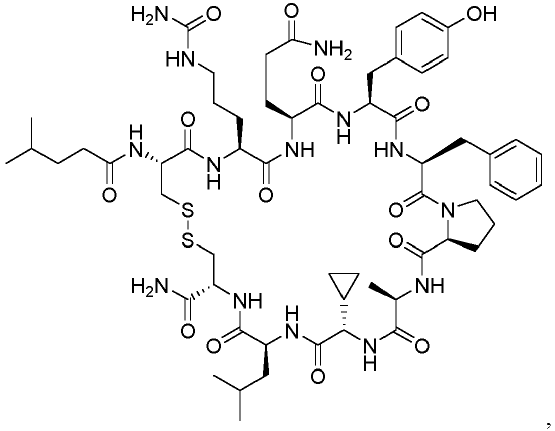
compound iHex-[Cys-Cit-Gln-Mcy-Phe-Pro-ala-Ile-Leu-Cys]-NHz (UPAR-188) of the following formula 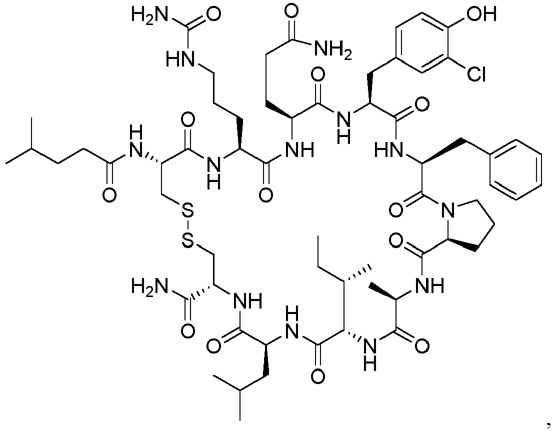
compound Ac-Asp-Leu-[Cys-Cit-Lys(DOTA)-Tyr-Phe-Pro-ala-Ile-Leu-Cys]-NH₂ (UPAR-189) of the following formula 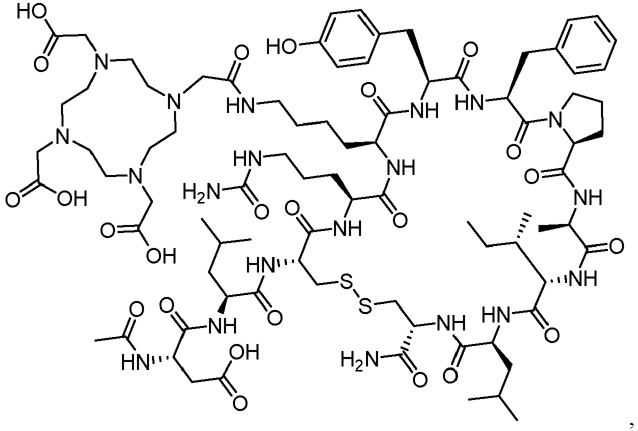
compound Pent-[Cys-Arg-Gln-Tyr-Phe-Pro-Oic-Ile-Nva-Cys]-NH₂ (UPAR-190) of the following formula 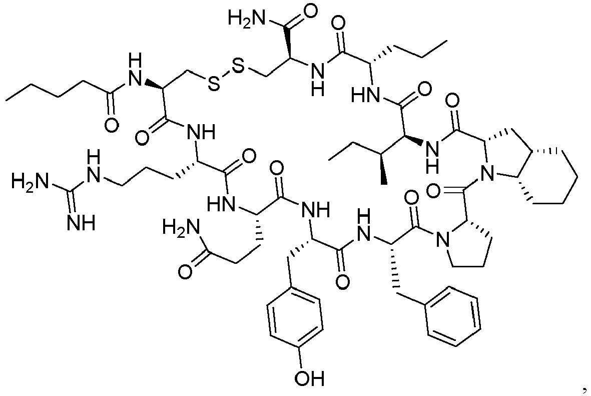
compound Butoc-[Cys-Thr-Lys(InDOTA)-Dopa-Phe-4Cfp-Hyp-Ile-Nva-Cys]-NH₂ (UPAR-191) of the following formula 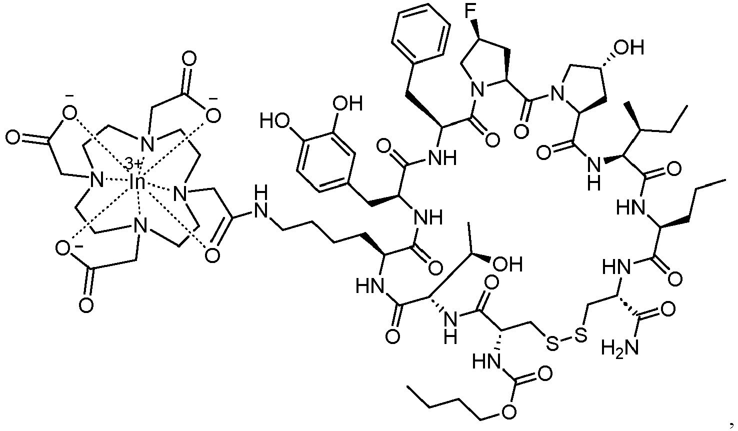
compound Butoc-[Cys-Thr-Orn(DOTA)-Tyr-Phe-4Cfp-Hyp-Ile-Nva-Cys]-NH₂ (UPAR-192) of the following formula 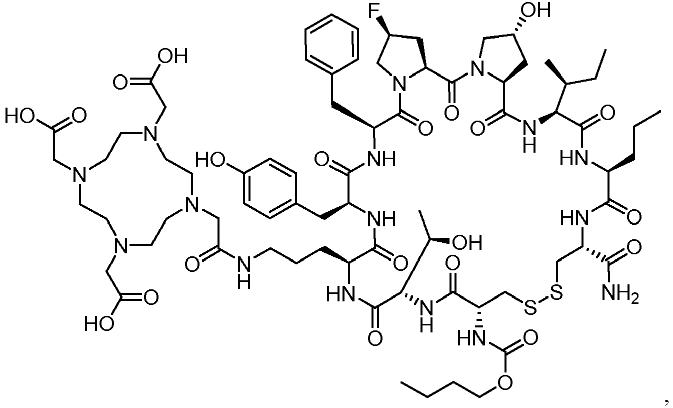
compound Oct-[Cys-Arg-Gln-Tyr-Phe-Pro-Hyp-Ile-Nva-Cys]-NH₂ (UPAR-193) of the following formula 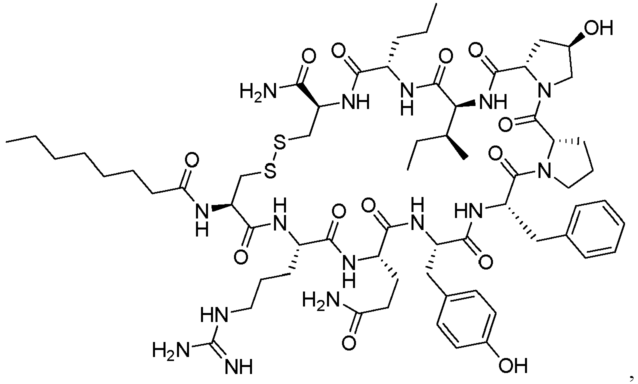
compound Butoc-[Cys-Thr-Lys(DOTA-Kzw)-Dopa-Phe-4Cfp-Hyp-Ile-Nva-Cys]-NH₂ (UPAR-194) of the following formula 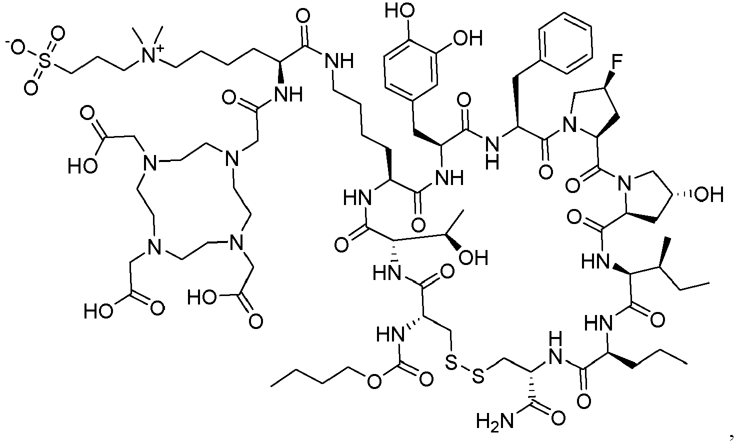
compound Pent-[Cys-Arg-Gln-Leu-Phe-Pro-Hyp-Ile-Nva-Cys]-NHz (UPAR-195) of the following formula 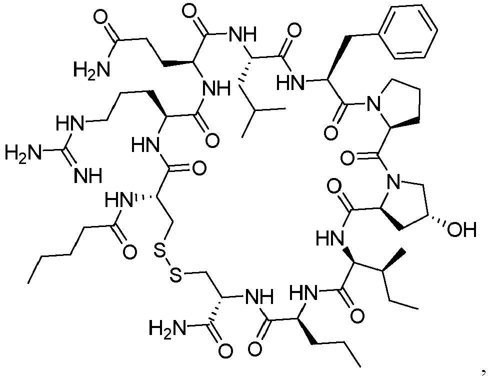
compound Pent-[Cys-Arg-Gln-Phg-Phe-Pro-Hyp-Ile-Nva-Cys]-NHz (UPAR-196) of the following formula 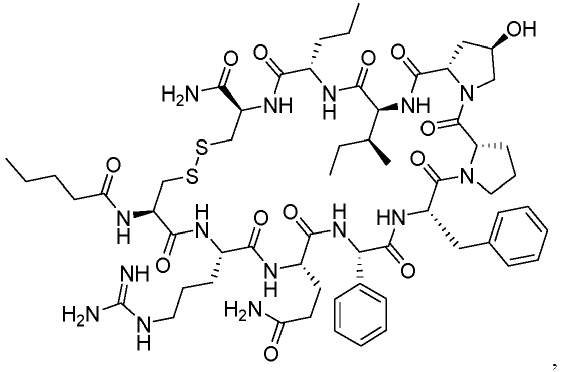
compound Pent-[Cys-Arg-Gln-Tyr-Leu-Pro-Hyp-Ile-Nva-Cys]-NH₂ (UPAR-197) of the following formula 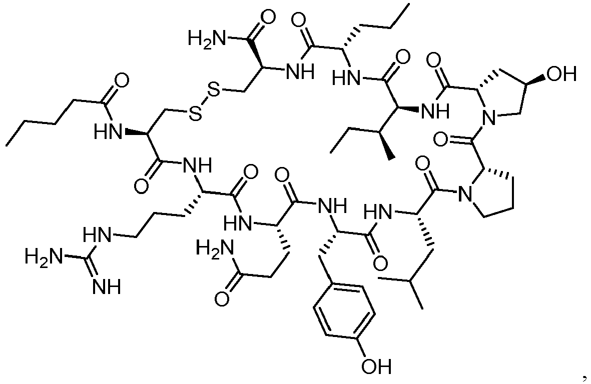
compound Ac-Asp-Leu-[Cys-Arg-Nle-tyr-Phe-Pro-Ala-Ile-Leu-Cys]-NH₂ (UPAR-198) of the following formula 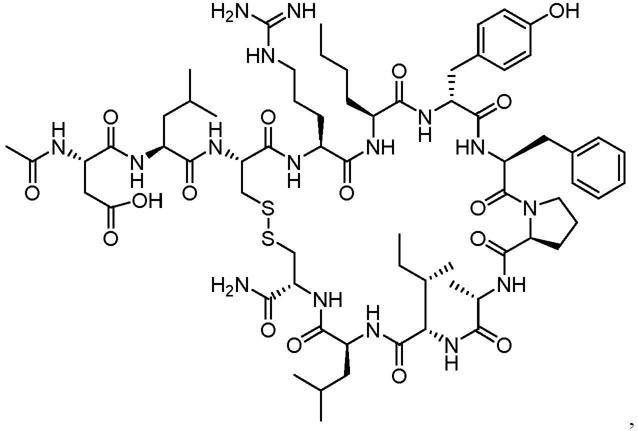
compound Butoc-[Cys-Thr-Lys(DOTA-PPAc)-Dopa-Phe-4Cfp-Hyp-Ile-Nva-Cys]-NH₂ (UPAR-199) of the following formula 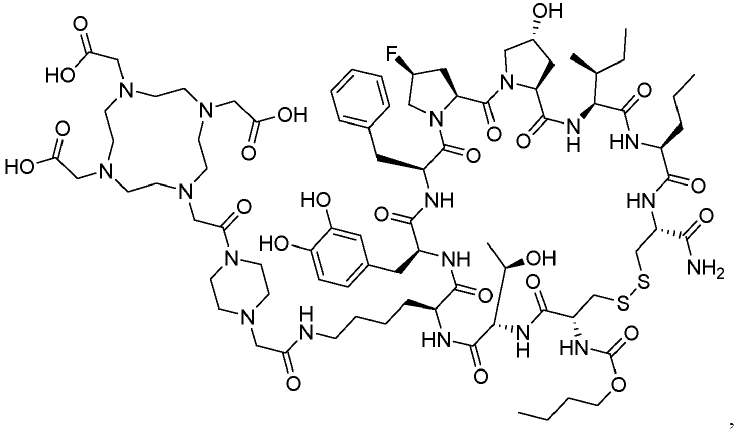
compound Pent-[Cys-Cit-Lys(DOTA-PEG12)-Tyr-Phe-Pro-Hyp-Ile-Nva-Cys]-NH₂ (UPAR-200) of the following formula 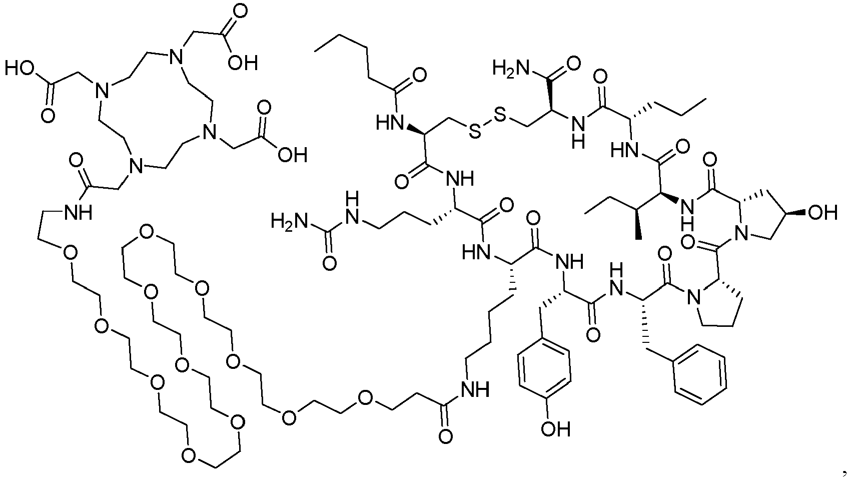
compound iHex-[Cys-Cit-Gln-Tyr-Phe-Pro-ala-Tle-Leu-Cys]-NH₂ (UPAR-201) of the following formula 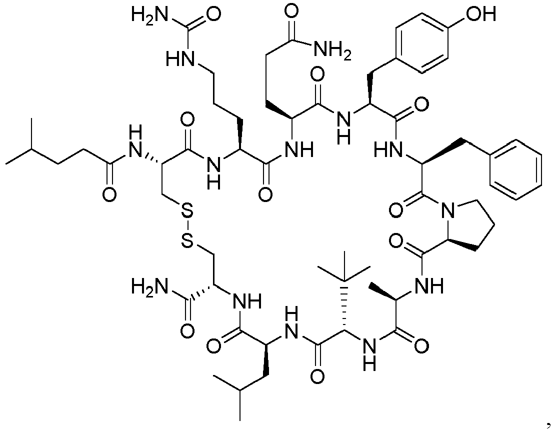
compound Hex-Ser-Leu-[Cys-Asp-Gln-Phe-Phe-Pro-Ala-Ile-Leu-Cys]-NH₂ (UPAR-202) of the following formula 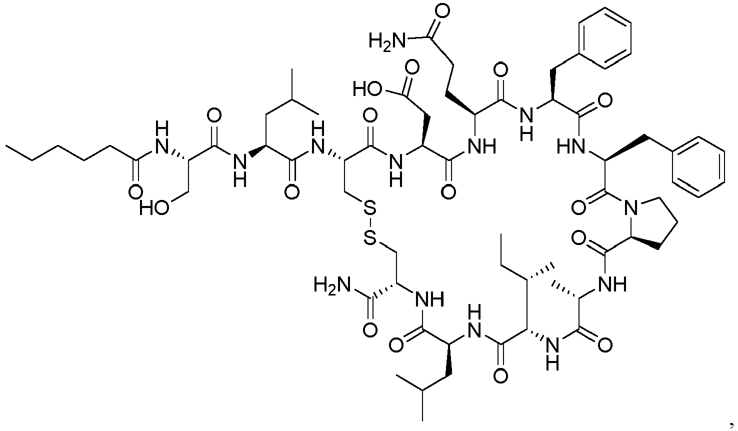
compound Pent-[Cys-Arg-Gln-Tyr-Phg-Pro-Hyp-Ile-Nva-Cys]-NHz (UPAR-203) of the following formula 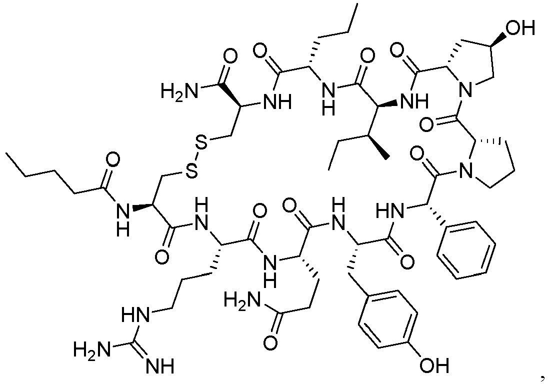
compound Pent-[Cys-Arg-Gln-Tyr-Phe-Pro-Hyp-Pcf-Nva-Cys]-NH₂ (UPAR-204) of the following formula 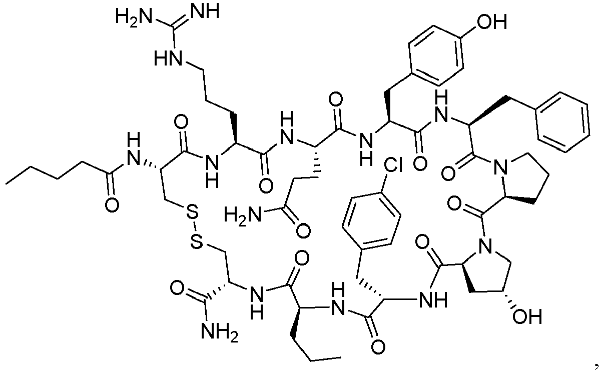
compound Pent-Ala-Cit-Gln-Tyr-Phe-Pro-Hyp-Ile-Nva-Smc-NHz (UPAR-205) of the following formula 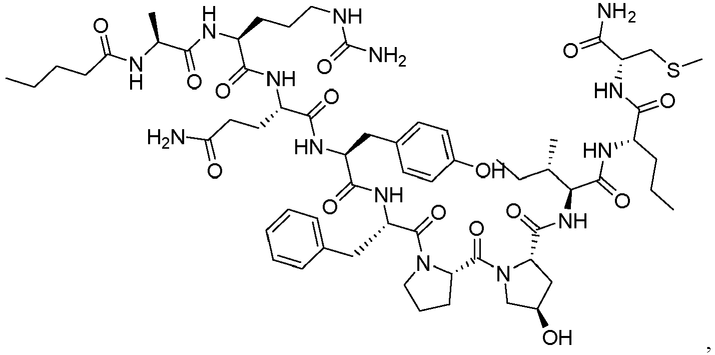
compound Pent-[Cys-Cit-Gln-Tyr-Phe-Pro-Hyp-Ile-Nva-Cys]-Gly-NH₂ (UPAR-206) of the following formula 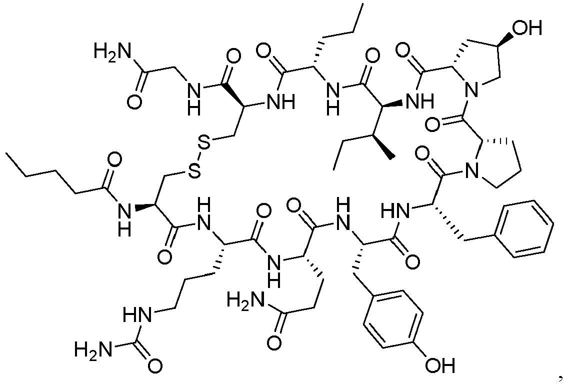
compound Pent-[Cys-Cit-Lys(DOTA)-Tyr-Mpa-Pro-4Tfp-Ile-Nva-Cys]-NH₂ (UPAR-207) of the following formula 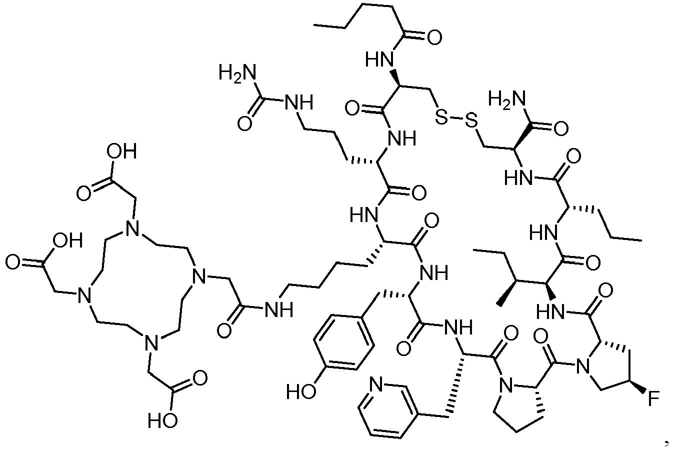
compound Cp-[Cys-Cit-Gln-Tyr-Phe-Pro-ala-Ile-Leu-Cys]-NH₂ (UPAR-208) of the following formula 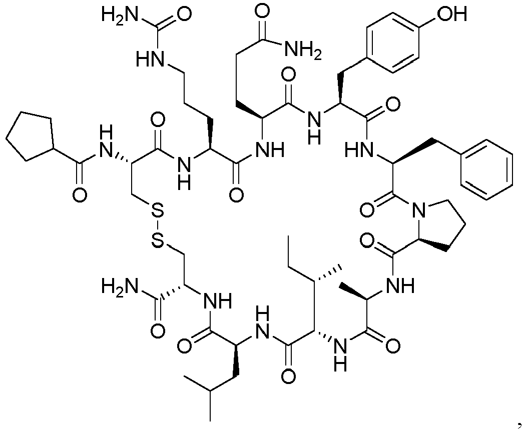
compound Pent-[Cys-Cit-Lys(DOTA)-Fso-Phe-Pro-Hyp-Ile-Nva-Cys]-NH₂ (UPAR-209) of the following formula 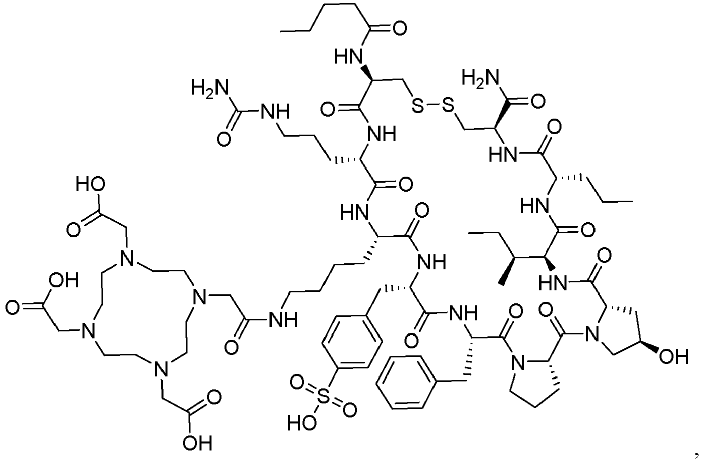
compound PrCAyl-[Cys-Cit-Lys(DOTA)-Tyr-Phe-Pro-Hyp-Ile-Nva-Cys]-NH₂ (UPAR-210) of the following formula 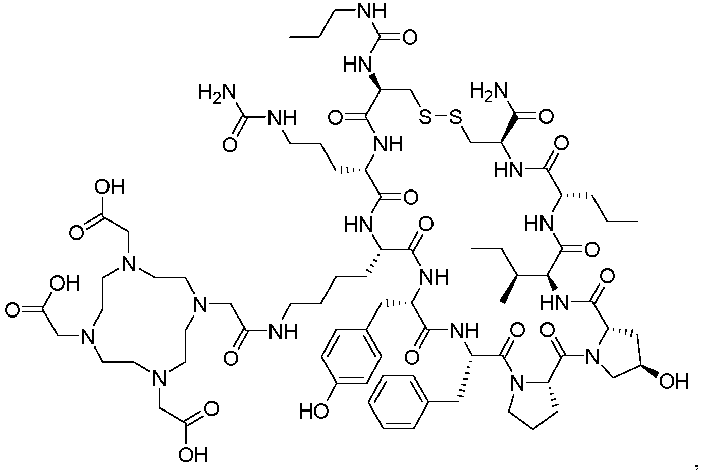
compound Pent-[Cys-Arg-Gln-Tyr-Phe-nma-Hyp-Ile-Nva-Cys]-NH₂ (UPAR-211) of the following formula 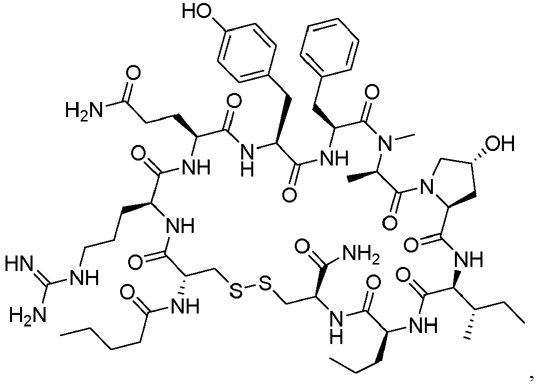
compound PrOAc-[Cys-Cit-Gln-Tyr-Phe-Pro-ala-Ile-Leu-Cys]-NHz (UPAR-212) of the following formula 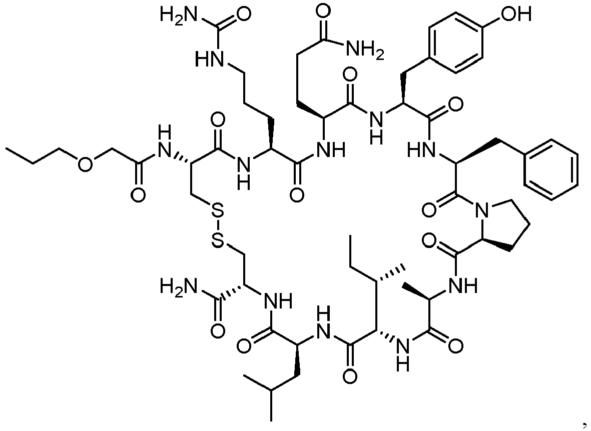
compound Pent-[Cys-Arg-Gln-Tyr-Phe-Pro-Hyp-Ile-Nva-Hcy]-NHz (UPAR-213) of the following formula 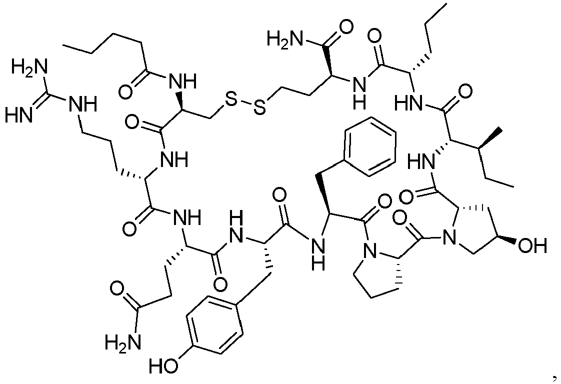
compound Pent-[Cys-Cit-Lys(DOTA)-Tyr-Phe-Pro-Chy-Ile-Nva-Cys]-NH₂ (UPAR-214) of the following formula 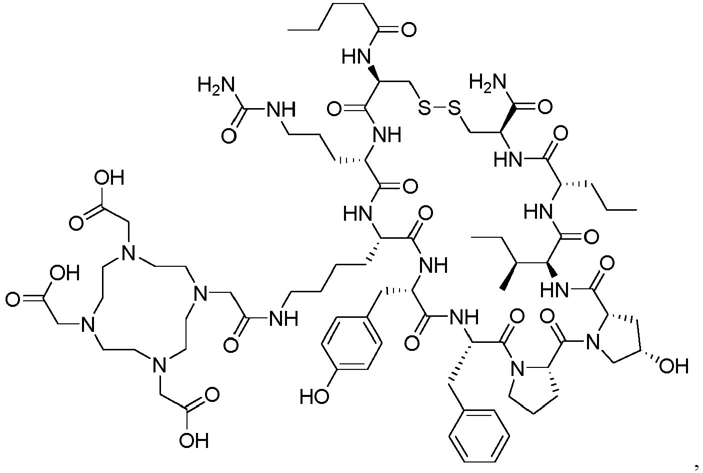
compound Pent-[Smc-Cit-Gln-Tyr-Phe-Pro-Hyp-Ile-Nva-Cys]-NHz (UPAR-215) of the following formula 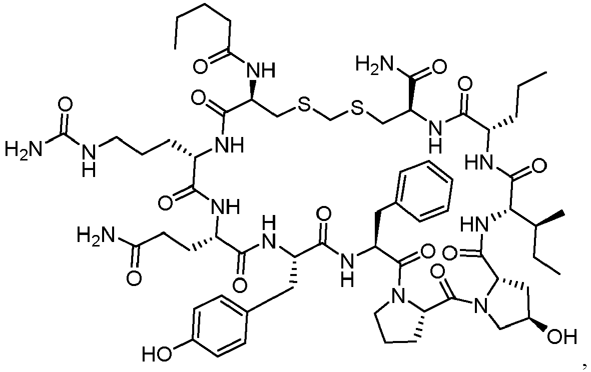
compound DOTA-O2Oc-Leu-[Cys-ala-Gln-Tyr-Phe-Pro-ala-Ile-Leu-Cys]-NH₂ (UPAR-216) of the following formula 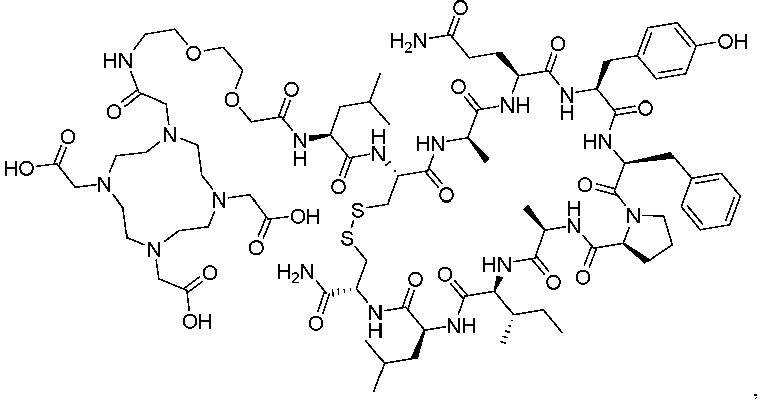
compound Pent-[Cys-Cit-Gln-Tyr-Phe-Pro-Hyp-Ile-Nva-Pen]-NH₂ (UPAR-217) of the following formula 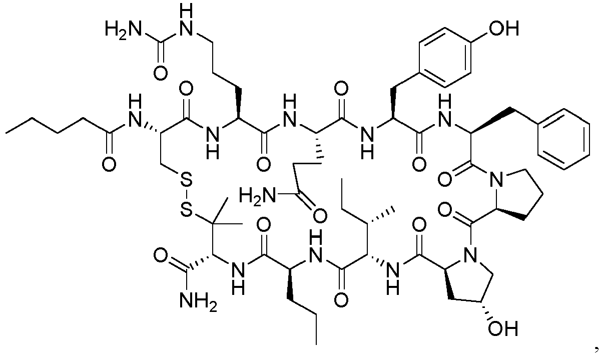
compound Ac-Asp-Leu-[Cys-Arg-Nle-Tyr-Phe-pro-Ala-Ile-Leu-Cys]-NH₂ (UPAR-218) of the following formula 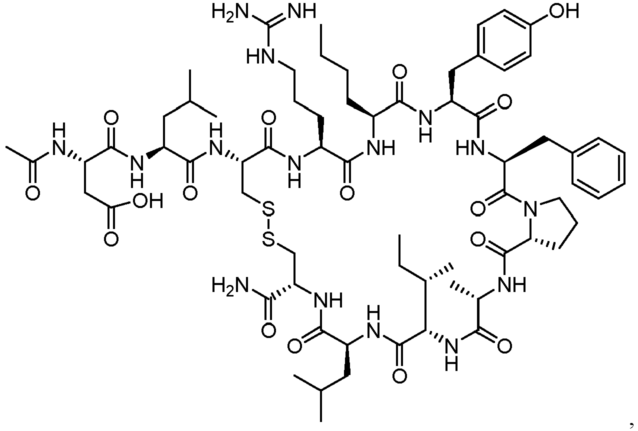
compound iHex-[Cys-Cit-Gln-Tyr-Phe-Pro-Apc(DOTA)-Ile-Leu-Cys]-NH₂ (UPAR-219) of the following formula 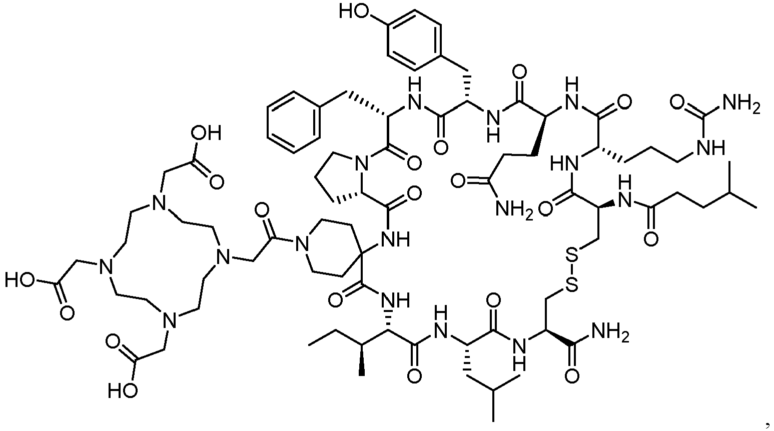
compound iHex-[Cys-Cit-Gln-Tyr-Phe-Pro-4Tfp-Ile-Leu-Cys]-NH₂ (UPAR-220) of the following formula 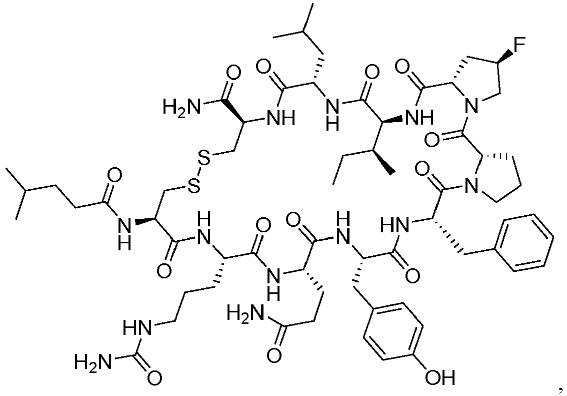
compound iHex-[Cys-Cit-Gln-Tyr-Ocf-Pro-ala-Ile-Leu-Cys]-NH₂ (UPAR-221) of the following formula 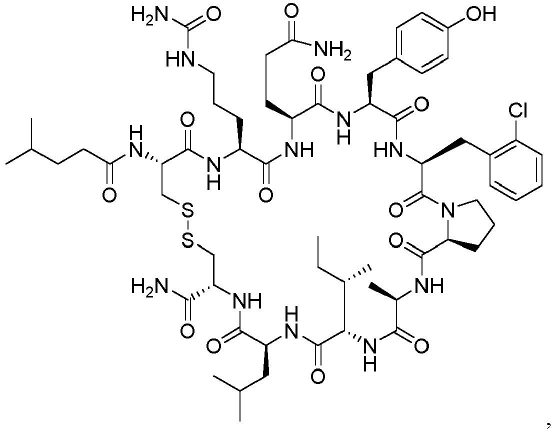
compound Pent-[Cys-Cit-Lys(DOTA)-Tyr-Ppa-Pro-Hyp-Ile-Nva-Cys]-NH₂ (UPAR-222) of the following formula 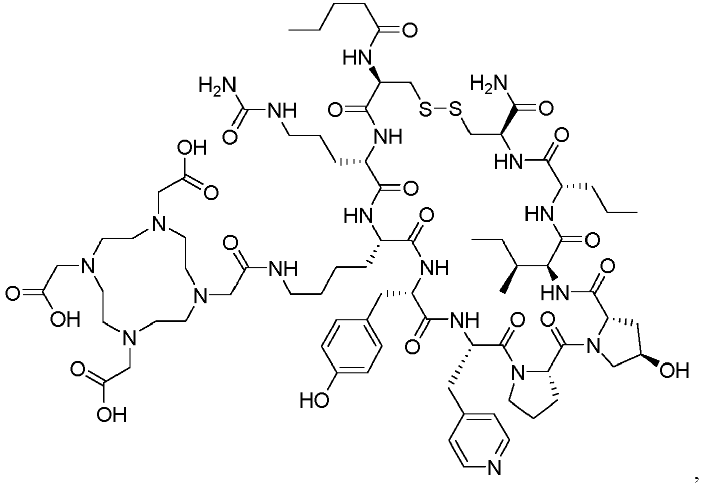
compound DOTA-Phe-[Cys-Arg-Gln-Tyr-Phe-Pro-Hyp-Ile-Nva-Cys]-NHz (UPAR-223) of the following formula 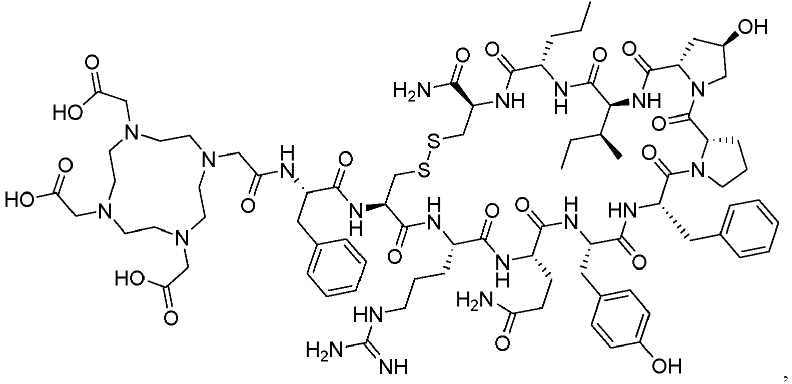
compound Pent-[Cys-Orn(Tris-Nta(Tris'))-Lys(DOTA)-Tyr-Phe-Pro-Hyp-Ile-Nva-Cys]-NH₂ (UPAR-224) of the following formula 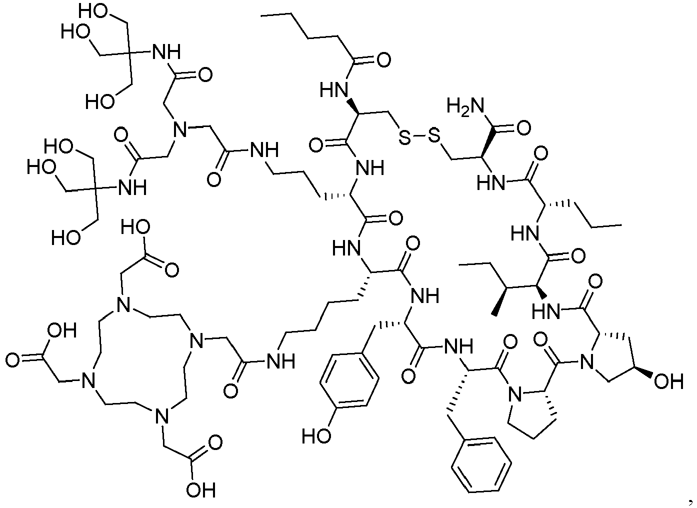
compound iHex-[Cys-Cit-Gln-Tyr-Phe-Pro-ala-Chg-Leu-Cys]-NHz (UPAR-225) of the following formula 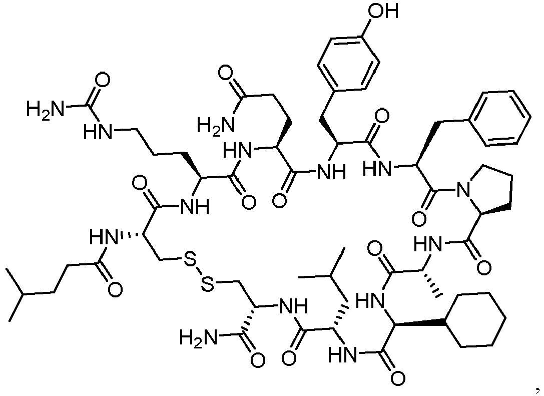
compound Pent-[Cys-Arg-Gln-Tyr-Phe-Pro-Tyr-Ile-Nva-Cys]-NHz (UPAR-226) of the following formula 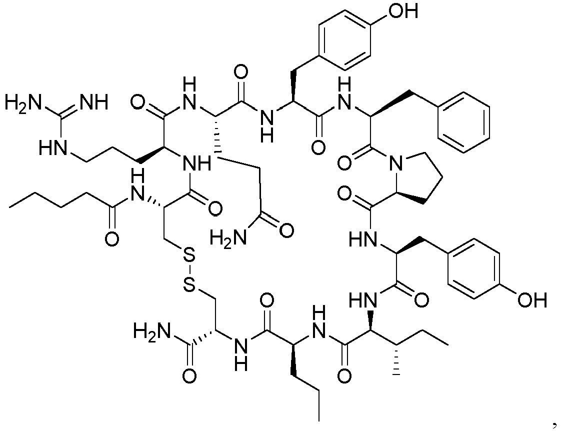
compound iHex-[Cys-Cit-Gln-Tyr-Mcf-Pro-ala-Ile-Leu-Cys]-NH₂ (UPAR-227) of the following formula 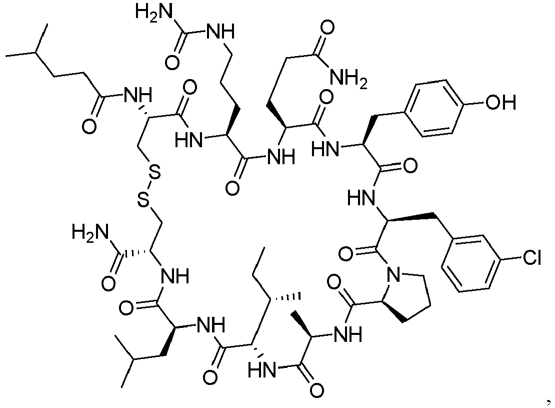
compound Butoc-[Cys-Thr-Dab(DOTA)-Tyr-Phe-4Cfp-Hyp-Ile-Nva-Cys]-NH₂ (UPAR-228) of the following formula 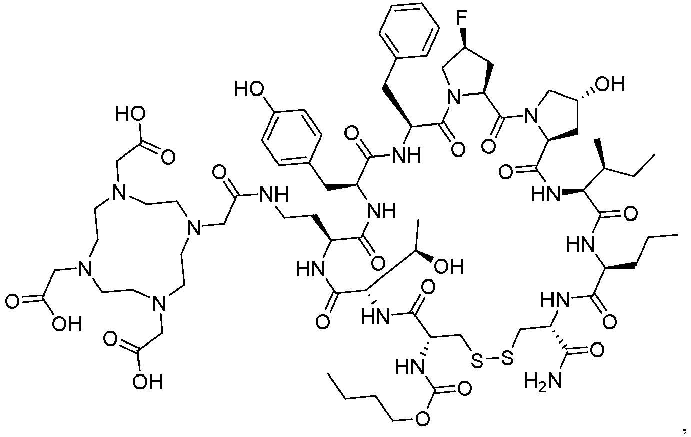
compound DOTA-O2Oc-Leu-[Cys-Cit-Gln-Tyr-Phe-Pro-Pro-Ile-Leu-Cys]-NH₂ (UPAR-229) of the following formula 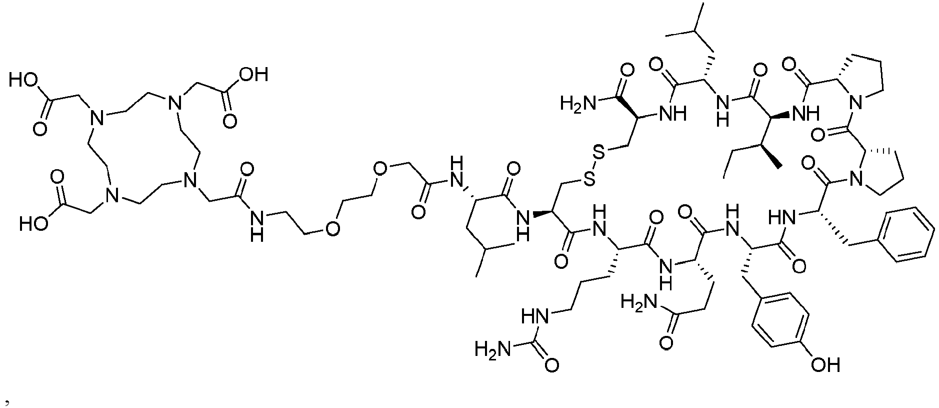
compound Ac-Asp-Leu-[Cys-Arg-Nle-Tyr-phe-Pro-Ala-Ile-Leu-Cys]-NH₂ (UPAR-230) of the following formula 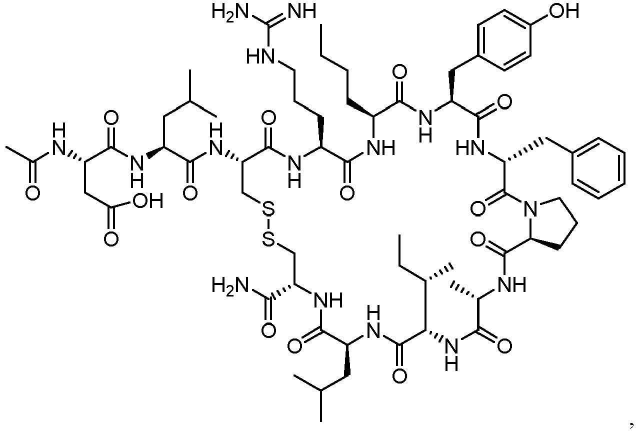
compound iHex-[Cys-ala-Gln-Tyr-Phe-Pro-ala-Ile-Leu-Cys]-NH₂ (UPAR-231) of the following formula 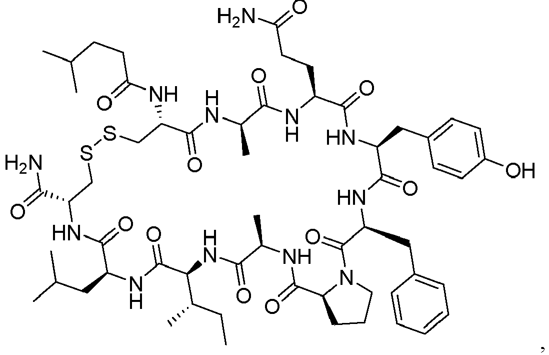
compound iHex-[Cys-Cit-Gln-Tyr-Phe-Pip-ala-Ile-Leu-Cys]-NH₂ (UPAR-232) of the following formula 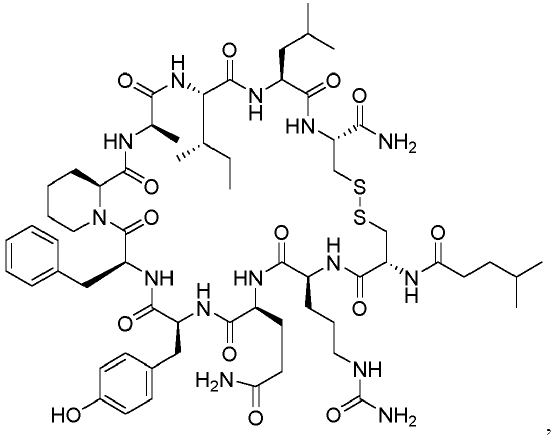
compound Pent-[Cys-Cit-Lys(DOTA)-Tyr-Pcf-Pro-Hyp-Ile-Nva-Cys]-NH₂ (UPAR-233) of the following formula 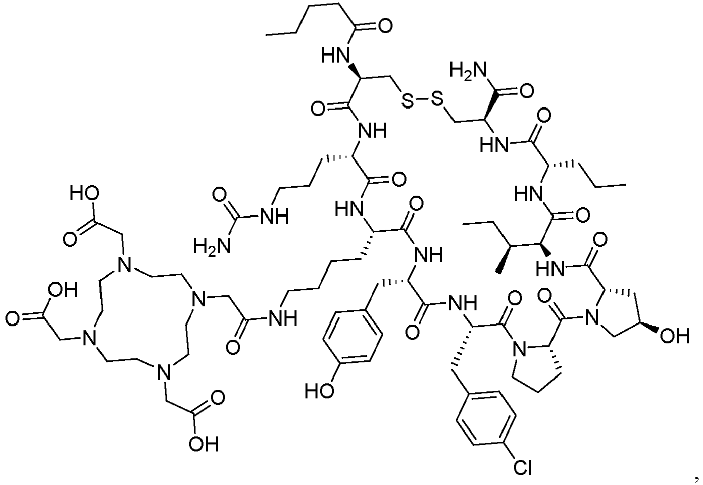
compound Butoc-[Cys-Thr-Orn(DOTA)-Ay3-Phe-4Cfp-Hyp-Ile-Nva-Cys]-NH₂ (UPAR-234) of the following formula 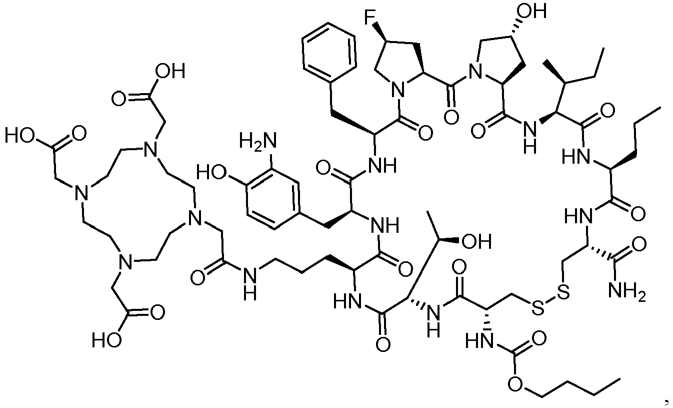
compound Hex-Asp-Leu-[Cys-Lys(DOTA)-Met-Tyr-Phe-Pro-Ala-Ile-Leu-Cys]-NH₂ (UPAR-235) of the following formula 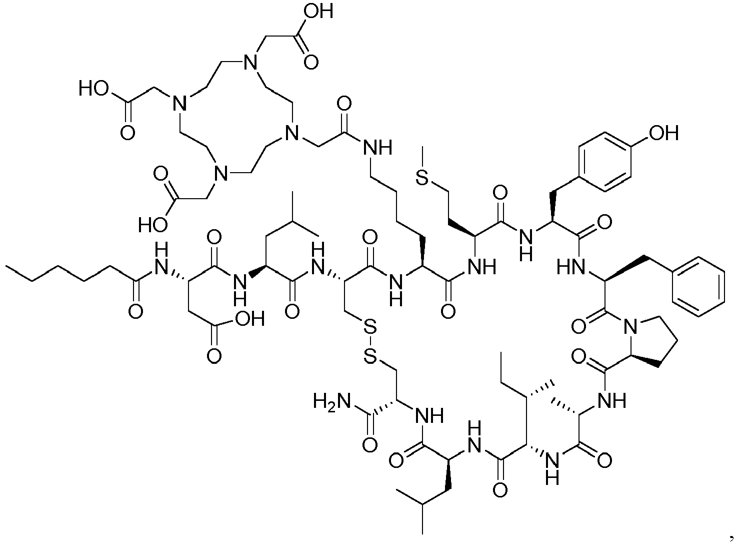
compound Pent-[Cys-Gly-Gln-Tyr-Phe-Pro-Hyp-Ile-Nva-Cys]-NHz (UPAR-236) of the following formula 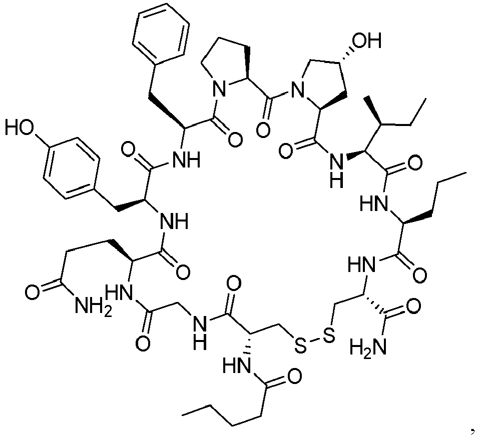
compound Butoc-[Cys-Thr-Lys(DOTA)-Tyr-Phe-4Cfp-Hyp-Ile-Nva-Cys]-NH₂ (UPAR-237) of the following formula 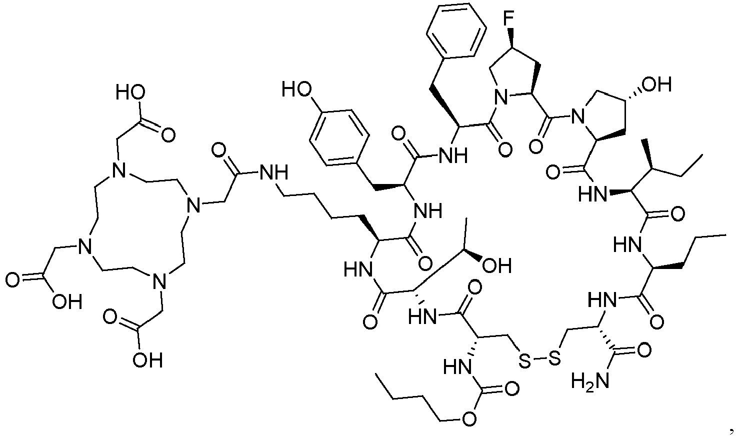
compound Butoc-[Cys-Thr-Lys(LuDOTA)-Dopa-Phe-4Cfp-Hyp-Ile-Nva-Cys]-NH₂ (UPAR-238) of the following formula 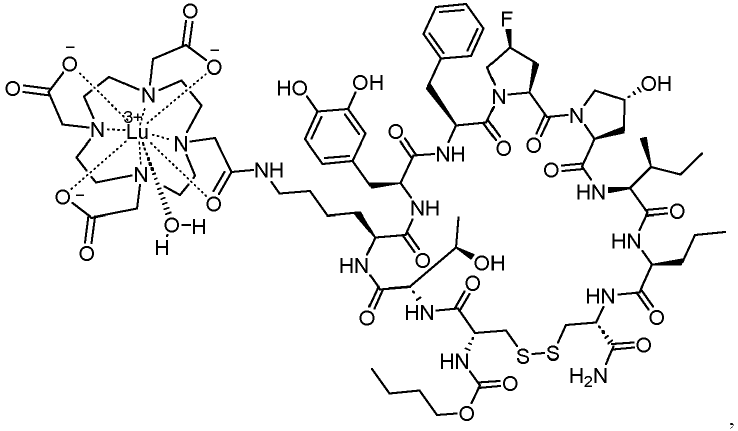
compound Ac-Asp-Leu-[Cys-Glu-Nle-Tyr-Phe-Pro-Ala-Ile-Leu-Cys]-NH₂ (UPAR-239) of the following formula 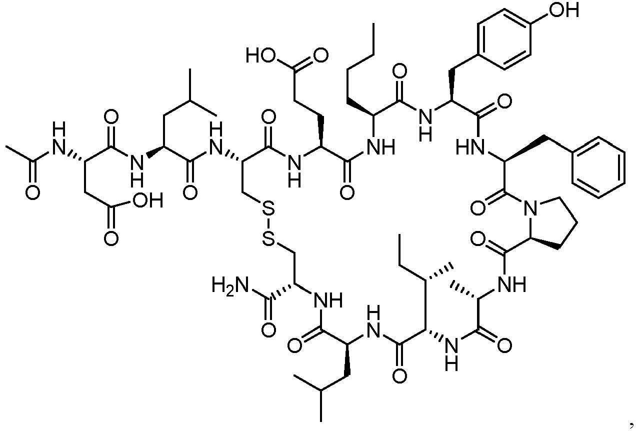
compound iHex-[Cys-Cit-Lys(DOTA)-Tyr-Phe-Pro-Hyp-Ile-Leu-Cys]-NH₂ (UPAR-240) of the following formula 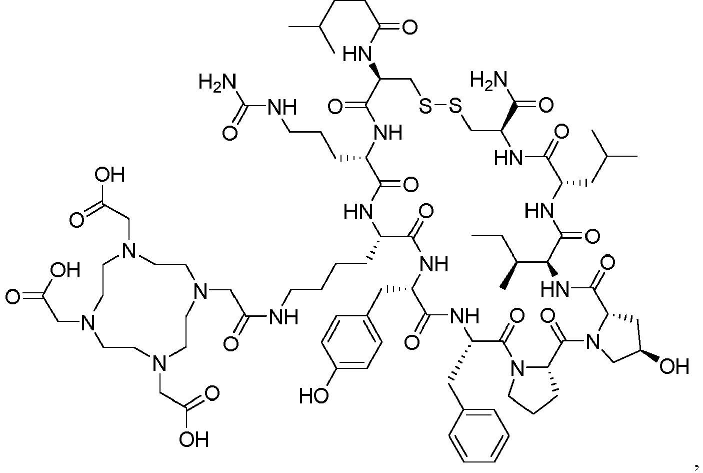
compound Butoc-[Cys-Thr-Lys(GaDOTA)-Dopa-Phe-4Cfp-Hyp-Ile-Nva-Cys]-NH₂ (UPAR-241) of the following formula 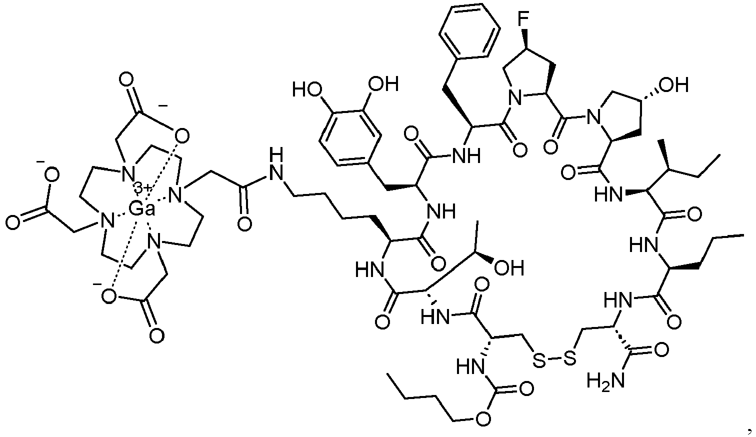
compound nBuCAyl-[Cys-Thr-Lys(DOTA)-Tyr-Phe-Pro-Hyp-Ile-Nva-Cys]-NH₂ (UPAR-242) of the following formula 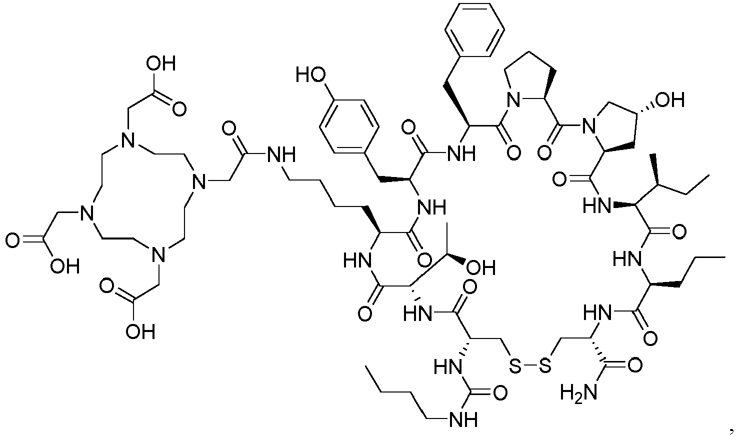
compound Ac-[Cys-Cit-Gln-Tyr-Phe-Pro-ala-Ile-Leu-Cys]-NHz (UPAR-243) of the following formula 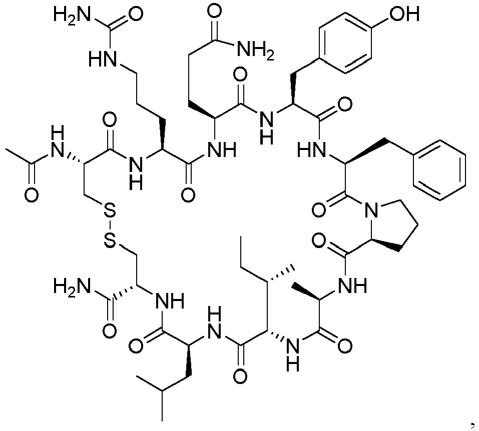
compound Ac-Asp-leu-[Cys-Arg-Nle-Tyr-Phe-Pro-Ala-Ile-Leu-Cys]-NH₂ (UPAR-244) of the following formula 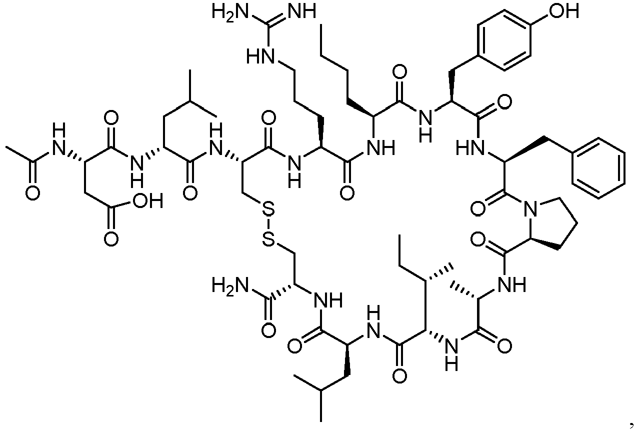
compound DOTA-O2Oc-Leu-[Cys-Cit-Gln-Tyr-Phe-Pro-ala-Ile-Leu-Cys]-NH₂ (UPAR-245) of the following formula 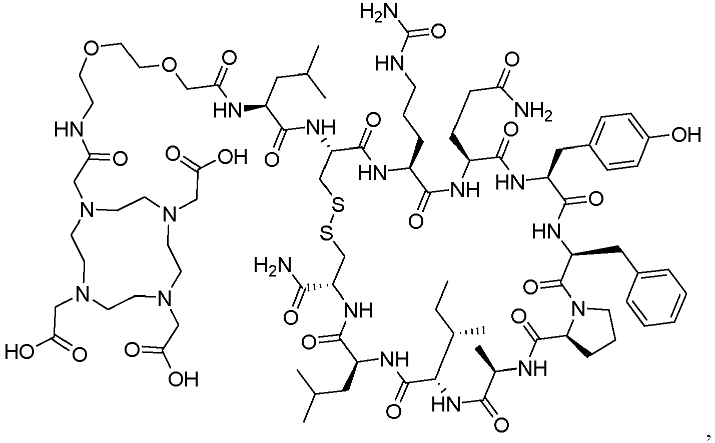
compound Pent-[Cys-Cit-Lys(DOTA)-Tyr-Phe-Pro-4Tfp-Ile-Nva-Cys]-NH₂ (UPAR-246) of the following formula 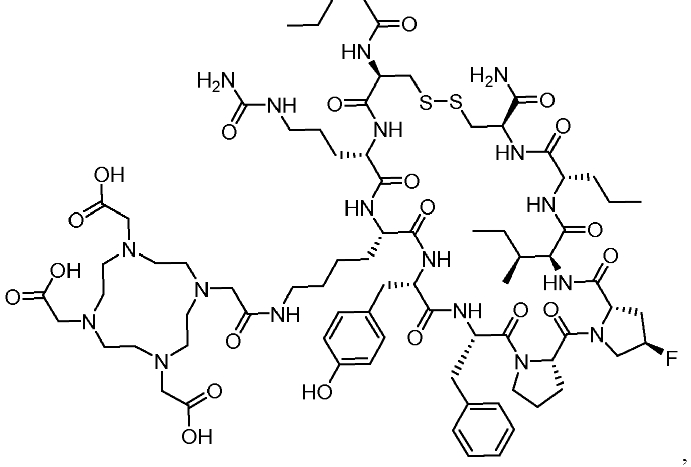
compound Pent-[Cys-Om(TrisPEG 12-PEG4-Glutar)-Lys(DOT A)- Tyr-Phe-Pro-Hyp-Ile- Nva-Cys]-NH₂ (UPAR-247) of the following formula 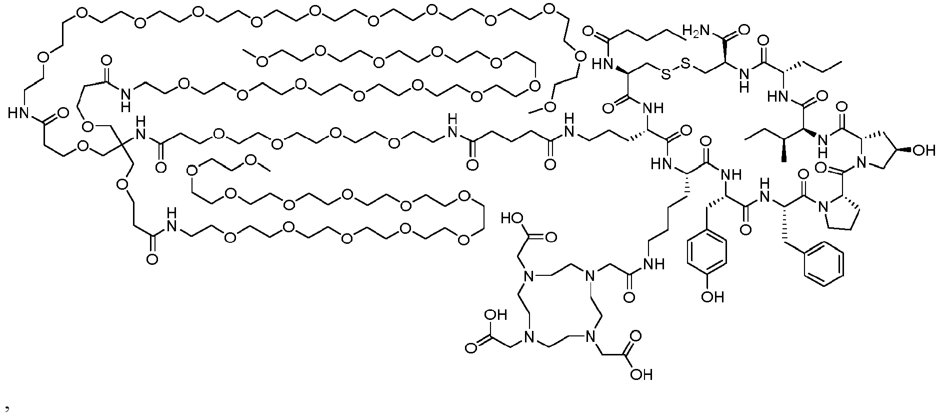
compound Butoc-[Cys-Thr-Lys(DOTA)-Tyr-Phe-Pro-Hyp-Ile-Nva-Cys]-NH₂ (UPAR-248) of the following formula 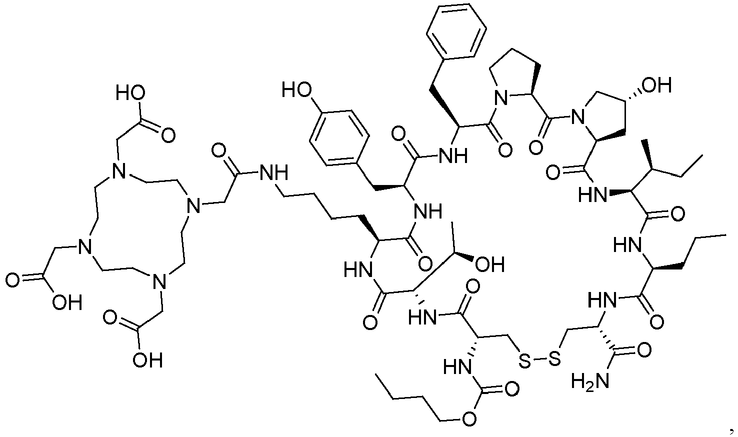
compound Butoc-[Cys-Thr-Lys(DOTA)-Dopa-Phe-4Cfp-Hyp-Ile-Nva-Cys]-NH₂ (UPAR-249) of the following formula 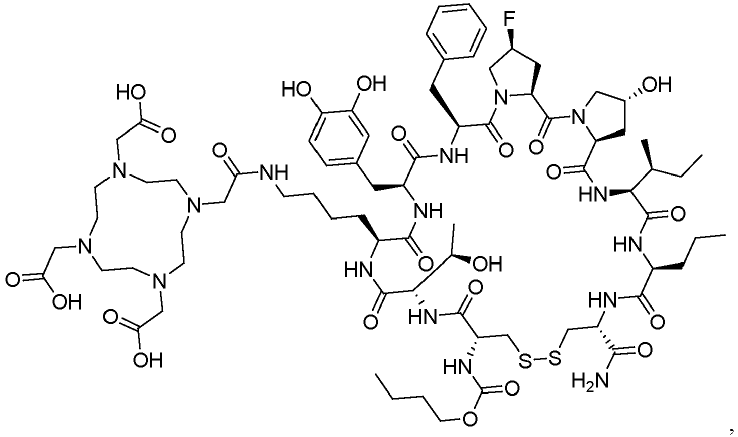
compound Butoc-[Cys-Thr-Lys(NOTA)-Dopa-Phe-4Cfp-Hyp-Ile-Nva-Cys]-NH₂ (UPAR-250) of the following formula 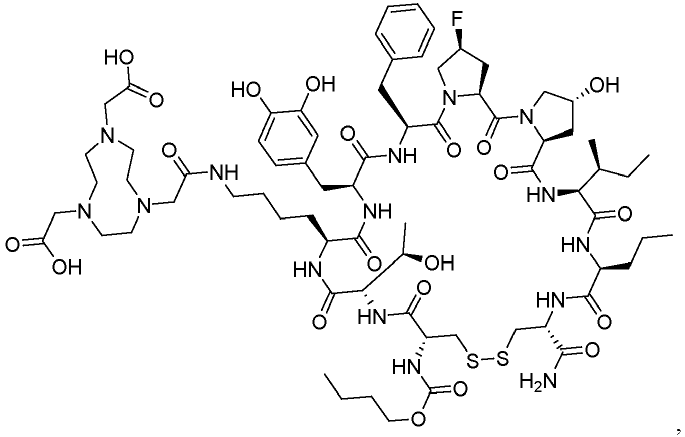
compound Pent-[Cys-Cit-Lys(GdDOTA)-Mpa-Phe-Pro-Hyp-Ile-Nva-Cys]-NH₂ (UPAR-251) of the following formula 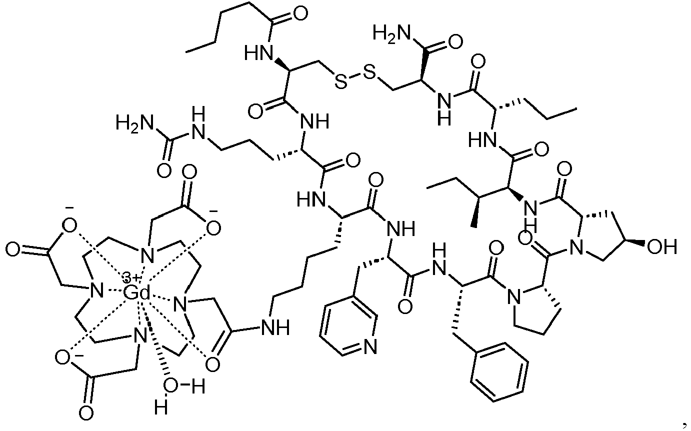
compound Pent-[Cys-Cit-Lys(DOTA)-Tyr-Tic-Pro-Hyp-Ile-Nva-Cys]-NH₂ (UPAR-252) of the following formula 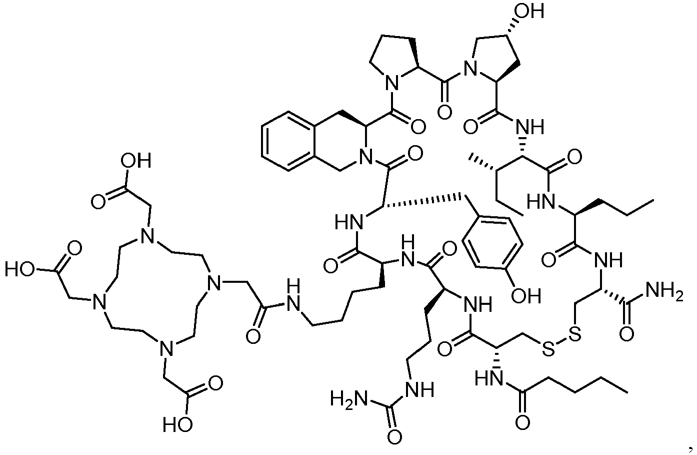
compound Pent-[Cys-Cit-Lys(DOTA)-Tyr-Mpa-Pro-Hyp-Ile-Nva-Cys]-NH₂ (UPAR-253) of the following formula 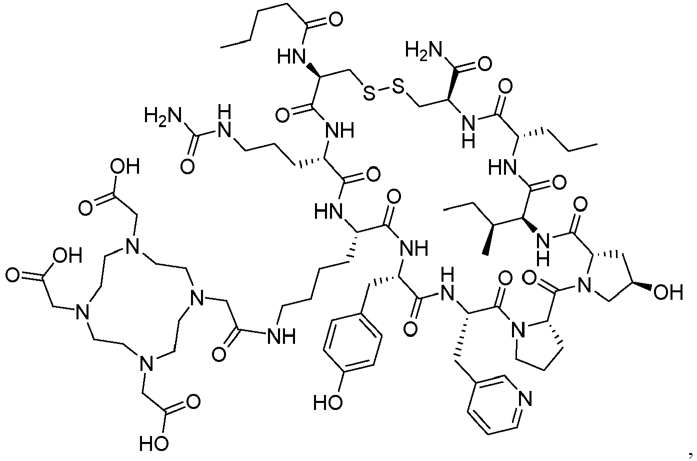
compound Pent-[Cys-Cit-Lys(LuDOTA)-Tyr-Egm-Pro-Hyp-Ile-Nva-Cys]-NH₂ (UPAR-254) of the following formula 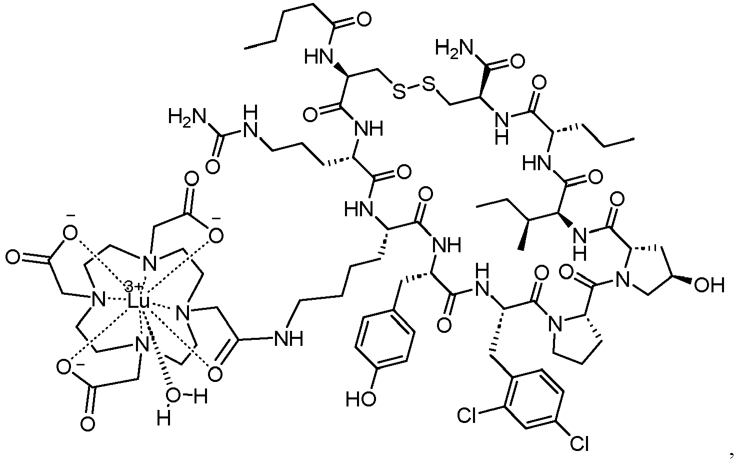
compound iHex-[Cys-Cit-Glu-Tyr-Phe-Pro-ala-Ile-Leu-Cys]-NH₂ (UPAR-255) of the following formula 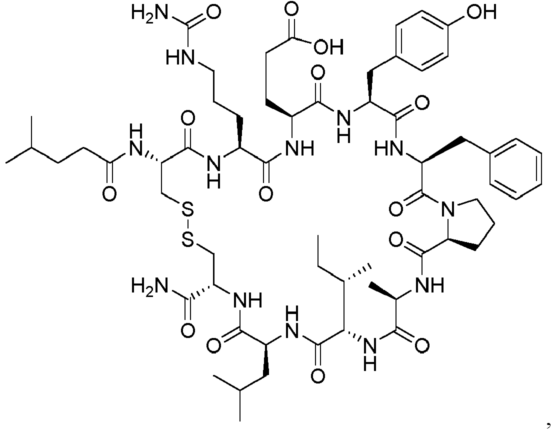
compound iHex-[Cys-Cit-Lys(DOTA)-Tyr-Phe-Pro-ala-Ile-Leu-Cys]-NH₂ (UPAR-256) of the following formula 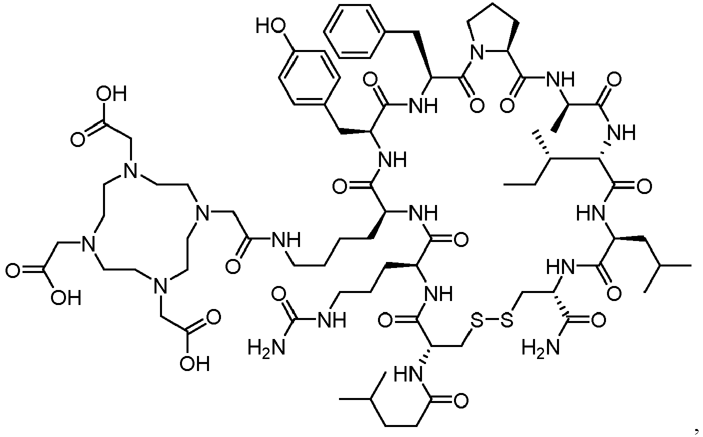
compound Pent-[Cys-Cit-Lys(DOTA)-Nif-Phe-Pro-Hyp-Ile-Nva-Cys]-NH₂ (UPAR-257) of the following formula 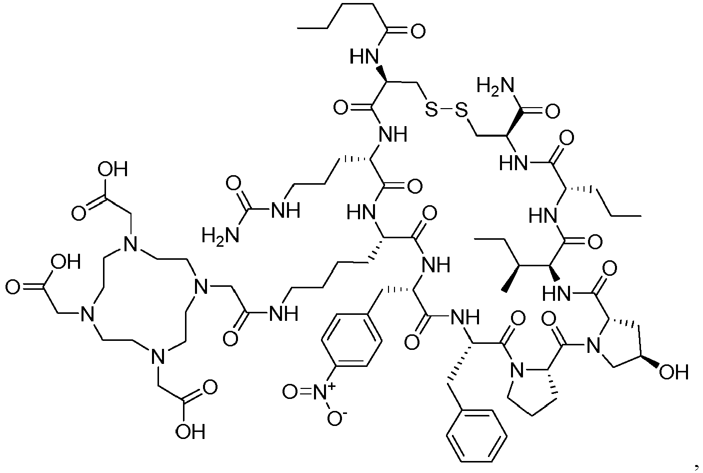
compound Pent-[Cys(2MeBn)-Arg-Gln-Tyr-Phe-Pro-Hyp-Ile-Nva-Cys]-NH₂ (UPAR-258) of the following formula 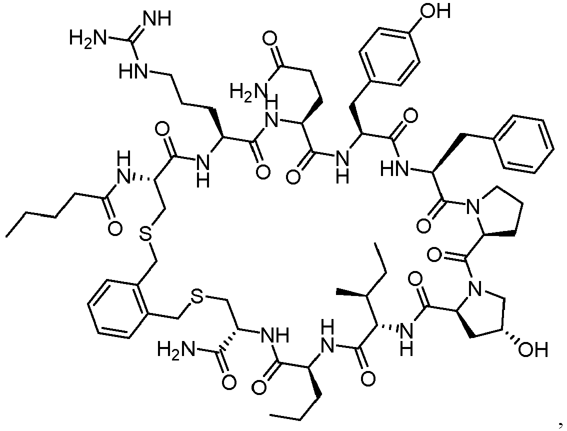
compound Pent-[Cys-Arg-Gln-2Ni-Phe-Pro-Hyp-Ile-Nva-Cys]-NH₂ (UPAR-259) of the following formula 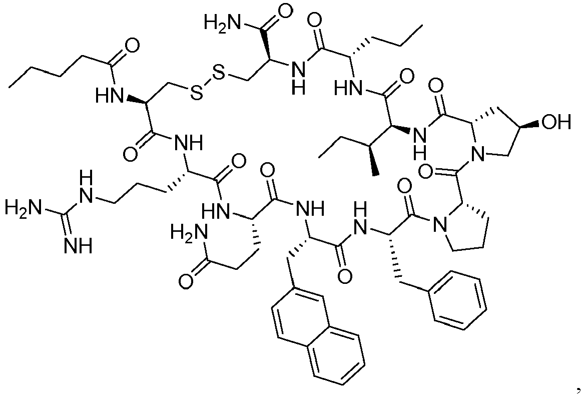
compound Pent-[Cys-Cit-Lys(DOTA)-Tyr-Phe-Pro-Hyp-Ile-Nva-Cys]-NH₂ (UPAR-261) of the following formula 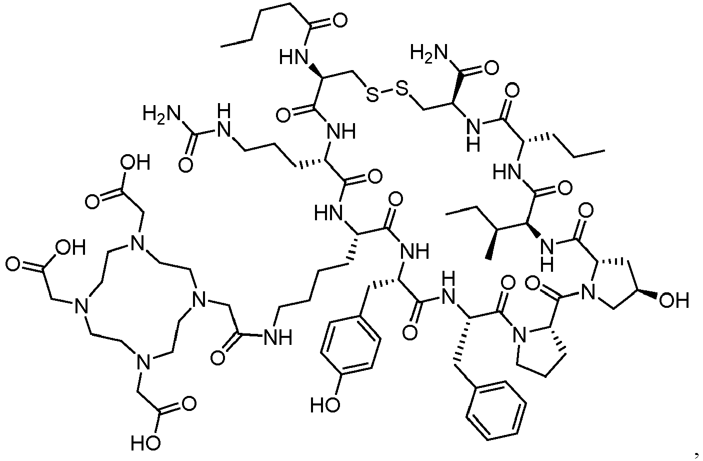
compound iHex-[Cys-Cit-Gln-Tyr-Phe-Pro-Oxa-Ile-Leu-Cys]-NHz (UPAR-262) of the following formula 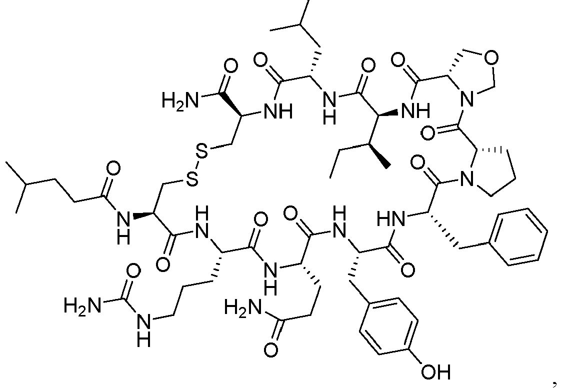
compound iHex-[Cys-Cit-Gln-Tyr-Ala-Pro-ala-Ile-Leu-Cys]-NHz (UPAR-263) of the following formula 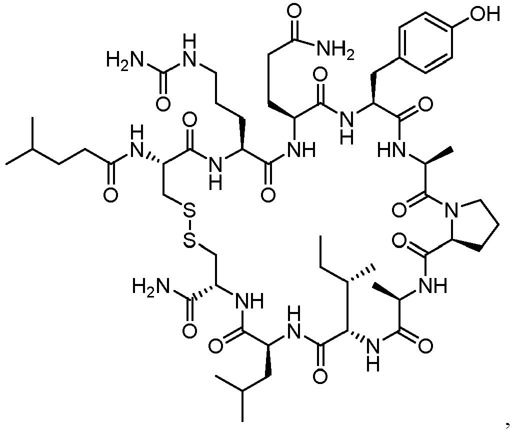
compound Pent-[Cys-Arg-Gln-Tyr-Phe-Pro-Hyp-Egz-Nva-Cys]-NH₂ (UPAR-264) of the following formula 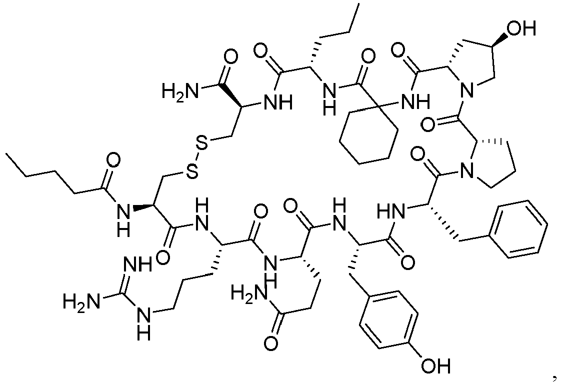
compound Pent-[Cys-Cit-Lys(DOTA)-Tyr-Phe-Pro-Nmo-Ile-Nva-Cys]-NH₂ (UPAR-265) of the following formula 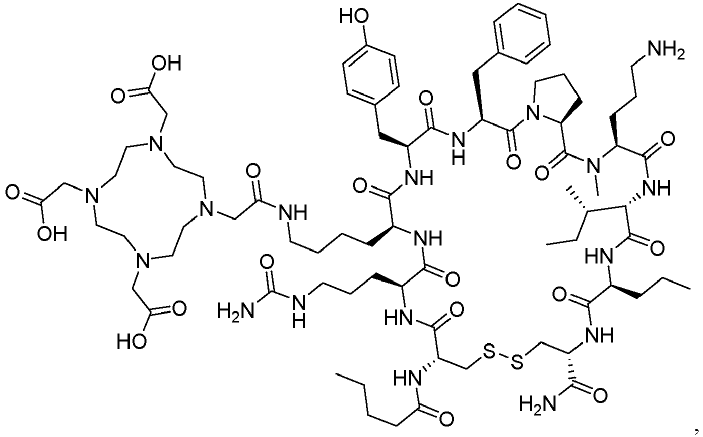
compound Pent-[Cys-Arg-Gln-Tyr-Phe-Cha-Hyp-Ile-Nva-Cys]-NH₂ (UPAR-266) of the following formula 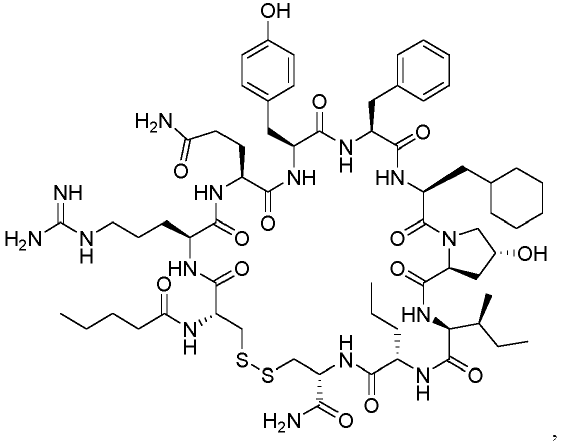
compound Prpoc-[Cys-Cit-Lys(DOTA)-Tyr-Phe-Pro-Hyp-Ile-Nva-Cys]-NH₂ (UPAR-267) of the following formula 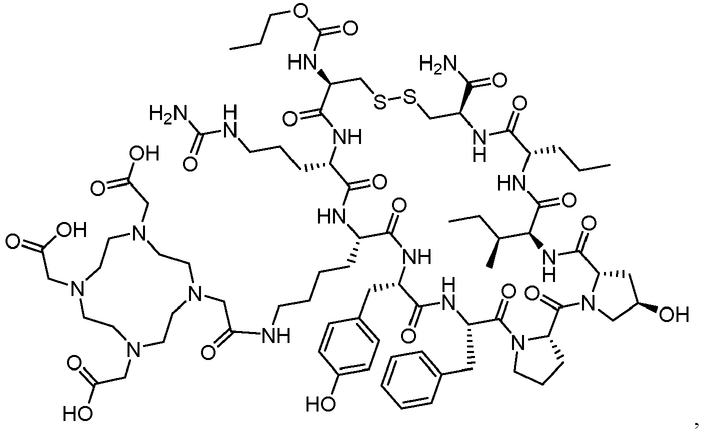
compound AGLU-Glutar-Nva-[Cys-Cit-Gln-Tyr-Phe-Pro-Hyp-Ile-Nva-Cys]-NH₂ (UPAR-268) of the following formula 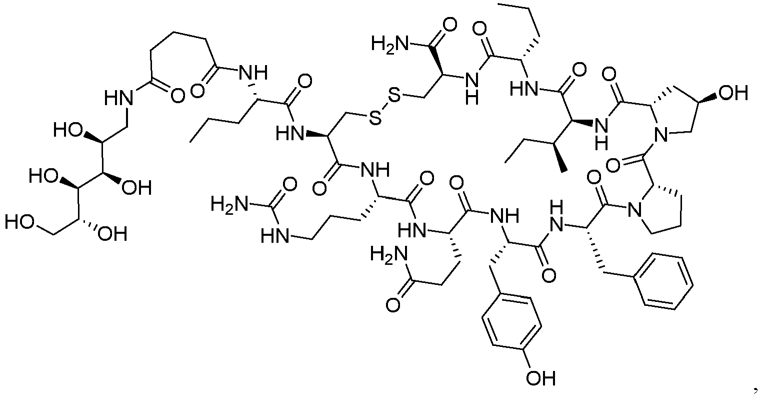
compound iHex-[Cys-Cit-Aib-Tyr-Phe-Pro-ala-Ile-Leu-Cys]-NHz (UPAR-269) of the following formula 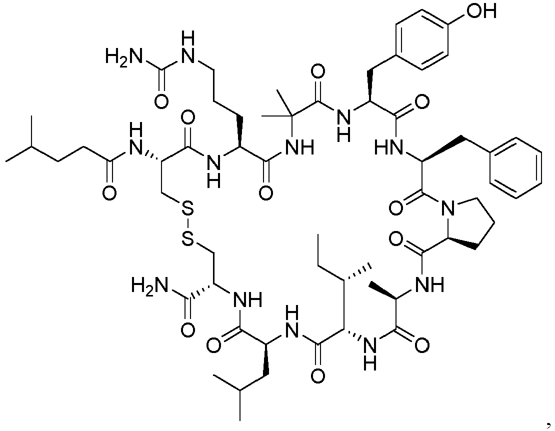
compound Ac-Asp-Leu-[Cys-Arg-Nle-Tyr-Phe-Pro-Ala-Ile-Leu-Cys]-NH₂ (UPAR-270) of the following formula 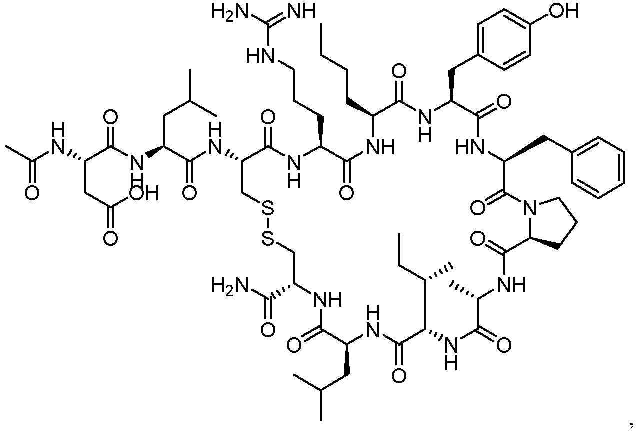
compound Pent-[Cys-Arg-Gln-Tyr-Phe-PPAc-Ile-Nva-Cys]-NH₂ (UPAR-271) of the following formula 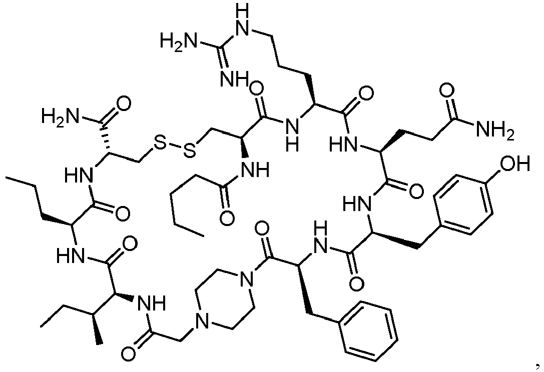
compound HPA-[Cys-Cit-Gln-Tyr-Phe-Pro-ala-Ile-Leu-Cys]-NHz (UPAR-272) of the following formula 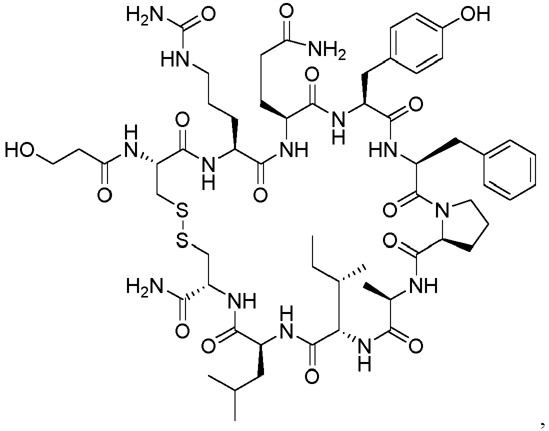
compound Pent-[Cys-Arg-Gln-Tyr-Bta-Pro-Hyp-Ile-Nva-Cys]-NHz (UPAR-273) of the following formula 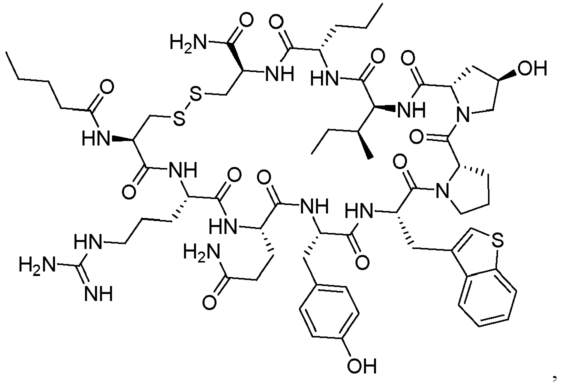
compound Ac-Asp-Leu-[Cys-Arg-nle-Tyr-Phe-Pro-Ala-Ile-Leu-Cys]-NHz (UPAR-274) of the following formula 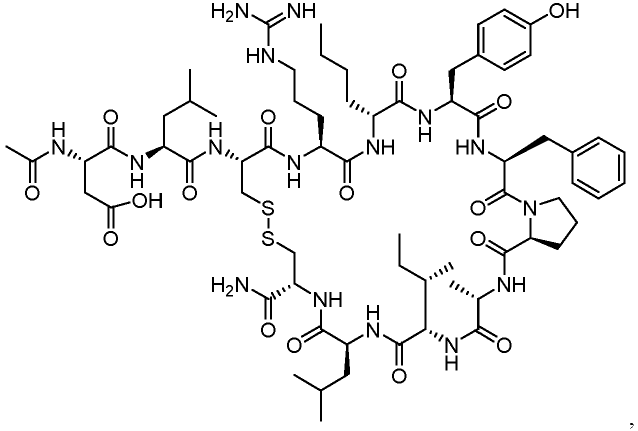
compound Butoc-[Cys-Cit-Lys(DOTA)-Tyr-Phe-Pro-Hyp-Ile-Nva-Cys]-NH₂ (UPAR-275) of the following formula 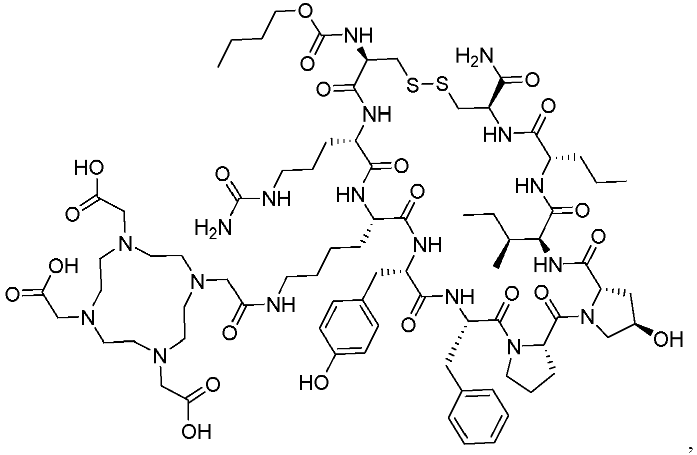
compound Butoc-[Cys-Thr-Orn(DOTA)-Pcnf-Phe-4Cfp-Hyp-Ile-Nva-Cys]-NH₂ (UPAR-276) of the following formula 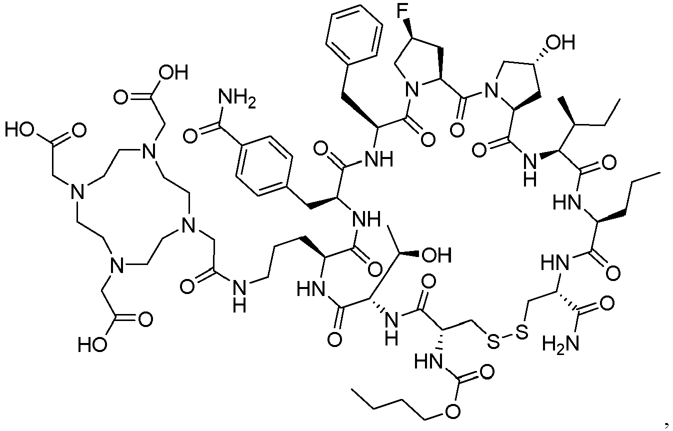
compound Pent-[Cys-Cit-Lys(DOTA)-Tyr-Phe-Pro-Nms-Ile-Nva-Cys]-NH₂ (UPAR-277) of the following formula 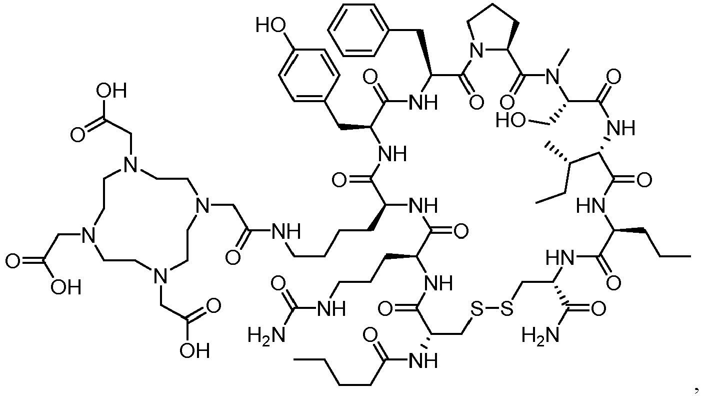
compound Pent-[Cys-Har-Lys(DOTA)-Tyr-Phe-Pro-Hyp-Ile-Nva-Cys]-NH₂ (UPAR-278) of the following formula 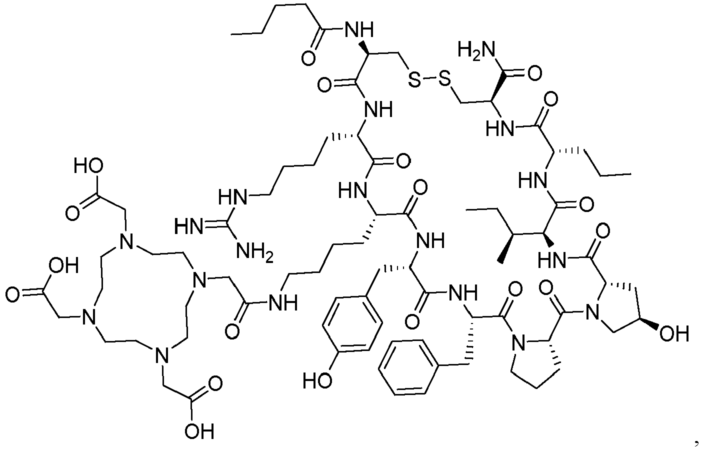
compound Pent-[Cys-Cit-Lys(LaDOTA)-Tyr-Phe-Pro-Aib-Ile-Nva-Cys]-NH₂ (UPAR-279) of the following formula 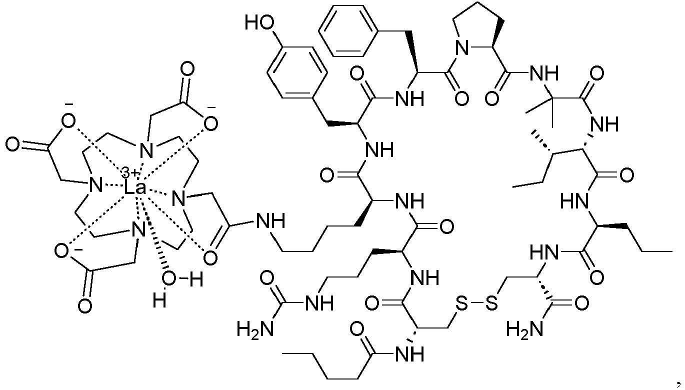
compound Butoc-[Cys-Thr-Orn(DOTA)-Dopa-Phe-4Cfp-Hyp-Ile-Nva-Cys]-NH₂ (UPAR-280) of the following formula 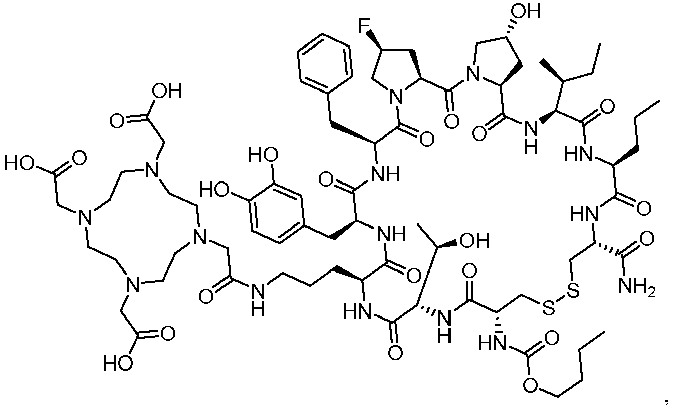
compound Pent-[Cys-Arg-Gln-Tyr-Phe-Pro-Leu-Ile-Nva-Cys]-NH₂ (UPAR-281) of the following formula 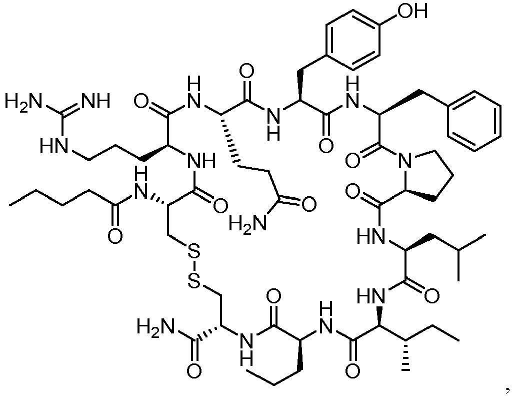
compound Pent-[Cys-Asn-Lys(DOTA)-Tyr-Phe-Pro-Hyp-Ile-Nva-Cys]-NH₂ (UPAR-283) of the following formula 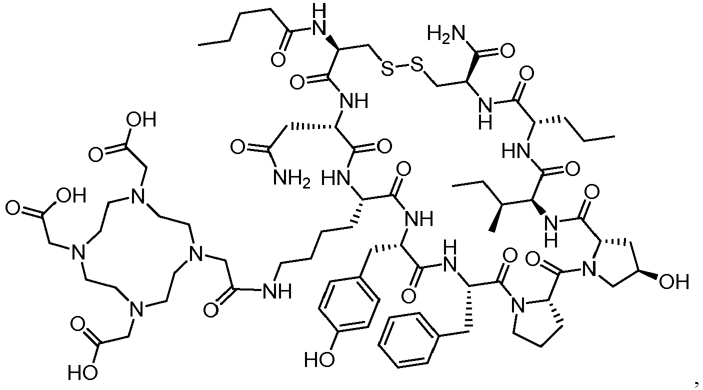
compound Butoc-[Cys-Thr-Lys(DOTA)-Dopa-Phe-Hyp-Hyp-Ile-Nva-Cys]-NH₂ (UPAR-284) of the following formula 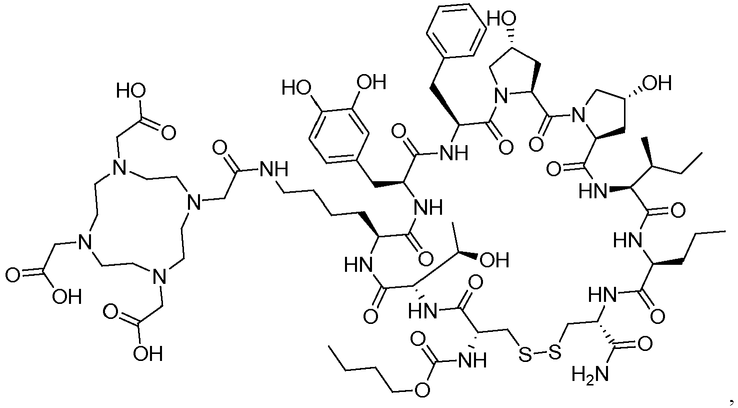
compound iHex-[Cys-Cit-Gln-Nmy-Phe-Pro-ala-Ile-Leu-Cys]-NHz (UPAR-285) of the following formula 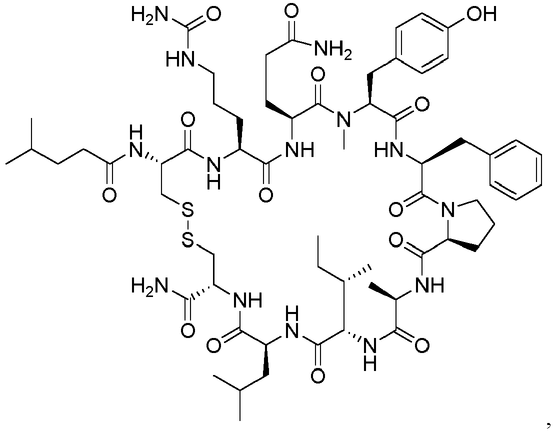
compound nBuCAyl-[Cys-Cit-Gln-Tyr-Phe-Pro-ala-Ile-Leu-Cys]-NH₂ (UPAR-286) of the following formula 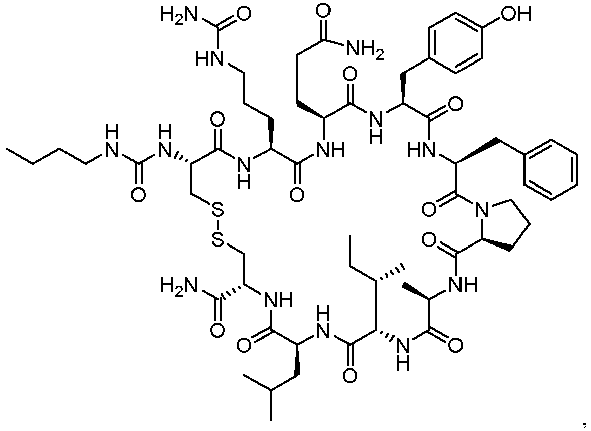
compound iHex-[Cys-Nmr-Gln-Tyr-Phe-Pro-ala-Ile-Leu-Cys]-NHz (UPAR-287) of the following formula 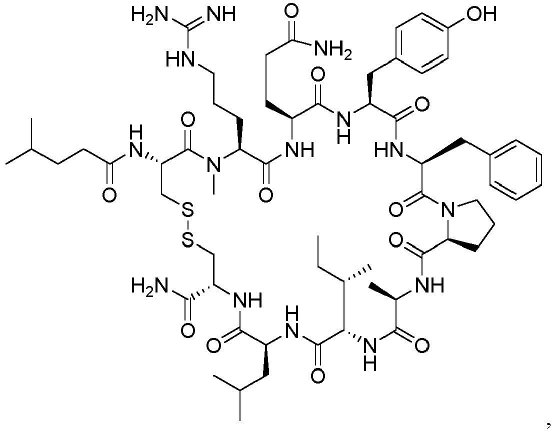
compound Pent-[Cys-Thr-Apc(DOTA)-Tyr-Phe-Pro-Hyp-Ile-Nva-Cys]-NH₂ (UPAR-288) of the following formula 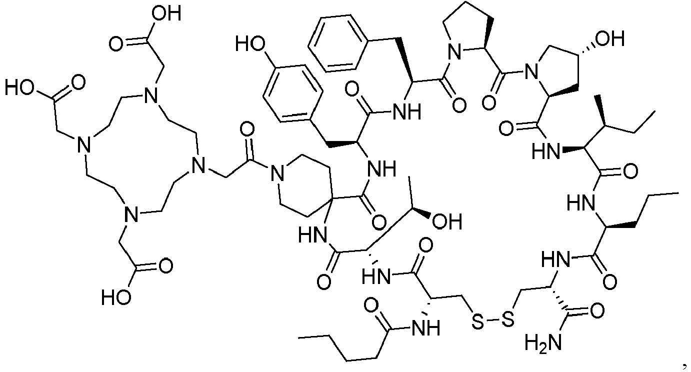
compound Butoc-[Cys-Thr-Lys(DOTA)-Tyr-Phe-Pro-4Tfp-Ile-Nva-Cys]-NH₂ (UPAR-289) of the following formula 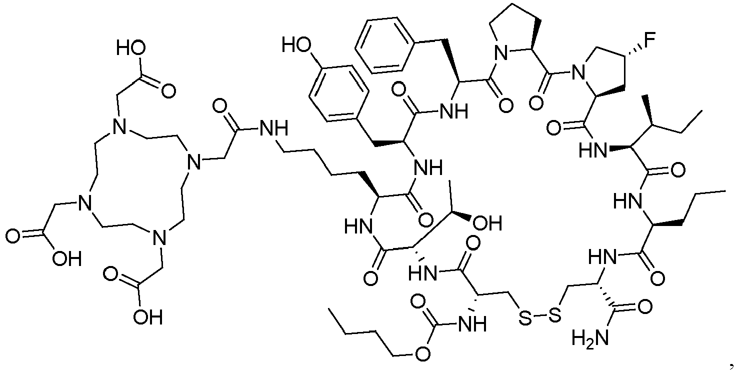
compound Ac-Asp-Leu-[Cys-Arg-Gln-Tyr-Phe-Pro-Ala-Ile-Leu-Cys]-NH₂ (UPAR-290) of the following formula 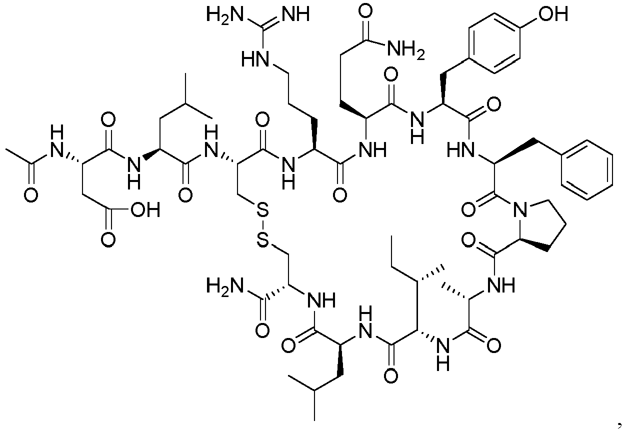
compound DOTA-Ahx-Leu-[Cys-Arg-Gln-Tyr-Phe-Pro-ala-Ile-Leu-Cys]-NH₂ (UPAR-291) of the following formula 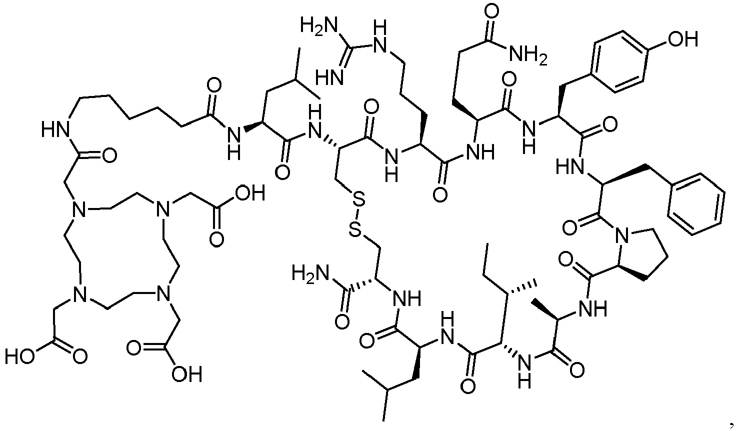
compound Pent-[Cys-Cit-Gln-Tyr-Phe-Pro-Hyp-Ile-Nva-Cysol]-OH (UPAR-292) of the following formula 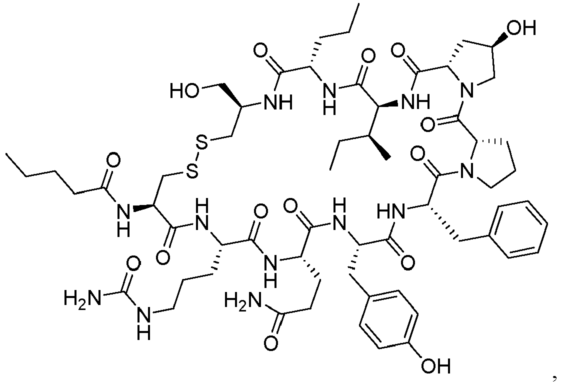
compound Succinyl-Nva-[Cys-Cit-Lys(DOTA)-Tyr-Phe-Pro-Hyp-Ile-Nva-Cys]-NH₂ (UPAR-293) of the following formula 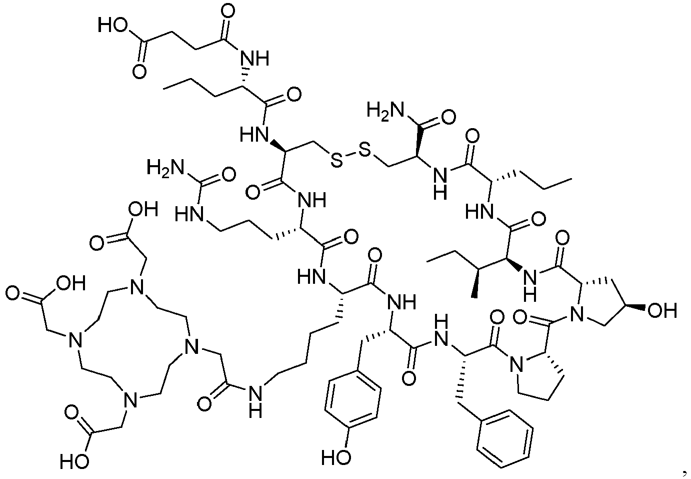
compound Butoc-[Cys-Thr-Lys(InDOTA)-Tyr-Phe-4Cfp-Hyp-Ile-Nva-Cys]-NH₂ (UPAR-294) of the following formula 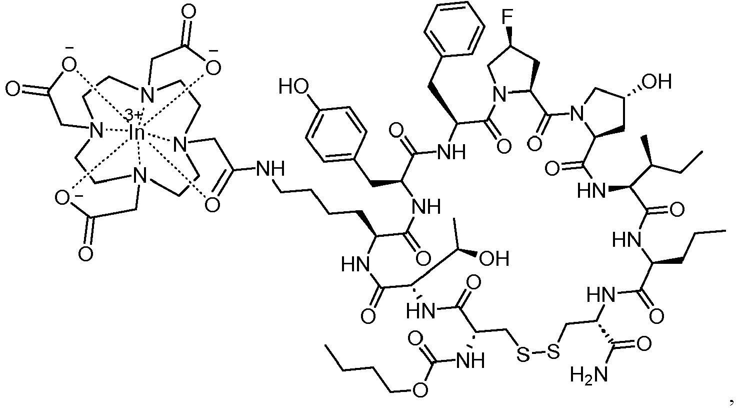
compound iHex-[Cys-Cit-Gln-Tyr-Phe-Pro-Tap-Ile-Leu-Cys]-NHz (UPAR-295) of the following formula 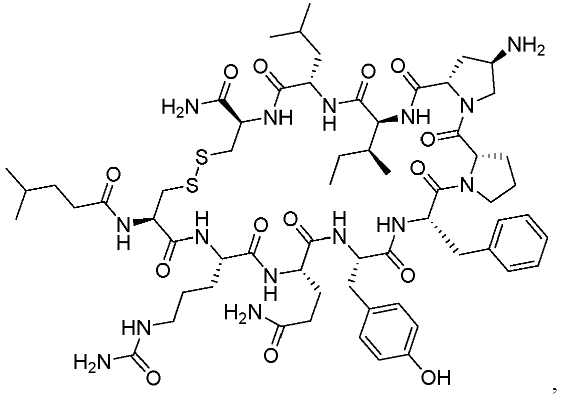
compound Pent-[Cys-Cit-Lys(LaDOTA)-Ppa-Phe-Pro-Hyp-Ile-Nva-Cys]-NH₂ (UPAR-296) of the following formula 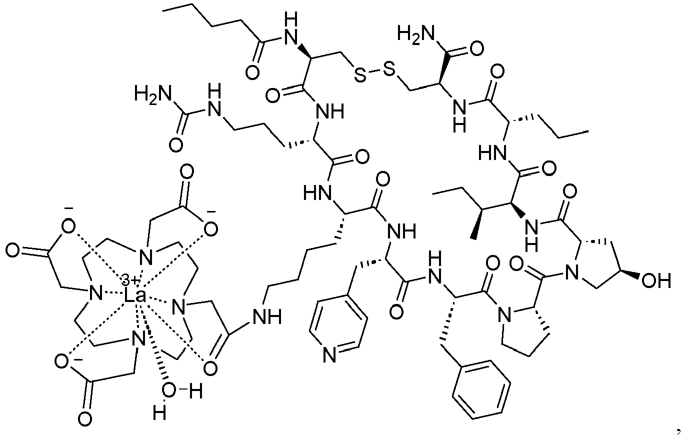
compound Succinyl-Nva-[Cys-Thr-Lys(DOTA)-Tyr-Phe-Pro-Hyp-Ile-Nva-Cys]-NH₂ (UPAR-297) of the following formula 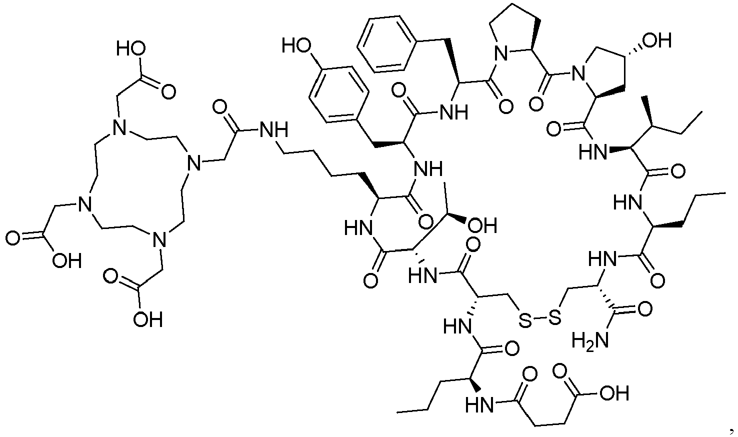
compound Pent-[Cys-Arg-Gln-Tyr-Phe-Lys-Hyp-Ile-Nva-Cys]-NH₂ (UPAR-298) of the following formula 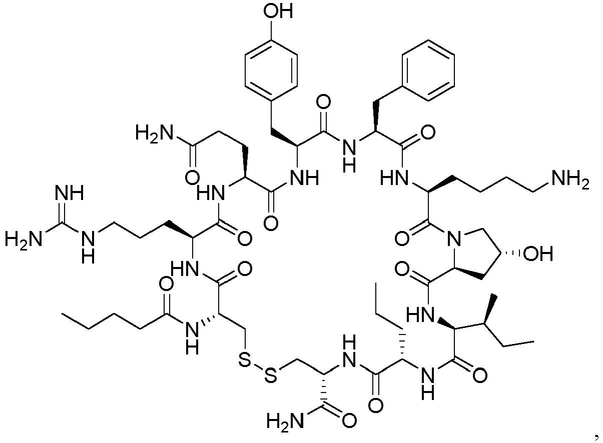
compound Pent-[Cys-Cit-Lys(DOTA)-Tyr-Phe-Pro-Aib-Ile-Nva-Cys]-NH₂ (UPAR-299) of the following formula 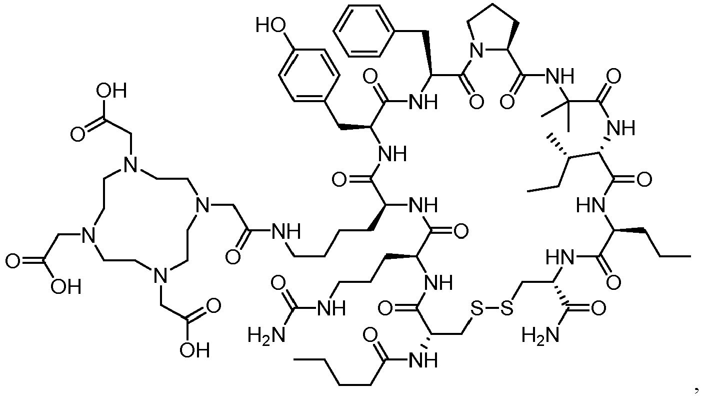
compound Pent-[Hcy-Arg-Gln-Tyr-Phe-Pro-Hyp-Ile-Nva-Cys]-NHz (UPAR-300) of the following formula 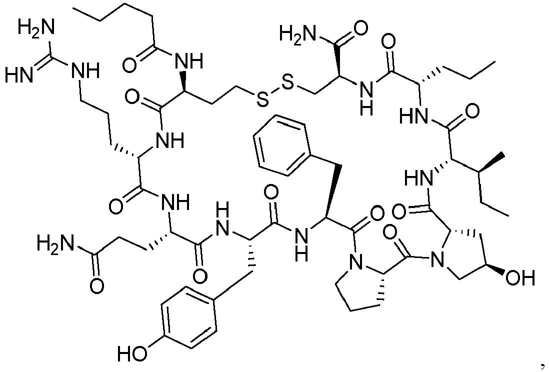
compound Pent-[Cys-Thr-Lys(DOTA)-Tyr-Phe-Pro-Hyp-Ile-Nva-Cys]-NH₂ (UPAR-301) of the following formula 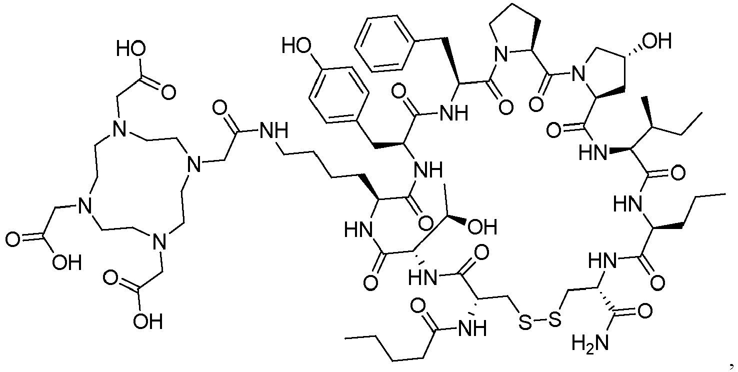
compound Pent-[Cys-Arg-Gln-Tyr-Phe-Nml-Hyp-Ile-Nva-Cys]-NH₂ (UPAR-302) of the following formula 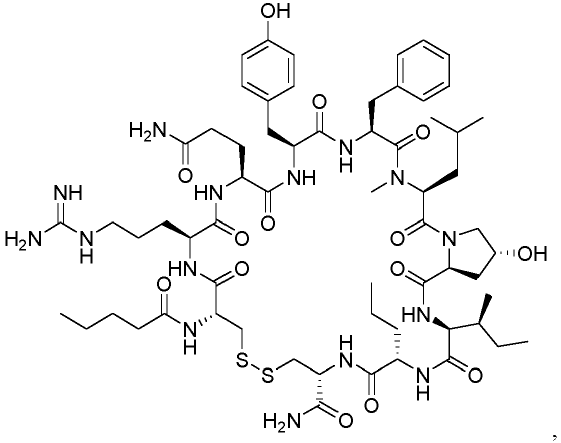
compound Pent-[Cys-Cit-Lys(LaDOTA)-Tyr-Phe-4Cfp-Hyp-Ile-Nva-Cys]-NH₂ (UPAR-303) of the following formula 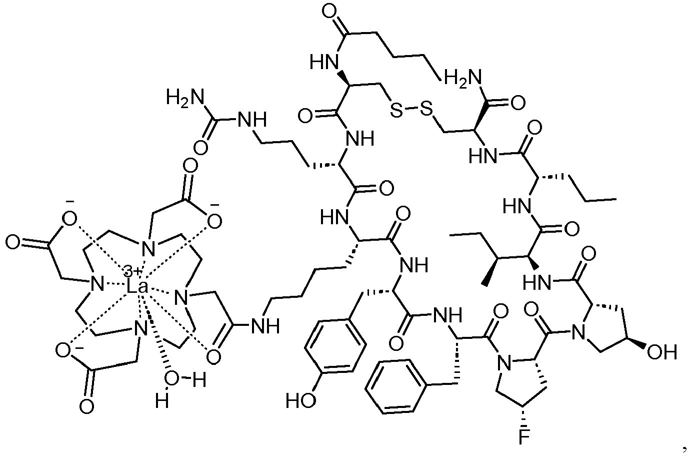
compound Butoc-[Cys-Cit-Lys(DOTA)-Tyr-Pcf-4Cfp-Hyp-Ile-Nva-Cys]-NH₂ (UPAR-304) of the following formula 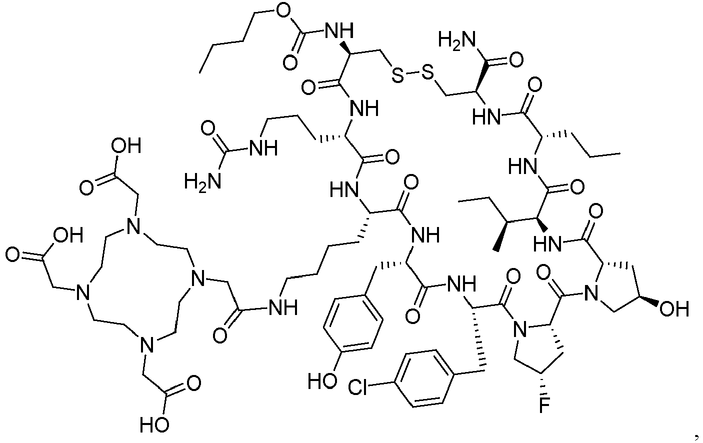
compound iHex-[Cys-Cit-Gln-Tyr-Phe-Pro-pro-Ile-Leu-Cys]-NHz (UPAR-305) of the following formula 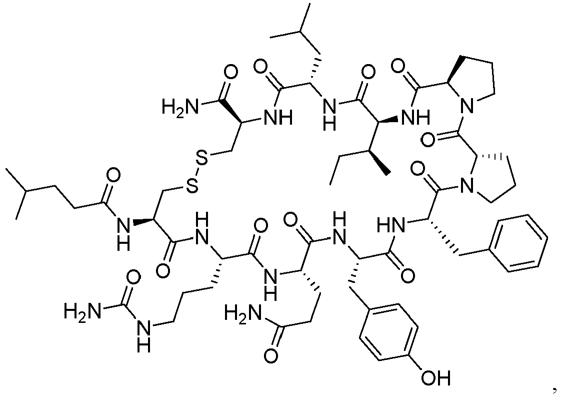
compound Ac-Asp-Leu-[Cys-Cit-Lys(DOTA)-Tyr-Phe-Pro-arg-Ile-Leu-Cys]-NH₂ (UPAR-306) of the following formula 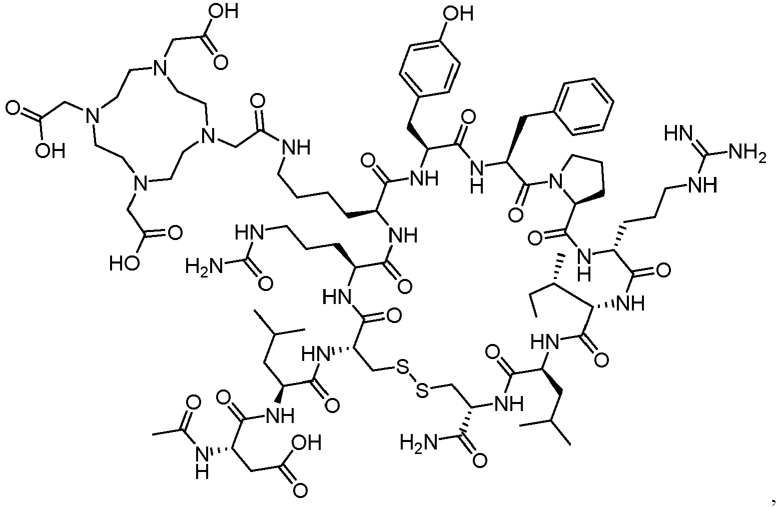
compound iHex-[Cys-Cit-Gln-Tyr-Phe-Pro-4Cfp-Ile-Leu-Cys]-NH₂ (UPAR-307) of the following formula 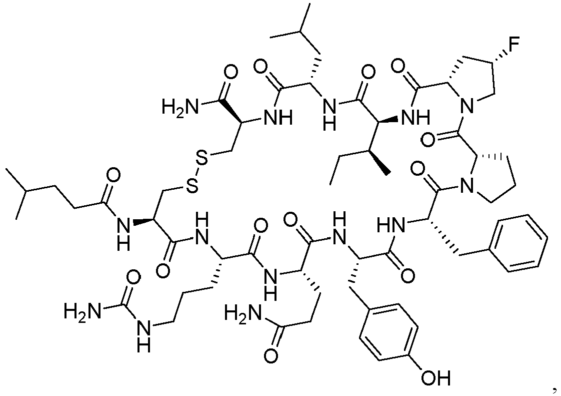
compound Pent-[Cys-Arg-Gln-Tyr-Trp-Pro-Hyp-Ile-Nva-Cys]-NHz (UPAR-308) of the following formula 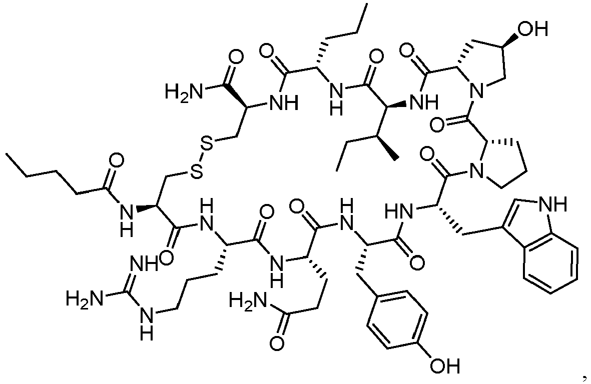
compound Pent-[Cys-Cit-Gln-Tyr-Phe-Pro-Hyp-Ile-Nva-Cys]-NHz (UPAR-309) of the following formula 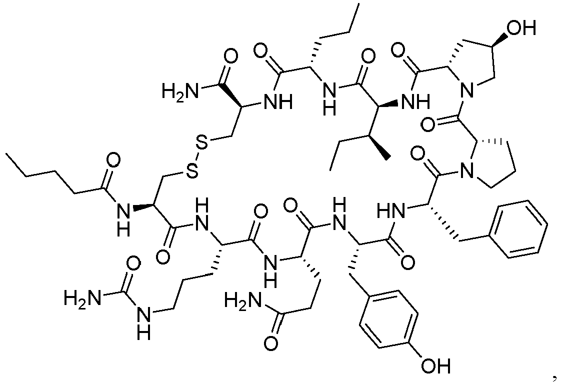
compound iHex-[Cys-Cit-Gln-Tyr-1Ni-Pro-ala-Ile-Leu-Cys]-NH₂ (UPAR-310) of the following formula 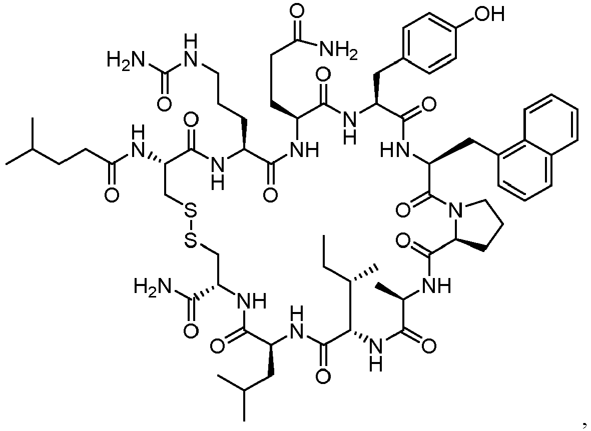
compound Pent-[Cys-Orn(mPEG12)-Lys(InDOTA)-Tyr-Phe-Pro-Hyp-Ile-Nva-Cys]-NH₂ (UPAR-311) of the following formula 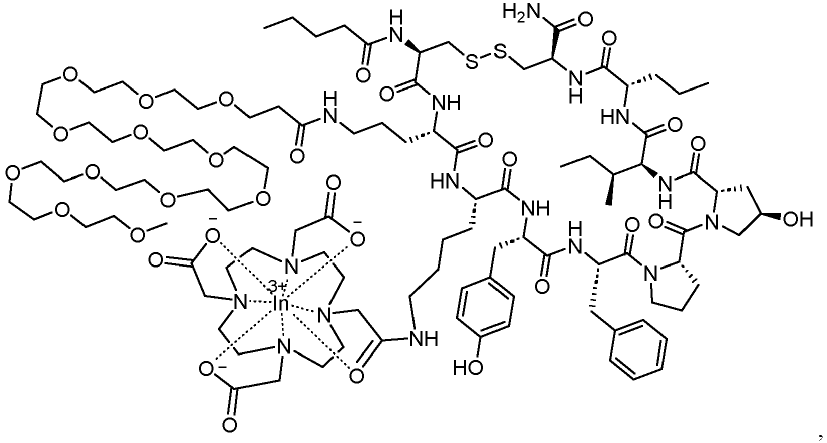
compound H-Met-Asp-Leu-[Cys-Arg-Met-Tyr-Phe-Pro-Ala-Ile-Leu-Cys]-NH₂ (UPAR-312) of the following formula 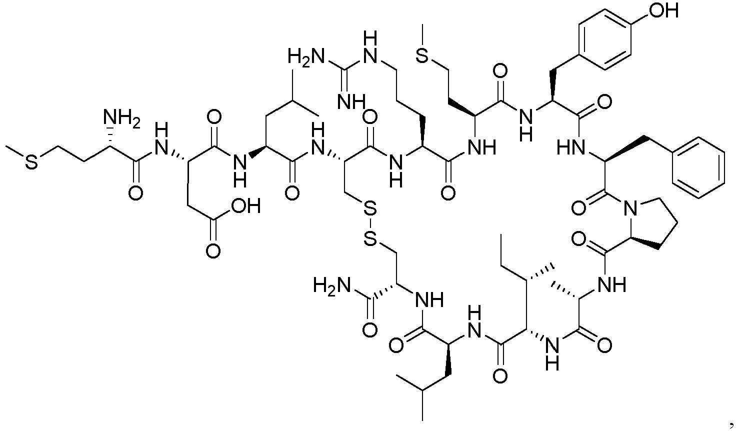
compound Pent-[Cys-Arg-Arg-Tyr-Phe-Pro-Tap-Ile-Nva-Cys]-NH₂ (UPAR-313) of the following formula 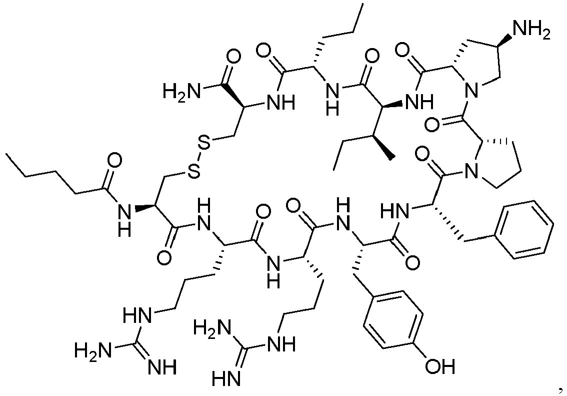
compound iHex-[Cys-Cit-Gln-Aph-Phe-Pro-ala-Ile-Leu-Cys]-NH₂ (UPAR-314) of the following formula 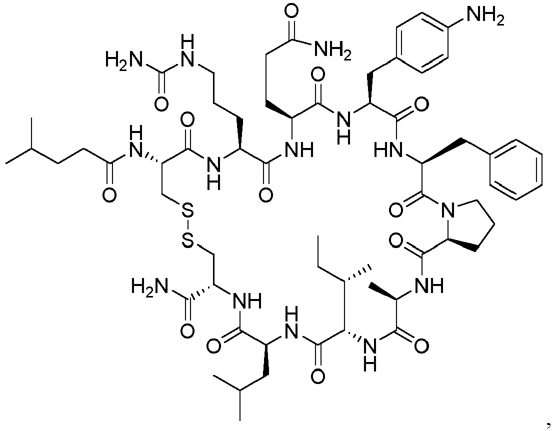
compound Pent-[Cys-Cit-Lys(DOTA)-Tyr-Egm-Pro-Hyp-Ile-Nva-Cys]-NHz (UPAR-315) of the following formula 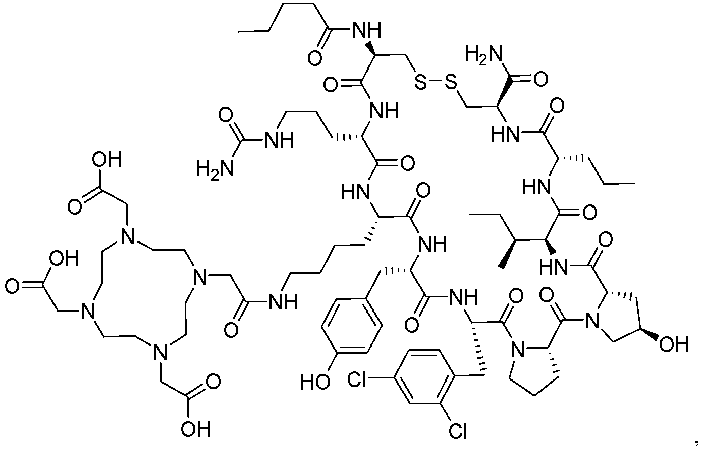
compound Pent-[Cys-Cit-Lys(DOTA)-Tyr-Phe-Pro-Nma-Ile-Nva-Cys]-NH₂ (UPAR-316) of the following formula 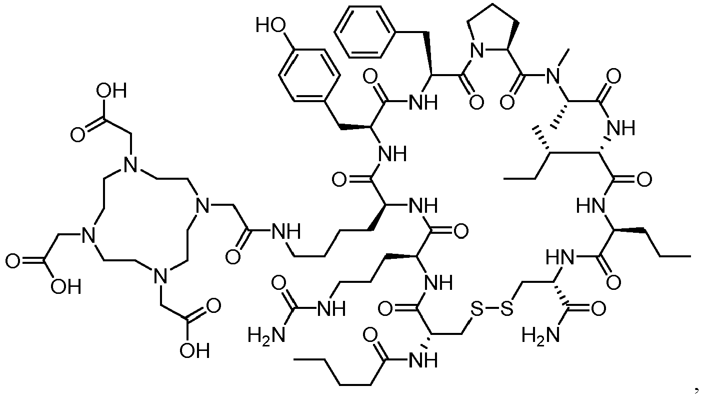
compound Pent-[Cys-Cit-Lys(Bio-Ttds)-Tyr-Phe-Pro-Nmk-Ile-Nva-Cys]-NH₂ (UPAR-317) of the following formula 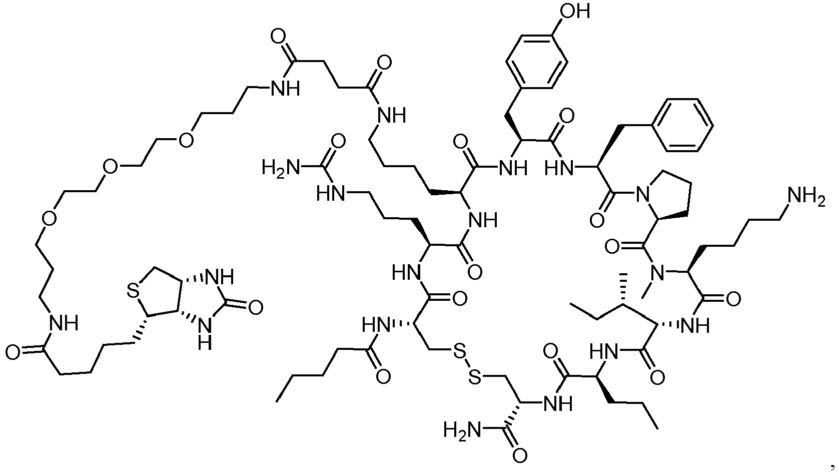
compound Hex-Thr-Leu-[Cys-Asp-Leu-Tyr-Phe-Pro-Ala-Ile-Leu-Cys]-NH₂ (UPAR-318) of the following formula 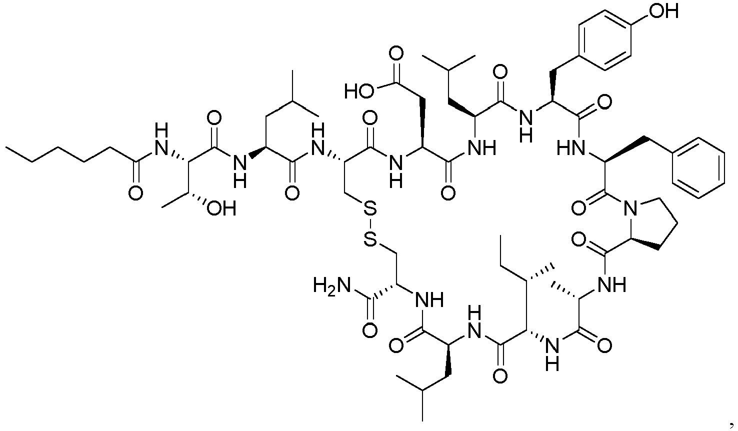
compound iHex-[Cys-Cit-Gln-Tyr-Phe-Ala-ala-Ile-Leu-Cys]-NH₂ (UPAR-319) of the following formula 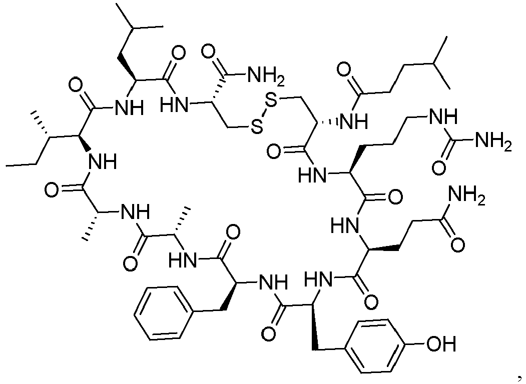
compound Pent-[Cys-Cit-Lys(DOTA)-Aph-Phe-Pro-Hyp-Ile-Nva-Cys]-NH₂ (UPAR-320) of the following formula 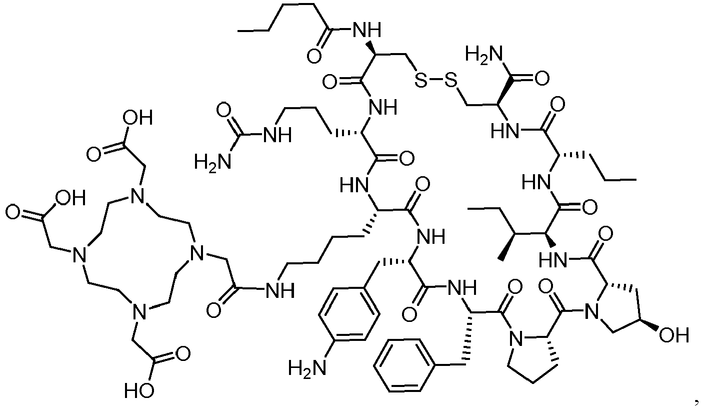
compound iHex-[Cys-Cit-Gln-Tyr-Phe-Oxa-ala-Ile-Leu-Cys]-NHz (UPAR-321) of the following formula 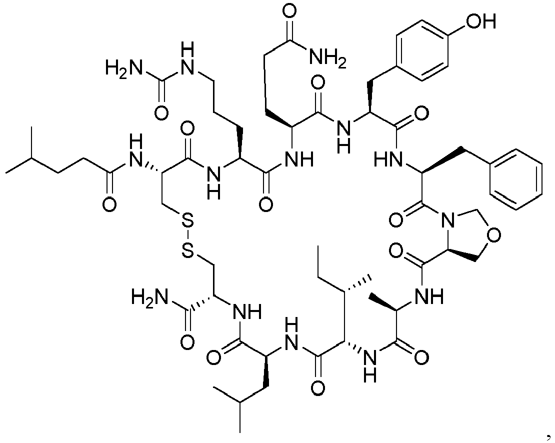
compound Pent-[Cys-Arg-Gln-Tyr-Phe-Pro-nva-Ile-Nva-Cys]-NHz (UPAR-322) of the following formula 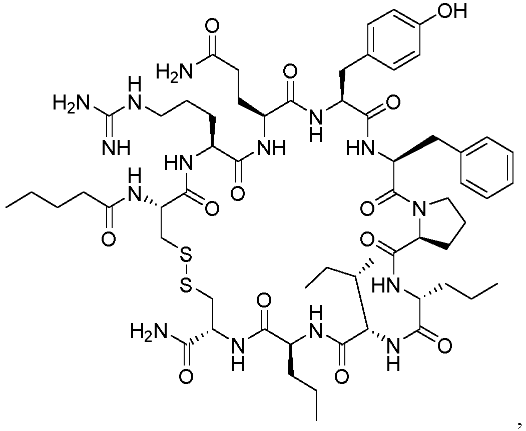
compound Ac-Asp-Leu-[Cys-Arg-Nle-Tyr-Phe-Pro-Ala-Ile-Leu-cys]-NH₂ (UPAR-323) of the following formula 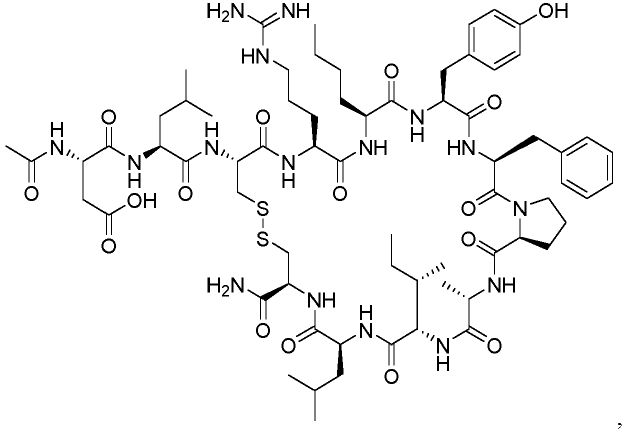
compound Pent-[Cys-Cit-Lys(DOTA)-His-Phe-Pro-Hyp-Ile-Nva-Cys]-NH₂ (UPAR-324) of the following formula 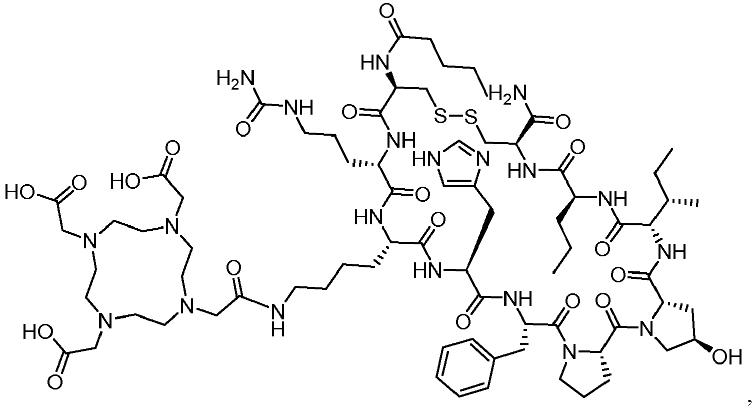
compound Pent-[Cys-Cit-Lys(DOTA)-Tyr-Phe-Pro-4Ap-Ile-Nva-Cys]-NH₂ (UPAR-325) of the following formula 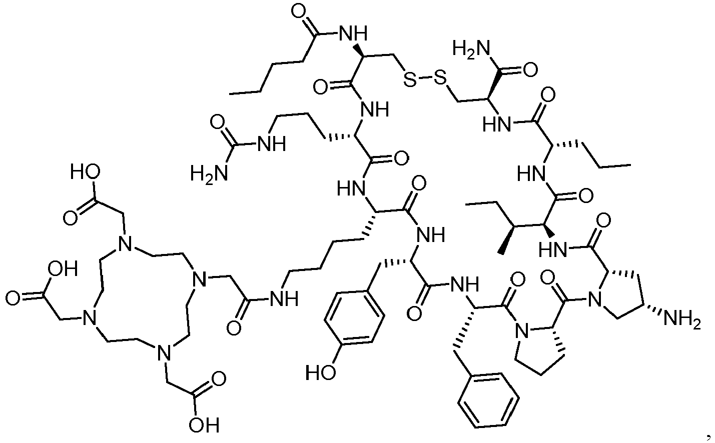
compound iHex-[Cys-Cit-Gln-Tyr-Phe-Pro-ala-Cha-Leu-Cys]-NHz (UPAR-326) of the following formula 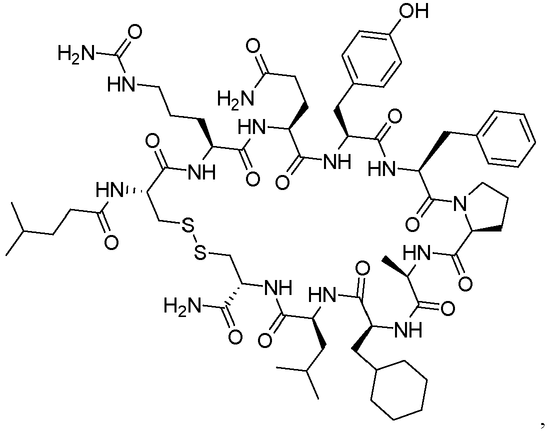
compound Pent-[Cys-Cit-Lys(DOTA)-Tyr-Phe-Pro-Nme-Ile-Nva-Cys]-NH₂ (UPAR-327) of the following formula 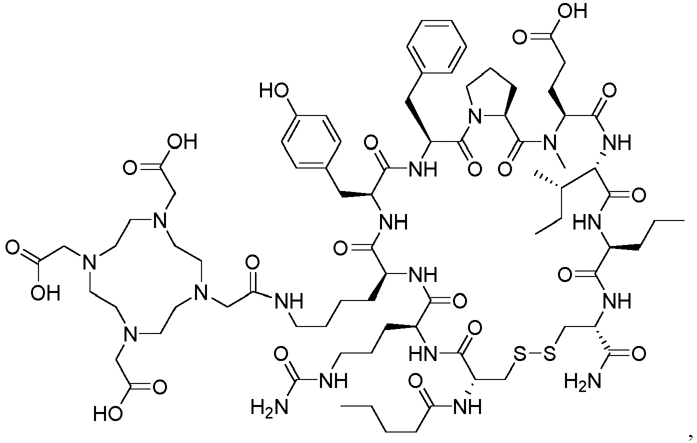
compound PentylSO2-[Cys-Arg-Gln-Tyr-Phe-Pro-Hyp-Ile-Nva-Cys]-NH₂ (UPAR-328) of the following formula 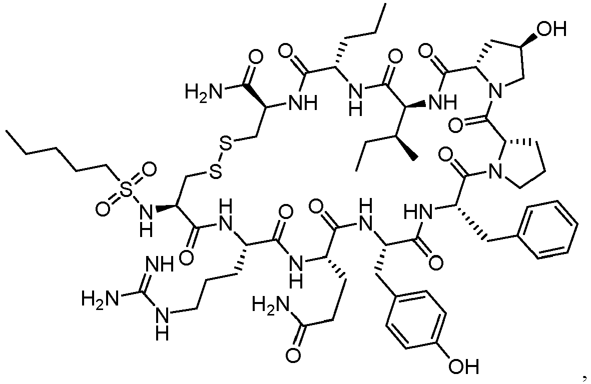
compound Pent-[Cys-Arg-Gln-Tyr-Phe-Pro-ser-Ile-Nva-Cys]-NHz (UPAR-329) of the following formula 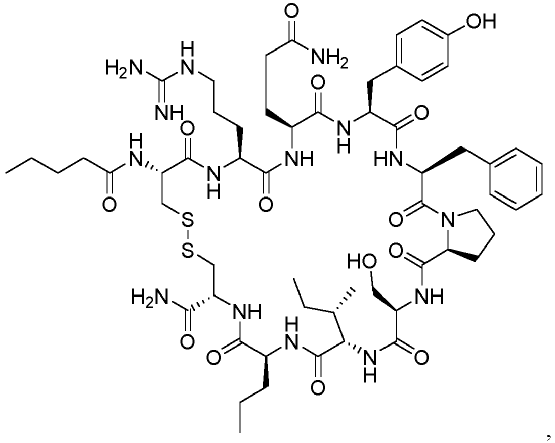
compound Pent-[Cys-Arg-Gln-Tyr-Phe-Pro-dap-Ile-Nva-Cys]-NHz (UPAR-330) of the following formula 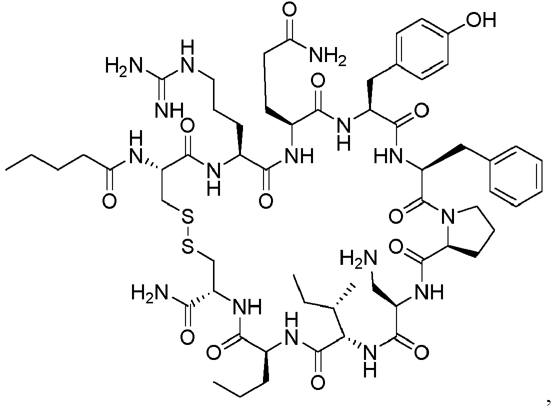
compound Pent-[Cys-Cit-Lys(InDOTA)-Tyr-Mpa-Pro-Hyp-Ile-Nva-Cys]-NH₂ (UPAR-331) of the following formula 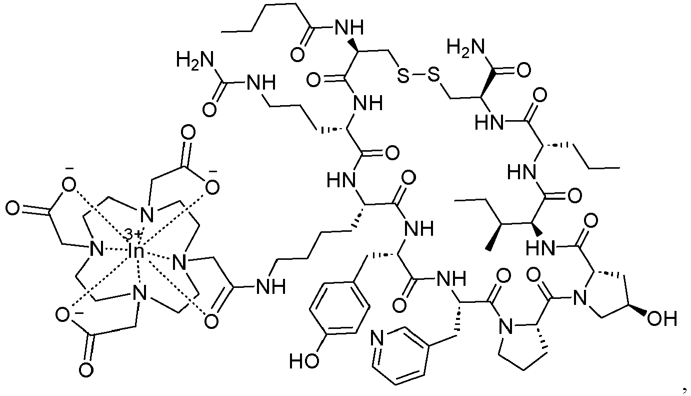
compound iHex-[Cys-Cit-Gln-Tyr-Pcf-Pro-ala-Ile-Leu-Cys]-NH₂ (UPAR-332) of the following formula 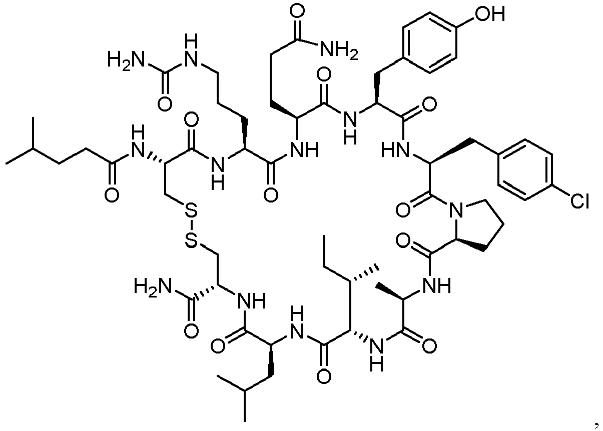
compound Dmba-[Cys-Cit-Gln-Tyr-Phe-Pro-ala-Ile-Leu-Cys]-NHz (UPAR-333) of the following formula 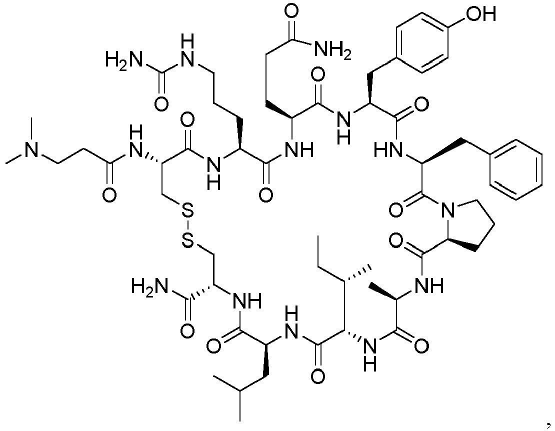
compound Ac-Gly-[Cys-Cit-Gln-Tyr-Phe-Pro-ala-Ile-Leu-Cys]-NH₂ (UPAR-334) of the following formula 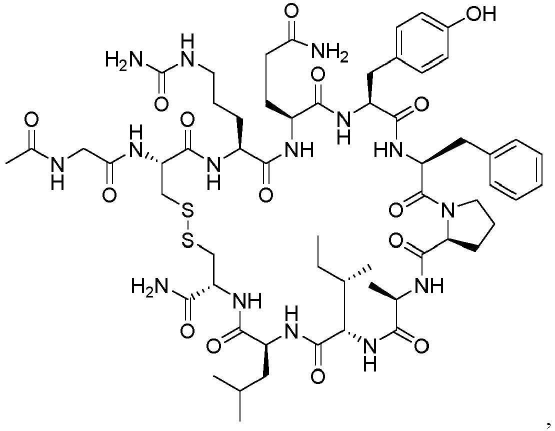
compound Pent-[Cys-Asn-Lys(LuDOTA)-Tyr-Phe-Pro-Hyp-Ile-Nva-Cys]-NH₂ (UPAR-335) of the following formula 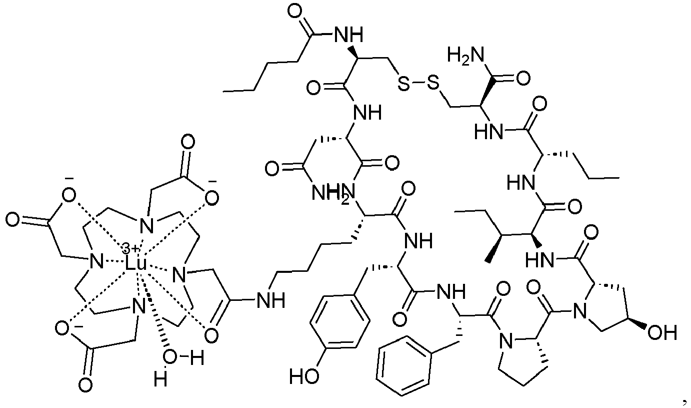
compound Pent-[Cys-Cit-Lys(DOTA)-Ppa-Phe-Pro-Hyp-Ile-Nva-Cys]-NH₂ (UPAR-336) of the following formula 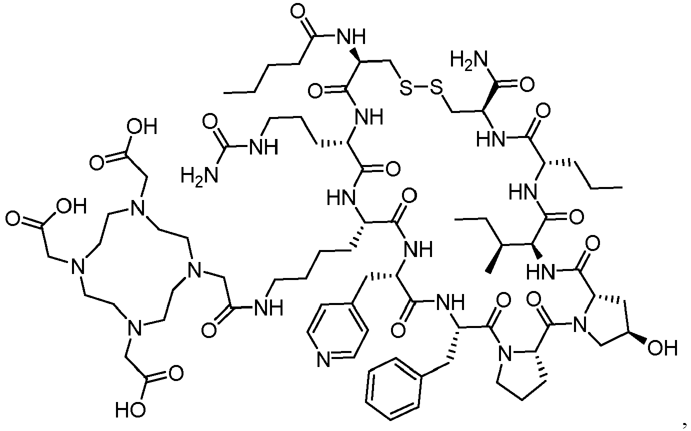
compound Pent-[Cys-Arg-Gln-Tyr-Phe-Nmb-Hyp-Ile-Nva-Cys]-NH₂ (UPAR-337) of the following formula 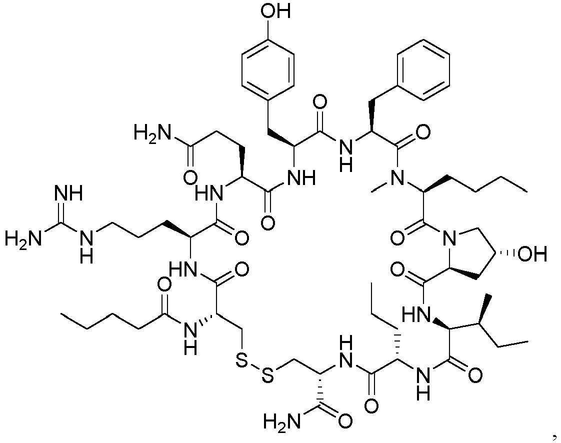
compound iHex-[Cys-Cit-Gln-Tyr-Phe-Pro-ala-Ile-Leu-Cys]-NHz (UPAR-338) of the following formula 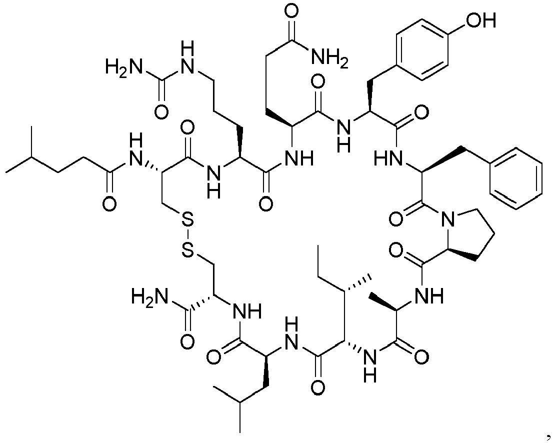
compound iHex-[Cys-Cit-Ala-Tyr-Phe-Pro-ala-Ile-Leu-Cys]-NHz (UPAR-339) of the following formula 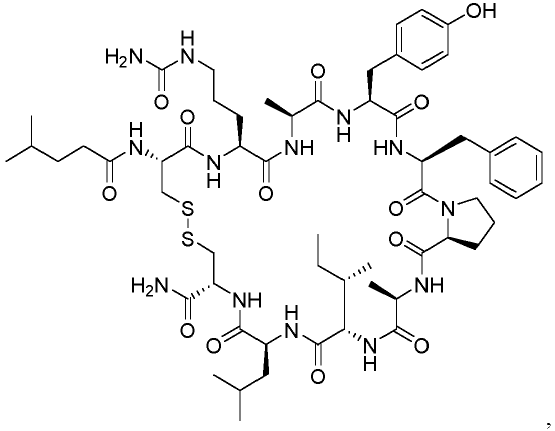
compound iHex-[Cys-Cit-Gln-Tyr-Phe-Pro-ala-Nmi-Leu-Cys]-NH₂ (UPAR-340) of the following formula 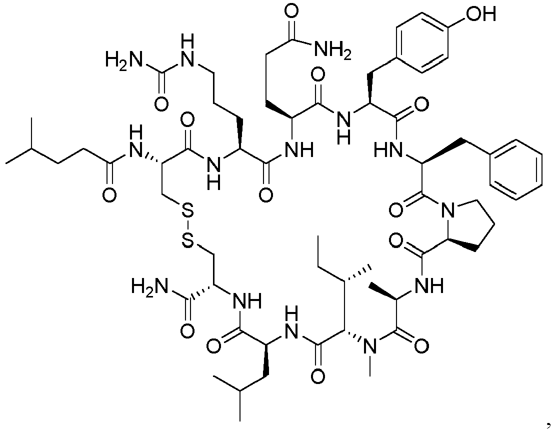
compound Ac-Asp-Leu-[Cys-Arg-Met-Tyr-Phe-Pro-Ala-Ile-Leu-Cys]-NH₂ (UPAR-341) of the following formula 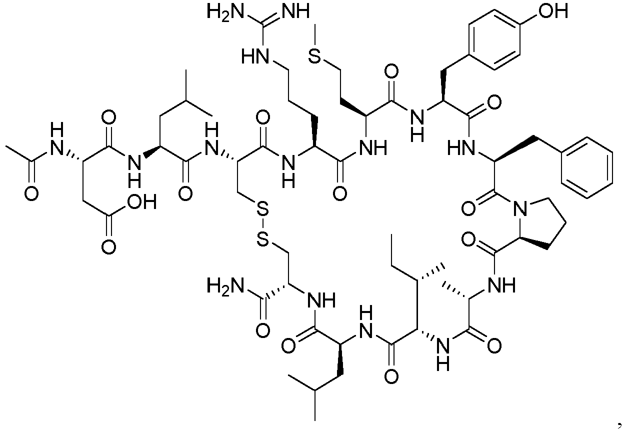
compound Pent-[Cys-Ser-Lys(DOTA)-Tyr-Phe-Pro-Hyp-Ile-Nva-Cys]-NH₂ (UPAR-342) of the following formula 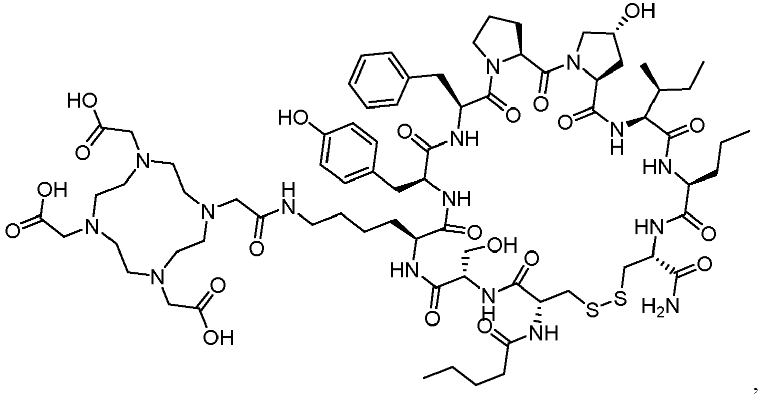
compound iHex-[Cys-Cit-Gln-Tyr-Tyr-Pro-ala-Ile-Leu-Cys]-NHz (UPAR-343) of the following formula 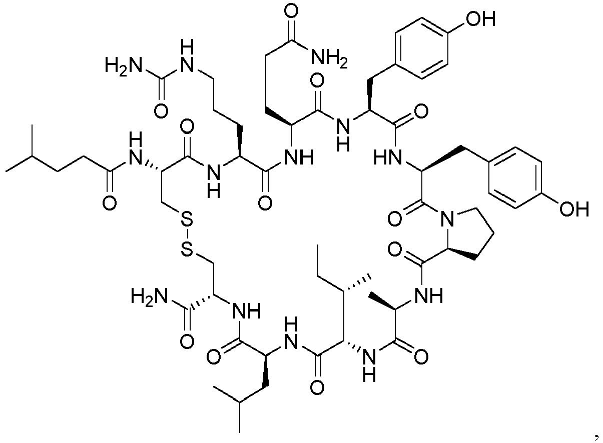
compound Pent-[Cys-Arg-Gln-Tyr-Phe-Pro-Asp-Ile-Nva-Cys]-NHz (UPAR-344) of the following formula 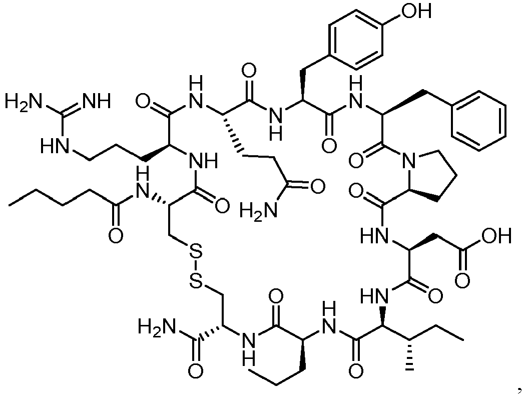
compound iHex-[Cys-Cit-Gln-Tyr-Phe-Pro-ala-Ile-Nle-Cys]-NHz (UPAR-345) of the following formula 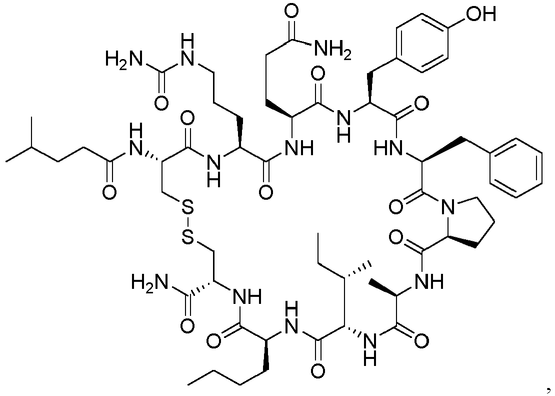
compound nBuCAyl-[Cys-Cit-Lys(DOTA)-Tyr-Phe-Pro-Hyp-Ile-Nva-Cys]-NHz (UPAR-346) of the following formula 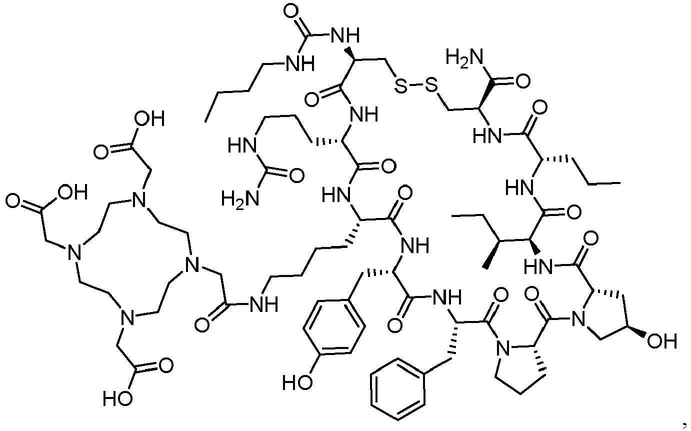
compound DOTA-Ttds-Nle-Asp-Leu-[Cys-Arg-Met-Tyr-Phe-Pro-Ala-Ile-Leu-Cys]-NH₂ (UPAR-347) of the following formula 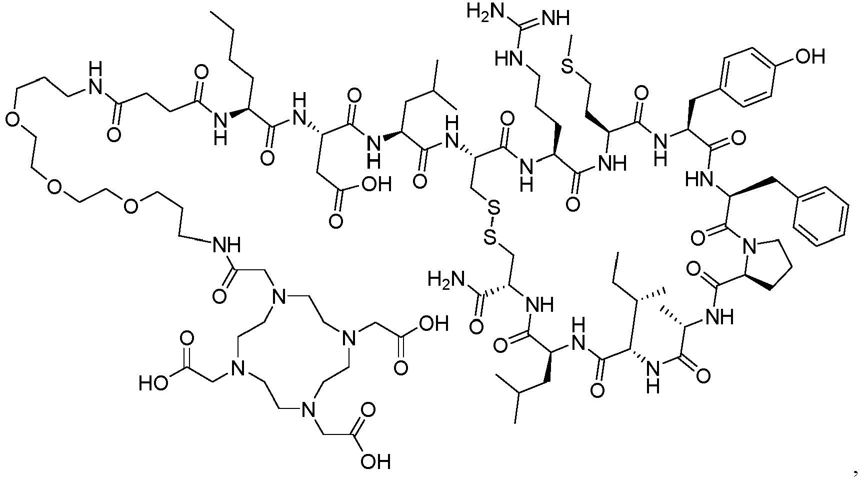
compound Pent-[Cys-Cit-Lys(LuDOTA)-Tyr-Phe-Pro-Nmg-Ile-Nva-Cys]-NH₂ (UPAR-348) of the following formula 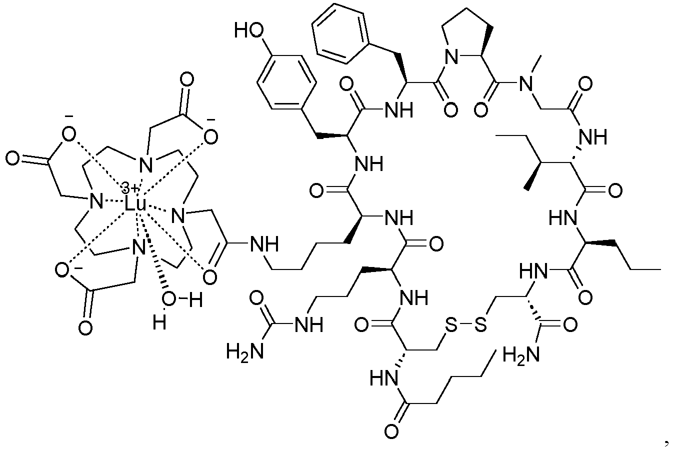
compound iHex-[Cys-Cit-Gln-Tyr-Phe-Pro-ala-Cpg-Leu-Cys]-NHz (UPAR-349) of the following formula 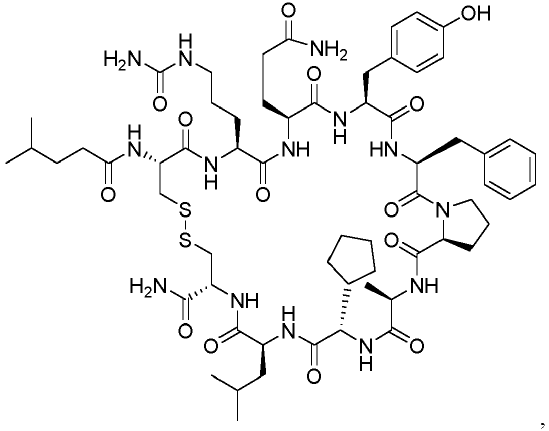
compound Pent-[Cys-Cit-Lys(LuDOTA)-Guf-Phe-Pro-Hyp-Ile-Nva-Cys]-NH₂ (UPAR-350) of the following formula 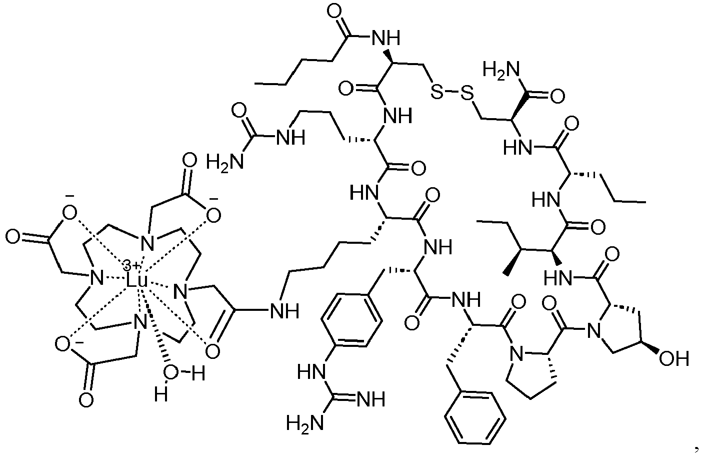
compound Hex-Asp-Leu-[Cys-Arg-Met-Tyr-Phe-Pro-Lys(DOTA)-Ile-Leu-Cys]-NH₂ (UPAR-351) of the following formula 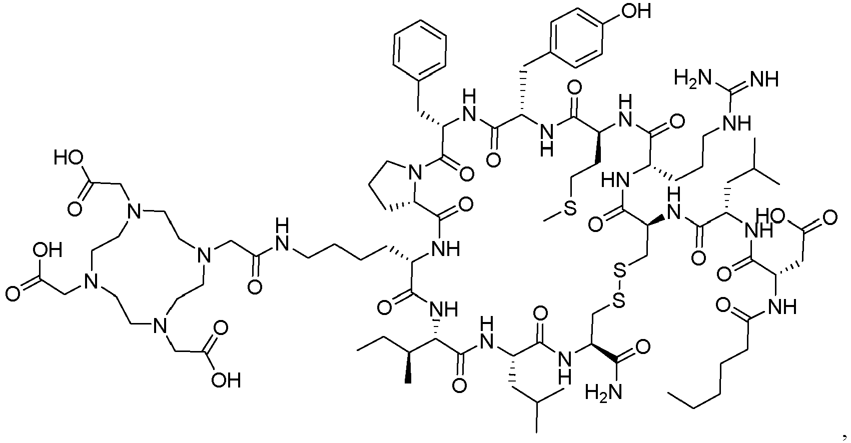
compound Pent-[Cys-Cit-Lys(LuDOTA)-Nif-Phe-Pro-Hyp-Ile-Nva-Cys]-NH₂ (UPAR-352) of the following formula 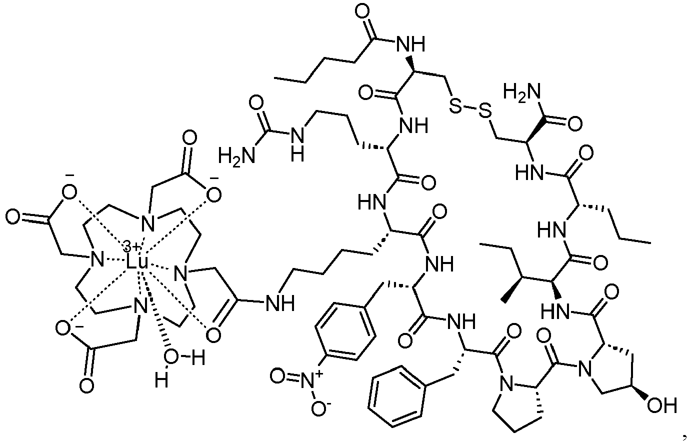
compound Pent-[Cys-Cit-Gln-Tyr-Phe-Pro-Hyp-Ile-Nva-Cys]-Bal-NH₂ (UPAR-353) of the following formula 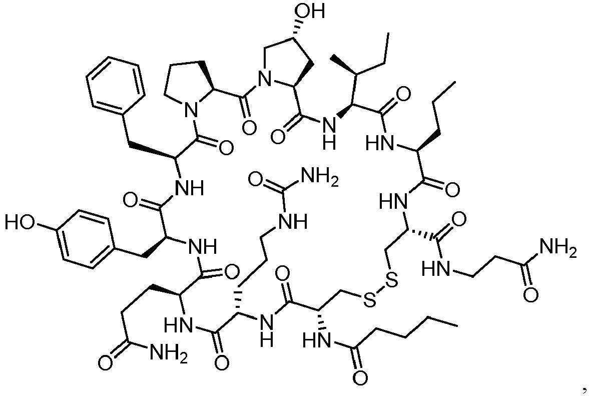
compound iHex-[Cys-Cit-Gln-Tyr-Phe-4Cfp-ala-Ile-Leu-Cys]-NH₂ (UPAR-354) of the following formula 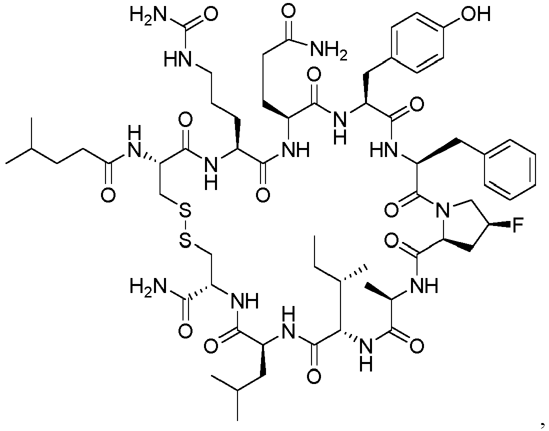
compound Pent-[Cys-Cit-Lys(InDOTA)-Tyr-Pcf-Pro-Hyp-Ile-Nva-Cys]-NH₂ (UPAR-355) of the following formula 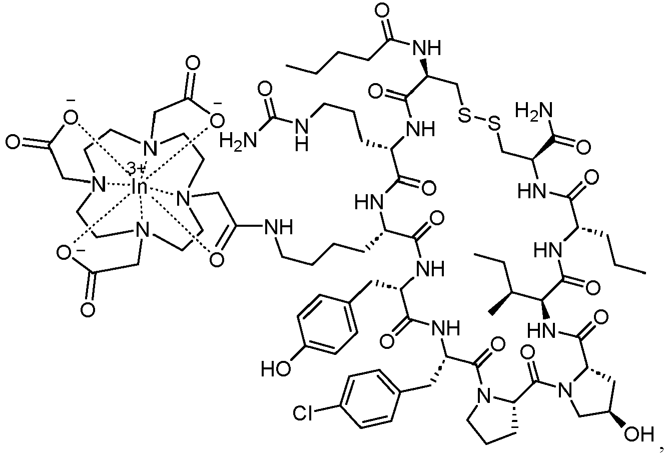
compound Bz-[Cys-Arg-Gln-Tyr-Phe-Pro-Hyp-Ile-Nva-Cys]-NH₂ (UPAR-356) of the following formula 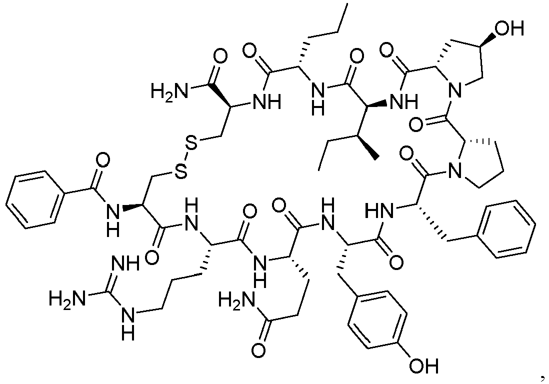
compound iHex-[Cys-Cit-Gln-Tyr-Phe-Pro-arg-Ile-Leu-Cys]-NHz (UPAR-357) of the following formula 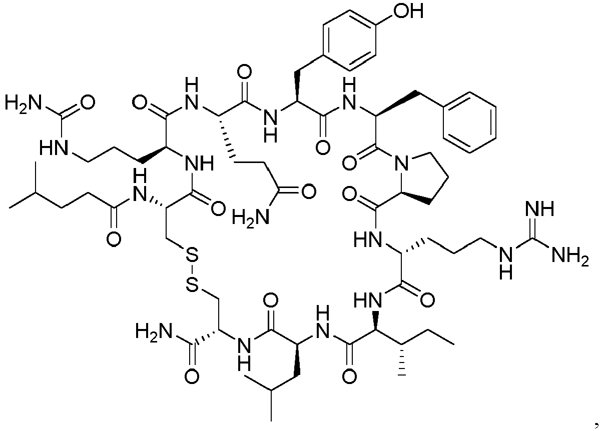
compound Butoc-[Cys-Thr-Gln-Tyr-Phe-4Cfp-Hyp-Ile-Nva-Cys]-NH₂ (UPAR-358) of the following formula 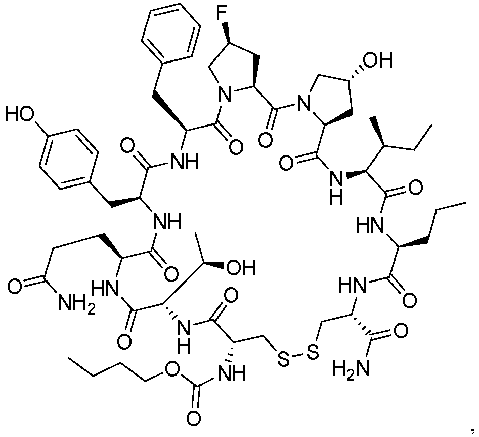
compound Glutar-[Cys-Cit-Gln-Tyr-Phe-Pro-ala-Ile-Leu-Cys]-NH₂ (UPAR-359) of the following formula 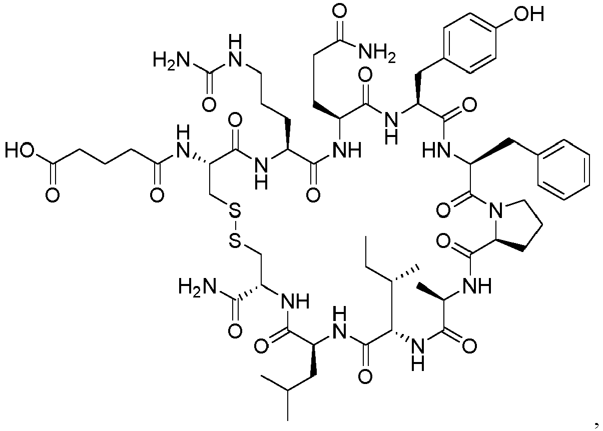
compound Pent-[Cys-Cit-Lys(DOTA)-Tyr-Thi-Pro-Hyp-Ile-Nva-Cys]-NH₂ (UPAR-360) of the following formula 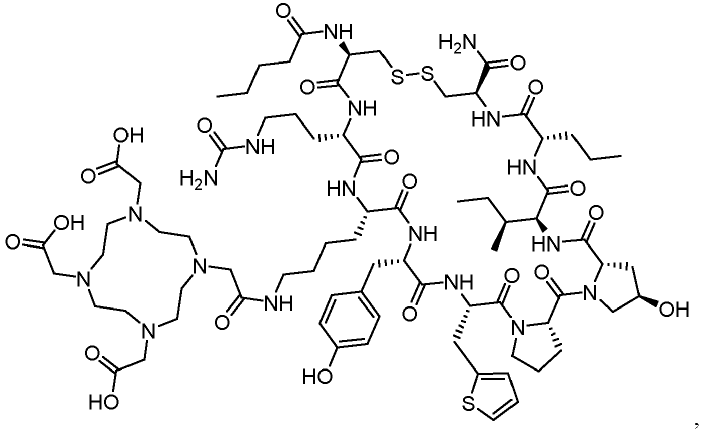
compound Pent-[Cys-Pro-Gln-Tyr-Phe-Pro-Hyp-Ile-Nva-Cys]-NHz (UPAR-361) of the following formula 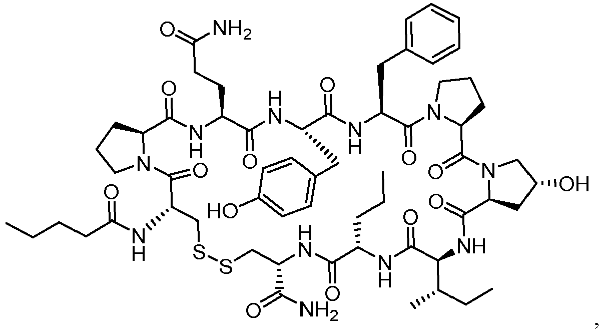
compound Pent-[Cys-Cit-Gln-Tyr-Phe-Pro-ala-Ile-Leu-Cys]-NHz (UPAR-362) of the following formula 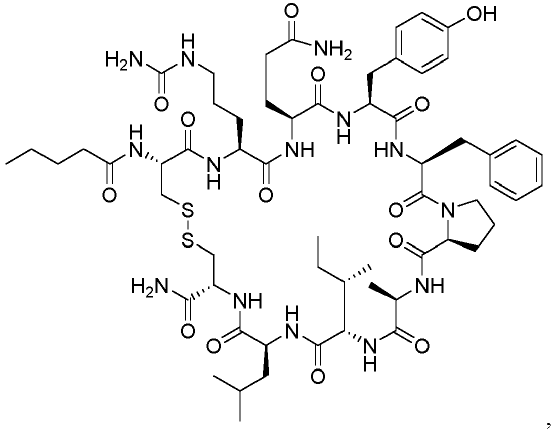
compound Hex-Asp-Leu-[Cys-Arg-Met-Tyr-Phe-Pro-Ala-Ile-Leu-Cys]-NH₂ (UPAR-363) of the following formula 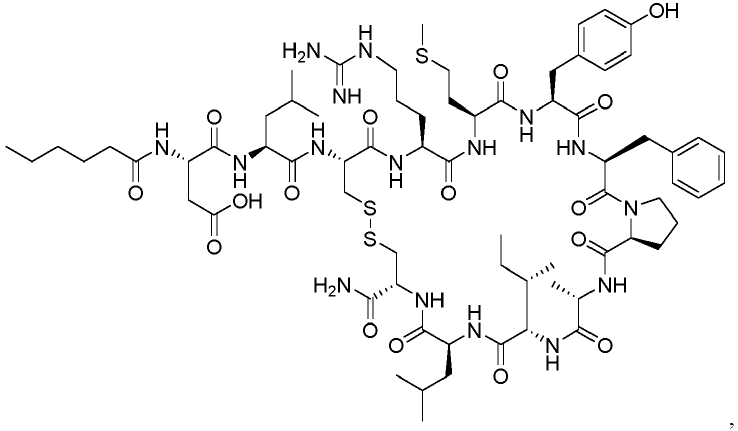
compound iHex-[Cys-cit-Gln-Tyr-Phe-Pro-ala-Ile-Leu-Cys]-NHz (UPAR-364) of the following formula 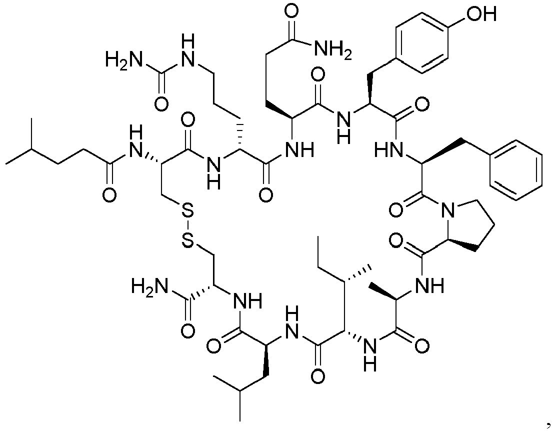
compound Pent-[Cys-Cit-Lys(DOTA)-Tyr-Phe-4Cfp-Hyp-Ile-Nva-Cys]-NH₂ (UPAR-365) of the following formula 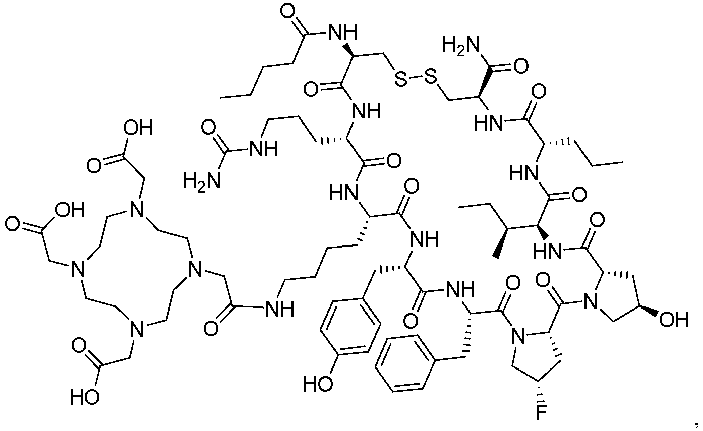
compound Pent-[Cys-Arg-Gln-Hfe-Phe-Pro-Hyp-Ile-Nva-Cys]-NHz (UPAR-366) of the following formula 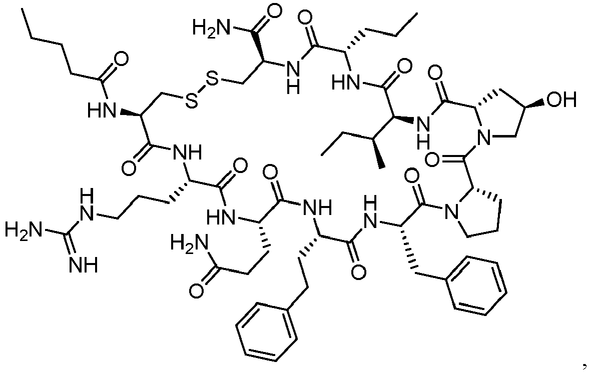
compound iHex-[Cys-Arg-Gln-Tyr-Phe-Pro-lys(DOTA)-Ile-Leu-Cys]-NH₂ (UPAR-367) of the following formula 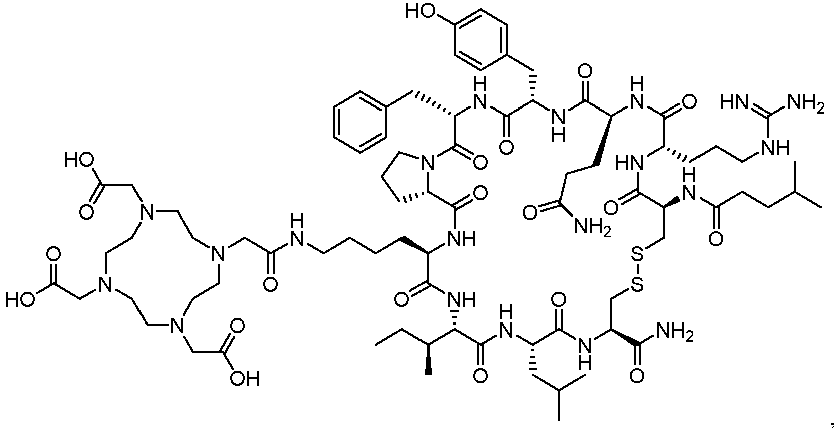
compound Butoc-[Cys-Thr-Lys(DOTA)-Tyr-Phe-4Cfp-Hyp-Ile-Hse-Cys]-NH₂ (UPAR-368) of the following formula 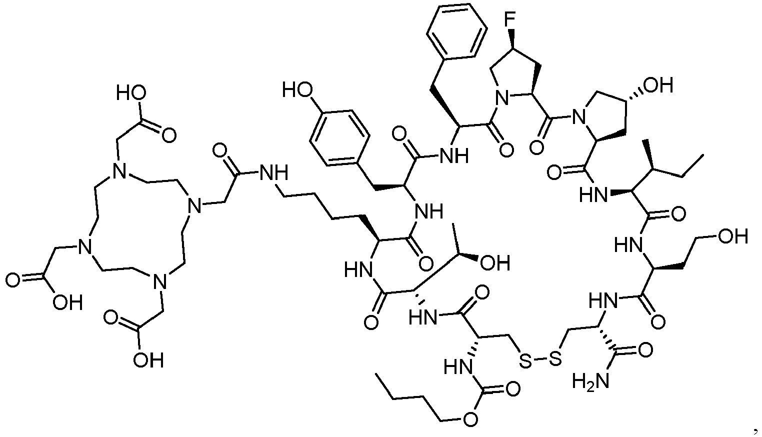
compound Butoc-[Cys-Cit-Lys(LuDOTA)-Tyr-Pcf-4Cfp-Hyp-Ile-Nva-Cys]-NH₂ (UPAR-369) of the following formula 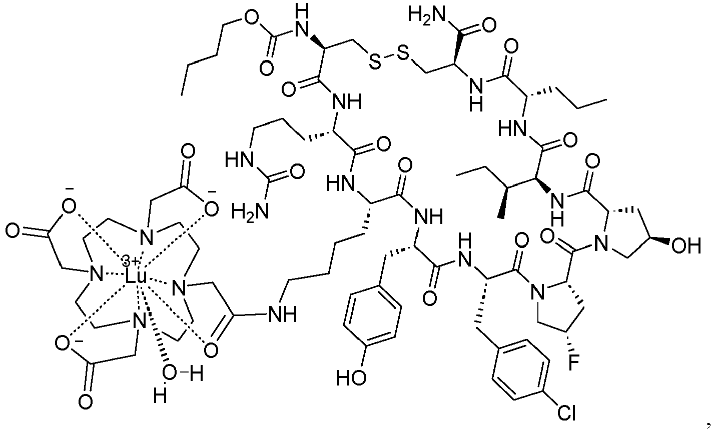
compound Pent-[Cys-Arg-Gln-Tyr-Phe-Pro-Hyp-Npg-Nva-Cys]-NHz (UPAR-370) of the following formula 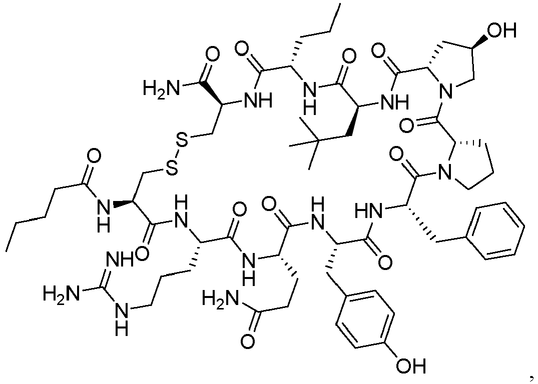
compound DOTA-Leu-[Cys-Cit-Gln-Tyr-Phe-Pro-Hyp-Ile-Nml-Cys]-NH₂ (UPAR-371) of the following formula 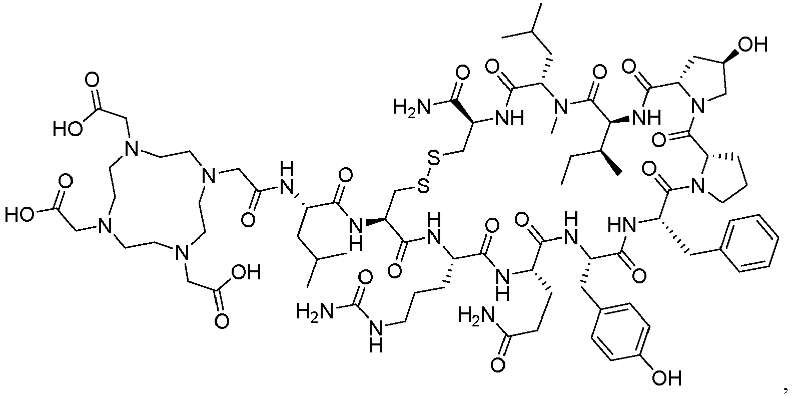
compound Ac-Cha-[Cys-Arg-Gln-Tyr-Phe-Pro-Hyp-Ile-Nva-Cys]-NHz (UPAR-372) of the following formula 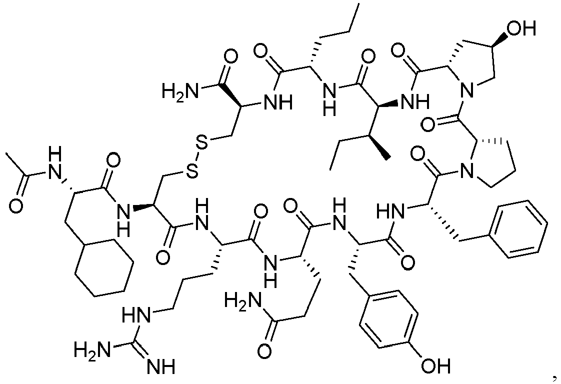
compound Pent-[Cys-Arg-Gln-Cha-Phe-Pro-Hyp-Ile-Nva-Cys]-NHz (UPAR-373) of the following formula 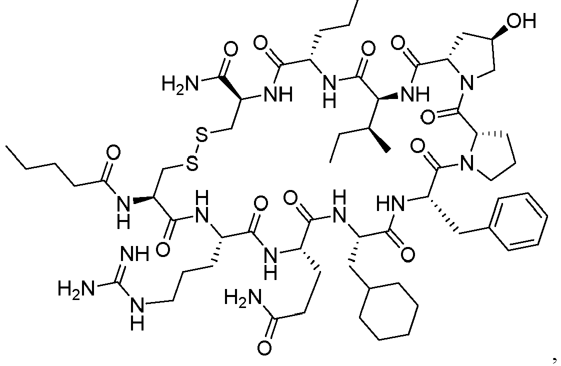
compound iHex-[Cys-Cit-Gln-Tyr-Phe-Eaz-ala-Ile-Leu-Cys]-NH₂ (UPAR-375) of the following formula 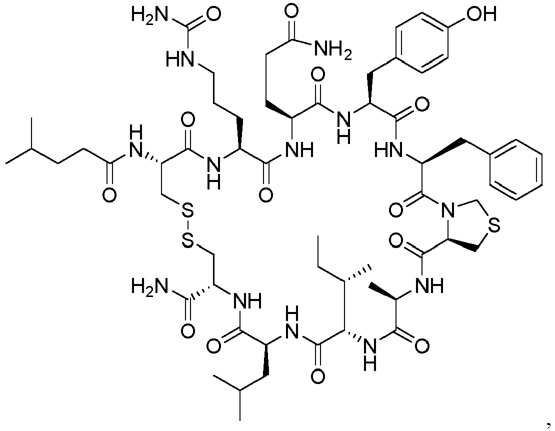
compound Pent-[Cys-Har-Lys(GdDOTA)-Tyr-Phe-Pro-Hyp-Ile-Nva-Cys]-NH₂ (UPAR-376) of the following formula 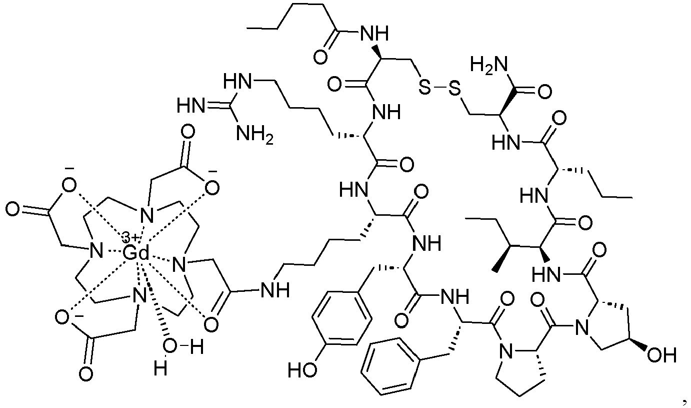
compound Pent-[Cys-Cit-Lys(DOTA)-Tyr-Phe-Pro-Tap-Ile-Nva-Cys]-NH₂ (UPAR-377) of the following formula 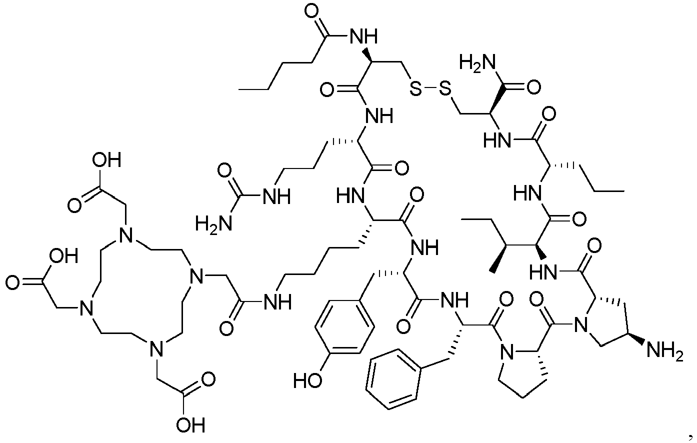
compound Butoc-[Cys-Thr-Lys(DOTA)-Tyr-Pnf-4Cfp-Hyp-Ile-Nva-Cys]-NH₂ (UPAR-378) of the following formula 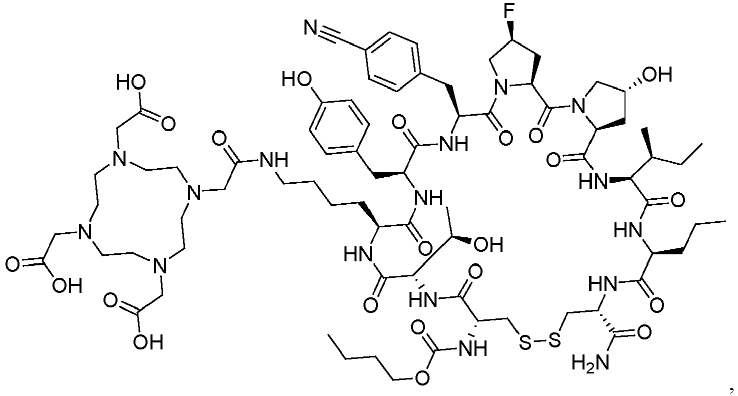
compound Pent-[Cys-Arg-Gln-Tyr-Phe-Pro-Hyp-Ile-Cpra-Cys]-NHz (UPAR-379) of the following formula 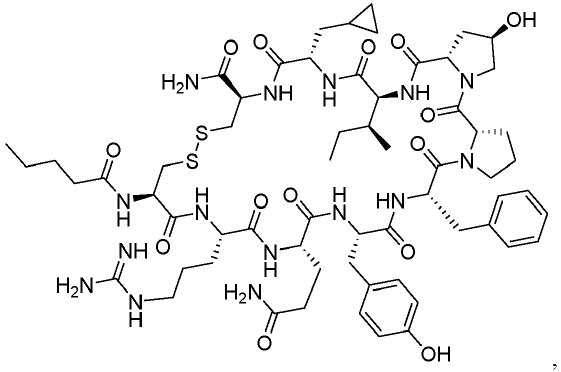
compound Pent-[Cys-Cit-Lys(DOTA)-Tyr-Phe-Pro-Hyp-Ile-Ser(Me)-Cys]-NH₂ (UPAR-380) of the following formula 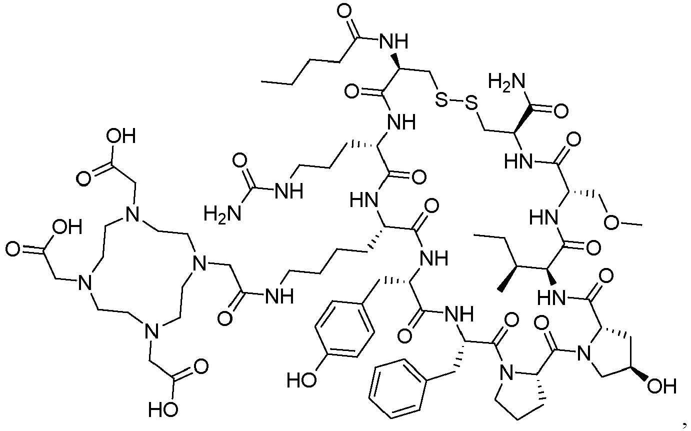
compound Ac-Leu-[Cys-Arg-Gln-Tyr-Phe-Pro-Ala-Ile-Leu-Cys]-NH₂ (UPAR-381) of the following formula 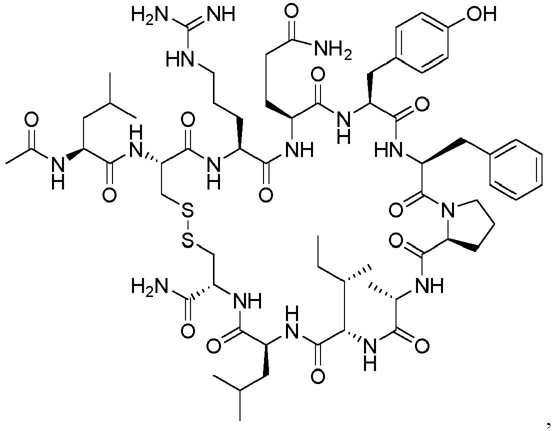
compound iHex-[Cys-Cit-Lys(DOTA)-Tyr-Phe-Pro-Pro-Ile-Leu-Cys]-NHz (UPAR-382) of the following formula 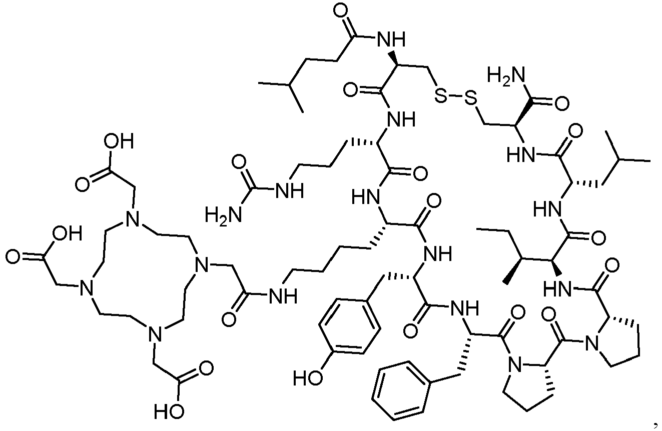
compound Pent-[Cys-Orn(mPEG12)-Lys(DOTA)-Tyr-Phe-Pro-Hyp-Ile-Nva-Cys]-NH₂ (UPAR-383) of the following formula 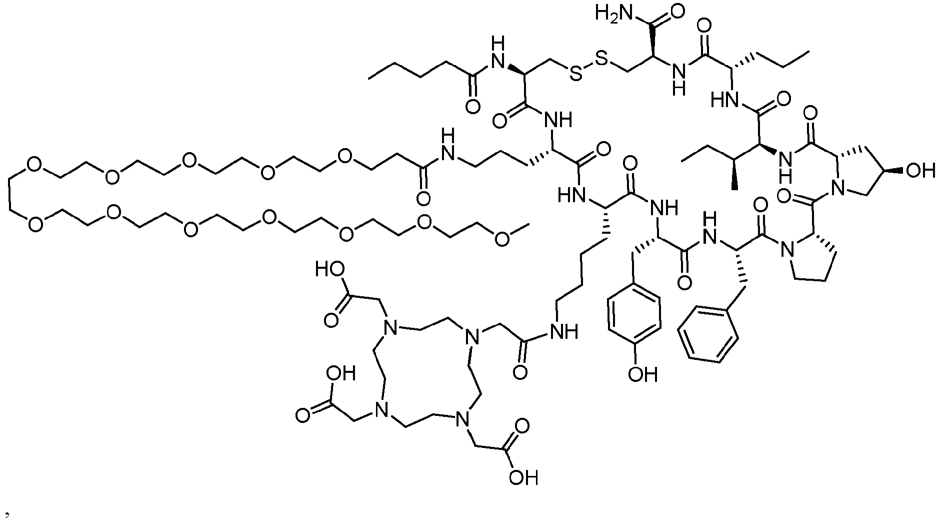
compound Pent-[Cys-Cit-Lys(DOTA)-Tyr-Phe-Pro-nma-Ile-Nva-Cys]-NH₂ (UPAR-384) of the following formula 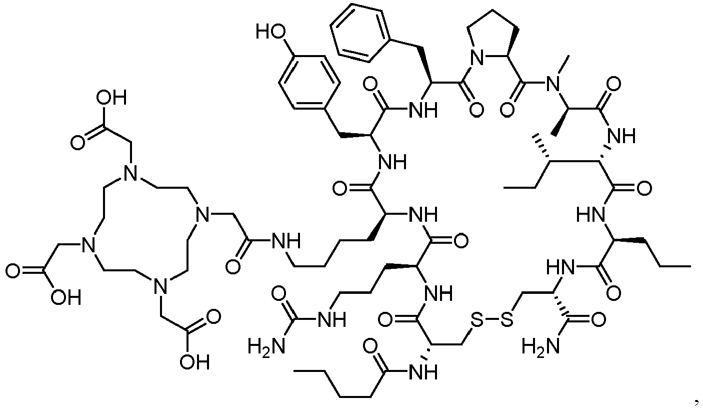
compound Pent-[Cys-Arg-Gln-Tyr-Phe-Pro-Hyp-Lys-Nva-Cys]-NHz (UPAR-385) of the following formula 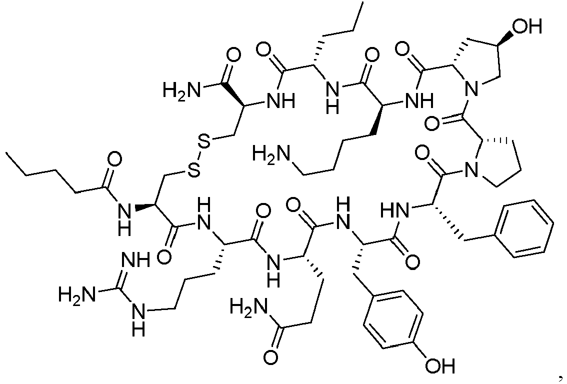
compound Pent-[Cys-Glu(AGLU)-Gln-Tyr-Phe-Pro-Hyp-Ile-Nva-Cys]-NH₂ (UPAR-386) of the following formula 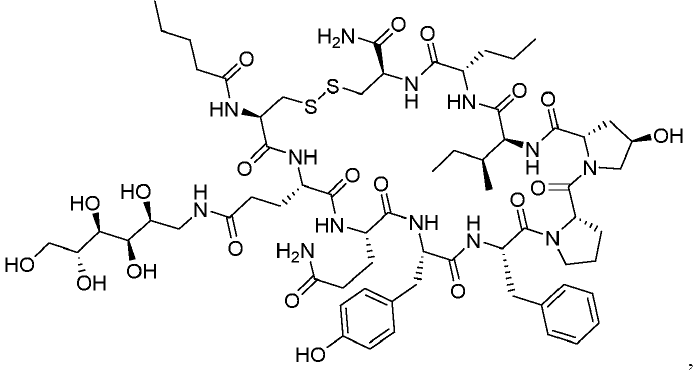
compound Pent-[Cys-Arg-Gln-Tyr-Phe-Oic-Hyp-Ile-Nva-Cys]-NHz (UPAR-387) of the following formula 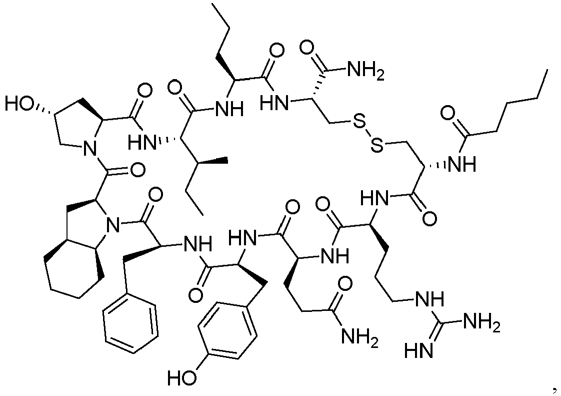
compound Pent-[Cys-Thr-Lys(DOTA)-Tyr-Phe-Chy-Hyp-Ile-Nva-Cys]-NH₂ (UPAR-388) of the following formula 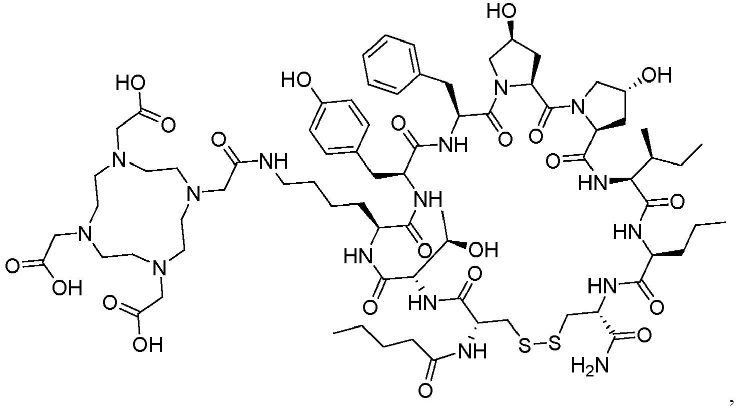
compound iHex-[Cys-Cit-Gln-Tyr-Phe-Pro-Tap(DOTA)-Ile-Leu-Cys]-NH₂ (UPAR-389) of the following formula 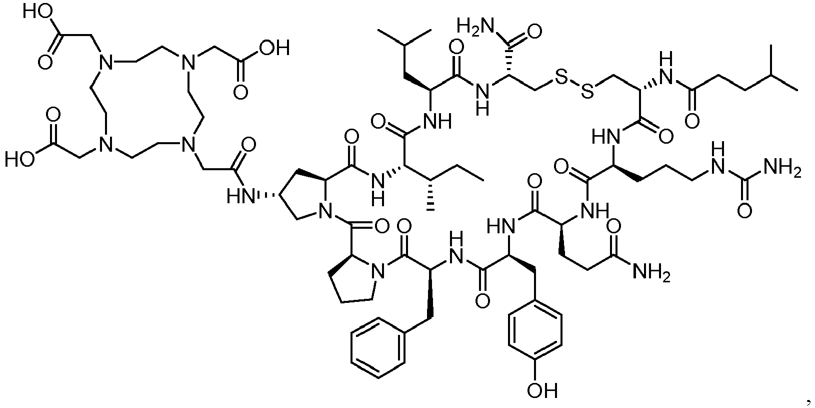
compound Pent-[Dab-Arg-Gln-Tyr-Phe-Pro-Hyp-Ile-Nva-Glu]-NHz (UPAR-390) of the following formula 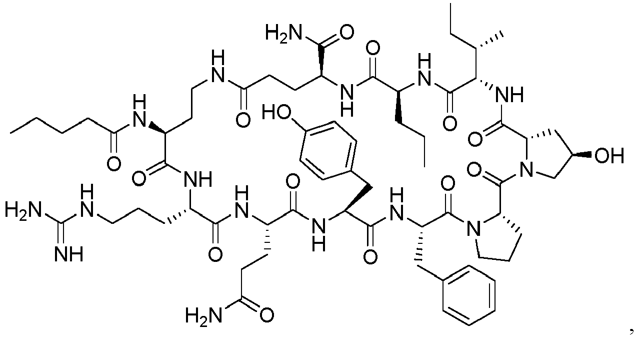
compound Pent-[Cys-Leu-Gln-Tyr-Phe-Pro-Hyp-Ile-Nva-Cys]-NHz (UPAR-391) of the following formula 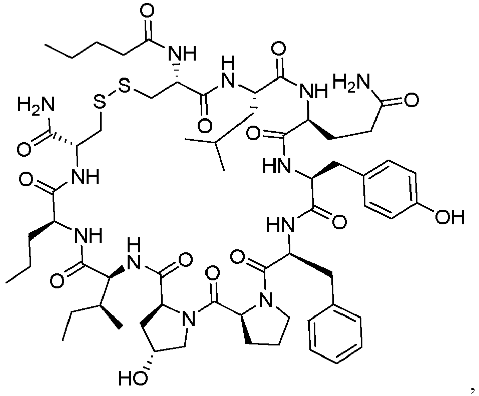
compound Ac-Asp-Leu-[Cys-asp-Lys(DOTA)-Tyr-Phe-Pro-ala-Ile-Leu-Cys]-NH₂ (UPAR-392) of the following formula 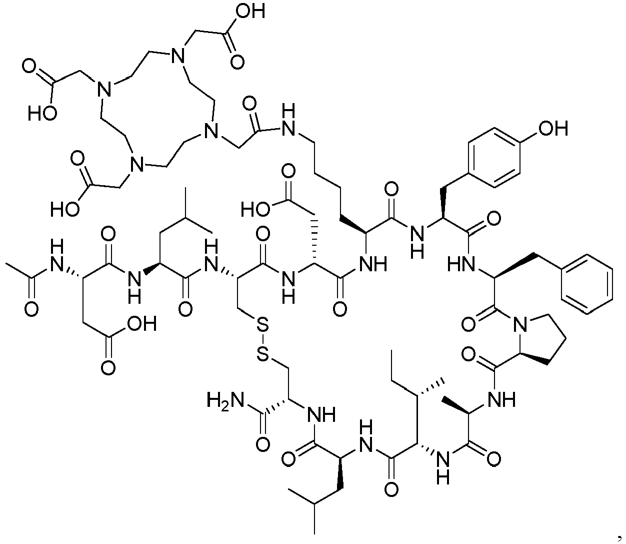
compound iHex-[Cys-Cit-Gln-Mpa-Phe-Pro-ala-Ile-Leu-Cys]-NHz (UPAR-393) of the following formula 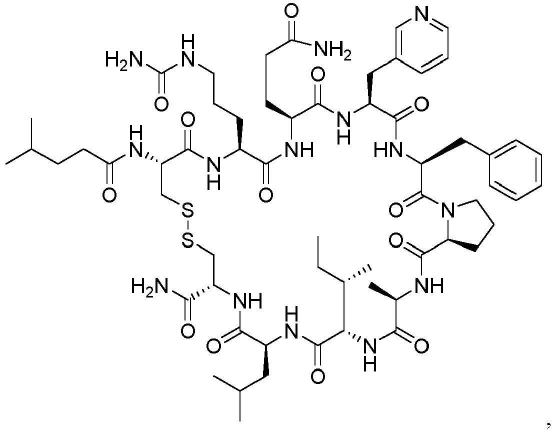
compound H-Ava-[Cys-Cit-Gln-Tyr-Phe-Pro-ala-Ile-Leu-Cys]-NHz (UPAR-394) of the following formula 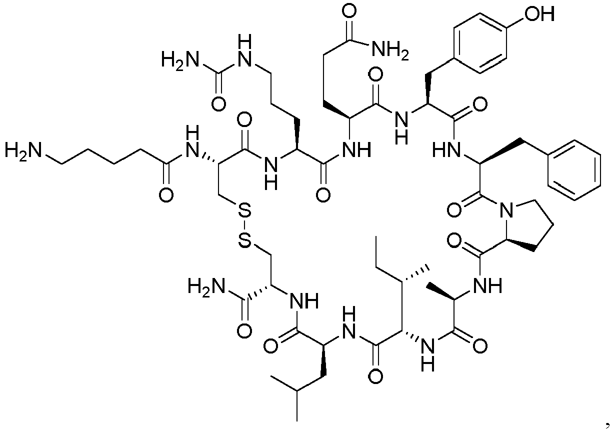
compound Pent-[Cys-Arg-Gln-Ocf-Phe-Pro-Hyp-Ile-Nva-Cys]-NH₂ (UPAR-395) of the following formula 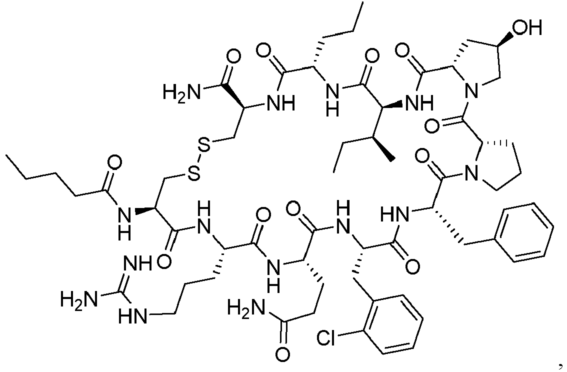
compound Pent-[Cys-Cit-Lys(DOTA)-Tyr-Phe-Pro-Pro-Ile-Leu-Cys]-NH₂ (UPAR-396) of the following formula 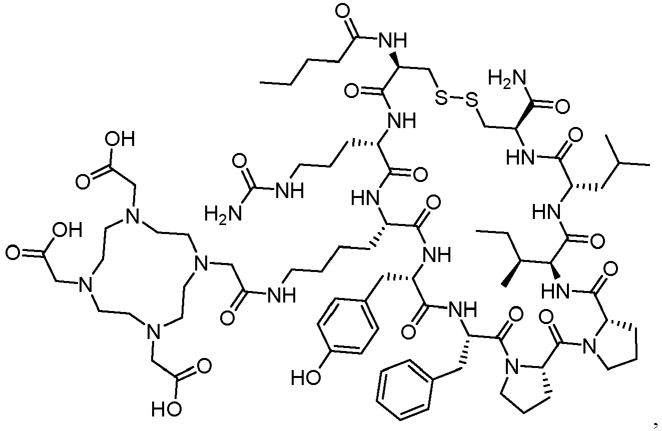
compound Pent-[Pen-Cit-Gln-Tyr-Phe-Pro-Hyp-Ile-Nva-Cys]-NHz (UPAR-397) of the following formula 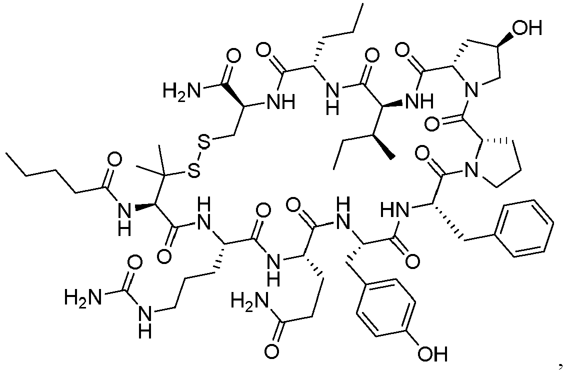
compound Pent-[Cys-Cit-Lys(DOTA)-Tyr-Nmf-Pro-Hyp-Ile-Nva-Cys]-NH₂ (UPAR-398) of the following formula 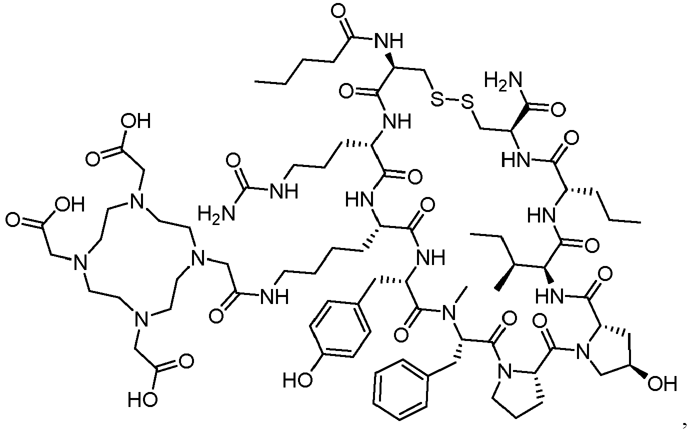
compound Pent-[Cys-Cit-Lys(DOTA)-Dopa-Phe-Pro-Hyp-Ile-Nva-Cys]-NH₂ (UPAR-399) of the following formula 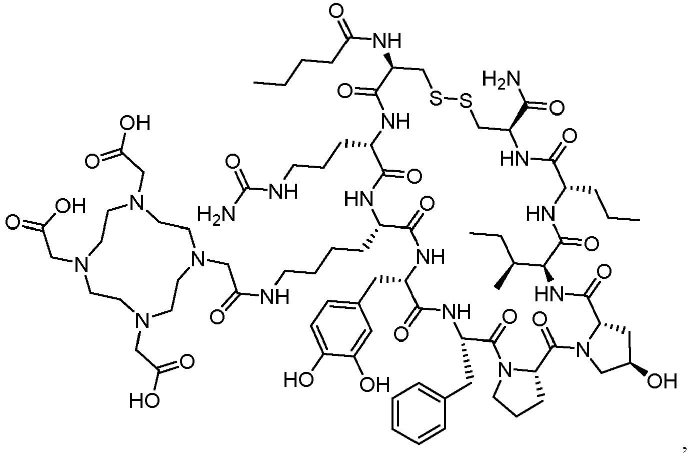
compound Pent-[Cys-Arg-Gln-Tyr-Phe-Phe-Hyp-Ile-Nva-Cys]-NH₂ (UPAR-400) of the following formula 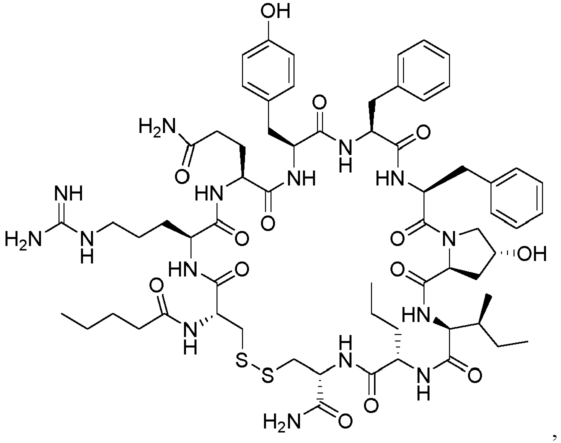
compound Pent-[Cys-Arg-Gln-Trp-Phe-Pro-Hyp-Ile-Nva-Cys]-NHz (UPAR-401) of the following formula 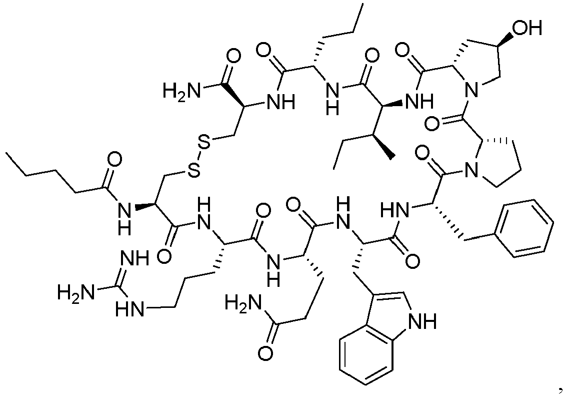
compound iHex-[Cys-Cit-Gln-Tyr-Phe-Pro-ala-Ile-Ala-Cys]-NHz (UPAR-402) of the following formula 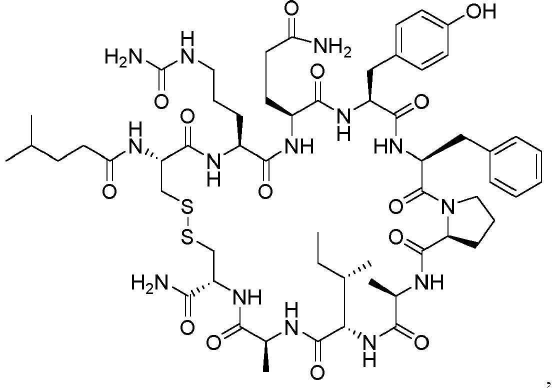
compound Pent-[Cys-Cit-Lys(DOTA)-Tyr-Phe-Hyp-Hyp-Ile-Nva-Cys]-NH₂ (UPAR-403) of the following formula 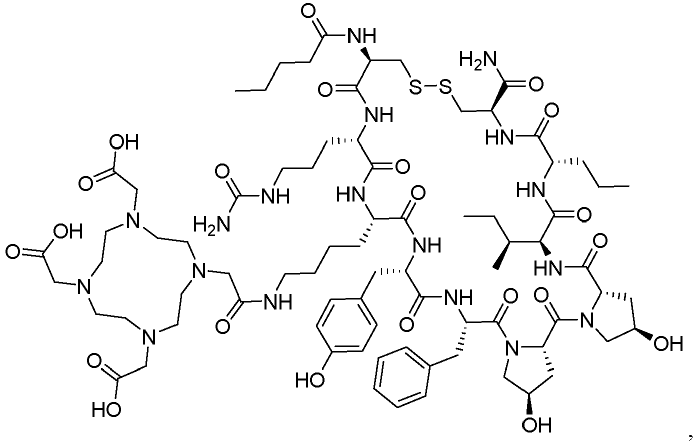
compound Pent-[Cys-Cit-Lys(InDOTA-PEG12)-Tyr-Phe-Pro-Hyp-Ile-Nva-Cys]-NH₂ (UPAR-404) of the following formula 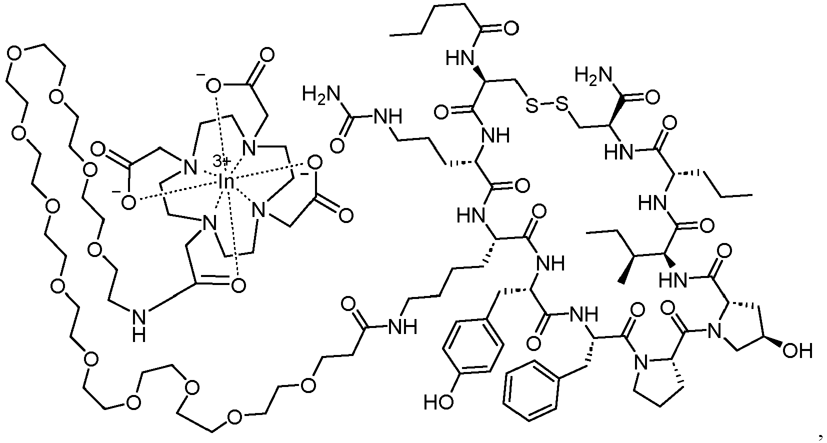
compound iHex-[Cys-Cit-Gln-Tyr-Phe-Pro-ala-Ile-Ile-Cys]-NH₂(UPAR-405) of the following formula 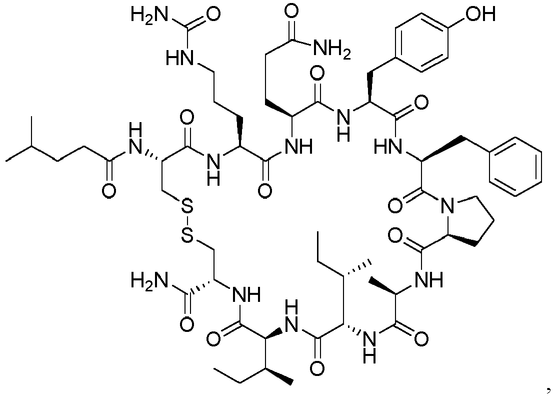
compound Ac-Asp-Leu-[Cys-Arg-Nle-Tyr-Phe-Pro-ala-Ile-Leu-Cys]-NH₂ (UPAR-406) of the following formula 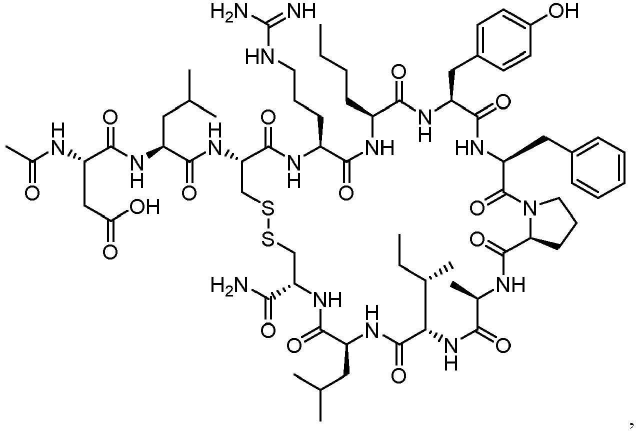
compound iHex-[Cys-Cit-Nmq-Tyr-Phe-Pro-ala-Ile-Leu-Cys]-NHz (UPAR-407) of the following formula 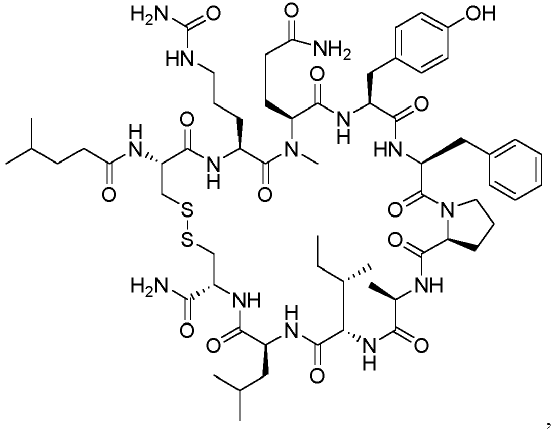
compound iHex-[Cys-Lys(LuDOTA)-Gln-Tyr-Phe-Pro-ala-Ile-Leu-Cys]-NH₂ (UPAR-408) of the following formula 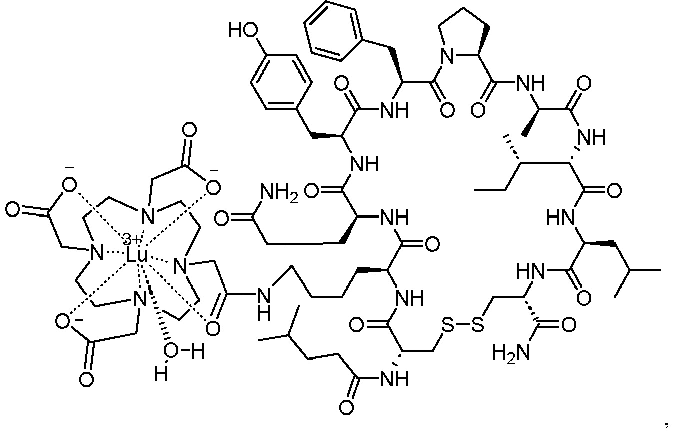
compound Pent-[Cys-Arg-Gly-Tyr-Phe-Pro-Hyp-Ile-Nva-Cys]-NHz (UPAR-409) of the following formula 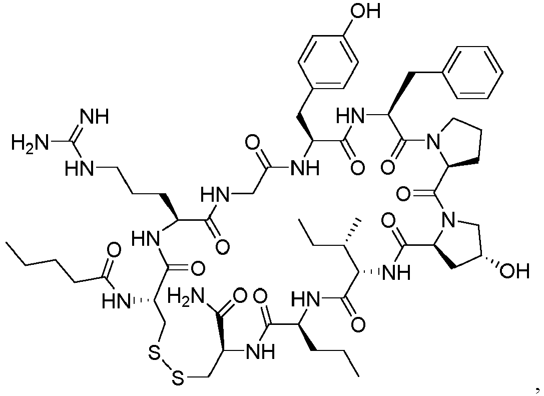
compound iHex-[Cys-Cit-Asp-Tyr-Phe-Pro-Hyp-Ile-Leu-Cys]-NHz (UPAR-410) of the following formula 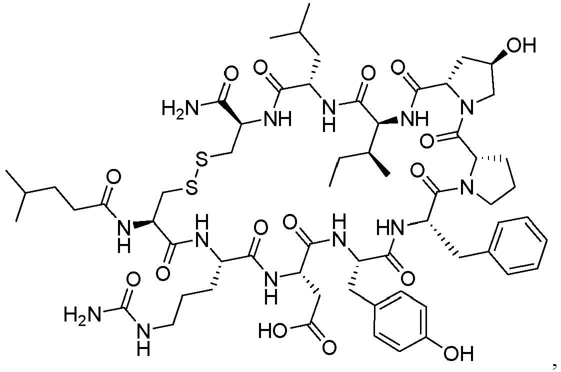
compound Pent-[Cys-Arg-Gln-Tyr-Phe-Pro-Hyp-Ile-Cbg-Cys]-NHz (UPAR-411) of the following formula 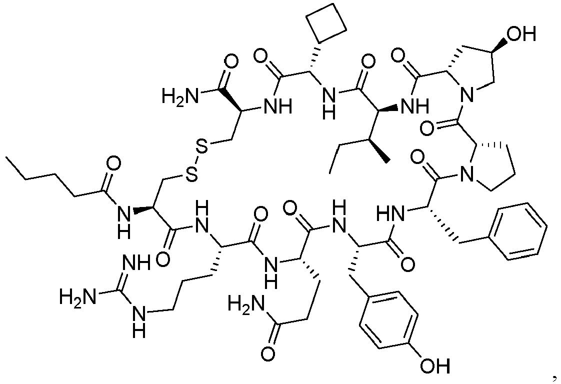
compound Ac-Asp-Leu-[cys-Arg-Nle-Tyr-Phe-Pro-Ala-Ile-Leu-Cys]-NH₂ (UPAR-412) of the following formula 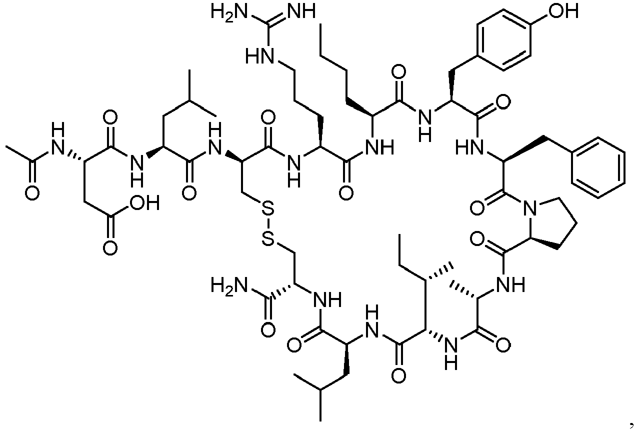
compound iHex-[Cys-Cit-Lys(Ac)-Tyr-Phe-Pro-ala-Ile-Leu-Cys]-NH₂ (UPAR-413) of the following formula 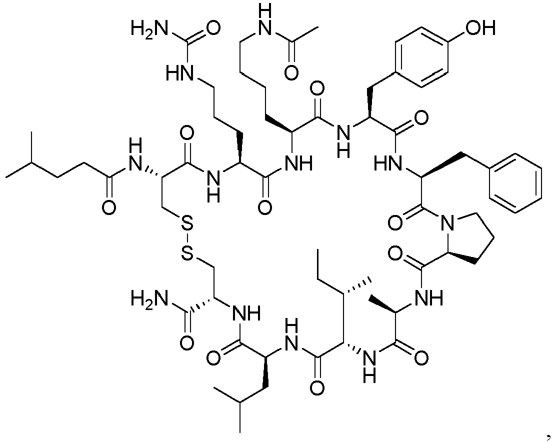
compound iHex-[Cys-ala-Gln-Tyr-Phe-Pro-Lys(DOTA)-Ile-Leu-Cys]-NH₂ (UPAR-414) of the following formula 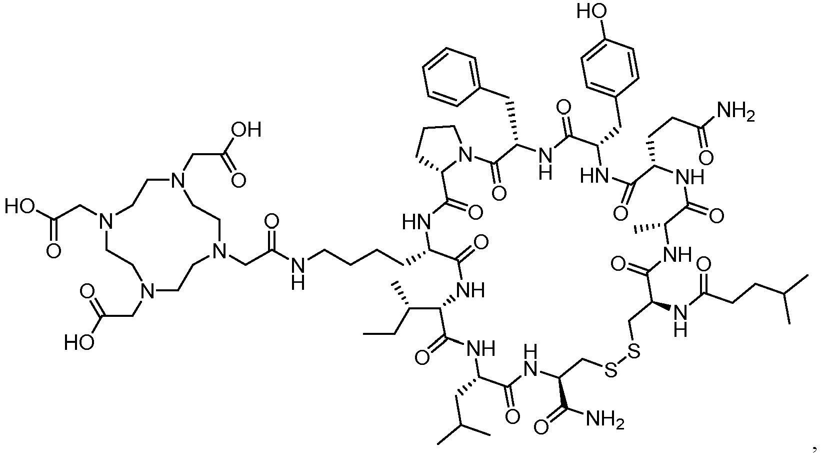
compound Butoc-[Cys-Thr-Orn(DOTA)-My-Phe-4Cfp-Hyp-Ile-Nva-Cys]-NH₂ (UPAR-415) of the following formula 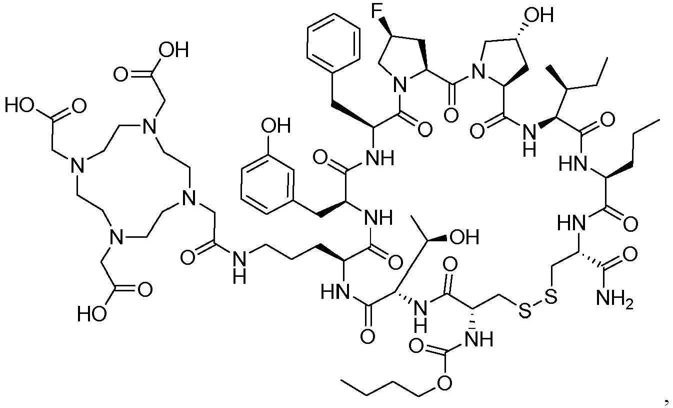
compound Pent-[Cys-Cit-Lys(DOTA)-Tyr-Amf-Pro-Hyp-Ile-Nva-Cys]-NH₂ (UPAR-416) of the following formula 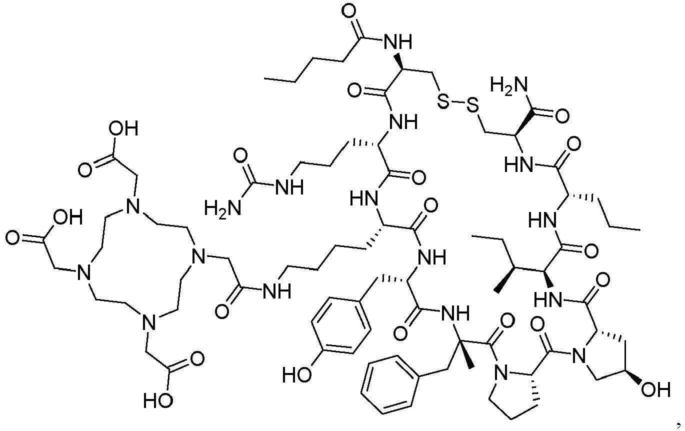
compound Pent-[Cys-Cit-Lys(DOTA)-Tyr-Aic-Pro-Hyp-Ile-Nva-Cys]-NH₂ (UPAR-417) of the following formula 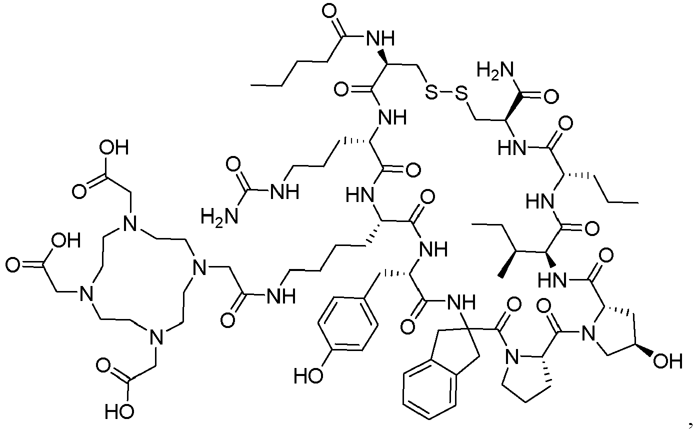
compound Ac-Leu-[Cys-Cit-Gln-Tyr-Phe-Pro-ala-Ile-Leu-Cys]-NH₂ (UPAR-418) of the following formula 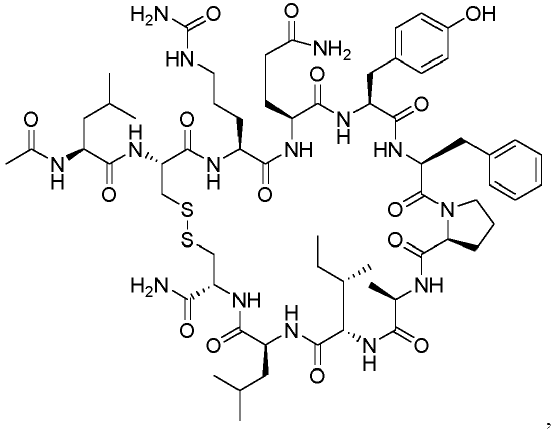
compound Pent-[Cys-Thr-Lys(DOTA)-Tyr-Phe-Aib-Hyp-Ile-Nva-Cys]-NH₂ (UPAR-419) of the following formula 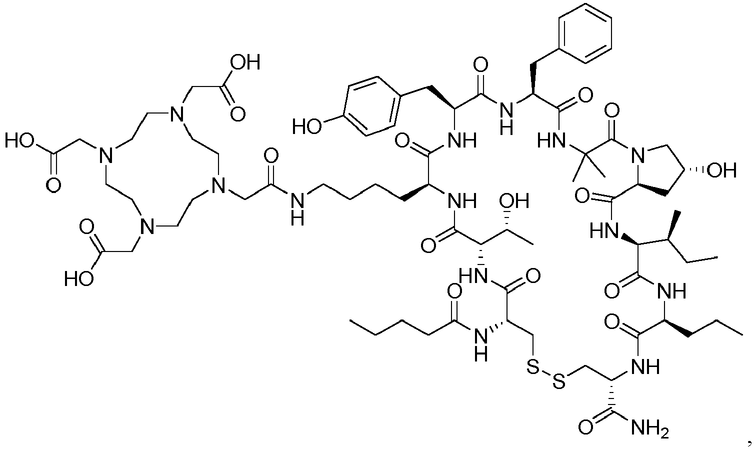
compound Ac-Asp-Leu-[Cys-Arg-Nle-Tyr-Phe-Pro-Ala-leu-Leu-Cys]-NH₂ (UPAR-420) of the following formula 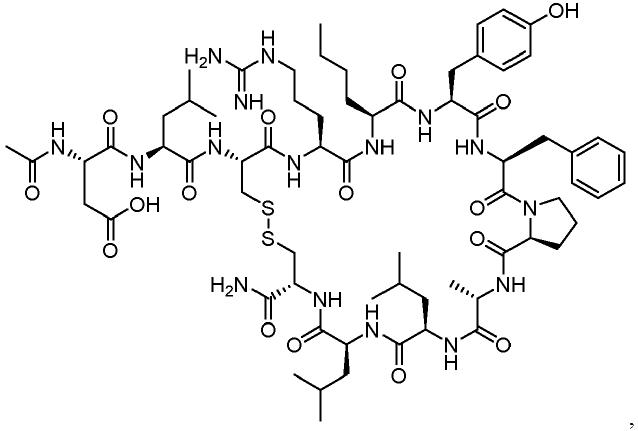
compound Butoc-[Cys-Thr-Lys(DOTA-Kzw)-Tyr-Phe-4Cfp-Hyp-Ile-Nva-Cys]-NH₂ (UPAR-422) of the following formula 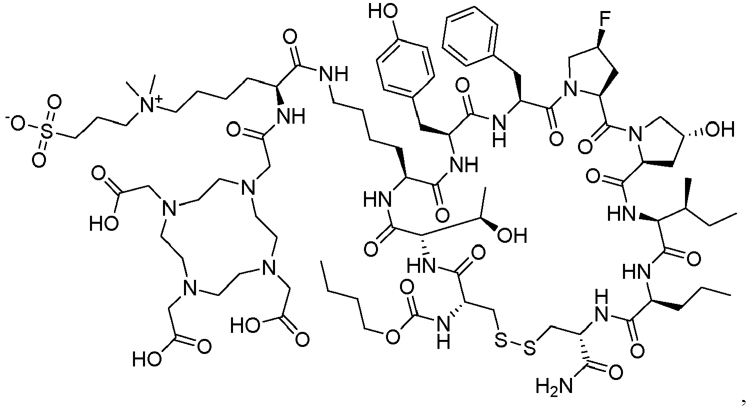
compound Ac-Asp-Leu-[Cys-Cit-Lys(DOTA)-Tyr-Phe-Pro-Arg-Ile-Leu-Cys]-NH₂ (UPAR-423) of the following formula 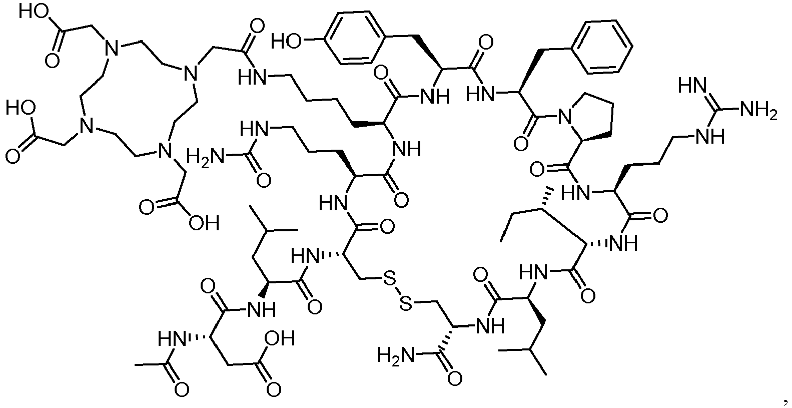
compound Butoc-[Cys-Thr-Lys(DOTA-asp)-Tyr-Phe-4Cfp-Hyp-Ile-Nva-Cys]-NH₂ (UPAR-424) of the following formula 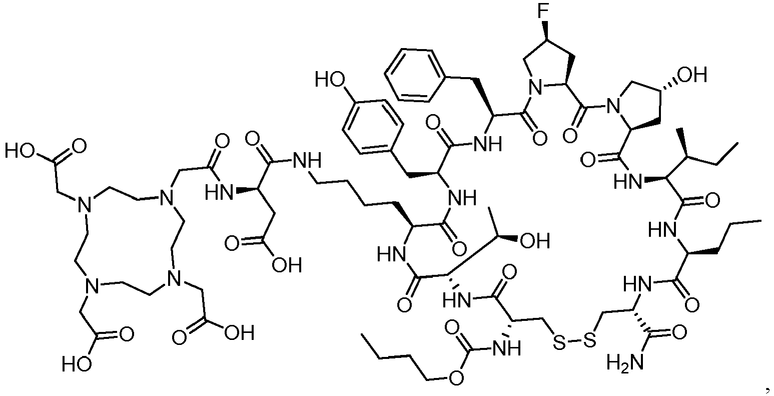
compound Pent-[Cys-Cit-Lys(DOTA)-Tyr-Phe-Pro-Gly-Ile-Nva-Cys]-NH₂ (UPAR-425) of the following formula 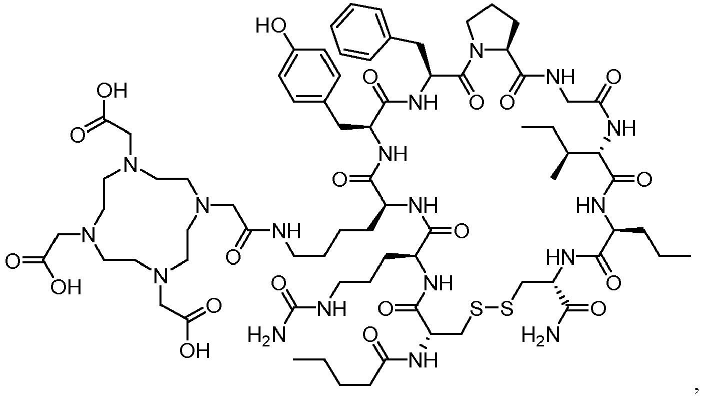
compound iHex-[Cys-Cit-Gln-Tyr-Phe-Aze-ala-Ile-Leu-Cys]-NH₂ (UPAR-426) of the following formula 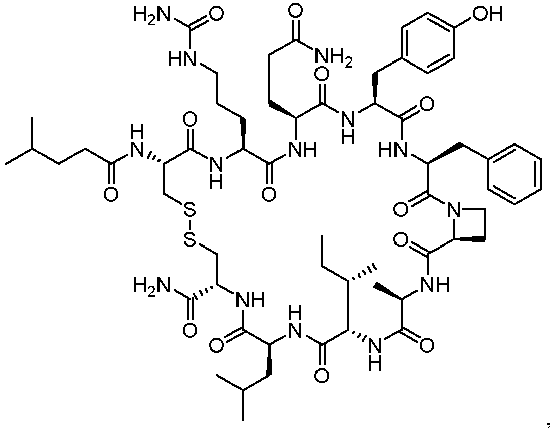
compound Hex-Asp-Leu-[Cys-Arg-Lys(DOTA)-Tyr-Phe-Pro-Ala-Ile-Leu-Cys]-NH₂ (UPAR-427) of the following formula 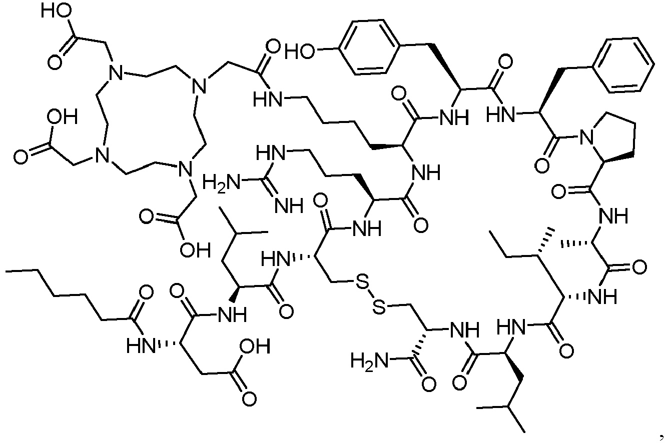
compound H-Nmb-[Cys-Thr-Lys(DOTA)-Tyr-Phe-4Cfp-Hyp-Ile-Nva-Cys]-NH₂ (UPAR-428) of the following formula 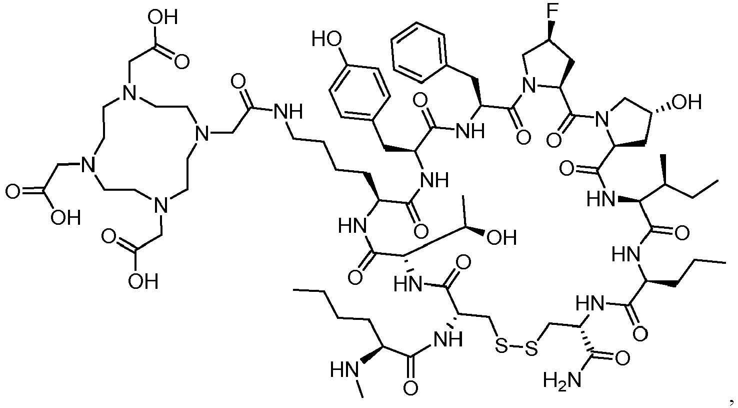
compound iHex-[Cys-Cit-Gln-Tyr-Phe-Pro-ala-Ile-Nva-Cys]-NHz (UPAR-429) of the following formula 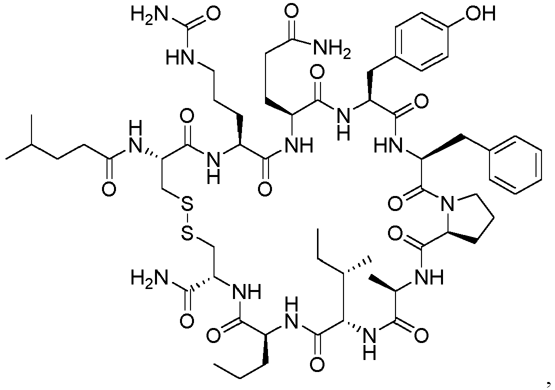
compound Pent-[Cys-Cit-Gln-Tyr-Phe-Pro-Hyp-Ile-Nva-Cys]-en (UPAR-430) of the following formula 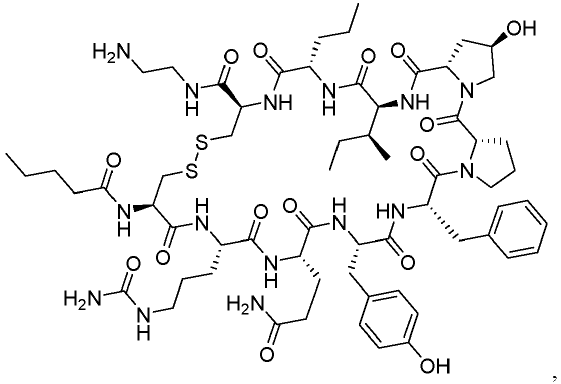
compound Ac-Asp-Leu-[Cys-Arg-Nle-Tyr-Phe-Pro-Pro-Ile-Leu-Cys]-NH₂ (UPAR-431) of the following formula 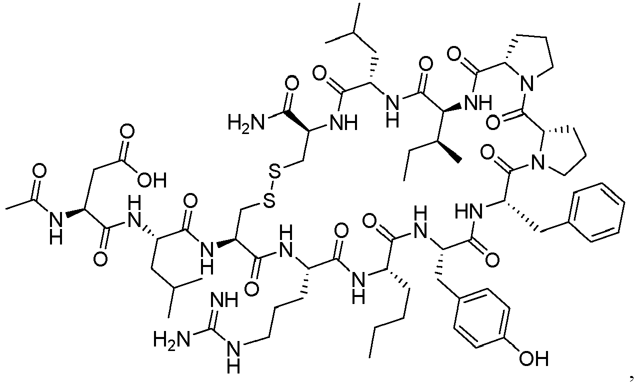
compound Ac-Asp-Leu-[Cys-Asp-Lys(DOTA)-Tyr-Phe-Pro-ala-Ile-Leu-Cys]-NH₂ (UPAR-432) of the following formula 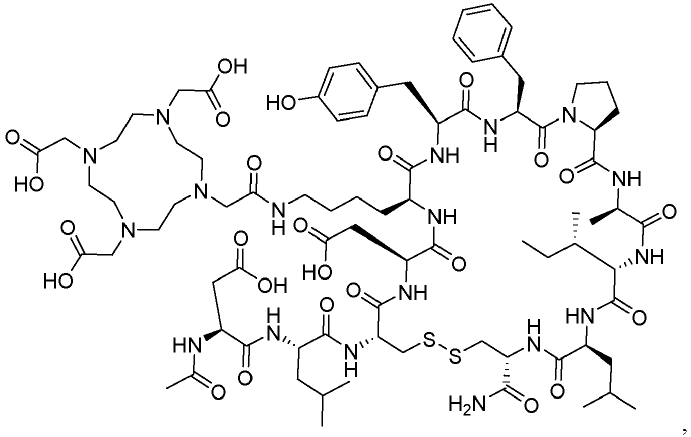
compound Pent-[Cys-Arg-Gln-Tyr-Phe-Leu-Hyp-Ile-Nva-Cys]-NH₂ (UPAR-433) of the following formula 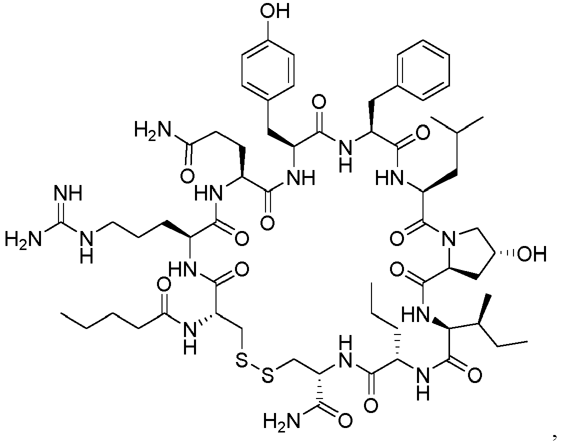
compound iHex-[Cys-Cit-Asp-Tyr-Phe-Pro-ala-Ile-Leu-Cys]-NHz (UPAR-434) of the following formula 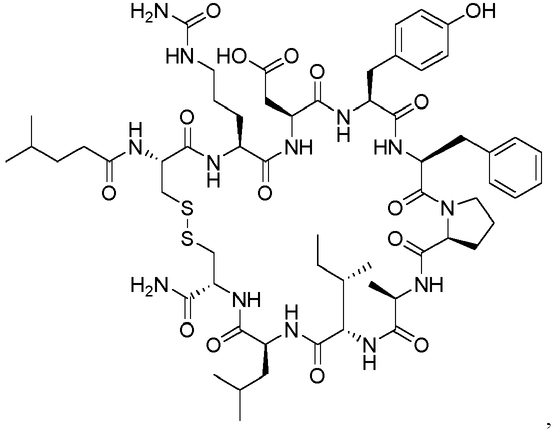
compound Pent-[Cys-Cit-Lys(GdDOTA)-Tyr-Phe-Pro-4Tfp-Ile-Nva-Cys]-NH₂ (UPAR-435) of the following formula 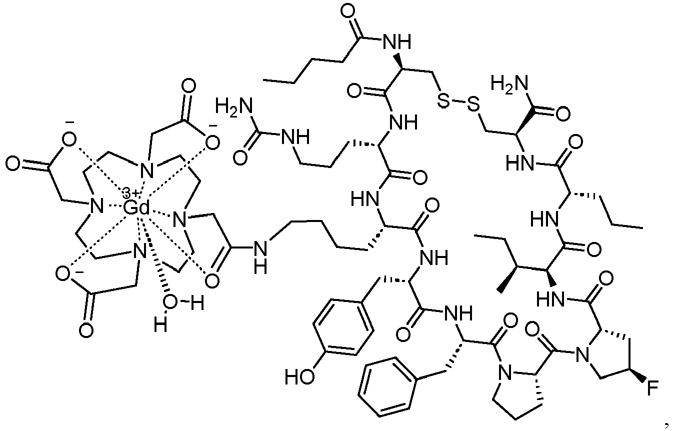
compound Pent-[Cys-Arg-Gln-Tyr-Phe-Pro-glu-Ile-Nva-Cys]-NHz (UPAR-436) of the following formula 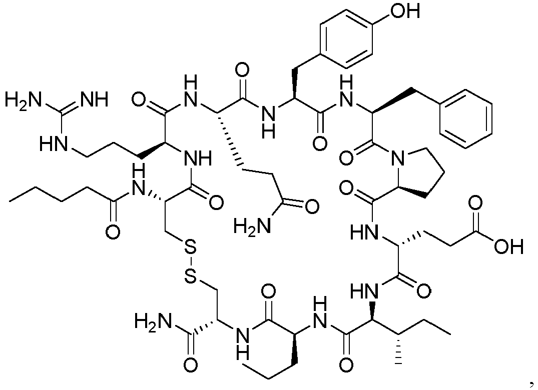
compound Pent-[Cys-Arg-Gln-Tyr-Phe-Pro-Hyp-Phg-Nva-Cys]-NHz (UPAR-437) of the following formula 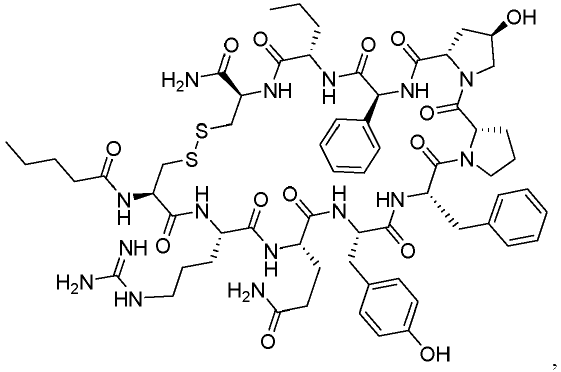
compound Pent-[Cys-Arg-Gln-Tyr-Phe-Pro-orn-Ile-Nva-Cys]-NH₂ (UPAR-438) of the following formula 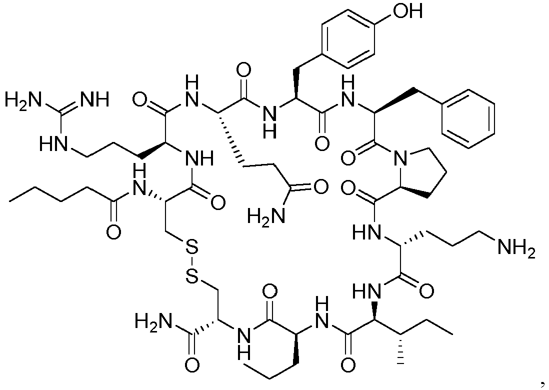
compound iHex-[Cys-Cit-Gln-Tyr-Phe-Hyp-ala-Ile-Leu-Cys]-NH₂ (UPAR-439) of the following formula 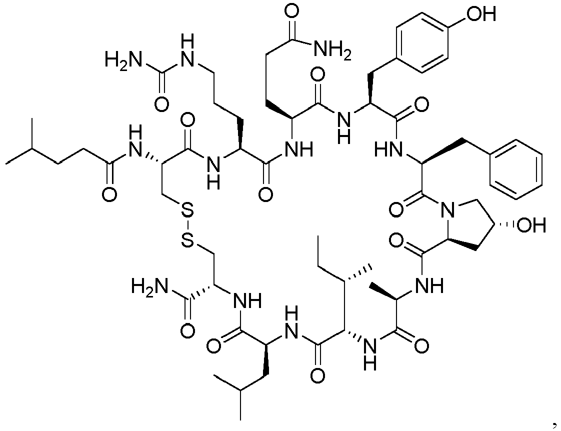
compound Ac-Asp-Leu-[Cys-Arg-Nle-Tyr-Phe-Pro-Aib-Ile-Leu-Cys]-NH₂ (UPAR-440) of the following formula 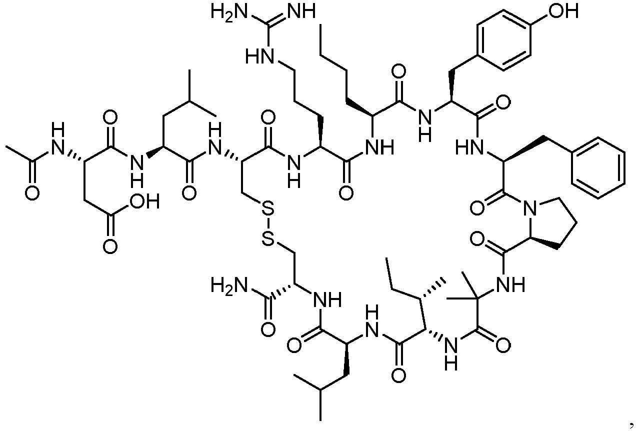
compound DOTA-Leu-[Cys-Cit-Gln-Tyr-Phe-Pro-ala-Ile-Leu-Cys]-NH₂ (UPAR-441) of the following formula 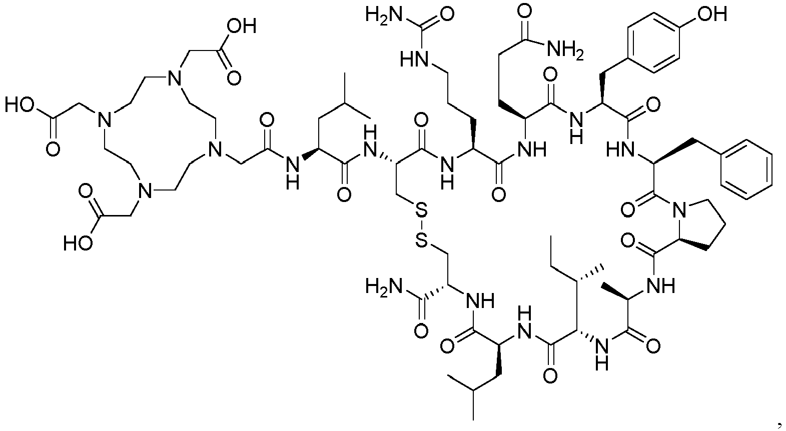
compound Pent-[Cys-Arg-Gln-Lys-Phe-Pro-Hyp-Ile-Nva-Cys]-NHz (UPAR-442) of the following formula 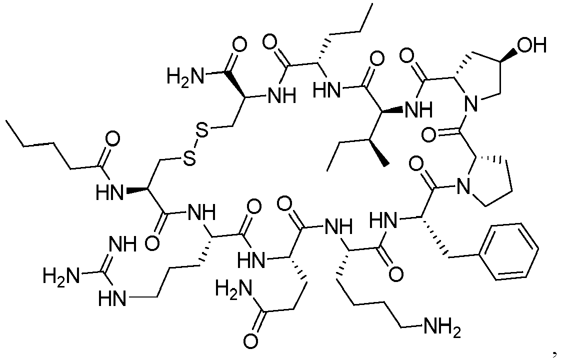
compound Butoc-[Cys-Thr-Lys(NODAGA)-Dopa-Phe-4Cfp-Hyp-Ile-Nva-Cys]-NH₂ (UPAR-444) of the following formula 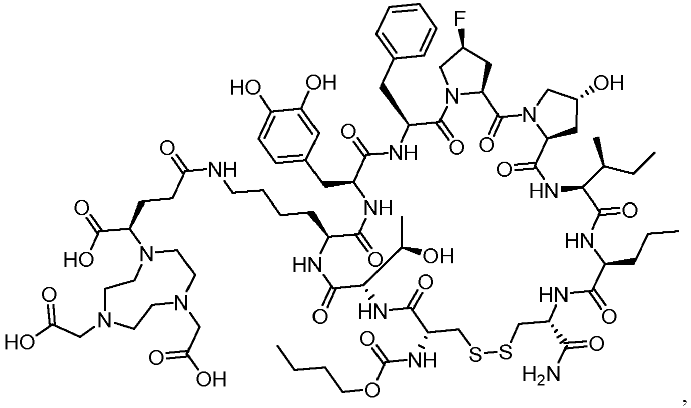
compound iHex-[Cys-Cit-Cit-Tyr-Phe-Pro-ala-Ile-Leu-Cys]-NH₂ (UPAR-445) of the following formula 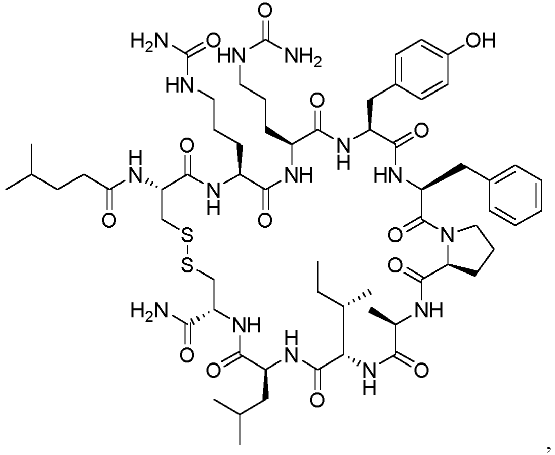
compound iHex-[Cys-Cit-Gln-Tyr-Phe-Pro-Aze-Ile-Leu-Cys]-NH₂ (UPAR-446) of the following formula 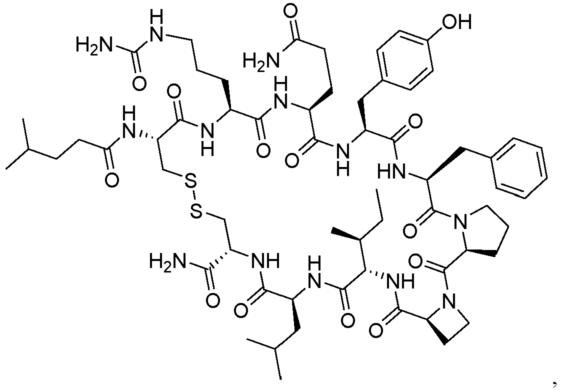
compound Pent-[Cys-Arg-Gln-Tyr-Phe-Pro-Hyp-Phe-Nva-Cys]-NHz (UPAR-447) of the following formula 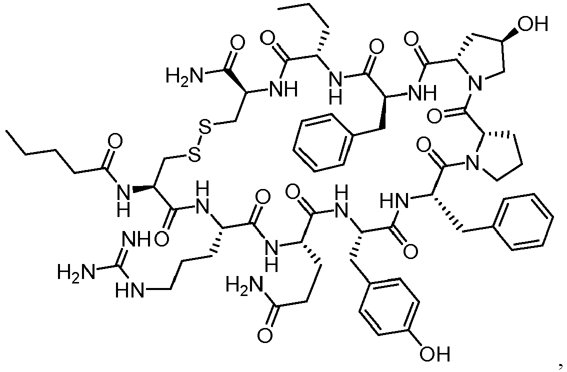
compound Pent-[Cys-Arg-Lys-Tyr-Phe-Pro-Hyp-Ile-Nva-Cys]-NHz (UPAR-448) of the following formula 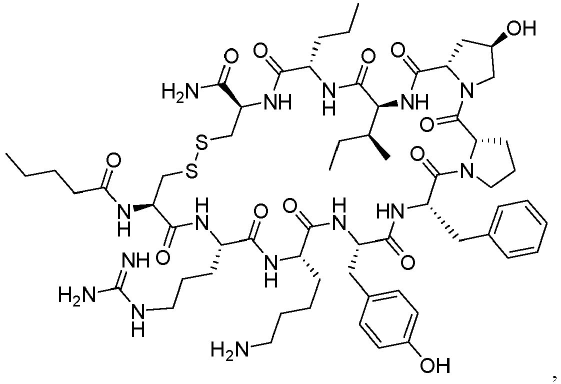
compound iHex-[Cys-Arg-Gln-Tyr-Phe-Pro-ala-Ile-Leu-Cys]-O2Oc-Lys(DOTA)-NH₂ (UPAR-449) of the following formula 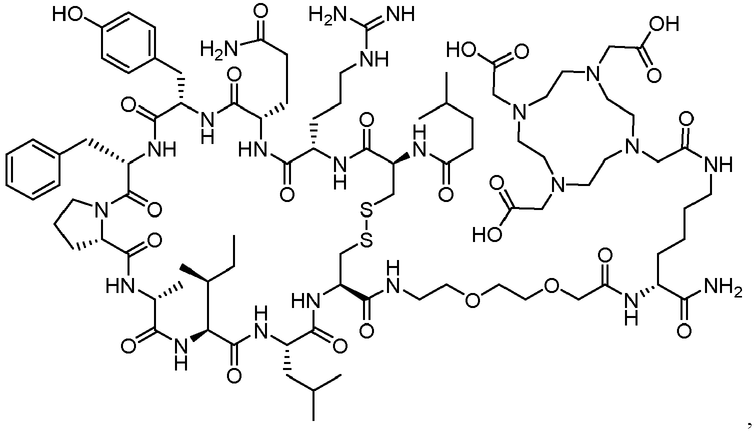
compound Pent-[Cys-Cit-Lys(DOTA)-Tyr-Ocf-Pro-Hyp-Ile-Nva-Cys]-NH₂ (UPAR-450) of the following formula 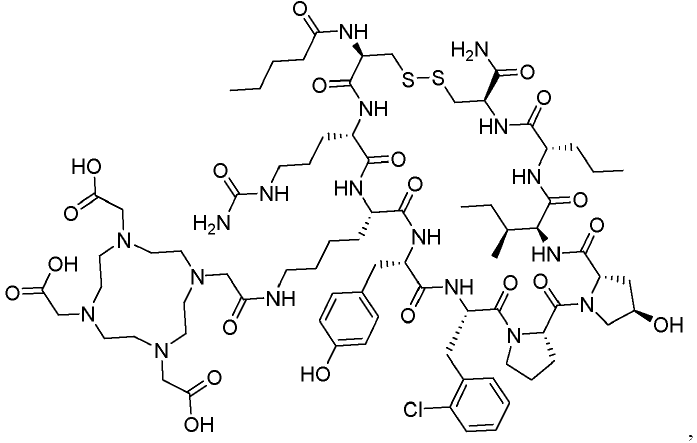
compound iHex-[Cys-Cit-Gln-Tyr-Phe-Pro-ala-Ile-Abu-Cys]-NH₂ (UPAR-451) of the following formula 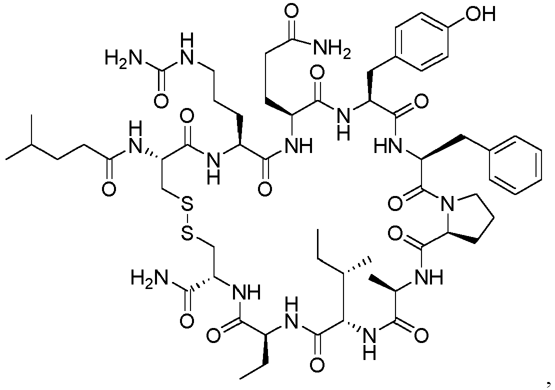
compound iHex-[Cys-Cit-Lys(DOTA)-Tyr-Phe-Pro-Aib-Ile-Leu-Cys]-NH₂ (UPAR-452) of the following formula 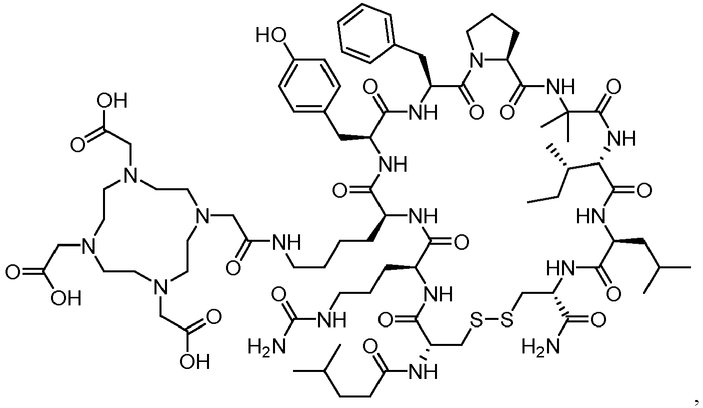
compound iHex-[Cys-Cit-Gln-Tyr-Phe-Pro-ala-Cbg-Leu-Cys]-NHz (UPAR-453) of the following formula 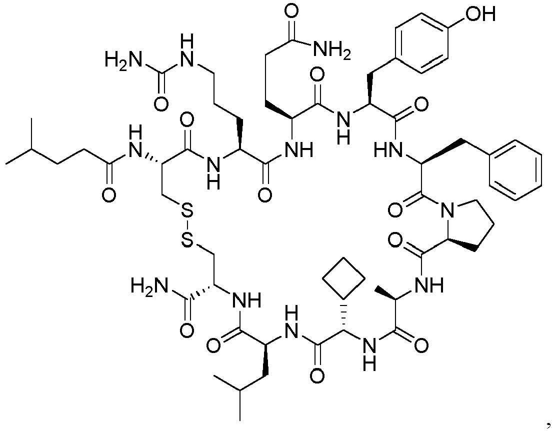
compound Pent-[Cys-Tyr-Gln-Tyr-Phe-Pro-Hyp-Ile-Nva-Cys]-NHz (UPAR-454) of the following formula 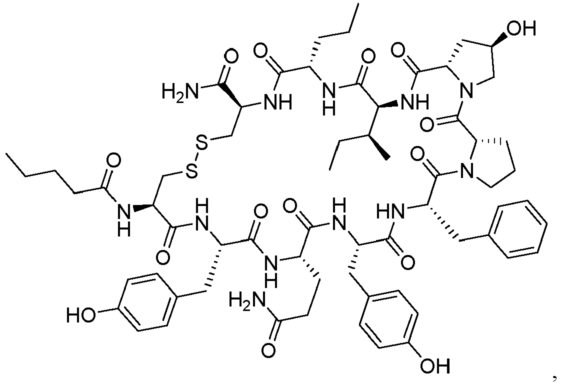
compound Butoc-[Cys-Thr-Dap(DOTA)-Tyr-Phe-4Cfp-Hyp-Ile-Nva-Cys]-NH₂ (UPAR-455) of the following formula 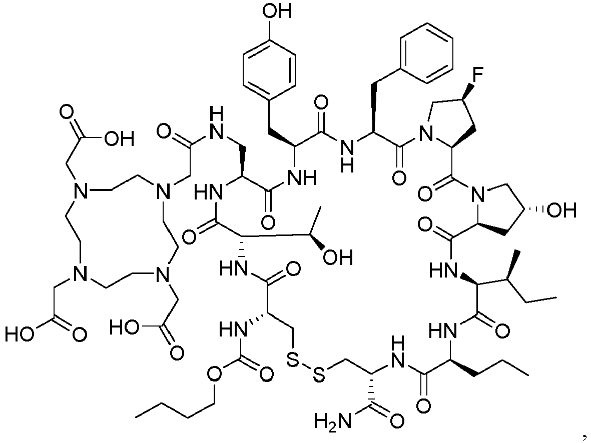
compound Butoc-[Cys-Thr-Dab(DOTA)-Dopa-Phe-4Cfp-Hyp-Ile-Nva-Cys]-NH₂ (UPAR-456) of the following formula 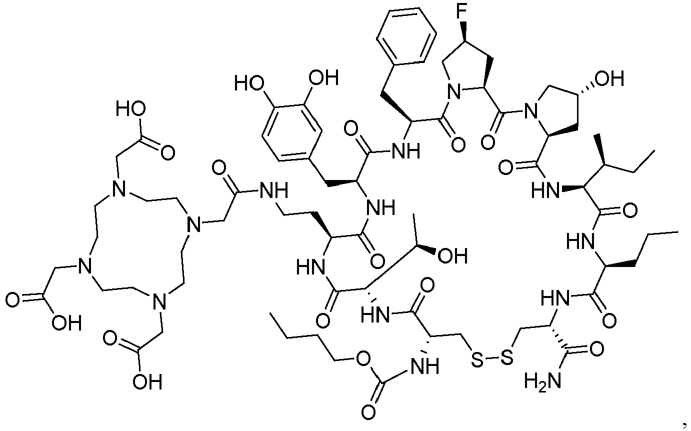
compound iHex-[Cys-Arg-Gln-Tyr-Phe-Pro-Apc(GdDOTA)-Ile-Leu-Cys]-NH₂ (UPAR-457) of the following formula 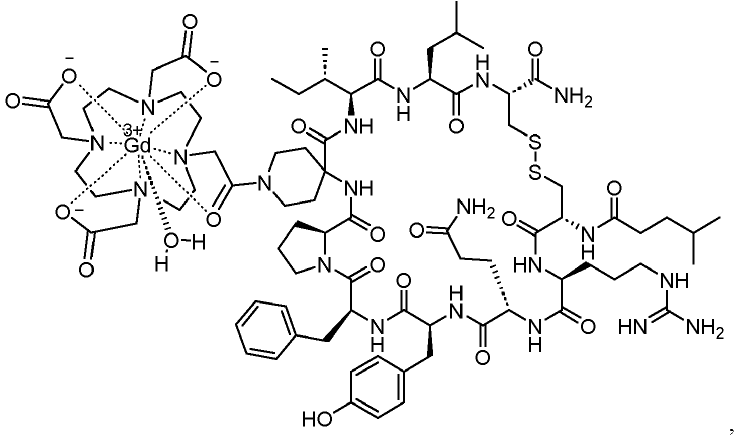
compound iHex-[Cys-Arg-Asp-Tyr-Phe-Pro-Hyp-Ile-Leu-Cys]-NHz (UPAR-458) of the following formula 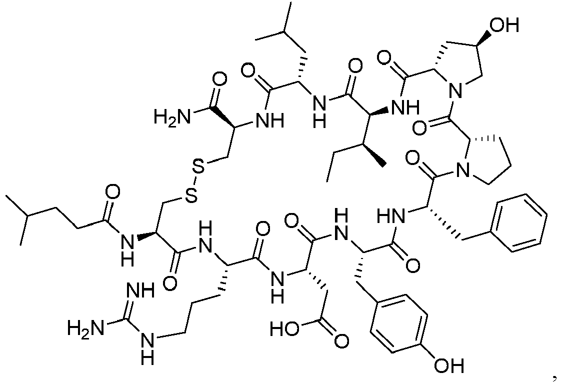
compound Butoc-[Cys-Thr-Lys(DOTA-asp)-Dopa-Phe-4Cfp-Hyp-Ile-Nva-Cys]-NH₂ (UPAR-459) of the following formula 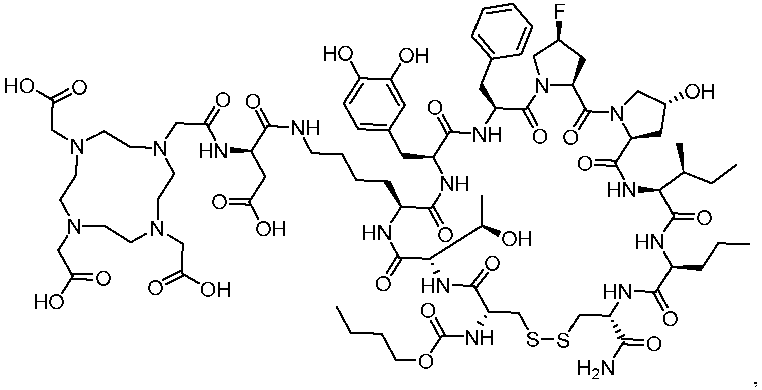
compound Succinyl-Nva-[Cys-Cit-Lys(GdDOTA)-Tyr-Phe-Pro-Hyp-Ile-Nva-Cys]-NH₂ (UPAR-460) of the following formula 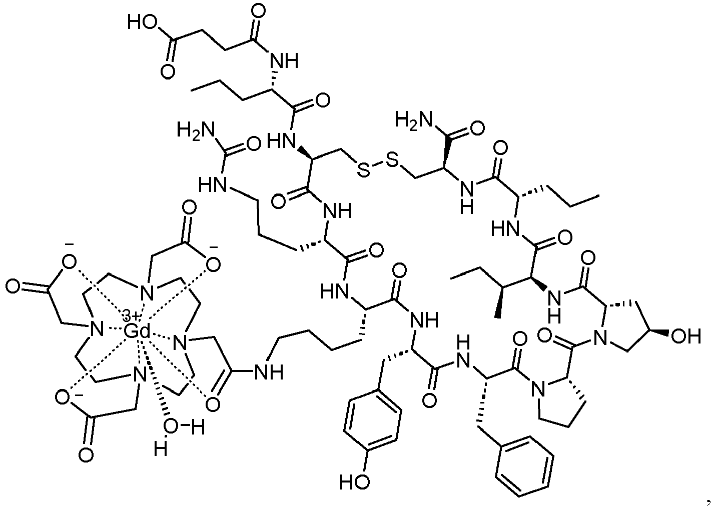
compound Pent-[Cys-Arg-Gln-Tyr-Phe-Pro-Hyp-Hfe-Nva-Cys]-NHz (UPAR-461) of the following formula 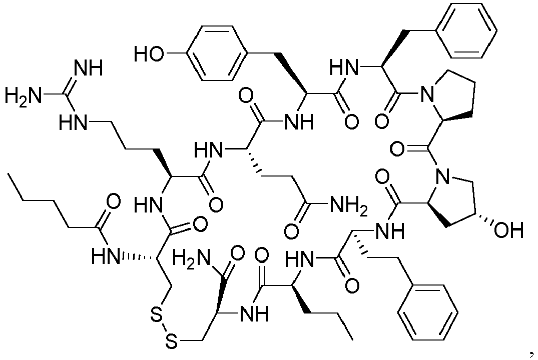
compound H-NEtg-[Cys-Cit-Gln-Tyr-Phe-Pro-ala-Ile-Leu-Cys]-NH₂ (UPAR-462) of the following formula 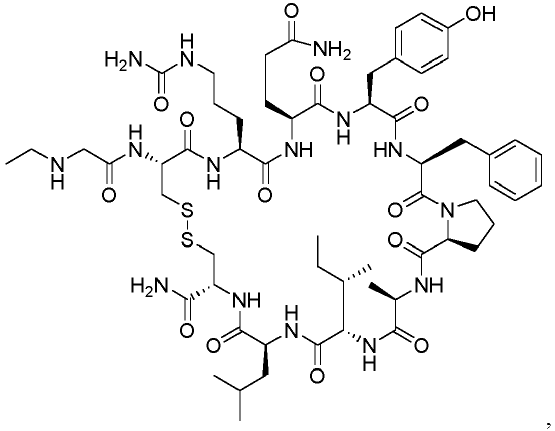
compound iHex-[Cys-Cit-Lys(GdDOTA)-Tyr-Phe-Pro-ala-Ile-Leu-Cys]-NH₂ (UPAR-463) of the following formula 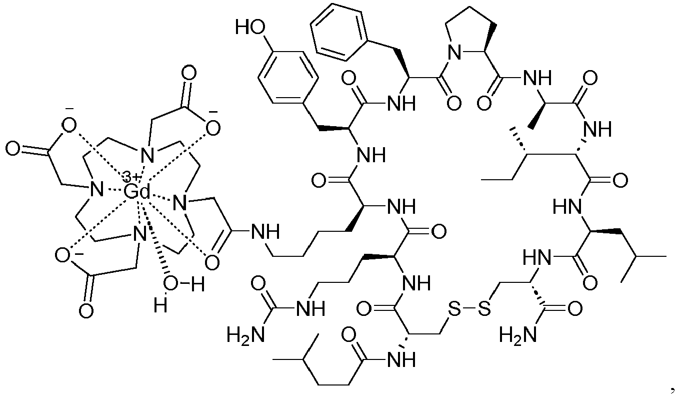
compound nBuCAyl-[Cys-Cit-Lys(LuDOTA)-Tyr-Phe-Pro-Hyp-Ile-Nva-Cys]-NH₂ (UPAR-464) of the following formula 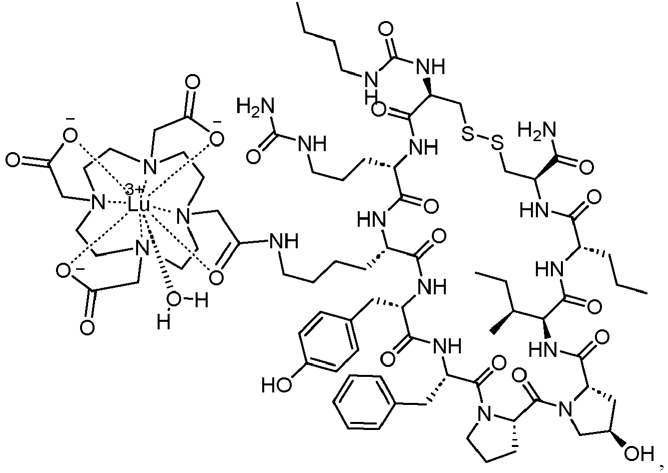
compound Pent-[Cys-Arg-Gln-Tyr-Phe-Pro-tyr-Ile-Nva-Cys]-NHz (UPAR-465) of the following formula 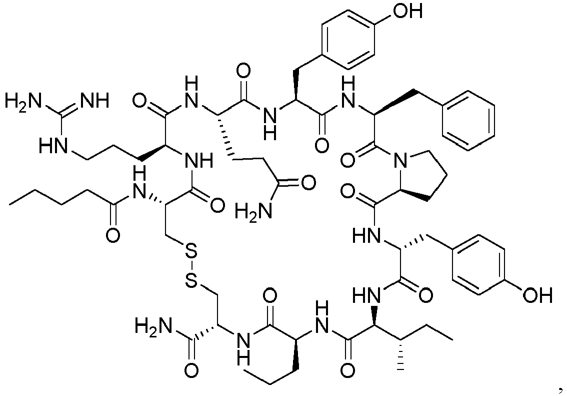
compound iHex-[Cys-Lys(DOTA)-Gln-Tyr-Phe-Pro-Pro-Ile-Leu-Cys]-NH₂ (UPAR-466) of the following formula 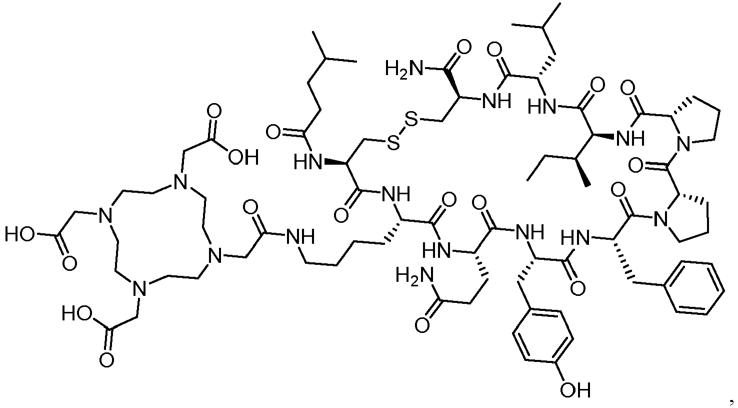
compound iHex-[Cys-arg-Gln-Tyr-Phe-Pro-ala-Ile-Leu-Cys]-NHz (UPAR-467) of the following formula 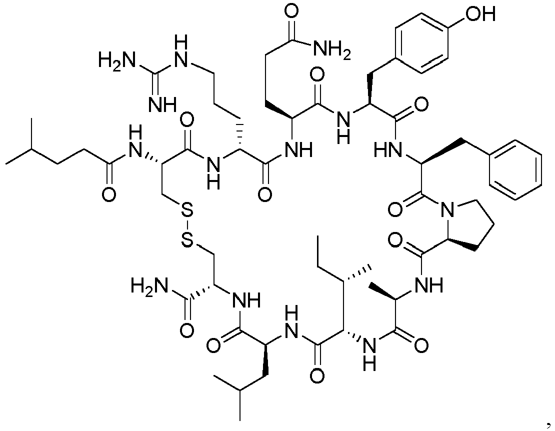
compound iHex-[Cys-Aib-Gln-Tyr-Phe-Pro-ala-Ile-Leu-Cys]-NHz (UPAR-468) of the following formula 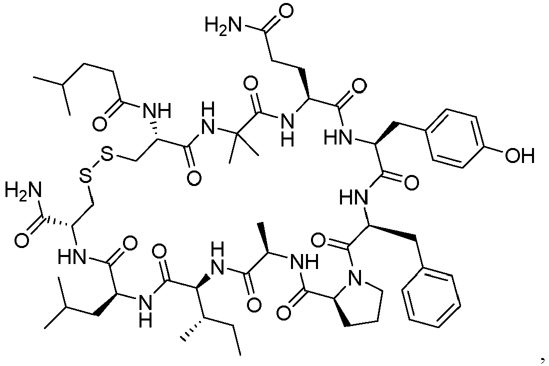
compound iHex-[Cys-Cit-Gln-Tyr-Phe-Pro-Pip-Ile-Leu-Cys]-NHz (UPAR-469) of the following formula 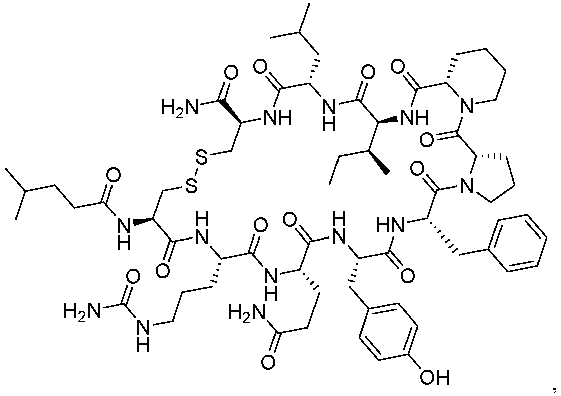
compound Pent-[Cys-Arg-Gln-Tyr-Phe-Nmv-Hyp-Ile-Nva-Cys]-NH₂ (UPAR-470) of the following formula 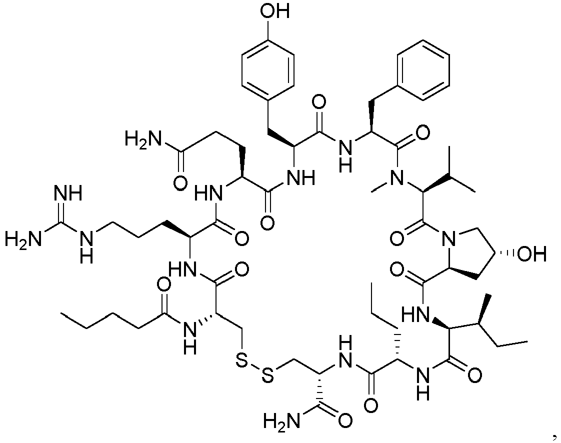
compound Pent-[Cys-Glu-Lys(DOTA)-Tyr-Phe-Pro-Hyp-Ile-Nva-Cys]-NH₂ (UPAR-471) of the following formula 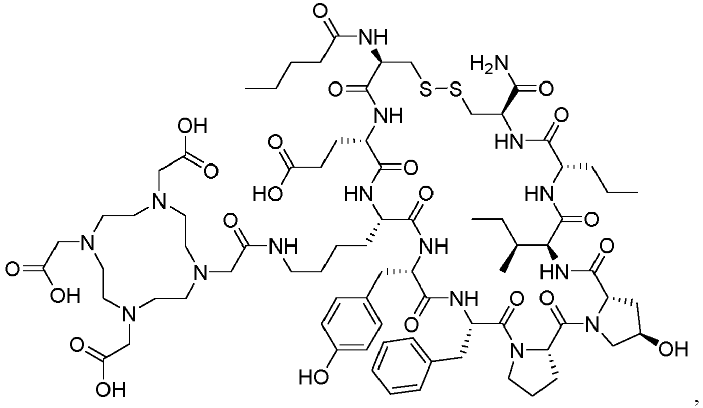
compound Pent-[Cys-Cit-Lys(DOTA)-Tyr-Phe-Pro-Pro-Ile-Nva-Cys]-NH₂ (UPAR-473) of the following formula 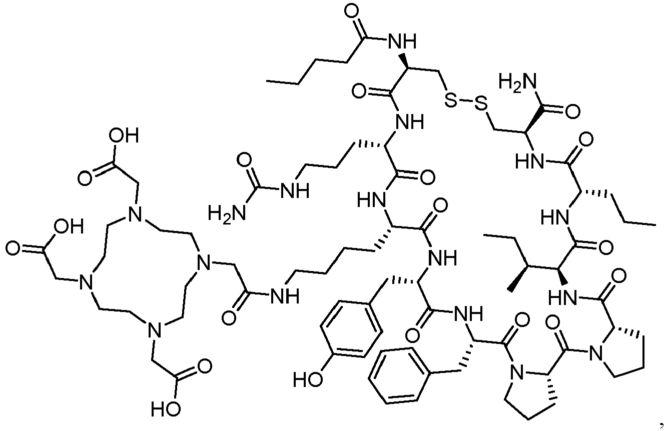
compound iHex-[Cys-Cit-Gln-Tyr-Phe-Pro-ala-Ile-Cha-Cys]-NHz (UPAR-474) of the following formula 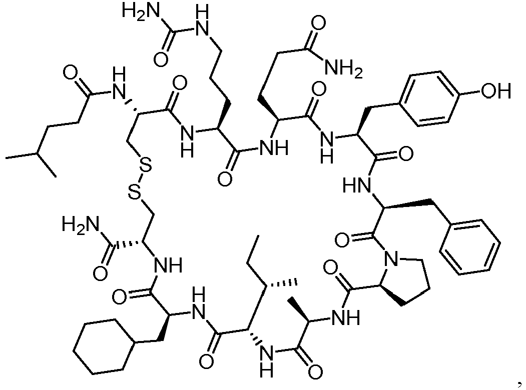
compound Hex-[Cys-Cit-Gln-Tyr-Phe-Pro-ala-Ile-Leu-Cys]-NHz (UPAR-475) of the following formula 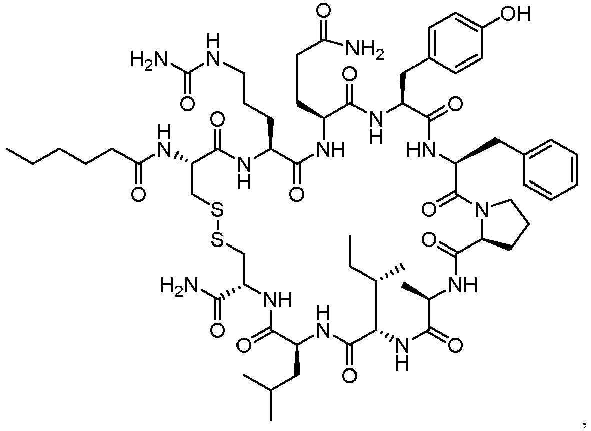
compound Butoc-[Cys-Cit-Lys(DOTA)-Tyr-Pcf-4Cfp-Hyp-Chg-Nva-Cys]-NH₂ (UPAR-476) of the following formula 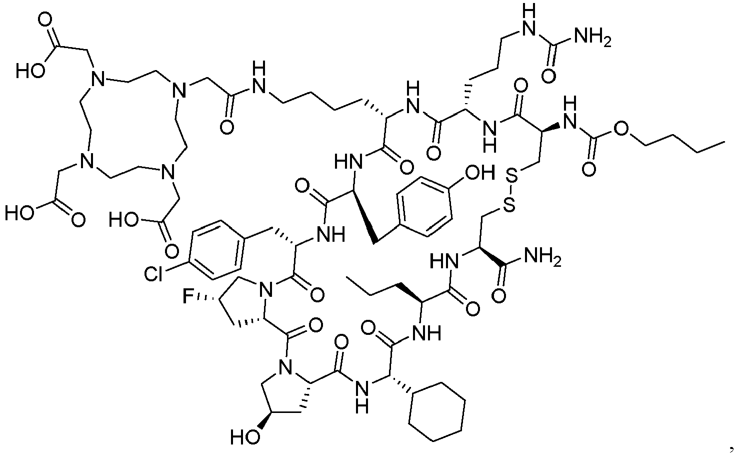
compound iHex-[Cys-Cit-Gln-Tyr-Phe-Pro-ala-Ile-Npg-Cys]-NHz (UPAR-477) of the following formula 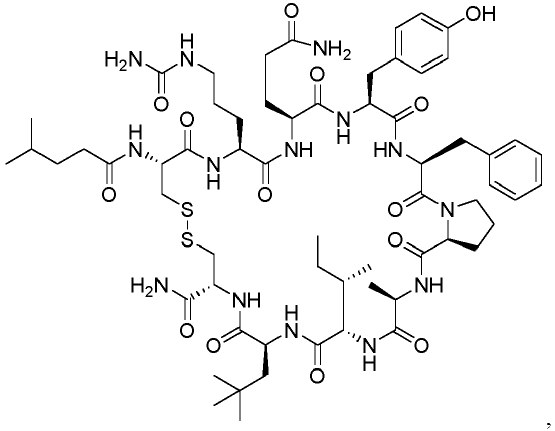
compound Pent-[Cys-Arg-Gln-Amf-Phe-Pro-Hyp-Ile-Nva-Cys]-NH₂ (UPAR-478) of the following formula 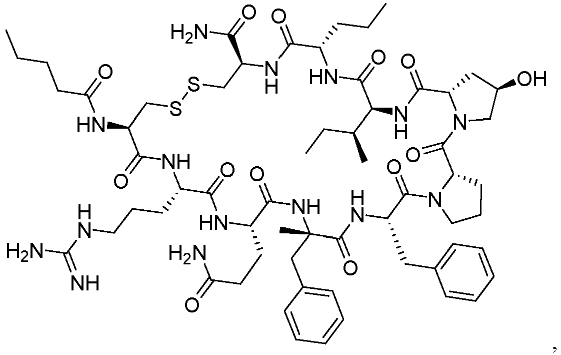
compound Butoc-[Cys-Thr-Lys(AF488-Ttds)-Dopa-Phe-4Cfp-Hyp-Ile-Nva-Cys]-NH₂ (UPAR-479) of the following formula 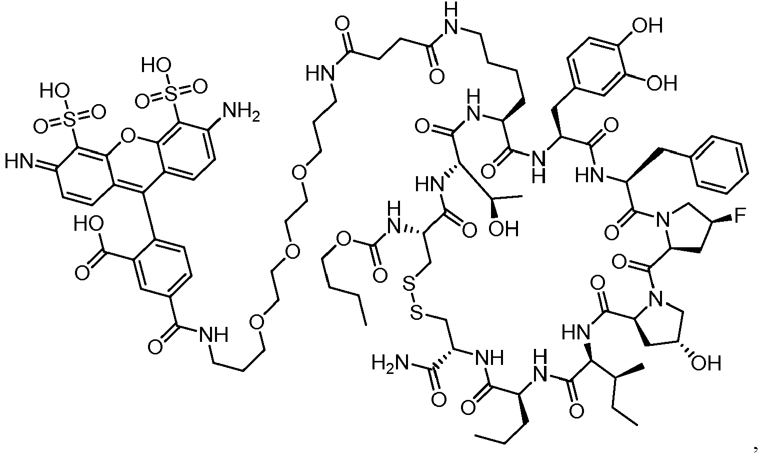
compound Pent-[Cys-Arg-Gln-Tyr-Phe-Pro-Hyp-Ile-Nml-Cys]-NHz (UPAR-480) of the following formula 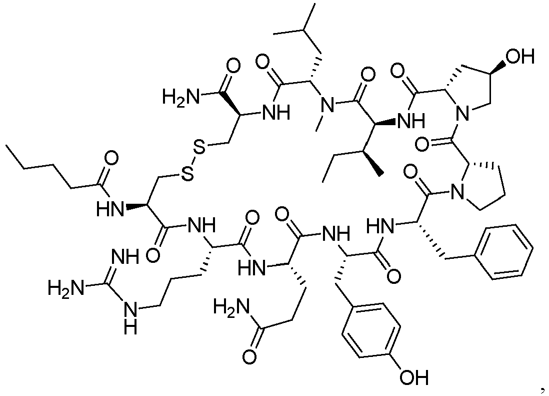
compound Pent-[Cys-Arg-Gln-Tyr-Phe-Pro-Hyp-Ile-Nva-AET] (UPAR-481) of the following formula 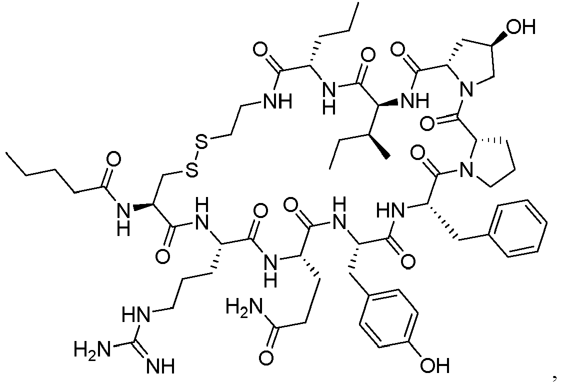
compound Ac-Egz-[Cys-Arg-Gln-Tyr-Phe-Pro-Hyp-Ile-Nva-Cys]-NH₂ (UPAR-482) of the following formula 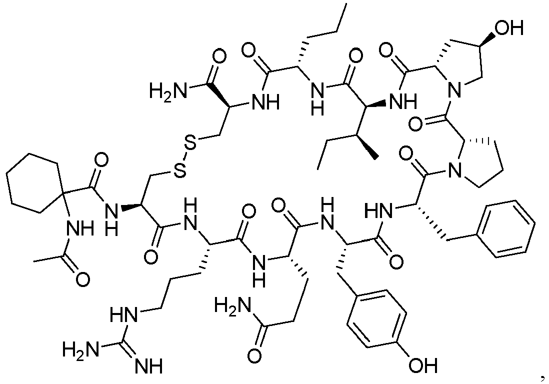
compound Ac-Nml-[Cys-Arg-Gln-Tyr-Phe-Pro-Hyp-Ile-Nva-Cys]-NH₂ (UPAR-483) of the following formula 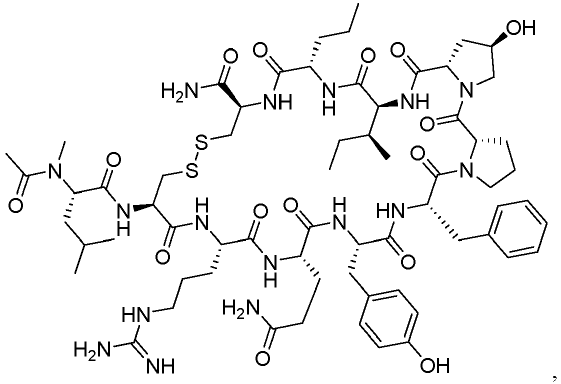
compound Ac-Hfe-[Cys-Arg-Gln-Tyr-Phe-Pro-Hyp-Ile-Nva-Cys]-NHz (UPAR-484) of the following formula 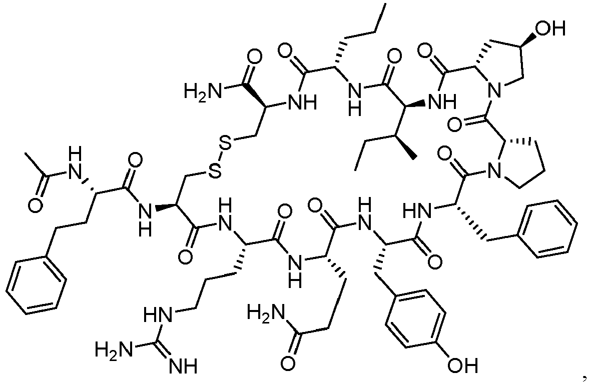
compound Ac-Aml-[Cys-Arg-Gln-Tyr-Phe-Pro-Hyp-Ile-Nva-Cys]-NH₂ (UPAR-485) of the following formula 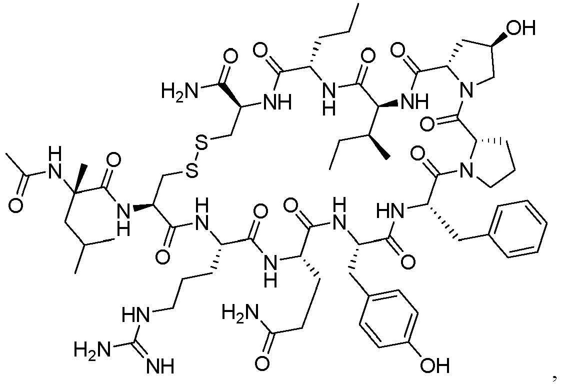
compound Ac-Phg-[Cys-Arg-Gln-Tyr-Phe-Pro-Hyp-Ile-Nva-Cys]-NHz (UPAR-486) of the following formula 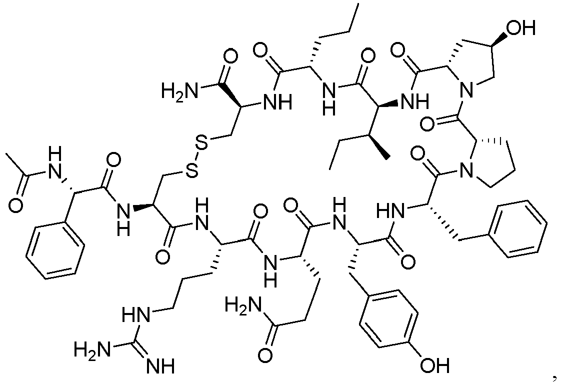
compound Me-Nml-[Cys-Arg-Gln-Tyr-Phe-Pro-Hyp-Ile-Nva-Cys]-NH₂ (UPAR-487) of the following formula 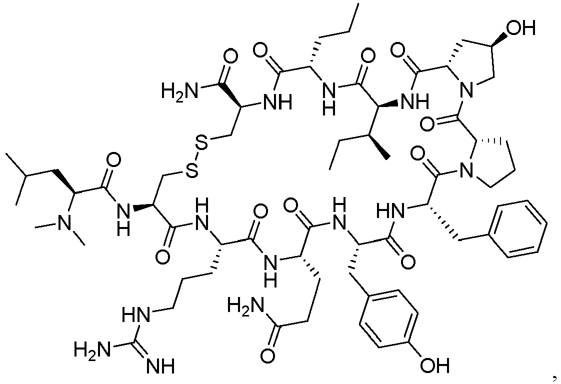
compound Ac-Phe-[Cys-Arg-Gln-Tyr-Phe-Pro-Hyp-Ile-Nva-Cys]-NHz (UPAR-488) of the following formula 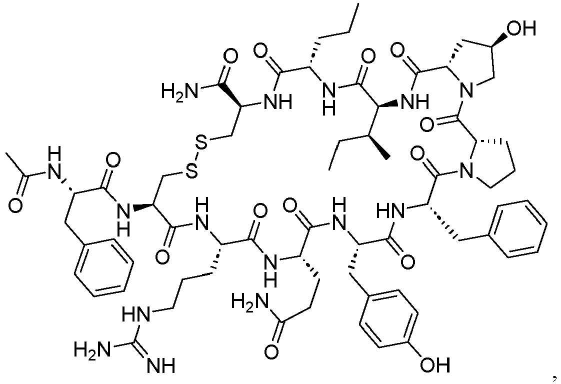
compound Butoc-[Cys-Thr-Orn(GaDOTA)-Pcnf-Phe-4Cfp-Hyp-Ile-Nva-Cys]-NH₂ (UPAR-489) of the following formula 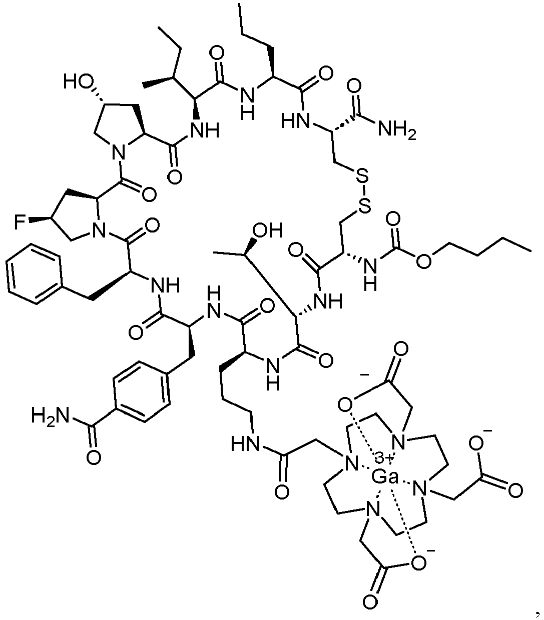
compound Butoc-[Cys-Thr-Orn(InDOTA)-Pcnf-Phe-4Cfp-Hyp-Ile-Nva-Cys]-NH₂ (UPAR-490) of the following formula 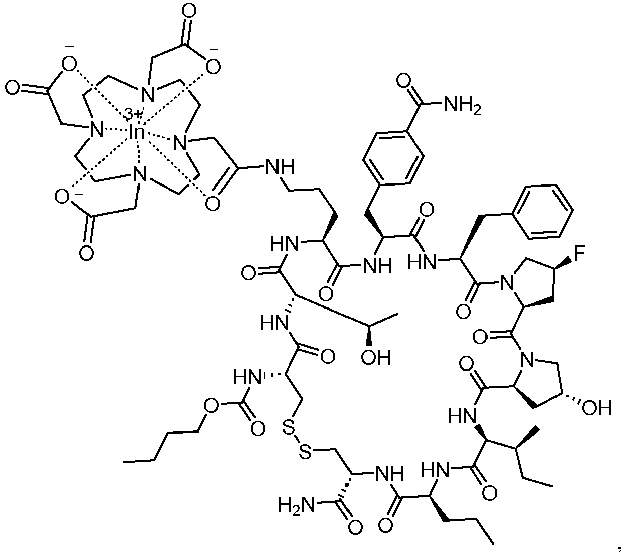
compound Butoc-[Cys-Phe-4Cfp-Hyp-Ile-Nva-Cys]-NH₂ (UPAR-491) of the following formula

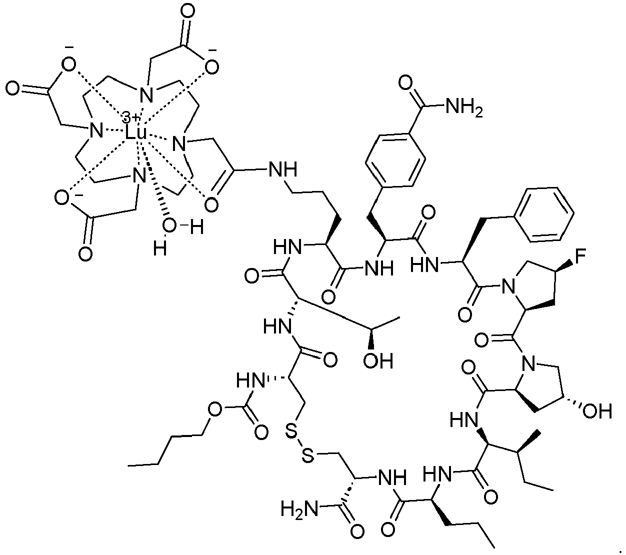

### Example 38: FACS Binding Assay

In order to determine binding of compounds according to the present invention to uPAR-expressing cells, a competitive FACS binding assay was established.

Cell surface uPAR-expressing HEC-265 human endometrial adenocarcinoma cells (Japanese Collection of Research Bioresources, #JCRB1142) were cultured in MEM Eagle with stable glutamine (Pan Biotech, #P04-09500) including 15% fetal bovine serum (Pan Biotech, #P30-3306) and 100 U/ml penicillin and 100 µg/mL streptomycin (Pan Biotech, #P06-07100). Cells were detached with Cell Dissociation Buffer (Thermo Scientific, #13151014) and washed in FACS buffer (PBS (Pan Biotech, #P04-36500) including 1% BSA (SeraCare, #AP-4510-80). Cells were diluted in FACS buffer to a final concentration of 500.000 cells per ml and 200 µl of the cell suspension are transferred to a u-shaped non-binding 96-well plate (Greiner Bio-One, #650901).

For IC₅₀ determination, cells were incubated with 10 nM Bio--Ttds--Ttds--Ttds--Ttds-D-Cha-FsrYLWS-NH2) in the presence of increasing concentrations of non-labeled test compounds at 4°C for 1 hour. Cells were washed twice in FACS buffer. An additional incubation step with 1 µg/mL APC-Streptavidin (Miltenyi; #130-106-791) in 50 µL FACS buffer for 30 minutes on ice was performed followed by two washes with FACS buffer. Cells were resuspended in 200 µl FACS buffer and analyzed in an Attune NxT flow cytometer. Median fluorescence intensities (APC channel) were calculated by Attune NxT software and plotted against peptide concentrations. Four parameter logistic (4PL) curve fitting and pIC₅₀ calculations were performed using ActivityBase software. The results of this assay as subject to Example 38 for each compound according to the present invention are presented in Table 12 (following Example 39). pIC₅₀ category A stands for pIC₅₀ values ≥8.0, category B for pIC₅₀ values between 7.5 and 7.9, category C for pIC₅₀ values between 7.0 and 7.4 and category D for pIC₅₀ values ≤ 6.9.

### Example 39: Surface plasmon resonance assay

Surface plasmon resonance (SPR) is an optical technique used to measure molecular interactions in real time. SPR occur when plane-polarized light hits a metal film under total internal reflection conditions. SPR signal is directly dependent on the refractive index of the medium on the sensor chip. In an SPR experiment, one molecule (i.e. uPAR) is immobilized on a sensor chip and binding to a second molecule (i.e. test compounds) is measured under flow. The binding of the compound on the surface changes the refractive index. In responses the reflection angle changes as compounds bind and dissociate on the immobilized protein on the surface. This response is measured in resonance units (RU). The RUs are proportional to the number of molecules bound to the surface. The RUs are recorded and displayed as a sensorgram in real time. Kinetic binding constants and equilibrium binding constants can be derived from the sensorgrams.

Surface plasmon resonance (SPR) studies were performed using a Biacore^{™} T200 or S200 SPR system (Cytiva, formerly GE Life Sciences) to assess binding of compounds to recombinant uPAR protein.

In this example recombinant protein comprising the extracellular domains of human uPAR (uPAR-Fc, R&D Systems, CAT 10378-UK-100, comprising amino acids Leu23-Arg303 according to UniProt entry Q03405.1 and Fc region of human IgG1 at the C-terminus) was immobilized on a Protein A-Chip (Cytiva, CAT 29127555) according to the supplier's instructions. uPAR-Fc was diluted in Running Buffer (10 mM HEPES, 150 mM NaCl, 50 µM EDTA, 0.1% DMSO, 0.05% Tween-20, pH 7.4) to a final concentration of 100 nM and then flushed over the Protein A-Chip to immobilized ~2000 RUs.

Stock solutions of test compounds were prepared by dissolving each compound in DMSO. DMSO stock solution were diluted 1: 1000 in Running Buffer without DMSO. Further sequential dilutions were made with Running Buffer containing 0.1% DMSO. SPR binding analyses were performed in Single Cycle Kinetic (SCK) mode at 25°C. Flow cell without immobilized uPAR-Fc served as reference flow cell. The chip was regenerated with 10 mM Glycine, pH 1,5 to remove the uPAR-Fc from the chip surface after each SCK run. After every three SCK measurements, a blank run with Running Buffer instead of test compound was included to correct for baseline drifts (double reference substraction method). Table 11 describes the protocol steps for SCK measurements on uPAR-Fc.

**Table 11: SPR protocol steps for SCK with uPAR-Fc immobilized on a Protein A-Chip.**

| **Step** | **Injected solution** | **Contact time** | **Flow rate** |
|---|---|---|---|
| Startup cycle as a triple run: | Running Buffer | 60 s | 30 µL/min |
| Washing & surface regeneration | Regeneration Solution | 5 s | |
| Binding target protein uPAR-Fc | 100 nM uPAR-Fc | 150 s | 5 µL/min |
| Washing (removal of unbound Target) | Running Buffer | 60 s | 30 µL/min |
| Washing (stabilization of baseline) | Running Buffer | 500 s | 30 µL/min |
| 1. Binding kinetics of test compound | Lowest compound concentration (2 - 8 - 40 nM) | 120 s | 30 µL/min |
| 2. Binding kinetics of test compound | Intermediated compound concentration (10 - 40 - 200 nM) | 120 s | 30 µL/min |
| 3. Binding kinetics of test compound | Highest compound concentration 50 - 200 - 1000 nM) | 120 s | 30 µL/min |
| Dissociation cycle | Running Buffer | 1200 s | 30 µL/min |
| Regeneration 2x | 10 mM Glycine, pH 1,5 | 60 s | 5 µL/min |

For each test compound, SPR raw data sensorgrams were recorded using the Biacore^{™} T200 or S200 control software. Sensorgrams were processed and analyzed with the Biacore^{™} Insight Evaluation software. The signal of the reference flow cell (without target) was subtracted from the active flow cell signal (immobilized target) to obtain a reference subtracted sensorgram. The reference subtracted sensorgram for each test compound was further corrected for baseline drift by subtracting the sensorgram of a reference subtracted blank run (Running Buffer only). The dissociation constant (K_{D}) was calculated from the double reference subtracted sensorgrams using the 1:1 Langmuir binding model. The pK_{D} value (negative decadic logarithm of dissociation constant) was calculated using Microsoft Excel.

The results of this assay for a selection of compounds according to the present invention are presented in Table 12. pK_{D} category A stands for pK_{D} values ≥ 8.8, category B for pK_{D} values from ≥ 8.0 to 8.8, category C for pK_{D} values from 7.0 to 8.0, and category D for pK_{D} values ≤ 7.0.

**Table 12: Compound ID, sequence, synthesis (described in Examples 1-37), exact calculated mass, LC/TOF-MS exact mass found, retention time (t_{R}) in minutes as determined by HPLC, pIC₅₀ category of FACS binding, and pKD category for uPAR SPR activity assay**

| **ID** | **Sequence** | **Synthesis** | **Calc. Mass** | **Found Mass** | **t_{R} / min** | **pIC50 CAT** | **pK_{D} CAT** |
|---|---|---|---|---|---|---|---|
| UPAR-127 (SEQ ID NO: 2) | Butoc-[Cys-Thr-Lys(DOTA)-Tyr-Phe-4Cfp-Hyp-Ile-Nva-Cys]-Bal-OH | IIf | 1758.801 | 1758.799 | 6.3 | A | B |
| UPAR-128 (SEQ ID NO: 3 | Butoc-[Cys-Thr-Lys(DOTA)-Tyr-Phe-Hyp-Hyp-Ile-Nva-Cys]-NH₂ | IId | 1684.784 | 1684.780 | 6.4 | A | A |
| UPAR-129 (SEQ ID NO: 4) | Butoc-[Cys-Thr-Lys(DOTA)-Tyr-Phe-4Cfp-Hyp-Ile-Dmdp-Cys]-NH₂ | VIc | 1701.791 | 1701.792 | 4.9 | B | C |
| UPAR-130 (SEQ ID NO: 5) | iHex-[Cys-lys(DOTA)-Gln-Tyr-Phe-Pro-ala-Ile-Leu-Cys]-NH₂ | IIa | 1665.826 | 1665.827 | 7.6 | D | D |
| UPAR-131 (SEQ ID NO: 6) | iHex-[Cys-Cit-Gln-Tyr-Phe-Pro-ala-Ile-Leu-Cys]-OH | IIIa | 1309.620 | 1309.725 | 7.3 | D | D |
| UPAR-132 (SEQ ID NO: 7) | Pent-[Cys-Cit-Lys(GdDOTA)-Tyr-Ocf-Pro-Hyp-Ile-Nva-Cys]-NH₂ | IX | 1891.689 | 1891.688 | 8.2 | A | A |
| UPAR-133 (SEQ ID NO: 8) | Ac-asp-Leu-[Cys-Arg-Nle-Tyr-Phe-Pro-Ala-Ile-Leu-Cys]-NH₂ | Ic | 1464.726 | 1464.744 | 7.5 | A | A |
| UPAR-134 (SEQ ID NO: 9) | Butoc-[Cys-Thr-Lys(DOTA)-Tyr-Thi-Pro-Hyp-Ile-Nva-Cys]-NH₂ | IId | 1674.746 | 1674.743 | 6.8 | A | A |
| UPAR-135 (SEQ ID NO: 10) | Pent-[Cys-Thr-Lys(DOTA)-Tyr-Phe-Hyp-Hyp-Ile-Nva-Cys]-NH₂ | IIa | 1668.789 | 1668.779 | 6.3 | A | A |
| UPAR-136 (SEQ ID NO: 11) | iHex-[Cys-Cit-Gln-Tyr-Phe-Pro-ala-Nva-Leu-Cys]-NH₂ | Ia | 1294.620 | 1294.662 | 7.2 | A | B |
| UPAR-137 (SEQ ID NO: 12) | Ac-Asp-Leu-[Cys-Arg-Nle-Tyr-Phe-Pro-Ala-Ile-leu-Cys]-NH₂ | Ic | 1464.726 | 1464.728 | 7.2 | B | B |
| UPAR-138 (SEQ ID NO: 13) | Pent-[Cys-Cit-Lys(DOTA)-Mpa-Phe-Pro-Hyp-Ile-Nva-Cys]-NH₂ | IIa | 1693.832 | 1693.828 | 6.2 | A | A |
| UPAR-139 (SEQ ID NO: 14) | But-[Cys-Cit-Gln-Tyr-Phe-Pro-ala-Ile-Leu-Cys]-NH₂ | Ia | 1280.605 | 1280.602 | 7.4 | B | B |
| UPAR-140 (SEQ ID NO: 15) | Hex-Lys(DOTA)-Leu-[Cys-Arg-Met-Tyr-Phe-Pro-Ala-Ile-Leu-Cys]-NH₂ | IIa | 1937.993 | 1938.001 | 7.8 | A | A |
| UPAR-141 (SEQ ID NO: 16) | Butoc-[Cys-Thr-Lys(DOTA)-Tyr-Phe-4Cfp-Hyp-Ile-Nva-Cys -Asp-NH₂ | IId | 1801.807 | 1801.802 | 6.2 | C | C |
| UPAR-142 (SEQ ID NO: 17) | DOTA-Leu-[Nmc-Cit-Gln-Tyr-Phe-Pro-Hyp-Ile-Nva-Cys]-NH₂ | Ib | 1751.837 | 1751.845 | 6.5 | D | C |
| UPAR-143 (SEQ ID NO: 18) | Hex-Thr-Leu-[Cys-Glu-Tyr-Tyr-Phe-Pro-Ala-Ile-Leu-Cys]-NH₂ | Ia | 1529.730 | 1529.838 | 8.7 | A | A |
| UPAR-144 (SEQ ID NO: 19) | iHex-[Cys-Cit-Gln-Dopa-Phe-Pro-ala-Ile-Leu-Cys]-NH₂ | Ia | 1324.631 | 1324.628 | 7.7 | A | A |
| UPAR-145 (SEQ ID NO: 20) | iHex-[Cys-Cit-Gln-Tyr-Phe-Pro-Lys(DOTA)-Ile-Leu-Cys]-NH₂ | IIa | 1751.874 | 1751.873 | 7.7 | A | A |
| UPAR-146 (SEQ ID NO: 21) | Pent-[Cys-Cit-Gln-Tyr-Phe-Pro-Hyp-Ile-Nva-Cys]-NHEt | IIId | 1350.646 | 1350.641 | 6.4 | B | B |
| UPAR-147 (SEQ ID NO: 22) | iHex-[Cys-Cit-Gln-Tyr-2Ni-Pro-ala-Ile-Leu-Cys]-NH₂ | Ia | 1358.652 | 1358.650 | 8.4 | B | C |
| UPAR-148 (SEQ ID NO: 23) | iHex-[Cys-Arg-Lys(InDOTA)-Tyr-Phe-Pro-ala-Ile-Leu-Cys]-NH₂ | IX | 1803.749 | 1803.744 | 7.8 | A | A |
| UPAR-149 (SEQ ID NO: 24) | Pent-[Cys-Cit-Lys(DOTA)-Guf-Phe-Pro-Hyp-Ile-Nva-Cys]-NH₂ | IIa | 1749.869 | 1749.861 | 6.6 | A | A |
| UPAR-150 (SEQ ID NO: 25) | Hex-Asp-Leu-[Cys-Arg-Met-Tyr-Phe-Pro-Ala-Ile-Leu-Cys]-Ttds-Lys(DOTA)-NH₂ | IIa | 2355.204 | 2355.207 | 7.9 | C | B |
| UPAR-151 (SEQ ID NO: 26) | Pent-[Cys-Cit-Lys(LuDOTA)-Tyr-Phe-Pro-nma-Ile-Nva-Cys]-NH₂ | IX | 1852.754 | 1852.754 | 7.8 | A | A |
| UPAR-152 (SEQ ID NO: 27) | iHex-[Cys-Arg-Gln-Tyr-Phe-Pro-Apc(DOTA)-Ile-Leu-Cys]-NH₂ | IIa | 1748.874 | 1748.974 | 7.5 | A | A |
| UPAR-153 (SEQ ID NO: 28) | Pent-[Cys-KMe3-Lys(DOTA)-Tyr-Phe-Pro-Hyp-Ile-Nva-Cys]-NH₂ | IIa | 1721.889 | 1721.886 | 6.8 | A | A |
| UPAR-154 (SEQ ID NO: 29) | Pent-[Cys-Arg-Gln-Tyr-Phe-Pro-Ser-Ile-Nva-Cys]-NH₂ | Ia | 1295.615 | 1295.619 | 7.0 | A | A |
| UPAR-155 (SEQ ID NO: 30) | Butoc-[Cys-Cit-Gln-Tyr-Phe-Pro-ala-Ile-Leu-Cys]-NH₂ | IVc | 1310.615 | 1310.614 | 7.9 | A | A |
| UPAR-156 (SEQ ID NO: 31) | Pent-[Cys-Arg-Gln-Tyr-Phe-Pro-Hyp-Ile-Aml-Cys]-NH₂ | Ia | 1349.662 | 1349.655 | 7.7 | B | B |
| UPAR-157 (SEQ ID NO: 32) | iHex-[Cys-Lys(DOTA)-Gln-Tyr-Phe-Pro-ala-Ile-Leu-Cys]-NH₂ | IIa | 1665.826 | 1665.826 | 7.5 | A | A |
| UPAR-159 (SEQ ID NO: 33) | iHex-[Cys-Cit-Gln-Ala-Phe-Pro-ala-Ile-Leu-Cys]-NH₂ | Ia | 1216.610 | 1216.661 | 7.0 | D | D |
| UPAR-160 (SEQ ID NO: 34) | iHex-[Cys-Cit-Arg-Tyr-Phe-Pro-ala-Ile-Leu-Cys]-NH₂ | Ia | 1336.678 | 1336.676 | 7.6 | A | A |
| UPAR-161 (SEQ ID NO: 35) | Pent-[Cys-Arg-Gln-Tyr-Phe-Pro-asp-Ile-Nva-Cys]-NH₂ | Ia | 1323.610 | 1323.611 | 6.7 | A | C |
| UPAR-162 (SEQ ID NO: 36) | Ac-Leu-[Cys-Arg-Lys(DOTA)-Tyr-Phe-Pro-ala-Ile-Leu-Cys]-NH₂ | IIa | 1750.890 | 1750.889 | 7.1 | A | A |
| UPAR-163 (SEQ ID NO: 37) | iHex-[Cys-Cit-Gln-Tyr-Phe-Tap-ala-Ile-Leu-Cys]-NH₂ | Ia | 1323.647 | 1323.691 | 7.0 | B | B |
| UPAR-164 (SEQ ID NO: 38) | iHex-[Cys-Cit-Gln-Tyr-Phe-Pro-ala-Ala-Leu-Cys]-NH₂ | Ia | 1266.589 | 1266.629 | 6.8 | D | D |
| UPAR-165 (SEQ ID NO: 39) | Butoc-[Cys-Thr-Lys(DOTA)-Mpa-Phe-4Cfp-Hyp-Ile-Nva-Cys]-NH₂ | IId | 1671.780 | 1671.777 | 5.4 | A | A |
| UPAR-166 (SEQ ID NO: 40) | Pent-[Cys-Cit-Lys(LaDOTA)-Tyr-Thi-Pro-Hyp-Ile-Nva-Cys]-NH₂ | IX | 1849.672 | 1849.671 | 7.6 | A | A |
| UPAR-167 (SEQ ID NO: 41) | iHex-[Cys-Har-Gln-Tyr-Phe-Pro-Hyp-Ile-Leu-Cys]-NH₂ | la | 1363.678 | 1363.676 | 7.9 | A | A |
| UPAR-168 (SEQ ID NO: 42) | iHex-[Cys-Ala-Gln-Tyr-Phe-Pro-ala-Ile-Leu-Cys]-NH₂ | la | 1222.588 | 1222.642 | 7.7 | A | A |
| UPAR-169 (SEQ ID NO: 43) | Butoc-[Cys-Cit-Lys(LuDOTA)-Tyr-Pcf-4Cfp-Hyp-Chg-Nva-Cys]-NH₂ | IX | 1974.711 | 1974.710 | 8.8 | A | A |
| UPAR-170 (SEQ ID NO: 44) | LuDOTA-Ahx-Leu-[Cys-Arg-Gln-Tyr-Phe-Pro-ala-Ile-Leu-Cys]-NH₂ | IX | 1993.844 | 1993.834 | 7.2 | A | ND |
| UPAR-171 (SEQ ID NO: 45) | Pent-[Cys-Arg-Gln-Tyr-Phe-Pro-Hyp-Ile-Phe-Cys]-NH₂ | Ia | 1369.631 | 1369.624 | 7.9 | B | B |
| UPAR-172 (SEQ ID NO: 46) | EtOPr-[Cys-Cit-Gln-Tyr-Phe-Pro-ala-Ile-Leu-Cys]-NH₂ | Ia | 1310.615 | 1310.610 | 7.3 | A | B |
| UPAR-173 (SEQ ID NO: 47) | Pent-[Cys-Cit-Lys(DOTA)-Tyr-Phe-Pro-Nmk-Ile-Nva-Cys]-NH₂ | IIa | 1737.895 | 1737.889 | 5.9 | D | D |
| UPAR-174 (SEQ ID NO: 48) | iHex-[Cys-Cit-Gln-Tyr-Phe-Pro-Hyp-Ile-Leu-Cys]-NH₂ | Ia | 1350.646 | 1350.692 | 8.0 | A | A |
| UPAR-176 (SEQ ID NO: 49) | iHex-[Cys-Arg-Lys(DOTA)-Tyr-Phe-Pro-ala-Ile-Leu-Cys]-NH₂ | IIa | 1693.868 | 1693.870 | 7.4 | A | A |
| UPAR-177 (SEQ ID NO: 50) | Pent-[Cys-Cit-Lys(DOTA)-Tyr-Phe-Pro-Nmg-Ile-Nva-Cys]-NH₂ | IIa | 1666.821 | 1666.824 | 6.7 | A | B |
| UPAR-178 (SEQ ID NO: 51) | iHex-[Cys-Cit-Gln-Tyr-Phe-4Tfp-ala-Ile-Leu-Cys]-NH₂ | Ia | 1326.626 | 1326.626 | 7.3 | A | B |
| UPAR-179 (SEQ ID NO: 52) | Pent-[Cys-Arg-Gln-Tyr-Phe-Pro-Hyp-Deg-Nva-Cys]-NH₂ | Ia | 1321.631 | 1321.619 | 6.9 | D | D |
| UPAR-180 (SEQ ID NO: 53) | iHex-[Cys-Cit-Gln-Tyr-Hfe-Pro-ala-Ile-Leu-Cys]-NH₂ | Ia | 1322.652 | 1322.652 | 8.0 | D | D |
| UPAR-181 (SEQ ID NO: 54) | Butoc-[Cys-Thr-Lys(DOTA-PPAc)-Tyr-Phe-4Cfp-Hyp-Ile-Nva-Cys]-NH₂ | IIg | 1812.859 | 1812.862 | 6.2 | A | A |
| UPAR-182 (SEQ ID NO: 55) | Pent-[Cys-Cit-Lys(InDOTA)-Tyr-Phe-Pro-Hyp-Ile-Nva-Cys]-NH₂ | IX | 1818.710 | 1818.710 | 7.7 | A | A |
| UPAR-183 (SEQ ID NO: 56) | Pent-[Cys-KMe3-Lys(LaDOTA)-Tyr-Phe-Pro-Hyp-Ile-Nva-Cys]-NH₂ | IX | 1857.780 | 1856.772 | 7.5 | A | A |
| UPAR-184 (SEQ ID NO: 57) | iHex-[Cys-Cit-Gln-Tyr-Phe-Pro-ala-Ile-Val-Cys]-NH₂ | Ia | 1294.620 | 1294.618 | 7.3 | A | B |
| UPAR-186 (SEQ ID NO: 58) | Pent-[Cys-Apc-Gln-Tyr-Phe-Pro-Hyp-Ile-Nva-Cys]-NH₂ | Ia | 1291.609 | 1291.599 | 7.5 | A | A |
| UPAR-187 (SEQ ID NO: 59) | iHex-[Cys-Cit-Gln-Tyr-Phe-Pro-ala-Cpa-Leu-Cys]-NH₂ | Ia | 1292.605 | 1292.602 | 7.3 | D | D |
| UPAR-188 (SEQ ID NO: 60) | iHex-[Cys-Cit-Gln-Mcy-Phe-Pro-ala-Ile-Leu-Cys]-NH₂ | IVe | 1342.597 | 1342.596 | 8.0 | A | A |
| UPAR-189 (SEQ ID NO: 61) | Ac-Asp-Leu- [Cys-Cit-Lys(DOTA)-Tyr-Phe-Pro-ala-Ile-Leu-Cys]-NH₂ | IIa | 1866.901 | 1867.054 | 7.2 | A | A |
| UPAR-190 (SEQ ID NO: 62) | Pent-[Cys-Arg-Gln-Tyr-Phe-Pro-Oic-Ile-Nva-Cys]-NH₂ | Ia | 1359.683 | 1359.677 | 8.2 | B | A |
| UPAR-191 (SEQ ID NO: 63) | Butoc-[Cys-Thr-Lys(InDOTA)-Dopa-Phe-4Cfp-Hyp-Ile-Nva-Cys]-NH₂ | IX | 1812.655 | 1812.645 | 6.1 | A | A |
| UPAR-192 (SEQ ID NO: 64) | Butoc-[Cys-Thr-Orn(DOTA)-Tyr-Phe-4Cfp-Hyp-Ile-Nva-Cys]-NH₂ | IId | 1672.764 | 1672.762 | 6.4 | A | A |
| UPAR-193 (SEQ ID NO: 65) | Oct-[Cys-Arg-Gln-Tyr-Phe-Pro-Hyp-Ile-Nva-Cys]-NH₂ | Ia | 1363.678 | 1363.684 | 8.4 | A | A |
| UPAR-194 (SEQ ID NO: 66) | Butoc-[Cys-Thr-Lys(DOTA-Kzw)-Dopa-Phe-4Cfp-Hyp-Ile-Nva-Cys]-NH₂ | VId | 1980.905 | 1980.908 | 6.9 | A | A |
| UPAR-195 (SEQ ID NO: 67) | Pent-[Cys-Arg-Gln-Leu-Phe-Pro-Hyp-Ile-Nva-Cys]-NH₂ | Ia | 1271.652 | 1271.646 | 8.1 | B | B |
| UPAR-196 (SEQ ID NO: 68) | Pent-[Cys-Arg-Gln-Phg-Phe-Pro-Hyp-Ile-Nva-Cys]-NH₂ | Ia | 1291.621 | 12691.609 | 7.6 | D | D |
| UPAR-197 (SEQ ID NO: 69) | Pent-[Cys-Arg-Gln-Tyr-Leu-Pro-Hyp-Ile-Nva-Cys]-NH₂ | Ia | 1287.647 | 1287.645 | 7.6 | B | B |
| UPAR-198 (SEQ ID NO: 70) | Ac-Asp-Leu-[Cys-Arg-Nle-tyr-Phe-Pro-Ala-Ile-Leu-Cys]-NH₂ | Ic | 1464.726 | 1464.730 | 7.3 | D | D |
| UPAR-199 (SEQ ID NO: 71) | Butoc-[Cys-Thr-Lys(DOTA-PPAc)-Dopa-Phe-4Cfp-Hyp-Ile-Nva-Cys]-NH₂ | IIg | 1828.854 | 1828.854 | 5.6 | A | A |
| UPAR-200 (SEQ ID NO: 72) | Pent-[Cys-Cit-Lys(DOTA-PEG 12)-Tyr-Phe-Pro-Hyp-Ile-Nva-Cys]-NH₂ | IIh | 2308.183 | 2308.175 | 7.7 | A | A |
| UPAR-201 (SEQ ID NO: 73) | iHex-[Cys-Cit-Gln-Tyr-Phe-Pro-ala-Tle-Leu-Cys]-NH₂ | Ia | 1308.636 | 1308.673 | 7.5 | C | C |
| UPAR-202 (SEQ ID NO: 74) | Hex-Ser-Leu-[Cys-Asp-Gln-Phe-Phe-Pro-Ala-Ile-Leu-Cys]-NH₂ | Ia | 1450.699 | 1450.808 | 9.1 | A | A |
| UPAR-203 (SEQ ID NO: 75) | Pent-[Cys-Arg-Gln-Tyr-Phg-Pro-Hyp-Ile-Nva-Cys]-NH₂ | Ia | 1307.616 | 1307.615 | 6.9 | D | C |
| UPAR-204 (SEQ ID NO: 76) | Pent-[Cys-Arg-Gln-Tyr-Phe-Pro-Hyp-Pcf-Nva-Cys]-NH₂ | Ia | 1389.577 | 1389.564 | 7.9 | A | A |
| UPAR-205 (SEQ ID NO: 77) | Pent-Ala-Cit-Gln-Tyr-Phe-Pro-Hyp-Ile-Nva-Smc-NH₂ | Va | 1306.674 | 1306.675 | 5.1 | D | D |
| UPAR-206 (SEQ ID NO: 78) | Pent-[Cys-Cit-Gln-Tyr-Phe-Pro-Hyp-Ile-Nva-Cys]-Gly-NH₂ | la | 1379.637 | 1379.635 | 6.0 | B | B |
| UPAR-207 (SEQ ID NO: 79) | Pent-[Cys-Cit-Lys(DOTA)-Tyr-Mpa-Pro-4Tfp-Ile-Nva-Cys]-NHz | IIa | 1711.823 | 1711.816 | 5.1 | A | B |
| UPAR-208 (SEQ ID NO: 80) | Cp-[Cys-Cit-Gln-Tyr-Phe-Pro-ala-Ile-Leu-Cys]-NH₂ | Ia | 1306.620 | 1306.615 | 7.8 | A | A |
| UPAR-209 (SEQ ID NO: 81) | Pent-[Cys-Cit-Lys(DOTA)-Fso-Phe-Pro-Hyp-Ile-Nva-Cys]-NH₂ | IIa | 1772.794 | 1772.786 | 6.7 | A | B |
| UPAR-210 (SEQ ID NO: 82) | PrCAyl-[Cys-Cit-Lys(DOTA)-Tyr-Phe-Pro-Hyp-Ile-Nva-Cys]-NH₂ | IVd | 1709.827 | 1709.824 | 7.1 | A | A |
| UPAR-211 (SEQ ID NO: 83) | Pent-[Cys-Arg-Gln-Tyr-Phe-nma-Hyp-Ile-Nva-Cys]-NH₂ | Ia | 1309.631 | 1309.629 | 6.4 | D | D |
| UPAR-212 (SEQ ID NO: 84) | PrOAc-[Cys-Cit-Gln-Tyr-Phe-Pro-ala-Ile-Leu-Cys]-NH₂ | Ia | 1310.615 | 1310.613 | 7.4 | B | C |
| UPAR-213 (SEQ ID NO: 85) | Pent-[Cys-Arg-Gln-Tyr-Phe-Pro-Hyp-Ile-Nva-Hcy]-NH₂ | Ia | 1335.647 | 1335.638 | 7.2 | A | B |
| UPAR-214 (SEQ ID NO: 86) | Pent-[Cys-Cit-Lys(DOTA)-Tyr-Phe-Pro-Chy-Ile-Nva-Cys]-NH₂ | IIa | 1708.832 | 1708.829 | 7.1 | A | A |
| UPAR-215 (SEQ ID NO: 87) | Pent-[Cys(meth)-Cit-Gln-Tyr-Phe-Pro-Hyp-Ile-Nva-Cys]-NH₂ | Vb | 1336.631 | 1336.626 | 5.8 | B | B |
| UPAR-216 (SEQ ID NO: 88) | DOTA-O2Oc-Leu-[Cys-ala-Gln-Tyr-Phe-Pro-ala-Ile-Leu-Cys]-NH₂ | Ib | 1768.853 | 1768.924 | 6.7 | D | D |
| UPAR-217 (SEQ ID NO: 89) | Pent-[Cys-Cit-Gln-Tyr-Phe-Pro-Hyp-Ile-Nva-Pen] - NH₂ | Ia | 1350.646 | 1350.648 | 5.9 | A | B |
| UPAR-218 (SEQ ID NO: 90) | Ac-Asp-Leu-[Cys-Arg-Nle-Tyr-Phe-pro-Ala-Ile-Leu-Cys]-NH₂ | Ic | 1464.726 | 1464.726 | 6.9 | D | D |
| UPAR-219 (SEQ ID NO: 91) | iHex-[Cys-Cit-Gln-Tyr-Phe-Pro-Apc(DOTA)-Ile-Leu-Cys]-NH₂ | IIa | 1749.858 | 1749.978 | 7.6 | A | B |
| UPAR-220 (SEQ ID NO: 92) | iHex-[Cys-Cit-Gln-Tyr-Phe-Pro-4Tfp-Ile-Leu-Cys]-NH₂ | Ia | 1352.642 | 1352.640 | 8.0 | A | A |
| UPAR-221 (SEQ ID NO: 93) | iHex-[Cys-Cit-Gln-Tyr-Ocf-Pro-ala-Ile-Leu-Cys]-NH₂ | Ia | 1342.597 | 1342.595 | 8.1 | A | A |
| UPAR-222 (SEQ ID NO: 94) | Pent-[Cys-Cit-Lys(DOTA)-Tyr-Ppa-Pro-Hyp-Ile-Nva-Cys]-NH₂ | IIa | 1709.827 | 1709.824 | 5.1 | A | B |
| UPAR-223 (SEQ ID NO: 95) | DOTA-Phe-[Cys-Arg-Gln-Tyr-Phe-Pro-Hyp-Ile-Nva-Cys]-NH₂ | Ib | 1770.822 | 1770.814 | 6.6 | C | C |
| UPAR-224 (SEQ ID NO: 96) | Pent-[Cys-Orn(Tris-Nta(Tris))-Lys(DOTA)-Tyr-Phe-Pro-Hyp-Ile-Nva-Cys]-NH₂ | VIb | 2044.985 | 2044.978 | 7.2 | A | A |
| UPAR-225 (SEQ ID NO: 97) | iHex-[Cys-Cit-Gln-Tyr-Phe-Pro-ala-Chg-Leu-Cys]-NH₂ | Ia | 1334.652 | 1334.649 | 8.4 | A | A |
| UPAR-226 (SEQ ID NO: 98) | Pent-[Cys-Arg-Gln-Tyr-Phe-Pro-Tyr-Ile-Nva-Cys]-NH₂ | Ia | 1371.647 | 1371.650 | 7.2 | A | A |
| UPAR-227 (SEQ ID NO: 99) | iHex-[Cys-Cit-Gln-Tyr-Mcf Pro-ala-Ile-Leu-Cys]-NH₂ | Ia | 1342.597 | 1342.597 | 8.2 | A | A |
| UPAR-228 (SEQ ID NO: 100) | Butoc-[Cys-Thr-Dab(DOTA)-Tyr-Phe-4Cfp-Hyp-Ile-Nva-Cys]-NH₂ | IId | 1658.748 | 1658.746 | 6.4 | A | A |
| UPAR-229 (SEQ ID NO: 101) | DOTA-O2Oc-Leu-[Cys-Cit-Gln-Tyr-Phe-Pro-Pro-Ile-Leu-Cys]-NH₂ | Ib | 1880.916 | 1880.910 | 7.4 | A | A |
| UPAR-230 (SEQ ID NO: 102) | Ac-Asp-Leu-[Cys-Arg-Nle-Tyr-phe-Pro-Ala-Ile-Leu-Cys]-NH₂ | Ic | 1464.726 | 1464.730 | 7.6 | D | D |
| UPAR-231 (SEQ ID NO: 103) | iHex-[Cys-ala-Gln-Tyr-Phe-Pro-ala-Ile-Leu-Cys]-NH₂ | Ia | 1222.588 | 1222.585 | 8.4 | C | C |
| UPAR-232 (SEQ ID NO: 104) | iHex-[Cys-Cit-Gln-Tyr-Phe-Pip-ala-Ile-Leu-Cys]-NH₂ | Ia | 1322.652 | 1322.653 | 8.0 | A | B |
| UPAR-233 (SEQ ID NO: 105) | Pent-[Cys-Cit-Lys(DOTA)-Tyr-Pcf-Pro-Hyp-Ile-Nva-Cys]-NH₂ | IIa | 1742.793 | 1742.786 | 7.6 | A | A |
| UPAR-234 (SEQ ID NO: 106) | Butoc-[Cys-Thr-Orn(DOTA)-Ay3-Phe-4Cfp-Hyp-Ile-Nva-Cys]-NH₂ | VIIb | 1687.775 | 1687.761 | 6.5 | A | A |
| UPAR-235 (SEQ ID NO: 107) | Hex-Asp-Leu-[Cys-Lys(DOTA)-Met-Tyr-Phe-Pro-Ala-Ile-Leu-Cys]-NH₂ | IIa | 1896.919 | 1896.922 | 9.0 | A | A |
| UPAR-236 (SEQ ID NO: 108) | Pent-[Cys-Gly-Gln-Tyr-Phe-Pro-Hyp-Ile-Nva-Cys]-NH₂ | Ia | 1222.551 | 1222.556 | 7.8 | A | A |
| UPAR-237 (SEQ ID NO: 109) | Butoc-[Cys-Thr-Lys(DOTA)-Tyr-Phe-4Cfp-Hyp-Ile-Nva-Cys]-NH₂ | IId | 1686.780 | 1686.775 | 6.8 | A | A |
| UPAR-238 (SEQ ID NO: 110) | Butoc-[Cys-Thr-Lys(LuDOTA)-Dopa-Phe-4Cfp-Hyp-Ile-Nva-Cys]-NH₂ | IX | 1874.692 | 1874.677 | 6.1 | A | A |
| UPAR-239 (SEQ ID NO: 111) | Ac-Asp-Leu-[Cys-Glu-Nle-Tyr-Phe-Pro-Ala-Ile-Leu-Cys]-NH₂ | Ic | 1437.667 | 1437.682 | 8.8 | A | A |
| UPAR-240 (SEQ ID NO: 112) | iHex-[Cys-Cit-Lys(DOTA)-Tyr-Phe-Pro-Hyp-Ile-Leu-Cys]-NH₂ | IIa | 1736.863 | 1736.863 | 7.8 | A | A |
| UPAR-241 (SEQ ID NO: 113) | Butoc-[Cys-Thr-Lys(GaDOTA)-Dopa-Phe-4Cfp-Hyp-Ile-Nva-Cy s]-NH₂ | IX | 1768.677 | 1768.667 | 5.7 | A | A |
| UPAR-242 (SEQ ID NO: 114) | nBuCAyl-[Cys-Thr-Lys(DOTA)-Tyr-Phe-Pro-Hyp-Ile-Nva-Cys]-NH₂ | IIe | 1667.805 | 1667.802 | 6.8 | A | A |
| UPAR-243 (SEQ ID NO: 115) | Ac-[Cys-Cit-Gln-Tyr-Phe-Pro-ala-Ile-Leu-Cys]-NH₂ | Ic | 1252.573 | 1252.631 | 6.5 | D | D |
| UPAR-244 (SEQ ID NO: 116) | Ac-Asp-leu-[Cys-Arg-Nle-Tyr-Phe-Pro-Ala-Ile-Leu-Cys]-NH₂ | Ic | 1464.726 | 1464.731 | 7.2 | A | A |
| UPAR-245 (SEQ ID NO: 117) | DOTA-O2Oc-Leu-[Cys-Cit-Gln-Tyr-Phe-Pro-ala-Ile-Leu-Cys]-NH₂ | Ib | 1854.901 | 1854.893 | 6.8 | A | A |
| UPAR-246 (SEQ ID NO: 118) | Pent-[Cys-Cit-Lys(DOTA)-Tyr-Phe-Pro-4Tfp-Ile-Nva-Cys]-NH₂ | IIa | 1710.827 | 1710.819 | 7.1 | A | A |
| UPAR-247 (SEQ ID NO: 119) | Pent-[Cys-Orn(TrisPEG)-Lys(DOTA)-Tyr-Phe-Pro-Hyp-Ile-Nva-Cys]-NH₂ | VIIIb | 3970.165 | 3970.169 | 8.6 | B | C |
| UPAR-248 (SEQ ID NO: 120) | Butoc-[Cys-Thr-Lys(DOTA)-Tyr-Phe-Pro-Hyp-Ile-Nva-Cys]-NH₂ | IId | 1668.789 | 1668.785 | 6.9 | A | A |
| UPAR-249 (SEQ ID NO: 121) | Butoc-[Cys-Thr-Lys(DOTA)-Dopa-Phe-4Cfp-Hyp-Ile-Nva-Cys]-NH₂ | IId | 1702.775 | 1702.766 | 7.0 | A | A |
| UPAR-250 (SEQ ID NO: 122) | Butoc-[Cys-Thr-Lys(NOTA)-Dopa-Phe-4Cfp-Hyp-Ile-Nva-Cys]-NH₂ | IId | 1601.727 | 1601.727 | 7.4 | A | A |
| UPAR-251 (SEQ ID NO: 123) | Pent-[Cys-Cit-Lys(GdDOTA)-Mpa-Phe-Pro-Hyp-Ile-Nva-Cys]-NH₂ | IX | 1842.728 | 1842.720 | 6.6 | A | A |
| UPAR-252 (SEQ ID NO: 124) | Pent-[Cys-Cit-Lys(DOTA)-Tyr-Tic-Pro-Hyp-Ile-Nva-Cys]-NH₂ | IIa | 1720.832 | 1720.828 | 5.9 | D | D |
| UPAR-253 (SEQ ID NO: 125) | Pent-[Cys-Cit-Lys(DOTA)-Tyr-Mpa-Pro-Hyp-Ile-Nva-Cys]-NH₂ | IIa | 1709.827 | 1709.823 | 5.2 | A | B |
| UPAR-254 (SEQ ID NO: 126) | Pent-[Cys-Cit-Lys(LuDOTA)-Tyr-Egm-Pro-Hyp-Ile-Nva-Cys]-NH₂ | IX | 1948.671 | 1948.670 | 8.9 | A | A |
| UPAR-255 (SEQ ID NO: 127) | iHex-[Cys-Cit-Glu-Tyr-Phe-Pro-ala-Ile-Leu-Cys]-NH₂ | Ia | 1309.620 | 1309.725 | 7.7 | A | A |
| UPAR-256 (SEQ ID NO: 128) | iHex-[Cys-Cit-Lys(DOTA)-Tyr-Phe-Pro-ala-Ile-Leu-Cys]-NH₂ | IIa | 1694.852 | 1694.853 | 7.6 | A | A |
| UPAR-257 (SEQ ID NO: 129) | Pent-[Cys-Cit-Lys(DOTA)-Nif-Phe-Pro-Hyp-Ile-Nva-Cys]-NH₂ | IIa | 1737.822 | 1737.822 | 8.0 | A | A |
| UPAR-258 (SEQ ID NO: 130) | Pent-[Cys(2MeBn)-Arg-Gln-Tyr-Phe-Pro-Hyp-Ile-Nva-Cys]-NH₂ | Vc | 1425.694 | 1425.688 | 7.9 | D | D |
| UPAR-259 (SEQ ID NO: 131) | Pent-[Cys-Arg-Gln-2Ni-Phe-Pro-Hyp-Ile-Nva-Cys]-NH₂ | Ia | 1355.652 | 1355.655 | 9.1 | A | A |
| UPAR-261 (SEQ ID NO: 132) | Pent-[Cys-Cit-Lys(DOTA)-Tyr-Phe-Pro-Hyp-Ile-Nva-Cys]-NH₂ | IIa | 1708.832 | 1708.827 | 7.3 | A | A |
| UPAR-262 (SEQ ID NO: 133) | iHex-[Cys-Cit-Gln-Tyr-Phe-Pro-Oxa-Ile-Leu-Cys]-NH₂ | Ia | 1336.631 | 1336.629 | 8.1 | A | A |
| UPAR-263 (SEQ ID NO: 134) | iHex-[Cys-Cit-Gln-Tyr-Ala-Pro-ala-Ile-Leu-Cys]-NH₂ | Ia | 1232.605 | 1232.669 | 6.3 | D | D |
| UPAR-264 (SEQ ID NO: 135) | Pent-[Cys-Arg-Gln-Tyr-Phe-Pro-Hyp-Egz-Nva-Cys]-NH₂ | Ia | 1333.631 | 1333.622 | 7.2 | A | A |
| UPAR-265 (SEQ ID NO: 136) | Pent-[Cys-Cit-Lys(DOTA)-Tyr-Phe-Pro-Nmo-Ile-Nva-Cys]-NH₂ | IIa | 1723.879 | 1723.866 | 5.8 | C | D |
| UPAR-266 (SEQ ID NO: 137) | Pent-[Cys-Arg-Gln-Tyr-Phe-Cha-Hyp-Ile-Nva-Cys]-NH₂ | Ia | 1377.694 | 1377.684 | 8.4 | A | A |
| UPAR-267 (SEQ ID NO: 138) | Prpoc-[Cys-Cit-Lys(DOTA)-Tyr-Phe-Pro-Hyp-Ile-Nva-Cys]-NH₂ | IId | 1710.811 | 1710.807 | 6.9 | C | C |
| UPAR-268 (SEQ ID NO: 139) | AGLU -Glutar- Nva- [Cys-Cit-Gln-Tyr-Phe-Pro-Hyp-Ile-Nva-Cys]-NH₂ | IVf | 1614.742 | 1614.742 | 6.8 | A | A |
| UPAR-269 (SEQ ID NO: 140) | iHex-[Cys-Cit-Aib-Tyr-Phe-Pro-ala-Ile-Leu-Cys]-NH₂ | Ia | 1265.630 | 1265.685 | 7.7 | B | B |
| UPAR-270 (SEQ ID NO: 141) | Ac-Asp-Leu-[Cys-Arg-Nle-Tyr-Phe-Pro-Ala-Ile-Leu-Cys]-NH₂ | Ic | 1464.726 | 1464.739 | 7.5 | A | A |
| UPAR-271 (SEQ ID NO: 142) | Pent-[Cys-Arg-Gln-Tyr-Phe-PPAc-Ile-Nva-Cys]-NH₂ | Ia | 1237.610 | 1237.616 | 5.7 | D | D |
| UPAR-272 (SEQ ID NO: 143) | HPA-[Cys-Cit-Gln-Tyr-Phe-Pro-ala-Ile-Leu-Cys]-NH₂ | Ia | 1282.584 | 1282.585 | 7.0 | C | D |
| UPAR-273 (SEQ ID NO: 144) | Pent-[Cys-Arg-Gln-Tyr-Bta-Pro-Hyp-Ile-Nva-Cys] - NH₂ | Ia | 1377.603 | 1377.609 | 8.0 | A | A |
| UPAR-274 (SEQ ID NO: 145) | Ac-Asp-Leu-[Cys-Arg-nle-Tyr-Phe-Pro-Ala-Ile-Leu-Cys]-NH₂ | Ic | 1464.726 | 1464.726 | 7.0 | B | B |
| UPAR-275 (SEQ ID NO: 146) | Butoc-[Cys-Cit-Lys(DOTA)-Tyr-Phe-Pro-Hyp-Ile-Nva-Cys]-NH₂ | IId | 1724.827 | 1724.817 | 7.3 | A | A |
| UPAR-276 (SEQ ID NO: 147) | Butoc-[Cys-Thr-Om(DOTA)-Pcnf-Phe-4Cfp-Hyp-Ile-Nva-Cys]-NH₂ | IId | 1699.775 | 1699.779 | 5.8 | A | A |
| UPAR-277 (SEQ ID NO: 148) | Pent-[Cys-Cit-Lys(DOTA)-Tyr-Phe-Pro-Nms-Ile-Nva-Cys]-NH₂ | IIa | 1696.832 | 1696.827 | 6.3 | C | C |
| UPAR-278 (SEQ ID NO: 149) | Pent-[Cys-Har-Lys(DOTA)-Tyr-Phe-Pro-Hyp-Ile-Nva-Cys]-NH₂ | IIa | 1721.863 | 1721.859 | 7.0 | A | A |
| UPAR-279 (SEQ ID NO: 150) | Pent-[Cys-Cit-Lys(LaDOTA)-Tyr-Phe-Pro-Aib-Ile-Nva-Cys]-NH₂ | IX | 1815.720 | 1815.719 | 7.7 | A | A |
| UPAR-280 (SEQ ID NO: 151) | Butoc-[Cys-Thr-Om(DOTA)-Dopa-Phe-4Cfp-Hyp-Ile-Nva-Cys]-NH₂ | IId | 1688.759 | 1688.774 | 5.9 | A | A |
| UPAR-281 (SEQ ID NO: 152) | Pent-[Cys-Arg-Gln-Tyr-Phe-Pro-Leu-Ile-Nva-Cys]-NH₂ | Ia | 1321.668 | 1321.668 | 7.5 | A | A |
| UPAR-283 (SEQ ID NO: 153) | Pent-[Cys-Asn-Lys(DOTA)-Tyr-Phe-Pro-Hyp-Ile-Nva-Cys]-NH₂ | IIa | 1665.789 | 1665.786 | 7.3 | A | A |
| UPAR-284 (SEQ ID NO: 154) | Butoc-[Cys-Thr-Lys(DOTA)-Dopa-Phe-Hyp-Hyp-Ile-Nva-Cys]-NH₂ | IId | 1700.779 | 1700.777 | 6.2 | A | A |
| UPAR-285 (SEQ ID NO: 155) | iHex-[Cys-Cit-Gln-Nmy-Phe-Pro-ala-Ile-Leu-Cys]-NH₂ | Ia | 1322.652 | 1322.715 | 7.6 | D | D |
| UPAR-286 (SEQ ID NO: 156) | nBuCAyl-[Cys-Cit-Gln-Tyr-Phe-Pro-ala-Ile-Leu-Cys]-NH₂ | IVd | 1309.631 | 1309.629 | 7.8 | A | A |
| UPAR-287 (SEQ ID NO: 157) | iHex-[Cys-Nmr-Gln-Tyr-Phe-Pro-ala-Ile-Leu-Cys]-NH₂ | Ia | 1321.668 | 1321.725 | 7.4 | D | D |
| UPAR-288 (SEQ ID NO: 158) | Pent-[Cys-Thr-Apc(DOTA)-Tyr-Phe-Pro-Hyp-Ile-Nva-Cys]-NH₂ | IIa | 1650.779 | 1650.776 | 6.7 | A | A |
| UPAR-289 (SEQ ID NO: 159) | Butoc-[Cys-Thr-Lys(DOTA)-Tyr-Phe-Pro-4Tfp-Ile-Nva-Cys]-NH₂ | IId | 1670.785 | 1670.780 | 6.9 | A | A |
| UPAR-290 (SEQ ID NO: 160) | Ac-Asp-Leu-[Cys-Arg-Gln-Tyr-Phe-Pro-Ala-Ile-Leu-Cys]-NH₂ | Ic | 1479.700 | 1479.718 | 7.1 | A | A |
| UPAR-291 (SEQ ID NO: 161) | DOTA-Ahx-Leu-[Cys-Arg-Gln-Tyr-Phe-Pro-ala-Ile-Leu-Cys]-NH₂ | Ib | 1821.927 | 1821.926 | 6.8 | A | A |
| UPAR-292 (SEQ ID NO: 162) | Pent-[Cys-Cit-Gln-Tyr-Phe-Pro-Hyp-Ile-Nva-Cysol]-OH | IIIc | 1309.620 | 1309.619 | 5.9 | A | A |
| UPAR-293 (SEQ ID NO: 163) | Succinyl-Nva-[Cys-Cit-Lys(DOTA)-Tyr-Phe-Pro-Hyp-Ile-Nva-Cys]-NHz | IIa | 1823.859 | 1823.851 | 7.2 | A | A |
| UPAR-294 (SEQ ID NO: 164) | Butoc-[Cys-Thr-Lys(InDOTA)-Tyr-Phe-4Cfp-Hyp-Ile-Nva-Cys]-NH₂ | IX | 1796.660 | 1796.647 | 6.4 | A | A |
| UPAR-295 (SEQ ID NO: 165) | iHex-[Cys-Cit-Gln-Tyr-Phe-Pro-Tap-Ile-Leu-Cys]-NH₂ | Ia | 1349.662 | 1349.706 | 7.7 | A | A |
| UPAR-296 (SEQ ID NO: 166) | Pent-[Cys-Cit-Lys(LaDOTA)-Ppa-Phe-Pro-Hyp-Ile-Nva-Cys]-NH₂ | IX | 1828.716 | 1828.714 | 6.6 | A | A |
| UPAR-297 (SEQ ID NO: 167) | Succinyl-Nva-[Cys-Thr-Lys(DOTA)-Tyr-Phe-Pro-Hyp-Ile-Nva-Cys]-NHz | IIa | 1767.821 | 1767.818 | 6.6 | A | A |
| UPAR-298 (SEQ ID NO: 168) | Pent-[Cys-Arg-Gln-Tyr-Phe-Lys-Hyp-Ile-Nva-Cys]-NH₂ | Ia | 1352.673 | 1352.666 | 5.8 | C | C |
| UPAR-299 (SEQ ID NO: 169) | Pent-[Cys-Cit-Lys(DOTA)-Tyr-Phe-Pro-Aib-Ile-Nva-Cys]-NH₂ | IIa | 1680.837 | 1680.838 | 7.1 | A | A |
| UPAR-300 (SEQ ID NO: 170) | Pent-[Hcy-Arg-Gln-Tyr-Phe-Pro-Hyp-Ile-Nva-Cys]-NH₂ | Ia | 1335.647 | 1335.636 | 7.3 | B | B |
| UPAR-301 (SEQ ID NO: 171) | Pent-[Cys-Thr-Lys(DOTA)-Tyr-Phe-Pro-Hyp-Ile-Nva-Cys]-NH₂ | IIa | 1652.794 | 1652.791 | 7.3 | A | A |
| UPAR-302 (SEQ ID NO: 172) | Pent-[Cys-Arg-Gln-Tyr-Phe-Nml-Hyp-Ile-Nva-Cys]-NH₂ | Ia | 1351.678 | 1351.666 | 7.6 | B | B |
| UPAR-303 (SEQ ID NO: 173) | Pent-[Cys-Cit-Lys(LaDOTA)-Tyr-Phe-4Cfp-Hyp-Ile-Nva-Cys]-NH₂ | IX | 1861.706 | 1861.705 | 7.7 | A | A |
| UPAR-304 (SEQ ID NO: 174) | Butoc-[Cys-Cit-Lys(DOTA)-Tyr-Pcf-4Cfp-Hyp-Ile-Nva-Cys]-NH₂ | IId | 1776.778 | 1776.774 | 7.6 | A | A |
| UPAR-305 (SEQ ID NO: 175) | iHex-[Cys-Cit-Gln-Tyr-Phe-Pro-pro-Ile-Leu-Cys]-NH₂ | Ia | 1334.652 | 1334.652 | 7.1 | D | D |
| UPAR-306 (SEQ ID NO: 176) | Ac-Asp-Leu- [Cys-Cit-Lys(DOTA)-Tyr-Phe-Pro-arg-Ile-Leu-Cys]-NH₂ | IIa | 1951.965 | 1952.118 | 6.6 | A | A |
| UPAR-307 (SEQ ID NO: 177) | iHex-[Cys-Cit-Gln-Tyr-Phe-Pro-4Cfp-Ile-Leu-Cys]-NH₂ | Ia | 1352.642 | 1352.641 | 8.2 | A | A |
| UPAR-308 (SEQ ID NO: 178) | Pent-[Cys-Arg-Gln-Tyr-Trp-Pro-Hyp-Ile-Nva-Cys]-NH₂ | Ia | 1360.642 | 1360.646 | 6.7 | A | B |
| UPAR-309 (SEQ ID NO: 179) | Pent-[Cys-Cit-Gln-Tyr-Phe-Pro-Hyp-Ile-Nva-Cys]-NH₂ | Ia | 1322.615 | 1322.614 | 7.4 | A | A |
| UPAR-310 (SEQ ID NO: 180) | iHex-[Cys-Cit-Gln-Tyr-1Ni-Pro-ala-Ile-Leu-Cys]-NH₂ | Ia | 1358.652 | 1358.722 | 8.1 | A | A |
| UPAR-311 (SEQ ID NO: 181) | Pent-[Cys-Om(mPEG 12)-Lys(InDOTA)-Tyr-Phe-Pro-Hyp-Ile-Nva-Cys]-NHz | IX | 2346.032 | 2346.028 | 8.4 | A | A |
| UPAR-312 (SEQ ID NO: 182) | H-Met-Asp-Leu-[Cys-Arg-Met-Tyr-Phe-Pro-Ala-Ile-Leu-Cys]-NH₂ | Ia | 1571.712 | 1571.710 | 6.7 | A | B |
| UPAR-313 (SEQ ID NO: 183) | Pent-[Cys-Arg-Arg-Tyr-Phe-Pro-Tap-Ile-Nva-Cys]-NH₂ | Ia | 1348.690 | 1348.691 | 6.7 | A | A |
| UPAR-314 (SEQ ID NO: 184) | iHex-[Cys-Cit-Gln-Aph-Phe-Pro-ala-Ile-Leu-Cys]-NH₂ | Ia | 1307.652 | 1307.651 | 7.0 | A | A |
| UPAR-315 (SEQ ID NO: 185) | Pent-[Cys-Cit-Lys(DOTA)-Tyr-Egm-Pro-Hyp-Ile-Nva-Cys]-NH₂ | IIa | 1776.754 | 1776.750 | 8.0 | A | A |
| UPAR-316 (SEQ ID NO: 186) | Pent-[Cys-Cit-Lys(DOTA)-Tyr-Phe-Pro-Nma-Ile-Nva-Cys]-NH₂ | IIa | 1680.837 | 1680.837 | 6.7 | B | B |
| UPAR-317 (SEQ ID NO: 187) | Pent-[Cys-Cit-Lys(Bio-Ttds)-Tyr-Phe-Pro-Nmk-Ile-Nva-Cys]-NH₂ | IIi | 1879.976 | 1879.994 | 5.9 | ND | C |
| UPAR-318 (SEQ ID NO: 188) | Hex-Thr-Leu-[Cys-Asp-Leu-Tyr-Phe-Pro-Ala-Ile-Leu-Cys]-NH₂ | Ia | 1465.735 | 1465.847 | 9.1 | A | A |
| UPAR-319 (SEQ ID NO: 189) | iHex-[Cys-Cit-Gln-Tyr-Phe-Ala-ala-Ile-Leu-Cys]-NH₂ | Ia | 1282.620 | 1282.660 | 7.1 | C | C |
| UPAR-320 (SEQ ID NO: 190) | Pent-[Cys-Cit-Lys(DOTA)-Apli-Phe-Pro-Hyp-Ile-Nva-Cys]-NH₂ | IIa | 1707.848 | 1707.845 | 6.4 | A | A |
| UPAR-321 (SEQ ID NO: 191) | iHex-[Cys-Cit-Gln-Tyr-Phe-Oxa-ala-Ile-Leu-Cys] - NH₂ | Ia | 1310.615 | 1311.616 | 7.6 | A | B |
| UPAR-322 (SEQ ID NO: 192) | Pent-[Cys-Arg-Gln-Tyr-Phe-Pro-nva-Ile-Nva-Cys]-NH₂ | Ia | 1307.652 | 1307.653 | 7.2 | B | B |
| UPAR-323 (SEQ ID NO: 193) | Ac-Asp-Leu-[Cys-Arg-Nle-Tyr-Phe-Pro-Ala-Ile-Leu-cys]-NH₂ | Ic | 1464.726 | 1464.725 | 6.9 | B | B |
| UPAR-324 (SEQ ID NO: 194) | Pent-[Cys-Cit-Lys(DOTA)-His-Phe-Pro-Hyp-Ile-Nva-Cys]-NH₂ | IIa | 1682.827 | 1682.824 | 6.1 | A | A |
| UPAR-325 (SEQ ID NO: 195) | Pent-[Cys-Cit-Lys(DOTA)-Tyr-Phe-Pro-4Ap-Ile-Nva-Cys]-NH₂ | IIa | 1707.848 | 1707.844 | 6.7 | A | A |
| UPAR-326 (SEQ ID NO: 196) | iHex-[Cys-Cit-Gln-Tyr-Phe-Pro-ala-Cha-Leu-Cys]-NH₂ | Ia | 1348.667 | 1348.712 | 8.3 | A | A |
| UPAR-327 (SEQ ID NO: 197) | Pent-[Cys-Cit-Lys(DOTA)-Tyr-Phe-Pro-Nme-Ile-Nva-Cys]-NH₂ | IIa | 1738.842 | 1738.839 | 6.3 | D | D |
| UPAR-328 (SEQ ID NO: 198) | PentylSO2-[Cys-Arg-Gln-Tyr-Phe-Pro-Hyp-Ile-Nva-Cys]-NH₂ | IVg | 1371.614 | 1371.619 | 7.8 | A | A |
| UPAR-329 (SEQ ID NO: 199) | Pent-[Cys-Arg-Gln-Tyr-Phe-Pro-ser-Ile-Nva-Cys]-NH₂ | Ia | 1295.615 | 1295.620 | 6.9 | A | A |
| UPAR-330 (SEQ ID NO: 200) | Pent-[Cys-Arg-Gln-Tyr-Phe-Pro-dap-Ile-Nva-Cys]-NH₂ | Ia | 1294.632 | 1294.633 | 6.3 | A | A |
| UPAR-331 (SEQ ID NO: 201) | Pent-[Cys-Cit-Lys(InDOTA)-Tyr-Mpa-Pro-Hyp-Ile-Nva-Cys]-NH₂ | IX | 1819.706 | 1819.706 | 5.4 | A | A |
| UPAR-332 (SEQ ID NO: 202) | iHex-[Cys-Cit-Gln-Tyr-Pcf-Pro-ala-Ile-Leu-Cys]-NH₂ | Ia | 1342.597 | 1342.597 | 8.3 | A | A |
| UPAR-333 (SEQ ID NO: 203) | Dmba-[Cys-Cit-Gln-Tyr-Phe-Pro-ala-Ile-Leu-Cys]-NH₂ | Ia | 1309.631 | 1309.737 | 6.2 | C | D |
| UPAR-334 (SEQ ID NO: 204) | Ac-Gly-[Cys-Cit-Gln-Tyr-Phe-Pro-ala-Ile-Leu-Cys]-NH₂ | Ic | 1309.595 | 1309.595 | 6.9 | D | D |
| UPAR-335 (SEQ ID NO: 205) | Pent-[Cys-Asn-Lys(LuDOTA)-Tyr-Phe-Pro-Hyp-Ile-Nva-Cys]-NH₂ | IX | 1837.707 | 1837.705 | 7.7 | A | A |
| UPAR-336 (SEQ ID NO: 206) | Pent-[Cys-Cit-Lys(DOTA)-Ppa-Phe-Pro-Hyp-Ile-Nva-Cys]-NH₂ | IIa | 1693.832 | 1693.824 | 6.2 | A | A |
| UPAR-337 (SEQ ID NO: 207) | Pent-[Cys-Arg-Gln-Tyr-Phe-Nmb-Hyp-Ile-Nva-Cys]-NH₂ | Ia | 1351.678 | 1351.669 | 7.8 | D | A |
| UPAR-338 (SEQ ID NO: 208) | iHex-[Cys-Cit-Gln-Tyr-Phe-Pro-ala-Ile-Leu-Cys]-NH₂ | Ia | 1308.636 | 1308.743 | 7.5 | A | A |
| UPAR-339 (SEQ ID NO: 209) | iHex-[Cys-Cit-Ala-Tyr-Phe-Pro-ala-Ile-Leu-Cys]-NH₂ | Ia | 1251.614 | 1251.668 | 7.7 | A | B |
| UPAR-340 (SEQ ID NO: 210) | iHex-[Cys-Cit-Gln-Tyr-Phe-Pro-ala-Nmi-Leu-Cys]-NH₂ | Ia | 1322.652 | 1323.692 | 7.5 | D | C |
| UPAR-341 (SEQ ID NO: 211) | Ac-Asp-Leu-[Cys-Arg-Met-Tyr-Phe-Pro-Ala-Ile-Leu-Cys]-NH₂ | Ic | 1482.682 | 1482.791 | 7.9 | A | A |
| UPAR-342 (SEQ ID NO: 212) | Pent-[Cys-Ser-Lys(DOTA)-Tyr-Phe-Pro-Hyp-Ile-Nva-Cys]-NH₂ | IIa | 1638.779 | 1638.773 | 6.8 | A | A |
| UPAR-343 (SEQ ID NO: 213) | iHex-[Cys-Cit-Gln-Tyr-Tyr-Pro-ala-Ile-Leu-Cys]-NH₂ | Ia | 1324.631 | 1325.694 | 6.4 | B | C |
| UPAR-344 (SEQ ID NO: 214) | Pent-[Cys-Arg-Gln-Tyr-Phe-Pro-Asp-Ile-Nva-Cys]-NH₂ | Ia | 1323.610 | 1323.615 | 6.9 | A | A |
| UPAR-345 (SEQ ID NO: 215) | iHex-[Cys-Cit-Gln-Tyr-Phe-Pro-ala-Ile-Nle-Cys]-NH₂ | Ia | 1308.636 | 1308.633 | 8.1 | A | B |
| UPAR-346 (SEQ ID NO: 216) | nBuCAyl-[Cys-Cit-Lys(DOTA)-Tyr-Phe-Pro-Hyp-Ile-Nva-Cys]-NH₂ | IIe | 1723.843 | 1723.833 | 7.2 | A | A |
| UPAR-347 (SEQ ID NO: 217) | DOTA-Ttds-Nle-Asp-Leu-[Cys-Arg-Met-Tyr-Phe-Pro-Ala-Ile-Leu-Cys]-NH₂ | Ib | 2242.120 | 2242.132 | 8.2 | A | A |
| UPAR-348 (SEQ ID NO: 218) | Pent-[Cys-Cit-Lys(LuDOTA)-Tyr-Phe-Pro-Nmg-Ile-Nva-Cys]-NH₂ | IX | 1838.738 | 1838.737 | 7.2 | A | ND |
| UPAR-349 (SEQ ID NO: 219) | iHex-[Cys-Cit-Gln-Tyr-Phe-Pro-ala-Cpg-Leu-Cys]-NH₂ | Ia | 1320.636 | 132.634 | 7.6 | A | B |
| UPAR-350 (SEQ ID NO: 220) | Pent-[Cys-Cit-Lys(LuDOTA)-Guf-Phe-Pro-Hyp-Ile-Nva-Cys]-NH₂ | IX | 1921.787 | 1921.786 | 7.0 | A | A |
| UPAR-351 (SEQ ID NO: 221) | Hex-Asp-Leu-[Cys-Arg-Met-Tyr-Phe-Pro-Lys(DOTA)-Ile-Leu-Cys]-NH₂ | IIa | 1981.983 | 1981.992 | 8.1 | A | A |
| UPAR-352 (SEQ ID NO: 222) | Pent-[Cys-Cit-Lys(LuDOTA)-Nif-Phe-Pro-Hyp-Ile-Nva-Cys]-NH₂ | IX | 1909.739 | 1909.739 | 8.9 | A | A |
| UPAR-353 (SEQ ID NO: 223) | Pent-[Cys-Cit-Gln-Tyr-Phe-Pro-Hyp-Ile-Nva-Cys]-Bal-NH₂ | Ia | 1393.652 | 1393.651 | 6.0 | B | B |
| UPAR-354 (SEQ ID NO: 224) | iHex-[Cys-Cit-Gln-Tyr-Phe-4Cfp-ala-Ile-Leu-Cys]-NH₂ | Ia | 1326.626 | 1326.624 | 7.6 | A | A |
| UPAR-355 (SEQ ID NO: 225) | Pent-[Cys-Cit-Lys(InDOTA)-Tyr-Pcf-Pro-Hyp-Ile-Nva-Cys]-NH₂ | IX | 1852.673 | 1852.672 | 8.3 | A | A |
| UPAR-356 (SEQ ID NO: 226) | Bz-[Cys-Arg-Gln-Tyr-Phe-Pro-Hyp-Ile-Nva-Cys]-NH₂ | Ia | 1341.600 | 1341.595 | 7.5 | A | A |
| UPAR-357 (SEQ ID NO: 227) | iHex-[Cys-Cit-Gln-Tyr-Phe-Pro-arg-Ile-Leu-Cys]-NH₂ | Ia | 1393.700 | 1393.765 | 6.9 | A | B |
| UPAR-358 (SEQ ID NO: 228) | Butoc-[Cys-Thr-Gln-Tyr-Phe-4Cfp-Hyp-Ile-Nva-Cys]-NH₂ | IVc | 1300.563 | 1300.563 | 6.7 | A | A |
| UPAR-359 (SEQ ID NO: 229) | Glutar-[Cys-Cit-Gln-Tyr-Phe-Pro-ala-Ile-Leu-Cys]-NH₂ | Id | 1324.594 | 1324.595 | 7.1 | D | D |
| UPAR-360 (SEQ ID NO: 230) | Pent-[Cys-Cit-Lys(DOTA)-Tyr-Thi-Pro-Hyp-Ile-Nva-Cys]-NH₂ | IIa | 1714.788 | 1714.785 | 7.2 | A | A |
| UPAR-361 (SEQ ID NO: 231) | Pent-[Cys-Pro-Gln-Tyr-Phe-Pro-Hyp-Ile-Nva-Cys]-NH₂ | Ia | 1262.583 | 1262.586 | 7.9 | A | A |
| UPAR-362 (SEQ ID NO: 232) | Pent-[Cys-Cit-Gln-Tyr-Phe-Pro-ala-Ile-Leu-Cys]-NH₂ | Ia | 1294.620 | 1294.729 | 7.3 | A | A |
| UPAR-363 (SEQ ID NO: 233) | Hex-Asp-Leu-[Cys-Arg-Met-Tyr-Phe-Pro-Ala-Ile-Leu-Cys]-NH₂ | Ia | 1538.745 | 1538.786 | 8.9 | A | A |
| UPAR-364 (SEQ ID NO: 234) | iHex-[Cys-cit-Gln-Tyr-Phe-Pro-ala-Ile-Leu-Cys]-NH₂ | Ia | 1308.636 | 1308.741 | 7.5 | D | D |
| UPAR-365 (SEQ ID NO: 235) | Pent-[Cys-Cit-Lys(DOTA)-Tyr-Phe-4Cfp-Hyp-Ile-Nva-Cys]-NH₂ | IIa | 1726.822 | 1726.819 | 7.0 | A | A |
| UPAR-366 (SEQ ID NO: 236) | Pent-[Cys-Arg-Gln-Hfe-Phe-Pro-Hyp-Ile-Nva-Cys]-NH₂ | Ia | 1319.652 | 1319.658 | 8.4 | B | C |
| UPAR-367 (SEQ ID NO: 237) | iHex-[Cys-Arg-Gln-Tyr-Phe-Pro-lys(DOTA)-Ile-Leu-Cys]-NH₂ | IIa | 1750.890 | 1750.890 | 6.7 | A | C |
| UPAR-368 (SEQ ID NO: 238) | Butoc-[Cys-Thr-Lys(DOTA)-Tyr-Phe-4Cfp-Hyp-Ile-Hse-Cys]-NH₂ | IId | 1688.759 | 1688.757 | 5.0 | A | C |
| UPAR-369 (SEQ ID NO: 239) | Butoc-[Cys-Cit-Lys(LuDOTA)-Tyr-Pcf-4Cfp-Hyp-Ile-Nva-Cys]-NH₂ | IX | 1948.696 | 1948.694 | 8.4 | A | A |
| UPAR-370 (SEQ ID NO: 240) | Pent-[Cys-Arg-Gln-Tyr-Phe-Pro-Hyp-Npg-Nva-Cys]-NH₂ | Ia | 1335.647 | 1335.636 | 7.7 | A | A |
| UPAR-371 (SEQ ID NO: 241) | DOTA-Leu-[Cys-Cit-Gln-Tyr-Phe-Pro-Hyp-Ile-Nml-Cys]-NH₂ | Ib | 1765.853 | 1765.845 | 6.6 | D | D |
| UPAR-372 (SEQ ID NO: 242) | Ac-Cha-[Cys-Arg-Gln-Tyr-Phe-Pro-Hyp-Ile-Nva-Cys]-NH₂ | Ic | 1432.700 | 1432.705 | 7.9 | A | A |
| UPAR-373 (SEQ ID NO: 243) | Pent-[Cys-Arg-Gln-Cha-Phe-Pro-Hyp-Ile-Nva-Cys]-NH₂ | Ia | 1311.683 | 1311.678 | 8.9 | A | A |
| UPAR-375 (SEQ ID NO: 244) | iHex-[Cys-Cit-Gln-Tyr-Phe-Eaz-ala-Ile-Leu-Cys] - NH₂ | Ia | 1326.592 | 1326.594 | 7.9 | A | A |
| UPAR-376 (SEQ ID NO: 245) | Pent-[Cys-Har-Lys(GdDOTA)-Tyr-Phe-Pro-Hyp-Ile-Nva-Cys]-NH₂ | IX | 1870.760 | 1870.758 | 7.3 | A | A |
| UPAR-377 (SEQ ID NO: 246) | Pent-[Cys-Cit-Lys(DOTA)-Tyr-Phe-Pro-Tap-Ile-Nva-Cys]-NH₂ | IIa | 1707.848 | 1707.845 | 6.9 | A | A |
| UPAR-378 (SEQ ID NO: 247) | Butoc-[Cys-Thr-Lys(DOTA)-Tyr-Pnf-4Cfp-Hyp-Ile-Nva-Cys]-NH₂ | IId | 1711.775 | 1711.771 | 5.9 | A | A |
| UPAR-379 (SEQ ID NO: 248) | Pent-[Cys-Arg-Gln-Tyr-Phe-Pro-Hyp-Ile-Cpra-Cys]-NHz | Ia | 1333.631 | 1333.636 | 7.5 | A | A |
| UPAR-380 (SEQ ID NO: 249) | Pent-[Cys-Cit-Lys(DOTA)-Tyr-Phe-Pro-Hyp-Ile-Ser(Me)-Cys]-NH₂ | IIa | 1710.811 | 1710.812 | 6.6 | A | B |
| UPAR-381 (SEQ ID NO: 250) | Ac-Leu-[Cys-Arg-Gln-Tyr-Phe-Pro-Ala-Ile-Leu-Cys]-NH₂ | Ic | 1364.673 | 1364.692 | 6.8 | A | A |
| UPAR-382 (SEQ ID NO: 251) | iHex-[Cys-Cit-Lys(DOTA)-Tyr-Phe-Pro-Pro-Ile-Leu-Cys]-NH₂ | IIa | 1720.868 | 1720.918 | 7.8 | A | A |
| UPAR-383 (SEQ ID NO: 252) | Pent-[Cys-Om(mPEG 12)-Lys(DOTA)-Tyr-Phe-Pro-Hyp-Ile-Nva-Cys]-NH₂ | VIa | 2236.151 | 2236.139 | 7.7 | A | A |
| UPAR-384 (SEQ ID NO: 253) | Pent-[Cys-Cit-Lys(DOTA)-Tyr-Phe-Pro-nma-Ile-Nva-Cys]-NH₂ | IIa | 1680.837 | 1680.833 | 7.1 | A | A |
| UPAR-385 (SEQ ID NO: 254) | Pent-[Cys-Arg-Gln-Tyr-Phe-Pro-Hyp-Lys-Nva-Cys]-NH₂ | Ia | 1336.642 | 1336.631 | 5.8 | D | C |
| UPAR-386 (SEQ ID NO: 255) | Pent-[Cys-Glu(AGLU)-Gln-Tyr-Phe-Pro-Hyp-Ile-Nva-Cys]-NH₂ | IIc | 1457.657 | 1457.656 | 7.3 | A | A |
| UPAR-387 (SEQ ID NO: 256) | Pent-[Cys-Arg-Gln-Tyr-Phe-Oic-Hyp-Ile-Nva-Cys]-NH₂ | Ia | 1375.678 | 1375.678 | 8.3 | A | A |
| UPAR-388 (SEQ ID NO: 257) | Pent-[Cys-Thr-Lys(DOTA)-Tyr-Phe-Chy-Hyp-Ile-Nva-Cys]-NH₂ | IIa | 1668.789 | 1668.782 | 6.1 | A | B |
| UPAR-389 (SEQ ID NO: 258) | iHex-[Cys-Cit-Gln-Tyr-Phe-Pro-Tap(DOTA)-Ile-Leu-Cys]-NH₂ | IIa | 1735.843 | 1735.968 | 7.7 | A | A |
| UPAR-390 (SEQ ID NO: 259) | Pent-[Dab-Arg-Gln-Tyr-Phe-Pro-Hyp-Ile-Nva-Glu]-NH₂ | IIb | 1328.724 | 1328.725 | 6.5 | D | D |
| UPAR-391 (SEQ ID NO: 260) | Pent-[Cys-Leu-Gln-Tyr-Phe-Pro-Hyp-Ile-Nva-Cys]-NH₂ | Ia | 1278.614 | 1278.617 | 8.1 | A | A |
| UPAR-392 (SEQ ID NO: 261) | Ac-Asp-Leu-[Cys-asp-Lys(DOTA)-Tyr-Phe-Pro-ala-Ile-Leu-Cys]-NH₂ | IIa | 1824.843 | 1824.995 | 6.9 | D | D |
| UPAR-393 (SEQ ID NO: 262) | iHex-[Cys-Cit-Gln-Mpa-Phe-Pro-ala-Ile-Leu-Cys]-NH₂ | Ia | 1293.636 | 1293.633 | 6.9 | A | A |
| UPAR-394 (SEQ ID NO: 263) | H-Ava-[Cys-Cit-Gln-Tyr-Phe-Pro-ala-Ile-Leu-Cys]-NH₂ | Ia | 1309.631 | 1309.627 | 6.5 | D | D |
| UPAR-395 (SEQ ID NO: 264) | Pent-[Cys-Arg-Gln-Ocf-Phe-Pro-Hyp-Ile-Nva-Cys]-NH₂ | Ia | 1339.597 | 1339.603 | 8.6 | A | A |
| UPAR-396 (SEQ ID NO: 265) | Pent-[Cys-Cit-Lys(DOTA)-Tyr-Phe-Pro-Pro-Ile-Leu-Cys]-NH₂ | IIa | 1706.852 | 1706.851 | 7.7 | A | A |
| UPAR-397 (SEQ ID NO: 266) | Pent-[Pen-Cit-Gln-Tyr-Phe-Pro-Hyp-Ile-Nva-Cys]-NH₂ | Ia | 1350.646 | 1350.648 | 6.3 | A | A |
| UPAR-398 (SEQ ID NO: 267) | Pent-[Cys-Cit-Lys(DOTA)-Tyr-Nmf-Pro-Hyp-Ile-Nva-Cys]-NH₂ | IIa | 1722.847 | 1722.843 | 6.4 | D | D |
| UPAR-399 (SEQ ID NO: 268) | Pent-[Cys-Cit-Lys(DOTA)-Dopa-Phe-Pro-Hyp-Ile-Nva-Cys]-NH₂ | IIa | 1724.827 | 1724.823 | 7.0 | A | A |
| UPAR-400 (SEQ ID NO: 269) | Pent-[Cys-Arg-Gln-Tyr-Phe-Phe-Hyp-Ile-Nva-Cys]-NH₂ | Ia | 1371.647 | 1371.644 | 7.9 | A | B |
| UPAR-401 (SEQ ID NO: 270) | Pent-[Cys-Arg-Gln-Trp-Phe-Pro-Hyp-Ile-Nva-Cys] - NH₂ | Ia | 1344.647 | 1344.644 | 8.2 | A | A |
| UPAR-402 (SEQ ID NO: 271) | iHex-[Cys-Cit-Gln-Tyr-Phe-Pro-ala-Ile-Ala-Cys]-NH₂ | Ia | 1266.589 | 1266.674 | 6.5 | B | B |
| UPAR-403 (SEQ ID NO: 272) | Pent-[Cys-Cit-Lys(DOTA)-Tyr-Phe-Hyp-Hyp-Ile-Nva-Cys]-NH₂ | IIa | 1724.827 | 1724.823 | 6.7 | A | A |
| UPAR-404 (SEQ ID NO: 273) | Pent-[Cys-Cit-Lys(InDOTA-PEG12)-Tyr-Phe-Pro-Hyp-Ile-Nva-Cys]-NH₂ | IX | 2418.064 | 2418.063 | 8.5 | A | A |
| UPAR-405 (SEQ ID NO: 274) | iHex-[Cys-Cit-Gln-Tyr-Phe-Pro-ala-Ile-Ile-Cys]-NH₂ | Ia | 1308.636 | 1308.634 | 7.5 | B | B |
| UPAR-406 (SEQ ID NO: 275) | Ac-Asp-Leu-[Cys-Arg-Nle-Tyr-Phe-Pro-ala-Ile-Leu-Cys]-NH₂ | Ic | 1464.726 | 1464.729 | 7.3 | A | A |
| UPAR-407 (SEQ ID NO: 276) | iHex-[Cys-Cit-Nmq-Tyr-Phe-Pro-ala-Ile-Leu-Cys] - NH₂ | Ia | 1322.652 | 1322.711 | 7.4 | D | C |
| UPAR-408 (SEQ ID NO: 277) | iHex-[Cys-Lys(LuDOTA)-Gln-Tyr-Phe-Pro-ala-Ile-Leu-Cys]-NH₂ | IX | 1837.743 | 1837.732 | 7.9 | A | ND |
| UPAR-409 (SEQ ID NO: 278) | Pent-[Cys-Arg-Gly-Tyr-Phe-Pro-Hyp-Ile-Nva-Cys]-NH₂ | Ia | 1250.594 | 1250.599 | 7.2 | A | A |
| UPAR-410 (SEQ ID NO: 279) | iHex-[Cys-Cit-Asp-Tyr-Phe-Pro-Hyp-Ile-Leu-Cys]-NH₂ | Ia | 1337.615 | 1337.615 | 8.5 | A | A |
| UPAR-411 (SEQ ID NO: 280) | Pent-[Cys-Arg-Gln-Tyr-Phe-Pro-Hyp-Ile-Cbg-Cys]-NH₂ | Ia | 1333.631 | 1333.625 | 7.7 | A | A |
| UPAR-412 (SEQ ID NO: 281) | Ac-Asp-Leu- [cys-Arg-Nle-Tyr-Phe-Pro-Ala-Ile-Leu-Cys]-NH₂ | Ic | 1464.726 | 1464.730 | 6.8 | D | D |
| UPAR-413 (SEQ ID NO: 282) | iHex-[Cys-Cit-Lys(Ac)-Tyr-Phe-Pro-ala-Ile-Leu-Cys]-NH₂ | Ia | 1350.683 | 1350.683 | 8.4 | A | A |
| UPAR-414 (SEQ ID NO: 283) | iHex-[Cys-ala-Gln-Tyr-Phe-Pro-Lvs(DOTA)-Ile-Leu-Cys]-NH₂ | IIa | 1665.826 | 1665.827 | 7.8 | B | C |
| UPAR-415 (SEQ ID NO: 284) | Butoc-[Cys-Thr-Om(DOTA)-My-Phe-4Cfp-Hyp-Ile-Nva-Cys]-NH₂ | VIIa | 1672.764 | 1672.768 | 6.1 | A | A |
| UPAR-416 (SEQ ID NO: 285) | Pent-[Cys-Cit-Lys(DOTA)-Tyr-Amf-Pro-Hyp-Ile-Nva-Cys]-NH₂ | IIa | 1722.847 | 1722.844 | 6.3 | D | D |
| UPAR-417 (SEQ ID NO: 286) | Pent-[Cys-Cit-Lys(DOTA)-Tyr-Aic-Pro-Hyp-Ile-Nva-Cys]-NH₂ | IIa | 1720.832 | 1720.828 | 6.2 | D | D |
| UPAR-418 (SEQ ID NO: 287) | Ac-Leu-[Cys-Cit-Gln-Tyr-Phe-Pro-ala-Ile-Leu-Cys]-NH₂ | Ic | 1365.657 | 1365.771 | 7.1 | A | A |
| UPAR-419 (SEQ ID NO: 288) | Pent-[Cys-Thr-Lys(DOTA)-Tyr-Phe-Aib-Hyp-Ile-Nva-Cys]-NH₂ | IIa | 1640.794 | 1640.783 | 6.6 | B | C |
| UPAR-420 (SEQ ID NO: 289) | Ac-Asp-Leu-[Cys-Arg-Nle-Tyr-Phe-Pro-Ala-leu-Leu-Cys]-NH₂ | Ic | 1464.726 | 1464.725 | 7.3 | C | C |
| UPAR-422 (SEQ ID NO: 290) | Butoc-[Cys-Thr-Lys(DOTA-Kzw)-Tyr-Phe-4Cfp-Hyp-Ile-Nva-Cys]-NH₂ | VId | 1964.910 | 1964.913 | 6.2 | A | A |
| UPAR-423 (SEQ ID NO: 291) | Ac-Asp-Leu- [Cys-Cit-Lys(DOTA)-Tyr-Phe-Pro-Arg-Ile-Leu-Cys]-NH₂ | IIa | 1951.965 | 1952.134 | 7.1 | A | A |
| UPAR-424 (SEQ ID NO: 292) | Butoc-[Cys-Thr-Lys(DOTA-asp)-Tyr-Phe-4Cfp-Hyp-Ile-Nva-Cys]-NH₂ | IIg | 1801.807 | 1801.804 | 6.4 | A | A |
| UPAR-425 (SEQ ID NO: 293) | Pent-[Cys-Cit-Lys(DOTA)-Tyr-Phe-Pro-Gly-Ile-Nva-Cys]-NH₂ | IIa | 1652.805 | 1652.810 | 6.6 | A | A |
| UPAR-426 (SEQ ID NO: 294) | iHex-[Cys-Cit-Gln-Tyr-Phe-Aze-ala-Ile-Leu-Cys]-NH₂ | Ia | 1294.620 | 1294.617 | 7.5 | A | B |
| UPAR-427 (SEQ ID NO: 295) | Hex-Asp-Leu-[Cys-Arg-Lys(DOTA)-Tyr-Phe-Pro-Ala-Ile-Leu-Cys]-NH₂ | IIa | 1921.979 | 1921.899 | 7.6 | A | A |
| UPAR-428 (SEQ ID NO: 296) | H-Nmb-[Cys-Thr-Lys(DOTA)-Tyr-Phe-4Cfp-Hyp-Ile-Nva-Cys]-NH₂ | IIa | 1713.827 | 1713.825 | 5.3 | A | A |
| UPAR-429 (SEQ ID NO: 297) | iHex-[Cys-Cit-Gln-Tyr-Phe-Pro-ala-Ile-Nva-Cys]-NH₂ | Ia | 1294.620 | 1294.710 | 7.2 | A | A |
| UPAR-430 (SEQ ID NO: 298) | Pent-[Cys-Cit-Gln-Tyr-Phe-Pro-Hyp-Ile-Nva-Cys]-en | IIIb | 1365.657 | 1365.656 | 5.6 | B | B |
| UPAR-431 (SEQ ID NO: 299) | Ac-Asp-Leu-[Cys-Arg-Nle-Tyr-Phe-Pro-Pro-Ile-Leu-Cys]-NH₂ | Ic | 1490.741 | 1490.757 | 7.7 | A | A |
| UPAR-432 (SEQ ID NO: 300) | Ac-Asp-Leu- [Cys-Asp-Lys(DOTA)-Tyr-Phe-Pro-ala-Ile-Leu-Cys]-NH₂ | IIa | 1824.843 | 1825.001 | 7.0 | B | C |
| UPAR-433 (SEQ ID NO: 301) | Pent-[Cys-Arg-Gln-Tyr-Phe-Leu-Hyp-Ile-Nva-Cys]-NH₂ | Ia | 1337.662 | 1337.658 | 7.8 | A | A |
| UPAR-434 (SEQ ID NO: 302) | iHex-[Cys-Cit-Asp-Tyr-Phe-Pro-ala-Ile-Leu-Cys]-NH₂ | Ia | 1295.604 | 1295.667 | 7.7 | A | A |
| UPAR-435 (SEQ ID NO: 303) | Pent-[Cys-Cit-Lys(GdDOTA)-Tyr-Phe-Pro-4Tfp-Ile-Nva-Cys]-NH₂ | IX | 1859.724 | 1859.723 | 7.7 | A | A |
| UPAR-436 (SEQ ID NO: 304) | Pent-[Cys-Arg-Gln-Tyr-Phe-Pro-glu-Ile-Nva-Cys]-NH₂ | Ia | 1337.626 | 1337.625 | 6.4 | B | C |
| UPAR-437 (SEQ ID NO: 305) | Pent-[Cys-Arg-Gln-Tyr-Phe-Pro-Hyp-Phg-Nva-Cys]-NH₂ | Ia | 1341.600 | 1341.590 | 7.2 | A | A |
| UPAR-438 (SEQ ID NO: 306) | Pent-[Cys-Arg-Gln-Tyr-Phe-Pro-om-Ile-Nva-Cys]-NH₂ | Ia | 1322.663 | 1322.663 | 5.8 | B | B |
| UPAR-439 (SEQ ID NO: 307) | iHex-[Cys-Cit-Gln-Tyr-Phe-Hyp-ala-Ile-Leu-Cys]-NH₂ | Ia | 1324.631 | 1324.732 | 7.0 | A | B |
| UPAR-440 (SEQ ID NO: 308) | Ac-Asp-Leu-[Cys-Arg-Nle-Tyr- Phe- Pro- Aib- Ile- Leu-Cys]-NH₂ | Ic | 1478.741 | 1478.741 | 7.6 | A | A |
| UPAR-441 (SEQ ID NO: 309) | DOTA-Leu-[Cys-Cit-Gln-Tyr-Phe-Pro-ala-Ile-Leu-Cys]-NH₂ | Ib | 1709.827 | 1709.821 | 6.7 | C | A |
| UPAR-442 (SEQ ID NO: 310) | Pent-[Cys-Arg-Gln-Lys-Phe-Pro-Hyp-Ile-Nva-Cys]-NH₂ | Ia | 1286.663 | 1286.657 | 5.8 | C | C |
| UPAR-444 (SEQ ID NO: 311) | Butoc-[Cys-Thr-Lys(NODAGA)-Dopa-Phe-4Cfp-Hyp-Ile-Nva-Cys]-NH₂ | IId | 1673.748 | 1673.747 | 7.3 | A | A |
| UPAR-445 (SEQ ID NO: 312) | iHex-[Cys-Cit-Cit-Tyr-Phe-Pro-ala-Ile-Leu-Cys]-NH₂ | Ia | 1337.662 | 1337.719 | 7.6 | A | A |
| UPAR-446 (SEQ ID NO: 313) | iHex-[Cys-Cit-Gln-Tyr-Phe-Pro-Aze-Ile-Leu-Cys]-NH₂ | Ia | 1320.636 | 1320.635 | 8.3 | A | A |
| UPAR-447 (SEQ ID NO: 314) | Pent-[Cys-Arg-Gln-Tyr-Phe-Pro-Hyp-Phe-Nva-Cys]-NH₂ | Ia | 1355.615 | 1355.604 | 7.6 | A | A |
| UPAR-448 (SEQ ID NO: 315) | Pent-[Cys-Arg-Lys-Tyr-Phe-Pro-Hyp-Ile-Nva-Cys]-NH₂ | Ia | 1321.668 | 1321.673 | 7.0 | A | A |
| UPAR-449 (SEQ ID NO: 316) | iHex-[Cys-Arg-Gln-Tyr-Phe-Pro-ala-Ile-Leu-Cys]-O2Oc-Lys(DOTA)-NH₂ | IIa | 1967.001 | 1966.999 | 6.9 | D | B |
| UPAR-450 (SEQ ID NO: 317) | Pent-[Cys-Cit-Lys(DOTA)-Tyr-Ocf-Pro-Hyp-Ile-Nva-Cys]-NH₂ | IIa | 1742.793 | 1742.786 | 7.5 | A | A |
| U PAR-451 (SEQ ID NO: 318) | iHex-[Cys-Cit-Gln-Tyr-Phe-Pro-ala-Ile-Abu-Cys]-NH₂ | Ia | 1280.605 | 1280.601 | 7.0 | A | B |
| UPAR-452 (SEQ ID NO: 319) | iHex-[Cys-Cit-Lys(DOTA)-Tyr-Phe-Pro-Aib-Ile-Leu-Cys]-NH₂ | IIa | 1708.868 | 1708.966 | 7.7 | A | A |
| UPAR-453 (SEQ ID NO: 320) | iHex-[Cys-Cit-Gln-Tyr-Phe-Pro-ala-Cbg-Leu-Cys]-NH₂ | Ia | 1306.620 | 1306.620 | 7.4 | A | B |
| UPAR-454 (SEQ ID NO: 321) | Pent-[Cys-Tyr-Gln-Tyr-Phe-Pro-Hyp-Ile-Nva-Cys]-NH₂ | Ia | 1328.593 | 1328.594 | 7.9 | A | A |
| UPAR-455 (SEQ ID NO: 322) | Butoc-[Cys-Thr-Dap(DOTA)-Tyr-Phe-4Cfp-Hyp-Ile-Nva-Cys] - NH₂ | IId | 1644.733 | 1644.730 | 6.3 | A | A |
| UPAR-456 (SEQ ID NO: 323) | Butoc-[Cys-Thr-Dab(DOTA)-Dopa-Phe-4Cfp-Hyp-Ile-Nva-Cys]-NH₂ | IId | 1674.743 | 1674.742 | 5.8 | A | A |
| UPAR-457 (SEQ ID NO: 324) | iHex-[Cys-Arg-Gln-Tyr-Phe-Pro-Apc(GdDOTA)-Ile-Leu-Cys]-NH₂ | IX | 1897.771 | 1897.770 | 7.9 | A | ND |
| UPAR-458 (SEQ ID NO: 325) | iHex-[Cys-Arg-Asp-Tyr-Phe-Pro-Hyp-Ile-Leu-Cys]-NH₂ | Ia | 1336.631 | 1336.631 | 8.0 | A | A |
| UPAR-459 (SEQ ID NO: 326) | Butoc-[Cys-Thr-Lys(DOTA-asp)-Dopa-Phe-4Cfp-Hyp-Ile-Nva-Cys]-NH₂ | IIg | 1817.802 | 1817.807 | 5.8 | A | A |
| UPAR-460 (SEQ ID NO: 327) | Succinyl-Nva-[Cys-Cit-Lys(GdDOTA)-Tyr-Phe-Pro-Hyp-Ile-Nva-Cys]-NH₂ | IX | 1972.755 | 1972.754 | 7.6 | A | A |
| UPAR-461 (SEQ ID NO: 328) | Pent-[Cys-Arg-Gln-Tyr-Phe-Pro-Hyp-Hfe-Nva-Cys]-NH₂ | Ia | 1369.631 | 1369.624 | 7.8 | A | A |
| UPAR-462 (SEQ ID NO: 329) | H-NEtg-[Cys-Cit-Gln-Tyr-Phe-Pro-ala-Ile-Leu-Cys]-NH₂ | IVb | 1295.615 | 1295.614 | 6.5 | C | C |
| UPAR-463 (SEQ ID NO: 330) | iHex-[Cys-Cit-Lys(GdDOTA)-Tyr-Phe-Pro-ala-Ile-Leu-Cys]-NH₂ | IX | 1843.749 | 1843.742 | 8.0 | A | ND |
| UPAR-464 (SEQ ID NO: 331) | nBuCAyl-[Cys-Cit-Lys(LuDOTA)-Tyr-Phe-Pro-Hyp-Ile-Nva-Cys]-NH₂ | IX | 1895.760 | 1895.758 | 7.8 | A | A |
| UPAR-465 (SEQ ID NO: 332) | Pent-[Cys-Arg-Gln-Tyr-Phe-Pro-tyr-Ile-Nva-Cys]-NH₂ | Ia | 1371.647 | 1371.641 | 7.0 | A | A |
| UPAR-466 (SEQ ID NO: 333) | iHex-[Cys-Lys(DOTA)-Gln-Tyr-Phe-Pro-Pro-Ile-Leu-Cys]-NH₂ | IIa | 1691.842 | 1691.841 | 7.9 | A | A |
| UPAR-467 (SEQ ID NO: 334) | iHex-[Cys-arg-Gln-Tyr-Phe-Pro-ala-Ile-Leu-Cys]-NH₂ | Ia | 1307.652 | 1307.649 | 7.6 | D | C |
| UPAR-468 (SEQ ID NO: 335) | iHex-[Cys-Aib-Gln-Tyr-Phe-Pro-ala-Ile-Leu-Cys]-NH₂ | Ia | 1236.604 | 1236.602 | 8.3 | A | B |
| UPAR-469 (SEQ ID NO: 336) | iHex-[Cys-Cit-Gln-Tyr-Phe-Pro-Pip-Ile-Leu-Cys] - NH₂ | Ia | 1348.667 | 1348.666 | 7.9 | B | B |
| UPAR-470 (SEQ ID NO: 337) | Pent-[Cys-Arg-Gln-Tyr-Phe-Nmv-Hyp-Ile-Nva-Cys]-NH₂ | Ia | 1337.662 | 1337.657 | 7.3 | D | D |
| UPAR-471 (SEQ ID NO: 338) | Pent-[Cys-Glu-Lys(DOTA)-Tyr-Phe-Pro-Hyp-Ile-Nva-Cys]-NH₂ | IIa | 1680.789 | 1680.787 | 7.2 | A | A |
| UPAR-473 (SEQ ID NO: 339) | Pent-[Cys-Cit-Lys(DOTA)-Tyr-Phe-Pro-Pro-Ile-Nva-Cys]-NH₂ | IIa | 1692.837 | 1692.835 | 7.4 | A | A |
| UPAR-474 (SEQ ID NO: 340) | iHex-[Cys-Cit-Gln-Tyr-Phe-Pro-ala-Ile-Cha-Cys]-NH₂ | Ia | 1348.667 | 1348.761 | 8.5 | D | D |
| UPAR-475 (SEQ ID NO: 341) | Hex-[Cys-Cit-Gln-Tyr-Phe-Pro-ala-Ile-Leu-Cys]-NH₂ | Ia | 1308.636 | 1308.635 | 8.1 | A | A |
| UPAR-476 (SEQ ID NO: 342) | Butoc-[Cys-Cit-Lys(DOTA)-Tyr-Pcf-4Cfp-Hyp-Chg-Nva-Cys]-NH₂ | IId | 1802.794 | 1802.790 | 8.0 | A | A |
| UPAR-477 (SEQ ID NO: 343) | iHex-[Cys-Cit-Gln-Tyr-Phe-Pro-ala-Ile-Npg-Cys]-NH₂ | Ia | 1322.652 | 1322.723 | 7.8 | C | C |
| UPAR-478 (SEQ ID NO: 344) | Pent-[Cys-Arg-Gln-Amf-Phe-Pro-Hyp-Ile-Nva-Cys]-NH₂ | Ia | 1319.652 | 1319.649 | 7.5 | C | C |
| UPAR-479 (SEQ ID NO: 345) | Butoc-[Cys-Thr-Lys(AF488-Ttds)-Dopa-Phe-4Cfp-Hyp-Ile-Nva-Cys]-NH₂ | VIIIa | 2134.772 | 2134.768 | 7.3 | ND | A |
| UPAR-480 (SEQ ID NO: 346) | Pent-[Cys-Arg-Gln-Tyr-Phe-Pro-Hyp-Ile-Nml-Cys]-NH₂ | Ia | 1349.662 | 1349.659 | 7.4 | D | D |
| UPAR-481 (SEQ ID NO: 347) | Pent-[Cys-Arg-Gln-Tyr-Phe-Pro-Hyp-Ile-Nva-AET] | IIIe | 1278.625 | 1278.623 | 7.1 | A | A |
| UPAR-482 (SEQ ID NO: 348) | Ac-Egz-[Cys-Arg-Gln-Tyr-Phe-Pro-Hyp-Ile-Nva-Cys]-NH₂ | Ic | 1404.668 | 1404.669 | 7.3 | A | A |
| UPAR-483 (SEQ ID NO: 349) | Ac-Nml-[Cys-Arg-Gln-Tyr-Phe-Pro-Hyp-Ile-Nva-Cys]-NH₂ | Ic | 1406.684 | 1406.684 | 7.3 | A | A |
| UPAR-484 (SEQ ID NO: 350) | Ac-Hfe-[Cys-Arg-Gln-Tyr-Phe-Pro-Hyp-Ile-Nva-Cys]-NH₂ | Ic | 1440.668 | 1440.669 | 7.5 | A | B |
| UPAR-485 (SEQ ID NO: 351) | Ac-Aml-[Cys-Arg-Gln-Tyr-Phe-Pro-Hyp-Ile-Nva-Cys]-NH₂ | Ic | 1406.684 | 1406.684 | 7.6 | A | A |
| UPAR-486 (SEQ ID NO: 352) | Ac-Phg-[Cys-Arg-Gln-Tyr-Phe-Pro-Hyp-Ile-Nva-Cys]-NH₂ | Ic | 1412.637 | 1412.638 | 7.0 | ND | A |
| UPAR-487 (SEQ ID NO: 353) | Me-Nml-[Cys-Arg-Gln-Tyr-Phe-Pro-Hyp-Ile-Nva-Cys]-NH₂ | IVa | 1378.689 | 1378.689 | 6.3 | ND | B |
| UPAR-488 (SEQ ID NO: 354) | Ac-Phe-[Cys-Arg-Gln-Tyr-Phe-Pro-Hyp-Ile-Nva-Cys]-NH₂ | Ic | 1449.653 | 1449.659 | 7.6 | ND | A |
| UPAR-489 (SEQ ID NO: 355) | Butoc-[Cys-Thr-Om(GaDOTA)-Pcnf-Phe-4Cfp-Hyp-Ile-Nva-Cys]-NH₂ | IX | 1765.677 | 1765.681 | 6.1 | A | A |
| UPAR-490 (SEQ ID NO: 356) | Butoc-[Cys-Thr-Om(InDOTA)-Pcnf-Phe-4Cfp-Hyp-Ile-Nva-Cys]-NH₂ | IX | 1809.656 | 1809.652 | 6.0 | A | A |
| UPAR-491 (SEQ ID NO: 357) | Butoc-[Cys-Thr-Om(LuDOTA)-Pcnf-Phe-4Cfp-Hyp-Ile-Nva-Cys]-NH₂ | IX | 1871.692 | 1871.692 | 5.9 | A | A |

### Example 40: Plasma stability assay

In order to determine the stability of selected compounds of the invention in human and mouse plasma, a plasma stability assay was carried out. Such plasma stability assay measures degradation of compounds of the present invention in blood plasma. This is an important characteristic of a compound as compounds, with the exception of pro-drugs, which rapidly degrade in plasma, generally show poor *in vivo* efficacy. The results show that those compounds are highly stable in human and mouse plasma. The stability is sufficient for the diagnostic, therapeutic and theragnostic use of these compounds according to the present invention.

The plasma stability samples were prepared by spiking 50 µl plasma aliquots (all sodium citrate 3.7%) with 1 µl of a 0.5 mM compound stock solution in DMSO. After vortexing the samples were incubated in a Thermomixer at 37°C for 0 and 6 hours. After incubation the samples were stored on ice until further treatment. All samples were prepared in duplicates.

A suitable internal standard was added to each sample (1 µl of a 0.5 mM stock solution in DMSO). Protein precipitation was performed using two different methods depending on the compound conditions as indicated in Table 13.
A) 250 µl of acetonitrile containing 1% trifluoroacetic acid was added. After incubation at room temperature for 30 min the precipitate was separated by centrifugation and 150 µl of the supernatant was diluted with 150 µl of 1% aqueous formic acid.
B) 150 µl of a zinc sulphate precipitation agent containing 78% 0.1 M zinc sulphate and 22% acetonitrile was added. After incubation at room temperature for 30 min the precipitate was separated by centrifugation. To 100 µl of the supernatant 10 µl of 1% formic acid was added followed by further incubation at 60°C for 10 min to complete the formation of the zinc chelate, if the compound contains a free DOTA moiety.

The determination of the analyte in the clean sample solutions was performed on an Agilent 1290 UHPLC system coupled to an Agilent 6530 Q-TOF mass spectrometer. The chromatographic separation was carried out on a Phenomenex BioZen XB-C18 HPLC column (50 x 2 mm, 1.7 µm particle size).

Two different elution gradients were applied using a mixture of 0.1% formic acid in water as eluent A and acetonitrile as eluent B:
A) 2% B to 41% in 7 min, 800 µl/min, 40°C.
B) 5% B to 50% B in 6 min, 800 µL/min, 40°C.

Mass spectrometric detection was performed in positive ion ESI mode by scanning the mass range from m/z 50 to 3000 with a sampling rate of 2 / sec.

From the mass spectrometric raw data the ion currents for the double or triple charged monoisotopic signal was extracted for both, the compound and the internal standard.

Quantitation was performed by external matrix calibration with internal standard using the integrated analyte signals.

Additionally, recovery was determined by spiking a pure plasma sample that only contained the internal standard after treatment with a certain amount of the compound.

Carry-over was evaluated by analysis of a blank sample (20% acetonitrile) after the highest calibration sample.

The results of this assay performed on some of the compounds according to the present invention are given in the following Table 13. The result is stated as "% intact compound remaining after 6 h incubation" and means that from the amount of material at the start of the experiment the stated percentage is detected as unchanged material at the end of the experiment by LC-MS quantification. Since all compounds are more than 50% intact after 6 h they are considered as stable enough for diagnostic and therapeutic applications.

**Table 13: Results of the plasma stability assay**

| | **Protein precipitation method** | **LC-MS method** | **% intact compound remaining after 6h incubation** | |
|---|---|---|---|---|
| **Compound** | | | **Human plasma** | **Mouse plasma** |
| UPAR-351 | A | B | ND | > 95% |
| UPAR-427 | A | B | ND | > 95% |
| UPAR-176 | A | A | ND | 57% |
| UPAR-408 | A | B | ND | 75% |
| UPAR-463 | A | B | ND | 81% |
| UPAR-457 | A | B | ND | 64% |
| UPAR-338 | A | B | ND | 73% |
| UPAR-418 | A | B | ND | 85% |
| UPAR-382 | B | B | ND | 78% |
| UPAR-389 | A | B | ND | 95% |
| UPAR-174 | A | B | ND | 71% |
| UPAR-306 | B | A | ND | 95% |
| UPAR-189 | B | A | ND | > 95% |
| UPAR-434 | A | B | ND | 90% |
| UPAR-261 | B | A | ND | 82% |
| UPAR-293 | B | B | ND | > 95% |
| UPAR-441 | B | A | ND | 83% |
| UPAR-245 | B | A | ND | 74% |
| UPAR-229 | B | A | ND | 90% |
| UPAR-216 | B | A | ND | 84% |
| UPAR-278 | A | A | ND | 87% |
| UPAR-301 | A | B | > 95% | 91% |
| UPAR-138 | B | A | ND | 91% |
| UPAR-403 | A | A | ND | 87% |
| UPAR-275 | A | B | ND | 70% |
| UPAR-200 | B | B | ND | > 95% |
| UPAR-249 | A | B | 64% | 22% |
| UPAR-276 | B | B | > 95% | 89% |

| | | | | |
|---|---|---|---|---|
| ND = not determined | | | | |

### Example 41: Proteolytic stability assay against NEP

Peptides are often sensitive to proteolytic cleavage in the blood (Werle and Bernkop-Schnürch 2006). If peptides degrade rapidly, in vivo elimination could be dominated by metabolism. Since metabolites often demonstrate poor binding to the target, performance of the compound during radiopharmaceutical use (diagnostic or therapeutic) can be significantly decreased. Therefore, evaluating the stability of the compound against proteases in the early stages of compound development is essential.

Two classes of proteases and peptidases can be found in the blood circulation: soluble and membrane-bound proteases. The conduction of a plasma stability study only covers the impact of the soluble fraction on compound stability, since all membrane-bound proteases are separated during blood collection (proteins expressed on the surface of blood vessels) or during plasma generation (proteins expressed on blood cells). Thus, the result of a plasma stability assay is not always predictive for in vivo behavior.

Membrane-bound peptidases are mainly responsible for the activation and deactivation of bioactive peptides, such as hormones, cytokines, and neurohormones (Antczak, De Meester et al. 2001). They activate hormones by cleavage of a pre-hormone and deactivate them through another cleavage step. Activation is usually very specific, as well as the activation peptidases, including ACE, ECE, and DPPIV. With a specific activation, signaling remains specific even if deactivation is more unspecific. Thus, several peptidases that are mainly responsible for hormone deactivation reveal a broad substrate pattern (e,g, neutral endopeptidase, meprines). It is not possible to assess all peptidases during early compound development. Therefore, more importance should be focused on those peptidases with broad substrate patterns, since it is more likely that the peptide sequence fits into this pattern.

NEP (neutral endopeptidase, EC 3.4.24.11, CD10, CALLA, endopeptidase 24.11, enkephalinase, neprilysin, membrane metallopeptidase A) is a membrane-bound metallopeptidase. It is expressed on neutrophils and highly active in blood (Antczak, De Meester et al. 2001). In addition, the cleavage pattern of NEP is very broad, covering many different peptide hormones, with more than 50 different natural substrates already described (Bayes-Genis, Barallat et al. 2016). NEP preferably cleaves amide bonds between a hydrophilic and hydrophobic amino acid (preferably leucine or phenylalanine), even in small cyclic peptides such as CNP. Plamboeck et al. (2005) showed a significant impact of NEP caused cleavage on the pharmacokinetic behavior of peptides (Plamboeck, Holst et al. 2005).

To assess the stability of compounds against NEP, the compounds were subjected to an in vitro assay based on the protocol described by Edelson et al. (Edelson, Makhlina et al. 2013).

Recombinant soluble human NEP (BioTechne, Wiesbaden, Germany) at a concentration of 100 ng/mL was mixed with the compound (10 µM) and a stable internal standard (10 µM) and incubated at 37°C. After several time points, samples were taken and analyzed with LC-MS.

The determination of the analyte in the clean sample solutions was performed on an Agilent 1290 UHPLC system coupled to an Agilent 6530 Q-TOF mass spectrometer. The chromatographic separation was carried out on a Phenomenex BioZen XB-C18 HPLC column (50 × 2 mm, 1.7 µm particle size) with gradient elution using a mixture of 0.1% formic acid in water as eluent A and acetonitrile as eluent B (2% B to 41% in 7 min, 800 µl/min, 40°C). Mass spectrometric detection was performed in positive ion ESI mode by scanning the mass range from m/z 50 to 3000 with a sampling rate of 2 / sec.

From the mass spectrometric raw data the ion currents for the double or triple charged monoisotopic signal was extracted for both, the compound and the internal standard.

Quantitation was performed by external matrix calibration with internal standard using the integrated analyte signals.

The activity of NEP was examined using a commercially available chromogenic substrate of NEP.

The results of this assay performed on some of the compounds according to the present invention are given in Table 14. The result is stated as "% intact compound remaining after 24 h incubation" and means that from the amount of material at the start of the experiment the stated percentage is detected as unchanged material at the end of the experiment by LC-MS quantification. Since all compounds are more than 50% intact after 24 h they are considered as stable enough for diagnostic and therapeutic applications.

**Table 14: Results of the NEP stability assay**

| **Compound** | **% intact compound remaining after 24h incubation** |
|---|---|
| UPAR-463 | 88% |
| UPAR-338 | 70% |
| UPAR-364 | > 95% |
| UPAR-382 | > 95% |
| UPAR-389 | > 95% |
| UPAR-174 | 90% |
| UPAR-306 | 88% |
| UPAR-189 | 94% |
| UPAR-434 | > 95% |
| UPAR-261 | 86% |
| UPAR-293 | > 95% |
| UPAR-441 | 61% |
| UPAR-245 | 72% |
| UPAR-229 | 84% |
| UPAR-466 | > 95% |
| UPAR-138 | 94% |
| UPAR-403 | 95% |
| UPAR-233 | > 95% |
| UPAR-346 | 89% |
| UPAR-246 | 89% |
| UPAR-276 | 88% |

### Example 42: ¹¹¹In-labeling of selected compounds

In order to serve as a diagnostically, therapeutically, or theranostically active agent, a compound needs to be labeled with a radioactive isotope. The labeling procedure needs to be appropriate to ensure a high radiochemical yield and purity of the radiolabeled compound of the invention. This example shows that the compounds of the present invention are appropriate for radiolabeling and can be labeled in high radiochemical yield and purity.

~90 MBq of ¹¹¹InCl₃ (in 0.02 M HCl; Curium, Germany) were mixed with 1 nmol of compound (UPAR-176, UPAR-256, UPAR-189, 200 µM stock solution diluted from 10 mM stock solution in DMSO with 0.1 M HEPES) per 30 MBq and radiolabeling buffer (1 M sodium acetate pH 5.5) at a final buffer concentration of 0.1 M. The mixture was heated to 80 °C for 25 min. After cooling down, ascorbic acid (Woerwag Pharma, Germany), DTPA (Heyl, Germany) and TWEEN-20 were added at a final concentration of 25 mg/mL, 0.1 mg/mL and 0.1%, respectively.

~90 MBq of ¹¹¹InCl₃ (in 0.02 M HCl; Curium, Germany) were mixed with 1 nmol of compound (200 µM stock solution diluted from 10 mM stock solution in DMSO with 0.1 M HEPES) per 30 MBq and radiolabeling buffer (1 M ammonium acetate pH 5.5) at a final buffer concentration of 0.1 M. The mixture was heated to 90 °C for 15 min. After cooling down, ascorbic acid (Woerwag Pharma, Germany), DTPA (Heyl, Germany) and TWEEN-20 were added at a final concentration of 25 mg/mL, 0.1 mg/mL and 0.1%, respectively.

Radiochemical purity was analyzed by HPLC. 5 µL of diluted labeling solution was analyzed with a Poroshell SB-C18 2.7 µm, 2.1 × 50 mm (Agilent). Eluent A: HzO, 0.1 % TFA eluent B: ACN, gradient from 5% B to 70% B within 15 min, flow rate 0.5 mL/min; detector: NaI (Raytest), DAD 230 nm. The peak eluting with the dead volume represents free radionuclide, the peak eluting with the peptide-specific retention time as determined with an unlabeled sample represents radiolabeled compound. Radiochemical purity was usually > 90% at end of synthesis. Exemplary radiochemical purities for ¹¹¹In-labeled compounds are shown in Table 15. An exemplary radiochromatogram is shown in Fig. 3 with all peaks labeled with their retention times.

**Table 15: Radiochemical purity of ¹¹¹In-labeled compounds at end of synthesis as measured by HPLC.**

| **Compound ID** | **HPLC retention time [min]** | **HPLC area%** |
|---|---|---|
| ¹¹¹In-UPAR-176 | 9.09 | 94.7 |
| ¹¹¹In-UPAR-256 | 9.12 | 94.0 |
| ¹¹¹In-UPAR-382 | 9.61 | 97.9 |
| ¹¹¹In-UPAR-389 | 9.42 | 91.4 |
| ¹¹¹In-UPAR-306 | 8.29 | 94.4 |
| ¹¹¹In-UPAR-189 | 8.83 | 95.6 |
| ¹¹¹In-UPAR-261 | 8.91 | 93.2 |
| ¹¹¹In-UPAR-229 | 8.98 | 96.4 |
| ¹¹¹In-UPAR-145 | 9.27 | 96.2 |
| ¹¹¹In-UPAR-466 | 9.60 | 96.8 |
| ¹¹¹In-UPAR-471 | 8.94 | 97.8 |
| ¹¹¹In-UPAR-301 | 8.95 | 97.1 |
| ¹¹¹In-UPAR-399 | 8.32 | 96.2 |
| ¹¹¹In-UPAR-403 | 8.25 | 98.3 |
| ¹¹¹In-UPAR-325 | 8.59 | 98.4 |
| ¹¹¹In-UPAR-377 | 8.76 | 96.1 |
| ¹¹¹In-UPAR-253 | 6.74 | 99.0 |
| ¹¹¹In-UPAR-233 | 9.44 | 97.5 |
| ¹¹¹In-UPAR-275 | 9.06 | 97.5 |
| ¹¹¹In-UPAR-383 | 9.63 | 95.3 |
| ¹¹¹In-UPAR-200 | 9.32 | 93.7 |
| ¹¹¹In-UPAR-192 | 9.04 | 96.9 |
| ¹¹¹In-UPAR-165 | 8.03 | 98.3 |
| ¹¹¹In-UPAR-249 | 8.79 | 98.5 |
| ¹¹¹In-UPAR-181 | 8.99 | 90.3 |
| ¹¹¹In-UPAR-424 | 9.07 | 96.9 |
| ¹¹¹In-UPAR-422 | 8.31 | 97.8 |
| ¹¹¹In-UPAR-280 | 8.37 | 96.3 |
| ¹¹¹In-UPAR-456 | 8.46 | 97.6 |
| ¹¹¹In-UPAR-199 | 8.38 | 97.4 |
| ¹¹¹In-UPAR-276 | 8.35 | 98.0 |
| ¹¹¹In-UPAR-294 | 8.68 | 97.9 |

### Example 43: ⁶⁸Ga-labeling of selected compounds

300 µL Ga-68 eluate (80 MBq; 0.1 M HCl) was mixed with radiolabeling buffer (2 M ammonium acetate buffer containing 25 mg/mL ascorbic acid pH 5.5) at a final buffer concentration of 0.2 M. Then 1 nmol of compound (200 µM stock solution diluted from 10 mM stock solution in DMSO with ultrapure water) per 20 MBq was added. The mixture was kept standing at room temperature (UPAR-250) for 15 minutes or 95 °C (UPAR-249, UPAR-276) for 10 min. After cooling down, 5 µL of a 5 mM EDTA solution was added. The reaction mixture was then transferred onto a pre-conditioned (50 µL absolute ethanol followed by 500 µL water) Water Oasis HLB 1 cc Vac cartridge. The column was washed with 200 µL water and the product eluted in 25 µL fractions of absolute ethanol into vials containing each 200 µL PBS (1X) buffer, 23 µL 25 mg/mL ascorbic acid and 2 µL 5 mM EDTA solution. The activity of each fraction was measured and the radiochemical purity of the 2-3 fractions containing the highest amount of radioactivity was determined and used for further experimentation.

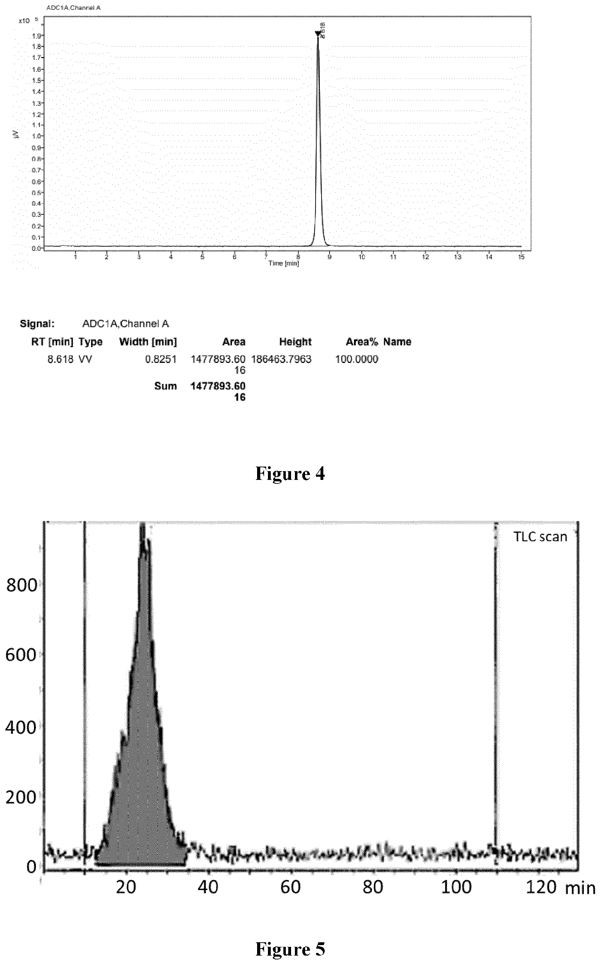

Radiochemical purity was analyzed by HPLC. 5 µL of radiolabeled product solution after cartridge purification was analyzed with a Poroshell SB-C18 2.7 µm, 2.1 × 50 mm (Agilent). Eluent A: HzO, 0.1 % TFA eluent B: ACN, gradient from 5% B to 70% B within 15 min, flow rate 0.5 mL/min; detector: NaI (Raytest), DAD 230 nm. The peak eluting with the dead volume represents free radionuclide, the peak eluting with the peptide-specific retention time as determined with an unlabeled sample represents radiolabeled compound. Radiochemical purity was usually > 90% at end of synthesis. Exemplary radiochemical purities for ⁶⁸Ga-labeled compounds are shown in Table 16 An exemplary radiochromatogram is shown in Fig. 4 with all peaks labeled with their retention times.

**Table 16: Radiochemical purity of ⁶⁸Ga- labeled compounds at end of synthesis as measured by HPLC.**

| **Compound ID** | **HPLC retention time [min]** | **HPLC area%** |
|---|---|---|
| ⁶⁸Ga-UPAR-249 | 8.73 | 99.4 |
| ⁶⁸Ga-UPAR-276 | 8.62 | 100 |
| ⁶⁸Ga-UPAR-250 | 8.67 | 100 |

### Example 44: ¹⁷⁷Lu-labeling of UPAR-249

An aliquot containing the desired amount of radioactivity of ¹⁷⁷LuCl₃ (in 0.04 M HCl; Isotopia, Israel) was mixed with the 3.5-fold volume of 1 M ammonium acetate (pH 5.5). Then 1 nmol of compound (200 µM stock solution diluted from 10 mM stock solution in DMSO with ultrapure water) per 30 MBq was added. The mixture was heated to 90 °C for 20 min. After cooling down, the reaction was quenched with equal volume of 50 mg/mL ascorbic acid: 10 mM DTPA (10:1) and formulated in PBS.

Radionuclide incorporation was analyzed by TLC (Whatman paper; 0.1 M EDTA) as > 99% at end of synthesis. An exemplary chromatogram is shown in Fig. 5.

### Example 45: Imaging studies

Radioactively labeled compounds can be detected by imaging methods such as SPECT and PET. Furthermore, the data acquired by such techniques can be confirmed by direct measurement of radioactivity contained in the individual organs prepared from an animal injected with a radioactively labeled compound of the disclosure. Thus, the biodistribution (the measurement of radioactivity in individual organs) of a radioactively labeled compound can be determined and analyzed. This example shows that the compounds of the present disclosure show a biodistribution appropriate for diagnostic imaging and therapeutic treatment of tumors.

### SPECT/CT Imaging

All animal experiments were conducted in compliance with the German or Greece animal protection laws. Female NMRI nude mice (6-8 weeks old, Janvier Labs, France) were inoculated with 1×10⁷ HEK-uPAR cells in the right shoulder. Cells were injected in serum free media with Matrigel (1:1). When tumors reached an appropriate size, the mice received ~30 MBq ¹¹¹In-labelled or ¹⁷⁷Lu-labelled compounds of the disclosure (diluted to ~ 100 µL with PBS) administered intravenously via the tail vein. SPECT/CT images were obtained using the γ-CUBE and the X-CUBE system (MOLECUBES NV, Gent, Belgium) applying exemplarily the following acquisition and reconstruction parameters (Table 17 and 18).

**Table 17: Acquisition and reconstruction parameters SPECT (γ-CUBE)**

| **SPECT parameters ¹¹¹In** | | | |
|---|---|---|---|
| **Animals per bed** | 2 or 3 | | |
| **Type of SPECT scanning** | Spiral, whole body | | |

| | **Peaks (KeV)** | | **Energy window (%)** |
|---|---|---|---|
| **Energy Window (s)** | **Peak 1** | 171.28 | ±10% |
| | **Peak 2** | 245.35 | ±10% |
| **Reconstruction** | Spiral protocol, MLEM algorithm, 500 µm resolution | | |

| **SPECT parameters ¹⁷⁷Lu** | | | |
|---|---|---|---|
| **Animals per bed** | 2 | | |
| **Type of SPECT scanning** | Spiral, whole body | | |

| | **Peaks (KeV)** | | **Energy window (%)** |
|---|---|---|---|
| **Energy Window (s)** | **Primary** | 113 | ±10% |
| | **Secondary** | 210 | ±10% |
| **Reconstruction** | Spiral protocol, MLEM algorithm, 500 µm resolution | | |

**Table 18: Acquisition and reconstruction parameters for CT (X-CUBE).**

| **CT Parameters** | |
|---|---|
| **Animals per bed** | 2 or 3 |
| **Type of CT scanning** | Spiral high-resolution |
| **Pitch** | 1.4 |
| **X-ray power (kVp)** | 50 |
| **Exposure time (ms)** | 32 |
| **Reconstruction resolution ((µm)** | 100 µm or 200 µm |
| **Binning** | 1 |

Imaging data were saved as DICOM files and analysed using VivoQuant^{™} software (Invicro, Boston, USA). Two to four animals were used per time point. Results are expressed as a percentage of injected dose per gram of tissue (%ID/g).

### PET/CT Imaging

All animal experiments were conducted in compliance with the German or Greece animal protection laws. Female NMRI nude mice (6-8 weeks old, Janvier Labs, France) were inoculated with 1×10⁷ HEK-uPAR cells in the right shoulder. When tumors reached an appropriate size, the mice received ~ 2-4 MBq ⁶⁸Ga-labelled compounds of the disclosure (diluted to ~ 100 µL with PBS) administered intravenously via the tail vein.

PET imaging for was performed 15 minutes, 1 hour, and 3 hours post-injection using either a β*-eye* tomographic system (BIOEMTECH, Athens, Greece) or a β-CUBE (MOLECUBES NV, Gent, Belgium). CT imaging was performed using a X-CUBE (MOLECUBES NV, Gent, Belgium).

**Table 19: Acquisition and reconstruction parameters for PET B-eye tomographic system**

| **PET parameters** | | | |
|---|---|---|---|
| **Animals per bed** | 1 | | |
| **Photodetectors** | Silicon Photomultipliers | | |
| **Scintillators** | LYSO:Ce | | |

| **Energy Window (s)** | **Peaks (KeV)** | | **Energy window (%)** |
|---|---|---|---|
| | **Peak** | 511 | ±11.8% |
| **Sensitivity** | 3.2% at the center of FOV | | |
| **Spatial resolution** | 1.3mm at the center of FOV | | |
| **Time resolution** | 450 ps FWHM/TOF | | |
| **Dynamic range** | 0.1 up to 10 MBq | | |
| **Reconstruction** | MLEM algorithm, 1mm Point Spread Function (PSF) modeling, | | |

**Table 20: Acquisition and reconstruction parameters for PET β-CUBE (MOLECUBES).**

| **PET parameters** | | | |
|---|---|---|---|
| **Animals per bed** | | | 4 (mouse hotel, whole body) |
| **Photodetectors** | | | Silicon Photomultipliers |
| **Scintillators** | | | LYSO:Ce |

| **Energy Window (s)** | **Peaks (KeV)** | | **Energy resolution (%)** |
|---|---|---|---|
| | **Peak** | 511 | ±13.8% |
| **Sensitivity** | Measured peak sensitivity is 12.38% | | |
| **Spatial resolution** | 0.753 mm at the center of FOV | | |
| **Reconstruction** | OSEM algorithm, 400 µm | | |

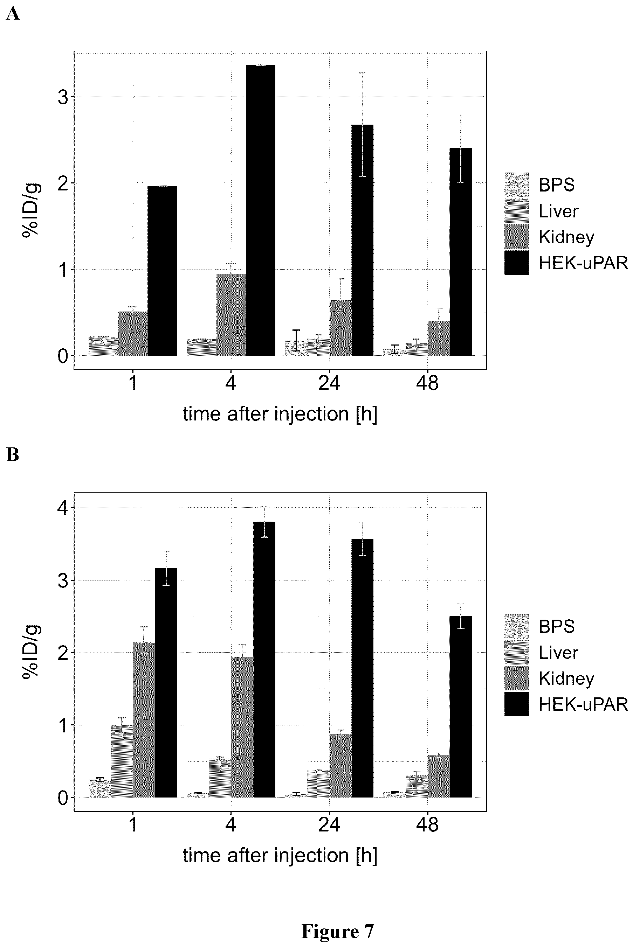

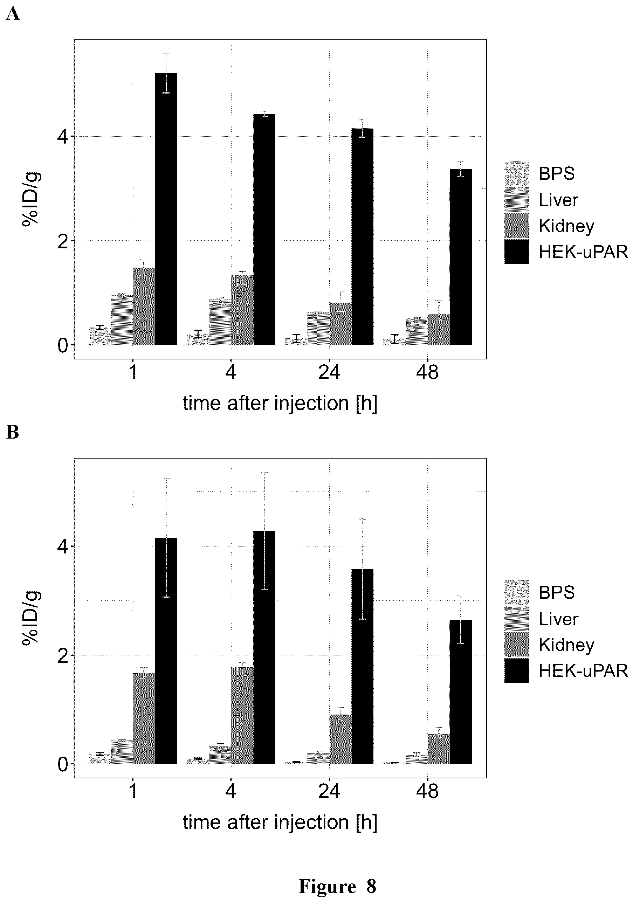

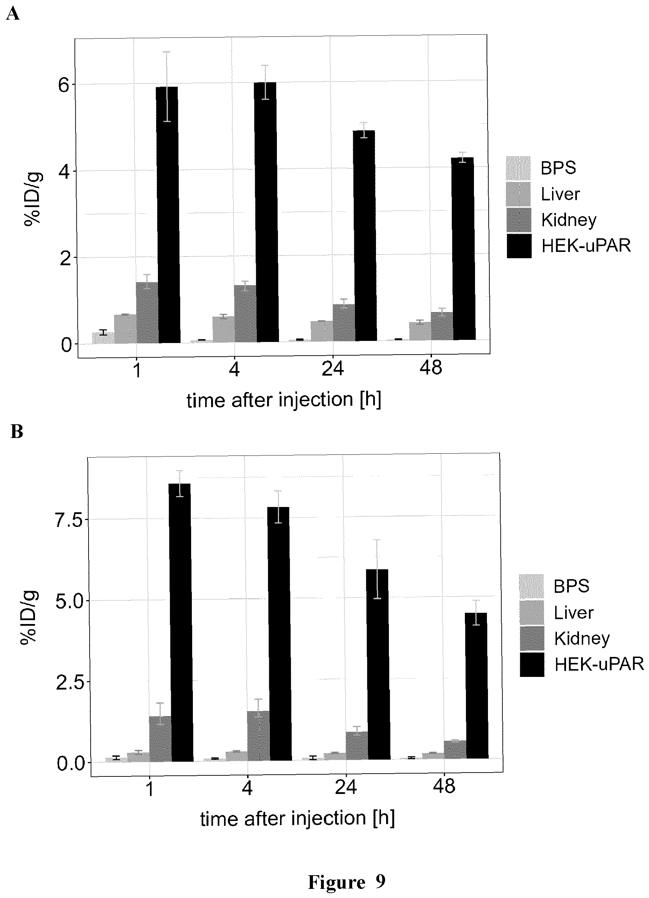

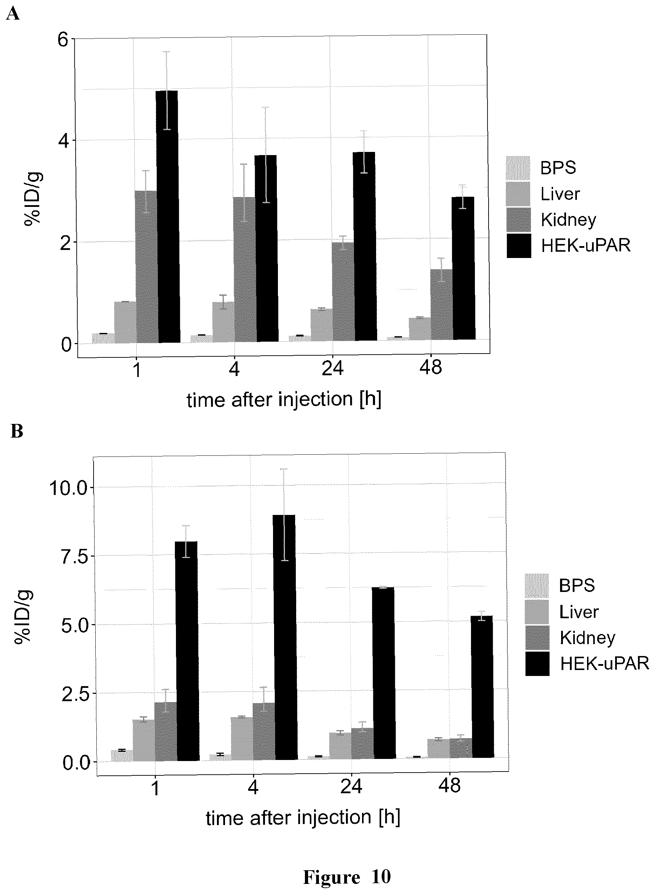

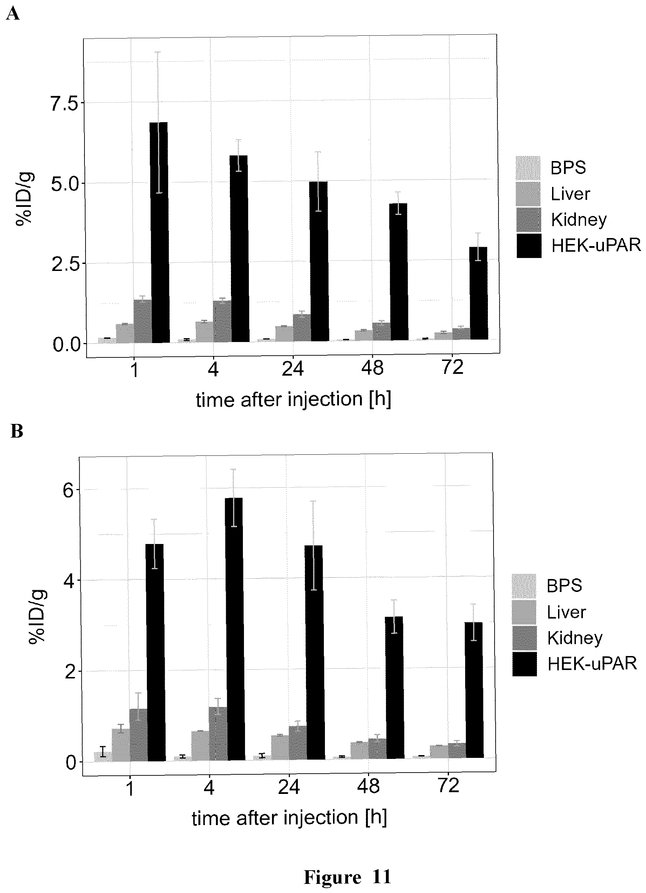

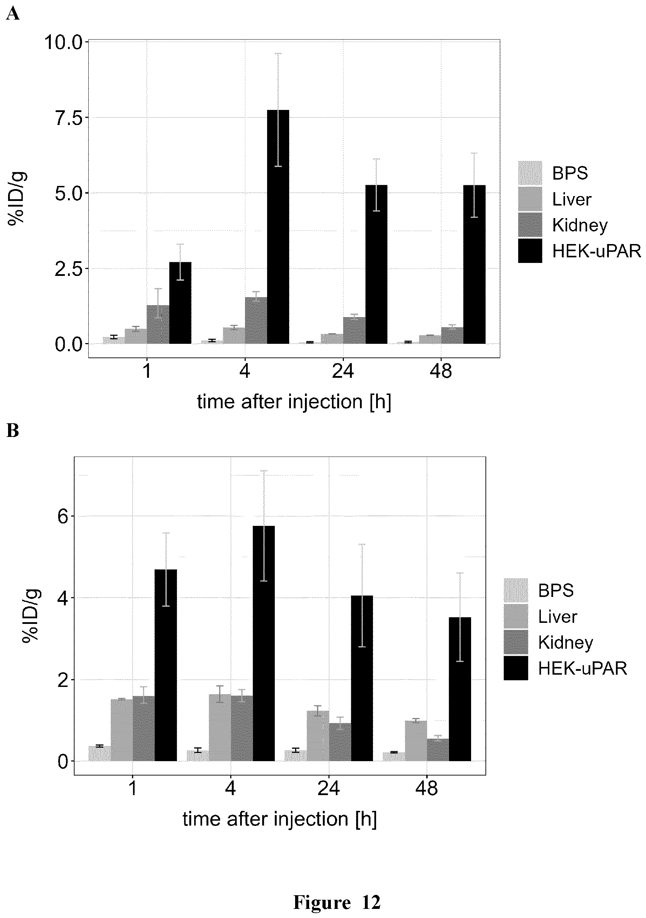

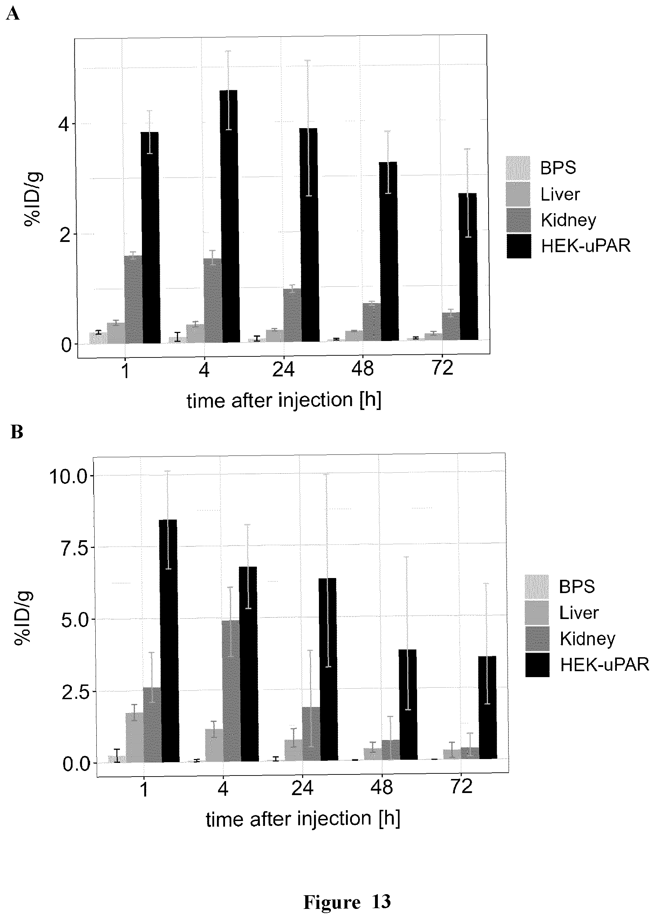

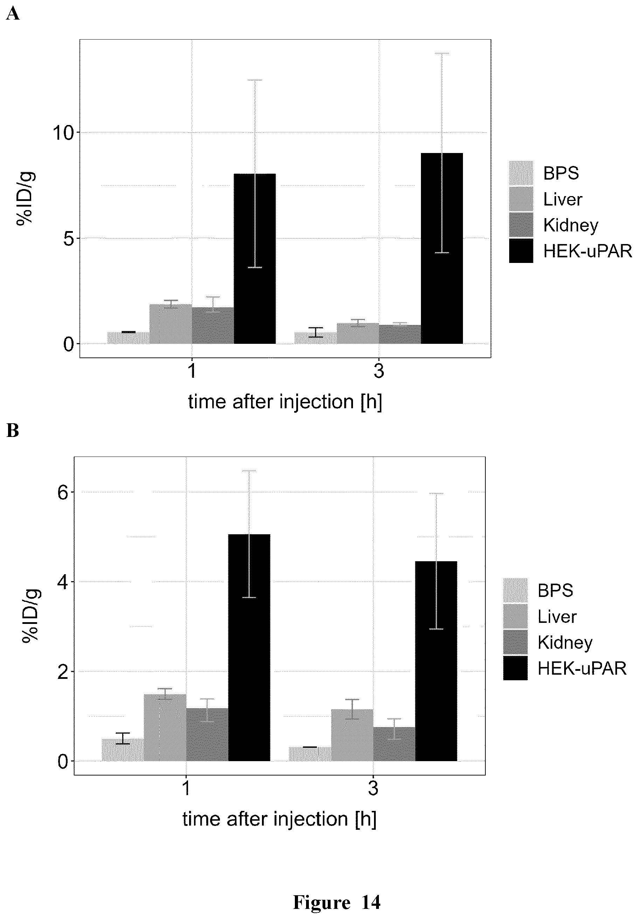

For quantification, VivoQuant^{™} software (Invicro, Boston, USA) was used to draw regions of interest (ROIs) in relevant tissues. A ROI was drawn over the left ventricle of the heart, serving as blood pool surrogate (BPS). Two to four animals were used per time point. Results are expressed as a percentage of injected dose per gram of tissue (%ID/g). Representative results are shown in Fig. 7 to 14. It can be observed that the compounds of the invention accumulate rapidly, i.e., as early as 1h post injection, in the uPAR expressing tissues (xenografts), as shown in the ¹¹¹In-SPECT/CT (Fig. 7 - 13A), ¹⁷⁷Lu-SPECT/CT (Fig. 13B) and 68Ga-PET/CT imaging experiments (Fig. 14). Furthermore, the retention of radioactivity in the tumor (target) tissues was confirmed up to 72 hours after injection (Fig. 11 & 13). This result shows that the specific accumulation of radioactivity to tumor tissues is retained for at least three days post injection. Importantly, little or no radioactivity was found in healthy tissues such as kidney, liver or the blood pool surrogate (BPS), indicating that the compounds of the invention achieve excellent biodistribution, i.e., high accumulation in the tumor tissues and low or no accumulation in healthy tissues, leading to favorable tumor-to-healthy tissue ratios.These results therefore demonstrate the suitability of the compounds of the invention as a diagnostic and/or therapeutic agent.

Imaging data were saved as DICOM files and analysed using VivoQuant^{™} software (Invicro, Boston, USA). Two to four animals were used per time point. Results are expressed as a percentage of injected dose per gram of tissue (%ID/g).

### References

The disclosure of each and any document recited herein is incorporated by reference.
Aime, S., A. Barge, M. Botta, M. Fasano, J. D. Ayala and G. Bombieri (1996). "Crystal structure and solution dynamics of the lutetium(III) chelate of DOTA." Inorganica Chimica Acta 246: 423-429.
Akdogan, O., A. Atak Yucel, Z. Gok Sargin, C. Sonmez, G. Esendagli Yilmaz and S. Ozenirler (2019). "Evaluation of Plasma Urokinase-Type Plasminogen Activator Receptor (UPAR) in Patients With Chronic Hepatitis B, C and Non-Alcoholic Fatty Liver Disease (NAFLD) as Serological Fibrosis Marker." J Clin Exp Hepatol 9(1): 29-33.
Alfano, M., N. Sidenius, B. Panzeri, F. Blasi and G. Poli (2002). "Urokinase-urokinase receptor interaction mediates an inhibitory signal for HIV-1 replication." Proc Natl Acad Sci U S A 99(13): 8862-8867.
AlHokbany, N., I. AlJammaz, B. AlOtaibi, Y. AlMalki, B. AlJammaz and S. M. Okarvi (2022). "Development of new copper-64 labeled rhodamine: a potential PET myocardial perfusion imaging agent." EJNMMI Radiopharm Chem 7(1): 19.
Allott, L., C. Da Pieve, J. Meyers, T. Spinks, D. M. Ciobota, G. Kramer-Marek and G. Smith (2017). "Evaluation of DFO-HOPO as an octadentate chelator for zirconium-89." Chem Commun (Camb) 53(61): 8529-8532.
Altintas, I., J. Eugen-Olsen, S. Seppala, J. Tingleff, M. A. Stauning, N. O. El Caidi, S. Elmajdoubi, H. Gamst-Jensen, M. B. Lindstrom, L. J. H. Rasmussen, K. T. Kristiansen, C. Rasmussen, J. O. Nehlin, T. Kallemose, H. Hyppola and O. Andersen (2021). "suPAR Cut-Offs for Risk Stratification in Patients With Symptoms of COVID-19." Biomark Insights 16: 11772719211034685.
Altschul, S. F., W. Gish, W. Miller, E. W. Myers and D. J. Lipman (1990). "Basic local alignment search tool." J Mol Biol 215(3): 403-410.
Altschul, S. F., T. L. Madden, A. A. Schaffer, J. Zhang, Z. Zhang, W. Miller and D. J. Lipman (1997). "Gapped BLAST and PSI-BLAST: a new generation of protein database search programs." Nucleic Acids Res 25(17): 3389-3402.
Amor, C., J. Feucht, J. Leibold, Y. J. Ho, C. Zhu, D. Alonso-Curbelo, J. Mansilla-Soto, J. A. Boyer, X. Li, T. Giavridis, A. Kulick, S. Houlihan, E. Peerschke, S. L. Friedman, V. Ponomarev, A. Piersigilli, M. Sadelain and S. W. Lowe (2020). "Senolytic CAR T cells reverse senescence-associated pathologies." Nature 583(7814): 127-132.
Andreasen, P. A., R. Egelund and H. H. Petersen (2000). "The plasminogen activation system in tumor growth, invasion, and metastasis." Cell Mol Life Sci 57(1): 25-40.
Antczak, C., I. De Meester and B. Bauvois (2001). "Ectopeptidases in pathophysiology." Bioessays 23(3): 251-260.
Appella, E., E. A. Robinson, S. J. Ullrich, M. P. Stoppelli, A. Corti, G. Cassani and F. Blasi (1987). "The receptor-binding sequence of urokinase. A biological function for the growth-factor module of proteases." Journal of Biological Chemistry 262(10): 4437-4440.
Archibald, S. J. and L. Allott (2021). "The aluminium-[(18)F]fluoride revolution: simple radiochemistry with a big impact for radiolabelled biomolecules." EJNMMI Radiopharm Chem 6(1): 30.
Babich, J. W. and A. J. Fischman (1995). "Effect of "co-ligand" on the biodistribution of 99mTc-labeled hydrazino nicotinic acid derivatized chemotactic peptides." Nucl Med Biol 22(1): 25-30.
Babich, J. W., H. Solomon, M. C. Pike, D. Kroon, W. Graham, M. J. Abrams, R. G. Tompkins, R. H. Rubin and A. J. Fischman (1993). "Technetium-99m-labeled hydrazino nicotinamide derivatized chemotactic peptide analogs for imaging focal sites of bacterial infection." J Nucl Med 34(11): 1964-1974.
Bajpai, A. and J. B. Baker (1985). "Urokinase binding sites on human foreskin cells. Evidence for occupancy with endogenous urokinase." Biochem Biophys Res Commun 133(3): 994-1000.
Banerjee, S. R., P. Schaffer, J. W. Babich, J. F. Valliant and J. Zubieta (2005). "Design and synthesis of site directed maleimide bifunctional chelators for technetium and rhenium." Dalton Trans(24): 3886-3897.
Barge, A., E. Cappelletti, G. Cravotto, A. Ferrigato, L. Lattuada, F. Marinoni and L. Tei (2009). "Synthesis of functionalised HP-DO3A chelating agents for conjugation to biomolecules." Org Biomol Chem 7(18): 3810-3816.
Bayes-Genis, A., J. Barallat and A. M. Richards (2016). "A Test in Context: Neprilysin: Function, Inhibition, and Biomarker." Journal of the American College of Cardiology 68(6): 639-653.
Brechbiel, M. W. and O. A. Gansow (1991). "Backbone-substituted DTPA ligands for 90Y radioimmunotherapy." Bioconjug Chem 2(3): 187-194.
Bum-Erdene, K., D. Liu, D. Xu, M. K. Ghozayel and S. O. Meroueh (2021). "Design and Synthesis of Fragment Derivatives with a Unique Inhibition Mechanism of the uPAR.uPA Interaction." ACS Med Chem Lett 12(1): 60-66.
Cai, Z. and C. J. Anderson (2014). "Chelators for copper radionuclides in positron emission tomography radiopharmaceuticals." J Labelled Comp Radiopharm 57(4): 224-230.
Campbell, D. B., R. D'Oronzio, K. Garbett, P. J. Ebert, K. Mimics, P. Levitt and A. M. Persico (2007). "Disruption of cerebral cortex MET signaling in autism spectrum disorder." Ann Neurol 62(3): 243-250.
Campbell, D. B., C. Li, J. S. Sutcliffe, A. M. Persico and P. Levitt (2008). "Genetic evidence implicating multiple genes in the MET receptor tyrosine kinase pathway in autism spectrum disorder." Autism Res 1(3): 159-168.
Cantero, D., H. Friess, J. Deflorin, A. Zimmermann, M. A. Brundler, E. Riesle, M. Korc and M. W. Buchler (1997). "Enhanced expression of urokinase plasminogen activator and its receptor in pancreatic carcinoma." Br J Cancer 75(3): 388-395.
Carlsen, E. A., M. Loft, A. Loft, A. K. Berthelsen, S. W. Langer, U. Knigge and A. Kjaer (2022). "Prospective phase II trial of prognostication by (68)Ga-NOTA-AE105 uPAR PET in patients with neuroendocrine neoplasms: Implications for uPAR targeted therapy." J Nucl Med: jnumed.121.263177.
Chang, C. A., H. Y. Lee and C. L. Chen (2013). "Simulated annealing and density functional theoretical prediction of macrocyclic ligand conformations, protonation sites and complex metal-ligand exchange reaction directions." Dalton Trans 42(18): 6397-6409.
Chebotareva, N., A. Vinogradov, V. Cao, A. Gindis, A. Berns, I. Alentov and N. Sergeeva (2022). "Serum levels of plasminogen activator urokinase receptor and cardiotrophin-like cytokine factor 1 in patients with nephrotic syndrome." Clin Nephrol 97(2): 103-110.
Colaprico, A., T. C. Silva, C. Olsen, L. Garofano, C. Cava, D. Garolini, T. S. Sabedot, T. M. Malta, S. M. Pagnotta, I. Castiglioni, M. Ceccarelli, G. Bontempi and H. Noushmehr (2016). "TCGAbiolinks: an R/Bioconductor package for integrative analysis of TCGA data." Nucleic Acids Res 44(8): e71. Conese, M., A. Nykjaer, C. M. Petersen, O. Cremona, R. Pardi, P. A. Andreasen, J. Gliemann, E. I. Christensen and F. Blasi (1995). "alpha-2 Macroglobulin receptor/Ldl receptor-related protein(Lrp)-dependent internalization of the urokinase receptor." J Cell Biol 131(6 Pt 1): 1609-1622.
Cortese, K., M. Sahores, C. D. Madsen, C. Tacchetti and F. Blasi (2008). "Clathrin and LRP-1-independent constitutive endocytosis and recycling of uPAR." PLoS One 3(11): e3730.
Cusnir, R., C. Imberti, R. C. Hider, P. J. Blower and M. T. Ma (2017). "Hydroxypyridinone Chelators: From Iron Scavenging to Radiopharmaceuticals for PET Imaging with Gallium-68." Int J Mol Sci 18(1).
Demoin, D. W., A. N. Dame, W. D. Minard, F. Gallazzi, G. L. Seickman, T. L. Rold, N. Bemskoetter, M. E. Fassbender, T. J. Hoffman, C. A. Deakyne and S. S. Jurisson (2016). "Monooxorhenium(V) complexes with 222-N2S2 MAMA ligands for bifunctional chelator agents: Syntheses and preliminary in vivo evaluation." Nucl Med Biol 43(12): 802-811.
Desai, B., J. Mattson, H. Paintal, M. Nathan, F. Shen, M. Beaumont, M. C. Malinao, Y. Li, J.
Canfield, B. Basham, R. de Waal Malefyt, T. McClanahan, G. Krishna and R. Fick, Jr. (2011).
"Differential expression of monocyte/macrophage- selective markers in human idiopathic pulmonary fibrosis." Exp Lung Res 37(4): 227-238.
Dillekas, H., M. S. Rogers and O. Straume (2019). "Are 90% of deaths from cancer caused by metastases?" Cancer Med 8(12): 5574-5576.
Dohn, L. H., H. Pappot, B. R. Iversen, M. Illemann, G. Høyer-Hansen, I. J. Christensen, P. Thind, L. Sailing, H. von der Maase and O. D. Laerum (2015). "uPAR Expression Pattern in Patients with Urothelial Carcinoma of the Bladder--Possible Clinical Implications." PLoS One 10(8): e0135824.
Du, B., X. Jiang, Y. Huang, S. Li, J. C. Lin, M. Yu and J. Zheng (2020). "Tailoring Kidney Transport of Organic Dyes with Low-Molecular-Weight PEGylation." Bioconjug Chem 31(2): 241-247.
Duriseti, S., D. H. Goetz, D. R. Hostetter, A. M. LeBeau, Y. Wei and C. S. Craik (2010).
"Antagonistic anti-urokinase plasminogen activator receptor (uPAR) antibodies significantly inhibit uPAR-mediated cellular signaling and migration." J Biol Chem 285(35): 26878-26888.
Edelson, J. D., M. Makhlina, K. R. Silvester, S. S. Vengurlekar, X. Chen, J. Zhang, C. J. Koziol-White, P. R. Cooper, T. J. Hallam, D. W. P. Hay and R. A. Panettieri (2013). "In vitro and in vivo pharmacological profile of PL-3994, a novel cyclic peptide (Hept-cyclo(Cys-His-Phe-d-Ala-Gly-Arg-d-Nle-Asp-Arg-Ile-Ser-Cys)-Tyr-[Arg mimetic]-NH(2)) natriuretic peptide receptor-A agonist that is resistant to neutral endopeptidase and acts as a bronchodilator." Pulmonary pharmacology & therapeutics 26(2): 229-238.
Egorova, B. V., O. A. Fedorova and S. N. Kalmykov (2019). "Cationic radionuclides and ligands for targeted therapeutic radiopharmaceuticals." Russian Chemical Reviews 88(9): 901-924.
Egorova, B. V., L. S. Zamurueva, A. D. Zubenko, A. V. Pashanova, A. A. Mitrofanov, A. B.
Priselkova, Y. V. Fedorov, A. L. Trigub, O. A. Fedorova and S. N. Kalmykov (2022). "Novel Hybrid Benzoazacrown Ligand as a Chelator for Copper and Lead Cations: What Difference Does Pyridine Make." Molecules 27(10).
Eisenwiener, K. P., M. I. Prata, I. Buschmann, H. W. Zhang, A. C. Santos, S. Wenger, J. C. Reubi and H. R. Macke (2002). "NODAGATOC, a new chelator-coupled somatostatin analogue labeled with [67/68Ga] and [111In] for SPECT, PET, and targeted therapeutic applications of somatostatin receptor (hsst2) expressing tumors." Bioconjug Chem 13(3): 530-541.
Elvin, P., I. Foltz, J. Kang, N. Carragher, S. Emery and Q. Zhou (2009). "Abstract #3091: A fully human antibody inhibits urokinase receptor function in vitro." Cancer Research 69(9_Supplement): 3091-3091.
Enocsson, H., C. Idoff, A. Gustafsson, M. Govender, F. Hopkins, M. Larsson, A. Nilsdotter-Augustinsson and J. Sjowall (2021). "Soluble Urokinase Plasminogen Activator Receptor (suPAR) Independently Predicts Severity and Length of Hospitalisation in Patients With COVID-19." Front Med (Lausanne) 8: 791716.
Enocsson, H., T. Lukic, M. Ziegelasch and A. Kastbom (2021). "Serum levels of the soluble urokinase plasminogen activator receptor (suPAR) correlates with disease activity in early rheumatoid arthritis and reflects joint damage over time." Transl Res 232: 142-149.
Enocsson, H., C. Sjowall and J. Wettero (2015). "Soluble urokinase plasminogen activator receptor--a valuable biomarker in systemic lupus erythematosus?" Clin Chim Acta 444: 234-241.
Enocsson, H., J. Wettero, T. Skogh and C. Sjowall (2013). "Soluble urokinase plasminogen activator receptor levels reflect organ damage in systemic lupus erythematosus." Transl Res 162(5): 287-296.
Feiner, I. V. J., M. Brandt, J. Cowell, T. Demuth, D. Vugts, G. Gasser and T. L. Mindt (2021). "The Race for Hydroxamate-Based Zirconium-89 Chelators." Cancers (Basel) 13(17).
Fibbi, G., M. Pucci, U. Semi, M. M. Cerinic and M. Del Rosso (1998). "Antisense targeting of the urokinase receptor blocks urokinase-dependent proliferation, chemoinvasion, and chemotaxis of human synovial cells and chondrocytes in vitro." Proc Assoc Am Physicians 110(4): 340-350.
Fisher, J. L., C. L. Field, H. Zhou, T. L. Harris, M. A. Henderson and P. F. Choong (2000).
"Urokinase plasminogen activator system gene expression is increased in human breast carcinoma and its bone metastases--a comparison of normal breast tissue, non-invasive and invasive carcinoma and osseous metastases." Breast Cancer Res Treat 61(1): 1-12.
Fosbol, M. O., S. Kurbegovic, H. H. Johannesen, M. A. Roder, A. E. Hansen, J. Mortensen, A. Loft, P. M. Petersen, J. Madsen, K. Brasso and A. Kjaer (2021). "Urokinase-Type Plasminogen Activator Receptor (uPAR) PET/MRI of Prostate Cancer for Noninvasive Evaluation of Aggressiveness: Comparison with Gleason Score in a Prospective Phase 2 Clinical Trial." J Nucl Med 62(3): 354-359. Fosbøl, M. Ø., S. Kurbegovic, H. H. Johannesen, M. A. Roder, A. E. Hansen, J. Mortensen, A. Loft, P. M. Petersen, J. Madsen, K. Brasso and A. Kjaer (2020). "Urokinase Plasminogen Activator Receptor (uPAR) PET/MRI of Prostate Cancer for Non-invasive Evaluation of Aggressiveness: a Prospective Phase II Clinical Trial Comparing with Gleason Score." Journal of Nuclear Medicine.
Franchi, S., V. Di Marco and M. Tosato (2022). "Bismuth chelation for targeted alpha therapy: Current state of the art." Nuclear Medicine and Biology 114-115: 168-188.
Gai, Y., L. Sun, W. Hui, Q. Ouyang, C. J. Anderson, G. Xiang, X. Ma and D. Zeng (2016). "New Bifunctional Chelator p-SCN-PhPr-NE3TA for Copper-64: Synthesis, Peptidomimetic Conjugation, Radiolabeling, and Evaluation for PET Imaging." Inorg Chem 55(14): 6892-6901.
Giovenzana, G. B., L. Lattuada, F. Travagin and L. Biondi (2020). "Synthesis of Two Novel Mixed Bifunctional Chelating Agents: DO2AP(tBu)4 and DO3AP(tBu)4." Synlett 31(13): 1291-1294.
Goodson, R. J., M. V. Doyle, S. E. Kaufman and S. Rosenberg (1994). "High-affinity urokinase receptor antagonists identified with bacteriophage peptide display." Proc Natl Acad Sci U S A 91(15): 7129-7133.
Graf, M., S. Reif, K. Hecht, R. Pelka-Fleischer, K. Pfister and H. Schmetzer (2005). "High expression of urokinase plasminogen activator receptor (UPA-R) in acute myeloid leukemia (AML) is associated with worse prognosis." Am J Hematol 79(1): 26-35.
Grieve, M. L. and B. M. Paterson (2021). "The Evolving Coordination Chemistry of Radiometals for Targeted Alpha Therapy." Australian Journal of Chemistry 75(2): 65-88.
Guo, Y., A. A. Higazi, A. Arakelian, B. S. Sachais, D. Cines, R. H. Goldfarb, T. R. Jones, H. Kwaan, A. P. Mazar and S. A. Rabbani (2000). "A peptide derived from the nonreceptor binding region of urokinase plasminogen activator (uPA) inhibits tumor progression and angiogenesis and induces tumor cell death in vivo." FASEB J 14(10): 1400-1410.
Guo, Z. L., D. R. Richardson, D. S. Kalinowski, Z. Kovacevic, K. C. Tan-Un and G. C. Chan (2016).
"The novel thiosemicarbazone, di-2-pyridylketone 4-cyclohexyl-4-methyl-3-thiosemicarbazone (DpC), inhibits neuroblastoma growth in vitro and in vivo via multiple mechanisms." J Hematol Oncol 9(1): 98.
Hall, W. V., DA. (2006). "Efficacy of antiangiogenic targeted toxins against glioblastoma multiforme." Neurosurg Focus. 20(4).
Hamie, L., E. Eid, O. Abbas, R. Safi, T. Nammour, H. Tamim, M. Makki, C. Stephan, D. Hasbani, H. Wehbe, N. Ghaoui, M. Hawa, N. Nasser, A. Eid, A. G. Kibbi and M. Kurban (2020). "SuPAR, a potential inflammatory mediator in psoriasis pathogenesis." Clin Exp Pharmacol Physiol 47(10): 1705-1712.
Haslam, A. and V. Prasad (2019). "Estimation of the Percentage of US Patients With Cancer Who Are Eligible for and Respond to Checkpoint Inhibitor Immunotherapy Drugs." JAMA Netw Open 2(5): e192535.
He, C., P. He, L. P. Liu and Y. S. Zhu (2001). "Analysis of expressions of components in the plasminogen activator system in high- and low-metastatic human lung cancer cells." J Cancer Res Clin Oncol 127(3): 180-186.
Heath, J. L., J. M. Weiss, C. P. Lavau and D. S. Wechsler (2013). "Iron deprivation in cancerpotential therapeutic implications." Nutrients 5(8): 2836-2859.
Hong, S. I., I. C. Park, Y. S. Son, S. H. Lee, B. G. Kim, J. I. Lee, T. W. Lee, Y. H. Kook, Y. I. Min and W. S. Hong (1996). "Expression of urokinase-type plasminogen activator, its receptor, and its inhibitor in gastric adenocarcinoma tissues." J Korean Med Sci 11(1): 33-37.
Hongisto, M., J. Lassus, T. Tarvasmaki, J. Sans-Rosello, H. Tolppanen, A. Kataja, T. Jantti, T. Sabell, M. Banaszewski, J. Silva-Cardoso, J. Parissis, R. Jurkko, J. Spinar, M. Castren, A. Mebazaa, J. Masip, V. P. Harjola, I. CardShock Study and G. N. the (2022). "Soluble urokinase-type plasminogen activator receptor improves early risk stratification in cardiogenic shock." Eur Heart J Acute Cardiovasc Care 11(10): 731-738.
Høyer-Hansen, G., E. Rønne, H. Solberg, N. Behrendt, M. Ploug, L. R. Lund, V. Ellis and K. Dano (1992). "Urokinase plasminogen activator cleaves its cell surface receptor releasing the ligand-binding domain." Journal of Biological Chemistry 267(25): 18224-18229.
Huang, F., Y. Li, R. Xu, A. Cheng, Y. Lv and Q. Liu (2020). "The Plasma Soluble Urokinase Plasminogen Activator Receptor Is Related to Disease Activity of Patients with ANCA-Associated Vasculitis." Mediators Inflamm 2020: 7850179.
Huang, Q., H. Xiong, P. Yan, T. Shuai, J. Liu, L. Zhu, J. Lu, K. Yang and J. Liu (2020). "The Diagnostic and Prognostic Value of suPAR in Patients with Sepsis: A Systematic Review and Meta-Analysis." Shock 53(4): 416-425.
Huang, Y., S. Huang, X. Zhuo and M. Lin (2023). "Predictive value of suPAR in AKI: a systematic review and meta-analysis." Clin Exp Nephrol 27(1): 1-11.
Ingham, A., L. Wharton, T. El Sayed, L. Southcott, B. L. McNeil, M. B. Ezhova, B. O. Patrick, M. G. Jaraquemada-Pelaez and C. Orvig (2022). "H(2)ampa horizontal line Versatile Chelator for [(203)Pb]Pb(2+), [(213)Bi]Bi(3+), and [(225)Ac]Ac(3)." Inorg Chem 61(24): 9119-9137.
Jankowski, L., M. Pruc, A. Gasecka, J. Chmielewski, T. Wojcik, L. Szarpak and M. Kowalczyk (2023). "A comprehensive review and meta-analysis of suPAR as a predictor of acute kidney injury." Ann Agric Environ Med 30(2): 364-368.
Kalman, F. K., Z. Baranyai, I. Toth, I. Banyai, R. Kiraly, E. Brucher, S. Aime, X. Sun, A. D. Sherry and Z. Kovacs (2008). "Synthesis, potentiometric, kinetic, and NMR Studies of 1,4,7,10-tetraazacyclododecane-1,7-bis(acetic acid)-4,10-bis(methylenephosphonic acid) (DO2A2P) and its complexes with Ca(II), Cu(II), Zn(II) and lanthanide(III) ions." Inorg Chem 47(9): 3851-3862. Kanchi, S., P. Singh and K. Bisetty (2014). "Dithiocarbamates as hazardous remediation agent: A critical review on progress in environmental chemistry for inorganic species studies of 20th century." Arabian Journal of Chemistry 7(1): 11-25.
Kimura, S., D. D'Andrea, T. Iwata, B. Foerster, F. Janisch, M. K. Parizi, M. Moschini, A. Briganti, M. Babjuk, P. Chlosta, P. I. Karakiewicz, D. Enikeev, L. M. Rapoport, V. Seebacher, S. Egawa, M. Abufaraj and S. F. Shariat (2020). "Expression of urokinase-type plasminogen activator system in non-metastatic prostate cancer." World J Urol 38(10): 2501-2511.
Knor, S., S. Sato, T. Huber, A. Morgenstem, F. Bruchertseifer, M. Schmitt, H. Kessler, R. Senekowitsch-Schmidtke, V. Magdolen and C. Seidl (2008). "Development and evaluation of peptidic ligands targeting tumour-associated urokinase plasminogen activator receptor (uPAR) for use in alpha-emitter therapy for disseminated ovarian cancer." Eur J Nucl Med Mol Imaging 35(1): 53-64.
Kolho, K. L., E. Valtonen, H. Rintamaki and E. Savilahti (2012). "Soluble urokinase plasminogen activator receptor suPAR as a marker for inflammation in pediatric inflammatory bowel disease." Scand J Gastroenterol 47(8-9): 951-955.
Kostelnik, T. I. and C. Orvig (2019). "Radioactive Main Group and Rare Earth Metals for Imaging and Therapy." Chem Rev 119(2): 902-956.
Kurtipek, G. S., R. Kesli, F. Tuncez Akyurek, F. Akyuret and Y. Terzi (2015). "Plasma-soluble urokinase plasminogen activator receptor (suPAR) levels in psoriasis patients and correlation with disease severity." Acta Dermatovenerol Alp Pannonica Adriat 24(4): 73-75.
Lahtinen, L., N. Huusko, H. Myohanen, A. K. Lehtivarjo, R. Pellinen, M. P. Turunen, S. Yla-Herttuala, E. Pirinen and A. Pitkanen (2009). "Expression of urokinase-type plasminogen activator receptor is increased during epileptogenesis in the rat hippocampus." Neuroscience 163(1): 316-328. LeBeau, A. M., S. Duriseti, S. T. Murphy, F. Pepin, B. Hann, J. W. Gray, H. F. VanBrocklin and C. S. Craik (2013). "Targeting uPAR with antagonistic recombinant human antibodies in aggressive breast cancer." Cancer Res 73(7): 2070-2081.
Legany, N., G. Toldi, J. H. Distler, C. Beyer, B. Szalay, L. Kovacs, B. Vasarhelyi and A. Balog (2015). "Increased plasma soluble urokinase plasminogen activator receptor levels in systemic sclerosis: possible association with microvascular abnormalities and extent of fibrosis." Clin Chem Lab Med 53(11): 1799-1805.
Leth, J. M., E. A. Newcombe, A. L. Gronnemose, J. T. Jorgensen, K. Qvist, A. S. Clausen, L. B. S. Knudsen, A. Kjaer, B. B. Kragelund, T. J. D. Jorgensen and M. Ploug (2023). "Targeted imaging of uPAR expression in vivo with cyclic AE105 variants." Sci Rep 13(1): 17248.
Li, M., N. J. Baumhover, D. Liu, B. S. Cagle, F. Boschetti, G. Paulin, D. Lee, Z. Dai, E. R. Obot, B. M. Marks, I. Okeil, E. A. Sagastume, M. Gabr, F. C. Pigge, F. L. Johnson and M. K. Schultz (2023). "Preclinical Evaluation of a Lead Specific Chelator (PSC) Conjugated to Radiopeptides for (203)Pb and (212)Pb-Based Theranostics." Pharmaceutics 15(2).
Li, W. P., D. S. Ma, C. Higginbotham, T. Hoffman, A. R. Ketring, C. S. Cutler and S. S. Jurisson (2001). "Development of an in vitro model for assessing the in vivo stability of lanthanide chelates." Nucl Med Biol 28(2): 145-154.
Li, Z.-B., G. Niu, H. Wang, L. He, L. Yang, M. Ploug and X. Chen (2008). "Imaging of Urokinase-Type Plasminogen Activator Receptor Expression Using a <sup>64</sup>Cu-Labeled Linear Peptide Antagonist by microPET." Clinical Cancer Research 14(15): 4758-4766.
Li, Z., C. Wang, J. Chen, X. Lian, C. Xiong, R. Tian, L. Hu, X. Xiong and J. Tian (2021). "uPAR targeted phototheranostic metal-organic framework nanoprobes for MR/NIR-II imaging-guided therapy and surgical resection of glioblastoma." Materials & Design 198.
Lima, L. M., Z. Halime, R. Marion, N. Camus, R. Delgado, C. Platas-Iglesias and R. Tripier (2014). "Monopicolinate cross-bridged cyclam combining very fast complexation with very high stability and inertness of its copper(II) complex." Inorg Chem 53(10): 5269-5279.
Lin, L., H. Gårdsvoll, Q. Huai, M. Huang and M. Ploug (2010). "Structure-based engineering of species selectivity in the interaction between urokinase and its receptor: implication for preclinical cancer therapy." J Biol Chem 285(14): 10982-10992.
Liu, D., D. Xu, M. Liu, W. E. Knabe, C. Yuan, D. Zhou, M. Huang and S. O. Meroueh (2017). "Small Molecules Engage Hot Spots through Cooperative Binding To Inhibit a Tight Protein-Protein Interaction." Biochemistry 56(12): 1768-1784.
Liu, S., Z. He, W. Y. Hsieh and P. E. Fanwick (2003). "Synthesis, characterization, and X-ray crystal structure of In(DOTA-AA) (AA = p-aminoanilide): a model for 111In-labeled DOTA-biomolecule conjugates." Inorg Chem 42(26): 8831-8837.
Lorenz, M., A. Evers and M. Wagner (2013). "Recent progress and future options in the development of GLP-1 receptor agonists for the treatment of diabesity." Bioorg Med Chem Lett 23(14): 4011-4018.
Lozza, C., I. Navarro-Teulon, A. Pelegrin, J. P. Pouget and E. Vives (2013). "Peptides in receptor-mediated radiotherapy: from design to the clinical application in cancers." Front Oncol 3: 247.
Lu, W., Y. Cong, D. Yang, D. Chen, G. Yang, Y. Wang, M. E. Van Dort, B. D. Ross, A. P. Mazar, B. B. Chu and H. Hong (2021). "Engineered Antibody Fragment against the Urokinase Plasminogen Activator for Fast Delineation of Triple-Negative Breast Cancer by Positron Emission Tomography." Mol Pharm 18(4): 1690-1698.
Lund, I. K., M. Illemann, T. Thurison, I. J. Christensen and G. Høyer-Hansen (2011). "uPAR as anti-cancer target: evaluation of biomarker potential, histological localization, and antibody-based therapy." Curr Drug Targets 12(12): 1744-1760.
Luther, T., M. Kotzsch, A. Meye, T. Langerholc, S. Fussel, N. Olbricht, S. Albrecht, D. Ockert, B. Muehlenweg, K. Friedrich, M. Grosser, M. Schmitt, G. Baretton and V. Magdolen (2003). "Identification of a novel urokinase receptor splice variant and its prognostic relevance in breast cancer." Thromb Haemost 89(4): 705-717.
Lyczko, K., M. Lyczko and M. Pruszyński (2020). "Lead(II) complexes with amide-appended tetraazamacrocyclic ligands - Synthesis, structure, characterization and calculation studies." Polyhedron 192.
Mabrouk, R. A. and R. Ali-Labib (2003). "Detection of urokinase plasminogen activator receptor and c-erbB-2 in sera of patients with breast and ovarian carcinoma." Clin Biochem 36(7): 537-543. Madunić, J. (2018). "The Urokinase Plasminogen Activator System in Human Cancers: An Overview of Its Prognostic and Predictive Role." Thromb Haemost 118(12): 2020-2036.
Maecke, H. R. and J. P. Andre (2007). "68Ga-PET radiopharmacy: A generator-based alternative to 18F-radiopharmacy." Ernst Schering Res Found Workshop(62): 215-242.
Magdolen, V., M. Burgle, N. A. de Prada, N. Schmiedeberg, C. Riemer, F. Schroeck, J. Kellermann, K. Degitz, O. G. Wilhelm, M. Schmitt and H. Kessler (2001). "Cyclo19,31[D-Cys19]-uPA19-31 is a potent competitive antagonist of the interaction of urokinase-type plasminogen activator with its receptor (CD87)." Biol Chem 382(8): 1197-1205.
Mahmood, N., A. Arakelian, H. A. Khan, I. Tanvir, A. P. Mazar and S. A. Rabbani (2020). "uPAR antibody (huATN-658) and Zometa reduce breast cancer growth and skeletal lesions." Bone Res 8: 18. Mahmood, N., C. Mihalcioiu and S. A. Rabbani (2018). "Multifaceted Role of the Urokinase-Type Plasminogen Activator (uPA) and Its Receptor (uPAR): Diagnostic, Prognostic, and Therapeutic Applications." Front Oncol 8: 24.
Manfredi, M., L. Van Hoovels, M. Benucci, R. De Luca, C. Coccia, P. Bernardini, E. Russo, A. Amedei, S. Guiducci, V. Grossi, X. Bossuyt, C. Perricone and M. Infantino (2023). "Soluble Urokinase Plasminogen Activator Receptor (suPAR) in Autoimmune Rheumatic and Non Rheumatic Diseases." J Pers Med 13(4).
Mani, T., F. Wang, W. E. Knabe, A. L. Sinn, M. Khanna, I. Jo, G. E. Sandusky, G. W. Sledge, Jr., D. R. Jones, R. Khanna, K. E. Pollok and S. O. Meroueh (2013). "Small-molecule inhibition of the uPAR.uPA interaction: synthesis, biochemical, cellular, in vivo pharmacokinetics and efficacy studies in breast cancer metastasis." Bioorg Med Chem 21(7): 2145-2155.
Mazzi, U., R. Schibli, H. J. Pietzsch, J. U. Künstler and H. Spies (2007). Technetium in Medicine. Technetium-99m Pharmaceuticals: 7-58.
McAuley, A., K. Beveridge, S. Subramanian and T. W. Whitcombe (1989). "Isolation and characterization of polydentate ligands derived from the reaction of pentaerythrityltetrabromide and ethylenediamine." Canadian Journal of Chemistry 67(10): 1657-1665.
McBride, W. J., R. M. Sharkey and D. M. Goldenberg (2013). "Radiofluorination using aluminum-fluoride (A118F)." EJNMMI Res 3(1): 36.
McDonagh, A. W., B. L. McNeil, B. O. Patrick and C. F. Ramogida (2021). "Synthesis and Evaluation of Bifunctional [2.2.2]-Cryptands for Nuclear Medicine Applications." Inorg Chem 60(13): 10030-10037.
McGinnis, S. and T. L. Madden (2004). "BLAST: at the core of a powerful and diverse set of sequence analysis tools." Nucleic Acids Res 32(Web Server issue): W20-25.
McMahon, B. J. and H. C. Kwaan (2015). "Components of the Plasminogen-Plasmin System as Biologic Markers for Cancer." Adv Exp Med Biol 867: 145-156.
Metrangolo, V., M. Ploug and L. H. Engelholm (2021). "The Urokinase Receptor (uPAR) as a "Trojan Horse" in Targeted Cancer Therapy: Challenges and Opportunities." Cancers (Basel) 13(21).
Mustjoki, S., N. Sidenius, C. F. Sier, F. Blasi, E. Elonen, R. Alitalo and A. Vaheri (2000). "Soluble urokinase receptor levels correlate with number of circulating tumor cells in acute myeloid leukemia and decrease rapidly during chemotherapy." Cancer Res 60(24): 7126-7132.
Needleman, S. B. and C. D. Wunsch (1970). "A general method applicable to the search for similarities in the amino acid sequence of two proteins." J Mol Biol 48(3): 443-453.
Nielsen, L. S., J. G. Hansen, L. Skriver, E. L. Wilson, K. Kaltoft, J. Zeuthen and K. Dano (1982). "Purification of zymogen to plasminogen activator from human glioblastoma cells by affinity chromatography with monoclonal antibody." Biochemistry 21(25): 6410-6415.
Nock, B. A., T. Maina, M. Behe, A. Nikolopoulou, M. Gotthardt, J. S. Schmitt, T. M. Behr and H. R. Macke (2005). "CCK-2/gastrin receptor-targeted tumor imaging with (99m)Tc-labeled minigastrin analogs." J Nucl Med 46(10): 1727-1736.
Nykjaer, A., M. Conese, E. I. Christensen, D. Olson, O. Cremona, J. Gliemann and F. Blasi (1997). "Recycling of the urokinase receptor upon internalization of the uPA:serpin complexes." EMBO J 16(10): 2610-2620.
Odendaal, A. Y., A. L. Fiamengo, R. Ferdani, T. J. Wadas, D. C. Hill, Y. Peng, K. J. Heroux, J. A. Golen, A. L. Rheingold, C. J. Anderson, G. R. Weisman and E. H. Wong (2011). "Isomeric trimethylene and ethylene pendant-armed cross-bridged tetraazamacrocycles and in vitro/in vivo comparisions of their copper(II) complexes." Inorg Chem 50(7): 3078-3086.
Østervig, R. M., L. Køber, J. L. Forberg, L. S. Rasmussen, J. Eugen-Olsen and K. Iversen (2015). "SuPAR - A future prognostic biomarker in emergency medicine." Scandinavian Journal of Trauma, Resuscitation and Emergency Medicine 23(S1).
Pandya, D. N., A. V. Dale, J. Y. Kim, H. Lee, Y. S. Ha, G. I. An and J. Yoo (2012). "New macrobicyclic chelator for the development of ultrastable 64Cu-radiolabeled bioconjugate." Bioconjug Chem 23(3): 330-335.
Paraskevas, T., F. Mulita, C. Michailides, G. I. Verras, E. Liolis, E. Oikonomou, I. Perdikaris, P. Perdikaris, K. Bouchagier, I. Panagiotopoulos, D. I. Kasartzian, D. Filis, L. Tchabashvili, D. Spyropoulou and D. Velissaris (2022). "The role of soluble urokinase plasminogen activator receptor (suPAR) in patients with cancer: a review of the current literature." Med Glas (Zenica) 19(2). Pearson, W. R. and D. J. Lipman (1988). "Improved tools for biological sequence comparison." Proc Natl Acad Sci U S A 85(8): 2444-2448.
Persson, M., H. H. El Ali, T. Binderup, A. Pfeifer, J. Madsen, P. Rasmussen and A. Kjaer (2014). "Dosimetry of 64Cu-DOTA-AE105, a PET tracer for uPAR imaging." Nucl Med Biol 41(3): 290-295. Persson, M., K. Juhl, P. Rasmussen, M. Brandt-Larsen, J. Madsen, M. Ploug and A. Kjaer (2014). "uPAR targeted radionuclide therapy with (177)Lu-DOTA-AE105 inhibits dissemination of metastatic prostate cancer." Mol Pharm 11(8): 2796-2806.
Persson, M., H. Liu, J. Madsen, Z. Cheng and A. Kjaer (2013). "First (18)F-labeled ligand for PET imaging of uPAR: in vivo studies in human prostate cancer xenografts." Nucl Med Biol 40(5): 618-624.
Persson, M., J. Madsen, S. Østergaard, M. M. Jensen, J. T. Jørgensen, K. Juhl, C. Lehmann, M. Ploug and A. Kjaer (2012). "Quantitative PET of human urokinase-type plasminogen activator receptor with 64Cu-DOTA-AE105: implications for visualizing cancer invasion." J Nucl Med 53(1): 138-145.
Persson, M., J. Madsen, S. Østergaard, M. Ploug and A. Kjaer (2012). "68Ga-labeling and in vivo evaluation of a uPAR binding DOTA- and NODAGA-conjugated peptide for PET imaging of invasive cancers." Nucl Med Biol 39(4): 560-569.
Persson, M., P. Rasmussen, J. Madsen, M. Ploug and A. Kjaer (2012). "New peptide receptor radionuclide therapy of invasive cancer cells: in vivo studies using 177Lu-DOTA-AE105 targeting uPAR in human colorectal cancer xenografts." Nucl Med Biol 39(7): 962-969.
Persson, M., D. Skovgaard, M. Brandt-Larsen, C. Christensen, J. Madsen, C. H. Nielsen, T. Thurison, T. L. Klausen, S. Holm, A. Loft, A. K. Berthelsen, M. Ploug, H. Pappot, K. Brasso, N. Kroman, L. Højgaard and A. Kjaer (2015). "First-in-human uPAR PET: Imaging of Cancer Aggressiveness." Theranostics 5(12): 1303-1316.
Plamboeck, A., J. J. Holst, R. D. Carr and C. F. Deacon (2005). "Neutral endopeptidase 24.11 and dipeptidyl peptidase IV are both mediators of the degradation of glucagon-like peptide 1 in the anaesthetised pig." Diabetologia 48(9): 1882-1890.
Pliyev, B. K. and M. Y. Menshikov (2010). "Release of the soluble urokinase-type plasminogen activator receptor (suPAR) by activated neutrophils in rheumatoid arthritis." Inflammation 33(1): 1-9. Ploug, M., S. Ostergaard, H. Gardsvoll, K. Kovalski, C. Holst-Hansen, A. Holm, L. Ossowski and K. Dano (2001). "Peptide-derived antagonists of the urokinase receptor. affinity maturation by combinatorial chemistry, identification of functional epitopes, and inhibitory effect on cancer cell intravasation." Biochemistry 40(40): 12157-12168.
Poty, S., P. Desogere, J. Simecek, C. Bernhard, V. Goncalves, C. Goze, F. Boschetti, J. Notni, H. J. Wester and F. Denat (2015). "MA-NOTMP: A Triazacyclononane Trimethylphosphinate Based Bifunctional Chelator for Gallium Radiolabelling of Biomolecules." ChemMedChem 10(9): 1475-1479.
Price, E. W. and C. Orvig (2014). "Matching chelators to radiometals for radiopharmaceuticals." Chem Soc Rev 43(1): 260-290.
Pyke, C., E. Ralfkiaer, E. Ronne, G. Hoyer-Hansen, L. Kirkeby and K. Dano (1994). "Immunohistochemical detection of the receptor for urokinase plasminogen activator in human colon cancer." Histopathology 24(2): 131-138.
Qin, D. D., D. Song, J. Huang, F. Yu and M. H. Zhao (2015). "Plasma-soluble urokinase-type plasminogen activator receptor levels are associated with clinical and pathological activities in lupus nephritis: a large cohort study from China." Lupus 24(6): 546-557.
Ramogida, C. F., J. F. Cawthray, E. Boros, C. L. Ferreira, B. O. Patrick, M. J. Adam and C. Orvig (2015). "H2CHXdedpa and H4CHXoctapa-chiral acyclic chelating ligands for (67/68)Ga and (111)In radiopharmaceuticals." Inorg Chem 54(4): 2017-2031.
Rasmussen, L. J. H., S. Ladelund, T. H. Haupt, G. E. Ellekilde, J. Eugen-Olsen and O. Andersen (2018). "Combining National Early Warning Score With Soluble Urokinase Plasminogen Activator Receptor (suPAR) Improves Risk Prediction in Acute Medical Patients: A Registry-Based Cohort Study." Crit Care Med 46(12): 1961-1968.
Rasmussen, L. J. H., J. E. V. Petersen and J. Eugen-Olsen (2021). "Soluble Urokinase Plasminogen Activator Receptor (suPAR) as a Biomarker of Systemic Chronic Inflammation." Front Immunol 12: 780641.
Rea, V. E., A. Lavecchia, C. Di Giovanni, F. W. Rossi, A. Gorrasi, A. Pesapane, A. de Paulis, P. Ragno and N. Montuori (2013). "Discovery of new small molecules targeting the vitronectin-binding site of the urokinase receptor that block cancer cell invasion." Mol Cancer Ther 12(8): 1402-1416.
Riser, L. M., M. M. Clausen, Z. Ujmajuridze, M. Farhadi, K. F. Andersen, A. Loft, J. Friborg and A. Kjaer (2022). "Prognostic Value of Urokinase-Type Plasminogen Activator Receptor PET/CT in Head and Neck Squamous Cell Carcinomas and Comparison with (18)F-FDG PET/CT: A Single-Center Prospective Study." J Nucl Med 63(8): 1169-1176.
Roldan, A. L., M. V. Cubellis, M. T. Masucci, N. Behrendt, L. R. Lund, K. Dano, E. Appella and F. Blasi (1990). "Cloning and expression of the receptor for human urokinase plasminogen activator, a central molecule in cell surface, plasmin dependent proteolysis." EMBO J 9(2): 467-474.
Rustamzadeh, E., C. Li, S. Doumbia, W. A. Hall and D. A. Vallera (2003). "Targeting the over-expressed urokinase-type plasminogen activator receptor on glioblastoma multiforme." J Neurooncol 65(1): 63-75.
Santeri, S., A. A. Peter, N. Kristiina, E. O. Jesper and H. Harri (2021). "suPAR cut-offs for stratification of low, medium, and high-risk acute medical patients in the emergency department." BMC Emerg Med 21(1): 149.
Saxena, A., P. M. Izmirly, S. W. Han, P. Briassouli, T. L. Rivera, H. Zhong, D. M. Friedman, R. M. Clancy and J. P. Buyon (2015). "Serum Biomarkers of Inflammation, Fibrosis, and Cardiac Function in Facilitating Diagnosis, Prognosis, and Treatment of Anti-SSA/Ro-Associated Cardiac Neonatal Lupus." J Am Coll Cardiol 66(8): 930-939.
Saylam Kurtipek, G., R. Kesli, F. Tuncez Akyurek, F. Akyurek, A. Ataseven and Y. Terzi (2018). "Plasma-soluble urokinase plasminogen activator receptor (suPAR) levels in Behcet's disease and correlation with disease activity." Int J Rheum Dis 21(4): 866-870.
Schwartz, D. A., M. J. Abrams, M. M. Hauser, F. E. Gaul, S. K. Larsen, D. Rauh and J. A. Zubieta (1991). "Preparation of hydrazino-modified proteins and their use for the synthesis of 99mTc-protein conjugates." Bioconjug Chem 2(5): 333-336.
Seemann, J., B. P. Waldron, F. Rösch and D. Parker (2015). "Approaching "Kit-Type" Labelling with Ga-68: The DATA Chelators." ChemMedChem 10: 1019 - 1026.
Serda, M., D. S. Kalinowski, N. Rasko, E. Potuckova, A. Mrozek-Wilczkiewicz, R. Musiol, J. G. Malecki, M. Sajewicz, A. Ratuszna, A. Muchowicz, J. Golab, T. Simunek, D. R. Richardson and J. Polanski (2014). "Exploring the anti-cancer activity of novel thiosemicarbazones generated through the combination of retro-fragments: dissection of critical structure-activity relationships." PLoS One 9(10): e110291.
Shiells, E. J., L. S. Natrajan, D. Sykes, M. Tropiano, P. Cooper, A. M. Kenwright and S. Faulkner (2011). "Lanthanide complexes of DOTA monoamide derivatives bearing an isophthalate pendent arm." Dalton Trans 40(43): 11451-11457.
Shou, L. H., D. Cao, X. H. Dong, Q. Fang, B. L. Xu and J. P. Fei (2016). "Bone Marrow Urokinase Plasminogen Activator Receptor Levels are Associated with the Progress of Multiple Myeloma." Chin Med Sci J 31(3): 155-160.
Skovgaard, D., M. Persson, M. Brandt-Larsen, C. Christensen, J. Madsen, T. L. Klausen, S. Holm, F. L. Andersen, A. Loft, A. K. Berthelsen, H. Pappot, K. Brasso, N. Kroman, L. Hojgaard and A. Kjaer (2017). "Safety, Dosimetry, and Tumor Detection Ability of (68)Ga-NOTA-AE105: First-in-Human Study of a Novel Radioligand for uPAR PET Imaging." J Nucl Med 58(3): 379-386.
Skovgaard, D., M. Persson and A. Kjaer (2017). "Imaging of Prostate Cancer Using Urokinase-Type Plasminogen Activator Receptor PET." PET Clin 12(2): 243-255.
Skovgaard, D., M. Persson and A. Kjaer (2017). "Urokinase Plasminogen Activator Receptor-PET with (68)Ga-NOTA-AE105: First Clinical Experience with a Novel PET Ligand." PET Clin 12(3): 311-319.
Smith, H. W. and C. J. Marshall (2010). "Regulation of cell signalling by uPAR." Nat Rev Mol Cell Biol 11(1): 23-36.
Smith, T. F. and M. S. Waterman (1981). "Comparison of biosequences." Advances in Applied Mathematics 2(4): 482-489.
Stauning, M. A., I. Altintas, T. Kallemose, J. Eugen-Olsen, M. B. Lindstrom, L. J. H. Rasmussen, H. Gamst-Jensen, J. O. Nehlin, O. Andersen and J. Tingleff (2021). "Soluble Urokinase Plasminogen Activator Receptor as a Decision Marker for Early Discharge of Patients with COVID-19 Symptoms in the Emergency Department." J Emerg Med 61(3): 298-313.
Stephens, R. W., J. Pollanen, H. Tapiovaara, K. C. Leung, P. S. Sim, E. M. Salonen, E. Ronne, N. Behrendt, K. Dano and A. Vaheri (1989). "Activation of pro-urokinase and plasminogen on human sarcoma cells: a proteolytic system with surface-bound reactants." J Cell Biol 108(5): 1987-1995. Stewart, C. E. and I. Sayers (2009). "Characterisation of urokinase plasminogen activator receptor variants in human airway and peripheral cells." BMC Mol Biol 10: 75.
Stimmel, J. B., M. E. Stockstill and F. C. Kull, Jr. (1995). "Yttrium-90 chelation properties of tetraazatetraacetic acid macrocycles, diethylenetriaminepentaacetic acid analogues, and a novel terpyridine acyclic chelator." Bioconjug Chem 6(2): 219-225.
Stoppelli, M. P., A. Corti, A. Soffientini, G. Cassani, F. Blasi and R. K. Assoian (1985). "Differentiation-enhanced binding of the amino-terminal fragment of human urokinase plasminogen activator to a specific receptor on U937 monocytes." Proc Natl Acad Sci U S A 82(15): 4939-4943. Straathof, N. J. W., C. B. Magnus, F. Zhuravlev and A. I. Jensen (2021). "Novel Bifunctional [16]aneS(4)-Derived Chelators for Soft Radiometals." Molecules 26(15).
Sudhini, Y. R., C. Wei and J. Reiser (2022). "suPAR: An Inflammatory Mediator for Kidneys." Kidney Dis (Basel) 8(4): 265-274.
Taubert, H., P. Wurl, T. Greither, M. Kappler, M. Bache, C. Lautenschlager, S. Fussel, A. Meye, A. W. Eckert, H. J. Holzhausen, V. Magdolen and M. Kotzsch (2010). "Co-detection of members of the urokinase plasminogen activator system in tumour tissue and serum correlates with a poor prognosis for soft-tissue sarcoma patients." Br J Cancer 102(4): 731-737.
Theilade, S., S. Lyngbaek, T. W. Hansen, J. Eugen-Olsen, M. Fenger, P. Rossing and J. L. Jeppesen (2015). "Soluble urokinase plasminogen activator receptor levels are elevated and associated with complications in patients with type 1 diabetes." J Intern Med 277(3): 362-371.
Theilade, S., P. Rossing, J. Eugen-Olsen, J. S. Jensen and M. T. Jensen (2016). "suPAR level is associated with myocardial impairment assessed with advanced echocardiography in patients with type 1 diabetes with normal ejection fraction and without known heart disease or end-stage renal disease." Eur J Endocrinol 174(6): 745-753.
Thuno, M., B. Macho and J. Eugen-Olsen (2009). "suPAR: the molecular crystal ball." Dis Markers 27(3): 157-172.
Toldi, G., B. Szalay, G. Beko, M. Bocskai, M. Deak, L. Kovacs, B. Vasarhelyi and A. Balog (2012). "Plasma soluble urokinase plasminogen activator receptor (suPAR) levels in systemic lupus erythematosus." Biomarkers 17(8): 758-763.
Toldi, G., B. Szalay, G. Beko, L. Kovacs, B. Vasarhelyi and A. Balog (2013). "Plasma soluble urokinase plasminogen activator receptor (suPAR) levels in ankylosing spondylitis." Joint Bone Spine 80(1): 96-98.
Trimarchi, H., R. Canzonieri, A. Schiel, C. Costales-Collaguazo, A. Stem, M. Paulero, T. Rengel, J. Andrews, A. Iotti, M. Forrester, F. Lombi, V. Pomeranz, R. Iriarte, A. Muryan and E. Zotta (2017). "In IgA Nephropathy, Glomerulosclerosis Is Associated with Increased Urinary CD80 Excretion and Urokinase-Type Plasminogen Activator Receptor-Positive Podocyturia." Nephron Extra 7(2): 52-61. Urbanovsky, P., J. Kotek, I. Cisarova and P. Hermann (2020). "Selective and clean synthesis of aminoalkyl-H-phosphinic acids from hypophosphorous acid by phospha-Mannich reaction." RSC Adv 10(36): 21329-21349.
Uzawa, A., Y. Kojima, Y. Ozawa, M. Yasuda, Y. Onishi, H. Akamine, N. Kawaguchi, K. Himuro and S. Kuwabara (2020). "Serum level of soluble urokinase plasminogen activator receptor (suPAR) as a disease severity marker of myasthenia gravis: a pilot study." Clin Exp Immunol 202(3): 321-324.
Van Buren, G., 2nd, M. J. Gray, N. A. Dallas, L. Xia, S. J. Lim, F. Fan, A. P. Mazar and L. M. Ellis (2009). "Targeting the urokinase plasminogen activator receptor with a monoclonal antibody impairs the growth of human colorectal cancer in the liver." Cancer 115(14): 3360-3368.
Vassalli, J. D., D. Baccino and D. Belin (1985). "A cellular binding site for the Mr 55,000 form of the human plasminogen activator, urokinase." J Cell Biol 100(1): 86-92.
Velissaris, D., N. Zareifopoulos, V. Karamouzos, C. Pierrakos and M. Karanikolas (2022). "Soluble urokinase plasminogen activator receptor (suPAR) in the emergency department: An update." Caspian J Intern Med 13(4): 650-665.
Wadas, T. J., E. H. Wong, G. R. Weisman and C. J. Anderson (2007). "Copper chelation chemistry and its role in copper radiopharmaceuticals." Curr Pharm Des 13(1): 3-16.
Wadas, T. J., E. H. Wong, G. R. Weisman and C. J. Anderson (2010). "Coordinating radiometals of copper, gallium, indium, yttrium, and zirconium for PET and SPECT imaging of disease." Chem Rev 110(5): 2858-2902.
Walther, R. and A. N. Zelikin (2021). "Chemical (neo)glycosylation of biological drugs." Adv Drug Deliv Rev 171: 62-76.
Werle, M. and A. Bernkop-Schnürch (2006). "Strategies to improve plasma half life time of peptide and protein drugs." Amino Acids 30(4): 351-367.
Woodin, K. S., K. J. Heroux, C. A. Boswell, E. H. Wong, G. R. Weisman, W. Niu, S. A. Tomellini, C. J. Anderson, L. N. Zakharov and A. L. Rheingold (2005). "Kinetic Inertness and Electrochemical Behavior of Copper(II) Tetraazamacrocyclic Complexes: Possible Implications for in Vivo Stability." European Journal of Inorganic Chemistry 2005(23): 4829-4833.
Woods, M., K. M. Payne, E. J. Valente, B. E. Kucera and V. G. Young, Jr. (2019). "Crystal Structures of DOTMA Chelates from Ce(3+) to Yb(3+) : Evidence for a Continuum of Metal Ion Hydration States." Chemistry 25(42): 9997-10005.
Xiao, W., Y. P. Hsu, A. Ishizaka, T. Kirikae and R. B. Moss (2005). "Sputum cathelicidin, urokinase plasminogen activation system components, and cytokines discriminate cystic fibrosis, COPD, and asthma inflammation." Chest 128(4): 2316-2326.
Yamamoto, M. S., R.; Mohanam, S; Velidi H. Rao; Janet M. Bruner; Garth L. Nicolson; Jasti S. Rao (1994). "Expression and Localization of Urokinase-type Plasminogen Activator Receptor in Human Gliomas." Cancer Res 54(18).
Yang, H., J. J. Wilson, C. Orvig, Y. Li, D. S. Wilbur, C. F. Ramogida, V. Radchenko and P. Schaffer (2022). "Harnessing alpha-Emitting Radionuclides for Therapy: Radiolabeling Method Review." J Nucl Med 63(1): 5-13.
Yang, L., H. Mao, Z. Cao, Y. A. Wang, X. Peng, X. Wang, H. K. Sajja, L. Wang, H. Duan, C. Ni, C. A. Staley, W. C. Wood, X. Gao and S. Nie (2009). "Molecular imaging of pancreatic cancer in an animal model using targeted multifunctional nanoparticles." Gastroenterology 136(5): 1514-1525 e1512.
Yang, L., H. K. Sajja, Z. Cao, W. Qian, L. Bender, A. I. Marcus, M. Lipowska, W. C. Wood and Y. A. Wang (2013). "uPAR-targeted optical imaging contrasts as theranostic agents for tumor margin detection." Theranostics 4(1): 106-118.
Yokel, R. A., A. M. Fredenburg, P. W. Durbin, J. Xu, M. K. Rayens and K. N. Raymond (2000). "The hexadentate hydroxypyridinonate TREN-(Me-3,2-HOPO) is a more orally active iron chelator than its bidentate analogue." J Pharm Sci 89(4): 545-555.
Yu, L., F. Z. Li, J. Y. Wu, J. Q. Xie and S. Li (2016). "Development of the aza-crown ether metal complexes as artificial hydrolase." J Inorg Biochem 154: 89-102.
Zabinska, M., K. Koscielska-Kasprzak, J. Krajewska, D. Bartoszek, H. Augustyniak-Bartosik and M. Krajewska (2021). "Immune Cells Profiling in ANCA-Associated Vasculitis Patients-Relation to Disease Activity." Cells 10(7).
Zhou, D., K. Bum-Erdene, D. Xu, D. Liu, D. Tompkins, R. S. Sulaiman, T. W. Corson, J. M. Chirgwin and S. O. Meroueh (2018). "Small molecules inhibit ex vivo tumor growth in bone." Bioorg Med Chem 26(23-24): 6128-6134.
Zhou, L., Y. Hayashi, T. Itoh, W. Wang, J. Rui and H. Itoh (2000). "Expression of urokinase-type plasminogen activator, urokinase-type plasminogen activator receptor, and plasminogen activator inhibitor-1 and -2 in hepatocellular carcinoma." Pathol Int 50(5): 392-397.
Zhou, X., L. Dong and L. Shen (2021). "Hydroxypyridinones as a Very Promising Platform for Targeted Diagnostic and Therapeutic Radiopharmaceuticals." Molecules 26(22).
Zhou, Y., J. Ren, P. Li, R. Ma, M. Zhou, N. Zhang, X. Kong, Z. Hu and X. Xiao (2019). "Expression of Urokinase-type Plasminogen Activator Receptor and its Soluble Form in Type 2 Diabetic Kidney Disease." Arch Med Res 50(5): 249-256.
Zimmermann, H. W., A. Koch, S. Seidler, C. Trautwein and F. Tacke (2012). "Circulating soluble urokinase plasminogen activator is elevated in patients with chronic liver disease, discriminates stage and aetiology of cirrhosis and predicts prognosis." Liver Int 32(3): 500-509.

## Claims

1. A compound comprising a cyclic peptide
of formula (I) 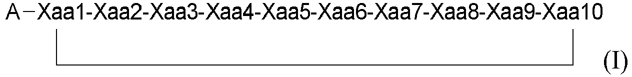
and optionally comprising a C-terminal modification group Cterm covalently attached to Xaa10, wherein the C-terminal modification group Cterm comprises a Z group,
wherein
the peptide sequence is drawn from left to right in N to C-terminal direction,
Xaa1 is a residue of an L-α-amino acid comprising a side chain,
wherein the side chain comprises a sulfur atom which is covalently attached to the sulfur atom of the thiol group of Xaa10,
wherein the carbonyl group of Xaa1 is covalently attached to the α-nitrogen atom of Xaa2, and wherein
Xaa1 is a residue of an amino acid of formula (III) 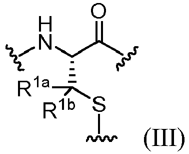 wherein
R^{1a} and R^{1b} are each and independently selected from a group consisting of -H and a methyl group,
Xaa2 is a residue of an α-amino acid comprising a side chain, preferably an L-α-amino acid comprising a side chain,
wherein the side chain optionally comprises a Z group,
wherein the carbonyl group of Xaa2 is covalently attached to the α-nitrogen atom of Xaa3 and wherein
Xaa2 is a residue of an amino acid of formula (IVa), (IVb), or (IVc) 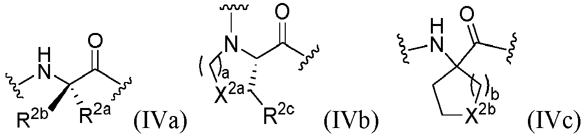 wherein
in formula (IVa)
R^{2a} is selected from the group consisting of -(CH₂)_{c}R^{2d}, -H, a (C₁-C₆)alkyl group, a (C₃-C₇)carbocycle, (C₃-C₇)heterocycle, an aryl group, and a heteroaryl group ,
wherein R^{2d} is selected from the group consisting of a polar, a positively charged, a negatively charged, a zwitterionic and a hydrophobic group, and wherein R^{2d} optionally comprises a Z group,
wherein c = 1, 2, 3, 4, or 5,
wherein optionally one or two hydrogens of said one, two, three, four or five -CH₂- groups of -(CH₂)_{c}R^{2d} are each and individually substituted by a methyl group, and/or wherein one -CH₂- group of - (CH₂)_{c}R^{2d} which is different from the terminal -CH₂- groups is optionally replaced by -O-, -S-, -SO- or -SO₂-,
R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F, preferably an -OH group,
or R^{2b} is -H under the proviso that R^{2a} is -H,
or R^{2a} and R^{2b} form together a 3-, 4-, 5-, 6-, or 7- membered carbocycle or heterocycle that is optionally substituted by one or two substituents,
in formula (IVb)
X^{2a} is selected from the group consisting of -CH₂-, -CH(OH)-, -CH(F)-, -CH(NHR^{2p})-, -NH-, -S- and -O-, wherein R^{2p} is selected from the group consisting of -H, -Ac, a (C₁-C₄)alkyl and a Z group,
R^{2c} is selected from the group consisting of -H, -OH, -F, -NH₂ and a (C₁-C₃)alkyl group,
a = 1 or 2,
in formula (IVc)
b = 1, 2 or 3,
X^{2b} is selected from the group consisting of -N(R^{2p})-, -O-, -S-, -SO-, -SO₂-, -NH-, and -CH₂-, wherein R^{2p} is selected from the group consisting of -Ac, a (C₁-C₃)alkyl group and a Z group,
Xaa3 is a residue of an α-amino acid comprising a side chain, preferably an L-α-amino acid comprising a side chain,
wherein the side chain optionally comprises a Z group,
wherein the carbonyl group of Xaa3 is covalently attached to the α-nitrogen atom of Xaa4, and wherein
Xaa3 is a residue of an amino acid of formula (Va), or (Vb), 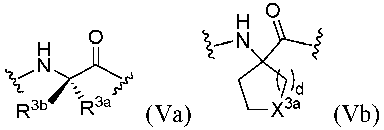 wherein
in formula (Va)
R^{3a} is selected from the group consisting of -(CH₂)ₑR^{3c}, -H, a (C₁-C₆)alkyl group, a (C₃-C₇)carbocycle, (C₃-C₇)heterocycle, an aryl group and a heteroaryl group and,
wherein R^{3c} is selected from the group consisting of a polar, a positively charged, a negatively charged, a zwitterionic and a hydrophobic group, wherein R^{3c} optionally comprises a Z group,
wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups is optionally replaced by -O-, -S-, -SO- or -SO₂-,
R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent,
or R^{3b} is -H under the proviso that R^{3a} is -H,
or R^{3a} and R^{3b} form together a 3-, 4-, 5-, 6-, or 7- membered carbocycle or heterocycle that is optionally substituted by one or two substituents,
in formula (Vb)
d = 1, 2 or 3,
X^{3a} is selected from the group consisting of -N(R^{3o})-, -O-, -S-, -SO-, -SO₂-, -NH-, and -CH₂-, wherein R^{3o} is selected from the group consisting of -Ac, a (C₁-C₃)alkyl group and a Z group,
Xaa4 is a residue of an L-α-amino acid comprising a side chain,
wherein the carbonyl group of Xaa4 is covalently attached to the α-nitrogen atom of Xaa5, and wherein
Xaa4 is a residue of an amino acid of formula (VI), 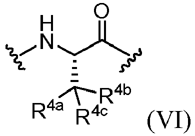 wherein
R^{4a} is selected from the group consisting of a (C₆-C₁₀)aryl group, a (C₅-C₁₀)heteroaryl group, a (C₅-C₁₀)carbocycle and a (C₅-C₁₀)heterocycle, and wherein each and any of the (C₆-C₁₀)aryl group, the (C₅-C₁₀)heteroaryl group, the (C₅-C₁₀)carbocycle and the (C₅-C₁₀)heterocycle is optionally substituted by 1, 2, or 3 substituents wherein, preferably, each substituent is individually and independently selected from the group consisting of -CONH₂, -OH, a halogen atom, a (C₁-C₆)alkyl group, -CN, -COOH, -NO₂, -NH₂, -NH-CNH-NH₂, -SO₃H, , an -O(C₁-C₆)alkyl group, wherein each substituent is optionally linked to the ring system of the (C₆-C₁₀)aryl group, the (C₅-C₁₀)heteroaryl group, the (C₅-C₁₀)carbocycle and the (C₅-C₁₀)heterocycle, and wherein said (C₁-C₆)alkyl group is optionally substituted by one or more fluorine atoms,
R^{4b} and R^{4c} are each and independently selected from the group consisting of -H and a methyl group,
Xaa5 is a residue of an L-α-amino acid comprising a side chain,
wherein the carbonyl group of Xaa5 is covalently attached to the α-nitrogen atom of Xaa6, and wherein
Xaa5 is a residue of an amino acid of formula (VII), 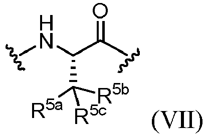 wherein
R^{5a} is selected from the group consisting of a (C₆-C₁₀)aryl group and a (C₅-C₁₀)heteroaryl group, and wherein each and any one of the (C₆-C₁₀)aryl group and the (C₅-C₁₀)heteroaryl group is optionally substituted by 1, 2, or 3 substituents, wherein, preferably, each substituent is individually and independently selected from the group consisting of a halogen atom, a (C₁-C₃)alkyl group, -CN, -NH₂, an -O(C₁-C₃)alkyl group, wherein the (C₁-C₃)alkyl group is optionally substituted by one or more fluorine atoms,
wherein R^{5b} and R^{5c} are each and independently selected from the group consisting of -H and a methyl group,
Xaa6 is a residue of an L-α-amino acid comprising a side chain and optionally comprising a substituent covalently attached to the α-nitrogen atom of Xaa6,
wherein the carbonyl group of Xaa6 is covalently attached to the α-nitrogen atom of Xaa7, and wherein
Xaa6 is a residue of an amino acid of formula (Villa), (VIIIb) or (VIIIc) 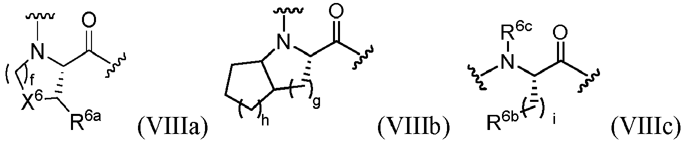 wherein
in formula (VIIIa)
f = 1 or 2,
R^{6a} is selected from the group consisting of -F, -H, -OH, and a (C₁-C₄)alkyl group,
X⁶ is absent or selected from the group consisting of -CH(R^{6d})-, -S-, -O- and -NH-,
wherein R^{6d} is selected from the group consisting of -F, -H, a (C₁-C₄)alkyl group and -OH and,
in formula (VIIIb)
g = 0, 1 or 2,
h = 1, 2 or 3,
in formula (VIIIc)
i = 1 or 2,
R^{6b} is selected from the group consisting of a (C₂-C₆)alkyl group, an aryl group, an aryl group substituted with 1 or 2 substituents, a (C₅-C₆)heteroaryl group, a (C₅-C₆)heteroaryl group substituted with 1 or 2 substituents, a (C₅-C₆)carbocycle, and a (C₅-C₆)carbocycle substituted with 1 or 2 substituents,
R^{6c} is selected from the group consisting of -H and a (C₁-C₃)alkyl group,
Xaa7 is a residue of an α-amino acid comprising a side chain and optionally comprising a substituent covalently attached to the α-nitrogen atom of Xaa7,
wherein the side chain optionally comprising a Z group,
wherein the carbonyl group of Xaa7 is covalently attached to the α-nitrogen atom of Xaa8, and wherein
Xaa7 is a residue of an amino acid of formula (IXa), (IXb), (IXc), (IXd), and (IXe) 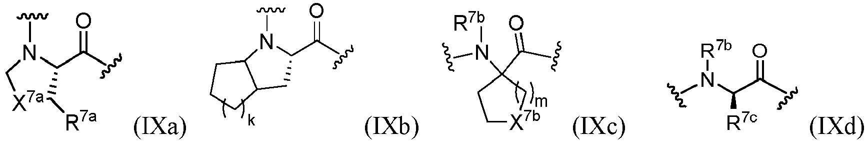 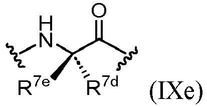 wherein
in formula (IXa)
R^{7a} is selected from the group consisting of -H, -OH, -F, -NH₂, and a (C₁-C₃)alkyl group,
X^{7a} is absent or selected from the group consisting of -CH(R^{7f})-, -S-, -O-, and -NH-, or is absent,
wherein R^{7f} is selected from the group consisting of -OH, -H, -F, -NH(R^{7g}) and a (C₁-C₆)alkyl group, and wherein R^{7g} is selected from the group consisting of -H, -Ac, a (C₁-C₃)alkyl group and a Z group,
in formula (IXb)
k = 1 or 2,
in formula (IXc)
m = 1, 2 or 3,
R^{7b} is selected from the group consisting of -H, and a (C₁-C₂)alkyl group, X^{7b} is absent or selected from the group consisting of -N(R^{7g})-, -O-, -S-, -SO-, -SOz- and -CH₂-,
wherein R^{7g} is selected from the group consisting of -H, -Ac, a (C₁-C₃)alkyl group and a Z group,
in formula (IXd)
R^{7b} is selected from the group consisting of -H, and a (C₁-C₂)alkyl group,
R^{7c} is selected from the group consisting of -H, and -(CH₂)ₙR^{7h},
wherein n = 1, 2, or 3, preferably n = 1, and wherein one of the one, two, or three -CH₂- groups of -(CH₂)ₙR^{7h} which is different from the terminal groups in formula (IXd) is optionally replaced by -O-, -S-, -SO-, or -SO-z,
wherein R^{7h} is selected from the group consisting of -H, -OH, -NR⁷ⁱR^{7k}, -N(CH₃)₃⁺, -NH-CNH-NH₂, -CONH₂, -NH-CO-NH₂ and a (C₁-C₃)alkyl group, an aryl group, a substituted aryl group substituted by one substituent, a heteroaryl group, a heteroaryl group substituted by one substituent, wherein, preferably, each substituent is individually and independently selected from the group consisting of a methyl group, a halogen atom, -NH₂ and -OH, wherein R⁷ⁱ and R^{7k} are each and independently selected from the group consisting of -H and a methyl group,
in formula (IXe)
R^{7d} is selected from the group consisting of a (C₁-C₆)alkyl group, a (C₃-C₇)carbocycle, (C₃-C₇)heterocycle, an aryl group, a heteroaryl group and -(CH₂)ₒR^{7l},
R^{7l}is selected from the group consisting of a polar, a positively charged, a negatively charged, a zwitterionic and a hydrophobic group, wherein R^{7l}optionally comprises a Z group,
o = 1, 2, 3, 4 or 5, and wherein one of the one, two, three, four or five -CH₂- groups linking R^{7l} to the α-C atom of Xaa7 in formula (IXe) are optionally substituted by a methyl group, and/or wherein one of said -CH₂- groups which is different from the terminal groups is optionally replaced by -O-, -S-, -SO-, or -SOz-,
R^{7e} is selected from the group consisting of -H, and a (C₁-C₃)alkyl group, wherein said (C₁-C₃)alkyl group is optionally substituted by a substituent preferably selected from the group consisting of -F, -Cl and -OH,
Xaa8 is a residue of an α-amino acid comprising a side chain,
wherein the carbonyl group of Xaa8 is covalently attached to the α-nitrogen atom of Xaa9, and wherein
Xaa8 is a residue of an amino acid of formula (Xa) or (Xb) 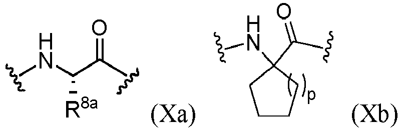 wherein
in formula (Xa)
R^{8a} is selected from the group consisting of a -CH(R^{8b})(R^{8c}) group (C₄-C₈)carbocycle, an aryl group, a and heteroaryl group and,
wherein R^{8b} is selected from the group consisting of -H and a (C₁-C₃)alkyl group,
wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
wherein the -CH₂- groups of the optional linker or any -CH₂-groups of the carbocycle groups of R^{8a} or of the (C₁-C₆)alkyl group of R^{8c} are optionally replaced by one atom selected from the group consisting of -O- and -S-, and
wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group,
in formula (Xb)
p = 1, 2, or 3,
a -CH₂- group in the ring of formula (Xb) is optionally replaced by one atom selected from the group consisting of -O- and -S-, and one or more H atoms of the ring -CH₂- groups are optionally substituted by a -F or -Cl atom,
Xaa9 is a residue of an α-amino acid comprising a side chain,
wherein the carbonyl group of Xaa9 is covalently attached to the nitrogen atom of the amine group of XaalO, and wherein
Xaa9 is a residue of an amino acid of formula (XI) 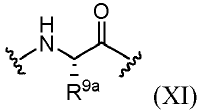 wherein
R^{9a} is selected from the group consisting of a (C₁-C₃)alkyl group, a (C₃-C₅)carbocycle and -CH₂R^{9b}, wherein R^{9b} is selected from the group consisting of a (C₃-C₄)carbocycle and a (C₁-C₄)alkyl group, wherein the -CH₂- group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, and wherein one or two H atoms in R^{9a} are optionally replaced by -F or -Cl,
Xaa10 is a residue of an amino thiol comprising an amine group, a thiol group and optionally a carbonyl group,
wherein the sulfur atom of the thiol group is covalently attached to the sulfur atom of the thiol group of Xaa1,
and wherein
Xaa10 is a residue of formula (XII), 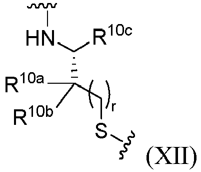 wherein
R^{10a} and R^{10b} are each and independently selected from the group consisting of -H and a methyl group,
r = 1 or 2,
R^{10c} is selected from the group consisting of -CONH₂, -H, -CO-Cterm, -CH₂(OH), and -CON(R^{10d})(R^{10e}), wherein Cterm comprises a Z group, wherein R^{10d} and R^{10e} are each and independently selected from the group consisting of -H and a methyl group,
an N-terminal modification group A,
the N-terminal modification group A is either a hydrophobic blocking group Abl, or Xaa0,
the N-terminal modification group A is covalently attached to the α-nitrogen atom of Xaa1,
wherein if the N-terminal modification group A is the blocking group Abl, the blocking group Abl is of formula (IIa), of formula (IIb) or of formula (IIc) 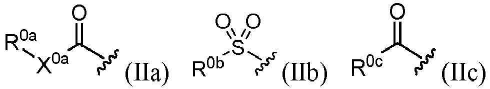
wherein X^{0a} is selected from the group consisting of -O-, -NH- and -S-wherein R^{0a} is selected from the group consisting of a (C₃-C₉)alkyl group, a (C₃-C₈)carbocycle, an aryl group, a heteroaryl group, and a (C₃-Cs)heterocycle, and wherein R^{0a} is optionally linked to X^{0a} by a linker comprising one or two -CH₂- groups, and wherein R^{0a} is optionally substituted by one or two substituents, wherein, preferably, each substituent is independently selected from the group consisting of -OH, a halogen atom, a (C₁-C₆)alkyl group, a (C₃-C₈)carbocycle, an aryl group, a heteroaryl group, and a (C₃-C₈)heterocycle,
wherein R^{0b} is selected from the group consisting of a (C₃-C₉)alkyl group, a (C₃-C₈)carbocycle, an aryl group, a heteroaryl group, and a (C₃-Cs)heterocycle, and wherein R^{0b} is optionally linked to the -SOz- moiety in formula (IIb) by a linker comprising one, two or three -CH₂- groups, ,
wherein R^{0c} is selected from the group consisting of a (C₃-C₉)alkyl group, a (C₃-C₈)carbocycle, an aryl group, a heteroaryl group, and a (C₃-C₈)heterocycle, and wherein R^{0c} is optionally linked to the carbonyl moiety in formula (IIc) by a linker comprising one, two or three -CH₂-groups, preferably the linker is absent or consists of one or two -CH₂-groups, wherein one -CH₂- group of R^{0c} is optionally replaced by -O- or -S-, and wherein R^{0c} is optionally substituted by one or two substituents,
wherein if the N-terminal modification group A is Xaa0,
Xaa0 is a residue of an α-amino acid comprising a side chain and optionally comprising up to three substituents covalently attached to the α-nitrogen atom of Xaa0, wherein the side chain comprises a hydrophobic group, and wherein, if present, one substituent covalently attached to the α-nitrogen atom of Xaa0 is an N-terminal extension group Nterm, and wherein the N-terminal extension group Nterm optionally comprises a Z group,
preferbal Xaa0 is a residue of formula (IId) or of formula (IIe) 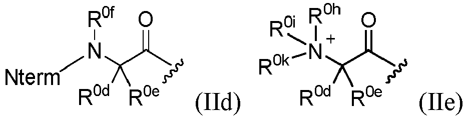
wherein R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, and wherein R^{0d} is optionally substituted by one or two substituents, wherein, preferably, each substituent is independently selected from the group consisting of -OH, -F, -Cl, a (C₁-C₆)alkyl group, a (C₃-C₈)carbocycle group, an aryl group, a heteroaryl group, and a (C₃-C₈)heterocycle,
wherein R^{0e} is selected from the group consisting of -H, an aryl group, a (C₁-C₆)alkyl group, and a (C₃-C₇) carbocycle,
or wherein R^{0d} and R^{0e} together form a 4-, 5-, 6-, 7- or 8- membered carbocycle or heterocycle, wherein the carbocycle and the heterocycle are optionally substituted by one or two substituents, wherein, preferably,
each substituent is independently selected from the group consisting of a methyl group, -F, -Cl, and -OH,
wherein R^{0f} is selected from the group consisting of -H and a (C₁-C₄)alkyl group,
wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide,
wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SO₂-, -O-, or -NH-, and wherein R^{0g} is optionally substituted by one, two or three substituents,
or a pharmaceutically acceptable salt or hydrate or pharmaceutically acceptable solvate thereof.

2. The compound or pharmaceutically acceptable salt or hydrate or pharmaceutically acceptable solvate of claim 1,
wherein
the N-terminal modification group A is a blocking group Abl, the blocking group Abl is of formula (IIa) 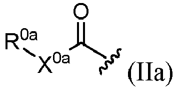
wherein X^{0a} is selected from the group consisting of -O-, -CH₂-, -NH-, and -S-,
wherein R^{0a} is selected from the group consisting of a (C₃-C₆)alkyl group, a phenyl group and a (C₅-C₆)carbocycle.

3. The compound or pharmaceutically acceptable salt or hydrate or pharmaceutically acceptable solvate of claim 1, wherein the N-terminal modification group A is Xaa0, wherein
Xaa0 is a residue of formula (IId) 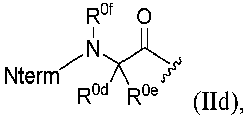
wherein R^{0d} is selected from the group consisting of a (C₂-C₈)alkyl group, a (C₃-C₈)carbocycle, an aryl group, a heteroaryl group and a (C₃-C₈)heterocycle, and wherein R^{0d} is optionally linked to the α-carbon atom of Xaa0 by a linker comprising one or two -CH₂- groups, and wherein R^{0d} is optionally substituted by one or two substituents, wherein, preferably, each substituent is individually and independently selected from the group consisting of -OH, -F, - Cl, a (C₁-C₆)alkyl group, a (C₃-C₈)carbocycle group, an aryl group, a heteroaryl group, and a (C₃-C₈)heterocycle,
wherein R^{0e} is selected from the group consisting of -H, an aryl group, a (C₁-C₆)alkyl group, and a (C₃-C₇) carbocycle,
or wherein R^{0d} and R^{0e} together form a 4-, 5-, 6-, 7- or 8- membered carbocycle or heterocycle, wherein the carbocycle and the heterocycle are optionally substituted by one or two substituents, wherein, preferably, each substituent is individually and independently selected from the group consisting of a methyl group, -F, -Cl, and -OH,
wherein R^{0f} is selected from the group consisting of -H and a (C₁-C₄)alkyl group,
wherein Nterm is selected from the group consisting of -H, -R^{0g}, R^{0g}-CO-, R^{0g}-NH-CO-, R^{0g}-O-CO-, R^{0g}-SO₂-, R^{0g}-S-CO-, R^{0g}-NH-CNH-, and a Z group, and wherein Nterm is optionally linked to the α-nitrogen atom of Xaa0 by a linker comprising one amino acid or a dipeptide,
wherein R^{0g} is selected from the group consisting of a (C₁-C₈)alkyl group, a (C₃-C₈)carbocycle, an aryl group, a heteroaryl group, and a (C₃-C₈)heterocycle, wherein if R^{0g} is a (C₁-C₈)alkyl group one of the -CH₂- groups in R^{0g} is optionally replaced by -S-, -SO-, -SOz-, -O-, or -NH-, and wherein R^{0g} is optionally substituted by one, two or three substituents, wherein, preferably, each substituent is individually and independently selected from the group consisting of -OH, -NH₂, a halogen atom, -COOH, -CONH₂, -SO₃H, -NH-CNH-NH₂, a (C₁-C₆)alkyl group, a (C₃-C₇)carbocycle, an aryl group, a heteroaryl group, and a (C₃-C₇)heterocycle.

4. The compound or pharmaceutically acceptable salt or hydrate or pharmaceutically acceptable solvate of claim 3, wherein
R^{0d} is selected from the group consisting of a (C₂-C₅)alkyl group, a (C₅-C₆)carbocycle, and a phenyl group.
R^{0e} is selected from the group consisting of -H and a methyl group;
R^{0f} is selected from the group consisting of -H and a methyl group and
Nterm is selected from the group consisting of -H, -R^{0g}, R^{0g}-CO-, R^{0g}-NH-CO- and a Z group.

5. The compound or pharmaceutically acceptable salt or hydrate or pharmaceutically acceptable solvate of any one of claims 1 to 4, wherein Xaa2 is a residue of an α- amino acid of formula (IVa) 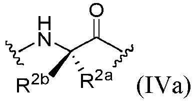 wherein
R^{2a} is selected from the group consisting of -(CH₂)_{c}R^{2d},-H, a (C₁-C₆)alkyl group, a (C₃-C₇)carbocycle, (C₃-C₇)heterocycle, an aryl group and a heteroaryl group,
wherein R^{2d} is selected from the group consisting of a polar, a positively charged, a negatively charged, a zwitterionic and a hydrophobic group, wherein R^{2d} optionally comprises a Z group,
wherein c = 1, 2, 3, 4, or 5, wherein optionally one or two hydrogens of said one, two, three, four or five -CH₂- groups of -(CH₂)_{c}R^{2d} are each and individually substituted by a methyl group, and/or wherein one -CH₂- group of -(CH₂)_{c}R^{2d} which is different from the terminal groups is optionally replaced by -O-, -S-, -SO- or -SOz-,
R^{2b} is selected from the group consisting of -H and a (C₁-C₃)alkyl group under the proviso that R^{2a} is selected from the group consisting of a (C₁-C₆)alkyl group, a (C₃-C₇)carbocycle, (C₃-C₇)heterocycle, an aryl group, a heteroaryl group and -(CH₂)_{c}R^{2d}, said (C₁-C₃)alkyl group is optionally substituted by a substituent, wherein the substituent is preferably selected from the group consisting of -OH, -COOH and -F,
or R^{2b} is -H under the proviso that R^{2a} is -H,
or R^{2a} and R^{2b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, -COOH, -CONH₂, -NH₂ and -OH,
preferably R^{2b} is H and R^{2a} is selected from the group consisting of -(CH₂)_{c}R^{2d} and -H.

6. The compound or pharmaceutically acceptable salt or hydrate or pharmaceutically acceptable solvate of claims 5, wherein c = 1, 2, or 3, and wherein optionally one hydrogen atom of said one, two or three -CH₂- groups of -(CH₂)_{c}R^{2d} is substituted by a methyl group, preferably c = 1 and one hydrogen atom is substituted by a methyl group and R^{2d} is OH.

7. The compound or pharmaceutically acceptable salt or hydrate or pharmaceutically acceptable solvate of any one of claims 1 to 6, wherein Xaa3 is a residue of an amino acid of formula (Va), 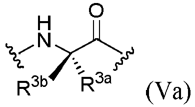 wherein
R^{3a} is selected from the group consisting of -(CH₂)ₑR^{3c}, -H, a (C₁-C₆)alkyl group, a (C₃-C₇)carbocycle, (C₃-C₇)heterocycle, an aryl group and a heteroaryl group ,
wherein R^{3c} is selected from the group consisting of a polar, a positively charged, a negatively charged, a zwitterionic and a hydrophobic group, wherein R^{3c} optionally comprises a Z group,
wherein e = 1, 2, 3, 4, or 5, wherein optionally one or two hydrogens of said one, two, three, four or five -CH₂- groups of -(CH₂)ₑR^{3c} are each and individually substituted by a methyl group, and/or wherein one -CH₂- group of -(CH₂)ₑR^{3c} which is different from the terminal groups is optionally replaced by -O-, -S-, -SO- or -SOz-,
R^{3b} is selected from the group consisting of -H and a (C₁-C₃)alkyl group under the proviso that R^{3a} is selected from the group consisting of a (C₁-C₆)alkyl group, a (C₃-C₇)carbocycle, (C₃-C₇)heterocycle, an aryl group, a heteroaryl group and -(CH₂)ₑR^{3c}, wherein said (C₁-C₃)alkyl group is optionally substituted by a substituent, wherein the substituent is preferably selected from the group consisting of -OH, -COOH, and -F,
or R^{3b} is -H under the proviso that R^{3a} is -H,
or
R^{3a} and R^{3b} form together a 3-, 4-, 5-, 6-, or 7- membered carbocycle or heterocycle that is optionally substituted by one or two substituents, wherein, preferably, each substituent is individually and independently selected from the group consisting of a methyl group, a halogen atom, - NH₂ and -OH.

8. The compound or pharmaceutically acceptable salt or hydrate or pharmaceutically acceptable solvate of any one of claims 1 to 7, wherein Xaa3 is a residue of an L-α amino acid of formula (Vc) 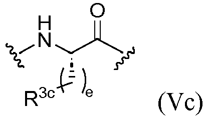 wherein
R^{3c} is selected from the group consisting of -N(R^{3d})(R^{3e}), -H, -OH, - N(R^{3f})(R^{3g})(R^{3h})⁺, -COOH, a halogen atom, -CN, -N₃, -NOz, - N(R^{3d})-CN(R^{3e})-N(R³ⁱ)(R^{3k}), -SO₂N(R^{3d})(R^{3l}), -CON(R^{3d})(R^{3l}), -NH-CO-N(R^{3d})(R^{3l}), -SO₃H, -R^{3m}, -NH-SO₂-R^{3m}, -CO-R³ⁿ, and -NH-COR3m,
wherein R^{3d}, R³ⁱ and R^{3k} are each and independently selected from the group consisting of -H and a (C₁-C₂)alkyl group,
wherein R^{3f} and R^{3g} are each and independently selected from the group consisting of a (C₁-C₃)alkyl group,
wherein R^{3e} is selected from the group consisting of a Z group, -H, -Ac, and a (C₁-C₃)alkyl group ,
wherein R^{3h} is selected from the group consisting of a (C₁-C₃)alkyl group and a substituted (C₂-C₄)alkyl group, wherein the substituted (C₂-C₄)alkyl group is substituted by at least one substituent preferably selected from the group consisting of OH-, -COOH, and -SO₃H,
wherein R^{3l} is selected from the group consisting of -H and R^{3m},
wherein R^{3m} is selected from the group consisting of a (C₁-C₆)alkyl group, a (C₃-C₇)carbocycle, a (C₃-C₇)heterocycle, a (C₆-C₁₀)aryl group and a (C₅-C₁₀)heteroaryl group, and R^{3m} is optionally substituted by one or two or three substituents, wherein, preferably, each substituent is individually and independently selected from the group consisting of a methyl group, -CONH₂, -COOH, a halogen atom, -NH-CNH-NH₂, -NH₂ and -OH,
wherein R³ⁿ is a Z group,
e = 1, 2, 3, 4, or 5, wherein optionally one or two hydrogens of said one, two, three, four or five -CH₂- groups of -(CH₂)ₑR^{3c} are each and individually substituted by a methyl group, and wherein one -CH₂-group which is different from the terminal groups is optionally replaced by -O-, -S-, -SO- or -SOz-.

9. The compound or pharmaceutically acceptable salt or hydrate or pharmaceutically acceptable solvate of claim 8, wherein wherein R^{3c} is selected from the group consisting of - NH(R^{3e}) and -CO-R³ⁿ, wherein R^{3e} and R³ⁿ are each a Z group, preferably R^{3c} is -NH(R^{3e}) and e is 3 or 4.

10. The compound or pharmaceutically acceptable salt or hydrate or pharmaceutically acceptable solvate of any one of claims 1 to 9, wherein R^{4a} is selected from the group consisting of a phenyl group, wherein the phenyl group, is optionally substituted by 1 or 2 substituents, wherein, preferably, each substituent is individually and independently selected from the group consisting of -CONH₂, -OH, -F, -Cl, a methyl group, -CN, -NH₂ and an -OCH₃ group, wherein each substituent is optionally linked via a linker, preferably a methylene bridge, to the ring system of the phenyl group, more preferably R^{4a} is either a 4-carbamoylphenyl or a 3,4-dihydroxyphenyl group.

11. The compound or pharmaceutically acceptable salt or hydrate or pharmaceutically acceptable solvate of any one of claims 1 to 10, wherein R^{5a} is selected from the group consisting of a phenyl group, a 2-thienyl group, a 3-pyridyl group, an 1-naphthyl group and a 3-benzothienyl group, wherein each and any of these aryl and heteroaryl groups, is optionally substituted by 1 or 2 substituents, wherein, preferably, each substituent is individually and independently selected from the group consisting of a halogen atom, a methyl group, -CN, - NH₂ and an -OCH₃ group, wherein the methyl group is optionally substituted by one or more fluorine atoms; more preferably R^{5a} is selected from the group consisting of a phenyl group and a 2-thienyl group, wherein each and any of the phenyl group and a 2-thienyl group, is optionally substituted by 1 or 2 substituents, wherein, preferably, each substituent is individually and independently selected from the group consisting of -F, -Cl and a methyl group, most preferably R^{5a} is a phenyl group.

12. The compound or pharmaceutically acceptable salt or hydrate or pharmaceutically acceptable solvate of any one of claims 1 to 11, wherein Xaa6 is a residue of an L-α-amino acid of formula (VIIIa) 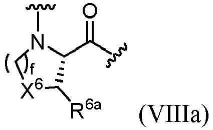 wherein
f = 1 or 2,
R^{6a} is selected from the group consisting of -H, -OH, -F and a (C₁-C₄)alkyl group,
X⁶ is absent or selected from the group consisting of -CH(R^{6d})-, -S-, -O- and -NH-,
wherein R^{6d} is selected from the group consisting of -F, -H, a (C₁-C₄)alkyl group and -OH.

13. The compound or pharmaceutically acceptable salt or hydrate or pharmaceutically acceptable solvate of claim 12, wherein X⁶ is -CH(R^{6d})- or -S-, and wherein R^{6d} is selected from the group consisting of -F, -H, a (C₁-C₄)alkyl group, and -OH, preferably
f = 1, X⁶ is -CH(R^{6d})-, and wherein R^{6d} is selected from the group consisting of -H, a methyl group, -OH and -F, more preferably R^{6d} is -F.

14. The compound or pharmaceutically acceptable salt or hydrate or pharmaceutically acceptable solvate of any one of claims 1 to 13, wherein Xaa7 is a residue of an L-α-amino acid of formula (IXa) optionally comprising a Z group 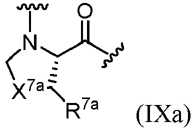 wherein
R^{7a} is selected from the group consisting of -H, -OH, -F, -NH₂ and a (C₁-C₃)alkyl group,
X^{7a} is absent or selected from the group consisting of -CH(R^{7f})-, -S-, -O-, -NH-, wherein R^{7f} is selected from the group consisting of -OH, -H, -F, -NH(R^{7g}) and a (C₁-C₆)alkyl group, and wherein R^{7g} is selected from the group consisting of -H, -Ac, a (C₁-C₃)alkyl group and a Z group.

15. The compound or pharmaceutically acceptable salt or hydrate or pharmaceutically acceptable solvate of claim 14, wherein X^{7a} is -CH(R^{7f})-, and wherein R^{7f} is selected from the group consisting of -OH, -H, , -F, -NH(R^{7g}) and a methyl group, and wherein R^{7g} is selected from the group consisting of -H, a methyl group and a Z group, preferably R^{7f} is -OH.

16. The compound or pharmaceutically acceptable salt or hydrate or pharmaceutically acceptable solvate of any one of claims 1 to 15, wherein Xaa8 is a residue of an L-α-amino acid of formula (Xa) 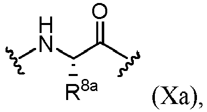 wherein
R^{8a} is selected from the group consisting of a -CH(R^{8b})(R^{8c}) group, a (C₄-C₈)carbocycle, an aryl group, and a heteroaryl group,
wherein R^{8b} is selected from the group consisting of -H and a (C₁-C₃)alkyl group,
wherein R^{8c} is selected from the group consisting of a (C₁-C₆)alkyl group, a (C₃-C₈)carbocycle, an aryl group and a heteroaryl group, and wherein R^{8c} is optionally linked to the β-carbon atom of Xaa8 by a linker comprising one or or two -CH₂- groups,
wherein the -CH₂- groups of the optional linker or any -CH₂-groups of the carbocycle groups of R^{8a} or of the (C₁-C₆)alkyl group of R^{8c} are optionally replaced by one atom selected from the group consisting of -O- and -S-, and
wherein one, two or three hydrogen atoms of R^{8a} which are different from the hydrogen atom bound to the β-carbon atom of Xaa8 in the -CH(R^{8b})(R^{8c}) group, are optionally replaced by atoms or groups each and independently selected from the group consisting of a halogen atom and a (C₁-C₃)alkyl group, preferably -F, -Cl or a methyl group.

17. The compound or pharmaceutically acceptable salt or hydrate or pharmaceutically acceptable solvate of claim 16, wherein R^{8b} is selected from the group consisting of -H and a methyl group, preferably R^{8b} is a methyl group and wherein R^{8c} is selected from the group consisting of a (C₂-C₄)alkyl group, a (C₃-C₆)carbocycle, a phenyl group and a 2-thienyl group, preferably R^{8c} is an ethyl group.

18. The compound or pharmaceutically acceptable salt or hydrate or pharmaceutically acceptable solvate of any one of claims 1 to 17, wherein Xaa9 is a residue of an L-α-amino acid of formula (XI) 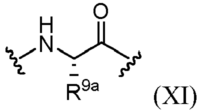 wherein
R^{9a} is selected from the group consisting of a (C₂-C₃)alkyl group, a cyclobutyl group and -CH₂R^{9b}, wherein R^{9b} is selected from the group consisting of a (C₃-C₄)carbocycle and a (C₁-C₃)alkyl group, wherein the -CH₂- group linking R^{9b} to the α-C atom of Xaa9 in formula (XI) is optionally substituted by a methyl group, wherein a -CH₂- group of R^{9a} is optionally replaced by one atom selected from the group consisting of -O- and -S-, preferably R^{9a} is a n-propyl group.

19. The compound or pharmaceutically acceptable salt or hydrate or pharmaceutically acceptable solvate of any one of claims 1 to 18,
wherein R^{10a} and R^{10b} are each -H, and
wherein R^{10c} is selected from the group consisting of, -CONH₂, -CO-Cterm and - CH₂(OH), and
wherein Cterm is a Z group.

20. The compound or pharmaceutically acceptable salt or hydrate or pharmaceutically acceptable solvate of any one of claims 1 to 19,
wherein the blocking group Abl is selected from the group consisting of Butoc, Hex, Pen, But, iHex, nBuCAyl, PrCAyl, Oct, EtOPr, Bz, Pha, Chex, Cp and PentylSOz, preferably the blocking group Abl is selected from the group consisting of Butoc, Hex, Pen, iHex, and nBuCAyl, more preferably the blocking group Abl is Butoc;
wherein Xaa1 is a residue of an amino acid selected from the group consisting of Cys and Pen, preferably Xaa1 is a residue of Cys;
wherein Xaa2 is a residue of an amino acid selected from the group consisting of Thr, Arg, Arg(Me), Asp, Glu, Gln, Lys, Lys(Me), Lys(Z), Cit, Ala, Aib, Har, Asn, Glu(Z), Orn(mPEG12), KMe3, Orn, Orn(Me), Orn(Z), Ser, Thp, Apc, Apc(Z), Gly, Pro, Tyr and Leu, wherein Z is a Z group; preferably Xaa2 is a residue of an amino acid selected from the group consisting of Thr, Arg, Arg(Me), Asp, Glu, Gln, Orn(DOTA), Lys, Lys(Me), Lys(DOTA), Cit, Ala, Aib, Har, Asn, Glu(AGLU), Orn(mPEG12), KMe3, Orn, Orn(Me), Orn(Tris-Nta(Tris)), Ser, Thp, Apc, Apc(DOTA), Gly, Pro, Tyr and Leu; more preefably Xaa2 is a residue of an amino acid selected from the group consisting of Thr and Cit, most preferably Xaa2 is a residue of Thr;
Xaa3 is a residue of an amino acid selected from the group consisting of Orn(Z), Lys(Z), Met, Arg, Arg(Me), Dap, Dab, Leu, Tyr, Gln, Glu, Glu(Z), Lys(Ac), Cit, Ala, Nle, Asp, Har, Asn, Thr, Apc(Z), Dab(Z), Dap(Z), Lys(AF488-Ttds), Lys, Lys(Me), KMe3, Orn, Orn(Me), Ser and Gly, wherein Z is a Z group; preferably Xaa3 is a residue of an amino acid selected from the group consisting of Orn(DOTA), Lys(DOTA), Met, Arg, Arg(Me), Dap, Dab, Leu, Tyr, Gln, Glu, Lys(Ac), Cit, Ala, Nle, Asp, Har, Asn, Thr, Apc(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Orn(Me), Ser and Gly; more preferably Xaa3 is a residue of an amino acid selected from the group consisting of Orn(DOTA), Lys(DOTA), Gln, Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw),most preferably Xaa3 is a residue of an amino acid selected from the group consisting of Orn(DOTA) and Lys(DOTA);
wherein Xaa4 is a residue of an amino acid selected from the group consisting of Pcnf, Dopa, Tyr, Phe, Aph, His, Mpa, My, Mcy, Guf, Ppa, Nif, Cha, Trp, Ay3, 2Ni and Ocf, preferably Xaa4 is a residue of an amino acid selected from the group consisting of Pcnf, Dopa, Tyr, Aph, Mpa, Nif, Ocf and My , more preferably Xaa4 is a residue of an amino acid selected from the group consisting of Pcnf and Dopa;
wherein Xaa5 is a residue of an amino acid selected from the group consisting of Phe, Thi, Pcf, Ocf, Egm, Mcf, 1Ni, Pnf, Mpa, Bta and Trp, preferably Xaa5 is a residue of an amino acid selected from the group consisting of Phe, Thi, Pcf, Ocf, Egm, Mcf, 1Ni, Pnf and Mpa; more preferably Xaa5 is a residue of an amino acid selected from the group consisting of Phe, Thi, Pcf, Ocf and Egm, most preferably Xaa5 is a residue of Phe;
wherein Xaa6 is a residue of an amino acid selected from the group consisting of 4Cfp, Pro, Hyp, Eaz, Oic, Nmb, Leu, Cha, Chy, 4Tfp, Pip, Oxa, Aze and Phe; preferably Xaa6 is a residue of an amino acid selected from the group consisting of 4Cfp, Pro and Hyp more preferably Xaa6 is a residue of 4Cfp;
wherein Xaa7 is a residue of an amino acid selected from the group consisting of Hyp, Pro, 4Cfp, Tap, Ala, Aib, Asp, Ser, Tyr, ser, dap, tyr, Leu, 4Tfp, Chy, 4Ap, ala, Arg, Apc(Z), Tap(Z), Gly, nma, Aze, Oic, Oxa, Lys(Z) and Nmg, wherein Z is a Z group, preferably Z is DOTA; preferably Xaa7 is a residue of an amino acid selected from the group consisting of Hyp, Pro, 4Cfp, Tap, Ala, Aib, 4Tfp, Chy, 4Ap, ala, Arg, Tap(Z), Lys(Z), Ser, ser, Leu, tyr and nma, wherein Z is a Z group, preferably Z is DOTA; more preferably Xaa7 is a residue of an amino acid selected from the group consisting of Hyp, Pro, Aib, ala, nma, 4Tfp, Lys(DOTA) and Tap, most preferably Xaa7 is a residue of Hyp;
wherein Xaa8 is a residue of an amino acid selected from the group consisting of Ile, Chg, Cha, Cpg, Cbg, Npg, Phe, Pcf, Egz, Hfe, Phg, Leu, Nle and Nva; preferably Xaa8 is a residue of an amino acid selected from the group consisting of Ile, Chg, Cha, Cpg, Phg, Pcf, Cbg and Npg; more preferably Xaa8 is a residue of an amino acid selected from the group consisting of Ile, Chg, Phg and Cha, most preferably Xaa8 is a residue of Ile;
wherein Xaa9 is a residue of an amino acid selected from the group consisting of Nva, Leu, Cpra, Abu, Ser(Me), Cbg, Npg, Ala, Nle, Ile and Val; preferably Xaa9 is a residue of an amino acid selected from the group consisting of Nva, Leu, Cpra, Abu, Ser(Me) and Cbg; more preferably Xaa9 is a residue of an amino acid selected from the group consisting of Nva, Leu and Cpra, most preferably Xaa9 is a residue of Nva;
and/or
Xaa10 is a residue of an amino acid selected from the group consisting of Cys-NH₂, Cysol-OH, AET, Hcy-NH₂, Pen-NH₂ and Cys-O2Oc-Lys(Z)-NH2, wherein Z is a Z group, preferably Z is DOTA; more preferably Xaa10 is a residue of an amino acid selected from the group consisting of Cys-NH₂ and Cysol-OH; most preferably Xaa10 is a residue of Cys-NH₂;
or any combination of any one of Xaa1, Xaa2, Xaa3, Xaa4, Xaa5, Xaa6, Xaa7, Xaa8, Xaa9, Xaa10 and blocking group Abl as defined herein.

21. The compound or pharmaceutically acceptable salt or hydrate or pharmaceutically acceptable solvate of any one of claims 1 to 20, wherein
a)
the blocking group Abl is selected from the group consisting of Butoc, Hex, Pen, But, iHex, nBuCAyl, PrCAyl, Oct, EtOPr, Bz, Pha, Chex, Cp and PentylSOz,
Xaa1 is a residue of an amino acid selected from the group consisting of Cys and Pen,
Xaa2 is a residue of an amino acid selected from the group consisting of is a residue of an amino acid selected from the group consisting of Thr, Arg, Arg(Me), Asp, Glu, Gln, Lys, Lys(Me), Lys(Z), Cit, Ala, Aib, Har, Asn, Glu(Z), Orn(mPEG12), KMe3, Orn, Orn(Me), Orn(Z), Ser, Thp, Apc, Apc(Z), Gly, Pro, Tyr and Leu,
Xaa3 is a residue of an amino acid selected from the group consisting of Orn(Z), Lys(Z), Met, Arg, Arg(Me), Dap, Dab, Leu, Tyr, Gln, Glu, Glu(Z), Lys(Ac), Cit, Ala, Nle, Asp, Har, Asn, Thr, Apc(Z), Dab(Z), Dap(Z), Lys(AF488-Ttds), Lys, Lys(Me), KMe3, Orn, Orn(Me), Ser and Gly,
Xaa4 is a residue of an amino acid selected from the group consisting of Pcnf, Dopa, Tyr, Phe, Aph, His, Mpa, My, Mcy, Guf, Ppa, Nif, Cha, Trp, Ay3, 2Ni and Ocf,
Xaa5 is a residue of an amino acid selected from the group consisting of Phe, Thi, Pcf, Ocf, Egm, Mcf, 1Ni, Pnf, Mpa, Bta and Trp,
Xaa6 is a residue of an amino acid selected from the group consisting of 4Cfp, Pro, Hyp, Eaz, Nmb, Oic, Leu, Cha, Chy, 4Tfp, Pip, Oxa, Aze and Phe,
Xaa7 is a residue of an amino acid selected from the group consisting of Hyp, Pro, 4Cfp, Tap, Ala, Aib, Asp, Ser, Tyr, ser, dap, tyr, Leu, 4Tfp, Chy, 4Ap, ala, Arg, Apc(Z), Tap(Z), Gly, nma, Aze, Oic, Oxa, Lys(Z) and Nmg,
Xaa8 is a residue of an amino acid selected from the group consisting of Ile, Chg, Cha, Cpg, Cbg, Npg, Phe, Pcf, Egz, Hfe, Phg, Leu, Nle and Nva,
Xaa9 is a residue of an amino acid selected from the group consisting of Nva, Leu, Cpra, Abu, Ser(Me), Cbg, Npg, Ala, Nle, Ile and Val, and
Xaa10 is a residue of an amino acid selected from the group consisting of Cys-NH₂, Cysol-OH, AET, Hcy-NH₂, Pen-NH₂ and Cys-O2Oc-Lys(Z)-NH₂,
wherein Z is a Z group
or b)
the blocking group Abl is selected from the group consisting of Butoc, Hex, Pen, iHex, and nBuCAyl,
Xaa1 is a residue of Cys,
Xaa2 is a residue of an amino acid selected from the group consisting of Thr, Arg, Arg(Me), Asp, Glu, Gln, Orn(DOTA), Lys, Lys(Me), Lys(DOTA), Cit, Ala, Aib, Har, Asn, Glu(AGLU), Orn(mPEG12), KMe3, Orn, Om(Me), Orn(Tris-Nta(Tris)), Ser, Thp, Apc, Apc(DOTA), Gly, Pro, Tyr and Leu,
Xaa3 is a residue of an amino acid selected from the group consisting of Orn(DOTA), Lys(DOTA), Met, Arg, Arg(Me), Dap, Dab, Leu, Tyr, Gln, Glu, Lys(Ac), Cit, Ala, Nle, Asp, Har, Asn, Thr, Apc(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Orn(Me), Ser and Gly,
Xaa4 is a residue of an amino acid selected from the group consisting of Pcnf, Dopa, Tyr, Aph, Mpa, Nif, Ocf and My,
Xaa5 is a residue of an amino acid selected from the group consisting of Phe, Thi, Pcf, Ocf, Egm, Mcf, 1Ni, Pnf and Mpa,
Xaa6 is a residue of an amino acid selected from the group consisting of 4Cfp, Pro and Hyp,
Xaa7 is a residue of an amino acid selected from the group consisting of of Hyp, Pro, 4Cfp, Tap, Ala, Aib, 4Tfp, Chy, 4Ap, ala, Arg, Tap(DOTA), Lys(DOTA), Ser, ser, Leu, tyr and nma,
Xaa8 is a residue of an amino acid selected from the group consisting of Ile, Chg, Cha, Cpg, Phg, Pcf, Cbg and Npg,
Xaa9 is a residue of an amino acid selected from the group consisting of Nva, Leu, Cpra, Abu, Ser(Me) and Cbg, and
Xaa10 is a residue of an amino acid selected from the group consisting of Cys-NH₂ and Cysol-OH;
or c)
the blocking group Abl is selected from the group consisting of Butoc, Hex, Pen, iHex and nBuCAyl,
Xaa1 is a residue of Cys,
Xaa2 is a residue of an amino acid selected from the group consisting of Thr and Cit,
Xaa3 is a residue of an amino acid selected from the group consisting of Orn(DOTA), Lys(DOTA), Gln, Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw),
Xaa4 is a residue of an amino acid selected from the group consisting of Pcnf, Dopa, Tyr, Aph, Mpa, Nif, Ocf and My,
Xaa5 is a residue of an amino acid selected from the group consisting of Phe, Thi, Pcf, Ocf and Egm,
Xaa6 is a residue of an amino acid selected from the group consisting of 4Cfp, Pro and Hyp.
Xaa7 is a residue of an amino acid selected from the group consisting of Hyp, Pro, Aib, ala, nma, 4Tfp, Lys(DOTA) and Tap,
Xaa8 is a residue of an amino acid selected from the group consisting of Ile, Phg, Chg and Cha,
Xaa9 is a residue of an amino acid selected from the group consisting of Nva, Leu and Cpra, and
Xaa10 is a residue of Cys-NH₂.

22. The compound or pharmaceutically acceptable salt or hydrate or pharmaceutically acceptable solvate of any one of claims 1 to 19,
wherein Xaa0 is a residue of an amino acid selected from the group consisting of Met, Leu, leu, Aml, Egz, Nva, nva, Nle, nle, Nmb, Phg and Cha, preferably Xaa0 is selected from the group consisting of Phg, Leu, Nva, Aml, Egz, Nle and Cha,
wherein Xaa1 is a residue of an amino acid selected from the group consisting of Cys and Pen, preferably Xaa1 is a residue of Cys;
wherein Xaa2 is a residue of an amino acid selected from the group consisting of Thr, Arg, Arg(Me), Asp, Glu, Gln, Lys, Lys(Me), Lys(Z), Cit, Ala, Aib, Har, Asn, Glu(Z), Orn(mPEG12), KMe3, Orn, Orn(Me), Orn(Z), Ser, Thp, Apc, Apc(Z), Gly, Pro, Tyr and Leu, wherein Z is a Z group; preferably Xaa2 is a residue of an amino acid selected from the group consisting of Thr, Arg, Arg(Me), Asp, Glu, Gln, Orn(DOTA), Lys, Lys(Me), Lys(DOTA), Cit, Ala, Aib, Har, Asn, Glu(AGLU), Orn(mPEG12), KMe3, Orn, Orn(Me), Orn(Tris-Nta(Tris)), Ser, Thp, Apc, Apc(DOTA), Gly, Pro, Tyr and Leu; more preefably Xaa2 is a residue of an amino acid selected from the group consisting of Thr and Cit, most preferably Xaa2 is a residue of Thr;
Xaa3 is a residue of an amino acid selected from the group consisting of Orn(Z), Lys(Z), Met, Arg, Arg(Me), Dap, Dab, Leu, Tyr, Gln, Glu, Glu(Z), Lys(Ac), Cit, Ala, Nle, Asp, Har, Asn, Thr, Apc(Z), Dab(Z), Dap(Z), Lys(AF488-Ttds), Lys, Lys(Me), KMe3, Orn, Orn(Me), Ser and Gly, wherein Z is a Z group; preferably Xaa3 is a residue of an amino acid selected from the group consisting of Orn(DOTA), Lys(DOTA), Met, Arg, Arg(Me), Dap, Dab, Leu, Tyr, Gln, Glu, Lys(Ac), Cit, Ala, Nle, Asp, Har, Asn, Thr, Apc(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Orn(Me), Ser and Gly; more preferably Xaa3 is a residue of an amino acid selected from the group consisting of Orn(DOTA), Lys(DOTA), Gln, Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw),most preferably Xaa3 is a residue of an amino acid selected from the group consisting of Orn(DOTA) and Lys(DOTA);
wherein Xaa4 is a residue of an amino acid selected from the group consisting of Pcnf, Dopa, Tyr, Phe, Aph, His, Mpa, My, Mcy, Guf, Ppa, Nif, Cha, Trp, Ay3, 2Ni and Ocf, preferably Xaa4 is a residue of an amino acid selected from the group consisting of Pcnf, Dopa, Tyr, Aph, Mpa, Nif, Ocf and My , more preferably Xaa4 is a residue of an amino acid selected from the group consisting of Pcnf and Dopa;
wherein Xaa5 is a residue of an amino acid selected from the group consisting of Phe, Thi, Pcf, Ocf, Egm, Mcf, 1Ni, Pnf, Mpa, Bta and Trp, preferably Xaa5 is a residue of an amino acid selected from the group consisting of Phe, Thi, Pcf, Ocf, Egm, Mcf, 1Ni, Pnf and Mpa; more preferably Xaa5 is a residue of an amino acid selected from the group consisting of Phe, Thi, Pcf, Ocf and Egm, most preferably Xaa5 is a residue of Phe;
wherein Xaa6 is a residue of an amino acid selected from the group consisting of 4Cfp, Pro, Hyp, Eaz, Oic, Nmb, Leu, Cha, Chy, 4Tfp, Pip, Oxa, Aze and Phe; preferably Xaa6 is a residue of an amino acid selected from the group consisting of 4Cfp, Pro and Hyp more preferably Xaa6 is a residue of 4Cfp;
wherein Xaa7 is a residue of an amino acid selected from the group consisting of Hyp, Pro, 4Cfp, Tap, Ala, Aib, Asp, Ser, Tyr, ser, dap, tyr, Leu, 4Tfp, Chy, 4Ap, ala, Arg, Apc(Z), Tap(Z), Gly, nma, Aze, Oic, Oxa, Lys(Z) and Nmg, wherein Z is a Z group, preferably Z is DOTA; preferably Xaa7 is a residue of an amino acid selected from the group consisting of Hyp, Pro, 4Cfp, Tap, Ala, Aib, 4Tfp, Chy, 4Ap, ala, Arg, Tap(Z), Lys(Z), Ser, ser, Leu, tyr and nma, wherein Z is a Z group, preferably Z is DOTA; more preferably Xaa7 is a residue of an amino acid selected from the group consisting of Hyp, Pro, Aib, ala, nma, 4Tfp, Lys(DOTA) and Tap, most preferably Xaa7 is a residue of Hyp;
wherein Xaa8 is a residue of an amino acid selected from the group consisting of Ile, Chg, Cha, Cpg, Cbg, Npg, Phe, Pcf, Egz, Hfe, Phg, Leu, Nle and Nva; preferably Xaa8 is a residue of an amino acid selected from the group consisting of Ile, Chg, Cha, Cpg, Phg, Pcf, Cbg and Npg; more preferably Xaa8 is a residue of an amino acid selected from the group consisting of Ile, Chg, Phg and Cha, most preferably Xaa8 is a residue of Ile;
wherein Xaa9 is a residue of an amino acid selected from the group consisting of Nva, Leu, Cpra, Abu, Ser(Me), Cbg, Npg, Ala, Nle, Ile and Val; preferably Xaa9 is a residue of an amino acid selected from the group consisting of Nva, Leu, Cpra, Abu, Ser(Me) and Cbg; more preferably Xaa9 is a residue of an amino acid selected from the group consisting of Nva, Leu and Cpra, most preferably Xaa9 is a residue of Nva;
and/or
Xaa10 is a residue of an amino acid selected from the group consisting of Cys-NH₂, Cysol-OH, AET, Hcy-NH₂, Pen-NH₂ and Cys-O2Oc-Lys(Z)-NH2, wherein Z is a Z group, preferably Z is DOTA; more preferably Xaa10 is a residue of an amino acid selected from the group consisting of Cys-NH₂ and Cysol-OH; most preferably Xaa10 is a residue of Cys-NH₂;
or any combination of any one of Xaa1, Xaa2, Xaa3, Xaa4, Xaa5, Xaa6, Xaa7, Xaa8, Xaa9, XaalO and Xaa0 as defined above.

23. The compound or pharmaceutically acceptable salt or hydrate or pharmaceutically acceptable solvate of any one of claims 1 to 19 and 22, wherein
a)
Xaa0 is a residue of an amino acid selected from the group consisting of Met, Leu, leu, Aml, Egz, Nva, nva, Nle, nle, Nmb, Phg and Cha,
Xaa1 is a residue of an amino acid selected from the group consisting of Cys and Pen,
Xaa2 is a residue of an amino acid selected from the group consisting of is a residue of an amino acid selected from the group consisting of Thr, Arg, Arg(Me), Asp, Glu, Gln, Lys, Lys(Me), Lys(Z), Cit, Ala, Aib, Har, Asn, Glu(Z), Orn(mPEG12), KMe3, Orn, Orn(Me), Orn(Z), Ser, Apc, Apc(Z), Gly, Pro, Tyr and Leu,
Xaa3 is a residue of an amino acid selected from the group consisting of Orn(Z), Lys(Z), Met, Arg, Arg(Me), Dap, Dab, Leu, Tyr, Gln, Glu, Glu(Z), Lys(Ac), Cit, Ala, Nle, Asp, Har, Asn, Thr, Apc(Z), Dab(Z), Dap(Z), Lys(AF488-Ttds), Lys, Lys(Me), KMe3, Orn, Orn(Me), Ser and Gly,
Xaa4 is a residue of an amino acid selected from the group consisting of Pcnf, Dopa, Tyr, Phe, Aph, His, Mpa, My, Mcy, Guf, Ppa, Nif, Cha, Trp, Ay3, 2Ni and Ocf,
Xaa5 is a residue of an amino acid selected from the group consisting of Phe, Thi, Pcf, Ocf, Egm, Mcf, 1Ni, Pnf, Mpa, Bta and Trp,
Xaa6 is a residue of an amino acid selected from the group consisting of 4Cfp, Pro, Hyp, Eaz, Oic, Leu, Cha, Chy, 4Tfp, Pip, Oxa, Aze and Phe,
Xaa7 is a residue of an amino acid selected from the group consisting of Hyp, Pro, 4Cfp, Tap, Ala, Aib, Asp, Ser, Tyr, ser, dap, tyr, Leu, 4Tfp, Chy, 4Ap, ala, Arg, arg, Apc(Z), Tap(Z), Gly, nma, Aze, Oic, Oxa, Nmr, Lys(Z) and Nmg,
Xaa8 is a residue of an amino acid selected from the group consisting of Ile, Chg, Cha, Cpg, Cbg, Npg, Phe, Pcf, Egz, Hfe, Phg, Leu, Nle and Nva,
Xaa9 is a residue of an amino acid selected from the group consisting of Nva, Leu, Cpra, Abu, Ser(Me), Cbg, Npg, Ala, Nle, Ile and Val, and
Xaa10 is a residue of an amino acid selected from the group consisting of Cys-NH₂, Cysol-OH, AET, Hcy-NH₂, Pen-NH₂ and Cys-O2Oc-Lys(Z)-NH₂,
wherein Z is a Z group, and
wherein an N-terminal modification group Nterm is covalently attached to the α-nitrogen atom of Xaa0, wherein Nterm is preferably selected from the group consisting of a - Ac, a methyl group and a Z group;
or b)
Xaa0 is a residue of an amino acid selected from the group consisting of Phg, Leu, Nva, Aml, Egz, Nle and Cha,
Xaa1 is a residue of Cys,
Xaa2 is a residue of an amino acid selected from the group consisting of Thr, Arg, Arg(Me), Asp, Glu, Gln, Orn(DOTA), Lys, Lys(Me), Lys(DOTA), Cit, Ala, Aib, Har, Asn, Glu(AGLU), Orn(mPEG12), KMe3, Orn, Orn(Me), Orn(Tris-Nta(Tris)), Ser, Apc, Apc(DOTA), Gly, Pro, Tyr and Leu,
Xaa3 is a residue of an amino acid selected from the group consisting of Orn(DOTA), Lys(DOTA), Met, Arg, Arg(Me), Dap, Dab, Leu, Tyr, Gln, Glu, Lys(Ac), Cit, Ala, Nle, Asp, Har, Asn, Thr, Apc(DOTA), Dab(DOTA), Dap(DOTA), Lys(DOTA-PPAc), Lys(DOTA-asp), Lys(DOTA-PEG12), Lys(DOTA-Kzw), Lys(AF488-Ttds), Lys(NOTA), Lys(NODAGA), Lys, Lys(Me), KMe3, Orn, Orn(Me), Ser and Gly,
Xaa4 is a residue of an amino acid selected from the group consisting of Pcnf, Dopa, Tyr, Aph, Mpa, Nif, Ocf and My,
Xaa5 is a residue of an amino acid selected from the group consisting of Phe, Thi, Pcf, Ocf, Egm, Mcf, 1Ni, Pnf and Mpa,
Xaa6 is a residue of an amino acid selected from the group consisting of 4Cfp, Pro and and Hyp,
Xaa7 is a residue of an amino acid selected from the group consisting of of Hyp, Pro, 4Cfp, Tap, Ala, Aib, 4Tfp, Chy, 4Ap, ala, Arg, Tap(DOTA), Lys(DOTA), Ser, ser, Leu, tyr and nma,
Xaa8 is a residue of an amino acid selected from the group consisting of Ile, Chg, Cha, Cpg, Phg, Pcf, Cbg and Npg,
Xaa9 is a residue of an amino acid selected from the group consisting of Nva, Leu, Cpra, Abu, Ser(Me) and Cbg, and
Xaa10 is a residue of an amino acid selected from the group consisting of Cys-NH₂ and Cysol-OH,
and wherein an N-terminal modification group Nterm is covalently attached to the α-nitrogen atom of Xaa0, wherein Nterm is preferably selected from the group consisting of a -Ac, a methyl group and a Z group;
or c)
Xaa0 is a residue of an amino acid selected from the group consisting of Phg, Leu, Nva, Aml, Egz, Nle and Cha,
Xaa1 is a residue of Cys,
Xaa2 is a residue of an amino acid selected from the group consisting of Thr and Cit,
Xaa3 is a residue of an amino acid selected from the group consisting of Orn(DOTA), Lys(DOTA), Gln, Dab(DOTA), Dap(DOTA), Lys(NOTA), Lys(NODAGA), Lys(DOTA-PPAc), Lys(DOTA-asp), Lys(DOTA-PEG12) and Lys(DOTA-Kzw),
Xaa4 is a residue of an amino acid selected from the group consisting of Pcnf, Dopa, Tyr, Aph, Mpa, Nif, Ocf and My,
Xaa5 is a residue of an amino acid selected from the group consisting of Phe, Thi, Pcf, Ocf and Egm,
Xaa6 is a residue of an amino acid selected from the group consisting of 4Cfp, Pro and Hyp.
Xaa7 is a residue of an amino acid selected from the group consisting of Hyp, Pro, Aib, ala, nma, 4Tfp, Lys(DOTA) and Tap,
Xaa8 is a residue of an amino acid selected from the group consisting of Ile, Phg, Chg and Cha,
Xaa9 is a residue of an amino acid selected from the group consisting of Nva, Leu and Cpra, and
Xaa10 is a residue of Cys-NH₂,
and wherein an N-terminal modification group Nterm is covalently attached to the α-nitrogen atom of Xaa0, wherein Nterm is preferably selected from the group consisting of a -Ac, a methyl group and a Z group.

24. The compound or pharmaceutically acceptable salt or hydrate or pharmaceutically acceptable solvate of any one of claims 1 to 23, wherein each Z group is each and independently selected from the group consisting of a chelator optionally comprising a linker moiety L and a bio-distribution modifier optionally comprising a linker.

25. The compound or pharmaceutically acceptable salt or hydrate or pharmaceutically acceptable solvate of any one of claims 1 to 24, wherein each Z group is each and independently a chelator optionally comprising a linker moiety L; preferably the chelator is selected from the group consisting of DOTA, DOTAGA, DOTAM, Crown, DOTP, NOTA, NODAGA, NODA-MPAA, HBED, TETA, CB-TE2A, DTPA, CHX-A"-DTPA, DFO, Macropa, HOPO ligand platform, TRAP, THP, AAZTA, DATA, NOPO, PCTA, PSC, sarcophagine, FSC, DEPA, DE4TA, NETA, NE3TA, H₄octapa, H₄CHXoctapa, H₄pypa, H₄py₄pa, HYNIC, NxS4-x (N4, N2S2, N3S) and ^{99m}Tc(CO)₃-chelators;
preferably the chelator is selected from the group consisting of DOTA, DOTAGA, DOTAM, Crown, NOTA, NODAGA, NODA-MPAA, CB-TE2A, CHX-A"-DTPA, DFO, Macropa, THP, AAZTA, NOPO, PCTA, PSC, sarcophagine, NETA, H₄CHXoctapa, H₄pypa and N4,more preferably the chelator is selected from the group consisting of DOTA, DOTAM, Macropa, NOTA, PSC and NODAGA, and most preferably the chelator is DOTA.

26. The compound or pharmaceutically acceptable salt or hydrate or pharmaceutically acceptable solvate of any one of claims 1 to 25, wherein the compound is selected from the group consisting of
compound Butoc-[Cys-Thr-Orn(DOTA)-Pcnf-Phe-4Cfp-Hyp-Ile-Nva-Cys]-NH₂ (UPAR-276) of the following formula 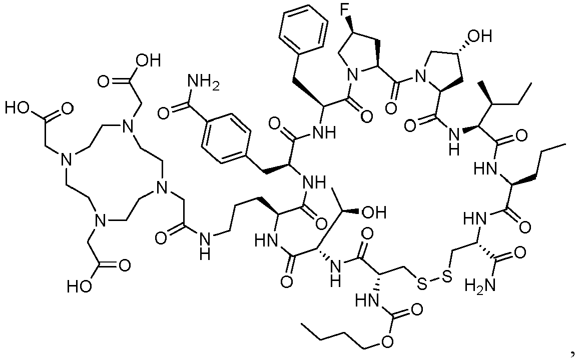
compound Butoc-[Cys-Thr-Lys(DOTA)-Dopa-Phe-4Cfp-Hyp-Ile-Nva-Cys]-NH₂ (UPAR-249) of the following formula 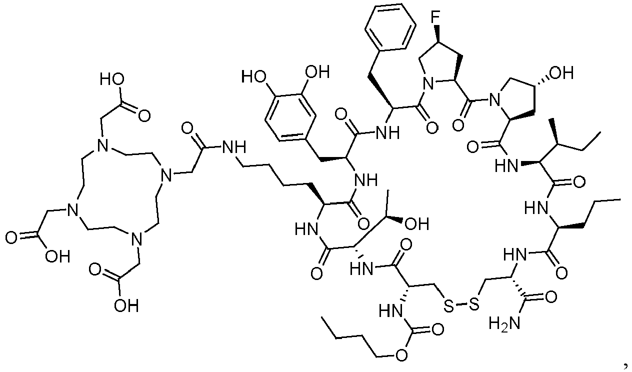
compound Butoc-[Cys-Thr-Lys(DOTA)-Tyr-Phe-Hyp-Hyp-Ile-Nva-Cys]-NH₂ (UPAR-128) of the following formula 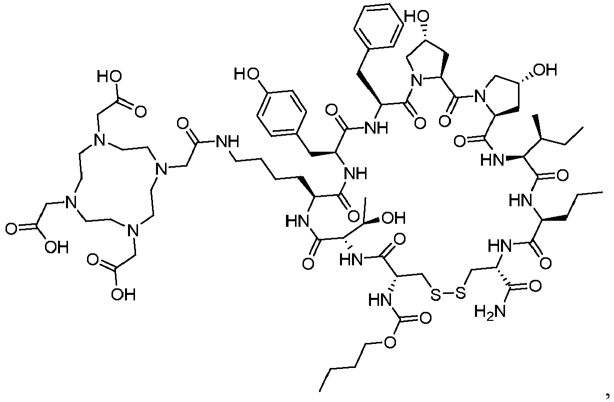
compound Butoc-[Cys-Thr-Lys(DOTA)-Tyr-Thi-Pro-Hyp-Ile-Nva-Cys]-NH₂ (UPAR-134) of the following formula 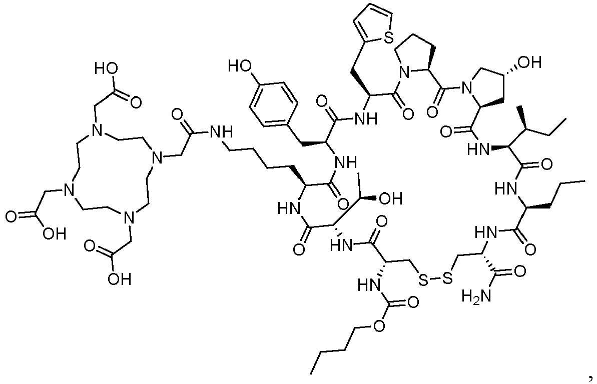
compound Pent-[Cys-Thr-Lys(DOTA)-Tyr-Phe-Hyp-Hyp-Ile-Nva-Cys]-NH₂ (UPAR-135) of the following formula 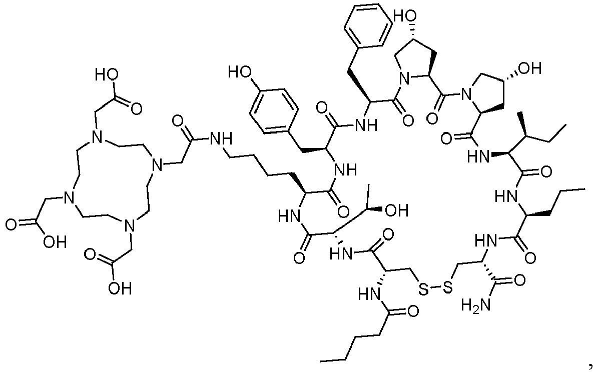
compound Pent-[Cys-Cit-Lys(DOTA)-Mpa-Phe-Pro-Hyp-Ile-Nva-Cys]-NH₂ (UPAR-138) of the following formula 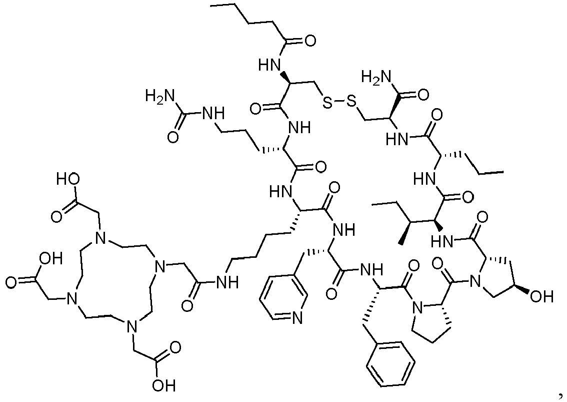
compound Hex-Lys(DOTA)-Leu-[Cys-Arg-Met-Tyr-Phe-Pro-Ala-Ile-Leu-Cys]-NH₂ (UPAR-140) of the following formula 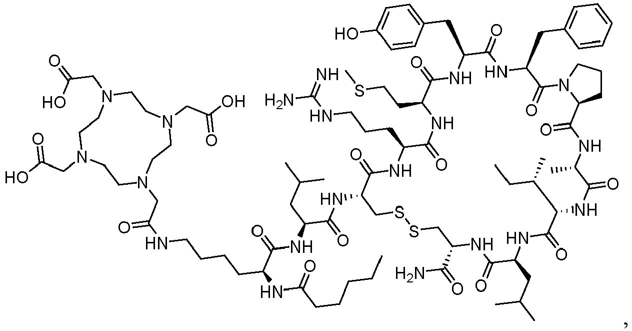
compound iHex-[Cys-Cit-Gln-Tyr-Phe-Pro-Lys(DOTA)-Ile-Leu-Cys]-NH₂ (UPAR-145) of the following formula 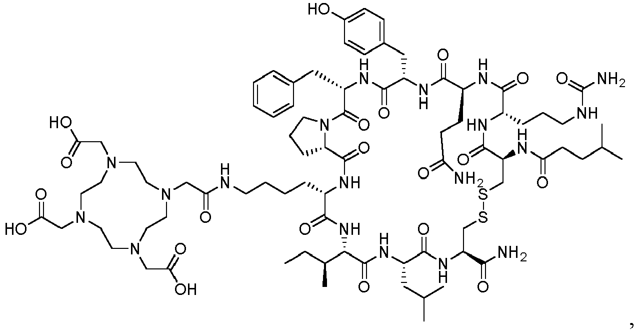
compound Pent-[Cys-Cit-Lys(DOTA)-Guf-Phe-Pro-Hyp-Ile-Nva-Cys]-NH₂ (UPAR-149) of the following formula 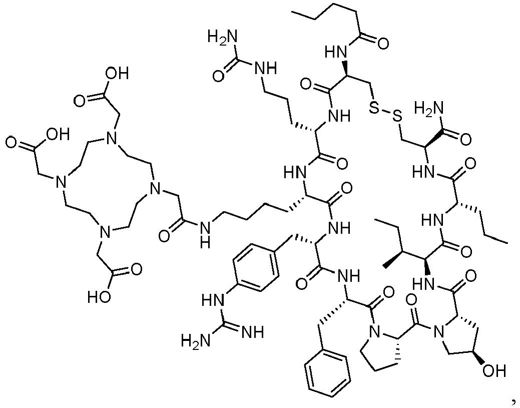
compound Pent-[Cys-KMe3-Lys(DOTA)-Tyr-Phe-Pro-Hyp-Ile-Nva-Cys]-NH₂ (UPAR-153) of the following formula 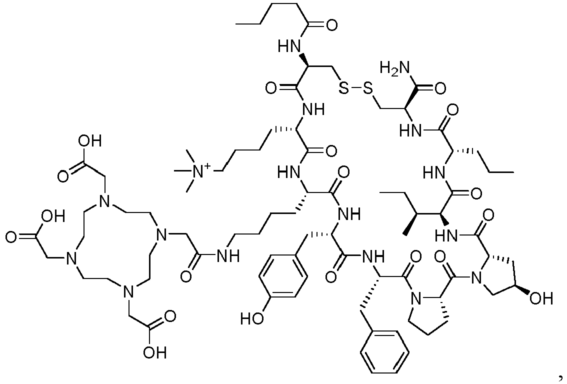
compound iHex-[Cys-Lys(DOTA)-Gln-Tyr-Phe-Pro-ala-Ile-Leu-Cys]-NH₂ (UPAR-157) of the following formula 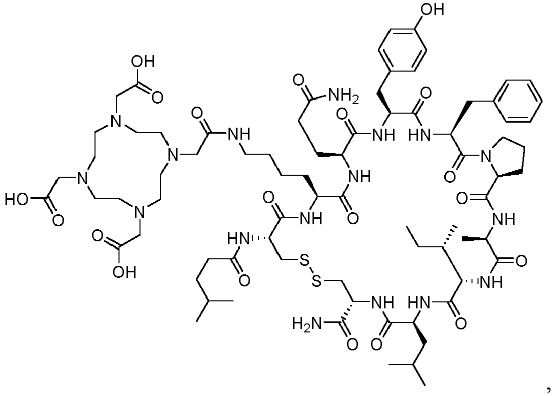
,compound Ac-Leu-[Cys-Arg-Lys(DOTA)-Tyr-Phe-Pro-ala-Ile-Leu-Cys]-NH₂ (UPAR-162) of the following formula 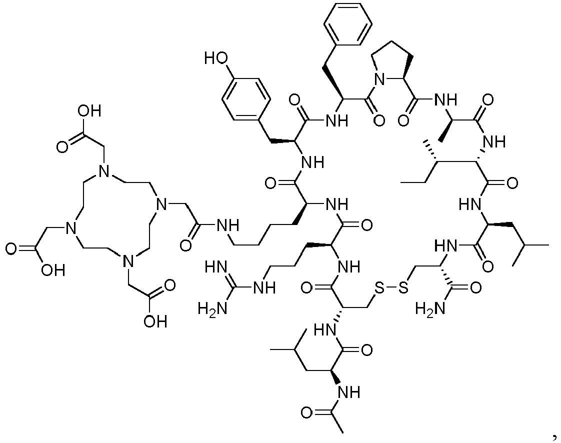
compound Butoc-[Cys-Thr-Lys(DOTA)-Mpa-Phe-4Cfp-Hyp-Ile-Nva-Cys]-NH₂ (UPAR-165) of the following formula 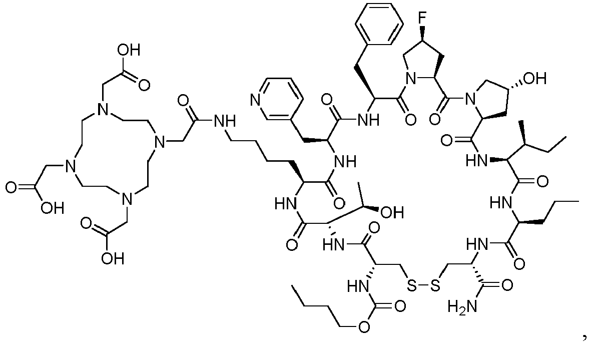
compound iHex-[Cys-Arg-Lys(DOTA)-Tyr-Phe-Pro-ala-Ile-Leu-Cys]-NH₂ (UPAR-176) of the following formula 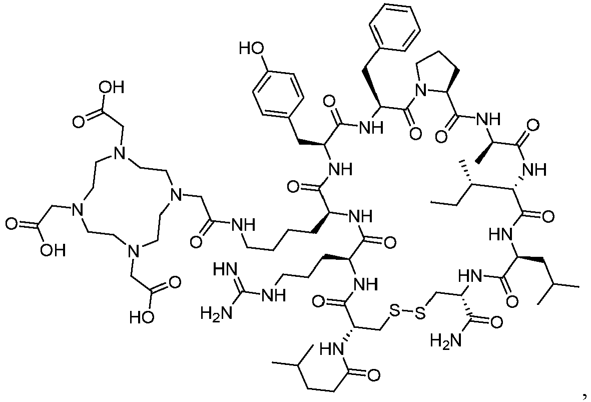
compound Butoc-[Cys-Thr-Lys(DOTA-PPAc)-Tyr-Phe-4Cfp-Hyp-Ile-Nva-Cys]-NH₂ (UPAR-181) of the following formula 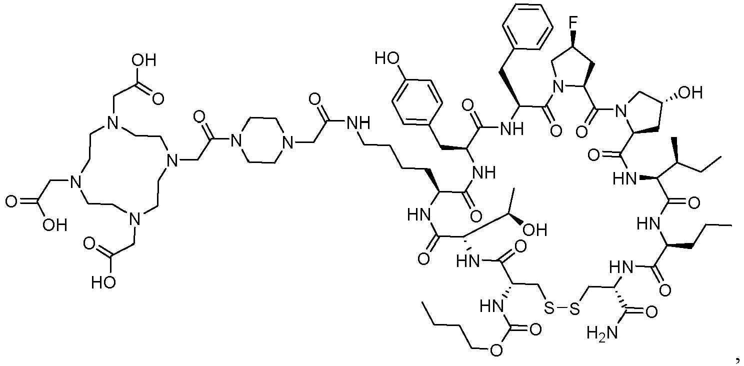
compound Ac-Asp-Leu-[Cys-Cit-Lys(DOTA)-Tyr-Phe-Pro-ala-Ile-Leu-Cys]-NH₂ (UPAR-189) of the following formula 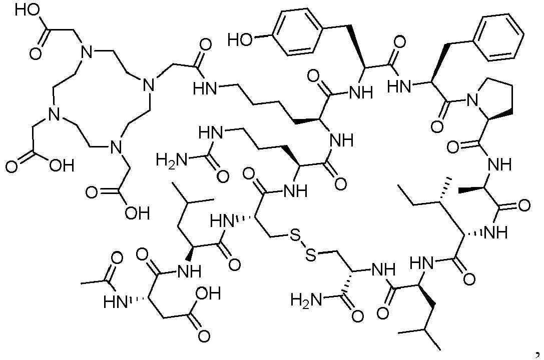
compound Butoc-[Cys-Thr-Orn(DOTA)-Tyr-Phe-4Cfp-Hyp-Ile-Nva-Cys]-NH₂ (UPAR-192) of the following formula 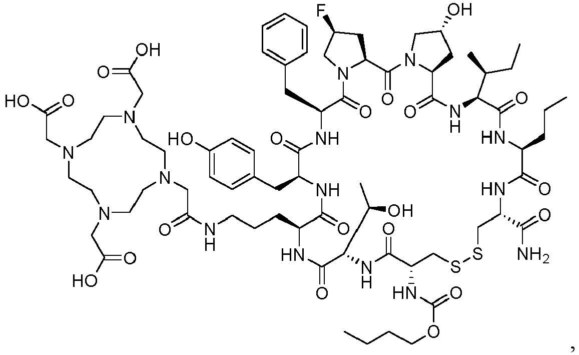
compound Butoc-[Cys-Thr-Lys(DOTA-Kzw)-Dopa-Phe-4Cfp-Hyp-Ile-Nva-Cys]-NH₂ (UPAR-194) of the following formula 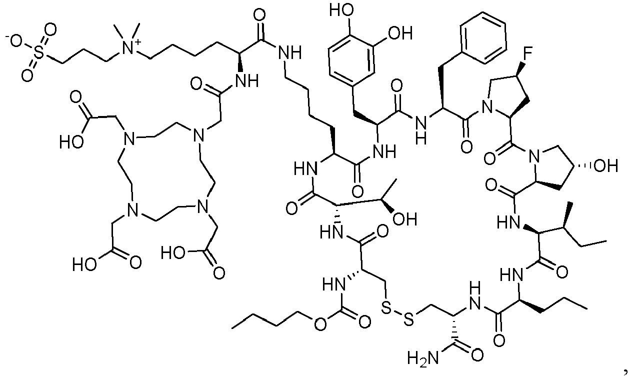
compound Butoc-[Cys-Thr-Lys(DOTA-PPAc)-Dopa-Phe-4Cfp-Hyp-Ile-Nva-Cys]-NH₂ (UPAR-199) of the following formula 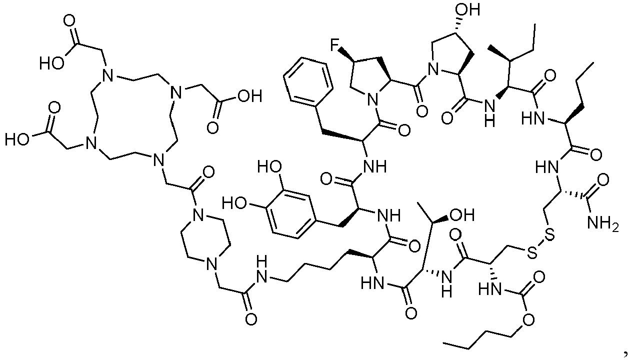
compound Pent-[Cys-Cit-Lys(DOTA-PEG12)-Tyr-Phe-Pro-Hyp-Ile-Nva-Cys]-NH₂ (UPAR-200) of the following formula 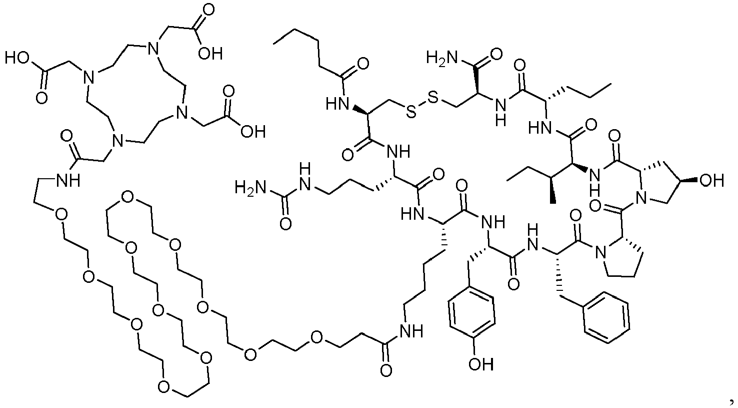
compound PrCAyl-[Cys-Cit-Lys(DOTA)-Tyr-Phe-Pro-Hyp-Ile-Nva-Cys]-NH₂ (UPAR-210) of the following formula 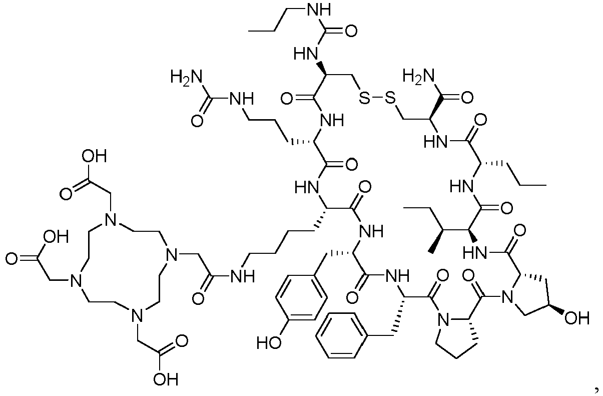
compound Pent-[Cys-Cit-Lys(DOTA)-Tyr-Phe-Pro-Chy-Ile-Nva-Cys]-NH₂ (UPAR-214) of the following formula 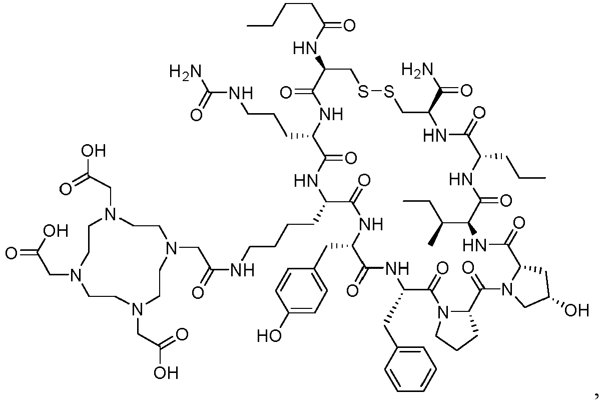
compound Pent-[Cys-Orn(Tris-Nta(Tris))-Lys(DOTA)-Tyr-Phe-Pro-Hyp-Ile-Nva-Cys]-NH₂ (UPAR-224) of the following formula 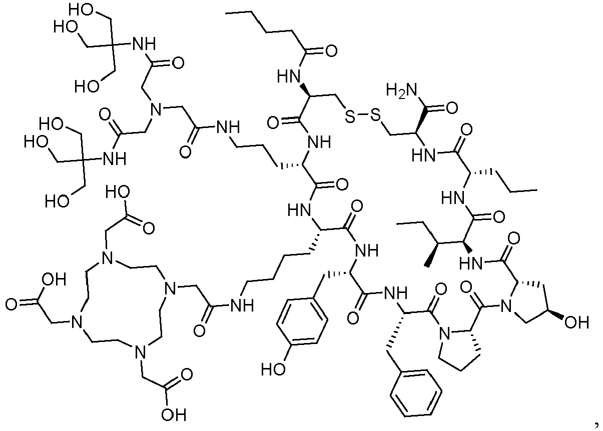
compound Butoc-[Cys-Thr-Dab(DOTA)-Tyr-Phe-4Cfp-Hyp-Ile-Nva-Cys]-NH₂ (UPAR-228) of the following formula 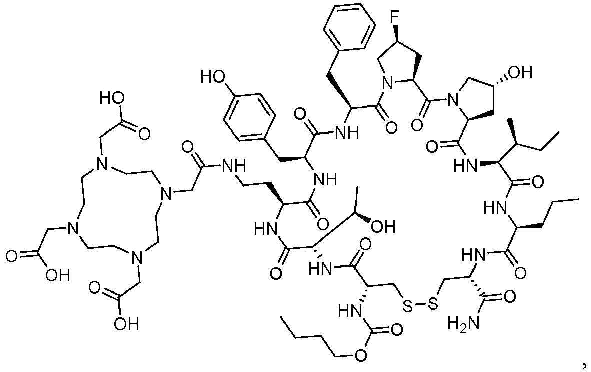
compound DOTA-O2Oc-Leu-[Cys-Cit-Gln-Tyr-Phe-Pro-Pro-Ile-Leu-Cys]-NH₂ (UPAR-229) of the following formula 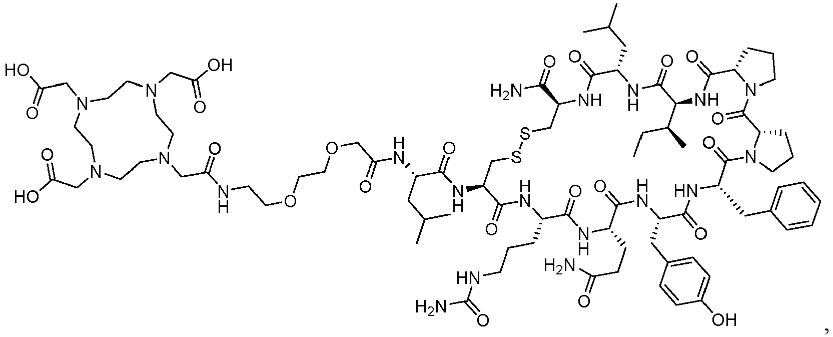
compound Pent-[Cys-Cit-Lys(DOTA)-Tyr-Pcf-Pro-Hyp-Ile-Nva-Cys]-NH₂ (UPAR-233) of the following formula 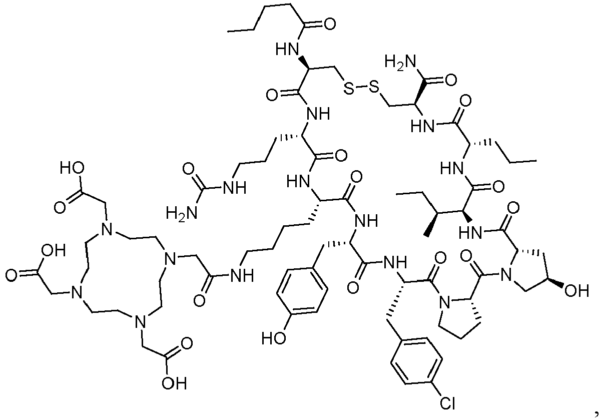
compound Butoc-[Cys-Thr-Orn(DOTA)-Ay3-Phe-4Cfp-Hyp-Ile-Nva-Cys]-NH₂ (UPAR-234) of the following formula 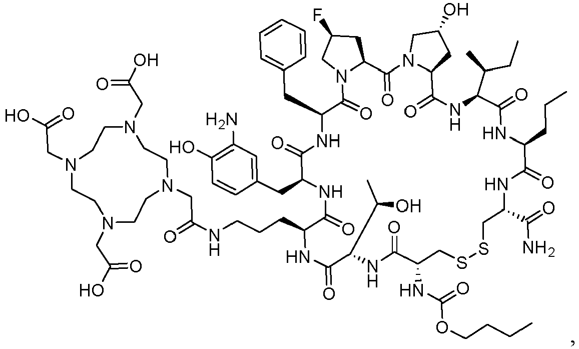
compound Hex-Asp-Leu-[Cys-Lys(DOTA)-Met-Tyr-Phe-Pro-Ala-Ile-Leu-Cys]-NH₂ (UPAR-235) of the following formula 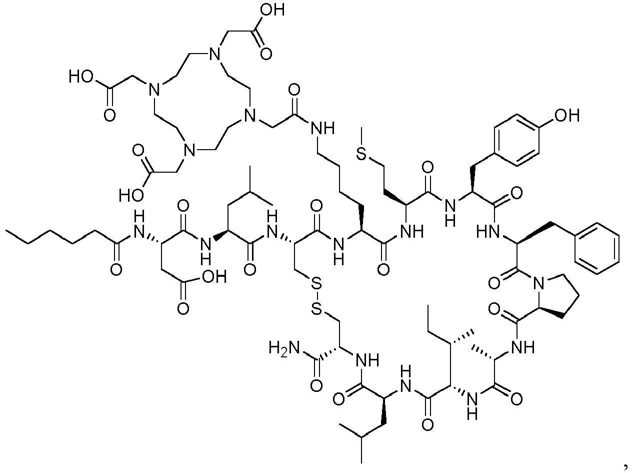
compound Butoc-[Cys-Thr-Lys(DOTA)-Tyr-Phe-4Cfp-Hyp-Ile-Nva-Cys]-NH₂ (UPAR-237) of the following formula 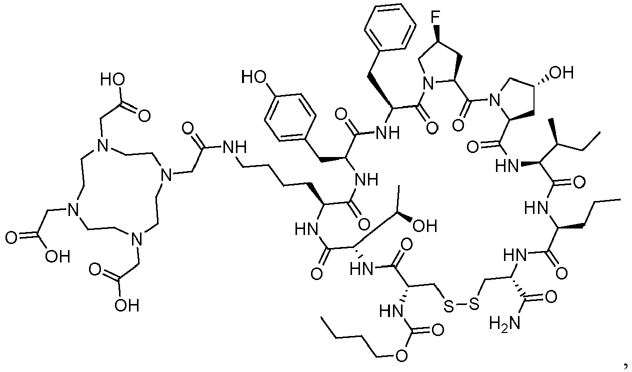
compound iHex-[Cys-Cit-Lys(DOTA)-Tyr-Phe-Pro-Hyp-Ile-Leu-Cys]-NH₂ (UPAR-240) of the following formula 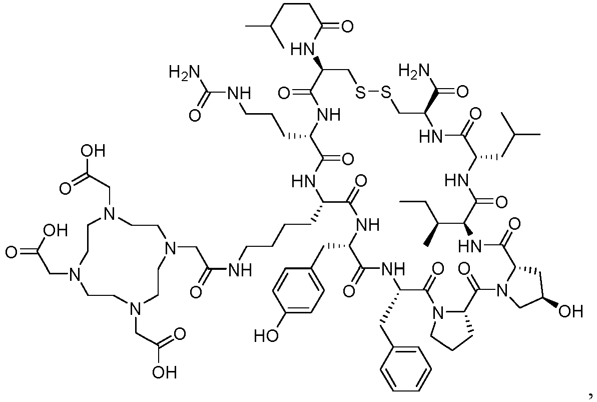
compound nBuCAyl-[Cys-Thr-Lys(DOTA)-Tyr-Phe-Pro-Hyp-Ile-Nva-Cys]-NH₂ (UPAR-242) of the following formula 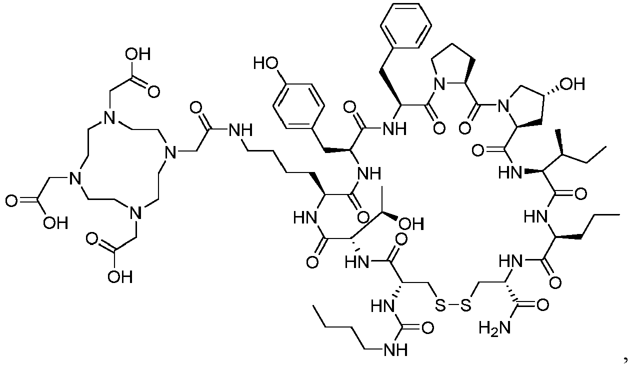
compound Pent-[Cys-Cit-Lys(DOTA)-Tyr-Phe-Pro-4Tfp-Ile-Nva-Cys]-NH₂ (UPAR-246) of the following formula 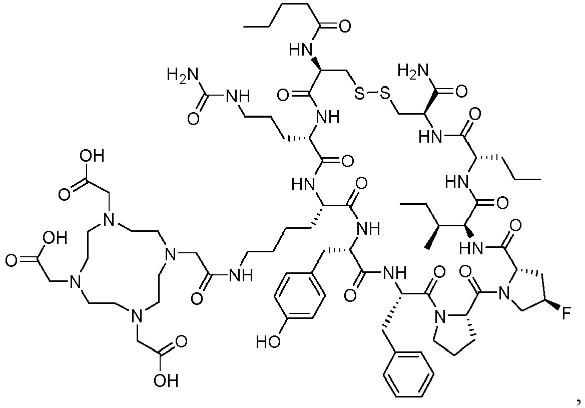
compound Butoc-[Cys-Thr-Lys(DOTA)-Tyr-Phe-Pro-Hyp-Ile-Nva-Cys]-NH₂ (UPAR-248) of the following formula 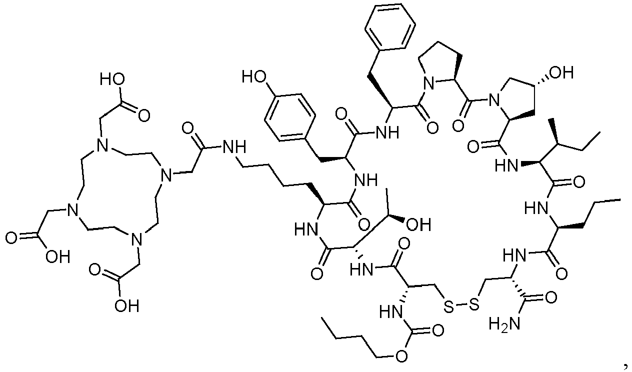
compound Butoc-[Cys-Thr-Lys(NOTA)-Dopa-Phe-4Cfp-Hyp-Ile-Nva-Cys]-NH₂ (UPAR-250) of the following formula 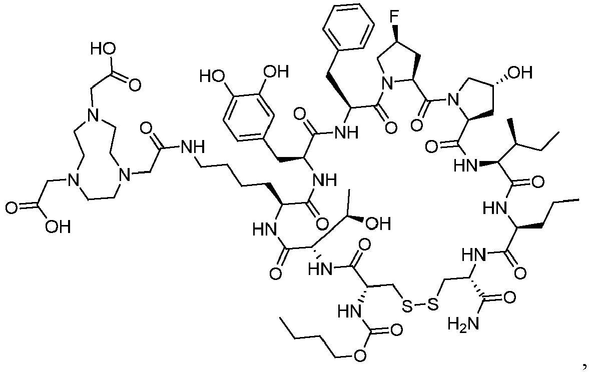
compound iHex-[Cys-Cit-Lys(DOTA)-Tyr-Phe-Pro-ala-Ile-Leu-Cys]-NH₂ (UPAR-256) of the following formula 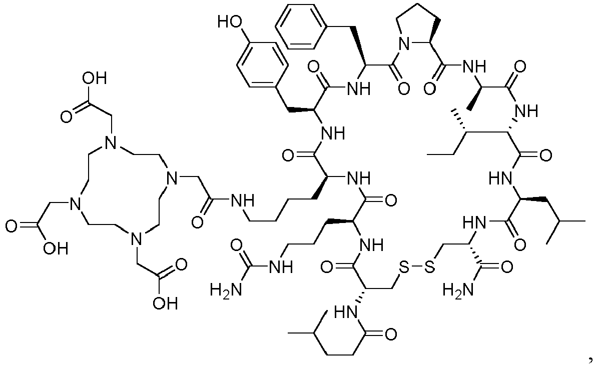
compound Pent-[Cys-Cit-Lys(DOTA)-Nif-Phe-Pro-Hyp-Ile-Nva-Cys]-NH₂ (UPAR-257) of the following formula 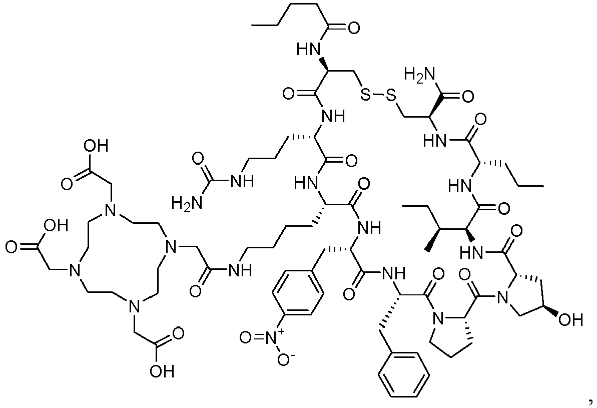
compound Pent-[Cys-Cit-Lys(DOTA)-Tyr-Phe-Pro-Hyp-Ile-Nva-Cys]-NH₂ (UPAR-261) of the following formula 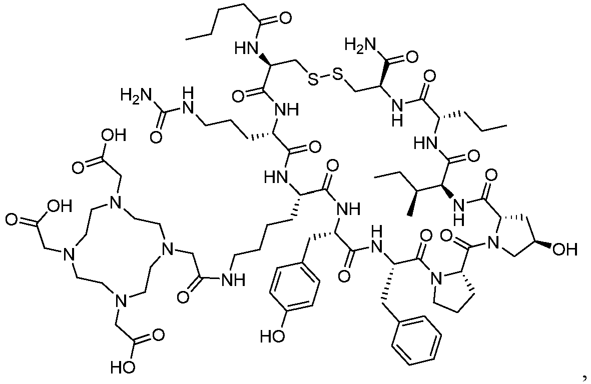
compound Butoc-[Cys-Cit-Lys(DOTA)-Tyr-Phe-Pro-Hyp-Ile-Nva-Cys]-NH₂ (UPAR-275) of the following formula 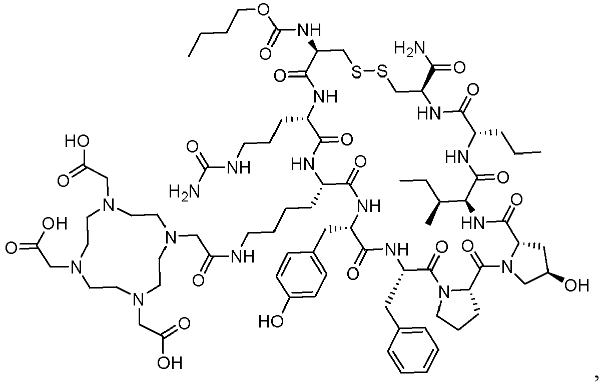
compound Pent-[Cys-Har-Lys(DOTA)-Tyr-Phe-Pro-Hyp-Ile-Nva-Cys]-NH₂ (UPAR-278) of the following formula 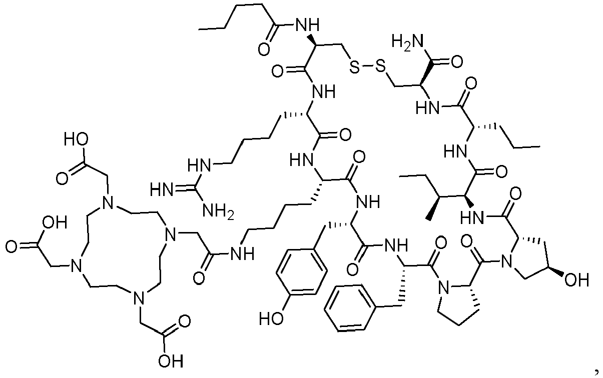
compound Butoc-[Cys-Thr-Orn(DOTA)-Dopa-Phe-4Cfp-Hyp-Ile-Nva-Cys]-NH₂ (UPAR-280) of the following formula 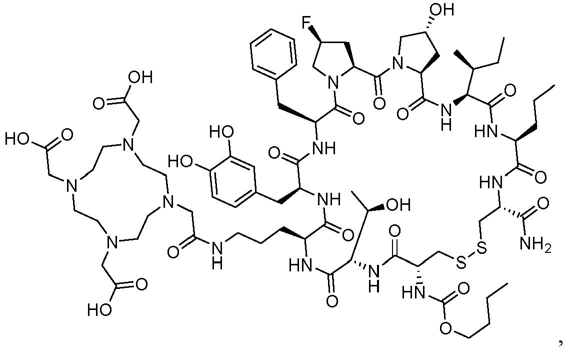
compound Pent-[Cys-Asn-Lys(DOTA)-Tyr-Phe-Pro-Hyp-Ile-Nva-Cys]-NH₂ (UPAR-283) of the following formula 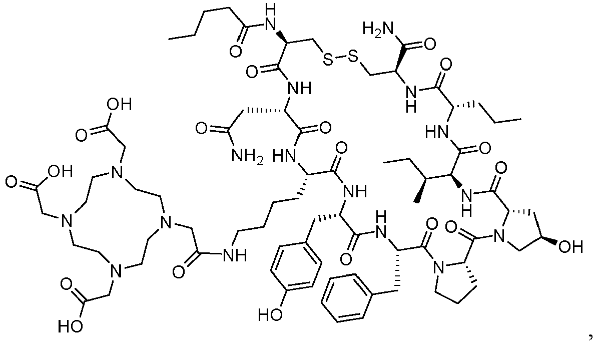
compound Butoc-[Cys-Thr-Lys(DOTA)-Dopa-Phe-Hyp-Hyp-Ile-Nva-Cys]-NH₂ (UPAR-284) of the following formula 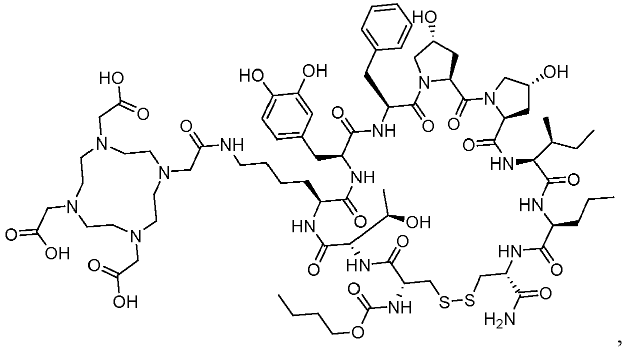
compound Butoc-[Cys-Thr-Lys(DOTA)-Tyr-Phe-Pro-4Tfp-Ile-Nva-Cys]-NH₂ (UPAR-289) of the following formula 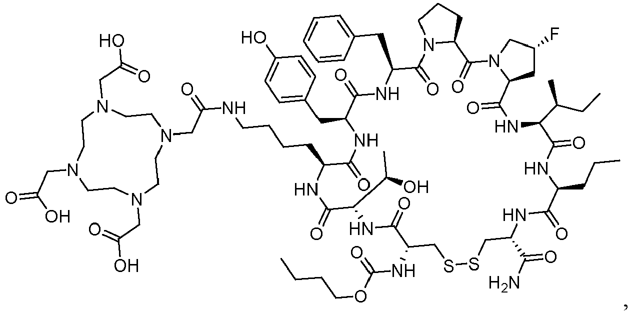
compound DOTA-Ahx-Leu-[Cys-Arg-Gln-Tyr-Phe-Pro-ala-Ile-Leu-Cys]-NH₂ (UPAR-291) of the following formula 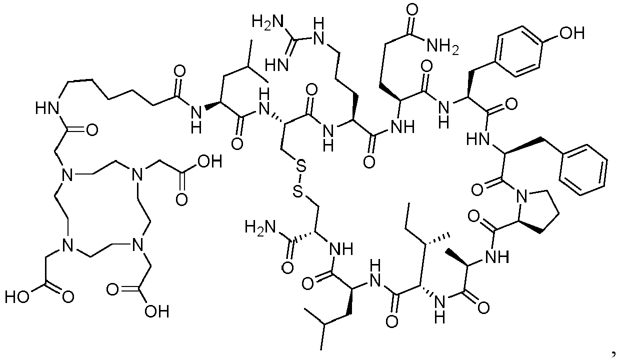
compound HO-Succinyl-Nva-[Cys-Cit-Lys(DOTA)-Tyr-Phe-Pro-Hyp-Ile-Nva-Cys]-NH₂ (UPAR-293) of the following formula 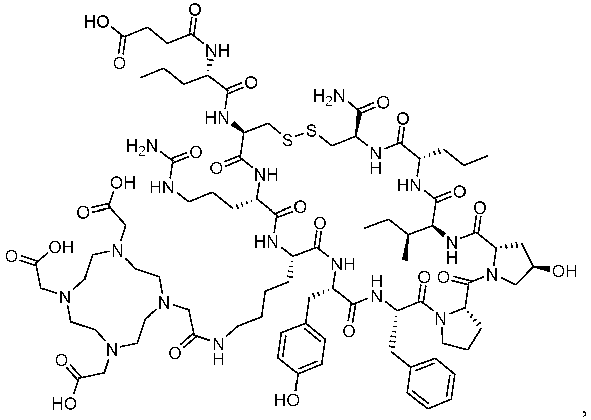
compound Succinyl-Nva-[Cys-Thr-Lys(DOTA)-Tyr-Phe-Pro-Hyp-Ile-Nva-Cys]-NH₂ (UPAR-297) of the following formula 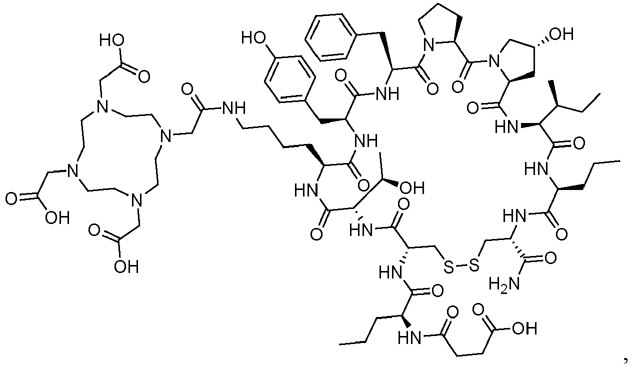
compound Pent-[Cys-Cit-Lys(DOTA)-Tyr-Phe-Pro-Aib-Ile-Nva-Cys]-NH₂ (UPAR-299) of the following formula 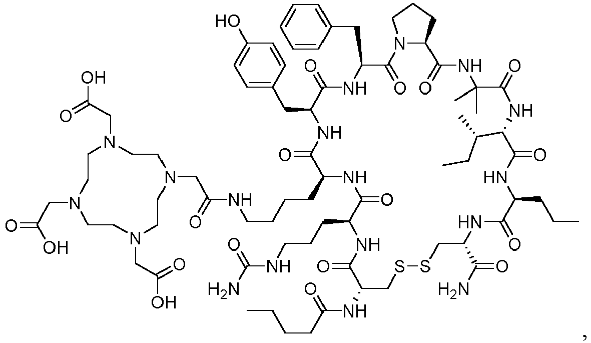
compound Pent-[Cys-Thr-Lys(DOTA)-Tyr-Phe-Pro-Hyp-Ile-Nva-Cys]-NH₂ (UPAR-301) of the following formula 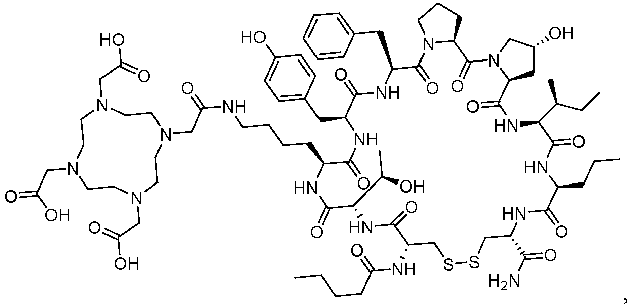
compound Butoc-[Cys-Cit-Lys(DOTA)-Tyr-Pcf-4Cfp-Hyp-Ile-Nva-Cys]-NH₂ (UPAR-304) of the following formula 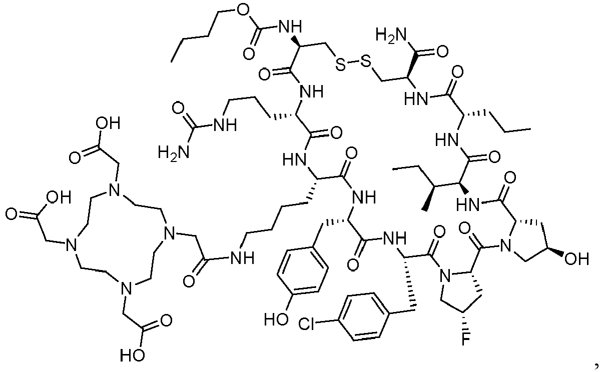
compound Pent-[Cys-Cit-Lys(DOTA)-Tyr-Egm-Pro-Hyp-Ile-Nva-Cys]-NH₂ (UPAR-315) of the following formula 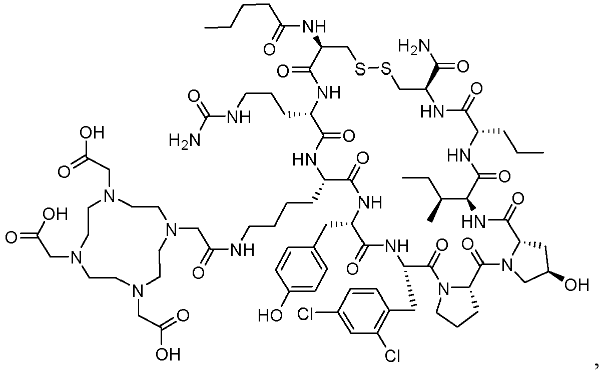
compound Pent-[Cys-Cit-Lys(DOTA)-Aph-Phe-Pro-Hyp-Ile-Nva-Cys]-NH₂ (UPAR-320) of the following formula 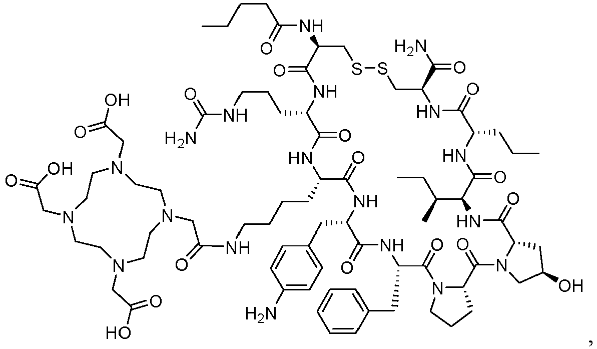
compound Pent-[Cys-Cit-Lys(DOTA)-Tyr-Phe-Pro-4Ap-Ile-Nva-Cys]-NH₂ (UPAR-325) of the following formula 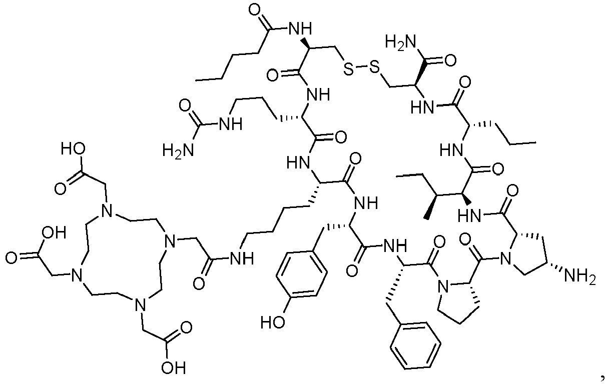
compound Pent-[Cys-Cit-Lys(DOTA)-Ppa-Phe-Pro-Hyp-Ile-Nva-Cys]-NH₂ (UPAR-336) of the following formula 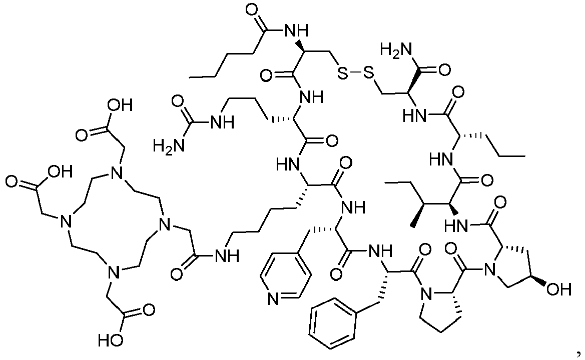
compound Pent-[Cys-Ser-Lys(DOTA)-Tyr-Phe-Pro-Hyp-Ile-Nva-Cys]-NH₂ (UPAR-342) of the following formula 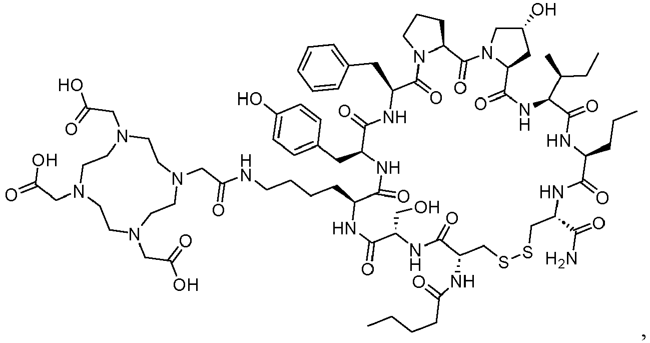
compound nBu-CAyl-[Cys-Cit-Lys(DOTA)-Tyr-Phe-Pro-Hyp-Ile-Nva-Cys]-NH₂ (UPAR-346) of the following formula 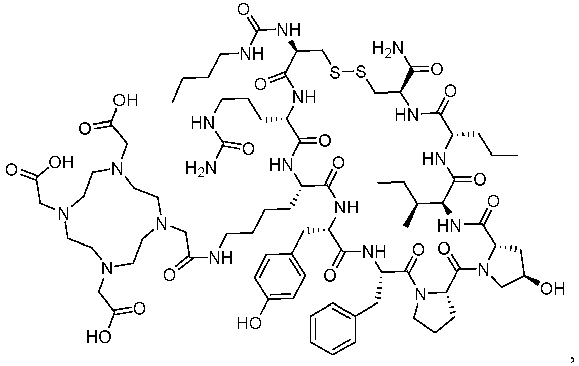
compound DOTA-Ttds-Nle-Asp-Leu-[Cys-Arg-Met-Tyr-Phe-Pro-Ala-Ile-Leu-Cys]-NH₂ (UPAR-347) of the following formula 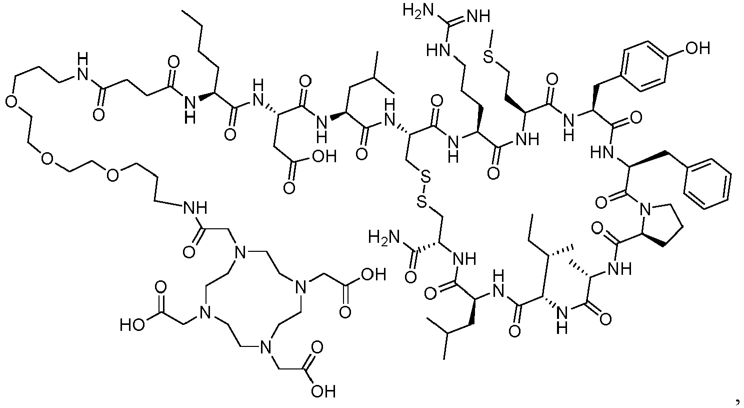
compound Hex-Asp-Leu-[Cys-Arg-Met-Tyr-Phe-Pro-Lys(DOTA)-Ile-Leu-Cys]-NH₂ (UPAR-351) of the following formula 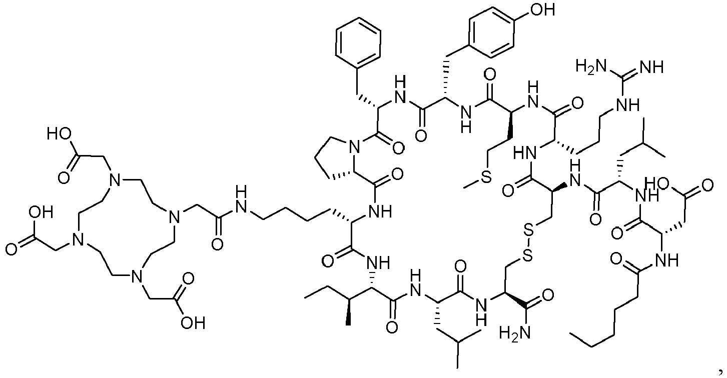
compound Pent-[Cys-Cit-Lys(DOTA)-Tyr-Thi-Pro-Hyp-Ile-Nva-Cys]-NH₂ (UPAR-360) of the following formula 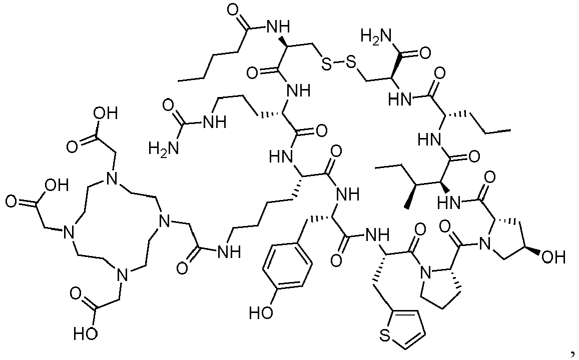
compound Pent-[Cys-Cit-Lys(DOTA)-Tyr-Phe-4Cfp-Hyp-Ile-Nva-Cys]-NH₂ (UPAR-365) of the following formula 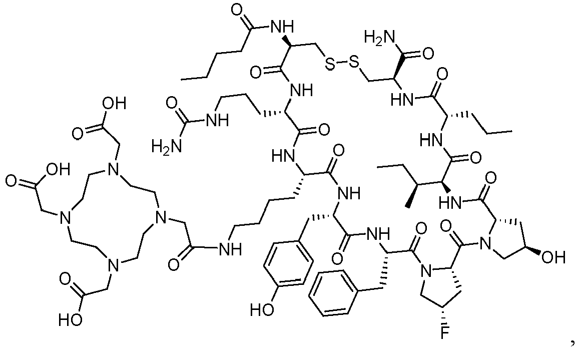
compound Pent-[Cys-Cit-Lys(DOTA)-Tyr-Phe-Pro-Tap-Ile-Nva-Cys]-NH₂ (UPAR-377) of the following formula 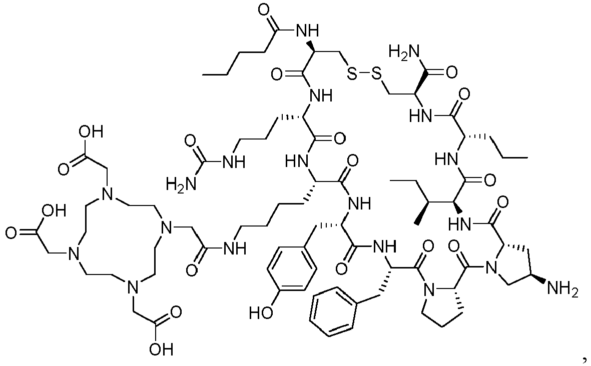
compound Butoc-[Cys-Thr-Lys(DOTA)-Tyr-Pnf-4Cfp-Hyp-Ile-Nva-Cys]-NH₂ (UPAR-378) of the following formula 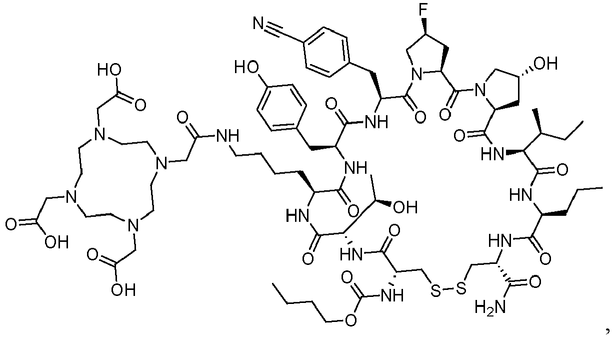
compound iHex-[Cys-Cit-Lys(DOTA)-Tyr-Phe-Pro-Pro-Ile-Leu-Cys]-NH₂ (UPAR-382) of the following formula 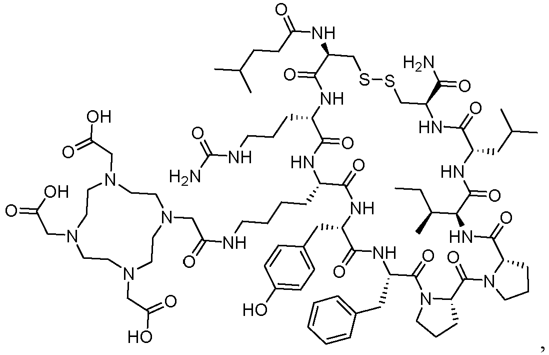
compound Pent-[Cys-Orn(mPEG12)-Lys(DOTA)-Tyr-Phe-Pro-Hyp-Ile-Nva-Cys]-NH₂ (UPAR-383) of the following formula 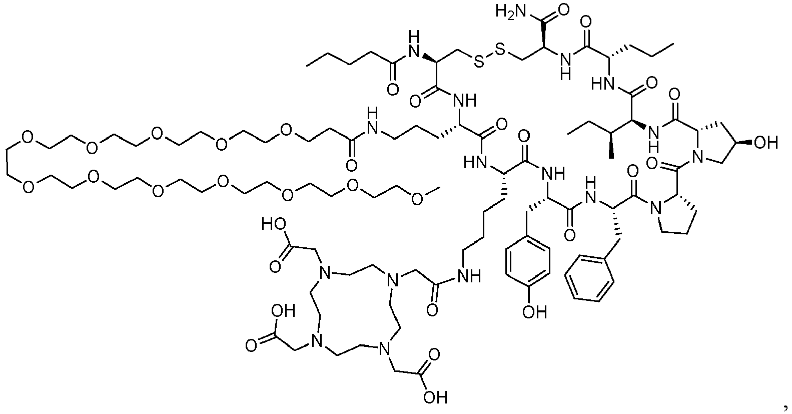
compound Pent-[Cys-Cit-Lys(DOTA)-Tyr-Phe-Pro-nma-Ile-Nva-Cys]-NH₂ (UPAR-384) of the following formula 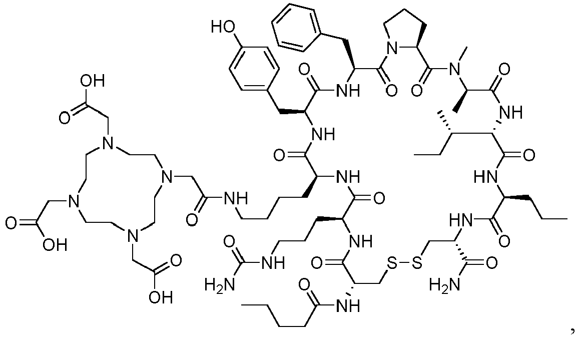
compound iHex-[Cys-Cit-Gln-Tyr-Phe-Pro-Tap(DOTA)-Ile-Leu-Cys]-NH₂ (UPAR-389) of the following formula 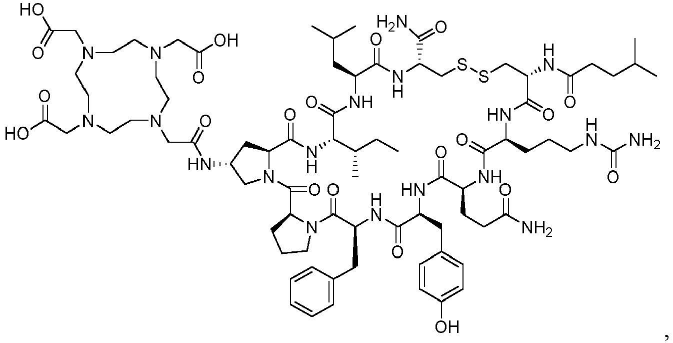
compound Pent-[Cys-Cit-Lys(DOTA)-Tyr-Phe-Pro-Pro-Ile-Leu-Cys]-NH₂ (UPAR-396) of the following formula 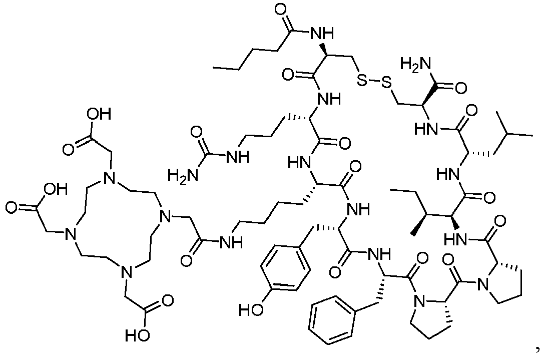
compound Pent-[Cys-Cit-Lys(DOTA)-Dopa-Phe-Pro-Hyp-Ile-Nva-Cys]-NH₂ (UPAR-399) of the following formula 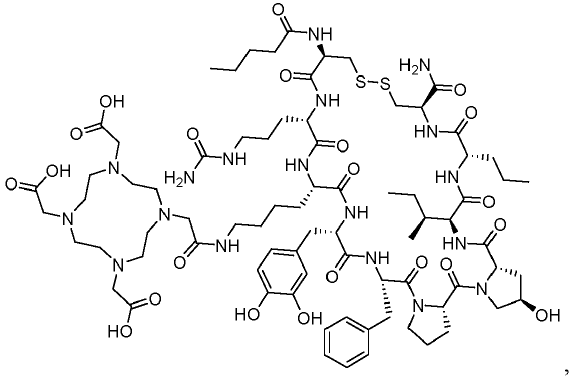
compound Pent-[Cys-Cit-Lys(DOTA)-Tyr-Phe-Hyp-Hyp-Ile-Nva-Cys]-NH₂ (UPAR-403) of the following formula 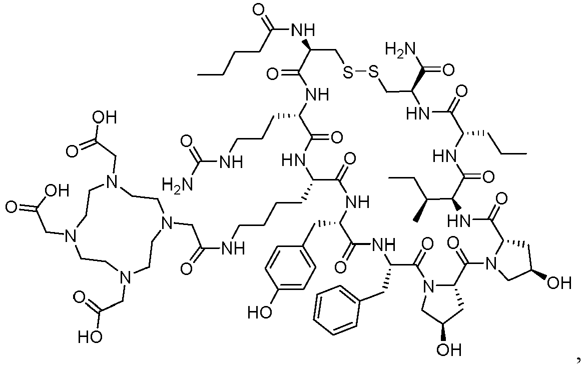
compound Butoc-[Cys-Thr-Orn(DOTA)-My-Phe-4Cfp-Hyp-Ile-Nva-Cys]-NH₂ (UPAR-415) of the following formula 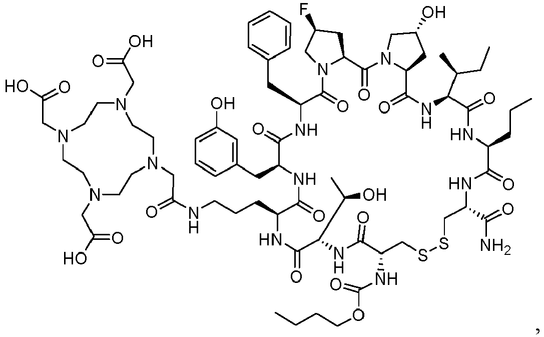
compound Butoc-[Cys-Thr-Lys(DOTA-Kzw)-Tyr-Phe-4Cfp-Hyp-Ile-Nva-Cys]-NH₂ (UPAR-422) of the following formula 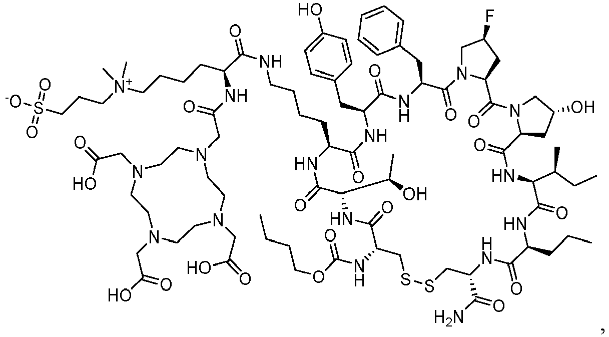
compound Ac-Asp-Leu-[Cys-Cit-Lys(DOTA)-Tyr-Phe-Pro-Arg-Ile-Leu-Cys]-NH₂ (UPAR-423) of the following formula 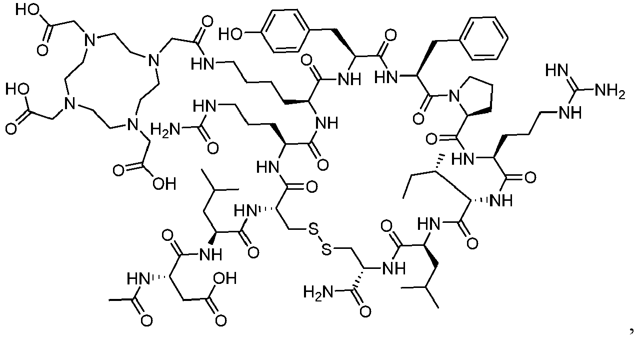
compound Butoc-[Cys-Thr-Lys(DOTA-asp)-Tyr-Phe-4Cfp-Hyp-Ile-Nva-Cys]-NH₂ (UPAR-424) of the following formula 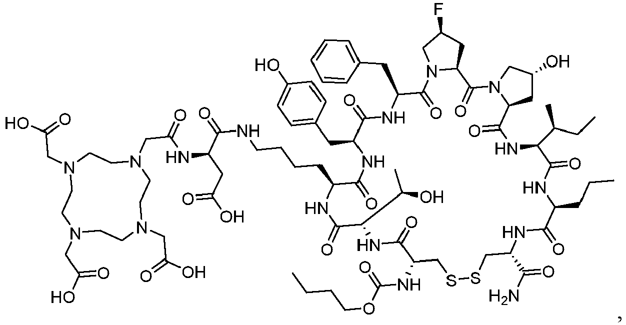
compound Hex-Asp-Leu-[Cys-Arg-Lys(DOTA)-Tyr-Phe-Pro-Ala-Ile-Leu-Cys]-NH₂ (UPAR-427) of the following formula 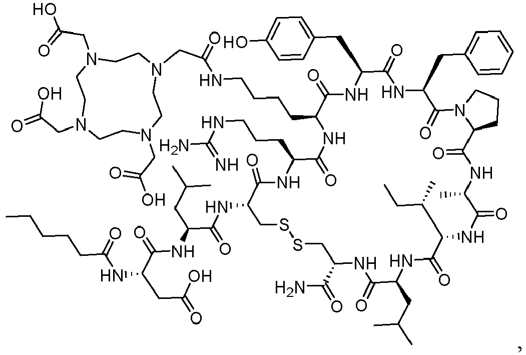
compound H-Nmb-[Cys-Thr-Lys(DOTA)-Tyr-Phe-4Cfp-Hyp-Ile-Nva-Cys]-NH₂ (UPAR-428) of the following formula 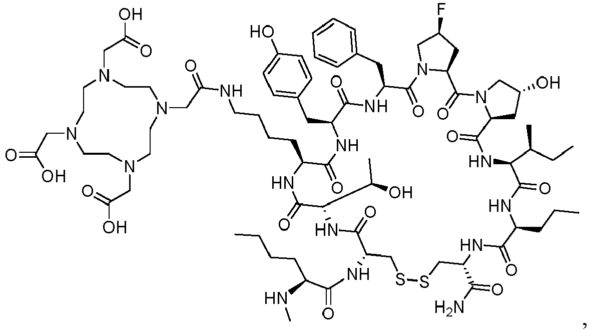
compound Butoc-[Cys-Thr-Lys(NODAGA)-Dopa-Phe-4Cfp-Hyp-Ile-Nva-Cys]-NH₂ (UPAR-444) of the following formula 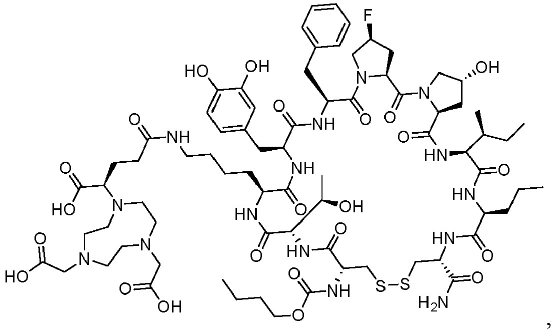
compound Pent-[Cys-Cit-Lys(DOTA)-Tyr-Ocf-Pro-Hyp-Ile-Nva-Cys]-NH₂ (UPAR-450) of the following formula 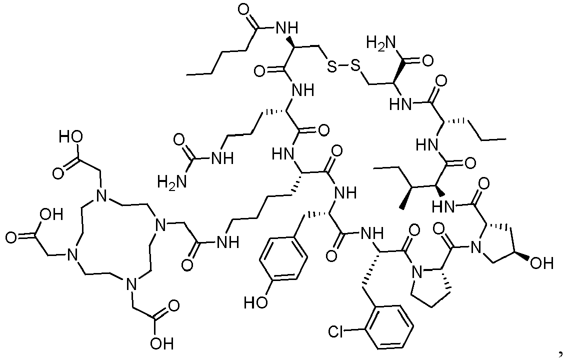
compound iHex-[Cys-Cit-Lys(DOTA)-Tyr-Phe-Pro-Aib-Ile-Leu-Cys]-NH₂ (UPAR-452) of the following formula 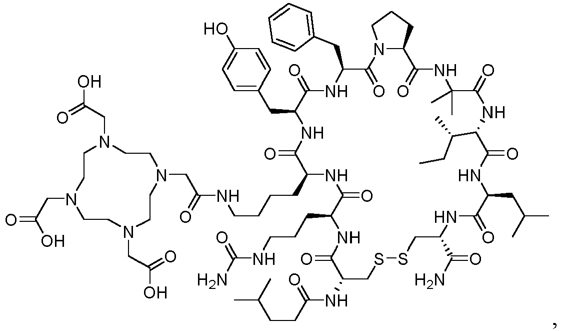
compound Butoc-[Cys-Thr-Dap(DOTA)-Tyr-Phe-4Cfp-Hyp-Ile-Nva-Cys]-NH₂ (UPAR-455) of the following formula 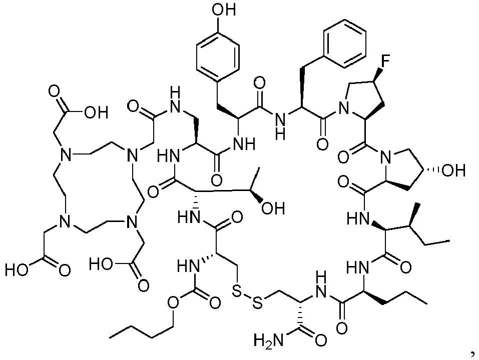
compound Butoc-[Cys-Thr-Dab(DOTA)-Dopa-Phe-4Cfp-Hyp-Ile-Nva-Cys]-NH₂ (UPAR-456) of the following formula 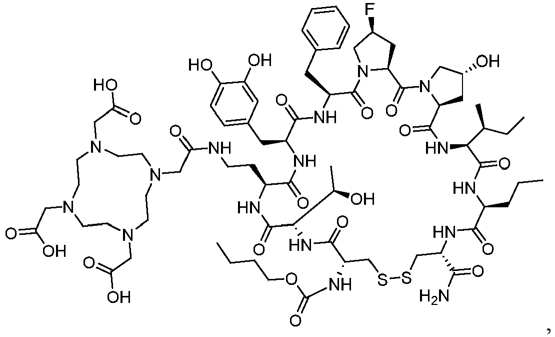
compound Butoc-[Cys-Thr-Lys(DOTA-asp)-Dopa-Phe-4Cfp-Hyp-Ile-Nva-Cys]-NH₂ (UPAR-459) of the following formula 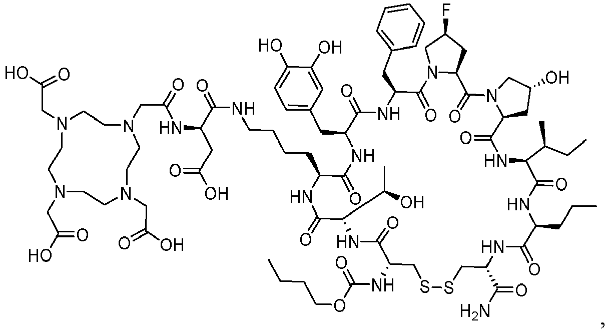
compound iHex-[Cys-Lys(DOTA)-Gln-Tyr-Phe-Pro-Pro-Ile-Leu-Cys]-NH₂ (UPAR-466) of the following formula 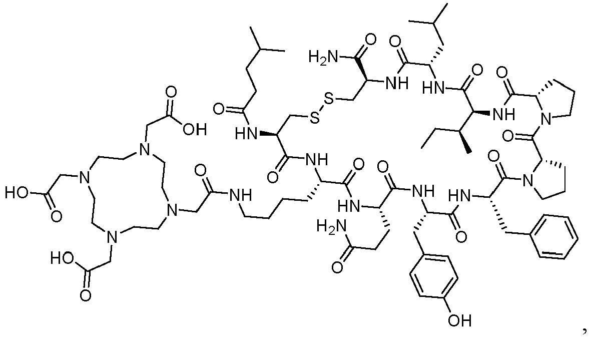
compound Pent-[Cys-Glu-Lys(DOTA)-Tyr-Phe-Pro-Hyp-Ile-Nva-Cys]-NH₂ (UPAR-471) of the following formula 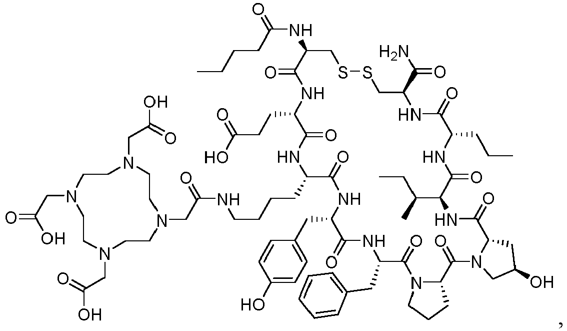
compound Pent-[Cys-Cit-Lys(DOTA)-Tyr-Phe-Pro-Pro-Ile-Nva-Cys]-NH₂ (UPAR-473) of the following formula 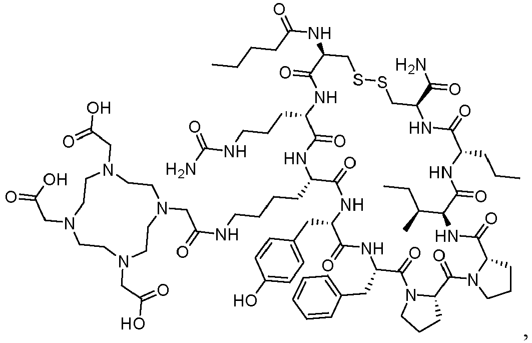
and compound Butoc-[Cys-Cit-Lys(DOTA)-Tyr-Pcf-4Cfp-Hyp-Chg-Nva-Cys]-NH₂ (UPAR-476) of the following formula 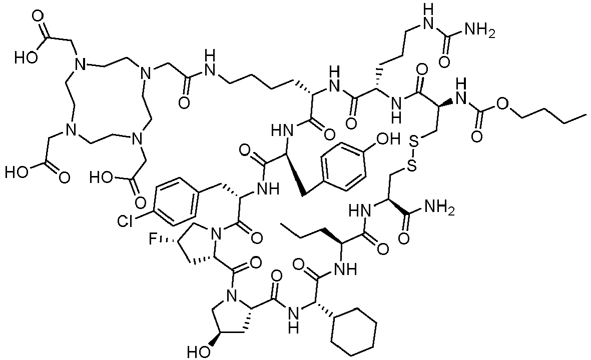 or pharmaceutically acceptable salt or hydrate or pharmaceutically acceptable solvate thereof.

27. The compound or pharmaceutically acceptable salt or hydrate or pharmaceutically acceptable solvate of any one of claims 1 to 26, wherein the compound comprises a diagnostically active nuclide or a therapeutically active nuclide,
wherein if the compound comprises a diagnostically active nuclidepreferably the diagnostically active nuclide is a diagnostically active radionuclide, and more preferably the diagnostically active radionuclide is selected from the group consisting of Al-¹⁸F, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁴⁹Tb, ¹⁵²Tb, ¹⁵⁵Tb, ¹⁶¹Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I, preferably selected from the group consisting of Al-¹⁸F, ⁴⁴Sc, ⁶⁴Cu, ⁶⁷Ga, ⁶⁸Ga, ⁸⁶Y, ⁸⁹Zr, ^{99m}Tc, ¹¹¹In, ²⁰³Pb, and more preferably selected from the group consisting of ⁶⁴Cu, ⁶⁸Ga, ¹¹¹In and ²⁰³Pb; and
wherein if the compound comprises a therapeutically active nuclide, preferably therapeutically active nuclide is a therapeutically active radionuclide, more preferably the therapeutically active radionuclide is selected from the group consisting of ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹²⁵I, ¹³¹I, and ²¹¹At, preferably selected from the group consisting of ⁶⁷Cu, ⁹⁰Y, ¹⁴⁹Tb, ¹⁶¹Tb, ¹⁷⁷Lu, ²¹²Pb, ²¹³Bi, ²²⁵Ac, ²²⁷Th, and more preferably selected from the group consisting of ⁹⁰Y, ¹⁷⁷Lu, ²¹²Pb and ²²⁵Ac.

28. The compound or pharmaceutically acceptable salt or hydrate or pharmaceutically acceptable solvate of any one of claims 1 to 27, wherein the compound interacts with a urokinase plasminogen activator surface receptor (uPAR), preferably with human uPAR having an amino acid sequence of SEQ ID NO: 1 or a homolog thereof, wherein the amino acid sequence of the homolog has an identity of at least 85% to the amino acid sequence of SEQ ID NO: 1; preferably the compound binds or is capable of binding to urokinase plasminogen activator surface receptor (uPAR)

29. The compound or pharmaceutically acceptable salt or hydrate or pharmaceutically acceptable solvate of any one of claims 1 to 28, for use in a method for the diagnosis of a disease or for use in a method for the treatment of a disease, preferably the disease is a disease targetable with a urokinase plasminogen activator surface receptor binding compound or a disease involving or being associated with urokinase plasminogen activator surface receptor (uPAR), preferably upregulated expression of urokinase plasminogen activator surface receptor (uPAR).

30. A composition, preferably a pharmaceutical composition, wherein the composition comprises a compound or pharmaceutically acceptable salt or hydrate or pharmaceutically acceptable solvate of any one of claims 1 to 28 and a pharmaceutically acceptable excipient.

31. A kit comprising a compound according to any one of claims 1 to 28, one or more optional excipient(s) and optionally one or more device(s), whereby the device(s) is/are selected from the group comprising a labeling device, a purification device, a handling device, a radioprotection device, an analytical device or an administration device.
